# EUROPEAN PATENT SPECIFICATION

(11) **EP 2 499 148 B1**
(45) Date of publication and mention of the grant of the patent: **18.01.2017**
(21) Application number: 10743003.5
(22) Date of filing: 03.08.2010
(51) Int. Cl.: C07D 498/04, C07D 498/18, C07F 7/08, A61K 31/4745, A61K 31/695, A61P 1/06, A61P 25/24

(54) **CONFORMATIONALLY CONSTRAINED, FULLY SYNTHETIC MACROCYCLIC COMPOUNDS**
KONFORMATIONSEINGESCHRÄNKTE VOLLSYNTHETSCHE MAKROCYCLISCHE VERBINDUNGEN
COMPOSÉS MACROCYCLIQUES ENTIÈREMENT SYNTHÉTIQUES PRÉSENTANT DES CONTRAINTES CONFORMATIONNELLES

(30) Priority: 05.08.2009 WO PCT/EP2009/060168
(43) Date of publication of application: 19.09.2012
(73) Proprietor: Polyphor AG, 4123 Allschwil (CH)
(72) Inventor: OBRECHT, Daniel, CH-4112 Bättwil (CH); ERMERT, Philipp, CH-4123 Allschwil (CH); OUMOUCH, Said, F-68200 Mulhouse (FR); LACH, Franck, F-51400 Les Grandes Loges (FR); LUTHER, Anatol, 79589 Binzen (DE); MARX, Karsten, CH-4055 Basel (CH); MÖHLE, Kerstin, CH-8907 Wettswil (CH)
(74) Representative: Braun, André jr.
(86) International application number: PCT/CH2010/000191
(87) International publication number: WO 2011/014973

(56) References cited:
- EP-A2- 0 807 639
- WO-A2-01/77113
- CHEN ET AL.: "Synthesis of Novel 4-tert-Alkyl Ether Proline-Based 16- and 17-Membered Macrocyclic Compounds", J. ORG. CHEM., vol. 67, 2002, pages 2730-2733, XP002580115,
- ARASAPPAN ET AL.: "Novel Dipeptide Macrocycles from 4-Oxo, -Thio, and -Amino-Substituted Proline Derivatives", J. ORG. CHEM., vol. 67, 2002, pages 3923-3926, XP002580116,
- CHEN ET AL.: "NOVEL POTENT HEPATITIS C VIRUS NS3 SERINE PROTEASE INHIBITORS DERIVED FROM PROLINE-BASED MACROCYCLES", J. MED. CHEM., vol. 49, 7 January 2006 (2006-01-07), pages 995-1005, XP002447441,
- MARSAULT ET AL.: "Discovery of a new class of macrocylic antagonists to the human motilin receptor", J. MED. CHEM., vol. 49, no. 24, 1 January 2006 (2006-01-01), pages 7190-7197, XP002484961,
- MARSAULT ET AL: "Potent macrocyclic antagonists to the motilin receptor presenting novel unnatural amino acids", BIOORG. MED. CHEM. LETT., vol. 17, no. 15, 1 August 2007 (2007-08-01), pages 4187-4190, XP022144671,
- ROBINSON ET AL.: "The design, structures and therapeutic potential of protein epitope mimetics", DRUG DISCOVERY TODAY, vol. 13, no. 21-22, 1 November 2008 (2008-11-01), pages 944-951, XP025574298,

## Description

Macrocyclic natural and synthetic products have played a crucial role in the development of new drugs, especially as anti-infectives (F. von Nussbaum, M. Brands, B. Hinzen, S. Weigand, D. Häbich, Angew. Chem. Int. Ed. Engl. 2006, 45, 5072-5129; D. Obrecht, J. A. Robinson, F. Bernardini, C. Bisang, S. J. DeMarco, K. Moehle, F. O. Gombert, Curr. Med. Chem. 2009, 16, 42-65), as anti-cancer drugs and in other therapeutic areas (C. E. Ballard, H. Yu, B. Wang, Curr. Med. Chem. 2002, 9, 471-498; F. Sarabia, S. Chammaa, A. S. Ruiz, L. M. Ortiz, F. J. Herrera, Curr. Med. Chem. 2004, 11, 1309-1332). They often display remarkable biological activities, and many macrocycles or their derivatives have been successfully developed into drugs (L. A. Wessjohann, E. Ruijter, D. Garcia-Rivera, W. Brandt, Mol. Divers. 2005, 9, 171-186; D. J. Newman, G. M. Gragg, K. M. Snader, J. Nat. Prod. 2003, 66, 1022-1037). The chemical diversity of macrocyclic natural products is immense and provides a tremendous source of inspiration for drug design.

Compounds with structures, which show some similarity to the structures of the macrocyclic compounds of the invention, are known from EP 0 807 639, which describes cyclic polypeptides having gastrointestinal motor stimulating activity, from Marsault et al. (J. Med. Chem. 2006, 49, 7190-7197), which describes a novel class of macrocyclic peptidomimetics, which are identified and optimized as potent antagonists to the human motilin receptor (hMOT-R) and from Marsault et al. (Bioorg. Med. Chem. Lett. 2007, 17, 4187-4190*),* which describes novel, potent small molecule receptor antagonists composed of a tripeptide cyclized backbone-to-backbone with a nonpeptidic tether and bear new unnatural amino acids containing basic side chains.

Macrocyclic natural and synthetic products are generally classified according to the chemical nature of the backbone, e.g. cyclic peptides (Y. Hamady, T. Shioiri, Chem. Rev. 2005, 105, 4441-4482; N.-H. Tan, J. Zhou, Chem. Rev. 2006, 106, 840-895); cyclic depsipeptides (F. Sarabia, S. Chammaa, A. S. Ruiz, L. M. Ortiz, F. J. Herrera, Curr. Med. Chem. 2004, 11, 1309-1332); macrocyclic lactones (macrolactones) and macrolides; macrocyclic lactams (macrolactams), macrocyclic amines, macrocyclic ethers, macrocyclic ureas and urethanes, and others. The conformational, physico-chemical, pharmacological and pharmacodynamic properties of macrocyclic natural and synthetic compounds depend largely on the *ring size,* the chemical nature of the *backbone,* and of *appended groups* (L. A. Wessjohann, E. Ruijter, D. Garcia-Rivera, W. Brandt, Mol. Divers. 2005, 9, 171-186). By modifying these *three parameters* nature has created a virtually unlimited repertoire of molecular diversity. Despite their undisputed interesting biological properties, many natural products show limitations for drug development, such as:
- High structural complexity
- Low metabolic stability
- Low oral bioavailability
- Low membrane permeability; i.e. intracellular targets not amenable
- Low tissue penetration
- Short half-life
- Chemical synthesis often complex and lengthy
- Often accessible only by fermentation or recombinant methods
- High production costs
- Complex quality control and development processes.

The present invention is defined by the appendant claims. Embodiments and examples of the description which are not covered by the claims are merely aspects of the present disclosure and do not form part of the invention.

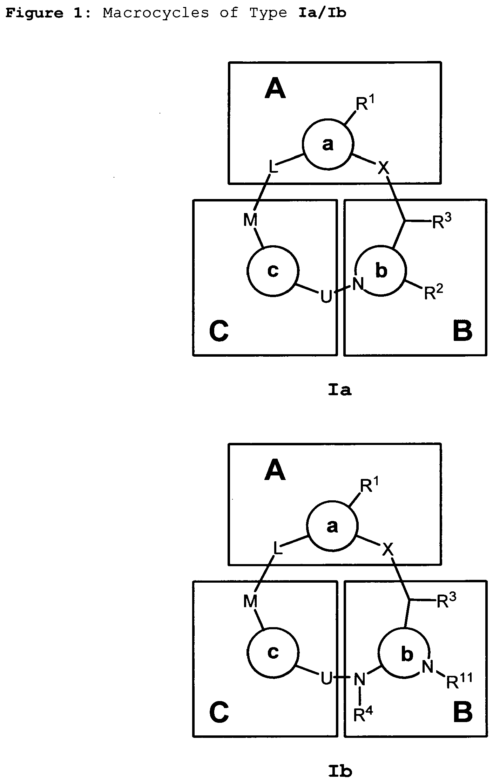

The invention describes now novel, fully synthetic, macrocyclic natural product-like molecules of type **Ia/Ib** (Figure 1), which can be synthesized in a modular approach by connecting suitably protected building blocks **A, B,** and **C** to a linear precursor followed by subsequent cyclization.

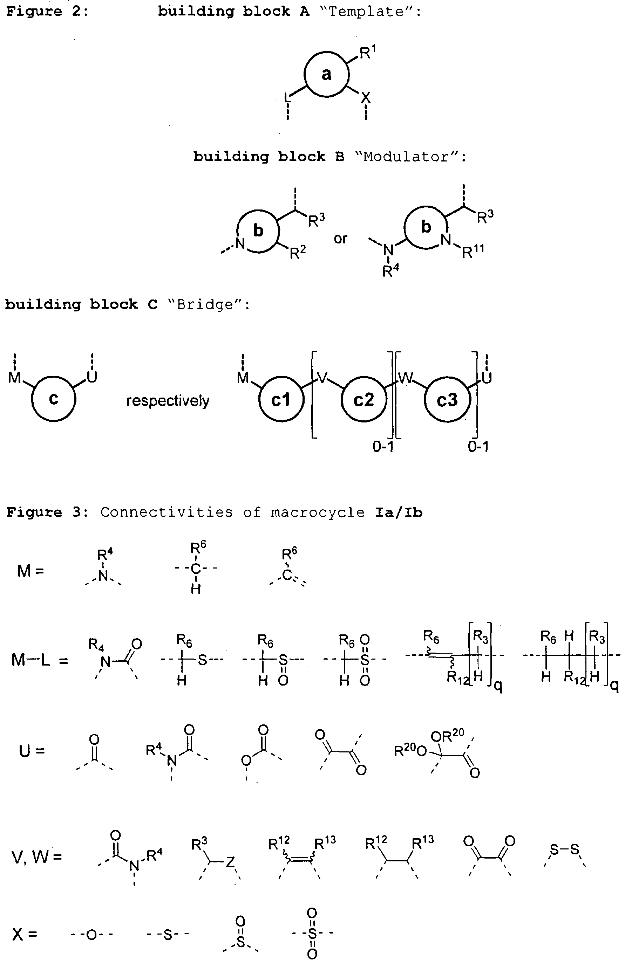

Building blocks **A** (Figure 2) serve as conformation-inducing templates ("Template") and are based on appropriately substituted and protected divalent phenol or thiophenol derivatives.

Building blocks **B** (Figure 2) are corresponding to appropriately substituted and protected tertiary amins.

Building blocks **B** serve as a conformational modulator ("Modulator") by influencing the conformation of the macrocycle, e.g. through *cis*/*trans-*isomerization of amides.

In macrocycles of type **Ia/Ib** the building blocks **A** and **B are** connected via the "Bridge" **C** (Figure 2), which can be constituted by one to three appropriately and independently substituted subunits **c1, c2** and **c3** derived from suitably substituted and protected precursors, most often from, but not limited to, appropriately substituted and protected amino acid or amine derivatives.

The connection of building block **A** to building block **B** occurs via an ether (X=O) or thioether (X=S) bond and to building block C via M-L as detailed below. The sulfur atom of a thioether linkage can easily and selectively be oxidized to the corresponding sulfoxide (S=O) or sulfone (S(=O)₂) both of which are therefore a part of the invention (Figur3).

The functional moiety U connects the bridge **C** with the nitrogen atom of modulator **B.** In most cases this is realized by an amide bond (secondary or tertiary), in which case the moiety U corresponds to a carbonyl group (-C(=O)-). Alternatively, U can be defined as a carbamoyl moiety (-NR⁴-C(=O)-) which corresponds to a urea moiety (including the N-atom of **B)** as functional connection between **B and C.** Similarly a carboxyl group (-O-C(=O)-) as U describes a carbamate linkage between **B** and **C.** In addition, U can represent an oxalyl group (-C(=O)-C(=O)-) or the corresponding acetal (-C(-OR²⁰)₂-C(=O)-).

Importantly, in case that R² of building block **B** constitutes an amine substituent, two alternatives for linking building block **B** with building block **C** is possible: Either the standard link via the heterocyclic nitrogen atom or, alternatively, the exocyclic amine functionality. This is reflected either by formula **Ia** or formula **Ib,** respectively, which are both integral part of this invention sharing equally common technical features.

The connectivity between **C** and **A** is described in a generic way with the functional unit M-L, which corresponds in most cases to a secondary or tertiary amide bond (M-L = -NR⁴-C(=O)-). Alternative connections of M-L are thioethers (-CHR⁶-S-) and its oxidation products, i.e. sulfoxides (-CHR⁶-S(=O)-) or sulfones (-CHR⁶-S(=O)₂-), as well as olefinic moieties (-CR⁶=CR¹²-(CHR³)q-) and their reduced forms, the aliphatic groups (-CHR⁶-CHR¹²-(CHR³)q-).

As already mentioned, the bridge **C** in turn can be constituted by one to three appropriately and independently substituted subunits **c1, c2** and **c3.** These subunits **c1** to **c3** are independently connected to each other by the generic groups V and W which can correspond to an amide bond (-C(=O)NR⁴), a methylene-heteroatom linkage (-CHR³-Z-), an alkene[1,2]diyl moiety (-CHR¹²=CHR¹³-), introduced by olefin metathesis, an alkane[1,2]diyl spacer (-CHR¹²-CHR¹³-), accessible from the metathesis product by hydrogenation, an oxalyl group (-C(=O)-C(=O)-) or a disulfide bridge (-S-S-).

The spatial orientation of the substituents in macrocycles of type **Ia/Ib** is modulated by the ring size and the stereochemical connectivity within building blocks **A,** B and **C.** Both, the macrocyclic backbone and the substituents can contribute to the biological activity of compounds of type **Ia/Ib.**

For most examples the backbone of macrocycles **Ia/Ib** contains an aromatic ether/thioether linkage and one or more tertiary amide bonds; in other cases a secondary amide bond, an aliphatic ether linkage, an ethylidene or an ethylene moiety may be part of the backbone as defined above. Ether linkages in macrocyclic molecules have been shown to be beneficial by favorably influencing physico-chemical and pharmacological properties, such as solubility in aqueous solutions, metabolic stability against proteolytic degradation, cell permeability and oral absorption (K. X. Chen et al., J. Med. Chem. 2006, 49, 995-1005). In addition, tertiary amide containing macrocycles show increased proteolytic stability, cell permeability and oral bioavailability compared to the parent molecules with secondary amide bonds (E. Biron, J. Chatterjee, O. Ovadia, D. Langenegger, J. Brueggen, D. Hoyer, H. A. Schmid, R. Jelinek, C. Gilon, A. Hoffmann, H. Kessler, Angew. Chem. Int. Ed. 2008, 47, 1-6; J. Chatterjee, O. Ovadia, G. Zahn, L. Marinelli, A. Hoffmann, C. Gilon, H. Kessler, J. Med. Chem. 2007, 50, 5878-5881). For example, the cyclic undecapeptide cyclosporin A (INN: Ciclosporin), which is used as immunosuppressant in organ transplants, contains seven N-methylated amino acids and possesses good oral bioavailability when formulated appropriately (P. R. Beauchesne, N. S. C. Chung, K. M. Wasan, Drug Develop. Ind. Pharm. 2007, 33, 211-220). Peptidyl *cis*/*trans* isomerization of proline or pipecolic acid containing polypeptides and proteins is a well known process in protein folding events. *In vivo,* this process can be mediated by peptidyl prolyl *cis*/*trans* isomerases such as the cyclophilins, the FK506-binding proteins and the parvulins (A. Bell, P. Monaghan, A. P. Page, Int. J. Parasitol. 2006, 36, 261-276). Besides their role in protein folding and in the immune system, peptidyl prolyl *cis*/*trans* isomerases have been implicated in cell cycle control (P. E. Shaw, EMBO Reports 2002, 3, 521-526) and therefore constitute interesting pharmaceutical targets. FK506 and cyclosporin A which bind to the FK506-binding protein and cyclophilins, respectively, are both macrocyclic natural products, with the former one containing a pipecolic acid residue.

For many existing and emerging biological targets it is difficult to find classical small molecule hits as starting points for drug development (J. A. Robinson, S. DeMarco, F. Gombert, K. Moehle, D. Obrecht, Drug Disc. Today 2008, 13, 944-951). Many of these extra- and intracellular "difficult targets" involve protein-protein interactions, such as receptor tyrosine kinases, growth factor receptors, transcription modulators, chaperones, and others. For several of them macrocyclic natural and synthetic compounds have been described as good starting points for drug discovery programs (e.g. D. Obrecht, J. A. Robinson, F. Bernardini, C. Bisang, S. J. DeMarco, K. Moehle, F. O. Gombert, Curr. Med. Chem. 2009, 16, 42-65).

The novel and fully synthetic macrocyclic compounds of type **Ia/Ib** described in the embodiments of this invention combine unique features of macrocyclic natural products with beneficial physico-chemical and pharmacological properties of small molecules, like:
- Natural product-like structural complexity
- Good solubility
- High metabolic stability
- Improved oral bioavailability
- Improved membrane permeability
- Extra- and intracellular targets amenable
- Improved tissue penetration
- Small molecule-like pharmacokinetics
- Modular chemical synthesis
- Synthesis process well suited for parallelization
- Reasonable production costs
- Small molecule-like QC and development processes

The main embodiment of the current invention of novel and fully synthetic macrocyclic compounds of type **Ia/Ib** according Figure 1 (detailed in Figure 2 and 3) is defined by groups of selected building blocks **A, B** and **C** as shown in Table 1 to 3 and by the appending substituents R¹-R⁵³ as detailed below.

**Building block A** is a bivalent radical selected from the group of Table 1.

As mentioned hereinabove, building blocks of type **A** act as templates and exert an important conformational constraint on products of type **Ia/Ib.** The structural effects of building blocks of type **A** depend largely on the relative orientation of the attachment vectors of -X- and -L- and on the spatial distance between these groups. Molecular modeling, performed on the prevalent examples wherein -L- is -C(=O)-, clearly indicates that distances (typically between 2.5 and 7.5 Å) and vector arrangements for -X- and -C(=O)- in **A1-A683** vary considerably, thus strongly influencing the conformations of macrocycles of type **Ia/Ib.**

**Table 1: Radicals A1-A683 (continued on the following pages)**

| | | | |
|---|---|---|---|
| 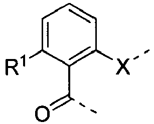 | 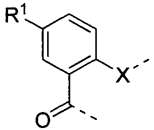 | 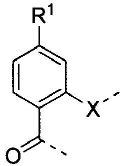 | 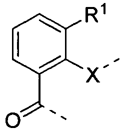 |
| **A1** (a1) | **A2** (a1) | **A3** (a1) | **A4 3**(a1) |
| 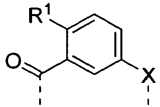 | 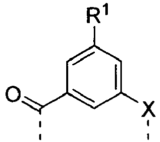 | 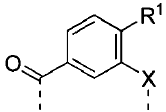 | 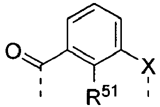 |
| **A5** (a1) | **A6** (a1) | **A7** (a1) | **A8** (a1) |
| 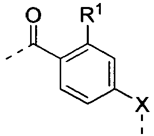 | 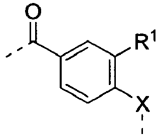 | 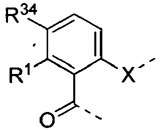 | 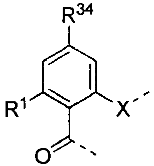 |
| **A9** (a1) | **A10** (a1) | **A11** (a1) | **A12** (a1) |
| 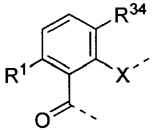 | 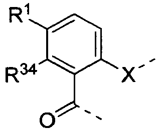 | 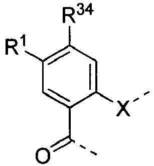 | 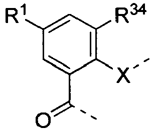 |
| **A13** (a1) | **A14** (a1) | **A15** (a1) | **A16** (a1) |
| 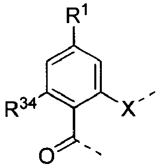 | 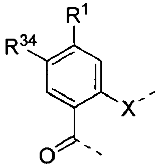 | 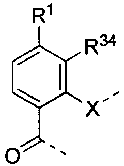 | 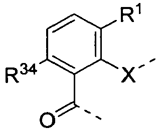 |
| **A17** (a1) | **A18** (a1) | **A19** (a1) | **A20** (a1) |
| 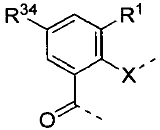 | 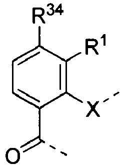 | 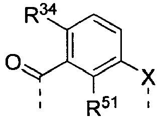 | 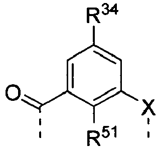 |
| **A21** (a1) | **A22** (a1) | **A23** (a1) | **A24** (a1) |
| 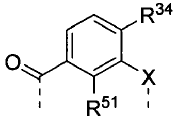 | 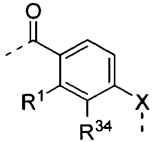 | 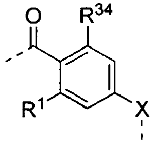 | 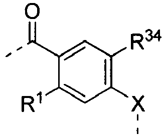 |
| **A25** (a1) | **A26** (a1) | **A27** (a1) | **A28** (a1) |
| 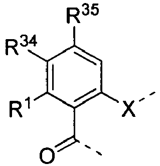 | 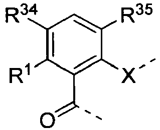 | 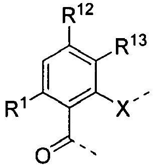 | 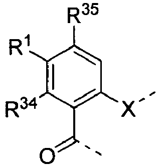 |
| **A29** (a1) | **A30** (a1) | **A31** (a1) | **A32** (a1) |
| 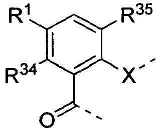 | 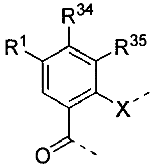 | 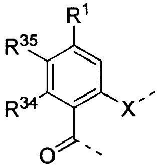 | 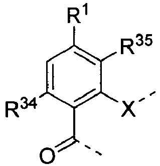 |
| **A33** (a1) | **A34** (a1) | **A35** (a1) | **A36** (a1) |
| 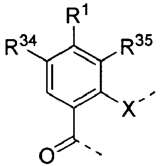 | 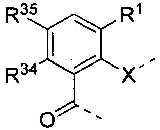 | 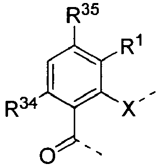 | 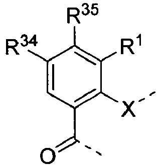 |
| **A37** (a1) | **A38** (a1) | **A39** (a1) | **A40** (a1) |
| 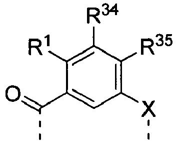 | 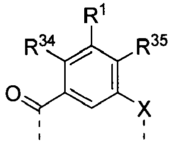 | 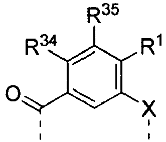 | 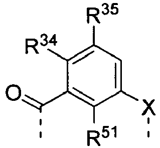 |
| **A41** (a1) | **A42** (a1) | **A43** (a1) | **A44** (a1) |
| 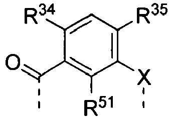 | 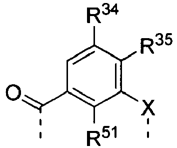 | 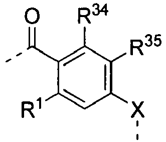 | 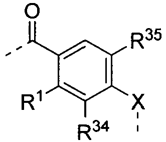 |
| **A45** (a1) | **A46** (a1) | **A47** (a1) | **A48** (a1) |
| 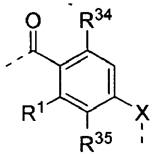 | 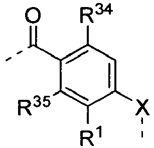 | 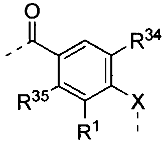 | 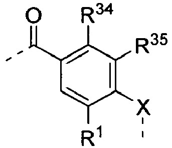 |
| **A49** (a1) | **A50** (a1) | **A51** (a1) | **A52** (a1) |
| 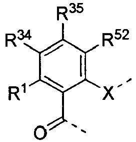 | 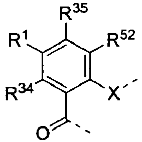 | 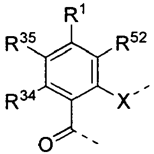 | 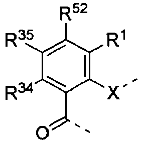 |
| **A53** (a1) | **A54** (a1) | **A55** (a1) | **A56** (a1) |
| 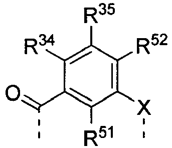 | 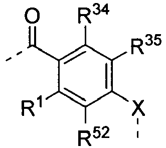 | 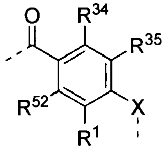 | 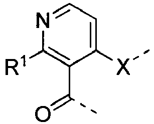 |
| **A57** (a1) | **A58** (a1) | **A59** (a1) | **A60** (a2) |
| 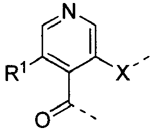 | 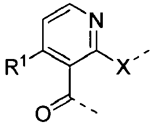 | 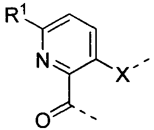 | 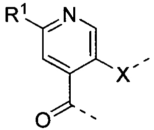 |
| **A61** (a2) | **A62** (a2) | **A63** (a2) | **A64** (a2) |
| 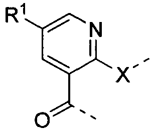 | 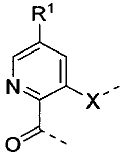 | 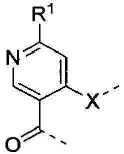 | 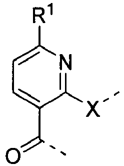 |
| **A65** (a2) | **A66** (a2) | **A67** (a2) | **A68** (a2) |
| 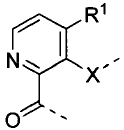 | 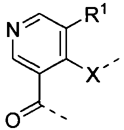 | 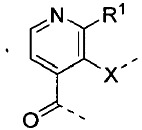 | 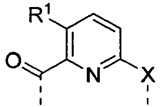 |
| **A69** (a2) | **A70** (a2) | **A71** (a2) | **A72** (a2) |
| 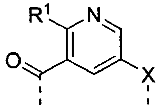 | 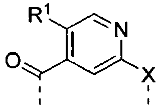 | 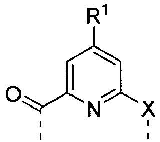 | 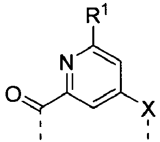 |
| **A73** (a2) | **A74** (a2) | **A75** (a2) | **A76** (a2) |
| 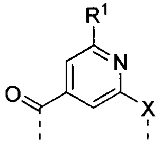 | 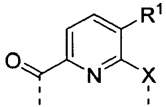 | 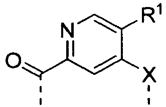 | 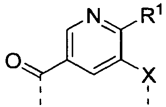 |
| **A77** (a2) | **A78** (a2) | **A79** (a2) | **A80** (a2) |
| 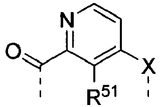 | 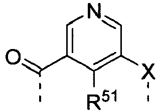 | 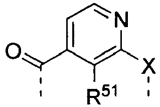 | 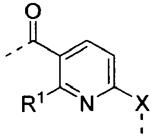 |
| **A81** (a2) | **A82** (a2) | **A83** (a2) | **A84** (a2) |
| 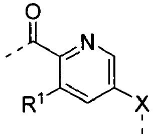 | 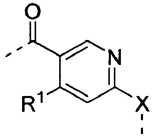 | 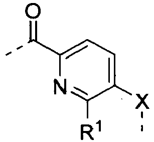 | 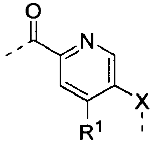 |
| **A85** (a2) | **A86** (a2) | **A87** (a2) | **A88** (a2) |
| 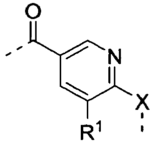 | 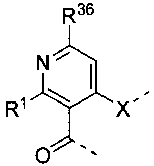 | 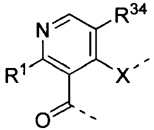 | 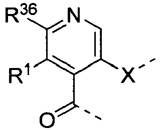 |
| **A89** (a2) | **A90** (a2) | **A91** (a2) | **A92** (a2) |
| 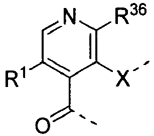 | 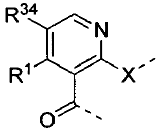 | 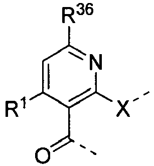 | 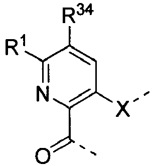 |
| **A93** (a2) | **A94** (a2) | **A95** (a2) | **A96** (a2) |
| 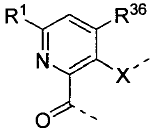 | 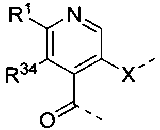 | 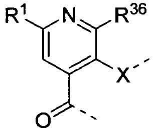 | 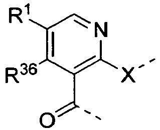 |
| **A97** (a2) | **A98** (a2) | **A99** (a2) | **A100** (a2) |
| 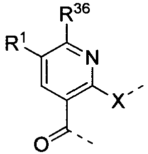 | 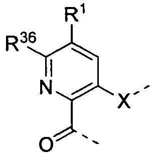 | 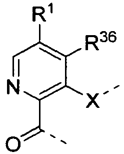 | 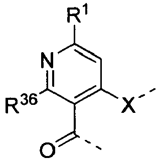 |
| **A101** (a2) | **A102** (a2) | **A103** (a2) | **A104** (a2) |
| 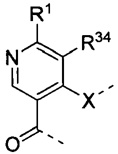 | 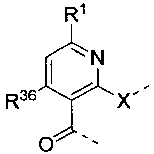 | 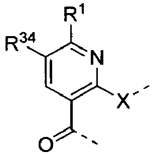 | 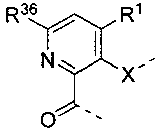 |
| **A105** (a2) | **A106** (a2) | **A107** (a2) | **A108** (a2) |
| 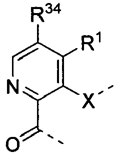 | 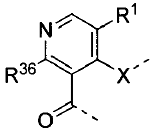 | 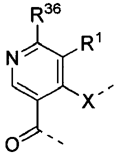 | 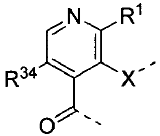 |
| **A109** (a2) | **A110** (a2) | **A111** (a2) | **A112** (a2) |
| 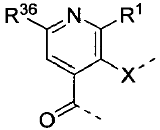 | 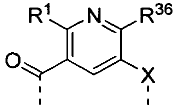 | 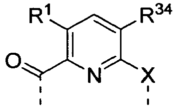 | 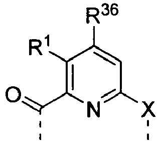 |
| **A113** (a2) | **A114** (a2) | **A115** (a2) | **A116** (a2) |
| 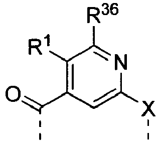 | 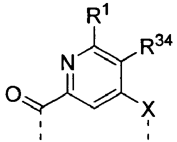 | 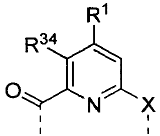 | 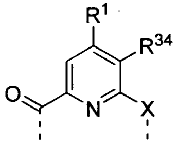 |
| **A117** (a2) | **A118** (a2) | **A119** (a2) | **A120** (a2) |
| 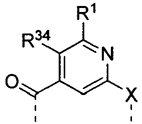 | 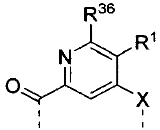 | 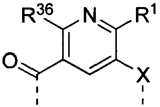 | 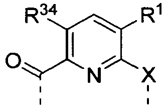 |
| **A121** (a2) | **A122** (a2) | **A123** (a2) | **A124** (a2) |
| 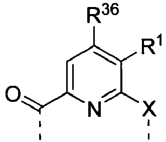 | 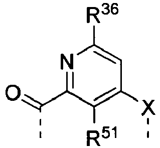 | 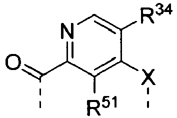 | 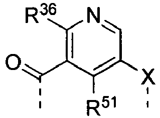 |
| **A125** (a2) | **A126** (a2) | **A127** (a2) | **A128** (a2) |
| 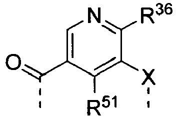 | 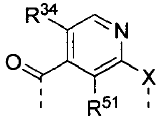 | 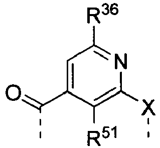 | 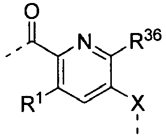 |
| **A129** (a2) | **A130** (a2) | **A131** (a2) | **A132** (a2) |
| 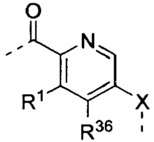 | 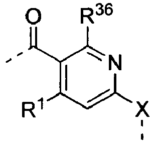 | 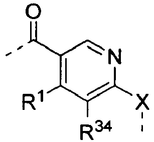 | 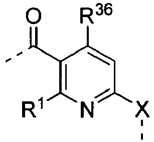 |
| **A133** (a2) | **A134** (a2) | **A135** (a2) | **A136** (a2) |
| 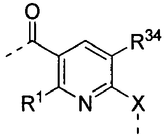 | 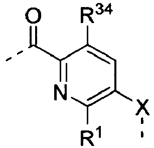 | 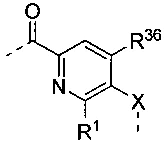 | 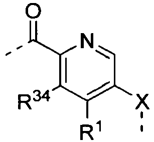 |
| **A137** (a2) | **A138** (a2) | **A139** (a2) | **A140** (a2) |
| 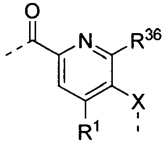 | 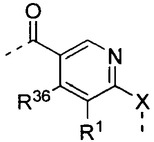 | 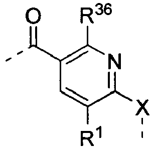 | 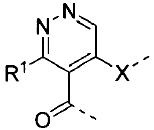 |
| **A141** (a2) | **A142** (a2) | **A143** (**a2**) | **A144** (a3) |
| 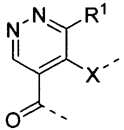 | 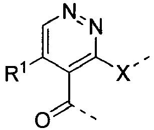 | 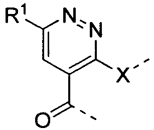 | 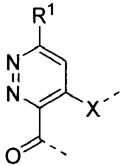 |
| **A145** (a3) | **A146** (a3) | **A147** (a3) | **A148** (a3) |
| 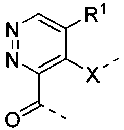 | 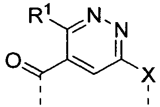 | 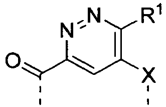 | 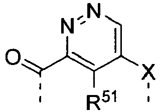 |
| **A149** (a3) | **A150** (a3) | **A151** (a3) | **A152** (a3) |
| 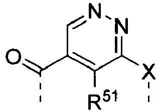 | 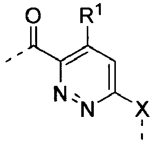 | 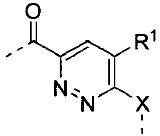 | 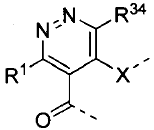 |
| **A153** (a3) | **A154** (a3) | **A155** (a3) | **A156** (a3) |
| 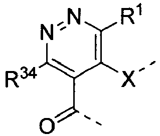 | 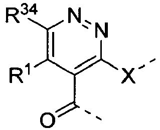 | 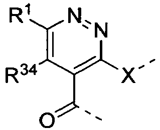 | 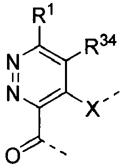 |
| **A157** (a3) | **A158** (a3) | **A159** (a3) | **A160** (a3) |
| 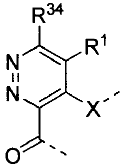 | 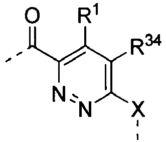 | 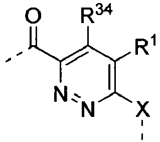 | 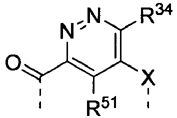 |
| **A161** (a3) | **A162** (a3) | **A163** (a3) | **A164** (a3) |
| 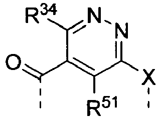 | 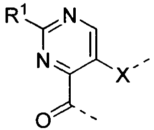 | 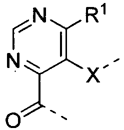 | 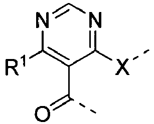 |
| **A165** (a3) | **A166** (a4) | **A167** (a4) | **A168** (a4) |
| 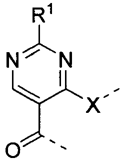 | 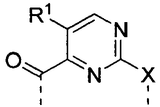 | 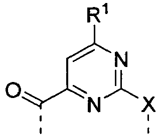 | 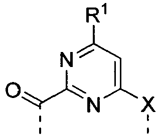 |
| **A169** (a4) | **A170** (a4) | **A171** (a4) | **A172** (a4) |
| 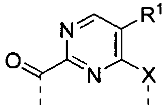 | 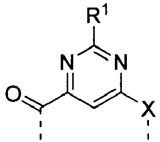 | 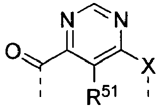 | 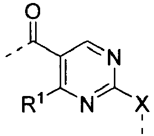 |
| **A173** (a4) | **A174** (a4) | **A175** (a4) | **A176** (a4) |
| 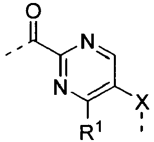 | 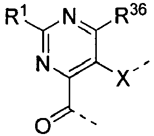 | 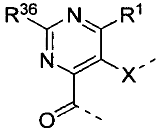 | 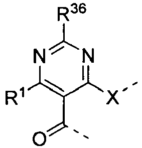 |
| **A177** (a4) | **A178** (a4) | **A179** (a4) | **A180** (a4) |
| 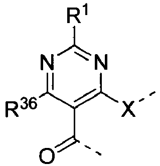 | 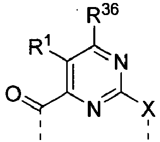 | 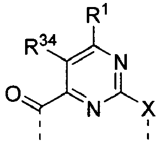 | 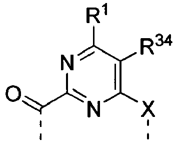 |
| **A181** (a4) | **A182** (a4) | **A183** (a4) | **A184** (a4) |
| 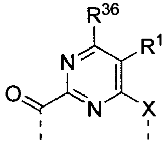 | 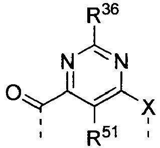 | 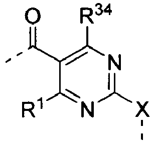 | 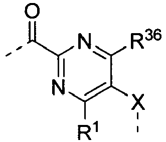 |
| **A185** (a4) | **A186** (a4) | **A187** (a4) | **A188** (a4) |
| 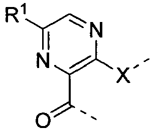 | 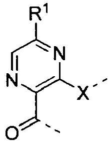 | 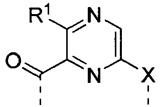 | 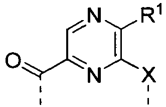 |
| **A189** (a5) | **A190** (a5) | **A191** (a5) | **A192** (a5) |
| 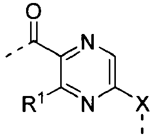 | 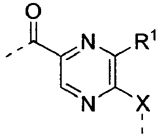 | 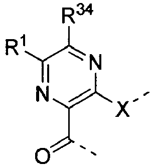 | 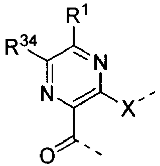 |
| **A193** (a5) | **A194** (a5) | **A195** (a5) | **A196** (a5) |
| 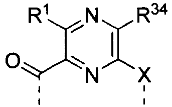 | 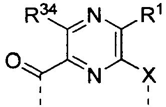 | 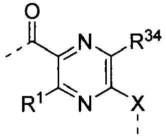 | 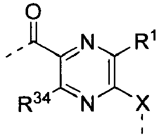 |
| **A197** (a5) | **A198** (a5) | **A199** (a5) | **A200** (a5) |
| 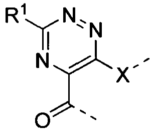 | 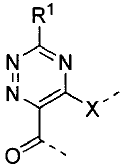 | 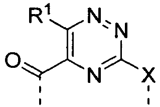 | 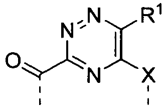 |
| **A201** (a6) | **A202** (a6) | **A203** (a6) | **A204** (a6) |
| 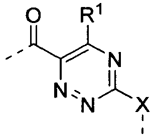 | 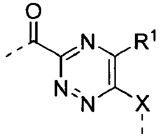 | 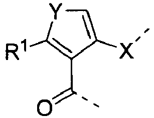 | 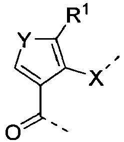 |
| **A205** (a6) | **A206** (a6) | **A207** (a7) | **A208** (a7) |
| 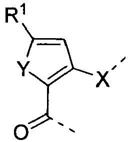 | 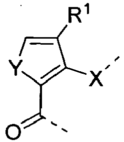 | 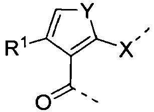 | 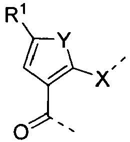 |
| **A209** (a7) | **A210** (a7) | **A211** (a7) | **A212** (a7) |
| 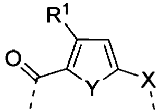 | 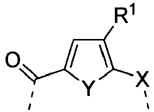 | 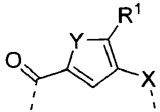 | 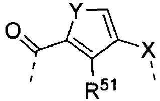 |
| **A213** (a7) | **A214** (a7) | **A215** (a7) | **A216** (a7) |
| 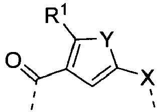 | 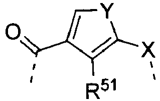 | 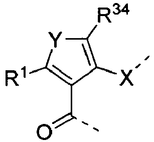 | 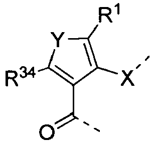 |
| **A217** (a7) | **A218** (a7) | **A219** (a7) | **A220** (a7) |
| 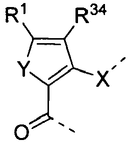 | 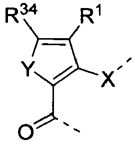 | 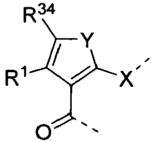 | 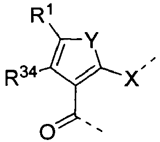 |
| **A221** (a7) | **A222** (a7) | **A223** (a7) | **A224** (a7) |
| 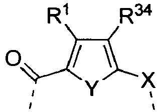 | 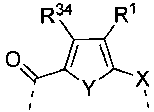 | 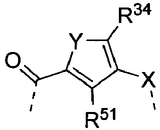 | 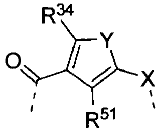 |
| **A225** (a7) | **A226** (a7) | **A227** (a7) | **A228** (a7) |
| 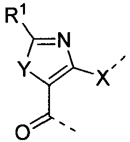 | 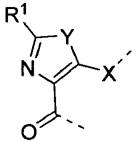 | 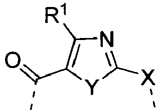 | 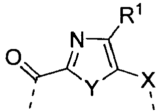 |
| **A229** (a8) | **A230** (a8) | **A231** (a8) | **A232** (a8) |
| 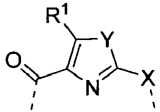 | 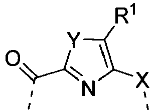 | 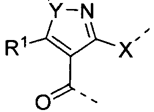 | 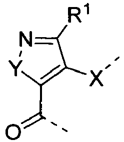 |
| **A233** (a8) | **A234** (a8) | **A235** (a9) | **A236** (a9) |
| 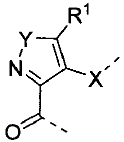 | 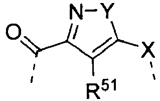 | 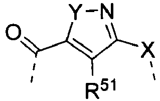 | 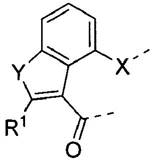 |
| **A237** (a9) | **A238** (a9) | **A239** (a9) | **A240** (a10) |
| 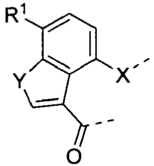 | 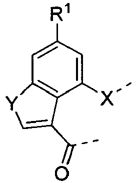 | 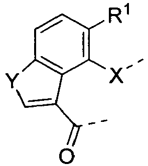 | 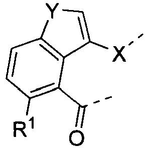 |
| **A241** (a10) | **A242** (a10) | **A243** (a10) | **A244** (a10) |
| 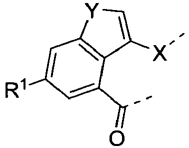 | 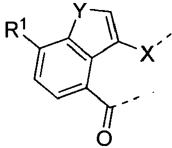 | 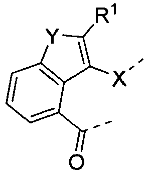 | 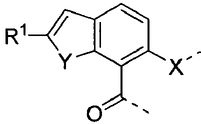 |
| **A245** (a10) | **A246** (a10) | **A247** (a10) | **A248** (a10) |
| 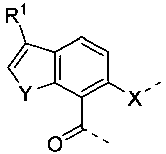 | 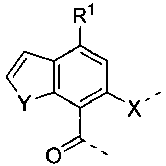 | 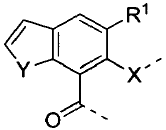 | 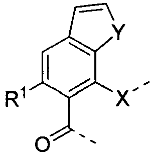 |
| **A249** (a10) | **A250** (a10) | **A251** (a10) | **A252** (a10) |
| 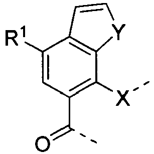 | 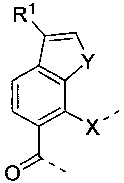 | 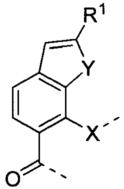 | 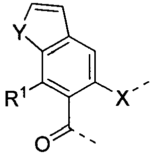 |
| **A253** (a10) | **A254** (a10) | **A255** (a10) | **A256** (a10) |
| 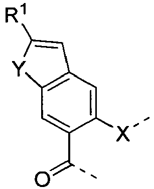 | 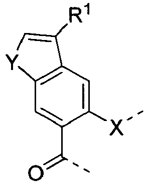 | 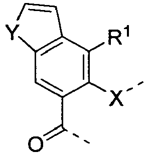 | 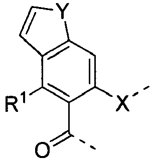 |
| **A257** (a10) | **A258** (a10) | **A259** (a10) | **A260** (a10) |
| 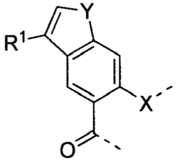 | 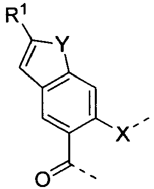 | 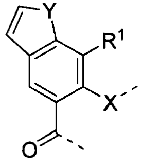 | 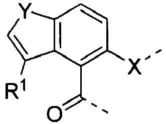 |
| **A261** (a10) | **A262** (a10) | **A263** (a10) | **A264** (a10) |
| 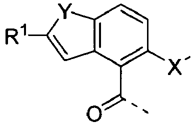 | 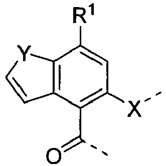 | 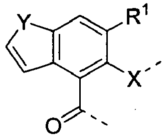 | 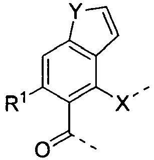 |
| **A265** (a10) | **A266** (a10) | **A267** (a10) | **A268** (a10) |
| 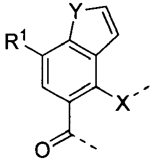 | 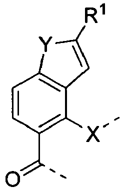 | 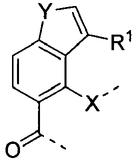 | 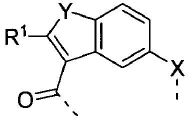 |
| **A269** (a10) | **A270** (a10) | **A271** (a10) | **A272** (a10) |
| 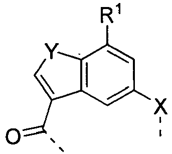 | 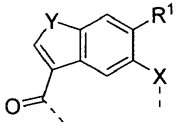 | 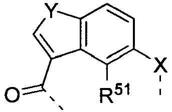 | 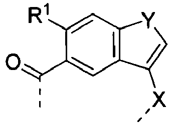 |
| **A273** (a10) | **A274** (a10) | **A275** (a10) | **A276** (a10) |
| 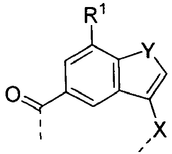 | 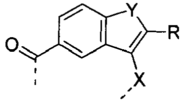 | 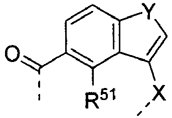 | 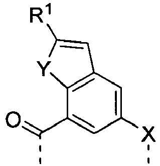 |
| **A277** (a10) | **A278** (a10) | **A279** (a10) | **A280** (a10) |
| 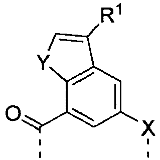 | 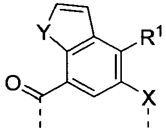 | 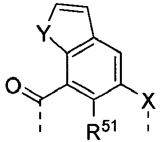 | 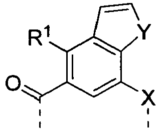 |
| **A281** (a10) | **A282** (a10) | **A283** (a10) | **A284** (a10) |
| 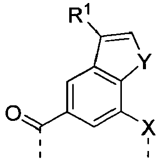 | 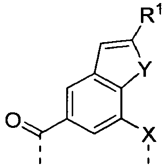 | 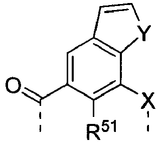 | 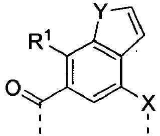 |
| **A285** (a10) | **A286** (a10) | **A287** (a10) | **A288** (a10) |
| 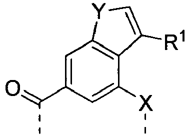 | 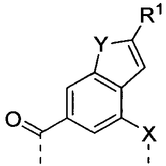 | 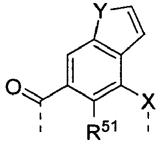 | 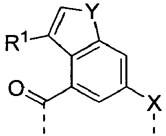 |
| **A289** (a10) | **A290** (a10) | **A291** (a10) | **A292** (a10) |
| 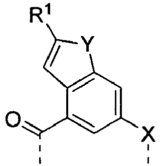 | 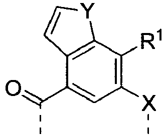 | 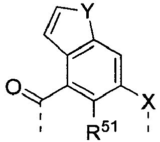 | 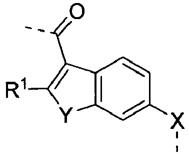 |
| **A293** (a10) | **A294** (a10) | **A295** (a10) | **A296** (a10) |
| 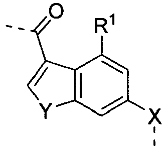 | 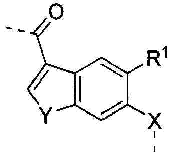 | 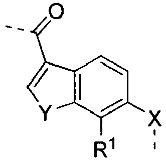 | 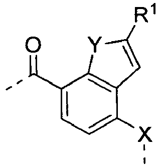 |
| **A297** (a10) | **A298**(a10) | **A299** (a10) | **A300** (a10) |
| 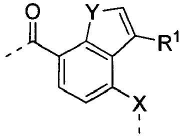 | 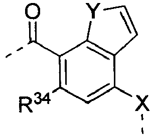 | 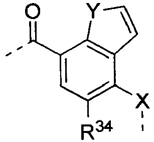 | 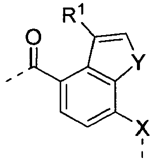 |
| **A301** (a10) | **A302** (a10) | **A303** (a10) | **A304** (a10) |
| 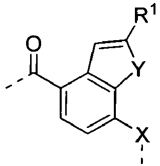 | 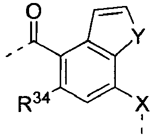 | 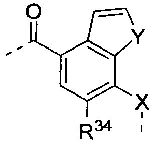 | 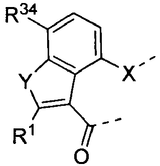 |
| **A305** (a10) | **A306** (a10) | **A307** (a10) | **A308** (a10) |
| 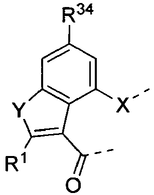 | 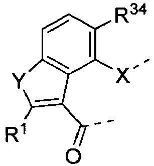 | 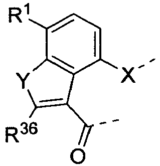 | 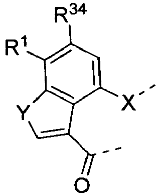 |
| **A309** (a10) | **A310** (a10) | **A311** (a10) | **A312** (a10) |
| 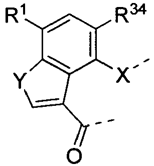 | 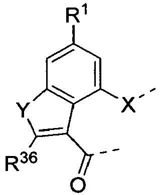 | 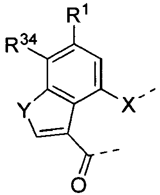 | 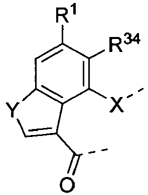 |
| **A313** (a10) | **A314** (a10) | **A315** (a10) | **A316** (a10) |
| 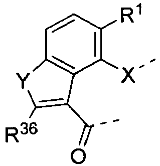 | 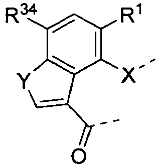 | 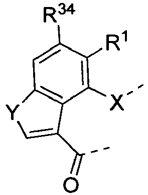 | 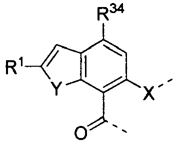 |
| **A317** (a10) | **A318** (a10) | **A319** (a10) | **A320** (a10) |
| 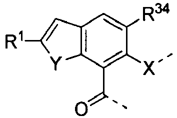 | 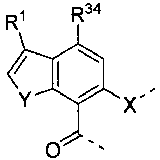 | 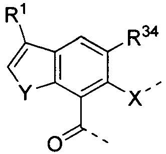 | 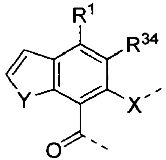 |
| **A321** (a10) | **A322** (a10) | **A323** (a10) | **A324** (a10) |
| 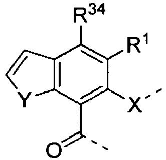 | 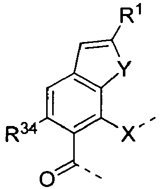 | 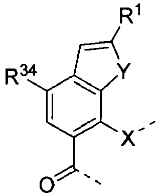 | 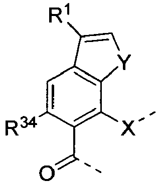 |
| **A325** (a10) | **A326** (a10) | **A327** (a10) | **A328** (a10) |
| 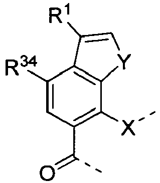 | 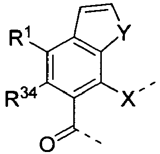 | 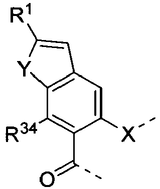 | 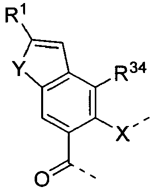 |
| **A329** (a10) | **A330** (a10) | **A331** (a10) | **A332** (a10) |
| 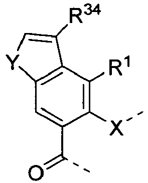 | 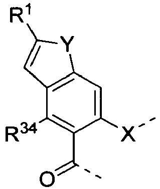 | 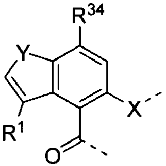 | 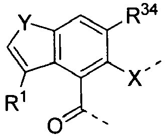 |
| **A333** (a10) | **A334** (a10) | **A335** (a10) | **A336** (a10) |
| 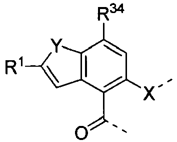 | 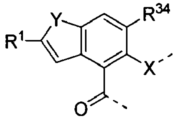 | 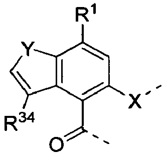 | 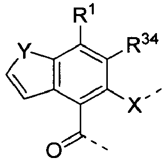 |
| **A337** (a10) | **A338** (a10) | **A339** (a10) | **A340** (a10) |
| 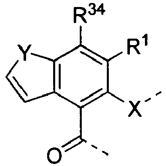 | 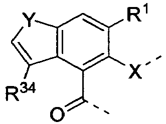 | 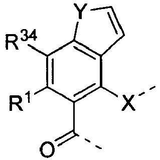 | 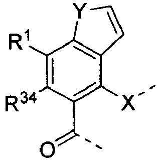 |
| **A341** (a10) | **A342** (a10) | **A343** (a10) | **A344** (a10) |
| 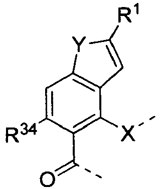 | 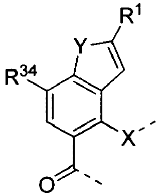 | 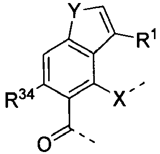 | 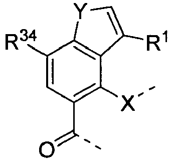 |
| **A345** (a10) | **A346** (a10) | **A347** (a10) | **A348** (a10) |
| 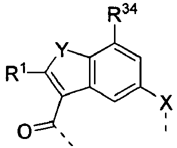 | 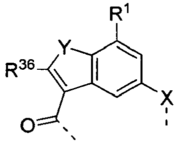 | 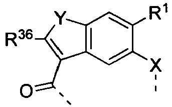 | 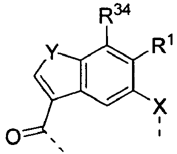 |
| **A349** (a10) | **A350** (a10) | **A351** (a10) | **A352** (a10) |
| 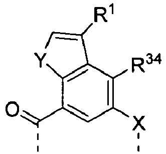 | 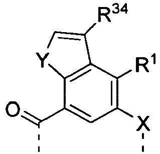 | 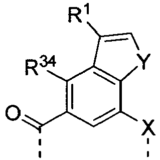 | 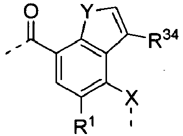 |
| **A353** (a10) | **A354** (a10) | **A355** (a10) | **A356** (a10) |
| 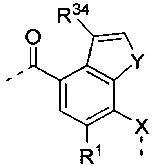 | 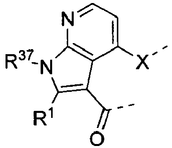 | 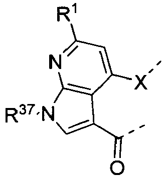 | 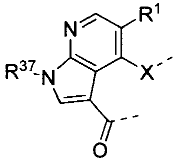 |
| **A357** (a10) | **A358** (a11) | **A359** (a11) | **A360** (a11) |
| 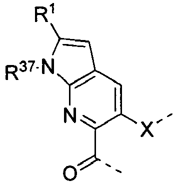 | 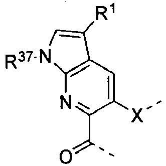 | 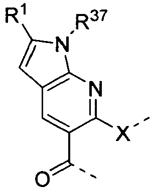 | 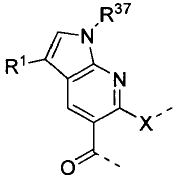 |
| **A361** (a11) | **A362** (a11) | **A363** (a11) | **A364** (a11) |
| 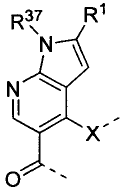 | 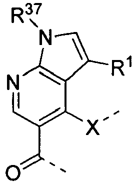 | 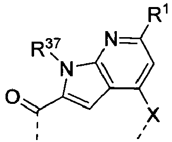 | 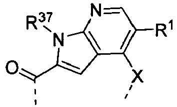 |
| **A365** (a11) | **A366** (a11) | **A367** (a11) | **A368** (a11) |
| 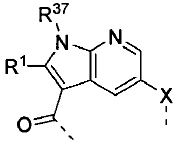 | 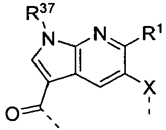 | 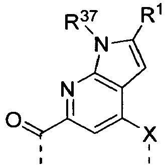 | 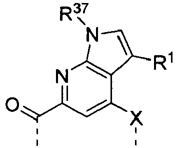 |
| **A369** (a11) | **A370** (a11) | **A371** (a11) | **A372** (a11) |
| 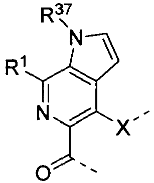 | 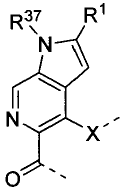 | 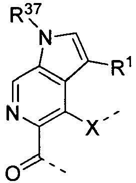 | 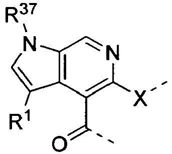 |
| **A373** (a12) | **A374** (a12) | **A375** (a12) | **A376** (a12) |
| 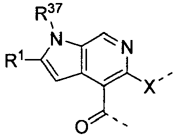 | 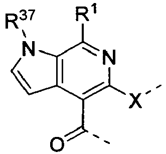 | 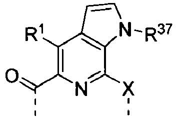 | 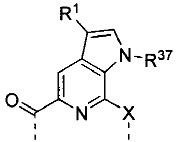 |
| **A377** (a12) | **A378** (a12) | **A379** (a12) | **A380** (a12) |
| 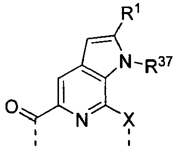 | 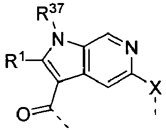 | 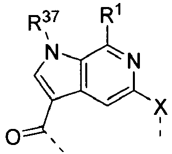 | 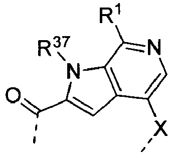 |
| **A381** (a12) | **A382** (a12) | **A383** (a12) | **A384** (a12) |
| 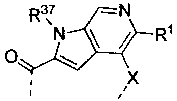 | 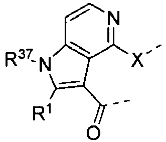 | 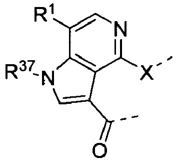 | 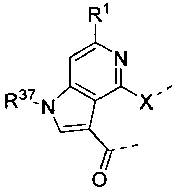 |
| **A385** (a12) | **A386** (a13) | **A387** (a13) | **A388** (a13) |
| 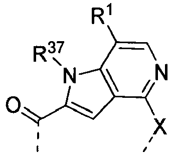 | 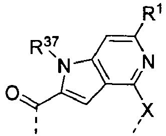 | 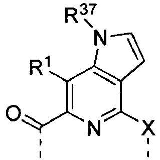 | 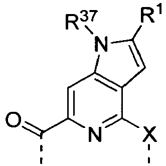 |
| **A389** (a13) | **A390** (a13) | **A391** (a13) | **A392** (a13) |
| 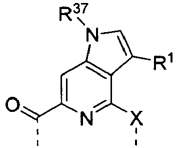 | 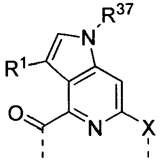 | 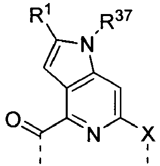 | 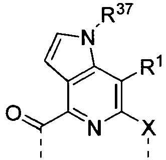 |
| **A393** (a13) | **A394** (a13) | **A395** (a13) | **A396** (a13) |
| 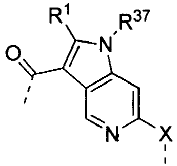 | 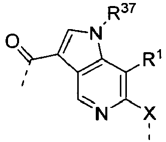 | 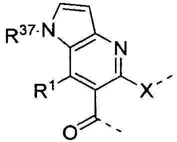 | 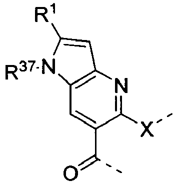 |
| **A397** (a13) | **A398** (a13) | **A399** (a14) | **A400** (a14) |
| 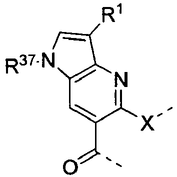 | 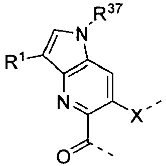 | 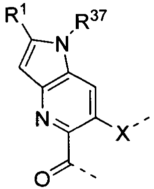 | 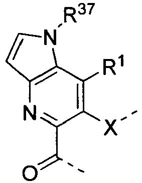 |
| **A401** (a14) | **A402** (a14) | **A403** (a14) | **A404** (a14) |
| 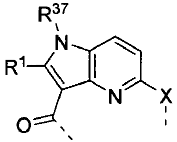 | 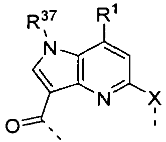 | 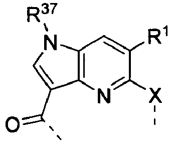 | 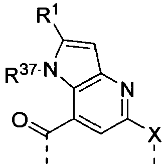 |
| **A405** (a14) | **A406** (a14) | **A407** (a14) | **A408** (a14) |
| 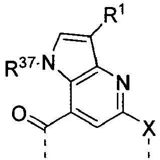 | 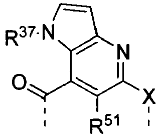 | 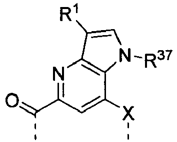 | 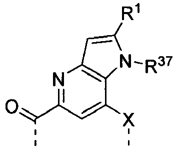 |
| **A409** (a14) | **A410** (a14) | **A411** (a14) | **A412** (a14) |
| 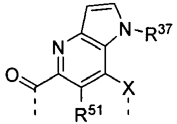 | 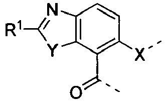 | 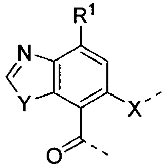 | 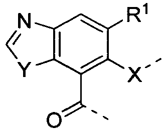 |
| **A413** (a14) | **A414** (a15) | **A415** (a15) | **A416** (a15) |
| 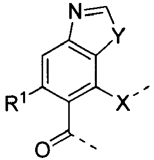 | 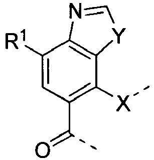 | 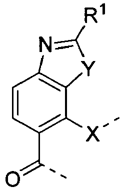 | 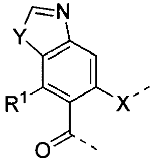 |
| **A417** (a15) | **A418** (a15) | **A419** (a15) | **A420** (a15) |
| 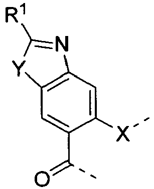 **A421** (a15) | 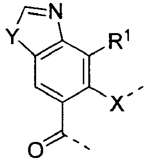 **A422** (a15) | 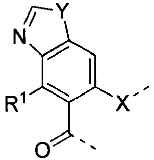 **A423** (a15) | 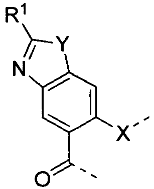 **A424** (a15) |
| 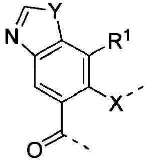 | 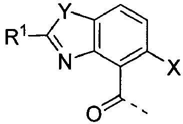 | 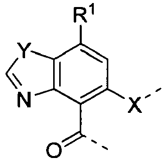 | 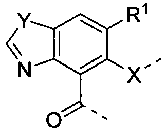 |
| **A425** (a15) | **A426** (a15) | **A427** (a15) | **A428** (a15) |
| 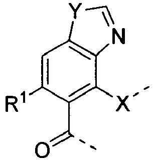 | 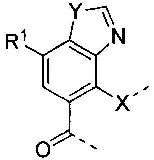 | 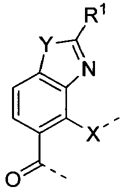 | 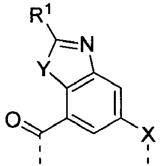 |
| **A429** (a15) | **A430** (a15) | **A431** (a15) | **A432** (a15) |
| 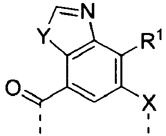 | 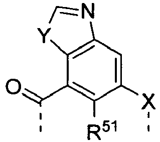 | 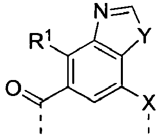 | 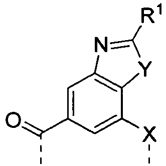 |
| **A433** (a15) | **A434** (a15) | **A435** (a15) | **A436** (a15) |
| 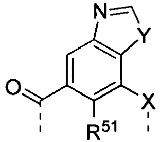 | 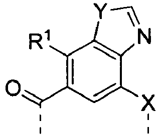 | 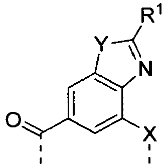 | 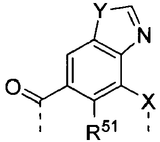 |
| **A437** (a15) | **A438** (a15) | **A439** (a15) | **A440** (a15) |
| 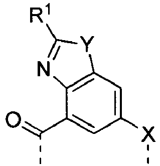 | 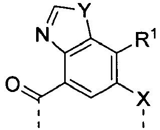 | 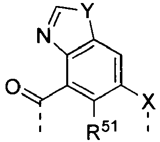 | 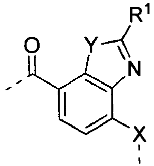 |
| **A441** (a15) | **A442** (a15) | **A443** (a15) | **A444** (a15) |
| 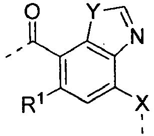 | 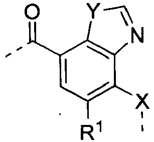 | 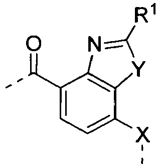 | 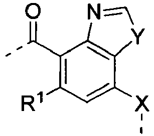 |
| **A445** (a15) | **A446** (a15) | **A447** (a15) | **A448** (a15) |
| 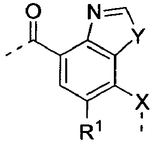 | 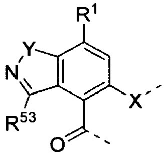 | 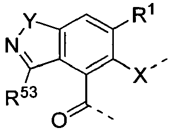 | 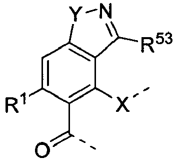 |
| **A449** (a15) | **A450** (a16) | **A451** (a16) | **A452** (a16) |
| 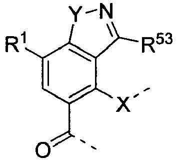 | 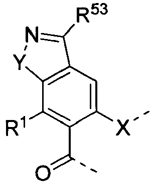 | 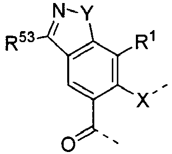 | 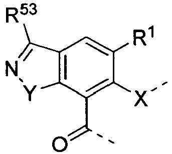 |
| **A453** (a16) | **A454** (a16) | **A455** (a16) | **A456** (a16) |
| 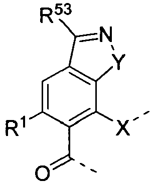 | 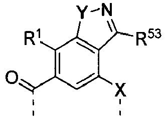 | 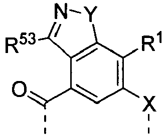 | 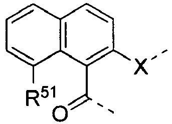 |
| **A457** (a16) | **A458** (a16) | **A459** (a16) | **A460** (a17) |
| 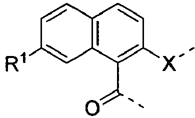 | 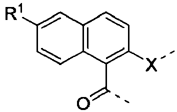 | 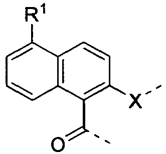 | 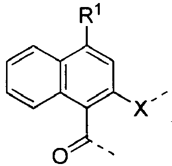 |
| **A461** (a17) | **A462** (a17) | **A463** (a17) | **A464** (a17) |
| 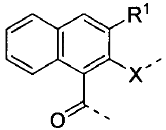 | 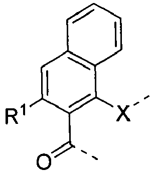 | 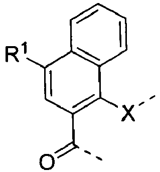 | 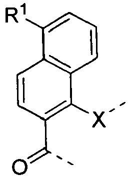 |
| **A465** (a17) | **A466** (a17) | **A467** (a17) | **A468** (a17) |
| 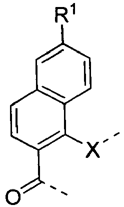 | 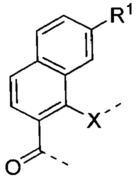 | 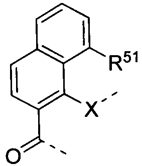 | 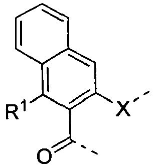 |
| **A469** (a17) | **A470** (a17) | **A471** (a17) | **A472** (a17) |
| 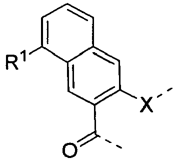 | 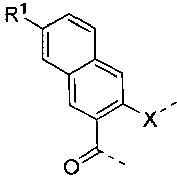 | 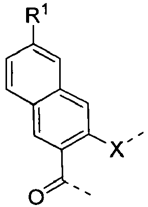 | 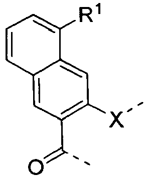 |
| **A473** (a17) | **A474** (a17) | **A475** (a17) | **A476** (a17) |
| 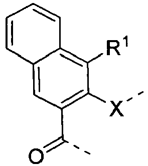 | 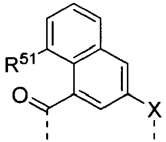 | 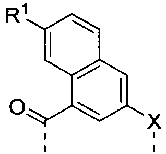 | 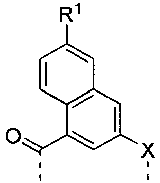 |
| **A477** (a17) | **A478** (a17) | **A479** (a17) | **A480** (a17) |
| 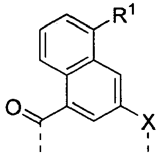 | 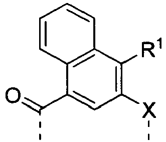 | 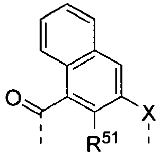 | 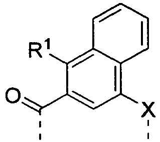 |
| **A481** (a17) | **A482** (a17) | **A483** (a17) | **A484** (a17) |
| 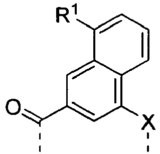 | 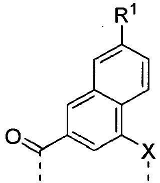 | 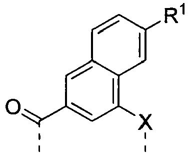 | 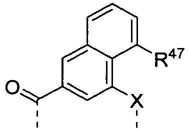 |
| **A485** (a17) | **A486** (a17) | **A487** (a17) | **A488** (a17) |
| 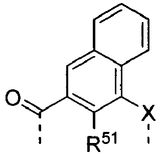 | 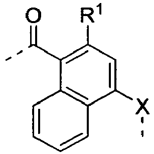 | 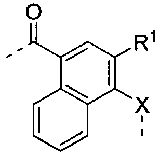 | 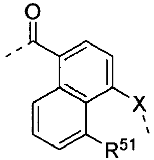 |
| **A489** (a17) | **A490** (a17) | **A491** (a17) | **A492** (a17) |
| 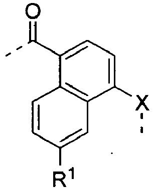 | 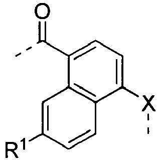 | 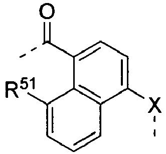 | 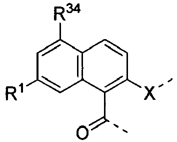 |
| **A493** (a17) | **A494** (a17) | **A495** (a17) | **A496** (a17) |
| 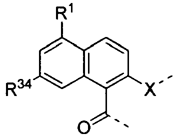 | 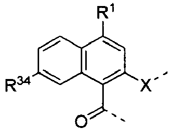 | 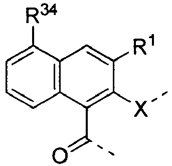 | 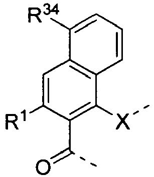 |
| **A497** (a17) | **A498** (a17) | **A499** (a17) | **A500** (a17) |
| 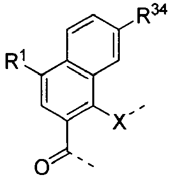 | 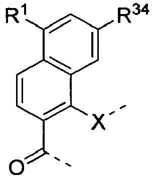 | 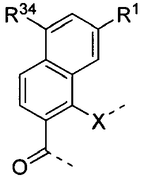 | 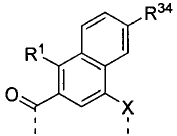 |
| **A501** (a17) | **A502** (a17) | **A503** (a17) | **A504** (a17) |
| 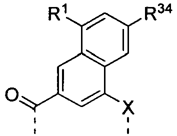 | 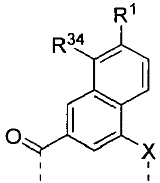 | 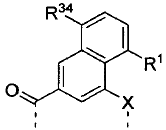 | 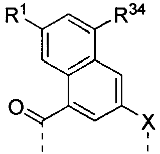 |
| **A505** (a17) | **A506** (a17) | **A507** (a17) | **A508** (a17) |
| 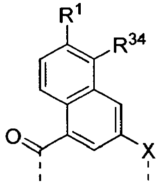 | 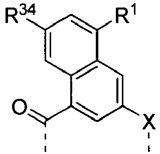 | 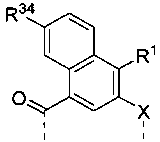 | 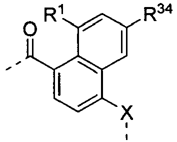 |
| **A509** (a17) | **A510** (a17) | **A511** (a17) | **A512** (a17) |
| 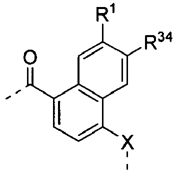 | 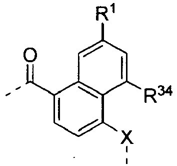 | 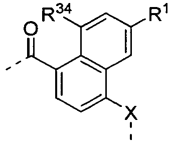 | 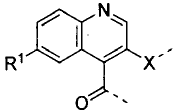 |
| **A513** (a17) | **A514** (a17) | **A515** (a17) | **A516** (a18) |
| 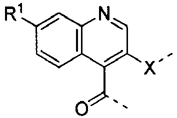 | 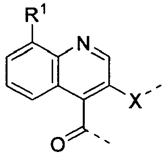 | 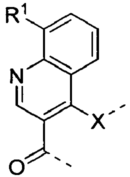 | 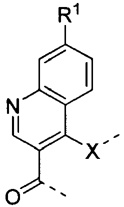 |
| **A517** (a18) | **A518** (a18) | **A519** (a18) | **A520** (a18) |
| 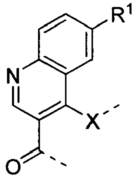 | 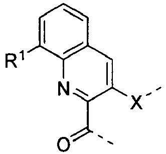 | 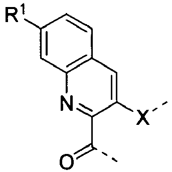 | 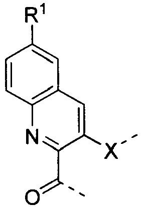 |
| **A521** (a18) | **A522** (a18) | **A523** (a18) | **A524** (a18) |
| 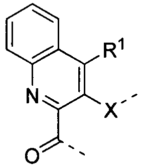 | 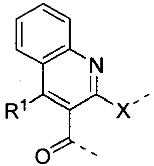 | 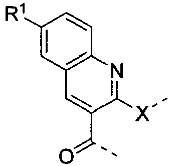 | 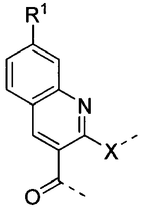 |
| **A525** (a18) | **A526** (a18) | **A527** (a18) | **A528** (a18) |
| 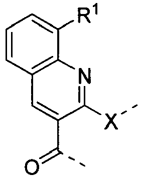 | 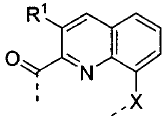 | 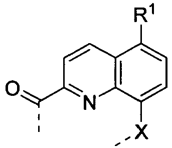 | 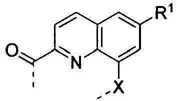 |
| **A529** (a18) | **A530** (a18) | **A531** (a18) | **A532** (a18) |
| 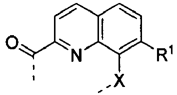 | 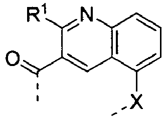 | 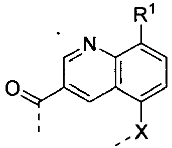 | 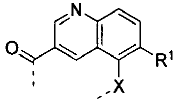 |
| **A533** (a18) | **A534** (a18) | **A535** (a18) | **A536** (a18) |
| 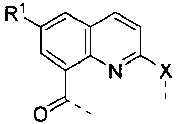 | 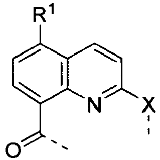 | 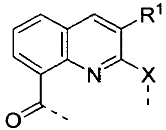 | 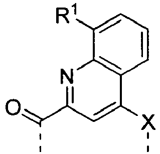 |
| **A537** (a18) | **A538** (a18) | **A539** (a18) | **A540** (a18) |
| 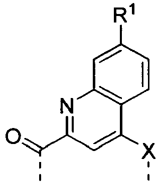 | 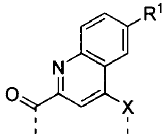 | 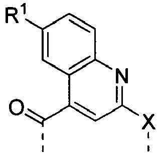 | 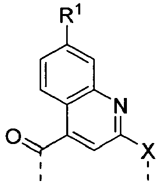 |
| **A541** (a18) | **A542** (a18) | **A543** (a18) | **A544** (a18) |
| 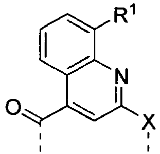 | 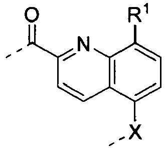 | 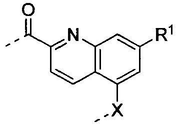 | 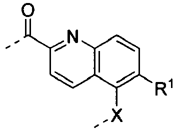 |
| **A545** (a18) | **A546** (a18) | **A547** (a18) | **A548** (a18) |
| 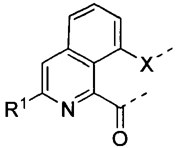 | 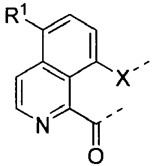 | 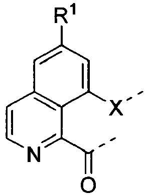 | 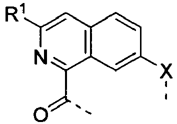 |
| **A549** (a19) | **A550** (a19) | **A551** (a19) | **A552** (a19) |
| 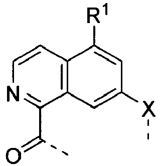 | 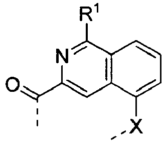 | 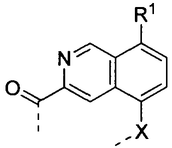 | 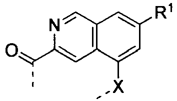 |
| **A553** (a19) | **A554** (a19) | **A555** (a19) | **A556** (a19) |
| 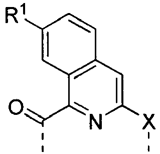 | 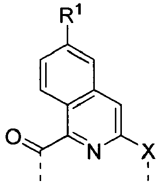 | 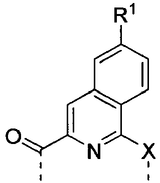 | 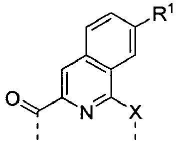 |
| **A557** (a19) | **A558** (a19) | **A559** (a19) | **A560** (a19) |
| 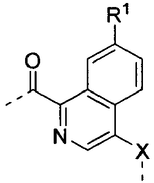 | 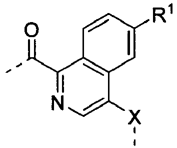 | 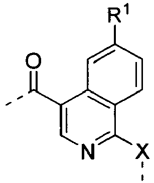 | 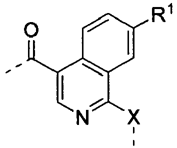 |
| **A561** (a19) | **A562** (a19) | **A563** (a19) | **A564** (a19) |
| 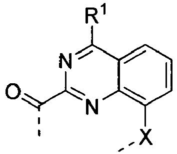 | 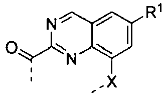 | 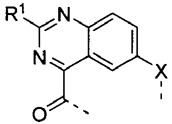 | 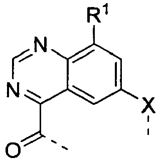 |
| **A565** (a20) | **A566** (a20) | **A567** (a20) | **A568** (a20) |
| 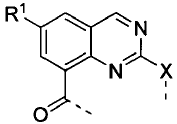 | 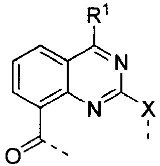 | 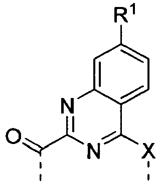 | 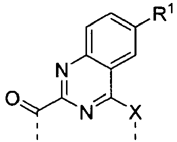 |
| **A569** (a20) | **A570** (a20) | **A571** (a20) | **A572** (a20) |
| 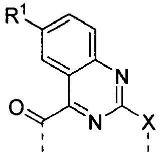 | 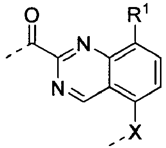 | 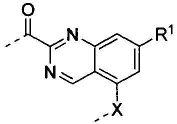 | 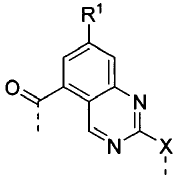 |
| **A573** (a20) | **A574** (a20) | **A575** (a20) | **A576** (a20) |
| 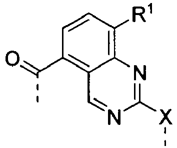 | 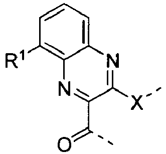 | 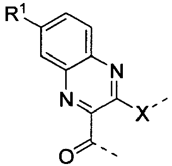 | 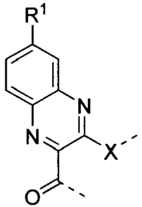 |
| **A577** (a20) | **A578** (a21) | **A579** (a21) | **A580** (a21) |
| 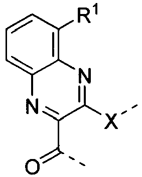 | 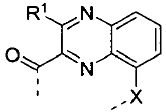 | 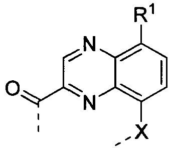 | 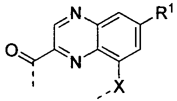 |
| **A581** (a21) | **A582** (a21) | **A583** (a21) | **A584** (a21) |
| 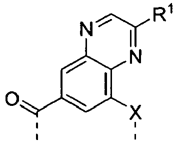 | 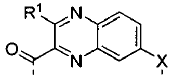 | 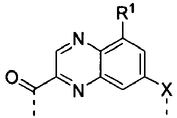 | 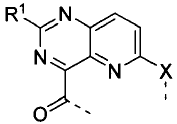 |
| **A585** (a21) | **A586** (a21) | **A587** (a21) | **A588** (a22) |
| 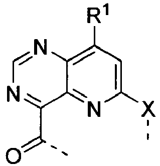 | 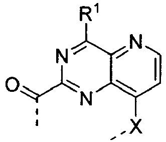 | 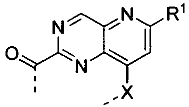 | 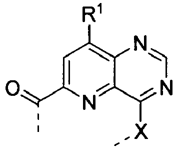 |
| **A589** (a22) | **A590** (a22) | **A591** (a22) | **A592** (a22) |
| 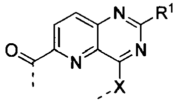 | 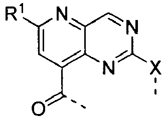 | 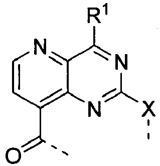 | 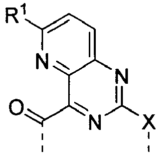 |
| **A593** (a22) | **A594** (a22) | **A595** (a22) | **A596** (a22) |
| 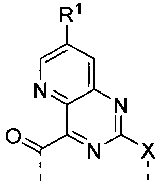 | 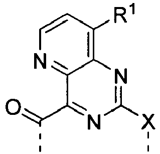 | 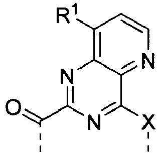 | 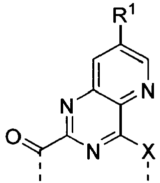 |
| **A597** (a22) | **A598** (a22) | **A599** (a22) | **A600** (a22) |
| 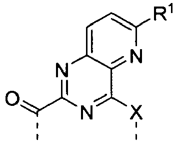 | 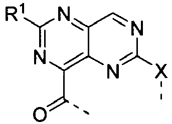 | 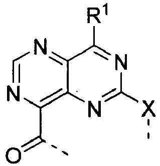 | 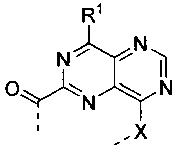 |
| **A601** (a22) | **A602** (a23) | **A603** (a23) | **A604** (a23) |
| 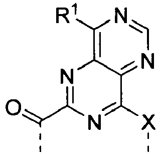 | 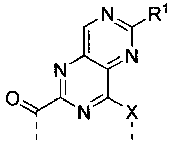 | 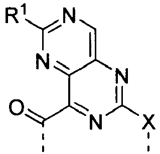 | 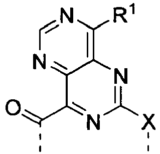 |
| **A605** (a23) | **A606** (a23) | **A607** (a23) | **A608** (a23) |
| 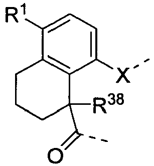 | 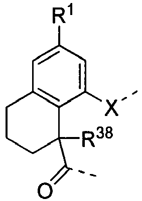 | 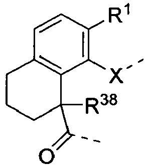 | 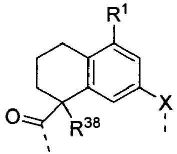 |
| **A609** (a24) | **A610** (a24) | **A611** (a24) | **A612** (a24) |
| 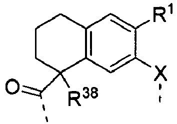 | 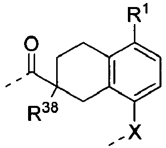 | 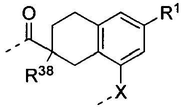 | 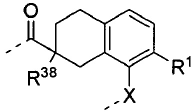 |
| **A613** (a24) | **A614** (a24) | **A615** (a24) | **A616** (a24) |
| 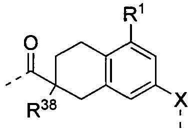 | 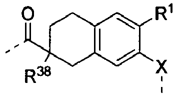 | 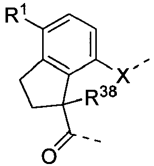 | 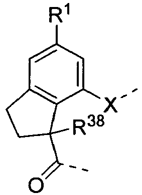 |
| **A617** (a24) | **A618** (a24) | **A619** (a25) | **A620** (a25) |
| 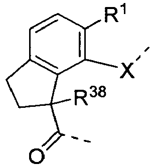 | 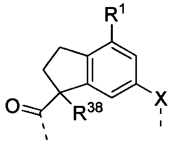 | 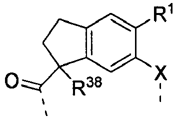 | 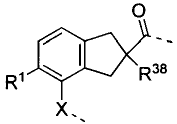 |
| **A621** (a25) | **A622** (a25) | **A623** (a25) | **A624** (a25) |
| 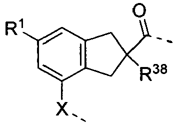 | 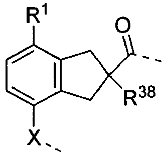 | 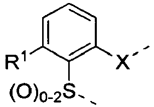 | 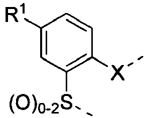 |
| **A625** (a25) | **A626** (a25) | **A627** (a1) | **A628** (a1) |
| 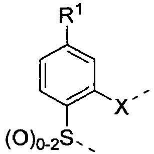 | 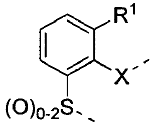 | 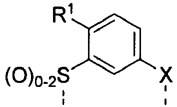 | 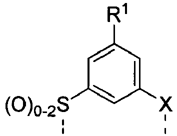 |
| **A629** (a1) | **A630** (a1) | **A631** (a1) | **A632** (a1) |
| 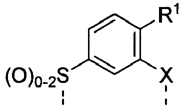 | 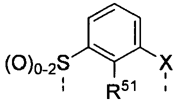 | 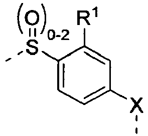 | 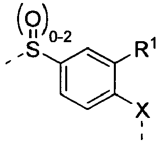 |
| **A633** (a1) | **A634** (a1) | **A635** (a1) | **A636** (a1) |
| 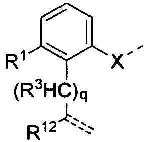 | 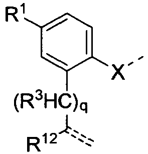 | 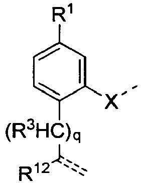 | 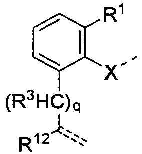 |
| **A637** (a1) | **A638** (a1) | **A639** (a1) | **A640** (a1) |
| 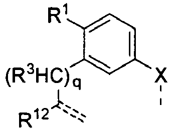 | 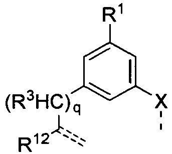 | 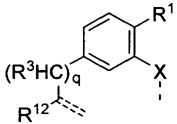 | 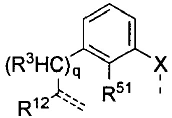 |
| **A641** (a1) | **A642** (a1) | **A643** (a1) | **A644** (a1) |
| 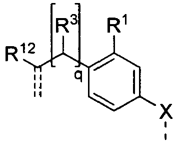 | 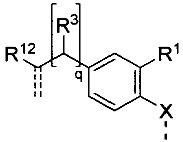 | 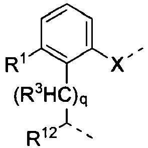 | 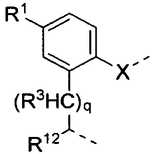 |
| **A645** (a1) | **A646** (a1) | **A647** (a1) | **A648** (a1) |
| 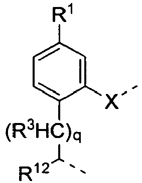 | 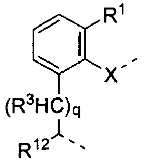 | 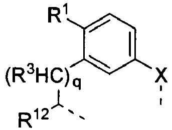 | 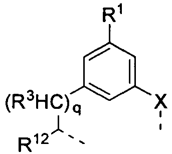 |
| **A649** (a1) | **A650** (a1) | **A651** (a1) | **A652** (a1) |
| 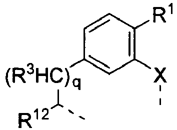 | 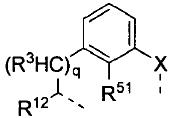 | 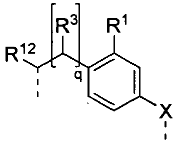 | 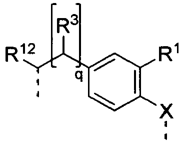 |
| **A653** (a1) | **A654** (a1) | **A655** (a1) | **A656** (a1) |
| 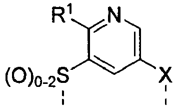 | 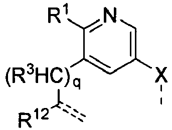 | 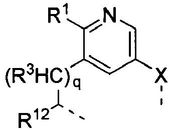 | 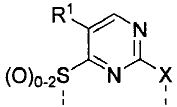 |
| **A657** (a2) | **A658** (a2) | **A659** (a2) | **A660** (a4) |
| 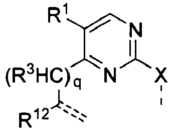 | 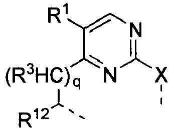 | 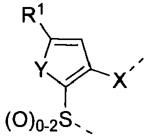 | 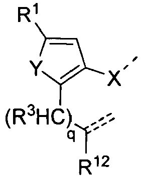 |
| **A661** (a4) | **A662** (a4) | **A663** (a7) | **A664** (a7) |
| 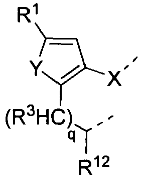 | 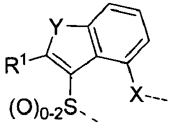 | 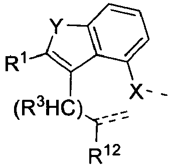 | 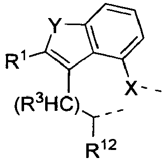 |
| **A665** (a7) | **A666** (a10) | **A667** (a10) | **A668** (a10) |
| 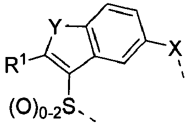 | 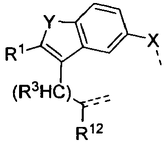 | 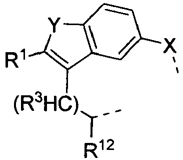 | 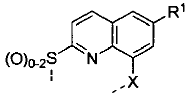 |
| **A669** (a10) | **A670** (a10) | **A671** (a10) | **A672** (a18) |
| 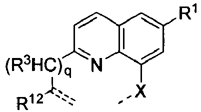 | 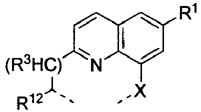 | 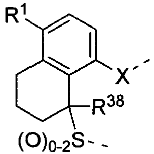 | 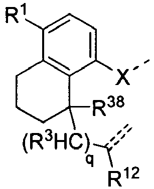 |
| **A673** (a18) | **A674** (a18) | **A675** (a24) | **A676** (a24) |
| 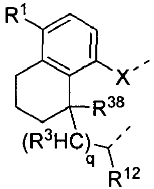 | 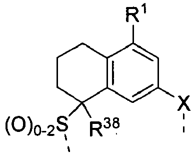 | 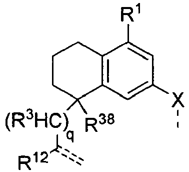 | 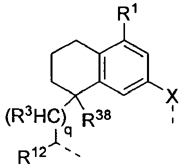 |
| **A677** (a24) | **A678** (a24) | **A679** (a24) | **A680** (a24) |
| 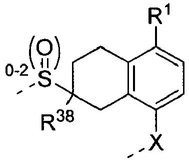 | 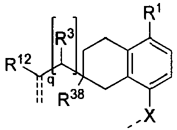 | | 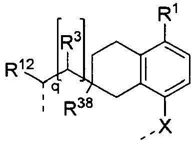 |
| **A681** (a24) | **A682** (a24) | | **A683** (a24) |

**Building block B** is a bivalent radical selected from the group of Table 2.

**B1-B22** are optionally substituted cyclic secondary amines carrying a moiety of type -CHR³-LG, wherein LG is a suitable leaving group that can be replaced by the nucleophilic groups of building blocks **A** forming an ether (-O-) or a thioether (-S-) linkage (or their oxidized forms) between building blocks of type **A** and **B.**

Appropriate LGs are for example -OH being transformed into the active LG *in situ* during a Mitsunobu reactions, or halogens like -Br or -I amenable to S_{N} reactions.

In most examples of type **Ia/Ib,** the secondary amine nitrogen of building block **B** forms a tertiary amide bond with a carboxyl group of building blocks of type **C.** In case suitable exocyclic amine functionality is present, it can be, instead of the ring-nitrogen, involved in the formation of a secondary or preferably tertiary amide connection. Such an alternative binding mode according formula **Ib** is realized with e.g. **B10.**

By virtue of inducing peptidyl *cis-trans* isomerizations or stabilizing *cis* amide bonds, building blocks of type **B** can function as conformational modulators in macrocycles of type **Ia/Ib.**

**Table 2: Radicals B1-B22 (continued on the following page)**

| | | | |
|---|---|---|---|
| 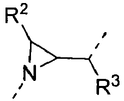 | 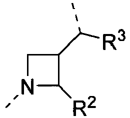 | 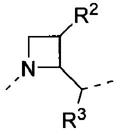 | 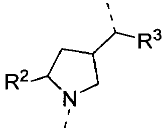 |
| **B1** (b1) | **B2** (b2) | **B3** (b2) | **B4** (b3) |
| 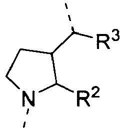 | 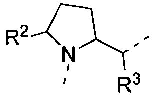 | 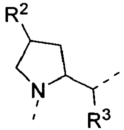 | 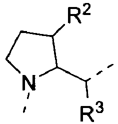 |
| **B5** (b3) | **B6** (b3) | **B7** (b3) | **B8** (b3) |
| 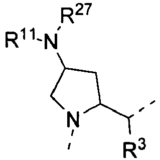 | 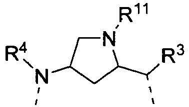 | 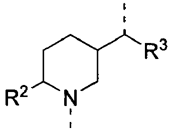 | 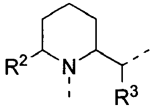 |
| **B9** (b3) | **B10** (b3) | **B11** (b4) | **B12** (b4) |
| 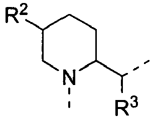 | 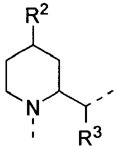 | 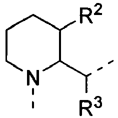 | 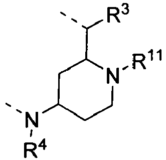 |
| **B13** (b4) | **B14** (b4) | **B15** (b4) | **B16** (b4) |
| 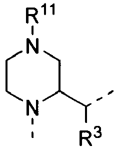 | 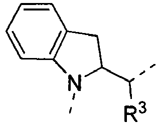 | 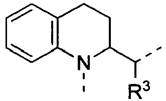 | 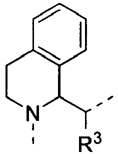 |
| **B17** (b5) | **B18** (b8) | **B19** (b10) | **B20** (b11) |
| 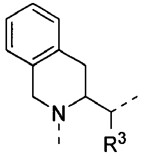 | | 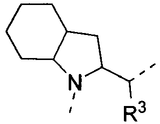 | |
| **B21** (b11) | | **B22** (b12) | |

**Building block C** is a bivalent radical selected from the group of Table 3.

As mentioned before the divalent moiety **C** may consist of one to three subunits **c1** to **c3.** As a consequence it directly influences the ring size of the macrocycle and can be regarded as spacer or linker. This linker ensemble **C** is joined to building block **A** via its *M*-terminus (i.e. N-terminus in case of an amino acid) and to building block **B** via its *U*-terminus (i.e. C-terminus in case of an amino acid) to form the macrocyclic ring of type **Ia/Ib.** As detailed above linker **C** contributes with the four groups M, U, V and W (cf. Figure 2 and 3) to the backbone of macrocycle **Ia/Ib.** For example, in case M is -NR⁴-, **C1** represents a linker moiety constituted of one to three α-amino acid derivatives connected along their main chains, while **C7-C10** are equivalent to moieties containing β-amino acids. The simplest situation in which at least one connection between the subunits **c1** to **c3** is realized by a non-amidic group are **C2-C5.** Finally **C58-C101** depict cases in which longer carbon chains (>3 C-atoms) separate at least one pair of the generic groups M, V, W, U.

**Table 3: Representations of Linker C (continued on the following pages)**

| | |
|---|---|
| 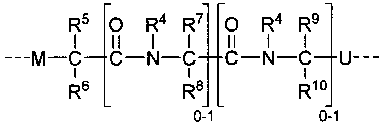 | 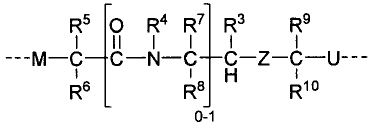 |
| **C1** | **C2** |
| 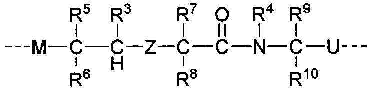 | 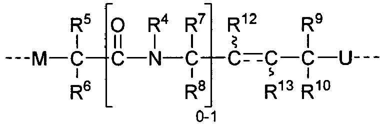 |
| **C3** | **C4** |
| 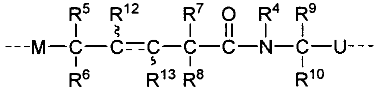 | 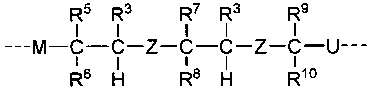 |
| **C5** | **C6** |
| 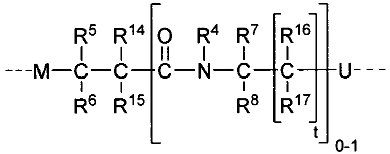 | 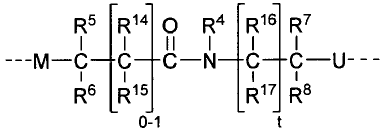 |
| **C7** | **C8** |
| 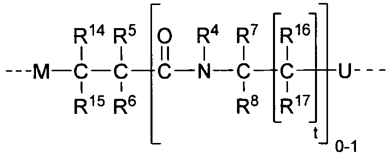 | 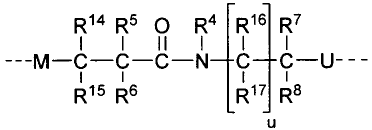 |
| **C9** | **C10** |
| 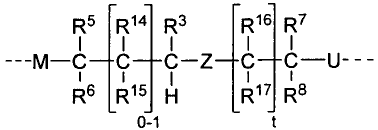 | 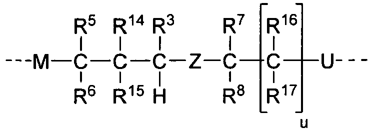 |
| **C11** | **C12** |
| 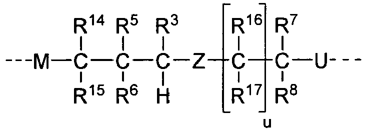 | 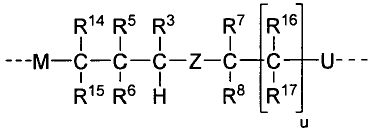 |
| **C13** | **C14** |
| 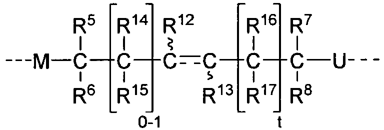 | 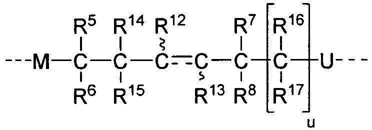 |
| **C15** | **C16** |
| 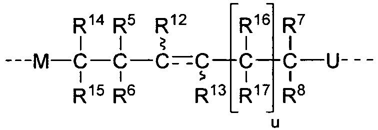 | 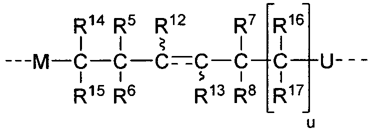 |
| **C17** | **C18** |
| 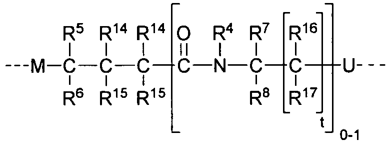 | 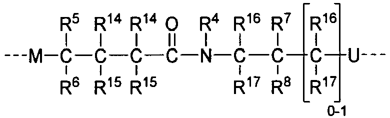 |
| **C19** | **C20** |
| 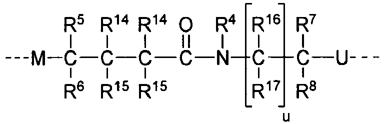 | 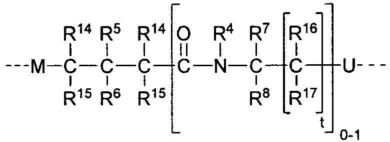 |
| **C21** | **C22** |
| 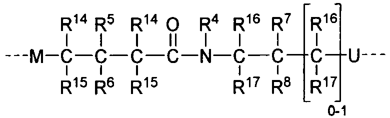 | 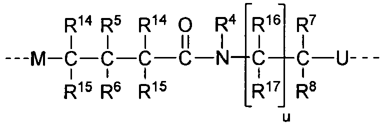 |
| **C23** | **C24** |
| 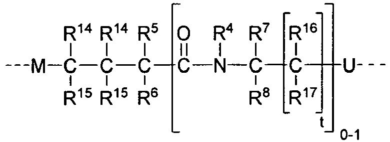 | 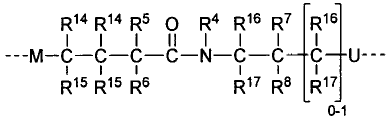 |
| **C25** | **C26** |
| 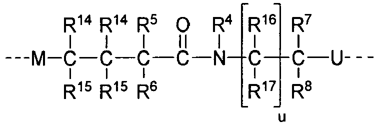 | 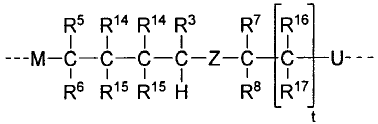 |
| **C27** | **C28** |
| 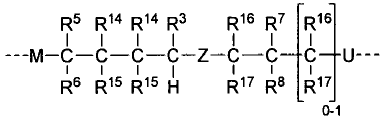 | 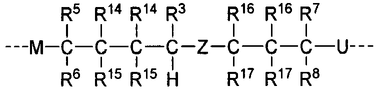 |
| **C29** | **C30** |
| 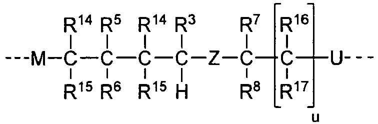 | 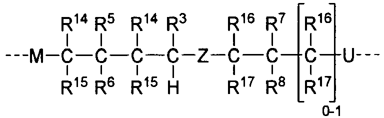 |
| **C31** | **C32** |
| 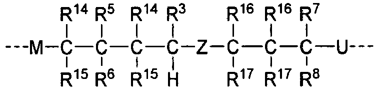 | 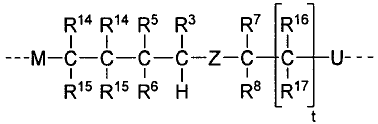 |
| **C33** | **C34** |
| 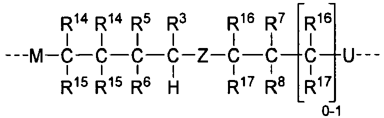 | 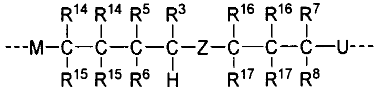 |
| **C35** | **C36** |
| 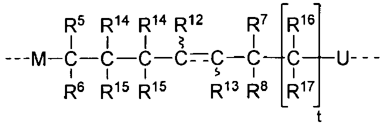 | 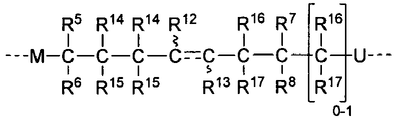 |
| **C37** | **C38** |
| 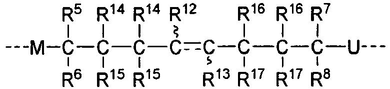 | 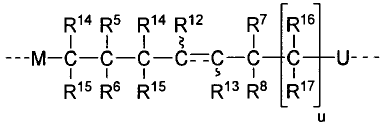 |
| **C39** | **C40** |
| 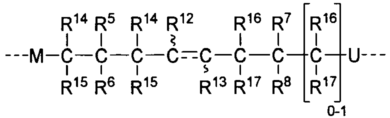 | 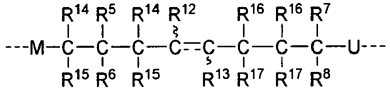 |
| **C41** | **C42** |
| 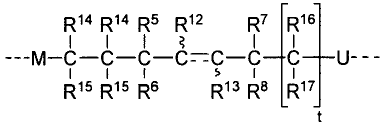 | 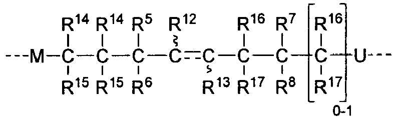 |
| **C43** | **C44** |
| 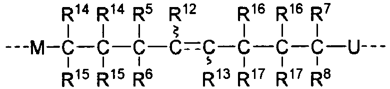 | 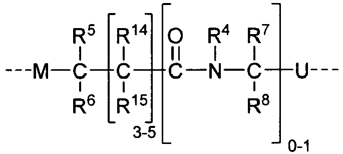 |
| **C45** | **C46** |
| 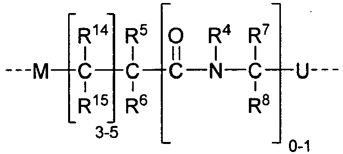 | 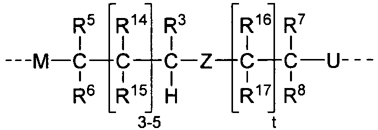 |
| **C47** | **C48** |
| 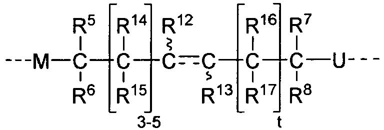 | 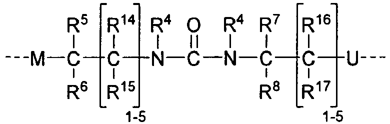 |
| **C49** | **C50** |
| 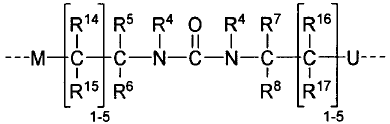 | 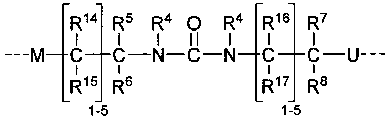 |
| **C51** | **C52** |
| 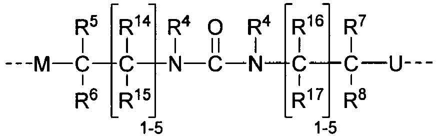 | |
| **C53** | |
| 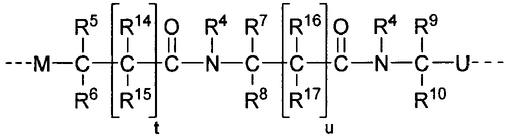 | |
| **C54** | |
| 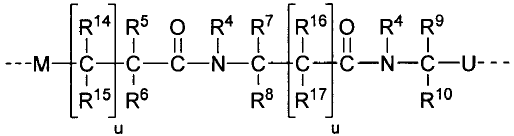 | |
| **C55** | |
| 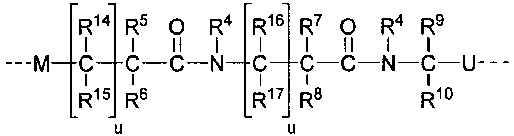 | |
| **C56** | |
| 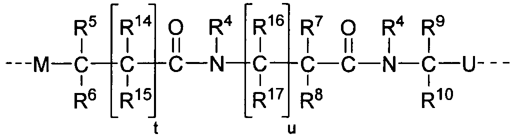 | |
| **C57** | |
| 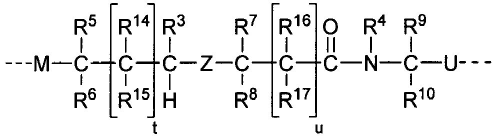 | |
| **C58** | |
| 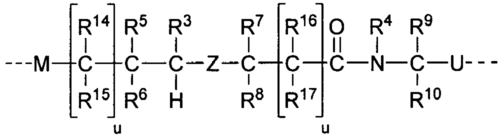 | |
| **C59** | |
| 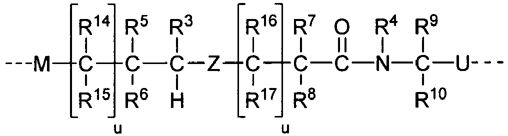 | |
| **C60** | |
| 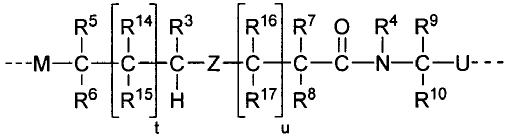 | |
| **C61** | |
| 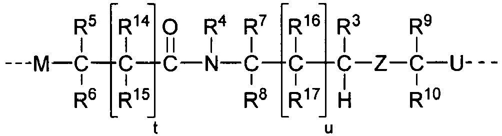 | |
| **C62** | |
| 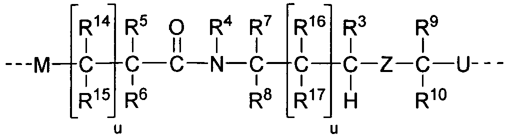 | |
| **C63** | |
| 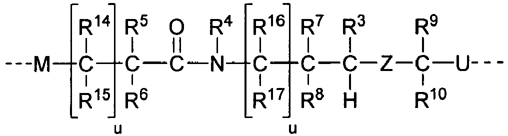 | |
| **C64** | |
| 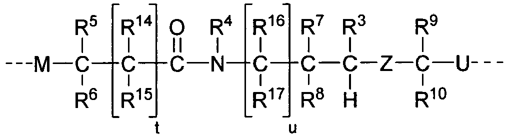 | |
| **C65** | |
| 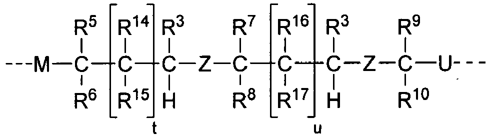 | |
| **C66** | |
| 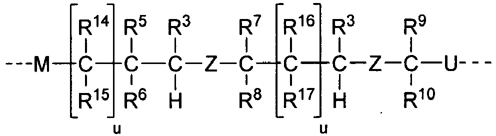 | |
| **C67** | |
| 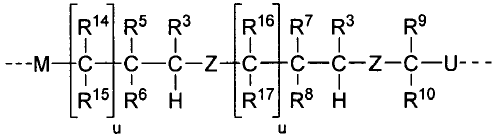 | |
| **C68** | |
| 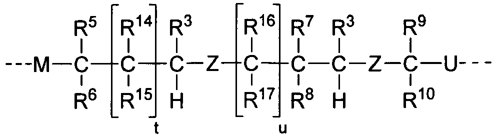 | |
| **C69** | |
| 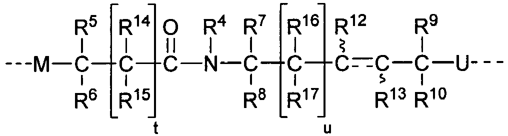 | |
| **C70** | |
| 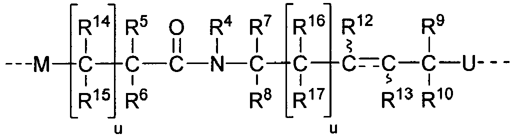 | |
| **C71** | |
| 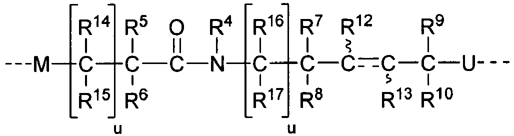 | |
| **C72** | |
| 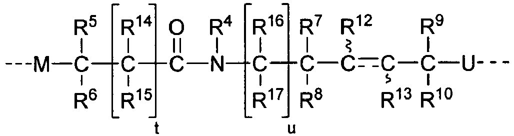 | |
| **C73** | |
| 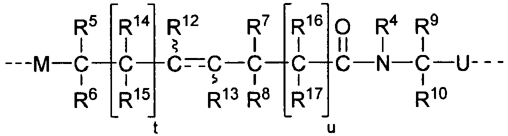 | |
| **C74** | |
| 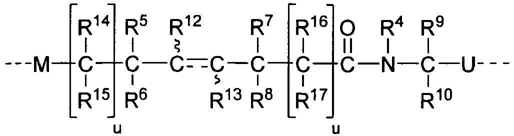 | |
| **C75** | |
| 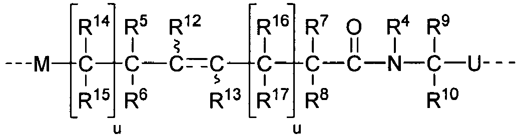 | |
| **C76** | |
| 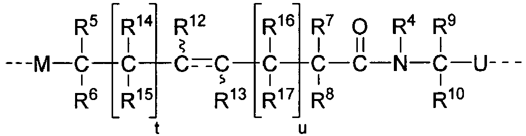 | |
| **C77** | |
| 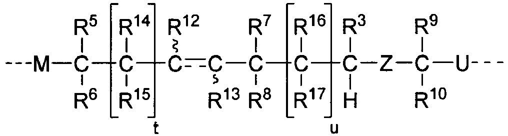 | |
| **C78** | |
| 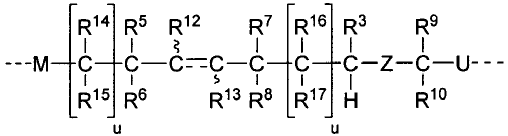 | |
| **C79** | |
| 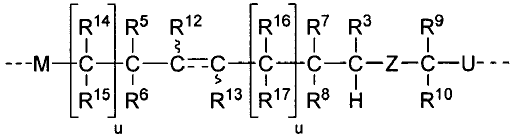 | |
| **C80** | |
| 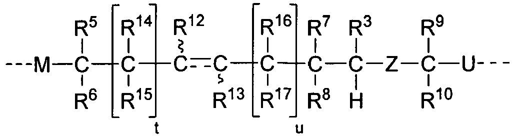 | |
| **C81** | |
| 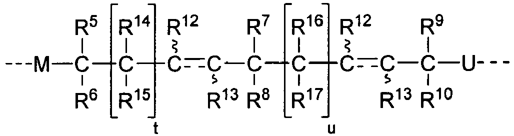 | |
| **C82** | |
| 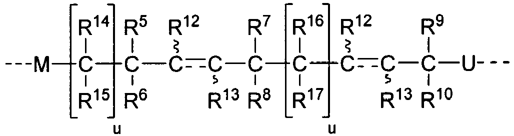 | |
| **C83** | |
| 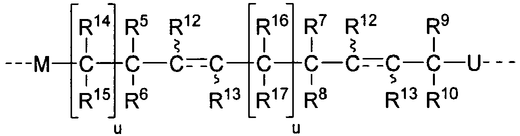 | |
| **C84** | |
| 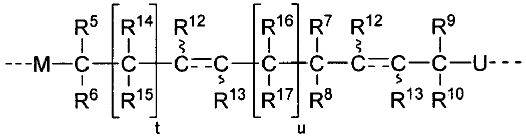 | |
| **C85** | |
| 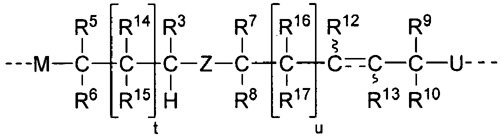 | |
| **C86** | |
| 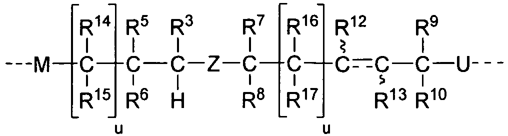 | |
| **C87** | |
| 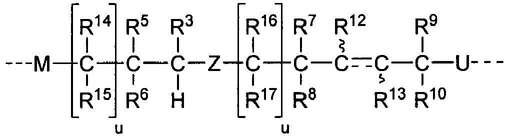 | |
| **C88** | |
| 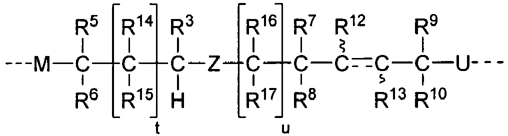 | |
| **C89** | |
| 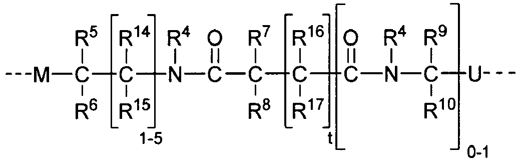 | |
| **C90** | |
| 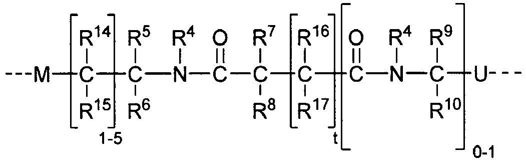 | |
| **C91** | |
| 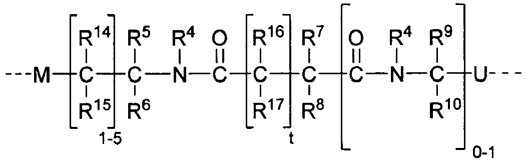 | |
| **C92** | |
| 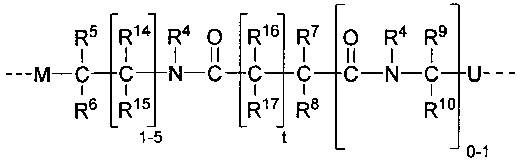 | |
| **C93** | |
| 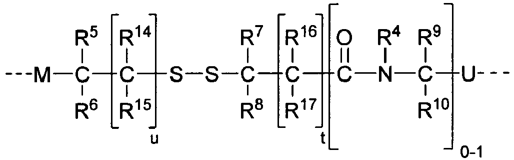 | |
| **C94** | |
| 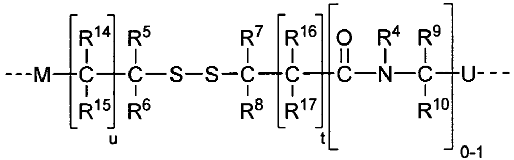 | |
| **C95** | |
| 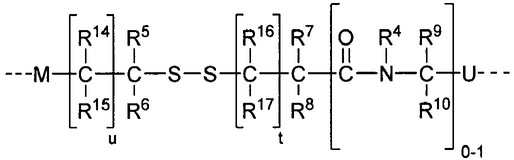 | |
| **C96** | |
| 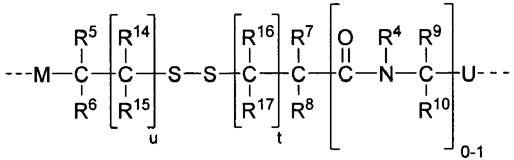 | |
| **C97** | |
| 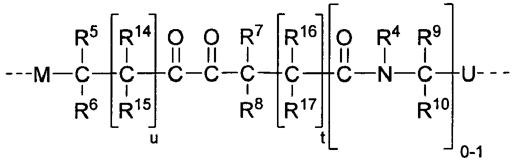 | |
| **C98** | |
| 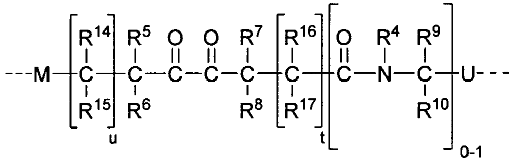 | |
| **C99** | |
| 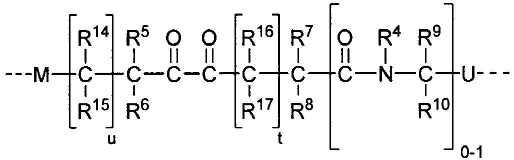 | |
| **C100** | |
| 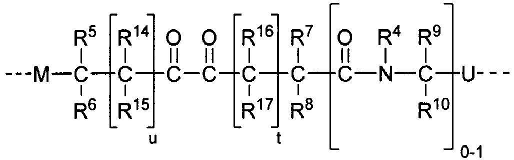 | |
| **C101** | |

According to the preceding definitions, macrocycles of type **Ia/Ib** contain at least one amide bond or isosteric surrogates thereof. As mentioned in the introduction, tertiary amide containing products generally show various ratios of *cis* and *trans* amide bond conformations in solution; this preference is in contrast to secondary amides that strongly prefer *trans* conformations. The occurrence of *cis* and/or *trans* conformations in macrocyclic natural products containing tertiary amide groups is well documented. In some cases a rapid equilibration between the *cis* and *trans* amide bonds, a so-called "peptidyl *cis*/ *trans* isomerization", is observed; whereas in other cases discrete *cis* and *trans* tertiary amide bonds are detected as two stable conformers in solution at room temperature. Consequently all possible stereoisomers (explicitly including atropisomers), conformers or rotamers of macrocycles **Ia/Ib** are part of this invention.

The substituents mentioned in formulae **Ia** and **Ib,** respectively in the above description of the building blocks **A, B** or **C** are defined in detail with the following terms:
R¹ is H; F; Cl; Br; I; CF₃; OCF₃; OCHF₂; NO₂; CN; alkyl; alkenyl; alkynyl; cycloalkyl; heterocycloalkyl; aryl; heteroaryl; arylalkyl; heteroarylalkyl; -(CR¹⁸R¹⁹)_{q}OR²⁰; -(CR¹⁸R¹⁹)_{q}SR²⁰; -(CR¹⁸R¹⁹)_{q}NR⁴R¹¹; -(CR¹⁸R¹⁹)_{q}OCONR⁴R¹¹; -(CR¹⁸R¹⁹)_{q}OCOOR²¹; -(CR¹⁸R¹⁹)_{q}NR⁹COOR²¹; -(CR¹⁹R¹⁹)_{q}NR⁴COR²²; -(CR¹⁸R¹⁹)_{q}NR⁴CONR⁹R¹¹; -(CR¹⁸R¹⁹)_{q}NR⁴SO₂R³³;-(CR¹⁸R¹⁹)_{q}NR⁴SO₂NR⁴R¹¹; -(CR¹⁸R¹⁹)_{q}COOR²¹; -(CR¹⁸R¹⁹)_{q}CONR⁴R¹¹; -(CR¹⁸R¹⁹)_{q}SO₂NR⁴R¹¹; -(CR¹⁸R¹⁹)_{q}PO(OR²¹)₂; -(CR¹⁸R¹⁹)_{q}OPO(OR²¹)₂; -(CR¹⁸R¹⁹)_{q}COR²²; - (CR¹⁸R¹⁹)_{q}SO₂R²³; -(CR¹⁸R¹⁹)_{q}OSO₃R²¹; -(CR¹⁸R¹⁹)_{q}R²⁴; -(CR¹⁸R¹⁹)_{q}R²⁵; or -(CR¹⁸R¹⁹) _{q}R²⁶;
R² is H; CF₃; alkyl; alkenyl; cycloalkyl; heterocycloalkyl; aryl; heteroaryl; arylalkyl; heteroarylalkyl; -(CR¹⁸R¹⁹)_{q}OR²⁰; -(CR¹⁸R¹⁹)_{q}SR²⁰; -(CR¹⁸R¹⁹)_{q}NR⁴R¹¹; -(CR¹⁸R¹⁹)_{q}OCONR⁴R¹¹; -(CR¹⁸R¹⁹)_{q}OCOOR²¹; -(CR¹⁸R¹⁹)_{q}NR⁴COOR²¹; -(CR¹⁸R¹⁹)_{q}NR⁴COR²²; -(CR¹⁸R¹⁹)_{q}NR⁹CONR⁴R¹¹; -(CR¹⁸R¹⁹)_{q}NR⁴SO₂R²³; -(CR¹⁸R¹⁹)_{q}NR⁴SO₂NR⁴R¹¹; - (CR¹⁸R¹⁹)_{q}COOR²¹; -(CR¹⁸R¹⁹)_{q}CONR⁴R¹¹; -(CR¹⁸R¹⁹)_{q}SO₂NR⁴R¹¹; -(CR¹⁸R¹⁹)_{q}PO(OR²¹)₂; -(CR¹⁸R¹⁹)_{q}COR²²; -(CR¹⁸R¹⁹)_{q}SO₂R²³; -(CR¹⁸R¹⁹)_{q}R²⁹; -(CR¹⁸R¹⁹)_{q}R²⁵; or -(CR¹⁸R¹⁹)_{q}R²⁶;
R³ is H; CF₃; alkyl; cycloalkyl; heterocycloalkyl; aryl; heteroaryl; arylalkyl; or heteroarylalkyl;
R⁴ is H; alkyl; alkenyl; cycloalkyl; heterocycloalkyl; aryl; heteroaryl; arylalkyl; heteroarylalkyl; or a suitable N-protecting group;
R⁵, R⁷ and R⁹ are independently defined as is H; F; CF₃; alkyl; alkenyl; cycloalkyl; heterocycloalkyl; aryl; heteroaryl; arylalkyl; heteroarylalkyl; -(CR¹⁸R¹⁹)_{q}OR²⁰; -(CR¹⁸R¹⁹)_{q}SR²⁰; -(CR¹⁸R¹⁹)_{q}NR⁴R¹¹; -(CR¹⁸R¹⁹)_{q}OCONR⁴R¹¹; -(CR¹⁸R¹⁹)_{q}OCOOR²¹; - (CR¹⁸R¹⁹)_{q}NR⁴COOR²¹; -(CR¹⁸R¹⁹)_{q}NR⁴COR²²; -(CR¹⁸R¹⁹)_{q}NR⁴CONR⁴R¹¹; -(CR¹⁸R¹⁹)_{q}NR⁴SO₂R²³; -(CR¹⁸R¹⁹)_{q}NR⁴SO₂NR⁴R¹¹; -(CR¹⁸R¹⁹)_{q}COOR²¹; -(CR¹⁸R¹⁹)_{q}CONR⁴R¹¹; -(CR¹⁸R¹⁹)_{q}SO²NR⁴R¹¹; -(CR¹⁸R¹⁹)_{q}PO(OR²¹)₂; -(CR¹⁸R¹⁹)_{q}COR²²; -(CR¹⁸R¹⁹)_{q}SO₂R²³; -(CR¹⁸R¹⁹)_{q}R²⁴; -(CR¹⁸R¹⁹)_{q}R²⁵; or -(CR¹⁸R¹⁹)_{q}R²⁶;
R⁶, R⁸ and R¹⁰ are independently defined as H; F; CF₃; alkyl; alkenyl; cycloalkyl; heterocycloalkyl; aryl; heteroaryl; arylalkyl; or heteroarylalkyl;
R¹¹ is H; alkyl; alkenyl; cycloalkyl; heterocycloalkyl; aryl; heteroaryl; arylalkyl; heteroarylalkyl; a suitable protecting group; -(CR¹⁸R¹⁹)ᵣOR²⁰; -(CR¹⁸R¹⁹)ᵣSR²⁰; -(CR¹⁸R¹⁹)ᵣNR⁴R²⁷; -(CR¹⁸R¹⁹)ᵣOCONR⁴R²⁷; -(CR¹⁸R¹⁹)ᵣOCOOR²¹; -(CR¹⁸R¹⁹)ᵣNR⁴COOR²¹; -(CR¹⁸R¹⁹)ᵣNR⁴CONR⁴R²⁷; -(CR¹⁸R¹⁹)ᵣNR⁴SO₂R²³; -(CR¹⁸R¹⁹)ᵣNR⁴SO₂NR⁴R²⁷; -(CR¹⁸R¹⁹)_{q}COOR²¹; -(CR¹⁸R¹⁹)_{q}CONR⁴R²⁷; -(CR¹⁸R¹⁹)_{q}COR²²; -(CR¹⁸R¹⁹)_{q}SO₂R²³; -(CR¹⁸R¹⁹)_{q}SO₂NR⁴R²⁷; -(CR¹⁸R¹⁹)_{q}R²⁴; -(CR¹⁸R¹⁹)ₛR²⁵; -(CR¹⁸R¹⁹)_{q}R²⁶; or -(CR¹⁸R¹⁹)_{q}R³¹;
R¹² and R¹³ are independently defined as H; or alkyl.
R¹⁴ and R¹⁶ are independently defined as H; F; CF₃; alkyl; alkenyl; cycloalkyl; heterocycloalkyl; aryl; heteroaryl; arylalkyl; heteroarylalkyl; -(CR¹⁸R¹⁹)_{q}OR²⁰; -(CR¹⁸R¹⁹)_{q}NR⁴R¹¹; -(CR¹⁸R¹⁹)_{q}NR⁴COOR²¹; -(CR¹⁸R¹⁹)_{q}NR⁴COR²²; -(CR¹⁸R¹⁹)_{q}NR⁴CONR⁴R¹¹; -(CR¹⁸R¹⁹)_{q}NR⁴SO₂R²³;-(CR¹⁸R¹⁹)_{q}NR⁹SO₂NR⁹R¹¹; -(CR¹⁸R¹⁹)_{q}COOR²¹; -(CR¹⁸R¹⁹)_{q}CONR⁴R¹¹; -(CR¹⁸R¹⁹)_{q}SO₂NR⁴R¹¹; or -(CR¹⁸R¹⁹)_{q}COR²²;
R¹⁵ and R¹⁷ are independently defined as H; F; CF₃; alkyl; alkenyl; cycloalkyl; heterocycloalkyl; aryl; heteroaryl; arylalkyl; or heteroarylalkyl;
R¹⁸ is H; F; CF₃; alkyl; alkenyl; cycloalkyl; heterocycloalkyl; aryl; heteroaryl; arylalkyl; heteroarylalkyl; -(CR²⁹R³⁰)ₛOR³¹; -(CR²⁹R³⁰)ₛSR³¹; -(CR²⁹R³⁰)ₛNR²⁸R³¹; -(CR²⁹R³⁰)ₛOCONR²⁸R³¹; -(CR²⁹R³⁰)ₛOCOOR²¹; -(CR²⁹R³⁰)ₛNR²⁸COOR²¹; -(CR²⁹R³⁰)ₛNR²⁸COR³¹; -(CR²⁹R³⁰)ₛNR²⁸CONR²⁸R³¹; - (CR²⁹R³⁰)ₛNR²⁸SO₂R²³; -(CR²⁹R³⁰)ₛNR²⁸SO₂NR²⁸R³¹; -(CR²⁹R³⁰)_{q}COOR²¹; -(CR²⁹R³⁰)_{q}CONR²⁸R³¹; -(CR²⁹R³⁰)_{q}SO₂NR²⁸R³¹; -(CR²⁹R³⁰)_{q}PO(OR²¹)₂; -(CR²⁹R³⁰)_{q}COR³¹; -(CR²⁹R³⁰)_{q}SO₂R²³; -(CR²⁹R³⁰)_{q}R²⁴; -(CR²⁹R³⁰)_{q}R²⁵; or -(CR²⁹R³⁰)_{q}R²⁶;
R¹⁹ is H; CF₃; alkyl; alkenyl; cycloalkyl; heterocycloalkyl; aryl; heteroaryl; arylalkyl; or heteroarylalkyl;
R²⁰ is H; alkyl; alkenyl; cycloalkyl; heterocycloalkyl; aryl; heteroaryl; arylalkyl; heteroarylalkyl; -(CR²⁹R³⁰)ᵣOR³¹; -(CR²⁹R³⁰)ᵣSR³¹; -(CR²⁹R³⁰)ᵣNR²⁸R³¹; -(CR²⁹R³⁰)ᵣOCONR²⁸R³¹; -(CR²⁹R³⁰)ᵣNR²⁸COOR²¹; -(CR²⁹R³⁰)ᵣNR²⁸COR³¹; -(CR²⁹R³⁰)ᵣNR²⁸CONR²⁸R³¹; -(CR²⁹R³⁰)ᵣNR²⁸SO₂R²³; -(CR²⁹R³⁰)ᵣNR²⁸SO₂NR²⁸R³¹; -(CR²⁹R³⁰)_{q}COOR²¹; - (CR²⁹R³⁰)_{q}CONR²⁸R³¹; -(CR²⁹R³⁰)_{q}SO₂NR²⁸R³¹; -(CR²⁹R³⁰)_{q}COR³¹; -(CR²⁹R³⁰)_{q}SO₂R²³; -(CR²⁹R³⁰)_{q}R²⁴; -(CR²⁹R³⁰)_{q}R²⁵; -(CR²⁹R³⁰)_{q}R²⁶; or -(CR²⁹R³⁰)_{q}R³¹;
R²¹ is H; alkyl; alkenyl; cycloalkyl; heterocycloalkyl; aryl; heteroaryl; arylalkyl; heteroarylalkyl; or a suitable O-protecting group;
R²² is alkyl; alkenyl; cycloalkyl; heterocycloalkyl; aryl; heteroaryl; arylalkyl; heteroarylalkyl; - (CR²⁹R³⁰)ₛOR³¹; -(CR²⁹R³⁰)ₛSR³¹; -(CR²⁹R³⁰)ₛNR²⁸R³¹; -(CR²⁹R³⁰)ₛOCONR²⁸R³¹; -(CR²⁹R³⁰)ₛNR²⁸COOR²¹; -(CR²⁹R³⁰)ₛNR²⁸COR³¹; -(CR²⁹R³⁰)ₛNR²⁸CONR²⁸R³¹; -(CR²⁹R³⁰)ₛNR²⁸SO₂R²³; -(CR²⁹R³⁰)ₛNR²⁸SO₂NR²⁸R³¹; -(CR²⁹R³⁰)ₛCOOR²¹; -(CR²⁹R³⁰)ₛCONR²⁸R³¹; -(CR²⁹R³⁰)ₛSO₂NR²⁸R³¹; -(CR²⁹R³⁰)ₜCOR³¹; -(CR²⁹R³⁰)ₛSO₂R²³; -(CR²⁹R³⁰)ₜR²⁴; -(CR²⁹R³⁰)ₜR²⁵; -(CR²⁹R³⁰)ₜR²⁶; or -(CR²⁹R³⁰)ₜR³¹;
R²³ is H; alkyl; alkenyl; cycloalkyl; heterocycloalkyl; aryl; heteroaryl; arylalkyl; heteroarylalkyl; or -(CR³²R³³)ₜR²⁴;
R²⁴ is aryl; heteroaryl; -C₆H₂R³⁹R³⁵R³¹; or a group of one of the formulae **H1-H34** listed in Table 4.

**Table 4: Groups of Formulae H1-H34 (continued on the following page)**

| | | | |
|---|---|---|---|
| 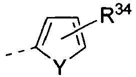 | 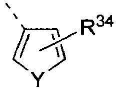 | 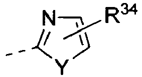 | 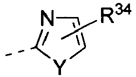 |
| **H1** | **H2** | **H3** | **H4** |
| 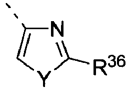 | 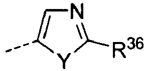 | 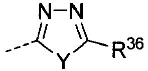 | 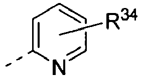 |
| **H5** | **H6** | **H7** | **H8** |
| 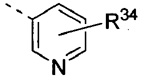 | 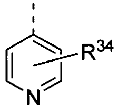 | 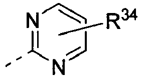 | 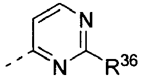 |
| **H9** | **H10** | **H11** | **H12** |
| 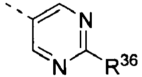 | 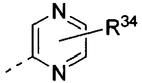 | 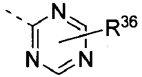 | 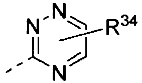 |
| **H13** | **H14** | **H15** | **H16** |
| 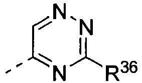 | 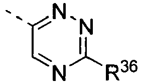 | 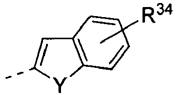 | 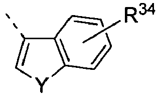 |
| **H17** | **H18** | **H19** | **H20** |
| 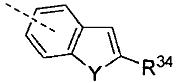 | 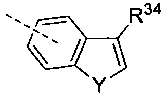 | 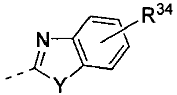 | 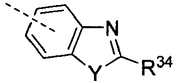 |
| **H21** | **H22** | **H23** | **H24** |
| 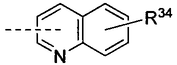 | 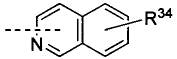 | 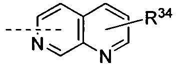 | 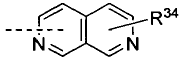 |
| **H25** | **H26** | **H27** | **H28** |
| 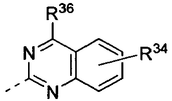 | 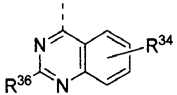 | 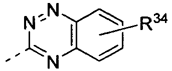 | 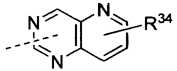 |
| **H29** | **H30** | **H31** | **H32** |
| 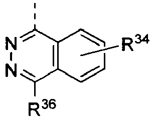 | | 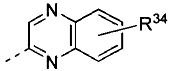 | |
| **H33** | | **H34** | |

R²⁵ is a group of one of formulae **H35-H41** as shown in Table 5 below.

**Table 5: Radicals of Formulae H35-H41**

| | | | |
|---|---|---|---|
| 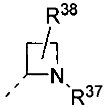 | 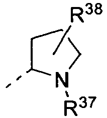 | 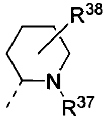 | 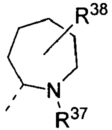 |
| **H35** | **H36** | **H37** | **H38** |
| 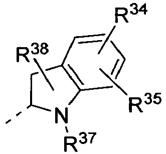 | 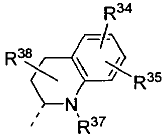 | | 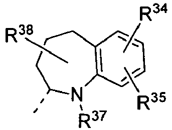 |
| **H39** | **H40** | | **H41** |

R²⁶ is a group of one of formulae **H42-H50** as shown in Table 6 below.

**Table 6: Groups of Formulae H42-H50**

| | | | |
|---|---|---|---|
| 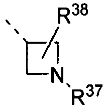 | 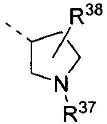 | 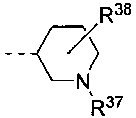 | 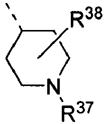 |
| **H42** | **H43** | **H44** | **H45** |
| 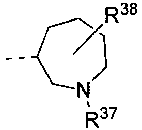 | 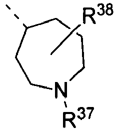 | 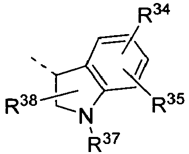 | 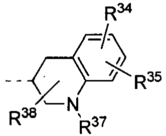 |
| **H46** | **H47** | **H48** | **H49** |
| 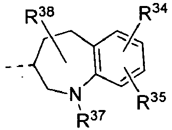 | | | |
| **H50** | | | |

R²⁷ is H; alkyl; lower alkenyl; cycloalkyl; heterocycloalkyl; aryl; heteroaryl; lower arylalkyl; lower heteroarylalkyl; a suitable protecting group; or -(CR²⁹R³⁰)_{q}R²⁴;
R²⁸ is H; alkyl; lower alkenyl; cycloalkyl; heterocycloalkyl; aryl; heteroaryl; lower arylalkyl; lower heteroarylalkyl; a suitable N-protecting group; -(CR³²R³³)ₛOR²¹; -(CR³²R³³)ₛNR⁴³R⁴²; -(CR³²R³³)ₛNR⁴²CONR⁴³R⁴²; -(CR³²R³³)ₛNR⁴²COR²¹; -(CR³²R³³)ₛNR⁴²SO₂NR²¹; -(CR³²R³³)_{q}COOR²¹; -(CR³²R³³)_{q}COR²³; or -(CR³²R³³)_{q}SO₂R²¹;
R²⁹ is H; F; CF₃; alkyl; alkenyl; cycloalkyl; heterocycloalkyl; aryl; heteroaryl; arylalkyl; heteroarylalkyl; -(CR³²R³³)ₛOR³¹; -(CR³²R³³)ₛSR³¹; -(CR³²R³³)ₛNR²⁸R³¹; -(CR³²R³³)ₛOCONR²⁸R³¹; -(CR³²R³³)ₛOCOOR²¹; -(CR³²R³³)ₛNR²⁸COOR²¹; - (CR³²R³³)ₛNR²⁸COR³¹; -(CR³²R³³)ₛNR²⁸CONR²⁸R³¹; -(CR³²R³³)ₛNR²⁸SO₂R²³; - (CR³²R³³)ₛNR²⁸SO₂NR²⁸R³¹; -(CR³²R³³)_{q}COOR²¹; -(CR³²R³³)_{q}CONR²⁸R³¹; -(CR³²R³³)_{q}SO²NR²⁸R³¹; -(CR³²R³³)_{q}PO(OR²¹)₂; -(CR³²R³³)_{q}COR³¹; -(CR³²R³³)_{q}SO₂R²³; or -(CR³²R³³)_{q}R³¹;
R³⁰ is H; F; CF₃; alkyl; alkenyl; cycloalkyl; heterocycloalkyl; aryl; heteroaryl; arylalkyl; or heteroarylalkyl;
R³¹ is H; alkyl; alkenyl; cycloalkyl; heterocycloalkyl; aryl; heteroaryl; arylalkyl; heteroarylalkyl; or a group of one of the formulae **H51-H55** as shown in Table 7 below.

**Table 7: Groups of Formulae H51-H55**

| | |
|---|---|
| 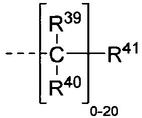 | 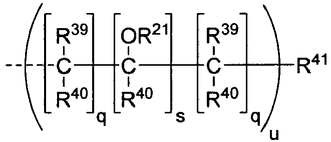 |
| **H51** | **H52** |
| 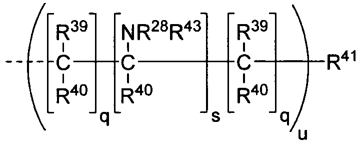 | 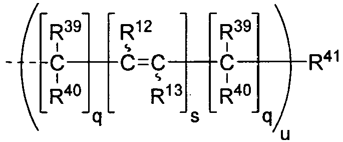 |
| **H53** | **H54** |
| 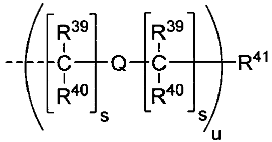 | |
| **H55** | |

R³² and R³³ are independently defined as H; F; CF₃; alkyl; alkenyl; cycloalkyl; heterocycloalkyl; aryl; heteroaryl; arylalkyl; or heteroarylalkyl;
R³⁴ and R³⁵ are independently defined as H; F; Cl; CF₃; OCF₃; OCHF₂; NO₂; CN; alkyl; alkenyl; alkynyl; cycloalkyl; heterocycloalkyl; aryl; heteroaryl; arylalkyl; heteroarylalkyl; -(CR²⁹R³⁰)_{q}OR³¹; -(CR²⁹R³⁰)_{q}SR³¹; -(CR²⁹R³⁰)_{q}NR²⁸R³¹; -(CR²⁹R³⁰)_{q}OCONR²⁸R³¹; -(CR²⁹R³⁰)_{q}NR²⁸COOR²¹; -(CR²⁹R³⁰)_{q}NR²⁸COR³¹; -(CR²⁹R³⁰)_{q}NR²⁸CONR²⁸R³¹; -(CR²⁹R³⁰)_{q}NR²⁸SO₂R²³; -(CR²⁹R³⁰)_{q}NR²⁸SO₂NR²⁸R³¹; -(CR²⁹R³⁰)_{q}COOR²¹; -(CR²⁹R³⁰)_{q}CONR²⁸R³¹; -(CR²⁹R³⁰)_{q}SO₂NR²⁸R³¹; -(CR²⁹R³⁰)_{q}COR³¹; -(CR²⁹R³⁰)_{q}SO₂R²³; or -(CR²⁹R³⁰)_{q}R³¹;
R³⁶ is H; alkyl; alkenyl; alkynyl; cycloalkyl; heterocycloalkyl; aryl; heteroaryl; arylalkyl; heteroarylalkyl; or -NR²⁸R³¹;
R³⁷ is H; alkyl; alkenyl; cycloalkyl; heterocycloalkyl; aryl; heteroaryl; arylalkyl; heteroarylalkyl; a suitable *N*-protecting group; -(CR²⁹R³⁰)ᵣOR³¹; -(CR²⁹R³⁰)ᵣSR³¹; -(CR²⁹R³⁰)ᵣNR²⁸R³¹; -(CR²⁹R³⁰)ᵣOCONR²⁸R³¹; -(CR²⁹R³⁰)ᵣNR²⁸COOR²¹; - (CR²⁹R³⁰)ᵣNR²⁸COR³¹; -(CR²⁹R³⁰)ᵣNR²⁸CONR²⁸R³¹; -(CR²⁹R³⁰)ᵣNR²⁸SO₂R²³; -(CR²⁹R³⁰)ᵣNR²⁸SO₂NR²⁸R³¹; -(CR²⁹R³⁰)_{q}COOR²¹; -(CR²⁹R³⁰)_{q}CONR²⁸R³¹; -(CR²⁹R³⁰)ᵣSO₂NR²⁸R³¹; -(CR²⁹R³⁰)_{q}COR³¹; -(CR²⁹R³⁰)_{q}SO₂R²³; or -(CR²⁹R³⁰)_{q}R³¹;
R³⁸ is H; alkyl; alkenyl; cycloalkyl; heterocycloalkyl; aryl; heteroaryl; arylalkyl; heteroarylalkyl; -(CR²⁹R³⁰)_{q}OR³¹; -(CR²⁹R³⁰)_{q}SR³¹; -(CR²⁹R³⁰)_{q}NR²⁸R³¹ -(CR²⁹R³⁰)_{q}NR²⁹COOR²¹; -(CR²⁹R³⁰)_{q}NR²⁸COR³¹; -(CR²⁹R³⁰)_{q}NR²⁸CONR²⁸R³¹; -(CR²⁹R³⁰)_{q}COOR²¹; - (CR²⁹R³⁰)_{q}CONR²⁸R³¹; -(CR²⁹R³⁰)_{q}COR³¹; or -(CR²⁹R³⁰)_{q}R³¹;
R³⁹ is H; F; CF₃; alkyl; alkenyl; cycloalkyl; heterocycloalkyl; aryl; heteroaryl; arylalkyl; heteroarylalkyl; -(CR³²R³³)ᵤOR²¹; -(CR³²R³³)ᵤNR²⁸R⁴³; -(CR³²R³³)ₜCOOR²¹; or -(CR³²R³³)ₜCONR²⁸R⁴³;
R⁴⁰ is H; F; CF₃; alkyl; alkenyl; cycloalkyl; heterocycloalkyl; aryl; heteroaryl; arylalkyl; heteroarylalkyl; -(CR³²R³³)ᵤOR²¹; -(CR³²R³³)ᵤNR²⁸R⁴³; -(CR³²R³³)ₜCOOR²¹; or - (CR³²R³³)ₜCONR²⁸R⁴³;
R⁴¹ is H; CF₃; alkyl; alkenyl; cycloalkyl; heterocycloalkyl; aryl; heteroaryl; arylalkyl; heteroarylalkyl; -OR²¹; -NR²⁸R⁴³; -NR²⁸COR²³; -NR²⁸COOR²¹; -NR²⁸SO₂R²³; -NR²⁸CONR²⁸R⁴³; -COOR²¹; -CONR²⁸R⁴³; -C(=NR⁴³) NR²⁸R⁴³; -NR²⁸C(=NR⁴³) NR²⁸R⁴³; or a group of one of the formulae **H56-H110** as shown in Table 8 below.

**Table 8: Groups of Formulae H56-H110 (continued on the following pages)**

| | | | |
|---|---|---|---|
| 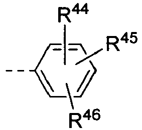 | 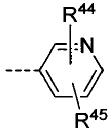 | 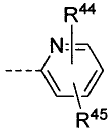 | 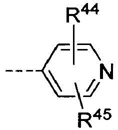 |
| **H56** | **H57** | **H58** | **H59** |
| 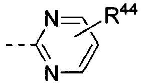 | 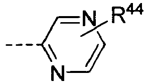 | 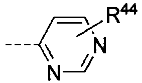 | 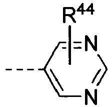 |
| **H60** | **H61** | **H62** | **H63** |
| 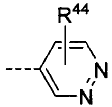 | 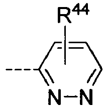 | 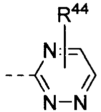 | 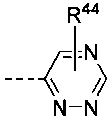 |
| **H64** | **H65** | **H66** | **H67** |
| 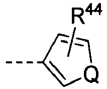 | 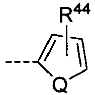 | 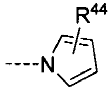 | 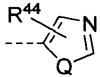 |
| **H68** | **H69** | **H70** | **H71** |
| 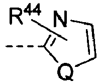 | 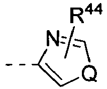 | 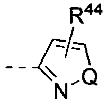 | 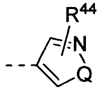 |
| **H72** | **H73** | **H74** | **H75** |
| 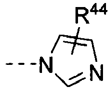 | 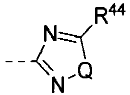 | 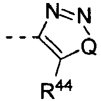 | 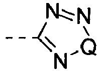 |
| **H76** | **H77** | **H78** | **H79** |
| 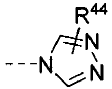 | 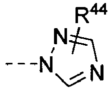 | 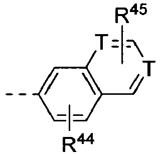 | 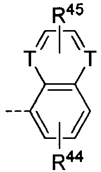 |
| **H80** | **H81** | **H82** | **H83** |
| 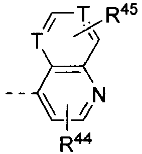 | 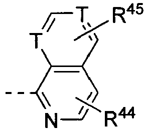 | 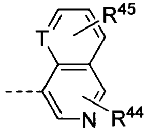 | 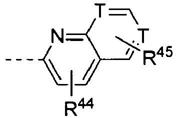 |
| **H84** | **H85** | **H86** | **H87** |
| 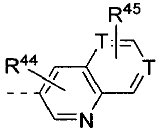 | 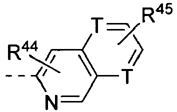 | 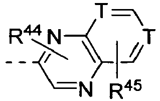 | 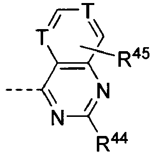 |
| **H88** | **H89** | **H90** | **H91** |
| 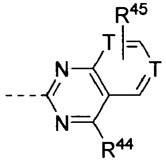 | 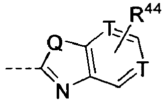 | 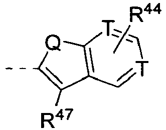 | 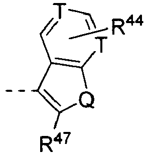 |
| **H92** | **H93** | **H94** | **H95** |
| 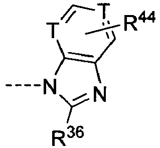 | 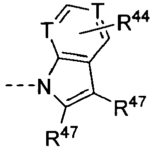 | 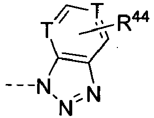 | 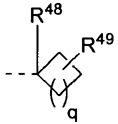 |
| **H96** | **H97** | **H98** | **H99** |
| 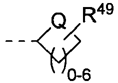 | 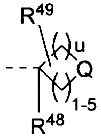 | 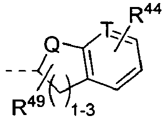 | 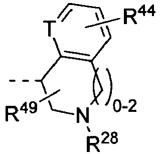 |
| **H100** | **H101** | **H102** | **H103** |
| 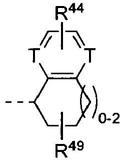 | 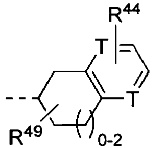 | 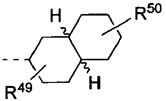 | 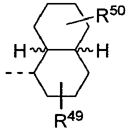 |
| **H104** | **H105** | **H106** | **H107** |
| 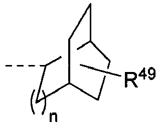 | 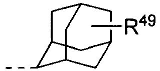 | | 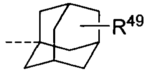 |
| **H108** | **H109** | | **H110** |

R⁴² is H; alkyl; alkenyl; cycloalkyl; cycloheteroalkyl; aryl; heteroaryl; -(CR²³R³³)ₛOR²¹; -(CR²³R³³)ₛNR⁴R⁴³; -(CR²³R³³)_{q}COOR²¹; or -(CR²³R³³)_{q}CONR⁴R⁴³;
R⁴³ is H; alkyl; alkenyl; cycloalkyl; heterocycloalkyl; aryl; heteroaryl; arylalkyl; heteroarylalkyl; or a suitable N-protecting group;
R⁴⁴, R⁴⁵ and R⁴⁶ are independently defined as H; F; CF₃; OCF₃; OCHF₂; NO₂; CN; alkyl; alkenyl; alkynyl; cycloalkyl; heterocycloalkyl; aryl; heteroaryl; arylalkyl; heteroarylalkyl; -OR²³; -NR²⁸R⁴³; -NR²⁸COR²³; -NR²⁸SO₂R²³; -NR²⁸CONR²⁸R⁴³; -COR²³; or -SO₂R²³;
R⁴⁷ is H; CF₃; alkyl; alkenyl; alkynyl; cycloalkyl; heterocycloalkyl; aryl; heteroaryl; arylalkyl; heteroarylalkyl; -COOR²¹; or -CONR²⁸R⁴³;
R⁴⁸ is H; F; CF₃; alkyl; alkenyl; cycloalkyl; cycloheteroalkyl; aryl; heteroaryl; -(CR²³R³³)ₜOR²¹; - (CR²³R³³)ₜNR²⁸R⁴³; -(CR²³R³³)ₜCOOR²¹; or -(CR²³R³³)ₜCONR²¹R⁴³
R⁴⁹ and R⁵⁰ are independently defined as H; F; CF₃; alkyl; alkenyl; cycloalkyl; heterocycloalkyl; aryl; heteroaryl; arylalkyl; heteroarylalkyl; -(CR³²R³³)_{q}OR²¹; -(CR³²R³³)_{q}NR²⁸R⁴³; -(CR³²R³³)_{q}COOR²¹; or -(CR³²R³³)_{q}CONR²⁸R⁴³;
R⁵¹ is H; F; Cl; CF₃; OCF₃; or lower alkyl;
R⁵² is H; F; Cl; CF₃; OCF₃; OCHF₂; NO₂; CN; lower alkyl; lower alkenyl; lower alkynyl; lower cycloalkyl; lower heterocycloalkyl; aryl; heteroaryl; lower arylalkyl; or lower heteroarylalkyl;
R⁵³ is CF₃; OCF₃; CN; lower alkyl; lower cycloalkyl; aryl; lower alkoxy; or aryloxy.

Taken together, the following pairs of said substituents can define cyclic structural elements:
Taken together (R⁴ and R¹¹); (R⁴ and R²⁷) ; (R⁵ and R⁶); (R⁵ and R⁷); (R⁵ and R⁹); (R⁵ and R¹⁴); (R⁵ and R¹⁶); (R⁷ and R⁸); (R⁷ and R⁹); (R⁷ and R¹⁶); (R⁹ and R¹⁰); (R¹⁴ and R¹⁵); (R¹⁶ and R¹⁷); (R¹⁸ and R¹⁹); (R²⁷ and R²⁸); (R²⁸ and R³¹); (R²⁸ and R⁴³); (R²⁹ and R³⁰); (R³² and R³³); (R³⁴ and R³⁵); (R³⁷ and R³⁸); (R³⁹ and R⁴⁰); (R³⁹ and R⁴¹); (R³⁹ and R⁴⁹); (R⁴² and R⁴³); (R⁴⁴ and R⁴⁵); or (R⁴⁴ and R⁴⁶) can form optionally substituted cycloalkyl or heterocycloalkyl moieties.

In addition, the structural elements -NR⁴R¹¹; -NR¹¹R²⁷; -NR²⁷R²⁸; -NR²⁸R³¹ or -NR²⁸R⁴³ can form one of the groups of formulae **H111-H118** as shown in Table 9 below.

**Table 9: Heterocyclic Groups Defined by Linking the Residues of the Disubstituted Amino Groups -NR⁴R¹¹; -NR¹¹R²⁷; -NR²⁷R²⁸; -NR²⁸R³¹ or -NR²⁸R⁴³.**

| | | | |
|---|---|---|---|
| 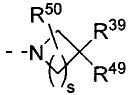 | 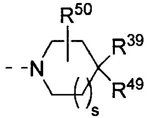 | 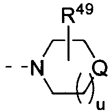 | 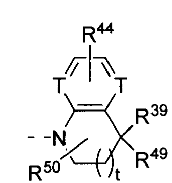 |
| **H111** | **H112** | **H113** | **H114** |
| 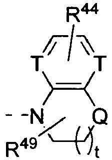 | 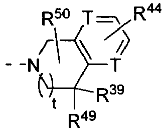 | 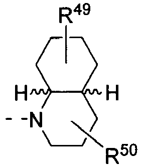 | 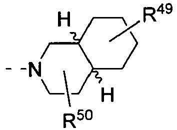 |
| **H115** | **H116** | **H117** | **H118** |

Variable heteroatoms and connector groups in the aforementioned structures are:
Z is O; S; S(=O); S(=O)₂; or NR²⁸;
Y is O; S; or NR⁴;
Q is O; S; or NR²⁸;
T is CR⁴⁶ or N; in case T occurs several times in the same ring structure each T is defined independently of the other.

The indices in the aforementioned structures are defined as:
- q: is an integer of 0-4;
- r: is an integer of 2-4;
- s: is an integer of 1-4;
- t: is an integer of 0-2;
- u: is an integer of 1-2.

Some of the aforementioned substituents can occur several times within the same molecular entity, for example, but not limited to R¹⁴, R¹⁵, R¹⁶, R¹⁷, R¹⁸, R¹⁹, R²⁸, R²⁹, R³⁰, R³¹, R³², R³³, R³⁹, R⁴⁰, R⁴¹, R⁴², R⁴³, R⁴⁴, R⁹⁵, R⁴⁶, and R⁵⁰. Each one of such substituents shall be selected independently from the other radicals of the same type and within the scope of the definition of the respective group.

"Salts" as understood herein are especially, but not limited to, the pharmaceutically acceptable salts of compounds of formula **Ia/Ib.** Such salts are formed, for example, as acid addition salts, preferably with organic or inorganic acids, from compounds of type **Ia/Ib** with a basic nitrogen atom. Suitable inorganic acids are, for example, halogen acids, such as hydrochloric acid, sulfuric acid, or phosphoric acid. Suitable organic acids are, for example, carboxylic, phosphonic, sulfonic or sulfamic acids; like acetic acid, propionic acid, octanoic acid, decanoic acid, dodecanoic acid, glycolic acid, lactic acid, fumaric acid, succinic acid, adipic acid, pimelic acid, suberic acid, azelaic acid, malic acid, tartaric acid, citric acid, amino acids, such as glutamic acid or aspartic acid, maleic acid, hydroxymaleic acid, methylmaleic acid, cyclohexanecarboxylic acid, adamantinecarboxylic acid, benzoic acid, salicylic acid, 4-aminosalicylic acid, phthalic acid, phenylacetic acid, mandelic acid, cinnamic acid, methane- or ethane-sulfonic acid, 2-hydroxyethanesulfonic acid, ethane-1,2-disulfonic acid, benzenesulfonic acid, 2-naphthalenesulfonic acid, 1,5-naphthalene-disulfonic acid, 2-, 3- or 4-methylbenzenesulfonic acid, methylsulfuric acid, ethylsulfuric acid, dodecylsulfuric acid, N-cyclohexylsulfamic acid, N-methyl-, N-ethyl- or N-propyl-sulfamic acid, or other organic protonic acids, such as ascorbic acid.

As used in this description, the term "alkyl", taken alone or in combinations (i.e. as part of another group, such as "arylalkyl") designates saturated, straight-chain or branched hydrocarbon radicals having up to 24, preferably up to 12, carbon atoms. Most preferred in the present invention, "alkyl" is "lower alkyl" which designates alkyl groups having up to 6 carbon atoms.

The term "alkenyl", taken alone or in combinations, designates straight chain or branched hydrocarbon radicals having up to 24, preferably up to 12, carbon atoms and containing at least one or, depending on the chain length, up to four olefinic double bonds. Such alkenyl moieties can exist as E or Z configurations, both of which are part of the invention.

The term "alkynyl", taken alone or in combinations, refers to an aliphatic hydrocarbon chain and includes, but is not limited to, straight and branched chains having 2 to 10 carbon atoms (unless explicitly specified otherwise) and containing at least one triple bond.

The term "cycloalkyl", taken alone or in combinations, refers to a saturated alicyclic moiety having from three to ten carbon atoms.

The term "heterocycloalkyl", taken alone or in combinations, describes a saturated or partially unsaturated heterocyclic moiety having from three to seven ring carbon atoms and one or more ring heteroatoms selected from nitrogen, oxygen and sulphur. This term includes, for example, azetidinyl, pyrrolidinyl, tetrahydrofuranyl, piperidinyl and the like.

The term "aryl", taken alone or in combinations, designates aromatic carbocyclic hydrocarbon radicals containing one or two six-membered rings, such as phenyl or naphthyl, which may be substituted by up to three substituents such as F, Cl, Br, CF₃, NO₂, lower alkyl or lower alkenyl.

The term "heteroaryl", taken alone or in combinations, designates aromatic heterocyclic radicals containing one or two five- and/or six-membered rings, at least one of them containing up to three heteroatoms selected from the group consisting of O, S and N and whereby the heteroaryl radicals or tautomeric forms thereof may be attached via any suitable atom. Said heteroaryl ring(s) are optionally substituted, e.g. as indicated above for "aryl".

The term "arylalkyl", as used herein, whether used alone or as part of another group, refers to the group -R^{a}-R^{b}, where R^{a} is an alkyl group as defined above, substituted by R^{b}, an aryl group, as defined above. Examples of arylalkyl moieties include, but are not limited to, benzyl, 1-phenylethyl, 2-phenylethyl, 3-phenylpropyl, 2-phenylpropyl and the like. Similarly, the term "lower arylalkyl", refers to the above moiety -R^{a}-R^{b} wherein R^{a} is a lower alkyl group.

The term "heteroarylalkyl", whether used alone or as part of another group, refers to the group -R^{a}-R^{c}, where R^{a} is an alkyl group as defined above, substituted by R^{c}, a heteroaryl group, as defined above. Analogously the term "lower heteroarylalkyl", refers to the above moiety -R^{a}-R^{c} but wherein R^{a} is a lower alkyl group.

The terms "alkoxy" and "aryloxy", taken alone or in combinations, refer to the group -O-R^{a}, wherein R^{a}, is an alkyl group or an aryl group as defined above.

"Amino" designates primary, secondary or tertiary amines. Particular secondary and tertiary amines are alkylamines, dialkylamines, arylamines, diarylamines, arylalkylamines and diarylamines wherein the alkyl or aryl is as herein defined and optionally substituted.

The term "optionally substituted" is intended to mean that a group, such as but not limited to alkyl, alkenyl, alkynyl, cycloalkyl, aryl, heteroaryl, heterocycloalkyl, alkoxy and aryloxy may be substituted with one or more substituents independently selected from, e.g., halogen (F, Cl, Br, I), cyano (-CN) , nitro (-NO₂), -SR^{a}, -S(O)R^{a}, -S(O)₂R^{a}, -R^{a}, -C(O)R^{a}, -C(O)OR^{a}, -C (O) NR^{b}R^{c}, -C (=NR^{a}) NR^{b}R^{c}, -OR^{a}, -OC(O)R^{a}, -OC(O)OR^{a}, -OC(O)NR^{b}R^{c}, -OS (O)R^{a}, -OS (O)₂R^{a}, -OS(O)NR^{b}R^{c}, -OS(O)₂NR^{b}R^{c}, -NR^{b}R^{c}, -NR^{a}C (O) R^{b}, -NR^{a}C (O) OR^{b}, -NR^{a}C (O) NR^{b}R^{c}, -NR^{a}C (=NR^{d})NR^{b}R^{c}, -NR^{a}S (O) R^{b}, -NR^{a}S(O)₂R^{b}, wherein R^{a}, R^{b}, R^{c}. and R^{d} are each independently, e.g., hydrogen, alkyl, alkenyl, alkynyl, cycloalkyl, aryl, heteroaryl, or heterocycloalkyl as described herein; or R^{b} and R^{c} may be taken together with the N-atom to which they are attached forming heterocycloalkyl or heteroaryl. These groups in turn can be substituted with any described moiety, including, but not limited to, one or more moieties selected from the group consisting of halogen (F, Cl, Br, or I), hydroxyl, amino, alkylamino (e.g., monoalkylamino, dialkylamino, or trialkylamino), arylamino (e.g. monoarylamino, diarylamino, or triarylamino), hydroxy, carboxy, alkoxy, aryloxy, nitro, cyano, sulfonic acid, sulfate, phosphonic acid, phosphate, or phosphonate.

Said groups, especially but not limited to hydroxy, amino and carboxyl, may be either unprotected or protected, if necessary, as well-known to those skilled in the art. Examples of suitable protecting groups are as detailed in P.G.M. Wuts, T.W. Greene, Greene's Protective Groups in Organic Synthesis, John Wiley and Sons, 4th Edition, 2006.

As used herein, all groups that can be substituted in one embodiment are indicated to be "optionally substituted", unless otherwise specified.

As mentioned earlier herein, the term "lower" designates radicals and compounds having up to 6 carbon atoms. Thus, for example, the term "lower alkyl" designates saturated, straight-chain, or branched hydrocarbon radicals having up to 6 carbon atoms, such as methyl, ethyl, n-propyl, isopropyl, n-butyl, s-butyl, isobutyl, t-butyl, and the like. Similarly, the term "lower cycloalkyl" designates saturated cyclic hydrocarbon radicals having up to 6 carbon atoms, such as cyclopropyl, cyclobutyl, cyclopentyl, cyclohexyl and the like.

The embodiments of the present invention shall include so-called "prodrugs" of the compounds of this invention. In general, such prodrugs will be functional derivatives of the compounds, which *in vivo* are readily convertible into the required compound. Conventional procedures for the selection and preparation of suitable prodrug derivatives are described, for example, in Hans Bundgaard, Design of Prodrugs, Elsevier, 1985**;** and in Valentino J. Stella et al., Prodrugs: Challenges and Rewards, Springer, 1st ed., 2007**.**

The term "isomer" comprises species of identical chemical formula, constitution and thus molecular mass, such as but not limited to C=C-double bond or amide cis/trans isomers, rotamers, conformers, diastereomers.

All possible stereoisomers (explicitly including atropisomers), conformers and rotamers as well as salts, solvates, clathrates, N-oxides, or isotopically enriched or enantiomerically enriched versions of macrocycles of type **Ia/Ib** are part of this invention.

In another embodiment of this invention, the macrocycles of type **Ia/Ib** are defined as above but wherein
M is -N(R⁴)-
and wherein all possible stereoisomers of such compounds or pharmaceutical acceptable salts thereof are included.

In a preferred embodiment of this invention, the macrocycles of type **Ia/Ib** are defined by groups of selected building blocks **A, B** and C and substituents R¹-R⁵³ as detailed below:
The building block of type **A** is selected from
   **A1**(a1)**; A2**(a1); **A3**(a1); **A4**(a1); **A5(**a1); **A6**(a1); **A7**(a1); **A9**(a1); **A10**(a1); **A73**(a2); **A80**(a2); **A170**(a4); **A171**(a4); **A209**(a7); **A210**(a7); **A240**(a10); **A241**(a10); **A242**(a10)**; A243**(a10); **A272**(a10); **A530**(a18); **A531**(a18); **A532**(a18); **A533**(a18); **A609**(a24); **A610**(a24); **A611**(a24); **A612**(a24); **A613**(a24); **A614**(a24); **A615**(a24); **A631**(a1); **A632**(a1); **A633**(a1); **A641**(a1); **A642**(a1); **A643**(a1); **A651**(a1); **A652**(a1)**;** or **A653**(a1);
the building block of type **B** is selected from
   **B4**(b3); **B5**(b3); **B6**(b3); **B7**(b3); **B8**(b3); **B9**(b3); **B10**(b3); **B12**(b4); **B13**(b4); **B14**(b4); **B15**(b4); **B16**(b4) or **B17**(b5);
the building block of type **C** is selected from
   **C1; C2; C3; C4; C5; C6; C7; C8; C9; C10; C11; C12; C13; C14; C15; C16; C17; C18; C19; C20; C21; C25; C26; C27; C28; C29; C30; C34; C35; C36; C37; C38; C39; C43; C44; C45; C46; C47; C48; C49; C50; C51; C52; C53; C54; C55; C56; C57; C58; C59; C60; C61; C62; C63; C64; C65; C66; C67; C68; C69; C70; C71; C72; C73; C74; C75; C76; C77; C78; C79; C80; C81; C86; C87; C88; C89; C90; C91; C92; or C93;**
and wherein
R¹ is H; F; C1; Br; I; CF₃; OCF₃; OCHF₂; NO_{2;} CN; lower alkyl; lower alkenyl; lower alkynyl; lower cycloalkyl; lower heterocycloalkyl; aryl; heteroaryl; lower arylalkyl; lower heteroarylalkyl; -(CR¹⁸R¹⁹)_{q}OR²⁰; -(CR¹⁸R¹⁹)_{q}NR⁴R¹¹; -(CR¹⁸R¹⁹)_{q}NR⁴COOR²¹; -(CR¹⁸R¹⁹)_{q}NR⁴COR²²; -(CR¹⁸R¹⁹)_{q}NR⁴CONR⁴R¹¹; -(CR¹⁸R¹⁹)_{q}NR⁴SO₂R²³; -(CR¹⁸R¹⁹)_{q}NR⁴SO2NR⁴R¹¹; -(CR¹⁸R¹⁹)_{q}COOR²¹; -(CR¹⁸R¹⁹)_{q}CONR⁴R¹¹; -(CR¹⁸R¹⁹)_{q}SO₂NR⁴R¹¹; -(CR¹⁸R¹⁹)_{q}PO(OR²¹)₂; -(CR¹⁸R¹⁹)_{q}COR²²; -(CR¹⁸R¹⁹)_{q}SO₂R²³; -(CR¹⁸R¹⁹)_{q}OSO₃R²¹; -(CR¹⁸R¹⁹)_{q}R²⁴; -(CR¹⁸R¹⁹)_{q}R²⁵; or -(CR¹⁸R¹⁹)_{q}R²⁶;
R² is H; CF₃; lower alkyl; lower alkenyl; cycloalkyl; heterocycloalkyl; aryl; heteroaryl; lower arylalkyl; lower heteroarylalkyl; -(CR¹⁸R¹⁹)_{q}OR²⁰; -(CR¹⁸R¹⁹)_{q}NR⁴R¹¹; -(CR¹⁸R¹⁹)_{q}NR⁴COOR²¹; -(CR¹⁸R¹⁹)_{q}NR⁴COR²²; -(CR¹⁸R¹⁹)_{q}NR⁴CONR⁴R¹¹; -(CR¹⁸R¹⁹)_{q}NR⁴SO₂R²³; -(CR¹⁸R¹⁹)_{q}NR⁴SO₂NR⁴R¹¹; -(CR¹⁸R¹⁹)_{q}COOR²¹; -(CR¹⁸R¹⁹)_{q}CONR⁴R¹¹; -(CR¹⁸R¹⁹)_{q}SO₂NR⁴R¹¹; - (CR¹⁸R¹⁹)_{q}PO(OR²¹)₂; -(CR¹⁸R¹⁹)_{q}COR²²; -(CR¹⁸R¹⁹)_{q}SO₂R²³; -(CR¹⁸R¹⁹)_{q}R²⁴; -(CR¹⁸R¹⁹)_{q}R²⁵; or -(CR¹⁸R¹⁹)_{q}R²⁶;
R⁴ is H; lower alkyl; lower alkenyl; lower cycloalkyl; lower heterocycloalkyl; or a suitable N-protecting group;
R⁵, R⁷ and R⁹ are independently defined as H; F; CF₃; lower alkyl; lower alkenyl; lower cycloalkyl; lower heterocycloalkyl; aryl; heteroaryl; lower arylalkyl; lower heteroarylalkyl; -(CR¹⁸R¹⁹)_{q}OR²⁰; -(CR¹⁸R¹⁹)_{q}NR⁴R¹¹; -(CR¹⁸R¹⁹)_{q}NR⁴COOR²¹; - (CR¹⁸R¹⁹)_{q}NR⁴COR²²; -(CR¹⁸R¹⁹)_{q}NR⁴CONR⁴R¹¹; -(CR¹⁸R¹⁹)_{q}NR⁴SO₂R²³; -(CR¹⁸R¹⁹)_{q}NR⁴SO₂NR⁴R¹¹; -(CR¹⁸R¹⁹)_{q}COOR²¹; -(CR¹⁸R¹⁹)_{q}CONR⁴R¹¹; -(CR¹⁸R¹⁹)_{q}SO²NR⁴R¹¹; -(CR¹⁸R¹⁹)_{q}PO(OR²¹)₂; -(CR¹⁸R¹⁹)_{q}COR²²; -(CR¹⁸R¹⁹)_{q}SO₂R²³; -(CR¹⁸R¹⁹)_{q}R²⁴; -(CR¹⁸R¹⁹)_{q}R²⁵; or -(CR¹⁸R¹⁹)_{q}R²⁶;
R⁶, R⁸ and R¹⁰ are independently defined as H; CF₃; or lower alkyl;
R¹¹ is H; alkyl; alkenyl; cycloalkyl; lower heterocycloalkyl; aryl; heteroaryl; lower arylalkyl; lower heteroarylalkyl; a suitable protecting group; -(CR¹⁸R¹⁹)ᵣOR²⁰; - (CR¹⁸R¹⁹)ᵣNR⁴R²⁷; - (CR¹⁸R¹⁹)ᵣNR⁴COOR²¹; -(CR¹⁸R¹⁹)ᵣNR⁴CONR⁴R²⁷; - (CR¹⁸R¹⁹)ᵣNR⁴SO₂R²³; -(CR¹⁸R¹⁹)ᵣNR⁴SO₂NR⁴R²⁷; -(CR¹⁸R¹⁹)_{q}COOR²¹; -(CR¹⁸R¹⁹)_{q}CONR⁴R²⁷; -(CR¹⁸R¹⁹)_{q}COR²²; - (CR¹⁸R¹⁹)_{q}SO₂R²³; -(CR¹⁸R¹⁹)_{q}SO₂NR⁴R²⁷; -(CR¹⁸R¹⁹)_{q}R²⁴; -(CR¹⁸R¹⁹)ₛR²⁵-(CR¹⁸R¹⁹)_{q}R²⁶; or -(CR¹⁸R¹⁹)_{q}R³¹;
R¹² and R¹³ are independently defined as H; or lower alkyl.
R¹⁴ and R¹⁶ are independently defined as H; F; CF₃; lower alkyl; lower alkenyl; lower cycloalkyl; lower heterocycloalkyl; aryl; heteroaryl; lower arylalkyl; lower heteroarylalkyl; -(CR¹⁸R¹⁹)_{q}OR²⁰; -(CR¹⁸R¹⁹)_{q}NR⁴R¹¹; -(CR¹⁸R¹⁹)_{q}NR⁴COOR²¹; -(CR¹⁸R¹⁹)_{q}NR⁴COR²²; -(CR¹⁸R¹⁹)_{q}NR⁴CONR⁴R¹¹; -(CR¹⁸R¹⁹)_{q}NR⁴SO₂R²³; -(CR¹⁸R¹⁹)_{q}COOR²¹; -(CR¹⁸R¹⁹)_{q}CONR⁴R¹¹; or -(CR¹⁸R¹⁹)_{q}COR²²;
R¹⁵ and R¹⁷ are independently defined as H; CF₃; or lower alkyl;
R¹⁸ is H; F; CF₃; lower alkyl; lower alkenyl; lower cycloalkyl; lower heterocycloalkyl; aryl; heteroaryl; lower arylalkyl; lower heteroarylalkyl; -(CR²⁹R³⁰)ₛOR³¹; -(CR²⁹R³⁰)ₛNR²⁸R³¹; -(CR²⁹R³⁰) ₛNR²⁸COOR²¹; -(CR²⁹R³⁰)ₛNR²⁸COR³¹; -(CR²⁹R³⁰)ₛNR²⁸CONR²⁸R³¹; -(CR²⁹R³⁰)ₛNR²⁸SO₂R²³; -(CR²⁹R³⁰) ₛNR²⁸SO₂NR²⁸R³¹; -(CR²⁹R³⁰)_{q}COOR²¹; -(CR²⁹R³⁰) _{q}CONR²⁸R³¹; -(CR²⁹R³⁰)_{q}SO₂NR²⁸R³¹; -(CR²⁹R³⁰)_{q}PO(OR²¹)₂; - (CR²⁹R³⁰)_{q}COR³¹; -(CR²⁹R³⁰)_{q}SO₂R²³; -(CR²⁹R³⁰)_{q}R²⁴; -(CR²⁹R³⁰)_{q}R²⁵; or -(CR²⁹R³⁰)_{q}R²⁶;
R¹⁹ is H; CF₃; or lower alkyl;
R²⁰ is H; lower alkyl; lower alkenyl; lower cycloalkyl; lower heterocycloalkyl; aryl; heteroaryl; lower arylalkyl; lower heteroarylalkyl; -(CR²⁹R³⁰)ᵣOR³¹; -(CR²⁹R³⁰)ᵣNR²⁸R³¹; -(CR²⁹R³⁰)ᵣNR²⁸COOR²¹; -(CR²⁹R³⁰)ᵣNR²⁸COR³¹; -(CR²⁹R³⁰)ᵣNR²⁸CONR²⁸R³¹; -(CR²⁹R³⁰)ᵣNR²⁸SO₂R²³; -(CR²⁹R³⁰)_{q}COOR²¹; -(CR²⁹R³⁰)_{q}CONR²⁸R³¹; -(CR²⁹R³⁰)_{q}SO₂NR²⁸R³¹; -(CR²⁹R³⁰)_{q}COR³¹; -(CR²⁹R³⁰)_{q}SO₂R²³; -(CR²⁹R³⁰)_{q}R²⁴; -(CR²⁹R³⁰)_{q}R²⁵; -(CR²⁹R³⁰)_{q}R²⁶; or - (CR²⁹R³⁰)_{q}R³¹;
R²² is lower alkyl; lower alkenyl; lower cycloalkyl; lower heterocycloalkyl; aryl; heteroaryl; lower arylalkyl; lower heteroarylalkyl; -(CR²⁹R³⁰)ₛOR³¹; -(CR²⁹R³⁰)ₛNR²⁸R³¹; -(CR²⁹R³⁰)ₛNR²⁸COOR²¹; -(CR²⁹R³⁰)ₛNR²⁸COR³¹; -(CR²⁹R³⁰)ₛNR²⁸CONR²⁸R³¹; -(CR²⁹R³⁰)ₛNR²⁸SO₂R²³; -(CR²⁹R³⁰)ₛCOOR²¹; -(CR²⁹R³⁰)ₛCONR²⁸R³¹; -(CR²⁹R³⁰)ₛSO₂NR²⁸R³¹; -(CR²⁹R³⁰)ₜCOR³¹; -(CR²⁹R³⁰)ₛSO²R²³; -(CR²⁹R³⁰)ₜR²⁴; -(CR²⁹R³⁰)ₜR²⁵; -(CR²⁹R³⁰)ₜR²⁶; or -(CR²⁹R³⁰)ₜR³¹;
R²⁹ is H; F; CF₃; lower alkyl; lower alkenyl; lower cycloalkyl; lower heterocycloalkyl; aryl; heteroaryl; lower arylalkyl; lower heteroarylalkyl; -(CR³²R³³)ₛOR³¹; -(CR³²R³³)ₛNR²⁸R³¹; - (CR³²R³³)ₛNR²⁸COOR²¹; -(CR³²R³³)ₛNR²⁸COR³¹; -(CR³²R³³)ₛNR²⁸CONR²⁸R³¹; -(CR³²R³³)ₛNR²⁸SO₂R²³; -(CR³²R³³)_{q}COOR²¹; -(CR³²R³³)_{q}CONR²⁸R³¹; -(CR³²R³³)_{q}SO₂NR²⁸R³¹; -(CR³²R³³)_{q}PO(O^{R21})₂; - (CR³²R³³)_{q}COR³¹; -(CR³²R³³)_{q}SO²R²³; or - (CR³²R³³)_{q}R³¹;
R³⁰ and R³³ are independently defined as H; CF₃; or lower alkyl;
R³⁴ and R³⁵ are independently defined as H; F; Cl; CF₃; OCF₃; OCHF₂; NO₂; CN; lower alkyl; lower alkenyl; lower alkynyl; lower cycloalkyl; lower heterocycloalkyl; aryl; heteroaryl; lower arylalkyl; lower heteroarylalkyl; -(CR²⁹R³⁰)_{q}OR³¹; -(CR²⁹R³⁰)_{q}NR²⁸R³¹; -(CR²⁹R³⁰)_{q}NR²⁸COOR²¹; -(CR²⁹R³⁰)_{q}NR²⁸COR³¹; -(CR²⁹R³⁰)_{q}NR²⁸CONR²⁸R³¹; -(CR²⁹R³⁰)_{q}NR²⁸SO₂R²³; -(CR²⁹R³⁰)_{q}COOR²¹; -(CR²⁹R³⁰)_{q}CONR²⁸R³¹; -(CR²⁹R³⁰)_{q}SO₂NR²⁸R³¹; -(CR²⁹R³⁰)_{q}COR³¹; -(CR²⁹R³⁰)_{q}SO₂R²³; or - (CR²⁹R³⁰)_{q}R³¹;
R³⁷ is H; lower alkyl; lower alkenyl; lower cycloalkyl; lower heterocycloalkyl; aryl; heteroaryl; lower arylalkyl; lower heteroarylalkyl; a suitable N-protecting group; -(CR²⁹R³⁰)ᵣOR³¹; -(CR²⁹R³⁰)ᵣNR²⁸R³¹; -(CR²⁹R³⁰)ᵣNR²⁸COOR²¹; -(CR²⁹R³⁰)ᵣNR²⁸COR³¹; -(CR²⁹R³⁰)ᵣNR²⁸CONR²⁸R³¹; -(CR²⁹R³⁰)ᵣNR²⁸SO₂R²³; -(CR²⁹R³⁰)_{q}COOR²¹; -(CR²⁹R³⁰)_{q}CONR²⁸R³¹; -(CR²⁹R³⁰)ᵣSO₂NR²⁸R³¹; -(CR²⁹R³⁰)_{q}COR³¹; -(CR²⁹R³⁰)_{q}SO₂R²³; or -(CR²⁹R³⁰)_{q}R³¹;
R³⁸ is H; lower alkyl; lower alkenyl; lower cycloalkyl; lower heterocycloalkyl; aryl; heteroaryl; lower arylalkyl; lower heteroarylalkyl; -(CR²⁹R³⁰)_{q}OR³¹; -(CR²⁹R³⁰)_{q}NR²⁸R³¹; -(CR²⁹R³⁰)_{q}NR²⁸COOR²¹; -(CR²⁹R³⁰)_{q}NR²⁸COR³¹; -(CR²⁹R³⁰)_{q}NR²⁸CONR²⁸R³¹; -(CR²⁹R³⁰)_{q}COOR²¹; -(CR²⁹R³⁰)_{q}CONR²⁸R³¹; -(CR²⁹R³⁰)_{q}COR³¹; or -(CR²⁹R³⁰)_{q}R³¹;
Z is O; S; S(=O); or S(=O)₂;
U is -C(=O)-; -NR⁴-C(=O)-; -C(=O)-C(=O)-; or
-C(-OR²⁰)₂-C(=O)- .

In a further preferred embodiment of this invention, the macrocycles of type **Ia/Ib** are defined by groups of selected building blocks **A, B** and **C** and substituents R¹-R⁵³ as detailed below:
The building block of type **A** is selected from
   **A1**(a1)**; A2**(a1); **A3**(a1); **A4**(a1); **A5**(a1); **A6**(a1); **A7**(a1); **A9**(a1); **A10**(a1); **A73**(a2); **A170**(a4); **A209**(a7); **A240**(a10); **A272**(a10); **A532**(a18); **A614**(a24) ; **A631**(a1); **A632**(a1); **A633**(a1); **A641**(a1); **A642**(a1); **A643**(a1); **A651**(a1); **A652**(a1)**;** or **A653**(a1);
the building block of type **B** is selected from
   **B7**(b3); **B8**(b3); **B9**(b3); **B10**(b3) ; or **B17**(b5);
the building block of type C is selected from
   **C1; C2; C4; C7; C8; C9; C10; C11; C12; C13; C14; C15; C16; C17; C18; C19; C20; C21; C25; C26; C27; C28; C29; C30; C34; C35; C36; C37; C38; C39; C43; C44; C45; C46; C47; C48; C49; C90; C91; C92;** or **C93;**
and wherein
R¹ is H; F; C1; CF₃; OCF₃; OCHF₂; NO₂; CN; lower alkyl; lower alkenyl; lower alkynyl; lower cycloalkyl; lower heterocycloalkyl; aryl; heteroaryl; lower arylalkyl; lower heteroarylalkyl; -(CR¹⁸R¹⁹)_{q}OR²⁰; -(CR¹⁸R¹⁹)_{q}NR⁴R¹¹; -(CR¹⁸R¹⁹)_{q}NR⁴COR²²; -(CR¹⁸R¹⁹)_{q}NR⁴CONR⁴R¹¹; -(CR¹⁸R¹⁹)_{q}NR⁴SO₂R²³; -(CR¹⁸R¹⁹)_{q}NR⁴SO₂NR⁴R¹¹; -(CR¹⁸R¹⁹)_{q}COOR²¹; -(CR¹⁸R¹⁹)_{q}CONR⁴R¹¹; -(CR¹⁸R¹⁹)_{q}SO₂NR⁴R¹¹; -(CR¹⁸R¹⁹)_{q}COR²²; -(CR¹⁸R¹⁹)_{q}SO₂R²³; -(CR¹⁸R¹⁹)_{q}R²⁴; -(CR¹⁸R¹⁹)_{q}R²⁵; or -(CR¹⁸R¹⁹)_{q}R²⁶;
R² is H; CF₃; lower alkyl; lower alkenyl; cycloalkyl; heterocycloalkyl; aryl; heteroaryl; lower arylalkyl; lower heteroarylalkyl; -(CR¹⁸R¹⁹)_{q}OR²⁰; -(CR¹⁸R¹⁹)_{q}NR⁴R¹¹; - (CR¹⁸R¹⁹)_{q}NR⁴COR²²; -(CR¹⁸R¹⁹)_{q}NR⁴CONR⁴R¹¹; -(CR¹⁸R¹⁹)_{q}NR⁴SO₂R²³; -(CR¹⁸R¹⁹)_{q}NR⁴SO₂NR⁴R¹¹; -(CR¹⁸R¹⁹)_{q}COOR²¹; -(CR¹⁸R¹⁹)_{q}CONR⁴R¹¹; -(CR¹⁸R¹⁹)_{q}SO₂NR⁴R¹¹; -(CR¹⁸R¹⁹)_{q}COR²²; -(CR¹⁸R¹⁹)_{q}SO₂R²³; -(CR¹⁸R¹⁹)_{q}R²⁴; - (CR¹⁸R¹⁹)_{q}R²⁵; or -(CR¹⁸R¹⁹)_{q}R²⁶;
R⁴ is H; lower alkyl; lower alkenyl; or a suitable N-protecting group;
R⁵, R⁷ and R⁹ are independently defined as H; CF₃; lower alkyl; lower alkenyl; lower cycloalkyl; lower heterocycloalkyl; aryl; heteroaryl; lower arylalkyl; lower heteroarylalkyl; -(CR¹⁸R¹⁹)_{q}OR²⁰; -(CR¹⁸R¹⁹)_{q}NR⁴R¹¹; -(CR¹⁸R¹⁹)_{q}NR⁴COR²²; -(CR¹⁸R¹⁹)_{q}NR⁴CONR⁴R¹¹; -(CR¹⁸R¹⁹)_{q}NR⁴SO₂R²³; -(CR¹⁸R¹⁹)_{q}COOR²¹; -(CR¹⁸R¹⁹)_{q}CONR⁴R¹¹; -(CR¹⁸R¹⁹)_{q}SO₂NR⁴R¹¹; -(CR¹⁸R¹⁹)_{q}COR²²; -(CR¹⁸R¹⁹)_{q}SO₂R²³; -(CR¹⁸R¹⁹)_{q}R²⁴; -(CR¹⁸R¹⁹)_{q}R²⁵; or -(CR¹⁸R¹⁹)_{q}R²⁶;
R⁶, R⁸ and R¹⁰ are independently defined as H; CF₃; or CH₃; R¹¹ is H; alkyl; alkenyl; cycloalkyl; lower heterocycloalkyl; aryl; heteroaryl; lower arylalkyl; lower heteroarylalkyl; a suitable protecting group; -(CR¹⁸R¹⁹)ᵣOR²⁰; -(CR¹⁸R¹⁹)ᵣNR⁴R²⁷; -(CR¹⁸R¹⁹)ᵣNR⁴CONR⁴R²⁷; -(CR¹⁸R¹⁹)ᵣNR⁴SO₂R²³; -(CR¹⁸R¹⁹)qCOOR²¹; -(CR¹⁸R¹⁹)_{q}CONR⁴R²⁷; -(CR¹⁸R¹⁹)_{q}COR²²; -(CR¹⁸R¹⁹)_{q}R²⁴; -(CR¹⁸R¹⁹)ₛR²⁵; -(CR¹⁸R¹⁹)_{q}R²⁶; or -(CR¹⁸R¹⁹)_{q}R³¹;
R¹² and R¹³ are independently defined as H; or lower alkyl.
R¹⁴ and R¹⁶ are independently defined as H; F; CF₃; lower alkyl; lower alkenyl; lower cycloalkyl; lower heterocycloalkyl; aryl; heteroaryl; lower arylalkyl; lower heteroarylalkyl; -(CR¹⁸R¹⁹)_{q}OR²⁰; -(CR¹⁸R¹⁹)_{q}NR⁴R¹¹; -(CR¹⁸R¹⁹)_{q}NR⁴COR²²; -(CR¹⁸R¹⁹)_{q}COOR²¹; or - (CR¹⁸R¹⁹)_{q}CONR⁴R¹¹;
R¹⁵ and R¹⁷ are independently defined as H; CF₃; or CH₃;
R¹⁸ is H; F; CF₃; lower alkyl; lower alkenyl; lower cycloalkyl; lower heterocycloalkyl; aryl; heteroaryl; lower arylalkyl; lower heteroarylalkyl; -(CR²⁹R³⁰)ₛOR³¹; -(CR²⁹R³⁰)ₛNR²⁸R³¹; -(CR²⁹R³⁰)ₛNR²⁸COR³¹; -(CR²⁹R³⁰)ₛNR²⁸CONR²⁸R³¹; -(CR²⁹R³⁰)ₛNR²⁸SO₂R²³; -(CR²⁹R³⁰)_{q}COOR²¹; -(CR²⁹R³⁰)_{q}CONR²⁸R³¹; -(CR²⁹R³⁰)_{q}SO₂NR²⁸R³¹; -(CR²⁹R³⁰)_{q}COR³¹; -(CR²⁹R³⁰)_{q}SO₂R²³; -(CR²⁹R³⁰)_{q}R²⁴; -(CR²⁹R³⁰)_{q}R²⁵; or -(CR²⁹R³⁰)_{q}R²⁶;
R¹⁹ is H; CF₃; or CH₃;
R²⁰ is H; lower alkyl; lower alkenyl; lower cycloalkyl; lower heterocycloalkyl; aryl; heteroaryl; lower arylalkyl; lower heteroarylalkyl; -(CR²⁹R³⁰)ᵣOR³¹; -(CR²⁹R³⁰)ᵣNR²⁸R³¹; -(CR²⁹R³⁰)ᵣNR²⁸COR³¹; -(CR²⁹R³⁰)ᵣNR²⁸CONR²⁸R³¹; -(CR²⁹R³⁰)ᵣNR²⁸SO₂R²³; -(CR²⁹R³⁰)_{q}COOR²¹; -(CR²⁹R³⁰)_{q}CONR²⁸R³¹; -(CR²⁹R³⁰)_{q}SO₂NR²⁸R³¹; -(CR²⁹R³⁰)_{q}COR³¹; -(CR²⁹R³⁰)_{q}SO₂R²³; -(CR²⁹R³⁰)_{q}R²⁴; -(CR²⁹R³⁰)_{q}R²⁵; -(CR²⁹R³⁰)_{q}R²⁶; or -(CR²⁹R³⁰)_{q}R³¹;
R²² is lower alkyl; lower alkenyl; lower cycloalkyl; lower heterocycloalkyl; aryl; heteroaryl; lower arylalkyl; lower heteroarylalkyl; -(CR²⁹R³⁰)ₛOR³¹; -(CR²⁹R³⁰)ₛNR²⁸R³¹; -(CR²⁹R³⁰)ₛNR²⁸COR³¹; -(CR²⁸R³⁰)ₛNR²⁸CONR²⁸R³¹; -(CR²⁸R³⁰)ₛNR²⁸SO₂R²³; -(CR²⁹R³⁰)ₛCOOR²¹; -(CR²⁹R³⁰)ₛCONR²⁸R³¹; -(CR²⁹R³⁰)ₛSO₂NR²⁸R³¹; -(CR²⁹R³⁰)ₜCOR³¹; -(CR²⁹R³⁰)ₛSO₂R²³; -(CR²⁹R³⁰)ₜR²⁴; -(CR²⁹R³⁰)ₜR²⁵; -(CR²⁹R³⁰)ₜR²⁶; or -(CR²⁹R³⁰)ₜR³¹;
R²⁹ is H; F; CF₃; lower alkyl; lower alkenyl; lower cycloalkyl; lower heterocycloalkyl; aryl; heteroaryl; lower arylalkyl; lower heteroarylalkyl; -(CR³²R³³)ₛOR³¹; -(CR³²R³³) ₛNR²⁸R³¹; -(CR³²R³³)ₛNR²⁸COR³¹; -(CR³²R³³)ₛNR²⁸CONR²⁸R³¹; -(CR³²R³³)_{q}COOR²¹; -(CR³²R³³)_{q}CONR²⁸R³¹; -(CR³²R³³)_{q}COR³¹; or -(CR³²R³³)_{q}R³¹;
R³⁰ and R³³ are independently defined as H; CF₃; or CH₃;
R³⁴ and R³⁵ are independently defined as H; F; Cl; CF₃; OCF₃; OCHF₂; lower alkyl; lower alkenyl; lower alkynyl; lower cycloalkyl; lower heterocycloalkyl; aryl; heteroaryl; lower arylalkyl; lower heteroarylalkyl; -(CR²⁹R³⁰)_{q}OR³¹; -(CR²⁹R³⁰)_{q}NR²⁸R³¹; -(CR²⁹R³⁰)_{q}NR²⁸COR³¹; -(CR²⁹R³⁰)_{q}NR²⁸CONR²⁸R³¹; -(CR²⁹R³⁰)_{q}NR²⁸SO₂R²³; -(CR²⁹R³⁰)_{q}COOR²¹; -(CR²⁹R³⁰)_{q}CONR²⁸R³¹; -(CR²⁹R³⁰)_{q}SO₂NR²⁸R³¹; -(CR²⁹R³⁰)_{q}COR³¹; -(CR²⁹R³⁰)_{q}SO₂R²³; or -(CR²⁹R³⁰)_{q}R³¹;
R³⁷ is H; lower alkyl; lower alkenyl; lower cycloalkyl; lower heterocycloalkyl; aryl; heteroaryl; lower arylalkyl; lower heteroarylalkyl; a suitable N-protecting group; -(CR²⁹R³⁰)ᵣOR³¹; -(CR²⁹R³⁰)ᵣNR²⁹R³¹; -(CR²⁹R³⁰)ᵣNR²⁸COOR²¹; -(CR²⁹R³⁰)ᵣNR²⁸COR³¹; -(CR²⁹R³⁰) ᵣNR²⁸CONR²⁸R³¹; -(CR²⁹R³⁰)_{q}COOR²¹; -(CR²⁹R³⁰)_{q}CONR²⁸R³¹; -(CR²⁹R³⁰)_{q}COR³¹; or -(CR²⁹R³⁰)_{q}R³¹;
R³⁸ is H; lower alkyl; lower alkenyl; lower cycloalkyl; lower heterocycloalkyl; aryl; heteroaryl; lower arylalkyl; lower heteroarylalkyl; -(CR²⁹R³⁰)_{q}OR³¹; -(CR²⁹R³⁰)_{q}NR²⁸R³¹; -(CR²⁹R³⁰)_{q}NR²⁸COR³¹; -(CR²⁹R³⁰)_{q}NR²⁸CONR²⁸R³¹; -(CR²⁹R³⁰)_{q}COOR²¹; -(CR²⁹R³⁰)_{q}CONR²⁸R³¹; -(CR²⁹R³⁰)_{q}COR³¹; or -(CR²⁹R³⁰)_{q}R³¹;
Z is O; S or S(=O)₂;
U is -C(=O)-; -NR⁴-C(=O)-; or -C(=O)-C(=O)-.

In a particularly preferred embodiment of this invention, the macrocycles of type Ia/Ib are defined by groups of selected building blocks A, B and C and substituents R¹-R⁵³ as detailed below:
The building block of type **A** is selected from
   **A1**(a1); **A2**(a1); **A3**(a1); **A4**(a1); **A5**(a1); **A6**(a1); **A7**(a1); **A9**(a1)**; A10**(a1); **A73**(a2); **A170**(a4); **A209**(a7); **A240**(a10); **A272**(a10); **A532**(a18); **A614**(a24)**; A631**(a1); **A632**(a1); **A633**(a1); **A641**(a1); **A642**(a1); **A643**(a1)**; A651**(a1)**; A652**(a1); or **A653**(a1).
The building block of type **B** is selected from
   **B7**(b3) ; **B8**(b3) ; **B9**(b3) ; **B10**(b3) ; or **B17**(b5).
The building block of type **C** is selected from
   **C1; C8; C9; C11; C12; C15; C17; C19; C20; C25;** or **C47;**
and wherein
R¹ is H; F; Cl; Br; CF₃; OCF₃; OCHF₂; CN; lower alkyl; lower alkenyl; cycloalkyl; heterocycloalkyl; aryl; heteroaryl; lower arylalkyl; lower heteroarylalkyl; -(CR²⁹R³⁰)_{q}OR²⁰; -(CR²⁹R³⁰)_{q}NR⁴R¹¹; -(CR²⁹R³⁰)_{q}NR⁴COR²²; -(CR²⁹R³⁰)_{q}NR⁴CONR⁴R¹¹; -(CR²⁹R³⁰)_{q}NR⁴SO₂R²³; -(CR²⁹R³⁰)_{q}COOR²¹; -(CR²⁹R³⁰)_{q}CONR⁴R¹¹; -(CR²⁹R³⁰)_{q}SO₂NR⁴R¹¹; -(CR²⁹R³⁰)_{q}COR²²; -(CR²⁹R³⁰)_{q}SO₂R²³; -(CR²⁹R³⁰)_{q}R²⁴; -(CR²⁹R³⁰)_{q}R²⁵; or - (CR²⁹R³⁰)_{q}R²⁶;
R² is H; CF₃; lower alkyl; lower alkenyl; lower cycloalkyl; lower heterocycloalkyl; aryl; heteroaryl; lower arylalkyl; lower heteroarylalkyl; -(CR²⁹R³⁰)_{q}OR²⁰; -(CR²⁹R³⁰)_{q}NR⁴R¹¹; -(CR²⁹R³⁰)_{q}NR⁴COR²²; -(CR²⁹R³⁰)_{q}NR⁴CONR⁴R¹¹; -(CR²⁹R³⁰)_{q}NR⁴SO₂R²³; -(CR²⁹R³⁰)_{q}COOR²¹; -(CR²⁹R³⁰)_{q}CONR⁴R¹¹_{;} -(CR²⁹R³⁰)_{q}SO₂NR⁴R¹¹; -(CR²⁹R³⁰)_{q}COR²²; -(CR²⁹R³⁰)_{q}SO₂R²³; -(CR²⁹R³⁰)_{q}R²⁹; -(CR²⁹R³⁰)_{q}R²⁵; or -(CR²⁹R³⁰)_{q}R²⁶;
R⁴ is H; acetyl; lower alkyl; lower alkenyl; or a suitable *N*-protecting group;
R⁵, R⁷ and R⁹ are independently defined as H; CF₃; lower alkyl; lower alkenyl; lower cycloalkyl; lower heterocycloalkyl; aryl; heteroaryl; lower arylalkyl; lower heteroarylalkyl; -(CR¹⁸R¹⁹)_{q}OR²⁰; -(CR¹⁸R¹⁹)_{q}NR⁴R¹¹; -(CR¹⁸R¹⁹)_{q}NR⁴COR²²; -(CR¹⁸R¹⁹)_{q}NR⁴CONR⁴R¹¹; -(CR¹⁸R¹⁹)_{q}NR⁴SO₂R²³: -(CR¹⁸R¹⁹)_{q}COOR²¹; -(CR¹⁸R¹⁹)_{q}CONR⁴R¹¹; -(CR¹⁸R¹⁹)_{q}SO₂NR⁴R¹¹; -(CR¹⁸R¹⁹)_{q}COR²²; -(CR¹⁸R¹⁹)_{q}SO₂R²³; -(CR¹⁸R¹⁹)_{q}R²⁴; -(CR¹⁸R¹⁹)_{q}R²⁵; or -(CR¹⁸R¹⁹)_{q}R²⁶;
R⁶, R⁸ and R¹⁰ are independently defined as H; CF₃; CH₃; or benzyl;
R¹¹ is H; alkyl; alkenyl; cycloalkyl; lower heterocycloalkyl; aryl; heteroaryl; lower arylalkyl; lower heteroarylalkyl; a suitable protecting group; -(CR¹⁸R¹⁹)ᵣOR²⁰; -(CR¹⁸R¹⁹)ᵣNR⁴R²⁷; -(CR¹⁸R¹⁹)ᵣNR⁴CONR⁴R²⁷; -(CR¹⁸R¹⁹)ᵣNR⁴SO₂R²³; -(CR¹⁸R¹⁹)_{q}COOR²¹; -(CR¹⁸R¹⁹)_{q}CONR⁴R²⁷; -(CR¹⁸R¹⁹)_{q}COR²²; -(CR¹⁸R¹⁹)_{q}R²⁴; -(CR¹⁸R¹⁹)ₛR²⁵; -(CR¹⁸R¹⁹)_{q}R²⁶; or -(CR¹⁸R¹⁹)_{q}R³¹;
R¹² and R¹³ are independently defined as H; or lower alkyl.
R¹⁴ and R¹⁶ are independently defined as H; F; CF₃; lower alkyl; lower alkenyl; lower cycloalkyl; lower heterocycloalkyl; aryl; heteroaryl; lower arylalkyl; lower heteroarylalkyl; -(CR²⁹R³⁰)_{q}OR²⁰; -(CR²⁹R³⁰)_{q}NR⁴R¹¹; -(CR²⁹R³⁰)_{q}NR⁴COR²²; -(CR²⁹R³⁰)_{q}COOR²¹; or -(CR²⁹R³⁰)_{q}CONR⁴R¹¹;
R¹⁵ and R¹⁷ are independently defined as H; CF₃; or CH₃;
R¹⁸ is H; F; CF₃; lower alkyl; lower alkenyl; lower cycloalkyl; lower heterocycloalkyl; aryl; heteroaryl; lower arylalkyl; lower heteroarylalkyl; - (CR²⁹R³⁰)ₛOR³¹; -(CR²⁹R³⁰)ₛNR²⁸R³¹; -(CR²⁹R³⁰)ₛNR²⁸COR³¹; -(CR²⁹R³⁰)ₛNR²⁸CONR²⁸R³¹; -(CR²⁹R³⁰)ₛNR²⁸SO₂R²³; -(CR²⁹R³⁰)_{q}COOR²¹; -(CR²⁹R³⁰)_{q}CONR²⁸R³¹; -(CR²⁹R³⁰)_{q}SO₂NR²⁸R³¹; -(CR²⁹R³⁰)_{q}COR³¹; -(CR²⁹R³⁰)_{q}SO₂R²³; -(CR²⁹R³⁰)_{q}R²⁴ ; -(CR²⁹R³⁰)_{q}R²5; or - (CR²⁹R³⁰)_{q}R²⁶;
R¹⁹ is H; CF₃; or CH₃;
R²⁰ is H; lower alkyl; lower alkenyl; lower cycloalkyl; lower heterocycloalkyl; aryl; heteroaryl; lower arylalkyl; lower heteroarylalkyl; -(CR²⁹R³⁰)ᵣOR³¹; -(CR²⁹R³⁰)ᵣNR²⁸R³¹; - (CR²⁹R³⁰) ᵣNR²⁸COR³¹; - (CR²⁹R³⁰)ᵣNR²⁸CONR²⁸R³¹; -(CR²⁹R³⁰)ᵣNR²⁸SO₂R²³; - (CR²⁹R³⁰)_{q}COOR²¹; -(CR²⁹R³⁰)_{q}CONR²⁸R³¹; -(CR²⁹R³⁰)_{q}SO₂NR²⁸R³¹; -(CR²⁹R³⁰)_{q}COR³¹; -(CR²⁹R³⁰)_{q}SO₂R²³; -(CR²⁹R³⁰)_{q}R²⁴; -(CR²⁹R³⁰)_{q}R²⁵; -(CR²⁹R³⁰)_{q}R²⁶; or -(CR²⁹R³⁰)_{q}R³¹;
R²² is lower alkyl; lower alkenyl; lower cycloalkyl; lower heterocycloalkyl; aryl; heteroaryl; lower arylalkyl; lower heteroarylalkyl; -(CR²⁹R³⁰)ₛOR³¹; -(CR²⁹R³⁰)ₛNR²⁸R³¹; -(CR²⁹R³⁰)ₛNR²⁸COR³¹; -(CR²⁹R³⁰)ₛNR²⁸CONR²⁸R³¹; -(CR²⁹R³⁰)ₛNR²⁸SO₂R²³; -(CR²⁹R³⁰)ₛCOOR²¹; -(CR²⁹R³⁰)ₛCONR²⁸R³¹; -(CR²⁹R³⁰)ₛSO₂NR²⁸R³¹; -(CR²⁹R³⁰)ₜCOR³¹; -(CR²⁹R³⁰)ₛSO₂R²³; -(CR²⁹R³⁰)ₜR²⁴; -(CR²⁹R³⁰)ₜR²⁵; -(CR²⁹R³⁰)ₜR²⁶; or -(CR²⁹R³⁰)ₜR31;
R²⁴ is aryl; heteroaryl; or -C₆H₂R³⁴R³⁵R³¹;
R²⁵ and R²⁶ are independently defined as heterocycloalkyl;
R²⁷ is H; lower alkyl; lower alkenyl; lower cycloalkyl; lower heterocycloalkyl; aryl; heteroaryl; lower arylalkyl; lower heteroarylalkyl; a suitable protecting group; or -(CR²⁹R³⁰)_{q}R²⁴;
R²⁸ is H; (CR³²R³³)ₛOR²¹; or -(CR³²R³³)ₛNR⁴³R⁴²;
R²⁹ is H; F; CF₃; lower alkyl; lower alkenyl; lower cycloalkyl; lower heterocycloalkyl; aryl; heteroaryl; lower arylalkyl; lower heteroarylalkyl; -(CR³²R³³)ₛOR³¹; -(CR³²R³³)ₛNR²⁸R³¹; -(CR³²R³³)ₛNR²⁸COR³¹; -(CR³²R³³)ₛNR²⁸CONR²⁸R³¹; -(CR³²R³³)_{q}COOR²¹; -(CR³²R³³)_{q}CONR²⁸R³¹; -(CR³²R³³)_{q}COR³¹; or -(CR³²R³³)_{q}R³¹;
R³⁰ and R³³ are independently defined as H; F; CF₃; or CH₃;
R³¹ is H; alkyl; alkenyl; cycloalkyl; heterocycloalkyl; aryl; heteroaryl; arylalkyl; or heteroarylalkyl;
R³² is H; F; CF₃; lower alkyl; lower alkenyl; lower cycloalkyl; lower heterocycloalkyl; aryl; heteroaryl; lower arylalkyl; or lower heteroarylalkyl;
R³⁴ and R³⁵ are independently defined as H; F; Cl; CF₃; lower alkyl; lower alkenyl; lower cycloalkyl; lower heterocycloalkyl; aryl; heteroaryl; lower arylalkyl; lower heteroarylalkyl; -(CR²⁹R³⁰)_{q}OR³¹; -(CR²⁹R³⁰)_{q}NR²⁸R³¹; - (CR²⁹R³⁰)_{q}NR²⁸COR³¹; -(CR²⁹R³⁰)_{q}NR²⁸CONR²⁸R³¹; -(CR²⁹R³⁰)_{q}NR²⁸SO₂R²³; -(CR²⁹R³⁰)_{q}COOR²¹; -(CR²⁹R³⁰)_{q}CONR²⁸R³¹; -(CR²⁹R³⁰)_{q}SO₂NR²⁸R³¹; -(CR²⁹R³⁰)_{q}COR³¹; -(CR²⁹R³⁰)_{q}SO₂R²³; or -(CR²⁹R³⁰)_{q}R³¹;
R³⁸ is H; lower alkyl; lower alkenyl; lower cycloalkyl; lower heterocycloalkyl; aryl; heteroaryl; lower arylalkyl; lower heteroarylalkyl; -(CR²⁹R³⁰)_{q}OR³¹; -(CR²⁹R³⁰)_{q}NR²⁸R³¹; -(CR²⁹R³⁰)_{q}NR²⁸COR³¹; -(CR²⁹R³⁰)_{q}NR²⁸CONR²⁸R³¹; -(CR²⁹R³⁰)_{q}COOR²¹; -(CR²⁹R³⁰)_{q}CONR²⁸R³¹; -(CR²⁹R³⁰)_{q}COR³¹; or -(CR²⁹R³⁰)_{q}R³¹;
R⁴² and R⁴³ are independently defined as H; lower alkyl; lower alkenyl; lower cycloalkyl; lower heterocycloalkyl; aryl; heteroaryl; lower arylalkyl; lower heteroarylalkyl; or a suitable *N*-protecting group;
X is O; S; S(=O); or S(=O)₂;
Y is O; S; or NR⁴;
Z is O; S or S(=O)₂;
U is -C(=O)-; -NR⁴-C(=O)-; or -C(=O)-C(=O)-;

This particularly preferred embodiment includes all possible stereoisomers (explicitly including atropisomers), conformers and rotamers as well as pharmaceutically acceptable salts, solvates, clathrates, isotopically enriched or enantiomerically enriched versions of above macrocyclic compounds.

In a further particularly preferred embodiment of this invention, the macrocycles of type **Ia/Ib** are defined by groups of selected building blocks **A, B** and **C** and substituents R¹-R⁵³ as detailed below:
The building block of type **A** is selected from
   **A2**(a1); **A5**(a2); **A9**(a1); **A73**(a2); **A209**(a7); **A272**(a10); **A532**(a18)**; A614**(a24); **A641**(a1); or **A651**(a1)**;**
the building block of type **B** is selected from
   **B7; B9; B10** or **B17;**
the building block of type **C** is selected from
   **C1; C8; C9; C11; C15; C17; C19; C20; C25;** or **C47;**
and wherein
R¹ is H; F; Cl; Br; CH₃; OCH₃; OH; or aryl;
R² is H; CH₃; heterocycloalkyl; aryl; benzyl; heteroaryl; -OR²⁰; -NR⁴R¹¹; -NR⁴COOR²¹; -NR⁴COR²²; -NR⁴CONR⁴R¹¹; or -NR⁴SO₂R²³;
R³ is H;
R⁴ is H; acetyl; lower alkyl; lower alkenyl; or a suitable N-protecting group;
R⁵, R⁷ and R⁹ are independently defined as H; CF₃; lower alkyl; lower alkenyl; lower cycloalkyl; lower heterocycloalkyl; aryl; heteroaryl; lower arylalkyl; lower heteroarylalkyl; -OR²⁰; -NR⁴R¹¹; -NR⁴COR²²; -NR⁹CONR⁴R¹¹; -NR⁴SO₂R²³; -COOR²¹;-CONR⁴R¹¹; or -COR²²;
R⁶, R⁸ and R¹⁰ are defined as H;
R¹¹ is H; alkyl; aryl; heteroaryl; lower arylalkyl; lower heteroarylalkyl; -(CR¹⁸R¹⁹)ᵣOR²⁰; -(CR¹⁸R¹⁹)ᵣNR⁹R²⁷; -(CR¹⁸R¹⁹)_{q}COOR²¹; -COR²²; - (CR¹⁸R¹⁹)_{q}R²⁴; or -(CR¹⁸R¹⁹)_{q}R³¹;
R¹² and R¹³ are defined as H;
R¹⁴ and R¹⁶ are independently defined as H; lower alkyl; or lower arylalkyl;
R¹⁵ and R¹⁷ are defined as H;
R¹⁸ and R¹⁹ are defined as H;
R²⁰ is H; aryl; heteroaryl; lower arylalkyl; lower heteroarylalkyl; or - (CR²⁹R³⁰)_{q}R²⁴;
R²¹ is H; lower alkyl; lower arylalkyl; or a suitable *O-*protecting group;
R²² is lower alkyl; lower alkenyl; aryl; heteroaryl; lower arylalkyl; lower heteroarylalkyl; -(CR²⁹R³⁰)ₛOR³¹; -(CR²⁹R³⁰)ₛNR²⁸R³¹; -(CR²⁹R³⁰)ₛCOOR²¹; -(CR²⁹R³⁰)ₜR²⁴; or - (CR²⁹R³⁰)ₜR³¹;
R²³ is H; lower alkyl; aryl; heteroaryl; or -R²⁴;
R²⁴ is aryl; heteroaryl; or -C₆H₂R³⁴R³⁵R³¹;
R²⁷ is H; acetyl; CH₃; or -(CH₂)_{q}R²⁴;
R²⁸ is H; -(CR³²R³³)ₛOR²¹; or -(CR³²R³³)ₛNR⁴³R⁴²;
R²⁹ is H; lower alkyl; aryl; or heteroaryl;
R³⁰, R³² and R³³ are independently defined as H; CF₃; or CH₃;
R³¹ is H; alkyl; cycloalkyl; heterocycloalkyl; aryl; heteroaryl; arylalkyl; or heteroarylalkyl;
R³⁴ and R³⁵ are independently defined as H; heterocycloalkyl; -OR³¹; or -NR²⁸R³¹;
R³⁸ is H; -NR²⁸R³¹; -NR²⁸COR³¹; or -NR²⁸COOR³¹;
R⁴² and R⁴³ are independently defined as H; CH₃; or benzyl;
X is O; S; S(=O); or S(=O)₂;
Y is O; S; or NR⁴;
Z is O; S or S(=O)₂;
U is -C(=O)-; -NR⁴-C(=O)-; or -C(=O)-C(=O)-.

This further particularly preferred embodiment includes all possible stereoisomers (explicitly including atropisomers), conformers and rotamers as well as pharmaceutically acceptable salts, solvates, clathrates, isotopically enriched or enantiomerically enriched versions of above macrocyclic compounds.

In another preferred embodiment of this invention, the macrocycles of formulae **Ia** and **Ib** as described in the main embodiment can contain as a "Linker" the building block **C** which is represented by the formula

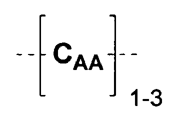

Examples of readily accessible amino acids defining possible subunits **C_{AA}** of the linker **C** are listed in Table 10 below. Only one stereoisomer of enantiomeric amino acids is cited, usually the L-enantiomer; it is understood that the complementary enantiomer is also part to the embodiment. Also not mentioned explicitly but naturally part of the embodiment are the simple N-Methyl derivatives of the listed amino acids.

**Table 10: Substances Representing Subunits of "Linkers" C (continued on the following pages)**

| **Code** | **Chemical Name** |
|---|---|
| Ala | L-Alanine |
| Arg | L-Arginine |
| Asn | L-Asparagine |
| Asp | L-Aspartic acid |
| Cys | L-Cysteine |
| Glu | L-Glutamic acid |
| Gln | L-Glutamine |
| Gly | Glycine |
| His | L-Histidine |
| Ile | L-Isoleucine |
| Leu | L-Leucine |
| Lys | L-Lysine |
| Met | L-Methionine |
| Phe | L-Phenylalanine |
| Pro | L-Proline |
| Ser | L-Serine |
| Thr | L-Threonine |
| Trp | L-Tryptophan |
| Tyr | L-Tyrosine |
| Val | L-Valine |
| Apa | 3-Amino-propanoic acid |
| H-β-HAla-OH | (3S)-3-Amino-butyric acid |
| H-β³-HVal-OH | (3R)-3-Amino-4-methyl-valeric acid |
| H-β³-HIle-OH | (3R, 4S)-3-Amino-4-methyl-hexanoic acid |
| H-β³-HLeu-OH | (3S)-3-Amino-5-methyl-hexanoic acid |
| H-β³-HMet-OH | (3S)-3-Amino-5-methylthio pentanoic acid |
| H-β³-HTyr-OH | (3S)-3-Amino-4-(4'-hydroxyphenyl)-butyric acid |
| H-β³-HHis-OH | (3S)-3-Amino-4-(imidazole-4'-yl)-butyric acid |
| H-β³-HPhe-OH | (3S)-3-Amino-4-phenyl butyric acid |
| H-β³-HTrp-OH | (3S)-3-Amino-4-(indol-3'-yl)-butyric acid |
| H-β³-HSer-OH | (3R)-3-Amino-4-hydroxy-butyric acid |
| H-β³-HAsp-OH | 3-Amino-pentanedioic acid |
| H-β³-HGlU-OH | (3S)-3-Amino-hexanedioic acid |
| H-β³-HLys-OH | (3S)-3,7-Diamino-heptanoic acid |
| H-β³-HArg-OH | (3S)-3-Amino-6-guanidino-hexanoic-acid |
| H-β³-HCys-OH | (3R)-3-Amino-4-mercapto-butyric acid |
| H-β³-HAsn-OH | (3S)-3-Amino-4-carbamoyl-butyric acid |
| H-β³-HGln-OH | (3S)-3-Amino-5-carbamoyl-pentanoic acid |
| H-β³-HThr-OH | (3R,4R)-3-Amino-4-hydroxy-pentanoic acid |
| Gaba | 4-Amino-butyric acid |
| H-γ⁴-DiHAla-OH | (4S)-4-Amino-pentanoic acid |
| H-γ⁴-DiHVal-OH | (4R)-4-Amino-5-methyl-hexanoic acid |
| H-γ⁴-DiHIle-OH | (4R, 5S)-4-Amino-5-methyl-heptanoic acid |
| H-γ⁴-DiHLeu-OH | (4R)-4-Amino-6-methyl-heptanoic acid |
| H-γ⁴-DiHMet-OH | (4R)-4-Amino-6-methylthio-hexanoic acid |
| H-γ⁴-DiHTyr-OH | (4R)-4-Amino-5-(4'-hydroxyphenyl)-pentanoic acid |
| H-γ⁴-DiHHis-OH | (4R)-4-Amino-5-(imidazole-4'-yl)-pentanoic acid |
| H-γ⁴-DiHPhe-OH | (4R)-4-Amino-5-phenyl-pentanoic acid |
| H-γ⁴-DiHTrp-OH | (4R)-4-Amino-5-(indol-3'-yl)-pentanoic acid |
| H-γ⁴-DiHSer-OH | (4R)-4-Amino-5-hydroxy-pentanoic acid |
| H-γ⁴-DiHAsp-OH | (4R)-4-Amino-hexanedioic acid |
| H-γ⁴-DiHGlu-OH | 4-Amino-heptanedioic acid |
| H-γ⁴-DiHLys-OH | (4S)-4,8-Diamino-octanoic acid |
| H-γ⁴-DiHArg-OH | (4S)-4-Amino-7-guanidino-heptanoic-acid |
| H-γ⁴-DiHCys-OH | (4R)-4-Amino-5-mercapto-pentanoic acid |
| H-γ⁴-DiHAsn-OH | (4R)-4-Amino-5-carbamoyl-pentanoic acid |
| H-γ⁴-DiHGln-OH | (3S)-3-Amino-5-carbamoyl-hexanoic acid |
| H-γ⁴-DiHThr-OH | (4R, 5R)-4-Amino-5-hydroxy-hexanoic acid |
| Cit | L-Citrulline |
| Orn | L-Ornithine |
| tBuA | L-t-Butylalanine |
| Sar | Sarcosine |
| Pen | L-Penicillamine |
| tBuG | L-tert.-Butylglycine |
| 4AmPhe | L-para-Aminophenylalanine |
| 3AmPhe | L-meta-Aminophenylalanine |
| 2AmPhe | L-ortho-Aminophenylalanine |
| Phe (mC (NH₂) =NH) | L-meta-Amidinophenylalanine |
| Phe (pC (NH₂) =NH) | L-para-Amidinophenylalanine |
| Phe (mNHC (NH₂) =NH) | L-meta-Guanidinophenylalanine |
| Phe (pNHC (NH₂) =NH) | L-para-Guanidinophenylalanine |
| 2Pal | (2S)-2-Amino-3-(pyridine-2'-yl)-propionic acid |
| 4Pal | (2S)-2-Amino-3-(pyridine-4'-yl)-propionic acid |
| Phg | L-Phenylglycine |
| Cha | L-Cyclohexylalanine |
| C₄al | L-3-Cyclobutylalanine |
| C₅al | L-3-Cyclopentylalanine |
| Nle | L-Norleucine |
| 2-Nal | L-2-Naphthylalanine |
| 1-Nal | L-1-Naphthylalanine |
| 4ClPhe | L-4-Chlorophenylalanine |
| 3ClPhe | L-3-Chlorophenylalanine |
| 2ClPhe | L-2-Chlorophenylalanine |
| 3,4Cl₂Phe | L-3,4-Dichlorophenylalanine |
| 4FPhe | L-4-Fluorophenylalanine |
| 3FPhe | L-3-Fluorophenylalanine |
| 2 FPhe | L-2-Fluorophenylalanine |
| Thi | L-β-2-Thienylalanine |
| Tza | L-2-Thiazolylalanine |
| Mso | L-Methionine sulfoxide |
| AcLys | N-Acetyllysine |
| Dap | 2,3-Diaminopropionic acid |
| Dab | 2,4-Diaminobutyric acid |
| Dbu | (2S)-2,3-Diamino-butyric acid |
| Abu | γ-Aminobutyric acid (GABA) |
| Aha | ε-Aminohexanoic acid |
| Aib | α-Aminoisobutyric acid |
| ACC | 1-Amino cyclopropane carboxylic acid |
| ACBC | 1-Amino cyclobutane carboxylic acid |
| ACPC | 1-Amino cyclopentane carboxylic acid |
| ACHC | 1-Amino cyclohexane carboxylic acid |
| Y (Bzl) | L-O-Benzyltyrosine |
| H (Bzl) | (3S)-2-Amino-3-(1'-benzylimidazole-4'-yl)-propionic acid |
| Bip | L-(4-phenyl)phenylalanine |
| S (Bzl) | L-O-Benzylserine |
| T (Bzl) | L-O-Benzylthreonine |
| alloT | (2S, 3S)-2-Amino-3-hydroxy-butyric acid |
| Leu3OH | (2S, 3R)-2-Amino-3-hydroxy-4-methyl-pentanoic acid |
| hAla | L-Homo-alanine |
| hArg | L-Homo-arginine |
| hCys | L-Homo-cysteine |
| hGlu | L-Homo-glutamic acid |
| hGln | L-Homo-glutamine |
| hHis | L-Homo-histidine |
| hIle | L-Homo-isoleucine |
| hLeu | L-Homo-leucine |
| hNle | L-Homo-norleucine |
| hLys | L-Homo-lysine |
| hMet | L-Homo-Methionine |
| hPhe | L-Homo-phenylalanine |
| hSer | L-Homo-serine |
| hThr | L-Homo-threonine |
| hTrp | L-Homo-tryptophan |
| hTyr | L-Homo-tyrosine |
| hVal | L-Homo-valine |
| hCha | L-Homo-cyclohexylalanine |
| Bpa | L-4-Benzoylphenylalanine |
| OctG | L-Octylglycine |
| Tic | (3S)-1,2,3,4-Tetrahydroisoquinoline-3-carboxylic acid |
| Tiq | (1S)-1,2,3,4-Tetrahydroisoquinoline-1-carboxylic acid |
| Oic | (2S, 3aS, 7aS)-1-Octahydro-1H-indole-2-carboxylic acid |
| 4AmPyrr1 | (2S, 4S)-4-Amino-pyrrolidine-2-carboxylic acid |
| 4AmPyrr2 | (2S, 4R)-4-Amino-pyrrolidine-2-carboxylic acid |
| 4PhePyrr1 | (2S, 4R)-4-Phenyl-pyrrolidine-2-carboxylic acid |
| 4PhePyrr2 | (2S, 4S)-4-Phenyl-pyrrolidine-2-carboxylic acid |
| 5PhePyrr1 | (2S, 5R)-5-Phenyl-pyrrolidine-2-carboxylic acid |
| 5PhePyrr2 | (2S, 5S)-5-Phenyl-pyrrolidine-2-carboxylic acid |
| 4Hypl | (4S)-L-Hydroxyproline |
| 4Hyp2 | (4R)-L-Hydroxyproline |
| 4Mpl | (4S)-L-Mercaptoproline |
| 4Mp2 | (4R)-L-Mercaptoproline |
| Pip | L-Pipecolic acid |
| H-β³-HCit-OH | (3S)-3-Amino-6-carbamidyl-hexanoic acid |
| H-β³-HOrn-OH | (3S)-3,6-Diamino-hexanoic acid |
| H-β³-HtBuA-OH | (3S)-3-Amino-5,5-dimethyl-hexanoic acid |
| H-β³-HSar-OH | N-Methyl-3-amino-propionic acid |
| H-β³-HPen-OH | (3R)-3-Amino-4-methyl-4-mercapto-pentanoic acid |
| H-β³-HtBuG-OH | (3R)-3-Amino-4,4-dimethyl-pentanoic acid |
| H-β³-H4AmPhe-OH | (3S)-3-Amino-4-(4'-aminophenyl)-butyric acid |
| H-β³-H3AmPhe-OH | (3S)-3-Amino-4-(3'-aminophenyl)-butyric acid |
| H-β³-H2AmPhe-OH | (3S)-3-Amino-4-(2'-aminophenyl)-butyric acid |
| H-β³-HPhe(mC(NH₂)=NH)-OH | (3S)-3-Amino-4-(3'-amidinophenyl)-butyric acid |
| H-β³-HPhe (pC (NH₂) =NH) -OH | (3S)-3-Amino-4-(4'-amidinophenyl)-butyric acid |
| H-β³-HPhe(mNHC(NH₂)=NH)-OH | (3S)-3-Amino-4-(3'-guanidinophenyl)-butyric acid |
| H-β³-HPhe(pNHC(NH₂)=NH)-OH | (3S)-3-Amino-4-(4'-guanidino-phenyl)-butyric acid |
| H-β³-H2Pal-OH | (3S)-3-Amino-4-(pyridine-2'-yl)-butyric acid |
| H-β³-H4Pal-OH | (3S)-3-Amino-4-(pyridine-4'-yl)-butyric acid |
| H-β³-HPhg-OH | (3R)-3-Amino-3-phenyl-propionic acid |
| H-β³-HCha-OH | (3S)-3-Amino-4-cyclohexyl-butyric acid |
| H-β³-HC₄al-OH | (3S)-3-Amino-4-cyclobutyl-butyric acid |
| H-β³-HC₅al-OH | (3S)-3-Amino-4-cyclopentyl-butyric acid |
| H-β³-HNle-OH | (3S)-3-Amino-heptanoic acid |
| H-β³-H2Nal-OH | (3S)-3-Amino-4-(2'-naphthyl)-butyric acid |
| H-β³-H1Nal-OH | (3S)-3-Amino-4-(1'-naphthyl)-butyric acid |
| H-β³-H4ClPhe-OH | (3S)-3-Amino-4-(4'-chlorophenyl)-butyric acid |
| H-β³-H3ClPhe-OH | (3S)-3-Amino-4-(3'-chlorophenyl)-butyric acid |
| H-β³-H2ClPhe-OH | (3S)-3-Amino-4-(2'-chlorophenyl)-butyric acid |
| H-β³-H3, 4Cl₂Phe-OH | (3S)-3-Amino-4-(3',4'-dichlorophenyl)-butyric acid |
| H-β³-H4FPhe-OH | (3S)-3-Amino-4-(4'-fluorophenyl)-butyric acid |
| H-β³-H3FPhe-OH | (3S)-3-Amino-4-(3'-fluorophenyl)-butyric acid |
| H-β³-H2FPhe-OH | (3S)-3-Amino-4-(2'-fluorophenyl)-butyric acid |
| H-β³-HThi-OH | (3R)-3-Amino-4-(2'-thienyl)-butyric acid |
| H-β³-HTza-OH | (3R)-3-Amino-4-(2'-thiazolyl)-butyric acid |
| H-β³-HMso-OH | (3R)-3-Amino-4-methylsulfoxyl-butyric acid |
| H-β³-HAcLys-OH | (3S)-7-Acetylamino-3-amino-heptanoic acid |
| H-β³-HDpr-OH | (3R)-3,4-diamino-butyric acid |
| H-β³-HA₂Bu-OH | (3S)-3,5-Diamino-pentanoic acid |
| H-β³-HDbu-OH | (3R)-3,4-Diamino-pentanoic acid |
| H-β³-HAib-OH | Amino-dimethyl acetic acid |
| H-β³-HCyp-OH | 1-Amino-cyclopentane-1-yl-acetic acid |
| H-β³-HY(Bzl)-OH | (3S)-3-Amino-4-(4'-benzyloxyphenyl)-butyric acid |
| H-β³-HH(Bzl)-OH | (3S)-3-Amino-4-(1'-benzylimidazole-4'-yl)-butyric acid |
| H-β³-HBip-OH | (3S)-3-Amino-4-biphenylyl-butyric acid |
| H-β³-HS(Bzl)-OH | (3S)-3-Amino-4-(benzyloxy)-butyric acid |
| H-β³-HT(Bzl)-OH | (3R, 4R)-3-Amino-4-benzyloxy-pentanoic acid |
| H-β³-HalloT-OH | (3R, 4S)-3-Amino-4-hydroxy-pentanoic acid |
| H-β³-HLeu3OH-OH | (3R, 4R)-3-Amino-4-hydroxy-5-methyl-hexanoic acid |
| H-β³-HhAla-OH | (3S)-3-Amino-pentanoic acid |
| H-β³-HhArg-OH | (3S)-3-Amino-7-guanidino-heptanoic acid |
| H-β³-HhCys-OH | (3R)-Amino-5-mercapto-pentanoic acid |
| H-β³-HhGlu-OH | (3S)-3-Amino-heptanedioic acid |
| H-β³-HhGln-OH | (3S)-3-Amino-6-carbamoyl hexanoic acid |
| H-β³-HhHis-OH | (3S)-3-Amino-5-(imidazole-4'-yl)-pentanoic acid |
| H-β³-HhIle-OH | (3S, 5S)-3-Amino-5-methyl-heptanoic acid |
| H-β³-HhLeu-OH | (3S)-3-Amino-6-methyl-heptanoic acid |
| H-β³-HhNle-OH | (3S)-3-Amino-octanoic acid |
| H-β³-DiAoc-OH | (3S)-3,8-Diamino-octanoic acid |
| H-β³-HhMet-OH | (3S)-3-Amino-6-methylthio-hexanoic acid |
| H-β³-HhPe-OH | (3S)-3-Amino-5-phenyl-pentanoic acid |
| H-β³-HhSer-OH | (3S)-3-Amino-5-hydroxy-pentanoic acid |
| H-β³-HhThr-OH | (3S, 5R)-3-Amino-5-hydroxy-hexanoic acid |
| H-β³-HhTrp-OH | (3S)-3-Amino-5-(indol-3'-yl)-pentanoic acid |
| H-β³-HhThr-OH | (3S)-3-Amino-5-(4'-hydroxyphenyl)-pentanoic acid |
| H-β³-HhCha-OH | (3S)-3-Amino-5-cyclohexyl-pentanoic acid |
| H-β³-HBpa-OH | (3S)-3-Amino-4-(4'-benzoylphenyl)-butyric acid |
| H-β³-HOctG-OH | (3S)-3-Amino-undecanoic acid |
| H-β³-HNle-OH | (3S)-3-Amino-heptanoic acid |
| H-β³-HTic-OH | (3S)-1,2,3,4-Tetrahydroisoquinoline-3-yl-acetic acid |
| H-β³-HTiq-OH | (1S)-1,2,3,4-Tetrahydroisoquinoline-1-acetic acid |
| H-β³-HOic-OH | (2S, 3aS, 7aS)-1-Octahydro-1H-indole-2-yl-acetic acid |
| H-β³-H4AmPyrr1-OH | (2S, 4S)-4-Amino-pyrrolidine-2-acetic acid |
| H-β³-H4AmPyrr2-OH | (2S, 4R)-4-Amino-pyrrolidine-2-acetic acid |
| H-β³-H4PhePyrr1-OH | (2S, 4R)-4-Phenyl-pyrrolidine-2-acetic acid |
| H-β³-H4PhePyrr2-OH | (2S, 4S)-4-Phenyl-pyrrolidine-2-acetic acid |
| H-β³-H5PhePyrr1-OH | (2S, 5R)-5-Phenyl-pyrrolidine-2-acetic acid |
| H-β³-H5PhePyrr2-OH | (2S, 5S)-5-Phenyl-pyrrolidine-2-acetic acid |
| H-β³-H4Hypl-OH | (2S, 4S)-4-Hydroxy-pyrrolidine-2-acetic acid |
| H-β³-H4Hyp2-OH | (2S, 4R)-4-Hydroxy-pyrrolidine-2-acetic acid |
| H-β³-H4Mp1-OH | (2R, 4S)-4-Mercapto-pyrrolidine-2-acetic acid |
| H-β³-H4Mp2-OH | (2R, 4R)-4-Mercapto-pyrrolidine-2-acetic acid |
| H-β³-HPip-OH | (2S)-Piperidine-2-acetic acid |
| H-β³-HPro-OH | (2S)-Pyrrolidine-2-acetic acid |
| Ahb | 4-Amino-2-hydroxy butyric acid |
| H-γ⁴-DiHCit-OH | (4S)-4-Amino-7-carbamidyl-heptanoic acid |
| H-γ⁴-DiHOrn-OH | (4S)-4,7-Diamino-heptanoic acid |
| H-γ⁴-DiHtBuA-OH | (4R)-4-Amino-6,6-dimethyl-heptanoic acid |
| H-γ⁴-DiHSar-OH | N-Methyl-4-amino-butyric acid |
| H-γ⁴-DiHPen-OH | (4R)-4-Amino-5-methyl-5-mercapto-hexanoic acid |
| H-γ⁴-DiHtBuG-OH | (4R)-4-Amino-5,5-dimethyl-hexanoic acid |
| H-γ⁴-DiH4AmPhe-OH | (4R)-4-Amino-5-(4'-aminophenyl)-pentanoic acid |
| H-γ⁴-DiH3AmPhe-OH | (4R)-4-Amino-5-(3'-aminophenyl)-pentanoic acid |
| H-γ⁴-DiH2AmPhe-OH | (4R)-4-Amino-5-(2'-aminophenyl)-pentanoic acid |
| H-γ⁴-DiHPhe (mC (NH₂) =NH) -OH | (4R)-4-Amino-5-(3'-amidinophenyl)-pentanoic acid |
| H-γ⁴-DiHPhe(pC(NH₂)=NH)-OH | (4R)-4-Amino-5-(4'-amidinophenyl)-pentanoic acid |
| H-γ⁴-DiHPhe(mNHC(NH₂)=NH)-OH | (4R)-4-Amino-5-(3'-guanidino-phenyl)-pentanoic acid |
| H-γ⁴-DiHPhe(pNHC(NH₂)=NH)-OH | (4R)-4-Amino-5-(4'-guanidino-phenyl)-pentanoic acid |
| H-γ⁴-DiH2Pal-OH | (4R)-4-Amino-5-(pyridine-4'-yl)-pentanoic acid |
| H-γ⁴-DiH4Pal-OH | (4R)-4-Amino-5-(pyridine-4'-yl)-pentanoic acid |
| H-γ⁴-DiHPhg-OH | (4R)-4-Amino-4-phenyl-butyric acid |
| H-γ⁴-DiHCha-OH | (4R)-4-Amino-5-cyclohexyl-pentanoic acid |
| H-γ⁴-DiHC₄al-OH | (4R)-4-Amino-5-cyclobutyl-pentanoic acid |
| H-γ⁴-DiHC₅al-OH | (4R)-4-Amino-5-cyclopentyl-pentanoic acid |
| H-γ⁴-DiHNle-OH | (4S)-4-Amino-octanoic acid |
| H-γ⁴-DiH2Nal-OH | (4S)-4-Amino-5-(2'-naphthyl)-pentanoic acid |
| H-γ⁴-DiH1Nal-OH | (4S)-4-Amino-5-(1'-naphthyl)-pentanoic acid |
| H-γ⁴-DiH4ClPhe-OH | (4R)-4-Amino-5-(4'-chlorophenyl)-pentanoic acid |
| H-γ⁴-DiH3ClPhe-OH | (4R)-4-Amino-5-(3'-chlorophenyl)-pentanoic acid |
| H-γ⁴-DiH2ClPhe-OH | (4R)-4-Amino-5-(2'-chlorophenyl)-pentanoic acid |
| H-γ⁴-DiH3, 4Cl₂Phe-OH | (4R)-4-Amino-5-(3',4'-dichloro-phenyl)-pentanoic acid |
| H-γ⁴-DiH4FPhe-OH | (4R)-4-Amino-5-(4'-fluorophenyl)-pentanoic acid |
| H-γ⁴-DiH3FPhe-OH | (4R)-4-Amino-5-(3'-fluorophenyl)-pentanoic acid |
| H-γ⁴-DiH2FPhe-OH | (4R)-4-Amino-5-(2'-fluorophenyl)-pentanoic acid |
| H-γ⁴-DiHThi-OH | (4R)-4-Amino-5-(2'-thienyl)-pentanoic acid |
| H-γ⁴-DiHTza-OH | (4R)-4-Amino-5-(2'-thiazolyl)-pentanoic acid |
| H-γ⁴-DiHMso-OH | (4R)-4-Amino-5-methylsulfoxyl-pentanoic acid |
| H-γ⁴-DiHAcLys-OH | (4S)-8-Acetylamino-4-amino-ocatanoic acid |
| H-γ⁴-DiHDpr-OH | (4R)-4,5-diamino-pentanoic acid |
| H-γ⁴-DiHA₂Bu-OH | (4R)-4,5-Diamino-hexanoic acid |
| H-γ⁴-DiHDbu-OH | (4R)-4,5-Diamion-hexanoic acid |
| H-γ⁴-DiHAib-OH | 3-Amino-3,3-dimethyl propionic acid |
| H-γ⁴-DiHCyp-OH | (1'-Amino-cyclopentane-1'-yl)-3-propionic acid |
| H-γ⁴-DiHY(Bzl)-OH | (4R)-4-Amino-5-(4'-benzyloxyphenyl)-pentanoic acid |
| H-γ⁴-DiHH(Bzl)-OH | (4R)-4-Amino-5-(1'-benzylimidazole-4'-yl)-pentanoic acid |
| H-γ⁴-DiHBip-OH | (4R)-4-Amino-5-biphenylyl-pentanoic acid |
| H-γ⁴-DiHS(Bzl)-OH | (4S)-4-Amino-5-(benzyloxy)-pentanoic acid |
| H-γ⁴-DiHT(Bzl)-OH | (4R, 5R)-4-Amino-5-benzyloxy-hexanoic acid |
| H-γ⁴-DiHalloT-OH | (4R, 5S)-4-Amino-5-hydroxy-hexanoic acid |
| H-γ⁴-DiHLeu3OH-OH | (4R, 5R)-4-Amino-5-hydroxy-6-methyl-heptanoic acid |
| H-γ⁴-DiHhAla-OH | (4S)-4-Amino-hexanoic acid |
| H-γ⁴-DiHhArg-OH | (4S)-4-Amino-8-guanidino-octanoic acid |
| H-γ⁴-DiHhCys-OH | (4R)-Amino-6-mercapto-hexanoic acid |
| H-γ⁴-DiHhGlu-OH | (4S)-4-Amino-ocatanedioic acid |
| H-γ⁴-DiHhGln-OH | (4S)-4-Amino-7-carbamoyl-heptanoic acid |
| H-γ⁴-DiHhHis-OH | (4S)-4-Amino-6-(imidazole-4'-yl)-hexanoic acid |
| H-γ⁴-DiHhIle-OH | (4S, 6S)-4-Amino-6-methyl-octanoic acid |
| H-γ⁴-DiHhLeu-OH | (4S)-4-Amino-7-methyl-ocatanoic acid |
| H-γ⁴-DiHhNle-OH | (4S)-4-Amino-nonanoic acid |
| H-γ⁴-DiHhLys-OH | (4S)-4,9-Diamino-nonanoic acid |
| H-γ⁴-DiHhMet-OH | (4R)-4-Amino-7-methylthioheptanoic acid |
| H-γ⁴-DiHhPhe-OH | (4S)-4-Amino-6-phenyl-hexanoic acid |
| H-γ⁴-DiHhSer-OH | (4R)-4-Amino-6-hydroxy-hexanoic acid |
| H-γ⁴-DiHhThr-OH | (4R, 6R)-4-Amino-6-hydroxy-heptanoic acid |
| H-γ⁴-DiHhTrp-OH | (4S)-4-Amino-6-(indol-3'-yl)-hexanoi cacid |
| H-γ⁴-DiHhTyr-OH | (4S)-4-Amino-6-(4'-hydroxyphenyl)-hexanoic acid |
| H-γ⁴-DiHhCha-OH | (4R)-4-Amino-5-cyclohexyl-pentanoic acid |
| H-γ⁴-DihBpa-OH | (4R)-4-Amino-5-(4'-benzoylphenyl)-pentanoic acid |
| H-γ⁴-DiHOctG-OH | (4S)-4-Amino-dodecanoic acid |
| H-γ⁴-DiHNle-OH | (4S)-4-Amino-octanoic acid |
| H-γ⁴-DiHTic-OH | (3R)-1',2',3',4'-Tetrahydroisoquinoline-3'-yl-3-propionic acid |
| H-γ⁴-DiHTiq-OH | (1'R)-1',2',3',4'-Tetrahydroisoquinoline-1'-yl-3-propionic acid |
| H-γ⁴-DiHOic-OH | (2'S, 3'aS, 7'aS)-1'-Octahydro-1H-indole-2'-yl-3-propionic acid |
| H-γ⁴-DiH4AmPyrr1-OH | (2'R, 4'S)-4'-Amino-pyrrolidine-2'-yl-3-propionic acid |
| H-γ⁴-DiH4AmPyrr2-OH | (2'R, 4'R)-4'-Amino-pyrrolidine-2'-yl-3-propionic acid |
| H-γ⁴-DiH4PhePyrr1-OH | (2'R, 4'R)-4'-Phenyl-pyrrolidine-2'-yl-3-propionic acid |
| H-γ⁴-DiH4PhePyrr2-OH | (2'R, 4'S)-4'-Phenyl-pyrrolidine-2'-yl-3-propionic acid |
| H-γ⁴-DiH5PhePyrr1-OH | (2'S, 5'R)-5'-Phenyl-pyrrolidine-2'-yl-3-propionic acid |
| H-γ⁴-DiH5PhePyrr2-OH | (2'S, 5'S)-5'-Phenyl-pyrrolidine-2'-yl-3-propionic acid |
| H-γ⁴-DiH4Hyp1-OH | (2'R, 4'S)-4'-Hydroxy-pyrrolidine-2'-yl-2-propionic acid |
| H-γ⁴-DiH4Hyp2-OH | (2'R, 4'R)-4'-Hydroxy-pyrrolidine-2'-yl-3-propionic acid |
| H-γ⁴-DiH4Mp1-OH | (2'R, 4'S)-4'-Mercapto-pyrrolidine-2'-yl-3-propionic acid |
| H-γ⁴-DiH4Mp2-OH | (2'R, 4'R)-4'-Mercapto-pyrrolidine-2'-yl-3-propionic acid |
| H-γ⁴-DiHPip-OH | (2'S)-Piperidine-2'-yl-3-propionic acid |
| H-γ⁴-DiHPro-OH | (2'S)-Pyrrolidine-2'-yl-3-propionic acid |
| (AEt) G | N-(2-Aminoethyl)glycine |
| (APr) G | N-(3-Amino-n-propyl)glycine |
| (ABu) G | N-(4-Amino-n-butyl)glycine |
| (APe) G | N-(5-Amino-n-pentyl)glycine |
| (GuEt) G | N-(2-Guanidinoethyl)glycine |
| (GuPr) G | N-(3-Guanidino-n-propyl)glycine |
| (GuBu) G | N-(4-Guanidino-n-butyl)glycine |
| (GuPe) G | N-(5-Guanidino-n-pentyl)glycine |
| (PEG₃-NH₂) G | N-[H₂N-(CH₂)₃-(OCH₂-CH₂)₂-O(CH₂)₃]glycine |
| (Me) G | N-Methylglycine |
| (Et) G | N-Ethylglycine |
| (Bu) G | N-Butylglycine |
| (Pe) G | N-Pentylglycine |
| (Ip) G | N-Isopropylglycine |
| (2MePr) G | N-(2-Methylpropyl)glycine |
| (3MeBu) G | N-(3-Methylbutyl)glycine |
| (1MePr) G | (1S)-N-(1-Methylpropyl)glycine |
| (2MeBu) G | (2S)-N-(2-Methylbutyl)glycine |
| (MthEt) G | N-(Methylthioethyl)glycine |
| (MthPr) G | N-(Methylthiopropyl)glycine |
| (Ben) G | N-(Benzyl)glycine |
| (PhEt) G | N-(2-Phenylethyl)glycine |
| (HphMe) G | N-([4'-hydroxyphenyl]methyl)glycine |
| (HphEt) G | N-(2-[4'-hydroxyphenyl]ethyl)glycine |
| (ImMe) G | N-(Imidazol-5-yl-methyl)glycine |
| (ImEt) G | N-(2-(Imidazol-5'-yl)ethyl)glycine |
| (InMe) G | N-(Indol-2-yl-methyl)glycine |
| (InEt) G | N-(2-(Indol-2'-yl)ethyl)glycine |
| (CboMe) G | N-(Carboxymethyl)glycine |
| (CboEt) G | N-(2-Carboxyethyl)glycine |
| (CboPr) G | N-(3-Carboxypropyl)glycine |
| (CbaMe) G | N-(Carbamoylmethyl)glycine |
| (CbaEt) G | N-(2-Carbamoylethyl)glycine |
| (CbaPr) G | N-(3-Carbamoylpropyl)glycine |
| (HyEt) G | N-(2-Hydroxyethyl)glycine |
| (HyPr) G | (2R)-N-(2-Hydroxypropyl)glycine |
| (Mcet) G | N-(2-Mercaptoethyl)glycine |

In a specific preferred embodiment of this invention, the macrocycles of type **Ia/Ib** are selected from the following list:
(2*S*,11*S*,19a*S*)-*N*-[2-(dimethylamino)ethyl]-15-fluoro-7,12-dimethyl-2-{[(1-naphthylamino)carbonyl]amino}-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecine-11-carboxamide;
(2*S*,11*S*,19a*S*)-*N*-(2-aminoethyl)-15-fluoro-7,12-dimethyl-2-{[2-(1-naphthyl)acetyl]amino}-5,8,13-trioxo-2,3,6,7,8,9,10,11,12,13, 19,19a-dodecahydro-1*H*,5*H*-pyrrolo[2,1-c*]*[1,4,7,12]benzoxatriaza-cyclopentadecine-11-carboxamide;
(2*S*,11*S*,19a*S*)-*N*-(2-{ [amino(imino)methyl]amino}ethyl)-15-fluoro-7,12-dimethyl-2-{[2-(1-naphthyl)acetyl]amino}-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecine-11-carboxamide;
(2*S*,11*S*,19a*S*)-15-fluoro-7,12-dimethyl-2-{[2-(1-naphthyl)-acetyl]amino}-5,8,13-trioxo-*N*-[2-(2-pyridinylamino)ethyl]-2,3,6,7,8,9,10,11,12,13,19,19a-dodecahydro-1*H*,5*H*-pyrrolo[2,1-*c*][1,4,7,12]benzoxatriazacyclopentadecine-11-carboxamide;
(2*S*,11*S*,19a*S*)-*N*-[2-(dimethylamino)ethyl]-15-fluoro-7,12-dimethyl-2-{[2-(1-naphthyl)ethyl]amino}-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecine-11-carboxamide;
(2*S*,11*S*,19a*S*)-*N*-[2-(acetylamino)ethyl]-15-fluoro-7,12-dimethyl-2-[2-(1-naphthyl)acetyl]amino-5,8,13-trioxo-2,3,6,7,8,9,10,11, 12,13,19,19a-dodecahydro-1*H*,5*H*-pyrrolo[2,1-*c*][1,4,7,12]benzoxatriazacyclopentadecine-11-carboxamide;
(2*S*,11*S*,19a*S*)-*N*-[2-(dimethylamino)ethyl]-15-fluoro-7,12-dimethyl-2-{[2-(2-naphthyl)acetyl]amino}-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecine-11-carboxamide;
(2*S*,11*S*,19a*S*)-*N*-[2-(dimethylamino)ethyl]-15-fluoro-7,12-dimethyl-5,8,13-trioxo-2-(2-[3-(trifluoromethyl)phenyl]-acetylamino)-2,3,6,7,8,9,10,11,12,13,19,19a-dodecahydro-1*H*,5*H-*pyrrolo[2,1-*c*][1,4,7,12]benzoxatriazacyclopentadecine-11-carboxamide;
(2*S*,11*S*,19a*S*)-*N*-[2-(dimethylamino)ethyl]-15-fluoro-2-[2-(3-methoxyphenyl)acetyl]amino-7,12-dimethyl-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecine-11-carboxamide;
(2*S*,11*S*,19a*S*)-*N*-[3-(dimethylamino)propyl]-15-fluoro-7,12-dimethyl-2-[2-(1-naphthyl)acetyl]amino-5,8,13-trioxo-2,3,6,7,8, 9,10,11,12,13,19,19a-dodecahydro-1*H*,5*H*-pyrrolo[2,1-*c*]-[1,4,7,12]benzoxatriazacyclopentadecine-11-carboxamide;
(2*S*,11*S*,19a*S*)-15-fluoro-7,12-dimethyl-2-[2-(1-naphthyl)-acetyl]amino-5,8,13-trioxo-*N*-(3-pyridinylmethyl)-2,3,6,7,8,9,10, 11,12,13,19,19a-dodecahydro-1*H*,5*H*-pyrrolo[2,1-*c*][1,4,7,12]-benzoxatriazacyclopentadecine-11-carboxamide;
*N*-[(4*S*,6*S*,10*S*)-14-methyl-6-[2-(2-naphthyl)acetyl]amino-9,15-dioxo-2-oxa-8,14-diazatricyclo [14.3.1.0^{4,8}]icosa-1(20),16,18-trien-10-yl]decanamide;
*N-*[*(*4*S,*6*S,*10*S*)-14-methyl-10-{[2-(2-naphthyl)acetyl]amino}-9,15-dioxo-2-oxa-8,14-diazatricyclo[14.3.1.0⁴,⁸]icosa-1(20),16,18-trien-6-yl]-2-(2-naphthyl)acetamide;
*N*-[(4*S*,6*S*,10*S*)-14-methyl-6-[2-(2-naphthyl)acetyl]amino-9,15-dioxo-2-oxa-8,14-diazatricyclo[14.3.1.0^{4,8}]icosa-1(20),16,18-trien-10-yl]-2-(1-naphthyl)acetamide;
*N*-[(4S,6S,10S)-14-methyl-9,15-dioxo-10-[(3-toluidinocarbonyl)-amino]-2-oxa-8,14-diazatricyclo[14.3.1.0^{4,8}]icosa-1(20),16,18-trien-6-yl]-2-(2-naphthyl)acetamide;
*N*-[(4*S*,6*S*,10*S*)-10-([3-(dimethylamino)anilino]carbonylamino)-14-methyl-9,15-dioxo-2-oxa-8,14-diazatricyclo[14.3.1.0^{4,8}]icosa-1(20),16,18-trien-6-yl]-2-(2-naphthyl)acetamide;
*N*-[(4S,6S,10S)-6-({[3-(dimethylamino)anilino]carbonyl}amino)-14-methyl-9,15-dioxo-2-oxa-8,14-diazatricyclo[14.3.1.0⁴,⁸]icosa-1(20),16,18-trien-10-yl]-2-(2-naphthyl)acetamide;
*N*-[(4*S*,6*S*,10*S*)-14-methyl-9,15-dioxo-6-[(3-phenylpropanoyl)-amino]-2-oxa-8,14-diazatricyclo[14.3.1.0^{4,8}] icosa-1(20),16,18-trien-10-yl]decanamide;
*N*-[(4*S*,6*S*,10*S*)-14-methyl-6-[(3-morpholinobenzoyl)amino]-9,15-dioxo-2-oxa-8,14-diazatricyclo[14.3.1.0^{4,8}]icosa-1(20),16,18-trien-10-yl]decanamide;
*N*-[(4*S*,6*S*,10*S*)-6-({[3-(dimethylamino)anilino]carbonyl}amino)-14-methyl-9,15-dioxo-2-oxa-8,14-diazatricyclo[14.3.1.0^{4,8}]icosa-1(20),16,18-trien-10-yl]decanamide;
*N*-[(4*S*,6*S*,10*S*)-6-({[3-(dimethylamino)anilino]carbonyl}amino)-14-methyl-9,15-dioxo-2-oxa-8,14-diazatricyclo[14.3.1.0^{4,8}]icosa-1(20),16,18-trien-10-yl]-2-(1-naphthyl)acetamide;
*N*-[(4*S*,6*S*,10*S*)-6-{[(3-methoxyanilino)carbonyl]amino}-14-methyl-9,15-dioxo-2-oxa-8,14-diazatricyclo[14.3.1.0^{4,8}]icosa-1(20),16,18-trien-10-yl]-2-(2-naphthyl)acetamide;
*N*-[(4*S*,6*S*,10*S*)-14-methyl-9,15-dioxo-6-{[2-(3-phenoxyphenyl)-acetyl]amino}-2-oxa-8,14-diazatricyclo[14.3.1.0^{4,8}]icosa-1(20),16,18-trien-10-yl]decanamide;
*N*-[(4*S*,6*S*,10*S*)-14-methyl-6-{[2-(2-naphthyl)acetyl]amino}-9,15-dioxo-2-oxa-8,14-diazatricyclo[14.3.1.0^{4,8}]icosa-1(20),16,18-trien-10-yl]-6-phenylhexanamide;
*N*-[(4*S*,6*S*,10*S*)-14-methyl-6-{[2-(2-naphthyl)acetyl]amino}-9,15-dioxo-2-oxa-8,14-diazatricyclo[14.3.1.0^{4.8}]icosa-1(20),16,18-trien-10-yl]-5-phenoxypentanamide;
(*E*)-*N-*[(4*S*,6*S*,10*S*)-14-methyl-6-[2-(2-naphthyl)acetyl]amino-9,15-dioxo-2-oxa-8,14-diazatricyclo[14.3.1.0^{4,8}]icosa-1(20),16,18-trien-10-yl]-3-decenamide;
*N*-[(4*S*,6*S*,10*S*)-14-methyl-6-{[2-(1-naphthyl)acetyl]amino}-9,15-dioxo-2-oxa-8,14-diazatricyclo[14.3.1.0^{4,8}]icosa-1(20),16,18-trien-10-yl]decanamide;
6-(benzyloxy)-*N*-[(4*S*, 6*S*,10*S*)-14-methyl-6-[2-(2-naphthyl)-acetyl]amino-9,15-dioxo-2-oxa-8,14-diazatricyclo[14.3.1.0^{4,8}]-icosa-1(20),16,18-trien-10-yl]hexanamide;
2-[1,1'-biphenyl]-4-yl-*N*-[(4*S*,6*S*,10*S*)-14-methyl-6-{[2-(2-naphthyl)acetyl]amino}-9,15-dioxo-2-oxa-8,14-diazatricyclo-[14.3.1.0^{4,8}]icosa-1(20),16,18-trien-10-yl]acetamide;
*N*-[(4*S*,6*S*,10*S*)-14-methyl-6-{[2-(2-naphthyl)acetyl]amino}-9,15-dioxo-2-oxa-8,14-diazatricyclo[14.3.1.0^{4,8}]icosa-1(20),16,18-trien-10-yl]-3,3-diphenylpropanamide;
6-(benzyloxy)-*N*-[(4*S*,6*S*,10*S*)-14-methyl-9,15-dioxo-10-[(6-phenylhexanoyl)amino]-2-oxa-8,14-diazatricyclo[14.3.1.0^{4,8}]-icosa-1(20),16,18-trien-6-yl]hexanamide;
2-[1,1'-biphenyl]-3-yl-N-[(4*S*,6*S*,10*S*)-6-([3-(dimethylamino)-anilino]carbonylamino)-14-methyl-9,15-dioxo-2-oxa-8,14-diazatricyclo[14.3.1.0^{4,8}]icosa-1(20),16,18-trien-10-yl]-acetamide;
2-[1,1'-biphenyl]-4-yl-*N*-[(4*S*,6*S*,10*S*)-6-([3-(dimethylamino)-anilino]carbonylamino)-14-methyl-9,15-dioxo-2-oxa-8,14-diazatricyclo[14.3.1.0^{4,8}]icosa-1(20),16,18-trien-10-yl]-acetamide;
*N*-[(4*S*,6*S*,10*S*)-10-[([1,1'-biphenyl]-4-ylsulfonyl)amino]-14-methyl-9,15-dioxo-2-oxa-8,14-diazatricyclo[14.3.1.0^{4,8}]icosa-1(20),16,18-trien-6-yl]-2-(2-naphthyl)acetamide;
*N*-[(4*S*,6*S*,10*S*)-6-{[(3-fluoroanilino)carbonyl]amino}-14-methyl-9,15-dioxo-2-oxa-8,14-diazatricyclo[14.3.1.0^{4,8}]icosa-1(20), 16,18-trien-10-yl]-2-(2-naphthyl)acetamide;
*N*-[(4*S*,6*S*,10*S*)-6-({[4-(dimethylamino)anilino]carbonyl}amino)-14-methyl-9,15-dioxo-2-oxa-8,14-diazatricyclo[14.3.1.0^{4,8}]icosa-1(20),16,18-trien-10-yl]-2-(2-naphthyl)acetamide;
phenyl *N*-[(4*S*,6*S*,10*S*)-14-methyl-10-{[2-(2-naphthyl)acetyl]-amino}-9,15-dioxo-2-oxa-8,14-diazatricyclo[14.3.1.0^{4,8}]icosa-1(20),16,18-trien-6-yl]carbamate;
*N*-[(4*S*,6*S*,10*S*)-14-methyl-9,15-dioxo-6-[2-(7-quinolinyl)acetyl]-amino-2-oxa-8,14-diazatricyclo[14.3.1.0^{4,8}]icosa-1(20),16,18-trien-10-yl]-2-(2-naphthyl)acetamide;
*N*-[(4*S*,6*S*,10*S*)-14-methyl-9,15-dioxo-6-{[2-(3-phenoxyphenyl)-acetyl]amino}-2-oxa-8,14-diazatricyclo[14.3.1.0^{4,8}]icosa-1(20),16,18-trien-10-yl]-2-(2-naphthyl)acetamide;
*N*-[(4*S*,6*S*,10*S*)-14-methyl-10-{[2-(2-naphthyl)acetyl]amino}-9,15-dioxo-2-oxa-8,14-diazatricyclo[14.3.1.0^{4,8}]icosa-1(20),16,18-trien-6-yl]decanamide;
*N*-[(4*S*,6*S*,10*S*)-14-methyl-10-{[2-(1-naphthyl)acetyl]amino}-9,15-dioxo-2-oxa-8,14-diazatricyclo[14.3.1.0^{4,8}]icosa-1(20),16,18-trien-6-yl]decanamide;
*N*-[(4*S*,6*S*,10*S*)-14-methyl-10-{[2-(1-naphthyl)acetyl]amino}-9,15-dioxo-2-oxa-8,14-diazatricyclo[14.3.1.0^{4,8}]icosa-1(20),16,18-trien-6-yl]hexanamide;
2-[1,1'-biphenyl]-4-yl-*N*-[(4*S*,6*S*,10*S*)-14-methyl-10-{[2-(1-naphthyl)acetyl]amino}-9,15-dioxo-2-oxa-8,14-diazatricyclo-[14.3.1.0^{4,8}]icosa-1(20),16,18-trien-6-yl]acetamide;
*N*-[(4*S*,6*S*,10*S*)-10-[(2-[1,1'-biphenyl]-4-ylacetyl)amino]-14-methyl-9,15-dioxo-2-oxa-8,14-diazatricyclo[14.3.1.0^{4,8}]icosa-1(20),16,18-trien-6-yl]-2,2-diphenylacetamide;
*N*-[(4*S*,6*S*,10*S*)-6-[(2-[1,1'-biphenyl]-4-ylacetyl)amino]-14-methyl-9,15-dioxo-2-oxa-8,14-diazatricyclo[14.3.1.0^{4,8}]icosa-1(20),16,18-trien-10-yl]-2,2-diphenylacetamide;
*N*-[(4*S*,6*S*,10*S*)-6-[(2,2-diphenylacetyl)amino]-14-methyl-9,15-dioxo-2-oxa-8,14-diazatricyclo[14.3.1.0^{4,8}]icosa-1(20),16,18-trien-10-yl]decanamide;
*N*-[(4*S*,6*S*,10*S*)-14-methyl-10-{[2-(1-naphthyl)acetyl]amino}-9,15-dioxo-2-oxa-8,14-diazatricyclo[14.3.1.0^{4,8}]icosa-1(20),16,18-trien-6-yl]-2,2-diphenylacetamide;
*N*-[(4*S*,6*S*,10*S*)-6-[(3,3-diphenylpropanoyl)amino]-14-methyl-9,15-dioxo-2-oxa-8,14-diazatricyclo[14.3.1.0^{4,8}]icosa-1(20),16,18-trien-10-yl]decanamide;
2-[1,1'-biphenyl]-4-yl-*N*-[(4*S*,6*S*,10*S*)-10-[(2-[1,1'-biphenyl]-4-ylacetyl)amino]-14-methyl-9,15-dioxo-2-oxa-8,14-diazatricyclo-[14.3.1.0^{4,8}]icosa-1(20),16,18-trien-6-yl]acetamide;
*N*-[(4*S*,6*S*,10*S*)-6-({[3-(dimethylamino)anilino]carbonyl}amino)-14-methyl-9,15-dioxo-2-oxa-8,14-diazatricyclo[14.3.1.0^{4,8}]icosa-1(20),16,18-trien-10-yl]-3,3-dimethylbutanamide;
*N*-[(4*S*,6*S*,10*S*)-6-({[3-(dimethylamino)anilino]carbonyl}amino)-9,15-dioxo-2-oxa-8,14-diazatricyclo[14.3.1.0^{4,8}]icosa-1(20), 16,18-trien-10-yl]-2-(2-naphthyl)acetamide;
benzyl *N-*[(4*S*,6*S*,10*S*)-6-{[2-(2-naphthyl)acetyl]amino}-9,15-dioxo-2-oxa-8,14-diazatricyclo[14.3.1.0⁴,⁸]icosa-1(20),16,18-trien-10-yl]carbamate;
*N*-[(4*S*,6*S*,10*S*)-6-{[2-(2-naphthyl)acetyl]amino}-9,15-dioxo-2-oxa-8,14-diazatricyclo[14.3.1.0^{4,8}] icosa-1(20),16,18-trien-10-yl]decanamide;
(4*S*,6*R*,15*S*)-11,16-dimethyl-*N*-(2-naphthylmethyl)-9,12,17-trioxo-6-{[3-(4-pyridinyl)propanoyl]amino}-2-thia-8,11,16-triaza-tricyclo[16.3.1.0^{4,8}]docosa-1(22),18,20-triene-15-carboxamide; tert-butyl *N*-[(4*S*,6*R*,15*S*)-11,16-dimethyl-15-{[(2-naphthylmethyl)amino]carbonyl}-9,12,17-trioxo-2-thia-8,11,16-triaza-tricyclo[16.3.1.0^{4,8}]docosa-1(22),18,20-trien-6-yl]carbamate; (4*S*,6*R*,15*S*)-11,16-dimethyl-*N*-(2-naphthylmethyl)-9,12,17-trioxo-6-(pentanoylamino)-2-thia-8,11,16-triazatricyclo[16.3.1.0^{4,8}]-docosa-1(22),18,20-triene-15-carboxamide;
(4*S*,6*R*,15*S*)-6-(acetylamino)-11,16-dimethyl-*N*-(2-naphthyl-methyl)-9,12,17-trioxo-2-thia-8,11,16-triazatricyclo-[16.3.1.0^{4,8}]docosa-1(22),18,20-triene-15-carboxamide;
(2*S*,11*S*,19a*S*)-15-fluoro-7,12-dimethyl-2-(1-naphthoylamino)-*N-*(2-naphthylmethyl)-5,8,13-trioxo-2,3,6,7,8,9,10,11,12,13, 19,19a-dodecahydro-1*H*,5*H*-pyrrolo[2,1-*c*][1,4,7,12]benzoxatriaza-cyclopentadecine-11-carboxamide;
(2*S*,11*S*,19a*S*)-15-fluoro-7,12-dimethyl-2-[2-(2-naphthyl)acetyl]-amino-*N*-(1-naphthylmethyl)-5,8,13-trioxo-2,3,6,7,8,9,10,11,12, 13,19,19a-dodecahydro-1*H*,5*H*-pyrrolo[2,1-*c*][1,4,7,12]benzoxatriazacyclopentadecine-11-carboxamide;
(2*S*,11*S*,19a*S*)-2-[(3-chlorobenzoyl)amino]-15-fluoro-7,12-dimethyl-*N*-(1-naphthylmethyl)-5,8,13-trioxo-2,3,6,7,8,9,10,11, 12,13,19,19a-dodecahydro-1*H*,5*H*-pyrrolo[2,1-*c*][1,4,7,12]benzoxatriazacyclopentadecine-11-carboxamide.

In another specific preferred embodiment of this invention, the macrocycles of type **Ia/Ib** are selected from the following list:
(2*S*,11*S*,19a*S*)-2-(acetylamino)-15-fluoro-*N*-[2-(1H-indol-3-yl)ethyl]-7,12-dimethyl-5,8,13-trioxo-2,3,6,7,8,9,10,11,12,13, 19,19a-dodecahydro-1*H*,5*H*-pyrrolo[2,1-*c*][1,4,7,12]benzoxatriazacyclopentadecine-11-carboxamide;
(2*S*,11*S*,19a*S*)-N-[2-(dimethylamino)ethyl]-15-fluoro-2-{[2-(1H-indol-3-yl)acetyl]amino}-7,12-dimethyl-5,8,13-trioxo-2,3,6,7, 8,9,10,11,12,13,19,19a-dodecahydro-1*H*,5*H*-pyrrolo[2,1-*c*]-[1,4,7,12]benzoxatriazacyclopentadecine-11-carboxamide;
(2*S*,11*S*,19a*S*)-15-fluoro-2-{[2-(1H-indol-3-yl)acetyl]amino}-*N-*[2-(1H-indol-3-yl)ethyl]-7,12-dimethyl-5,8,13-trioxo-2,3,6,7,8, 9,10,11,12,13,19,19a-dodecahydro-1*H*,5*H*-pyrrolo[2,1-*c*]-[1,4,7,12]benzoxatriazacyclopentadecine-11-carboxamide;
(2*S*,11*S*,19a*S*)-2-{[2-(dimethylamino)acetyl]amino}-15-fluoro-*N-*[2-(1H-indol-3-yl)ethyl]-7,12-dimethyl-5,8,13-trioxo-2,3,6,7,8, 9,10,11,12,13,19,19a-dodecahydro-1*H*,5*H*-pyrrolo[2,1-*c*]-[1,4,7,12]benzoxatriazacyclopentadecine-11-carboxamide;
*tert*-butyl *N*-[(2*S*,11*S*,19a*S*)-15-fluoro-11-({[2-(1H-indol-3-yl)ethyl]amino}carbonyl)-7,12-dimethyl-5,8,13-trioxo-2,3,6,7,8, 9,10,11,12,13,19,19a-dodecahydro-1*H*,5*H*-pyrrolo[2,1-*c*]-[1,4,7,12]benzoxatriazacyclopentadecin-2-yl]carbamate;
(2*S*,11*S*,19a*S*)-*N*-[2-(dimethylamino)ethyl]-15-fluoro-7,12-dimethyl-2-{[2-(1-naphthyl)acetyl]amino}-5,8,13-trioxo-2,3,6,7, 8,9,10,11,12,13,19,19a-dodecahydro-1*H*,5*H*-pyrrolo[2,1-*c*]-[1,4,7,12]benzoxatriazacyclopentadecine-11-carboxamide;
benzyl *N*-[(4*S*,6*S*,10*S*)-14-methyl-6-{[2-(2-naphthyl)acetyl]-amino}-9,15-dioxo-2-oxa-8,14-diazatricyclo[14.3.1.0^{4,8}]icosa-1(20),16,18-trien-10-yl]carbamate;
benzyl *N*-[(4*S*,6*S*,13*S*)-6-{[2-(1H-indol-3-yl)acetyl]amino}-11,15-dimethyl-9,12,16-trioxo-2-oxa-8,11,15-triazatricyclo-[15.3.1.0^{4,8}]henicosa-1(21),17,19-trien-13-yl]carbamate;
*N*-[(4*S*,6*S*,13*S*)-6-{[2-(1*H*-indol-3-yl)acetyl]amino}-11,15-dimethyl-9,12,16-trioxo-2-oxa-8,11,15-triazatricyclo-[15.3.1.0^{4,8}]henicosa-1(21),17,19-trien-13-yl]decanamide.

### Synthesis of the Building Blocks

Readily available examples of amino acids representing subunits of the Bridge **C** are detailed to the level of fully-defined structures in Table 10. Additional analogs can be accessed smoothly, and a plethora of literature precedents are published. Therefore this section focuses on synthetic approaches towards building blocks of the Template **A** and the Modulator **B.** Functional groups not involved in ring connections of the macrocyclic backbone can be diversified by standard methods of organic synthesis, preferably by parallel/combinatorial chemistry introducing so-called high variation substituents. These derivatization methods are well-known to those skilled in the art and do not require further exemplification (selected references: A. R. Katritzky et al. (eds), Comprehensive Functional Group Transformations, Pergamon, 1995**;** S. Patai, Z. Rappoport (eds), Chemistry of Functional Groups, Wiley, 1999**;** J. March, Advanced Organic Chemistry, 4 ed., Wiley, 1992**;** D. Obrecht, J.M. Villalgordo (eds), Solid-Supported Combinatorial and Parallel Synthesis of Small-Molecular-Weight Compound Libraries, Pergamon, 1998**;** W. Bannwarth et al. (eds), Combinatorial Chemistry: From Theory to Application, 2 ed., Wiley-VCH 2006**).**

### Synthesis of Template A Building Blocks

### A) General Transformations

The majority of the building blocks of type **A** are based on readily available substances carrying a carboxylic acid group and either an aromatic or heteroaromatic hydroxy (Ar/Hetar-OH) or thiol moiety (Ar/Hetar-SH). The -COOH group may be attached to the same ring as the -OH/-SH group or to an annelated ring which in turn may be aromatic or partially unsaturated. In one subset of building blocks the -COOH group is replaced by a sulfanyl group (-SH) leading to sulfanyl phenols (mercapto phenols) and sulfanyl thiophenols (mercapto thiophenols) as building blocks. Yet another small subset carries instead of the -COOH an alkenyl group (-(CHR³)_{q}-CR¹²=CH₂; q=0-4).

In general phenol derivatives are more abundantly described in the literature than the corresponding thiophenols. However, transformations of phenols into thiophenols are well established. Therefore the phenolic systems can be regarded as precursors towards their thio-analogs. Alternatively thiophenols might be derived from the corresponding aryl halides or diazonium salts.

Selected examples of general scope for transformations introducing a sulfanyl group (-SH), i.e. Ar/Hetar-X → Ar/Hetar-SH (X= OH, F, Cl, Br, I, N₂⁺), are the following T-I to T-VII:
T-I: A sequence of broad applicability is the transformation of a phenol into a thiocarbamate with *N*,*N*-dimethylthiocarbamoyl chloride, followed by Newman-Kwart rearrangement and subsequent hydrolysis (A. Gallardo-Godoy et al., J. Med. Chem. 2005, 48, 2407-2419; P. Beaulieu et al., Bioorg. Med. Chem. Lett. 2006, 16, 4987-4993; H. Sugiyama et al., Chem. Pharm. Bull. 2007, 55, 613-624; S. Lin et al., Org. Prep. Proced. Int. 2000; 547-556).
T-II: The direct transformation of an -OH adjacent to a pyridinic nitrogen (i.e. equivalent to the pyridone tautomer) can be accomplished by heating with P₂S₅ (K. Hirai et al., Heterocycles 1994, 38, 277-280).
T-III: As an alternative to phenols, halogen-substituted (esp. with F or Cl) aromatic ring systems might serve as precursors. In case the halogen is in a position activated by an electron withdrawing group in *ortho-* or *para*-position the -SH moiety or a protected surrogate can be introduced under mild conditions by nucleophilic aromatic substitution reactions (S_{N}Ar) (G.J. Atwell et al., J. Med. Chem. 1994, 37, 371-380). Especially in the field of heterocyclic compounds, where the electron withdrawing effect is exerted by pyridine-like nitrogen atoms, this type of substitution is often utilized (S. McCombie et al., Heterocycles, 1993, 35, 93-97).
T-IV: Similarly, in Sandmeyer-type reactions a diazonium group (-N₂⁺) is replaced (C. Mukherjee, E. Biehl, Heterocycles 2004, 63, 2309-2318).
T-V: In positions not activated for an S_{N}Ar the substitution of halogen atoms (esp. Br or I) can be accomplished via the corresponding organolithium or Grignard reagents (J.L. Kice, A. G. Kutateladze, J. Org. Chem. 1993, 58, 917-923; P.C. Kearney et al., J. Am. Chem. Soc. 1993, 115, 9907-9919). Alternatively, transition metal-catalyzed transformations are feasible for this type of reaction, e.g. Cu-catalyzed substitution with benzothioic *S*-acid (N. Sawada et al., Tetrahedron Lett. 2006, 47, 6595-6597), or Pd-catalyzed substitution with KS-Si(*i*-Pr)₃ followed by desilylation of the introduced -SSi(*i*-Pr)₃ group (A. M. Rane et al., Tetrahedron Lett. 1994, 35, 3225-3226).

The thus introduced -SH moieties constitute a thia-bridge -Sin the later macrocyclic products and can be selectively transformed into higher oxidation states. Therefore the building blocks with sulfanyl moieties are also regarded as building blocks for the introduction of sulfinyl (-S(=O)-; i.e. sulfoxide) and sulfonyl (-S(=O)₂-; i.e. sulfone) moieties. Suitable oxidation methods are:
T-VI: The selective oxidation of a thioether (-S-) to a sulfoxide (-S(=O)-) can be highly selectively and mildly achieved with hexamethylenetetramine-bromine HMTAB (K. Choudhary et al.; J. Phys. Org. Chem. 2000, 13, 283-292); under these conditions primary hydroxyl groups for example are not affected. In a number of related reactions chlorotrimethylsilane showed high selectivity, too (Y.-J. Chen et al., Tetrahedron Lett. 2000, 41, 5233-5236).
T-VII: Stronger oxidants directly transfer the sulfanyl (-S-) into the sulfonyl group (-S(=O)₂-). Among the many reagents mentioned in literature the system periodic acid/ chromium(VI)oxide for example can be applied in the presence of C=C-double bonds (US2007/293548 A1).

Hydroxyl groups attached to aromatic rings (Ar-OH or Hetar-OH) in turn, if not already part of a commercially available substance, can be introduced by various methods, e.g. H-I to H-IV:
H-I: Analogously to T-III) the hydroxy group or its surrogate can be introduced by an S_{N}Ar reaction of halogen atoms, esp. Cl or F, *ortho* or *para* to an electron withdrawing substituent (W. Cantrell, Tetrahedron Lett. 2006, 47, 4249-4251) or to a pyridinic nitrogen atom (S.D. Taylor et al., J. Org. Chem. 2006, 71, 9420-9430).
H-II: Sandmeyer-type hydroxylations of aromatic amines via intermediate diazonium salts (P. Madsen et al., J. Med. Chem. 2002, 45, 5755-5775) .
H-III: The substitution of halogen atoms (esp. Br and I), which not activated for an S_{N}Ar, can be achieved by transition metal-catalyzed C-O-couplings. Predominant are Pd-catalysts (K.W. Anderson et al., J. Am. Chem. Soc. 2006, 128, 10694-10695; B.J. Gallon et al., Angew. Chem., Int. Ed. 2007, 46, 7251-7254), but also others find application, like Cu-catalysts (J.E. Ellis, S.R. Lenger, Synth. Commun. 1998, 28, 1517-1524).
H-IV: Of broad scope is also a two-step process which first transforms halogen atoms (Cl, Br and I) into a boronate and then oxidatively cleaves the carbon-boron bond to the phenol (J.R. Vyvyan et al., J. Org. Chem. 2004, 69, 2461-2468).

The carboxylic acid group of template **A** building blocks, if not already present in commercially available substances, can be introduced by standard procedures like C-I to C-IV:
C-I: The oxidation of functional groups like hydroxymethyl (-CH₂-OH) or aldehyde (-C(=O)H) is achieved under mild conditions (G.V.M. Sharma et al., Synth. Commun. 2000, 30, 397-406; C. Wiles et al., Tetrahedron Lett. 2006, 47, 5261-5264). Also methyl groups on benzene rings can be oxidized; however, as harsh reaction conditions are usually required, its applicability is limited. In contrast, the relatively acidic methyl groups *ortho* or *para* to a pyridine nitrogen can be oxidized under milder conditions; making this the method of choice for many pyridine analogs (T. R. Kelly, F. Lang, J. Org. Chem. 1996, 61, 4623-4633).
C-II: Halogen atoms can easily be replaced by a carboxyl group or surrogate thereof, e.g. by halogen metal exchange and subsequent carboxylation of the intermediate Grignard or organolithium species (C.G. Screttas, B.R. Steele, J. Org. Chem. 1989, 54, 1013-1017), or by utilizing Mander's reagent (methyl cyanoformate)(A. Lepretre et al., Tetrahedron 2000, 56, 265-274).
C-III: In the case that acidified ring positions are to be carboxylated, a viable method is deprotonation with a strong base (usually *tert*-butyl lithium) followed by carboxylation of the intermediate organolithium species in analogy to C-II). C-IV: Hydrolysis of ester, amide or nitrile groups. The CN group in turn can easily be introduced by treating organic halides with CuCN (Rosenmund-von Braun reaction: C. F. Koelsch, A. G. Whitney, J. Org. Chem., 1941, 6, 795-803).

Applied to commercially available starting materials these general transformations offer a tool box for accessing a huge variety of Templates **A.** Additional literature examples are cited below within the sections on specific derivatives.

### B) Specific Transformations

Selected transformations of broad applicability leading to specific building block categories of hydroxy-aryl or hydroxy-heteroaryl derivatives are described below:

### A1-A59 and A627-A656 Phenyl Derivatives

A plethora of hydroxy benzoic acids with diverse substitution patterns are commercially available and can be directly utilized for constructing the macrocyclic backbone. Even not so common tetrasubstituted hydroxy benzoic acids **(A53-A59)** can be constructed by processes relying on the general procedures mentioned above, e.g. carboxylation of pentasubstituted phenol derivatives (K. Sung, R.J. Lagow, J. Mater. Chem. 1996, 6, 917-918; E. Marzi, M. Schlosser, Tetrahedron 2005, 61, 3393-3402; K.C. Nicolaou et al., Angew. Chem., Int. Ed. 1999, 38, 3334-3339). Alternative approaches to tetrasubstituted hydroxy benzoic acids involve, for example, the oxidation of benzaldehydes followed by the introduction of substituents into the remaining free positions (K.C. Nicolaou et al., Chem. Eur. J. 2000, 6, 3095-3115).

In the sulfanyl thiophenol (mercapto thiophenol) series **A627-A636** simpler derivatives are commercially available. The synthesis of more advanced ones can be achieved by subjecting suitable precursors to the general procedures T-I to T-V. Once incorporated in the macrocycle **Ia/Ib** the thioether (sulfanyl group; -S-) can be easily and selectively oxidized to the sulfoxide or sulfone by applying the general procedures T-VI and T-VII. Therefore **A627-A636** should also be regarded as building blocks for inserting a sulfinyl (-S(=O)-) or sulfonyl (-S(=O)₂-) group into macrocycle **Ia/Ib.**

A C=C-double bond connectivity between Template **A** und Bridge **C** is conveniently introduced by metathesis or ring closing metathesis reactions. The corresponding building blocks **A** are based on phenol/thiophenol derivatives carrying a vinyl or alkenyl substituent (-(CHR³)_{q}-CR¹²=CH₂; q=0-4). A number of vinyl (q=O) or allyl (q=1) analogs are commercially available. Depending on the length q of the methylene tether, different approaches are feasible. Vinyl (q=O) and allyl (q=1) can be easily obtained by cross coupling reactions of the corresponding halo-substituted (Cl, Br, I) phenols/thiophenols, e.g. by Heck, Suzuki or Stille reaction (L. Joucla et al., Tetrahedron Lett. 2008, 49, 4738-4741; B. Soederberg et al., J. Org. Chem. 1997, 62, 5838-5845; Y. Yamamoto et al., Chem. Lett. 2006, 35, 704-705). Very versatile is the mild Negishi coupling which is not only useful for introducing vinyl (T. Yamamoto, T. Yamakawa, J. Org. Chem. 2009, 74, 3603-3605) or allyl substituents (F. Kneisel et al., Synthesis 2005, 2625-2629) but is also suitable for longer side chains with terminal olefin group (q=2-4; e.g. N. Kurono, Tetrahedron 1999, 55, 6097-6108). Additional approaches involve, e.g.: a) the synthesis of ortho-allyl phenols by Claisen rearrangement (W.A.L. Otterlo et al., Tetrahedron 2005, 61, 7746-7755) or b) the chain elongation of benzaldehydes or phenyl acetaldehydes by addition of vinyl or allyl Grignard reagents followed by reduction of the thus obtained secondary alcohols to the corresponding methylene compounds with trimethylsilane/TFA (B. Dahl et al., Tetrahedron Lett. 2004, 45, 9599-9602).

Double bonds in the macrocyclic backbone can be easily hydrogenated and should therefore be regarded as building blocks for introducing optionally substituted methylene linkers (-(CHR³)_{q}-CR¹²-) as well.

### A60-A143 and A657-A659 Pyridine Derivatives

As with the previous class of compounds, also for pyridines a large number of substances is commercially available and can be incorporated into the macrocycle directly or easily transferred into suitably substituted pyridines by the general methods mentioned above. Selected literature examples are available for transformations of type C-III (M. Shimano et al. Tetrahedron Lett. 1998, 39; 4363-4366), H-11 (L. Carpino et al., J. Org. Chem. 2004, 69; 54-61), C-I (T.R. Kelly, F. Lang, Tetrahedron Lett. 1995, 36, 5319-5322), or C-IV (J.L. LaMattina, R.L. Taylor, J. Org. Chem. 1981, 46, 4179-4182). For the related sulfanyl/sulfinyl/sulfonyl pyridines **A657** and alkenyl/alkyl pyridines **A658/A659** compare the section about the corresponding phenyl derivatives above.

### A144-A165 Pyridazine Derivatives

In analogy to general transformation C-I, readily available, methyl pyridazines can be oxidized to the corresponding 3- or 4-carboxylic acids (M. Morishita et al., Chem. Pharm. Bull. 1994, 42, 371-372; M. Winn et al., J. Med. Chem. 1993, 36, 2676-2688). Of equally broad scope is the hydrolysis of the corresponding nitriles under chemical (G. Heinisch, D. Lassnigg, Arch. Pharm. (Weinheim, Ger.) 1987, 320, 1222-1226) or enzymatic conditions (nitrilase from *Rhodococcus sp.:* N. Klempier et al., Tetrahedron Lett. 1991, 32, 341-344).

In addition, the pyridazine core can be built up from β-ketoesters which are subjected to a Staudinger reaction followed by an aza-Wittig-cyclization of the intermediate azide (S.V. Galiullina et al., Russ. J. Org. Chem. 2007, 43, 607-614; M. Guillaume et al., Synthesis 1995, 8, 920-922). In another approach β-ketoesters are cyclocondensed with the monohydrazone of α, β-diketones (E.E. Schweizer, K.-J. Lee, J. Org. Chem. 1982, 47, 2768-2773).

### A166-A188 and A660-A662 Pyrimidine Derivatives

As for pyridines, a large number of suitable building blocks are commercially available and can be directly used or easily transferred into the targeted compounds by the standard procedures mentioned above, including transformations of type C-I (Y. Honma et al., Chem. Pharm. Bull. 1982, 30, 4314-4324), or C-IV (I.V. Oleinik, O.A. Zagulyaeva, Chem. Heterocycl. Compd. 1993, 29, 427-431). In addition, the pyrimidine 2-carboxylic acid core can be constructed by cyclocondensation of oxalates with malonamidine derivatives (G.A. Howard et al., J. Chem. Soc. 1944, 476-477). Different substitution patterns are accessible by reacting malonates with amidines (M. Otsuka et al., Chem. Pharm. Bull. 1985, 33, 515-519).

For the related sulfanyl/sulfinyl/sulfonyl pyrimidines **A660** and alkenyl/alkyl pyrimidines **A661/A662** compare the section about the corresponding phenyl derivatives above.

### A189-A200 Pyrazine Derivatives

Pyrazine carboxylic acids are easily obtained by cyclocondensation of α,β-diaminopropionic acid with α,β-dicarbonyl derivatives (J. Bostroem et al., Bioorg. Med. Chem. 2007, 15, 4077-4084). Selected standard protocol examples are cited for C-I (J.R. Young et al., Bioorg. Med. Chem. Lett. 2000, 10, 1723-1728), for C-III (N. Ple et al., Tetrahedron 1998, 54, 9701-9710), and for C-II (A.P. Krapcho et al., J. Heterocycl. Chem. 1997, 34, 27-32). Highly chemoselective oxidations of type C-I can also be achieved by biotransformation with *Pseudomonas putida* (A. Kiener, Angew. Chem. 1992, 104, 748-749).

### A201-A206 Triazine Derivatives

A possible route to suitably substituted precursors of bifunctional triazines is the cyclocondensation of amidrazones with α,β-diketones or α,β-diketoesters (M. Sagi et al., Heterocycles 1990, 30, 1009-1021). In addition, α,γ-diketoesters are described as suitable starting material, in which case a multi-step reaction sequence proceeds via intermediate 4-nitrosopyrazoles (R. Fusco, S. Rossi, Tetrahedron 1958, 3, 209-224).

### A207-A228 and A663-A665 Derivatives of Furan, Thiophene and Pyrrole

Furan-3-carboxylic acids of type **A207/208** can be synthesized from 2-carbonyl-3-oxo-butanoates by bromination and subsequent cyclization (A. Becker, Helv. Chim. Acta 1949, 32, 1114-1122; R. Richter, Helv. Chim. Acta 1949, 32, 1123-1136). The analogous thiophenes **A207/208** can be prepared from 3-methoxycarbonyltetrahydrothiophene-4-one by oxidation (M.R. Banks et al., J. Chem. Res. (M) 1984, 369-389) or by condensation with aldehydes and subsequent isomerization (R. Jaunin, Helv. Chim. Acta 1980, 63, 1542-1553). Pyrroles **A207/208** can be obtained from suitably substituted *N*-protected 3-amino-acrylates by treatment with 2-chloro-acetyl chloride followed by cyclization (E. Benary, R. Konrad, Chem. Ber. 1923, 56, 44-52).

Furans **A209/210** are accessible in multi-step reactions starting from methylenephosphoranes and aldehydes (H.H. Wasserman, G.M. Lee, Tetrahedron Lett. 1994, 35, 9783-9786). Thiophenes **A209/210** can be prepared from 2-mercaptoacetate and acetylene carboxylates (H. Fiesselmann, G. Pfeiffer, Chem. Ber. 1954, 87, 848-856).

The pyrroles **A209/210** are obtained by condensing β-alanine esters with 2,3-dioxo-pent-4-enoates (H.H. Wasserman et al., Tetrahedron Lett. 1989, 30, 1721-1724) or by reacting 3-oxo-propanoates with glycine esters (A. Treibs, A. Ohorodnik, Liebigs Ann. Chem. 1958, 611, 139-149).

Furan carboxylic acids of type **A211/212** can be synthesized from diazomalonates and suitably substituted alkynes in a two-step-procedure catalyzed by Rh(II)-acetate (P. Muller, C. Gränicher, Helv. Chim. Acta 1993, 76, 521-534). The corresponding thiophenes **A211-214** are accessible by carboxylation method C-III (J. Sicé, J. Am. Chem. Soc. 1953, 75, 3697-3700), which is also applied for the synthesis of furans **A213/214** (D.G. Manly, E.D. Amstutz, J. Org. Chem. 1956, 21, 516-519).

Pyrroles **A211/212** can be prepared from suitably substituted 2-chloroethylidene-malonates by a tandem Staudinger reaction/ aza-Wittig protocol (F.-P. Montforts et al., Liebigs Ann. Chem. 1990, 1037-1043). Pyrroles **A213/214** can be obtained from N-protected glutamate, which is transformed into the didehydro derivative and cyclized in the presence of LiCuMe₂ (M.M. Paz, F.J. Sardina, J. Org. Chem. 1993, 58, 6990-6995).

Thiophenes **A215/216** are available from 3-bromo-thiophenes in a multi-step sequence involving general reactions T-IV and C-II (E.C. Taylor, D.E. Vogel, J. Org. Chem. 1985, 50, 1002-1004). Pyrroles **A215/216** can be prepared from amino-oxoacetate and oxalyl chloride, alcoholysis of the isocyanate and reaction with (3-bromoacetonyl)triphenylphosphonium bromide (J.P. Bazureau et al., Tetrahedron Lett. 1988, 29, 1921-1922), or from *N*-Pfp-protected 3-oxo-prolinates (F.-A. Marcotte, W.D. Lubell, Org. Lett. 2002, 4, 2601-2603).

Furans **A217/218** can be obtained from acetylene carboxylates by reaction with ethoxyvinylidene-tetracarbonyl-ferrocene complexes (Atiq-ur-Rehman et al., J. Am. Chem. Soc. 1993, 115, 9848-9849). Thiophenes **A217/218** are accessible from formylsuccinates by cyclization in the presence of methanol, hydrogen chloride and hydrogen sulfide (S. Mitra et al., J. Chem. Soc. 1939, 1116-1117), or from 2,4-dibromothiophenes by carboxylation of type C-II (D. Spinelli et al., J. Chem. Res. (M) 1993, 8, 1873-1890). Pyrroles **A217/218** (keto-tautomer) can be obtained from suitably substituted aminomethylene succinates by base-induced cyclization (C.A. Grob, P. Ankli, Helv. Chim. Acta 1949, 32, 2023-2038).

For the related sulfanyl/sulfinyl/sulfonyl analogs **A663** and alkenyl/alkyl analogs **A664/A665** compare the section about.the corresponding phenyl derivatives above.

### A229-A234 Oxazole, Thiazole and Imidazole Derivatives

Oxazoles of type **A229** are obtained by reaction of acetyl isocyanates and diazoacetate followed by cyclization (O. Tsuge et al., Tetrahedron 1973, 29, 1983-1990), whereas the thiazoles **A229** can be synthesized from bromomalonates and thioamides (F.A.J. Kerdesky et al., J. Med. Chem. 1991, 34, 2158-2165), and the imidazoles **A229** from aminomalonate and acetimidates (M.S. Poonian, E.F. Nowoswiat, J. Org. Chem. 1980, 45, 203-208). The oxazoles and thiazoles of type **A230** are accessible by cyclizing monoethyl acetamidomalonate in the presence of trifluoroacetic anhydride (J. Morgan et al., J. Chem. Soc., Perkin Trans. 1, 1997, 5, 613-620) or phosphorus pentasulfide (A.G. Long, A. Tulley, J. Chem. Soc. 1964, 1190-1192). The imidazoles **A230** can be obtained from aminomalonate by reaction with trimethylorthoformate and subsequent cyclization (R.S. Hosmane, B.B. Lim, Tetrahedron Lett. 1985, 26, 1915-1918). Oxazoles of type **A231** might be accessed from hydroxyacetaldehyde dimer (C.M. Shafer, T.F. Molinski, J. Org. Chem. 1998, 63, 551-555), and the corresponding thiazoles **A231** from β-ketoesters (P.-F. Zhang, Z.-C. Chen, Synth. Comm. 2001, 31, 415-420). Imidazoles **A231** can be prepared from *N*-protected glycines in a multi-step reaction sequence (G. van Lommen et al., Bioorg. Med. Chem. Lett. 2005, 15, 497-500; J. Singh et al., Tetrahedron Lett. 1993, 34, 211-214).

Oxazoles of type **A232** can be synthesized from diazoacetates by rhodium-catalyzed reaction with cyanoformate (G. Shi et al., J. Fluorine Chem. 1991, 52, 149-157). Oxazoles of type **A233** can be obtained by heating acetylene carboxylates with diazoacetates (R. Huisgen, H. Blaschke, Chem. Ber. 1965, 98, 2985-2997). Thiazoles **A233** are available from suitably substituted cysteine esters by reaction with diphosgene followed by bromination/ elimination (G. Serra et al., Heterocycles 1995, 41, 2701-2711). Oxazoles **A234** can be obtained from suitably substituted hydroxyacetonitriles and oxalyl chloride (K. van Aken, G. Hoornaert, J. Chem. Soc., Chem. Comm. 1992, 12, 895-896), and the thiazoles **A234** from 2-mercaptoacetate and cyanoformate (G. Satzinger, Liebigs Ann. Chem. 1978, 473-511).

### A235-A239 Isoxazole, Isothiazole and Pyrazole Derivatives

Isoxazoles and pyrazoles of type **A235** can be synthesized from (2-methoxymethylene)-malonates by reaction with hydroxylamine (K. Bowden et al., J. Chem. Soc. C 1968, 172-185) or hydrazine (T.M. Willson et al., Bioorg. Med. Chem. Lett. 1996, 6, 1047-1050).

The isothiazoles **A236** can be obtained from suitably substituted *O*-toluenesulfonyloxyiminoacetates by reacting with thioglycollates (B. Rezessy et al., Tetrahedron Lett. 1992, 33, 6523-6526); and the corresponding pyrazoles **A236** can be prepared either from suitably substituted 2-oxopropionates and hydrazinoacetates (R.N. Comber et al., Carbohyd. Res. 1992, 216, 441-452) or from 3-oxopropionates by 2-diazotation and subsequent cyclization (F.J.L. Herrera, C.U. Baelo, Carbohyd. Res. 1985, 143, 161-174).

The isoxazoles **A237** are accessible by treatment of 4-chloro-3-oxo-butanoates with isopentylnitrite (G. Hesse, G. Krehbiel, Chem. Ber. 1955, 88, 130-133). The pyrazoles **A237** can be obtained from malonates and diazoacetate (A. Bertho, H. Nüssel, Liebigs Ann. Chem. 1927, 457, 278-307). Pyrazoles of type **A238** (keto-isomers) can be synthesized from ketosuccinic acids and hydrazines (K.J. Duffy et al., J. Med. Chem. 2001, 44, 3730-3745).

Isoxazoles of type **A239** can be synthesized from 3-substituted 2-bromomaleic acids by esterification and subsequent reaction with hydroxyurea (C. Bennouna et al., Bull. Soc. Chim. Fr. 1980, 2, 478-480). Isothiazoles **A239** can be prepared from 3-substituted 2-aminofumaramides by reaction with hydrogen sulfide and hydrolysis of the formed amides (J. Lykkeberg, P. Krogsgaard-Larsen, Acta Chem. Scand. B 1976, 30, 781-785). The corresponding pyrazoles are accessible by cyclocondensation of maleates with hydrazines followed by oxidation (G.P. Lahm et al., Bioorg. Med. Chem. Lett. 2007, 17, 6274-6279).

### A240-A357 and A666-A671 Benzofuran, Benzothiophene and Indole Derivatives

Benzothiophenes **A240** can be prepared in a multi-step sequence transforming 2-hydroxy-benzaldehydes into the 3-isopropoxy-2,3-dihydrobenzothiophen-2-ones and carboxylating the 3-position (A.V. Kalinin et al., J. Org. Chem. 2003, 68, 5992-5999). A possible route to benzofurans of type **A241-A243** involves condensation of suitably substituted cyclohexane-1,3-diones and 3-bromo-2-oxo-propionic acid followed by Pd-catalyzed dehydrogenation (G. Kneen, P.J. Maddocks, Synth. Comm. 1986, 16, 1635-1640). Indoles **A244-A247** can be obtained from 2-bromo-3-nitro-benzoates by a Stille coupling to the corresponding 1-ethoxy-styrene and subsequent Pd-catalyzed cyclization in the presence of CO (R.W. Clawson et al., Tetrahedron 2006, 62, 10829-10834). Benzofurans of type **A248-A251** can be synthesized from suitably substituted 2,6-dihydroxy-benzoates by treatment with 2-chloro-ketones (F.H. Curd, A. Robertson, J. Chem. Soc. 1933, 714-720). The corresponding indoles **A250/251** might be accessible from 6-hydroxy-3-methyl-2-nitro-benzoic acid in a multi-step reaction sequence consisting of hydrogenation, cyclization, diazotization and hydroxylation (H.D. Hollis Showalter et al., J. Org. Chem. 1996, 61, 1155-1158). Benzothiophenes **A250/251** could be obtained from 2-(thiophen-3-yl-)acetaldehydes by reaction with propargyl alcohol followed by iodo-cyclization, oxidation and hydroxylation H-IV (J.P. Waldo et al., J. Org. Chem. 2008, 73, 6679-6685).

The benzothiophenes of type **A252-A255** are accessible from 3-methyl-thiophene-2-carboxylates (J.W. Terpstra, A.M. van Leusen, J. Org. Chem. 1986, 51, 230-238). Indoles **A252-A255** can be synthesized from methyl 2-methoxy-4-methyl-benzoates by bromination, nitration, reaction with dimethylformamide followed by reductive cyclization (P.L. Beaulieu et al., Bioorg. Med. Chem. Lett. 2006, 16, 4987-4993).

Possible precursors of benzofurans **A256-A259** and **A264-A267** are suitably substituted 2,4-dihydroxy-benzoates that might be alkylated in the 4-position with bromoacetaldehyde dialkyl acetals followed by cyclization (M. Dixit et al., Synlett 2006, 10, 1497-1502). Indoles of type **A256-A259** and **A264-A267** can be obtained from suitably substituted 5-hydroxy-indoles via diethyl carbamates which undergo anionic Fries rearrangement (E.J. Griffen et al., J. Org. Chem. 1995, 60, 1484-1485). The benzothiophenes of type **A260** and **A263** are accessible from 4,6-dibromo-benzene-1,3-carbaldehyde by treatment with methoxide and 2-mercaptoacetate followed by cyclization, decarboxylation, demethylation and oxidation of the aldehyde to the acid (A.E. Jakobs et al., Tetrahedron 1994, 50, 9315-9324).

Benzofurans of type **A268-A271** can be synthesized from the corresponding 4-hydroxy-benzofurans by carboxylation (T. Reichstein, R. Hirt, Helv. Chim. Acta 1933, 16, 121-129) or by reacting 5-carbomethoxy-6-hydroxy-salicylaldehydes with bromo-acetates followed by saponification and cyclization (R.T. Foster, A. Robertson, J. Chem. Soc. 1948, 115-116). Preparation of the corresponding benzothiophenes **A268-A271** can start from a suitably substituted 4-oxo-tetrahydrobenzothiophene which is subjected to acylation and subsequent aromatization (P.P. Yadav et al., Bioorg. Med. Chem. 2005, 13, 1497-1505). Similarly indoles **A268-A271** can be prepared from 4-oxo-tetrahydroindoles, or alternatively by Claisen rearrangement of the allyl ether of 4-amino-salicylic acid followed by cleavage of the double bond and cyclization (T. Kakigami et al., Chem. Pharm. Bull. 1988, 46, 42-52). Benzofurans of type **A272-A275** can be synthesized from *O*-protected 4-hydroxy-salicylaldehydes by reaction with diazoacetate followed by dehydration (M. E. Dudley et al., Synthesis 2006, 1711-1714). Benzothiophenes **A272-A275** are accessible from 5-bromo-benzothiophene by Friedel-Crafts acylation as key step (S. Mitsumori et al., J. Med. Chem. 2003, 46, 2446-2455), and the corresponding indoles **A272-A275** by Nenitzescu reaction of para-benzoquinones with substituted 2-aminoacrylates (E.A. Steck et al., J. Org. Chem. 1959, 24, 1750-1752). Benzofurans of type **A276-A279** can be obtained from 3-acetyl-4-hydroxy-benzoates by bromination and subsequent base-induced cyclization (G. Doria et al., Farmaco 1980, 35, 674-680).

Benzothiophenes **A276-A279** can be prepared from 4-fluorobenzoates and thioglycolate by intramolecular Friedel-Crafts acylation (D.L. Gernert et al., Bioorg. Med. Chem. Lett. 2004, 14, 2759-2764).

Benzofurans and benzothiophenes of type **A284-A287** can be synthesized from suitably substituted 3-furaldehydes or 3-formyl-thiophenes by cyclocondensation with succinates (D. Simoni et al., J. Med. Chem. 2006, 49, 3143-3152) . Indoles of type **A284** and **A287** are accessible from 3-methoxy-4-amino-benzoates by a reaction sequence consisting of iodination, Sonogashira coupling and cyclization (J. Ezquerra et al., J. Org. Chem. 1996, 61, 5804-5812). Benzofurans and benzothiophenes of type **A288-A291** can be obtained from suitably substituted 2-furaldehydes (D. Simoni et al., J. Med. Chem. 2006, 49, 3143-3152). Indoles **A288-A291** are available by cyclocondensation of pyrrole-2-carbaldehydes and diethyl succinate (C. Fuganti, S. Serra, J. Chem. Res. (M) 1998, 10, 2769-2782). Indoles of type **A292-A295** can be prepared from *N*-protected furo[3,2-*b*]pyrrole-5-carboxylates by decarboxylation and subsequent Diels-Alder reaction (A. Krutosikova, M. Hanes, Collect. Czech. Chem. Comm. 1992, 57, 1487-1494).

Benzofurans of type **A296-A299** can be obtained from suitably substituted (*p*-acetoxyphenoxy)acetyl chlorides by reacting with cyanide followed by ZnCl₂-mediated cyclization with 1,3-dihydroxy-benzene (L. Crombie et al., J. Chem. Soc., Perkin Trans. 1, 1987, 2783-2786). The corresponding benzothiophenes **A296-A299** can be synthesized from suitably substituted 3-bromothiophenols (S. Mitsumori et al., J. Med. Chem. 2003, 46, 2446-2455). Indoles **A296-A299** are accessible from *O*-protected 6-hydroxyindoles (M. Fedouloff et al., Bioorg. Med. Chem. 2001, 9, 2119-2128).

Benzofurans **A300-A303** can be obtained from suitably substituted 3-acetyl-furans via the silylenol ether (A. Benitez et al., J. Org. Chem. 1996, 61, 1487-1492).

Benzofurans **A304-A307** can be synthesized either from 2-allyl-3-allyloxy-4-methoxy-benzaldehydes by isomerization/metathesis and oxidation (W.A.L. van Otterlo et al., Tetrahedron 2005, 61, 7746-7755) or from substituted 2-hydroxy-3-methoxy-6-bromo-benzaldehydes by reduction of the alcohol, formation of the phosphonium salt, cyclization and subsequent carboxylation (K. Hagihara et al., Bioorg. Med. Chem. Lett. 2007, 17, 1616-1621). The corresponding benzothiophenes **A304-A307** are accessible from suitably substituted methyl thiophene-2-carboxylates by transformation into the 1-(2'-thienyl)-1,4-dioxobutanes followed by BF₃-mediated cyclization, Vilsmeier-Haack formylation of the 4-position and subsequent oxidation (S.S. Samanta et al., J. Chem. Soc., Perkin Trans. 1, 1997, 3673-3678).

The indoles **A304-A307** can be obtained by Diels-Alder reaction of the silylenolate of *N*-protected 2-acetylpyrrole with propionate followed by air oxidation (M. Ohno et al., Heterocycles 1991, 32, 1199-1202).

The mentioned synthetic routes are also feasible for preparing the more highly substituted derivatives **A308-A357.**

For the related sulfanyl/sulfinyl/sulfonyl and alkenyl/alkyl analogs **A666-A671** compare the section about the corresponding phenyl derivatives above.

### A358-A372 Pyrrolo[2,3-b]pyridine Derivatives

Pyrrolopyridines **A365/366** can be synthesized from 7-azaindoles via pyridine-N-oxides (X. Wang et al., J. Org. Chem. 2006, 71, 4021-4023) by a carboxylation reaction of type C-II (A. L'Heureux et al., Tetrahedron Lett. 2004, 45, 2317-2320). Pyrrolopyridines of type **A367/368** can be obtained from suitably substituted 4-chloro-3-formyl-pyridines by treatment with azidoacetate and subsequent Hemetsberger-Knittel reaction (P.J. Roy et al., Synthesis 2005, 2751-2757).

Pyrrolopyridines of type **A369/370** are accessible from the corresponding 5-chloro-pyrrolopyridine by formylation in a Duff reaction, oxidation and hydrolysis (R.H. Bahekar et al., Bioorg. Med. Chem. 2007, 15, 6782-6795). The synthesis of pyrrolopyridines **A371/372** can start from 4-chloro-7-azaindole and proceed via the *N*-oxide (T. Storz et al., Synthesis 2008, 201-214).

### A373-A385 Pyrrolo[2,3-c]pyridine Derivatives

Pyrrolopyridines of type **A379** can be obtained from suitably substituted 4-iodo-3-nitro-pyridines involving Sonogashira coupling and TiCl₄-mediated cyclization as key reactions (T. Sakamoto et al., Chem. Pharm. Bull. 1986, 34, 2362-2368). Pyrrolopyridines of type **A382/383** can be prepared from 2-methoxy-4-iodo-5-aminopyridines by Sonogashira coupling with TMS-acetylene, CuI-mediated cyclization, formylation and oxidation (D. Mazeas et al., Heterocycles 1999, 50, 1065-1080). Pyrrolopyridines of type **A384/385** are accessible from suitably substituted 4-methoxy-pyrrole-2-carbaldehydes by reductive amination with 3,3-diethoxy-2-amino-propionate and subsequent cyclization (S.K. Singh et al., Heterocycles 1997, 44, 379-392).

### A386-A398 Pyrrolo[3,2-c]pyridine Derivatives

Pyrrolopyridines **A387/388** are accessible from N-alkylated 2-formyl-pyrroles via 2-pyrrylacryl azides (J.S. New et al., J. Med. Chem. 1989, 32, 1147-1156). Pyrrolopyridines of type **A389/390** can be obtained from 2-methoxy-3-formyl-pyridines by reaction with azidoacetate and subsequent Hemetsberger-Knittel reaction (P.J. Roy et al., Synthesis 2005, 2751-2757).

### A399-A413 Pyrrolo[3,2-b]pyridine Derivatives

Pyrrolopyridines of type **A406/407** can be obtained from 2-(6-methoxy-3-nitro-2-pyridyl)acetates by Knoevenagel condensation with formaldehyde followed by Pd-catalyzed cyclization in the presence of hydrogen and CO (B.C.G. Soederberg et al., Synthesis 2008, 6, 903-912).

Pyrrolopyridines **A410** and **A413** can be synthesized from suitably substituted 2-chloro-3-nicotinonitriles by Sonogashira coupling with TMS-acetylene and subsequent cyclization as key steps (T. Sakamoto et al., Chem. Pharm. Bull. 1986, 34, 2362-2368). Alternatively, their preparation can be achieved by reacting 3-nitro-pyridines with vinylmagnesium bromide (Z. Zhang et al., J. Org. Chem. 2002, 67, 2345-2347). Pyrrolopyridines **A408** and **A412** are available from 2-alkynyl-3-amino-pyridines by CuI-catalyzed cyclization (A.M. Palmer et al., Bioorg. Med. Chem. 2008, 16, 1511-1530).

### A414-A449 Derivatives of Benzoxazole, Benzothiazole and Benzimidazole

Benzoxazoles **A415/416** can be prepared from *N*-acylated 3-chloro-4-anisidines via benzyne-formation and carboxylation (D.R. Reavill, S.K. Richardson, Synth. Comm. 1990, 20, 1423-1436). The corresponding benzimidazoles **A415/416** are accessible from 2-amino-3-halo-benzoates by acylation of the amine, nitration and reductive cyclization (K. Kubo et al., J. Med. Chem. 1993, 36, 1772-1784).

Benzimidazoles of type **A417-A419** can be obtained from the corresponding 4-acetamido-2-methoxy-benzoates by chlorination in 5-position, nitration in 3-position and reductive cyclization (S. Bolgunas et al., J. Med. Chem. 2006, 49, 4762-4766). Benzimidazoles **A420-A422** are accessible from the corresponding 5-methoxy-6-methyl-benzimidazoles by oxidations of type C-I (B.D. Palmer et al., J. Med. Chem. 1999, 42, 2373-2382). Benzoxazoles of type **A423-A425** can be obtained by condensing 4-methylene-2-oxazolin-5-ones and 4-triphenylphosphoranylidene-3-oxobutanoates, followed by aromatization (F. Clerici et al., Tetrahedron 1991, 47, 8907-8916). The synthesis of the corresponding benzimidazoles **A423-A425** is possible starting from 4-amino-2-hydroxy-5-nitrobenzoates by acylation, reduction of the nitro group and cyclization (A. Tanaka et al., Chem. Pharm. Bull. 1994, 42, 560-569). Benzoxazoles of type **A426-A428** can be synthesized starting from 2,5-dihydroxybenzoate (D. Diez-Martin et al., Tetrahedron 1992, 48, 7899-7939). Benzimidazoles **A429-A431** are accessible from *O*-protected 3,4-diamino-2-hydroxy-benzoates by acylation and subsequent cyclization (Y. Hirokawa et al., Chem. Pharm. Bull. 2002, 50, 941-959; A. Viger, P.B. Dervan, Bioorg. Med. Chem. 2006, 14, 8539-8549). Benzothiazoles of type **A438-A440** can be synthesized by heating suitably substituted 4-amino-3-methoxy-benzoates with thiocyanate in the presence of copper(II)-salts and subsequent 2-desamination (I.A. Ismail et al., J. Org. Chem. 1980, 45, 2243-2246). Benzimidazoles **A441-A443** can be prepared by a multi-step sequence from 8-aminoquinolines via the corresponding 5,6-dihydro-4*H*-imidazoquinolines (R.C. Elderfield, F.J. Kreysa, J. Am. Chem. Soc. 1948, 70, 44-48). Benzimidazoles **A444** and **A447** can be obtained by reaction of suitably substituted 3-amino-4-methoxy-benzoates with nitriles followed by NaOCl-induced cyclization (J. Regan et al., Bioorg. Med. Chem. Lett. 1998, 8, 2737-2742).

### A450-A459 Derivatives of Benzisoxazole, Benzisothiazole and Indazole

Benzisoxazoles **A456** and **A459** can be synthesized starting from 2,6-dihydroxy-3-formyl-4-methyl-benzoates by reaction with hydroxylamine followed by thermal cyclization (D.H.R. Barton et al., J. Chem. Soc. C 1971, 2166-2174); this method also is suitable for benzisoxazoles of types **A450-A455** and **A457/458.** The preparation of indazoles **A457** has been described as reaction between 3-amino-2-methoxy-4-methylbenzoate and iso-amylnitrite (S. Bolgunas et al., J. Med. Chem. 2006, 49, 4762-4766).

### A460-A515 Naphthalene Derivatives

A large number of suitably substituted naphthalene derivatives is commercially available. In addition, naphthalenes **A460-A465** and **A496-A499** can be obtained from the corresponding 2-hydroxy-naphthalenes via lithiation and carboxylation (cf. C-III) (K. Takahashi et al., Tetrahedron 1994, 50, 1327-1340). Higher substituted analogs can be prepared in a multi-step sequence from suitably substituted 2-bromotoluenes via 2-tetralone-1-carboxylates (F.C. Goerth et al., Eur. J. Org. Chem. 2000, 2605-2612).

Naphthalenes of type **A478-A483** and **A508-A511** can be synthesized either by demethylation of 3-methoxynaphthalene-1-carboxylates (R.E. Royer et al., J. Med. Chem. 1995, 38, 2427-2432) or by H-II type reaction of 3-aminonaphthalene-1-carboxylates (K.J. Duffy et al., J. Med. Chem. 2001, 44, 3730-3745).

The naphthalenes **A484-A489** and **A504-A507** can easily be built up by condensation of succinic esters with either benzaldehydes (A.M. El-Abbady et al., J. Org. Chem. 1961, 26, 4871-4873; M. Kitamura et al., Angew. Chem. Int. Ed. 1999, 38, 1229-1232) or with benzophenones (F.G. Baddar et al., J. Chem. Soc. 1955, 1714-1718).

Naphthalene derivatives of type **A490-A495** and **A512-A515** can be obtained from 2-methoxynaphthalenes by bromination in 4-position and C-II type carboxylation (J.A. O'Meara et al., J. Med. Chem. 2005, 48, 5580-5588) or from 2-chloro-naphthalene and phthalic anhydride (G. Heller, Chem. Ber. 1912, 45, 674-679).

### A516-A548 and A672-A674 Quinoline Derivatives

The synthesis of quinolines **A516-A518** can be accomplished by reacting suitably substituted isatins with phenacylbromides (H. John, J. Prakt. Chem. 1932, 133, 259-272; E.J. Cragoe et al., J. Org. Chem. 1953, 18, 552-560).

Quinolines **A522-A524** are easily accessible from 2-amino-benzaldehydes via a modified Friedlander synthesis (D.L. Boger, J.-H. Chen, J. Org. Chem. 1995, 60, 7369-7371). Similarly, quinoline derivatives **A527-A529** can be obtained from 2-amino-benzaldehydes by condensation with malonic acid (J. Tröger, C. Cohaus, J. Prakt. Chem. 1927, 117, 97-116).

Quinolines **A525** can be synthesized from the corresponding 2-cyanoquinoline-1-oxides by rearrangement (C. Kaneko, S. Yamada, Chem. Pharm. Bull. 1967, 15, 663-669). Quinolines **A526** is accessible from substituted 2-aminoacetophenones by reaction with 3-chloro-3-oxopropionate and subsequent base-induced cyclization (A. Capelli et al., Bioorg. Med. Chem. 2002, 10, 779-802).

Quinolines of type **A530-A533** can be constructed from 2-anisidines by reacting with 2-oxosuccinic esters followed by thermal cyclization (L. Musajo, M. Minchilli, Chem. Ber. 1941, 74, 1839-1843). Syntheses of quinolines **A534-A536** and **A537-A539** can be achieved via the corresponding halogen substituted analogs followed by carboxylation of type C-II (F. Cottet et al., Eur. J. Org. Chem. 2003, 8, 559-1568).

For the synthesis of quinolines **A543-A545** the condensation of isatins with malonic acid is frequently described in the literature (e.g. W. Borsche, W. Jacobs, Chem. Ber. 1914, 47, 354-363; J.A. Aeschlimann, J. Chem. Soc. 1926, 2902-2911). Derivatives of type **A546-A548** can be obtained in a multi-step reaction starting with 3-chloroanilines (A.M. Spivey, F.H.S. Curd, J. Chem. Soc. 1949, 2656-2662).

For the related sulfanyl/sulfinyl/sulfonyl quinolines **A672** and alkenyl/alkyl quinolines **A673/A674** compare the section about the corresponding phenyl derivatives above.

### A549-A564 Isoquinoline Derivatives

Isoquinolines of type **A549-A553** with a carboxyl in 1-position can be prepared from benzaldehydes in a multi-step sequence consisting of transformation into aminoethanes, reaction with oxalic ester, cyclization, and aromatization (M. Keizo et al., Chem. Pharm. Bull. 1982, 30, 4170-4174; S. Naoki et al., Chem. Pharm. Bull. 1989, 37, 1493-1499).

Isoquinoline-3-carboxylates **A554-A556** are accessible from hydroxy-phenylalanines by Bischler-Napieralski reaction and subsequent aromatization, or alternatively from 2-methyl benzaldehydes by reacting with azidoacetate, thermal cyclization and aromatization (Y. Fukuda et al., J. Med. Chem. 1999, 42, 1448-1458; T. R. Burke et al., Heterocycles 1992, 34, 757-764). Compounds of type **A557/558** can be synthesized by reaction of 2-aminobenzoic acids and 5-chloro-3-carboxy-1,2,4-pyrazines in the presence of amylnitrite (A.M. d'A. Rocha Gonsalves, T.M.V.D. Pinho e Melo, Tetrahedron 1992, 48, 6821-6826), whereas isoquinolines **A559/560** can be prepared from 2-formylbenzoic acids via isothiochromenone (D.J. Dijksman, G.T. Newbold, J. Chem. Soc. 1951, 1213-1217).

Access to isoquinolines **A561/562** is feasible by transformation of isoquinolines into the corresponding Reissert compounds, nitration in 4-position and hydrolysis (M. Sugiura et al., Chem. Pharm. Bull. 1992, 40, 2262-2266).

Isoquinolines of type **A563/564** are accessible from (2-methoxycarbonyl-phenyl)acetic acids by reaction with methyl formate followed by cyclization and amination (H.E. Ungnade et al., J. Org. Chem. 1945, 10, 533-536).

### A565-A577 Quinazoline Derivatives

The most general routes to quinazolines use appropriately substituted phenyl derivatives onto which the pyrimido ring is cyclized, e.g. the cyclocondensation of 2-amino benzamides with oxalates (M. Suesse et al., Helv. Chim. Acta 1986, 69, 1017-1024), of *ortho*-carbonyl substituted phenyl oxalamic acid esters with ammonium formate (S. Ferrini et al., Org. Lett. 2007, 9, 69-72), of 2-amino benzonitriles with carbonylformimidate or chloroformamidine (A. McKillop et al., Tetrahedron Lett. 1982, 23, 3357-3360; N. Harris et al., J. Med. Chem. 1990, 33, 434-444), or of *ortho*-oxalyl anilides with ammonia (M.T. Bogert, F.P. Nabenhauer, J. Am. Chem. Soc. 1924, 46, 1702-1707).

### A578-A587 Quinoxaline Derivatives

The synthesis of quinoxalines **A578-581** via their 2-carbaldehydes is well-documented (E. Lippmann et al., Zeitschr. Chem. 1990, 30, 251-252). Representative structures of type **A582-587** are available by cyclocondensation of α,β-dicarbonyl derivatives or β-ketoesters with appropriately substituted *ortho-*phenylendiamines (S. Grivas et al., Acta Chem. Scand. 1993, 47, 521-528; A. Zychilinski, I. Ugi, Heterocycles 1998, 49, 29-32; D. Zhou et al., Bioorg. Med. Chem. 2008, 16, 6707-6723). In addition, it is possible to introduce a carboxyl group into the 2-position of the quinoxaline ring by biotransformation with *Arthrobacter nicotianae* (T. Yoshida et al., Biosci. Biotech. Biochem. 2002, 66, 2388-2394).

### A588-A601 Pyrido[5,4-d]pyrimidine Derivatives

The bicyclic cores **A588-A601** can be accessed by annelating suitably substituted pyridines. Feasible starting materials for these cyclocondensation reactions include pyridine-2,3-dicarboxylic acids (A. Tikad et al., Synlett 2006, 12, 1938-1942), 3-aminopyridine-2-carboxylates (M. Hayakawa et al., Bioorg. Med. Chem. 2006, 14, 6847-6858) or 3-aminopyridine-2-nitriles (J.B. Smaill et al., J. Med. Chem. 2000, 43, 1380-1397).

### A602-A608 Pyrimido[5,4-d]pyrimidine Derivatives

Access to the pyrimidopyrimidine group can be achieved via the well-known 4,6-dichloro (G. Rewcastle et al., J. Med. Chem. 1997, 40; 12, 1820-1826) or 2,4,6,8-tetrachloro derivatives (J. Northen et al., J. Chem. Soc., Perkin Trans. 1, 2002, 108-115) utilizing the general methods described above.

### A609-A618 and A675-A683 Tetraline Derivatives

A large number of reaction sequences towards tetralines occur via 1- or 2-tetralones as key intermediates. Their carbonyl groups are elaborated into carboxyl moieties by the action of trimethylsilyl cyanide (TMSCN) or diethyl cyanophosphonate (DECNP) and subsequent hydrolysis (M. Meyer et al., J. Med. Chem. 1997, 40, 1049-1062; F. Berardi et al., J. Med. Chem. 2004, 47, 2308-2317). These tetralones in turn are synthesized by an intramolecular Friedel-Crafts acylation of cyclization precursors derived from phenylacetonitriles and 2-phenyl malonates (R.S. Natekar, S. D. Samant, Indian J. Chem., Sect. B: Org. Chem. Incl. Med. Chem. 2002, 41, 187-190; L. Gong, H. Parnes, J. Labelled Compd. Radiopharm. 1996, 38, 425-434). Alternatively, the intramolecular cyclization can be achieved by a Buchner reaction (A. Cheung et al., Bioorg. Med. Chem. Lett. 2003, 13, 133-138).

The synthesis of enantiomerically pure analogs is described by D. Obrecht et al., Helv. Chim. Acta 1995, 78, 1567-1587.

For the related sulfanyl/sulfinyl/sulfonyl and alkenyl/alkyl tetralines **A675-A683** compare the section about the corresponding phenyl derivatives above.

### A619-A626 Indane Derivatives

Indane derivatives **A619-A623** with a carboxyl residue in the 1-position are accessible from easily available 3-cyano-indenes by hydrogenation to the indane followed by hydrolysis to the carboxylic acid moiety (T. McLean et al., J. Med. Chem. 2006, 49, 4269-4274). Furthermore, inadan-1-ones can be transferred into indan-1-caboxylic acids, for example via oxiranes (D.-I. Kato et al., J. Org. Chem. 2003, 68, 7234-7242), or in a Corey-Seebach-type reaction via 1,3-dithianes (Y.-P. Pang et al., J. Org. Chem. 1991, 56, 4499-4508).

Indane derivatives of types **A624-A626** with a carboxyl group in 2-position can be obtained from readily accessible indan-1-ones by treatment of the corresponding enolate with dimethyl carbonate, subsequent hydrogenation of the carbonyl group and final hydrolysis (T. Tanaka et al., Chem. Pharm. Bull. 1994, 42, 1756-1759; U. Hacksell et al. J. Med. Chem. 1981, 24, 429-434). Alternatively the indane ring system can be built up starting from *ortho*-xylenes by NBS bromination of both methyl groups, followed by alkylative spirocyclization with the enolate of barbituric acid and finally decarboxylative ring cleavage to indan-2-caboxylic acids (G.A. Kraus et al., J. Org. Chem. 2002, 67, 5857-5859).

### Synthesis of Modulator B Building Blocks

The Modulator **B** moieties of macrocycle **Ia/Ib** are derived from appropriately substituted aminoalcohols, wherein the amino and alcohol group, which contribute to the ring connectivity, are separated by 2-4 C-atoms.

If not already present in a commercial building block, the substituent R³ can be introduced by standard nucleophilic addition of organometallic reagents to carbonyl or carboxyl derivatives. For **B18-B21,** carrying no additional C-substituent on their ring system, such precursors are commercially available. Similarly, in the case of **B9, B10, B16** and **B17** the diversification of the substitution pattern can be easily achieved by standard transformations of the commercial analogs with free amine functionalities (i.e. -NH₂ → -NR¹¹R⁷ in the case of **B9** and -NH → -NR¹¹ for **B10, B16** and **B17**).

### B1 Aziridine Derivatives

Usually, the access to hydroxymethyl aziridines relies on reaction sequences constructing the aziridine ring. The starting materials with the broadest applicability are β-keto-esters: Transformation into the β-hydroxyimino analog, intramolecular cyclization to the aziridine and reduction of the ester group to the alcohol leads to building blocks of type **B1** (e.g. T. Sakai et al., J. Org. Chem. 2005, 70, 1369-1375). An alternative approach uses α,β-dihaloesters which are elaborated into substances of type **B1** via aziridination with ammonia and reduction of the ester group (P. Davoli et al., Tetrahedron 2001, 57, 1801-1812).

### B2-B3 Azetidine Derivatives

The standard approach to hydroxymethyl azetidines subjects easily accessible *O*-protected glycidols successively to epoxide-ring opening with azide, transformation of the thus obtained alcohol group into a suitable leaving group (e.g. tosylate or sulfate), and reduction of the azide to amine with concomitant intramolecular cyclization (F. Hosono et al., Tetrahedron 1994, 50, 13335-13346; D.-G. Liu, G.-Q. Lin, Tetrahedron Lett. 1999, 40, 337-340).

### B4-B8 Pyrrolidine Derivatives and B11-B15 Piperidine Derivatives

The synthetic pathways to pyrrolidine and piperidine building blocks **B** rely on the same strategy, and intramolecular cyclization reactions are the predominant route applicable to diversely substituted substrates. Amines carrying a residue with a leaving group in the ω-position lead directly to the desired saturated ring systems by intramolecular nucleophilic substitution (G. Ceulemans et al., Tetrahedron 1997, 53, 14957-14974; S. H. Kang, D. H. Ryu, Tetrahedron Lett. 1997, 38, 607-610; J. L. Ruano et al., Synthesis 2006, 687-691). Also *N-*haloamines can be directly transformed into the desired compounds by a Hofmann-Loffler-Freytag reaction (M. E. Wolff, Chem. Rev. 1963, 63, 55-64). Alternatively, amines carrying two substituents, each with an alkene or alkyne bond, can be subjected to a ring closing metathesis (RCM) reaction (Y. Coquerel, J. Rodriguez, Eur. J. Org. Chem. 2008, 1125-1132) and subsequent reduction of the partially unsaturated ring to the saturated heterocycle.

Another possible access, reduction of aromatic five- or six-membered heterocycles to their saturated analogs, is described in the literature. Due to the large number of commercially available pyridines this approach is especially useful for the synthesis of the piperidine system (J. Bolos et al., J. Heterocycl. Chem. 1994, 31, 1493-1496; A. Solladie-Cavallo et al., Tetrahedron Lett. 2003, 44, 8501-8504; R. Naef et al., J. Agric. Food Chem. 2005, 53, 9161-9164).

### General processes for the synthesis of macrocyclic compounds Ia/Ib

General procedures for the synthesis of libraries of macrocyclic compounds of general structure **Ia/Ib** are described below. It will be immediately apparent to those skilled in the art how these procedures have to be modified for the synthesis of individual macrocyclic compounds of type **Ia/Ib.**

The macrocyclic compounds of the invention are obtained by cyclization of suitable linear precursors which are derived from optionally substituted bifunctional phenols or thiophenols **A** ("Template"), substituted amino alcohols **B** ("Modulator"), and one to three building blocks forming "Bridge" **C.**

Phenol or thiophenol building blocks **A** comprise optionally substituted hydroxyaryl, hydroxyheteroaryl, mercaptoaryl, and mercapto-heteroaryl carboxylic acids, mercaptophenols, hydroxy-mercapto-heteroaryl compounds, alkenyl phenols or alkenyl thiophenols.

Variable substituents are introduced by pre- or postcyclative derivatization of one or more orthogonally protected attachment points (e.g. amino groups, carboxyl groups, hydroxyl groups) on **B**, **C** or **A.** Variable R-groups may also be introduced as side chain motifs of the subunits of Bridge **C.**

The macrocyclic products of the invention can be prepared either in solution or on solid support.

The essential ring closure reaction is possible between any of the building blocks; and macrocycles **Ia/Ib** are obtained by e.g.
- Macrolactamization between **C** and **B**;
- Macrolactamization between **A** and **C;**
- Macrolactamization between any two subunits of Bridge **C;**
- Arylether or arylthioether formation between **A** and **B**;
- Arylthioether formation between **A** and **C;**
- Ring closing metathesis (RCM) reaction between any two subunits of **C;** or
- Ring closure metathesis reaction between **A** and **C.**

### SW-1: Synthesis workflow for the preparation of side-chain protected macrocycles Ia/Ib by macrolactamization in solution

Macrocycles of structure **Ia/Ib** with orthogonally protected exocyclic functional groups (attachment points for derivatizations) are prepared in solution by the process outlined below. Throughout all steps the orthogonal protection of the side chains stays intact and is not affected by protecting group manipulations of the main chain. Within this workflow SW-1 the generic group M is NR⁴ or CHR⁶; and both V and W are CONR⁴.
a1) Condensation of an appropriately protected hydroxy- or mercapto-aryl/heteroaryl carboxylic acid PG¹-**A**-OH and a suitable C-terminally and side-chain protected C-subunit building block H-NR⁴-**c1**-CO-OPG² to form PG¹-**A**-NR⁴-**c1**-CO-OPG²; the related compounds PG¹-**A**-CHR⁶-**c1**-CO-OPG² are obtained if a mercaptophenol or hydroxy-mercapto heteroaryl compound is used as **A** building block and connected to a C-terminally and side-chain protected compound LG-CHR⁶-**c1**-CO-OPG² by S-alkylation (LG represents a leaving group like halide, alkyl- or aryl-sulfonate).
b1) If required, deprotection of the aryl/heteroaryl hydroxy or mercapto group;
c1) Aryl/heteroaryl ether or thioether formation with a suitably *N*-protected amino alcohol PG³-**B**-OH leading to the fully protected linear precursor PG³-**B**-**A-**M-**c1**-CO-OPG²;
d1) Cleavage of the "main chain" protective groups affording the free amino acid H-**B**-**A**-M-**c1**-CO-OH, which is either subjected to (i) immediate macrocyclization (step e1) or (ii) chain elongation by one (steps f1-i1) or (iii) two additional **C**-subunits followed my macrocyclization (steps j1-₀1).

### (i) Direct macrolactamization

e1) Intramolecular amide coupling to *cyclo*(**B**-**A**-M-**c1**-CO-) as macrocyclic product.

### (ii) Chain elongation by one additional C-subunit and subsequent macrolactamization

f1) *N*-Reprotection of the product of step d1;
g1) Coupling with a second suitably *C*-protected amino acid to PG₃-**B**-**A**-M-**c1**-V-**c2**-CO-OPG² ;
h1) Cleavage of the "main chain" protective groups;
i1) Macrolactamization to the side-chain protected macrocycle *cyclo*(**B**-**A**-M-**c1**-V-**c2**-CO-).

### (iii) Chain elongation by two additional C-subunits and subsequent macrolactamization

j1) *N*-Protection of the product obtained in step d1;
k1) Coupling with a second suitably C-protected amino acid;
11) Cleavage of the *C*-terminal protective group or cleavage of *N*- and *C*-terminal protective groups followed by reprotection of the *N*-terminus;
m1) Coupling with a third suitably *C*-protected amino acid yielding PG³-**B**-**A**-M-**c1**-V-**c2**-W-**c3**-CO-;
n1) Cleavage of the "main chain" protective groups;
o1) Macrolactamization to the side-chain protected macrocycle cyclo(**B**-**A**-M-**c1**-V-**c2**-W-**c3**-CO-).

In addition to the steps described above, the free carboxylic acid functionality of the *N*-reprotected product of step f1, or of a linear precursor (cf. steps a1, g1, k1, m1) after selective deprotection can be further elaborated by chain extensions/ homologizations (e.g. Arndt-Eistert reaction) or functional group interconversions like Curtius rearrangement; ultimately affording homologous macrocycles or those where the connection between Modulator **B** and Bridge **C** corresponds to a urea moiety.

### SW-2: Synthesis workflow for the preparation of side-chain protected macrocycles Ia/Ib by macrolactamization in solution

As an alternative to SW-1 the protected cyclization precursor PG³-**B**-**A**-M-**c1**-CO-OPG² (wherein M is NR⁴) can also be synthesized by an inverted order of reaction steps:
a2) Arylether or arylthioether formation between a hydroxyl or mercapto-aryl/heteroaryl ester H-**A**-OPG⁴ and a suitably protected amino alcohol PG³-**B**-OH to afford PG³-**B**-**A**-OPG⁴;
b2) Deprotection of the carboxylic acid group to PG³-**B**-**A**-OH;
c2) Condensation with a C-terminally and side-chain protected building block H-M-**c1**-CO-OPG² to PG³-**B**-**A**-M-**c1**-CO-OPG².

Possible subsequent steps are as described in SW-1.

### SW-3: Synthesis workflow for the preparation of side-chain protected macrocycles Ia/Ib by macrolactamization in solution

As an alternative to SW-1(ii) macrocycles of type cyclo(**B**-**A**-M-**c1**-V-**c2**-CO-) can be synthesized by interconnecting the **C**-subunits (i.e. by formation of the V moiety) as last and macrocyclization step:
a3) Synthesis of PG¹-**A**-M-**c1**-CO-OPG² as described above (cf. al);
b3) Deprotection to H-**A**-M-**c1**-CO-OPG² (cf. b1);
c3) N-acylation of aminoalcohol H-**B**-OH with a suitably N-terminally protected amino acid PG⁵-NR**⁴**-**c2**-CO-OH to afford amido-alcohol PG⁵-NR⁴-**c2**-CO-**B**-OH (cf. a1);
d3) Arylether or arylthioether bond formation between the products of steps b3 and c3 to PG⁵-NR⁴**-c2**-CO-**B**-**A**-M-**c1**-CO-OPG² (cf. c1);
e3) Cleavage of the "main chain" protective groups to give the cyclization precursor H-NR**⁴**-**c2**-CO-**B**-**A**-M-**c1**-CO-OH (cf. d1);
f3) Macrolactamization to the side-chain protected macrocycle cyclo(**c2**-CO-**B**-**A**-M-**c1**-V-) i.e. cyclo(**B**-**A**-M-**c1**-V-**c2**-CO-) wherein M is NR⁴ and V is CONR⁴ (cf. e1 and i1).

Similar to SW-1 (iii) a third **C**-subunit could be introduced by subjecting H-**B**-OH to two successive N-acylation, N-deprotection sequences affording PG⁵-NR**⁴**-**c2**-W-**c3**-CO-**B**-OH. Subsequent ether or thioether formation and main chain deprotection (cf. d3 and e3) yield H-NR**⁴**-**c2**-W-**c3**-CO-**B**-**A**-M-**c1**-CO-OH which is finally cyclized to macrolactam *cyclo*(**B**-**A**-M-**c1**-V-**c2**-W-**c3**-CO-) wherein M is NR⁴ and both V and W are CONR⁴.

### General procedures for synthetic steps utilized in SW-1 to SW-3

In all general procedures below M represents NR⁴ or CHR⁶.

### Amidation reactions (steps a1, g1, k1, m1, c2)

An appropriately protected (preferably as acetyloxy or acetylmercapto) and optionally substituted aryl/heteroaryl carboxylic acid (PG¹-**A**-OH) is converted into the corresponding acid chloride and then condensed with a suitably protected amino acid ester H-NR⁴-**c1**-CO-OPG² in the presence of an auxiliary base (e.g. *i*-Pr₂NEt, Et₃N, pyridine, collidine) in solvents like CH₂Cl₂, CHCl₃, THF to afford PG¹-**A**-NR⁴-**c1**-CO-OPG². Alternatively this product can be obtained by amide coupling of an acyloxy or acylmercapto aryl/heteroaryl carboxylic acid (PG¹-**A**-OH) with an amino acid ester H-NR⁹-**c1**-CO-OPG² in the presence of a coupling reagent (e.g. benzotriazole derivatives like HBTU, HCTU, PyBOP; their aza analogs like HATU; or carbodiimides like EDC). Hydroxyaryl or heteroaryl carboxylic acids H-**A**-OH do not necessarily require protection of the phenolic OH-group and can directly be coupled with the H-NR⁴-**c1**-CO-OPG² to the free phenol derivative H-**A**-NR**⁴**-**c1**-CO-OPG² in the presence of a coupling reagent.

### Deprotection of aromatic hydroxy or mercapto groups (step b1)

Deacylation of PG**¹**-**A**-NR**⁴**-**c1**-CO-OPG² to the corresponding free hydroxyl or mercapto aryl/heteroaryl amide H-**A**-NR**⁴**-**c1**-CO-OPG² is achieved by aminolysis, which is advantageously carried out with a dialkylaminoalkyl amine in solvents like degassed THF at 0-25°C. Acyl amine side products formed in the course of the reaction are easily removed by extraction with acidic aqueous solutions.

### Arylether or arylthioether formation between A and B (steps c1, a2)

Alkylation of the phenol or thiophenol H-**A**-M-**c1**-CO-OPG² with a suitably N-protected amino alcohol PG³-**B**-OH to the ether or thioether PG³-**B**-**A**-M-**c1**-CO-OPG² is accomplished with azodicarboxylic acid derivatives such as DEAD, DIAD, TMAD or ADDP in the presence of trialkyl or triaryl phosphines in solvents like benzene, toluene, CH₂Cl₂, CHCl₃ or THF at 0°C to room temperature. As a variation, the reaction is induced with CMBP in toluene at temperatures of 20-110°C.

In an alternative approach, the alcohol PG³-**B**-OH is converted into the corresponding sulfonate (e.g. mesylate, tosylate or triflate) or halide (e.g. chloride, bromide or iodide) and subsequently treated with the phenol/thiophenol H-**A**-M-**c1**-CO-OPG² in the presence of an auxiliary base such as NaH or K₂CO₃ in solvents like DMF, DMSO, NMP, HMPA, or THF, to yield PG**³**-**B**-**A**-M-**c1**-CO-OPG².

### Cleavage of the main chain protective groups (steps d1, h1, l1)

Simultaneous or stepwise cleavage of the main chain protective groups provides the linear amino acids as cyclization precursors. The preferred protecting groups are Alloc as PG³ and/or allylester as PG², which can be cleaved simultaneously by palladium catalysts (e.g. Pd(PPh₃)₄) in the presence of 1,3-dimethyl barbituric acid in solvents like CH₂Cl₂ or EtOAc or mixtures thereof.

### N-Reprotection (steps f1, j1, l1)

N-Reprotection is achieved by applying standard amino acid protection protocols with chloroformates or *N*-hydroxy-succinimidyl carbonates in solvents like dioxane, and if required in the presence of a base such as aqueous Na₂CO₃.

### Macrolactamization (steps e1, i1, o1)

Macrolactamization occurs upon treatment of the cyclization precursor with coupling reagents like T3P or FDPP (if required in the presence of an auxiliary base such as *i*-Pr₂NEt) in solvents like CH₂Cl₂ or DMF under high dilution conditions and at temperatures ranging from 20 to 100°C.

Due to their synthetic importance macrolactamizations are a well-investigated class of transformations. The favorable application of FDPP as cyclization mediator is described e.g. by J. Dudash et al., Synth. Commun. 1993, 23, 349-356; or R. Samy et al., J. Org. Chem. 1999, 64, 2711-2728. Many other coupling reagents were successfully utilized in related head to tail cyclizations and might be applied instead; examples include benzotriazole derivatives like HBTU, HCTU, PyBOP; or their aza analogs such as HATU, as well as DPPA, and carbodiimides like EDC or DIC (P. Li, P.P. Roller, Curr. Top. Med. Chem. 2002, 2, 325-341; D.L. Boger et al., J. Am. Chem. Soc. 1999, 121, 10004-10011). Still another route to macrolactams relies on the intramolecular reaction of an active ester with an *in situ* released amino group (e.g. by carbamate deprotection or azide reduction) as demonstrated in the synthesis of peptide alkaloids and vancomycin model systems (U. Schmidt et al., J. Org. Chem. 1982, 47, 3261-3264; K.C. Nicolaou et al., Chem. Commun. 1997, 1899-1900).

### SW-4: Synthesis workflow for the preparation of side-chain protected macrocycles Ia/Ib by ring-closing metathesis in solution

Ring-closing metathesis (RCM) of olefinic precursors to macrocyclic compounds is well documented (e.g. A. Fürstner et al., Chem. Eur. J. 2001, 7, 4811-4820) and supplements the macrocyclization strategies described above. RCM of the examples was accomplished either between subunits of Bridge **C** or between Template **A** and Bridge **C:**

### (i) RCM between subunits of Bridge C

a4) Coupling of an optionally substituted alkenyl amine building block H-NR⁴-**c1**-CR¹²=CH₂ with the carboxylic acid derivatives PG¹-**A**-OH or H-**A**-OH to afford PG¹-**A**-NR⁴-**c1**-CR¹²=CH₂ or H-**A**-NR⁴-**c1**-CR¹²=CH₂;
b4) If required release of the aryl/heteroaryl hydroxyl or mercapto group;
c4) N-acylation of the aminoalcohol H-**B**-OH with an optionally substituted alkenyl carboxylic acid as second **C**-subunit to afford the amido alcohol H₂C=CR¹³-**c2**-CO-**B**-OH;
d4) Arylether or arylthioether formation to the cyclization precursor H₂C=CR¹³-**c2**-CO-**B**-**A**-NR⁴-**c1**-CR¹²=CH₂;
e4) Ring-closing metathesis to *cyclo*(CR¹³-**c2**-CO-**B**-**A**-NR⁴-**c1**-CR¹²=) i.e. *cyclo*(**B-A-**NR⁴**-c1**-CR¹²=CR¹³-**c2**-CO-)**.**

### (ii)RCM between Template A and Bridge C

a5) Identical to step c4 as described above;
b5) Arylether formation with an alkenyl-substituted phenol H-**A**=CH₂ to the cyclization precursor H₂C=CR¹²-**c1**-CO-**B-A**=CH₂;
c5) Ring-closing metathesis to *cyclo*(**B-A**=CR¹²-**c1**-CO-).

Optionally the olefinic double bonds of the macrocycles obtained in step e4 or step c5 can be hydrogenated to the *cyclo*(**B**-**A**-NR⁴-**c1**-CHR¹²-CHR¹³-**c2**-CO-) or *cyclo*(**B**-**A**-CHR**¹²**-**c1**-CO-).

In analogy to SW-1(ii)/(iii) it is also feasible to prepare olefinic macrocycles with extended Bridges **C** such as *cyclo*(**B-A-NR⁴-c1**-CR¹²=CR¹³**-c2**-W**-c3-**CO-), *cyclo*(**B**-**A**=CR**¹²**-**c1**-V-**c2**-CO-) or *cyclo*(**B**-**A**=CR**¹²**-**c1**-V-**c2**-W-**c3**-CO-) and subsequently the respective hydrogenated analogs.

### General procedures for synthetic steps utilized in SW-4

Apart from the RCM all steps are conducted in analogy to the transformations described for SW-1 to SW-3.

### Ring-closing metathesis (steps e4 and c5)

The ring-closing metathesis is conveniently performed in solvents like CH₂Cl₂ or toluene at temperatures of 20-100°C in the presence of indenylidene-ruthenium complexes such as dichloro-(3-phenyl-1*H*-inden-1-ylidene)bis(tricyclohexyl-phos-phine)-ruthenium(II); [1,3-bis(2,4,6-trimethylphenyl)-2-imidazolidinylidene]-dichloro-(3-phenyl-1*H*-inden-1-ylidene(tricyclohexylphosphine)-ruthenium(II); or [1,3-bis(2,4,6-trimethylphenyl)-2-imidazolidinylidene]-dichloro-(3-phenyl-1*H-*inden-1-ylidene)(pyridyl)ruthenium(II) (S. Monsaert et al., Eur. J. Inorg. Chem. 2008, 432-440 and references cited therein).

### SW-5: Synthesis workflow for derivatizations of attachment points in solution

The macrocyclic compounds obtained according to SW-1 to SW-4 can be further modified by transformations involving attachment points like, but not limited to, amino, carboxyl or hydroxyl groups. In addition, aromatic halides or sulfonates can be subjected to transition-metal catalyzed C-C or C-hetero-coupling reactions. The orthogonal protection of the attachment points allows stepwise deprotections and derivatizations which are carried out in a parallel fashion to generate substance libraries:
a6) Cleavage of the first protective group;
b6) Derivatization of the unmasked functional group;
c6) Cleavage of the second protective group;
d6) Derivatization of the liberated functional group; etc.

### General procedures for synthetic steps utilized in SW-5

### Protecting group cleavage (steps a6 and c6)

The utilized amine protecting groups (e.g. Boc, Cbz, Teoc, Alloc, Fmoc, etc.), carboxylic acid protecting groups (e.g. tert-butyl, benzyl, allyl, methyl, etc.) or alcohol protecting groups (e.g. tert-butyl, benzyl, allyl, acetyl, benzoyl, pivaloyl) are removed under standard conditions (P.G.M. Wuts, T.W. Greene, Greene's Protective Groups in Organic Synthesis, John Wiley and Sons, 4th Edition, 2006**;** P.J. Koncienski, Protecting Groups, 3rd ed., Georg Thieme Verlag 2005).

### Attachment point derivatizations (steps b6 and d6)

Derivatizations of the liberated functional groups are based on standard synthesis procedures (A. R. Katritzky et al. (eds), Comprehensive Functional Group Transformations, Pergamon, 1995**;** S. Patai, Z. Rappoport (eds), Chemistry of Functional Groups, Wiley, 1999**;** J. March, Advanced Organic Chemistry, 4 ed., Wiley, 1992**;** leading reviews for Mitsunobu reaction: O. Mitsunobu, Synthesis 1981, 1-28; D.L. Hughes, Org. Reactions; Wiley, 1992, Vol. 42; leading reviews for reductive amination/alkylation: A.F. Abdel-Magid et al., J. Org. Chem. 1996, 61, 3849; E.W. Baxter, A.B. Reitz, Org. Reactions, Wiley, 2002, Vol. 59).

Such Prototypical transformations include, but are not limited to:
(i) Amino group derivatizations such as
   - Amidations with carbonyl chlorides, carboxylic acid anhydrides, active esters; or with carboxylic acids in the presence of coupling reagents (cf. the general procedures);
   - Formation of sulfonamides with sulfonyl chlorides;
   - Reductive alkylation with carbonyl compounds; or alkylation with alkyl halides, alkylsulfonates or Michael acceptors;
   - Formation of ureas by reacting with isocyanates or their equivalents like carbamoyl chlorides or hydroxysuccinimidyl esters;
   - Transformation into thioureas with isothiocyanates or their equivalents;
   - Carbamate formation by reacting with chloroformates or their surrogates such as hydroxysuccinimidyl carbonates;
   - N-arylation to the corresponding N-aryl or N-heteroaryl derivatives with activated aromatic or heteroaromatic halides or sulfonates in the presence of an auxiliary base and/or Pd catalyst (e.g. Buchwald couplings).
(ii) Carboxyl group derivatizations like
   - Amidation with amines in the presence of a coupling reagent;
   - Esterification with alcohols.
(iii) Alcoholic hydroxyl group derivatizations as
   - Alkylation to alkyl ethers with alkyl halides or alkylsulfonates;
   - Transformation into aryl or heteroaryl ethers by reaction with (a) phenols in the presence of azodicarboxylic acid derivatives and triaryl or trialkyl phosphines (Mitsunobu type reactions); or (b) suitably activated aryl or heteroaryl halides or sulfonates;
   - Oxidation to carbonyl compounds, which in turn can be further elaborated by e.g. reductive amination, Wittig reaction or related olefination reactions, etc.;
   - Esterification with carboxylic acids or their activated surrogates.
(iv) Aryl halide or sulfonate derivatizations by e.g. Suzuki, Sonogashira, Buchwald or Negishi coupling reactions etc.

### SW-6: Synthesis workflow for derivatizations of attachment points on solid phase

As an alternative to SW-5, macrocyclic compounds **Ia/Ib** with one or more orthogonally protected exocyclic functional groups and one free primary amino group can be converted into fully derivatized products on solid support by:
a7) Attachment of the macrocyclic amine to an appropriately functionalized solid support by reductive amination;
b7) Acylation, carbamoylation, oxycarbonylation or sulfonylation of the secondary amine functionality generated in the previous step a7;
c7) Removal of the protecting group from the second attachment point;
d7) Derivatization of the liberated second functional group whereby e.g. amino groups can be alkylated or converted into amides, ureas, thioureas carbamates, or sulfonamides; and carboxylic acid moieties can be transformed into amides or esters;
e7) Repetitions of steps c7 and d7 if a third, fourth etc. attachment point is available;
f7) Release of the final product from the solid support.

In case of macrocyclic carboxylic acids the attachment to a polymer-supported amine is followed by derivatizations and release in analogy to c7 to f7:
a8) Attachment of an amine to an appropriately functionalized solid support by reductive amination;
b8) Coupling of the macrocyclic carboxylic acid to the polymer-supported amine of step a8;
c8-f8) Derivatizations and release in analogy to steps c7-f7.

### General procedures for synthetic steps utilized in SW-6

### The functionalized solid support

The solid support (polymer, resin) is preferably a derivative of polystyrene cross-linked with 1-5% divinylbenzene, of polystyrene coated with polyethyleneglycol (Tentagel^{R}), or of polyacrylamide (D. Obrecht, J.-M. Villalgordo, Solid-Supported Combinatorial and Parallel Synthesis of Small-Molecular-Weight Compound Libraries, Tetrahedron Organic Chemistry Series, Vol. 17, Pergamon 1998). It is functionalized by means of a linker, i.e. an α, ω-bifunctional spacer molecule with an anchoring group for the solid support on one end, and on the other end a selectively cleavable functional group that is used for subsequent transformations and finally for release of the product. For the examples of the present invention linkers are used that release an N-acyl (amide, urea, carbamate) or an N-sulfonyl (sulfonamide) derivative under acidic conditions. These kinds of linkers have been applied in the backbone amide linker (BAL) strategy for solid-phase synthesis of linear and cyclic peptides (K.J. Jensen et al., J. Am. Chem. Soc. 1998, 120, 5441-5452; J. Alsina et al., Chem. Eur. J. 1999, 5, 2787-2795) and heterocyclic compounds as well (T.F. Herpin et al., J. Comb. Chem. 2000, 2, 513-521; M. del Fresno et al., Tetrahedron Lett. 1998, 39, 2639-2642; N.S. Gray et al., Tetrahedron Lett. 1997, 38, 1161-1164).

Examples of such functionalized resins include DFPE polystyrene (2-(3,5-dimethoxy-4-formylphenoxy)ethyl polystyrene), DFPEM polystyrene (2-(3,5-dimethoxy-4-formylphenoxy)ethoxymethyl polystyrene), FMPB resins (4-(4-formyl-3-methoxyphenoxy)butyryl AM-resin), FMPE polystyrene HL (2-(4-formyl-3-methoxyphenoxy) ethyl polystyrene HL), FMPB NovaGel (4-(4-formyl-3-methoxy-phenoxy)butyryl NovaGel; a PEG PS resin).

### Attachment of the macrocyclic amine to the functionalized resin (steps a7 and b7) and subsequent N-acylation or N-sulfonylation

The macrocyclic primary amine is attached to the functionalized solid support by reductive amination preferably with NaBH(OAc)₃ as reducing agent in 1,2-dichloroethane and in the presence of trimethyl orthoformate.

The use of reductive aminations for such processes as well as the subsequent *N*-acylation or *N*-sulfonylation are well-documented; for example NaBH₃CN in DMF or in methanol, or NaBH(OAc)₃ in DMF/acetic acid or in dichloromethane/acetic acid have been used (cf. references cited for the functionalized solid support). The *N*-acylation is favorably conducted with carboxylic acids in the presence of coupling reagents like PyBOP, PyBroP, or HATU or with carboxylic acid fluorides/ chlorides or carboxylic acid anhydrides.

### Deprotection (steps c7)

The second attachment point is an Alloc or Fmoc protected amino group or a carboxyl group protected as allyl ester. Standard methods (cf. SW-5) are applied for their deprotection and derivatization.

### Release from the resin (step f7)

The final products are detached from the solid support by acids dissolved in organic solvents and/or H₂O. The use of TFA in dichloromethane, of TFA in dichloromethane in the presence of a scavenger such as H₂O or dimethyl sulfide, or of TFA/H₂O and TFA/H₂O/dimethylsulfide has been described (cf. references cited for the functionalized solid support).

### Attachment of the macrocyclic carboxylic acid to the functionalized resin (steps a8 and b8)

A primary amine is attached to the functionalized solid support by reductive amination preferably using NaBH(OAc)₃ in 1,2-dichloroethane in the presence of trimethyl orthoformate. Subsequent acylation with the macrocyclic carboxylic acids is favorably conducted in the presence of coupling reagents like HATU, PyBOP, or PyBroP.

It is worth mentioning that the initially attached primary amine corresponds to an attachment point derivatization of the carboxylic acid.

### SW-7: Synthesis workflow for the preparation of side-chain protected macrocycles Ia/Ib on solid support

Macrocyclic compounds of general formula **Ia/Ib** with highly variable side chain motifs in Bridge **C** can advantageously be prepared in a parallel array synthesis on solid support with an appropriately protected precursor comprising building blocks **A** and **B** as starting material. After its immobilization the precursor is coupled with one to three subunits of Bridge **C** and macrocyclizd to the product which is finally cleaved from the resin:
a9) Condensation (cf. SW-2) of a suitable hydroxy or mercapto aryl/heteroaryl carboxylic acid ester H-**A**-OPG⁴ with an appropriately *N*-protected amino alcohol PG³-**B**-OH, which is substituted with an orthogonally protected primary amino group;
b9) Removal of the protective group from the primary amine;
c9) Attachment to the solid support in a reductive alkylation step (cf. SW-6, a7) providing the polymer-supported PG'-**B-A**-OPG⁴ carrying a free secondary side chain amino group;
d9) Acylation, carbamoylation, oxycarbonylation or sulfonylation of the secondary amine (cf. SW-6, b7);
e9) Cleavage of the "main chain" amine protecting group to H-**B**-**A**-OPG⁴ which is then condensed with either (i) one **C**-subunit or (ii) two **C**-subunits:

### (i) Synthesis of a macrocycle Ia/Ib with a Bridge C consisting of two subunits

f9) Coupling of H-**B**-**A**-OPG⁴ with an appropriately protected **C-**subunit PG⁵-NR⁴-**c2**-CO-OH to PG⁵-NR⁴-**c2**-CO-**B**-**A**-OPG⁴;
g9) Cleavage of the main chain acid protective group PG⁴ affording PG⁵-NR⁴-**c2**-CO-**B**-**A**-OH;
h9) Coupling of PG⁵-NR**⁴**-**c2**-CO-**B**-**A**-OH with a suitably protected **C**-subunit H-NR⁹-**c1**-CO-OPG² to PG⁵-NR**⁴**-**c2**-CO-**B**-**A**-NR**⁴**-**c1**-CO-OPG²;
i9) Removal of the carboxylic acid protective group PG²;
j9) Removal of the amine protective group PG⁵;
k9) Macrolactamization of the linear cyclization precursor on solid support to afford cyclo(**B**-**A**-M-**c1-**V-**c2**-CO-) wherein M is NR⁴ and V is CONR⁴_{;}
19) Detachment of the final product.

### (ii) Synthesis of a macrocycle I with a Bridge C consisting of three subunits

f10) Coupling of H-**B**-**A**-OPG⁴ with an appropriately protected **C-**subunit PG⁵-NR⁴-**c3**-CO-OH to PG⁵-NR**⁴**-**c3**-CO-**B**-**A**-OPG⁴;
g10) Removal of the amine protective group PG⁵;
h10) Coupling with the second **C**-subunit PG⁵-NR**⁴**-**c2**-CO-OH to PG⁵-NR⁴-**c2**-CO-NR⁴-**c3**-CO-**B**-**A**-OPG⁴;
i10) Cleavage of the main chain acid protective group PG⁴;
j10) Coupling with the suitably protected third C-subunit H-NR⁴-**c1**-CO-OPG² to PG⁵-NR⁴-**c2**-CO-NR⁴-**c3**-CO-**B**-**A**-NR⁴-**c1**-CO-OPG²; k10) Removal of the carboxylic acid protective group PG²;
110) Removal of the amine protective group PG⁵;
m10) Macrolactamization of the linear cyclization precursor on solid support to afford *cyclo*(**B-A**-M-**c1**-V-**c2**-W-**c3**-CO-) wherein M is NR⁴ and both V and W are CONR⁴;
n10) Detachment of the final product.

Related examples for macrocyclizations (step k9 or m10) on solid support are published by C. Cabrele et al., J. Org. Chem. 1999, 64, 4353-4361.

In an alternative approach an equivalent of the product of step d9 can be obtained by reductive alkylation of the product from step b9 with a bifunctional linker such as (4-(4-formyl-3,5-dimethoxy)phenoxy)butyric acid in solution, subsequent derivatization of the resulting secondary amine and coupling to a suitable polymeric support such as aminomethyl polystyrene.

In another variation solid phase chemistry and solution chemistry can be combined. In some instances it proved advantageous to synthesize the linear cyclization precursor on solid support (product of steps j9 or 110), then to release it and to conduct the macrolactamization in solution with soluble coupling reagents as described above (cf. SW-1) or with polymer-supported coupling reagents such as *N*-cyclohexyl-carbodiimide-*N'*-methylpolystyrene or *N*-alkyl-2-chloro pyridinium triflate resin (S. Crosignani et al, Org. Lett. 2004, 6, 4579-4582).

Further viable alternatives for the synthesis of macrocycles **Ia/Ib** on solid support could involve
- Macrolactamization in other positions, e.g. between the Modulator **B** and Bridge **C** corresponding to an adaptation of SW-1 to solid phase chemistry; or
- Alternative macrocyclizations modes, e.g. similar to the RCM route of SW-4.

### Properties and usefulness

The macrocycles of type **Ia/Ib** of the present invention interact with specific biological targets. In particular, they show agonistic or antagonistic activity on the motilin receptor (MR receptor), on the serotonin receptor of subtype 5-HT_{2B} (5-HT_{2B} receptor), on the prostaglandin F2α receptor (FP receptor), on the purinoreceptor P2Y₁, on the voltage-gated potassium channel Kᵥ1.3, or on the canonical (β-catenin-dependent) Wnt pathway. Accordingly, these compounds are useful for the treatment of motility disorders of the gastrointestinal tract such as functional dyspepsia, diabetic gastroparesis or constipation type irritable bowl syndrome; of CNS related diseases like migraine, schizophrenia, psychosis, depression or Alzheimer disease; of ocular hypertension such as associated with glaucoma and of preterm labour; of CNS inflammatory disorders like multiple sclerosis; of thromboembolic disorders; of cell proliferation disorders, especially various cancer types; or of bone and cartilage-related disorders.

The macrocycles, as such or after further optimization, may be administered per se or may be applied as an appropriate formulation together with carriers, diluents or excipients well-known in the art.

When used to treat or prevent the diseases mentioned above the macrocycles can be administered singly, as mixtures of several macrocycles, or in combination with other pharmaceutically active agents. The macrocycles can be administered per se or as pharmaceutical compositions.

Pharmaceutical compositions comprising macrocycles of the invention may be manufactured by means of conventional mixing, dissolving, granulating, coated tablet-making, levigating, emulsifying, encapsulating, entrapping or lyophilizing processes. Pharmaceutical compositions may be formulated in conventional manner using one or more physiologically acceptable carriers, diluents, excipients or auxiliaries which facilitate processing of the active macrocycles into preparations which can be used pharmaceutically. Proper formulation depends upon the method of administration chosen.

For topical administration the macrocycles of the invention may be formulated as solutions, gels, ointments, creams, suspensions, etc. as are well-known in the art.

Systemic formulations include those designed for administration by injection, e.g. subcutaneous, intravenous, intramuscular, intrathecal or intraperitoneal injection, as well as those designed for transdermal, transmucosal, oral or pulmonary administration.

For injections, the macrocycles of type **Ia/Ib** may be formulated in adequate solutions, preferably in physiologically compatible buffers such as Hank's solution, Ringer's solution, or physiological saline buffer. The solutions may contain formulatory agents such as suspending, stabilizing and/or dispersing agents. Alternatively, the macrocycles of the invention may be in powder form for combination with a suitable vehicle, e.g., sterile pyrogen-free water, before use.

For transmucosal administration, penetrants appropriate to the barrier to be permeated are used in the formulation as known in the art.

For oral administration, the compounds can be readily formulated per se or by combining the active macrocycles of the invention with pharmaceutically acceptable carriers well known in the art. Such carriers enable the macrocycles of type **Ia/Ib** to be formulated as tablets, pills, dragees, capsules, liquids, gels, syrups, slurries, suspensions etc., for oral ingestion by a patient to be treated. For oral formulations such as, for example, powders, capsules and tablets, suitable excipients include fillers such as sugars, (e.g. lactose, sucrose, mannitol or sorbitol) or such as cellulose preparations (e.g. maize starch, wheat starch, rice starch, potato starch, gelatin, gum tragacanth, methyl cellulose, hydroxypropylmethyl cellulose, sodium carboxymethylcellulose); and/or granulating agents; and/or binding agents such as polyvinylpyrrolidone (PVP). If desired, desintegrating agents may be added, such as cross-linked polyvinylpyrrolidones, agar, or alginic acid or a salt thereof, such as sodium alginate. Solid dosage forms may be sugar-coated or enteric-coated using standard techniques. For oral liquid preparations such as, for example, suspensions, elixirs and solutions, suitable carriers, excipients or diluents include water, glycols, oils, alcohols, etc. In addition, flavoring agents, preservatives, coloring agents and the like may be added.

For buccal administration, the composition may take the form of tablets, lozenges, etc. formulated as usual.

For administration by inhalation, the macrocycles of the invention are conveniently delivered in form of an aerosol spray from pressurized packs or a nebulizer, with the use of a suitable propellant, e.g. hydrofluoroalkanes (HFA) such as HFA 134a (1,1,1,2,-tetrafluoroethane); carbon dioxide or another suitable gas. In the case of a pressurized aerosol the dose unit may be determined by providing a valve to deliver a metered amount. Capsules and cartridges of e.g. gelatin for use in an inhaler or insufflator may be formulated containing a powder mix of the macrocycles of the invention and a suitable powder base such as lactose or starch.

The compounds may also be formulated in rectal or vaginal compositions such as suppositories together with appropriate suppository bases like cocoa butter or other glycerides.

In addition to the formulations described above, the macrocycles of the invention may also be formulated as depot preparations. Such slow release, long acting formulations may be administered by implantation (e.g. subcutaneously or intramuscularly) or by intramuscular injection. For the manufacture of such depot preparations the macrocycles of the invention may be formulated with suitable polymeric or hydrophobic materials (e.g. as an emulsion in an acceptable oil) or with ion exchange resins, or as sparingly soluble salts.

Furthermore, other pharmaceutical delivery systems may be employed such as liposomes and emulsions. Certain organic solvents such as dimethylsulfoxide may also be employed. Additionally, the macrocycles of type **Ia/Ib** may be delivered using a sustained-release system, such as semi-permeable matrices of solid polymers containing the therapeutic agent. Various sustained-release materials have been established and are well-known by those skilled in the art. Sustained-release capsules may, depending on their chemical nature, release the compounds over a period of a few days up to several months. Depending on the chemical nature and the biological stability of the therapeutic agent, additional strategies for stabilization may be employed.

As the macrocycles of the invention may contain charged residues, they may be included in any of the above-described formulations as such or as pharmaceutically acceptable salts. Pharmaceutically acceptable salts tend to be more soluble in aqueous and other protic solvents than the corresponding free base or acid forms.

The macrocycles of the invention, or compositions thereof, will generally be used in an amount effective to achieve the intended purpose. It is understood that the amount used will depend on a particular application.

For example, the therapeutically effective dose for systemic administration can be estimated initially from in vitro assays: A dose can be formulated in animal models to achieve a circulating macrocycle concentration range that includes the IC₅₀ or EC₅₀ as determined in the cell culture (i.e. the concentration of a test compound that shows half maximal inhibitory concentration in case of antagonists or half maximal effective concentration in case agonists). Such information can be used to more accurately determine useful doses in humans. Initial dosages can also be determined from in vivo data, e.g. animal models, using techniques that are well known in the art.

Dosage amounts for applications such as gastroparesis or schizophrenia etc. may be adjusted individually to provide plasma levels of the active compound that are sufficient to maintain the therapeutic effect. Therapeutically effective serum levels may be achieved by administering multiple doses each day.

In cases of local administration or selective uptake, the effective local concentration of the macrocycles of the invention may not be related to plasma concentration.

Those having the ordinary skill in the art will be able to optimize therapeutically effective dosages without undue experimentation.

The amount of macrocycle administered will, of course, be dependent on the subject being treated, on the subject's weight, the severity of the affliction, the method of administration and the judgment of the prescribing physician.

Normally, a therapeutically effective dose of the macrocycles described herein will provide therapeutic benefit without causing substantial toxicity.

Toxicity of the macrocycles can be determined by standard pharmaceutical procedures in cell cultures or experimental animals, e.g., by determining the LD₅₀ (the dose lethal to 50% of the population) or the LD₁₀₀ (the dose lethal to 100% of the population). The dose ratio between toxic and therapeutic effect is the therapeutic index. Compounds which exhibit high therapeutic indices are preferred. The data obtained from cell culture assays and animal studies can be used in formulating a dosage range that is not toxic for use in humans. The dosage of the macrocycles of the invention lies preferably within a range of circulating concentrations that include the effective dose with little or no toxicity. The dosage may vary within the range depending upon the dosage form and the route of administration. The exact formulation, route of administration and dose can be chosen by the individual physician in view of the patient's condition (cf. E. Fingl et al. in L. Goodman und A. Gilman (eds), The Pharmacological Basis of Therapeutics, 5th ed. 1975, Ch.1, p.1).

Another embodiment of the present invention may also include compounds, which are identical to the compounds of formula **Ia/Ib,** except that one or more atoms are replaced by an atom having an atomic mass number or mass different from the atomic mass number or mass usually found in nature, e.g. compounds enriched in ²H (D), ³H, ¹¹C, ¹⁴C, ¹²⁵I etc. These isotopic analogs and their pharmaceutical salts and formulations are considered useful agents in therapy and/or diagnostics, for example, but not limited to, fine-tuning of *in vivo* half-life.

### Examples

The following examples illustrate the invention in more detail but are not intended to limit its scope in any way. Before specific examples are described in detail the used abbreviations and applied general methods are listed.
Ac: acetyl
ADDP: azodicarboxylic dipiperidide
All: allyl
Alloc: allyloxycarbonyl
AllocCl: allyl chloroformate
AllocOSu: allyloxycarbonyl-*N*-hydroxysuccinimide
AM-resin: aminomethyl resin
AM-PS: aminomethyl polystyrene
aq.: aqueous
arom.: aromatic
Bn: benzyl
BnBr: benzyl bromide
Boc: *tert*-butoxycarbonyl
br.: broad
n-Bu: n-butyl
cat.: catalytic
CEP: *N*-carboethoxyphthalimide
Cbz: benzyloxycarbonyl
CbzOSu: *N*-(benzyloxycarbonyloxy)succinimide
Cl-HOBt: 6-chloro-1-hydroxybenzotriazole
CMBP: cyanomethylenetributyl-phosphorane
m-CPBA: 3-chloroperbenzoic acid
d: day(s) or doublet (spectral)
DBU: 1,8-diazabicyclo[5.4.0]undec-7-ene
DCE: 1,2-dichloroethane
DEAD: diethyl azodicarboxylate
DFPE polystyrene: 2-(3,5-dimethoxy-4-formylphenoxy)ethyl polystyrene
DIAD: diisopropyl azodicarboxylate
DIC: *N,N'*-diisopropylcarbodiimide
DMF: dimethylformamide
DMSO: dimethyl sulfoxide
DPPA: diphenyl phosphoryl azide
DVB: divinylbenzene
EDC: 1-[3-(dimethylamino)propyl-3-ethylcarbodiimide
equiv.: equivalent
Et: ethyl
Et₃N: triethylamine
Et₂O: diethyl ether
EtOAc: ethyl acetate
FC: flash chromatography
FDPP: pentafluorophenyl diphenylphosphinate
Fmoc: 9-fluorenylmethoxycarbonyl
FmocOSu: 9-fluorenylmethoxycarbonyl-N-hydroxysuccinimide
h: hour(s)
HATU: *O*-(7-azobenzotriazol-1-yl)-*N*,*N*,*N'*,*N'*-tetramethyluronium hexafluorophosphate
HBTU: *O*-(benoztriazol-1-yl)-*N,N,N',N'*-tetramethyluronium hexafluorophosphate
HCTU: *O*-(1H-6-chlorobenoztriazol-1-yl)-N,N,N',N'-tetramethyluronium hexafluorophosphate
HL: high loading
HOAt: 1-hydroxy-7-azabenzotriazole
HOBt.H₂O: 1-hydroxybenzotriazole hydrate
HMPA: hexamethylphosphoramide
i.v.: in vacuo
m: multiplet (spectral)
MeCN: acetonitrile
MeOH: methanol
Me: methyl
NMP: 1-methyl-2-pyrrolidinone
NS: 4-nitrobenzenesulfonyl
Pd(PPh₃)4: tetrakis(triphenylphosphine)palladium(0)
PEG PS resin: polyethyleneglycol coated polystyrene resin
PG: protective group
Ph: phenyl
PPh₃: triphenylphosphine
prep.: preparative
*i*-Pr: isopropyl
*i*-Pr₂NEt: *N*-ethyl-*N*,*N*-diisopropylamine
PyBOP: (Benzotriazol-1-yloxy)tripyrrolidinophosphonium hexafluorophosphate
PyBroP: Bromotripyrrolidinophosphonium hexafluorophosphate
q: quartet (spectral)
quant.: quantitative
rt: room temperature
sat.: saturated
soln: solution
t: triplet (spectral)
TBAF: tetrabutylammonium fluoride
Teoc: 2-(trimethylsilyl)ethoxycarbonyl
TeocONp: 2-(trimethylsilyl)ethyl 4-nitrophenyl carbonate
TFA: trifluoroacetic acid
THF: tetrahydrofuran
tlc: thin layer chromatography
TMAD: tetramethylazodicarboxamide
T3P: propanphosphonic acid cyclic anhydride
p-TsOH: p-toluenesulfonic acid

### General Methods

**TLC:** Merck (silica gel 60 F254, 0.25 mm).
**Flash chromatography** (FC): Fluka silica gel 60 (0.04-0.063 mm) and Interchim Puriflash IR 60 silica gel (0.04-0.063 mm).

### I. Analytical HPLC-MS and HPLC methods:

Rₜ in min (purity at 220 nm in %), m/z [M+H]⁺
UV wave length 220 nm, 254 nm
MS: Electrospray Ionization
Volume of injection: 5 µL

### Method 1a and 1b

Column: XBridge C18 2.5 µm, 2.1x50 mm (186003085 - Waters AG) Mobile Phases: A: 0.1% TFA in Water; B: 0.085% TFA in MeCN Column oven temperature: 45°C
Gradient:

| **Time [min.]** | **Flow [µl/min]** | **%A** | **%B** |
|---|---|---|---|
| 0 | 500 | 97 | 3 |
| 0.1 | 500 | 97 | 3 |
| 3 | 500 | 3 | 97 |
| 3.6 | 500 | 3 | 97 |
| 3.7 | 500 | 97 | 3 |
| 4.3 | 500 | 97 | 3 |

MS scan range:
100 - 800 Da; centroid mode (**Method 1a**)
300 - 2000 Da; centroid mode (**Method 1b**)

scan time: 1 sec

### Method 2

Column: Gemini NX C18 3 µm, 2.1x50 mm (00B-4453-B0 - Phenomenex Inc.)
Mobile Phases: A: 0.1% TFA in Water; B: 0.085% TFA in MeCN Column oven temperature: 45°C
Gradient:

| **Time [min.]** | **Flow [µl/min]** | **%A** | **%B** |
|---|---|---|---|
| 0 | 800 | 97 | 3 |
| 0.1 | 800 | 97 | 3 |
| 2.2 | 800 | 3 | 97 |
| 2.5 | 800 | 3 | 97 |
| 2.55 | 1000 | 97 | 3 |
| 2.75 | 1000 | 97 | 3 |
| 2.8 | 800 | 97 | 3 |

MS scan range: 100 - 2000 Da; centroid mode
scan time: 1 sec

### Method 3: cf. Method 2;

Mobile Phases: A: 1 mM ammonium bicarbonate pH 10; B: MeCN

### Method 4a-4b

Column: Gemini NX C18 3 µm, 2.1x50 mm (00B-4453-B0 - Phenomenex Inc.)
Mobile Phases: A: 0.1% TFA in Water; B: 0.085% TFA in MeCN Column oven temperature: 45°C
Gradient:

| **Time [min.]** | **Flow [µl/min]** | **%A** | **%B** |
|---|---|---|---|
| 0 | 800 | 97 | 3 |
| 0.1 | 800 | 97 | 3 |
| 2.7 | 800 | 3 | 97 |
| 3 | 800 | 3 | 97 |
| 3.05 | 1000 | 97 | 3 |
| 3.25 | 1000 | 97 | 3 |
| 3.3 | 800 | 97 | 3 |

MS scan range:
100 - 2000 Da; centroid mode (**Method 4a**)
350 - 2000 Da; profile mode (**Method 4b**)

scan time: 1 sec

### Method 5a-5b: cf. Method 4a-4b;

Mobile Phases: A: 1 mM ammonium bicarbonate pH 10; B: MeCN

### Method 6-7:

Column: Acquity UPLC BEH C18 1.7 µm, 2.1x50 mm (cod. 186002350 - Waters AG)
Mobile Phases: A: 0.1% TFA in Water; B: 0.085% TFA in MeCN Column oven temperature: 55°C
Gradient:

| **Time [min.]** | **Flow [µl/min]** | **%A** | **%B** |
|---|---|---|---|
| 0 | 1250 | 97 | 3 |
| 0.05 | 1250 | 97 | 3 |
| 1.65 | 1250 | 3 | 97 |
| 1.95 | 1250 | 3 | 97 |
| 2.00 | 1250 | 97 | 3 |
| 2.30 | 1250 | 97 | 3 |

MS scan range:
100 - 1650 Da; centroid mode (**Method 6**)
100 - 1650 Da; profile mode (**Method 7**)

scan time: 0.5 sec

### Method 8: cf. Method 7;

Mobile Phases: A: 1 mM ammonium bicarbonate pH 10; B: MeCN

### Method 9a-9c:

Column: Acquity UPLC BEH C18 1.7 µm, 2.1x100 mm (cod. 186002350 - Waters AG)
Mobile Phases: A: 0.1% TFA in Water/MeCN 95/5 (v/v); B: 0.085% TFA in MeCN
Column oven temperature: 55°C
Gradient:

| **Time** [**min**.] | **Flow [µl/min]** | **%A** | **%B** |
|---|---|---|---|
| 0 | 700 | 99 | 1 |
| 0.2 | 700 | 99 | 1 |
| 2.5 | 700 | 3 | 97 |
| 2.85 | 700 | 3 | 97 |
| 2.86 | 700 | 99 | 1 |
| 3.20 | 700 | 99 | 1 |

MS scan range:
100 - 800 Da; profile mode (**Method 9a**)
100 - 1200 Da; profile mode (**Method 9b**)
200 - 1400 Da; profile mode (**Method 9c**)

scan time: 1 sec

### Method 10a-10d:

Column: Ascentis Express C18 2.7 µm, 3×50 mm (Supelco Inc.)
Mobile Phases: A: 0.1% TFA in Water; B: 0.085% TFA in MeCN
Gradient:

| **Time [min]** | **Flow [µl/min]** | **%A** | **%B** |
|---|---|---|---|
| 0 | 1300 | 97 | 3 |
| 0.5 | 1300 | 97 | 3 |
| 2.95 | 1300 | 3 | 97 |
| 3.15 | 1300 | 3 | 97 |
| 3.17 | 1300 | 97 | 3 |
| 3.2 | 1300 | 97 | 3 |

MS scan range:
95 - 1800 Da; centroid mode; Voltage: 40 V (Method **10a**)
95 - 1800 Da; profile mode; Voltage: 40 V (Method **10b**)
95 - 1800 Da; centroid mode; Voltage: 20 V (Method **10c**)
95 - 1800 Da; centroid mode; Voltage: 80 V (Method **10d**)

### Methods 11a-11c: cf. Methods 10a-10c;

Mobile Phases: A: 1 mM ammonium bicarbonate pH 10; B: MeCN

### Method 12:

Column: AtlantisT3 3 µm, 2x50 mm (Waters AG)
Mobile Phases: A: 0.1% TFA in Water; B: 0.085% TFA in MeCN
Gradient:

| **Time [min]** | **Flow [µl/min]** | **%A** | **%B** |
|---|---|---|---|
| 0 | 800 | 97 | 3 |
| 0.1 | 800 | 97 | 3 |
| 2.9 | 800 | 3 | 97 |
| 3.2 | 800 | 3 | 97 |
| 3.22 | 800 | 97 | 3 |
| 3.3 | 800 | 97 | 3 |

MS scan range: 90 - 800 Da; centroid mode; Voltage: 40 V

### Method 13:

Column: Ascentis Express C18 2.1 µm, 3x50 mm (Supelco Inc.)
Mobile Phases: A: 0.1% TFA in Water; B: 0.085% TFA in MeCN
Gradient:

| **Time [min]** | **Flow [µl/min]** | **%A** | **%B** |
|---|---|---|---|
| 0 | 1400 | 97 | 3 |
| 0.5 | 1400 | 97 | 3 |
| 1.95 | 1400 | 3 | 97 |
| 2.15 | 1400 | 3 | 97 |
| 2.18 | 1400 | 97 | 3 |
| 2.3 | 1400 | 97 | 3 |

MS scan range: 100 - 1650 Da; centroid mode; Voltage: 40 V

### General Analytical HPLC (x% CH₃CN):

Rₜ in min (purity at 220 nm in %)
   Column: Develosil RPAq 5 µM, 4.6 x 50 mm (Phenomenex Inc.)
   Flow rate: 1.5 ml/min
      0.0-0.5 min (x% CH₃CN, 100-x% H₂O containing 0.1% TFA);
      0.5-5.0 min (x% CH₃CN, 100-x% H₂O containing 0.1% TFA to 100% CH₃CN)
      5.0-6.2 min (100% CH₃CN)
Details: see individual experiment below.

### II. Preparative HPLC methods:

### 1. Reverse Phase - Acidic conditions

Column: XBridge C18 5 µm, 30 x 150 mm (Waters AG)

### Mobile phases:

**A:** 0.1% TFA in Water/Acetonitrile 95/5 v/v
**B:** 0.1% TFA in Water/Acetonitrile 5/95 v/v

### 2. Reverse Phase - Basic conditions

**Column:** XBridge C18 5 µm, 30 x 150 mm (Waters AG)

### Mobile phases:

**A:** 10 mM Ammonium Bicarbonate pH 10/Acetonitrile 95/5 v/v
**B:** Acetonitrile

### 3. Normal Phase

**Column:** VP 100/21 NUCLEOSIL 50-10, 21 x 100 mm (Macherey-Nagel AG)
**Mobile phases:**
   **A:** Hexane
   **B:** Ethylacetate
   **C:** Methanol
**NMR Spectroscopy:** Bruker Avance 300, ¹H-NMR (300 MHz) in the indicated solvent at ambient temperature. Chemical shifts δ in ppm, coupling constants J in Hz.

### Specific Examples

In the examples below and if no other sources are cited, leading reference for standard conditions of protecting group manipulations (protection and deprotection) are 1) P.G.M. Wuts, T.W. Greene, Greene's Protective Groups in Organic Synthesis, John Wiley and Sons, 4th Edition, 2006**;** 2) P.J. Koncienski, Protecting Groups, 3rd ed., Georg Thieme Verlag 2005**;** and M. Goodman (ed.), Methods of Organic Chemistry (Houben-Weyl), Vol E22a, Synthesis of Peptides and Peptidomimetics, Georg Thieme Verlag 2004**.**

### Starting materials

### Template A building blocks (Scheme 1):

2-Acetoxy-5-fluoro benzoic acid (**2**) was prepared according to the method of C.M. Suter and A.W. Weston, J. Am. Chem. Soc. 1939, 61, 2317-2318.

3-Acetoxybenzoic acid (**3**) and 4-acetoxybenzoic acid (**4**) are commercially available.

5-Hydroxy nicotinic acid (**5**) is commercially available; its conversion into **6** is described below.

8-Acetoxyquinoline-2-carboxylic acid (**8**) was prepared according to the method of R.W. Hay, C.R. Clark, J. Chem. Soc. Dalton 1977, 1993-1998.

(*S*)-2-tert-Butoxycarbonylamino-8-hydroxy-1,2,3,4-tetrahydronaphthalene-2-carboxylic acid (**10**) was prepared according to the method of M.M. Altorfer, Dissertation Universität Zurich, 1996**.**

3-Mercaptobenzoic acid (**11**) is commercially available; for its conversion into **12** see below.

Ethyl 3-hydroxybenzoate (**142**) and 3-mercaptophenol (**143**) are commercially available.

3-Hydroxythiophene-2-carboxylic acid (**144**) is commercially available; for its conversion into **145** see below. 5-Hydroxy-1,2-dimethyl-1-*H*-indole-3-carboxylic acid (**146**) is commercially available; its conversion into **147** is shown below.

3-Methoxybenzyl bromide (**148**) is commercially available; for conversion into **149** see below.

Methyl 5-bromosalicyclic acid (**151**) was prepared according to the method of T. Esumi et al. Bioorg. Med. Chem. Lett. 2004, 14, 2621-2625.

### Modulator B building blocks (Scheme 2)

Commercially available are:
tert-Butyl(3*S*,5*S*)-5-(hydroxymethyl)pyrrolidin-3-ylcarbamate (**13**) as well as the corresponding HCl salt (**13·**HCl);
tert-Butyl(3*R*,5*S*)-5-(hydroxymethyl)pyrrolidin-3-ylcarbamate (**17**) as well as the corresponding HCl salt (**17·**HCl);
(*S*)-tert-Butyl 3-(hydroxymethyl)piperazine-1-carboxylate hydrochloride (**21·**HCl); and
(*R*)-tert-Butyl 3-(hydroxymethyl)piperazine-1-carboxylate hydrochloride (**83·**HCl) (cf. Scheme 5).

(2*S*,4*S*)-Allyl 2-(hydroxymethyl)-4-((2-(trimethylsilyl)ethoxy)-carbonylamino)pyrrolidine-1-carboxylate (**16**) was prepared from amino alcohol **13** in three steps by 1) alloc protection of the secondary amino group with allyloxycarbonyl-N-hydroxysuccinimide (AllocOSu) in CH₂Cl₂, 2) cleavage of the Boc group with dioxane-HCl, and 3) Teoc protection of the primary amino group with 2-(trimethylsilyl)ethyl 4-nitrophenyl carbonate (Teoc-ONp) in CH₂Cl₂ in the presence of Et₃N) applying standard conditions.

Data of **16:** C₁₅H₂₈N₂O₅Si (344.5): Flow injection MS (APCI): 689 [2M+H]⁺), 345 ([M+H]⁺). ¹H-NMR (DMSO-d₆): 7.28 (d, J = 6.1, 1 H), 5.90 (m, 1 H), 5.25 (qd, J = 1.7, 17.2, 1 H), 5.16 (qd, J = 1.5, 10.5, 1 H), 4.90 (br. t, 1 H), 4. 54-4 . 42 (m, 2 H), 4.04-3.97 (m, 2 H), 3.90 (q, J = 6.8, 1 H), 3.80-3.66 (br. m and dd, 2 H), 3.57-3.43 (br. m, 2 H), 2.96 (br. m, 1 H), 2.19 (br. m, 1 H), 1.78 (br. m, 1 H), 0.89 (t, J ca. 8.3, 2 H), 0.00 (s, 9 H).

(2S,4R)-Allyl 2-(hydroxymethyl)-4-((2-(trimethylsilyl)ethoxy)-carbonylamino)pyrrolidine-1-carboxylate (20) was prepared from amino alcohol hydrochloride **17**·HCl, applying the same transformations as described for **16** with the exception of the Alloc protection step which was performed with allyl chloroformate in CH₂Cl₂ in the presence of aq. NaHCO₃ soln.

Data of **20:** C₁₅H₂₈N₂O₅Si (344.5) : LC-MS (method 9a): Rₜ = 1.98, 345 ([M+H]⁺); 317; 259. ¹H-NMR (DMSO-d₆): 7.26 (d, J = 6.6, 1 H), 5.89 (m, 1 H), 5.25 (br. d, J = 17.0, 1 H), 5.15 (br. d, J = 10.2, 1 H), 4.75 (m, 1 H), 4.48 (m, 2 H), 4.16-3.98 (m, 3 H), 3.82 (br. m, 1 H), 3.48-3.30 (m, 3 H), 3.21 (m, 1 H), 2.01 (m, 1 H), 1.80 (m, 1 H), 0.89 (t, J = 8.3, 2 H), 0.00 (s, 9 H).

(*S*)-1-Allyl 4-tert-butyl 2-(hydroxymethyl)piperazine-1,4-dicarboxylate (**22**) was prepared from amino alcohol hydrochloride **21**·HCl, applying allyl chloroformate in CH₂Cl₂ in the presence of aq. NaHCO₃ soln.

Data of **22**: C₁₄H₂₄N₂O₅ (300.4): LC-MS (method 9a): Rₜ = 1.70, 201 ([M+H]⁺). ¹H-NMR (DMSO-d₆): 5.90 (m, 1 H), 5.29 (qd, J = 1.7, 17.3, 1 H), 5.18 (qd, J = 1.5, 10.5, 1 H), 4.81 (t, J = 4.9, 1 H), 4.53 (d-like m, J ca. 5.1, 2 H), 4.04-3.75 (br. m, 4 H), 3.39 (m, 2 H), 2.95-2.70 (br. m, 3 H), 1.40 (s, 9 H).

(2*S*,4*R*)-2-(Trimethylsilyl)ethyl 4-(tert-butoxycarbonylamino)-2-(hydroxymethyl)pyrrolidine-1-carboxylate (**152**)

**152** was obtained by Teoc protection of the secondary amino group of **17** with 2-(trimethylsilyl)ethyl 4-nitrophenyl carbonate (Teoc-ONp) in CH₂Cl₂ in the presence of Et₃N applying standard conditions.

Data of **152:** C₁₆H₃₂N₂O₅Si (360.5). ¹H-NMR (DMSO-d₆) : 7.03 (br. s, 1 H), 4.71 (m, 1 H), 4.07-4.01 (m, 3 H), 3.76 (m, 1 H), 3.45-3.35 (br. m, 3 H), 3.11 (m, 1 H), 2.00 (m, 1 H), 1.75 (m, 1 H), 1.36 (s, 9 H), 0.91 (t, J = 7.8, 2 H), 0.00 (s, 9 H).

(2*S*,4*S*)-Allyl 2-(hydroxymethyl)-4-phenoxypyrrolidine-1-carboxylate (**156**)

BH₃-S(CH₃)₂ (5.47 mL, 57.7 mmol) was added to a soln of (2*S*,4*S*)-1-(tert-butoxycarbonyl)-4-phenoxypyrrolidine-2-carboxylic acid (**153,** 8.87 g, 28.9 mmol) in THF (170 mL). The mixture was heated to 50°C for 2 h, cooled to 0°C and treated with H₂O (100 mL), followed by an aqueous workup (CH₂Cl₂, H₂O; Na₂SO₄. FC (hexane/EtOAc) afforded **154** (8.46 g, quant. yield).

Cleavage of the Boc protective group with HCl-dioxane and introduction of the alloc protective group with allyl chloroformate in CH₂Cl₂ in the presence of sat. aq. NaHCO₃ soln afforded **156.**

Data of **156:** C₁₅H₁₉NO₄ (277.3). LC-MS (method 4a): Rₜ = 1.89 (91), 278 ([M+H]⁺). ¹H-NMR (DMSO-d₆): 7.30 (t, J = 7.9, 2 H), 6.97-6.91 (m, 3 H), 5.93 (m, 1 H), 5.29 (br. d, J = 17.5, 1 H), 5.19 (br. d, J = 10.5, 1 H), 5.01 (br. m, 1 H), 4.75 (br. m, 1 H), 4.53 (br. m, 2 H), 3.86 (br. m, 1 H), 3.78 (dd, J = 5.2, 12.1, 1 H), 3.67 (br. m, 1 H), 3.39-3.30 (br. m, 2 H), 2.24 (br. m, 2 H).

(2*S*,4*S*)-Allyl 4-(4-bromobenzyloxy)-2-(hydroxymethyl) pyrrolidine-1-carboxylate (**161**)

To a suspension of NaH (dispersion, 60% in oil; 3.8 g; 95 mmol) in dry THF (53 mL) was slowly added at 0°C a soln of (2*S*,4*S*)-1-(tert-butoxycarbonyl)-4-hydroxypyrrolidine-2-carboxylic acid (**157**; 10.0 g, 43 mmol) in THF (83 mL). The mixture was stirred for 15 min, followed by the addition of NaI (7.8 g, 52 mmol). 4-Bromobenzyl bromide (13 g, 52 mmol) in THF (32 mL) was slowly added and the mixture was stirred at rt for 15 h. The mixture was distributed between EtOAc and 0.2 M aq. HCl soln. The organic layer was separated, dried (Na₂SO₄), concentrated and purified by FC (hexane/EtOAc/MeOH gradient) to give **158** (17.3 g, 99%). The acid **158** was reduced with BH₃-S(CH₃)2 in THF, applying the procedure described for the synthesis of **154.** Cleavage of the Boc protective group with HCl-dioxane and introduction of the alloc protective group with allyl chloroformate in CH₂Cl₂ in the presence of sat. aq. NaHCO₃ soln applying standard conditions afforded **161.**

Data of **161:** C₁₆H₂₀BrNO₄ (370.2). LC-MS (method 10a): Rₜ = 2.05 (94), 370/372 ([M+H]⁺). ¹H-NMR (DMSO-d₆): 7.55 (d, J = 8.4, 2 H), 7.28 (d, J = 8.4, 2 H), 5.92 (m, 1 H), 5.28 (br. d, J ca. 17.2, 1 H), 5.18 (br. qd, J ca. 1.5, 10.5, 1 H), 4.73 (q-like m, 1 H), 4. 58-4. 42 (m, 4 H), 4.12 (br. m, 1 H), 3.78 (br. m, 1 H), 3.61-3.55 (m, 2 H), 3.43-3.29 (m, 2 H), 2.19 (m, 1 H), 2.03 (m, 1 H).

(2*S*,4*S*)-Allyl 4-benzyl-2-(hydroxymethyl)pyrrolidine-1-carboxylate (**165**) was prepared from (2*S*,4*S*)-4-benzyl-1-(tert-butoxycarbonyl)pyrrolidine-2-carboxylic acid (**162**) applying conditions as described for the synthesis of **156.**

Data of **165:** C₁₆H₂₁NO₃ (275.3). LC-MS (method 10a): Rₜ = 1.93 (91), 276 ([M+H]⁺). ¹H-NMR (DMSO-d₆): 7.29 (t, J = 7.3, 2 H), 7.21 (d, J = 7.4, 3 H), 5.90 (m, 1 H), 5.24 (br. d, J ca. 16.8, 1 H), 5.16 (dd, J = 1.2, 10.5, 1 H), 4.69 (br. s, 1 H), 4.47 (br. s, 2 H), 3.71 (br. m, 1 H), 3.59-3.53 (m, 3 H), 2.85 (m, 1 H), 2.65 (d, J = 7.2, 2 H); 2.27 (m, 1 H), 2.03 (m, 1 H), 1.57 (m, 1 H).

(2S,4R)-tert-butyl 2-(hydroxymethyl)-4-((2-(trimethylsilyl)-ethoxy)carbonylamino)pyrrolidine-1-carboxylate (**167**) was obtained from the protected amino alcohol **20** by cleavage of the alloc group (Pd(PPh₃)₄, pyrrolidine in CH₂Cl₂/EtOAc) and introduction of a Boc group (BoC₂O, CH₂Cl₂, 1 M aq. NaOH).

Data of **167:** C₁₆H₃₂NO₅Si (360.5). FI-MS: 361 ([M+H]⁺). ¹H-NMR (DMSO-d₆): 7.21 (d, J = 6.9, 1 H), 4.72 (br. m, 1 H), 4.15-3.98 (m, 3 H), 3.75 (br. m, 1 H), 3.40-3.31 (m, 3 H), 3.06 (dd, J = 6.6, 10.6, 1 H), 2.05 (br. m, 1 H), 1.78 (br. m, 1 H), 1.36 (s, 9 H), 0.89 (t, J = 8.3, 2 H), 0.00 (s, 9 H).

### Building blocks for subunits of Bridge C (Scheme 3):

### (S)-5-Allyl 1-benzyl 2-(methylamino)pentanedioate hydrochloride (27·HCl)

A mixture of Boc-L-Glu(OAll)-OH (**23**; 33 g, 115 mmol) and NaHCO₃ (27 g, 322 mmol) in DMF (500 mL) was stirred for 1 h at rt followed by slow addition of benzyl bromide (35 mL, 299 mmol) in DMF (15 mL). Stirring was continued for 16 h followed by aqueous workup (Et₂O, sat. aq. NaHCO₃ soln, sat aq. NaCl soln) and purification by FC (CH₂Cl₂/MeOH 100:0 to 98:2) to give the corresponding benzyl ester (34.4 g, 79%), which was dissolved in dioxane (40 mL) and treated with 4M HCl-dioxane (400 mL) for 1 h. The volatiles were evaporated. The residue was crystallized from Et₂O to afford **24**·HCl (23.8 g, 83%).

4-Nitrobenzenesulfonyl chloride (39 g, 178 mmol) was added at 0°C to a soln of **24**·HCl (46.5 g, 148 mmol) and pyridine (42 mL, 519 mmol) in CH₂Cl₂ (700 mL). The mixture was stirred for 15 h followed by aqueous workup (CH₂Cl₂, 1 M aq. HCl soln) and purification of the crude by FC (hexane/EtOAc 80:20 to 75:25) to yield **25** (55.54 g, 81%).

A soln of **25** (41.3 g, 89 mmol) in dry DMF (200 mL) was cooled to 0°C. Methyliodide (5.8 mL, 94 mmol) in DMF (100 mL) was slowly added, followed by a soln of DBU (14 mL, 94 mmol) in DMF (100 mL). The mixture was stirred for 4 h at rt followed by aqueous workup (EtOAc, 1 M aq. HCl soln, H₂O, sat. aq. NaHCO₃ soln, sat. aq. NaCl soln) to afford **26** (42.8 g, 99%).

A soln of **26** (17.4 g, 37 mmol) in dry, degassed CH₃CN (270 mL) was treated with thiophenol (6.7 mL, 66 mmol) and Cs₂CO₃ (39 g, 121 mmol) at rt for 16 h. The mixture was filtered and the residue was washed with Et₂O. The filtrate was carefully concentrated (bath temperature 20°C) and immediately purified by FC (hexane/EtOAc 80:20 to 50:50). The combined product fractions were carefully concentrated, immediately treated with 4M HCl-dioxane (20 mL) for 5 min and concentrated to give **27**·HCl (8.62 g, 72%).

Data of **27**·HCl: C₁₆H₂₁NO₄·HCl (291.3, free base). LC-MS (method 9b): R_{t =} 1.44, 292 ([M+H]⁺). ¹H-NMR (DMSO-d₆) : 9.57 (br. s, NH₂⁺), 7.45-7.34 (m, 5 arom. H), 5.88 (M, 1 H), 5.32-5.19 (m, 4 H), 4.53 (td, J = 1.3, 5.4, 1 H), 4.13 (br. t, J ca. 6.0, 1 H), 2.69-2.40 (m, 2 H), 2.56 (s, 3 H), 2.30-2.05 (m, 2 H).

(*R*)-5-Allyl 1-benzyl 2-(methylamino)pentanedioate hydrochloride (**29**·HCl) was prepared from Boc-D-Glu(OAll)OH (**28**) applying the methods described above for the synthesis of the enantiomer (**27**·HCl).

Data of **29**·HCl: C₁₆H₂₁NO_{4·}HCl (291.3, free base). LC-MS (method 9b): R_{t =} 1.44, 292 ([M+H]⁺). ¹H-NMR (DMSO-d₆): 9.92 (br. s, NH⁺), 9.54 (br. s, NH⁺), 7.45-7.34 (m, 5 arom. H), 5.88 (M, 1 H), 5.32-5.19 (m, 4 H), 4.53 (td, J = 1.3, 5.4, 1 H), 4.13 (br. t, J ca. 6.0, 1 H), 2.69-2.40 (m, 2 H), 2.56 (s, 3 H), 2.30-2.05 (m, 2 H).

### (S)-Allyl 2-(benzyloxycarbonylamino)-3-(methylamino)propanoate hydrochloride (32·HCl)

Cbz-L-Ser-OH (**30**) was converted into amino acid **31** by β-lactone formation and opening with HNCH₃Si(CH₃)₃ (J. Kim et al., Inorg. Chem. 1998, 37, 3835-3841), following literature procedures (J.K. Kretsinger, J.P. Schneider, J. Am. Chem. Soc. 2003, 125, 7907-7913; E.S. Ratemi, J.C. Vederas, Tetrahedron Lett. 1994, 35, 7605-7608).

A soln of **31**·HCl (2.2 g, 7.6 mmol) in allyl alcohol (55 mL) was treated with thionyl chloride (1.7 mL, 23 mmol) for 15 min at rt and for 1.5 h at 70°C. The volatiles were evaporated. The crude product was dissolved in CH₂Cl₂ and washed with aq. NaHCO₃ soln. The aqueous layer was extracted with CH₂Cl₂ and with EtOAc. The combined organic phase was dried (Na₂SO₄)_{,} filtered, and concentrated. The resulting oil (2.18 g) was dissolved in CH₂Cl₂ (80 mL), treated with 4M HCl-dioxane (20 mL), stirred for 5 min and concentrated to afford **32**·HCl (2.5 g, quant.). Data of **32**·HCl: C₁₅H₂₀N₂O₄·HCl (292.3, free base). LC-MS (method 9a): R_{t =} 1.26, 293 ([M+H]⁺). ¹H-NMR (DMSO-d₆) : 9.20 (br. s, NH⁺), 9.03 (br. s, NH⁺), 8.02 (d, J = 8.2, NH), 7.38-7.30 (m, 5 arom. H), 5.89 (m, 1 H), 5.33 (d, J = 17.3, 1 H), 5.23 (d, J = 10.5, 1 H), 5.08 (s, 2 H), 4.63 (d, J = 5.3, 2 H), 4.56 (m, 1 H), 3.35 (br. m, 1 H), 3.25 (br. m, 1 H), 2.56 (br. s, 3 H).

As an alternative, **32**·HCl was prepared from Cbz-L-Dap-OH applying the method described below for the synthesis of the enantiomer **36**·HCl.

### (R)-Allyl 2-(benzyloxycarbonylamino)-3-(methylamino)propanoate hydrochloride (36·HCl)

Cbz-D-DapOH was converted into the allylester-pTsOH salt **33**·pTsOH according to the procedure of T.M. Kamenecka, S.J. Danishefsky, Chem. Eur. J. 2001, 7, 41-63.

The amino ester **33**·pTsOH was converted into the free base by extraction (CH₂Cl₂, sat. aq. NaHCO₃ soln) and treated with 4-nitrobenzenesulfonyl chloride (1.05 equiv.) in CH₂Cl₂ in the presence of pyridine (3.0 equiv.) to give the p-nitrophenyl sulfonamide **34.**

At 0°C, a soln of methyl iodide (2.3 mL, 37 mmol) in DMF (80 mL) was added to a soln of **34** (16.4 g, 35 mmol) in DMF (80 mL). A soln of DBU (5.6 mL, 37 mmol) in DMF (80 mL) was slowly added over 2 h. The mixture was stirred at rt for 1.5 h, followed by an aqueous workup (EtOAc, 1 M HCl soln, H₂O, sat. aq. NaHCO₃ soln, sat. aq. NaCl soln) to afford **35** (17.07 g, quant.).

At 0°C, thiophenol (3.02 mL, 29.6 mmol) was added (dropwise, rapidly) to a mixture of **35** (7,85 g, 16.5 mmol) and K₂CO₃ (7.95 g, 57.5 mmol) in DMF (78 mL). The mixture was stirred for 2.5 h at 0-10°C. The mixture was diluted with EtOAc and washed with H₂O and sat. aq. NaCl soln. The organic layer was extracted with ice-cold 1 M aq. HCl soln. The aqueous phase (base extract) was poured onto aq. Na₂CO₃ soln to reach pH ca. 7; 2 M aq. NaOH soln was added to reach pH ca. 10, followed by extraction with EtOAc. The organic phase was dried (Na₂SO₄) and concentrated. The remaining oil (2.72 g) was dissolved in CH₂Cl₂ (30 mL) and treated with 4M HCl-dioxane (10 mL) to afford after evaporation of the volatiles **36**·HCl (3.34 g, 62%). Data of **36**·HCl: C₁₅H₂₀N₂O₄·HCl (292.3, free base). LC-MS (method 7): Rₜ = 0.88, 293 ([M+H]⁺). ¹H-NMR (DMSO-d₆): 9.06 (br. s, NH⁺), 8.94 (br. s, NH⁺), 8.00 (d, J = 8.3, NH), 7.38-7.30 (m, 5 arom. H), 5.88 (m, 1 H), 5.33 (d, J = 17.3, 1 H), 5.23 (d, J = 10.5, 1 H), 5.08 (s, 2 H), 4.63 (d, J = 5.3, 2 H), 4.56 (m, 1 H), 3.35 (br. m, 1 H), 3.20 (br. m, 1 H), 2.57 (br. s, 3 H).

### (S)-Allyl 2-(benzyloxycarbonylamino)-4-(methylamino)butanoate hydrochloride (40·HCl)

Cbz-L-Dab-OH (**37**) was converted into the allylester-pTsOH salt **38**·pTsOH (T.M. Kamenecka, S.J. Danishefsky, l.c.)

A mixture of **38**·pTsOH (45 g, 97 mmol) in CH₂Cl₂ (600 mL) was cooled to 0°C. MeOH (60 mL) was added, followed by ethyl trifluoroacetate (23 mL, 194 mmol). Et₃N (53 mL, 387 mmol) was added dropwise. The mixture was stirred at 0°C for 15 min, then at rt for 4 h. The volatiles were evaporated. The residue was dissolved in EtOAc, washed (1 M aq. HCl soln, sat. aq. Na₂CO₃ soln), dried (Na₂SO₄), filtered and concentrated to afford the corresponding trifluoroacetamide (32 g, 84%). N-Methylation of the acetamide (21.78 g, 56 mmol; applying CH₃I and K₂CO₃ in DMF) following the procedure described by Chu-Biao Xue et al. J. Med. Chem. 2001, 44, 2636-2660 - with the exception that the transformation was performed at rt for 4 h - afforded **39** (25 g, ca. 90%).

Treatment of **39** (8.0 g, ca. 18 mmol) in THF (80 mL) with Pd(PPh₃)₄ (0.2 g) and morpholine (8.5 mL, 98 mmol) at rt for 3 h afforded after aqueous workup (EtOAc, 1 M aq HCl soln) the corresponding trifluoroacetamido acid (7.3 g) which was treated with NH₃ (25% in H₂O; 50 mL) for 2 h and concentrated to give the corresponding amino acid (8 g). This material was dissolved in allyl alcohol (150 mL) and treated at 0°C with thionyl chloride (6.6 mL, 91 mmol). The mixture was stirred at 0°C for 15 min and at rt for 3 h and concentrated to give **40**·HCl (7.6 g, used in the next step without further purification)

Data of **40**·HCl: C₁₆H₂₂N₂O₄·HCl (306.3, free base). Flow injection MS (ESI, positive modus): 307 ([M+H]⁺). ¹H-NMR (DMSO-d₆): 8.97 (br. s, NH₂⁺), 7.92 (d, J = 7.8, NH), 7.40-7.25 (m, 5 arom. H), 5.88 (m, 1 H), 5.32 (d, J = 17.2, 1 H), 5.22 (d, J = 10.5, 1 H), 5.05 (s, 2 H), 4.60 (d, J = 5.2, 2 H), 4.22 (m, 1 H), 2.94 (m, 2 H), 2.50 (s, 3 H, superimposed by DMSO-d signal), 2.10 (m, 1 H), 2.00 (m, 1 H).

### (S)-Allyl 2-(benzyloxycarbonylamino)-5-(methylamino)pentanoate hydrochloride (44·HCl)

Cbz-L-Orn-OH (**41**) was converted into the allylester-pTsOH salt **42**·pTsOH (T.M. Kamenecka and S.J. Danishefsky, l.c.).

The ester **42**·pTsOH (5.5 g, 11 mmol) was converted into **43** (3.97 g, 83%) applying the conditions described for the synthesis of **39,** with the exception that the N-methylation was continued at rt for 8 h.

The allyl ester group was then cleaved applying the conditions described for the treatment of **39.** The saponification of the resulting trifluoroacetamido acid was performed according to the procedure of Chu-Biao Xue et al. J. Med. Chem. 2001, 44, 2636-2660, with the exception that 2 equiv. of LiOH were used. The resulting amino acid (3.80 g, containing LiCl ca. 9 mmol) was treated at rt with allyl alcohol (100 mL) and thionyl chloride (3.0 mL, 41 mmol). The mixture was heated for 2 h at 70°C. Stirring was continued at rt for 17 h. The volatiles were evaporated; and the resulting solid was washed with CH₂Cl₂ to afford **44·**HCl (3.62 g, ca. 75% w/w; yield 83%, used without further purification).

Data of **44**·HCl: C₁₇H₂₄N₂O₄·HCl (320.4, free base). LC-MS (method 9b): Rₜ = 1.48, 321 ([M+H]⁺). ¹H-NMR (DMSO-d₆) : 9.26 (br. s, NH₂⁺), 7.86 (d, J = 7.7, NH), 7.39-7.13 (m, 5 arom. H), 5.89 (m, 1 H), 5.31 (br. d, J = 17.3, 1 H), 5.20 (br. d, J = 10.4, 1 H), 5.04 (s, 2 H), 4.58 (d, J = 5.2, 2 H), 4.05 (br. m, 1 H), 2.81 (br. m, 2 H), 2.44 (s, 3 H), 1.80-1.60 (br. m, 4 H),

Sarcosine allyl ester (**46**) was prepared as p-TsOH salt (T.M. Kamenecka and S.J. Danishefsky, l.c.).

2-((Allyloxycarbonyl)(methyl)amino)acetic acid (**47**) and 3-((Allyloxycarbonyl)(methyl)amino)propanoic acid (**49**) were prepared according to the method of M. Mori et al., Chem. Pharm. Bull. 2000, 48, 716-728.

(S)-2-(Benzyloxycarbonylamino)pent-4-enoic acid (**51**) was prepared from (*S*)-allylglycine by *N*-protection (CBzOSu, dioxane, aq. Na₂CO₃) in analogy to the procedure of D.R. Ijzendoorn et al., Org. Lett 2006, 8, 239-242.

Methyl 1-aminocyclopentanecarboxylate hydrochloride (**169**·HCl) was obtained by treatment of 1-aminocyclopentanecarboxylic acid (**168**) with MeOH and SOCl₂ as described by L.V. Adriaenssens et al., J. Org. Chem. 2007, 72, 10287-10290, for the synthesis β-aminoacid methylesters hydrochlorides.

(*S*)-Methyl 3-amino-4-(naphthalen-2-yl)butanoate hydrochloride (**171**·HCl) was obtained by treatment of (*S*)-3-amino-4-(naphthalen-2-yl)butanoic acid hydrochloride (**170**·HCl) with MeOH and SOCl₂ (L.V. Adriaenssens et al., l.c.).

### (S)-Benzyl 3-(2-(allyloxy)-2-oxoethoxy)-2-(methylamino)propanoate hydrochloride (178·HCl)

Boc-Ser-OH (**172**; 4.0 g, 19.5 mmol) was converted into the allyl ether **173** according to the procedure of M.P. Glenn et al. J. Med. Chem. 2002, 45 (2), 371-381. The crude product **173** was converted into the benzyl ester **174** (4.95 g, 76% over the two steps) applying the procedure described for the synthesis of **24** with the exception that 1.7 equivalents of benzyl bromide were used and the volatiles were evaporated prior to aqueous workup. Ag₂O (9.64 g, 41.6 mmol), acetic acid (0.79 mL, 13.9 mmol) and methyliodide (17.3 mL, 277 mmol) were successively added to a soln of **174** (4.65 g, 13.9 mmol) in DMF (60 mL). The suspension was stirred for 24 h, filtered through a pad of celite and distributed between Et₂O and sat. aq. NaHCO₃ soln. The organic layer was separated, washed (H₂O, sat. aq. NaCl soln), dried (Na₂SO₄), filtered and concentrated. FC (hexane/acetone 100:0 to 90:10) yielded **175** (4.37 g used without further purification).

At rt, N-methylmorpholine-N-oxide (0.84 g, 7.2 mmol) and osmium tetroxide (2.5% w/w in tert butanol; 3.47 mL, 0.27 mmol) were added to a soln of **175** (1.93 g, ca. 4 mmol) in acetone (60 mL) and H₂O (15 mL). The mixture was stirred for 18 h. Celite (20 g) and Na₂S₂O₃ (5 g) were added and stirring was continued for 45 min. The mixture was filtered through celite. The residue was washed with acetone. The filtrate was concentrated and evaporated with toluene. FC (hexane/EtOAc 50:50 to 100:0) afforded **176** (1.3 g, 60%).

A soln of **176** (2.63 g, 6.86 mmol) in THF (50 mL) and H₂O (50 mL) was treated with NaIO₄ (2.93 g, 13.7 mmol) and stirred at rt for 1.5 h. The mixture was diluted with H₂O and extracted with CH₂Cl₂. The organic layer was dried (Na₂SO₄), filtered and concentrated. The product (2.45 g) thus obtained was dissolved in CH₂Cl₂ (25 mL). CH₃CN (25 mL) and H₂O (40 mL) were added. NaIO₄ (2.93 g, 13.7 mmol) and ruthenium(III)-chloride hydrate (77 mg, 0.34 mmol) were added. The mixture was vigorously stirred for 2 h and distributed between CH₂Cl₂ and H₂O. The organic layer was separated, washed (sat. aq. NaCl soln), dried (Na₂SO₄), filtered and concentrated. A soln of the product (2.29 g) in dry DMF (33 mL) was treated with NaHCO₃ (1.05 g, 12.5 mmol) and allyl bromide (1.05 mL, 12.5 mmol). The mixture was stirred for 3 d followed by an aqueous workup (Et₂O, H₂O, sat. aq. NaCl soln; Na₂SO₄) to afford **177** (2.37 g, 85%).

At -10°C, TFA (24 mL) was added to a soln of **177** (2.4 g, 5.9 mmol) in CH₂Cl₂ (24 mL). The soln was stirred for 1.5 h at this temperature, poured onto sat. aq. NaHCO₃ soln and extracted with CH₂Cl₂. The organic layer was separated, dried (Na₂SO₄), filtered and concentrated (bath temperature 30°C) to give 1.7 g of a light brown oil, which was dissolved in CH₂Cl₂ (20 mL) and treated with 4M HCl-dioxane (7.3 mL). The volatiles were evaporated to afford **178**·HCl (2.13 g, quant.).

Data of **178**·HCl: C₁₆H₂₁NO₅·HCl (307.3, free base). LC-MS (method 5a) : Rₜ = 1.99 (86), 308 ([M+H]⁺). ¹H-NMR (DMSO-d₆): 9.58 (br. s, NH₂⁺), 7.46-7.33 (m, 5 H), 5.92 (m, 1 H), 5.32 (qd, J = 1.6, 17.2, 1 H), 5.28 (s, 2 H), 5.23 (qd, J = 1.4, 10.5, 1 H), 4.62 (d, J = 5.5, 2 H), 4.45 (t-like s, 1 H), 4.26 (s, 2 H), 4.18-4.04 (m, 2 H), 2.63 (s, 3 H).

### (S)-Allyl 2-(benzyloxycarbonylamino)-6-(methylamino)hexanoate hydrochloride (182·HCl)

The amino ester **182**·HCl was prepared from Cbz-L-Lys-OH (**179**) applying the methods described for the synthesis of **44**·HCl.

An analytical sample of **182**·HCl was purified by prep. HPLC (method 1).

Data of **182**·HCl: C₁₈H₂₆N₂O₄·HCl (334.4, free base). LC-MS (method 10a) : Rₜ = 1.39 (98), 335 ([M+H]⁺). ¹H-NMR (DMSO-d₆) :
8.34 (br. s, NH₂⁺), 7.77 (d, J = 7.8, 1H), 7.29 (m, 5 H), 5.90 (m, 1 H), 5.31 (dd, J = 1.6, 17.2, 1 H), 5.22 (dd, J = 1.4, 10.5, 1 H), 5.07 (d, J = 12.5, 1 H), 5.01 (d, J = 12.5, 1 H), 4.59 (d, J = 5.3, 2 H), 4.05 (m, 1 H), 2.94-2.73 (m, 2 H), 2.54 (s, 3 H), 1.75-1.30 (several m, 6 H).

### (S)-Allyl 6-bromo-2-(tert-butoxycarbonylamino)hexanoate (185)

At 0°C, allyl bromide (3.9 mL, 45 mmol) was slowly added to a soln of Boc-L-6-hydroxynorleucine (**183**; 8.55 g, 34.6 mmol) and NaHCO₃ (7.26 g, 86.4 mmol) in DMF (120 mL). The mixture was stirred for 4 h at 0°C to rt, followed by the addition of more NaHCO₃ (7.26 g, 86.4 mmol) and allyl bromide (3.9 mL, 45 mmol). Stirring was continued for 18 h. The mixture was distributed between EtOAc and aq. NaCl soln. The organic phase was separated, washed (diluted aq. NaCl soln, H₂O, sat. aq. NaCl soln), dried (Na₂SO₄), filtered and concentrated. FC (hexane/EtOAc 2:1 to 1:2) afforded **184** (9.17 g, 92%).

The alcohol **184** was converted into the bromide **185** following the procedure described for the synthesis of (2*S*)-2 [(benzyloxycarbonyl)amino]-6-bromohexanoic acid methyl ester (R. Bambal and R.P. Hanzlik, J. *Org. Chem.* **1994,** *59*, 729-732). Data of **185:** C₁₄H₂₄BrNO₄ (350.2). LC-MS (method 10c): R_{t =} 2.30 (91), 352/350 ([M+H]⁺). ¹H-NMR (DMSO-d₆): 7.29 (d, J = 7.8, 1 H), 5.89 (m, 1 H), 5.32 (dd, J = 1.6, 17.2, 1 H), 5.21 (dd, J = 1.3, 10.5, 1 H), 4.59-4.55 (m, 2 H), 3.94 (m, 1 H), 3.51 (t, J = 6.6, 2 H), 1.83-1.57 (2 m, 4 H), 1.47-1.33 (m, 2 H), 1.38 (s, 9 H).

### Synthesis of (S)-allyl 5-amino-2-hydroxypentanoate (190)

(2S)-2-amino-5-phthalimidopentanoic acid hydrochloride (**187**·HCl; 17.3 g, 65%) was obtained from L-ornithine hydrochloride (**186**·HCl; 15 g, 89 mmol) and *N*-carbethoxyphthalimide (CEP; 19.5 g, 89 mmol; M.J. Bunegar et al., Org. Process Res. Dev. 1998, 2, 334-336); **187**·HCl was isolated by diluting the mixture with H₂O and 3 M aq. HCl soln until all precipitate was dissolved. The aq. soln was washed twice with EtOAc and concentrated to ca. 20% of its volume. A precipitate was formed which was collected, washed with cold H₂O and Et₂O and dried i.v.

H₂SO₄ (95%w/w; 3.4 mL, 60 mmol) was added at 0°C to a soln of **187**·HCl (4.5 g, 15 mmol) in H₂O (40 mL). The mixture was carefully warmed until all starting material was dissolved. NaNO₂ (4.2 g, 60 mmol) in H₂O (20 mL) was added at rt over 2 h. The mixture was stirred for 16 h. The precipitate formed was collected, washed with cold H₂O (10 mL) and dried i.v. to give **188** (3.37 g) which was used without further purification.

KOH (6.21 g, 111 mmol) was added to a soln of **188** (7.28 g, 28 mmol) in H₂O (100 mL). The mixture was stirred for 3 h at rt, acidified with 1 M aq. HCl soln to pH ca. 1 and heated to reflux for 1 h. The mixture was extracted with EtOAc. The aqueous phase was concentrated and dried i.v. to give crude **189** (11.5 g) which was dissolved in allyl alcohol (122 mL). SOCl₂ (8.07 mL, 111 mmol) was added at 0°C. The mixture was heated to 70°C for 2.5 h. The mixture was concentrated to afford **190**·HCl (13.17 g, ca. 80%; containing ca. 50% w/w KCl)

Data of **190**·HCl: C₈H₁₅NO₃·HCl (173.2, free base). FIA-MS: 174 ([M+H]⁺). ¹H-NMR (D₂O): 5.89 9 (m, 1 H), 5.36-5.10 (m, 2 H), ca. 4.6 (m, 2 H, partially superimposed by HDO signal), 4.30 (m, 1 H), 2.94 (t-like m, 2 H), 1.88-1.63 (m, 4 H).

### Synthesis of the A-C fragment H-A-M-c1-CO-OPG²

### Procedure A

### A.1: Acid chloride formation

Oxalyl chloride (3.5-5.0 equiv.) was added to a mixture of the acetoxyaryl carboxylic acid (Ac-**A**-OH) and dry Et₂O or CH₂Cl₂. The resulting mixture was stirred at rt for 15 min followed by the addition of a few drops (ca 50-100 µL) of dry DMF. Stirring was continued for 16 h. The mixture was filtered. The filtrate was concentrated and the residue dried i.v. to afford the crude acetoxyaryl carboxylic acid chloride (Ac-**A**-Cl), which was immediately used in the next step.

### A.2: Amide coupling

A mixture of the amino ester salt (H-NR⁴-**c1**-CO-OAll·HX), the crude acetoxyaryl carboxylic acid chloride (Ac-**A**-Cl, 1.1-1.5 equiv.) and dry CH₂Cl₂ or THF was cooled to 0°C. An auxiliary base (sym-collidine or *i*-Pr₂NEt; 3.0 equiv.) was added dropwise. The mixture was stirred at rt for 16 h. The mixture was distributed between EtOAc and 1 M aq. HCl soln. The organic phase was washed (1 M aq. HCl soln, then sat. aq. NaHCO₃ soln or sat aq. NaCl soln), dried (Na₂SO₄), filtered and concentrated. FC (hexane/EtOAc gradients) gave the acetoxyaryl amide (Ac-**A**-NR⁴-**c1**-CO-OAll).

### A.3: Deacetylation

A soln of acetoxyarylamide (Ac-**A**-NR⁴-**c1**-CO-OAll) in dry THF was treated at 0°C with 3-dimethylaminopropylamine (3.0-4.5 equiv.). The soln was stirred at rt for 1-5 h. The mixture was distributed between EtOAc and ice-cold 0.1 M or 1 M aq. HCl soln. The organic phase was washed (0.1 or 1 M aq. HCl soln, sat. aq. NaCl soln), dried (Na₂SO₄), filtered and concentrated to afford the hydroxyaryl amide (H-**A**-NR⁴-**c1**-CO-OAll).

### Synthesis of the linear cyclization precursor H-B-A-M-c1-CO-OH

### Procedure B

### B.1.1: Mitsunobu aryl ether synthesis using PPh₃/DEAD

A mixture of the hydroxyaryl amide (H-**A**-M-**c1**-CO-OAll; M = NR⁴, CHR⁶) and PPh₃ (1.5 equiv.) was dried i.v. for 15 min. Under argon a soln of alcohol (HO-**B**-Alloc, 1.2 equiv.) in dry benzene was added and the resulting soln was cooled to 0°C. A soln of DEAD (40% in toluene, 1.2 equiv.) in benzene was slowly added (by syringe pump). The mixture was stirred at rt for 18 h and concentrated. FC (hexane/EtOAc gradients) gave the protected amino acid Alloc-**B-A**-M-**c1**-CO-OAll used in the next step without further purification.

### B.1.2: Mitsunobu aryl ether synthesis using CMBP

A soln of the hydroxyaryl amide (H-**A**-M-**c1**-CO-OAll; M= NR⁴, CHR⁶), the alcohol (HO-**B**-Alloc, 1.2-1.3 equiv.) and CMBP (2 equiv.) was heated in dry toluene at reflux for 1-4 h. The soln was concentrated. FC (hexane/EtOAc gradients) afforded the protected amino acid (Alloc-**B-A**-M-**c1**-CO-OAll).

### B.2: Cleavage of the allyl/alloc protective groups

Pd(PPh₃)₄ (0.05-0.1 equiv.) was added to a mixture of the protected amino acid (Alloc-**B-A**-M-**c1**-CO-OAll) and 1,3-dimethylbarbituric acid (2.5 equiv.) in degassed EtOAc/CH₂Cl₂ (ca. 1:1). The resulting soln was stirred at rt for 1-3 h and concentrated. FC (EtOAc, CH₂Cl₂/EtOH, or CH₂Cl₂/MeOH gradients) afforded the free amino acid (H-**B-A**-M-**c1**-CO-OH).

### Synthesis of the cyclization precursor H-B-A-M-c1-V-c2-CO-OH

### Procedure C

### C.1: Allyl carbamate formation

At 0°C allylchloroformate (1.1 equiv.) was slowly added to a mixture of amino acid (H-**B-A**-M-**c1**-CO-OH) and Na₂CO₃ (1.5-3 equiv.) in dioxane/H₂O 1:1. The mixture was stirred at rt for 15 h. The mixture was diluted with EtOAc and treated with 1 M aq. HCl soln until pH ca. 2 was reached. The organic phase was separated, washed (sat. aq. NaCl soln), dried (Na₂SO₄), filtered, concentrated and dried i.v. to afford the alloc protected amino acid (Alloc-**B-A**-M-**c1**-CO-OH).

### C.2: Amide coupling

*i-*Pr₂NEt (5.0 equiv.) was slowly added to a mixture of the alloc protected amino acid (Alloc-**B-A**-M-**c1**-CO-OH), the amino acid ester salt (H-NR⁴-**c2**-CO-OAll·p-TsOH, 1.2 equiv.), HOAt (1.5 equiv.) and HATU (1.5 equiv.) in DMF. The mixture was stirred at rt for 20 h followed by distribution between EtOAc and ice-cold 0.5 M aq. HCl soln. The organic phase was washed (0.5 M aq. HCl soln, H₂O, sat. aq. NaHCO₃ soln, sat. aq. NaCl soln), dried (Na₂SO₄), filtered and concentrated. FC (hexane/EtOAc gradients) afforded the protected amino acid (Alloc-**B-A**-M-**c1**-V-**c2**-CO-OAll; V= CONR⁴).

### C.3: Cleavage of the allyl/alloc protective groups

Pd(PPh₃)₄ (0.1 equiv.) was added to a mixture of the protected amino acid (Alloc-**B**-**A**-M-**c1**-V-**c2**-CO-OAll) and 1,3-dimethylbarbituric acid (2.5 equiv.) in degassed EtOAc/CH₂Cl₂ 1:1. The resulting soln was stirred at rt for 1-2 h and concentrated. FC (EtOAc, CH₂Cl₂/EtOH, or CH₂Cl₂/MeOH gradients) afforded the free amino acid (H-**B-A**-M-**c1-**V-**c2**-CO-OH).

### Synthesis of the C-B fragment PG⁵-NR⁴-c2-CO-B-OH

### Procedure D

### Synthesis in two steps, via amidoester and subsequent saponification

*i*-Pr₂NEt (5.0 equiv.) was slowly added to a mixture of the N-protected amino acid (Alloc-NR⁴-**c2**-CO-OH, 2.2 equiv.), the aminoalcohol hydrochloride HO-**B**-H·HCl, Cl-HOBt (0.25 equiv.) and HCTU (2.5 equiv.) in DMF. The resulting soln was stirred at rt for 17 h, followed by distribution between EtOAc and sat. aq. Na₂CO₃ soln. The organic phase was washed (1 M aq. HCl soln, sat. aq. NaCl soln), dried (Na₂SO₄), filtered and concentrated. FC (hexane/EtOAc or CH₂Cl₂/MeOH gradients) afforded the corresponding amidoester, which was dissolved in THF/H₂O 4:1 and treated with lithium hydroxide monohydrate (3.0 equiv.) for 2 h at rt. The mixture was concentrated to about 50% of the original volume, diluted with EtOAc and extracted with 1 M aq. NaOH soln. The organic phase was washed (H₂O, sat. aq. NaCl soln), dried (Na₂SO₄), filtered and concentrated to afford the amidoalcohol (HO-**B**-CO-**c2**-NR⁴-Alloc).

### Synthesis of cyclization precursor H-NR⁴-c2-CO-B-A-M-c1-CO-OH

### Procedure E

### E.1.1: Mitsunobu aryl ether synthesis using PPh₃/DEAD

A mixture of the hydroxyaryl amide (H-**A**-M-**c1**-CO-OAll) and PPh₃ (1.5-4.5 equiv.) was dissolved in benzene. The soln was concentrated and the residue was dried i.v. for 15-30 min. Under argon, a soln of alcohol HO-**B**-CO-**c2**-NR⁴-Alloc (1.2-2.3 equiv.) in dry and degassed benzene was added and the resulting mixture was cooled to 0°C. A soln of DEAD (40% in toluene, 1.2-4.5 equiv.) was slowly added. The mixture was stirred at rt for 18 h. In case of incomplete consumption of the hydroxyaryl amide as indicated by tlc control, additional triphenylphosphine (1.0-1.3 equiv.) and DEAD (40% in toluene, 1.0 equiv.) and alcohol (1.0 equiv.) were added and stirring was continued for 18 h. The mixture was concentrated. FC (hexane/EtOAc, CH₂Cl₂/EtOH, or CH₂Cl₂/MeOH gradients) afforded Alloc-NR⁴-**c2**-CO-**B-A**-M-**c1**-CO-OAll used in the next step without further purification.

### E.1.2: Mitsunobu aryl ether synthesis using CMBP

CMBP (2-3 equiv.) was added to a mixture of the hydroxyaryl amide (H-**A-M**-**c1**-CO-OAll) and the alcohol (HO-**B**-CO-**c2**-NR⁴-Alloc, 1.2-2.2 equiv.) in dry toluene. The mixture was heated at reflux for 16 h and concentrated.

### FC (hexane/EtOAc gradients) afforded the protected amino acid (Alloc-NR⁹-c2-CO-B-A-M-c1-CO-OAll).

### E.2: Cleavage of the allyl/alloc protective groups

Pd(PPh₃)₄ (0.05-0.1 equiv.) was added to a mixture of the protected amino acid (Alloc-NR**⁴**-**c2**-CO-**B**-**A**-M-**c1**-CO-OAll) and 1,3-dimethylbarbituric acid (2.4 equiv.) in degassed EtOAc/CH₂Cl₂ 1:1. The resulting soln was stirred at rt for 1-3 h and concentrated. FC (EtOAc, CH₂Cl₂/EtOH, or CH₂Cl₂/MeOH gradients) afforded the free amino acid (H-NR⁴-**c2**-CO-**B-A**-M-**c1**-CO-OH).

### Synthesis of macrocyclic compounds cyclo(B-A-M-c1-CO-) and cyclo(B-A-M-c1-V-c2-CO-)

### Procedure F

The macrolactamization was typically performed at final concentrations ranging from 0.01 M to 0.001 M.

### F.1.1: T3P mediated lactam formation

A soln of the precursor (H-**B**-**A**-M-**c1-**CO-OH, H-**B-A**-M-**c1**-V-**c2**-CO-OH or H-NR⁴-**c2**-CO-**B-A**-M-**c1**-CO-OH) in dry CH₂Cl₂ was added within 2 h by syringe pump to a soln of T3P (50% in EtOAc, 2 equiv.) and *i*-Pr₂NEt (4 equiv.) in dry CH₂Cl₂. The soln was stirred at rt for 20 h, extracted with sat. aq. Na₂CO₃ soln and with H₂O, dried (Na₂SO4), filtered and then concentrated. FC (hexane/EtOAc/MeOH or CH₂Cl₂/MeOH gradients) afforded macrocycle *cyclo*(**B**-**A**-M-**c1**-CO-) or *cyclo*(**B**-**A**-M-**c1**-V-**c2**-CO-) wherein V is NR⁴.

### F.1.2: FDPP mediated lactam formation

A soln of the precursor (H-**B-A**-M-**c1**-CO-OH, H-**B-A**-M-**c1**-V-**c2**-CO-OH or H-NR⁴-**c2**-CO-**B-A**-M-**c1**-CO-OH) in dry DMF was added within 2 h to a soln of FDPP (2.0 equiv.) in dry DMF. The soln was stirred at rt for 20 h. The volatiles were evaporated and the residue taken up in EtOAc and washed (sat. aq. NaHCO₃ soln, H₂O, sat. aq. NaCl soln). The organic phase was dried (Na₂SO₄), filtered and concentrated. FC (hexane/EtOAc/MeOH or CH₂Cl₂/MeOH gradients) afforded the macrocycle *cyclo*(**B**-**A**-M-**c1**-CO-) or *cyclo*(**B**-**A**-M-**c1**-V-**c2**-CO- wherein V is NR⁴.

### Attachment of substituents to the macrocyclic core structures: Synthesis of the final products

### Deprotection reactions

### Procedure H

A soln of a macrocyclic benzyl ester or benzyl ether in MeOH or MeOH/THF (ca 100 mL per g of starting material) was hydrogenolyzed for 2 h at rt and at normal pressure in the presence of palladium hydroxide on activated charcoal (moistened with 50% H₂O; 0.5 g per g of starting material). The mixture was filtered through a pad of celite. The residue was washed (MeOH, MeOH/CH₂Cl₂ 1:1, THF). The combined filtrate and washings were concentrated to obtain a macrocyclic acid or alcohol.

### Procedure I

### I.1: Teoc deprotection with dioxane-HCl

A soln of a macrocyclic Teoc-amine (1.5 mmol) in dioxane (18 mL) was treated with 4M HCl in dioxane (18 mL) and stirred at rt for 4-16 h. The mixture was treated with Et₂O and filtered. The solid was washed with Et₂O and dried i.v. to give the macrocyclic amine hydrochloride.

### I.2: Teoc deprotection with TBAF in THF

A soln of TBAF (1 M in THF, 3 equiv.) was added at 0°C to a soln of a macrocyclic Teoc-amine (1.3 mmol) in THF (34 mL). Stirring at 0°C to rt was continued for 3 h. The soln was distributed between CH₂Cl₂ and H₂O. The organic phase was washed (H₂O), dried (Na₂SO₄), filtered and concentrated to provide after FC the macrocyclic amine.

### Procedure J

A soln of a macrocyclic Boc-amine in dioxane (10 mL per g of starting material) was treated with 4M HCl in dioxane (20 mL per g of starting material) and stirred at rt for 2 h. The mixture was filtered. The solid was washed with Et₂O and dried i.v. to give the macrocyclic amine hydrochloride.

### Procedure K

**K.1:** A soln of a macrocyclic benzylcarbamate (0.9 mmol) in MeOH (52 mL) was hydrogenolyzed for 4 h at rt and at normal pressure in the presence of palladium hydroxide on activated charcoal (moistened with 50% H₂O; 0.3 g). The mixture was filtered through a pad of celite. The residue was washed (MeOH). The combined filtrate and washings were concentrated to obtain the macrocyclic amine.
**K.2:** A soln of a macrocyclic benzylcarbamate (0.16 mmol) in MeOH (2 mL) was treated with NH₃ soln (7 M in MeOH; 4 equiv.) and hydrogenolyzed for 3 h at rt and at normal pressure in the presence of 5% palladium on activated charcoal (moistened with 50% H₂O; 32 mg). The mixture was filtered through a pad of celite. The residue was washed (MeOH). The combined filtrate and washings were concentrated to give the macrocyclic amine.
**K.3:** A soln of the macrocyclic benzylamine (0.7 mmol) in acetic acid (11 mL) was hydrogenolyzed for 15 h at rt and at normal pressure in the presence of palladium hydroxide on activated charcoal (moistened with 50% H₂O; 0.14 g). The mixture was filtered through a pad of celite. The residue was washed (MeOH). The combined filtrate and washings were concentrated to obtain the macrocyclic amine.

### Acylation, carbamoylation, sulfonylation, and alkylation reactions

### Procedure L

### L.1: Amide coupling of a macrocyclic amine with

### L.1.1: carboxylic acid anhydrides or acylchlorides

At 0°C a soln of an amino macrocycle (free amine or hydrochloride; 0.09 mmol) in CH₂Cl₂ (1 mL) was successively treated with pyridine (10 equiv.) and the carboxylic acid anhydride (1.05-5 equiv.) or a carboxylic acid chloride (1.05-2.0 equiv.). The soln was stirred at rt for 15 h. After the addition of MeOH (0.1 mL) the soln was stirred for 10 min and concentrated. The resulting crude product was co-evaporated with toluene and purified by chromatography (FC, normal phase or reverse phase prep. HPLC) to give an *N*-acylamino macrocycle.

### L.1.2: carboxylic acid and polymer-supported carbodiimide

A soln of an amino macrocycle (free amine or hydrochloride; 0.09 mmol), a carboxylic acid (1.2 equiv.), HOBt·H₂O (1.2 equiv.) in CH₂Cl₂ (1 mL) was treated with N-cyclohexyl-carbodiimide-*N'*-methylpolystyrene (1.9 mmol/g; 1.5 equiv.) and *i*-Pr₂NEt (3.0 equiv.). The mixture was stirred for 15 h at rt. (Polystyrylmethyl)trimethylammonium bicarbonate (3.5 mmol/g; 3 equiv.) was added and stirring was continued for 1 h. The mixture was diluted with CH₂Cl₂/MeOH 9:1 (2 mL) and filtered. The polymer was washed twice with CH₂Cl₂/MeOH 8:2 (5 mL). The combined filtrate and washings were concentrated. Purification of the crude product by chromatography (FC, normal phase or reverse phase prep. HPLC) afforded an *N*-acylamino macrocycle.

### L.1.3: carboxylic acid and HATU

A soln of an amino macrocycle (free amine or hydrochloride; 0.145 mmol), a carboxylic acid (2.0 equiv.), HATU (2.0 equiv.), HOAt (2.0 equiv.) in DMF (2 mL) was treated with *i*-Pr₂NEt (4.0 equiv.). The mixture was stirred for 15 h at rt. The solvent was removed. The residue was distributed between CHCl₃ and sat. aq. NaHCO₃ soln. The organic phase was washed (H₂O), dried (Na₂SO₄), filtered and concentrated. Purification of the crude product by chromatography (FC, normal phase or reverse phase prep. HPLC) afforded an *N*-acylamino macrocycle.

### L.2: Amide coupling of a macrocyclic carboxylic acid with an amine and HATU

A soln of a macrocyclic carboxylic acid (0.78 mmol), an amine (2.0 equiv.), HATU (2.0 equiv.), HOAt (2.0 equiv.) in DMF (6 mL) was treated with *i*-Pr₂NEt (4.0 equiv.). The mixture was stirred for 15 h at rt. The solvent was removed. The residue was distributed between CHCl₃ and sat. aq. NaHCO₃ soln. The organic phase was washed (H₂O), dried (Na₂SO₄), filtered and concentrated. Purification of the crude product by chromatography (FC, normal phase or reverse phase prep. HPLC) afforded a macrocyclic amide.

### L.3: Sulfonamide formation with sulfonyl chlorides

At 0°C a soln of an amino macrocycle (0.24 mmol) in CH₂Cl₂ (1 mL) was successively treated with triethylamine (2.0 equiv.) and the sulfonyl chloride (1.0 equiv.). The mixture was stirred at 0°C to rt for 0.5-16 h, followed by an aqueous workup (CHCl₃, sat. aq. Na₂CO₃ soln; Na₂SO₄). The crude product was purified by chromatography (FC, normal phase or reverse phase prep. HPLC) to afford the macrocyclic sulfonamide.

### L.4: Urea formation with isocyanates or equivalents of isocyanates

A soln of an amino macrocycle (0.1 mmol) in CH₂Cl₂ was treated at rt with an isocyanate (1.1 equiv.) or with a succinimidyl carbamate (1.1 equiv.) and *i*-Pr₂NEt (3 equiv.). Aqueous workup (CH₂Cl₂, sat. aq. Na₂CO₃ soln; Na₂SO₄) and purification by chromatography (FC, normal phase or reverse phase prep. HPLC) afforded the targeted urea.

### N-Alkylation by reductive amination

### Procedure M

### M.1: N,N-Diethylamino macrocycles by reductive amination

At 0°C NaBH(OAc)₃ (5 equiv.) and acetaldehyde (1 mL) were added to a soln of the amino macrocycle (free amine or hydrochloride; 0.09 mmol) in THF (1 mL). The mixture was stirred at 0°C to rt for 15 h. The mixture was diluted with CHCl₃ and washed with sat. aq. NaHCO₃ soln. The organic phase was dried (Na₂SO₄), filtered and concentrated. Purification of the crude product by chromatography (FC, normal phase or reverse phase prep. HPLC) afforded the diethylamino macrocycle.

### M.2: Synthesis of secondary amines by reductive amination

Activated molecular sieve powder (3 Å; 1 mg per mg of starting material) was added at rt to a soln of an amino macrocycle (0.8 mmol) and an aldehyde (1.1 equiv.) in THF (3.5 mL). The suspension was stirred for 4 h at rt, followed by the addition of acetic acid (1.1 equiv.) and NaBH(OAc)₃ (2.0 equiv.). The mixture was stirred for 18 h and filtered. Aqueous workup of the filtrate (CH₂Cl₂, sat. aq. Na₂CO₃ soln; Na₂SO₄) and purification of the crude product by chromatography (FC, normal phase or reverse phase prep. HPLC) afforded the alkylamino macrocycle.

### M.3: Synthesis of tertiary amines by N-methylation of secondary amines

At 0°C formaldehyde soln (36.5% in H₂O; 5 equiv.) and NaBH(OAc)₃ (2.5 equiv.) were added to a soln of the macrocyclic amine (0.16 mmol) in THF (3 mL). The mixture was stirred at rt for 3 h followed by aqueous workup (CH₂Cl₂, sat. aq. Na₂CO₃ soln; Na₂SO₄). Purification of the crude product by chromatography (FC, normal phase or reverse phase prep. HPLC) afforded the desired N-methyl-N-alkylamino macrocycle.

If the HCl salt of the macrocyclic amine was used, the transformation was carried out in the presence of 2 equiv. of *i*-Pr₂NEt.

### M.4: Synthesis of tertiary amines by debenzylation/ N-methylation

A soln of a tert. benzylamine (0.1 mmol) in MeOH (2.0 mL) / acetic acid (0.5 mL) was hydrogenolyzed for 2 h at rt and normal pressure in the presence of palladium hydroxide on activated charcoal (moistened with 50% H₂O; 20 mg). Aq. formaldehyde soln (36.5% in H₂O; 10 equiv.) was added and hydrogenation was continued for 1 h.

The mixture was filtered through a pad of celite. The residue was washed (MeOH). The combined filtrate and washings were concentrated followed by aqueous workup (CHCl₃, sat. aq. Na₂CO₃ soln; Na₂SO₄). The crude product was purified by chromatography (FC, normal phase or reverse phase prep. HPLC) to afford a tertiary methylamino macrocycle.

### M.5: Synthesis of tertiary amines by reductive amination of secondary amines

*i*-Pr₂NEt (2 equiv.) was added at 0°C to a mixture of the macrocyclic amine (0.15 mmol) and DCE (1.0 mL). The aldehyde (1.2 equiv.) was added. The mixture was stirred for 0.5 h, followed by the addition of NaBH(OAc)₃ (3 equiv.). Stirring was continued at rt for 15 h. After aqueous workup (CHCl₃, 1 M aq. Na₂CO₃ soln; Na₂SO₄) the crude product was purified by chromatography (FC, normal phase or reverse phase prep. HPLC) to afford the macrocyclic tertiary amine.

### Miscellaneous transformations

### Procedure N: Methylester cleavage

A soln of the methylester (57 µMol) in THF (1.5 mL) and MeOH (0.5 mL) was treated with H₂O (0.5 mL) and lithium hydroxide monohydrate (3 equiv.) for 2 h at rt. The mixture was acidified by addition of aq. 1 M HCl and concentrated. The crude product was purified by chromatography (FC, normal phase or reverse phase prep. HPLC).

### Procedure O: Conversion of macrocyclic primary amines into piperazines by N-alkylation

A mixture of the amino macrocycle (1.4 mmol) and N-benzyl-2-chloro-N-(2-chloroethyl)ethanamine (1.5 equiv.) in ethanol (15 mL) was treated with NaHCO₃ (4 equiv.) and heated to reflux for 3 h. Aqueous workup (EtOAc, half-sat. aq. NaHCO₃ soln; Na₂SO₄) and chromatography (FC, normal phase or reverse phase prep. HPLC) afforded the N-benzylpiperazino macrocycle.

### Procedure P: Suzuki couplings

A soln of Na₂CO₃ (3.0 equiv.) in H₂O (1.6 mL) was added to a mixture of the macrocyclic arylbromide (0.25 mmol), the boronic acid (2.0 equiv.) and Pd(PPh₃)₄ (0.1 equiv.) in DME (5.2 mL). The mixture was heated to reflux for 1 h, followed by aqueous workup (EtOAc, sat. aq. Na₂CO₃ soln; Na₂SO₄). Purification of the crude product by chromatography (FC, normal phase or reverse phase prep. HPLC) afforded the biaryl-substituted macrocyclic product.

### Procedure Q: O-Alkylation of macrocyclic alcohols

At 0°C NaH (60% in oil, 2 equiv.) was added portionwise to a soln of the macrocyclic alcohol (0.38 mmol) and the alkylhalide (1.1 equiv.) in THF (3.7 mL)/DMF (1.3 mL). The mixture was stirred at rt for 2 h, treated with 1 M aq. HCl soln and extracted with EtOAc. The organic phase was dried (Na₂SO₄), filtered and concentrated. Purification of the crude product by chromatography (FC, normal phase or reverse phase prep. HPLC) afforded the macrocyclic alkyl ether.

### Procedures R and S: Vide infra "Transformations on solid support"

### Synthesis of the A-B fragments PG³-B-A-OH and PG³-B-A=CH₂

### 1. Synthesis of 3-(((2S,4S)-1-(allyloxycarbonyl)-4-(tert-butoxycarbonylamino)pyrrolidin-2-yl)methoxy)benzoic acid (192) (Scheme 4)

A soln of ethyl 3-hydroxybenzoate (**142**; 3.1 g, 18.6 mmol), alcohol **14** (5.66 g, 18.8 mmol) and CMBP (9.0 g, 37.3 mmol) in toluene (60 mL) was heated to reflux for 2 h. Evaporation of the volatiles and FC (hexane/EtOAc) gave **191** (7.3 g, 87%).

A soln of ester **191** (4.3 g, 9.5 mmol) in THF/MeOH/H₂O (3:1:1; 55 mL) was cooled to 0°C, treated with LiOH·H₂O (801 mg, 19.1 mmol) and stirred at 0°C to rt for 16 h. The mixture was treated with 1 M aq. NaH₂PO₄ soln (100 mL) and repeatedly extracted with CH₂Cl₂. The combined organic layer was dried (Na₂SO₄), filtered and concentrated to yield **192** (3.86 g, 96%). Data of **192:** C₂₁H₂₈N₂O₇ (420.5). LC-MS (method 10c): Rₜ = 1.97 (97), 421 ([M+H]⁺).

### 2. Synthesis of (2S,4S)-4-(4-bromobenzyloxy)-2-((3-(but-3-enyl)phenoxy)methyl)pyrrolidine (194)

### 2.1 Synthesis of 3-(But-3-enyl)phenol (149)(Scheme 1)

3-Methoxybenzyl bromide (**148**; 3.3 g, 16.3 mmol) was converted into 1-(but-3-enyl)-3-methoxybenzene (2.3 g, 86%) according to the procedure of A.B. Smith et al., Org. Lett. 2005, 7, 315-318, describing the synthesis of (3-but-enyl-phenoxy)-tert-butyl dimethylsilane.

At 0°C, BBr₃ (0.775 mL, 7.9 mmol) was slowly added to a soln of 1-(but-3-enyl)-3-methoxybenzene (1.36 g, 8.4 mmol) in dry CH₂Cl₂ (48 mL). Stirring at 0°C was continued for 3 h, then 1 M aq. NaHCO₃ soln (20 mL) was added and stirring continued for 30 min. The mixture was diluted with CH₂Cl₂ (100 mL) and 1 M aq. NaHCO₃ soln (100 mL). The organic layer was separated. The aqueous layer was extracted with CH₂Cl₂. The combined organic layer was dried (Na₂S₄) , filtered and concentrated. FC (hexane/EtOAc 98:2 to 80:20) afforded **149** (896 mg, 72%).

Data of **149:** C₁₀H₁₂O (148.2). ¹H-NMR (DMSO-d₆): 9.21 (s, OH), 7.04 (t, J = 7.5, 1 H), 6.76-6.55 (m, 3 H), 5.82 (m, 1 H), 5.06-4.93 (m, 2 H), 2.59 (t, J = 7.7, 2 H), 2.30 (q-like m, 1 H).

### 2.2 Synthesis of amine 194 (Scheme 4)

A mixture of phenol **149** (1.095 g, 7.3 mmol), alcohol **161** (2.28 g, 6.16 mmol) and PPh₃ (2.42 g, 9.24 mmol) was dissolved in CHCl₃ (46 mL) and degassed. ADDP (2.33 g, 9.24 mmol) was added and the mixture was stirred for 20 h at rt. The mixture was diluted with CH₂Cl₂, washed with sat. aq. NaHCO₃ soln, dried (Na₂SO₄), filtered and concentrated. FC (hexane/EtOAc gradient) afforded **193** (2.94 g, 95%).

To a degassed soln of **193** (200 mg, 0.4 mmol) in CH₂Cl₂/EtOAc (1:1, 5.2 mL) were added Pd(PPh₃)₄ (2.5 mg) and 1,3-dimethylbarbituric acid (125 mg, 0.8 mmol). The mixture was stirred for 45 min at rt followed by an aqueous workup (CH₂Cl₂, sat. aq. Na₂CO₃ soln; Na₂SO₄) and FC (EtOAc/MeOH gradient) to yield **194** (160 mg, 96%).

Data of **194:** C₂₂H₂₆BrNO₂ (416.3). LC-MS (method l0a) : Rₜ = 2.00 (82), 416/418 ([M+H]⁺). ¹H-NMR (DMSO-d₆): 7.51 (d, J = 8.3, 2 H), 7.28 (d, J = 8.4, 2 H), 7.17 (t, J = 7.8, 1 H), 6.79-6.70 (m, 3 H), 5.83 (m, 1 H), 5.03 (qd, J = 1.7, 17.2, 1 H), 4.96 (d-like m, J ca. 10.2, 1 H), 4.42 (s, 2 H), 4.11 (m, 1 H), 3.91 (d, J = 6. 3, 2 H), 3.45 (m, 1 H), 2.97 (d, J = 4. 3, 2 H), 2.63 (t, J ca. 7.7, 2 H), 2.31 (q-like m, 2 H), 2.15 (m, 1 H), 1.66 (m, 1 H).

### 3. Synthesis of 5-bromo-2-(((2S,4R)-4-(tert-butoxycarbonylamino)-1-((2-(trimethylsilyl)ethoxy)carbonyl)pyrrolidin-2-yl)-methoxy)benzoic acid (196) (Scheme 4)

At 0°C DEAD (40% in toluene; 3.5 mL, 7.6 mmol) was slowly added to a mixture of phenol **151** (1.76 g, 7.6 mmol), alcohol **152** (1.83 g, 5.1 mmol) and PPh₃ (2.0 g, 7.6 mmol) in benzene (50 mL). The mixture was stirred at rt for 16 h, followed by evaporation of the volatiles and FC (hexane/EtOAc 2:1) to yield **195** (2.78 g, 95%).

A soln of ester **195** (2.6 g, 4.5 mmol) in THF (20 mL) was treated with 2M aq. LiOH soln (22.5 mL, 45 mmol). The mixture was heated to 50°C for 15 h. The organic solvent was evaporated. The remaining aqueous phase was acidified with 3 M aq. HCl soln and extracted with EtOAc. The organic phase was dried (Na₂SO₄), filtered and concentrated to give **196** (2.44 g, 96%).

Data of **196:** C₂₃H₃₅BrN₂O₇Si (559.5). LC-MS (method 10a): Rₜ = 2.49 (98), 559/561 ([M+H]⁺).

### 4. Synthesis of 3-(((2S,4S)-1-(allyloxycarbonyl)-4-(4-bromobenzyloxy)pyrrolidin-2-yl)methoxy)benzoic acid (198) (Scheme 4)

TMAD (5.0 g, 28 mmol) in degassed benzene (100 mL) was slowly added at 0°C to a soln of ethyl 3-hydroxybenzoate (**142**; 4.7 g, 28 mmol), alcohol **161** (6.92 g, 19 mmol) and PPh₃ (7.4 g, 28 mmol) in benzene (300 mL). The resulting suspension was diluted with benzene (100 mL) and stirred at rt for 20 h. Et₂O was added and the mixture was filtered. The filtrate was concentrated and purified by FC (hexane/EtOAc 75:25 to 70:30) to afford **197** (6.24 g, 64%).

A soln of **197** (883 mg, 1.7 mmol) in THF (5 mL) was treated with 2 M aq. LiOH soln (3 mL, 6 mmol) and MeOH (2.5 mL) for 2 h at rt. The volatiles were partially evaporated. The remaining soln was acidified with 1 M aq. HCl soln and extracted with EtOAc. The organic phase was dried (Na₂SO₄), filtered and concentrated to yield **198** (770 mg, 92%).

Data of **198:** C₂₃H₂₄BrNO₆ (490.3). LC-MS (method lOa): Rₜ = 2.30 (98), 492/490 ([M+H]⁺).

### Synthesis of the A-C fragments H-A-M-c1-CO-OPG² and H-A-M-c1-CR¹²=CH₂

### 1. Synthesis of (S)-5-allyl 1-benzyl 2-(5-fluoro-2-hydroxy-N-methylbenzamido)pentanedioate (54) (Scheme 4)

Following procedure **A** (steps **A.1-A.3**), the reaction of 2-acetoxy-5-fluoro benzoic acid (**2**, 11.78 g, 59 mmol) and oxalylchloride (18 mL, 206 mmol) in dry CH₂Cl₂ (516 mL) in the presence of DMF (50 µL) afforded 2-acetoxy-5-fluoro benzoyl chloride (**52**).

Reaction of acid chloride **52** with (*S*)-5-allyl 1-benzyl 2-(methylamino)pentanedioate hydrochloride (**27**·HCl, 15.0 g, 46 mmol) in THF (260 mL) in the presence of *i*-Pr₂NEt (23 mL, 137 mmol) yielded acetate **53** (19.35 g, 90%), which was treated with 3-dimethylamino-1-propylamine (23 mL, 185 mmol) in THF (200 mL) to afford after aqueous workup (EtOAc, 0.1 M aq. HCl soln, sat. aq. NaCl soln) and after FC (hexane/EtOAc 8:2 to 7:3) the phenol **54** (14.4 g, 81%).

Data of **54:** C₂₃H₂₄FNO₆ (429.4). HPLC (30% CH₃CN) : Rₜ = 3.79 (87 %). LC-MS (method 9a): Rₜ = 2.09, 430 ([M+H]⁺).

### 2. Synthesis of (R)-5-allyl 1-benzyl 2-(5-fluoro-2-hydroxy-N-methylbenzamido)pentanedioate (56) (Scheme 4)

Following procedure **A** (steps **A.1-A.3),** the reaction of 2-acetoxy-5-fluoro benzoic acid (**2**, 13.0 g, 67 mmol) and oxalylchloride (20 mL, 233 mmol) in dry CH₂Cl₂ (585 mL) in the presence of DMF (50 µL) afforded 2-acetoxy-5-fluoro benzoyl chloride (**52**).

Reaction of acid chloride **52** with (*R*)-5-allyl 1-benzyl 2-(methylamino)pentanedioate hydrochloride (**29**·HCl, 17.0 g, 52 mmol) in THF (280 mL) in the presence of *i*-Pr₂NEt (27 mL, 156 mmol) yielded **55** (21.5 g, 88%), which was treated with 3-dimethylamino-1-propylamine (26 mL, 205 mmol) in THF (200 mL) to afford after aqueous workup (EtOAc, 0.1 M aq. HCl soln, sat. aq. NaCl soln) and after FC (hexane/EtOAc 8:2 to 7:3) phenol **56** (14.8 g, 75%).

Data of **56:** C₂₃H₂₄FNO₆ (429.4). HPLC (30% CH₃CN): Rₜ = 3.79 (89). LC-MS (method 9c): Rₜ = 2.11, 430 ([M+H]⁺).

### 3. Synthesis of (S)-allyl 2-(benzyloxycarbonylamino)-3-(3-hydroxy-N-methylbenzamido)propanoate (59) (Scheme 4)

Following procedure **A** (steps **A.1-A.3**), the reaction of 3-acetoxybenzoic acid (**3**, 6.0 g, 33 mmol) and oxalylchloride (14 mL, 164 mmol) in dry Et₂O (216 mL) in the presence of DMF (50 µL) afforded 3-acetoxybenzoyl chloride (**57**, 7.0 g, quant.). Reaction of **57** (7.0 g, 35 mmol) with (S)-allyl 2-(benzyloxycarbonylamino)-3-(methylamino)propanoate hydrochloride (**32**·HCl, 10.5 g, 32 mmol) in CH₂Cl₂ (285 mL) in the presence of 2,4,6-collidine (12.8 mL, 96 mmol) yielded 58 (12.34 g, 82%).

The acetate **58** (12.82 g, 28.2 mmol) was treated with 3-dimethylamino-1-propylamine (10.6 mL, 84.6 mmol) in THF (114 mL) to afford phenol **59** (10.45 g, 90%).

Data of **59:** C₂₂H₂₄N₂O₆ (412.4). HPLC (10% CH₃CN): Rₜ = 3.91 (96). LC-MS (method 9a): Rₜ = 1.77, 413 ([M+H]⁺).

### 4. Synthesis of (R)-allyl 2-(benzyloxycarbonylamino)-3-(3-hydroxy-N-methylbenzamido)propanoate (61) (Scheme 4)

Following procedure **A** (steps **A.1-A.3),** the reaction of 3-acetoxybenzoic acid (**3**, 5.82 g, 32.3 mmol) and oxalylchloride (11.1 mL, 129 mmol) in dry Et₂O (210 mL) in the presence of DMF (50 µL) afforded 3-acetoxybenzoyl chloride (**57**, 6.5 g, 100%). Reaction of **57** (6.5 g, 32.3 mmol) with (*R*)-allyl 2-(benzyloxycarbonylamino)-3-(methylamino)propanoate hydrochloride (**36**·HCl, 8.5 g, 26 mmol) in CH₂Cl₂ (220 mL) in the presence of 2,4,6-collidine (10.3 mL, 77.6 mmol) yielded **60** (10.73 g, 92%).

Acetate **60** (15.46 g, 34 mmol) was treated with 3-dimethylamino-1-propylamine (12.8 mL, 102 mmol) in THF (140 mL) to afford phenol **61** (12.92 g, 92%).

Data of **61:** C₂₂H₂₄N₂O₆ (412.4). LC-MS (method 2): Rₜ = 1.77 (98), 413 ([M+H]⁺).

### 5. Synthesis of (S)-allyl 2-(benzyloxycarbonylamino)-4-(3-hydroxy-N-methylbenzamido)butanoate (63) (Scheme 4)

Following procedure **A** (steps **A.1-A.3),** the reaction of 3-acetoxybenzoic acid (**3**, 7.65 g, 43 mmol) and oxalylchloride (18.2 mL, 213 mmol) in dry CH₂Cl₂ (140 mL) in the presence of DMF (300 µL) afforded after 3 h at rt 3-acetoxybenzoyl chloride **(57).**

Reaction of **57** with (S)-allyl 2-(benzyloxycarbonylamino)-5-(methylamino)butanoate hydrochloride (**40**·HCl, 8.7 g, 28 mmol) in THF (140 mL) in the presence of *i*-Pr₂NEt (15 mL, 85 mmol) yielded **62** (8.1g, 61%).

Acetate **62** (4.85 g, 10 mmol) was treated with 3-dimethylamino-1-propylamine (3.8 mL, 31 mmol) in THF (90 mL) to afford phenol **63** (4.23 g, 95%).

Data of **63:** C₂₃H₂₆N₂O₆ (426.5). LC-MS: (method 6): R_{t =} 1.06 (99), 427 ([M+H]⁺).

### 6. Synthesis of (S)-allyl 2-(benzyloxycarbonylamino)-5-(3-hydroxy-N-methylbenzamido)pentanoate (65) (Scheme 4)

Following procedure **A** (steps **A.1-A.3**), the reaction of 3-acetoxybenzoic acid (**3**, 10 g, 58 mmol) and oxalylchloride (19 mL, 218 mmol) in dry CH₂Cl₂ (450 mL) in the presence of DMF (500 µL) afforded 3-acetoxybenzoyl chloride (**57**).

Reaction of **57** with (S)-allyl 2-(benzyloxycarbonylamino)-5-(methylamino)pentanoate hydrochloride (**44**·HCl, 17.3 g, 48 mmol) in THF (200 mL) in the presence of *i*-Pr₂NEt (25 mL, 145 mmol) yielded **64** (12.08 g, 51%), which was treated with 3-dimethylamino-1-propylamine (9.3 mL, 75 mmol) in THF (240 mL) to afford after aqueous workup (EtOAc, 1 M aq. HCl soln, sat. aq. NaHCO₃ soln, sat. aq. NaCl soln) phenol **65** (10.84 g, 98%).

Data of **65:** C₂₄H₂₈N₂O₆ (440.5). LC-MS (method 6): Rₜ = 1.15 (91), 441 ([M+H]⁺).

### 7. Synthesis of (S)-5-allyl 1-benzyl 2-(4-hydroxy-N-methylbenzamido)pentanedioate (68) (Scheme 4)

Following procedure **A** (steps **A.1-A.3),** the reaction of 4-acetoxybenzoic acid (**4**, 10.7 g, 59.5 mmol) and oxalylchloride (17.7 mL, 206 mmol) in dry CH₂Cl₂ (350 mL) in the presence of DMF (50 µL) afforded 4-acetoxybenzoyl chloride (**66**).

Reaction of **66** with (*S*)-5-allyl 1-benzyl 2-(methylamino)pentanedioate hydrochloride (**27**·HCl, 15.0 g, 46 mmol) in THF (250 mL) in the presence of *i*-Pr₂NEt (23.3 mL, 137 mmol) yielded **67** (16.24 g, 78%).

Treatment of **67** (15.2 g, 33.5 mmol) with 3-dimethylamino-1-propylamine (12.6 mL, 101 mmol) in THF (140 mL) afforded phenol **68** (14.86 g, quant.).

Data of **68:** C₂₃H₂₅NO₆ (411.4). LC-MS (method 9b): Rₜ = 1.96, 412 ([M+H]⁺).

### 8. Synthesis of (S)-allyl 2-(benzyloxycarbonylamino)-3-(5-hydroxy-N-methylnicotinamido)propanoate (71) (Scheme 4)

A mixture of 5-hydroxy nicotinic acid (**5**, 3.5 g, 25.1 mmol) and acetic anhydride (23 mL, 243 mmol) was heated at 95°C for 45 min and cooled to rt. The mixture was filtered. The solid was washed (H₂O, Et₂O) and dried i.v. to give 5-acetoxynicotinic acid (**6**; 3.76 g, 82%) (cf. Scheme 1)

5-Acetoxynicotinic acid (**6**; 5.7 g, 31.5 mmol) was suspended in CHCl₃ (stabilized with amylene, 230 mL). Oxalylchloride (9.0 mL, 105 mmol) was added followed by DMF (ca. 50 µL). The mixture was stirred at rt for 15 h, then concentrated, co-evaporated with dry CH₂Cl₂ and dried i.v. to afford 5-acetoxynicotinoyl chloride (**69**). (S)-allyl 2-(benzyloxycarbonylamino)-3-(methylamino)propanoate hydrochloride (**32**·HCl, 8.6 g, 26.2 mmol) and THF (225 mL) were added. The mixture was cooled to 0°C. Et₃N (13 mL, 92 mmol) was slowly added. The mixture was stirred at 0°C to rt for 18 h. 3-dimethylamino-1-propylamine (9.9 mL, 78.6 mmol) was added and stirring at rt was continued for 2 h. The mixture was distributed between EtOAc and 1 M aq. NaH₂PO₄ soln. The organic layer was separated, washed (sat. aq. NaCl soln), dried (Na₂SO₄), filtered and concentrated. FC (CH₂Cl₂/MeOH 19:1) afforded phenol **71** (8.81 g, 81%).

Data of **71:** C₂₁H₂₃N₃O6 (413.4). LC-MS (method 6): Rₜ = 0.94 (92), 414 ([M+H]⁺).

### 9. Synthesis of allyl (2S)-2-[(benzyloxy)carbonyl]amino-3-[((2S)-2-[(tert-butoxycarbonyl)amino]-8-hydroxy-1,2,3,4-tetrahydro-2-naphthalenylcarbonyl)(methyl)amino]propanoate (72) (Scheme 4)

A mixture of **10** (3.0 g, 9.76 mmol), HATU (5.57 g, 14.6 mmol), HOAt (1.99 g, 14.6 mmol) and **32**·HCl (6.4 g, 19.5 mmol) was dissolved in DMF (113 mL). *i*-Pr₂NEt (8.36 mL, 48.8 mmol) was added. The mixture was stirred at rt for 3 d, then distributed between H₂O and EtOAc. The organic phase was dried (Na₂SO₄), filtered, and concentrated. FC (hexane/EtOAc 75:25 to 50:50) afforded **72** (2.58 g, 45%).

Data of **72:** C₃₁H₃₉N₃O₈ (581.3). LC-MS (method 7): Rₜ = 1.27 (97), 582 ([M+H]⁺).

### 10. Synthesis of 5-allyl 1-benzyl (2S)-2-[[(8-hydroxy-2-quinolinyl)carbonyl](methyl)amino]pentanedioate(75) (Scheme 4)

Following procedure **A** (steps **A.1-A.3**), the reaction of 8-acetoxyquinoline-2-carboxylic acid (8, 2.22 g 9.6 mmol) and oxalylchloride (2.1 mL, 24 mmol) in dry CH₂Cl₂ (90 mL) (no addition of DMF) afforded after 2 h at rt acetoxyquinoline-2-carboxylic acid chloride (**73**).

Reaction of **73** with (*S*)-5-allyl 1-benzyl 2-(methylamino)-pentanedioate hydrochloride (**27**·HCl, 2.3 g, 8.0 mmol) in CH₂Cl₂ (200 mL) in the presence of *i*-Pr₂NEt (5.5 mL, 32 mmol) yielded after 2.5 h at rt and purification by FC (hexane/EtOAc gradient) **74** (3.03 g, 74%), which was treated with 3-dimethylamino-1-propylamine (2.3 mL, 18 mmol) in THF (54 mL) to afford after aqueous workup (EtOAc, 1 M aq. HCl soln, sat. aq. NaHCO₃ soln, sat. aq. NaCl soln) the phenol **75** (2.79 g, 99%). Data of **75:** C₂₆H₂₆N₂O₆ (462.5). LC-MS (method 7): Rₜ = 1.29 (94), 463 ([M+H]⁺).

### 11. Synthesis of N-allyl-3-hydroxy-N-methylbenzamide (77) (Scheme 4)

Following procedure **A** (steps **A.1-A.3),** the reaction of 3-acetoxybenzoic acid (**3**, 23.7 g, 132 mmol) and oxalylchloride (45.3 mL, 527 mmol) in dry Et₂O (800 mL) in the presence of DMF (100 µL) afforded 3-acetoxybenzoyl chloride (**57**).

Reaction of **57** with N-allylmethylamine (10.1 ml, 105 mmol) in CH₂Cl₂ (500 mL) in the presence of 2,4,6-collidine (42 mL, 316 mmol) yielded **76** (24 g, 98%).

The acetate **76** (10.9 g, 46.7 mmol) was treated with 3-dimethylamino-1-propylamine (17.5 mL, 140 mmol) in THF (90 mL) to afford after aqueous workup (EtOAc, 1 M aq. HCl soln, sat. aq. NaCl soln) the phenol **77** (9.0 g, 100%).

Data of **77:** C₁₁H₁₃NO₂ (191.2). LC-MS (method 2): Rₜ = 1.52 (99), 192 ([M+H]⁺).

### 12. Synthesis of (S)-5-allyl-1-benzyl 2-(3-mercapto-N-methylbenzamido)pentanedioate (80) (Scheme 4)

Acetic anhydride (0.46 mL, 4.86 mmol) was added at 0°C to a soln of 3-mercaptobenzoic acid (**11**, 250 mg, 1.62 mmol) in 1 M aq. NaOH soln (5.0 mL, 5.0 mmol). The mixture was stirred at 0°C for 1 h. A precipitate formed; and the mixture was acidified by the addition of 1 M aq. HCl soln and filtered. The solid was dried i.v. to afford 3-(acetylthio)benzoic acid (**12**; 280 mg, 88%) (cf. Scheme 1).

Oxalyl chloride (0.34 mL, 3.97 mmol) was added to a mixture of **12** (260 mg, 1.33 mmol) and CHCl₃ (stabilized with amylene; 16 mL). DMF (7 µL) was added. The mixture was stirred at rt for 2 h. The volatiles were evaporated to afford 3-(acetylthio)benzoyl chloride (**78**).

(S)-5-allyl 1-benzyl 2-(methylamino)pentanedioate hydrochloride (**27**-HCl, 434 mg, 1.33 mmol) and dry THF (5 mL) were added. The mixture was cooled to 0°C, followed by the addition of *i*-Pr₂NEt (0.79 mL, 4.6 mmol). The mixture was stirred at rt for 16 h and distributed between EtOAc and 1 M aq. HCl soln. The organic phase was separated, dried Na₂SO₄), filtered and concentrated. FC (hexane/EtOAc 2:1) afforded the acetate **79** (420 mg, 67%).

At rt, a soln of **79** (246 mg, 0.52 mmol) in degassed THF (3.6 mL) was treated with 3-dimethylamino-1-propylamine (0.13 mL, 1.05 mmol) for 1 h. The mixture was distributed between EtOAc and 1 M aq. HCl soln. The organic phase was separated, dried (Na₂SO₄), filtered and concentrated. FC (hexane/EtOAc 2:1) afforded **80** (153 mg, 68%).

Data of **80:** C₂₃H₂₅NO₅S (427.5). LC-MS (method 7): Rₜ = 1.39 (84), 428 ([M+H]⁺).

### 13. Synthesis of (S)-allyl 2-(benzyloxycarbonylamino)-4-(5-fluoro-2-hydroxybenzamido) butanoate (199) (Scheme 4)

Following procedure **A** (step **A.1**), the reaction of 2-acetoxy-5-fluoro benzoic acid (**2;** 10.5 g, 53.1 mmol) and oxalyl chloride (15.8 mL, 184 mmol) in dry CH₂Cl₂ (460 mL) in the presence of DMF (0.2 mL) afforded 2-acetoxy-5-fluoro benzoyl chloride **(52).** (S)-Allyl-4-amino-2-(benzyloxycarbonylamino) butanoate toluene-4-sulfonate (**38**˙pTsOH; 19.0 g, 40.9 mmol) and THF (230 mL) were added. The mixture was cooled to 0°C and *i*-Pr₂NEt (21 mL, 123 mmol) was added dropwise. The mixture was stirred at rt for 2 h. 3-Dimethylaminopropylamine (15.5 mL, 123 mmol) was added. The mixture was stirred at rt for 2 h followed by aqueous workup (CH₂Cl₂, 1 M aq. NaH₂PO₄ soln). The organic layer was separated, dried (Na₂SO₄), filtered and concentrated. FC (hexane/EtOAc) afforded **199** (15.5 g, 88%).

Data of **199:** C₂₂H₂₃FN₂O₆ (430.4) . LC-MS (method 10a): Rₜ = 2.14 (90), 431 ([M+H]⁺).

### 14. Synthesis of (S)-allyl 2-(benzyloxycarbonylamino)-5-(3-hydroxybenzamido) pentanoate (200) (Scheme 4)

Following procedure **A** (step **A.1**)**,** the reaction of 3-acetoxy benzoic acid **(3,** 4.2 g, 23.3 mmol) and oxalyl chloride (7.5 mL, 87.6 mmol) in dry CH₂Cl₂ (160 mL) in the presence of DMF (0.1 mL) afforded after 2 h at rt 3-acetoxybenzoyl chloride **(57).**

(S)-Allyl-5-amino-2-(benzyloxycarbonylamino)pentanoate toluene-4-sulfonate (**42˙**pTsOH; 9.3 g, 19.5 mmol) and THF (70 mL) were added. The mixture was cooled to 0°C and *i*-Pr₂NEt (10.0 mL, 58.4 mmol) was added within 5 min. The mixture was stirred at rt for 2 h followed by the addition of 3-dimethylaminopropylamine (7.4 mL, 58.4 mmol) . Stirring was continued for 2 h. The mixture was distributed between EtOAc and 1 M aq. HCl soln. The organic layer was separated, washed (H₂O sat. aq. NaHCO₃ soln, sat. aq. NaCl soln), dried (Na₂SO₄), filtered and concentrated. FC (EtOAc/hexane) afforded **200** (5.83 g, 70%).

Data of **200:** C₂₃H₂₆N₂O₆ (426.5). LC-MS (method 4b): Rₜ = 1.96 (97), 427 ([M+H]⁺).

### 15. Synthesis of (S)-allyl 2-(benzyloxycarbonylamino)-5-(5-fluoro-2-hydroxy-N-methylbenzamido) pentanoate (202) (Scheme 4)

Following procedure **A** (steps **A.1-A.3)** the reaction of 2-acetoxy-5-fluoro benzoic acid **(2;** 5.6 g, 28.6 mmol) and oxalyl chloride (9.2 mL, 107 mmol) in dry CH₂Cl₂ (220 mL) in the presence of DMF (0.05 mL) afforded after 6 h at rt 2-acetoxy-5-fluoro benzoyl chloride **(52).**

Reaction of the acid chloride **52** with (S)-allyl-2-(benzyloxycarbonylamino)-5-(methylamino)pentanoate hydrochloride (**44˙**HCl**;** 8.5 g, 23.8 mmol) in THF (130 mL) in the presence of *i*-Pr₂NEt (12.2 mL, 71.5 mmol) yielded **201** (4.86 g, 41%).

Acetate **201** was treated with 3-dimethylaminopropylamine (3.64 mL, 29.1 mmol) in THF (45 mL) to afford after aqueous workup (EtOAc, 1 M aq. HCl soln, sat. aq. NaCl soln) the phenol **202** (4.08 g, 92%).

Data of **202:** C₂₄H₂₇FN₂O₆ (458.5). LC-MS (method 4b): Rₜ = 2.08 (83), 459 ([M+H]⁺).

### 16. Synthesis of 5-allyl 1-benzyl (2S)-2-[[(3-hydroxy-2-thienyl)carbonyl](methyl)amino]pentanedioate (205) (Scheme 4)

At 0°C acetic anhydride (6.5 mL, 69.4 mmol) was added to a soln of 3-hydroxythiophene-2-carboxylic acid **(144;** 2.0 g, 13.9 mmol) in 2 M aq. NaOH (34.7 mL, 69.4 mmol). The mixture was stirred for 5 h, acidified with 1 M aq. HCl soln and extracted with EtOAc. The organic layer was separated, dried (Na₂SO₄), filtered and concentrated to give 3-acetoxythiophene-2-carboxylic acid **(145;** 1.74 g, 67%) (cf. Scheme 1).

Following procedure **A** (steps **A.1-A.3)** the reaction of **145** (1.74 g, 9.3 mmol) and oxalyl chloride (8.0 mL, 93 mmol) in dry CH₂Cl₂ (130 mL) in the presence of DMF (0.05 mL) afforded after 2.5 h at rt 2-(chlorocarbonyl)thiophen-3-yl acetate **(203).** Reaction of the acid chloride **203** with (*S*)-5-allyl-1-benzyl-2-(methylamino) pentanedioate hydrochloride (**27˙**HCl; 2.80 g, 8.5 mmol) in THF (95 mL) in the presence of *i*-Pr₂NEt (4.4 mL, 25.6 mmol) yielded after aqueous workup (EtOAc, 1 M aq. HCl soln, H₂O sat. aq. NaCl soln) the crude acetate **204** (3.56 g), which was treated with 3-dimethylaminopropylamine (1.95 mL, 15.5 mmol) in THF (87 mL) to afford after aqueous workup (EtOAc, 1 M aq. HCl soln, sat. aq. NaCl soln) and after FC (CH₂Cl₂ phenol **205** (1.65 g, 42%).

Data of **205:** C₂₁H₂₃NO₆S (417.5). LC-MS (method 7): Rₜ = 1.43 (93), 418 ([M+H]⁺).

### 17. Synthesis of (S)-allyl 2-(benzyloxycarbonylamino)-6-(5-hydroxy-N-methylnicotinamido)hexanoate (207) (Scheme 4)

Following procedure **A** (step **A.1),** the reaction of 5-acetoxynicotinic acid **(6,** 41 g, 226 mmol, synthesized as described above, cf. phenol **71)** and oxalyl chloride (71.8 mL, 849 mmol) in CHCl₃ (1660 mL) in the presence of DMF (0.26 mL) afforded after 1.5 h at rt 5-acetoxy-nicotinoyl chloride **(69).** At 0°C a suspension of the acid chloride **69** in CH₂Cl₂ (100 mL) was added over 10 min to a mixture of (*S*)-allyl-2-(benzyloxycarbonylamino)-6-(methylamino)hexanoate hydrochloride (**182**˙HCl; 70.0 g, 189 mmol) in CH₂Cl₂ (260 mL) and 1 M aq. Na₂CO₃ soln (360 mL). The mixture was stirred at rt for 2 h. The organic layer was separated; the aqueous layer was extracted twice with CH₂Cl₂ (250 mL). The combined organic layer was dried (Na₂SO₄), filtered and concentrated. FC (hexane/EtOAc/MeOH gradient) afforded **206** (33.5 g, 36%).

Following procedure **A** (step **A.3),** acetate **206** was treated with 3-dimethylaminopropylamine (25.4 mL, 202 mmol) in THF (330 mL) to afford after 2 h at rt and aqueous workup (EtOAc, 1 M aq. NaH₂PO₄ soln) **207** (29.1 g, 95%) .

Data of **207:** C₂₄H₂₉N₃O₆ (455.5). LC-MS (method 4a): Rₜ = 1.77 (96), 456 ([M+H]⁺).

### 18. Synthesis of 5-allyl 1-benzyl (2S)-2-[[(5-hydroxy-1,2-dimethyl-1H-indol-3-yl)carbonyl](methyl)amino]pentanedioate (210) (Scheme 4)

Pyridine (6.3 mL, 78 mmol) was added to a suspension of 5-hydroxy-1,2-dimethyl-1*H*-indole-3-carboxylic acid **(146;** 4.0 g, 19 mmol) in EtOAc (40 mL). Acetic anhydride (3.7 mL, 39 mmol) was added. The mixture was stirred at rt for 3 h followed by distribution between CH₂Cl₂ and 1 M aq. HCl soln. The organic layer was separated, dried (Na₂SO₄), filtered and concentrated. FC (CH₂Cl₂/MeOH 100:0 to 97:3). The product was suspended in Et₂O filtered and dried i.v. to afford 5-acetoxy-1,2-dimethyl-1*H*-indole-3-carboxylic acid **(147;** 3.62 g, 75%) (cf. Scheme 1). Following procedure **A** (step **A.1),** the reaction of **147** (3.6 g, 15 mmol) and oxalyl chloride (3.1 mL, 37 mmol) in dry CH₂Cl₃ (90 mL) afforded after 2 h at rt 3-(chlorocarbonyl)-1,2-dimethyl-1H-indol-5-yl acetate **(208).**

At 0°C (S)-5-allyl-1-benzyl-2-(methylamino) pentanedioate hydrochloride (**27**˙HCl; 4.0 g 12 mmol) was added portionwise to a mixture of the acid chloride **208** and *i*-Pr₂NEt (8.4 mL, 49 mmol) in CH₂Cl₂ (43 mL). The mixture was stirred for 2 h at rt followed by distribution between EtOAc and 1 M aq. HCl soln. The organic layer was separated, washed (sat. aq. NaHCO₃ soln, sat. aq. NaCl soln), dried (Na₂SO₄), filtered and concentrated. FC (CH₂Cl₂/MeOH 100:0 to 97:3) afforded **209** (6.34 g, 99%). Treatment of **209** with 3-dimethylaminopropylamine (6.8 mL, 54 mmol) in THF (70 mL) at rt for 16 h afforded after aqueous workup (EtOAc, 1 M aq. HCl soln, sat. aq. NaHCO₃ soln, sat. aq. NaCl soln) **210** (5.40 g, 93%).

Data of **210:** C₂₇H₃₀N₂O₆ (478.5). LC-MS (method 4b): Rₜ = 2.20 (95), 479 ([M+H]⁺).

### 19. Synthesis of (S)-allyl 2-(tert.-butoxycarbonylamino)-5-(3-mercapto-N-methylbenzamido) pentanoate (212) (Scheme 4)

Following procedure **A** (steps **A.1-A.3)** the reaction of 3-(acetylthio)benzoic acid **(12,** 10.0 g, 51 mmol; synthesized as described above, cf. thiophenol **80)** and oxalyl chloride (16.0 mL, 192 mmol) in dry CH₂Cl₂ (380 mL) in the presence of DMF (0.1 mL) afforded after 3.5 h at rt 3-(acetylthio)benzoyl chloride **(78).**

Reaction of the acid chloride **78** with (S)-allyl-2-(benzyloxycarbonylamino)-5-(methylamino) pentanoate hydrochloride (**44**˙HCl; 15.2 g, 43 mmol) in THF (175 mL) in the presence of *i*-Pr₂NEt (22 mL, 128 mmol) yielded after aqueous workup (EtOAc, 1 M aq. HCl soln) and FC (hexane/EtOAc) **211** (12.31 g, 57%). Treatment of **211** (8.0 g, 16 mmol) with 3-dimethylaminopropylamine (6.1 mL, 48 mmol) in THF (135 mL) afforded after 1 h and aqueous workup (EtOAc, 1 M aq. HCl soln) **212** (7.49 g, quant. yield; used without further purification). Data of **212:** C₂₄H₂₈N₂O₅S (456.5). LC-MS (method 10a): Rₜ = 2.12 (83), 457 ([M+H]⁺). ¹H-NMR (DMSO-d₆): 7.83-7.74 (m, 1 H), 7.39-7.28 (m, 8 H), 7.08 (m, 1 H), 5.89 (m, 1 H), 5.61 (s, 1 H), 5.31 (d, J = 17.0, 1 H), 5.21 (td, J = 1.4, 10.5, 1 H), 5.05 (s, 2 H), 4.58 (br. s, 2 H), 4.12, 4.04 (2 br. m, 1 H), 3.49, 3.38 (2 br. m, 1 H), 3.18 (br. m, 1 H), 2.89, 2.81 (2 br. s, 3 H), 1.80-1.40 (br. m, 4 H).

### 20. Synthesis of (S)-allyl 2-(benzyloxycarbonylamino)-6-(3-hydroxyphenylthio)hexanoate (213) (Scheme 4)

A mixture of **185** (4.97 g, 14.2 mmol) and NaHCO₃ (3.57 g, 42.6 mmol) was suspended in degassed anhydrous DMF (85 mL). At 0°C a soln of 3-mercaptophenol **(143;** 2.17 mL, 21.3 mmol) in DMF (5 mL) was added dropwise. The mixture was stirred for 6 h at rt. The mixture was distributed between EtOAc and aq. NaCl soln. The organic phase was separated, washed (H₂O, NaCl soln), dried (Na₂SO₄), filtered and concentrated to yield after FC (hexane/EtOAc 3:1) **213** (5.6 g, quant. yield).

Data of **213:** C₂₀H₂₉NO₅S (395.5). LC-MS (method 10c): Rₜ = 2.24 (95), 396 ([M+H]⁺).

### Synthesis of the C-B fragments PG⁵-NR⁴-c2-CO-B-OH and H₂C=CR¹²-c2-CO-B-OH

### 1. Synthesis of allyl N-2-[(2S,4S)-4-[(tert-butoxycarbonyl)-amino]-2-(hydroxymethyl)tetrahydro-1H-pyrrol-1-yl]-2-oxoethyl-N-methylcarbamate (81) (Scheme 5)

A soln of (2-((allyloxycarbonyl)(methyl)amino)acetic acid **(47,** 8.0 g, 46 mmol) and aminoalcohol **13** (11.0 g, 51 mmol) in DMF (120 mL) was cooled to 0°C. 2,4,6-Collidine (11 mL, 82 mmol) was added followed by HATU (22 g, 58 mmol). The mixture was stirred for 1 h at 0°C then for 16 h at rt followed by distribution between EtOAc and sat. aq. Na₂CO₃ soln. The organic phase was washed (1 M aq. HCl soln, sat. aq. NaCl soln), dried (Na₂SO₄) filtered and concentrated. FC (EtOAc/MeOH 100:0 to 95:5) afforded amidoalcohol **81** (14.7 g, 86%).

Data of **81:** C₁₇H₂₉N₃O₆ (371.4). HPLC (20% CH₃CN) : Rₜ = 2.94 (97). LC-MS (method 9c): Rₜ = 1.55, 743 ([2M+H]⁺), 372 ([M+H]⁺).

### 2. Synthesis of allyl N-2-[(2S,4R)-4-[(tert-butoxycarbonyl)-amino]-2-(hydroxymethyl)tetrahydro-1H-pyrrol-1-yl]-2-oxoethyl-N-methylcarbamate (82) (Scheme 5)

Following procedure **D,** the reaction of the aminoalcohol **17**˙HCl (10.0 g, 39.6 mmol) and 2-((allyloxycarbonyl)(methyl)amino) acetic acid **(47,** 15.1 g, 87 mmol) in DMF (100 mL) in the presence of HCTU (40.9 g, 98.9 mmol), Cl-HOBt (1.68 g, 9.89 mmol) and *i*-Pr₂NEt (33.6 mL, 198 mmol) afforded after FC (hexane/EtOAc 20:80 to 0:100) the corresponding amido ester intermediate (13.7 g) which was saponified with lithium hydroxide monohydrate (3.28 g, 78.1 mmol) in THF (350 mL) and H₂O (90 mL) to yield amidoalcohol **82** (8.89 g, 61%).

Data of **82:** C₁₇H₂₉N₃O₆ (371.4). LC-MS (method 9b): Rₜ = 1.57; 372 ([M+H]⁺), 316, 272 ([M+H-Boc]⁺), 156.

### 3. Synthesis of tert-butyl (3R)-4-{2-[[(allyloxy)carbonyl]-(methyl)amino]acetyl}-3-(hydroxymethyl)tetrahydro-1(2H)-pyrazinecarboxylate (84) (Scheme 5)

Following procedure **D,** the reaction of (*R*)-*tert*-butyl 3-(hydroxymethyl)piperazine-1-carboxylate hydrochloride (**83**˙HCl, 19.7 g, 78 mmol) and 3-((allyloxycarbonyl)(methyl)amino)acetic acid **(47,** 30 g, 172 mmol) in DMF (188 mL) in the presence of HCTU (81.0 g, 195 mmol), C1-HOBt (3.3 g, 19 mmol) and *i*-Pr₂NEt (67 mL, 390 mmol) afforded after FC (EtOAc) the corresponding amido ester intermediate (40 g) which was saponified with lithium hydroxide monohydrate (9.5 g, 228 mmol) in THF (1020 mL) and H₂O (245 mL) to yield after FC (EtOAc) amidoalcohol **84;** 22.8g, 79%).

Data of **84:** C₁₇H₂₉N₃O₆ (371.4). LC-MS (method 7): Rₜ = 0.99 (93), 372 ([M+H]⁺).

### 4. Synthesis of benzyl N-((1S)-1-[(2S,4S)-4-[(tert-butoxycarbonyl)amino]-2-(hydroxymethyl)tetrahydro-1H-pyrrol-1-yl]-carbonyl-3-butenyl)carbamate (85) (Scheme 5)

Aminoalcohol-hydrochloride **13**˙HCl (3.7 g, 14.7 mmol) was added to a soln of acid **51** (5.22 g, 14.7 mmol) in DMF (80 ml). The mixture was cooled to 0°C. HATU (7.0 g, 18.4 mmol) and 2,4,6-collidine (3.51 ml, 26.4 mmol) were added. The soln was stirred at 0°C to rt for 17 h, followed by distribution between EtOAc and sat. aq. Na₂CO₃ soln. The organic phase was washed (1 M aq. HCl soln, sat. aq. NaHCO₃ soln, sat. aq. NaCl soln), dried (Na₂SO₄), filtered and concentrated. FC (hexane/EtOAc 30:70 to 20:80) afforded the amidoalcohol **(85,** 5.78 g, 88%)

Data of **85:** C₂₃H₃₃N₃O₆ (447.5). LC-MS (method 2): Rₜ = 1.92 (92), 448 ([M+H]⁺).

### 5. Synthesis of allyl N-3-[(2S,4R)-4-[(tert-butoxycarbonyl)-amino]-2-(hydroxymethyl)tetrahydro-1H-pyrrol-1-yl]-3-oxopropyl-N-methylcarbamate (86) (Scheme 5)

Following procedure **D,** the reaction of aminoalcohol **17˙**HCl (7.5 g, 30 mmol) and 3-((allyloxycarbonyl)(methyl)amino)propanoic acid **(49,** 12.3 g, 66 mmol) in DMF (77 mL) in the presence of HCTU (31.0 g, 75.0 mmol), Cl-HOBt (1.27 g, 7.5 mmol) and *i*-Pr₂NEt (25.6 mL, 150 mmol) afforded after FC (CH₂Cl₂/MeOH 100:0 to 97:3) the corresponding amido ester intermediate (17.1 g) which was saponified with lithium hydroxide monohydrate (3.8 g, 90 mmol) in THF (388 mL) and H₂O (105 mL) to yield amidoalcohol **86** (10.48 g, 86%).

Data of **86:** C₁₈H₃₁N₃O₆ (385.4). HPLC (10% CH₃CN) : Rₜ = 3.49 (88). LC-MS (method 9a): R_{t =} 1.62; 386 ([M+H]⁺), 330 ([M+H-tBu]⁺), 286 ([M+H-Boc]⁺).

### Core 01: Synthesis of Ex.1 (Scheme 6)

### Synthesis of Mitsunobu product 87

To a soln of **54** (350 mg, 0.82 mmol), **16** (590 mg, 1,7 mmol) and PPh₃ (1069 mg, 4.08 mmol) in dry degassed CHCl₃ (11 mL) was added ADDP (1028 mg, 4.08 mmol) in one portion at 0°C, under an N₂ atmosphere. The resulting mixture was stirred for 16 h at rt. The mixture was filtered and the slurry washed further with Et₂O. The combined filtrates were concentrated *in vacuo.* The crude residue was purified by FC (CH₂Cl₂/EtOH 100:0 to 99:1) to afford **87** (1.05 g containing residual triphenylphosphine oxide), which was used in the next step without further purification.

### Synthesis of amino acid 88

Following procedure **B.2,** the reaction of **87** (441 mg, contaminated with triphenylphosphine oxide, ca. 0.5 mmol), 1,3-dimethylbarbituric acid (219 mg, 1.4 mmol) and Pd(PPh₃)₄ (34 mg) in EtOAc/CH₂Cl₂ (55:45, 10 mL) yielded after 1,5 h and subsequent FC (CH₂Cl₂/MeOH 100:0 to 80:20) amino acid **88** (267 mg, 72%).

Data of **88:** C₃₁H₄₂FN₃O₈Si (631.7). LC-MS (method 9a):
Rₜ = 2.02, 632 ([M+H]⁺). HPLC (30% CH₃CN) : Rₜ = 3.41 (96).

### Synthesis of macrolactam Ex.1

According to procedure **F.1.1** amino acid **88** (75 mg, 0.12 mmol) in dry CH₂Cl₂ (6 mL) was added within 4 h to T3P (50% in EtOAc, 0.21 mL, 0.36 mmol) and *i*-Pr₂NEt (0.1 mL, 0.59 mmol) in dry CH₂Cl₂ (6 mL) to give after FC (CH₂Cl₂/MeOH 100:0 to 96:4) macrolactam **Ex.1** (45 mg, 61%).

Data of **Ex.1:** C₃₁H₄₀FN₃O₇Si (613.7). LC-MS (method 7):
Rₜ = 1.45 (41), 614 ([M+H]⁺); 1.47 (44), 614 ([M+H]⁺).
¹H-NMR (DMSO-d₆): complex spectrum, several isomers; 7.45-7.01 (m, 8 H), 6,78-6.58 (2 m, 1 H), 5.42-5.06 (m, 3 H), 4.50-3.50 (several m, 7 H), 3.30-1.40 (several m, 7 H), 2.84, 2.70, 2.66 (s, 3 H),0.97-0.82 (m, 2 H), 0.03, 0.02, 0.00 (s, 9 H).

### Core 02: Synthesis of Ex.2 (Scheme 11)

### Synthesis of the protected macrolactam Ex.2

A soln of T3P (50% in EtOAc, 0.75 mL, 1.27 mmol) and i-Pr₂NEt (0.36 mL, 2.2 mmol) in dry CH₂Cl₂ (20 mL) was added within 2 h to a soln of amino acid **98** (250 mg, 0.43 mmol) in dry CH₂Cl₂ (730 mL). The soln was stirred at rt for 20 h, followed by extraction with sat. aq. Na₂CO₃ soln. The organic phase was dried (Na₂SO₄), filtered and concentrated. FC (CH₂Cl₂/MeOH 100:0 to 95:5) afforded **Ex.2** (187 mg, 77%).
Data of **Ex.2:** C₃₀H₃₆FN₃O₇ (569.6) LC-MS (method 7): Rₜ = 1.35 (62), 570 ([M+H]⁺); 1.39 (15), 570 ([M+H]⁺).
¹H-NMR (DMSO-d₆): complex spectrum, several isomers; 7.46-7.30 (m, 5 H), 7.27-7.06 (m, 2 H), 6.98-6.67 (4 dd, 1 H), 5.54-5.06 (m, 3 H), 4.68-3.48 (m, 6 H), 3.05-1.98 (m 10 H; s at 2.82, 2.69, 2.64), 1.44-1.41 (3 s, 9 H).

### Core 03: Synthesis of Ex.3, Ex.4, and Ex.5 (Scheme 7)

### Synthesis of Mitsunobu product 89

Following procedure **E.1.1,** the reaction of phenol **54** (7.8 g, 18 mmol), alcohol **81** (16 g, 43 mmol), DEAD (40% in toluene, 37 mL, 82 mmol), and PPh₃ (21 g, 80 mmol) in dry benzene (250 mL) afforded after FC (CH₂Cl₂/EtOH 100:0 to 95:5) the protected amino acid **89** (15.9 g, contaminated with ca. 30% triphenylphosphine oxide, used in the next step without further purification).

### Synthesis of amino acid 90

Following procedure **E.2,** the reaction of **89** (9.6 g, contaminated with triphenylphosphine oxide, ca. 9 mmol), 1,3-dimethylbarbituric acid (5.0 g, 32.0 mmol) and Pd(PPh₃)₄ (0.4 g) in EtOAc/CH₂Cl₂ (55:45, 266 mL) yielded after 1,5 h and after FC (CH₂Cl₂/MeOH 90:10 to 50:50) amino acid **90** (4.34 g, 76%).

Data of **90:** C₃₃H₄₃FN₄O₉ (658.7). HPLC (10% CH₃CN) : Rₜ = 3.87 (99). LC-MS (method 9a): Rₜ = 1.77, 659 ([M+H]⁺).

### Synthesis of the protected macrolactam Ex.3

According to procedure **F.1.2,** amino acid **90** (2.5 g, 3.80 mmol)in dry DMF (50 mL) was treated with FDPP (2.51 g, 6.53 mmol) in DMF (400 mL) to afford after FC (EtOAc/MeOH 100:0 to 95:5) macrolactam **Ex.3** (2.29 g, 94%).

Data of **Ex.3:** C₃₃H₄₁FN₄O₈ (640.7). HPLC (30% CH₃CN) : Rₜ = 3.20 (96). LC-MS (method 9c): Rₜ = 2.06, 641 ([M+H]⁺). ¹H-NMR (CDCl₃) : 7.45-7.32 (m, 5 H), 7.06 (m, 1 H), 6.94-6.88 (m, 2 H), 5.57 (dd, J = 2.8, 12.6, 1 H), 5.42 (br. m, 1 H), 5.26 (d, J = 12.2, 1 H), 5.15 (d, J = 12.2, 1 H), 4.90 (dd, J = 2.5, 11.0, 1 H), 4.34 (d, J = 17.2, 1H), 4.35-4.11 (m, 3 H), 3.82 (br. t, J ca. 8.5, 1 H), 3.65 (d, J = 17.3, 1 H), 3.29 (t, J ca. 8. 8, 1 H), 3.14 (s, 3 H), 2.65 (s, 3 H), 2.51-1.98 (several m, 5 H), 1.76 (td, J = 8.2, 12.7, 1 H), 1.36 (s, 9 H).

### Synthesis of acid Ex.4

According to procedure **H,** ester **Ex.3** (2.0 g, 3.1 mmol) was hydrogenolyzed in MeOH (120 mL)/THF (40 mL) in the presence of catalyst (1 g) for 2 h to afford **Ex.4** (1.68 g, 97%).

Data of **Ex.4:** C₂₆H₃₅FN₄O₈ (550.6). HPLC (5% CH₃CN) : Rₜ = 3.60 (86). LC-MS: (method 9c): Rₜ = 1.53; 551 ([M+H]⁺), 451 ([M+H-Boc]⁺).

### Synthesis of amine Ex.5:

According to procedure **J,** ester **Ex.3** (100 mg, 0.16 mmol) in dioxane (3 mL) was treated with 4M HCl-dioxane (3 mL) to afford **Ex.5**˙HCl (100 mg, quant.).

Data of **Ex.5˙**HCl**:** C₂₈H₃₃FN₄O₆˙HCl (540.6, free base). LC-MS: (method 9c): Rₜ = 1.44, 541 ([M+H]⁺).

### Core 04: Synthesis of Ex.56 and Ex.57 (Scheme 8)

### Synthesis of Mitsunobu product 91

Following procedure **E.1.1** the reaction of phenol **54** (8.0 g, 19 mmol), alcohol **82** (16.0 g, 43 mmol), DEAD (40% in toluene, 38 mL, 84 mmol), and PPh₃ (22 g, 84 mmol) in dry benzene (260 mL) afforded after FC the protected amino acid **91** (33.5 g, contaminated with triphenylphosphine oxide) which was used in the next step without further purification).

### Synthesis of amino acid 92

Following procedure **E.2,** the reaction of **91** (33.5 g, contaminated with triphenylphosphine oxide), 1,3-dimethylbarbituric acid (16 g, 102 mmol) and Pd(PPh₃)₄ (0.2 g) in EtOAc/CH₂Cl₂ (45:55, 340 mL) yielded after 3 h and after FC (CH₂Cl₂/EtOH 100:0 to 70:30 then CH₂Cl₂/MeOH 90:10 to 70:30) amino acid **92** (4.8 g, 39% over two steps based on phenol **54).** Data of **92:** C₃₃H₄₃FN₄O₉ (658.7). HPLC (10% CH₃CN) : Rₜ = 3.80 (95). LC-MS (method 9c): Rₜ = 1.81, 659 ([M+H]⁺).

### Synthesis of the protected macrolactam Ex.56

According to procedure **F.1.1,** amino acid **92** (3.8 g, 5.80 mmol) in dry CH₂Cl₂ (40 mL) was treated with T3P (50% in EtOAc, 6.8 mL, 12 mmol) and *i*-Pr₂NEt (4.0 mL, 23 mmol) in dry CH₂Cl₂ (510 mL) to afford after FC (EtOAc/MeOH 100:0 to 95:5) macrolactam **Ex.56** (3.23 g, 87%).

Data of **Ex.56:** C₃₃H₄₁FN₄O₈ (640.7). HPLC (30% CH₃CN) : Rₜ = 3.49 (88). LC-MS (method 9c): Rₜ = 2.02, 641 ([M+H]⁺). ¹H-NMR (CDCl₃) : 7.41-7.32 (m, 5 H), 7. 04 (m, 1 H), 6.94-6.83 (m, 2 H), 5.54 (dd, J = 3.0, 12.7, 1 H), 5.25 (d, J = 12.2, 1 H), 5.14 (d, J = 12.2, 1H), 4.89 (dd, J = 2.1, 11.0, 1H), 4.63 (br. m, 1 H), 4.39-4.10 (m, 4 H), 3.79-3.64 (m, 2 H), 3.49 (br. m, 1 H), 3.12 (s, 3 H), 2.64 (s, 3 H), 2.51-2.36 (m, 2 H), 2.23-1.98 (m, 4H),1.44 (s, 9H).

### Synthesis of acid Ex.57

According to procedure **H,** ester **Ex.56** (2.25 g, 3.5 mmol) was hydrogenolyzed in MeOH (120 mL)/THF (40 mL) in the presence of catalyst (1.1 g) for 2 h to afford, after washing of the filtration residue with warm (50°C) MeOH/THF 3:1, acid **Ex.57** (1.9 g, 98%).

Data of **Ex.57:** C₂₆H₃₅FN₄O₈ (550.6). HPLC LC-MS: (method 2): Rₜ = 1.54 (82), 551 ([M+H]⁺).

### Core 05: Synthesis of Ex.85 and Ex.86 (Scheme 9)

### Synthesis of Mitsunobu product 93

Following procedure **E.1.1,** the reaction of phenol **56** (6.6 g, 15 mmol), alcohol **81** (13 g, 35 mmol), DEAD (40% in toluene, 32 mL, 69 mmol), and PPh₃ (18 g, 69 mmol) in dry benzene (220 mL) afforded after FC (CH₂Cl₂/MeOH 100:0 to 94:6) the protected amino acid **93** (34.5 g, crude product used in the next step without further purification).

### Synthesis of amino acid 94

Following procedure **E.2,** the reaction of **93** (34.5 g, crude material), 1,3-dimethylbarbituric acid (17 g, 106 mmol) and Pd(PPh₃)₄ (0.1 g) in EtOAc/CH₂Cl₂ (55:45, 350 mL) yielded after 3 h and after FC (CH₂Cl₂/EtOH 100:0 to 70:30 then CH₂Cl₂/MeOH 90:10 to 70:30) amino acid **94** (5.6 g, 55% over two steps based on phenol **56).**

Data of **94:** C₃₃H₄₃FN₄O₉ (658.7). HPLC (10% CH₃CN): Rₜ = 3.79 (96). LC-MS (method 9c): Rₜ = 1.77, 659 ([M+H]⁺).

### Synthesis of the protected macrolactam Ex.85

According to procedure **F.1.1,** amino acid **94** (2.75 g, 4.2 mmol) in dry CH₂Cl₂ (35 mL) was treated with T3P (50% in EtOAc, 4.9 mL, 8.3 mmol) and *i*-Pr₂NEt (2.9 mL, 17 mmol) in dry CH₂Cl₂ (355 mL) to yield after FC (EtOAc/MeOH 100:0 to 95:5) macrolactam **Ex.85** (2.47 g, 92%).

Data of **Ex.85:** C₃₃H₄₁FN₄O₈ (640.7). HPLC (30% CH₃CN): Rₜ = 3.52 (96). LC-MS (method 9c): R_{t =} 2.06; 641 ([M+H]⁺), 541 ([M+H-Boc]⁺). ¹H-NMR (CDCl₃): two isomers, ratio 85:15, 7.42-7.31 (m, 5 H), 7.08-6.77 (m, 3 H), 5.33 (d, J = 8.3, 1 H), 5.23 (d, J = 12.2, 1 H), 5.17 (d, J = 12.1, 1 H), 4. 84 (dd, J = 2. 9, 8. 9, 1 H), 4.37-4.25 (m, 3 H), 4.11 (dd, J = 4.2, 12.0, 1 H), 3.89 (t, J = 8.3, 1 H), 3.80 (d, J = 8.9, 1 H), 3.61 (d, J = 17.1, 1 H), 3.16 (t, J = 9.1, 1 H), 3.13 (s, 2.55 H, NCH₃ of major isomer), 3.03 (s, 0.45 H, NCH₃ of minor isomer), 2.98 (s, 2.55 H, NCH₃ of major isomer), 2.87 (0.45 H, NCH₃ of minor isomer), 2.64-2.41 (m, 2 H), 2.27-2.09 (m, 1 H), 1.98-1.83 (m, 2 H), 1.79-1.66 (m, 2 H), 1.45 (s, 7.65 H, Boc, major isomer), 1.35 (s, 1.35 H, Boc, minor isomer).

### Synthesis of acid Ex.86

According to procedure **H,** ester **Ex.85** (2.0 g, 3.1 mmol) was hydrogenolyzed in MeOH (120 mL) / THF (40 mL) in the presence of the catalyst (1 g) for 2 h to afford, after washing of the filtration residue with warm (50°C) MeOH/TFH 3:1, acid **Ex.86** (1.67 g, 97%).

Data of **Ex.86:** C₂₆H₃₅FN₄O₈ (550.6). LC-MS: (method 3): Rₜ = 1.10 (83), 551 ([M+H]⁺); 1.17 (15), 551 ([M+H]⁺).

### Core 06: Synthesis of Ex.104 and Ex.105 (Scheme 10)

### Synthesis of Mitsunobu product 95

Following procedure **E.1.2,** the reaction of phenol **56** (13.1 g, 30.5 mmol), alcohol **82** (13.6 g, 36.6 mmol), and CMBP (14.7 g, 61 mmol) in dry toluene (500 mL) afforded after FC (hexane/EtOAc 50:50 to 30:70) the protected amino acid **95** (16 g, 67%).

### Synthesis of amino acid 96

Following procedure **E.2,** the reaction of **95** (16.0 g, 20 mmol), 1,3-dimethylbarbituric acid (8 g, 49 mmol) and Pd(PPh₃)₄ (0.1 g) in EtOAc/CH₂Cl₂ (55:45, 220 mL) yielded after 3 h and after FC (CH₂Cl₂/EtOH 100:0 to 70:30 then CH₂Cl₂/MeOH 90:10 to 70:30) amino acid **96** (11 g, 81%).

Data of **96:** C₃₃H₄₃FN₄O₉ (658.7). LC-MS (method 2): Rₜ = 1.63 (97), 659 ([M+H]⁺).

### Synthesis of the protected macrolactam Ex.104

According to procedure **F.1.1,** amino acid **96** (4.0 g, 6.1 mmol) in dry CH₂Cl₂ (40 mL) was treated with T3P (50% in EtOAc, 7.2 mL, 12.1 mmol) and *i*-Pr₂NEt (4.2 mL, 24.3 mmol) in dry CH₂Cl₂ (1160 mL) to give after FC (CH₂Cl₂/MeOH 100:0 to 95:5) macrolactam **Ex.104** (2.32 g, 60%).

Data of **Ex.104:** C₃₃H₄₁FN₄O₈ (640.7). LC-MS (method 7): Rₜ = 1.21 (47), 641 ([M+H]⁺); 1.24 (53), 641 ([M+H]). ¹H-NMR (DMSO-d₆): complex spectrum, mixture of isomers, 7.44-6.65 (m, 9 H), 5.32-5.05 (m, 2 H), 4.70-3.30 (several m, 9 H), 2.92 (s, NCH₃ of major isomer), 2.84 (s, NCH₃ of major isomer), 2.30-1.70 (several m, 6 H), 1.40, 1.38 (2 s, 9 H).

### Synthesis of acid Ex.105

According to procedure **H,** ester **Ex.104** (2.15 g, 3.3 mmol) was hydrogenolyzed in MeOH (215 mL) in the presence of catalyst (1.07 g) for 4 h to afford acid **Ex.105** (1.72 g, 93%).

Data of **Ex. 105:** C₂₆H₃₅FN₄O₈ (550.6). LC-MS: (method 7): Rₜ = 0.91 (45), 551 ([M+H]⁺); 0.95 (38), 551 ([M+H]⁺).

### Core 07: Synthesis of Ex.115 and Ex.116 (Scheme 11)

### Synthesis of Mitsunobu product 97

A mixture of the phenol **54** (6.42 g, 14.9 mmol), alcohol **22** (4.04 g, 13.5 mmol), and PPh₃ (9.73 g, 37.1 mmol) was dried i.v. for 15 min. and dissolved in dry, degassed chloroform (130 mL). The soln was cooled to 0°C. A soln of ADDP (9.36 g, 37.1 mmol) in chloroform (20 mL) was slowly added. The mixture was stirred at rt for 3 h followed by the addition of more **22** (4.04 g, 13.5 mmol) and PPh₃ (5.97 g, 22.8 mmol) in chloroform (20 mL). Again, the mixture was cooled to 0°C; and a soln of ADDP (5.74 g, 22.7 mmol) in chloroform (20 mL) was slowly added. The soln was stirred at rt for 16 h and concentrated. The residue was suspended in Et₂O and filtered. The solid was washed with Et₂O. The combined filtrate and washings were concentrated. FC (CH₂Cl₂/EtOAc 10:1) gave **97** (7.73 g, 73%).

### Synthesis of amino acid 98

Following procedure **B.2,** the reaction of **97** (7.72 g, 11 mmol), 1,3-dimethylbarbituric acid (4.1 g, 26.0 mmol) and Pd(PPh₃)₄ (0.63 g) in EtOAc/CH₂Cl₂ (53:47, 190 mL) yielded after 2 h and after FC (EtOAc, then CH₂Cl₂/MeOH 95:5 to 90:10) amino acid **98** (4.31 g, 67%).

Data of **98:** C₃₀H₃₈FN₃O₈ (587.6). HPLC (10% CH₃CN) : Rₜ = 3.86 (84). LC-MS (method 9a): Rₜ = 1.76; 588 ([M+H]⁺), 488 ([M+H-Boc]⁺).

### Synthesis of the Alloc protected amino acid 99

Following procedure **C.1,** the reaction of amino acid **98** (4.3 g, 7.3 mmol), allyl chloroformate (0.86 mL, 8.0 mmol) and Na₂CO₃ (1.2 g, 11 mmol) in dioxane (62 mL) and H₂O (60 mL) gave acid **99** (5.07 g, 100%).

### Synthesis of the protected diamide 100

Following procedure **C.2,** acid **99** (4.9 g, 7.3 mmol) was reacted with sarcosine allylester p-toluenesufonate (**46˙**p-TsOH, 2.6 g, 8.8 mmol), HOAt (1.5 g, 11 mmol), HATU (4.2 g, 11 mmol) and *i-*Pr₂NEt (6.2 mL, 36 mmol) in DMF (75 mL) to afford the protected amino acid **100** (4.37 g, 76%).

Data of **100:** C₄₀H₅₁FN₄O₁₁ (782.8). HPLC (50% CH₃CN) : Rₜ = 3.56 (99). LC-MS (method 9a): Rₜ = 2.45; 783 ([M+H]⁺), 683 ([M+H-Boc]⁺).

### Synthesis of the deprotected amino acid 101

Following procedure **C.3,** the reaction of the protected amino acid **100** (4.36 g, 5.6 mmol), 1,3-dimethylbarbituric acid (2.1 g, 13 mmol) and Pd(PPh₃)₄ (0.32 g) in EtOAc/CH₂Cl₂ (45:55, 106 mL) yielded amino acid **101** (3.46 g, 93%).

Data of **101:** C₃₃H₄₃FN₄O₉ (658.7). LC-MS (method 9b): Rₜ = 1.74; 659 ([M+H]⁺), 559 ([M+H-Boc]⁺).

### Synthesis of the protected macrolactam Ex.115

According to procedure **F.1.1,** amino acid **101** (3.44 g, 5.2 mmol) in dry CH₂Cl₂ (50 mL) was treated with T3P (50% in EtOAc, 6.2 mL, 10 mmol) and *i*-Pr₂NEt (3.6 mL, 21 mmol) in dry CH₂Cl₂ (470 mL) to give after FC (CH₂Cl₂/MeOH 95:5) macrolactam **Ex.115** (2.95 g, 90%).

Data of **Ex.115:** C₃₃H₄₁FN₉O₈ (640.7). HPLC (20% CH₃CN) : Rₜ = 4.05 (93). LC-MS (method 9c): Rₜ = 2.08; 641 ([M+H]⁺). ¹H-NMR (DMSO-d₆): complex spectrum, mixture of isomers, 7.38 (s, 5 H), 7.35-6.95 (several m, 2 H), 6.81-6.72 (several m, 0.4 H), 6.64 (dd, J = 3.1, 8.2, 0.25 H), 6.39 (dd, J = 3.2, 7.7, 0.25 H), 6.30 (dd, J = 3.3, 8.2, 0.1 H), 5.37-4.99 (m, 3 H), 4.60-3.60 (several m, 9 H), 3.20-2.60 (several m and s, 8 H), 2.40-1.70 (several m, 4 H), 1.45, 1.43, 1.42, 1.38 (4 s, Boc).

### Synthesis of acid Ex.116

According to procedure **H,** ester **Ex.115** (1.2 g, 1.9 mmol) was hydrogenolyzed in MeOH (120 mL) in the presence of catalyst (0.6 g) for 2 h to afford acid **Ex.116** (1.02 g, 99%).

Data of **Ex.116:** C₂₆H₃₅FN₄O₈ (550.6). HPLC (10% CH₃CN) : Rₜ = 3.47 (20), 3.55 (75). LC-MS: (method 9c): Rₜ = 1.53, 1.58; 551 ([M+H]⁺).

### Core 08: Synthesis of Ex.132 and Ex.133 (Scheme 12)

### Synthesis of Mitsunobu product 102

Following procedure **E.1.2,** the reaction of phenol **56** (2.0 g, 4.7 mmol), alcohol **84** (2.08 g, 5.6 mmol), and CMBP (2.25 g, 9.3 mmol) in dry toluene (80 mL) afforded after 3 h and after FC (hexane/EtOAc 1:1 to 1:2) the protected amino acid **102** (2.06 g, 56%).

### Synthesis of amino acid 103

Following procedure **E.2,** the reaction of **102** (2.05 g, 2.6 mmol), 1,3-dimethylbarbituric acid (1.0 g, 6.3 mmol) and Pd(PPh₃)₄ (0.15 g) in EtOAc/CH₂Cl₂ (55:45; 45 mL) yielded after 2 h and after FC (EtOAc, then CH₂Cl₂/MeOH 95:5 to 70:30) amino acid **103** (1.45 g, 85%).

Data of **103:** C₃₃H₄₃FN₄O₉ (658.7). HPLC (5% CH₃CN): Rₜ = 4.04 (97). LC-MS (method 9c): Rₜ = 1.87, 659 ([M+H]⁺).

### Synthesis of the protected macrolactam Ex.132

According to procedure **F.1.1,** the amino acid **103** (1.44 g, 2.19 mmol) in dry CH₂Cl₂ (40 mL) was treated with T3P (50% in EtOAc, 2.6 mL, 4.37 mmol) and *i*-Pr₂NEt (1.5 mL, 8.74 mmol) in dry CH₂Cl₂ (170 mL) to give after FC (CH₂Cl₂/MeOH 95:5) macrolactam **Ex.132** (1.36 g, 96%).

Data of **Ex. 132:** C₃₃H₄₁FN₄O₈ (640.7). LC-MS (method 2) : Rₜ = 1.93 (100), 641 ([M+H]⁺); LC-MS (method 9c): Rₜ = 2.12, 641 ([M+H]⁺). ¹H-NMR (DMSO-d₆): complex spectrum, mixture of isomers, 7.38 (s, 5 H), 7.35-6.99 (several m, 2 H), 6.85-6.73 (several m, 0.4 H), 6.65 (dd, J = 3.1, 8.2, 0.25 H), 6.39 (dd, J = 3.1, 7.9, 0.25 H), 6.30 (dd, J = 3.3, 8.1, 0.1 H), 5.37-4.99 (m, 3 H), 4.6-3.6 (several m, 9 H), 3.2-2.6 (several m and s, 8 H), 2.4-1.7 (several m, 4 H), 1.45, 1.43, 1.41, 1.38 (4 s, Boc).

### Synthesis of acid Ex.133

According to procedure **H,** ester **Ex.132** (1.13 g, 1.7 mmol) was hydrogenolyzed in MeOH (110 mL) in the presence of catalyst (0.56 g) for 4 h to afford acid **Ex.133** (0.92 g, 94%).

Data of **Ex.133:** C₂₆H₃₅FN₉O₈ (550.6). HPLC (5% CH₃CN) : Rₜ = 3.65 (27), 3.72 (71). LC-MS: (method 9c): Rₜ = 1.53, 551 ([M+H]⁺); 1.57, 551 ([M+H]⁺).

### Core 09: Synthesis of Ex.142 and Ex.143 (Scheme 13)

### Synthesis of Mitsunobu product 104

Following procedure **E.1.2,** the reaction of phenol **54** (3.1 g, 7.2 mmol), alcohol **86** (3.34 g, 8.7 mmol), and CMBP (3.49 g, 14.4 mmol) in dry toluene (123 mL) afforded after 3 h and after FC (hexane/EtOAc 1:1 to 1:2) the protected amino acid **104** (4.11 g, 71%).

### Synthesis of amino acid 105

Following procedure **E.2,** the reaction of **104** (4.07 g, 5.1 mmol), 1,3-dimethylbarbituric acid (1.9 g, 12 mmol) and Pd(PPh₃)₄ (0.3 g) in EtOAc/CH₂Cl₂ (45:55, 90 mL) yielded after 2 h and after FC (EtOAc, then CH₂Cl₂/MeOH 95:5 to 70:30) amino acid **105** (3.19 g, 93%).

Data of **105:** C₃₄H₄₅FN₄O₉ (672.7). HPLC (5% CH₃CN): Rₜ = 3.96 (88). LC-MS (method 9c): Rₜ = 1.83, 673 ([M+H]⁺).

### Synthesis of the protected macrolactam Ex.142

According to procedure **F.1.1,** amino acid **105** (2.4 g, 3.6 mmol) in dry CH₂Cl₂ (40 mL) was treated with T3P (50% in EtOAc, 4.2 mL, 7.1 mmol) and *i*-Pr₂NEt (2.4 mL, 14.2 mmol) in dry CH₂Cl₂ (300 mL) to give after FC (CH₂Cl₂/MeOH 95:5) macrolactam **Ex.142** (1.92 g, 82%).

Data of **Ex.142:** C₃₄H₄₃FN₉O₈ (654.7). HPLC (30% CH₃CN) : Rₜ = 3.50 (89). LC-MS (method 9b): Rₜ = 2.01; 655 ([M+H]⁺), 599 ([M+H-tBu]⁺), 555 ([M+H-Boc]⁺). ¹H-NMR (DMSO-d₆): complex spectrum, mixture of isomers, 7.41-7.38 (m, 5 H), 7.37-7.14 (m, 3 H), 6.80-6.67 (m, 1 H), 5.45-5.13 (m, 3 H), 4.60-3.30 (several m, 8 H), 3.10-2.50 (several m and s, 8 H), 2.50-1.80 (several m, 6 H), 1.39, 1.38, 1.36 (3 s, Boc).

### Synthesis of acid Ex.143

According to procedure **H,** ester **Ex.142** (1.07 g, 1.6 mmol) was hydrogenolyzed in MeOH (100 mL) in the presence of catalyst (0.53 g) for 4 h to afford acid **Ex.143** (0.92 g, 99%).

Data of **Ex.143:** C₂₇H₃₇FN₄O₈ (564.6). LC-MS: (method 2): Rₜ = 1.54 (91), 565 ([M+H]⁺).

### Core 10: Synthesis of Ex.164 and Ex.165 (Scheme 14)

### Synthesis of Mitsunobu product 106

Following procedure **B.1.2,** the reaction of phenol **63** (4.2 g, 9.8 mmol), alcohol **16** (4.4 g, 13 mmol), and CMBP (4.8 g, 20 mmol) in dry toluene (120 mL) afforded after 4 h and FC (hexane/EtOAc 50:50) the protected amino acid **106** (6.37 g, 86%).

### Synthesis of amino acid 107

Following procedure **B.2,** the reaction of **106** (1.18 g, 1.6 mmol), 1,3-dimethylbarbituric acid (0.6 g, 3.8 mmol) and Pd(PPh₃)₄ (90 mg) in EtOAc/CH₂Cl₂ (60:40, 15 mL) yielded after 3 h and after FC (CH₂Cl₂/EtOH 100:0 to 80:20) the amino acid **107** (0.86 g, 87%).

Data of **107:** C₃₁H₄₉N₄O₈Si (628.8). LC-MS: (method 6): Rₜ = 1.08 (88), 629 ([M+H]⁺).

### Synthesis of the protected macrolactam Ex.164

According to procedure **F.1.2,** amino acid **107** (310 mg, 0.49 mmol) in dry DMF (5 mL) was treated with FDPP (379 mg, 0.99 mmol) in dry DMF (500 mL) to afford after FC (hexane/EtOAc/MeOH 50:50:0 to 0:95:5) macrolactam **Ex.164** (131 mg, 43%).

Data of **Ex. 164**: C₃₁H₄₂N₄O₇Si (610.8). LC-MS: (method 7): Rₜ = 1.34 (98), 611 ([M+H]⁺). ¹H-NMR (DMSO-d₆): 7.42-7.27 (m, 8 H), 6.98 (dd, J = 1.4, 8.2, 1 H), 6.91 (d, J = 7.5, 1 H), 6.84 (s, 1 H), 4.98 (s, 2 H), 4.50 (d, J = 11.9, 1 H), 4.35-4.15 (m, 3 H), 4.0.6-3.96 (m, 4 H), 3.21 (m, 1 H), 3.10-2.95 (m, 2 H), 2.87 (s, 3 H), 2.30-1.80 (m, 4 H), 0.91 (t, J = 8.3, 2 H), 0.00 (s, 9 H).

### Synthesis of amine Ex.165

At 0°C a soln of TBAF in THF (1 M, 3.9 mL, 3.9 mmol) was added to a soln of **Ex.164** (1.2 g, 1.96 mmol) in THF (42 mL). The soln was stirred at 0°C to rt for 15 h, followed by the addition of TBAF in THF (1 M, 1.18 mL, 1.18 mmol). Stirring was continued for 2 h. The soln was distributed between CH₂Cl₂ and H₂O. The aqueous phase was repeatedly extracted with CH₂Cl₂. The combined organic phase was dried (Na₂SO₄) , filtered and concentrated. FC (CH₂Cl₂/MeOH 100:0 to 90:10) afforded **Ex.165** (0.76 g, 83%).

Data of **Ex.165:** C₂₅H₃₀N₄O₅ (466.52). LC-MS: (method 4a) : Rₜ = 1.49 (99), 467 ([M+H]⁺).

### Core 11a: Synthesis of Ex.181 and Ex.182 (Scheme 15)

### Synthesis of Mitsunobu product 108

Following procedure **B.1.2,** the reaction of phenol **65** (10.7 g, 24 mmol), alcohol **16** (10.0 g, 29 mmol), and CMBP (12.0 g, 49 mmol) in dry toluene (362 mL) afforded after FC (hexane/EtOAc 50:50 to 70:30) the protected amino acid **108** (14.55 g, 78%).

### Synthesis of amino acid 109

Following procedure **B.2,** the reaction of **108** (14.50 g, 19 mmol), 1,3-dimethylbarbituric acid (7.0 g, 47.0 mmol) and Pd(PPh₃)₄ (0.1 g) in EtOAc/CH₂Cl₂ (55:45, 203 mL) yielded after 3 h and after FC (CH₂Cl₂/MeOH 99:1 to 90:10) amino acid **109** (11.26 g, 92%).

Data of **109:** C₃₂H₄₆N₉O₈Si (642.8). LC-MS: (method 6): Rₜ = 1.13 (94), 643 ([M+H]⁺).

### Synthesis of the protected macrolactam Ex.181

According to procedure **F.1.1,** amino acid **109** (4.0 g, 6.2 mmol) in dry CH₂Cl₂ (100 mL) was treated with T3P (50% in EtOAc, 7.4 mL, 12.4 mmol) and *i*-Pr₂NEt (4.3 mL, 24.8 mmol) in dry CH₂Cl₂ (560 mL). Prior to aqueous workup, the CH₂Cl₂ was replaced by EtOAc. FC (hexane/EtOAc 50:50 to 0:100) afforded the macrolactam **Ex.181** (2.11 g, 54%).

Data of **Ex.181**: C₃₂H₄₄N₄O₇Si (624.8). LC-MS (method 7): Rₜ = 1.37 (99), 625 ([M+H]⁺). ¹H-NMR (DMSO-d₆): 7.46 (d, J = 8.0, 1 H), 7.42 (d, J = 7.2, 1H), 7.34-7.23 (m, 6 H), 7.06 (d, J = 8.2, 1 H), 6.82 (d, J = 7.4, 1 H), 6.78 (s, 1 H), 5.02-4.86 (m, 3H), 4.13 (t, J = 8.5, 1 H), 4.06-3.67 (m, 7 H), 3.05 (br. m, 1 H), 2.88 (br. m, 1 H), 2.88 (s, 3 H), 2.15 (m, 2 H), 1.51 (br. m, 2 H), 1.33 (br. m, 1 H), 1.12 (br. m, 1 H), 0.91 (t-like m, J ca. 8.4, 2 H), 0.00 (s, 9 H).

### Synthesis of amine Ex.182

According to procedure **I.2,** carbamate **Ex.181** (844 mg, 1.3 mmol) in THF (34 mL) was treated with TBAF soln (4.1 mL) to afford after FC (CH₂Cl₂/MeOH 90:10) amine **Ex.182** (620 mg, 95%)

Data of **Ex.182:** C₂₆H₃₂N₄O₅ (480.5). LC-MS: (method 2): Rₜ = 1.35 (99), 481 ([M+H]⁺).

### Core 11b: Synthesis of Ex.560 and Ex.561 (Scheme 15)

### Synthesis of Mitsunobu product 214

Following procedure **B.1.2,** the reaction of phenol **65** (2.60 g, 5.9 mmol), alcohol **156** (1.96 g, 7.08 mmol) and CMBP (2.85 g, 11.8 mmol) in toluene (84 mL) afforded after 3 h and after FC (hexane/EtOAc gradient) **214** (3.85 g, 93%).

### Synthesis of amino acid 215

Following procedure **B.2,** the reaction of **214** (3.8 g, 5.4 mmol), 1,3-dimethylbarbituric acid (2.12 g, 13.6 mmol) and Pd(PPh₃)₄ (31 mg) in EtOAc/CH₂Cl₂ (1:1, 50 mL) afforded after 20 h at rt, evaporation of the volatiles and repeated washing (EtOAc) the solid **215** (3.04 g, 97%).

Data of **215:** C₃₂H₃₇N₃O₇ (575.6). LC-MS: (method 4a): Rₜ = 1.81 (84), 576 ([M+H]⁺).

### Synthesis of macrolactam Ex.560

FDPP (1.73 g, 4.5 mmol) was added to a suspension of amino acid **215** (1.51 g, 2.62 mmol) in dry DMF (460 mL). The mixture was stirred at rt for 20 h and concentrated. FC (hexane/EtOAc gradient) afforded **Ex.560** (0.92 g, 63%).

Data of **Ex.560:** C₃₂H₃₅N₃O₆ (557.6). LC-MS: (method 4b): Rₜ = 2.29 (95), 558 ([M+H]⁺). ¹H-NMR (DMSO-d₆): 7.48 (d, J = 7.8, 1 H), 7.37-7.26 (m, 8 H), 7.06-6.94 (m, 4 H), 6.85-6.76 (m, 2 H), 5.01 (s, 2 H), 4.94-4.82 (m, 2 H), 4.39 (t-like m, 1 H), 4.14-4.04 (m, 2 H) , 3.95 (q-like m, 1 H), 3.10 (m, 1 H), 3.00-2.82 (m, 2 H), 2.91 (s, 3 H), 2.58 (m, 1 H), 2.19 (td, J = 6.4, 13.3, 1H), 1.65-1.50 (m, 2 H), 1.38 (m, 1 H), 1.17 (m, 1 H).

### Synthesis of amine Ex.561

A soln of the benzyl carbamate **Ex.560** (0.87 g, 1.55 mmol) in MeOH (25 mL) was hydrogenolyzed in the presence of 10% palladium on activated charcoal (185 mg) at rt and normal pressure for 4 h. The mixture was filtered through a pad of celite. The filtrate was concentrated. FC (CH₂Cl₂/MeOH 80:20) afforded **Ex.561** (0.65 g, 99%).

Data of **Ex.561:** C₂₄H₂₉N₃O₄ (423.5). LC-MS: (method 4a): Rₜ = 1.56 (98), 424 ([M+H]⁺).

### Core 11c: Synthesis of Ex.567 and Ex.568 (Scheme 15)

### Synthesis of Mitsunobu product 216

Following procedure **B.1.2,** the reaction of phenol **65** (3.0 g, 6.8 mmol), alcohol **161** (3.0 g, 8.2 mmol) and CMBP (3.8 g, 16 mmol) in toluene (76 mL) afforded after 1.5 h and after FC (hexane/EtOAc/MeOH gradient) **216** (3.88 g, 71%).

### Synthesis of amino acid 217

Following procedure **B.2,** the reaction of **216** (25.4 g, 32 mmol), 1,3-dimethylbarbituric acid (13 g, 80 mmol) and Pd(PPh₃)₄ (1.9 g) in EtOAc/CH₂Cl₂ (50:50, 410 mL) afforded after 20 h at rt, filtration and washing (CH₂Cl₂) the solid **217** (18.05 g, 84%). Data of **217:** C₃₃H₃₉BrN₃O₇ (668.6). LC-MS: (method 10a): Rₜ = 1.75 (88), 668/670 ([M+H]⁺).

### Synthesis of macrolactam Ex.567

According to procedure **F.1.1,** a mixture of **217** (1.95 g, 2.9 mmol) and *i*-Pr₂NEt (2 mL, 12 mmol) in CH₂Cl₂ (85 mL) was treated with T3P (50% in EtOAc, 4.3 mL, 7.3 mmol) in CH₂Cl₂ (440 mL). After stirring at rt for 20 h, evaporation of the volatiles, aqueous workup (CH₂Cl₂, sat. aq. NaHCO₃ soln; Na₂SO₄) and FC (hexane/EtOAc 25:75) **Ex.567** (1.1 g, 57%) was obtained.

Data of **Ex.567:** C₃₃H₃₆BrN₃O₆ (650.5). LC-MS: (method 10b): Rₜ = 2.33 (96), 652/650 ([M+H]⁺). ¹H-NMR (DMSO-d₆): 7.56 (d, J = 8.4, 2 H), 7.42 (d, J = 7.8, 1H), 7.34-7.27 (m, 8 H), 6.90 (dd, J = 1.9, 10.1, 1 H), 6.84 (d, J = 7.5, 1 H), 6.78 (s, 1 H), 5.00 (s, 2 H), 4.81 (d, J = 12.7, 1 H), 4.48 (d, J = 12.2, 1 H), 4.43 (d, J = 12.2, 1 H), 4.27 (t-like m, 1 H), 4.07-3.89 (m, 4 H), 3.07 (m, 1 H), 2.93 (m, 1 H), 2.93 (s, 3 H), 2.70 (dd, J = 7.3, 10.3, 1 H), 2.30 (m, 1 H), 2.10 (m, 1 H), 1.65-1.50 (m, 2 H), 1.36 (m, 1 H), 1.17 (m, 1 H).

### Synthesis of amine Ex.568

A soln of **Ex.567** (1.5 g, 2.3 mmol) in MeOH (31 mL) was treated with NH₃ (7 M in MeOH; 1 mL, 7 mmol) and hydrogenolyzed at rt and normal pressure in the presence of 5% palladium on activated charcoal (moistened with 50% H₂O; 400 mg) for 15 h. The mixture was filtered through a pad of celite. The filtrate was concentrated, dissolved in CH₂Cl₂, washed (sat. aq. NaHCO₃ soln), filtered and concentrated to afford **Ex.568** (0.98 g, 97%).

Data of **Ex.568:** C₂₅H₃₁N₃O₄ (437.5). LC-MS: (method 10a): Rₜ = 1.41 (96), 438 ([M+H]⁺).

### Core 11d: Synthesis of Ex.586 and Ex.587 (Scheme 15)

### Synthesis of Mitsunobu product 218

Following procedure **B.1.2,** the reaction of the phenol **65** (0.94 g, 2.1 mmol), alcohol **165** (0.705 g, 2.5 mmol) and CMBP (1.03 g, 4.27 mmol) in toluene (22 mL) afforded after 1.5 h and after FC (hexane/EtOAc 1:1) **218** (1.2 g, 80%).

### Synthesis of amino acid 219

Following procedure **B.2,** the reaction of **218** (1.19 g, 1.17 mmol), 1,3-dimethylbarbituric acid (0.64 g, 4.1 mmol) and Pd(PPh₃)₄ (0.1 g) in EtOAc/CH₂Cl₂ (55:45, 29 mL) afforded after 2 h and after FC (EtOAc, then CH₂Cl₂/MeOH 100:0 to 80:20) **219** (0.88 g, 89%).

Data of **219:** C₃₃H₃₉N₃O₆ (573.7) . LC-MS: (method 10a): Rₜ = 1.67 (87), 574 ([M+H]⁺).

### Synthesis of macrolactam Ex.586

According to procedure **F.1.1,** a mixture of **219** (0.87 g, 1.5 mmol) in CH₂Cl₂ (30 mL) was treated with T3P (50% in EtOAc, 2.7 mL, 4.5 mmol) and *i*-Pr₂NEt (1.0 mL, 6 mmol) in CH₂Cl₂ (300 mL). After stirring at rt for 0.5 h, aqueous workup (CH₂Cl₂, sat. aq. Na₂CO₃ soln; Na₂SO₄) and FC (hexane/EtOAc 25:75), **Ex.586** (0.313 g, 37%) was obtained.

Data of **Ex.586:** C₃₃H₃₇N₃O₅ (555.6). LC-MS: (method 10b): Rₜ = 2.22 (96), 556 ([M+H]⁺). ¹H-NMR (DMSO-d₆) : 7.43 (d, J = 8.1, 1 H), 7.39-7.18 (m, 11 H), 6.86-6.79 (m, 3 H), 4.95-4.85 (m, 3 H), 4.14 (br. t, J = 8. 4, 1 H), 3.91 (d, J = 11.3, 1 H), 3.76 (br. q, J = 7.2, 1 H), 3.55 (t-like m, 1 H), 3.08 (m, 1 H), 2.93 (m, 1 H), 2.91 (s, 3 H), 2.67 (d, J = 6.5, 2 H), 2.37 (t, J = 10.4, 1 H), 2.25 (m, 1 H), 2.01-1.93 (m, 2 H), 1.65-1.50 (m, 2 H), 1.32 (m, 1 H), 1.15 (m, 1 H).

### Synthesis of amine Ex.587

According to procedure **K,** the benzyl carbamate **Ex.586** (279 mg, 0.5 mmol) was hydrogenolyzed in MeOH (17 mL) in the presence of the catalyst (56 mg) for 3 h to afford **Ex.587** (203 mg, 96%). Data of **Ex.587:** C₂₅H₃₁N₃O₃ (421.5). LC-MS (method 10a) : Rₜ = 1.47 (99), 422 ([M+H]⁺).

### Core 12: Linear synthesis of Ex.196 and Ex.197 (Scheme 16)

### Synthesis of Mitsunobu product 110

Following procedure **B.1.1,** the reaction of phenol **59** (5.22 g, 12.6 mmol), alcohol **16** (5.2 g, 15.2 mmol), PPh₃ (5.0 g, 19 mmol) in dry benzene (124 mL) and DEAD (40% in toluene, 7.0 mL, 15.2 mmol) in dry benzene (36 mL) afforded after FC (hexane/EtOAc 60:40 to 40:60) the protected amino acid **110** (8.3 g, 88%, contaminated with triphenylphosphine oxide, used in the next stop without further purification).

### Synthesis of amino acid 111

Following procedure **B.2,** the reaction of **110** (4.15 g, 5.62 mmol), 1,3-dimethylbarbituric acid (2.19 g, 14.0 mmol) and Pd(PPh₃)₄ (0.71 g) in EtOAc/CH₂Cl₂ 1:1 (60 mL) yielded after 1 h and after FC (CH₂Cl_{2/}EtOH 95:5 to 90:10 then CH₂Cl_{2/}MeOH 90:10 to 70:30) amino acid **111** (2.75 g, 80%).

Data of **111:** C₃₀H₄₂N₄O₈Si (614.8). HPLC (10% CH₃CN) : R_{t =} 3.82 (99). LC-MS (method 9a): Rₜ = 1.81, 615 ([M+H]⁺).

### Synthesis of the alloc protected amino acid 112

Following procedure **C.1,** the reaction of amino acid **111** (1.5 g, 2.4 mmol ) , allyl chloroformate (0.29 mL, 2.68 mmol) and Na₂CO₃ (0.72 g, 6.83 mmol) in dioxane (40 mL) and H₂O (40 mL) gave acid **112** (1.7 g, 100%).

### Synthesis of the protected amino acid 113

Following procedure **C.2,** the acid **112** (1.7 g, 2.4 mmol) was reacted with sarcosine allylester p-toluenesufonate (**46**˙p-TsOH, 0.88 g, 2.9 mmol), HOAt (0.5 g, 3.6 mmol), HATU (1.4 g, 3.6 mmol) and *i*-Pr₂NEt (2.1 mL, 12 mmol) in DMF (25 mL) to afford the protected amino acid **113** (1.51 g, 75%).

Data of **113:** C₄₀H₅₅N₅O₁₁Si (809.9). HPLC (40% CH₃CN): Rₜ = 4.43 (91). LC-MS (method 9c): Rₜ = 2.51, 810 ([M+H]⁺).

### Deprotection to amino acid 114

Following procedure **C.3,** the reaction of the protected amino acid **113** (1.5 g, 1.85 mmol), 1,3-dimethylbarbituric acid (0.72 g, 4.6 mmol) and Pd(PPh₃)₄ (0.23 g) in EtOAc/CH₂Cl₂ (1:1, 25 mL) yielded amino acid **114** (1.05 g, 83%).

Data of **114:** C₃₃H₄₇N₅O₉Si (685.8). HPLC (10% CH₃CN): Rₜ = 3.85 (95). LC-MS (method 9c): Rₜ = 1.78, 686 ([M+H]⁺).

### Synthesis of the protected macrolactam Ex.196

According to procedure **F.1.2,** amino acid **114** (1.0 g, 1.46 mmol) in dry DMF (20 mL) was treated with FDPP (1.12 g, 2.92 mmol) in dry DMF (130 mL) to yield after FC (EtOAc) the macrolactam **Ex.196** (0.61 g, 63%).

Data of **Ex.196:** C₃₃H₄₅N₅O₈Si (667.8). LC-MS (method 1a): Rₜ = 2.66 (100), 668 ([M+H]⁺). LC-MS (method 9c): Rₜ = 2.12, 668 ([M+H]⁺), 640. ¹H-NMR (CDCl₃) : 7.34-7.26 (m, 6 H), 7.17 (d, J = 7.6, 1 H), 7.02 (s, 1 H), 6.91 (d, J = 9.5, 1 H), 5.49 (d, J = 9.5, 2 H), 5.10 (m, 1 H), 5.06 (s, 2 H), 4.39-4.13 (m, 5 H), 4.00-3.95 (m, 2 H), 3.65 (m, 1 H), 3.36 (br. s, 2 H), 3.14 (m, 2 H), 3.09 (s, 3 H), 2.74 (s, 3 H), 2.45 (m, 1 H), 2.08 (m, 1 H), 0.98 (m, 2 H), 0.00 (s, 9 H). ¹H-NMR (DMSO-d₆) : 7.98 (d, J = 9.9, 1 H), 7.52 (d, J = 7.9, 1 H), 7.36-7.27 (m, 6 H), 7.18 (s, 1 H), 7.06 (dd, J = 1.8, 8.1, 1 H), 6.83 (d, J = 7.5, 1 H), 5.12 (d, J = 12.5, 1 H), 5.04 (d, J = 12.5, 1 H), 4.87 (d, J = 8.8, 1 H), 4.25-3.89 (m, 8 H), 3.71-3.66 (m, 2 H), 3.20 (m, 1 H), 3.02 (m, 1 H), 2.97 (s, 3 H), 2.65 (s, 3 H), 2.20 (m, 1 H), 2.09 (m, 1 H), 0.92 (t, J = 8.2, 2 H), 0.00 (s, 9 H).

### Synthesis of amine Ex.197

According to procedure **I.1,** carbamate **Ex.196** (120 mg, 0.18 mmol) in dioxane (3 mL) was treated with 4M HCl-dioxane (3 mL) to afford **Ex.197H˙**HCl (59 mg, 58%).

Data of **Ex.197˙**HCl**:** C₂₇H₃₃N₅O₆˙HCl (523.5, free base). HPLC (5% CH₃CN) : Rₜ = 3.05 (83). LC-MS (method 9c): Rₜ = 1.12, 524 ([M+H]⁺). ¹H-NMR (DMSO-d₆) : 8.53 (br. s, NH₃⁺), 8.03 (d, J = 9.9, 1 H), 7.41-7.31 (m, 7 H), 7.15 (m, 1 H), 6.85 (d, J = 7.5, 1 H), 5.14 (d, J = 12.5, 1 H), 5.04 (d, J = 12.5, 1 H), 4.86 (dd, J ca. 2.2, 11.0, 1 H), 4.42-4.13 (m, 2 H), 4.05 (t, J = 8.5, 1 H), 3.96 (d, J = 17.8, 1 H), 3.85-3.75 (m, 2 H), 3.65 (br. m, 1 H), ca. 3.3-3.1 (m, 3 H, partially superimposed by the H₂O signal), 2.97 (s, 3 H), 2.67 (s, 3 H), 2.42 (m, 1 H), 2.18 (br. q, J ca. 11.1, 1 H).

### Core 12: Convergent synthesis of Ex.197 and Ex.198 (Scheme 17)

### Synthesis of Mitsunobu product 115

Following procedure **E.1.1,** phenol **59** (4.6 g, 11 mmol) was treated for 40 h with alcohol **81** (5.0 g, 13 mmol), DEAD (40% in toluene, 6.1 mL, 13 mmol) and PPh₃ (4.4 g, 17 mmol) in dry benzene (150 mL). After 2h and after 18 h, additional PPh₃ (1.82 g, 6.9 mmol), alcohol **81** (2.04 g, 5.5 mmol) in benzene (50 mL), and DEAD (40% in toluene, 2.55 mL, 5.6 mmol) in benzene (13 mL) were added. FC (hexane/EtOAc 50:50 to 90:10) afforded the protected amino acid **115.1** (2.5 g, 29%). Following procedure **E.1.2,** the reaction of phenol **59** (2.9 g, 7.0 mmol), alcohol **81,** (5.7 g, 15 mmol) and CMBP (5.1 g, 21 mmol) in dry toluene (121 mL) afforded after FC (hexane/EtOAc 20:80 to 90:10) the protected amino acid **115.2** (2.92 g, 54%).

### Synthesis of amino acid 116

Following procedure **E.2,** the reaction of **115.1** (3.17 g, 4.14 mmol), 1,3-dimethylbarbituric acid (1.62 g, 10.3 mmol) and Pd(PPh₃)₄ (0.53 g) in EtOAc/CH₂Cl₂ (1:1, 46 mL) yielded after 1 h and after FC (CH₂Cl₂/MeOH 90:10 to 70:30) the amino acid **116.1** (1.86 g, 70%).

Data of **116.1:** C₃₂H₄₃N₅O₉ (641.7). HPLC (5% CH₃CN) : Rₜ = 3.65 (100). LC-MS (method 9c): Rₜ = 1.60, 642 ([M+H]⁺).

Following procedure **E.2,** the reaction of **115.2** (2.9 g, 3.8 mmol), 1,3-dimethylbarbituric acid (1.5 g, 9.5 mmol) and Pd(PPh₃)₄ (0.48 g) in EtOAc/CH₂Cl₂ (1:1, 46 mL) yielded after 1 h and after FC (CH₂Cl₂/MeOH 90:10 to 70:30) the amino acid **116.2** (2.0 g, 83%).

Data of **116.2:** C₃₂H₄₃N₅O₉ (641.7). HPLC (5% CH₃CN) : Rₜ = 3.73 (98). LC-MS (method 9c): Rₜ = 1.61, 642 ([M+H]⁺).

### Synthesis of the protected macrolactam Ex.198

According to procedure **F.1.1,** the amino acid **116.1** (1.0 g, 1.6 mmol) in dry CH₂Cl₂ (200 mL) was treated with T3P (50% in EtOAc, 1.8 mL, 3.1 mmol) and *i*-Pr₂NEt (1.1 mL, 6.2 mmol) in dry CH₂Cl₂ (1400 mL) to afford after FC (EtOAc/MeOH 95:5 to 80:20) the macrolactam **Ex.198** (containing 15% of the epimer **Ex.231;** 0.38 g, 39%).
Data of **Ex.198**: C₃₂H₄₁N₅O₈ (623.7). LC-MS: (method 2): Rₜ = 1.78 (84), 624 ([M+H]⁺); 1.82 (15). LC-MS (method 9c): Rₜ = 1.87, 624 ([M+H]⁺).
¹H-NMR (CDCl₃): 7.42-7.25 (m, 7 H), 7.07 (s, 1 H), 7.00 (d, J = 8.2, 1 H), 5.59 (d, J = 9.5, 1 H), 5.38 (br. d, J ca. 7.9, 1 H), 5.18 (dd, J = 2.5, 12.2, 1 H), 5.13 (s, 2 H), 4.43-4.01 (m, 5 H), 3.73 (m, 1 H), 3.47 (d, J = 17.7, 1 H), 3.33 (d, J = 17.7, 1 H), 3.20-3.11 (m, 2 H), 3.17 (s, 3 H), 2.81 (s, 3 H), 2.50 (m, 1 H), 2.15 (m, 1 H), 1.51 (s, Boc, major isomer), 1.45 (s, Boc, minor isomer); ¹H-NMR (DMSO-d₆): 7.97 (d, J = 10.3, 1 H), 7.41-7.30 (m, 7 H), 7.18 (s, 1 H), 7.09 (d, J = 8.2, 1 H), 6.85 (J = 7.6, 1 H), 5.12 (d, J = 12.5, 1 H), 5.05 (d, J = 12.6, 1 H), 4.89 (J = 9.6, 1 H), 4.30-3.55 (m, 6 H), 3.40 (2 H, superimposed by H₂O signal), 3.25-3.00 (m, 2 H), 2.99 (s, 3 H), 2.65 (s, 3 H), 2.22 (m, 1 H), 2.05 (br. q, 1 H), 1.41, (s, 9 H).

According to procedure **F.1.1,** amino acid **116.2** (0.85 g, 1.3 mmol) in dry CH₂Cl₂ (170 mL) was treated with T3P (50% in EtOAc, 1.56 mL, 2.6 mmol) and *i*-Pr₂NEt (0.91 mL, 5.3 mmol) in dry CH₂Cl₂ (1190 mL) to afford after FC (EtOAc/MeOH 95:5 to 80:20) the macrolactam **Ex.198** and its epimer **Ex.231** (ca 1:1 mixture; 0.61 g, 73%).

Data of the mixture **Ex.198/Ex.231:** C₃₂H₄₁N₅O₈ (623.7). LC-MS: (method 2): Rₜ = 1.78 (44), 624 ([M+H]⁺); 1.82 (56), 624 ([M+H]⁺). ¹H-NMR (CDCl₃): complex spectrum, mixture of epimers, 7.41-7.20 (m, 6 H), 7.07-6.92 (m, 3 H) 5.8-4.8 (several m, 5 H), 4.3-3.0 (several m, 10 H), 3.16 (s, NCH₃), 2.81 (s, NCH₃), 2.58-2.45 (m, 1 H), 2.19-2.03 (m, 1 H), 1.51, 1.41 (2 s, 9 H)

### Synthesis of amine Ex.197

According to procedure **J,** carbamate **Ex.198/Ex.231** (ca. 85:15, 749 mg, 1.2 mmol) in dioxane (7.5 mL) was treated with 4M HCl-dioxane (15 mL) to afford. **Ex.197**˙HCl/**Ex.232**˙HCl (607 mg, 90%).
Data of **Ex.197˙**HCl**/Ex.232˙**HCl**:** C₂₇H₃₃N₅O_{6˙}HCl (523.5, free base). LC-MS (method 2) : Rₜ = 1.26 (75), 1.33 (14); 524 ([M+H]⁺).
¹H-NMR (DMSO-d₆), major component **Ex.197˙**HCl**:** spectrum identical with the one described above for compound **Ex.197˙**HCl (cf. Scheme 16).

According to procedure **J,** carbamate **Ex.198/Ex.231** (ca. 1:1, 1.32 g, 2.12 mmol) in dioxane (13 mL) was treated with 4M HCl-dioxane (26 mL) to afford after separation of the isomers by preparative RP-HPLC (method 1) **Ex.197˙**TFA (460 mg, 34%) and **Ex**.**232˙**TFA (470 mg, 35%).
Data of **Ex.197˙**TFA: C₂₇H₃₃N₅O_{6˙}C₂HF₃O₂ (523.5, free base). LC-MS (method 2): Rₜ = 1.25 (99), 524 ([M+H]⁺). LC-MS (method 7): Rₜ = 0.74 (97), 524 ([M+H]⁺) . ¹H-NMR (DMSO-d₆): 8.34 (br. s, NH₃⁺), 8.07 (d, J = 9.9, 1 H), 7.43-7.33 (m, 6 H), 7.20 (s, 1 H), 7.10 (dd, J = 1.5, 8.2, 1 H), 6.87 (d, J = 7.4, 1 H), 5.17 (d, J = 12.5, 1 H), 5.05 (d, J = 12.5, 1 H), 4.87 (br. dd, 1 H), 4.27-4.16 (m, 2 H), 4.06 (t, J = 8.6, 1 H), 4.01-3.91 (m, 2 H), 3.82 (t-like dd, J ca. 8.1, 1 H), 3.70 (br. m, 1 H), 3.35-3.20 (m, 3 H), 2.98 (s, 3 H), 2.70 (s, 3 H), 2.49 (m, 1 H), 2.18 (br. q, J ca. 11.0, 1 H).
Data of **Ex**.**232˙**TFA: See below; Core 14.

### Core 13: Synthesis of Ex.215 and Ex.216 (Scheme 18)

### Synthesis of Mitsunobu product 117

Following procedure **B.1.1,** the reaction of phenol **59** (2.1 g, 5.1 mmol), alcohol **20** (2.1 g, 6.1 mmol), PPh₃ (2.0 g, 7.6 mmol) in dry benzene (50 mL) and DEAD (40% in toluene, 2.8 mL, 6.1 mmol) in dry benzene (14 mL) afforded, after further addition of PPh₃ (0.84 g, 3.2 mmol), alcohol **20** (0.88 g, 2.6 mmol) in benzene (21 mL) and DEAD (40% in toluene, 1.2 mL, 2.6 mmol) in benzene (6 mL) and after FC (hexane/EtOAc 50:50) the protected amino acid **117** (3.8 g, 100%).

### Synthesis of amino acid 118

Following procedure **B.2,** the reaction of **117** (7.63 g, 10.3 mmol), 1,3-dimethylbarbituric acid (4.03 g, 25.8 mmol) and Pd(PPh₃)₄ (1.31 g) in EtOAc/CH₂Cl₂ (1:1, 110 mL) yielded after 1 h and after FC (CH₂Cl₂/MeOH 95:5 to 70:30) amino acid **118** (3.48 g, 60%).

Data of **118:** C₃₀H₄₂N₄O₈Si (614.8). HPLC (10% CH₃CN) : Rₜ = 3.88 (100). LC-MS (method 9a): Rₜ = 1.80, 615 ([M+H]⁺).

### Synthesis of the alloc protected amino acid 119

Following procedure **C.1,** the reaction of amino acid **118** (3.36 g, 5.5 mmol), allyl chloroformate (0.64 mL, 6.0 mmol) and Na₂CO₃ (0.87 g, 8.2 mmol) in dioxane (51 mL) and H₂O (51 mL) gave acid **119** (3.51 g, 92%).

### Synthesis of the protected amino acid 120

Following procedure **C.2,** acid **119** (3.47 g, 5.0 mmol) was reacted with sarcosine allylester p-toluenesufonate (**46˙**p-TsOH, 1.8 g, 6.0 mmol), HOAt (1.0 g, 7.4 mmol), HATU (2.8 g, 7.4 mmol) and *i*-Pr₂NEt (4.2 mL, 25 mmol) in DMF (108 mL) to afford the protected amino acid **120** (3.52 g, 88%).

Data of **120:** C₄₀H₅₅N₅O₁₁Si (809.9). LC-MS: (method 4b): Rₜ = 2.51 (95), 810 ([M+H]⁺)

### Deprotection to amino acid 121

Following procedure **C.3,** the reaction of the protected amino acid **120** (3.49 g, 4.31 mmol), 1,3-dimethylbarbituric acid (1.68 g, 10.8 mmol) and Pd(PPh₃)₄ (0.55 g) in EtOAc/CH₂Cl₂ (1:1; 50 mL) yielded amino acid **121** (2.72 g,92%).

Data of **121:** C₃₃H₄₇N₅O₉Si (685.8). LC-MS: (method 4b): Rₜ = 1.84 (94), 686 ([M+H]⁺)

### Synthesis of the protected macrolactam Ex.215

According to procedure **F.1.2,** amino acid **121** (1.33 g, 1.94 mmol) in dry DMF (27 mL) was treated with FDPP (1.49 g, 3.88 mmol) in dry DMF (164 mL) to yield after FC (EtOAc/MeOH 95:5) macrolactam **Ex.215** (0.89 g, 68%).

Data of **Ex.215:** C₃₃H₄₅N₅O₈Si (667.8). LC-MS: (method 1b): Rₜ = 2.60 (99), 668 ([M+H]⁺). LC-MS: (method 9c): Rₜ = 2.14, 668 ([M+H]⁺). ¹H-NMR (DMSO-d₆): 7.94 (d, J = 9.8, 1 H), 7.39-7.27 (m, 7 H), 7.11 (s, 1 H), 6.97 (dd, J = 1.5, 8.2, 1 H), 6.82 (d, J = 7.5, 1 H), 5.05 (s, 2 H), 4.83 (br. d, 1 H), 4.25 (br. m, 1 H), 4.17-3.96 (m, 5 H), 3.73 (br. q, J ca. 16.8, 2 H), 3.47 (m, 1 H), 3.33 (m, 1 H), 3.19 (m, 2 H), 2.96 (s, 3 H), 2.67 (s, 3 H), 2.20 (m, 1 H), 2.00 (m, 1 H), 0.91 (t, J = 8.4, 2 H), 0.00 (s, 9 H).

### Synthesis of amine Ex.216

According to procedure **I.1,** carbamate **Ex.215** (881 mg, 1.3 mmol) in dioxane (16 mL) was treated with 4M HCl-dioxane (16 mL) to afford **Ex.216·**HCl (666 mg, 90%).

Data of **Ex.216·**HCl: C₂₇H₃₃N₅O₆**·**HCl (523.5, free base). HPLC (5% CH₃CN): Rₜ = 3.11 (91). LC-MS (method 9c): Rₜ = 1.19, 524 ([M+H]⁺).

### Core 14: Synthesis of Ex.231 and Ex.232 (Scheme 19)

### Synthesis of Mitsunobu product 122

A mixture of phenol **61** (4.6 g, 11.2 mmol) and PPh₃ (5.27 g, 20.1 mmol) was dissolved in benzene. The soln was concentrated and the residue was dried i.v. for 20 min. A soln of the alcohol **81,** (7.46 g, 20.1 mmol) in dry, degassed benzene (120 mL) was added. The resulting mixture was cooled to 0°C. DEAD (40% in toluene, 11.5 mL, 25.1 mmol)
in benzene (10 mL) was slowly added. The soln was stirred at rt for 16 h. More PPh₃ (1.46 g, 5.6 mmol), alcohol **81** (1.04 g, 2.8 mmol) and at 0°C, a soln of DEAD (40% in toluene, 2.6 mL, 5.7 mmol) in benzene (2 mL) were added and stirring at rt was continued for 7 h. More PPh₃ (1.46 g, 5.6 mmol), alcohol **81** (1.04 g, 2.8 mmol), and at 0°C, a soln of DEAD (40% in toluene, 2.6 mL, 5.7 mmol) in benzene (2 mL) were added. Stirring at rt was continued for 16 h. The mixture was concentrated. FC (hexane/EtOAc 30:70 to 0:100) afforded **122** (12.8 g, contaminated with ca. 40% triphenylphosphine oxide, yield ca. 90%), which was used in the next step without further purification.

### Synthesis of amino acid 123

Following procedure **E.2,** the reaction of the protected amino acid **122** (contaminated with ca. 40% of triphenylphosphine oxide, 12.8 g, ca. 10 mmol), 1,3-dimethylbarbituric acid (3.91 g, 25.1 mmol) and Pd(PPh₃)₄ (1.27 g) in EtOAc/CH₂Cl₂ (1:1, 120 mL) yielded after 1 h and after FC (CH₂Cl₂/MeOH 100:0 to 70:30 then CHCl₃/MeOH 70:30) the amino acid **123** (2.80 g, 44%).

Data of **123:** C₃₂H₄₃N₅O₉ (641.7). LC-MS: (method 2) : Rₜ = 1.56 (94), 642 ([M+H]⁺).

### Synthesis of the protected macrolactam Ex.231

According to procedure **F.1.2,** amino acid **123** (3.29 g, 5.13 mmol) in dry DMF (150 mL) was added within 4 h at 60°C to FDPP (3.94 g, 10.3 mmol) in dry DMF (4980 mL) to afford after 16 h at 60°C and after FC (EtOAc/MeOH 100:0 to 95:5) macrolactam **Ex.231** (containing ca. 15% of its epimer **Ex.198;** 2.5 g, 78%). Data of **Ex.231:** C₃₂H₄₁N₅O₈ (623.7). LC-MS: (method 2): Rₜ = 1.78 (12), 1.82 (83), 624 ([M+H]⁺). LC-MS: (method 7): Rₜ = 1.16 (18), 624 ([M+H]⁺); 1.18 (80), 624 ([M+H]⁺). ¹H-NMR (CDCl₃): complex spectrum, two epimers; 7.38-7.22 (m, 6 H), 7.06-6.90 (m, 3 H), 5.80-4.80 (several m, 4 H), 5.08, 5.12 (2 s, 2 H), 4.43-2.80 (several br. m, 15 H), 2.51 (m, 1 H), 2.19-2.03 (m, 1 H), 1.50, 1.42 (2 s, 9 H).

### Synthesis of amine Ex.232

According to procedure **J,** carbamate **Ex.231** (containing 15% of the epimer **Ex.198;** 1.42 g, 2.3 mmol) in dioxane (30 mL) was treated with 4M HCl-dioxane (45 mL) to afford after preparative RP-HPLC (method 1) **Ex.232·**TFA (1.10 g, 71%) and **Ex.197·**TFA (0.27 g, 17%).
Data of **Ex.232·**TFA**:** C₂₇H₃₃N₅O₆**·**C₂HF₃O₂ (523.5, free base). LC-MS (method 2): Rₜ = 1.32 (99), 524 ([M+H]⁺). ¹H-NMR (DMSO-d₆): complex spectrum, mixture of isomers; 8.40 (br. s), 8.20 (br. s), 7.84 (d, J = 7.1), 7.50-6.80 (several m), 5.25-3.40 (several m, partially superimposed by the H₂O signal), 3.30-2.80 (m), 3.04 (s, NCH₃), 2.98 (s, NCH₃), 2.67 (s, NCH₃), 2.64 (s, NCH₃), 2.6-1.9 (several m).
Data of **Ex.197·**TFA: See above; Core 12.

### Core 15 and Core 16:

### Synthesis of Ex.238 and Ex.239 (Scheme 20)

### Synthesis of Mitsunobu product 124

Following procedure **E.1.1,** phenol **77** (1.63 g, 8.5 mmol), alcohol **85** (5.72 g, 12.8 mmol) and PPh₃ (4.02 g, 15.3 mmol) in dry benzene (80 mL) were treated with DEAD (40% in toluene, 8.79 mL, 19.2 mmol) for 20 h. Purification by FC(hexane/EtOAc 20:80 to 100:0) then (hexane/EtOAc 50:50 to 20:80) afforded the protected amino acid **124** (1.96 g, 37%).

### Synthesis of the macrocycle Ex.238

### Dichloro-[1,3-bis(mesityl)-2-imidazoldinylidene]-(3-phenyl-1H-inden-1-ylidene)(tricyclohexylphosphine)ruthenium (II)

(Umicore M2 catalyst; 88 mg) was added to a soln of **124** (1160 mg, 1.29 mmol) in dry, degassed CH₂Cl₂ (170 mL). The soln was stirred in a sealed tube at 40°C for 68 h, followed by 45 h at rt. During this period further equal portions of catalyst (in total 350 mg) were added after 20 h, 28 h, 44 h, and 52 h. The soln was concentrated. FC (hexane/EtOAc 70:30 to 0:100) gave **Ex.238** (350 mg, 46%, mixture of two isomers, ratio > 9:1, acceptable for the use in the next step). An analytical sample (69 mg) was further purified by preparative RP-HPLC (method 2) to afford pure **Ex.238** (major isomer; 45 mg).

Data of **Ex.238** (major isomer): C₃₂H₄₀N₄O₇ (592.6). LC-MS: (method 4a): Rₜ = 2.23 (92), 593 ([M+H]⁺). ¹H-NMR (CDCl₃): 7.62-7.31 (m, 6 H), 7.07 (d, J = 7.6, 1 H), 6.99 (dd, J = 2.0, 7.9, 1 H), 6.85 (s, 1 H), 5.69-5.61 (m, 2 H), 5.48 (d, J = 8.2, 1 H), 5.21 (m, 1 H), 5.10 (s, 2 H), 4.76 (d, J = 10.1, 1 H), 4.54 (dt, J = 3.5, 7.9, 1 H), 4.41-4.25 (m, 2 H), 4.13 (d, J = 10.7, 1 H), 3.97 (m, 1 H), 3.62 (m, 2 H), 3.48 (m, 1 H), 3.10 (s, 3 H), 2.73 (m, 1 H), 2.60-2.45 (m, 2 H), 2.02 (m, 1 H), 1.46 (s, 9 H).

### Synthesis of amine Ex.239

A soln of **Ex.238** (430 mg, 0.73 mmol) in MeOH/THF 1:3, 36 mL) was hydrogenated for 3.5 h at rt under normal pressure in the presence of palladium hydroxide on activated charcoal (moistened with 50% H₂O; 215 mg). The mixture was filtered through a pad of celite. The filtrate was concentrated to give **Ex.239** (355 mg, quant.; used in the next step without further purification).

An analytical sample (68 mg) was purified by preparative RP-HPLC (method 2) to afford pure **Ex.239** (37 mg).

Data of **Ex.239:** C₂₄H₃₆N₄O₅ (460.6): LC-MS (method 7): Rₜ = 0.88 (97), 461 ([M+H]⁺). ¹H-NMR (DMSO-d₆): 7.36 (t, J = 7.8, 1 H), 7.25 (d, J = 6.1, 1 H), 7.03 (dd, J = 1.6, 8.2, 1 H), 6.88-6.65 (m, 2 H), 4.51 (d, J = 8.3, 1 H), 4.18 (t, J = 10.3, 2 H), 4.09 (br. s, 1 H), 3.96 (br. m, 2 H), 3.19-2.72 (m, 3 H), 2.92 (s, 3 H), 2.34 (m, 2 H), 2.05 (br. q, 1 H), 1.82 (m, 1 H), 1.60-0.85 (m, 5 H), 1.40 (s, 9 H), 0.82 (m, 1 H).

### Core 17: Synthesis of Ex.248 and Ex.249 (Scheme 21)

### Synthesis of Mitsunobu product 125

Following procedure **E.1.1,** phenol **68** (6.0 g, 14.6 mmol), alcohol **82** (9.75 g, 26.2 mmol), and PPh₃ (6.88 g, 26.2 mmol) were treated in dry benzene (160 mL) with DEAD (40% in toluene, 15 mL, 32.8 mmol) for 40 h. After 18 h and after 25 h, more PPh₃ (1.27 g, 4.8 mmol) and DEAD (40% in toluene, 2.23 mL, 4.9 mmol) in benzene (2 mL) were added. FC (hexane/EtOAc 30:70 to 20:80) afforded the protected amino acid **125** (16.85 g, contaminated with ca. 40% triphenylphosphine oxide, yield ca. 85%), which was used in the next step without further purification.

### Synthesis of amino acid 126

Following procedure **E.2,** the reaction of **125** (16.8 g, contaminated with ca. 40% of triphenylphosphine oxide, ca. 12 mmol), 1,3-dimethylbarbituric acid (4.80 g, 30.8 mmol) and Pd(PPh₃)₄ (1.56 g) in EtOAc/CH₂Cl₂ (1:1, 170 mL) yielded after 1 h and after FC (CH₂Cl₂/MeOH 0:100 to 70:30, then CHCl₃/MeOH 70:30) amino acid **126** (4.15 g, ca. 52%).

Data of **126:** C₃₃H₄₄N₄O₉ (640.7). HPLC (10% CH₃CN) : Rₜ = 3.67 (69). LC-MS (method 9c): Rₜ = 1.75, 641 ([M+H]⁺).

### Synthesis of the protected macrolactam Ex.248

According to procedure **F.1.1,** amino acid **126** (4.55 g, 7.1 mmol) in dry CH₂Cl₂ (120 mL) was added within 3 h to T3P (50% in EtOAc, 8.37 ml, 14.2 mmol) and *i*-Pr₂NEt (4.83 ml, 28.4 mmol) in dry CH₂Cl₂ (6660 mL). Prior to aqueous workup, CH₂Cl₂ was replaced with EtOAc. FC (CH₂Cl₂/MeOH 100:0 to 95:5) yielded macrolactam **Ex.248** (2.38 g, 54%).

Data of **Ex.248**: C₃₃H₄₂N₄O₈ (622.7). LC-MS: (method 2) : Rₜ = 1.83 (100), 623 ([M+H]⁺). LC-MS: (method 9c): Rₜ = 1.97, 623 ([M+H]⁺). ¹H-NMR (DMSO-d₆): 7.45-7.34 (m, 5 H), 7.15-6.78 (m, 5 H), 5.25 (s, 2 H), 5.08 (d, J = 12.8, 1 H), 4.62 (d, J = 13.5, 2 H), 4.29 (m, 1 H), 4.09 (d, J = 7.3, 1 H), 3.89 (d, J = 12.4, 1 H), 3.54 (br. t, 1 H), 3.27 (m, 1 H), 3.07 (s, 3 H), 2.80 (m, 1 H), 2.71 (s, 3 H), 2.28-2.06 (m, 4 H), 1.94 (m, 1 H), 1.71 (m, 1 H), 1.39 (s, 9 H).

### Synthesis of acid Ex.249

According to procedure H, the ester **Ex.248** (2.16 g, 3.5 mmol) was hydrogenolyzed in MeOH (130 mL)/THF (40 mL) in the presence of the catalyst (1.09 g) for 2.5 h to afford the acid **Ex.249** (1.83 g, 99%).

Data of **Ex.249:** C₂₆H₃₆N₄O₈ (532.6). LC-MS: (method 2): Rₜ = 1.42 (95), 533 ([M+H]⁺).

### Core 18: Synthesis of Ex.272, Ex.273, and Ex.274 (Scheme 22)

### Synthesis of Mitsunobu product 127

Following procedure **E.1.1,** the reaction of phenol **71** (6.47 g, 15,7 mmol), alcohol **81** (10.5 g, 28.2 mmol), DEAD (40% in toluene, 26 mL, 56.3 mmol), and PPh₃ (14.8 g, 56.3 mmol) in dry benzene (380 mL) afforded after 2 h at rt and after aqueous workup (EtOAc, sat. aq. Na₂CO₃ soln, sat. aq. NaCl soln), drying (Na₂SO₄), concentration of the organic layer and FC (hexane/EtOAc 30:70, 0:100, then CH₂Cl₂/MeOH 90:10) the protected amino acid **127** (12.0 g, 99%).

### Synthesis of amino acid 128

Following procedure **E.2,** the reaction of **127** (12.0 g, 16 mmol), 1,3-dimethylbarbituric acid (5.9 g, 38.0 mmol) and Pd(PPh₃)₄ (0.9 g) in EtOAc/CH₂Cl₂ (55:45, 275 mL) yielded after 2 h and after FC (EtOAc, then CH₂Cl₂/MeOH 90:10 to 60:40) amino acid **128** (9.05 g, 90%).

Data of **128:** C₃₁H₄₂N₆O₉ (642.7). LC-MS: (method 7): Rₜ = 0.90 (94), 643 ([M+H]⁺).

### Synthesis of the protected macrolactam Ex.272

According to procedure **F.1.1,** amino acid **128** (5.04 g, 7.8 mmol) in dry CH₂Cl₂ (100 mL) was treated with T3P (50% in EtOAc, 9.2 mL, 16 mmol) and *i*-Pr₂NEt (5.4 mL, 31 mmol) in dry CH₂Cl₂ (700 mL) to afford after FC (CH₂Cl₂/MeOH 39:1 to 19:1) the epimeric macrolactams **Ex.272** (1.90 g, 38%).

Data of **Ex.272**: C₃₁H₄₀N₆O₈ (624.7). LC-MS: (method 2): Rₜ = 1.61 (99), 625 ([M+H]⁺). LC-MS: (method 7): Rₜ = 1.01 (99), 625 ([M+H]⁺). ¹H-NMR (DMSO-d₆): 8.47 (d, J = 2.6, 1 H), 8.12 (s, 1 H), 7.95 (d, J = 9.6, 1 H), 7.61 (s, 1 H), 7.40-7.29 (m, 6 H), 5.10 (d, J = 12.6, 1 H), 5.04 (d, J = 12.6, 1 H), 4.98 (br. d, J = 10.7, 1 H), 4.16 (br. d, J = 11.8, 1 H), 4.10-3.90 (m, 4 H), 3.71 (br. t, J ca. 8.4, 1 H), 3.65-3.40 (m, 2 H), 3.23 (br. dd, J = 11.1, 15.2, 1 H), 3.04 (s, 3 H), 2.92 (t, J = 9.6, 1 H), 2.66 (s, 3 H), 2.12 (m, 1 H), 2.09 (br. q, 1 H), 1.42 (s, 9 H) .

### Synthesis of amine Ex.273

According to procedure **J,** carbamate **Ex.272** (3.12 g, 5 mmol) in dioxane (31 mL) was treated with 4M HCl-dioxane (62 mL) to afford **Ex.273·**2HCl (2.9 g, 97%)

Data of **Ex.273·**2HCl: C₂₆H₃₂N₆O₆**·**2HCl (524.5, free base). LC-MS (method 2): Rₜ = 1.31 (92), 525 ([M+H]⁺).

### Synthesis of amine Ex.274

According to procedure **K,** carbamate **Ex.272** (200 mg, 0.32 mmol) was hydrogenolyzed in MeOH (20 mL) in the presence of the catalyst (100 mg) to afford **Ex.274** (154 mg, 97%).

Data of **Ex.274:** C₂₃H₃₄N₆O₆. (490.5). LC-MS (method 2): Rₜ = 1.26 (98), (491 ([M+H]⁺).

### Core 19: Synthesis of Ex.297 and Ex.298 (Scheme 23)

### Synthesis of Mitsunobu product 129

Following procedure **E.1.2,** the reaction of phenol **75** (4.58 g, 9.9 mmol), alcohol **81** (5.5 g, 15 mmol), and CMBP (4.8 g, 20 mmol) in dry toluene (24 mL) afforded after FC (hexane/EtOAc 1:3) the protected amino acid **129** (5.54 g, 68%).

### Synthesis of amino acid 130

Following procedure **E.2,** the reaction of **129** (5.53 g, 6.8 mmol), 1,3-dimethylbarbituric acid (2.5 g, 16 mmol) and Pd(PPh₃)₄ (0.39 g) in EtOAc/CH₂Cl₂ 55:45 (118 mL) yielded after 2 h and after FC (CH₂Cl₂/MeOH 95:5 to 70:30) amino acid **130** (1.45 g, 85%).

Data of **130:** C₃₆H₄₅N₅O₉ (691.7). LC-MS (method 7): Rₜ = 1.09 (96), 692 ([M+H]⁺).

### Synthesis of the protected macrolactam Ex.297

According to procedure **F.1.1,** amino acid **130** (2.57 g, 3.7 mmol) in dry CH₂Cl₂ (40 mL) was treated with T3P (50% in EtOAc, 4.4 mL, 7.4 mmol) and *i*-Pr₂NEt (2.5 mL, 14.9 mmol) in dry CH₂Cl₂ (330 mL) to give after FC (CH₂Cl₂/MeOH 99:1 to 90:10) macrolactam **Ex.297** (2.5 g, contaminated with ca. 20% *i*-Pr₂NEt; yield 80%).

Data of **Ex.297:** C₃₆H₄₃N₅O₈ (673.7). LC-MS: (method 7): Rₜ = 1.18 (93), 674 ([M+H]⁺).

Aqueous workup (EtOAc, 1 M aq. NaH₂PO₄ soln) of an analytical sample (100 mg) afforded pure **Ex.297** (81 mg).

LC-MS: (method 2): Rₜ = 2.20 (93), 674 ([M+H]⁺). ¹H-NMR (DMSO-d₆): complex spectrum, several isomers, 8.51 (d, J = 8.5, 0.2 H), 8.47 (d, J = 8.7, 0.1 H), 8.40 (d, J = 8.5, 0.55 H), 8.32 (d, J = 8.5, 0.15 H), 7.68-7.10 (several m, 10 H), 5.96 (br. s, 0.3 H), 5.90 (br. s, 0.3 H), 5.4-5.0 (m, 2.4 H), 4.8-3.8 (several m, 8 H), 3.3-2.5 (several m and s, 8 H), 2.5-1.6 (several m, 4 H), 1.42, 1.41, 1.36, 1.26 (4 s, Boc).

### Synthesis of acid Ex.298

According to procedure **H,** the ester **Ex.297** (2.0 g, contaminated with ca. 20% *i*-Pr₂NEt 2.4 mmol) was hydrogenolyzed in MeOH (200 mL) in the presence of catalyst (1 g) for 3 h.

The crude product was suspended in Et₂O (20 mL) stirred for 20 min, filtered, washed (Et₂O) and dried to afford **Ex.298** (1.63 g, contaminated with 15% *i*-Pr₂NEt, quant. yield).

Aqueous workup (CH₂Cl₂, 1 M aq. NaH₂PO₄ soln) of an analytical sample (200 mg) afforded pure **Ex.298** (135 mg).

Data of **Ex.298:** C₂₉H₃₇N₅O₈ (583.6). LC-MS: (method 4a): Rₜ = 1.78 (86), 584 ([M+H]⁺).

### Core 20: Synthesis of Ex.311 (Scheme 24)

### Synthesis of Mitsunobu product 131

A soln of phenol **72** (200 mg, 0.34 mmol), alcohol **16** (178 mg, 0.52 mmol) and PPh₃ (180 mg, 0.69 mmol) in benzene (5 mL) was degassed. At 0°C, DEAD (40% in toluene, 0.32 mL, 0.69 mmol) was added. The mixture was stirred at rt for 15 h. Additional alcohol **16** (178 mg, 0.52 mmol) and PPh₃ (180 mg, 0.69 mmol) were added. DEAD (40% in toluene, 0.32 mL, 0.69 mmol) was added at 0°C. The mixture was stirred for 20 h and concentrated. FC (CH₂Cl₂/EtOAc 100:0 to 80:20) afforded **131** (containing ca. 20% of diethyl hydrazine-1,2-dicarboxylate; used without further purification).

### Synthesis of amino acid 132

Following procedure **B.2,** the reaction of **131** (250 mg, ca. 80%, 0.22 mmol), 1.3-dimethylbarbituric acid (107 mg, 0.69 mmol) and Pd(PPh₃)₄ (16 mg) in EtOAc/CH₂Cl₂ (55:45, 4.8 mL) yielded after 3 h and after FC (EtOAc/MeOH 100:0 to 90:10, then CH₂Cl₂/MeOH 90:10 to 80:20) **132** (177 mg, yield over the two steps: 73%). Data of **132:** C₃₉H₅₇N₅O₁₀Si (784.0): LC-MS: (method 7): Rₜ = 1.31, 784.2 ([M+H]⁺).

### Synthesis of the alloc protected amino acid 133

Following procedure **C.1,** the reaction of **132** (150 mg, 0.19 mmol), allyl chloroformate (23 µL, 0.21 mmol) and Na₂CO₃ (61 mg, 0.57 mmol) in dioxane (1.5 mL) and H₂O (1.5 mL) gave, after 2 h at 0°C, acid **133** (154 mg, 92%).

### Synthesis of the protected amino acid 134

Following procedure **C.2,** acid **133** (140 mg, 0.16 mmol) was reacted with sarcosine allylester p-toluenesulfonate (**46·**pTsOH, 58 mg, 0.194 mmol), HOAt (33 mg, 0.24 mmol), HATU (92 mg, 0.24 mmol) and *i*-Pr₂NEt (0.138 mL, 0.81 mmol) in DMF (2.4 mL) to afford the protected amino acid **134** (106 mg, 67%).

Data of **134:** C₄₉H₇₀N₆O₁₃Si (979.2). LC-MS: (method 7): Rₜ = 1.68, 979.3 ([M+H]⁺).

### Synthesis of amino acid 135

Following procedure **C.3,** the reaction of the protected amino acid **134** (100 mg, 0.10 mmol), 1.3-dimethylbarbituric acid (38 mg, 0.25 mmol) and Pd(PPh₃)₄ (6 mg) in EtOAc/CH₂Cl₂ (45:55, 1.9 mL) yielded after 16 h and after FC (EtOAc, then CH₂Cl₂/MeOH 90:10) **135** (70 mg, 80%).

Data of **135:** C₄₂H₆₂N₆O₁₁Si (855.1). LC-MS: (method 7): Rₜ = 1.30, 855.5 ([M+H]⁺).

### Synthesis of the protected macrolactam Ex.311

According to procedure **F.1.1,** a soln of the amino acid **135** (60 mg, 0.07 mmol) in dry CH₂Cl₂ (2 mL), was added within 2 h to T3P (50% in EtOAc; 84 µL, 0.14 mmol) and *i*-Pr₂NEt (48 µL, 0.28 mmol) in CH₂Cl₂ (5 mL). Then sat. aq. NaHCO₃ soln was added and the mixture was extracted with CH₂Cl₂. The organic phase was dried (Na₂SO₄), filtered and concentrated. FC (EtOAc) afforded **Ex.311** (26 mg, 44%).

Data of **Ex.311:** (C₄₂H₆₀N₆O₁₀Si (837.0). LC-MS: (method 7): Rₜ = 1.51 (90), 837.4 ([M+H]⁺). ¹H-NMR (CDCl₃): 7.26 (s, 5 H), 7.09 (t, J = 8.4, 1 H), 6.78 (d-like m, 1 H), 6.61 (d, J = 7.4, 1 H), 5.50-4.90 (several br. m, 5 H), 4.90-3.80 (several br. m, 8 H), 3.69 (br. t, J ca. 8.5, 1 H), 3.6-2.3 (several br. m, 14 H), 2.12 (m, 1 H), 1.61 (m, 1 H), 1.38 (s, 9 H), 1.24 (s, 2 H), 0.93 (br. t, J ca. 8.0, 2 H), 0.00, -0.03 (2 s, 9 H).

### Core 21: Synthesis of Ex.312 - Ex.315 (Scheme 25)

### Synthesis of Mitsunobu product 136

Alcohol **82** (217 mg, 0.58 mmol) and CMBP (212 mg, 0.88 mmol) were dissolved in dry degassed toluene (7 mL) and heated at 100°C for 30 min. A soln of **80** (250 mg, 0.58 mmol) in toluene (2 mL) was added dropwise. Stirring at 100°C was continued for 1 h. The volatiles were evaporated. FC (hexane/EtOAc 2:1 to 1:1) yielded **136** (290 mg, 63%).

### Synthesis of amino acid 137

Following procedure **E.2** the reaction of **136** (250 mg, 0.32 mmol), 1,3-dimethylbarbituric acid (120 mg, 0.77 mmol) and Pd(PPh₃)₄ (18 mg) in EtOAc/CH₂Cl₂ (45:55, 5.5 mL) yielded after 0.5 h and after FC (CH₂Cl₂/MeOH 95:5 to 70:30) amino acid **137** (164 mg, 78%).

Data of **137:** C₃₃H₄₄N₄O₈S (656.8). LC-MS (method 7): Rₜ = 1.15 (95), 657 ([M+H]⁺).

### Synthesis of the protected macrolactam Ex.312

According to procedure **F.1.1,** a soln of the amino acid **137** (100 mg, 0.15 mmol) in dry CH₂Cl₂ (2 mL) was added over 2 h to T3P (50% in EtOAc, 0.18 mL, 0.31 mmol) and *i*-Pr₂NEt (0.1 mL, 0.61 mmol) in dry CH₂Cl₂ (13 mL). Stirring at rt was continued for 1 h, followed by aqueous workup (EtOAc, sat. aq. NaHCO₃ soln, Na₂SO₄) and FC (EtOAc) to afford **Ex.312** (56 mg, 57%).

Data of **Ex.312:** C₃₃H₄₂N₄O₇S (638.7). LC-MS (method 7): Rₜ = 1.33 (95), 639 ([M+H]⁺). ¹H-NMR (CDCl₃): 7.37-7.23 (m, 8 H), 6.92 (br. s, 1 H), 5.25 (m, 2 H), 5.17 (s, 1 H), 4.88 (d, J = 16.2, 1 H), 4.62 (br. m, 1 H), 4.46 (br. t-like m, 1 H), 4.31 (br. m, 1 H), 4.17 (dd, J = 4.1, 14.2, 1 H), 3.72 (dd, J = 4.8, 10.7, 1 H), 3.50 (m, 1 H), 3.30-2.80 (several m, 2 H), 3.14 (s, 3 H), 3.01 (s, 3 H), 2.60-1.90 (several m, 6 H), 1.46 (s, 9 H).

### Synthesis of sulfone Ex.313

m-CPBA (70% w/w; 10 mg, 41 µmol) was added at 0°C to a soln of **Ex.312** (20 mg, 31 µmol) in CH₂Cl₂ (0.5 mL). The mixture was stirred for 15 min followed by the addition of m-CPBA (9 mg, 37 µmol). The mixture was allowed to warm to rt over 1 h, diluted with CH₂Cl₂ and washed with aq. Na₂S₂O₃ soln and with aq. NaHCO₃ soln. The organic phase was dried (Na₂SO₄), filtered and concentrated. FC (EtOAc/MeOH 100:0 to 90:10) afforded **Ex.313** (8 mg, 38%).

Data of **Ex.313:** C₃₃H₄₂N₄O₉S (670.7). LC-MS (method 6): Rₜ = 1.24 (95), 671 ([M+H]⁺). ¹H-NMR (CDCl₃): 7.89 (td, J = 1.7, 7.3, 1 H), 7.71 (s, 1 H), 7.43-7.28 (m, 7 H), 5.17 (d, J = 12.0, 1 H), 5.10 (d, J = 12.0, 1 H), 5.01 (dd, J = 5.9, 9.1, 1 H), 4.96-4.85 (m, 2 H), 4.71 (d, J = 15.4, 1 H), 4.57 (br. m, 1 H), 4.33 (br. m, 2 H), 3.85 (dd, J = 7.8, 12.3, 1 H), 3.25 (s, 3 H), 3.20 (m, 1 H), 3.10 (m, 1 H), 2,97 (s, 3 H), 2.73-2.54 (m, 2 H), 2.45-2.23 (m, 2 H), 2.17 (m, 1 H), 1.99 (m, 1 H), 1.46 (s, 9 H).

### Synthesis of acid Ex.314

A soln of the benzyl ester **Ex.312** (1.69 g, 2.6 mmol) in THF/MeOH (3:1; 36 mL) was treated with H₂O (9 mL) and at 0°C LiOH**·**H₂O (0.22 g, 5.2 mmol) was added. The mixture was stirred for 1 h at 0°C, acidified to pH 5 with 1 M aq. NaH₂PO₄ soln and extracted with CHCl₃. The organic layer was dried (Na₂SO₄), filtered and concentrated. The resulting solid was suspended in Et₂O and filtered to give **Ex.314** (1.4 g, 96%).

Data of **Ex.314:** C₂₆H₃₆N₄O₇S (548.6) . LC-MS (method 7) : Rₜ = 1.29 (97), 549 ([M+H]⁺).

### Synthesis of acid Ex.315

According to procedure **H,** a soln of the benzyl ester **Ex.313** (1.69 g, 2.5 mmol) was hydrogenolyzed in MeOH (65 mL) in the presence the catalyst (1.69 g) for 4 h, filtered through a pad of celite and concentrated to afford **Ex.315** (1.35 g, 92%).

Data of **Ex.315:** C₂₆H₃₆N₄O₉S (580.6). LC-MS (method 4b): Rₜ = 1.62 (99), 581 ([M+H]⁺).

### Core 22: Synthesis of Ex.342 and Ex.343 (Scheme 26)

### Synthesis of Mitsunobu product 220

At 0°C DEAD (40% in toluene, 33 mL, 71.6 mmol) was slowly added to a soln of phenol **199** (15.42 g, 35.8 mmol), alcohol **14** (12.9 g, 43 mmol) and PPh₃ (18.8 g, 71.6 mmol) in dry benzene (306 mL). The mixture was stirred for 3.5 h at 0°C to rt. H₂O (0.64 mL, 35.8 mmol) was added and stirring continued for 5 min. The mixture was concentrated. The residue was suspended in Et₂O (150 mL) and filtered. The filtrate was concentrated. This washing procedure was repeated twice to afford after concentration of the filtrate and FC (hexane/EtOAc) the protected amino acid **220** (20.3 g, 80%; contaminated with diethyl hydrazine-1,2-dicarboxylate, used without further purification).

### Synthesis of amino acid 221

Following procedure **B.2,** the reaction of **220** (19.86 g, 27.8 mmol), 1,3-dimethylbarbituric acid (10.87 g, 69.6 mmol) and Pd(PPh₃)₄ (0.32 g) in EtOAc/CH₂Cl₂ (55:45, 300 mL) yielded after 2 h and evaporation of the volatiles a solid which was filtered and washed with EtOAc to give **221** (13.95 g, 85%).

Data of **221:** C₂₉H₃₇FN₄O₈ (588.6). LC-MS (method 10a): Rₜ = 1.58 (95), 589 ([M+H]⁺).

### Synthesis of the protected macrolactam Ex.342

Amino acid **221** (12.58 g, 21.4 mmol) was added in portions to a soln of T3P (50% in EtOAc, 37.8 mL, 64.1 mmol) and *i*-Pr₂NEt (18.3 mL, 106.9 mmol) in dry CH₂Cl₂ (4280 mL). Stirring at rt was continued for 2 h, followed by evaporation of the volatiles. FC (hexane/EtOAc/MeOH gradient) afforded the macrolactam **Ex.342** (9.4 g, contaminated with 10% of the cyclic dimer). Further purification of this material (1.0 g) by FC (hexane/EtOAc/MeOH gradient) afforded pure **Ex.342** (0.81 g). Data of **Ex.342:** C₂₉H₃₅FN₄O₇ (570.6). LC-MS (method 10b): Rₜ = 2.05 (97), 571 ([M+H]⁺), 471. ¹H-NMR (DMSO-d₆) : 8.21 (br. t, 1 H), 7.57 (d, J = 6.7, 1 H), 7.50 (d, J = 8.8, 1 H), 7.40-7.31 (m, 7 H), 7.22 (d, J = 6.6, 1 H), 5.06 (d, J = 12.4, 1 H), 4.99 (d, J = 12.6, 1 H), 4.45-4.40 (m, 2 H), 4.35-4.25 (m, 2 H), 4.13 (m, 1 H), 4.03 (m, 1 H), 3.64 (m, 1 H), 3.30 (m, 2 H, superimposed by H₂O signal), 2.27 (m, 1 H), 1.99-1.86 (m, 3 H), 1.39 (s, 9 H).

### Synthesis of amine Ex.343

According to procedure **K,** benzyl carbamate **Ex.342** (5.0 g, 8.7 mmol) was hydrogenolyzed in MeOH (100 mL) in the presence of catalyst (656 mg) for 2 h to afford after filtration, evaporation of the volatiles and FC (CH₂Cl₂/MeOH 95:5 to 80:20) amine **Ex.343** (3.2 g, 84%).

Data of **Ex.343:** C₂₁H₂₉FN₄O₅ (436.5). LC-MS (method 10b): Rₜ = 1.37 (100), 437 ([M+H]⁺), 381, 337.

### Core 23: Synthesis of Ex.363 and Ex.364 (Scheme 27)

### Synthesis of the amide 222

T3P (50% in EtOAc, 6.8 mL, 11.5 mmol) was added to a soln of acid **192** (2.4 g, 5.8 mmol) and amine hydrochloride **178·**HCl (1.8 g, 5.8 mmol) in dry CH₂Cl₂ (65 mL). *i*-Pr₂NEt (4.1 mL, 23.9 mmol) was slowly added; and the mixture was stirred for 5 h at rt. The mixture was distributed between 1 M aq. NaH₂PO₄ soln and CH₂Cl₂. The organic phase was dried (Na₂SO₄), filtered and concentrated. FC (hexane/EtOAc gradient) afforded amide **222** (3.49 g, 93%).

Data of **222:** C₃₇H₄₇N₃O₁₁ (709.8). LC-MS (method 10a): Rₜ = 2.50 (96), 710 ([M+H]⁺).

### Synthesis of amino acid 223

Following procedure **B.2,** the reaction of **222** (3.45 g, 4.86 mmol), 1,3-dimethylbarbituric acid (1.9 g, 12.1 mmol) and Pd(PPh₃)₄ (28 mg) in EtOAc/CH₂Cl₂ (1:1, 60 mL) yielded after 4 h and after FC (CH₂Cl_{2/}MeOH 90:10 to 70:30) amino acid **223** (2.32 g, 81%).

Data of **223:** C₃₀H₃₉N₃O₉ (585.6). LC-MS (method 10a): Rₜ = 1.62 (97), 586 ([M+H]⁺).

### Synthesis of the protected macrolactam Ex.363

According to procedure **F.1.2,** the amino acid **223** (2.3 g, 3.9 mmol) in dry DMF (50 mL) was treated with FDPP (3.15 g, 8.2 mmol) in DMF (700 mL). After completion of the addition, the mixture was concentrated. FC (hexane/EtOAc/MeOH gradient) afforded macrolactam **Ex.363** (1.78 g, 80%).

Data of **Ex.363:** C₃₀H₃₇N₃O₈ (567.6). LC-MS (method 10b) : Rₜ = 2.00 (98), 568 ([M+H]⁺). ¹H-NMR (DMSO-d₆): 7.43-7.34 (m, 5 H), 7.27 (t, J = 7.8, 1 H), 7.20 (d, J = 7.2, 1 H), 7.01 (m, 1 H), 6.85 (br. s, 1 H), 6.79 (d, J = 7.4, 1 H), 5.22 (s, 2 H), 5.05 (d, J = 12.5, 1 H), 4.76 (dd, J = 3.7, 9.8, 1 H), 4.08 (t, J = 8.7, 1 H), 3.94-3.80 (m, 4 H), 3.59 (dd, J = 3.7, 12.3, 1 H), 3.26 (br. t, J ca. 8.5, 1 H), 3.15 (d, J = 14.1, 1 H), 2.78 (s, 3 H), 2.22-2.03 (m, 3 H), 1.37 (s, 9 H).

### Synthesis of acid Ex.364

Ester **Ex.363** (1.5 g, 2.6 mmol) was hydrogenolyzed in MeOH (30 mL) in the presence of 10% palladium on charcoal (moistened with 50% H₂O; 0.34 g) for 4 h, filtered through a pad of celite and concentrated to afford **Ex.364** (1.26 g, 97%).

Data of **Ex.364:** C₂₃H₃₁N₃O₈ (477.5). LC-MS (method 10c): Rₜ = 1.42 (98), 478 ([M+H]⁺).

### Core 24a: Synthesis of Ex.379 and Ex.380 (Scheme 28)

### Synthesis of Mitsunobu product Ex.379

Following procedure **B.1.2,** the reaction of phenol **200** (4.31 g, 10.1 mmol), alcohol **14** (3.64 g, 12.1 mmol) and CMBP (4.88 g, 20.2 mmol) in dry toluene (180 mL) afforded after 2 h and after FC (hexane/EtOAc/MeOH gradient) the protected amino acid **224** (8.8 g, contaminated with ca. 20% of tributylphosphine oxide; used without any further purification).

### Synthesis of amino acid 225

Following procedure **B.2,** the reaction of **224** (8.8 g), 1,3-dimethylbarbituric acid (3.86 g, 24.7 mmol) and Pd(PPh₃)₄ (57 mg) in EtOAc/CH₂Cl₂ (1:1, 88 mL) yielded after 3 h, dilution with EtOAc and centrifugation an orange solid, which was washed (EtOAc) to give **225** (6.02 g, ca. 80% over the two steps).

Data of **225:** C₃₀H₄₀N₄O₈ (584.6). LC-MS (method 4a): Rₜ = 1.70 (90), 585 ([M+H]⁺). Purity based on ¹H-NMR ca. 80%; used without further purification.

### Synthesis of macrolactam Ex.379

According to procedure **F.1.1,** the amino acid **225** (2.0 g, 3.06 mmol) and *i*-Pr₂NEt (1.0 mL, 5.9 mmol) in dry DMF (20 mL) was treated with T3P (50% in EtOAc; 3.6 mL, 6.1 mmol) and *i*-Pr₂NEt (1.2 mL, 7 mmol) in CH₂Cl₂ (550 mL) to afford after evaporation of the volatiles and FC (hexane/EtOAc/MeOH gradient) **Ex.379** (1.23 g, 71%).

Data of **Ex.379:** C₃₀H₃₈N₄O₇ (566.6). LC-MS (method 10c): Rₜ = 1.94 (98), 567 ([M+_{H]}⁺). ¹H-NMR (DMSO-d₆): 7.98 (d, J = 6.8, 0.5 H), 7.63 (br. t, 0.5 H), 7.56 (d, J = 7.5, 0.5 H), 7.37-7.11 (several m, 8.5 H), 7.00 (d, J = 7.4, 1 H), 6.87 (d, J = 7.4, 0.5 H), 6.82 (s, 0.5 H), 5.03-4.87 (s and m, 3 H), 4.41 (m, 0.5 H), 4.28 (t-like m, 0.5 H), 4.13 (m, 1 H), 3.97-3.65 (m, 3 H), 3.04 (br. d, J ca. 11.1, 1 H), 2.81 (m, 1 H), 2.30 (t, J = 9.5, 1 H), 2.14 (m, 1 H), 1.90 (m, 1 H), 1.66 (m, 2 H), 1.40, 1.34 (2 s, 9 H), 1.40-1.15 (m, 2 H).

### Synthesis of amine Ex.380

According to procedure **K,** benzyl carbamate **Ex.379** (700 mg, 1.23 mmol) was hydrogenolyzed in MeOH (7.0 mL) in the presence of catalyst (139 mg) for 2 h. After filtration and evaporation of the volatiles, **Ex.380** (550 mg, 100%) was obtained.

Data of **Ex.380:** C₂₂H₃₂N₄O₅ (432.5). LC-MS (method 10a): Rₜ = 1.34 (100), 433 ([M+H]⁺).

### Core 24b: Synthesis of Ex.392 and Ex.393 (Scheme 28)

### Synthesis of Mitsunobu product 226

Following procedure **B.1.2,** the reaction of phenol **200** (2.74 g, 6.4 mmol), alcohol **156** (2.14 g, 7.7 mmol) and CMBP (3.1 g, 12.8 mmol) in dry toluene (96 mL) afforded after 2 h and after FC (hexane/EtOAc gradient) the protected amino acid **226** (3.16 g, 72%).

### Synthesis of amino acid 227

Following procedure **B.2,** the reaction of **226** (2.98 g, 4.35 mmol), 1,3-dimethylbarbituric acid (1.7 g, 10.9 mmol) and Pd(PPh₃)₄ (25 mg) in EtOAc/CH₂Cl₂ (1:1, 58 mL) yielded after 4 h, evaporation of the volatiles and repeated washing (EtOAc) of the residue the amino acid **227** (2.42 g, 97%).

Data of **227:** C₃₁H₃₅N₃O₇ (561.6). LC-MS (method 10a): Rₜ = 1.59 (97), 562 ([M+H]⁺).

### Synthesis of macrolactam Ex.392

To a soln of HATU (1.24 g, 3.3 mmol), HOAt (0.44 g, 3.2 mmol) and *i*-Pr₂NEt (1.2 mL, 6.5 mmol) in DMF (1600 mL) was added within 2 h via syringe pump a soln of **227** (0.916 g, 1.6 mmol) in DMF (40 mL). The mixture was stirred for 20 h at rt, followed by evaporation of the volatiles. FC (hexane/EtOAc/MeOH gradient) afforded **Ex.392** (0.78 g, 88%).

Data of **Ex.392:** C₃₁H₃₃N₃O₆ (543.6). LC-MS (method 10b): Rₜ = 2.04 (96), 544 ([M+H]⁺).

### Synthesis of amine Ex.393

According to procedure **K,** benzyl carbamate **Ex.392** (66 mg, 0.12 mmol) was hydrogenolyzed in MeOH (1.5 mL) in the presence of catalyst (25 mg) for 1 h. After filtration, evaporation of the volatiles and FC (CH₂Cl₂/MeOH 95:5 to 80:20) **Ex.393** (40 mg, 80%) was obtained.

Data of **Ex.393:** C₂₃H₂₇N₃O₄ (409.5). LC-MS (method 10a): Rₜ = 1.34 (99), 410 ([M+H]⁺). ¹H-NMR (DMSO-d₆): 7.93 (d, J = 6.2, 0.67 H), 7.75 (br. s, 0.33 H), 7.37-6.81 (m, 8 H), 6.74 (d, J = 8.0, 1 H), 4.94 (d, J = 13.8, 1 H), 4.86 m, 1 H), 4.47-4.30 (m, 1 H), 4.25-4.04 (m, 2 H), 3.74 (br. s, 1 H), 3.12 (t-like m, 1 H), 2.80 (m, 1 H), 2.66 (dd, J = 5.7, 11.1, 1 H), 2.50-2.00 (several m, 2 H), 1.80-1.50 (br. m, 3 H), 1.31 (m, 1 H).

### Core 24c: Synthesis of Ex.398 (Scheme 28)

### Synthesis of the amide 228

A soln of **198** (750 mg, 1.53 mmol) in CH₂Cl₂ (14 mL) was treated with SOCl₂ (0.34 mL, 4.59 mmol) and heated to 45°C for 1 h. The volatiles were evaporated. **190˙**HCl (ca. 50%w/w; 1.01 g, 2.4 mmol) and CH₂Cl₂ (14 mL) were added. The mixture was cooled to 0°C. *i*-Pr₂NEt (0.785 mL, 4.5 mmol) was slowly added and stirring was continued for 1 h. The volatiles were evaporated. Aqueous workup (EtOAc, 0.2 M aq. HCl soln) and FC (hexane/EtOAc 1:1) afforded **228** (693 mg, 70%).

Data of **228:** C₃₁H₃₇BrN₂O₈ (645.5). LC-MS (method 10a): Rₜ = 2.32 (95), 647/645 ([M+H]⁺).

### Synthesis of amino acid 229

Following procedure **B.2,** the reaction of **228** (680 mg, 1.05 mmol), 1,3-dimethylbarbituric acid (395 mg, 2.5 mmol) and Pd(PPh₃)₄ (61 mg) in EtOAc/CH₂Cl₂ (9:8, 17 mL) yielded after 2 h, evaporation of the volatiles and washing (EtOAc) **229** (571 mg, quant. yield).

Data of **229:** C₂₄H₂₉BrN₂O₆ (521.4). LC-MS (method 10a): Rₜ = 1.39 (94), 521/523 ([M+H]⁺).

### Synthesis of macrolactam Ex.398

A suspension of amino acid **229** (550 mg, 1.06 mmol) in dry CH₂Cl₂ (100 mL) was added over 2 h to a soln of T3P (50% in EtOAc; 1.5 mL, 2.6 mmol) and *i*-Pr₂NEt (0.72 mL, 4.2 mmol) in CH₂Cl₂ (930 mL). The mixture was stirred for 2 h. More T3P 50% in EtOAc; 1.5 mL, 2.6 mmol) and *i*-Pr₂NEt (0.72 mL, 4.2 mmol) in CH₂Cl₂ (30 mL) were added and stirring was continued until completion of the transformation. Evaporation of the solvent, aqueous workup (EtOAc, 0.1 M aq. HCl soln, sat. aq. NaHCO₃ soln; Na₂SO₄) and FC (CH₂Cl₂/MeOH 100:0 to 95:5) afforded **Ex.398** (254 mg, 47%).

Data of **Ex.398:** C₂₄H₂₇BrN₂O₅ (503.3). LC-MS (method 10a): Rₜ = 1.81 (97), 505/503 ([M+H]⁺). ¹H-NMR (DMSO-d₆): 8.16-6.77 (several m, 9 H), 4.96-4.81 (m, 1 H), 4.71 (d, J = 6.9, exchanged upon addition of D₂O, OH), 4.54-3.97 (several m, 6 H), 3.65-3.30 (several m, 2 H), 3.11 (d-like m, 1 H), 2.90-2.60 (several m, 1 H), 2.40-1.50 (several br. m, 6 H).

### Core 25: Synthesis of Ex.403 and Ex.404 (Scheme 29)

### Synthesis of Mitsunobu product 230

At rt CMBP (2.44 g, 9.6 mmol) was added to a soln of alcohol **167** (2.31 g, 6.41 mmol) in toluene (100 mL). The mixture was heated to 100°C for 30 min, followed by the addition of a soln of phenol **202** (2.82 g, 6.15 mmol) in toluene (25 mL). The mixture was stirred for 3 h at 100°C and concentrated. FC (hexane/acetone) afforded **230** (3.17 g, 62%).

Data of **230:** C₄₀H₅₇FN₄O₁₀Si (800.9). LC-MS (method 4b): Rₜ = 2.67 (93), 801 ([M+H]⁺).

### Synthesis of amino ester 231

At 0°C TBAF (1 M in THF; 15.7 mL, 15.7 mmol) was added to a soln of **230** (3.15 g, 3.96 mmol) in THF (50 mL). The mixture was stirred at 0°C to rt for 3 h. More TBAF soln (15.7 mL) was added and stirring continued for 4 h. The mixture was concentrated and the residue dissolved in CH₂Cl₂, washed (sat. aq. NaHCO₃ soln, sat. aq. NaCl soln), dried (Na₂SO₄), filtered and concentrated. FC (CH₂Cl₂/MeOH/conc. aq. NH₃ soln 95:5:0.5 to 90:10:0.5) afforded **231** (2.06 g, 87%).

### Synthesis of amino acid 232

A soln of **231** (2.0 g, 3.05 mmol), 1,3-dimethylbarbituric acid (0.59 g, 3.81 mmol) and Pd(PPh₃)₄ (0.35 g) in EtOAc/CH₂Cl₂ (50:50, 35 mL) was stirred at rt for 2 h, concentrated and purified by FC (CH₂Cl₂/MeOH 100:0 to 90:10, then 70:30) to yield **232** (1.36 g, 72%).

Data of **232:** C₃₁H₄₁FN₄O₈ (616.6). LC-MS (method 7): Rₜ = 1.09 (98), 617 ([M+H]⁺).

### Synthesis of the macrolactam Ex.403

According to procedure **F.1.2,** a soln of **232** (1.34 g, 2.17 mmol) in dry DMF (100 mL) was treated with FDPP (1.67 g, 4.35 mmol) in DMF (2070 mL). Purification by FC (hexane/EtOAc/MeOH 60:40:0 to 0:90:10) afforded **Ex.403** (0.934 g, 73%).

Data of **Ex.403:** C₃₁H₃₉FN₄O₇ (598.6). LC-MS (method 4b): Rₜ = 2.16 (100), 599 ([M+H]⁺). ¹H-NMR (DMSO-d₆): 8.69, 8.51 (2 br. s, 1 H), 7.51 (d, J = 7.5, 1H), 7.40-7.28 (m, 5 H), 7.19-7.04 (m, 3 H), 5.01 (s, 2 H), 4.82, 4.55 (2 br. s, 1 H), 4.20-3.80 (m, 4 H), 3.75-3.26 (m, 4 H, partially superimposed by the H₂O signal), 2.70 (s, 3 H), 2.66 (m, 1 H), 2.22 (m, 1 H), ca. 1.8-1.2 (br. m, 4 H), 1.42, 1.35 (2 s, 9 H).

### Synthesis of amine Ex.404

According to procedure **K,** benzyl carbamate **Ex.403** (180 mg, 0.30 mmol) was hydrogenolyzed in MeOH (2.0 mL) in the presence of catalyst (34 mg) for 2 h. After filtration and evaporation of the volatiles, **Ex.404** (139 mg, 99%) was obtained.

Data of **Ex.404:** C₂₃H₃₃FN₄O₅ (464.5). LC-MS (method 4a): Rₜ = 1.61 (98), 465 ([M+H]⁺).

### Core 26: Synthesis of Ex.415 and Ex.416 (Scheme 30)

### Synthesis of Mitsunobu product 233

According to procedure **E.1.1,** a mixture of phenol **205** (1.65 g, 3.95 mmol), alcohol **82** (1.75 g, 4.7 mmol) and PPh₃ (3.08 g, 11.7 mmol) in dry benzene (42 mL) was treated at 0°C with DEAD (40% in toluene, 5.4 mL, 11.7 mmol) in benzene (28 mL). After 16 h at rt, additional PPh₃ (1.36 g, 5.2 mmol) and alcohol **82** (0.35 g, 0.94 mmol) in benzene (8.4 mL) were added, followed by DEAD (40% in toluene, 2.4 mL, 5.2 mmol) in benzene (11 mL). Stirring was continued for 7 h. Evaporation of the volatiles and repeated FC (CH₂Cl₂/MeOH and hexane/EtOAc) afforded **233** (2.27 g, 75%, contaminated with triphenylphosphine oxide; used without further purification).

### Synthesis of amino acid 234

Following procedure **E.2,** the reaction of **233** (2.26 g, 2.93 mmol), 1,3-dimethylbarbituric acid (1.14 g, 7.33 mmol) and Pd(PPh₃)₄ (0.37 g) in EtOAc/CH₂Cl₂ (50:50, 32 mL) yielded after 30 min and after FC (EtOAc, then CH₂Cl₂/MeOH 90:10 to 60:40) **234** (1.40 g, 74%).

Data of **234:** C₃₁H₄₂N₄O₉S (646.7). LC-MS (method 4b): Rₜ = 1.76 (95), 647 ([M+H]⁺).

### Synthesis of the protected macrolactam Ex.415

According to procedure **F.1.1,** amino acid **234** (500 mg, 0.77 mmol) in dry CH₂Cl₂ (11 mL) was treated with T3P (50% in EtOAc, 0.92 mL, 1.5 mmol) and *i*-Pr₂NEt (0.53 mL, 3.1 mmol) in CH₂Cl₂ (66 mL). After stirring at rt for 1 h, aqueous workup (EtOAc, sat. aq. NaHCO₃ soln) and FC (EtOAc), **Ex.415** (0.34 g, 69%) was obtained.

Data of **Ex.415:** C₃₁H₄₀N₄O₈S (628.7). LC-MS (method 4b): Rₜ = 2.05 96), 629 ([M+H]⁺). ¹H-NMR (CDCl₃): 7.39-7.29 (m, 6 H), 6.74 (d, J = 5.5, 1 H), 5.21 (J = 12.3, 1 H), 5.15 (d, J = 12.3, 1 H), 5.15 (m, 1 H), 5.00 (br. dd, J = 2.7, 10.6, 1 H), 4.40 (br. t, J = 7.4, 1 H), 4.23-4.18 (m, 2 H), 3.98 (br. d, J = 10.2, 1 H), 3.75-3.61 (m, 2 H), 3.45 (br. d, 1 H), 3.06 (s, 3 H), 2.91 (s, 3 H), 2.58-2.06 (m, 7 H), 1.45 (s, 9 H).

### Synthesis of acid Ex.416

According to procedure **H,** ester **Ex.415** (530 mg, 0.84 mmol) was hydrogenolyzed in MeOH (9 mL)/THF (9 mL) in the presence of catalyst (0.55 g) for 6 h to afford after filtration and washing of the residue with CHCl₃ **Ex.416** (494 mg, quant. yield).

Data of **Ex.416:** C₂₄H₃₄N₄O₈S (538.6). LC-MS (method 4b): Rₜ = 1.61 (92), 539 ([M+H]⁺).

### Core 27: Synthesis of Ex.424 and Ex.425 (Scheme 31)

### Synthesis of Mitsunobu product 235

Following procedure **B.1.2,** the reaction of phenol **207** (12.1 g, 26.5 mmol), alcohol **14** (9.57 g, 31.8 mmol) and CMBP (12.8 g, 53.1 mmol) in dry toluene (354 mL) afforded after FC (hexane/EtOAc 50:50 to 0:100) the protected amino acid **235** (19.2 g, 98%).

### Synthesis of amino acid 236

Following procedure **B.2,** the reaction of **235** (19.1 g, 25.8 mmol), 1,3-dimethylbarbituric acid (10.1 g, 64.7 mmol) and Pd(PPh₃)₄ (0.3 g) in EtOAc/CH₂Cl₂ (55:45, 278 mL) yielded after 2 h and after FC (CH₂Cl₂/MeOH 98:2 to 90:10, then 70:30) **236** (14.38 g, 91%).

Data of **236:** C₃₁H₄₃N₅O₈ (613.7). LC-MS (method 4a): Rₜ = 1.65 (95), 614 ([M+H]⁺)_{.}

### Synthesis of the protected macrolactam Ex.424

According to procedure **F.1.1,** the amino acid **236** (3.37 g, 5.5 mmol) in dry CH₂Cl₂ (50 mL) was treated with T3P (50% in EtOAc; 9.7 mL, 16.5 mmol) and *i*-Pr₂Et (4.7 mL, 27.6 mmol) in CH₂Cl₂ (1100 mL). Aqueous workup (CH₂Cl₂, 1 M aq. Na₂CO₃ soln) and FC (CH₂Cl₂/MeOH 100:0 to 95:5) yielded **Ex.424** (2.58 g, 79%).

Data of **Ex.424:** C₃₁H₄₁N₅O₇ (595.6). LC-MS (method 11a): Rₜ = 1.87 (95), 596 ([M+H]⁺). ¹H-NMR (DMSO-d₆) : 8.39 (d, J = 2.7, 1 H), 8.15 (d, J = 1.5, 1 H), 7.37-7.26 (m, 7 H), 7.20 (d, J = 6.3, 1 H), 4.99 (s, 2 H), 4.65 (d, J = 9.8, 1H), 4.23-3.93 (m, 5 H), 3.16-2.94 (m, 2 H), 3.14 (t, J ca. 9.0, 1 H), 2.94 (s, 3 H), 2.30 (m, 1 H), 2.04 (br. q, 1 H), 1.67 (m, 1 H), 1.41 (s, 9 H), 1.30-0.93 (m, 5 H).

### Synthesis of amine Ex.425

According to procedure **K**, benzyl carbamate **Ex.424** (600 mg, 1.0 mmol) was hydrogenolyzed in MeOH (12 mL) in the presence of catalyst (113 mg) for 2 h to afford **Ex.425** (482 mg, quant. yield).

Data of **Ex.425:** C₂₃H₃₅N₅O₅ (461.5). LC-MS (method 4a): Rₜ = 1.32 (99), 462 ([M+H]⁺).

### Core 28: Synthesis of Ex.435, Ex.436 and Ex.437 (Scheme 32)

### Synthesis of Mitsunobu product 237

Tributylphosphine (7.0 mL, 28.2 mmol) was added to a soln of phenol **210** (4.5 g, 9.4 mmol) and alcohol **82** (5.2 g, 14.1 mmol) in dry, degassed benzene (235 mL). The mixture was cooled to 0°C, followed by the slow addition of TMAD (4.85 g, 28.1 mmol) in benzene (40 mL). The soln was stirred for 30 min at 0°C and for 15 h at rt and filtered. The filtrate was concentrated and purified by FC (hexane/EtOAc 40:60 to 25:75) to yield **237** (7.2 g, 92%).

### Synthesis of amino acid 238

Following procedure **B.2,** the reaction of **237** (7.15 g, 8.6 mmol), 1,3-dimethylbarbituric acid (3.35 g, 21.5 mmol) and Pd(PPh₃)₄ (0.5 g) in EtOAc/CH₂Cl₂ (45:55, 145 mL) yielded after 16 h and after FC (CH₂Cl₂/MeOH 100:0 to 70:30) **238** (4.80 g, 79%).

Data of **238:** C₃₇H₄₉N₅O₉ (707.8). LC-MS (method 10a): Rₜ = 1.76 (98), 708 ([M+H]⁺).

### Synthesis of the protected macrolactam Ex.435

According to procedure **F.1.1,** the amino acid **238** (4.7 g, 6.6 mmol) in dry CH₂Cl₂ (100 mL) was treated with T3P (50% in EtOAc; 9.9 mL, 16.7 mmol) and *i*-Pr₂NEt (4.6 mL, 26.8 mmol) in CH₂Cl₂ (570 mL). Aqueous workup (CH₂Cl₂, sat. aq. NaHCO₃ soln) after completion of the addition and FC (EtOAc/CH₃CN 95:5) yielded **Ex.435**(2.7 g, 58%).

Data of **Ex.435:** C₃₇H₄₇N₅O₈ (689.8) . LC-MS (method 10a): Rₜ = 2.06 (100), 690 ([M+H]⁺). ¹H-NMR (C₆D₆): 7.34-7.09 (m, 8 H), 6.87 (d, J = 9.0, 1 H), 5.59 (br. d, J ca. 12.7, 1 H), 5.20 (br. d, J ca. 12.7, 1 H), 5.15 (br. d, J ca. 12.6, 1 H), 4.75-4.60 (m, 3 H), 4.20 (br. d, J ca. 7.6, 1 H), 3.88 (br. d, J ca. 9.4, 1 H), 3.73-3.62 (m, 2 H), ca. 3.2 (m, 1 H), 3.08 (s, 3 H), 2.97 (s, 3 H), 2.75 (s, 3 H), 2.72-2.57 (m, 2 H), 2.27 (s, 3 H), 2.27-2.04 (m, 2 H), 1.58 (s, 9 H), 1.54-1.41 (m, 2 H).

### Synthesis of acid Ex.436

According to procedure **H,** a soln of **Ex.435** (300 mg, 0.43 mmol) in MeOH (18 mL)/THF (6 mL) was hydrogenolyzed in the presence of catalyst (163 mg) to yield **Ex.436** (250 mg, 91%).

Data of **Ex.436:** C₃₀H₄₁N₅O₈ (599.6). LC-MS (method 10a): Rₜ = 1.62 (98), 600 ([M+H]⁺).

### Synthesis of amine Ex.437

TFA (1 mL) was added at 0°C to a soln of **Ex.435** (320 mg, 0.46 mmol) in CH₂Cl₂ (3 mL). The soln was allowed to warm to rt over 2 h. The volatiles were evaporated. Residual solvent was removed by co-evaporation (twice) with CHCl₃ to afford **Ex.437·**CF₃CO₂H (322 mg, 98%).

Data of **Ex.437·**CF₃CO₂H: C₃₂H₃₉N₅O₆**·**C₂HF₃O₂ (589.7, free base). LC-MS (method 10a): Rₜ = 1.48 (97), 590([M+H]⁺).

### Core 29: Synthesis of Ex.447 and Ex.448 (Scheme 33)

### Synthesis of bis-olefine 240

A mixture of **194** (1.515 g, 3.64 mmol), **51** (2.72 g, 5.46 mmol), HATU (2.075 g, 5.46 mmol) and HOAt (743 mg, 5.46 mmol) in DMF (36 mL) was cooled to 0°C. *i*-Pr₂NEt (2.0 mL, 11.7 mmol) was slowly added and the soln was stirred for 2 h. Aqueous workup (EtOAc, 1 M aq. Na₂O₃ soln; Na₂SO₄) and FC (hexane/EtOAc afforded **240** (1.96 g, 84%).

Data of **240:** C₃₅H₃₉BrN₂O₅ (647.6). LC-MS (method 10a): Rₜ = 2.99 (93), 649/647 ([M+H]⁺).

### Synthesis of macrocycle Ex.447

[1,3-Bis(2,4,6-trimethylphenyl)-2-imidazolidinylidene]dichloro-(3-phenyl-1H-inden-1-ylidene)pyridyl)ruthenium(II) (Umicore M31 catalyst; 10 mg) was added to a degassed soln of **240** (80 mg, 0.124 mmol) in CH₂Cl₂ (15 mL). The soln was stirred at 40°C for 24 h. Evaporation of the solvent and purification by prep. HPLC method 1 afforded **Ex.447** (24 mg, 31%).

Data of **Ex.447:** C₃₃H₃₅BrN₂O₅ (619.5). LC-MS (method 10b): Rₜ = 2.79 (95), 621/619([M+H]⁺).

### Synthesis of amino alcohol Ex.448

A soln of **Ex.447** (79 mg, 0.128 mmol) in MeOH (1.5 mL) and THF (0.5 mL) was hydrogenated for 5 h under normal pressure at rt in the presence of palladium hydroxide on activated charcoal (moistened with 50% H₂O; 30 mg). The mixture was filtered through a pad of celite. The filtrate was concentrated, suspended in CH₂Cl₂ and filtered. The solid was then further purified by prep HPLC method 1 to afford **Ex.448·**CF₃CO₂H (24 mg, 50%).

Data of **Ex.448·**CF₃CO₂H: C₁₈H₂₆N₂O₃**·**C₂HF₃O₂ (318.4, free base). LC-MS (method 10a): 1.22 (91), 319 ([M+H]⁺). ¹H-NMR (DMSO-d₆): 7.96 (br. s, 3 H), 7.16 (t, J = 7.7, 1 H), 6.78-6.72 (m, 3 H), 5.34 (br. s, 1 H), 4.76 (d, J = 11.1, 1 H), 4.18-4.10 (m, 4 H), 3.84 (dd, J = 6.7, 10.0, 1 H), 2.83 (t-like m, J ca. 9.4, 1 H), 2.70 (m, 1 H), 2.45 (m, 1 H), 2.25 (m, 1 H), 2.07 (m, 1 H), 1.80-0.80 (several br. m, 8 H).

### Core 30: Synthesis of Ex.451, Ex.452 and Ex.453 (Scheme 34)

### Synthesis of Mitsunobu product 241

Following procedure **B.1.2,** the reaction of thiophenol **212** (2.0 g, 4.4 mmol), alcohol **161** (1.9 g, 5.3 mmol) and CMBP (1.6 g, 6.6 mmol) in dry toluene (87 mL) afforded after 1 h and after FC (hexane/Et₂O 30:70 to 0:100, then hexane/EtOAc 50:50 to 30:70) the protected amino acid **241** (2.43 g, crude, used without further purification).

### Synthesis of amino acid 242

Following procedure **B.2,** the reaction of **241** (2.38 g) 1,3-dimethylbarbituric acid (1.1 g, 7.1 mmol) and Pd(PPh₃)₄ (0.17 g) in EtOAc/CH₂Cl₂ (55:45, 52 mL) yielded after 2 h and after FC (EtOAc, then CH₂Cl₂/MeOH 100:0 to 80:20) **242** (1.89 g, 64% over the two steps).

Data of **242:** C₃₃H₃₈BrN₃O₆S (684.6). LC-MS (method 10a): Rₜ = 1.83 (94), 686/684 ([M+H]⁺).

### Synthesis of the protected macrolactam Ex.451

According to procedure **F.1.1,** amino acid **242** (1.5 g, 2.2 mmol) in dry CH₂Cl₂ (45 mL) was treated with T3P (50% in EtOAc; 4.5 mL, 7.7 mmol) and *i*-Pr₂NEt (2.6 mL, 15 mmol) in CH₂Cl₂ (1080 mL). After 3 h at rt, aqueous workup (CH₂Cl₂, sat. aq. Na₂CO₃ soln) and FC (hexane/EtOAc/MeOH) yielded **Ex.451** (1.15 g, 79%).

Data of **Ex.451:** C₃₃H₃₆BrN₃O₅S (666.6). LC-MS (method 10b): Rₜ = 2.33 (96), 668/666 ([M+H]⁺). ¹H-NMR (DMSO-d₆) : 7.85 (s, 0.5 H), 7.69 (d, J = 8.0, 0.5 H), 7.57 (d, J = 7.6, 2 H), 7.50 (d, J = 7.9, 0.5 H), 7.39-7.18 (m, 10.5 H), 5.08 (d, J = 12.6, 0.5 H), 4.99 (s, 1 H), 4.96 (d, J = 12.8, 0.5 H), 4.49-4.38 (4 d, 2 H), 4.16 (t-like m, 0.5 H), 4.00-3.65 (m, 3 H), 3.60-3.45 (m, 1 H), 3.12-2.98 (m, 3 H), 2.91, 2.89 (2 s, 3 H), 2.55-2.44 (m, 1 H, superimposed by DMSO-d signal), 2.35-2.15 (br. m, 2 H), 1.70-1.20 (several br. m, 4 H).

### Synthesis of the sulfone Ex.452

m-CPBA (70%; 462 mg, 1.88 mmol) was added at 0°C to a soln of **Ex.451** (500 mg, 0.75 mmol) in CH₂Cl₂ (12 mL). The mixture was stirred for 15 min at 0°C and for 2 h at rt. The mixture was diluted with CH₂Cl₂, washed (sat. aq. NaHCO₃ soln, 10% aq. Na₂S₂O₃ soln, sat. aq. NaHCO₃ soln), dried (Na₂SO₄), filtered and concentrated to yield **Ex.452** (471 mg, 89%).

Data of **Ex.452:** C₃₃H₃₆BrN₃O₇S (698.6). LC-MS (method 10a): Rₜ = 2.13 (95), 700/698 ([M+H]⁺). ¹H-NMR (DMSO-d₆): 8.16 (s, 0.75 H), 8.05-7.26 (several m, 13.25 H), 5.01 (d, J = 12.6, 0.75 H), 4.97 (s, 0.5 H), 4.89 (d, J = 12.6, 0.75 H), 4.53 (s, 1.5 H), 4.53-4.10 (m, 2.5 H), 4.10-3.65 (m, 2 H), 3.45 (s, 2 H), 3.25-2.75 (br. m, 3 H), 2.97 (s, 3 H), ca. 2.6 (m, 1 H), 2.35-2.20 (m, 1 H), 1.80-1.30 (m, 4 H).

### Synthesis of amine Ex.453

NH₃ soln (7 M in MeOH, 0.49 mL, 3.43 mmol) was added to a soln of **Ex.452** (600 mg, 0.86 mmol) in MeOH (13 mL). The soln was hydrogenolyzed at rt under normal pressure for 17 h in the presence of 5% palladium on activated charcoal (moistened with 50% H₂O; 165 mg). The mixture was filtered through a pad of celite and Na₂CO₃. The filtrate was concentrated, dissolved in 1 M aq. HCl soln (15 mL) and washed with EtOAc. Sat. aq. Na₂CO₃ soln was added. The aqueous layer was treated with sat. aq. Na₂CO₃ soln and repeatedly extracted with CHCl₃. The combined organic phase was dried (Na₂SO₄), filtered and concentrated to give **Ex.453** (369 mg, 88%).

Data of **Ex.453:** C₂₅H₃₁N₃O₅S (485.6). LC-MS (method 10a): Rₜ = 1.29 (98), 486 ([M+H]⁺).

### Core 31: Synthesis of Ex.460, Ex.461, Ex.462 and Ex.466 (Scheme 35)

### Synthesis of Mitsunobu product 243

A soln of phenol **213** (770 mg, 1.9 mmol) in dry benzene (10 mL) was added to a soln of PPh₃ (776 mg, 2.9 mmol) and alcohol **161** (865 mg, 2.3 mmol) in benzene (25 mL). The soln was cooled to 0°C. DEAD (40% in toluene, 1.33 mL, 2.9 mmol) was added slowly. The mixture was stirred at rt for 40 h followed by addition of more alcohol **161** (360 mg, 0.97 mmol) in benzene (3 mL), PPh₃ (510 mg, 1.9 mmol) and DEAD (40% in toluene, 0.9 mL, 2 mmol). Stirring was continued for 19 h. The mixture was treated with MeOH (1 mL), pre-adsorbed on silica gel and purified by FC (hexane/EtOAc 80:20 to 75:25) to give **243** (810 mg, 56%).

### Synthesis of amino acid 244

Following procedure **B.2,** the reaction of **243** (949 mg, 1.27 mmol) 1,3-dimethylbarbituric acid (476 mg, 3.0 mmol) and Pd(PPh₃)₄ (73 mg) in EtOAc/CH₂Cl₂ (55:45, 22 mL) yielded after 3 h and after FC (EtOAc; CH₂Cl₂/MeOH 95:5 to 75:25) **244** (492 mg, 62%).

Data of **244:** C₂₉H₃₉BrN₂O₆S (623.6). LC-MS (method 10a): Rₜ = 1.93 (99), 625/623 ([M+H]⁺).

### Synthesis of the protected macrolactam Ex.460

According to procedure **F.1.1,** amino acid **244** (491 mg, 0.79 mmol) in dry CH₂Cl₂ (10 mL) was treated with T3P (50% in EtOAc; 0.93 mL, 1.57 mmol) and *i*-Pr₂NEt (0.54 mL, 3.1 mmol) in CH₂Cl₂ (385 mL). After 4 h at rt, aqueous workup (CH₂Cl₂, sat. aq. NaHCO₃ soln) and FC (hexane/EtOAc 1:2) yielded **Ex.460** (471 mg, 99%) .

Data of **Ex.460:** C₂₉H₃₇BrN₂O₅S (605.6). LC-MS (method 10a): Rₜ = 2.84 (99), 607/605 ([M+H]⁺). ¹H-NMR (DMSO-d₆) : 7.58 (d, J = 8.4, 2 H), 7.46 (d, J = 8.5, 1 H), 7.34 (d, J = 8.4, 2 H), 7.21-7.15 (m, 2 H), 6.82 (d, J = 7.6, 1 H), 6.72 (d, J = 8.2, 1 H), 4.96 (d, J = 9.5, 1 H), 4.53 (s, 2 H), 4.41 (t-like m, 1 H), 4.30-4.10 (m, 3 H), 3.54 (s, 2 H), 3.12 (br. t, J = 12.5, 1 H), 2.78 (t-like m, 1 H), 2.35 (d, J = 13. 9, 1 H), 2.11 (m, 1 H), 1.77 (m, 1 H), 1.61-1.32 (m, 5 H), 1.40 (s, 9 H).

### Synthesis of sulfone Ex.461

m-CPBA (70%; 440 mg, 1.78 mmol) was added at 0°C to a soln of **Ex.460** (432 mg, 0.71 mmol) in CH₂Cl₂ (10 mL). The mixture was stirred for 15 min at 0°C and for 2 h at rt. The mixture was diluted with CH₂Cl₂, washed (sat. aq. NaHCO₃ soln, 10% aq. Na₂S₂O₃ soln, sat. aq. NaHCO₃ soln), dried (Na₂SO₄), filtered and concentrated to yield **Ex.461** (443 mg, 97%).

Data of **Ex.461:** C₂₉H₃₇BrN₂O₇S (637.6). LC-MS (method 10a): Rₜ = 2.41 (93), 639/637 ([M+H]⁺). ¹H-NMR (CDCl₃): 7.54-7.42 (m, 4 H), 7.28-7.24 (m, 3 H), 7.20 (td, J = 2.6, 6.8, 1 H), 5.16 (d, J = 8. 4, 1 H), 4. 91 (dd, J = 1.7, 12. 8, 1 H), 4. 55 (s, 2 H), 4.40 (t-like m, 1 H), 4.31-4.24 (m, 2 H), 4.15-4.04 (m, 2 H), 3.26-3.16 (m, 3 H), 2.35-2.26 (m, 2 H), 1.76-1.65 (m, 2 H) 1.42 (s, 9 H), 1.25-1.13 (m, 2 H), 1.05-0.80 (m, 2 H).

### Synthesis of amine Ex.462

A soln of **Ex.461** (414 mg, 0.649 mmol) in dioxane (2.0 mL) was treated at rt with 4M HCl-dioxane (4.0 mL) for 2 h. The mixture was concentrated. The remaining solid was washed with Et₂O and dried i.v. to afford **Ex.462·**HCl (325 mg, 87%).
Data of **Ex.462·**HCl: cf. **Table 47b**

### Synthesis of amine Ex.466

A soln of **Ex.460** (650 mg, 1.073 mmol) in dioxane (3.5 mL) was treated at rt with 4M HCl-dioxane (6.5 mL) for 4 h. The mixture was concentrated. The remaining solid was washed with Et₂O and dried i.v. to afford **Ex.466·**HCl (523 mg, 90%).
Data of **Ex.466·**HCl: cf. **Table 47b**

### Core 32: Synthesis of Ex.470 - Ex.475 (Scheme 36)

### Synthesis of acid 246

*i*-Pr₂NEt (2.2 mL, 12.8 mmol) was added to a mixture of acid **196** (2.4 g, 4.3 mmol), aminoester hydrochloride **169·**HCl (0.925 g, 5.15 mmol), HATU (2.44 g, 6.4 mmol) and HOAt (0.876 g, 6.4 mmol) in DMF (50 mL). The mixture was stirred at rt for 12 h, treated with 1 M aq. HCl soln and extracted with EtOAc. The organic phase was dried (Na₂SO₄), filtered and concentrated. FC (hexane/EtOAc 1:1) afforded **245** (2.42 g, 82%) which was dissolved in THF (34 mL) and treated with 2 M aq. LiOH soln (26.2 mL, 52.4 mmol). The mixture was heated to 60°C for 5 h. The mixture was acidified with 1 M aq. HCl soln and extracted with EtOAc. The organic phase was dried (Na₂SO₄), filtered and concentrated to give **246** (2.3 g 97%).

Data of **246:** C₂₉H₄₄BrN₃O₈Si (670.6). LC-MS (method 10a): Rₜ = 2.50 (100), 670/672 ([M+H]⁺).

### Synthesis of amino acid 249

*i*-Pr₂NEt (1.59 mL, 9.3 mmol) was added to a mixture of acid **246** (2.1 g, 3.1 mmol), aminoester hydrochloride **171·**HCl (1.04 g, 3.7 mmol), HATU (1.41 g, 3.7 mmol) and HOAt (0.50 g, 3.7 mmol) in DMF (53 mL). The mixture was stirred at rt for 48 h, diluted with H₂O (170 mL), acidified with 1 M aq. HCl soln (30 mL) and extracted with EtOAc. The organic phase was dried (Na₂SO₄), filtered and concentrated. FC (hexane/EtOAc 2:1 to 1:1) afforded **247** (2.58 g, 83%)

At 0°C, TBAF (1 M in THF; 8.4 mL, 8.4 mmol) was added to a soln of **247** (2.52 g, 2.8 mmol) in THF (58 mL). The mixture was stirred at 0°C to rt for 15 h, treated with sat. aq. NaHCO₃ soln and extracted with EtOAc.

The organic phase was dried (Na₂SO₄), filtered and concentrated. FC (CH₂Cl₂/MeOH 100:0 to 98:2) yielded **248** (1.75 g, 82%), which was dissolved in THF (13 mL) and treated with 2 M aq. LiOH soln (3.45 mL, 6.9 mmol). The mixture was heated to 60°C for 2 h and concentrated. The residue was dissolved in H₂O, acidified with 3 M aq. HCl soln to pH ca. 1 and extracted with EtOAc. The organic phase was dried (Na₂SO₄), filtered and concentrated to give a colorless solid (2.2 g), which was suspended in Et₂O and filtered to afford **249·**HCl (1.69 g, 94%). Data of **249·**HCl: C₃₇H₄₅BrN₄O₇ (737.6, internal salt). LC-MS (method 10d): Rₜ = 1.92 (97), 737/739 ([M+H]⁺).

### Synthesis of macrolactam Ex.470

According to procedure **F.1.1,** amino acid **249** (120 mg, 0.16 mmol) in dry CH₂Cl₂ (20 mL) was treated with T3P (50% in EtOAc; 0.24 mL, 0.41 mmol) and *i*-Pr₂NEt (0.11 mL, 0.65 mmol) in CH₂Cl₂ (140 mL). After 2 h at rt, aqueous workup (CH₂Cl₂, sat. aq. NaHCO₃ soln) and FC (hexane/EtOAc 10:90 then CH₂Cl₂/MeOH 90:10) **Ex.470** (80 mg, 68%) was obtained.

Data of **Ex.470:** C₃₇H₄₃BrN₄O₆ (719.6). LC-MS (method 10a): Rₜ = 2.41 (97), 719/721 ([M+H]⁺). ¹H-NMR (DMSO-d₆): 7.89-7.79 (m, 4 H), 7.64 (s, 1 H), 7.57-7.36 (m, 6 H), 7.10-7.06 (m, 2 H), 4.31 (br. d, J ca. 8.9, 1 H), 4.24 (br. t, 1 H), 4.12 (m, 1 H), 4.03 (d, J ca. 7.6, 1 H), 3.95 (m, 1 H), 3.71 (t-like m, 1 H), 3.44-3.30 (m, 3 H), 3.15 (br. t, J ca. 12.7, 1 H), 2.98 (dd, J = 6.7, 13.2, 1 H), 2.37 (m, 1 H), 2.31 (d, J = 13.1, 1 H), 2.05-1.90 (m, 3 H), 1.65-1.37 (m, 5 H), 1.37 (s, 9 H).

### Synthesis of the Suzuki product Ex.471

A soln of Na₂CO₃ (13 mg, 0.125 mmol) in H₂O (0.225 mL) was added to a suspension of **Ex.470** (30 mg, 0.042 mmol), 2-naphthaleneboronic acid (14 mg, 0.083 mmol) and Pd(PPh₃)₄ (5 mg) in DME (1.75 mL). The mixture was heated to reflux for 2 h, diluted with H₂O and extracted with EtOAc. The organic layer was dried (Na₂SO₄), filtered and concentrated. FC (CH₂Cl₂/MeOH) yielded **Ex.471** (20 mg, 62%).
Data of **Ex.471:** cf. **Table 48b**

### Synthesis of amine Ex.474

A soln of **Ex.471** (15 mg, 0.02 mmol) in dioxane (0.91 mL) was treated with 4M HCl-dioxane (0.66 mL) at rt for 15 h. The volatiles were evaporated to afford **Ex.474·**HCl (14 mg, quant. yield).
Data of **Ex.474·**HCl: cf. **Table 48b**

### Synthesis of the Suzuki product Ex.472

**Ex.472** (20 mg, 62%) was obtained from **Ex.470** (30 mg, 0.042 mmol) and 1*H*-indol-4-ylboronic acid (6.7 mg, 0.42 mmol) by following the conditions described for the synthesis of **Ex.471.**
Data of **Ex.472:** cf. **Table 48b**

### Synthesis of amine Ex.475

A soln of **Ex.472** (14 mg, 0.02 mmol) in CH₃CN (1.0 mL) and H₂O (0.05 mL) was treated with bismuth(III)-trifluoromethane-sulphonate (12.1 mg, 0.018 mmol) and heated to 75°C for 5 h. The mixture was diluted with EtOAc, washed (sat. aq. Na₂CO₃ soln), dried (Na₂SO₄), filtered and concentrated. FC (CH₂Cl₂/MeOH 95:5 to 90:10) afforded **Ex.475** (10 mg, 83%).
Data of **Ex.475:** cf. **Table 48b**

### Synthesis of amine Ex.473

A soln of **Ex.470** (15 mg, 0.02 mmol) in dioxane (0.8 mL) was treated with 4M HCl-dioxane (0.7 mL) at rt for 15 h. The volatiles were evaporated to afford **Ex.473·**HCl (13 mg, 92%).
Data of **Ex.473·**HCl**:** cf. **Table 48b**

### Transformations on solid support

### Core 33: Synthesis of Ex.476 (Scheme 37)

### Synthesis of N-methyl-L-alanine allyl ester hydrochloride (257˙HCl)

4M HCl-dioxane (3.0 mL) was added to a suspension of N-methyl L-alanine (**256,** 1.05 g, 10.1 mmol) in CH₂Cl₂ (16 mL). PCl₅ (2.54 g, 12.2 mmol) was added portionwise at 0°C. The mixture was allowed to warm to rt over 15 h. The volatiles were evaporated. The residue was dissolved in CH₂Cl₂ (10 mL) and allyl alcohol (0.83 mL, 12.2 mmol) was added dropwise. The mixture was stirred for 2 h and concentrated to give **257·**HCl (1.80 g, 98%).

Data of **257·**HCl: C₇H₁₃NO₂**·**HCl (143.2, free base). ¹H-NMR (DMSO-d₆): 9,79 (br. s, 1 H), 9.40 (br. s, 1 H), 5.94 (m, 1 H), 5.38 (qd, J = 1.6, 17.3, 1 H), 5.28 (qd, J = 1.4, 10.5, 1 H), 4.85 (m, 2 H), 4.41 (q, J ca. 6.8, 1 H), 2.55 (s, 3 H), 1.48 (d, J = 7.2, 3 H).

### Synthesis of 9H-fluoren-9-ylmethyl (2S,4R)-4-[(tert-butoxycarbonyl)amino]-2-(hydroxymethyl)tetrahydro-1H-pyrrole-1-carboxylate (252)

**252** was obtained by Fmoc protection of the secondary amino group of **17·**HCl with (9H-fluoren-9-yl)methyl 2,5-dioxo-pyrrolidin-1-yl carbonate (FmocOSu) in dioxane in the presence of aq. NaHCO₃ soln under standard conditions.

Data of **252:** C₂₅H₃₀N₂O₅ (438.5) . LC-MS (method 6): Rₜ = 1.26 (99), 439 ([M+H]⁺).

### Synthesis of 9H-fluoren-9-ylmethyl (2S,4R)-2-(3-[(allyloxy)-carbonyl]phenoxymethyl)-4-aminotetrahydro-1H-pyrrole-1-carboxylate hydrochloride (254·HCl)

Oxalyl chloride (64 mL, 749 mmol) was added to a soln of 3-acetoxybenzoic acid (30 g, 167 mmol) in CH₂Cl₂ (300 mL). Few drops of DMF were added. The soln was stirred for 4 h at rt. The volatiles were evaporated. The residue was dissolved in CH₂Cl₂ (300 mL) and at 0°C allyl alcohol (34 mL, 500 mmol) was added. Triethylamine (23 mL, 167 mmol) was slowly added. The mixture was stirred for 1 h at rt followed by an aqueous workup (CH₂Cl₂, 1 M aq. HCl soln; Na₂SO₄). FC (hexane/EtOAc 17:3) yielded **250** (24.3 g, 65%).

The material was dissolved in THF (485 mL) and treated for 3 h at 0°C to rt with 3-dimethylaminopropylamine (25 mL, 1.8 mmol). The soln was diluted with EtOAc and washed with 0.1 M aq. HCl soln, then with sat. aq. NaCl soln, dried (Na₂SO₄) and concentrated to afford **251** (17.13 g, 85%).

A soln of phenol **251** (3.6 g, 20 mmol), alcohol **252** (11 g, 24 mmol) and PPh₃ (6.4 g, 24 mmol) in benzene (140 mL) was cooled to 0°C. DEAD (40% in toluene, 11 mL, 24 mmol) was slowly added. The mixture was stirred at rt for 16 h. The volatiles were evaporated. FC (CH₂Cl₂/EtOAc) afforded **253** (7.35 g, 61%).

Data of **253:** C₃₅H₃₈N₂O₇ (598.7). LC-MS (method 4b): Rₜ = 2.72 (96), 599 ([M+H]⁺), 243.

A soln of **253** (4.0 g, 6.68 mmol) in dioxane (11 mL) was treated with 4M HCl-dioxane (65 mL) for 3 h at rt.

The volatiles were evaporated to yield **254·**HCl (3.75 g, containing ca. 5% of dioxane, quant. yield).

Data of **254·**HCl: C₃₀H₃₀N₂O₅ (498.6, free base). LC-MS (method 4b): Rₜ = 2.00 (97), 499([M+H]⁺).

### Synthesis of 4-(4-((N-((3R,5S)-1-(((9H-fluoren-9-yl)methoxy)-carbonyl)-5-((3-(allyloxycarbonyl)phenoxy)methyl)pyrrolidin-3-yl)benzamido)methyl)-3,5-dimethoxyphenoxy)butanoic acid (255)

NaBH₃CN (117 mg, 1.86 mmol) was added to a mixture of **254·**HCl (1.0 g, 1.87 mmol) and 4-(4-formyl-3,5-dimethoxyphenoxy)-butanoic acid (501 mg, 1.86 mmol) in MeOH (33 mL). The mixture was stirred for 1 h at rt. More 4-(4-formyl-3,5-dimethoxy-phenoxy)butanoic acid (76 mg, 0.28 mmol) and NaBH₃CN (18 mg, 0.28 mmol) were added and stirring was continued for 15 min, followed by evaporation of the volatiles. The residue was dissolved in EtOAc, washed with 1 M aq. HCl soln and concentrated. The residue was dissolved in H₂O (13 mL). Dioxane (20 mL) and NaHCO₃ (471 mg, 5.6 mmol) were added. The mixture was cooled to 0°C followed by the addition of benzoyl chloride (0.20 mL, 1.68 mmol). The mixture was stirred for 75 min at 0°C. Aqueous workup (EtOAc, 1 M aq HCl soln; Na₂SO₄) and FC (CH₂Cl₂/MeOH 100:0 to 95:5) gave **255** (930 mg, 58%).

Data of **255:** C₅₀H₅₀N₂O₁₁ (854.9). LC-MS (method 4b): Rₜ = 2.64 (91), 855([M+H]+).

### Synthesis of resin 259

Aminomethyl-PS (0.90 mmol/g; 8.5 g, 7.65 mmol) was swollen in DMF (170 mL) for 30 min and filtered. DMF (85 mL), **255** (6.5 g, 6.89 mmol), *i*-Pr₂NEt (2.6 mL, 15.3 mmol) and HATU (2.91 g, 7.65 mmol) were successively added to the resin and the mixture was shaken for 15 h. The resin was filtered, washed three times with DMF, capped by treatment with acetic anhydride for 30 min (1.4 mL, 15.3 mmol) and *i*-Pr₂NEt (2.6 mL, 15.3 mmol) in DMF (85 mL). The mixture was filtered. The resin was washed three times with DMF, and CH₂Cl₂, twice with MeOH and three times with DMF. The resin was twice treated with 2%(v/v) DBU in DMF (85 mL) for 10 min and filtered. The resin was washed three times with DMF and CH₂Cl₂, twice with MeOH and dried i.v. to afford resin **259** (12.95 g, loading 0.56 mmol/g).

### Synthesis of resin 260

### a) First coupling step

Resin **259** (120 mg, 0.067 mmol) was swollen in DMF (1 mL) for 1 h and filtered. DMF (1 mL), Fmoc-3-aminopropanoic acid (Fmoc-Apa-OH; 42 mg, 0.134 mmol), *i*-Pr₂NEt (0.046 mL, 0.269 mmol) and HATU (51 mg, 0.134 mmol) were added. The mixture was shaken for 1 h and filtered. The resin was washed with DMF. The coupling step was repeated. The mixture was filtered and the resin washed three times each with DMF and CH₂Cl₂.

### b) First allyl ester cleavage

CH₂Cl₂ (1 mL), phenylsilane (0.08 mL, 0.612 mmol) and Pd(PPh₃)₄ (8 mg, 0.007 mmol) were added to the resin. The suspension was shaken for 15 min. The resin was filtered and washed with CH₂Cl₂. The deprotection step was repeated. The resin was filtered and washed three times with CH₂Cl₂ and DMF, twice with MeOH and then three times with DMF.

### c) Second coupling step

DMF (1 mL), N-methylalanine allylester hydrochloride (**257**·HCl; 18 mg, 0.10 mmol), *i*-Pr₂NEt (0.046 mL, 0.269 mmol) and HATU (51 mg, 0.134 mmol) were added. The mixture was shaken for 3 h and filtered. The mixture was filtered and the resin washed three times each with DMF and CH₂Cl₂.

### d) Second allyl ester cleavage was performed applying the conditions described above for the first allyl ester cleavage step.

### e) Fmoc cleavage

The resin was twice treated with 2% (v/v) DBU in DMF (1 mL) for 10 min and filtered. The resin was washed five times with DMF, then twice with MeOH and CH₂Cl₂ to afford resin **260.**

### Synthesis of the macrocycle Ex.476

### 1. Cyclization on solid support

The resin **260** was treated with DMF (1 mL) and FDPP (52 mg, 0.134 mmol) for 15 h. The mixture was washed five times with DMF. DMF (1 mL) and *i*-Pr₂NEt (0.012 mL, 0.067 mmol) were added to the resin and the mixture was shaken for 15 h. The resin was filtered and washed three times each with DMF and CH₂Cl₂, twice with MeOH and three times with CH₂Cl₂.

A soln of 20% (v/v) TFA in CH₂Cl₂ (1 mL) was added to the resin. The mixture was shaken for 10 min and filtered. The resin was washed with CH₂Cl₂. The cleavage step was repeated. The combined filtrates and washings were concentrated and dried i.v. Purification by prep. HPLC method 1 afforded **Ex.476** (5 mg, 16%). Data of **Ex.476:** C₂₆H₃₀N₄O₅ (478.5). LC-MS (method 10a): Rₜ = 1.33 (86), 479 ([M+H]⁺).

¹H-NMR (DMSO-d₆): 8.53 (d, J = 6.4, 1 H), 7.85-7.78 (m, 3 H), 7.56-7.44 (m, 3 H), 7.38 (t, J = 7.8, 1 H), 7.06 (d, J ca. 7.4, 1 H), 6.92 (d, J ca. 7.3, 1 H), 6.48 (br. s, 1 H), 4.61 (m, 1 H), 4.41 (m, 1 H), 4.27 (m, 2 H), ca. 3.8 (m, 2 H, superimposed by H₂O signal), 3.49 (br. d, 1 H), 3.28 (m, 1 H), 3.05 (m, 1 H), 2.99 (s, 3 H), 2.85 (m, 1 H), 2.35-2.26 (m, 3 H), 1.26 (d, J = 6.9, 3 H).

### 2. Cyclization in solution

The resin **260** was treated with 20%(v/v) TFA in CH₂Cl₂ (1 mL) for 10 min and filtered. The resin was washed (CH₂Cl₂) and this cleavage step was repeated.

The combined filtrates and washings were concentrated, dissolved in CH₃CN, again concentrated and dried i.v. Purification by prep. HPLC method 1 afforded **261**·CF₃CO₂H (27 mg, 66%).

Data of **261·**CF₃CO₂H: C₂₆H₃₂N₄O₆·CF₃CO₂H (496.5, free base). LC-MS (method 13): Rₜ = 0.80 (97), 497 ([M+H]⁺).

FDPP (13 mg, 0.033 mmol) was added to a soln of **261**·CF₃CO₂H (10 mg, 0.016 mmol) in DMF (16 mL). *i*-Pr₂NEt (0.003 mL, 0.016 mmol) was added. The soln was stirred at rt for 15 h and concentrated. Purification of the residue by prep. HPLC method 1 afforded **Ex.476** (7 mg, 88%).

LC-MS (method 10a): Rₜ = 1.33 (96), 479 ([M+H]⁺).

### General Procedures for the synthesis of final products on solid support

Procedure **R,** exemplified below for **Core 03,** is generally applicable to macrocyclic core structures with an exocyclic carboxyl and amino group.

Procedure **S,** exemplified below for **Core 11a** and **Core 27,** is generally applicable to macrocyclic core structures with two exocyclic amino groups.

### Procedure R: Derivatization of a carboxyl group and an amino group

### Core 03 (Scheme 38)

### Synthesis of the N-protected amino acid Ex.591

A soln of **Ex.4** (5.59 g, 10.2 mmol) in dioxane (23 mL) was treated with 4M HCl-dioxane (45 mL) for 3 h. Et₂O was added. The mixture was filtered. The solid was washed (Et₂O) and dried i.v. to afford **Ex.590**·HCl (5.46 g; crude, used without further purification).

A soln of **Ex.590**·HCl (5.46 g) in dioxane (130 mL) and H₂O (110 mL) was treated with sat. aq. Na₂CO₃ soln (14.8 mL). Allyl chloroformate (1.18 mL, 11.1 mmol) was added. The mixture was stirred for 1 h, concentrated to ca. 70% of the original volume and acidified with cold 0.5 M HCl soln (200 mL). The mixture was extracted with EtOAc. The organic layer was dried (Na₂SO₄), filtered and concentrated to afford **Ex.591** (5.72 g, quant. yield, contains ca. 5% of dioxane).

Data of **Ex.591:** C₂₅H₃₁FN₄O₈ (534.5). LC-MS (method 4b): Rₜ = 1.58 (91), 535 ([M+H]⁺).

### General procedure for the synthesis of the resins 262

DFPE polystyrene (1% DVB, 100-200 mesh, loading 0.89 mmol/g; 10 g, 8.9 mmol) was swollen in DCE (100 mL) for 1 h. The resin was filtered. DCE (60 mL), a soln of the amine (5-10 equiv.) in DCE (40 mL) and finally trimethyl orthoformate (50 mL) were added. The resin was shaken for 0.5 h at rt, followed by the addition of sodium triacetoxyborohydride (5-10 equiv.). The mixture was shaken for 18 h and the resin was filtered. The resin was successively washed with MeOH, three times each with DCE, DMF, 10% *i*-Pr₂NEt in DMF, DMF, CH₂Cl₂, MeOH and dried i.v. to afford resin **262:**
**262a** (10.8 g, 0.7 mmol/g) was obtained from DFPE polystyrene (1% DVB, 100-200 mesh, loading 0.87 mmol/g; 10 g, 8.7 mmol), N,N-dimethylethylenediamine (3.8 g, 43.5 mmol) and NaBH(OAc)₃ (9.2 g, 43.5 mmol).
**262b** (10.94 g, 0.61 mmol/g) was obtained from DFPE polystyrene (1% DVB, 100-200 mesh, loading 0.87 mmol/g; 10 g, 8.7 mmol), 3-pyridinemethylamine (9.4 g, 87 mmol) and NaBH(OAc)₃ (18 g, 87 mmol).
**262c** (21.1 g, 0.83 mmol/g) was obtained from DFPE polystyrene (1% DVB, 100-200 mesh, loading 0.87 mmol/g; 20 g, 17 mmol), isobutylamine (6.4 g, 87 mmol) and NaBH(OAc)₃ (18 g, 87 mmol).
**262d** (11.74 g, 0.77 mmol/g) was obtained from DFPE polystyrene (1% DVB, 100-200 mesh, loading 0.87 mmol/g; 10 g, 8.7 mmol), 2-naphthylmethylamine (6.8 g, 43.5 mmol) and NaBH(OAc)₃ (9.2 g, 43.5 mmol).
**262e** (2.25 g, 0.60 mmol/g) was obtained from DFPE polystyrene (1% DVB, 100-200 mesh, loading 0.89 mmol/g; 2.0 g, 1.78 mmol), 1-naphthylmethylamine (1.4 g, 8.9 mmol) and NaBH(OAc)₃ (1.9 g, 8.9 mmol).

### Synthesis of resins 265

### 1) Immobilization of acid Ex.591

A soln of *i*-Pr₂NEt (6 equiv.) in CH₂Cl₂ (0.15 mL) was added to resin **262** (loading 0.82 mmol/g; 100 mg, 0.082 mmol). A soln of carboxylic acid **Ex.591** (0.67 equiv.) in CH₂Cl₂/DMF (1:1; 0.45 mL) and a soln of HATU (3 equiv.) and HOAt (3 equiv.) in DMF (0.4 mL) were added. The mixture was shaken for 16 h and filtered. The resin was washed three times with DMF and CH₂Cl₂.

### 2) Capping

A soln of *i*-Pr₂NEt (6 equiv.) in CH₂Cl₂ (0.15 mL) was added. Acetic anhydride (6 equiv.) in CH₂Cl₂/DMF (1:1; 0.75 mL) was added. The mixture was shaken for 0.5 h and filtered. The capping step was repeated. The resin was washed three times with DMF and CH₂Cl₂ to give resin **263.**

### 3) Cleavage of the Alloc group

The conditions described for the synthesis of resin **141** (see below) were applied.

### 4) Derivatization

The conditions described for the second derivatization step of resin **141** were applied (see below). In case of an isocyanate/ isocyanate equivalent or a sulfonyl chloride the derivatization step was repeated once.

### 5) Release of the final products:

The final products of general structure **266** were cleaved from solid support and purified as described for the products of **Core 11a** (see below).

Exception: Resins **265a-b** were treated with 20% TFA in CH₂Cl₂ for 3 x 30 min.

### Procedure S: Derivatization of two amino groups

### Core 11a (Scheme 39)

### Synthesis of amine Ex.594

A soln of **Ex.181** (2.0 g, 3.2 mmol) in MeOH (200 mL) was hydrogenolyzed for 3 h at rt under normal pressure in the presence of palladium hydroxide on activated charcoal (15-20% Pd, moistened with 50% H₂O; 400 mg). The mixture was filtered through a pad of celite. The residue was washed (MeOH). The combined filtrate and washings were concentrated and dried i.v. to give the corresponding amine (1.57 g), which was dissolved in CH₂Cl₂ (8 mL) and treated with sat. aq. NaHCO₃ soln (2.9 mL) and allyl chloroformate (0.36 mL, 3.43 mmol). The mixture was stirred at rt for 2 h. The organic phase was separated and concentrated. Purification of the residue by FC (EtOAc) afforded allyl carbamate **138** (1.65 g, 92%).

TBAF soln (1 M in THF, 7 mL, 7 mmol) was added at 0°C to a soln of **138** (1.29 g, 2.24 mmol) in THF (53 mL). The soln was stirred at 0°C to rt for 3 h and concentrated. The residue was distributed between CH₂Cl₂ and sat. aq. NaHCO₃ soln. The aqueous phase was separated and extracted with CH₂Cl₂. The combined organic phase was dried (Na₂SO₄), filtered and concentrated. The residue was dissolved in CH₂Cl₂ (10 mL) and treated for 20 min with 25% aq. HCl soln (0.29 mL). The volatiles were evaporated and the residue was dried i.v. to afford **Ex.594**·HCl (1.14 g; contaminated with ca. 15% tetrabutylammonium salt and used without further purification; yield ca. 90%).

Data of **Ex.594**·HCl: C₂₂H₃₀N₄O₅·HCl (430.5, free base). LC-MS (method 4a): Rₜ = 1.22 (92), 431.3 [M+H]⁺.

### Synthesis of resin 140

DFPE polystyrene (1% DVB, 100-200 mesh, loading 0.89 mmol/g; 200 mg, 0.178 mmol) was swollen in DCE (2 mL) for 1 h. The resin was filtered. A soln of amine hydrochloride **Ex.594**·HCl (ca 85% w/w, 166 mg, 0.303 mmol) in DCE (1.33 mL) and trimethyl orthoformate (0.66 mL, 6.02 mmol) were added. The resin was shaken for 1 h at rt, followed by addition of sodium triacetoxyborohydride (75 mg, 0.356 mmol). The mixture was shaken for 15 h and the resin was filtered. The resin was successively washed three times each with DMF, 10% *i*-Pr₂NEt in DMF, DMF, CH₂Cl₂ and dried i.v. to afford resin **140** (293 mg).

### Synthesis of resin 141

### 1) First derivatization step

Resin **140** (loading 0.58 mmol/g; 120 mg, 0.07 mmol) was swollen in DMF (1 mL) for 30 min and filtered. The pre-swelling step was repeated. A soln of *i*-Pr₂NEt (8 equiv.) in CH₂Cl₂ (0.15 mL) was added to the resin. A soln of a) the carboxylic acid R^{III}CO₂H (4 equiv.) in CH₂Cl₂/DMF (1:1; 0.45 mL) and a soln of HATU (4 equiv.) in DMF (0.4 mL), or b) the carboxylic acid chloride R^{III}COCl (4 equiv.) in CH₂Cl₂ (0.45 mL) and CH₂Cl₂/DMF (1:1; 0.4 mL), or c) the isocyanate R^{III}NCO or succinimidyl carbamate R^{III}NHCO₂Su (4 equiv.) in CH₂Cl₂/DMF (1:1; 0.85 mL), or d) the sulfonyl chloride R^{III}:SO₂Cl (4 equiv.) in CH₂Cl₂/DMF (1:1; 0.85 mL), or e) the chloroformate R^{III}OCOCl (4 equiv.) in CH₂Cl₂ (0.45 mL) and CH₂Cl₂/DMF (1:1; 0.45 mL) were added. The mixture was shaken for 1 h and filtered. The resin was washed with DMF. The coupling step was repeated. The resin was washed three times with DMF and CH₂Cl₂.

### 2) Cleavage of the Alloc group

Phenylsilane (10 equiv.) in CH₂Cl₂ (0.5 mL), and Pd(PPh₃)₄ (0.2 equiv.) in CH₂Cl₂ (0.5 mL), were added to the resin. The mixture was shaken for 15 min and filtered. The resin was washed with CH₂Cl₂ and the deprotection step was repeated.

The resin was filtered, washed three times each with CH₂Cl₂, DMF and twice with MeOH and three times with CH₂Cl₂.

### 3) Second derivatization step

The carboxylic acids R^{IV}CO₂H, the isocyanates R^{IV}NCO or isocyanate equivalents R^{IV}NCO₂Su and the sulfonyl chlorides R^{IV}SO₂Cl were coupled analogously to the first derivatization step, however with two exceptions: a) For couplings of carboxylic PyBOP was used as coupling reagent and b) all couplings were conducted only once.

The resin was filtered, washed three times each with DMF, twice with MeOH and three times with CH₂Cl₂ to afford resin **141.**

### Release of the final products

The resin **141** was treated twice with 20% TFA in CH₂Cl₂ (1 mL) for 10 min, filtered and washed three times with CH₂Cl₂. The combined filtrates and washings were concentrated. The residue was treated with CH₃CN, evaporated and dried i.v. Purification of the crude product by normal phase or reversed phase prep. HPLC afforded products of general structure **267.**

### Core 27 (Scheme 40)

### Synthesis of amine Ex.593

A soln of **Ex.424** (19.2 g, 32 mmol) in MeOH (385 mL) was hydrogenolyzed for 2 h at rt under normal pressure in the presence of palladium hydroxide on activated charcoal (15-20% Pd, moistened with 50% H₂O; 4.0 g). The mixture was filtered through a pad of celite. The residue was washed (MeOH). The combined filtrate and washings were concentrated and dried i.v. to give the corresponding amine (15.5 g), which was dissolved in CH₂Cl₂ (90 mL) and treated with sat. aq. NaHCO₃ soln (32 mL) and allyl chloroformate (4.40 mL, 41.5 mmol). The mixture was stirred at rt for 2 h. Additional allyl chloroformate (0.9 mL, 8.5 mmol) was added and stirring was continued for 1 h. The mixture was diluted with sat. aq. NaHCO₃ soln and extracted with CH₂Cl₂. The organic phase was separated and concentrated. Purification of the residue by FC (CH₂Cl₂/MeOH 95:5) afforded the allyl carbamate **Ex.592** (15.48 g, 85%).

The material was dissolved in CH₂Cl₂ (73 mL) and treated at 0°C with TFA (73 mL) for 30 min. Stirring was continued at rt for 1 h. The volatiles were evaporated. The residue was treated with sat. aq. Na₂CO₃ soln and repeatedly extracted with CHCl₃. The organic phase was dried (Na₂SO₄), filtered and concentrated. The resulting free amine was dissolved in dioxane (147 mL) and treated with 4M HCl-dioxane (29.5 mL). A precipitate was formed. The volatiles were evaporated. The residue was suspended in CH₂Cl₂ and the volatiles were evaporated to afford **Ex.593**·2HCl (14.49 g, 87%).

Data of **Ex.593**·2HCl: C₂₂H₃₁N₅O₅·2HCl (445.5, free base). LC-MS (method 10a): Rₜ = 0.98 (96), 446 ([M+H]⁺).

### Synthesis of resins 269

Resins **269** was prepared from **Ex.593** by applying the conditions described as for the synthesis of resin **141** from **Ex.594.** The second derivatization step was repeated once if an isocyanate or isocyanate equivalent or sulfonyl chloride was used.

### Release of the final products

The final products of general structure **270** were cleaved from solid support and purified as described for the products of **Core 11a** (see above).

### Synthesis of final products

Advanced macrocyclic intermediates and final products depicted in **Tables 21a-49a** (Scheme 26) were prepared starting from the suitable precursor macrocyclic acid or macrocyclic amine applying the general procedures **(H-Q, R, S)** described above. Deviations from general procedures are indicated in **Tables 21a-49a.**

Analytical data of these intermediates and final products are depicted in **Tables 21b-49b.**

IUPAC names of all examples are listed in **Tables 20** and **21c-49c.**

The generic macrocyclic ring structures (Cores) related to **Tables 20-49** are depicted in **Scheme 41** in the order of their core numbers.

Reagents used in the derivatizations are commercially available with the exception of few N-succinimidyl carbamates which were synthesized from amines, anilines or heteroaryl amines according to the procedure of K. Takeda et al. Tetrahedron Lett. 1983, 24, 4569-4572.

### Detailed description of selected examples

### Core 03: Synthesis of selected advanced intermediates and final products (Scheme 42)

### Synthesis of amide Ex.27

A mixture of **Ex.4** (432 mg, 0.79 mmol), HATU (597 mg, 1.57 mmol) and HOAt (214 mg, 1.57 mmol) was dissolved in DMF (6 mL). *N,N-*Dimethylethylenediamine (173 µL, 1.57 mmol) and *i*-Pr₂NEt (537 µL, 3.14 mmol) were added. The soln was stirred at rt for 15 h and concentrated. The residue was dissolved in CHCl₃ and washed with sat. aq. NaHCO₃ soln and with H₂O. The organic phase was dried (Na₂SO₄), filtered and concentrated. FC (CH₂Cl₂/MeOH/conc. aq. NH₃ soln 100:0:0 to 90:10:0.5) afforded **Ex.27** (405 mg, 83%).
Data of **Ex.27:** cf. **Table 21b**

### Synthesis of amine Ex.28

A soln of **Ex.27** (400 mg, 0.64 mmol) in dioxane (4 mL) was treated at rt with 4M HCl-dioxane (8 mL) for 2 h. The volatiles were evaporated. The residue was dissolved in CH₂Cl₂/MeOH, concentrated and dried i.v. to afford **Ex.28**·HCl (343 mg, 90%).
Data of **Ex.28:** cf. **Table 21b**

### Synthesis of amide Ex.11

A mixture of **Ex.28**·HCl (75 mg, 0.126 mmol), 1*H*-indole-3-acetic acid (44 mg, 0.253 mmol), HATU (96 mg, 0.253 mmol) and HOAt (34 mg, 0.253 mmol) was dissolved in DMF (2 mL). *i*-Pr₂NEt (87 µL, 0.505 mmol) was added. The soln was stirred at rt for 15 h and concentrated. The residue was dissolved in CHCl₃ and washed with sat. aq. NaHCO₃ soln and with H₂O. The organic phase was dried (Na₂SO₄), filtered and concentrated. FC (CH₂Cl₂/MeOH/conc. aq. NH₃ soln 100:0:0 to 90:10:1) afforded **Ex.11** (50 mg, 58%).
Data of **Ex.11:** cf. **Table 21b**
¹H-NMR (DMSO-d₆): 10.81 (s, 1 H), 8.26 (d, J = 7.4, 1 H), 7.62 (t, J = 5.5, 1 H), 7.46 (d, J = 7.9, 1 H), 7.37-7.15 (m, 4 H), 7.09 (d, J = 2.2, 1 H), 7.04 (t, J = 7.5, 1 H), 6. 92 (t, J ca. 7. 4, 1 H), 5.08 (d, J ca. 12. 5, 1 H), 4.74 (d, J = 8. 9, 1 H), 4.37 (d, J = 11.0, 1 H), 4.25 (d, J = 17.7, 1 H), 4.22-4.13 (m, 2 H), 3.97 (d, J = 17.6, 1 H), 3.78 (t, J = 8.3, 1 H), 3.41 (s, 2 H), 3.24 (m, 1 H), 3.15 (m, 1 H), 2.98 (t, J = 9.2, 1 H), 2.88 (s, 3 H), 2.53 (s, 3 H), 2.41-2.27 (m, 4 H), 2.17 (s, 6 H), 2.04 (m, 1 H), 1.83 (t-like m, 2 H), 1.69 (q-like m, 1 H).

### Synthesis of amide Ex.48

**Ex.48** (58 mg, 83%) was obtained from **Ex.28**·HCl (60 mg, 0.101 mmol) and 2-naphthylacetic acid (23 mg, 0.121 mmol) following the procedure described for the synthesis of **Ex.49** (see below). After purification by FC, the material was submitted to an aqueous extraction (CHCl₃, aq. NaHCO₃ soln).
Data of **Ex.48:** cf. **Table 21b**

### Synthesis of amide Ex.49

A mixture of **Ex.28**-HC1 (60 mg, 0.101 mmol), 1-naphthylacetic acid (23 mg, 0.121 mmol), and HOBt·H₂0 (19 mg, 0.121 mmol) was dissolved in CH₂Cl₂ (1 mL). *N*-Cyclohexyl-carbodiimide-*N'-*methylpolystyrene (1.9 mmol/g; 80 mg, 0.152 mmol) and *i*-Pr₂NEt (52 µL, 0.303 mmol) were added. The mixture was stirred for 15 h at rt. (Polystyrylmethyl)-trimethylammonium bicarbonate (3.5 mmol/g; 87 mg, 0.303 mmol) was added and stirring was continued for 1 h. The mixture was diluted with CH₂Cl_{2/}MeOH 9:1 (2 mL) and filtered. The polymer was washed with twice with CH₂Cl₂/MeOH 8:2 (5 mL). The combined filtrate and washings were concentrated. Purification of the crude product by FC (CH₂Cl₂/MeOH/conc. aq. NH₃ soln 100:0:0 to 90:10:1) afforded **Ex.49** (58 mg, 83%).
Data of **Ex.49:** cf. **Table 21b**
¹H-NMR (DMSO-d₆): 8.45 (d, J = 7.3, 1 H), 8.00-7.87 (m, 2 H), 7.79 (d, J = 8.0, 1 H), 7.62 (t, J = 5.5, 1 H), 7.53-7.25 (m, 6 H), 7.19 (dd, J = 3.0, 8.4, 1 H), 5.10 (d, J = 12.3, 1 H), 4.75 (d, J = 8.9, 1 H), 4. 39 (d, J = 10.8, 1 H), 4.27 (d, J = 17.8, 1 H), 4.28-4.08 (m, 2 H), 3.95 (d, J = 17.9, 1 H), 3.83 (m, 1 H), 3.81 (s, 2 H), 3.24 (m, 1 H), 3.16 (m, 1 H), 3.03 (t, J = 9.2, 1 H), 2.87 (s, 3 H), 2.54 (s, 3 H), 2.42-2.27 (m, 4 H), 2.16 (s, 6 H), 2.02 (m, 1 H), 1.84 (t-like m, 2 H), 1.71 (q, J ca. 9.4, 1 H).

### Synthesis of amine Ex.486

A mixture of activated molecular sieves (powder, 4Å; 120 mg) and **Ex.28** (free base obtained from acid **Ex.591** (Scheme 38) by coupling of *N,N*-dimethylethylenediamine according to Procedure **L.2** and subsequent cleavage of the Alloc group with Pd(PPh₃)₄ and morpholine in THF; 60 mg, 0.115 mmol) was suspended in THF (1 mL). 2-(Naphthalen-1-yl)acetaldehyde (20 mg, 0.115 mmol) in THF (0.25 mL) was added. The mixture was stirred at rt for 4 h. A soln of acetic acid (0.0073 mL, 0.127 mmol) in THF (0.25 mL) was added, followed by NaBH(OAc)₃ (49 mg, 0.231 mmol) and stirring was continued for 15 h. Aqueous workup (CHCl₃, sat. aq. NaHCO₃ soln; Na₂SO₄) and purification by FC (CH₂Cl₂/MeOH/conc. aq. NH₃ soln 98:2:2 to 90:10:2) and by prep. HPLC method 1 afforded **Ex.486**·CF₃CO₂H (6 mg; 6%).
Data of **Ex.486**·CF3CO₂H: cf. **Table 21b**

### Synthesis of amide Ex.30

A mixture of **Ex.4** (400 mg, 0.73 mmol), HATU (552 mg, 1.45 mmol), HOAt (198 mg, 1.45 mmol) and tryptamine (233 mg, 1.45 mmol) was dissolved in DMF (6 mL). *i*-Pr₂NEt (497 µL, 2.91 mmol) was added. The soln was stirred at rt for 15 h followed by aqueous workup (CHCl₃, sat. aq. NaHCO₃ soln, H₂O). The organic phase was dried (Na₂SO₄), filtered and concentrated. FC (CH₂Cl₂/MeOH 100:0 to 95:5) afforded **Ex.30** (410 mg, 81%).
Data of **Ex.30:** cf. **Table 21b**
¹H-NMR (DMSO-d₆): 10.80 (s, 1 H), 7.91 (t, J = 5. 6, 1 H), 7.56 (d, J = 7.7, 1 H), 7.32 (d, J = 8.0, 1 H), 7.27-7.12 (m, 5 H), 7.06 (t, J = 7.5, 1 H), 6.97 (t, J = 7.4, 1 H), 5.08 (d, J = 12.4, 1 H), 4.75 (d, J = 9.3, 1 H), 4.34 (d, J = 10.9, 1 H), 4.24 (d, J = 17.8, 1 H), 4.10 (t-like m, 1 H), 3.97 (d, J = 17.7, 1 H), 3.86 (m, 1 H), 3.77 (m, 1 H), 3.42-3.30 (m, 2 H), 2.96-2.83 (m, 3 H), 2.89 (s, 3 H), 2.50 (s, 3 H, superimposed by DMSO-d signal), 2.27 (m, 2 H), 2.08 (m, 1 H), 1.84 (t-like m, 2 H), 1.65 (q, J = 10.8, 1 H), 1.34 (s, 9 H).

### Synthesis of amine Ex.55

A soln of **Ex.30** (380 mg, 0.55 mmol) in dioxane (4 mL) was treated at rt with 4M HCl-dioxane (8 mL) for 4 h. The volatiles were evaporated. The residue was dissolved in dioxane (4 mL) and treated again for 2 h with 4M HCl-dioxane (8 mL). The volatiles were evaporated. The residue was washed with Et₂O and purified by FC (CH₂Cl₂/MeOH/conc. aq. NH₃ soln 90:10:0 to 90:10:1) to afford **Ex.55** (136 mg, 42%).
Data of **Ex.55:** cf. **Table 21b**

### Synthesis of amide Ex.498

A mixture of **Ex.5**·HCl (874 mg, 1.52 mmol), 1-naphthylacetic acid (564 mg, 3.03 mmol), HATU (1.15 g, 3.03 mmol) and HOAt (412 mg, 3.03 mmol) was dissolved in DMF (9 mL). *i*-Pr₂NEt (0.778 mL, 4.54 mmol) was added. The soln was stirred at rt for 2 h and concentrated. The residue was dissolved in CH₂Cl₂ and washed with sat. aq. NaHCO₃ soln and with H₂O. The organic phase was dried (Na₂SO₄), filtered and concentrated. Purification by FC (EtOAc/MeOH gradient) afforded **Ex.498** contaminated with some *i*-Pr₂NEt which was removed by extraction (CH₂Cl₂, 1 M aq. HCl, sat. aq. NaHCO₃ soln) to give **Ex.498** (940 mg, 87%).
Data of **Ex.498:** cf. **Table 21b**

### Synthesis of acid Ex.499

A soln of **Ex.498** (871 mg, 1.23 mmol) in MeOH (9 mL) was hydrogenolyzed under normal pressure at rt in the presence of palladium hydroxide on activated charcoal (moistened with 50% H₂O; 0.14 g) for 2 h. The mixture was filtered through a pad of celite. The filtrate was concentrated to afford **Ex.499** (732 mg; 96%).
Data of **Ex.499:** cf. **Table 21b**

### Synthesis of amide Ex.488

A mixture of **Ex.499** (70 mg, 0.11 mmol), HATU (86 mg, 0.23 mmol) and HOAt (31 mg, 0.23 mmol) was suspended in DMF (1 mL). 3-Dimethylaminopropylamine (0.028 mL, 0.23 mol) and *i*-Pr₂NEt (0.039 mL, 0.23 mmol) were added. The mixture was stirred for 3 h and concentrated. Aqueous workup (CHCl₃, sat. aq. NaHCO₃ soln, H₂O; Na₂SO₄) and purification by prep. HPLC method 2 afforded **Ex.488** (53 mg, 66%).
Data of **Ex.488:** cf. **Table 21b**

### Synthesis of amide Ex.485

A mixture of **Ex.499** (70 mg, 0.11 mmol), HATU (86 mg, 0.23 mmol) and HOAt (31 mg, 0.23 mmol) was suspended in DMF (1 mL). *N*-(2-Aminoethyl)pyridine-2-amine (31 mg, 0.23 mol) and *i*-Pr₂NEt (0.039 mL, 0.23 mmol) were added. The mixture was stirred for 3 h and concentrated. Aqueous workup (CHCl₃, sat. aq. NaHCO₃ soln, H₂O; Na₂SO₄) and purification by prep. HPLC method 2 afforded **Ex.485** (50 mg, 60%).
Data of **Ex.485:** cf. **Table 21b**

### Synthesis of amide Ex.500

A mixture of **Ex.499** (250 mg, 0.40 mmol), HATU (307 mg, 0.81 mmol) and HOAt (110 mg, 0.81 mmol) was suspended in DMF (2.5 mL). *tert*. Butyl-2-aminoethylcarbamate (129 mg, 0.81 mmol) and *i*-Pr₂NEt (0.138 mL, 0.81 mmol) were added. The mixture was stirred for 3 h and concentrated. Aqueous workup (CHCl₃, sat. aq. NaHCO₃ soln, H₂O; Na₂SO₄) and purification by prep. HPLC method 2 afforded **Ex.500** (227 mg, 74%).
Data of **Ex.500:** cf. **Table 21b**

### Synthesis of amine Ex.483

4M HCl-dioxane (2 mL) was added to a soln of **Ex.500** (201 mg, 0.26 mmol) in dioxane (2 mL). The mixture was stirred for 2 h at rt and concentrated to afford **Ex.483**·HCl (200 mg, 97%).
Data of **Ex.483**·HCl: cf. **Table 21b**
¹H-NMR (DMSO-d₆): 8.46 (d, J = 7.1, 1 H), 8.00-7.78 (several m and br. s, 7 H), 7.52-7.21 (several m, 7 H), 5.15 (d, J = 12.3, 1 H), 4.76 (d, J = 8.8, 1 H), 4.40 (d, J = 10.8, 1 H), 4.28 (d, J = 17.7, 1 H), 4.25-4.12 (m, 3 H), 4.03-3.94 (m, 2 H), 3.81 (m, 1 H), 3.81 (s, 2 H), 3.35 (m, 1 H, superimposed by H₂O signal), 3.01 (m, 1 H), 2.90 (m, 1 H), 2.88 (s, 3 H), 2.56 (s, 3 H), 2.42-2.33 (m, 2 H), 2.10 (m, 1 H), 1.91-1.82 (m, 2 H), 1.70 (m, 1 H).

### Synthesis of guanidine Ex.484

Triethylamine (0.066 mL) was added to a suspension of **Ex.483**·HCl (110 mg, 0.158 mmol) in CHCl₃ (1 mL). 1,3-Di-Boc-(trifluoromethylsulfonyl)-guanidine (65 mg, 0.17 mmol) was added. The mixture was stirred for 22 h at rt, followed by aqueous workup (CHCl₃, sat. aq. Na₂CO₃ soln, sat. aq. NaCl soln; Na₂SO₄) and purification by FC (EtOAc/MeOH 100:0 to 80:20) to afford a colorless solid (74 mg) which was dissolved in dioxane (1 mL) and treated with 4M HCl-dioxane (1 mL) for 2 h. More 4M HCl-dioxane (1 mL) was added and stirring was continued for 15 h. The volatiles were evaporated and the residue purified by prep. HPLC (method 1) to afford **Ex.484**·CF₃CO₂H.
Data of **Ex.484**·CF₃CO₂H: cf. **Table 21b**

### Synthesis of amide Ex.487

At 0°C, pyridine (0.081 mL, 1.0 mmol) and acetic anhydride (0.047 mL, 0.5 mmol) were added to a mixture of **Ex.483**·HCl (70 mg, 0.10 mmol) in CH₂Cl₂ (1 mL). The mixture was stirred for 2 h and concentrated. Purification by prep. HPLC (method 1) afforded **Ex.487** (53 mg, 75%).
Data of **Ex.487:** cf. **Table 21b**

### Synthesis of urea Ex.482

**Ex.482**·CF₃CO₂H (11 mg, 25%) was obtained from resin **263a** (0.7 mmol/g; 118 mg, 0.082 mmol) and 1-naphthyl isocyanate (84 mg, 0.498 mmol) according to procedure **R.**

The product was purified by prep. HPLC (method 1).
Data of **Ex.482**·CF₃CO₂H: cf. **Table 21b**

### Synthesis of amide Ex.495

**Ex.495**·CF₃CO₂H (11 mg, 24%) was obtained from resin **263a** (0.7 mmol/g; 118 mg, 0.083 mmol) and 3-(trifluoromethyl)phenylacetic acid (102 mg, 0.498 mmol) according to procedure **R.**

The product was purified by prep. HPLC (method 1).
Data of **Ex.495**·CF₃CO₂H: cf. **Table 21b**

### Synthesis of amide Ex.496

**Ex.496**·CF₃CO₂H (10 mg, 23%) was obtained from resin **263a** (0.7 mmol/g; 118 mg, 0.083 mmol) and 3-methoxyphenylacetic acid (83 mg, 0.489 mmol) according to procedure **R.**

The product was purified by prep. HPLC (method 1).
Data of **Ex.496**·CF₃CO₂H: cf. **Table 21b**

### Synthesis of amide Ex.497

**Ex.497**·CF3CO₂H (8 mg, 18%) was obtained from the resin **263b** (0.61 mmol/g; 130 mg, 0.079 mmol) and 1-naphthylacetic acid (93 mg, 0.498 mmol) according to procedure **R.**

The product was purified by prep. HPLC (method 1).
Data of **Ex.497**·CF₃CO₂H: cf. **Table 21b**

### Synthesis of amide Ex.501

**Ex.501** (18 mg, 44%) was obtained from resin **263e** (0.602 mmol/g; 130 mg, 0.078 mmol) and 2-naphthylacetic acid (86 mg, 0.498 mmol) according to procedure **R.**

The product was purified by prep. HPLC (method 3).
Data of **Ex.501:** cf. **Table 21b**

### Synthesis of amide Ex.502

**Ex.502** (19 mg, 48%) was obtained from resin **263e** (0.602 mmol/g; 130 mg, 0.078 mmol/g) and 3-chlorobenzoic acid (95 mg, 0.498 mmol) according to procedure **R.**

The product was purified by prep. HPLC (method 3).
Data of **Ex.502:** cf. **Table 21b**
¹H-NMR (DMSO-d₆): 8.71 (d, J = 7.1, 1 H), 8.39 (t-like m, 1 H), 8.10 (m, 1 H), 7.95 (m, 1 H), 7.87-7.82 (m, 2 H), 7.75 (d, J ca. 7.8, 1 H), 7.59-7.42 (m, 6 H), 7.29-7.22 (m, 2 H), 6.91 (dd, J = 3.0, 8.4, 1 H), 5.15 (d, J ca. 12.1, 1 H), 4.85-4.70 (m, 2 H), 4.50-4.10 (m, 4 H), 4.03 (d, J = 18.1, 1 H), 3.88 (t, J ca. 8.2, 1 H), 3.08 (t-like m, 1 H), 2.90 (m, 1 H), 2.89 (s, 3 H), 2.57 (s, 3 H), 2.50-2.25 (m, 2 H), 2.10-1.80 (m, 4 H).

### Synthesis of amide Ex.503

**Ex.503** (17 mg, 43%) was obtained from resin **263d** (0.77 mmol/g; 108 mg, 0.083 mmol) and 1-naphthoic acid (85.8 mg, 0.498 mmol) according to procedure **R.**

The product was purified by prep. HPLC (method 3).
Data of **Ex.503:** cf. **Table 21b**

### Synthesis of amide Ex.12

A mixture of **Ex.55** (68 mg, 0.092 mmol), 1*H*-indole-3-acetic acid (32 mg, 0.184 mmol), HATU (70 mg, 0.184 mmol) and HOAt (25 mg, 0.184 mmol) was dissolved in DMF (2 mL). *i*-Pr₂NEt (63 µL, 0.367 mmol) was added. The soln was stirred at rt for 15 h and concentrated. The residue was dissolved in CHCl₃ and washed with sat. aq. NaHCO₃ soln and with H₂O. The organic phase was dried (Na₂SO₄), filtered and concentrated. Purification by prep. HPLC, method 1, afforded **Ex.12** (38 mg, 55%).
Data of **Ex.12:** cf. **Table 21b**
¹H-NMR (DMSO-d₆): 10.81 (s, 2 H), 8.26 (d, J = 7.2, 1 H), 7.93 (t, J = 5.7, 1 H), 7.57 (d, J = 7. 8, 1 H), 7.46 (d, J = 7. 7, 1 H), 7.38-6.90 (m, 11 H), 5.10 (d, J = 12.1, 1 H), 4.76 (d, J = 9.3, 1 H), 4.38 (d, J = 10.8, 1 H), 4.26 (d, J = 17.8, 1 H), 4.23-4.11 (m, 2 H), 3.96 (d, J = 18.0, 1 H), 3.78 (t, J = 8.3, 1 H), 3.7-3.25 (m, 3 H), 3.60 (s, 2 H), 3.01-2.81 (m, 2 H), 2.88 (s, 3 H), ca. 2.5 (s, 3 H, superimposed by DMSO-d signal), 2.33 (m, 2 H), 2.06 (m, 1 H), 1.85 (t-like m, 2 H), 1.63 (q, J ca. 10.7, 1 H).

### Synthesis of amide Ex.16

A mixture of **Ex.55** (68 mg, 0.092 mmol), *N,N*-dimethyl glycine (19 mg, 0.184 mmol), HATU (70 mg, 0.184 mmol) and HOAt (25 mg, 0.184 mmol) was dissolved in DMF (2 mL). *i*-Pr₂NEt (63 µL, 0.367 mmol) was added. The soln was stirred at rt for 15 h and concentrated. The residue was dissolved in CHCl₃ and washed with sat. aq. NaHCO₃ soln and with H₂O. The organic phase was dried (Na₂SO₄), filtered and concentrated. Purification by prep. HPLC, method 1, afforded **Ex.16**·TFA (40 mg, 55%).
Data of **Ex.16**·TFA: cf. **Table 21b**
¹H-NMR (DMSO-d₆): 10.81 (s, 1 H), 9.66 (br. s, NH⁺), 8.75 (d, J = 6.9, 1 H), 7.90 (t, J = 5.6, 1 H), 7.56 (d, J = 7.8, 1 H), 7.34-7.14 (m, 5 H), 7.06 (t, J ca. 7.5, 1 H), 6.97 (t, J = 7.4, 1 H), 5.08 (d, J = 12.3, 1 H), 4.78 (d, J = 9.2, 1 H), 4.39 (d, J = 10.7, 1 H), 4.24 (d, J = 17.8, 1 H), 4.24-4.14 (m, 2 H), 4.00 (d, J = 17.8, 1 H), 3.96-3.75 (m, 3 H), 3.45-3.35 (m, 2 H), 3.0-2.67 (m, 3 H), 2.90 (s, 3 H), 2.75 (s, 6 H), 2.50 (s, 3 H, superimposed by DMSO-d signal), 2.5-2.27 (m, 2 H), 2.08 (m, 1 H), 1.85 (t-like m, 2 H), 1.64 (q, J = 10.8, 1 H).

### Synthesis of amide Ex.53

Pyridine (2 mL) and acetic anhydride (0.14 mL, 1.48 mmol) were added to a soln of **Ex.5**·HCl (95 mg, 0.15 mmol) in dry CH₂Cl₂ (2 mL). The soln was stirred at rt for 20 h. The soln was diluted with EtOAc and washed with 1 M aq. HCl soln, sat. aq. NaCl soln, sat. aq. NaHCO₃ soln, and sat. aq. NaCl soln. The organic phase was dried (Na₂SO₄), filtered and concentrated. FC of the crude product afforded **Ex.53** (60 mg, 70%).
Data of **Ex.53:** cf. **Table 21b**

### Synthesis of acid Ex.54

A soln of **Ex.53** (58 mg, 0.01 mmol) in MeOH (5 mL) was hydrogenolyzed at rt and normal pressure for 2 h in the presence of palladium hydroxide on activated charcoal (moistened with 50% H₂O; 50 mg). The mixture was filtered through a pad of celite. The residue was washed (MeOH). The combined filtrate and washings were concentrated and dried i.v. to yield **Ex.54** (45 mg, 92%).
Data of **Ex.54:** cf. **Table 21b**

### Synthesis of amide Ex.9

A mixture of **Ex.54** (45 mg, 0.091 mmol), HATU (52 mg, 0.137 mmol) HOAt (19 mg, 0.137 mmol) and tryptamine (22 mg, 0.137 mmol) was dissolved in DMF (1 mL). *i*-Pr₂NEt (47 µL, 0.274 mmol) was added. The soln was stirred at rt for 20 h followed by aqueous workup (CHCl₃, sat. aq. NaHCO₃ soln, H₂O). The organic phase was dried (Na₂SO₄), filtered and concentrated. FC (CH₂Cl₂/MeOH 100:0 to 86:14) afforded **Ex.9** (36 mg, 62%).
Data of **Ex.9:** cf. **Table 21b**
¹H-NMR (DMSO-d₆) : 10.81 (s, 1 H), 8.06 (d, J = 7.0, 1 H), 7.93 (t, J = 5.6, 1 H), 7.56 (d, J = 7.8, 1 H), 7.34-7.14 (m, 5 H), 7.05 (t, J ca. 7.5, 1 H), 6.97 (t, J ca. 7.4, 1 H), 5.09 (d, J = 12.4, 1 H), 4.75 (d, J = 9.1, 1 H), 4.38 (d, J = 10.8, 1 H), 4.26 (d, J = 17.7, 1 H), 4.19-4.10 (m, 2 H), 3.97 (d, J = 17.9, 1 H), 3.78 (t, J = 8.3, 1 H), 3.43-3.30 (m, 2 H), 2.96-2.83 (m, 3 H), 2.89 (s, 3 H), 2.50 (s, 3 H, superimposed by DMSO-d signal), 2.40-2.27 (m, 2 H), 2.08 (m, 1 H), 1.85 (m, 2 H), 1.71 (s, 3 H), 1.62 (q, J ca. 10.6, 1 H).

### Core 10: Synthesis of selected advanced intermediates and final products (Scheme 43)

### Synthesis of amide Ex.166

Pyridine (0.48 mL, 5.9 mmol) and valeroyl chloride (0.141 mL, 1.83 mmol) were added to a mixture of **Ex.165** (345 mg, 0.59 mmol) and CH₂Cl₂ (6.0 mL). The mixture was stirred at 0°C for 1 h followed by the addition of MeOH (0.1 mL). Stirring was continued for 10 min. The volatiles were evaporated. Purification by FC (EtOAc/MeOH 100:0 to 95:5) afforded **Ex.166** (220 mg, 67%).
Data of **Ex.166:** cf. **Table 28b**

### Synthesis of amine Ex.167

A soln of **Ex.166** (190 mg, 0.345 mmol) in MeOH/THF (3:1; 16 mL) was hydrogenolyzed under normal pressure at rt for 2 h in the presence of palladium hydroxide on activated charcoal (moistened with 50% H₂O; 95 mg). The mixture was filtered through a pad of celite. The filtrate was concentrated to afford **Ex.167** (152 mg, quant. yield).
Data of **Ex.167:** cf. **Table 28b**

### Synthesis of amide Ex.168

A mixture of **Ex.165** (438 mg, 0.94 mmol), 2-naphthylacetic acid (350 mg, 1.88 mmol), HATU (714 mg, 1.88 mmol) and HOAt (256 mg, 1.88 mmol) was dissolved in DMF (5 mL). *i*-Pr₂NEt (0.65 mL, 3.7 mmol) was added. The soln was stirred at rt for 20 h and concentrated. The residue was dissolved in CHCl₃ and washed with sat. aq. NaHCO₃ soln. The organic phase was dried (Na₂SO₄), filtered and concentrated. FC (EtOAc) afforded **Ex.168** (404 mg, 68%).
Data of **Ex.168:** cf. **Table 28b**
¹H-NMR (DMSO-d₆): 8.45 (d, J = 6.4, 1 H), 7.95-7.83 (m, 3 H), 7.74 (s, 1 H), 7.51-7.41 (m, 4 H), 7.36-7.26 (m, 6 H), 7.03 (dd, J = 1.3, 8.2, 1 H), 6.96 (d, J = 7.5, 1 H), 6.86 (s, 1 H), 4.99 (s, 2 H), 4.55 (d, J = 12.1, 1 H), 4.32 (t-like m, 1 H), 4.24-4.04 (m, 4 H), 3.61 (s, 2 H), ca. 3.2 (m, 1 H), 3.15-2.95 (m, 2 H), 2.89 (s, 3 H), 2.35-2.18 (m, 2 H), 2.06 (m, 1 H), 1.90 (m, 1 H).

### Synthesis of amide Ex.169

A mixture of **Ex.165** (293 mg, 0.63 mmol), 1-pyrrolidineacetic acid (162 mg, 1.25 mmol), HATU (478 mg, 1.25 mmol) and HOAt (171 mg, 1.25 mmol) was dissolved in DMF (5 mL). *i*-Pr₂NEt (0.43 mL, 2.5 mmol) was added. The soln was stirred at rt for 20 h and concentrated. The residue was dissolved in CHCl₃ and washed with sat. aq. NaHCO₃ soln. The organic phase was dried (Na₂SO₄), filtered and concentrated. FC (EtOAc, then EtOAc/MeOH 9:1) afforded **Ex.169** (277 mg, 76%).
Data of **Ex.169:** cf. **Table 28b**

### Synthesis of amide Ex.170

A soln of **Ex.167** (62 mg, 0.15 mmol), 3-(pyridine-4-yl)propanoic acid (27 mg, 0.179 mmol) and HOBt·H₂O (27 mg, 0.179 mmol) in CH₂Cl₂ (1 mL) was treated with N-cyclohexyl-carbodiimide-*N'-*methylpolystyrene (1.9 mmol/g; 118 mg, 0.223 mmol) and *i*-Pr₂NEt (0.076 mL, 0.447 mmol). The mixture was stirred for 15 h at rt. (Polystyrylmethyl)trimethylammonium bicarbonate (3.5 mmol/g; 128 mg, 0.447 mmol) was added and stirring was continued for 1 h. The mixture was diluted with CH₂Cl_{2/}MeOH 9:1 (2 mL) and filtered. The polymer was washed twice with CH₂Cl₂/MeOH 8:2 (5 mL). The combined filtrate and washings were concentrated. Purification by FC (CH₂Cl₂/MeOH 100:0 to 95:5) afforded **Ex.170** (64 mg, 78%) .
Data of **Ex.170:** cf. **Table 28b**

### Synthesis of amide Ex.171

Pyridine (0.12 mL, 1.5 mmol) and 2-naphthoyl chloride (74 mg, 0.38 mmol) were added to a mixture of **Ex.167** (62 mg, 0.15 mmol) and CH₂Cl₂ (1.0 mL) . The mixture was stirred at 0°C for 15 h. More 2-naphthoyl chloride (42 mg, 0.22 mmol) was added. Stirring was continued for 1 h followed by addition of MeOH (0.1 mL). Stirring was continued for 10 min. Aqueous workup (CHCl₃, sat. aq. NaHCO₃ soln; Na₂SO₄) and purification by prep. HPLC (method 3) afforded **Ex.171** (36 mg, 42%).
Data of **Ex.171:** cf. **Table 28b**

### Synthesis of amine Ex.172

A soln of **Ex.168** (380 mg, 0.60 mmol) in MeOH/THF (3:1; 32 mL) was hydrogenolyzed under normal pressure at rt for 2 h in the presence of palladium hydroxide on activated charcoal (moistened with 50% H₂O; 190 mg). The mixture was filtered through a pad of celite. The filtrate was concentrated to afford **Ex.172** (292 mg, 97%).
Data of **Ex.172:** cf. **Table 28b**

### Synthesis of amine Ex.173

A soln of **Ex.169** (257 mg, 0.445 mmol) in MeOH/THF (3:1; 16 mL) was hydrogenolyzed under normal pressure at rt for 2 h in the presence of palladium hydroxide on activated charcoal (moistened with 50% H₂O; 190 mg). The mixture was filtered through a pad of celite. The filtrate was concentrated to afford **Ex.173** (190 mg, 96%).
Data of **Ex.173:** cf. **Table 28b**

### Synthesis of amide Ex.177

A soln of **Ex.172** (65 mg 0.13 mmol), 1-naphthylacetic acid (29 mg, 0.16 mmol) and HOBt·H₂O (24 mg, 0.16 mmol) in CH₂Cl₂ (1 mL) was treated with *N*-cyclohexyl-carbodiimide-*N'*-methylpolystyrene (1.9 mmol/g; 103 mg, 0.19 mmol) and *i*-Pr₂NEt (0.067 mL, 0.39 mmol). The mixture was stirred for 15 h at rt and filtered. The filtrate was submitted to aqueous workup (CHCl₃, sat. aq. NaHCO₃ soln; Na₂SO₄). Purification by prep. HPLC (method 3) afforded **Ex.177** (59 mg, 68%).
Data of **Ex.177:** cf. **Table 28b**
¹H-NMR (DMSO-d₆) 8.41 (d, J = 6.4, 1 H), 8.34 (d, J = 7.1, 1 H), 8.03 (d, J = 8.2, 1 H), 7.87-7.72 (m, 6 H), 7.49-7.38 (m, 7 H), 7.31 (t, J = 7.8, 1 H), 7.00 (dd, J = 1.5, 8.2, 1 H), 6.94 (d, J = 7.5, 1 H), 6.85 (s, 1 H), 4.53 (t, J = 7.3, 1 H), 4.44 (d, J = 12.8, 1 H), 4.27-3.96 (m, 5 H), 3.96 (d, J = 13.8, 1 H), 3.87 (d, J = 14.9, 1 H), 3.57 (s, 2 H), 3.11 (m, 1 H), 2.94 (m, 1 H), 2.85 (s, 3 H), 2.35-2.20 (m, 2 H), 2.05-1.80 (m, 2 H).

### Synthesis of amide Ex.179

A soln of **Ex.173** (55 mg 0.12 mmol), 1-naphthylacetic acid (28 mg, 0.15 mmol) and HOBt·H₂O (23 mg, 0.15 mmol) in CH₂Cl₂ (1 mL) was treated with *N*-cyclohexyl-carbodiimide-*N'*-methylpolystyrene (1.9 mmol/g; 98 mg, 0.19 mmol) and *i*-Pr₂NEt (0.064 mL, 0.372 mmol). The mixture was stirred for 15 h at rt. The mixture was filtered. The filtrate was submitted to aqueous workup (CHCl₃, sat. aq. NaHCO₃ soln; Na₂SO₄). Purification by prep. HPLC (method 3) afforded **Ex.179** (64 mg, 84%).
Data of **Ex.179:** cf. **Table 28b**

### Core 11a: Synthesis of selected advanced intermediates and final products (Scheme 44)

### Synthesis of amide Ex.184

A mixture of **Ex.182** (500 mg, 1.04 mmol), 2-naphthylacetic acid (232 mg, 1.25 mmol), HATU (791 mg, 2.08 mmol) and HOAt (283 mg, 2.08 mmol) was dissolved in DMF (15 mL). *i*-Pr₂NEt (712 µL, 4.16 mmol) was added. The soln was stirred at rt for 20 h and concentrated. The residue was dissolved in CHCl₃ and washed with sat. aq. NaHCO₃ soln and with H₂O. The organic phase was dried (Na₂SO₄), filtered and concentrated. FC (EtOAc, then CH₂Cl₂/MeOH 95:5) afforded **Ex.184** (637 mg, 94%).
Data of **Ex.184:** cf. **Table 29.1.b**
¹H-NMR (DMSO-d₆) : 8.41 (d, J = 7.0, 1 H), 7.90-7.83 (m, 3 H), 7.77 (s, 1 H), 7.53-7.44 (m, 4 H), 7.32-7.22 (m, 6 H), 7.04 (d, J = 8.4, 1 H), 6.86 (d, J = 7.4, 1 H), 6.81 (s, 1 H), 5.02-4.90 (m, 3 H), 4.19 (t, J ca. 8.6, 1 H), 4.14-3.96 (m, 2 H), 3.83 (t-like m, 2 H), 3.63 (s, 2 H), ca. 3.3 (m, 1 H, superimposed by H₂O signal), 3.05 (m, 1 H), 2.95 (m, 1 H), 2.91 (s, 3 H), 2.27 (m, 1 H), 2.16 (br. q, J ca. 11.3, 1 H), 1.54 (m, 2 H), 1.31 (m, 1 H), 1.15 (m, 1 H).

### Synthesis of amine Ex.194

A soln of **Ex.181** (600 mg, 0.96 mmol) in MeOH (52 mL) was hydrogenolyzed for 4 h at normal pressure and rt in the presence of palladium hydroxide on activated charcoal (moistened with 50% H₂O; 300 mg). The mixture was filtered through a pad of celite. The filtrate was concentrated to afford **Ex.194** (471 mg, 100%).
Data of **Ex.194:** cf. **Table 29.1.b**

### Synthesis of amide Ex.532

At 0°C tert.-butylacetyl chloride (0.195 mL, 1.39 mmol) was slowly added to a mixture of **Ex.194** (620 mg, 1.26 mmol) and pyridine (0.51 mL, 6.32 mmol) in CH₂Cl₂ (6 mL). The mixture was stirred at rt for 2 h, then tert.-butylacetyl chloride (0.04 mL, 0.32 mmol) was added and stirring was continued for 1 h. Aqueous workup (CH₂Cl₂, sat. aq. Na₂CO₃ soln; Na₂SO₄) and FC (hexane/EtOAc/MeOH gradient) afforded **Ex.532** (679 mg, 91%).
Data of **Ex.532:** cf. **Table 29.1.b**

### Synthesis of amine Ex.533

At 0°C TFA (3.2 mL) was added to a soln of **Ex.532** (656 mg, 1.11 mmol) in CH₂Cl₂ (3.2 mL). The soln was stirred at 0°C to rt for 1 h. Aqueous workup (CH₃Cl/*i*-PrOH 9:1, aq. Na₂CO₃ soln; Na₂SO₄) afforded **Ex.533** (395 mg, 80%).
Data of **Ex.533:** cf. **Table 29.1.b**

### Synthesis of urea Ex.552

A soln of **Ex.533** (90 mg, 0.20 mmol) in THF (1 mL) was treated with 2,5-dioxopyrrolidin-1-yl-3-(dimethylamino)phenylcarbamate (62 mg, 0.22 mmol) and *i*-Pr₂NEt (0.069 mL, 0.41 mmol) for 18 h at rt. Aqueous workup (CHCl₃, aq. Na₂CO₃ soln; Na₂SO₄) and prep. HPLC (method 3) afforded **Ex.552** (102 mg, 83%).
Data of **Ex.552:** cf. **Table 29.1.b**

### Synthesis of urea Ex.553

2,5-Dioxopyrrolidin-1-yl-3-(dimethylamino)phenylcarbamate (177 mg, 0.64 mmol) and *i*-Pr₂NEt (0.2 mL, 1.16 mmol) were added to a soln of **Ex.594** (obtained from **Ex.594**·HCl by aqueous extraction (CH₂Cl₂, sat. aq. Na₂CO₃ soln; 250 mg, 0.58 mmol) in THF (2 mL). The mixture was stirred at rt for 18 h. Aqueous workup (CHCl₃, sat. aq. Na₂CO₃ soln; Na₂SO₄) and FC (hexane/EtOAc/MeOH gradient) afforded **Ex.553** (313 mg, 91%).

Data of **Ex.553:** C₃₁H₄₀N₆O₆ (592.7). LC-MS (method 4a): Rₜ = 1.47 (99), 593 ([M+H]⁺).

### Synthesis of amine Ex.554

Pd(PPh₃)₄ (5.6 mg) and 1,3-dimethylbarbituric acid (188 mg, 1.2 mmol) were added to a soln of **Ex.553** (285 mg, 0.48 mmol) in degassed CH₂Cl₂/EtOAc (45:55; 5.1 mL). The mixture was stirred at rt for 2 h. More Pd(PPh₃)₄ (5.6 mg) was added and stirring continued for 1 h. The volatiles were evaporated. FC (CH₂Cl₂/MeOH 90:10 to 50:50) afforded **Ex.554** (234 mg, 93%).

Data of **Ex.554:** C₂₇H₃₆N₆O₄ (508.6) : LC-MS (method 4a): Rₜ = 1.20 (96), 509 ([M+H]⁺).

### Synthesis of amide Ex.508

A mixture of **Ex.554** (120 mg, 0.236 mmol), 2-naphthylacetic acid (88 mg, 0.47 mmol), HATU (179 mg, 0.47 mmol) and HOAt (64 mg, 0.472 mmol) in DMF (1.8 mL) was treated with *i*-Pr₂NEt (0.162 mmol, 0.94 mmol) and stirred at rt for 16 h. The mixture was concentrated. Aqueous workup (EtOAc, sat. aq. NaHCO₃ soln; Na₂SO₄) and FC (hexane/EtOAc/MeOH gradient) gave the product contaminated with a salt of *i*-Pr₂NEt. The material was again extracted (CH₂Cl₂, sat. aq. Na₂CO₃ soln; Na₂SO₄) and purified by FC (CH₂Cl₂/MeOH 100:0 to 95:5) to afford **Ex.508** (89 mg, 57%).
Data of **Ex.508:** cf. **Table 29.1.b**
¹H-NMR (DMSO-d₆): 8.48 (d, J = 8.1, 1 H), 8.29 (s, 1 H), 7.83-7.73 (m, 3 H), 7.69 (s, 1 H), 7.45-7.29 (m, 4 H), 7.07-7.01 (m, 2 H), 6.90-6.84 (m, 3 H), 6.69 (d, J ca. 8.5, 1 H), 6.39 (d, J = 7.5, 1 H), 6.34 (dd, J = 2.2, 8.2, 1 H), 4.96 (d, J = 12.8, 1 H), 4.23 (t, J = 8.0, 1 H), 4.10-3.96 (m, 3 H), 3.71 (t-like m, 1 H), 3.61 (s, 2 H), 3.10 (m, 1 H), 2.90 (m, 1 H), 2.90 (s, 3 H), 2.86 (s, 6 H), 2.42-2.27 (m, 2 H), 2.03 (m, 1 H), 1.67-1.46 (m, 2 H), 1.37 (m, 1 H), 1.17 (m, 1 H).

### Synthesis of amide Ex.186

**Ex.186** (11 mg, 41%) was obtained by treatment of resin **140** (0.77 mmol/g; 50 mg, 0.038 mmol) with 2-naphthylacetic acid (65 mg, 0.35 mmol; first coupling step) and with 2-naphthylacetic acid (70 mg, 0.375 mmol; second coupling step) according to procedure **S.**
Data of **Ex.186:** cf. **Table 29.1.b**
¹H-NMR (DMSO-d₆): 8.38 (d, J = 7.0, 2 H), 7.91-7.69 (m, 8 H), 7.54-7.27 (m, 7 H), 7.03 (dd, J = 1.5, 8.2, 1 H), 6.86-6.82 (m, 2 H), 4.94 (d, J = 12.7, 1 H), 4.19 (t, J = 8.6, 1 H), 4.11-3.94 (m, 3 H), 3.71 (dd, J = 9.2, 16.5, 1 H), 3.62 (s, 2 H), 3.58 (s, 2 H), 3.08 (m, 1 H), 2.89 (m, 1 H), 2.89 (s, 3 H), 2.5 (m, 1 H, superimposed by DMSO-d signal), 2.30 (m, 1 H), 2.14 (q-like m, 1H), 1.64-1.49 (m, 2 H), 1.34 (m, 1H), 1.14 (m, 1 H).

The ¹H-NMR spectrum is identical with the spectrum of the sample prepared in soln, cf. **Table 29.1.**

### Synthesis of amide Ex.504

**Ex.504** (10 mg, 26%) was obtained by treatment of resin **140** (0.48 mmol/g; 120 mg, 0.058 mmol) with 2-naphthylacetic acid (108 mg, 0.576 mmol; first coupling step) and with decanoic acid (50 mg, 0.288 mmol; second coupling step) according to procedure **S.**

The product was purified by prep. HPLC (method 1).
Data of **Ex.504:** cf. **Table 29.1.b**

### Synthesis of amide Ex.505

**Ex.505** (7 mg, 33%) was obtained by treatment of resin **140** (0.61 mmol/g; 50 mg, 0.031 mmol) with 2-naphthylacetic acid (114 mg, 0.61 mmol; first coupling step) and with 1-naphthylacetic acid (28 mg, 0.15 mmol; second coupling step) according to procedure **S.**

The product was purified by prep. HPLC (method 3).
Data of **Ex.505:** cf. **Table 29.1.b**

### Synthesis of amide Ex.506

**Ex.506** (10 mg, 45%) was obtained by treatment of resin **140** (0.67 mmol/g; 50 mg, 0.034 mmol) with 2-naphthylacetic acid (63 mg, 0.34 mmol; first coupling step) and with 1-isocyanato-3-methylbenzene (14 mg, 0.10 mmol; second coupling step; THF/DMF 1:1 was used instead of CH₂Cl₂/DMF) according to procedure **S.**

The product was purified by prep. HPLC (method 3).
Data of **Ex.506:** cf. **Table 29.1.b**

### Synthesis of amide Ex.507

**Ex.507** (11 mg, 48%) was obtained by treatment of resin **140** (0.67 mmol/g; 50 mg, 0.034 mmol) with 2-naphthylacetic acid (63 mg, 0.34 mmol; first coupling step) and with 2,5-dioxopyrrolidin-1-yl-3-(dimethylamino)phenylcarbamate (28 mg, 0.10 mmol; second coupling step; THF/DMF 1:1 was used instead of CH₂Cl₂/DMF) according to procedure **S.**

The product was purified by prep. HPLC (method 3).
Data of **Ex.507:** cf. **Table 29.1.b**

### Synthesis of amide Ex.509

**Ex.509** (20 mg, 48%) was obtained by treatment of resin **140** (0.66 mmol/g; 100 mg, 0.066 mmol) with 3-phenylpropanoic acid (80 mg, 0.53 mmol; first coupling step) and with decanoic acid (46 mg, 0.26 mmol; second coupling step) according to procedure **S.**

The product was purified by prep. HPLC (method 3).
Data of **Ex.509:** cf. **Table 29.1.b**

### Synthesis of amide Ex.510

**Ex.510** (11 mg, 20%) was obtained by treatment of resin **140** (0.66 mmol/g; 100 mg, 0.066 mmol) with 3-morpholinobenzoic acid (110 mg, 0.528 mmol; first coupling step) and with decanoic acid (46 mg, 0.26 mmol; second coupling step) according to procedure **S.**

The product was purified by prep. HPLC (method 3).
Data of **Ex.510:** cf. **Table 29.1.b**

### Synthesis of amide Ex.511

**Ex.511**·CF₃CO₂H (26 mg, 55%) was obtained by treatment of resin **140** (0.61 mmol/g; 100 mg, 0.061 mmol) with 2,5-dioxopyrrolidin-1-yl-3-(dimethylamino)phenylcarbamate (135 mg, 0.49 mmol; first coupling step) and with decanoic acid (42 mg, 0.24 mmol; second coupling step) according to procedure **S.**

The product was purified by prep. HPLC (method 1).
Data of E**x.511**·CF₃CO₂H: cf. **Table 29.1.b** ¹H-NMR (DMSO-d₆): 8.37 (br. s, 1 H), 8.10 (d, J = 8.3, 1 H), 7.34 (t, J ca. 7.8, 1 H), 7.12-7.03 (m, 3 H), 6.89-6.79 (m, 3 H), 6.55-6.47 (m, 2 H), 4.97 (d, J = 12.5, 1 H), 4.22 (t, J = 8.3, 1 H), 4.08-3.91 (m, 3 H), 3.70 (dd, J = 7.5, 9.7, 1 H), 3.16 (m, 1 H), 2.96 (m, 1 H), 2.92 (s, 9 H), 2.45-2.31 (m, 3 H), 2.04 (t-like m, 3 H), 1.64-1.41 (m, 5 H), 1.19 (br. s, 12 H), 0.83 (t, J = 6.7, 3 H).

### Synthesis of amide Ex.512

**Ex.512**·CF₃CO₂H (27 mg, 56%) was obtained by treatment of resin **140** (0.61 mmol/g; 100 mg, 0.061 mmol) with 2,5-dioxopyrrolidin-1-yl-3-(dimethylamino)phenylcarbamate (135.mg, 0.49 mmol; first coupling step) and with 1-naphthylacetic acid (45 mg, 0.24 mmol; second coupling step) according to procedure **S.**

The product was purified by prep. HPLC (method 1).
Data of **Ex.512**·CF₃CO₂H: cf. **Table 29.1.b**

### Synthesis of amide Ex.513

**Ex.513** (10 mg, 25%) was obtained by treatment of resin **140** (0.61 mmol/g; 100 mg, 0.061 mmol) with 1-isocyanato-3-methoxy-benzene (73 mg, 0.49 mmol; first coupling step) and with 2-naphthylacetic acid (45 mg, 0.24 mmol; second coupling step) according to procedure **S.**

The product was purified by prep. HPLC (method 3).
Data of **Ex.513:** cf. **Table 29.1.b**

### Synthesis of amide Ex.514

**Ex.514** (22 mg, 54%) was obtained by treatment of resin **140** (0.61 mmol/g; 100 mg, 0.061 mmol) with 2-(3-methoxyphenyl)-acetic acid (81 mg, 0.49 mmol; first coupling step) and with 2-naphthylacetic acid (45 mg, 0.24 mmol; second coupling step) according to procedure **S.**

The product was purified by prep. HPLC (method 3).
Data of **Ex.514:** cf. **Table 29.1.b**

### Synthesis of amide Ex.515

**Ex.515** (21 mg, 48%) was obtained by treatment of resin **140** (0.61 mmol/g; 100 mg, 0.061 mmol) with 2-(3-phenoxyphenyl)-acetic acid (111 mg, 0.49 mmol; first coupling step) and with decanoic acid (42 mg, 0.24 mmol; second coupling step) according to procedure **S.**

The product was purified by prep. HPLC (method 3).
Data of **Ex.515:** cf. **Table 29.1.b**

### Synthesis of amide Ex.516

**Ex.516** (27 mg, 64%) was obtained by treatment of resin **140** (0.61 mmol/g; 100 mg, 0.061 mmol) with 2-naphthylacetic acid (91 mg, 0.49 mmol; first coupling step) and with 6-phenylhexanoic acid (46 mg, 0.24 mmol; second coupling step) according to procedure **S.**

The product was purified by prep. HPLC (method 3).
Data of **Ex.516:** cf. **Table 29.1.b**

### Synthesis of amide Ex.517

**Ex.517** (24 mg, 57%) was obtained by treatment of resin **140** (0.61 mmol/g; 100 mg, 0.061 mmol) with 2-naphthylacetic acid (91 mg, 0.49 mmol; first coupling step) and with 5-phenoxypentanoic acid (48 mg, 0.24 mmol; second coupling step) according to procedure **S.**

The product was purified by prep. HPLC (method 3).
Data of **Ex.517:** cf. **Table 29.1.b**

### Synthesis of amide Ex.518

**Ex.518** (21 mg, 52%) was obtained by treatment of resin **140** (0.61 mmol/g; 100 mg, 0.061 mmol) with 2-naphthylacetic acid (91 mg, 0.48 mmol; first coupling step) and with (E)-dec-3-enoic acid (42 mg, 0.24 mmol; second coupling step) according to procedure **S.**

The product was purified by prep. HPLC (method 3).
Data of **Ex.518:** cf. **Table 29.1.b**

### Synthesis of amide Ex.519

**Ex.519** (24 mg, 59%) was obtained by treatment of resin **140** (0.61 mmol/g; 100 mg, 0.061 mmol) with 1-naphthylacetic acid (91 mg, 0.49 mmol; first coupling step) and with decanoic acid (42 mg, 0.24 mmol; second coupling step) according to procedure **S.**

The product was purified by prep. HPLC (method 3).
Data of **Ex.519:** cf. **Table 29.1.b**

### Synthesis of amide Ex.520

**Ex.520** (22 mg, 59%) was obtained by treatment of resin **140** (0.61 mmol/g; 100 mg, 0.061 mmol) with (Z)-hex-3-enoic acid (56 mg, 0.49 mmol; first coupling step) and with 1-naphthylacetic acid (45 mg, 0.24 mmol; second coupling step) according to procedure **S.**

The product was purified by prep. HPLC (method 3).
Data of **Ex.520:** cf. **Table 29.1.b**

### Synthesis of amide Ex.521

**Ex.521** (30 mg, 68%) was obtained by treatment of resin **140** (0.61 mmol/g; 100 mg, 0.061 mmol) with 2-naphthylacetic acid (91 mg, 0.49 mmol; first coupling step) and with 6-(benzyloxy)hexanoic acid (54 mg, 0.24 mmol; second coupling step) according to procedure **S.**

The product was purified by prep. HPLC (method 3).
Data of **Ex.521:** cf. **Table 29.1.b**

### Synthesis of amide Ex.522

**Ex.522** (27 mg, 62%) was obtained by treatment of resin **140** (0.61 mmol/g; 100 mg, 0.061 mmol) with 2-naphthylacetic acid (91 mg, 0.49 mmol; first coupling step) and with 2-(biphenyl-4-yl)acetic acid (52 mg, 0.24 mmol; second coupling step) according to procedure **S.**

The product was purified by prep. HPLC (method 3).
Data of **Ex.522:** cf. **Table 29.1.b**

### Synthesis of amide Ex.523

**Ex.523** (28 mg, 63%) was obtained by treatment of resin **140** (0.61 mmol/g; 100 mg, 0.061 mmol) with 2-naphthylacetic acid (91 mg, 0.49 mmol; first coupling step) and with 3,3-diphenylpropanoic acid (55 mg, 0.24 mmol; second coupling step) according to procedure **S.**

The product was purified by prep. HPLC (method 3).
Data of **Ex.523:** cf. **Table 29.1.b**

### Synthesis of amide Ex.524

**Ex.524** (17 mg, 38%) was obtained by treatment of resin **140** (0.61 mmol/g; 100 mg, 0.061 mmol) with 6-(benzyloxy)hexanoic acid (108 mg, 0.49 mmol; first coupling step) and with 6-phenylhexanoic acid (94 mg, 0.48 mmol; second coupling step repeated twice) according to procedure **S.**

The product was purified by prep. HPLC (method 3).
Data of **Ex.524:** cf. **Table 29.1.b**

### Synthesis of amide Ex.525

**Ex.525** (27 mg, 54%) was obtained by treatment of resin **140** (0.61 mmol/g; 100 mg, 0.061 mmol) with 2,5-dioxopyrrolidin-1-yl-3-(dimethylamino)phenylcarbamate (135 mg, 0.49 mmol; first coupling step) and with 2-(biphenyl-3-yl)acetic acid (52 mg, 0.24 mmol; second coupling step) according to procedure **S.**

The product was purified by prep. HPLC (method 3).
Data of **Ex.525:** cf. **Table 29.1.b**
¹H-NMR (DMSO-d₆): 8.39 (d, J = 7.9, 1 H), 8.25 (br. s, 1 H), 7.61 (d, J = 7.0, 2 H), 7.53 (s, 1 H), 7.48-7.42 (m, 3 H), 7.38-7.30 (m, 3 H), 7.22 (d, J = 7.6, 1 H), 7.06-7.02 (m, 2 H), 6.93 (br. s, 1 H), 6.87-6.83 (m, 2 H), 6.70 (d, J = 7.7, 1 H), 6.39-6.37 (m, 2 H), 4.96 (d, J = 12.7, 1 H), 4.23 (t, J = 8.4, 1 H), 4.09-3.96 (m, 3 H), 3.73 (t-like m, 1 H), 3.52 (s, 2 H), 3.07 (m, 1 H), 2.93 (m, 1 H), 2.89 (s, 3 H), 2.86 (s, 6 H), 2.50-2.27 (m, 2 H), 2.01 (m, 1 H), 1.64-1.52 (m, 2 H), 1.35 (m, 1 H), 1.14 (m, 1 H).

### Synthesis of amide Ex.526

**Ex.526** (27 mg, 54%) was obtained by treatment of resin **140** (0.61 mmol/g; 100 mg, 0.061 mmol) with 2,5-dioxopyrrolidin-1-yl-3-(dimethylamino)phenylcarbamate (135 mg, 0.49 mmol; first coupling step) and with 2-(biphenyl-4-yl)acetic acid (52 mg, 0.24 mmol; second coupling step) according to procedure **S.**

The product was purified by prep. HPLC (method 3).
Data of **Ex.526:** cf. **Table 29.1.b**

### Synthesis of amide Ex.527

**Ex.527** (27 mg, 61%) was obtained by treatment of resin **140** (0.61 mmol/g; 100 mg, 0.061 mmol) with 2-naphthylacetic acid (91 mg, 0.48 mmol; first coupling step) and with biphenyl-4-sulfonyl chloride (62 mg, 0.24 mmol; second coupling step) according to procedure **S.**

The product was purified by prep. HPLC (method 3).
Data of **Ex.527:** cf. **Table 29.1.b**

### Synthesis of amide Ex.537

**Ex.537** (25 mg, 63%) was obtained by treatment of resin **140** (0.61 mmol/g; 100 mg, 0.061 mmol) with 1-fluoro-3-isocyanatobenzene (67 mg, 0.49 mmol; first coupling step) and with 2-naphthylacetic acid (45 mg, 0.24 mmol; second coupling step) according to procedure **S.**

The product was purified by prep. HPLC (method 3).
Data of **Ex.537:** cf. **Table 29.1.b**

### Synthesis of amide Ex.538

**Ex.538**·CF₃CO₂H (30 mg, 62%) was obtained by treatment of resin **140** (0.61 mmol/g; 100 mg, 0.061 mmol) with 2,5-dioxopyrrolidin-1-yl 4-(dimethylamino)phenylcarbamate (79 mg, 0.49 mmol; first coupling step) and with 2-naphthylacetic acid (45 mg, 0.24 mmol; second coupling step) according to procedure **S.**

The product was purified by prep. HPLC (method 1).
Data of **Ex.538**·CF₃CO₂H: cf. **Table 29.1.b**

### Synthesis of amide Ex.539

**Ex.539** (18 mg, 46%) was obtained by treatment of resin **140** (0.61 mmol/g; 100 mg, 0.061 mmol) with phenylchloroformate (76 mg, 0.49 mmol; first coupling step) and with 2-naphthylacetic acid (45 mg, 0.24 mmol; second coupling step) according to procedure **S.**

The product was purified by prep. HPLC (method 3).
Data of **Ex.539:** cf. **Table 29.1.b**

### Synthesis of amide Ex.540

**Ex.540** (26 mg, 53%) was obtained by treatment of resin **140** (0.61 mmol/g; 100 mg, 0.061 mmol) with 2-(quinolin-7-yl)acetic acid (92 mg, 0.49 mmol; first coupling step) and with 2-naphthylacetic acid (45 mg, 0.24 mmol; second coupling step) according to procedure **S.**

The product was purified by prep. HPLC (method 3).
Data of **Ex.540:** cf. **Table 29.1.b**

### Synthesis of amide Ex.541

**Ex.541** (30 mg, 68%) was obtained by treatment of resin **140** (0.61 mmol/g; 100 mg, 0.061 mmol) with 2-(3-phenoxyphenyl)acetic acid (111 mg, 0.49 mmol; first coupling step) and with 2-naphthylacetic acid (45 mg, 0.24 mmol; second coupling step) according to procedure **S.**

The product was purified by prep. HPLC (method 3).
Data of **Ex.541:** cf. **Table 29.1.b**

### Synthesis of amide Ex.542

**Ex.542** (23 mg, 56%) was obtained by treatment of resin **140** (0.61 mmol/g; 100 mg, 0.061 mmol) with decanoic acid (84 mg, 0.49 mmol; first coupling step) and with 2-naphthylacetic acid (45 mg, 0.24 mmol; second coupling step) according to procedure **S.**

The product was purified by prep. HPLC (method 3).
Data of **Ex.542:** cf. **Table 29.1.b**

### Synthesis of amide Ex.543

**Ex.543** (25 mg, 61%) was obtained by treatment of resin **140** (0.61 mmol/g; 100 mg, 0.061 mmol) with decanoic acid (84 mg, 0.49 mmol; first coupling step) and with 1-naphthylacetic acid (45 mg, 0.24 mmol; second coupling step) according to procedure **S.**

The product was purified by prep. HPLC (method 3).
Data of **Ex.543:** cf. **Table 29.1.b**

### Synthesis of amide Ex.544

**Ex.544** (20 mg, 54%) was obtained by treatment of resin **140** (0.61 mmol/g; 100 mg, 0.061 mmol) with hexanoic acid (57 mg, 0.49 mmol; first coupling step) and with 1-naphthylacetic acid (45 mg, 0.24 mmol; second coupling step) according to procedure **S.**

The product was purified by prep. HPLC (method 3).
Data of **Ex.544:** cf. **Table 29.1.b**

### Synthesis of amide Ex.545

**Ex.545** (28 mg, 65%) was obtained by treatment of resin **140** (0.61 mmol/g; 100 mg, 0.061 mmol) with 2-(biphenyl-4-yl)acetic acid (104 mg, 0.49 mmol; first coupling step) and with 1-naphthylacetic acid (45 mg, 0.24 mmol; second coupling step) according to procedure **S.**

The product was purified by prep. HPLC (method 3).
Data of **Ex.545:** cf. **Table 29.1.b**

### Synthesis of amides Ex.546 and Ex.551

**Ex.546** (9 mg, 20%) and **Ex.551** (8 mg, 18%) were obtained by treatment of resin **140** (0.61 mmol/g; 100 mg, 0.061 mmol) with 2,2-diphenylacetic acid (104 mg, 0.49 mmol; first coupling step) and with 2-(biphenyl-4-yl)acetic acid (52 mg, 0.24 mmol; second coupling step) according to procedure **S.**

The products were purified by prep. HPLC (method 1).
Data of **Ex.546:** cf. **Table 29.1.b**
Data of **Ex.551:** cf. **Table 29.1.b**

### Synthesis of amide Ex.547

**Ex.547** (29 mg, 65%) was obtained by treatment of resin **140** (0.61 mmol/g; 100 mg, 0.061 mmol) with 2-(biphenyl-4-yl)acetic acid (104 mg, 0.49 mmol; first coupling step) and with 2,2-diphenylacetic acid (52 mg, 0.24 mmol; second coupling step) according to procedure **S.**

The product was purified by prep. HPLC (method 3).
Data of **Ex.547:** cf. **Table 29.1.b**

### Synthesis of amide Ex.548

**Ex.548** (12 mg, 28%) was obtained by treatment of resin **140** (0.61 mmol/g; 100 mg, 0.061 mmol) with 2,2-diphenylacetic acid (104 mg, 0.49 mmol; first coupling step) and with decanoic acid (42 mg, 0.24 mmol; second coupling step) according to procedure **S.**

The product was purified by prep. HPLC (method 1).
Data of **Ex.548:** cf. **Table 29.1.b**

### Synthesis of amide Ex.549

**Ex.549** (10 mg, 23%) was obtained by treatment of resin **140** (0.61 mmol/g; 100 mg, 0.061 mmol) with 2,2-diphenylacetic acid (104 mg, 0.49 mmol; first coupling step) and with 1-naphthylacetic acid (45 mg, 0.24 mmol; second coupling step) according to procedure **S.**

The product was purified by prep. HPLC (method 1).
Data of **Ex.549:** cf. **Table 29.1.b**

### Synthesis of amide Ex.550

**Ex.550** (21 mg, 49%) was obtained by treatment of resin **140** (0.61 mmol/g; 100 mg, 0.061 mmol) with 3,3-diphenylpropanoic acid (110 mg, 0.49 mmol; first coupling step) and with decanoic acid (42 mg, 0.24 mmol; second coupling step) according to procedure **S.**

The product was purified by prep. HPLC (method 3).
Data of **Ex.550:** cf. **Table 29.1.b**

### Core 12: Synthesis of selected advanced intermediates and final products (Scheme 45)

### Synthesis of amide Ex.200

A mixture of **Ex.197**·TFA (60 mg, 0.094 mmol), 1*H*-indole-3-acetic acid (25 mg, 0.14 mmol), HATU (54 mg, 0.14 mmol) and HOAt (19 mg, 0.14 mmol) was dissolved in DMF (1.5 mL). *i*-Pr₂NEt (81 µL, 0.471 mmol) was added. The soln was stirred for 18 h at rt and concentrated. The residue was dissolved in CHCl₃ and washed (sat. aq. NaHCO₃ soln, H₂O). The organic phase was dried (Na₂SO₄), filtered and concentrated, followed by FC (EtOAc, then CH₂Cl₂/MeOH 95:5) to afford **Ex.200** (50 mg, 78%).
Data of **Ex.200:** cf. **Table 30b**
¹H-NMR (DMSO-d₆): 10.86 (s, 1 H), 8.42 (d, J = 7.8, 1 H), 8.01 (d, J = 10.0, 1 H), 7.58 (d, J = 7.8, 1 H), 7.36-7.19 (m, 9 H), 7.07-7.02 (m, 2 H), 6.97 (t, J = 7.1, 1 H), 6.86 (d, J = 7.6, 1 H), 5.08 (s, 2 H), 4.88 (d, J = 8.7, 1 H), 4.30-4.10 (m, 2 H), 4.13 (d, J = 10.9, 1 H), 4.01 (t-like m, 1 H), 3.95 (d, J = 18.0, 1 H), 3.75-3.70 (m, 2 H), 3.56 (s, 2 H), 3.4-3.2 (m, 2 H, partially superimposed by H₂O signal), 3.04 (t, J = 9.9, 1 H), 2.98 (s, 3 H), 2.65 (s, 3 H), 2.27 (m, 1 H), 2.09 (q, J = 11.7, 1 H).

### Synthesis of amine Ex.202

A soln of **Ex.200** (320 mg, 0.47 mmol) in MeOH (28 mL) was hydrogenolyzed under normal pressure at rt for 4 h in the presence of palladium hydroxide on activated charcoal (moistened with 50% H₂O; 158 mg). The mixture was filtered through a pad of celite. The residue was washed (MeOH). The combined filtrate and washings were concentrated and dried i.v. to yield **Ex.202** (250 mg, 97%).
Data of **Ex.202:** cf. **Table 30b**

### Synthesis of amide Ex.213

A soln of **Ex.202** (60 mg, 0.11 mmol) in dry CH₂Cl₂ (1 mL) was treated with pyridine (89 µL, 1.1 mmol). Decanoyl chloride (46 µL, 0.22 mmol) was slowly added at 0°C. The mixture was stirred at 0°C to rt for 18 h followed by the addition of MeOH (0.1 mL). Stirring was continued for 10 min. The volatiles were evaporated. The residue was treated three times with toluene and evaporated. Purification by prep. HPLC (method 1) and subsequent FC (EtOAc/MeOH 90:10 to 80:20) afforded **Ex.213** (27 mg, 35%).
Data of **Ex.213:** cf. **Table 30b**
¹H-NMR (DMSO-d₆): 10.86 (s, 1 H), 8.53 (d, J = 9.8, 1 H), 8.44 (d, J = 7.7, 1 H), 7.57 (d, J = 7.7, 1 H), 7.35-7.30 (m, 3 H), 7.27 (s, 1 H), 7.19-6.95 (m, 3 H), 6.84 (d, J = 7.5, 1 H), 4.86 (dd, J = 2.4, 11.2, 1 H), 4.60 (q, J = 8.4, 1 H), 4.25 (q-like m, 1 H), 4.14 (d, J = 10.7, 1 H), 4.04-3.82 (m, 3 H), 3.73 (t, J ca. 8.5, 1 H), 3.55 (s, 2 H), 3.24 (d, J = 7.8, 2 H), 3.09 (t, J = 9.5, 1 H), 2.99 (s, 3 H), 2.67 (s, 3 H), 2.26 (m, 1 H), 2.15 (t, J = 7.2, 2 H), 2.09 (m, 1 H), 1.51 (t-like m, 2 H), 1.24 (s, 12 H), 0.85 (t, J = 6.6, 3 H).

### Core 21: Synthesis of selected advanced intermediates and final products (Scheme 46)

### Synthesis of amide Ex.316

A mixture of **Ex.314** (500 mg, 0.91 mmol), HATU (693 mg, 1.82 mmol) and HOAt (248 mg, 1.82 mmol) in DMF (7 mL) was treated with 2-naphthylmethylamine (287 mg, 1.82 mmol) and *i*-Pr₂NEt (0.6 mL, 3.65 mmol) for 15 h. The volatiles were evaporated. Aqueous workup (CHCl₃, sat. aq. NaHCO₃ soln, H₂O; Na₂SO₄) and FC (CH₂Cl₂/MeOH 97:3 to 95:5)
afforded **Ex.316** (542 mg, 86%).
Data of **Ex.316:** cf. **Table 37.2.b**

### Synthesis of amide Ex.317

A mixture of **Ex.314** (500 mg, 0.91 mmol), HATU (693 mg, 1.82 mmol) and HOAt (248 mg, 1.82 mmol) in DMF (7 mL) was treated with *N,N*-dimethylethylenediamine (0.2 mL, 1.82 mmol) and *i-*Pr₂NEt (0.6 mL, 3.65 mmol) for 15 h. The volatiles were evaporated. Aqueous workup (CHCl₃, sat. aq. NaHCO₃ soln, H₂O; Na₂SO₄) and FC (CH₂Cl₂/MeOH 90:10 to 80:20) afforded **317** (406 mg, 72%).
Data of **Ex.317:** cf. **Table 37.2.b**

### Synthesis of amine Ex.318

A soln of **Ex.316** (500 mg, 0.727 mmol) in dioxane (4.5 mL) was treated with 4M HCl-dioxane (9 mL) at rt for 3 h. The volatiles were evaporated to give **Ex.318**·HCl (479 mg, quantitative yield).
Data of **Ex.318**·HCl: cf. **Table 37.2.b**

### Synthesis of amine Ex.319

A soln of **Ex.317** (360 mg, 0.582 mmol) in dioxane (3.6 mL) was treated with 4M HCl-dioxane (7.3 mL) at rt for 3 h. The volatiles were evaporated to give **Ex.319**·HCl (391 mg, quant. yield).
Data of **Ex.319**·HCl: cf. **Table 37.2.b**

### Synthesis of amide Ex.320

A suspension of **Ex.318** (80 mg, 0.128 mmol) in CH₂Cl₂ (1.5 mL) was treated at rt with pyridine (0.10 mL, 1.28 mmol) and acetic anhydride (0.06 mL, 0.64 mmol) for 15 h. MeOH (0.15 mL) was added and stirring was continued for 15 min. The volatiles were evaporated and the residue purified by prep. HPLC (method 1) to afford **Ex.320** (35 mg, 43%).
Data of **Ex.320:** cf. **Table 37.2.b**

### Synthesis of amide Ex.321

A mixture of **Ex.318** (80 mg, 0.13 mmol), 3-(pyridine-4-yl)propanoic acid (21 mg, 0.14 mmol), HATU (73 mg, 0.19 mmol) and HOAt (26 mg, 0.19 mmol) in DMF (2.2 mL) was treated with *i-*Pr₂NEt (0.11 mL, 0.64 mmol) for 15 h. The volatiles were evaporated. Aqueous workup (CHCl₃, sat. aq. NaHCO₃ soln, H₂O; Na₂SO₄) and prep. HPLC (method 1) afforded **Ex.321**·CF₃CO₂H (42 mg, 39%).
Data of **Ex.321**·CF₃CO₂H: cf. **Table 372.2.b**

### Synthesis of amide Ex.322

Pyridine (0.10 mL, 1.28 mmol) and valeroyl chloride (0.031 mL, 0.256 mmol) were added to a mixture of **Ex.318** (80 mg, 0.13 mmol) and CH₂Cl₂ (1.5 mL). The mixture was stirred at rt for 15 h followed by the addition of MeOH (0.15 mL). Stirring was continued for 10 min. The volatiles were evaporated. Purification by prep. HPLC (method 1) afforded **Ex.322** (43 mg, 49%).
Data of **Ex.322:** cf. **Table 37.2.b**

### Synthesis of amide Ex.326

A mixture of **Ex.319** (65 mg, 0.11 mmol), 3-(pyridine-4-yl)propanoic acid (33 mg, 0.22 mmol), HATU (84 mg, 0.22 mmol) and HOAt (30 mg, 0.22 mmol) in DMF (1.5 mL) was treated with *i*-Pr₂NEt (0.075 mL, 0.44 mmol) for 15 h. Aqueous workup (CHCl₃, sat. aq. NaHCO₃ soln, H₂O; Na₂SO₄) and prep. HPLC (method 2) afforded **Ex.326** (42 mg, 39%).
Data of **Ex.326:** cf. **Table 37.2.b**

### Core 24a: Synthesis of selected advanced intermediates and final products (Scheme 47)

### Synthesis of amide Ex.382

*i*-Pr₂NEt (0.079 mL, 0.462 mmol) was added to a mixture of **Ex.380** (100 mg, 0.231 mmol), 2-naphthylacetic acid (86 mg, 0.462 mmol), HATU (176 mg, 0.462 mmol) and HOAt (63 mg, 0.462 mmol) in DMF (1 mL). The mixture was stirred at rt for 16 h and concentrated. Aqueous workup (CH₂Cl₂, sat. aq. NaHCO₃ soln, Na₂SO₄) and FC (hexane/EtOAc/MeOH gradient) and FC (CH₂Cl₂/MeOH) gave **Ex.382** (101 mg, 72%).
Data of **Ex.382:** cf. **Table 40.1.b**

### Synthesis of amine Ex.384

A soln of **Ex.382** (82 mg, 0.137 mmol) in dioxane (1 mL) was treated with 4M HCl-dioxane (1.0 mL) for 2 h at rt. The volatiles were evaporated. The residue was washed with Et₂O to give **Ex.384**·HCl (70 mg, 96%)
Data of **Ex.384**·HCl: cf. **Table 40.1.b**

### Synthesis of amide Ex.386

2,5-Dioxopyrrolidin-1-yl-3-(dimethylamino)phenylcarbamate (36 mg, 0.129 mmol) and *i*-Pr₂NEt (0.06 mL, 0.352 mmol) were added to a suspension of **Ex.384**·HCl (63 mg, 0.117 mmol) in THF (1.0 mL). Stirring of the mixture at rt for 16 h followed by aqueous workup (CH₂Cl₂, sat. aq. Na₂CO₃ soln, Na₂SO₄) and FC (hexane/EtOAc/MeOH gradient) gave **Ex.386** (43 mg, 55%).
Data of **Ex.386:** cf. **Table 40.1.b**

### Synthesis of amide Ex.388

At rt **Ex.379** (93 mg, 0.164 mmol) was treated with 4M HCl-dioxane (3 mL) for 4 h. The volatiles were evaporated to afford **Ex.387**·HCl (86 mg).

*i*-Pr₂NEt (0.084 mL, 0.49 mmol) was added to a mixture of crude **Ex.387**·HCl (82 mg), 2-naphthylacetic acid (36 mg, 0.196 mmol), HATU (93 mg, 0.245 mmol) and HOAt (33 mg, 0.245 mmol) in DMF (2.4 mL). The mixture was stirred at rt for 20 h and concentrated. Aqueous workup (CH₂Cl₂, 1 M aq. Na₂CO₃ soln, Na₂SO₄) and FC (hexane/EtOAc/MeOH gradient) gave **Ex.388** (64 mg, 61%).
Data of **Ex.388:** cf. **Table 40.1.b**

### Synthesis of amide Ex.389

*i*-Pr₂NEt (0.079 mL, 0.462 mmol) was added to a mixture of **Ex.380** (100 mg, 0.231 mmol), decanoic acid (80 mg, 0.462 mmol), HATU (176 mg, 0.462 mmol) and HOAt (63 mg, 0.462 mmol) in DMF (1 mL). The mixture was stirred at rt for 16 h and concentrated. Aqueous workup (CH₂Cl₂, sat. aq. NaHCO₃ soln, Na₂SO₄) and FC (hexane/EtOAc/MeOH gradient) gave **Ex.389** (97 mg, 71%).
Data of **Ex.389:** cf. **Table 40.1.b**

### Synthesis of amine Ex.390

A soln of **Ex.389** (81 mg, 0.138 mmol) in dioxane (1 mL) was treated with 4M HCl-dioxane (1.0 mL) for 2 h at rt. The volatiles were evaporated. The residue was washed with Et₂O to give **Ex.390**·HCl (76 mg, 91%).
Data of **Ex.390**·HCl: cf. **Table 40.1.b**

### Synthesis of amide Ex.391

*i*-Pr₂NEt (0.062 mL, 0.12 mmol) was added to a mixture of **Ex.390**·HCl (63 mg 0.12 mmol), 2-naphthylacetic acid (45 mg, 0.241 mmol), HATU (92 mg, 0.241 mmol) and HOAt (33 mg, 0.241 mmol) in DMF (1 mL). The mixture was stirred at rt for 2 h and concentrated. Aqueous workup (CH₂Cl₂, sat. aq. NaHCO₃ soln, Na₂SO₄) and FC (hexane/EtOAc/MeOH gradient) afforded **Ex.391** (56 mg, 70%).
Data of **Ex.391:** cf. **Table 40.1.b**

The generic macrocyclic ring structures (Cores) related to **Tables 20-49** are depicted in **Scheme 41** in the order of their core numbers.

**Table 20: Examples of Core 01 and Core 02 (Ex.1-Ex.2)**

| **No** | | | **IUPAC name** |
|---|---|---|---|
| **Core 01** | **R²** | **R⁵⁴** | |
| **Ex.1** | 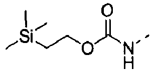 | OCH₂Ph | 8-benzyl 2-[2-(trimethylsilyl)ethyl] (2S,8S,16aS)-12-fluoro-9-methyl-5,10-dioxo-2,3,5,6,7,8,9,10,16,16a-decahydro-1*H-*pyrrolo[2,1-c][1,4,9]benzoxadiazacyclododecine-2,8-dicarboxylate |
| **Core 02** | **R¹¹** | **R⁵⁴** | |
| **Ex**.**2** | 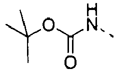 | OCH₂Ph | 9-benzyl 2-(*tert*-butyl) (9S,17aS)-13-fluoro-10-methyl-6,11-dioxo-3,4,6,7,8,9,10,11,17,17a-decahydropyrazino[2,1-*c*][[1,4,9]benzoxadiazacyclododecine-2,9(1*H*)-dicarboxylate |

**Table 21a: Examples of Core 03 (Ex.3-Ex.55, Ex.482-Ex.503 and Ex.590-Ex.591; continued on the following pages)**

| **No** | **R⁵⁴** | **R²** | **Starting material** | **General Procedure** | **Reagent** | **Purification Method** | **Yield (isolated salt)** |
|---|---|---|---|---|---|---|---|
| **Ex.3-Ex.5:** *cf. experimental description* | | | | | | | |
| **Ex**.**6** | OH | 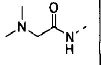 | **Ex.52** | H | H₂, Pd(OH)₂-C^{*)} | Crude product | 70% |
| **Ex.7** | 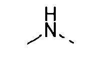 | 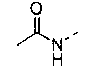 | **Ex.25** | L.1.1 | acetic anhydride (5 equiv.) | prep. HPLC, method 1 | 56% |
| **Ex.8** | 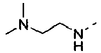 | 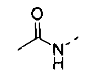 | **Ex.28** | L.1.1 | acetic anhydride (10 equiv.) | prep. HPLC, method 1 | 26% (TFA salt) |
| **Ex**.**9** | 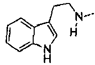 | 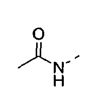 | **Ex.53** | L.2 | tryptamine (1.5 equiv.) HATU (1.5 equiv.) HOAt (1.5 equiv.) *i*-Pr₂NEt (3 equiv.) | FC (CH₂Cl₂/MeOH) | 62% |
| **Ex**.**10** | 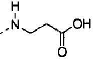 | 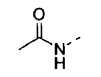 | **Ex.31** | N | LiOH·H₂O | prep. HPLC, method 1 | 57% |
| **Ex**.**11** | 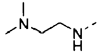 | 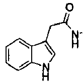 | **Ex.28** | L.1.3 | 3-indoleacetic acid | FC (CH₂Cl₂/MeOH/aq. NH₃) | 58% |
| **Ex**.**12** | 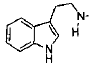 | 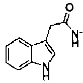 | **Ex.55** | L.1.3 | 3-indoleacetic acid | prep. HPLC, method 1 | 55% |
| **Ex**.**13** | 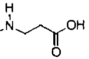 | 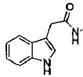 | **Ex.34** | N | LiOH·H₂O | prep. HPLC, method 1 | 60% |
| **Ex.14** | 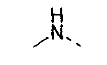 | 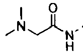 | **Ex.25** | **) | N,N-dimethyl glycine | prep. HPLC, method 1 | 22% (TFA salt) |
| **Ex**.**15** | 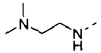 | 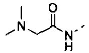 | **Ex.28** | L.1.3 | N,N-dimethyl glycine | prep. HPLC, method 1 | 42% (TFA salt) |
| **Ex**.**16** | 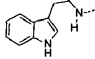 | 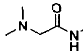 | **Ex.55** | L.1.3 | N,N-dimethyl glycine | prep. HPLC, method 1 | 56% (TFA salt) |
| **Ex.17** | 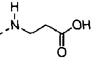 | 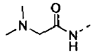 | **Ex.33** | N | LiOH·H₂O | prep. HPLC, method 1 | 77% (TFA salt) |
| **Ex**.**18** | 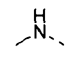 | 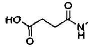 | **Ex**.**25** | L.1.1 | succinic anhydride (1.05 equiv) | prep. HPLC, method 1 | 62% |
| **Ex**.**19** | 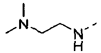 | 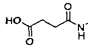 | **Ex.28** | L.1.1 | succinic anhydride (1.05 equiv) pyridine (49 equiv.) | prep. HPLC, method 1 | 67% (TFA salt) |
| **Ex.20** | 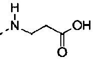 | 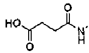 | **Ex.32** | N | LiOH·H₂O | prep. HPLC, method 1 | 72% |
| **Ex.21** | 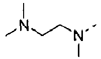 | 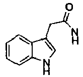 | **Ex.23** | L.1.3 | 3-indoleacetic acid | prep. HPLC, method 1 | 32% |
| **Ex.22** | 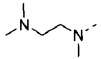 | 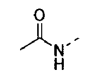 | **Ex.23** | L.1.1 | acetic anhydride (10 equiv) pyridine (120 equiv.) | prep. HPLC, method 1 | 53% (TFA salt) |
| **Ex.23** | 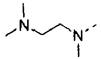 | NH₂ | **Ex.29** | J | HCl-dioxane | prep. HPLC, method 1 | 52% (TFA salt) |
| **Ex.24** | 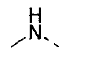 | 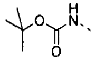 | **Ex.4** | L.2 | methylamine-HCl (10 equiv.), HATU (1.5 equiv.) HOAt (1.5 equiv.) i-Pr₂NEt (13 equiv.) | FC (CH₂Cl₂/MeOH) | 89% |
| **Ex.25** | 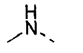 | NH₂ | **Ex.24** | J | HCl-dioxane | crude product | 84% (HCl salt) |
| **Ex.26** | 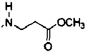 | 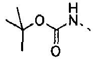 | **Ex.4** | L.2 | β-alaninemethylester hydrochloride | FC (CH₂Cl₂/MeOH) | 97% |
| **Ex.27** | 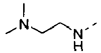 | 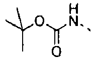 | **Ex.4** | L.2 | N,N-dimethylethylenediamine | FC (CH₂Cl₂/MeOH/aq. NH₃) | 83% |
| **Ex.28** | 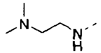 | NH₂ | **Ex.27** | J | HCl-dioxane | (crude product) | 90% (HCl salt) |
| **Ex**.**29** | 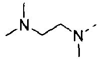 | 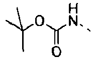 | **Ex.4** | L.2 | N,N,N'-trimethyl-ethylenediamine | FC (CH₂Cl₂/MeOH/aq. NH₃) | 74% |
| **Ex.30** | 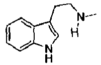 | 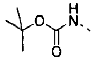 | **Ex.4** | L.2 | tryptamine | FC (CH₂Cl₂/MeOH) | 81% |
| **Ex.31** | 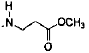 | 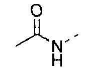 | **Ex.51** | L.1.1 | acetic anhydride (5 equiv) | prep. HPLC, method 1 | 72% |
| **Ex.32** | 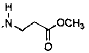 | 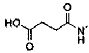 | **Ex.51** | L.1.1 | succinic anhydride (1.05 equiv) | prep. HPLC, method 1 | 68% |
| **Ex.33** | 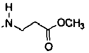 | 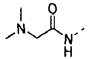 | **Ex.51** | L.1.3 | N,N-dimethyl glycine | prep. HPLC, method 1 | 64% (TFA salt) |
| **Ex.34** | 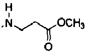 | 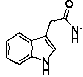 | **Ex.51** | L.1.3 | 3-indoleacetic acid | prep. HPLC, method 1 | 24% |
| **Ex.35** | 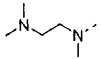 | 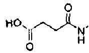 | **Ex.23** | L_{.}1.1 | succinic anhydride (1.05 equiv) | prep. HPLC, method 1 | 63% (TFA salt) |
| **Ex.36** | 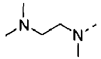 | 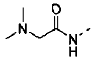 | **Ex.23** | L.1.3 | N,N-dimethyl glycine | prep. HPLC, method 1 | 40% (TFA salt) |
| **Ex.37** | 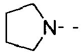 | 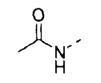 | **Ex.42** | L.1.1 | acetic anhydride (5 equiv) | prep. HPLC, method 1 | 61% |
| **Ex.38** | 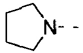 | 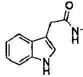 | **Ex.42** | L.1.3 | 3-indoleacetic acid | prep. HPLC, method 1 | 43% |
| **Ex.39** | 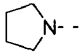 | 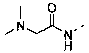 | **Ex.42** | L.1.3 | N,N-dimethyl glycine | prep. HPLC, method 1 | 56% (TFA salt) |
| **Ex.40** | 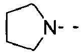 | 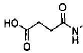 | Ex.42 | L.1.1 | succinic anhydride (1.05 equiv) | prep. HPLC, method 1 | 37% |
| **Ex.41** | 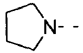 | 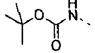 | **Ex.4** | L.2 | pyrrolidine | FC (CH₂Cl₂/MeOH) | 76% |
| **Ex.42** | 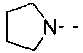 | NH₂ | **Ex.41** | J | HCl-dioxane | (crude product) | 90% (HCl salt) |
| **Ex.43** | 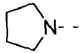 | 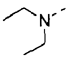 | **Ex.42** | M.1 | acetaldehyde | prep. HPLC, method 1 | 67% (TFA salt) |
| **Ex.44** | 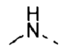 | 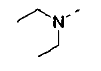 | **Ex.25** | M.1 | acetaldehyde | prep. HPLC, method 1 | 33% (TFA salt) |
| **Ex.45** | 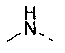 | 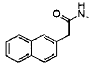 | **Ex.25** | L.1.3 | 2-naphthylacetic acid | prep. HPLC, method 1 | 46% |
| **Ex.46** | 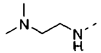 | 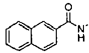 | **Ex.28** | L.1.1 | 2-naphthoyl chloride (3.6 equiv.) | FC (CH₂Cl₂/MeOH/aq. NH₃) | 85% |
| **Ex.47** | 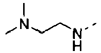 | 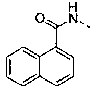 | **Ex.28** | L.1.2 | 1-naphthoic acid | FC (CH₂Cl₂/MeOH/ aq. NH₃) | 85% |
| **Ex.48** | 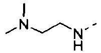 | 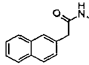 | **Ex.28** | L.1.2 | 2-naphthylacetic acid | FC (CH₂Cl₂/MeOH/ aq. NH₃) | 83% |
| **Ex.49** | 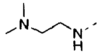 | 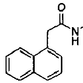 | **Ex.28** | L.1.2 | 1-naphthylacetic acid | FC (CH₂Cl₂/MeOH/ aq. NH₃) | 83% |
| **Ex.50** | 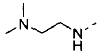 | 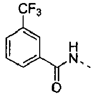 | **Ex.28** | L.1.1 | 3-(trifluoromethyl)-benzoyl chloride (4 equiv.) | FC (CH₂Cl₂/MeOH/ aq. NH₃) | 24% |
| **Ex.51** | 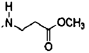 | NH₂ | **Ex.26** | J | HCl-dioxane | crude product | 93% (HCl salt) |
| **Ex.52** | OCH₂Ph | 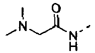 | **Ex.5** | L.1.3 | N,N-dimethylglycine (1.7 equiv.) HATU (1.0 equiv.) HOAt (2.0 equiv.) i-Pr₂NEt (4.0 equiv.) | prep. HPLC, method 1 | 88% (TFA salt) |
| **Ex.53** | OCH₂Ph | 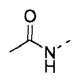 | **Ex**.5 | L.1.1 | Acetic anhydride (10 equiv.) pyridine/CH₂Cl₂ 1:1 | FC | 70% |
| **Ex.54** | OH | 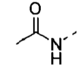 | **Ex.54** | H | H₂, Pd(OH)₂-C | crude product | 92% |
| **Ex.55** | 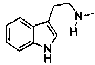 | NH₂ | **Ex.30** | J | HCl-dioxane | FC (CH₂Cl₂/MeOH/aq. NH₃) | 42% |
| **Ex.482** | 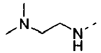 | 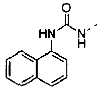 | **262a** | R | 1-naphthyl isocyanate | prep. HPLC, method 1 | 25% (TFA salt) |
| **Ex.483** | 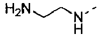 | 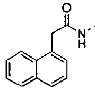 | **Ex.500** | J | HCl-dioxane | crude product | 97% (HCl salt) |
| **Ex.484** | 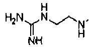 | 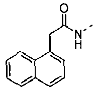 | **Ex.483** | - | cf. detailed description of examples | prep. HPLC, method 1 | 18% (TFA salt) |
| **Ex.485** | 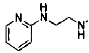 | 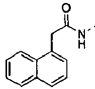 | **Ex.499** | L.2 | N-(2-amino-ethyl)pyridine-2-amine (2 equiv.) i-Pr₂NEt (2 equiv.) | prep. HPLC, method 2 | 60% |
| **Ex.486** | 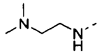 | 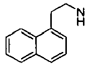 | **Ex.28** | M.2 | 2-(naphthalene-1-yl)acetaldehyde | FC(CH₂Cl₂/MeOH/ aq. NH₃), then prep. HPLC, method 1 | 6% (TFA salt) |
| **Ex.487** | 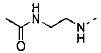 | 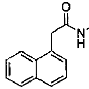 | **Ex.483** | L.1.1 | acetic anhydride (5 equiv.) pyridine (10 equiv) | prep. HPLC, method 1 | 75% |
| **Ex.488** | 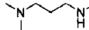 | 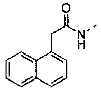 | **Ex.499** | L.2 | N,N-dimethylpropylene-diamine (2 equiv.) *i*-Pr₂NEt (2 equiv.) | prep. HPLC, method 2 | 66% |
| **Ex.489** | 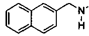 | 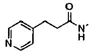 | **262d** | R | 3-(pyridin-4-yl)propanoic acid | FC (CH₂Cl₂/EtOAc/ MeOH) | 37% |
| **Ex.490** | 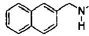 | 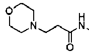 | **262d** | R | 3-morpholinopropanoic acid hydrochloride | prep. HPLC, method 1 | 52% (TFA salt) |
| **Ex.491** | 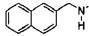 | 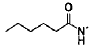 | **262d** | R | hexanoic acid | prep. HPLC, method 3 | 43% |
| **Ex.492** | 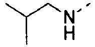 | 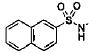 | **262c** | R | naphthalene-2-sulfonyl chloride | prep. HPLC, method 3 | 24% |
| **Ex.493** | 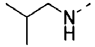 | 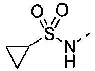 | **262c** | R | cyclopropanesulfonyl chloride | prep. HPLC, method 1 | 35% |
| **Ex.494** | 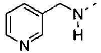 | 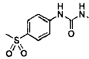 | **262b** | R | 2,5-dioxopyrrolidin-1-yl-4-(methylsulfonyl)-phenylcarbamate | prep. HPLC, method 1 | 34% (TFA salt) |
| **Ex.495** | 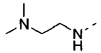 | 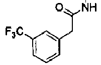 | **262a** | R | 3-(trifluoromethyl) phenylacetic acid | prep. HPLC, method 1 | 24% (TFA salt) |
| **Ex.496** | 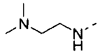 | 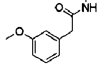 | **262a** | R | 3-methoxy phenylacetic acid | prep. HPLC, method 1 | 24% (TFA salt) |
| **Ex.497** | 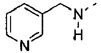 | 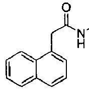 | **262b** | R | 1-naphthylacetic acid | prep. HPLC, method 1 | 18% (TFA salt) |
| **Ex.498** | OCH₂Ph | 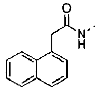 | **Ex.5** | L.1.3 | 1-naphthylacetic acid (2 equiv.) i-Pr₂NEt (3 equiv.) | FC (EtOAc/MeOH) | 87% |
| **Ex.499** | OH | 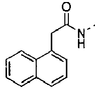 | **Ex.498** | H | H₂, Pd(OH)₂-C | crude product | 96% |
| **Ex.500** | 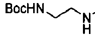 | 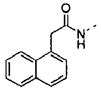 | **Ex.499** | L.2 | tert. butyl 2-aminoethylcarbamate (2 equiv.) i-Pr₂NEt (2 equiv.) | FC (CH₂Cl₂/MeOH/aq. NH₃) | 74% |
| **Ex.501** | 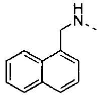 | 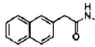 | **262e** | R | 2-naphthylacetic acid | prep. HPLC, method 3 | 44% |
| **Ex.502** | 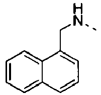 | 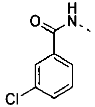 | **262e** | R | 3-chlorobenzoic acid | prep. HPLC, method 3 | 48% |
| **Ex.503** | 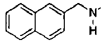 | 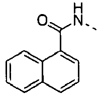 | **262d** | R | 1-naphthoic acid | prep. HPLC, method 3 | 43% |
| **Ex.590** | OH | NH₂ | **Ex.4** | ***) | HCl-dioxane | crude product | quant. (HCl salt) |
| **Ex.591** | OH | 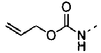 | **Ex.590** | ***) | AllocCl | crude product | 95% |

| | | | | | | | |
|---|---|---|---|---|---|---|---|
| *) Prior to debenzylation starting material **Ex.52·**TFA was converted into the free base (CHCl₃, aq. Na₂CO₃ soln) **) The amide coupling was performed at room temperature with N,N-dimethyl glycine (2.2 equiv.) in CH₂Cl₂, in the presence of T3P (50% in EtOAc; 2.2 equiv.) and i-Pr₂NEt (3 equiv.). ***) cf. detailed description | | | | | | | |

**Table 21b: Examples of Core 03 (Ex.3-Ex.55, Ex.482-Ex.503 and Ex.590-Ex.591; continued on the following pages)**

| **No** | **R⁵⁴** | **R²** | **FORMULA** | **MONOISOTOPIC MASS** | **Rt (purity at 220nm)** | **[M+H]⁺ found** | **LC-MS-Method** |
|---|---|---|---|---|---|---|---|
| **Ex.3-Ex.5:** *cf. experimental description* | | | | | | | |
| **Ex.6** | **OH** | 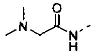 | C25H34FN5O7 | 535.2 | 1.05 (99) | 536.3 | Method 2 |
| **Ex.7** | 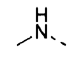 | 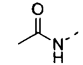 | C24H32FN5O6 | 505.2 | 1.21 (87) | 506.3 | Method 2 |
| **Ex.9** | 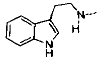 | 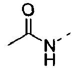 | C33H39FN6O6 | 634.3 | 2.12 (99) | 635.4 | Method 1a |
| **Ex.10** | 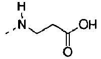 | 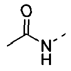 | C26H34FN5O8 | 563.2 | 1.20 (91) | 564.2 | Method 2 |
| **Ex.11** | 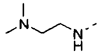 | 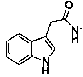 | C35H44FN7O6 | 677.3 | 1.34 (93) | 678.4 | Method 2 |
| **Ex.12** | 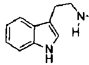 | 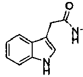 | C41H44FN7O6 | 749.3 | 1.73 (92) | 750.4 | Method 2 |
| **Ex. 13** | 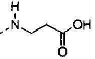 | 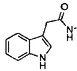 | C34H39FN6O8 | 678.3 | 1.45 (87) | 679.3 | Method 2 |
| **Ex.14** | 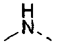 | 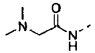 | C26H37FN6O6 | 548.3 | 1.08 (88) | 549.3 | Method 2 |
| **Ex.15** | 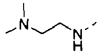 | 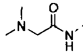 | C29H44FN7O6 | 605.3 | 0.99 (96) | 606.4 | Method 2 |
| **Ex.16** | 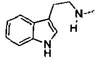 | 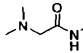 | C35H44FN7O6 | 677.3 | 1.41 (97) | 678.4 | Method 2 |
| **Ex.17** | 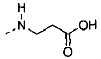 | 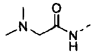 | C28H39FN6O8 | 606.3 | 1.09 (94) | 607.3 | Method 2 |
| **Ex.18** | 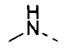 | 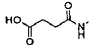 | C26H34FN5O8 | 563.2 | 1.20 (88) | 564.3 | Method 2 |
| **Ex.19** | 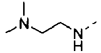 | 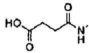 | C29H41FN6O8 | 620.3 | 1.08 (100) | 621.3 | Method 2 |
| **Ex.20** | 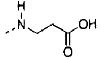 | 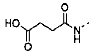 | C28H36FN5O10 | 621.2 | 1.18 (91) | 622.2 | Method 2 |
| **Ex.21** | 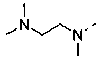 | 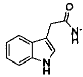 | C36H46FN7O6 | 691.4 | 1.35 (88) | 692.4 | Method 2 |
| **Ex.22** | 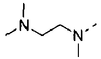 | 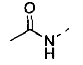 | C28H41FN6O6 | 576.3 | 1.12 (96) | 577.4 | Method 2 |
| **Ex.23** | 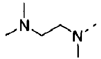 | NH₂ | C26H39FN6O5 | 534.3 | 0.96 (88) | 535.4 | Method 2 |
| **Ex.24** | 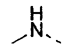 | 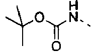 | C27H38FN5O7 | 563.3 | 1.54 (93) | 564.3 | Method 2 |
| **Ex.25** | 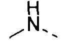 | NH₂ | C22H30FN5O5 | 463.2 | 1.06 (91) | 464.2 | Method 2 |
| **Ex.26** | 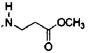 | 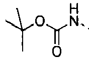 | C30H42FN5O9 | 635.3 | 1.61 (87) | 636.4 | Method 2 |
| **Ex.27** | 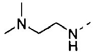 | 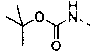 | C30H45FN6O7 | 620.3 | 1.53 (92) | 621.3 | Method 4a |
| **Ex.28** | 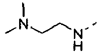 | NH₂ | C25H37FN6O5 | 520.3 | 0.93 (94) | 521.3 | Method 2 |
| **Ex.29** | 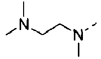 | 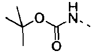 | C31H47FN6O7 | 634.4 | 1.41 (96) | 635.4 | Method 2 |
| **Ex.30** | 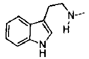 | 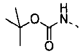 | C36H45FN6O7 | 692.3 | 1.79 (99) | 693.4 | Method 2 |
| **Ex.31** | 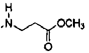 | 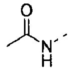 | C27H36FN5O8 | 577.3 | 1.32 (90) | 578.3 | Method 2 |
| **Ex.32** | 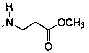 | 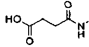 | C29H38FN5O10 | 635.3 | 1.30 (83) | 636.2 | Method 2 |
| **Ex.33** | 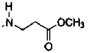 | 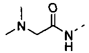 | C29H41FN6O8 | 620.3 | 1.18 (100) | 621.3 | Method 2 |
| **Ex.34** | 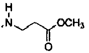 | 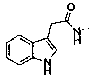 | C35H41FN6O8 | 692.3 | 1.56 (90) | 693.3 | Method 2 |
| **Ex.35** | 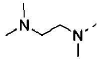 | 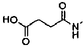 | C30H43FN6O8 | 634.3 | 1.10 (94) | 635.3 | Method 2 |
| **Ex.36** | 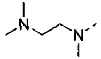 | 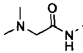 | C30H46FN7O6 | 619.4 | 1.00 (91) | 620.3 | Method 2 |
| **Ex.37** | 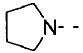 | 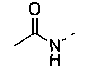 | C27H36FN5O6 | 545.3 | 1.36 (93) | 546.3 | Method 2 |
| **Ex.38** | 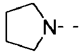 | 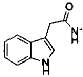 | C35H41FN6O6 | 660.3 | 1.60 (94) | 661.3 | Method 2 |
| **Ex.39** | 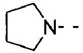 | 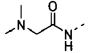 | C29H41FN6O6 | 588.3 | 1.19 (94) | 589.3 | Method 2 |
| **Ex.40** | 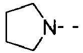 | 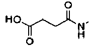 | C29H38FN5O8 | 603.3 | 1.33 (94) | 604.3 | Method 2 |
| **Ex.41** | 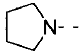 | 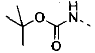 | C30H42FN5O7 | 603.3 | 1.67 (90) | 604.3 | Method 2 |
| **Ex.42** | 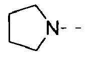 | NH₂ | C25H34FN5O5 | 503.3 | 1.17 (92) | 504.2 | Method 2 |
| **Ex.43** | 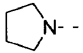 | 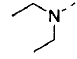 | C29H42FN5O5 | 559.3 | 1.26 (96) | 560.3 | Method 2 |
| **Ex.44** | 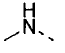 | 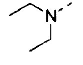 | C26H38FN5O5 | 519.3 | 1.13 (97) | 520.3 | Method 2 |
| **Ex.45** | 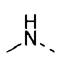 | 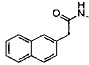 | C34H38FN5O6 | 631.3 | 1.65 (97) | 632.2 | Method 2 |
| **Ex.46** | 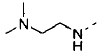 | 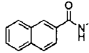 | C36H43FN6O6 | 674.3 | 1.49 (97) | 675.5 | Method 2 |
| **Ex.47** | 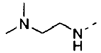 | 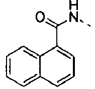 | C36H43FN6O6 | 674.3 | 1.43 (96) | 675.5 | Method 2 |
| **Ex.48** | 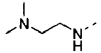 | 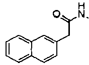 | C37H45FN6O6 | 688.3 | 1.50 (95) | 689.5 | Method 2 |
| **Ex.49** | 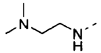 | 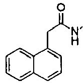 | C37H45FN6O6 | 688.3 | 1.48 (95) | 689.5 | Method 2 |
| **Ex.50** | 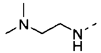 | 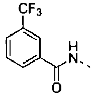 | C33H40F4N6O6 | 692.3 | 1.49 (97) | 693.5 | Method 2 |
| **Ex.51** | 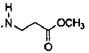 | NH₂ | C25H34FN5O7 | 535.2 | 1.05 | 536.3 | Method 9c |
| **Ex.52** | OCH₂Ph | 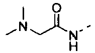 | C32H40FN5O7 | 625.3 | 1.47 | 626.3 | Method 9c |
| **Ex.53** | OCH₂Ph | 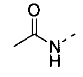 | C30H35FN4O7 | 582.3 | 1.65 | 582.9 | Method 9c |
| **Ex.54** | OH | 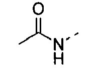 | C23H29FN4O7 | 492.2 | 1.04 | 493.1 | Method 9c |
| **Ex.55** | 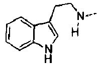 | NH₂ | C31H37FN6O5 | 592.3 | 1.38 | 593.0 | Method 9c |
| **Ex.482** | 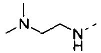 | 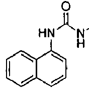 | C36H44FN7O6 | 689.3 | 1.43 (91) | 690.4 | Method 10a |
| **Ex.483** | 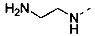 | 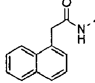 | C35H41FN6O6 | 660.3 | 1.42 (85) | 661.4 | Method 10a |
| **Ex.484** | 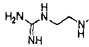 | 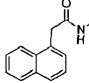 | C36H43FN8O6 | 702.3 | 1.46 (90) | 703.3 | Method 10a |
| **Ex.485** | 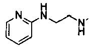 | 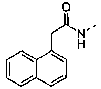 | C40H44FN7O6 | 737.3 | 1.79 (93) | 738.4 | Method 11a |
| **Ex.486** | 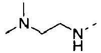 | 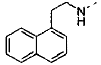 | C37H47FN6O5 | 674.4 | 1.34 (80) | 675.3 | Method 10a |
| **Ex.487** | 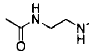 | 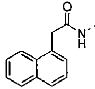 | C37H43FN6O7 | 702.3 | 1.56 (78) | 703.4 | Method 10a |
| **Ex.488** | 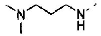 | 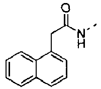 | C38H47FN6O6 | 702.4 | 1.79 (91) | 703.5 | Method 11a |
| **Ex.489** | 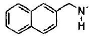 | 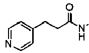 | C40H43FN6O6 | 722.3 | 1.52 (97) | 723.4 | Method 10a |
| **Ex.490** | 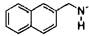 | 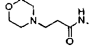 | C39H47FN6O7 | 730.4 | 1.68 (97) | 731.4 | Method 4a |
| **Ex.491** | 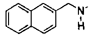 | 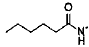 | C38H46FN5O6 | 687.3 | 2.14 (96) | 688.4 | Method 4a |
| **Ex.492** | 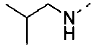 | 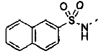 | C35H42FN5O7S | 695.3 | 1.94 (93) | 696.4 | Method 10a |
| **Ex.493** | 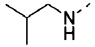 | 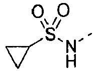 | C28H40FN5O7S | 609.3 | 1.58 (87) | 610.3 | Method 10a |
| **Ex.494** | 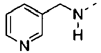 | 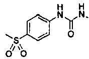 | C35H40FN7O8S | 737.3 | 1.21 (95) | 738.3 | Method 10a |
| **Ex.495** | 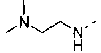 | 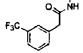 | C34H42F4N6O6 | 706.3 | 1.50 (91) | 707.4 | Method 10a |
| **Ex.496** | 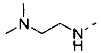 | 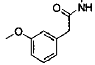 | C34H45FN6O7 | 668.3 | 1.31 (93) | 669.4 | Method 10a |
| **Ex.497** | 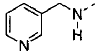 | 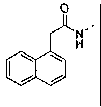 | C39H41FN6O6 | 708.3 | 1.47 (87) | 709.4 | Method 10a |
| **Ex.498** | OCH₂Ph | 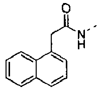 | C40H41FN4O7 | 708.3 | 2.13 (90) | 709.4 | Method 10a |
| **Ex.499** | OH | 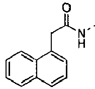 | C33H35FN4O7 | 618.3 | 1.63 (90) | 619.2 | Method 10a |
| **Ex.500** | 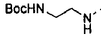 | 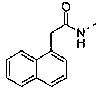 | C40H49FN6O8 | 760.4 | 1.90 (95) | 761.5 | Method 10a |
| **Ex.501** | 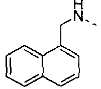 | 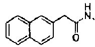 | C44H44FN5O6 | 757.3 | 2.27 (93) | 758.4 | Method 4a |
| **Ex.502** | 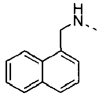 | 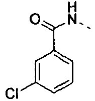 | C39H39ClFN5O6 | 727.3 | 2.22 (95) | 728.3 | Method 4a |
| **Ex.503** | 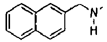 | 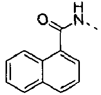 | C43H42FN5O6 | 743.3 | 2.17 (97) | 744.4 | Method 4a |
| **Ex.590** | OH | NH₂ | C21H27FN4O6 | 450.2 | 1.05 (93) | 451.0 | Method 4b |
| **Ex.591** | OH | 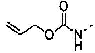 | C25H31FN4O8 | 534.2 | 1.58 (91) | 535.2 | Method 4b |

**Table 21c: Examples of Core 03 (Ex.3-Ex.55, Ex.482-Ex.503 and Ex.590-Ex.591; continued on the following pages)**

| **No** | **R⁵⁴** | **R²** | **IUPAC name** |
|---|---|---|---|
| **Ex**.**3** | OCH₂Ph | 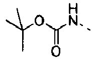 | benzyl (2*S*,11*S*,19a*S*)-2-[(*tert*-butoxycarbonyl)amino]-15-fluoro-7,12-dimethyl-5,8,13-trioxo-2,3,6,7,8,9,10,11,12,13,19,19a-dodecahydro-1*H*,5*H*-pyrrolo[2,1-c] [1,4,7,12]benzoxatriazacyclopentadecine-11-carboxylate |
| **Ex.4** | OH | 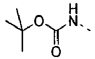 | (2S,11S,19aS)-2-[(tert-butoxycarbonyl)amino]-15-fluoro-7,12-dimethyl-5,8,13-trioxo-2,3,6,7,8,9,10,11,12,13,19,19a-dodecahydro-1*H*,5*H*-pyrrolo[2,1-c][1,4,7,12]benzoxatriazacyclopentadecine-11-carboxylic acid |
| **Ex**.**5** | OCH₂Ph | NH₂ | benzyl (2*S*,11*S*,19a*S*)-2-amino-15-fluoro-7,12-dimethyl-5,8,13-trioxo-2,3,6,7,8,9,10,11,12,13,19,19a-dodecahydro-1*H*,5*H*-pyrrolo[2,1-c][1,4,7,12]benzoxatriazacyclopentadecine-11-carboxylate |
| **Ex.6** | OH | 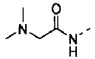 | (2*S*,11*S*,19a*S*)-2-{[2-(dimethylamino)acetyl]amino}-15-fluoro-7,12-dimethyl-5,8,13-trioxo-2,3,6,7,8,9,10,11,12,13,19,19a-dodecahydro-1*H*,5*H*-pyrrolo[2,1-c][1,4,7,12]benzoxatriazacyclopentadecine-11-carboxylic acid |
| **Ex.7** | 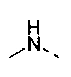 | 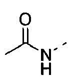 | (2*S*,11*S*,19a*S*)-2-(acetylamino)-15-fluoro-*N*,7,12-trimethyl-5,8,13-trioxo-2,3,6,7,8,9,10,11,12,13,19,19a-dodecahydro-1*H*,5*H*-pyrrolo[2,1-c] [1,4,7,12]benzoxatriazacyclopentadecine-11-carboxamide |
| **Ex**.**8** | 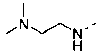 | 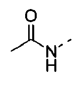 | *(*2*S,* 11*S,* 19a*S)-*2*-(*acetylamino)*-N-*[2*-*(dimethylamino)ethyl]*-*15-fluoro-7,12-dimethyl-5,8,13-trioxo-2,3,6,7,8,9,10,11,12,13,19,19a-dodecahydro-1*H*,5*H-*pyrrolo[2,1-c][1,4,7,12]benzoxatriazacyclopentadecine-11-carboxamide |
| **Ex.9** | 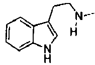 | 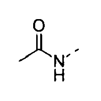 | (2*S*,11*S*,19a*S*)-2-(acetylamino)-15-fluoro-*N*-[2-(1H-indol-3-yl)ethyl]-7,12-dimethyl-5,8,13-trioxo-2,3,6,7,8,9,10,11,12,13,19,19a-dodecahydro-1*H*,5*H-*pyrrolo[2,1-c][1,4,7,12]benzoxatriazacyclopentadecine-11-carboxamide |
| **Ex.10** | 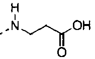 | 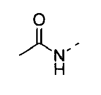 | 3-({[(2*S*,11*S*,19a*S*)-2-(acetylamino)-15-fluoro-7,12-dimethyl-5,8,13-trioxo-2,3,6,7,8,9,10,11,12,13,19,19a-dodecahydro-1*H*,5*H*-pyrrolo[2,1-c] [1,4,7,12]benzoxatriazacyclopentadecin-11-yl]carbonyl}amino)propanoic acid |
| **Ex**. **11** | 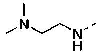 | 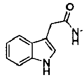 | (2*S*,11*S*,19a*S*)-N-[2-(dimethylamino)ethyl]-15-fluoro-2-{[2-(1H-indol-3-yl)acetyl]amino}-7,12-dimethyl-5,8,13-trioxo-2,3,6,7,8,9,10,11,12,13,19,19a-dodecahydro-1*H*,5*H*-pyrrolo[2,1-c][1,4,7,12]benzoxatriazacyclopentadecine-11-carboxamide |
| **Ex.12** | 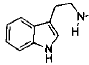 | 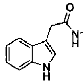 | (2*S*,11*S*,19a*S*)-15-fluoro-2-{[2-(1H-indol-3-yl)acetyl]amino}-*N*-[2-(1H-indol-3-yl)ethyl]-7,12-dimethyl-5,8,13-trioxo-2,3,6,7,8,9,10,11,12,13,19,19a-dodecahydro-1*H*,5*H*-pyrrolo[2,1-c][1,4,7,12]benzoxatriazacyclopentadecine-11-carboxamide |
| **Ex.13** | 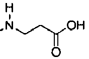 | 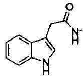 | 3-{[((2*S*,11*S*,19a*S*)-15-fluoro-2-{[2-(1H-indol-3-yl)acetyl]amino}-7,12-dimethyl-5,8,13-trioxo-2,3,6,7,8,9,10,11,12,13,19,19a-dodecahydro-1*H*,5*H*-pyrrolo[2,1-c][1,4,7,12]benzoxatriazacyclopentadecin-11-yl)carbonyl]amino}propanoic acid |
| **Ex.14** | 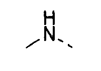 | 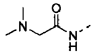 | (2*S*,11*S*,19a*S*)-2-{[2-(dimethylamino)acetyl]amino}-15-fluoro-*N*,7,12-trimethyl-5,8,13-trioxo-2,3,6,7,8,9,10,11,12,13,19,19a-dodecahydro-1*H*,5*H*-pyrrolo[2,1-c][1,4,7,12]benzoxatriazacyclopentadecine-11-carboxamide |
| **Ex.15** | 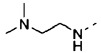 | 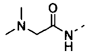 | (2*S*,11*S*,19a*S*)-2-{[2-(dimethylamino)acetyl]amino}-N-[2-(dimethylamino)ethyl]-15-fluoro-7,12-dimethyl-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecine-11-carboxamide |
| **Ex.16** | 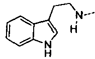 | 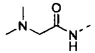 | (2*S*,11*S*,19a*S*)-2-{[2-(dimethylamino)acetyl]amino}-15-fluoro-*N*-[2-(1H-indol-3-yl)ethyl]-7,12-dimethyl-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecine-11-carboxamide |
| **Ex.17** | 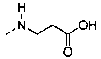 | 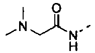 | 3-{[((2*S*,11*S*,19a*S*)-2-{[2-(dimethylamino)acetyl]amino}-15-fluoro-7,12-dimethyl-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecin-11-yl)carbonyl]amino}propanoic acid |
| **Ex.18** | 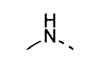 | 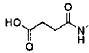 | 4-({(2*S*,11*S*,19a*S*)-15-fluoro-7,12-dimethyl-11-[(methylamino)carbonyl]-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecin-2-yl}amino)-4-oxobutanoic acid |
| **Ex.19** | 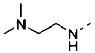 | 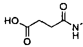 | 4-{[(2*S*,11*S*,19a*S*)-11-({[2-(dimethylamino)ethyl]amino}carbonyl)-15-fluoro-7,12-dimethyl-5,8,13-trioxo-2,3,6,7,8,9,10,11,12,13,19,19a-dodecahydro-1*H*,5*H-*pyrrolo[2,1-*c*][1,4,7,12]benzoxatriazacyclopentadecin-2-yl]amino}-4-oxobutanoic acid |
| **Ex.20** | 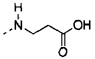 | 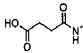 | 4-[((2*S*,11*S*,19a*S*)-15-fluoro-11-{[(3-hydroxy-3-oxopropyl)amino]carbonyl}-7,12-dimethyl-5,8,13-trioxo-2,3,6,7,8,9,10,11,12,13,19,19a-dodecahydro-1*H*,5*H-*pyrrolo[2,1-c][1,4,7,12]benzoxatriazacyclopentadecin-2-yl)amino]-4-oxobutanoic acid |
| **Ex.21** | 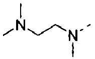 | 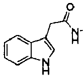 | (2*S*,11*S*,19a*S*)-N-[2-(dimethylamino)ethyl]-15-fluoro-2-{[2-(1*H*-indol-3-yl)acetyl]amino}-*N*,7,12-trimethyl-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecine-11-carboxamide |
| **Ex.22** | 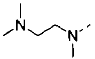 | 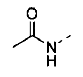 | (2*S*,11*S*,19a*S*)-2-(acetylamino)-*N*-[2-(dimethylamino)ethyl]-15-fluoro-*N*,7,12-trimethyl-5,8,13-trioxo-2,3,6,7,8,9,10,11,12,13,19,19a-dodecahydro-1*H*,5*H-*pyrrolo[2,1-*c*][1,4,7,12]benzoxatriazacyclopentadecine-11-carboxamide |
| **Ex.23** | 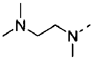 | NH₂ | (2*S*,11*S*,19a*S*)-2-amino-*N*-[2-(dimethylamino)ethyl]-15-fluoro-*N*,7,12-trimethyl-5,8,13-trioxo-2,3,6,7,8,9,10,11,12,13,19,19a-dodecahydro-1*H*,5*H*-pyrrolo[2,1-c][1,4,7,12]benzoxatriazacyclopentadecine-11-carboxamide |
| **Ex.24** | 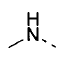 | 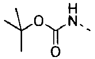 | tert-butyl *N*-{(2*S*,11*S*,19a*S*)-15-fluoro-7,12-dimethyl-11-[(methylamino)carbonyl]-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecin-2-yl}carbamate |
| **Ex.25** | 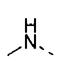 | NH₂ | (2*S*,11*S*,19a*S*)-2-amino-15-fluoro-*N*,7,12-trimethyl-5,8,13-trioxo-2,3,6,7,8,9,10,11,12,13,19,19a-dodecahydro-1*H*,5*H*-pyrrolo[2,1-c][1,4,7,12]benzoxatriazacyclopentadecine-11-carboxamide |
| **Ex.26** | 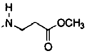 | 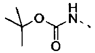 | methyl 3-[({(2*S*,11*S*,19a*S*)-2-[(*tert*-butoxycarbonyl)amino]-15-fluoro-7,12-dimethyl-5,8,13-trioxo-2,3,6,7,8,9,10,11,12,13,19,19a-dodecahydro-1*H*,5*H-*pyrrolo[2,1-c][1,4,7,12]benzoxatriazacyclopentadecin-11-yl}carbonyl)amino]propanoate |
| **Ex.27** | 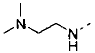 | 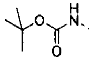 | *tert*-butyl N-[(2*S*,11*S*,19a*S*)-11-({[2-(dimethylamino)ethyl]amino}carbonyl)-15-fluoro-7,12-dimethyl-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecin-2-yl]carbamate |
| **Ex.28** | 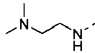 | NH₂ | (2S,11S,19aS)-2-amino-N-[2-(dimethylamino)ethyl]-15-fluoro-7,12-dimethyl-5,8,13-trioxo-2,3,6,7,8,9,10,11,12,13,19,19a-dodecahydro-1*H*,5H-pyrrolo[2,1-*c*] [1, 4,7,12]benzoxatriazacyclopentadecine-11-carboxamide |
| **Ex.29** | 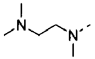 | 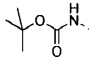 | *tert*-butyl *N*-((2*S*,11*S*,19a*S*)-11-{[[2-(dimethylamino)ethyl](methyl)amino]carbonyl}-15-fluoro-7,12-dimethyl-5,8,13-trioxo-2,3,6,7,8,9,10,11,12,13,19,19a-dodecahydro-1*H*,5H-pyrrolo[2,1-c][1,4,7,12]benzoxatriazacyclopentadecin-2-yl)carbamate |
| **Ex.30** | 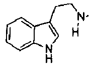 | 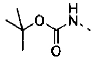 | *tert*-butyl *N*-[(2*S*,11*S*,19a*S*)-15-fluoro-11-({[2-(1*H*-indol-3-yl)ethyl]amino}carbonyl)-7,12-dimethyl-5,8,13-trioxo-2,3,6,7,8,9,10,11,12,13,19,19a-dodecahydro-1H,5H-pyrrolo[2,1-c][1,4,7,12]benzoxatriazacyclopentadecin-2-yl]carbamate |
| **Ex.31** | 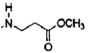 | 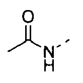 | methyl 3-({[(2*S*,11*S*,19a*S*)-2-(acetylamino)-15-fluoro-7,12-dimethyl-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecin-11-yl]carbonyl}amino)propanoate |
| **Ex.32** | 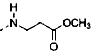 | 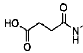 | 4-[((2*S*,11*S*,19a*S*)-15-fluoro-11-{[(3-methoxy-3-oxopropyl)amino]carbonyl}-7,12-dimethyl-5,8,13-trioxo-2,3,6,7,8,9,10,11,12,13,19,19a-dodecahydro-1*H*,5*H-*pyrrolo[2,1-*c*][1,4,7,12]benzoxatriazacyclopentadecin-2-yl)amino]-4-oxobutanoic acid |
| **Ex.33** | 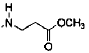 | 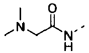 | methyl 3-{[((2*S*,11*S*,19a*S*)-2-{[2-(dimethylamino)acetyl]amino}-15-fluoro-7,12-dimethyl-5,8,13-trioxo-2,3,6,7,8,9,10,11,12,13,19,19a-dodecahydro-1*H*,5*H-*pyrrolo[2,1-c][1,4,7,12]benzoxatriazacyclopentadecin-11-yl)carbonyl]amino}propanoate |
| **Ex.34** | 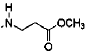 | 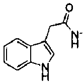 | methyl 3-{[((2*S*,11*S*,19a*S*)-15-fluoro-2-{[2-(1*H*-indol-3-yl)acetyl]amino}-7,12-dimethyl-5,8,13-trioxo-2,3,6,7,8,9,10,11,12,13,19,19a-dodecahydro-1*H*,5*H-*pyrrolo[2,1-c][1,4,7,12]benzoxatriazacyclopentadecin-11-yl)carbonyl]amino}propanoate |
| **Ex.35** | 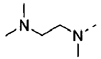 | 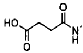 | 4-[((2*S*,11*S*,19a*S*)-11-{[[2-(dimethylamino)ethyl](methyl)amino]carbonyl}-15-fluoro-7,12-dimethyl-5,8,13-trioxo-2,3,6,7,8,9,10,11,12,13,19,19a-dodecahydro-1*H*,5*H*-pyrrolo[2,1-c][1,4,7,12]benzoxatriazacyclopentadecin-2-yl)amino]-4-oxobutanoic acid |
| **Ex.36** | 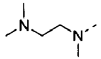 | 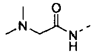 | (2*S*,11*S*,19a*S*)-2-{[2-(dimethylamino)acetyl]amino}-*N*-[2-(dimethylamino)ethyl]-15-fluoro-*N*,7,12-trimethyl-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecine-11-carboxamide |
| **Ex.37** | 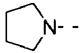 | 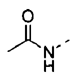 | *N*-[(2*S*,11*S*,19a*S*)-15-fluoro-7,12-dimethyl-5,8,13-trioxo-11-(1-pyrrolidinylcarbonyl)-2,3,6,7,8,9,10,11,12,13,19,19a-dodecahydro-1*H*,5*H-*pyrrolo[2,1-*c*][1,4,7,12]benzoxatriazacyclopentadecin-2-yl]acetamide |
| **Ex.38** | 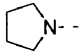 | 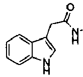 | *N*-[(2*S*,11*S*,19a*S*)-15-fluoro-7,12-dimethyl-5,8,13-trioxo-11-(1-pyrrolidinylcarbonyl)-2,3,6,7,8,9,10,11,12,13,19,19a-dodecahydro-1*H*,5*H-*pyrrolo[2,1-*c*][1,4,7,12]benzoxatriazacyclopentadecin-2-yl]-2-(1H-indol-3-yl)acetamide |
| **Ex.39** | 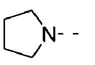 | 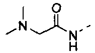 | *N*-[(2*S*,11*S*,19a*S*)-15-fluoro-7,12-dimethyl-5,8,13-trioxo-11-(1-pyrrolidinylcarbonyl)-2,3,6,7,8,9,10,11,12,13,19,19a-dodecahydro-1*H*,5*H-*pyrrolo[2,1-c][1,4,7,12]benzoxatriazacyclopentadecin-2-yl]-2-(dimethylamino)acetamide |
| **Ex.40** | 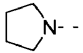 | 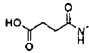 | 4-{[(2*S*,11*S*,19a*S*)-15-fluoro-7,12-dimethyl-5,8,13-trioxo-11-(1-pyrrolidinylcarbonyl)-2,3,6,7,8,9,10,11,12,13,19,19a-dodecahydro-1*H*,5*H-*pyrrolo[2,1-*c*][1,4,7,12]benzoxatriazacyclopentadecin-2-yl]amino}-4-oxobutanoic acid |
| **Ex.41** | 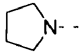 | 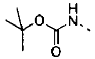 | tert-butyl *N*-[(2*S*,11*S*,19a*S*)-15-fluoro-7,12-dimethyl-5,8,13-trioxo-11-(1-pyrrolidinylcarbonyl)-2,3,6,7,8,9,10,11,12,13,19,19a-dodecahydro-1*H*,5*H-*pyrrolo[2,1-*c*][1,4,7,12]benzoxatriazacyclopentadecin-2-yl]carbamate |
| **Ex.42** | 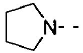 | NH₂ | (2*S*,11*S*,19a*S*)-2-amino-15-fluoro-7,12-dimethyl-11-(1-pyrrolidinylcarbonyl)-2,3,6,7,9,10,11,12,19,19a-decahydro-1*H*,5*H*-pyrrolo[2,1-c] [1,4,7,12]benzoxatriazacyclopentadecine-5,8,13-trione |
| **Ex.43** | 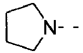 | 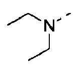 | (2*S*,11*S*,19a*S*)-2-(diethylamino)-15-fluoro-7,12-dimethyl-11-(1-pyrrolidinylcarbonyl)-2,3,6,7,9,10,11,12,19,19a-decahydro-1*H*,5*H*-pyrrolo[2,1-c][1,4,7,12]benzoxatriazacyclopentadecine-5,8,13-trione |
| **Ex.44** | 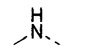 | 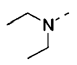 | (2*S*,11*S*,19a*S*)-2-(diethylamino)-15-fluoro-*N*,7,12-trimethyl-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecine-11-carboxamide |
| **Ex.45** | 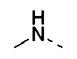 | 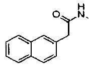 | (2*S*,11*S*,19a*S*)-15-fluoro-*N*,7,12-trimethyl-2-{[2-(2-naphthyl)acetyl]amino}-5,8,13-trioxo-2,3,6,7,8,9,10,11,12,13,19,19a-dodecahydro-1H,5H-pyrrolo[2,1-c][1,4,7,12]benzoxatriazacyclopentadecine-11-carboxamide |
| **Ex.46** | 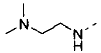 | 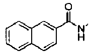 | (2S,11S,19aS)-N-[2-(dimethylamino)ethyl]-15-fluoro-7,12-dimethyl-2-(2-naphthoylamino)-5,8,13-trioxo-2,3,6,7,8,9,10,11,12,13,19,19a-dodecahydro-1*H*,5*H-*pyrrolo[2,1-c][1,4,7,12]benzoxatriazacyclopentadecine-11-carboxamide |
| **Ex.47** | 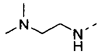 | 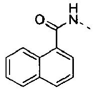 | *(2S,11S,19aS)-N-[2-(dimethylamino)ethyl]-15-fluoro-7,12-dimethyl-2-(1-*naphthoylamino)-5,8,13-trioxo-2,3,6,7,8,9,10,11,12,13,19,19a-dodecahydro-1*H*,5*H-*pyrrolo[2,1-*c*][1,4,7,12]benzoxatriazacyclopentadecine-11-carboxamide |
| **Ex.48** | 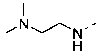 | 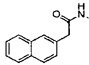 | (2*S*,11*S*,19a*S*)-*N*-[2-(dimethylamino)ethyl]-15-fluoro-7,12-dimethyl-2-{[2-(2-naphthyl)acetyl]amino}-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecine-11-carboxamide |
| **Ex.49** | 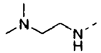 | 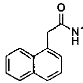 | (2*S*,11*S*,19a*S*)-*N*-[2-(dimethylamino)ethyl]-15-fluoro-7,12-dimethyl-2-{[2-(1-naphthyl)acetyl]amino}-5,8,13-trioxo-2,3,6,7,8,9,10,11,12,13,19,19a-dodecahydro-1H,5H-pyrrolo[2,1-c][1,4,7,12]benzoxatriazacyclopentadecine-11-carboxamide |
| **Ex.50** | 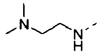 | 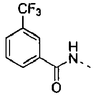 | (2*S*,11*S*,19a*S*)-N-[2-(dimethylamino)ethyl]-15-fluoro-7,12-dimethyl-5,8,13-trioxo-2-{[3-(trifluoromethyl)benzoyl]amino}-2,3,6,7,8,9,10,11,12,13,19,19a-dodecahydro-1*H*,5*H*-pyrrolo[2,1-*c*][1,4,7,12]benzoxatriazacyclopentadecine-11-carboxamide |
| **Ex.51** | 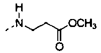 | NH₂ | methyl 3-({[(2*S*,11*S*,19a*S*)-2-amino-15-fluoro-7,12-dimethyl-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecin-11-yl]carbonyl}amino)propanoate |
| **Ex.52** | OCH₂Ph | 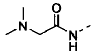 | benzyl (2*S*,11*S*,19a*S*)-2-{[2-(dimethylamino)acetyl]amino}-15-fluoro-7,12-dimethyl-5,8,13-trioxo-2,3,6,7,8,9,10,11,12,13,19,19a-dodecahydro-1*H*, 5*H*-pyrrolo [2,1-*c*][1,4,7,12]benzoxatriazacyclopentadecine-11-carboxylate |
| **Ex.53** | OCH₂Ph | 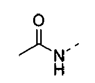 | benzyl (2*S*,11*S*,19a*S*)-2-(acetylamino)-15-fluoro-7,12-dimethyl-5,8,13-trioxo-2,3,6,7,8,9,10,11,12,13,19,19a-dodecahydro-1*H*,5*H*-pyrrolo[2,1-*c*] [1,4,7,12]benzoxatriazacyclopentadecine-11-carboxylate |
| **Ex.54** | OH | 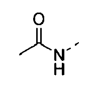 | (2*S*,11*S*,19a*S*)-2-(acetylamino)-15-fluoro-7,12-dimethyl-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecine-11-carboxylic acid |
| **Ex.55** | 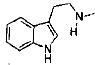 | NH₂ | (2*S*,11*S*,19a*S*)-2-amino-15-fluoro-*N*-[2-(1H-indol-3-yl)ethyl]-7,12-dimethyl-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecine-11-carboxamide |
| **Ex.482** | 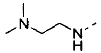 | 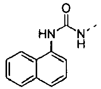 | (2*S*,11*S*,19a*S*)-*N*-[2-(dimethylamino)ethyl]-15-fluoro-7,12-dimethyl-2-{[(1-naphthylamino)carbonyl]amino}-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecine-11-carboxamide |
| **Ex.483** | 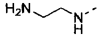 | 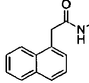 | (2*S*,11*S*,19a*S*)-*N*-(2-aminoethyl)-15-fluoro-7,12-dimethyl-2-{[2-(1-naphthyl)acetyl]amino}-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecine-11-carboxamide |
| **Ex.484** | 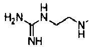 | 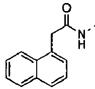 | (2*S*,11*S*,19a*S*)-*N*-(2-{[amino(imino)methyl]amino}ethyl)-15-fluoro-7,12-dimethyl-2-{[2-(1-naphthyl)acetyl]amino}-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecine-11-carboxamide |
| **Ex.485** | 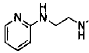 | 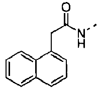 | (2*S*,11*S*,19a*S*)-15-fluoro-7,12-dimethyl-2-{[2-(1-naphthyl)acetyl]amino}-5,8,13-trioxo-*N*-[2-(2-pyridinylamino)ethyl]-2,3,6,7,8,9,10,11,12,13,19,19a-dodecahydro-1*H*,5*H*-pyrrolo[2,1-*c*][1,4,7,12]benzoxatriazacyclopentadecine-11-carboxamide |
| **Ex.486** | 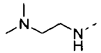 | 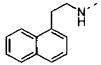 | (2*S*,11*S*,19a*S*)-*N*-[2-(dimethylamino)ethyl]-15-fluoro-7,12-dimethyl-2-{[2-(1-naphthyl)ethyl]amino}-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecine-11-carboxamide |
| **Ex.487** | 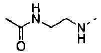 | 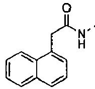 | (2*S*,11*S*,19a*S*)-*N*-[2-(acetylamino)ethyl]-15-fluoro-7,12-dimethyl-2-{[2-(1-naphthyl)acetyl]amino}-5,8,13-trioxo-2,3,6,7,8,9,10,11,12,13,19,19a-dodecahydro-1*H*,5H-pyrrolo[2,1-c][1,4,7,12]benzoxatriazacyclopentadecine-11-carboxamide |
| **Ex.488** | 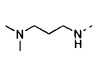 | 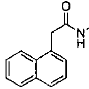 | (2*S*,11*S*,19a*S*)-*N*-[3-(dimethylamino)propyl]-15-fluoro-7,12-dimethyl-2-{[2-(1-naphthyl)acetyl]amino}-5,8,13-trioxo-2,3,6,7,8,9,10,11,12,13,19,19a-dodecahydro-1*H*,5*H*-pyrrolo[2,1-c][1,4,7,12]benzoxatriazacyclopentadecine-11-carboxamide |
| **Ex.489** | 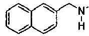 | 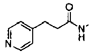 | (2*S*,11*S*,19a*S*)-15-fluoro-7,12-dimethyl-*N*-(2-naphthylmethyl)-5,8,13-trioxo-2-{[3-(4-pyridinyl)propanoyl]amino}-2,3,6,7,8,9,10,11,12,13,19,19a-dodecahydro-1*H*,5*H-*pyrrolo[2,1-c][1,4,7,12]benzoxatriazacyclopentadecine-11-carboxamide |
| **Ex.490** | 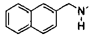 | 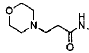 | (2S,11S,19aS)-15-fluoro-7,12-dimethyl-2-[(3-morpholinopropanoyl)amino]-N-(2-naphthylmethyl)-5,8,13-trioxo-2,3,6,7,8,9,10,11,12,13,19,19a-dodecahydro-1*H*,5*H-*pyrrolo[2,1-c][1,4,7,12]benzoxatriazacyclopentadecine-11-carboxamide |
| **Ex.491** | 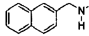 | 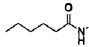 | (2*S*,11*S*,19a*S*)-15-fluoro-2-(hexanoylamino)-7,12-dimethyl-*N*-(2-naphthylmethyl)-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecine-11-carboxamide |
| **Ex.492** | 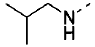 | 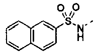 | (2*S*,11*S*,19a*S*)-15-fluoro-*N*-isobutyl-7,12-dimethyl-2-[(2-naphthylsulfonyl)amino]-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecine-11-carboxamide |
| **Ex.493** | 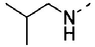 | 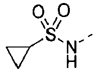 | (2*S*,11*S*,19a*S*)-2-[(cyclopropylsulfonyl)amino]-15-fluoro-*N*-isobutyl-7,12-dimethyl-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecine-11-carboxamide |
| **Ex.494** | 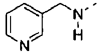 | 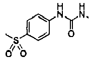 | (2*S*,11*S*,19a*S*)-15-fluoro-7,12-dimethyl-2-({[4-(methylsulfonyl)anilino]carbonyl}amino)-5,8,13-trioxo-*N*-(3-pyridinylmethyl)-2,3,6,7,8,9,10,11,12,13,19,19a-dodecahydro-1*H*,5*H*-pyrrolo[2,1-*c*][1,4,7,12]benzoxatriazacyclopentadecine-11-carboxamide |
| **Ex.495** | 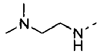 | 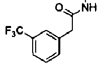 | (2*S*,11*S*,19a*S*)-*N*-[2-(dimethylamino)ethyl]-15-fluoro-7,12-dimethyl-5,8,13-trioxo-2-({2-[3-(trifluoromethyl)phenyl]acetyl}amino)-2,3,6,7,8,9,10,11,12,13,19,19a-dodecahydro-1*H*,5*H*-pyrrolo[2,1-*c*][1,4,7,12]benzoxatriazacyclopentadecine-11-carboxamide |
| **Ex.496** | 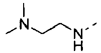 | 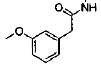 | (2*S*,11*S*,19a*S*)-*N*-[2-(dimethylamino)ethyl]-15-fluoro-2-{[2-(3-methoxyphenyl)acetyl]amino}-7,12-dimethyl-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecine-11-carboxamide |
| **Ex.497** | 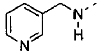 | 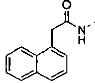 | (2*S*,11*S*,19a*S*)-15-fluoro-7,12-dimethyl-2-{[2-(1-naphthyl)acetyl]amino}-5,8,13-trioxo-*N*-(3-pyridinylmethyl)-2,3,6,7,8,9,10,11,12,13,19,19a-dodecahydro-1*H*,5*H-*pyrrolo[2,1-*c*][1,4,7,12]benzoxatriazacyclopentadecine-11-carboxamide |
| **Ex.498** | OCH₂Ph | 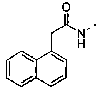 | benzyl (2*S*,11*S*,19a*S*)-15-fluoro-7,12-dimethyl-2-{[2-(1-naphthyl)acetyl]amino}-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecine-11-carboxylate |
| **Ex.499** | OH | 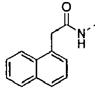 | (2*S*,11*S*,19a*S*)-15-fluoro-7,12-dimethyl-2-{[2-(1-naphthyl)acetyl]amino}-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecine-11-carboxylic acid |
| **Ex.500** | 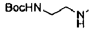 | 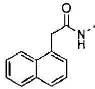 | *tert*-butyl *N*-(2-{[((2*S*,11*S*,19a*S*)-15-fluoro-7,12-dimethyl-2-{[2-(1-naphthyl)acetyl]amino}-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecin-11-yl)carbonyl]amino}ethyl)carbamate |
| **Ex.501** | 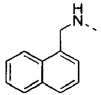 | 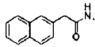 | (2*S*,11*S*,19a*S*)-15-fluoro-7,12-dimethyl-2-{[2-(2-naphthyl)acetyl]amino}-*N*-(1-naphthylmethyl)-5,8,13-trioxo-2,3,6,7,8,9,10,11,12,13,19,19a-dodecahydro-1*H*,5*H-*pyrrolo[2,1-*c*][1,4,7,12]benzoxatriazacyclopentadecine-11-carboxamide |
| **Ex.502** | 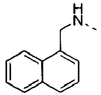 | 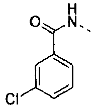 | (2S,11S,19aS)-2-[(3-chlorobenzoyl)amino]-15-fluoro-7,12-dimethyl-*N*-(1-naphthylmethyl)-5,8,13-trioxo-2,3,6,7,8,9,10,11,12,13,19,19a-dodecahydro-1*H*,5*H-*pyrrolo[2,1-c][1,4,7,12]benzoxatriazacyclopentadecine-11-carboxamide |
| **Ex.503** | 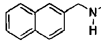 | 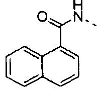 | (2*S*,11*S*,19a*S*)-15-fluoro-7,12-dimethyl-2-(1-naphthoylamino)-*N*-(2-naphthylmethyl)-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecine-11-carboxamide |
| **Ex.590** | OH | NH₂ | (2*S*,11*S*,19a*S*)-2-amino-15-fluoro-7,12-dimethyl-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecine-11-carboxylic acid |
| **Ex.591** | OH | 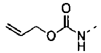 | (2*S*,11*S*,19a*S*)-2-{[(allyloxy)carbonyl]amino}-15-fluoro-7,12-dimethyl-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecine-11-carboxylic acid |

**Table 22a: Examples of Core 04 (Ex.56-Ex.84, continued on the following pages)**

| **No** | **R⁵⁴** | **R²** | **Starting material** | **General Procedure** | **Reagent** | **Purification Method** | **Yield (isolated salt)** |
|---|---|---|---|---|---|---|---|
| **Ex.56-Ex.57:** *cf. experimental description* | | | | | | | |
| **Ex.58** | 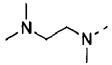 | 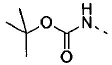 | **Ex.57** | L.2 | N,N,N'-trimetylethylene-diamine | FC (CH₂Cl₂/MeOH/aq. NH₃) | 86% |
| **Ex.59** | 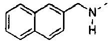 | 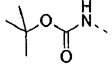 | **Ex.57** | L.2 | 2-naphthylmethylamine | FC (CH₂Cl₂/MeOH) | 91% |
| **Ex.60** | 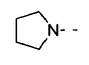 | 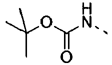 | **Ex.57** | L.2 | pyrrolidine | FC (CH₂Cl₂/MeOH) | 79% |
| **Ex.61** | 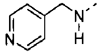 | 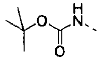 | **Ex.57** | L.2 | 4-(aminomethyl)-pyridine | FC (CH₂Cl₂/MeOH) | 81% |
| **Ex.62** | 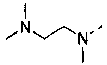 | NH₂ | **Ex.58** | J | HCl-dioxane | crude product | 98% (HCl salt) |
| **Ex.63** | 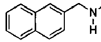 | NH₂ | **Ex.59** | J | HCl-dioxane | crude product | 76% (HCl salt) |
| **Ex.64** | 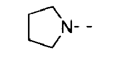 | NH₂ | **Ex.60** | J | HCl-dioxane | crude product | 94% (HCl salt) |
| **Ex.65** | 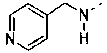 | NH₂ | **Ex.61** | J | HCl-dioxane | crude product | 91% (HCl salt) |
| **Ex.66** | 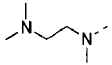 | 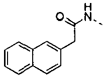 | **Ex.62** | L.1.3 | 2-naphthylacetic acid | prep. HPLC, method 1 | 67% (TFA salt) |
| **Ex.67** | 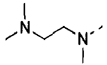 | 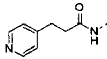 | **Ex.62** | L.1.2 | 3-(pyridine-4-yl)propanoic acid | prep. HPLC, method 2 | 56% |
| **Ex. 68** | 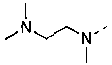 | 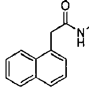 | **Ex.62** | L.1.2 | 1-naphthylacetic acid (1.5 equiv.) | prep. HPLC, method 1 | 24% (TFA salt) |
| **Ex.69** | 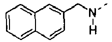 | 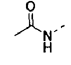 | **Ex.63** | L.1.1 | acetic anhydride (5 equiv.) | prep. HPLC, method 1 | 75% |
| **Ex.70** | 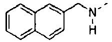 | 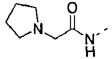 | **Ex.63** | L.1.3 | 1-pyrrolidinacetic acid | prep. HPLC, method 1 | 62% (TFA salt) |
| **Ex.71** | 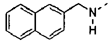 | 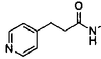 | **Ex.63** | L.1.2 | 3-(pyridine-4-yl)propanoic acid | prep. HPLC, method 1 | 22% (TFA salt) |
| **Ex.72** | 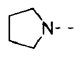 | 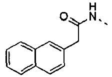 | **Ex.64** | L.1.3 | 2-naphthylacetic acid | prep. HPLC, method 2 | 51% |
| **Ex.73** | 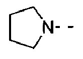 | 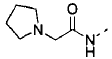 | **Ex.64** | L.1.2 | 1-pyrrolidinacetic acid (1.7 equiv.) | prep. HPLC, method 1 | 39% (TFA salt) |
| **Ex.74** | 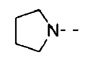 | 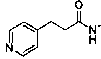 | **Ex.64** | L.1.2 | 3-(pyridine-4-yl)propanoic acid | prep. HPLC, method 2 | 29% |
| **Ex.75** | 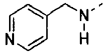 | 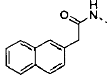 | **Ex.65** | L.1.3 | 2-naphthylacetic acid | prep. HPLC, method 1 | 37% (TFA salt) |
| **Ex.76** | 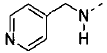 | 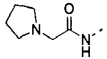 | **Ex.65** | L.1.2 | 1-pyrrolidinacetic acid (1.7 equiv.) | prep. HPLC, method 2 | 45% |
| **Ex.77** | 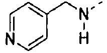 | 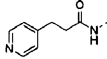 | **Ex.65** | L.1.2 | 3-(pyridine-4-yl)propanoic acid | prep. HPLC, method 2 | 45% |
| **Ex.78** | 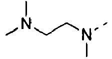 | 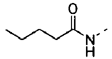 | **Ex.62** | L.1.1 | valeroyl chloride (1.6 equiv.) | prep. HPLC, method 1 | 56% |
| **Ex.79** | 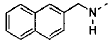 | 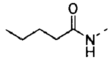 | **Ex.63** | L.1.1 | valeroyl chloride (1.6 equiv.) | prep. HPLC, method 1 | 77% |
| **Ex.80** | 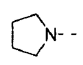 | 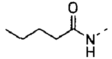 | **Ex.64** | L.1.1 | valeroyl chloride (1.3 equiv.) | prep. HPLC, method 1 | 46% |
| **Ex.81** | 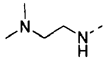 | NH₂ | **Ex.84** | J | HCl-dioxane | crude product | 99% (HCl salt) |
| **Ex.82** | 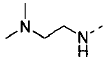 | 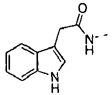 | **Ex.81** | L.1.3 | 3-indoleacetic acid (1.5 equiv.) HATU (1.5 equiv.) HOAt (1.5 equiv.) i-Pr₂NEt (5 equiv.) | prep. HPLC, method 1, then FC (CH₂Cl₂/MeOH/ aq. NH₃) | 15% |
| **Ex.83** | 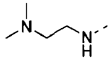 | 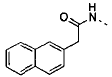 | **Ex.81** | L.1.3 | 2-naphthylacetic acid (1.2 equiv.) | FC (CH₂Cl₂/MeOH/aq. NH₃), then prep. HPLC, method 1 | 58% |
| **Ex.84** | 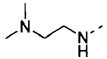 | 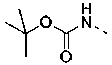 | **Ex.57** | L.2 | 2-dimethylaminoethylamine | FC (CH₂Cl₂/MeOH) | 89% |

**Table 22b: Examples of Core 04 (Ex.56-Ex.84; continued on the following pages)**

| **No** | **R⁵⁴** | **R²** | **FORMULA** | **MONOISOTOPIC MASS** | **Rt (purity at 220nm)** | **[M+H]⁺ found** | **LC-MS-Method** |
|---|---|---|---|---|---|---|---|
| **Ex.56-Ex.57:** *cf. experimental description* | | | | | | | |
| **Ex.58** | 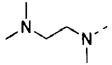 | 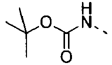 | C31H47FN607 | 634.4 | 1.44 (91) | 635.5 | Method 2 |
| **Ex.59** | 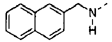 | 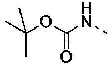 | C37H44FN5O7 | 689.3 | 1.89 (87) | 690.5 | Method 2 |
| **Ex.60** | 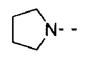 | 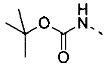 | C30H42FN5O7 | 603.3 | 1.67 (84) | 604.4 | Method 2 |
| **Ex.61** | 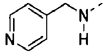 | 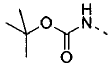 | C32H41FN6O7 | 640.3 | 1.42 (92) | 641.4 | Method 2 |
| **Ex.62** | 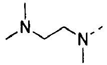 | NH₂ | C26H39FN6O5 | 534.3 | 0.97 (91) | 535.4 | Method 2 |
| **Ex.63** | 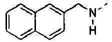 | NH₂ | C32H36FN5O5 | 589.3 | 1.53 (95) | 590.4 | Method 2 |
| **Ex.64** | 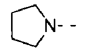 | NH₂ | C25H34FN5O5 | 503.3 | 1.23 (82) | 504.3 | Method 2 |
| **Ex.65** | 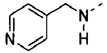 | NH₂ | C27H33FN6O5 | 540.3 | 0.97 (97) | 541.4 | Method 2 |
| **Ex.66** | 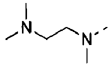 | 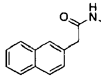 | C38H47FN6O6 | 702.4 | 1.51 (97) | 703.5 | Method 2 |
| **Ex.67** | 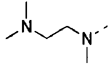 | 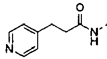 | C34H46FN7O6 | 667.4 | 1.08 (94) | 668.5 | Method 2 |
| **Ex.68** | 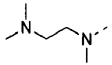 | 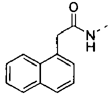 | C38H47FN6O6 | 702.4 | 1.51 (88) | 703.5 | Method 2 |
| **Ex.69** | 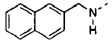 | 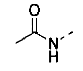 | C34H38FN5O6 | 631.3 | 1.66 (90) | 632.3 | Method 2 |
| **Ex.70** | 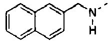 | 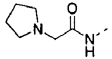 | C38H45FN6O6 | 700.3 | 1.56 (95) | 701.5 | Method 2 |
| **Ex.71** | 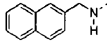 | 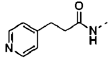 | C40H43FN6O6 | 722.3 | 1.55 (93) | 723.5 | Method 2 |
| **Ex.72** | 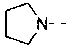 | 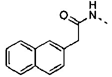 | C37H42FN5O6 | 671.3 | 1.73 (87) | 672.4 | Method 2 |
| **Ex.73** | 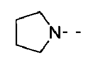 | 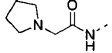 | C31H43FN6O6 | 614.3 | 1.28 (90) | 615.4 | Method 2 |
| **Ex.74** | 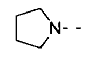 | 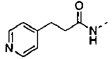 | C33H41FN6O6 | 636.3 | 1.29 (91) | 637.4 | Method 2 |
| **Ex.75** | 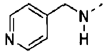 | 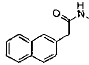 | C39H41FN6O6 | 708.3 | 1.50 (92) | 709.4 | Method 2 |
| **Ex.76** | 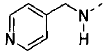 | 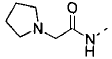 | C33H42FN7O6 | 651.3 | 1.07 (91) | 652.4 | Method 2 |
| **Ex.77** | 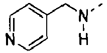 | 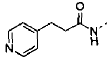 | C35H40FN7O6 | 673.3 | 1.07 (90) | 674.5 | Method 2 |
| **Ex.78** | 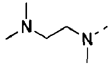 | 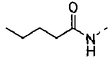 | C31H47FN6O6 | 618.4 | 1.33(98) | 619.4 | Method 2 |
| **Ex**.**79** | 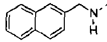 | 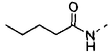 | C37H44FN5O6 | 673.3 | 1.81 (91) | 674.4 | Method 2 |
| **Ex**.**80** | 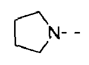 | 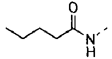 | C30H42FN5O6 | 587.3 | 1.56 (93) | 588.4 | Method 2 |
| **Ex.81** | 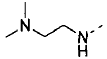 | NH₂ | C25H37FN6O5 | 520.3 | 1.10 (88) | 521.4 | Method 2 |
| **Ex.82** | 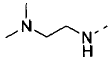 | 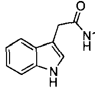 | C35H44FN7O6 | 677.3 | 1.36 (93) | 678.5 | Method 2 |
| **Ex**.**83** | 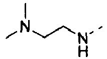 | 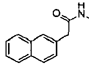 | C37H45FN6O6 | 688.3 | 1.49 (93) | 689.5 | Method 2 |
| **Ex.84** | 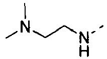 | 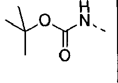 | C30H45FN6O7 | 620.3 | 1.38 (87) | 621.5 | Method 2 |

**Table 22c: Examples of Core 04 (Ex.56-Ex.84; continued on the following pages)**

| **No** | **R⁵⁴** | **R²** | **IUPAC name** |
|---|---|---|---|
| **Ex.56** | OCH₂Ph | 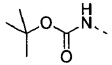 | benzyl (2*R*,11*S*,19a*S*)-2-[(*tert*-butoxycarbonyl)amino]-15-fluoro-7,12-dimethyl-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecine-11-carboxylate |
| **Ex.57** | OH | 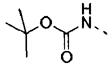 | (2*R*,11*S*,19a*S*)-2-[(*tert*-butoxycarbonyl)amino]-15-fluoro-7,12-dimethyl-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecine-11-carboxylic acid |
| **Ex.58** | 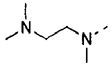 | 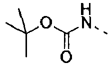 | tert-butyl *N*-((2*R*,11*S*,19a*S*)-11-{[[2-(dimethylamino)ethyl](methyl)amino]carbonyl}-15-fluoro-7,12-dimethyl-5,8,13-trioxo-2,3,6,7,8,9,10,11,12,13,19,19a-dodecahydro-1*H*,5*H*-pyrrolo[2,1-c][1,4,7,12]benzoxatriazacyclopentadecin-2-yl)carbamate |
| **Ex.59** | 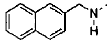 | 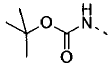 | *tert*-butyl *N*-((2*R*,11*S*,19a*S*)-15-fluoro-7,12-dimethyl-11-{[(2-naphthylmethyl)amino]carbonyl}-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecin-2-yl)carbamate |
| **Ex.60** | 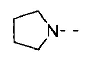 | 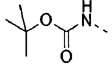 | tert-butyl *N*-[(2*R*,11*S*,19a*S*)-15-fluoro-7,12-dimethyl-5,8,13-trioxo-11-(1-pyrrolidinylcarbonyl)-2,3,6,7,8,9,10,11,12,13,19,19a-dodecahydro-1*H*,5*H-*pyrrolo[2,1-*c*][1,4,7,12]benzoxatriazacyclopentadecin-2-yl]carbamate |
| **Ex.61** | 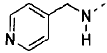 | 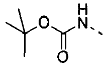 | tert-butyl *N*-((*2R*,11*S*,19a*S*)-15-fluoro-7,12-dimethyl-5,8,13-trioxo-11-{[(4-pyridinylmethyl)amino]carbonyl}-2,3,6,7,8,9,10,11,12,13,19,19a-dodecahydro-1*H*,5*H*-pyrrolo[2,1-*c*][1,9,7,12]benzoxatriazacyclopentadecin-2-yl)carbamate |
| **Ex.62** | 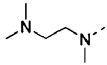 | NH₂ | (*2R*,11*S*,19a*S*)-2-amino-*N*-[2-(dimethylamino)ethyl]-15-fluoro-*N*,7,12-trimethyl-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecine-11-carboxamide |
| **Ex.63** | 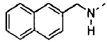 | NH₂ | (2R,11S,19aS)-2-amino-15-fluoro-7,12-dimethyl-N-(2-naphthylmethyl)-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecine-11-carboxamide |
| **Ex.64** | 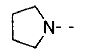 | NH₂ | (2*R*,11*S*,19a*S*)-2-amino-15-fluoro-7,12-dimethyl-11-(1-pyrrolidinylcarbonyl)-2,3,6,7,9,10,11,12,19,19a-decahydro-1*H*,5*H*-pyrrolo[2,1-c][1,4,7,12]benzoxatriazacyclopentadecine-5,8,13-trione |
| **Ex.65** | 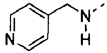 | NH₂ | (2*R*,11*S*,19a*S*)-2-amino-15-fluoro-7,12-dimethyl-5,8,13-trioxo-*N*-(4-pyridinylmethyl)-2,3,6,7,8,9,10,11,12,13,19,19a-dodecahydro-1*H*,5*H*-pyrrolo[2,1-c][1,4,7,12]benzoxatriazacyclopentadecine-11-carboxamide |
| **Ex.66** | 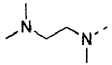 | 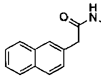 | (2*R*,11*S*,19a*S*)-*N*-[2-(dimethylamino)ethyl]-15-fluoro-*N*,7,12-trimethyl-2-{[2-(2-naphthyl)acetyl]amino}-5,8,13-trioxo-2,3,6,7,8,9,10,11,12,13,19,19a-dodecahydro-1*H*,5*H*-pyrrolo[2,1-c][1,4,7,12]benzoxatriazacyclopentadecine-11-carboxamide |
| **Ex.67** | 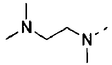 | 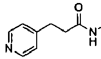 | (2*R*,11*S*,19a*S*)-*N*-[2-(dimethylamino)ethyl]-15-fluoro-*N*,7,12-trimethyl-5,8,13-trioxo-2-{[3-(4-pyridinyl)propanoyl]amino}-2,3,6,7,8,9,10,11,12,13,19,19a-dodecahydro-1*H*,5*H*-pyrrolo[2,1-*c*][1,4,7,12]benzoxatriazacyclopentadecine-11-carboxamide |
| **Ex.68** | 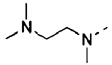 | 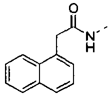 | (2*R*,11*S*,19a*S*)-*N*-[2-(dimethylamino)ethyl]-15-fluoro-*N*,7,12-trimethyl-2-{[2-(1-naphthyl)acetyl]amino}-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecine-11-carboxamide |
| **Ex.69** | 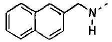 | 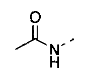 | (2*R*,11*S*,19a*S*)-2-(acetylamino)-15-fluoro-7,12-dimethyl-N-(2-naphthylmethyl)-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecine-11-carboxamide |
| **Ex.70** | 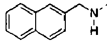 | 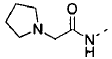 | (2*R*,11*S*,19a*S*)-15-fluoro-7,12-dimethyl-*N*-(2-naphthylmethyl)-5,8,13-trioxo-2-{[2-(1-pyrrolidinyl)acetyl]amino}-2,3,6,7,8,9,10,11,12,13,19,19a-dodecahydro-1*H*,5*H-*pyrrolo[2,1-*c*][1,4,7,12]benzoxatriazacyclopentadecine-11-carboxamide |
| **Ex.71** | 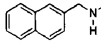 | 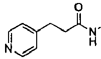 | (2*R*,11*S*,19a*S*)-15-fluoro-7,12-dimethyl-*N*-(2-naphthylmethyl)-5,8,13-trioxo-2-{[3-(4-pyridinyl)propanoyl]amino}-2,3,6,7,8,9,10,11,12,13,19,19a-dodecahydro-1*H*,5*H-*pyrrolo[2,1-c][1,4,7,12]benzoxatriazacyclopentadecine-11-carboxamide |
| **Ex.72** | 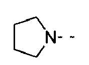 | 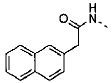 | *N*-[(2*R*,11*S*,19a*S*)-15-fluoro-7,12-dimethyl-5,8,13-trioxo-11-(1-pyrrolidinylcarbonyl)-2,3,6,7,8,9,10,11,12,13,19,19a-dodecahydro-1*H*,5*H-*pyrrolo[2,1-*c*][1,4,7,12]benzoxatriazacyclopentadecin-2-yl]-2-(2-naphthyl)acetamide |
| **Ex.73** | 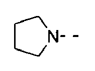 | 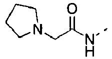 | *N*-[(2*R*,11*S*,19a*S*)-15-fluoro-7,12-dimethyl-5,8,13-trioxo-11-(1-pyrrolidinylcarbonyl)-2,3,6,7,8,9,10,11,12,13,19,19a-dodecahydro-1*H*,5*H-*pyrrolo[2,1-*c*][1,4,7,12]benzoxatriazacyclopentadecin-2-yl]-2-(1-pyrrolidinyl)acetamide |
| **Ex.74** | 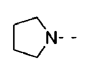 | 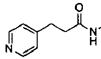 | *N*-[(2*R*,11*S*,19a*S*)-15-fluoro-7,12-dimethyl-5,8,13-trioxo-11-(1-pyrrolidinylcarbonyl)-2,3,6,7,8,9,10,11,12,13,19,19a-dodecahydro-1*H*,5*H-*pyrrolo[2,1-*c*][1,9,7,12]benzoxatriazacyclopentadecin-2-yl]-3-(4-pyridinyl)propanamide |
| **Ex.75** | 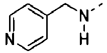 | 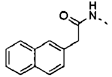 | (2*R*,11*S*,19a*S*)-15-fluoro-7,12-dimethyl-2-{[2-(2-naphthyl)acetyl]amino}-5,8,13-trioxo-*N*-(4-pyridinylmethyl)-2,3,6,7,8,9,10,11,12,13,19,19a-dodecahydro-1*H*,5*H-*pyrrolo[2,1-*c*][1,4,7,12]benzoxatriazacyclopentadecine-11-carboxamide |
| **Ex.76** | 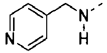 | 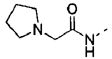 | (2*R*,11*S*,19a*S*)-15-fluoro-7,12-dimethyl-5,8,13-trioxo-*N*-(4-pyridinylmethyl)-2-{[2-(1-pyrrolidinyl)acetyl]amino}-2,3,6,7,8,9,10,11,12,13,19,19a-dodecahydro-1*H*,5*H*-pyrrolo[2,1-*c*][1,4,7,12]benzoxatriazacyclopentadecine-11-carboxamide |
| **Ex.77** | 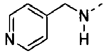 | 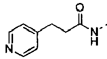 | (2*R*,11*S*,19a*S*)-15-fluoro-7,12-dimethyl-5,8,13-trioxo-*N*-(4-pyridinylmethyl)-2-{[3-(4-pyridinyl)propanoyl]amino}-2,3,6,7,8,9,10,11,12,13,19,19a-dodecahydro-1*H*,5*H*-pyrrolo[2,1-*c*][1,4,7,12]benzoxatriazacyclopentadecine-11-carboxamide |
| **Ex.78** | 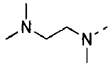 | 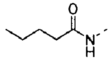 | (2*R*,11*S*,19a*S*)-*N*-[2-(dimethylamino)ethyl]-15-fluoro-*N*,7,12-trimethyl-5,8,13-trioxo-2-(pentanoylamino)-2,3,6,7,8,9,10,11,12,13,19,19a-dodecahydro-1*H*,5*H-*pyrrolo[2,1-*c*][1,4,7,12]benzoxatriazacyclopentadecine-11-carboxamide |
| **Ex.79** | 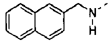 | 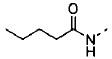 | (2*R*,11*S*,19a*S*)-15-fluoro-7,12-dimethyl-*N*-(2-naphthylmethyl)-5,8,13-trioxo-2-(pentanoylamino)-2,3,6,7,8,9,10,11,12,13,19,19a-dodecahydro-1*H*,5*H*-pyrrolo[2,1-c][1,4,7,12]benzoxatriazacyclopentadecine-11-carboxamide |
| **Ex.80** | 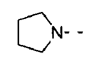 | 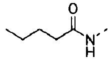 | *N*-[(2*R*,11*S*,19a*S*)-15-fluoro-7,12-dimethyl-5,8,13-trioxo-11-(1-pyrrolidinylcarbonyl)-2,3,6,7,8,9,10,11,12,13,19,19a-dodecahydro-1*H*,5*H-*pyrrolo[2,1-*c*][1,4,7,12]benzoxatriazacyclopentadecin-2-yl]pentanamide |
| **Ex.81** | 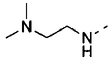 | NH₂ | (2*R*,11*S*,19a*S*)-2-amino-*N*-[2-(dimethylamino)ethyl]-15-fluoro-7,12-dimethyl-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecine-11-carboxamide |
| **Ex.82** | 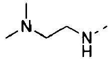 | 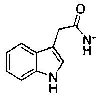 | (2*R*,11*S*,19a*S*)-*N*-[2-(dimethylamino)ethyl]-15-fluoro-2-{[2-(1*H*-indol-3-yl)acetyl]amino}-7,12-dimethyl-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecine-11-carboxamide |
| **Ex.83** | 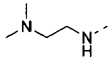 | 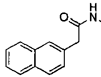 | (2*R*,11*S*,19a*S*)-*N*-[2-(dimethylamino)ethyl]-15-fluoro-7,12-dimethyl-2-{[2-(2-naphthyl)acetyl]amino}-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecine-11-carboxamide |
| **Ex.84** | 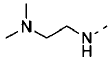 | 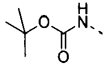 | tert-butyl *N*-[(2R,11*S*,19a*S*)-11-({[2-(dimethylamino)ethyl]amino}carbonyl)-15-fluoro-7,12-dimethyl-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecin-2-yl]carbamate |

**Table 23a: Examples of Core 05 (Ex.85-Ex.103; continued on the following pages)**

| **No** | **R²** | **R⁵⁴** | **Starting material** | **General Procedure** | **Reagent** | **Purification Method** | **Yield (isolated salt)** |
|---|---|---|---|---|---|---|---|
| **Ex.85-Ex.86:** *cf. experimental description* | | | | | | | |
| **Ex.87** | 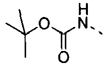 | 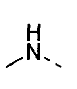 | **Ex.86** | L.2 | methylamine-hydrochloride (10 equiv.) HATU (1.5 equiv.) HOAt (1.5 equiv.) i-Pr₂NEt (13 equiv.) | FC (CH₂Cl₂/MeOH) | 76% |
| **Ex. 88** | 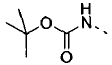 | 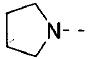 | **Ex.86** | L.2 | pyrrolidine | FC (CH₂Cl₂/MeOH) then prep. HPLC, method 2 | 64% |
| **Ex.89** | 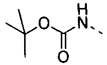 | 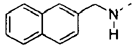 | **Ex.86** | L.2 | 2-naphthylmethylamine | FC (EtOAc/MeOH) | 93% |
| **Ex.90** | NH₂ | 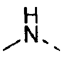 | **Ex.87** | J | HCl-dioxane | crude product | 86% (HCl salt) |
| **Ex.91** | NH₂ | 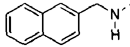 | **Ex.89** | J | HCl-dioxane | crude product | 98% (HCl salt) |
| **Ex.92** | NH₂ | 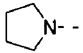 | **Ex.88** | J | HCl-dioxane | crude product | 94% (HCl salt) |
| **Ex.93** | 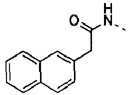 | 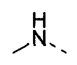 | **Ex.90** | L.1.2 | 2-naphthylacetic acid | prep. HPLC, method 3 | 66% |
| **Ex.94** | 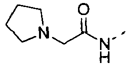 | 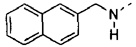 | **Ex.91** | L.1.2 | 1-pyrrolidineacetic acid (1.7 equiv.) | prep. HPLC, method 1 | 46% (TFA salt) |
| **Ex.95** | 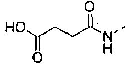 | 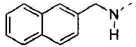 | **Ex.91** | L.1.1 | succinic anhydride (1.05 equiv.) | prep. HPLC, method 2 | 47% (NH4⁺ salt) |
| **Ex.96** | 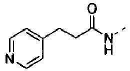 | 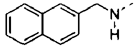 | **Ex. 91** | L.1.2 | 3-(pyridine-4-yl)propanoic acid (3.7 equiv.) | FC (CH₂Cl₂/MeOH) | 74% |
| **Ex.97** | 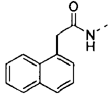 | 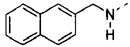 | **Ex.91** | L.1.2 | 1-naphthylacetic acid (1.4 equiv.) | prep. HPLC, method 3 | 72% |
| **Ex.98** | 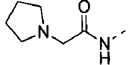 | 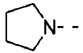 | **Ex.92** | L.1.2 | 1-pyrrolidineacetic acid | prep. HPLC, method 1 | 68% (TFA salt) |
| **Ex.99** | 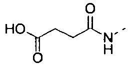 | 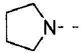 | **Ex.92** | L.1.1 | succinic anhydride (1.05 equiv.) | prep. HPLC, method 2 | 40% (NH4⁺ salt) |
| **Ex.10 0** | 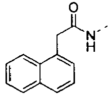 | 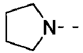 | **Ex.92** | L.1.2 | 1-naphthylacetic acid | FC (EtOAc/MeOH) | 83% |
| **Ex.10 1** | 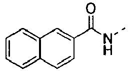 | 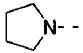 | **Ex.92** | L.1.1 | 2-naphthoyl chloride (1.6 equiv.) | FC (EtOAc/MeOH) | 84% |
| **Ex.10 2** | 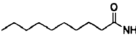 | 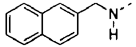 | **Ex.91** | L.1.1 | decanoyl chloride (4.1 equiv.) pyridine (15 equiv.) | prep. HPLC, method 3 | 64% |
| **Ex.10 3** | 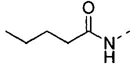 | 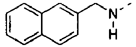 | **Ex.91** | L.1.1 | valeroyl chloride (2.0 equiv.) | prep. HPLC, method 3 | 87% |

**Table 23b: Examples of Core 05 (Ex.85-Ex.103; continued on the following pages)**

| **No** | **R²** | **R⁵⁴** | **FORMULA** | **MONOISOTOPIC MASS** | **Rt (purity at 220nm)** | **[M+H]⁺ found** | **LC-MS-Method** |
|---|---|---|---|---|---|---|---|
| **Ex.85-Ex.86:** *cf. experimental description* | | | | | | | |
| **Ex.87** | 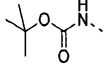 | 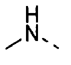 | C27H38FN5O7 | 563.3 | 1.52 (78) | 564.4 | Method 2 |
| **Ex.88** | 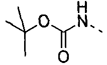 | 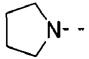 | C30H42FN5O7 | 603.3 | 1.59 (64), 1.63 (27) | 604.4/604.4 | Method 2 |
| **Ex.89** | 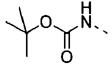 | 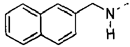 | C37H44FN5O7 | 689.3 | 1.89 (97) | 690.5 | Method 2 |
| **Ex.90** | NH₂ | 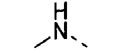 | C22H30FN5O5 | 463.2 | 1.02 (95) | 464.3 | Method 2 |
| **Ex.91** | NH₂ | 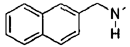 | C32H36FN5O5 | 589.3 | 1.51 (98) | 590.4 | Method 2 |
| **Ex.92** | NH₂ | 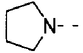 | C25H34FN5O5 | 503.3 | 1.20 (97) | 504.3 | Method 2 |
| **Ex.93** | 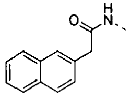 | 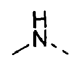 | C34H38FN5O6 | 631.3 | 1.65 (98) | 632.3 | Method 2 |
| **Ex.94** | 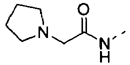 | 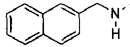 | C38H45FN6O6 | 700.3 | 1.55 (100) | 701.5 | Method 2 |
| **Ex.95** | 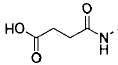 | 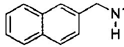 | C36H40FN5O8 | 689.3 | 1.65 (98) | 690.5 | Method 2 |
| **Ex.96** | 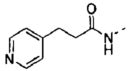 | 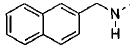 | C40H43FN6O6 | 722.3 | 1.51 (96) | 723.5 | Method 2 |
| **Ex.97** | 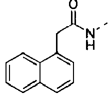 | 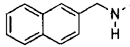 | C44H44FN5O6 | 757.3 | 1.96 (92) | 758.5 | Method 2 |
| **Ex.98** | 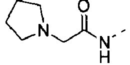 | 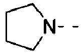 | C31H93FN6O6 | 614.3 | 1.25 (98) | 615.3 | Method 2 |
| **Ex.99** | 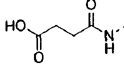 | 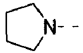 | C29H38FN5O8 | 603.3 | 1.34 (100) | 604.4 | Method 2 |
| **Ex.100** | 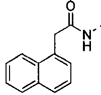 | 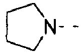 | C37H42FN5O6 | 671.3 | 1.73 (85) | 672.4 | Method 3 |
| **Ex.101** | 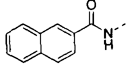 | 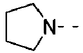 | C36H40FN5O6 | 657.3 | 1.73 (98) | 658.4 | Method 2 |
| **Ex.102** | 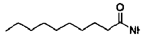 | 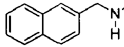 | C42H54FN5O6 | 743.4 | 2.16 (95) | 744.6 | Method 3 |
| **Ex.103** | 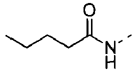 | 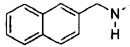 | C37H44FN5O6 | 673.3 | 1.83 (96) | 674.5 | Method 2 |

**Table 23c: Examples of Core 05 (Ex.85-Ex.103; continued on the following pages)**

| **No** | **R²** | **R⁵⁴** | **IUPAC name** |
|---|---|---|---|
| **Ex.85** | 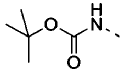 | OCH₂Ph | benzyl (2*S*,11*R*,19a*S*)-2-[(*tert*-butoxycarbonyl)amino]-15-fluoro-7,12-dimethyl-5,8,13-trioxo-2,3,6,7,8,9,10,11,12,13,19,19a-dodecahydro-1*H*,5*H-*pyrrolo[2,1-*c*][1,4,7,12]benzoxatriazacyclopentadecine-11-carboxylate |
| **Ex.86** | 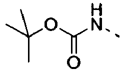 | OH | (2S,11R,19aS)-2-[(tert-butoxycarbonyl)amino]-15-fluoro-7,12-dimethyl-5,8,13-trioxo-2,3,6,7,8,9,10,11,12,13,19,19a-dodecahydro-1*H*,5*H-*pyrrolo[2,1-*c*][1,4,7,12]benzoxatriazacyclopentadecine-11-carboxylic acid |
| **Ex.87** | 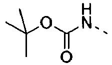 | 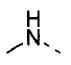 | tert-butyl *N*-{(2*S*,11*R*,19a*S*)-15-fluoro-7,12-dimethyl-11-[(methylamino)carbonyl]-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecin-2-yl}carbamate |
| **Ex.88** | 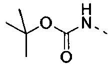 | 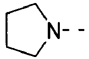 | tert-butyl *N*-[(2*S*,11*R*,19a*S*)-15-fluoro-7,12-dimethyl-5,8,13-trioxo-11-(1-pyrrolidinylcarbonyl)-2,3,6,7,8,9,10,11,12,13,19,19a-dodecahydro-1*H*,5*H-*pyrrolo[2,1-*c*][1,4,7,12]benzoxatriazacyclopentadecin-2-yl]carbamate |
| **Ex.89** | 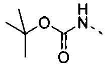 | 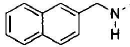 | tert-butyl *N*-((2*S*,11*R*,19a*S*)-15-fluoro-7,12-dimethyl-11-{[(2-naphthylmethyl)amino]carbonyl}-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecin-2-yl)carbamate |
| **Ex.90** | NH₂ | 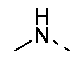 | (2*S*,11*R*,19a*S*)-2-amino-15-fluoro-*N*,7,12-trimethyl-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecine-11-carboxamide |
| **Ex.91** | NH₂ | 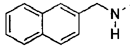 | (2*S*,11*R*,19aS)-2-amino-15-fluoro-7,12-dimethyl-*N*-(2-naphthylmethyl)-5,8,13-trioxo-2,3,6,7,8,9,10,11,12,13,19,19a-dodecahydro-1*H*,5*H*-pyrrolo[2,1-c][1,4,7,12]benzoxatriazacyclopentadecine-11-carboxamide |
| **Ex.92** | NH₂ | 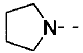 | (2*S*,11*R*,19a*S*)-2-amino-15-fluoro-7,12-dimethyl-11-(1-pyrrolidinylcarbonyl)-2,3,6,7,9,10,11,12,19,19a-decahydro-1*H*,5*H*-pyrrolo[2,1-c][1,4,7,12]benzoxatriazacyclopentadecine-5,8,13-trione |
| **Ex.93** | 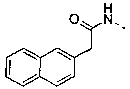 | 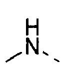 | (2*S*,11*R*,19a*S*)-15-fluoro-*N*,7,12-trimethyl-2-{[2-(2-naphthyl)acetyl]amino}-5,8,13-trioxo-2,3,6,7,8,9,10,11,12,13,19,19a-dodecahydro-1*H*,5*H-*pyrrolo[2,1-*c*][1,4,7,12]benzoxatriazacyclopentadecine-11-carboxamide |
| **Ex.94** | 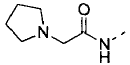 | 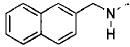 | (2*S*,11*R*,19a*S*)-15-fluoro-7,12-dimethyl-N-(2-naphthylmethyl)-5,8,13-trioxo-2-{[2-(1-pyrrolidinyl)acetyl]amino}-2,3,6,7,8,9,10,11,12,13,19,19a-dodecahydro-1*H*,5*H*-pyrrolo[2,1-*c*][1,4,7,12]benzoxatriazacyclopentadecine-11-carboxamide |
| **Ex.95** | 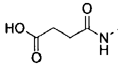 | 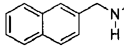 | 4-[((2*S*,11*R*,19a*S*)-15-fluoro-7,12-dimethyl-11-{[(2-naphthylmethyl)amino]carbonyl}-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecin-2-yl)amino]-4-oxobutanoic acid |
| **Ex.96** | 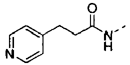 | 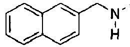 | (2*S*,11*R*,19a*S*)-15-fluoro-7,12-dimethyl-N-(2-naphthylmethyl)-5,8,13-trioxo-2-{[3-(4-pyridinyl)propanoyl]amino}-2,3,6,7,8,9,10,11,12,13,19,19a-dodecahydro-1*H*,5*H*-pyrrolo[2,1-*c*][1,4,7,12]benzoxatriazacyclopentadecine-11-carboxamide |
| **Ex.97** | 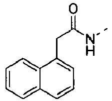 | 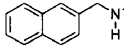 | (2*S*,11*R*,19a*S*)-15-fluoro-7,12-dimethyl-2-{[2-(1-naphthyl)acetyl]amino}-*N-*(2-naphthylmethyl)-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecine-11-carboxamide |
| **Ex.98** | 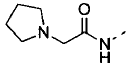 | 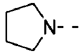 | *N*-[(2*S*,11*R*,19a*S*)-15-fluoro-7,12-dimethyl-5,8,13-trioxo-11-(1-pyrrolidinylcarbonyl)-2,3,6,7,8,9,10,11,12,13,19,19a-dodecahydro-1*H*,5*H-*pyrrolo[2,1-c][1,4,7,12]benzoxatriazacyclopentadecin-2-yl]-2-(1-pyrrolidinyl)acetamide |
| **Ex.99** | 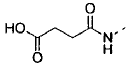 | 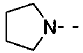 | 4-{[(2*S*,11*R*,19a*S*)-15-fluoro-7,12-dimethyl-5,8,13-trioxo-11-(1-pyrrolidinylcarbonyl)-2,3,6,7,8,9,10,11,12,13,19,19a-dodecahydro-1*H*,5*H-*pyrrolo[2,1-*c*][1,4,7,12]benzoxatriazacyclopentadecin-2-yl]amino}-4-oxobutanoic acid |
| **Ex.100** | 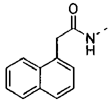 | 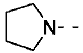 | *N*-[(2*S*,11*R*,19a*S*)-15-fluoro-7,12-dimethyl-5,8,13-trioxo-11-(1-pyrrolidinylcarbonyl)-2,3,6,7,8,9,10,11,12,13,19,19a-dodecahydro-1*H*,5*H-*pyrrolo[2,1-*c*][1,4,7,12]benzoxatriazacyclopentadecin-2-yl]-2-(1-naphthyl)acetamide |
| **Ex.101** | 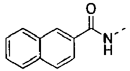 | 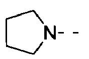 | *N*-[(2*S*,11*R*,19a*S*)-15-fluoro-7,12-dimethyl-5,8,13-trioxo-11-(1-pyrrolidinylcarbonyl)-2,3,6,7,8,9,10,11,12,13,19,19a-dodecahydro-1*H*,5*H-*pyrrolo[2,1-*c*][1,4,7,12]benzoxatriazacyclopentadecin-2-yl]-2-naphthamide |
| **Ex.102** | 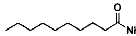 | 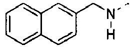 | (2*S*,11*R*,19a*S*)-2-(decanoylamino)-15-fluoro-7,12-dimethyl-*N*-(2-naphthylmethyl)-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecine-11-carboxamide |
| **Ex.103** | 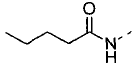 | 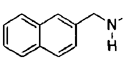 | (2*S*,11*R*,19a*S*)-15-fluoro-7,12-dimethyl-*N*-(2-naphthylmethyl)-5,8,13-trioxo-2-(pentanoylamino)-2,3,6,7,8,9,10,11,12,13,19,19a-dodecahydro-1*H*,5*H-*pyrrolo[2,1-*c*][1,4,7,12]benzoxatriazacyclopentadecine-11-carboxamide |

**Table 24a: Examples of Core 06 (Ex.104-Ex.114; continued on the following page)**

| **No** | **R²** | **R⁵⁴** | **Starting material** | **General Procedure** | **Reagent** | **Purification Method** | **Yield (isolated salt)** |
|---|---|---|---|---|---|---|---|
| **Ex.104-Ex.105:** *cf. experimental description* | | | | | | | |
| **Ex.106** | 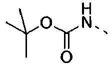 | 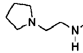 | **Ex.105** | L.2 | N-(2-aminoethyl)pyrolidine | FC (CH₂Cl₂/MeOH/aq.NH₃) | 78% |
| **Ex**.**107** | 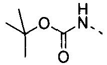 | 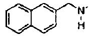 | **Ex.105** | L.2 | 2-naphthylmethylamine | FC (CH₂Cl₂/MeOH) | 96% |
| **Ex**.**108** | NH₂ | 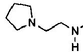 | **Ex.106** | J | HCl-dioxane | crude product | 99% (HCl salt) |
| **Ex.109** | NH₂ | 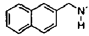 | **Ex.107** | J | HCl-dioxane | crude product | 95% (HCl salt) |
| **Ex.110** | 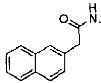 | 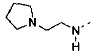 | **Ex**.**108** | L.1.2 | 2-naphthylacetic acid | prep. HPLC, method 1 and workup (CHCl₃/aq. NaHCO₃ soln) | 51% |
| **Ex.111** | 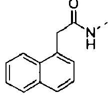 | 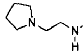 | **Ex.108** | L.1.2 | 1-naphthylacetic acid | prep. HPLC, method 1 | 55% (TFA salt) |
| **Ex.112** | 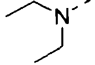 | 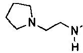 | **Ex**.**108** | M.1 | acetaldehyde | FC (CH₂Cl₂/MeOH/aq.NH₃) | 48% |
| **Ex.113** | 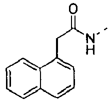 | 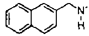 | **Ex.109** | L.1.2 | 1-naphthylacetic acid (3.7 equiv.) | prep. HPLC, method 3 | 77% |
| **Ex.114** | 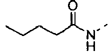 | 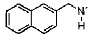 | **Ex.109** | L.1.1 | valeroyl chloride (2 equiv.) | prep. HPLC, method 3 | 84% |

**Table 24b: Examples of Core 06 (Ex.104-Ex.114; continued on the following page)**

| **No** | **R²** | **R⁵⁴** | **FORMULA** | **MONOISOTOPIC MASS** | **Rt (purity at 220nm)** | **[M+H]⁺ found** | **LC-MS-Method** |
|---|---|---|---|---|---|---|---|
| **Ex.104-Ex.105:** *cf. experimental description* | | | | | | | |
| **Ex.106** | 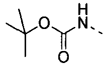 | 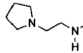 | C32H47FN607 | 646.4 | 1.36 (43), 1.42(27), 1.44 (27) | 647.5/647.5 647.5 | Method 2 |
| **Ex.107** | 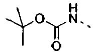 | 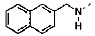 | C37H44FN5O7 | 689.3 | 1.94 (42)/ 1.98 (50) | 690.5/690.5 | Method 2 |
| **Ex**.**108** | NH₂ | 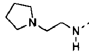 | C27H39FN6O5 | 546.3 | 1.37 (50), 1.44 (38) | 547.5/547.5 | Method 3 |
| **Ex.109** | NH₂ | 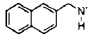 | C32H36FN5O5 | 589.3 | 1.55 (96) | 590.3 | Method 2 |
| **Ex.110** | 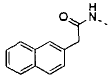 | 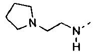 | C39H47FN6O6 | 714.4 | 1.62 (44), 1.66 (52) | 715.4/715.4 | Method 2 |
| **Ex.111** | 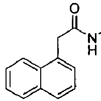 | 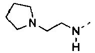 | C39H47FN6O6 | 714.4 | 1.51 (46), 1.56 (54) | 715.5/715.5 | Method 2 |
| **Ex.112** | 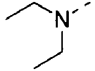 | 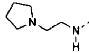 | C31H47FN6O5 | 602.4 | 1.14 (57), 1.18 (39) | 603.4/603.5 | Method 2 |
| **Ex.113** | 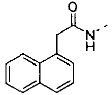 | 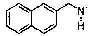 | C44H44FN5O6 | 757.3 | 2.15 (52), 2.19 (41) | 758.4/758.4 | Method 4a |
| **Ex.114** | 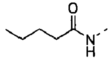 | 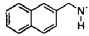 | C37H44FN5O6 | 673.3 | 2.00 (58), 2.05 (41) | 674.4/674.4 | Method 4a |

**Table 24c: Examples of Core 06 (Ex.104-Ex.114; continued on the following page)**

| **No** | **R²** | **R⁵⁴** | **IUPAC name** |
|---|---|---|---|
| **Ex.104** | 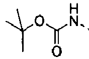 | OCH₂Ph | benzyl (2*R*,11*R*,19a*S*)-2-[(*tert*-butoxycarbonyl)amino]-15-fluoro-7,12-dimethyl-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecine-11-carboxylate |
| **Ex.105** | 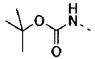 | OH | (2R,11R,19aS)-2-[(tert-butoxycarbonyl)amino]-15-fluoro-7,12-dimethyl-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecine-11-carboxylic acid |
| **Ex.106** | 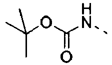 | 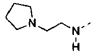 | *tert*-butyl *N*-[(2*R*,11*R*,19a*S*)-15-fluoro-7,12-dimethyl-5,8,13-trioxo-11-({[2-(1-pyrrolidinyl)ethyl]amino}carbonyl)-2,3,6,7,8,9,10,11,12,13,19,19a-dodecahydro-1*H*,5*H*-pyrrolo[2,1-*c*][1,4,7,12]benzoxatriazacyclopentadecin-2-yl]carbamate |
| **Ex.107** | 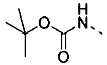 | 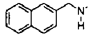 | *tert*-butyl *N*-((2*R*,11*R*,19a*S*)-15-fluoro-7,12-dimethyl-11-{[(2-naphthylmethyl)amino]carbonyl}-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecin-2-yl)carbamate |
| **Ex.108** | NH₂ | 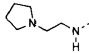 | (2*R*,11*R*,19a*S*)-2-amino-15-fluoro-7,12-dimethyl-5,8,13-trioxo-*N*-[2-(1-pyrrolidinyl)ethyl]-2,3,6,7,8,9,10,11,12,13,19,19a-dodecahydro-1*H*,5*H-*pyrrolo[2,1-*c*][1,4,7,12]benzoxatriazacyclopentadecine-11-carboxamide |
| **Ex.109** | NH₂ | 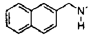 | (2*R*,11*R*,19a*S*)-2-amino-15-fluoro-7,12-dimethyl-*N*-(2-naphthylmethyl)-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecine-11-carboxamide |
| **Ex.110** | 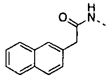 | 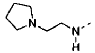 | (2*R*,11*R*,19a*S*)-15-fluoro-7,12-dimethyl-2-{[2-(2-naphthyl)acetyl]amino}-5,8,13-trioxo-*N*-[2-(1-pyrrolidinyl)ethyl]-2,3,6,7,8,9,10,11,12,13,19,19a-dodecahydro-1*H*,5*H*-pyrrolo[2,1-*c*][1,4,7,12]benzoxatriazacyclopentadecine-11-carboxamide |
| **Ex.111** | 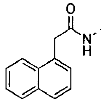 | 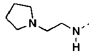 | (*2*R,11*R*,19a*S*)-15-fluoro-7,12-dimethyl-2-{[2-(1-naphthyl)acetyl]amino}-5,8,13-trioxo-*N*-[2-(1-pyrrolidinyl)ethyl]-2,3,6,7,8,9,10,11,12,13,19,19a-dodecahydro-1*H*,5*H*-pyrrolo[2,1-*c*][1,4,7,12]benzoxatriazacyclopentadecine-11-carboxamide |
| **Ex.112** | 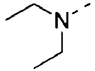 | 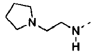 | (2*R*,11*R*,19a*S*)-2-(diethylamino)-15-fluoro-7,12-dimethyl-5,8,13-trioxo-*N*-[2-(1-pyrrolidinyl)ethyl]-2,3,6,7,8,9,10,11,12,13,19,19a-dodecahydro-1*H*,5*H-*pyrrolo[2,1-*c*][1,4,7,12]benzoxatriazacyclopentadecine-11-carboxamide |
| **Ex.113** | 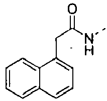 | 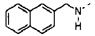 | (2*R*,11*R*,19a*S*)-15-fluoro-7,12-dimethyl-2-{[2-(1-naphthyl)acetyl]amino}-*N*-(2-naphthylmethyl)-5,8,13-trioxo-2,3,6,7,8,9,10,11,12,13,19,19a-dodecahydro-1*H*,5*H-*pyrrolo[2,1-c][1,4,7,12]benzoxatriazacyclopentadecine-11-carboxamide |
| **Ex.114** | 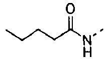 | 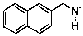 | (2*R*,11*R*,19a*S*)-15-fluoro-7,12-dimethyl-*N*-(2-naphthylmethyl)-5,8,13-trioxo-2-(pentanoylamino)-2,3,6,7,8,9,10,11,12,13,19,19a-dodecahydro-1*H*,5*H*-pyrrolo[2,1-*c*][1,4,7,12]benzoxatriazacyclopentadecine-11-carboxamide |

**Table 25a: Examples of Core 07 (Ex.115-Ex.131; continued on the following pages)**

| **No** | **R¹¹** | **R⁵⁴** | **Starting material** | **General Procedure** | **Reagent** | **Purification Method** | **Yield, (isolated salt)** |
|---|---|---|---|---|---|---|---|
| **Ex.115-Ex.116:** *cf. experimental description* | | | | | | | |
| **Ex.117** | 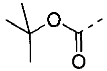 | 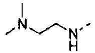 | **Ex.116** | L.2 | N,N-dimethylethylenediamine | FC (CH₂Cl₂/MeOH/aq. NH₃) | 73% |
| **Ex.118** | 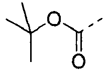 | 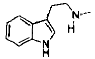 | **Ex.116** | L.2 | tryptamine | FC (CH₂Cl₂/MeOH) | 98% |
| **Ex.119** | 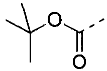 | 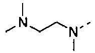 | **Ex.116** | L.2 | N,N,N'-trimethylethylenediamine | FC (CH₂Cl₂/MeOH) | 79% |
| **Ex.120** | 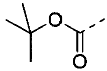 | 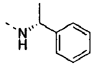 | **Ex.116** | L.2 | D-(+)-α-methylbenzylamine | prep. HPLC, method 1 | 70% |
| **Ex.121** | H | 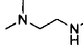 | **Ex.117** | J | HCl-dioxane | crude product | 98% (HCl salt) |
| **Ex.122** | H | 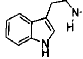 | **Ex.118** | J | HCl-dioxane | crude product | 98% (HCl salt) |
| **Ex.123** | H | 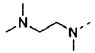 | **Ex.119** | J | HCl-dioxane THF/CH₂Cl₂ as cosolvent | crude product | quant. (HCl salt) |
| **Ex.124** | H | 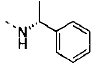 | **Ex.120** | J | HCl-dioxane | crude product | 95% (HCl salt) |
| **Ex.125** | 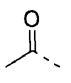 | 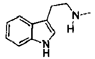 | **Ex.122** | L.1.1 | acetic anhydride (1.1 equiv.) pyridine/CH₂Cl₂ 1:1 (3 mL) | prep. HPLC, method 1 | 62% |
| **Ex.126** | 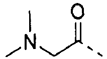 | 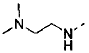 | **Ex.121** | L.1.3 | N,N-dimethylglycine | prep. HPLC, method 1 | 39% (TFA salt) |
| **Ex.127** | 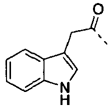 | 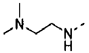 | **Ex.121** | L.1.3 | 3-indoleacetic acid | prep. HPLC, method 1 | 20% (TFA salt) |
| **Ex.128** | 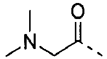 | 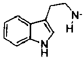 | **Ex.122** | L.1.3 | N,N-dimethylglycine | prep. HPLC, method 1 | 21% (TFA salt) |
| **Ex.129** | 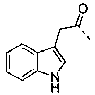 | 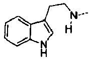 | **Ex.122** | L.1.3 | 3-indoleacetic acid | prep. HPLC, method 1 | 45% |
| **Ex.130** | 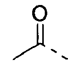 | 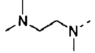 | **Ex.123** | L.1.1 | acetic anhydride (5 equiv.) | prep. HPLC, method 1 | 14% (TFA salt) |
| **Ex.131** | 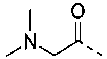 | 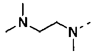 | **Ex.123** | L.1.3 | N,N-dimethylglycine | prep. HPLC, method 1 | 44% (TFA salt) |

**Table 25b: Examples of Core 07 (Ex.115-Ex.131; continued on the following pages)**

| **No** | **R¹¹** | **R⁵⁴** | **FORMULA** | **MONOISOTOPIC MASS** | **Rt (purity at 220nm)** | **[M+H]⁺ found** | **LC-MS-Method** |
|---|---|---|---|---|---|---|---|
| **Ex.115-Ex.116:** *cf. experimental description* | | | | | | | |
| **Ex.117** | 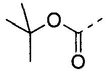 | 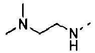 | C30H45FN6O7 | 620.3 | 1.45 | 621.2 | Method 9c |
| **Ex.118** | 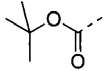 | 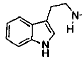 | C36H45FN6O7 | 692.3 | 1.94 | 693.1 | Method 9c |
| **Ex.119** | 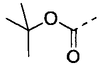 | 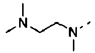 | C31H47FN6O7 | 634.4 | 1.48 | 635.4 | Method 9c |
| **Ex.120** | 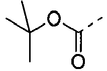 | 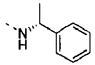 | C34H44FN5O7 | 653.3 | 1.99 | 653.9 | Method 9c |
| **Ex.121** | H | 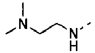 | C25H37FN6O5 | 520.3 | 1.23 (17), 1.29 (80) | 521.3/ 521.3 | Method 3 |
| **Ex.122** | H | 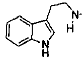 | C31H37FN6O5 | 592.3 | 1.40 (89) | 593.3 | Method 2 |
| **Ex.123** | H | 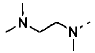 | C26H39FN6O5 | 534.3 | 1.32 (96) | 535.3 | Method 3 |
| **Ex.124** | H | 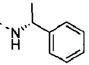 | C29H36FN5O5 | 553.3 | 1.41 (98) | 554.3 | Method 2 |
| **Ex.125** | 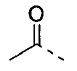 | 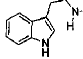 | C33H39FN6O6 | 634.3 | 1.54 (100) | 635.3 | Method 2 |
| **Ex.126** | 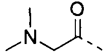 | 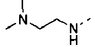 | C29H44FN7O6 | 605.3 | 1.37 (83) | 606.4 | Method 3 |
| **Ex.127** | 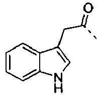 | 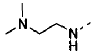 | C35H44FN7O6 | 677.3 | 1.32 (12), 1.38 (84) | 678.3/ 678.3 | Method 2 |
| **Ex.128** | 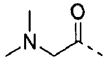 | 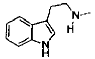 | C35H44FN7O6 | 677.3 | 1.40 (98) | 678.4 | Method 2 |
| **Ex.129** | 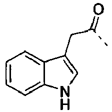 | 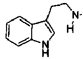 | C41H44FN7O6 | 749.3 | 1.73 (85) | 750.4 | Method 2 |
| **Ex.130** | 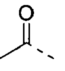 | 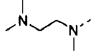 | C28H41FN6O6 | 576.3 | 1.10 (98) | 577.3 | Method 2 |
| **Ex.131** | 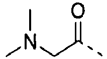 | 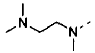 | C30H46FN7O6 | 619.4 | 1.41 (96) | 620.4 | Method 3 |

**Table 25c: Examples of Core 07 (Ex.115-Ex.131; continued on the following pages)**

| **No** | **R¹¹** | **R⁵⁴** | **IUPAC name** |
|---|---|---|---|
| **Ex.115** | 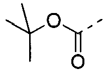 | OCH₂Ph | 12-benzyl 2-(tert-butyl) (12*S*,20a*S*)-16-fluoro-8,13-dimethyl-6,9,14-trioxo-3,4,7,8,9,10,11,12,13,14,20,20a-dodecahydro-6*H*-pyrazino[2,1-*c*][1,4,7,12]benzoxatriazacyclopentadecine-2,12(1*H*)-dicarboxylate |
| **Ex.116** | 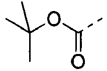 | OH | (12*S*,20a*S*)-2-(tert-butoxycarbonyl)-16-fluoro-8,13-dimethyl-6,9,14-trioxo-1,2,3,4,7,8,9,10,11,12,13,14,20,20a-tetradecahydro-6*H*-pyrazino[2,1-*c*][1,4,7,12]benzoxatriazacyclopentadecine-12-carboxylic acid |
| **Ex**.**117** | 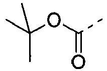 | 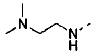 | *tert*-butyl (12*S*,20a*S*)-12-({[2-(dimethylamino)ethyl]amino}carbonyl)-16-fluoro-8,13-dimethyl-6,9,14-trioxo-3,4,7,8,9,10,11,12,13,14,20,20a-dodecahydro-6*H-*pyrazino[2,1-*c*][1,4,7,12]benzoxatriazacyclopentadecine-2(1*H*)-carboxylate |
| **Ex.118** | 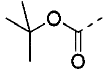 | 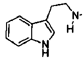 | *tert*-butyl (12*S*,20a*S*)-16-fluoro-12-({[2-(1*H*-indol-3-yl)ethyl]amino}carbonyl)-8,13-dimethyl-6,9,14-trioxo-3,4,7,8,9,10,11,12,13,14,20,20a-dodecahydro-6*H-*pyrazino[2,1-*c*][1,4,7,12]benzoxatriazacyclopentadecine-2(1*H*)-carboxylate |
| **Ex.119** | 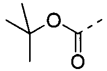 | 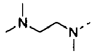 | *tert*-butyl (12*S*,20a*S*)-12-{[[2-(dimethylamino)ethyl](methyl)amino]carbonyl}-16-fluoro-8,13-dimethyl-6,9,14-trioxo-3,4,7,8,9,10,11,12,13,14,20,20a-dodecahydro-6*H*-pyrazino[2,1-*c*][1,4,7,12]benzoxatriazacyclopentadecine-2(1*H*)-carboxylate |
| **Ex.120** | 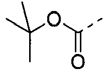 | 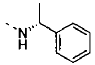 | *tert*-butyl (12*S*,20a*S*)-16-fluoro-8,13-dimethyl-6,9,14-trioxo-12-({[(1*R*)-1-phenylethyl]amino}carbonyl)-3,4,7,8,9,10,11,12,13,14,20,20a-dodecahydro-6*H-*pyrazino[2,1-*c*][1,4,7,12]benzoxatriazacyclopentadecine-2(1*H*)-carboxylate |
| **Ex.121** | H | 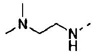 | (12*S*,20a*S*)-*N*-[2-(dimethylamino)ethyl]-16-fluoro-8,13-dimethyl-6,9,14-trioxo-1,2,3,4,7,8,9,10,11,12,13,14,20,20a-tetradecahydro-6*H*-pyrazino[2,1-c][1,4,7,12]benzoxatriazacyclopentadecine-12-carboxamide |
| **Ex.122** | H | 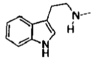 | (12*S*,20a*S*)-16-fluoro-*N*-[2-(1*H*-indol-3-yl)ethyl]-8,13-dimethyl-6,9,14-trioxo-1,2,3,4,7,8,9,10,11,12,13,14,20,20a-tetradecahydro-6*H*-pyrazino[2,1-*c*][1,4,7,12]benzoxatriazacyclopentadecine-12-carboxamide |
| **Ex.123** | H | 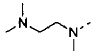 | (12*S*,20a*S*)-*N*-[2-(dimethylamino)ethyl]-16-fluoro-*N*,8,13-trimethyl-6,9,14-trioxo-1,2,3,4,7,8,9,10,11,12,13,14,20,20a-tetradecahydro-6*H*-pyrazino[2,1-*c*][1,4,7,12]benzoxatriazacyclopentadecine-12-carboxamide |
| **Ex.124** | H | 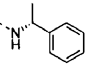 | (12*S*,20a*S*)-16-fluoro-8,13-dimethyl-6,9,14-trioxo-*N*-[(1*R*)-1-phenylethyl]-1,2,3,4,7,8,9,10,11,12,13,14,20,20a-tetradecahydro-6H-pyrazino[2,1-*c*][1,4,7,12]benzoxatriazacyclopentadecine-12-carboxamide |
| **Ex.125** | 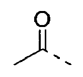 | 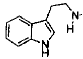 | (12*S*,20a*S*)-2-acetyl-16-fluoro-*N*-[2-(1*H*-indol-3-yl)ethyl]-8,13-dimethyl-6,9,14-trioxo-1,2,3,4,7,8,9,10,11,12,13,14,20,20a-tetradecahydro-6*H*-pyrazino[2,1-*c*][1,4,7,12]benzoxatriazacyclopentadecine-12-carboxamide |
| **Ex.126** | 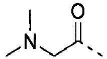 | 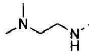 | (12*S*,20a*S*)-2-[2-(dimethylamino)acetyl]-*N*-[2-(dimethylamino)ethyl]-16-fluoro-8,13-dimethyl-6,9,14-trioxo-1,2,3,4,7,8,9,10,11,12,13,14,20,20a-tetradecahydro-6*H*-pyrazino[2,1-*c*][1,4,7,12]benzoxatriazacyclopentadecine-12-carboxamide |
| **Ex.127** | 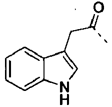 | 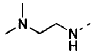 | (12*S*,20a*S*)-*N*-[2-(dimethylamino)ethyl]-16-fluoro-2-[2-(1*H*-indol-3-yl)acetyl]-8,13-dimethyl-6,9,14-trioxo-1,2,3,4,7,8,9,10,11,12,13,14,20,20a-tetradecahydro-6*H*-pyrazino[2,1-*c*][1,4,7,12]benzoxatriazacyclopentadecine-12-carboxamide |
| **Ex.128** | 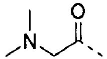 | 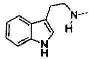 | (12*S*,20a*S*)-2-[2-(dimethylamino)acetyl]-16-fluoro-N-[2-(1*H*-indol-3-yl)ethyl]-8,13-dimethyl-6,9,14-trioxo-1,2,3,4,7,8,9,10,11,12,13,14,20,20a-tetradecahydro-6*H*-pyrazino[2,1-*c*][1,4,7,12]benzoxatriazacyclopentadecine-12-carboxamide |
| **Ex.129** | 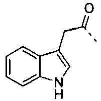 | 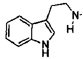 | (12*S*,20a*S*)-16-fluoro-2-[2-(1*H*-indol-3-yl)acetyl]-*N*-[2-(1*H*-indol-3-yl)ethyl]-8,13-dimethyl-6,9,14-trioxo-1,2,3,4,7,8,9,10,11,12,13,14,20,20a-tetradecahydro-6*H*-pyrazino[2,1-*c*][1,4,7,12]benzoxatriazacyclopentadecine-12-carboxamide |
| **Ex.130** | 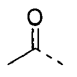 | 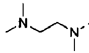 | (12S,20aS)-2-acetyl-N-[2-(dimethylamino)ethyl]-16-fluoro-*N*,8,13-trimethyl-6,9,14-trioxo-1,2,3,4,7,8,9,10,11,12,13,14,20,20a-tetradecahydro-6*H-*pyrazino[2,1-*c*][1,4,7,12]benzoxatriazacyclopentadecine-12-carboxamide |
| **Ex.131** | 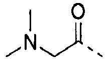 | 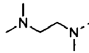 | (12*S*,20a*S*)-2-[2-(dimethylamino)acetyl]-*N*-[2-(dimethylamino)ethyl]-16-fluoro-*N*,8,13-trimethyl-6,9,14-trioxo-1,2,3,4,7,8,9,10,11,12,13,14,20,20a-tetradecahydro-6*H*-pyrazino[2,1-*c*][1,4,7,12]benzoxatriazacyclopentadecine-12-carboxamide |

**Table 26a: Examples of Core 08 (Ex.132-Ex.141; continued on the following page)**

| **No** | **R¹¹** | **R⁵⁴** | **Starting material** | **General Procedure** | **Reagent** | **Purification Method** | **Yield (isolated salt)** |
|---|---|---|---|---|---|---|---|
| **Ex.132-Ex.133:** *cf. experimental description* | | | | | | | |
| **Ex.134** | 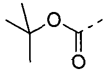 | 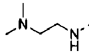 | **Ex.133** | L.2 | N,N-dimethylethylenediamine | FC (CH₂Cl₂/MeOH/aq. NH₃) | 80% |
| **Ex.135** | H | 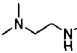 | **Ex.134** | J | HCl-dioxane | crude product | 100% (HCl salt) |
| **Ex.136** | 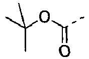 | 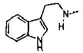 | **Ex.133** | L.2 | tryptamine | FC (CH₂Cl₂/MeOH /aq.NH₃) | 89% |
| **Ex.137** | H | 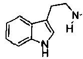 | **Ex.136** | J | HCl-dioxane | crude product | 100% (HCl salt) |
| **Ex.138** | 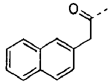 | 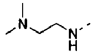 | **Ex.135** | L.1.2 | 2-naphthylacetic acid | prep. HPLC, method 1 | 71% (TFA salt) |
| **Ex.139** | 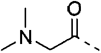 | 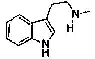 | **Ex.137** | L.1.2 | N,N-dimethyl glycine | prep. HPLC, method 1 | 40% (TFA salt) |
| **Ex.140** | 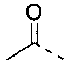 | 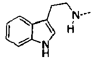 | **Ex.137** | L.1.1 | acetic anhydride (5 equiv.) | prep. HPLC, method 1 | 50% |
| **Ex.141** | 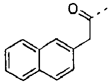 | 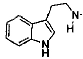 | **Ex.137** | L.1.2 | 2-naphthylacetic acid | prep. HPLC, method 3, then FC (EtOAc/MeOH) | 39% |

**Table 26b: Examples of Core 08 (Ex.132-Ex.141; continued on the following page)**

| **No** | **R¹¹** | **R⁵⁴** | **FORMULA** | **MONOISOTOPIC MASS** | **Rt (purity at 220nm)** | **[M+H]⁺ found** | **LC-MS-Method** |
|---|---|---|---|---|---|---|---|
| **Ex.132-Ex.133:** *cf. experimental description* | | | | | | | |
| **Ex.134** | 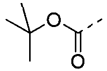 | 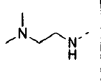 | C30H45FN6O7 | 620.3 | 1.40 (20), 1.45 (77) | 621.5/621.5 | Method 2 |
| **Ex.135** | H | 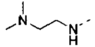 | C25H37FN6O5 | 520.3 | 0.94 (85) | 521.4 | Method 2 |
| **Ex.136** | 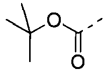 | 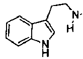 | C36H45FN6O7 | 692.3 | 1.83 (91) | 693.5 | Method 2 |
| **Ex.137** | H | 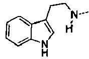 | C31H37FN6O5 | 592.3 | 1.41 (80) | 593.4 | Method 2 |
| **Ex.138** | 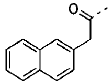 | 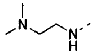 | C37H45FN6O6 | 688.3 | 1.46 (15), 1.51 (84) | 689.5/ 689.5 | Method 2 |
| **Ex.139** | 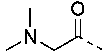 | 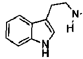 | C35H44FN7O6 | 677.3 | 1.43 (92) | 678.5 | Method 2 |
| **Ex.140** | 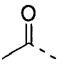 | 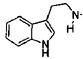 | C33H39FN6O6 | 634.3 | 1.57 (97) | 635.5 | Method 2 |
| **Ex.141** | 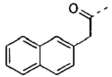 | 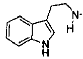 | C43H45FN6O6 | 760.3 | 1.86 (95) | 761.5 | Method 2 |

**Table 26c: Examples of Core 08 (Ex.132-Ex.141; continued on the following page)**

| **No** | **R¹¹** | **R⁵⁴** | **IUPAC name** |
|---|---|---|---|
| **Ex.132** | 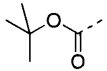 | OCH₂Ph | 12-benzyl 2-(*tert*-butyl) (12*R*,20a*R*)-16-fluoro-8,13-dimethyl-6,9,14-trioxo-3,4,7,8,9,10,11,12,13,14,20,20a-dodecahydro-6*H*-pyrazino[2,1-*c*][1,4,7,12]benzoxatriazacyclopentadecine-2,12(1*H*)-dicarboxylate |
| **Ex.133** | 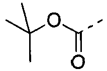 | OH | (12*R*,20a*R*)-2-(*tert*-butoxycarbonyl)-16-fluoro-8,13-dimethyl-6,9,14-trioxo-1,2,3,4,7,8,9,10,11,12,13,14,20,20a-tetradecahydro-6*H*-pyrazino[2,1-*c*][1,4,7,12]benzoxatriazacyclopentadecine-12-carboxylic acid |
| **Ex.134** | 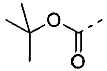 | 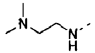 | *tert*-butyl (12*R*,20a*R*)-12-({[2-(dimethylamino)ethyl]amino}carbonyl)-16-fluoro-8,13-dimethyl-6,9,14-trioxo-3,4,7,8,9,10,11,12,13,14,20,20a-dodecahydro-6*H-*pyrazino[2,1-*c*][1,4,7,12]benzoxatriazacyclopentadecine-2(1H)-carboxylate |
| **Ex.135** | H | 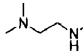 | (12*R*,20a*R*)-*N*-[2-(dimethylamino)ethyl]-16-fluoro-8,13-dimethyl-6,9,14-trioxo-1,2,3,4,7,8,9,10,11,12,13,14,20,20a-tetradecahydro-6*H*-pyrazino[2,1-*c*][1,4,7,12]benzoxatriazacyclopentadecine-12-carboxamide |
| **Ex.136** | 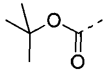 | 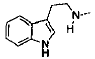 | *tert*-butyl (12*R*,20a*R*)-16-fluoro-12-({[2-(1*H*-indol-3-yl)ethyl]amino}carbonyl)-8,13-dimethyl-6,9,14-trioxo-3,4,7,8,9,10,11,12,13,14,20,20a-dodecahydro-6H-pyrazino[2,1-*c*][1,4,7,12]benzoxatriazacyclopentadecine-2(1*H*)-carboxylate |
| **Ex.137** | H | 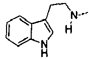 | (12*R*,20a*R*)-16-fluoro-*N*-[2-(1H-indol-3-yl)ethyl]-8,13-dimethyl-6,9,14-trioxo-1,2,3,4,7,8,9,10,11,12,13,14,20,20a-tetradecahydro-6*H*-pyrazino[2,1-*c*][1,4,7,12]benzoxatriazacyclopentadecine-12-carboxamide |
| **Ex.138** | 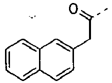 | 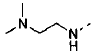 | (12*R*,20a*R*)-*N*-[2-(dimethylamino)ethyl]-16-fluoro-8,13-dimethyl-2-[2-(2-naphthyl)acetyl]-6,9,14-trioxo-1,2,3,4,7,8,9,10,11,12,13,14,20,20a-tetradecahydro-6*H*-pyrazino[2,1-*c*][1,4,7,12]benzoxatriazacyclopentadecine-12-carboxamide |
| **Ex.139** | 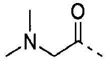 | 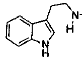 | (12*R*,20a*R*)-2-[2-(dimethylamino)acetyl]-16-fluoro-N-[2-(1*H*-indol-3-yl)ethyl]-8,13-dimethyl-6,9,14-trioxo-1,2,3,4,7,8,9,10,11,12,13,14,20,20a-tetradecahydro-6*H*-pyrazino[2,1-*c*][1,4,7,12]benzoxatriazacyclopentadecine-12-carboxamide |
| **Ex.140** | 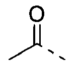 | 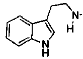 | (12*R*,20a*R*)-2-acetyl-16-fluoro-N-[2-(1H-indol-3-yl)ethyl]-8,13-dimethyl-6,9,14-trioxo-1,2,3,4,7,8,9,10,11,12,13,14,20,20a-tetradecahydro-6*H*-pyrazino[2,1-*c*][1,4,7,12]benzoxatriazacyclopentadecine-12-carboxamide |
| **Ex.141** | 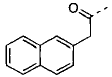 | 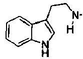 | (12*R*,20a*R*)-16-fluoro-N-[2-(1*H*-indol-3-yl)ethyl]-8,13-dimethyl-2-[2-(2-naphthyl)acetyl]-6,9,14-trioxo-1,2,3,4,7,8,9,10,11,12,13,14,20,20a-tetradecahydro-6*H*-pyrazino[2,1-*c*][1,4,7,12]benzoxatriazacyclopentadecine-12-carboxamide |

**Table 27a: Examples of Core 09 (Ex.142-Ex.163; continued on the following pages)**

| **No** | **R²** | **R⁵⁴** | **Starting material** | **General Procedure** | **Reagent** | **Purification Method** | **Yield (isolated salt)** |
|---|---|---|---|---|---|---|---|
| **Ex.142-Ex.143:** *cf. experimental description* | | | | | | | |
| **Ex.144** | 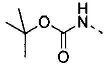 | 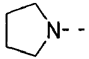 | **Ex.143** | L.2 | pyrrolidine | FC (CH₂Cl₂/MeOH) | 91% |
| **Ex.145** | 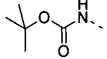 | 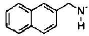 | **Ex.143** | L.2 | 2-naphthylmethylamine | FC (CH₂Cl₂/MeOH) | 72% |
| **Ex.146** | 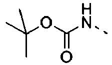 | 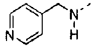 | **Ex.143** | L.2 | 4-(aminomethyl)pyridine | FC (CH₂Cl₂/MeOH) | 87% |
| **Ex.147** | NH₂ | 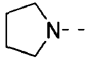 | **Ex.144** | J | HCl-dioxane | crude product | 100% (HCl salt) |
| **Ex.148** | NH₂ | 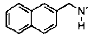 | **Ex.145** | J | HCl-dioxane | crude product | 97% (HCl salt) |
| **Ex.149** | NH₂ | 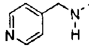 | **Ex.146** | J | HCl-dioxane | crude product | 87% (HCl salt) |
| **Ex.150** | 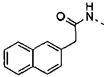 | 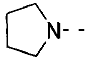 | **Ex.147** | L.1.2 | 2-naphthylacetic acid | Prep. HPLC, method 3 | 72% |
| **Ex.151** | 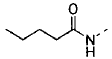 | 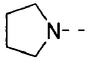 | **Ex.147** | L.1.1 | valeroyl chloride (2.0 equiv.) | Prep. HPLC, method 3 | 73% |
| **Ex.152** | 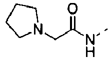 | 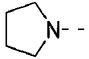 | **Ex.147** | L.1.2 | 1-pyrriolidinacetic acid | Prep. HPLC, method 2 | 26% |
| **Ex.153** | 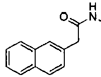 | 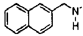 | **Ex.148** | L.1.2 | 2-naphthylacetic acid | Prep. HPLC, method 3 | 68% |
| **Ex.154** | 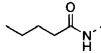 | 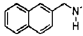 | **Ex.148** | L.1.1 | valeroyl chloride (2.0 equiv.) | Prep. HPLC, method 3 | 76% |
| **Ex.155** | 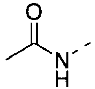 | 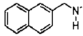 | **Ex.148** | L.1.1 | acetic anhydride (5.0 equiv.) | Prep. HPLC, method 3 | 72% |
| **Ex.156** | 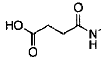 | 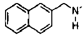 | **Ex.148** | L.1.1 | succinic anhydride (1.05 equiv.) | Prep. HPLC, method 2 | 69% (NH₄⁺ salt) |
| **Ex.157** | 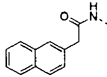 | 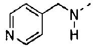 | **Ex.149** | L.1.2 | 2-naphthylacetic acid (1.7 equiv.) | Prep. HPLC, method 2 | 24% |
| **Ex.158** | 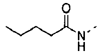 | 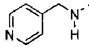 | **Ex.149** | L.1.1 | valeroyl chloride (2.0 equiv.) | Prep. HPLC, method 2 | 42% |
| **Ex.159** | 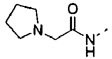 | 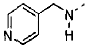 | **Ex**.**149** | L.1.2 | 1-pyrriolidinacetic acid (2.5 equiv.) | Prep. HPLC, method 2 | 55% |
| **Ex.160** | 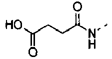 | 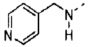 | **Ex.149** | L.1.1 | succinic anhydride (1.05 equiv.) | Prep. HPLC, method 2 | 69% (NH₄⁺ salt) |
| **Ex.161** | 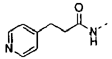 | 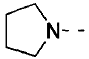 | **Ex.147** | L.1.2 | 3-(pyridine-4-yl)propanoic acid | Prep. HPLC, method 2 | 53% |
| **Ex.162** | 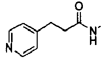 | 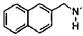 | **Ex.148** | L.1.2 | 3-(pyridine-4-yl)propanoic acid | Prep. HPLC, method 2 | 48% |
| **Ex.163** | 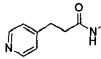 | 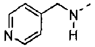 | **Ex.149** | L.1.2 | 3-(pyridine-4-yl)propanoic acid (1.7 equiv.) | Prep. HPLC, method 2 | 63% |

**Table 27b: Examples of Core 09 (Ex.142-Ex.163; continued on the following pages)**

| **No** | **R²** | **R⁵⁴** | **FORMULA** | **MONOISOTOPIC MASS** | **Rt (purity at 220nm)** | **[M+H]⁺ found** | **LC-MS-Method** |
|---|---|---|---|---|---|---|---|
| **Ex.142-Ex.143:** *cf. experimental description* | | | | | | | |
| **Ex.144** | 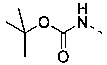 | 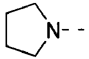 | C31H44FN5O7 | 617.3 | 1.66 (90) | 618.4 | Method 2 |
| **Ex.145** | 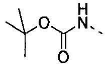 | 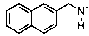 | C38H46FN5O7 | 703.3 | 1.90 (90) | 704.5 | Method 2 |
| **Ex.146** | 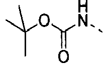 | 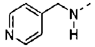 | C33H43FN6O7 | 654.3 | 1.39 (92) | 655.5 | Method 2 |
| **Ex.147** | NH₂ | 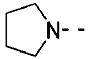 | C26H36FN5O5 | 517.3 | 1.36 (79) | 518.4 | Method 3 |
| **Ex.148** | NH₂ | 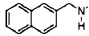 | C33H38FN5O5 | 603.3 | 1.53 (98) | 604.4 | Method 2 |
| **Ex.149** | NH₂ | 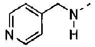 | C28H35FN6O5 | 554.3 | 1.25 (8), 1.31 (85) | 555.4 | Method 3 |
| **Ex.150** | 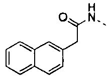 | 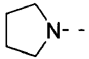 | C38H44FN5O6 | 685.3 | 1.72 (98) | 686.5 | Method 2 |
| **Ex.151** | 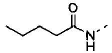 | 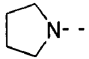 | C31H44FN5O6 | 601.3 | 1.55 (98) | 602.5 | Method 2 |
| **Ex.152** | 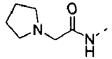 | 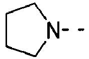 | C32H45FN6O6 | 628.3 | 1.52 (94) | 629.5 | Method 3 |
| **Ex.153** | 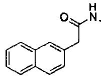 | 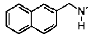 | C45H46FN5O6 | 771.3 | 1.94 (96) | 772.5 | Method 2 |
| **Ex.154** | 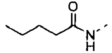 | 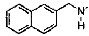 | C38H46FN5O6 | 687.3 | 1.82 (97) | 688.5 | Method 2 |
| **Ex.155** | 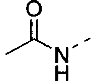 | 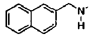 | C35H40FN5O6 | 645.3 | 1.67 (99) | 646.4 | Method 2 |
| **Ex.156** | 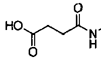 | 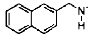 | C37H42FN5O8 | 703.3 | 1.65 (99) | 704.5 | Method 2 |
| **Ex.157** | 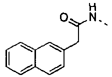 | 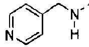 | C40H43FN6O6 | 722.3 | 1.48 (94) | 723.8 | Method 2 |
| **Ex.158** | 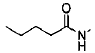 | 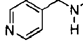 | C33H43FN6O6 | 638.3 | 1.50 (95) | 639.5 | Method 3 |
| **Ex.159** | 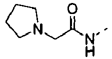 | 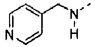 | C34H44FN7O6 | 665.3 | 1.47 (96) | 666.5 | Method 3 |
| **Ex.160** | 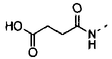 | 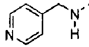 | C32H39FN6O8 | 654.3 | 1.04 (96) | 655.4 | Method 3 |
| **Ex.161** | 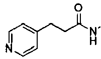 | 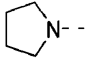 | C34H43FN6O6 | 650.3 | 1.47 (91) | 651.5 | Method 3 |
| **Ex.162** | 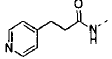 | 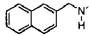 | C41H45FN6O6 | 736.3 | 1.55 (96) | 737.6 | Method 2 |
| **Ex.163** | 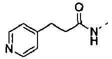 | 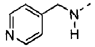 | C36H42FN7O6 | 687.3 | 1.42 (99) | 688.5 | Method 3 |

**Table 27c: Examples of Core 09 (Ex.192-Ex.163; continued on the following pages)**

| **No** | **R²** | **R⁵⁴** | IUPAC name |
|---|---|---|---|
| **Ex.142** | 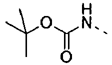 | OCH₂Ph | benzyl (2*R*,12*S*,20a*S*)-2-[(*tert*-butoxycarbonyl)amino]-16-fluoro-8,13-dimethyl-5,9,14-trioxo-2,3,5,6,7,8,9,10,11,12,13,14,20,20a-tetradecahydro-1*H-*pyrrolo[2,1-*c*][1,9,8,13]benzoxatriazacyclohexadecine-12-carboxylate |
| **Ex.143** | 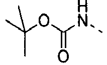 | OH | (2*R*,12*S*,20a*S*)-2-[(*tert*-butoxycarbonyl)amino]-16-fluoro-8,13-dimethyl-5,9,14-trioxo-2,3,5,6,7,8,9,10,11,12,13,14,20,20a-tetradecahydro-1*H*-pyrrolo[2,1-*c*][1,4,8,13]benzoxatriazacyclohexadecine-12-carboxylic acid |
| **Ex.144** | 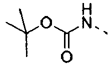 | 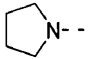 | *tert*-butyl *N*-[(2*R*,12*S*,20a*S*)-16-fluoro-8,13-dimethyl-5,9,14-trioxo-12-(1-pyrrolidinylcarbonyl)-2,3,5,6,7,8,9,10,11,12,13,14,20,20a-tetradecahydro-1*H-*pyrrolo[2,1-*c*][1,4,8,13]benzoxatriazacyclohexadecin-2-yl]carbamate |
| **Ex.145** | 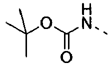 | 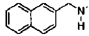 | *tert*-butyl *N*-((2*R*,12*S*,20a*S*)-16-fluoro-8,13-dimethyl-12-{[(2-naphthylmethyl)amino]carbonyl}-5,9,14-trioxo-2,3,5,6,7,8,9,10,11,12,13,14,20,20a-tetradecahydro-1*H*-pyrrolo[2,1-*c*][1,4,8,13]benzoxatriazacyclohexadecin-2-yl)carbamate |
| **Ex.146** | 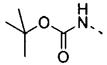 | 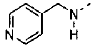 | *tert*-butyl *N*-((2*R*,12*S*,20a*S*)-16-fluoro-8,13-dimethyl-5,9,14-trioxo-12-{[(4-pyridinylmethyl)amino]carbonyl}-2,3,5,6,7,8,9,10,11,12,13,14,20,20a-tetradecahydro-1*H*-pyrrolo[2,1-*c*][1,4,8,13]benzoxatriazacyclohexadecin-2-yl)carbamate |
| **Ex.147** | NH₂ | 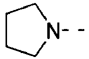 | (2*R*,12*S*,20a*S*)-2-amino-16-fluoro-8,13-dimethyl-12-(1-pyrrolidinylcarbonyl)-2,3,7,8,10,11,12,13,20,20a-decahydro-1*H*-pyrrolo[2,1-*c*][1,4,8,13]benzoxatriazacyclohexadecine-5,9,14(6*H*)-trione |
| **Ex.148** | NH₂ | 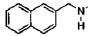 | (2*R*,12*S*,20a*S*)-2-amino-16-fluoro-8,13-dimethyl-N-(2-naphthylmethyl)-5,9,14-trioxo-2,3,5,6,7,8,9,10,11,12,13,14,20,20a-tetradecahydro-1*H*-pyrrolo[2,1-*c*][1,4,8,13]benzoxatriazacyclohexadecine-12-carboxamide |
| **Ex.149** | NH₂ | 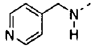 | (2*R*,12*S*,20aS*)*-2-amino-16-fluoro-8,13-dimethyl-5,9,14-trioxo-N-(4-pyridinylmethyl)-2,3,5,6,7,8,9,10,11,12,13,14,20,20a-tetradecahydro-1*H-*pyrrolo[2,1-*c*][1,4,8,13]benzoxatriazacyclohexadecine-12-carboxamide |
| **Ex.150** | 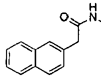 | 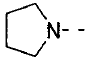 | *N*-[(2*R*,12*S*,20a*S*)-16-fluoro-8,13-dimethyl-5,9,14-trioxo-12-(1-pyrrolidinylcarbonyl)-2,3,5,6,7,8,9,10,11,12,13,14,20,20a-tetradecahydro-1*H-*pyrrolo[2,1-*c*][1,4,8,13]benzoxatriazacyclohexadecin-2-yl]-2-(2-naphthyl)acetamide |
| **Ex.151** | 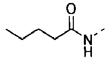 | 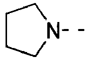 | *N*-[(2R,12S,20aS)-16-fluoro-8,13-dimethyl-5,9,14-trioxo-12-(1-pyrrolidinylcarbonyl)-2,3,5,6,7,8,9,10,11,12,13,14,20,20a-tetradecahydro-1*H-*pyrrolo[2,1-*c*][1,4,8,13]benzoxatriazacyclohexadecin-2-yl]pentanamide |
| **Ex.152** | 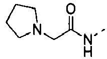 | 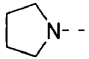 | *N*-[(2*R*,12*S*,20a*S*)-16-fluoro-8,13-dimethyl-5,9,14-trioxo-12-(1-pyrrolidinylcarbonyl)-2,3,5,6,7,8,9,10,11,12,13,14,20,20a-tetradecahydro-1*H-*pyrrolo[2,1-*c*][1,4,8,13]benzoxatriazacyclohexadecin-2-yl]-2-(1-pyrrolidinyl)acetamide |
| **Ex.153** | 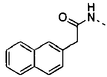 | 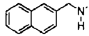 | (2*R*,12*S*,20a*S*)-16-fluoro-8,13-dimethyl-2-{[2-(2-naphthyl)acetyl]amino}-*N*-(2-naphthylmethyl)-5,9,14-trioxo-2,3,5,6,7,8,9,10,11,12,13,14,20,20a-tetradecahydro-1*H*-pyrrolo[2,1-*c*][1,4,8,13]benzoxatriazacyclohexadecine-12-carboxamide |
| **Ex.154** | 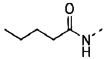 | 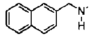 | (2*R*,12*S*,20a*S*)-16-fluoro-8,13-dimethyl-*N*-(2-naphthylmethyl)-5,9,14-trioxo-2-(pentanoylamino)-2,3,5,6,7,8,9,10,11,12,13,14,20,20a-tetradecahydro-1*H-*pyrrolo[2,1-*c*][1,4,8,13]benzoxatriazacyclohexadecine-12-carboxamide |
| **Ex.155** | 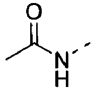 | 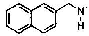 | (2*R*,12*S*,20a*S*)-2-(acetylamino)-16-fluoro-8,13-dimethyl-*N*-(2-naphthylmethyl)-5,9,14-trioxo-2,3,5,6,7,8,9,10,11,12,13,19,20,20a-tetradecahydro-1*H-*pyrrolo[2,1-c][1,4,8,13]benzoxatriazacyclohexadecine-12-carboxamide |
| **Ex.156** | 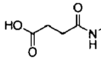 | 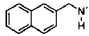 | 4-[((2*R*,12*S*,20a*S*)-16-fluoro-8,13-dimethyl-12-{[(2-naphthylmethyl)amino]carbonyl}-5,9,14-trioxo-2,3,5,6,7,8,9,10,11,12,13,14,20,20a-tetradecahydro-1*H*-pyrrolo[2,1-*c*][1,4,8,13]benzoxatriazacyclohexadecin-2-yl)amino]-4-oxobutanoic acid |
| **Ex.157** | 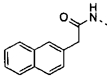 | 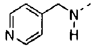 | (2*R*,12*S*,20a*S*)-16-fluoro-8,13-dimethyl-2-{[2-(2-naphthyl)acetyl]amino}-5,9,14-trioxo-*N*-(4-pyridinylmethyl)-2,3,5,6,7,8,9,10,11,12,13,14,20,20a-tetradecahydro-1*H*-pyrrolo[2,1-*c*][1,4,8,13]benzoxatriazacyclohexadecine-12-carboxamide |
| **Ex.158** | 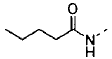 | 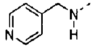 | (2*R*,12*S*,20a*S*)-16-fluoro-8,13-dimethyl-5,9,14-trioxo-2-(pentanoylamino)-*N*-(4-pyridinylmethyl)-2,3,5,6,7,8,9,10,11,12,13,19,20,20a-tetradecahydro-1*H-*pyrrolo[2,1-*c*][1,4,8,13]benzoxatriazacyclohexadecine-12-carboxamide |
| **Ex.159** | 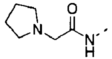 | 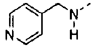 | (2*R*,12*S*,20a*S*)-16-fluoro-8,13-dimethyl-5,9,14-trioxo-N-(4-pyridinylmethyl)-2-{[2-(1-pyrrolidinyl)acetyl]amino}-2,3,5,6,7,8,9,10,11,12,13,14,20,20a-tetradecahydro-*1*H-pyrrolo[2,1-c][1,4,8,13]benzoxatriazacyclohexadecine-12-carboxamide |
| **Ex.160** | 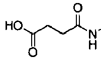 | 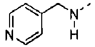 | 4-[((2*R*,12*S*,20a*S*)-16-fluoro-8,13-dimethyl-5,9,14-trioxo-12-{[(4-pyridinylmethyl)amino]carbonyl}-2,3,5,6,7,8,9,10,11,12,13,14,20,20a-tetradecahydro-1*H*-pyrrolo[2,1-*c*][1,4,8,13]benzoxatriazacyclohexadecin-2-yl)amino]-4-oxobutanoic acid |
| **Ex.161** | 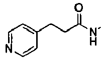 | 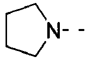 | N-[(2*R*,12*S*,20a*S*)-16-fluoro-8,13-dimethyl-5,9,14-trioxo-12-(1-pyrrolidinylcarbonyl)-2,3,5,6,7,8,9,10,11,12,13,14,20,20a-tetradecahydro-1*H-*pyrrolo[2,1-*c*][1,4,8,13]benzoxatriazacyclohexadecin-2-yl]-3-(4-pyridinyl)propanamide |
| **Ex.162** | 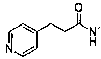 | 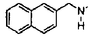 | (2*R*,12*S*,20aS)-16-fluoro-8,13-dimethyl-*N*-(2-naphthylmethyl)-5,9,14-trioxo-2-{[3-(4-pyridinyl)propanoyl]amino}-2,3,5,6,7,8,9,10,11,12,13,14,20,20a-tetradecahydro-1*H*-pyrrolo[2,1-c][1,4,8,13]benzoxatriazacyclohexadecine-12-carboxamide |
| **Ex.163** | 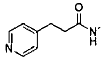 | 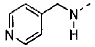 | (2*R*,12*S*,20a*S*)-16-fluoro-8,13-dimethyl-5,9,14-trioxo-*N*-(4-pyridinylmethyl)-2-{[3-(4-pyridinyl)propanoyl]amino}-2,3,5,6,7,8,9,10,11,12,13,14,20,20a-tetradecahydro-1*H*-pyrrolo[2,1-*c*][1,4,8,13]benzoxatriazacyclohexadecine-12-carboxamide |

**Table 28a: Examples of Core 10 (Ex.164-Ex.180; continued on the following pages)**

| **No** | **R²** | **R⁵** | **Starting material** | **General Procedure** | **Reagent** | **Purification Method** | **Yield (isolated salt)** |
|---|---|---|---|---|---|---|---|
| **Ex.164-Ex.165:** *cf. experimental description* | | | | | | | |
| **Ex.166** | 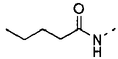 | 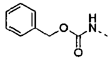 | **Ex.165** | L.1.1 | valeroyl chloride (2.0 equiv.) | FC (EtOAc/MeOH) | ca.70% |
| **Ex.167** | 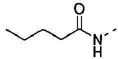 | NH₂ | **Ex.166** | K.1 | H₂, Pd(OH)₂-C | crude product | quant. |
| **Ex.168** | 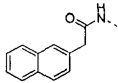 | 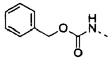 | **Ex.165** | L.1.3 | 2-naphthylacetic acid | FC (EtOAc) | 68% |
| **Ex.169** | 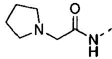 | 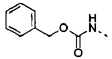 | **Ex.165** | L.1.3 | 1-pyrrolidinacetic acid | FC (EtOAc/MeOH) | 76% |
| **Ex.170** | 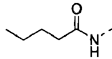 | 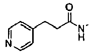 | **Ex.167** | L.1.2 | 3-(pyridine-4-yl)propanoic acid | FC (CH₂Cl₂/MeOH) | 78% |
| **Ex.171** | 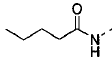 | 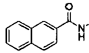 | **Ex.167** | L.1.1 | 2-naphthoyl chloride (2.6 equiv.) | Prep. HPLC, method 3 | 42% |
| **Ex.172** | 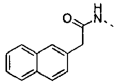 | NH₂ | **Ex.168** | K.1 | H₂, Pd(OH)₂-C | crude product | 97% |
| **Ex.173** | 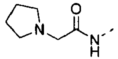 | NH₂ | **Ex.169** | K.1 | H₂, Pd(OH)₂-C | crude product | 96% |
| **Ex.174** | 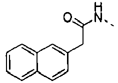 | 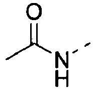 | **Ex.172** | L.1.1 | acetic anhydride (5 equiv.) | Prep. HPLC, method 3 | 97% |
| **Ex.175** | 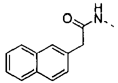 | 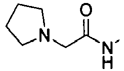 | **Ex.172** | L.1.2 | 1-pyrrolidinacetic acid (2.2 equiv.) | Prep. HPLC, method 3 | 58% |
| **Ex.176** | 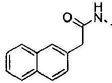 | 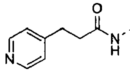 | **Ex.172** | L.1.2 | 3-(pyridine-4-yl)propanoic acid | Prep. HPLC, method 3 | 71% |
| **Ex.177** | 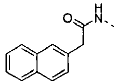 | 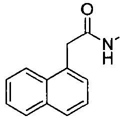 | **Ex.172** | L.1.2*⁾ | 1-naphthylacetic acid | Prep. HPLC, method 3 | 68% |
| **Ex.178** | 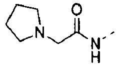 | 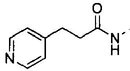 | **Ex.173** | L.1.2 | 3-(pyridine-4-yl)propanoic acid | Prep. HPLC, method 2 | 48% |
| **Ex.179** | 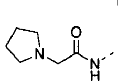 | 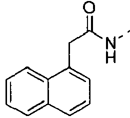 | **Ex.173** | L.1.2*⁾ | 1-naphthylacetic acid | Prep. HPLC, method 3 | 84% |
| **Ex.180** | 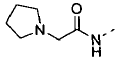 | 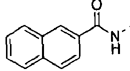 | **Ex.173** | L.1.1*⁾ | 2-naphthoyl chloride (1.5 equiv.) | Prep. HPLC, method 3 | 78% |

| | | | | | | | |
|---|---|---|---|---|---|---|---|
| *) The treatment with (polystyrylmethyl)trimethylammonium bicarbonate was replaced by an aqueous workup (CHCl₃, sat. aq. NaHCO₃ soln) | | | | | | | |

**Table 28b: Examples of Core 10 (Ex.164-Ex.180; continued on the following pages)**

| **No** | **R²** | **R⁵** | **FORMULA** | **MONOISOTOPIC MASS** | **Rt (purity at 220nm)** | **[M+H]⁺ found** | **LC-MS-Method** |
|---|---|---|---|---|---|---|---|
| **Ex.164-Ex.165:** *cf. experimental description* | | | | | | | |
| **Ex.166** | 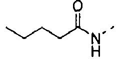 | 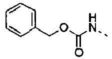 | C30H38N4O6 | 550.3 | 1.96 (99) | 551.3 | Method 4a |
| **Ex.167** | 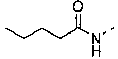 | NH₂ | C22H32N4O4 | 416.2 | 1.4 (99) | 417.3 | Method 4a |
| **Ex.168** | 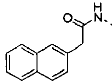 | 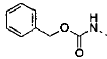 | C37H38N4O6 | 634.3 | 2.2 (98) | 635.3 | Method 4a |
| **Ex.169** | 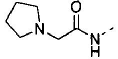 | 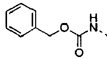 | C31H39N5O6 | 577.3 | 1.57 (99) | 578.4 | Method 4a |
| **Ex.170** | 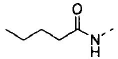 | 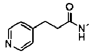 | C30H39N5O5 | 549.3 | 1.47 (97) | 550.4 | Method 4a |
| **Ex.171** | 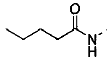 | 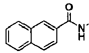 | C33H38N4O5 | 570.3 | 2.1 (100) | 571.3 | Method 4a |
| **Ex.172** | 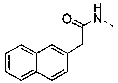 | NH₂ | C29H32N4O4 | 500.2 | 1.60 (91) | 501.3 | Method 4a |
| **Ex.173** | 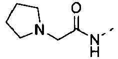 | NH₂ | C23H33N5O4 | 443.3 | 1.48 (99) | 444.2 | Method 5a |
| **Ex.174** | 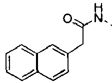 | 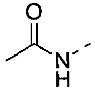 | C31H34N4O5 | 542.3 | 1.82 (99) | 543.2 | Method 4a |
| **Ex.175** | 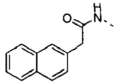 | 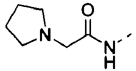 | C35H41N5O5 | 611.3 | 1.69 (96) | 612.2 | Method 4a |
| **Ex.176** | 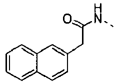 | 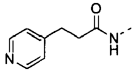 | C37H39N5O5 | 633.3 | 1.68 (99) | 634.3 | Method 4a |
| **Ex.177** | 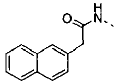 | 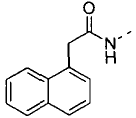 | C41H40N4O5 | 668.3 | 2.24 (92) | 669.3 | Method 4a |
| **Ex.178** | 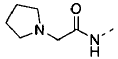 | 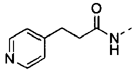 | C31H40N6O5 | 576.3 | 1.07 (98) | 577.3 | Method 4a |
| **Ex**.**179** | 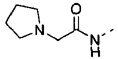 | 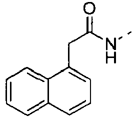 | C35H41N5O5 | 611.3 | 1.69 (93) | 612.3 | Method 4a |
| **Ex.180** | 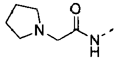 | 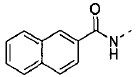 | C34H39N5O5 | 597.3 | 1.69 (97) | 597.8 | Method 4a |

**Table 28c: Examples of Core 10 (Ex.164-Ex.180; continued on the following pages)**

| **No** | **R²** | **R⁵** | **IUPAC name** |
|---|---|---|---|
| **Ex.164** | 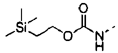 | 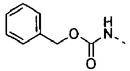 | benzyl *N*-[(4*S*,6*S*,10*S*)-13-methyl-9,14-dioxo-6-({[2-(trimethylsilyl)ethoxy]carbonyl}amino)-2-oxa-8,13-diazatricyclo[13.3.1.0^{4,8}]nonadeca-1(19),15,17-trien-10-yl]carbamate |
| **Ex.165** | NH₂ | 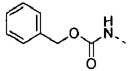 | benzyl *N*-[(4*S*,6*S*,10*S*)-6-amino-13-methyl-9,14-dioxo-2-oxa-8,13-diazatricyclo[13.3.1.0^{4,8}]nonadeca-1(19),15,17-trien-10-yl]carbamate |
| **Ex.166** | 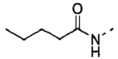 | 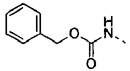 | benzyl *N*-[(4*S*,6*S*,10*S*)-13-methyl-9,14-dioxo-6-(pentanoylamino)-2-oxa-8,13-diazatricyclo[13.3.1.0^{4,8}]nonadeca-1(19),15,17-trien-10-yl]carbamate |
| **Ex.167** | 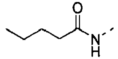 | NH₂ | *N*-[(4*S*,6*S*,10S)-10-amino-13-methyl-9,14-dioxo-2-oxa-8,13-diazatricyclo[13.3.1.0^{4,8}]nonadeca-1(19),15,17-trien-6-yl]pentanamide |
| **Ex.168** | 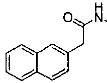 | 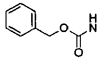 | benzyl *N*-[(4*S*,6*S*,10*S*)-13-methyl-6-{[2-(2-naphthyl)acetyl]amino}-9,14-dioxo-2-oxa-8,13-diazatricyclo[13.3.1.0^{4,8}]nonadeca-1(19),15,17-trien-10-yl]carbamate |
| **Ex.169** | 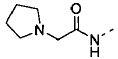 | 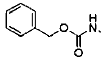 | benzyl *N*-[(4*S*,6*S*,10*S*)-13-methyl-9,14-dioxo-6-{[2-(1-pyrrolidinyl)acetyl]amino}-2-oxa-8,13-diazatricyclo[13.3.1.0^{4,8}]nonadeca-1(19),15,17-trien-10-yl]carbamate |
| **Ex.170** | 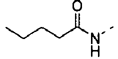 | 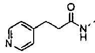 | *N*-[(4*S*,6*S*,10*S*)-13-methyl-9,14-dioxo-10-{[3-(4-pyridinyl)propanoyl]amino}-2-oxa-8,13-diazatricyclo[13.3.1.0^{4,8}]nonadeca-1(19),15,17-trien-6-yl]pentanamide |
| **Ex.171** | 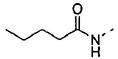 | 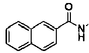 | *N*-[(4*S*,6*S*,10*S*)-13-methyl-9,14-dioxo-6-(pentanoylamino)-2-oxa-8,13-diazatricyclo[13.3.1.0^{4,8}]nonadeca-1(19),15,17-trien-10-yl]-2-naphthamide |
| **Ex.172** | 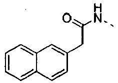 | NH₂ | *N*-[(4*S*,6*S*,10*S*)-10-amino-13-methyl-9,14-dioxo-2-oxa-8,13-diazatricyclo[13.3.1.0^{4,8}]nonadeca-1(19),15,17-trien-6-yl]-2-(2-naphthyl)acetamide |
| **Ex.173** | 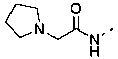 | NH₂ | *N*-[(4*S*,6*S*,10*S*)-10-amino-13-methyl-9,14-dioxo-2-oxa-8,13-diazatricyclo[13.3.1.0^{4,8}]nonadeca-1(19),15,17-trien-6-yl]-2-(1-pyrrolidinyl)acetamide |
| **Ex.174** | 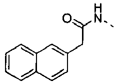 | 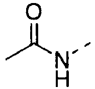 | *N*-[(4*S*,6*S*,10*S*)-10-(acetylamino)-13-methyl-9,14-dioxo-2-oxa-8,13-diazatricyclo[13.3.1.0^{4,8}]nonadeca-1(19),15,17-trien-6-yl]-2-(2-naphthyl)acetamide |
| **Ex.175** | 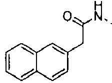 | 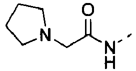 | *N*-[(4*S*,6*S*,10*S*)-13-methyl-9,14-dioxo-10-{[2-(1-pyrrolidinyl)acetyl]amino}-2-oxa-8,13-diazatricyclo[13.3.1.0^{4,8}]nonadeca-1(19),15,17-trien-6-yl]-2-(2-naphthyl)acetamide |
| **Ex.176** | 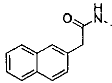 | 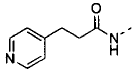 | *N*-[(4*S*,6*S*,10*S*)-13-methyl-6-{[2-(2-naphthyl)acetyl]amino}-9,14-dioxo-2-oxa-8,13-diazatricyclo[13.3.1.0^{4,8}]nonadeca-1(19),15,17-trien-10-yl]-3-(4-pyridinyl)propanamide |
| **Ex.177** | 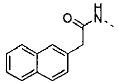 | 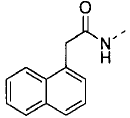 | *N*-[(4*S*,6*5*,10*S*)-13-methyl-6-{[2-(2-naphthyl)acetyl]amino}-9,14-dioxo-2-oxa-8,13-diazatricyclo[13.3.1.0^{4,8}]nonadeca-1(19),15,17-trien-10-yl]-2-(1-naphthyl)acetamide |
| **Ex.178** | 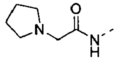 | 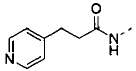 | *N*-[(4*S*,6*S*,10*S*)-13-methyl-9,14-dioxo-6-{[2-(1-pyrrolidinyl)acetyl]amino}-2-oxa-8,13-diazatricyclo[13.3.1.0^{4,8}]nonadeca-1(19),15,17-trien-10-yl]-3-(4-pyridinyl)propanamide |
| **Ex.179** | 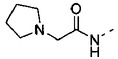 | 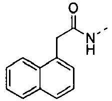 | *N*-[(4*S*,6*S*,10*S*)-13-methyl-9,14-dioxo-6-{[2-(1-pyrrolidinyl)acetyl]amino}-2-oxa-8,13-diazatricyclo[13.3.1.0^{4,8}]nonadeca-1(19),15,17-trien-10-yl]-2-(1-naphthyl)acetamide |
| **Ex.180** | 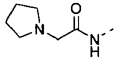 | 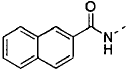 | *N*-[(4*S*,6*S*,10*S*)-13-methyl-9,14-dioxo-6-{[2-(1-pyrrolidinyl)acetyl]amino}-2-oxa-8,13-diazatricyclo[13.3.1.0^{4,8}]nonadeca-1(19),15,17-trien-10-yl]-2-naphthamide |

**Table 29.1.a: Examples of Core 11a (Ex.181-Ex.195, Ex.504-Ex.559 and Ex.594; continued on the following pages)**

| **No** | **R²** | **R⁵** | **Starting material** | **General Procedure** | **Reagent** | **Purification Method** | **Yield (isolated salt)** |
|---|---|---|---|---|---|---|---|
| **Ex.181-Ex.182:** *cf. experimental description* | | | | | | | |
| **Ex.183** | 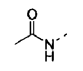 | 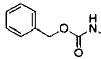 | **Ex.182** | L.1.1 | acetic anhydride (5 equiv.) | FC (CH₂Cl₂/MeOH) | 75% |
| **Ex.184** | 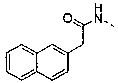 | 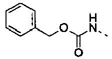 | **Ex.182** | L.1.3 | 2-naphthylacetic acid (1.2 equiv.) | FC (EtOAc, then CH₂Cl₂/MeOH) | 94% |
| **Ex.185** | 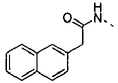 | NH₂ | **Ex.184** | K.1 | H₂, Pd(OH)₂-C | crude product | 99% |
| **Ex.186** | 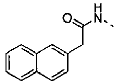 | 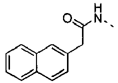 | **Ex.185** | L.1.3 | 2-naphthylacetic acid (1.2 equiv.) | prep. HPLC, method 3 | 60% |
| **Ex.186** | 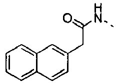 | 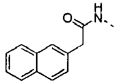 | **Ex.594** | S | 2-naphthylacetic acid | prep. HPLC, method 3 | 32% |
| **Ex.187** | 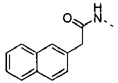 | 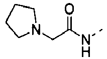 | **Ex.185** | L.1.3 | pyrrolidin-1-acetic acid (1.2 equiv.) | prep. HPLC, method 3 then prep. HPLC, method 1 | 38% (TFA salt) |
| **Ex**.**188** | 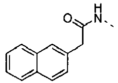 | 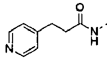 | **Ex.185** | L.1.3 | 3-(pyridine-4-yl)propanoic acid (1.2 equiv.) | prep. HPLC, method 3 then prep. HPLC, method 1 | 32% (TFA salt) |
| **Ex.189** | 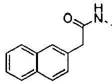 | 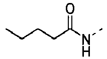 | **Ex.185** | L.1.1 | valeroyl chloride (2 equiv.) | prep. HPLC, method 3 | 52% |
| **Ex.190** | NH₂ | 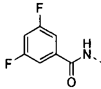 | **Ex.193** | I.2 | TBAF (4 equiv.) in THF | crude product, contaminated with TBAF | ca 70% |
| **Ex.191** | 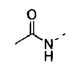 | 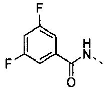 | **Ex.190** | L.1.1 | Acetic anhydride (10 equiv.) Pyridine/CH₂Cl₂ 1:1 (3 mL) | prep. HPLC, method 3 | 62% |
| **Ex.192** | 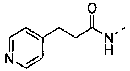 | 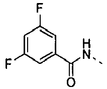 | **Ex.190** | L.1.3 | 3-(pyridine-4-yl)propanoic acid (1.2 equiv.) | prep. HPLC, method 3 | 63% |
| **Ex.193** | 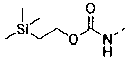 | 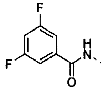 | **Ex.194** | L.1.1 | 3,5-difluorobenzoyl chloride (2 equiv.) | FC (EtOAc) | 85% |
| **Ex.194** | 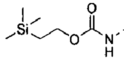 | NH₂ | **Ex.181** | K.1 | H₂, Pd(OH)₂-C | crude product | quant. |
| **Ex.195** | 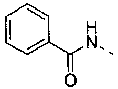 | 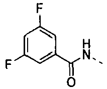 | **Ex.190** | L.1.1 | benzoyl chloride (2 equiv.) | prep. HPLC, method 3 | 78% |
| **Ex.504** | 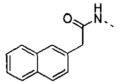 | CH₃(CH₂)₈CONH | **140** | S | cf. detailed description | prep. HPLC method 1 | 26% |
| **Ex.505** | 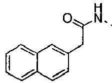 | 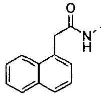 | **140** | S | cf. detailed description | prep. HPLC method 3 | 33% |
| **Ex.506** | 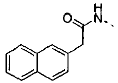 | 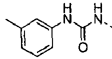 | **140** | S | cf. detailed description | prep. HPLC method 3 | 45% |
| **Ex.507** | 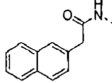 | 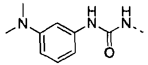 | **140** | S | cf. detailed description | prep. HPLC method 3 | 48% |
| **Ex.508** | 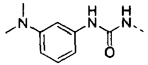 | 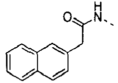 | **Ex.554** | L.1.3 | cf. detailed description | prep. HPLC method 3 | 57% |
| **Ex.509** | 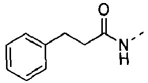 | CH₃(CH₂)₈CONH | **140** | S | cf. detailed description | prep. HPLC method 3 | 48% |
| **Ex.510** | 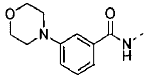 | CH₃(CH₂)₈CONH | **140** | S | cf. detailed description | prep. HPLC method 3 | 20% |
| **Ex.511** | 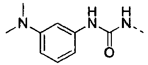 | CH₃(CH₂)₈CONH | **140** | S | cf. detailed description | prep. HPLC method 1 | 55% (TFA salt) |
| **Ex.512** | 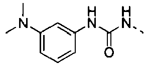 | 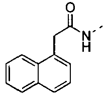 | **140** | S | cf. detailed description | prep. HPLC method 1 | 56% (TFA salt) |
| **Ex.513** | 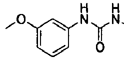 | 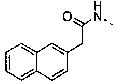 | **140** | S | cf. detailed description | prep. HPLC method 3 | 25% |
| **Ex.514** | 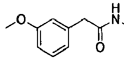 | 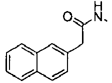 | **140** | S | cf. detailed description | prep. HPLC method 3 | 54% |
| **Ex.515** | 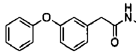 | CH₃(CH₂)₈CONH | **140** | S | cf. detailed description | prep. HPLC method 3 | 48% |
| **Ex.516** | 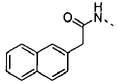 | 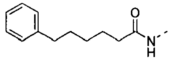 | **140** | S | cf. detailed description | prep. HPLC method 3 | 64% |
| **Ex.517** | 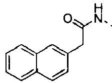 | 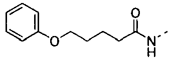 | **140** | S | cf. detailed description | prep. HPLC method 3 | 57% |
| **Ex.518** | 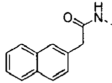 | 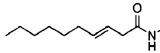 | **140** | S | cf. detailed description | prep. HPLC method 3 | 52% |
| **Ex.519** | 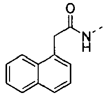 | CH₃(CH₂)₈CONH | **140** | S | cf. detailed description | prep. HPLC method 3 | 59% |
| **Ex.520** | 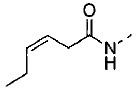 | 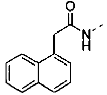 | **140** | S | cf. detailed description | prep. HPLC method 3 | 59% |
| **Ex.521** | 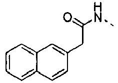 | 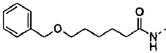 | **140** | S | cf. detailed description | prep. HPLC method 3 | 68% |
| **Ex.522** | 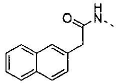 | 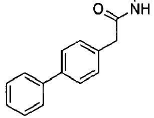 | **140** | S | cf. detailed description | prep. HPLC method 3 | 62% |
| **Ex.523** | 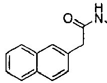 | 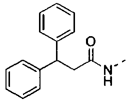 | **140** | S | cf. detailed description | prep. HPLC method 3 | 63% |
| **Ex.524** | 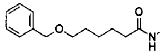 | 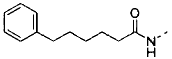 | **140** | S | cf. detailed description | prep. HPLC method 3 | 38% |
| **Ex.525** | 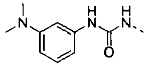 | 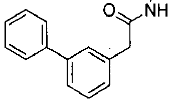 | **140** | S | cf. detailed description | prep. HPLC method 3 | 54% |
| **Ex**.**526** | 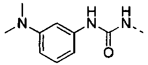 | 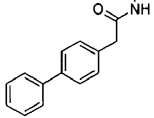 | **140** | S | cf. detailed description | prep. HPLC method 3 | 54% |
| **Ex**.**527** | 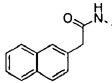 | 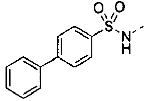 | **140** | S | cf. detailed description | prep. HPLC method 3 | 61% |
| **Ex.528** | 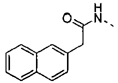 | 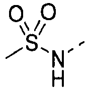 | **140** | S | 1. 2-naphthylacetic acid | prep. HPLC method 3 | 45% |
| | | | | | 2. methanesulfonyl chloride | | |
| **Ex.529** | 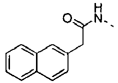 | 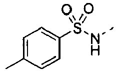 | **140** | S | 1. 2-naphthyl-acetic acid | prep. HPLC method 3 | 61% |
| | | | | | 2. toluene-4-sulfonyl chloride | | |
| **Ex.530** | 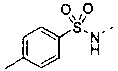 | 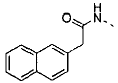 | **140** | S | 1. toluene-4-sulfonyl chloride | prep. HPLC method 3 | 19% |
| | | | | | 2. 2-naphthyl-acetic acid | | |
| **Ex.531** | 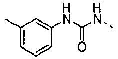 | 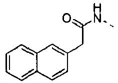 | **140** | S | 1. 3-methylphenyl isocyanate | prep. HPLC method 3 | 44% |
| | | | | | 2. 2-naphthylacetic acid | | |
| **Ex.532** | 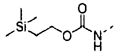 | 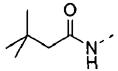 | **Ex.194** | L.1.1 | cf. detailed description | FC (hexane/EtOAc/ MeOH) | 91% |
| **Ex.533** | NH₂ | 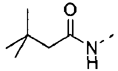 | **Ex.532** | - | cf. detailed description | crude product | 80% |
| **Ex.534** | 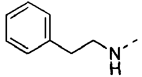 | 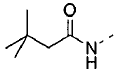 | **Ex.533** | M.2 | 2-phenylacetaldehyde | prep. HPLC method 3 | 59% |
| **Ex.535** | 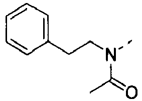 | 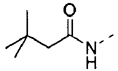 | **Ex**.**558** | L.1.1 | 3,3-dimethylbutanoyl chloride (1.1 equiv.) pyridine (5 equiv.) | FC (hexane/EtOAc/ MeOH) | 91% |
| **Ex.536** | 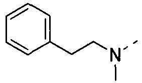 | 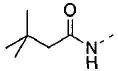 | **Ex.559** | L.1.1 | 3,3-dimethylbutanoyl chloride (1.1 equiv.) pyridine (5 equiv.) | FC (hexane/EtOAc/ MeOH) | 74% |
| **Ex.537** | 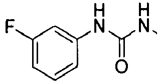 | 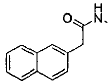 | **140** | S | cf. detailed description | prep. HPLC method 3 | 63% |
| **Ex.538** | 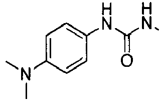 | 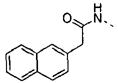 | **140** | S | cf. detailed description | prep. HPLC method 1 | 62% (TFA salt) |
| **Ex.539** | 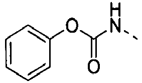 | 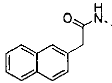 | **140** | S | cf. detailed description | prep. HPLC method 3 | 46% |
| **Ex.540** | 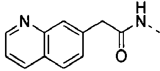 | 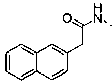 | **140** | S | cf. detailed description | prep. HPLC method 3 | 53% |
| **Ex.541** | 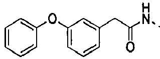 | 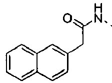 | **140** | S | cf. detailed description | prep. HPLC method 3 | 68% |
| **Ex.542** | CH₃(CH₂)₈CONH | 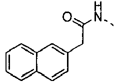 | **140** | S | cf. detailed description | prep. HPLC method 3 | 56% |
| **Ex.543** | CH₃(CH₂)₈CONH | 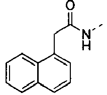 | **140** | S | cf. detailed description | prep. HPLC method 3 | 61% |
| **Ex.544** | CH₃(CH₂)₄CONH | 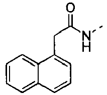 | **140** | S | cf. detailed description | prep. HPLC method 3 | 54% |
| **Ex.545** | 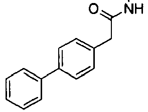 | 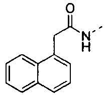 | **140** | S | cf. detailed description | prep. HPLC method 3 | 65% |
| **Ex.546** | 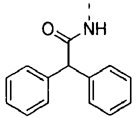 | 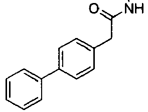 | **140** | S | cf. detailed description | prep. HPLC method 1 | 20% |
| **Ex.547** | 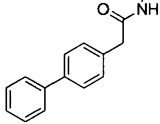 | 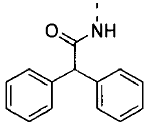 | **140** | S | cf. detailed description | prep. HPLC method 3 | 65% |
| **Ex.548** | 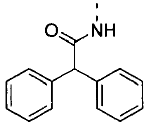 | CH₃(CH₂)₈CONH | **140** | S | cf. detailed description | prep. HPLC method 1 | 28% |
| **Ex.549** | 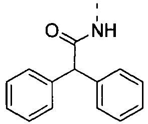 | 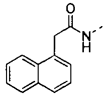 | **140** | S | cf. detailed description | prep. HPLC method 1 | 23% |
| **Ex.550** | 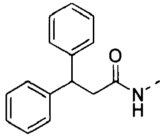 | CH₃(CH₂)₈CONH | **140** | S | cf. detailed description | prep. HPLC method 3 | 49% |
| **Ex.551** | 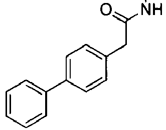 | 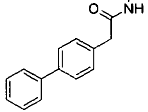 | **140** | S | cf. detailed description | prep. HPLC method 1 | 18% |
| **Ex.552** | 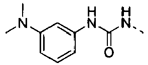 | 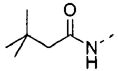 | **Ex.533** | L.4 | cf. detailed description | prep. HPLC method 3 | 83% |
| **Ex.553** | 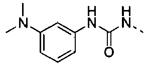 | 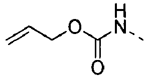 | **Ex.594** | L.4 | cf. detailed description | FC (hexane/EtOAc/ MeOH) | 91% |
| **Ex.554** | 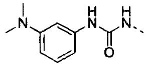 | NH₂ | **Ex.534** | B.2 | cf. detailed description | FC (CH₂Cl₂/MeOH) | 93% |
| **Ex.555** | 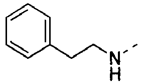 | 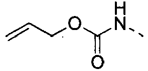 | **Ex.594** | M.2 | 2-phenylacetaldehyde | prep. HPLC method 3 | 73% |
| **Ex.556** | 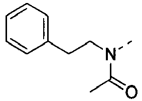 | 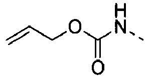 | **Ex.555** | L.1.1 | acetic anhydride (5 equiv.) pyridine (10 equiv.) | FC (hexane/EtOAc/ MeOH) | 67% |
| **Ex**.**557** | 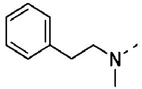 | 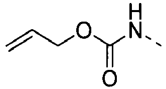 | **Ex.555** | M.3 | aq. formaldehyde soln | FC (hexane/EtOAc/ MeOH) | 86% |
| **Ex**.**558** | 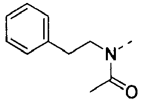 | NH₂ | **Ex**.**556** | B.2 | Pd(PPh₃)₄, 1,3-dimethyl-barbituric acid | FC (CH₂Cl₂/MeOH/ aq. NH₃) | 96% |
| **Ex.559** | 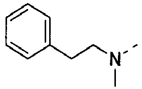 | NH₂ | **Ex.556** | B.2 | Pd(PPh₃)₄, 1,3-dimethyl-barbituric acid | FC (CH₂Cl₂MeOH/ aq. NH₃) | 86% |
| **Ex.594** | NH₂ | 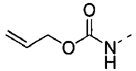 | **Ex.181** | *) | 1. H₂, Pd(OH)₂-C | *) | ca 80% (HCl salt) |
| | | | | | 2. AllocCl | | |
| | | | | | 3. TBAF in THF | | |

| | | | | | | | |
|---|---|---|---|---|---|---|---|
| *) cf. detailed description | | | | | | | |

**Table 29.1.b: Examples of Core 11a (Ex.181-Ex.195, Ex.504-Ex.559 and Ex.594; continued on the following pages)**

| **No** | **R²** | **R⁵** | **FORMULA** | **MONOISOTOPIC MASS** | **Rt (purity at 220nm)** | **[M+H]⁺ found** | **LC-MS-Method** |
|---|---|---|---|---|---|---|---|
| **Ex.181-Ex.182:** *cf. experimental description* | | | | | | | |
| **Ex.183** | 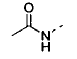 | 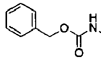 | C28H34N4O6 | 522.3 | 1.56 (99) | 523.3 | Method 2 |
| **Ex.184** | 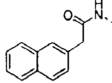 | 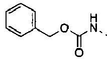 | C38H40N4O6 | 648.3 | 1.92 (96) | 649.5 | Method 2 |
| **Ex.185** | 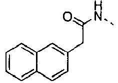 | NH₂ | C30H34N4O4 | 514.3 | 1.48 (98) | 515.4 | Method 2 |
| **Ex.186** | 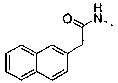 | 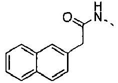 | C42H42N4O5 | 682.3 | 2.00 (91) | 683.5 | Method 2 |
| **Ex.186** | 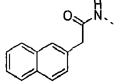 | 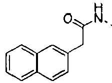 | C42H42N4O5 | 682.3 | 2.26 (98) | 683.3 | Method 4a |
| **Ex.187** | 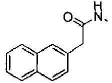 | 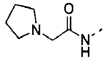 | C36H43N5O5 | 625.3 | 1.62 (100) | 626.4 | Method 2 |
| **Ex.188** | 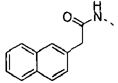 | 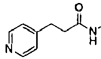 | C38H41N5O5 | 647.3 | 1.60 (99) | 648.3 | Method 2 |
| **Ex.189** | 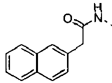 | 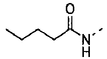 | C35H42N4O5 | 598.3 | 1.88 (85), 1.93 (8) | 599.5 | Method 2 |
| **Ex.190** | NH₂ | 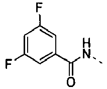 | C25H28F2N4O4 | 486.2 | 1.42 (98) | 487.3 | Method 4a |
| **Ex.191** | 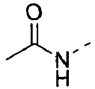 | 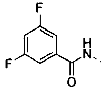 | C27H302N4O5 | 528.2 | 1.69 (99) | 529.3 | Method 4a |
| **Ex.192** | 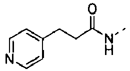 | 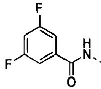 | C33H35F2N5O5 | 619.3 | 1.50 (99) | 620.3 | Method 4a |
| **Ex.193** | 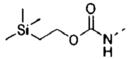 | 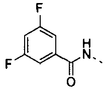 | C31H40F2N4O6Si | 630.3 | 2.13 (97) | 631.4 | Method 2 |
| **Ex.194** | 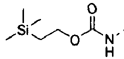 | NH₂ | C24H38N4O5Si | 490.3 | 1.62 (99) | 491.3 | Method 2 |
| **Ex.195** | 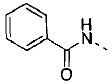 | 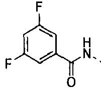 | C32H32F2N4O5 | 590.2 | 1.99 (98) | 591.3 | Method 4a |
| **Ex.504** | 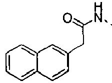 | CH₃(CH₂)₈CONH | C40H52N4O5 | 668.4 | 2.54 (99) | 669.3 | Method 4b |
| **Ex.505** | 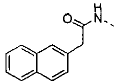 | 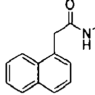 | C42H42N4O5 | 682.3 | 2.23 (95) | 683.3 | Method 4b |
| **Ex.506** | 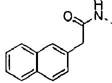 | 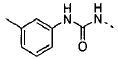 | C38H41N5O5 | 647.3 | 2.19 (99) | 648.3 | Method 4a |
| **Ex.507** | 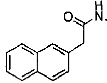 | 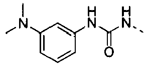 | C39H44N6O5 | 676.3 | 1.75 (94) | 677.3 | Method 4a |
| **Ex.508** | 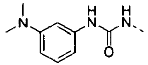 | 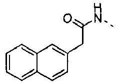 | C39H44N6O5 | 676.3 | 1.72 (94) | 677.3 | Method 4b |
| **Ex.509** | 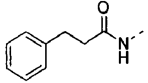 | CH₃(CH₂)₈CONH | C37H52N4O5 | 632.4 | 2.43 (98) | 633.3 | Method 4b |
| **Ex.510** | 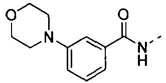 | CH₃(CH₂)₈CONH | C39H55N5O6 | 689.4 | 2.31 (99) | 690.3 | Method 4b |
| **Ex.511** | 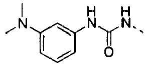 | CH₃(CH₂)₈CONH | C37H54N6O5 | 662.4 | 1.96 (99) | 663.4 | Method 4a |
| **Ex.512** | 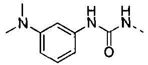 | 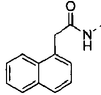 | C39H44N6O5 | 676.3 | 1.72 (98) | 677.3 | Method 4a |
| **Ex.513** | 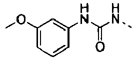 | 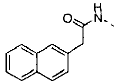 | C38H41N5O6 | 663.3 | 2.13 (95) | 664.3 | Method 4a |
| **Ex.514** | 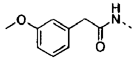 | 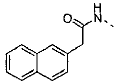 | C39H42N4O6 | 662.3 | 2.12 (98) | 663.3 | Method 4a |
| **Ex.515** | 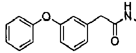 | CH₃(CH₂)₈CONH | C42H54N4O6 | 710.4 | 2.60 (97) | 711.4 | Method 4a |
| **Ex.516** | 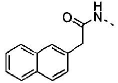 | 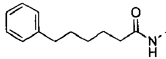 | C42H48N4O5 | 688.4 | 2.36 (97) | 689.4 | Method 4a |
| **Ex.517** | 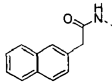 | 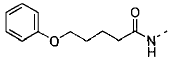 | C41H46N4O6 | 690.3 | 2.26 (98) | 691.4 | Method 4a |
| **Ex.518** | 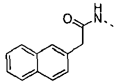 | 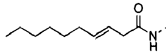 | C40H50N4O5 | 666.4 | 2.36 (92) | 667.5 | Method 10a |
| **Ex.519** | 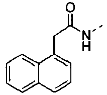 | CH₃(CH₂)₈CONH | C40H52N4O5 | 668.4 | 2.53 (94) | 669.4 | Method 4a |
| **Ex.520** | 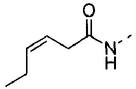 | 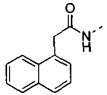 | C36H42N4O5 | 610.3 | 2.15 (98) | 611.3 | Method 4a |
| **Ex.521** | 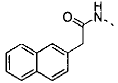 | 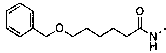 | C43H50N4O6 | 718.4 | 2.14 (92) | 719.5 | Method 10a |
| **Ex.522** | 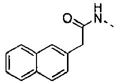 | 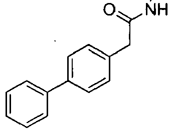 | C44H44N4O5 | 708.3 | 2.35 (98) | 709.4 | Method 4a |
| **Ex.523** | 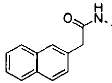 | 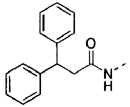 | C45H46N4O5 | 722.4 | 2.33 (97) | 723.4 | Method 4a |
| **Ex.524** | 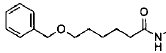 | 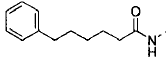 | C43H56N4O6 | 724.4 | 2.28 (95) | 725.5 | Method 10a |
| **Ex.525** | 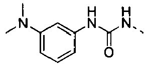 | 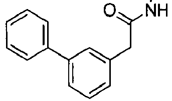 | C41H46N6O5 | 702.4 | 1.63 (97) | 703.4 | Method 10a |
| **Ex.526** | 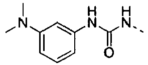 | 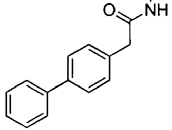 | C41H46N6O5 | 702.4 | 1.63 (97) | 703.4 | Method 10a |
| **Ex.527** | 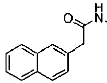 | 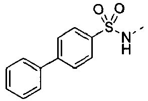 | C42H42N4O6S | 730.3 | 2.37 (95) | 731.3 | Method 4a |
| **Ex.528** | 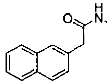 | 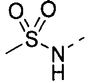 | C31H36N4O6S | 592.2 | 1.92 (95) | 593.2 | Method 4a |
| **Ex.529** | 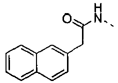 | 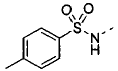 | C37H40N4O6S | 668.3 | 2.18 (96) | 669.3 | Method 4a |
| **Ex.530** | 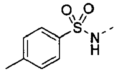 | 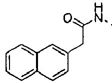 | C37H40N4O6S | 668.3 | 2.20 (91) | 669.3 | Method 4b |
| **Ex.531** | 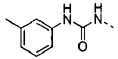 | 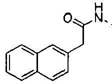 | C38H41N5O5 | 647.3 | 2.19 (98) | 648.3 | Method 4b |
| **Ex.532** | 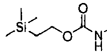 | 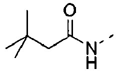 | C30H48N4O6Si | 588.3 | 2.27 (99) | 589.2 | Method 4b |
| **Ex.533** | NH₂ | 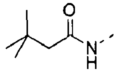 | C24H36N4O4 | 444.3 | 1.36 (99) | 445.3 | Method 4b |
| **Ex.534** | 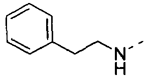 | 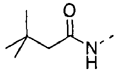 | C32H44N4O4 | 548.3 | 1.70 (86) | 549.4 | Method 4a |
| **Ex.535** | 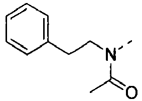 | 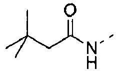 | C34H46N4O5 | 590.4 | 2.06 (95) | 591.4 | Method 4a |
| **Ex.536** | 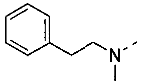 | 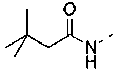 | C33H46N4O4 | 562.4 | 1.78 (97) | 563.4 | Method 4a |
| **Ex.537** | 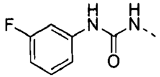 | 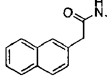 | C37H38FN5O5 | 651.3 | 2.18 (92) | 652.1 | Method 4a |
| **Ex.538** | 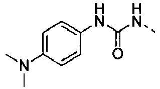 | 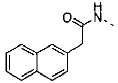 | C39H44N6O5 | 676.3 | 1.70 (98) | 677.3 | Method 4a |
| **Ex.539** | 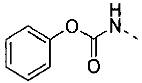 | 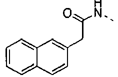 | C37H38N4O6 | 634.3 | 2.20 (97) | 635.3 | Method 4a |
| **Ex.540** | 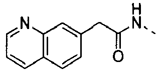 | 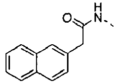 | C41H41N5O5 | 683.3 | 1.49 (98) | 684.1 | Method 10a |
| **Ex.541** | 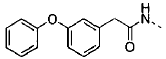 | 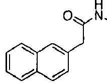 | C44H44N4O6 | 724.3 | 2.34 (96) | 725.4 | Method 4a |
| **Ex.542** | CH₃(CH₂)₈CONH | 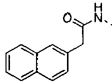 | C40H52N4O5 | 668.4 | 2.54 (99) | 669.4 | Method 4a |
| **Ex.543** | CH₃(CH₂)₈CONH | 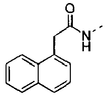 | C40H52N4O5 | 668.4 | 2.54 (95) | 669.4 | Method 4a |
| **Ex.544** | CH₃(CH₂)₄CONH | 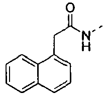 | C36H44N4O5 | 612.3 | 2.18 (98) | 613.3 | Method 4a |
| **Ex.545** | 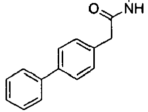 | 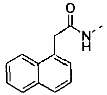 | C44H44N4O5 | 708.3 | 2.33 (97) | 709.4 | Method 4a |
| **Ex.546** | 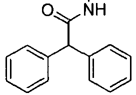 | 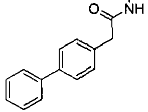 | C46H46N4O5 | 734.4 | 2.30 (99) | 735.4 | Method 10a |
| **Ex.547** | 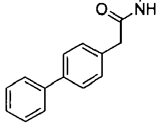 | 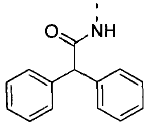 | C46H46N4O5 | 734.4 | 2.42 (99) | 735.4 | Method 4a |
| **Ex.548** | 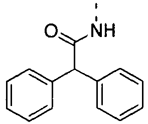 | CH₃(CH₂)₈CONH | C42H54N4O5 | 694.4 | 2.61 (98) | 695.4 | Method 4a |
| **Ex.549** | 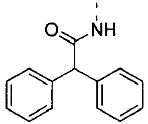 | 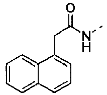 | C44H44N4O5 | 708.3 | 2.34 (97) | 709.4 | Method 4a |
| **Ex.550** | 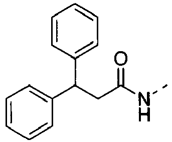 | CH₃(CH₂)₈CONH | C43H56N4O5 | 708.4 | 2.58 (98) | 709.5 | Method 4a |
| **Ex.551** | 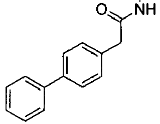 | 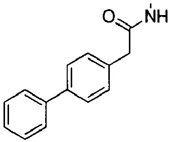 | C46H46N4O5 | 734.4 | 2.29 (95) | 735.4 | Method 10a |
| **Ex.552** | 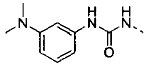 | 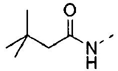 | C33H46N6O5 | 606.4 | 1.54 (99) | 607.3 | Method 4b |
| **Ex.553** | 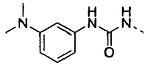 | 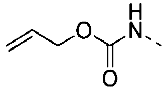 | C31H40N6O6 | 592.3 | 1.47 (99) | 593.3 | Method 4a |
| **Ex.554** | 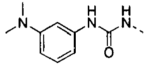 | NH₂ | C27H36N6O4 | 508.3 | 1.20 (95) | 509.2 | Method 4a |
| **Ex.555** | 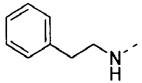 | 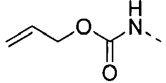 | C30H38N4O5 | 534.3 | 1.64 (93) | 535.3 | Method 4a |
| **Ex.556** | 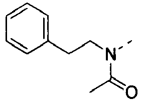 | 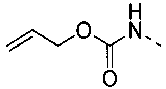 | C32H40N4O6 | 576.3 | 2.00 (93) | 577.3 | Method 4a |
| **Ex.557** | 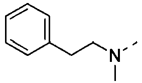 | 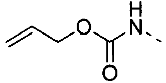 | C31H40N4O5 | 548.3 | 1.65 (92) | 549.4 | Method 4a |
| **Ex.558** | 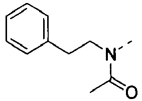 | NH₂ | C28H36N4O4 | 492.3 | 1.58 (94) | 493.2 | Method 4a |
| **Ex.559** | 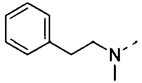 | NH₂ | C27H36N4O3 | 464.3 | 1.32 (86) | 465.3 | Method 4a |
| **Ex.594** | NH₂ | 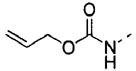 | C22H30N4O5 | 430.2 | 1.22 (92) | 431.3 | Method 4a |

**Table 29.1.c: Examples of Core 11a (Ex.181-Ex.195, Ex.504-Ex.559 and Ex.594; continued on the following pages)**

| **No** | **R²** | **R⁵** | **IUPAC name** |
|---|---|---|---|
| **Ex.181** | 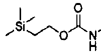 | 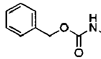 | benzyl *N*-[(4*S*,6*S*,10*S*)-14-methyl-9,15-dioxo-6-({[2-(trimethylsilyl)ethoxy]carbonyl}amino)-2-oxa-8,14-diazatricyclo[14.3.1.0^{4,8}]icosa-1(20),16,18-trien-10-yl]carbamate |
| **Ex.182** | NH₂ | 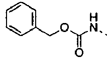 | benzyl *N*-[(4*S*,6*S*,10*S*)-6-amino-14-methyl-9,15-dioxo-2-oxa-8,14-diazatricyclo[14.3.1.0^{4,8}]icosa-1(20),16,18-trien-10-yl]carbamate |
| **Ex.183** | 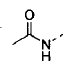 | 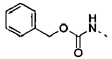 | benzyl N-[(4*S*,6*S*,10*S*)-6-(acetylamino)-14-methyl-9,15-dioxo-2-oxa-8,14-diazatricyclo[14.3.1.0^{4,8}]icosa-1(20),16,18-trien-10-yl]carbamate |
| **Ex.184** | 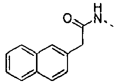 | 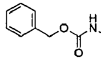 | benzyl *N*-[(4*S*,6*S*,10*S*)-14-methyl-6-{[2-(2-naphthyl)acetyl]amino}-9,15-dioxo-2-oxa-8,14-diazatricyclo[14.3.1.0^{4,8}]icosa-1(20),16,18-trien-10-yl]carbamate |
| **Ex.185** | 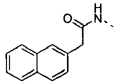 | NH₂ | *N*-[(4*S*,6*S*,10*S*)-10-amino-14-methyl-9,15-dioxo-2-oxa-8,14-diazatricyclo[14.3.1.0^{4,8}]icosa-1(20),16,18-trien-6-yl]-2-(2-naphthyl)acetamide |
| **Ex.186** | 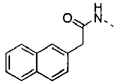 | 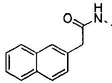 | *N*-[(4*S*,6*S*,10*S*)-14-methyl-10-{[2-(2-naphthyl)acetyl]amino}-9,15-dioxo-2-oxa-8,14-diazatricyclo[14.3.1.0^{4,8}]icosa-1(20),16,18-trien-6-yl]-2-(2-naphthyl)acetamide |
| **Ex.187** | 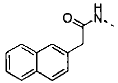 | 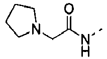 | *N*-[(4*S*,6*S*,10*S*)-14-methyl-9,15-dioxo-10-{[2-(1-pyrrolidinyl)acetyl]amino}-2-oxa-8,14-diazatricyclo[14.3.1.0^{4,8}]icosa-1(20),16,18-trien-6-yl]-2-(2-naphthyl)acetamide |
| **Ex.188** | 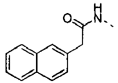 | 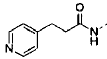 | *N*-[(4*S*,6*S*,10*S*)-14-methyl-6-{[2-(2-naphthyl)acetyl]amino}-9,15-dioxo-2-oxa-8,14-diazatricyclo[14.3.1.0^{4,8}]icosa-1(20),16,18-trien-10-yl]-3-(4-pyridinyl)propanamide |
| **Ex.189** | 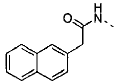 | 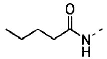 | *N*-[(4*S*,6*S*,10*S*)-14-methyl-6-{[2-(2-naphthyl)acetyl]amino}-9,15-dioxo-2-oxa-8,14-diazatricyclo[14.3.1.0^{4,8}]icosa-1(20),16,18-trien-10-yl]pentanamide |
| **Ex**.**190** | NH₂ | 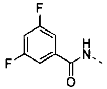 | *N*-[(4*S*,6*S*,10*S*)-6-amino-14-methyl-9,15-dioxo-2-oxa-8,14-diazatricyclo[14.3.1.0^{4,8}]icosa-1(20),16,18-trien-10-yl]-3,5-difluorobenzamide |
| **Ex.191** | 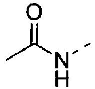 | 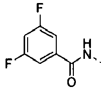 | *N*-[(4*S*,6*S*,10*S*)-6-(acetylamino)-14-methyl-9,15-dioxo-2-oxa-8,14-diazatricyclo[14.3.1.0^{4,8}]icosa-1(20),16,18-trien-10-yl]-3,5-difluorobenzamide |
| **Ex.192** | 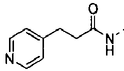 | 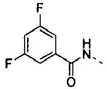 | 3,5-difluoro-*N*-[(4*S*,6*S*,10*S*)-14-methyl-9,15-dioxo-6-{[3-(4-pyridinyl)propanoyl]amino}-2-oxa-8,14-diazatricyclo[14.3.1.0^{4,8}]icosa-1(20),16,18-trien-10-yl]benzamide |
| **Ex.193** | 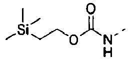 | 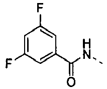 | 2-(trimethylsilyl)ethyl *N*-[(4*S*,6*S*,10*S*)-10-[(3,5-difluorobenzoyl)amino]-14-methyl-9,15-dioxo-2-oxa-8,14-diazatricyclo[14.3.1.0^{4,8}]icosa-1(20),16,18-trien-6-yl]carbamate |
| **Ex.194** | 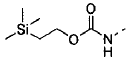 | NH₂ | 2-(trimethylsilyl)ethyl *N*-[(4*S*,6*S*,10*S*)-10-amino-14-methyl-9,15-dioxo-2-oxa-8,14-diazatricyclo[14.3.1.0^{4,8}]icosa-1(20),16,18-trien-6-yl]carbamate |
| **Ex.195** | 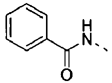 | 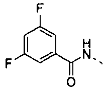 | *N*-[(4*S*,6*S*,10*S*)-6-(benzoylamino)-14-methyl-9,15-dioxo-2-oxa-8,14-diazatricyclo[14.3.1.0^{4,8}]icosa-1(20),16,18-trien-10-yl]-3,5-difluorobenzamide |
| **Ex.504** | 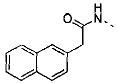 | CH₃(CH₂)₈CONH | *N*-[(4*S*,6*S*,10*S*)-14-methyl-6-{[2-(2-naphthyl)acetyl]amino}-9,15-dioxo-2-oxa-8,14-diazatricyclo[14.3.1.0^{4,8}]icosa-1(20),16,18-trien-10-yl]decanamide |
| **Ex.505** | 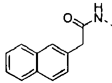 | 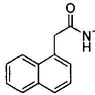 | *N*-[(4*S*,6*S*,10*S*)-14-methyl-6-{[2-(2-naphthyl)acetyl]amino}-9,15-dioxo-2-oxa-8,14-diazatricyclo[14.3.1.0^{4,8}]icosa-1(20),16,18-trien-10-yl]-2-(1-naphthyl)acetamide |
| **Ex.506** | 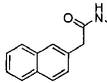 | 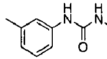 | *N*-[(4*S*,6*S*,10*S*)-14-methyl-9,15-dioxo-10-[(3-toluidinocarbonyl)amino]-2-oxa-8,14-diazatricyclo[14.3.1.0^{4,8}]icosa-1(20),16,18-trien-6-yl]-2-(2-naphthyl)acetamide |
| **Ex.507** | 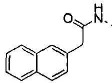 | 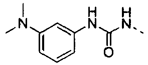 | *N*-[(4*S*,6*S*,10*S*)-10-({[3-(dimethylamino)aniline]carbonyl}amino)-14-methyl-9,15-dioxo-2-oxa-8,14-diazatricyclo[14.3.1.0^{4,8}]icosa-1(20),16,18-trien-6-yl]-2-(2-naphthyl)acetamide |
| **Ex.508** | 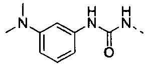 | 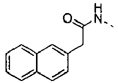 | *N*-[(4S,6S,10S)-6-({[3-(dimethylamino)anilino]carbonyl}amino)-14-methyl-9,15-dioxo-2-oxa-8,14-diazatricyclo[14.3.1.0^{4,8}]icosa-1(20),16,18-trien-10-yl]-2-(2-naphthyl)acetamide |
| **Ex.509** | 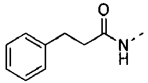 | CH₃(CH₂)₈CONH | *N*-[(4*S*,6*S*,10*S*)-14-methyl-9,15-dioxo-6-[(3-phenylpropanoyl)amino]-2-oxa-8,14-diazatricyclo[14.3.1.0^{4,8}]icosa-1(20),16,18-trien-10-yl]decanamide |
| **Ex.510** | 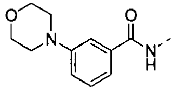 | CH₃(CH₂)₈CONH | N-[(4*S*,6*S*,10*S*)-14-methyl-6-[(3-morpholinobenzoyl)amino]-9,15-dioxo-2-oxa-8,14-diazatricyclo[14.3.1.0^{4,8}]icosa-1(20),16,18-trien-10-yl]decanamide |
| **Ex.511** | 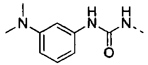 | CH₃(CH₂)₈CONH | *N*-[(4S,6S,10S)-6-({[3-(dimethylamino)anilino]carbonyl}amino)-14-methyl-9,15-dioxo-2-oxa-8,14-diazatricyclo[14.3.1.0^{4,8}]icosa-1(20),16,18-trien-10-yl]decanamide |
| **Ex.512** | 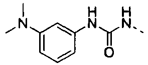 | 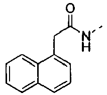 | *N*-[(4S,6S,10S)-6-({[3-(dimethylamino)anilino]carbonyl}amino)-14-methyl-9,15-dioxo-2-oxa-8,14-diazatricyclo[14.3.1.0^{4,8}]icosa-1(20),16,18-trien-10-yl]-2-(1-naphthyl)acetamide |
| **Ex.513** | 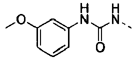 | 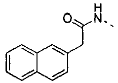 | *N*-[(4*S*,6*S*,10*S*)-6-{[(3-methoxyanilino)carbonyl]amino}-14-methyl-9,15-dioxo-2-oxa-8,14-diazatricyclo[14.3.1.0^{4,8}]icosa-1(20),16,18-trien-10-yl]-2-(2-naphthyl)acetamide |
| **Ex.514** | 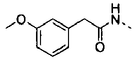 | 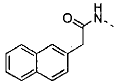 | 2-(3-methoxyphenyl)-*N*-[(4*S*,6*S*,10*S*)-14-methyl-10-{[2-(2-naphthyl)acetyl]amino}-9,15-dioxo-2-oxa-8,14-diazatricyclo[14.3.1.0^{4,8}]icosa-1(20),16,18-trien-6-yl]acetamide |
| **Ex.515** | 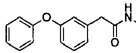 | CH₃(CH₂)₈CONH | *N*-[(4*S*,6*S*,10*S*)-14-methyl-9,15-dioxo-6-{[2-(3-phenoxyphenyl)acetyl]amino}-2-oxa-8,14-diazatricyclo[14.3.1.0^{4,8}]icosa-1(20),16,18-trien-10-yl]decanamide |
| **Ex.516** | 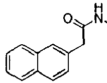 | 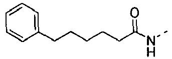 | *N*-[(4*S*,6*S*,10*S*)-14-methyl-6-{[2-(2-naphthyl)acetyl]amino}-9,15-dioxo-2-oxa-8,14-diazatricyclo[14.3.1.0^{4,8}]icosa-1(20),16,18-trien-10-yl]-6-phenylhexanamide |
| **Ex.517** | 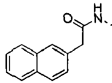 | 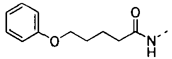 | *N*-[(4*S*,6*S*,10*S*)-14-methyl-6-{[2-(2-naphthyl)acetyl]amino}-9,15-dioxo-2-oxa-8,14-diazatricyclo[14.3.1.0^{4,8}]icosa-1(20),16,18-trien-10-yl]-5-phenoxypentanamide |
| **Ex.518** | 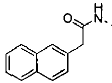 | 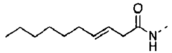 | (*E*)-*N*-[(4*S*,6*S*,10*S*)-14-methyl-6-{[2-(2-naphthyl)acetyl]amino}-9,15-dioxo-2-oxa-8,14-diazatricyclo[14.3.1.0^{4,8}]icosa-1(20),16,18-trien-10-yl]-3-decenamide |
| **Ex.519** | 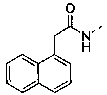 | CH₃(CH₂)₈CONH | *N*-[(4*S*,6*S*,10*S*)-14-methyl-6-{[2-(1-naphthyl)acetyl]amino}-9,15-dioxo-2-oxa-8,14-diazatricyclo[14.3.1.0^{4,8}]icosa-1(20),16,18-trien-10-yl]decanamide |
| **Ex.520** | 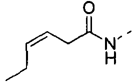 | 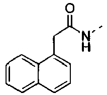 | (*Z*)-*N*-[(4*S*,6*S*,10*S*)-14-methyl-10-{[2-(1-naphthyl)acetyl]amino}-9,15-dioxo-2-oxa-8,19-diazatricyclo[14.3.1.0^{4,8}]icosa-1(20),16,18-trien-6-yl]-3-hexenamide |
| **Ex.521** | 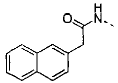 | 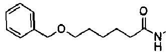 | 6-(benzyloxy)-*N*-[(4*S*,6*S*,10*S*)-14-methyl-6-{[2-(2-naphthyl)acetyl]amino}-9,15-dioxo-2-oxa-8,14-diazatricyclo[14.3.1.0^{4,8}]icosa-1(20),16,18-trien-10-yl]hexanamide |
| **Ex.522** | 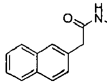 | 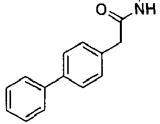 | 2-[1,1'-biphenyl]-4-yl-*N*-[(4*S*,6*S*,10*S*)-14-methyl-6-{[2-(2-naphthyl)acetyl]amino}-9,15-dioxo-2-oxa-8,14-diazatricyclo[14.3.1.0^{4,8}]icosa-1(20),16,18-trien-10-yl]acetamide |
| **Ex.523** | 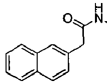 | 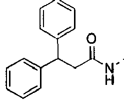 | *N*-[(4*S*,6*S*,10*S*)-14-methyl-6-{[2-(2-naphthyl)acetyl]amino}-9,15-dioxo-2-oxa-8,14-diazatricyclo[14.3.1.0^{4,8}]icosa-1(20),16,18-trien-10-yl]-3,3-diphenylpropanamide |
| **Ex.524** | 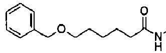 | 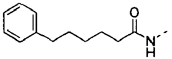 | 6-(benzyloxy)-*N*-[(4*S*,6*S*,10*S*)-14-methyl-9,15-dioxo-10-[(6-phenylhexanoyl)amino]-2-oxa-8,14-diazatricyclo[14.3.1.0^{4,8}]icosa-1(20),16,18-trien-6-yl]hexanamide |
| **Ex.525** | 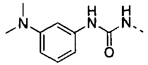 | 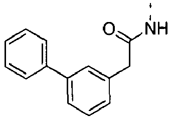 | 2-[1,1'-biphenyl]-3-yl-*N*-[(4*S*,6*S*,10*S*)-6-({[3-(dimethylamino)anilino]carbonyl}amino)-14-methyl-9,15-dioxo-2-oxa-8,14-diazatricyclo[14.3.1.0^{4,8}]icosa-1(20),16,18-trien**-**10-yl]acetamide |
| **Ex**.**526** | 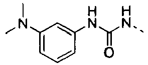 | 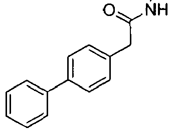 | 2-[1,1'-biphenyl]-4-yl-N-[(4*S*,6*S*,10*S*)-6-({[3-(dimethylamino)anilino]carbonyl}amino)-14-methyl-9,15-dioxo-2-oxa-8,14-diazatricyclo[14.3.1.0^{4,8}]icosa-1(20),16,18-trien-10-yl]acetamide |
| **Ex.527** | 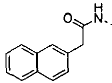 | 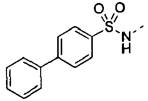 | *N*-[(4*S*,6*S*,10*S*)-10-[([1,1'-biphenyl]-4-ylsulfonyl)amino]-14-methyl-9,15-dioxo-2-oxa-8,14-diazatricyclo[14.3.1.0^{4,8}]icosa-1(20),16,18-trien-6-yl]-2-(2-naphthyl)acetamide |
| **Ex.528** | 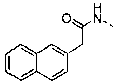 | 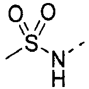 | *N*-[(4*S*,6*S*,10*S*)-14-methyl-10-[(methylsulfonyl)amino]-9,15-dioxo-2-oxa-8,14-diazatricyclo[14.3.1.0^{4,8}]icosa-1(20),16,18-trien-6-yl]-2-(2-naphthyl)acetamide |
| **Ex.529** | 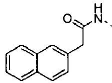 | 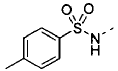 | *N*-[(4*S*,6*S*,10*S*)-14-methyl-10-{[(4-methylphenyl)sulfonyl]amino}-9,15-dioxo-2-oxa-8,14-diazatricyclo[14.3.1.0^{4,8}]icosa-1(20),16,18-trien-6-yl]-2-(2-naphthyl)acetamide |
| **Ex.530** | 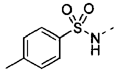 | 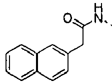 | *N*-[(4*S*,6*S*,10*S*)-14-methyl-6-{[(4-methylphenyl)sulfonyl]amino}-9,15-dioxo-2-oxa-8,14-diazatricyclo[14.3.1.0^{4,8}]icosa-1(20),16,18-trien-10-yl]-2-(2-naphthyl)acetamide |
| **Ex.531** | 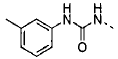 | 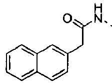 | *N*-[(4*S*,6*S*,10*S*)-14-methyl-9,15-dioxo-6-[(3-toluidinocarbonyl)amino]-2-oxa-8,14-diazatricyclo[14.3.1.0^{4,8}]icosa-1(20),16,18-trien-10-yl]-2-(2-naphthyl)acetamide |
| **Ex.532** | 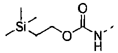 | 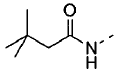 | 2-(trimethylsilyl)ethyl *N*-[(4*S*,6*S*,10*S*)-10-[(3,3-dimethylbutanoyl)amino]-14-methyl-9,15-dioxo-2-oxa-8,14-diazatricyclo[14.3.1.0^{4,8}]icosa-1(20),16,18-trien-6-yl]carbamate |
| **Ex.533** | NH₂ | 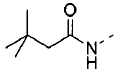 | *N*-[(4*S*,6*S*,10*S*)-6-amino-14-methyl-9,15-dioxo-2-oxa-8,14-diazatricyclo[14.3.1.0^{4,8}]icosa-1(20),16,18-trien-10-yl]-3,3-dimethylbutanamide |
| **Ex.534** | 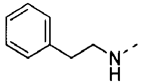 | 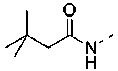 | 3,3-dimethyl-*N*-[(4*S*,6*S*,10*S*)-14-methyl-9,15-dioxo-6-(phenethylamino)-2-oxa-8,14-diazatricyclo[14.3.1.0^{4,8}]icosa-1(20),16,18-trien-10-yl]butanamide |
| **Ex.535** | 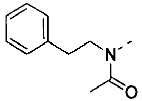 | 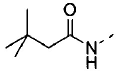 | *N*-[(4*S*,6*S*,10*S*)-6-[acetyl(phenethyl)amino]-14-methyl-9,15-dioxo-2-oxa-8,14-diazatricyclo[14.3.1.0^{4,8}]icosa-1(20),16,18-trien-10-yl]-3,3-dimethylbutanamide |
| **Ex.536** | 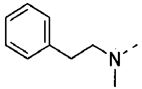 | 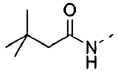 | 3,3-dimethyl-N-[(4*S*,6*S*,10*S*)-14-methyl-6-[methyl(phenethyl)amino]-9,15-dioxo-2-oxa-8,14-diazatricyclo[14.3.1.0^{4,8}]icosa-1(20),16,18-trien-10-yl]butanamide |
| **Ex.537** | 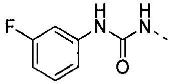 | 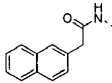 | *N*-[(4*S*,6*S*,10*S*)-6-{[(3-fluoroanilino)carbonyl]amino}-14-methyl-9,15-dioxo-2-oxa-8,14-diazatricyclo[14.3.1.0^{4,8}]icosa-1(20),16,18-trien-10-yl]-2-(2-naphthyl)acetamide |
| **Ex.538** | 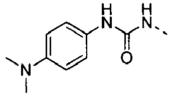 | 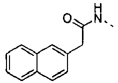 | *N*-[(4*S*,6*S*,10*S*)-6-({[4-(dimethylamino)anilino]carbonyl}amino)-14-methyl-9,15-dioxo-2-oxa-8,14-diazatricyclo[14.3.1.0^{4,8}]icosa-1(20),16,18-trien-10-yl]-2-(2-naphthyl)acetamide |
| **Ex.539** | 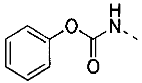 | 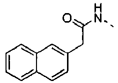 | phenyl *N*-[(4*S*,6*S*,10*S*)-14-methyl-10-{[2-(2-naphthyl)acetyl]amino}-9,15-dioxo-2-oxa-8,14-diazatricyclo[14.3.1.0^{4,8}]icosa-1(20),16,18-trien-6-yl]carbamate |
| **Ex.540** | 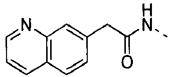 | 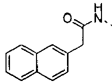 | *N*-[(4*S*,6*S*,10*S*)-14-methyl-9,15-dioxo-6-{[2-(7-quinolinyl)acetyl]amino}-2-oxa-8,14-diazatricyclo[14.3.1.0^{4,8}]icosa-1(20),16,18-trien-10-yl]-2-(2-naphthyl)acetamide |
| **Ex.541** | 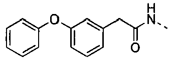 | 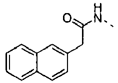 | *N*-[(4*S*,6*S*,10*S*)-14-methyl-9,15-dioxo-6-{[2-(3-phenoxyphenyl)acetyl]amino}-2-oxa-8,14-diazatricyclo[14.3.1.0^{4,8}]icosa-1(20),16,18-trien-10-yl]-2-(2-naphthyl)acetamide |
| **Ex.542** | CH₃(CH₂)₈CONH | 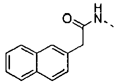 | *N*-[(4*S*,6*S*,10*S*)-14-methyl-10-{[2-(2-naphthyl)acetyl]amino}-9,15-dioxo-2-oxa-8,14-diazatricyclo[14.3.1.0^{4,8}]icosa-1(20),16,18-trien-6-yl]decanamide |
| **Ex.543** | CH₃(CH₂)₈CONH | 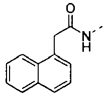 | *N*-[(4*S*,6*S*,10*S*)-14-methyl-10-{[2-(1-naphthyl)acetyl]amino}-9,15-dioxo-2-oxa-8,14-diazatricyclo[14.3.1.0^{4,8}]icosa-1(20),16,18-trien-6-yl]decanamide |
| **Ex.544** | CH₃(CH₂)₄CONH | 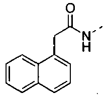 | *N*-[(4*S*,6*S*,10*S*)-14-methyl-10-{[2-(1-naphthyl)acetyl]amino}-9,15-dioxo-2-oxa-8,14-diazatricyclo[14.3.1.0^{4,8}]icosa-1(20),16,18-trien-6-yl]hexanamide |
| **Ex.545** | 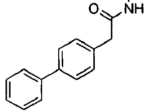 | 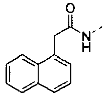 | 2-[1,1'-biphenyl]-4-yl-*N*-[(4*S*,6*S*,10*S*)-14-methyl-10-{[2-(1-naphthyl)acetyl]amino}-9,15-dioxo-2-oxa-8,14-diazatricyclo[14.3.1.0^{4,8}]icosa-1(20),16,18-trien-6-yl]acetamide |
| **Ex.546** | 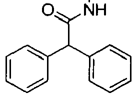 | 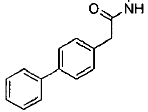 | *N*-[(4*S*,6*S*,10*S*)-10-[(2-[1,1'-biphenyl]-4-ylacetyl)amino]-14-methyl-9,15-dioxo-2-oxa-8,14-diazatricyclo[14.3.1.0^{4,8}]icosa-1(20),16,18-trien-6-yl]-2,2-diphenylacetamide |
| **Ex**.**547** | 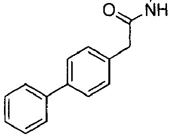 | 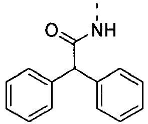 | *N*-[(4*S*,6*S*,10*S*)-6-[(2-[1,1'-biphenyl]-4-ylacetyl)amino]-14-methyl-9,15-dioxo-2-oxa-8,14-diazatricyclo[14.3.1.0^{4,8}]icosa-1(20),16,18-trien-10-yl]-2,2-diphenylacetamide |
| **Ex.548** | 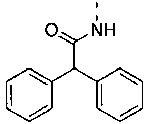 | CH₃(CH₂)₈CONH | *N*-[(4*S*,6*S*,10*S*)-6-[(2,2-diphenylacetyl)amino]-14-methyl-9,15-dioxo-2-oxa-8,14-diazatricyclo[14.3.1.0^{4,8}]icosa-1(20),16,18-trien-10-yl]decanamide |
| **Ex.549** | 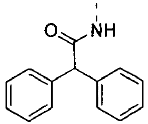 | 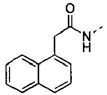 | *N*-[(4*S*,6*S*,10*S*)-14-methyl-10-{[2-(1-naphthyl)acetyl]amino}-9,15-dioxo-2-oxa-8,14-diazatricyclo[14.3.1.0^{4,8}]icosa-1(20),16,18-trien-6-yl]-2,2-diphenylacetamide |
| **Ex.550** | 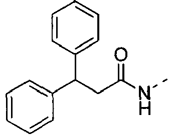 | CH₃(CH₂)₈CONH | *N*-[(4*S*,6*S*,10*S*)-6-[(3,3-diphenylpropanoyl)amino]-14-methyl-9,15-dioxo-2-oxa-8,14-diazatricyclo[14.3.1.0^{4,8}]icosa-1(20),16,18-trien-10-yl]decanamide |
| **Ex.551** | 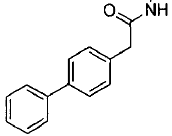 | 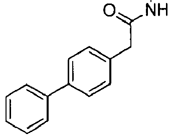 | 2-[1,1'-biphenyl]-4-yl-*N*-[(4*S*,6*S*,10*S*)-10-[(2-[1,1'-biphenyl]-4-ylacetyl)amino]-14-methyl-9,15-dioxo-2-oxa-8,14-diazatricyclo[14.3.1.0^{4,8}]icosa-1(20),16,18-trien-6-yl]acetamide |
| **Ex.552** | 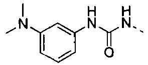 | 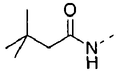 | *N*-[(4*S*,6*S*,10*S*)-6-({[3-(dimethylamino)anilino]carbonyl}amino)-14-methyl-9,15-dioxo-2-oxa-8,14-diazatricyclo[14.3.1.0^{4,8}]icosa-1(20),16,18-trien-10-yl]-3,3-dimethylbutanamide |
| **Ex.553** | 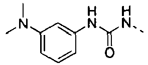 | 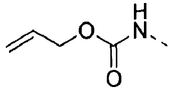 | allyl *N*-[(4*S*,6*S*,10*S*)-6-({[3-(dimethylamino)anilino]carbonyl}amino)-14-methyl-9,15-dioxo-2-oxa-8,14-diazatricyclo[14.3.1.0^{4,8}]icosa-1(20),16,18-trien-10-yl]carbamate |
| **Ex.554** | 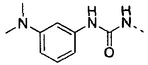 | NH₂ | *N*-[(4*S*,6*S*,10*S*)-10-amino-14-methyl-9,15-dioxo-2-oxa-8,14-diazatricyclo[14.3.1.0^{4,8}]icosa-1(20),16,18-trien-6-yl]-*N*'-[3-(dimethylamino)phenyl]urea |
| **Ex.555** | 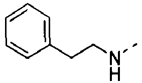 | 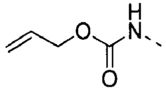 | allyl *N*-[(4*S*,6*S*,10*S*)-14-methyl-9,15-dioxo-6-(phenethylamino)-2-oxa-8,14-diazatricyclo[14.3.1.0^{4,8}]icosa-1(20),16,18-trien-10-yl]carbamate |
| **Ex.556** | 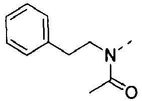 | 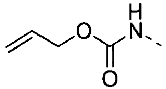 | allyl *N*-[(4*S*,6*S*,10*S*)-6-[acetyl(phenethyl)amino]-14-methyl-9,15-dioxo-2-oxa-8,14-diazatricyclo[14.3.1.0^{4,8}]icosa-1(20),16,18-trien-10-yl]carbamate |
| **Ex.557** | 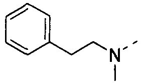 | 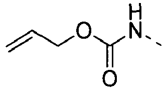 | allyl *N*-[(4*S*,6*S*,10*S*)-14-methyl-6-[methyl(phenethyl)amino]-9,15-dioxo-2-oxa-8,14-diazatricyclo[14.3.1.0^{4,8}]icosa-1(20),16,18-trien-10-yl]carbamate |
| **Ex.558** | 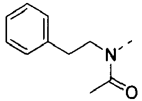 | NH₂ | *N*-[(4*S*,6*S*,10*S*)-10-amino-14-methyl-9,15-dioxo-2-oxa-8,14-diazatricyclo[14.3.1.0^{4,8}]icosa-1(20),16,18-trien-6-yl]-*N-*phenethylacetamide |
| **Ex**.**559** | 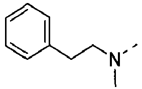 | NH₂ | (4*S*,6*S*,10*S*)-10-amino-14-methyl-6-[methyl(phenethyl)amino]-2-oxa-8,14-diazatricyclo[14.3.1.0^{4,8}]icosa-1(20),16,18-triene-9,15-dione |
| **Ex.594** | NH₂ | 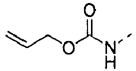 | allyl *N*-[(4*S*,6*S*,10*S*)-6-amino-14-methyl-9,15-dioxo-2-oxa-8,14-diazatricyclo[14.3.1.0^{4,8}]icosa-1(20),16,18-trien-10-yl]carbamate |

**Table 29.2.a: Examples of Core 11b (Ex.560-Ex.566)**

| **No** | **R²** | **R⁵** | **Starting material** | **General Procedure** | **Reagent** | **Purification Method** | **Yield (isolated salt)** |
|---|---|---|---|---|---|---|---|
| **Ex.560-Ex.561:** *cf. experimental description* | | | | | | | |
| **Ex.562** | 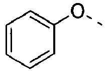 | 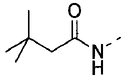 | **Ex.561** | L.1.1 | 3,3-dimethylbutanoyl chloride (1.5 equiv.) pyridine (5 equiv.) | FC (CH₂Cl₂/MeOH) | 85% |
| **Ex.563** | 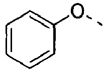 | 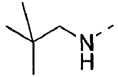 | **Ex.561** | M.2 | Pivalaldehyde (1.0 equiv.) | FC (CH₂Cl₂/MeOH) | 30% |
| **Ex.564** | 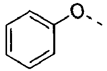 | 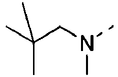 | **Ex.563** | M.3 | aq. formaldehyde soln | FC (CH₂Cl₂/MeOH) | 93% |
| **Ex.565** | 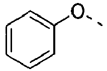 | 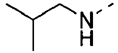 | **Ex.561** | M.2 | isobutyraldehyde | FC (CH₂Cl₂/MeOH) | 63% |
| **Ex.566** | 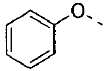 | 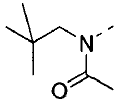 | **Ex.563** | L.1.1 | acetic anhydride (1.2 mL), pyridine (1.1 mL), CH₂Cl₂ (1.2 mL) | crude product | 95% |

**Table 29.2.b: Examples of Core 11b (Ex.560-Ex.566)**

| **No** | **R²** | **R⁵** | **FORMULA** | **MONOISOTOPIC MASS** | **Rt (purity at 220nm)** | **[M+H]⁺ found** | **LC-MS-Method** |
|---|---|---|---|---|---|---|---|
| **Ex.560-Ex.561:** *cf. experimental description* | | | | | | | |
| **Ex.562** | 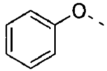 | 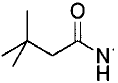 | C30H39N3O5 | 521.3 | 2.21 (97) | 522.4 | Method 4a |
| **Ex.563** | 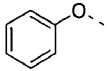 | 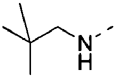 | C29H39N3O4 | 493.3 | 1.80 (98) | 494.3 | Method 4a |
| **Ex.564** | 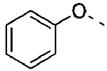 | 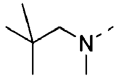 | C30H41N3O4 | 507.3 | 1.61 (96) | 508.2 | Method 10b |
| **Ex.565** | 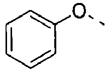 | 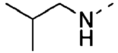 | C28H37N3O4 | 479.3 | 1.57 (99) | 480.3 | Method 10b |
| **Ex.566** | 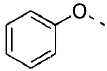 | 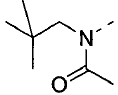 | C31H41N3O5 | 535.3 | 2.07 (94) | 536.3 | Method 10a |

**Table 29.2.c: Examples of Core 11b (Ex.560-Ex.566)**

| **No** | **R²** | **R⁵** | **IUPAC name** |
|---|---|---|---|
| **Ex.560** | 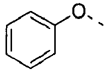 | 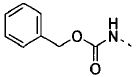 | benzyl *N*-[(4*S*,6*S*,10*S*)-14-methyl-9,15-dioxo-6-phenoxy-2-oxa-8,14-diazatricyclo[14.3.1.0^{4,8}]icosa-1(20),16,18-trien-10-yl]carbamate |
| **Ex.561** | 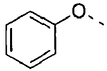 | NH₂ | (4*S*,6*S*,10*S*)-10-amino-14-methyl-6-phenoxy-2-oxa-8,14-diazatricyclo[14.3.1.0^{4,8}]icosa-1(20),16,18-triene-9,15-dione |
| **Ex.562** | 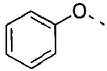 | 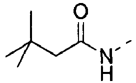 | 3,3-dimethyl-*N*-[(4*S*,6*S*,10*S*)-14-methyl-9,15-dioxo-6-phenoxy-2-oxa-8,14-diazatricyclo[14.3.1.0^{4,8}]icosa-1(20),16,18-trien-10-yl]butanamide |
| **Ex.563** | 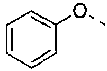 | 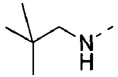 | (4*S*,6*S*,10*S*)-14-methyl-10-(neopentylamino)-6-phenoxy-2-oxa-8,14-diazatricyclo[14.3.1.0^{4,8}]icosa-1(20),16,18-triene-9,15-dione |
| **Ex.564** | 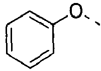 | 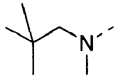 | (4*S*,6*S*,10*S*)-14-methy1-10-[methyl(neopentyl)amino]-6-phenoxy-2-oxa-8,14-diazatricyclo[14.3.1.0^{4,8}]icosa-1(20),16,18-triene-9,15-dione |
| **Ex.565** | 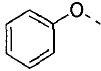 | 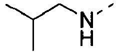 | (4*S*,6*S*,10*S*)-10-(isobutylamino)-14-methyl-6-phenoxy-2-oxa-8,14-diazatricyclo[14.3.1.0^{4,8}]icosa-1(20),16,18-triene-9,15-dione |
| **Ex.566** | 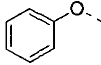 | 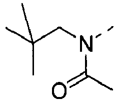 | *N*-[(4*S*,6*S*,10*S*)-14-methyl-9,15-dioxo-6-phenoxy-2-oxa-8,14-diazatricyclo[14.3.1.0^{4,8}]icosa-1(20),16,18-trien-10-yl]-N-neopentylacetamide |

**Table 29.3.a: Examples of Core 11c (Ex.567-Ex.585; continued on the following pages)**

| **No** | **R²** | **R⁵** | **Starting material** | **General Procedure** | **Reagent** | **Purification Method** | **Yield (isolated salt)** |
|---|---|---|---|---|---|---|---|
| **Ex.567-Ex.568:** *cf. experimental description* | | | | | | | |
| **Ex.569** | 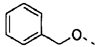 | 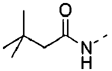 | **Ex**.**568** | L.1.1 | 3,3-dimethylbutanoyl chloride (1.1 equiv.) i-Pr₂NEt (5 equiv.) | FC (hexane/ EtOAc) | 76% |
| **Ex.570** | 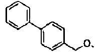 | NH₂ | **Ex.584** | K.2 | H₂, 5%Pd-C, NH₃-MeOH | crude product | 81% (HCl salt)^{*)} |
| **Ex.571** | OH | 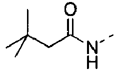 | **Ex.569** | H | H₂, Pd(OH)₂-C, MeOH/AcOH 2:1 | crude product | 93% |
| **Ex.572** | OCH₃ | 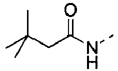 | **Ex.571** | **) | **) | FC (EtOAc) | 38% |
| **Ex.573** | 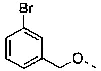 | 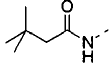 | **Ex.571** | Q | 3-bromobenzyl bromide | FC (hexane/EtOAc) | 60% |
| **Ex.574** | OH | 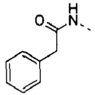 | **Ex.585** | H | H₂, Pd(OH)₂-C, MeOH/AcOH 4:1 | crude product | 100% |
| **Ex.575** | 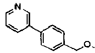 | 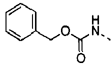 | **Ex.567** | P | pyridine-3-boronic acid | prep. HPLC method 3 | 91% |
| **Ex.576** | 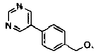 | 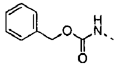 | **Ex.567** | P | pyrimidine-5-boronic acid | prep. HPLC method 3 | 86% |
| **Ex.577** | 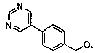 | NH₂ | **Ex.576** | K.2 | H₂, 5%Pd-C, NH₃-MeOH | crude product | 93% |
| **Ex.578** | 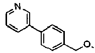 | NH₂ | **Ex.575** | K.2 | H₂, 5%Pd-C, NH₃-MeOH | crude product | 91% |
| **Ex.579** | 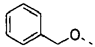 | 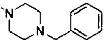 | **Ex.568** | O | N-benzyl-2-chloro-N-(2-chloroethyl) ethanamine | FC (hexane/ EtOAc/MeOH) | 61% |
| **Ex.580** | OH | 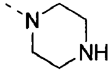 | **Ex579** | K.3 | H₂, Pd(OH)₂-C, AcOH | FC (CH₂Cl₂/MeOH/ aq.NH₃) | 94% |
| **Ex.581** | OH | 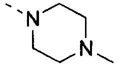 | **Ex.580** | M.3 | aq. formaldehyde soln | FC (CH₂Cl₂/MeOH/ aq.NH₃) | 70% |
| **Ex.582** | OH | 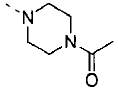 | **Ex**.**580** | L.1.1 | acetyl chloride (1.0 equiv.) triethylamine (2 equiv.) 0°C, 30 min | FC (CH₂Cl₂/MeOH) | 75% |
| **Ex.583** | OH | 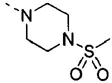 | **Ex**.**580** | L.3 | methanesulfonyl chloride 0°C, 30 min | FC (hexane/ EtOAc/MeOH) | 63% |
| **Ex.584** | 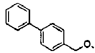 | 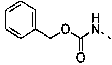 | **Ex**.**567** | P | phenylboronic acid (2 equiv.) | FC (EtOAc/hexane) | 75% |
| **Ex.585** | 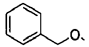 | 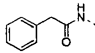 | **Ex**.**568** | L.1.1 | phenylacetyl chloride (1 equiv.) DIPEA (3 equiv.) | FC (EtOAc) | 70% |

| | | | | | | | |
|---|---|---|---|---|---|---|---|
| *) the crude product was converted into the HCl salt **) dimethyl sulfate, benzyltriethylammonium chloride, toluene, 2 M aq. NaOH | | | | | | | |

**Table 29.3.b: Examples of Core 11c (Ex.567-Ex.585; continued on the following pages)**

| **No** | **R²** | **R⁵** | **FORMULA** | **MONOISOTOPIC MASS** | **Rt (purity at 220nm)** | **[M+H]⁺ LC-MS-Method found** | |
|---|---|---|---|---|---|---|---|
| **Ex.567-Ex.568:** *cf. experimental description* | | | | | | | |
| **Ex.569** | 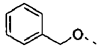 | 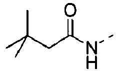 | C31H41N3O5 | 535.3 | 2.22 (92) | 536.4 | Method 4a |
| **Ex.570** | 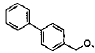 | NH₂ | C31H35N3O4 | 513.3 | 1.88 (97) | 514.3 | Method 4a |
| **Ex.571** | OH | 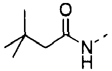 | C24H35N3O5 | 445.3 | 1.59 (95) | 446.3 | Method 4a |
| **Ex.572** | OCH₃ | 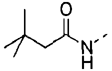 | C25H37N3O5 | 459.3 | 1.62 (96) | 460.2 | Method 10a |
| **Ex.573** | 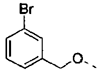 | 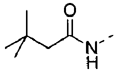 | C31H40BrN3O5 | 613.2 | 2.33 (93) | 616.2 | Method 4a |
| **Ex.574** | OH | 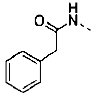 | C26H31N3O5 | 465.2 | 1.35 (87) | 466.3 | Method 10a |
| **Ex.575** | 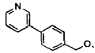 | 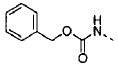 | C38H40N4O6 | 648.3 | 1.57 (94) | 649.1 | Method 10a |
| **Ex.576** | 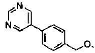 | 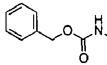 | C37H39N5O6 | 649.3 | 1.90 (98) | 650.3 | Method 10a |
| **Ex.577** | 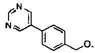 | NH₂ | C29H33N5O4 | 515.3 | 1.30 (95) | 516.3 | Method 10a |
| **Ex.578** | 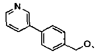 | NH₂ | C30H34N4O4 | 514.3 | 1.08 (95) | 515.3 | Method 10a |
| **Ex.579** | 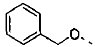 | 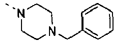 | C36H44N4O4 | 596.3 | 1.61 (97) | 597.3 | Method 10a |
| **Ex.580** | OH | 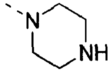 | C22H32N4O4 | 416.2 | 0.80 (98) | 417.2 | Method 10a |
| **Ex.581** | OH | 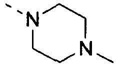 | C23H34N4O4 | 430.3 | 0.83 (99) | 431.2 | Method 10a |
| **Ex.582** | OH | 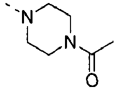 | C24H34N4O5 | 458.3 | 0.82 (97) | 459.2 | Method 10a |
| **Ex.583** | OH | 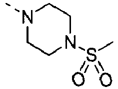 | C23H34N4O6S | 494.2 | 0.84 (94) | 495.2 | Method 10a |
| **Ex.584** | 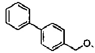 | 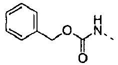 | C39H41N3O6 | 647.3 | 2.46 (65%) | 648.3 | Method 12 |
| **Ex.585** | 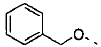 | 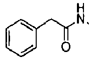 | C33H37N3O5 | 555.3 | 1.99 (88%) | 557.3 | Method 10a |

**Table 29.3.c: Examples of Core 11c (Ex.567-Ex.585; continued on the following pages)**

| **No** | **R²** | **R⁵** | **IUPAC name** |
|---|---|---|---|
| **Ex**.**567** | 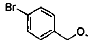 | 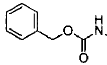 | benzyl *N*-[(4*S*,6*S*,10*S*)-6-[(4-bromobenzyl)oxy]-14-methyl-9,15-dioxo-2-oxa-8,14-diazatricyclo[14.3.1.0^{4,8}]icosa-1(20),16,18-trien-10-yl]carbamate |
| **Ex**.**568** | 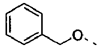 | NH₂ | (4*S*,6*S*,10*S*)-10-amino-6-(benzyloxy)-14-methyl-2-oxa-8,14-diazatricyclo[14.3.1.0^{4,8}]icosa-1(20),16,18-triene-9,15-dione |
| **Ex.569** | 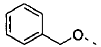 | 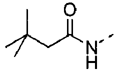 | *N*-[(4*S*,6*S*,10*S*)-6-(benzyloxy)-14-methyl-9,15-dioxo-2-oxa-8,14-diazatricyclo[14.3.1.0^{4,8}]icosa-1(20),16,18-trien-10-yl]-3,3-dimethylbutanamide |
| **Ex.570** | 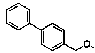 | NH₂ | (4*S*,6*S*,10*S*)-10-amino-6-([1,1'-biphenyl]-4-ylmethoxy)-14-methyl-2-oxa-8,19-diazatricyclo[14.3.1.0^{4,8}]icosa-1(20),16,18-triene-9,15-dione |
| **Ex.571** | OH | 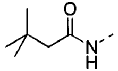 | *N*-[(4*S*,6*S*,10*S*)-6-hydroxy-14-methyl-9,15-dioxo-2-oxa-8,14-diazatricyclo[14.3.1.0^{4,8}]icosa-1(20),16,18-trien-10-yl]-3,3-dimethylbutanamide |
| **Ex.572** | OCH₃ | 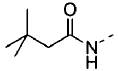 | *N*-[(4*S*,6*S*,10*S*)-6-methoxy-14-methyl-9,15-dioxo-2-oxa-8,14-diazatricyclo[14.3.1.0^{4,8}]icosa-1(20),16,18-trien-10-yl]-3,3-dimethylbutanamide |
| **Ex.573** | 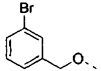 | 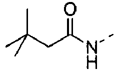 | *N*-[(4*S*,6*S*,10*S*)-6-[(3-bromobenzyl)oxy]-14-methyl-9,15-dioxo-2-oxa-8,14-diazatricyclo[14.3.1.0^{4,8}]icosa-1(20),16,18-trien-10-yl]-3,3-dimethylbutanamide |
| **Ex.574** | OH | 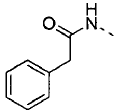 | *N*-[(4*S*,6*S*,10*S*)-6-hydroxy-14-methyl-9,15-dioxo-2-oxa-8,14-diazatricyclo[14.3.1.0^{4,8}]icosa-1(20),16,18-trien-10-yl]-2-phenylacetamide |
| **Ex.575** | 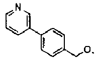 | 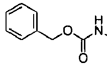 | benzyl *N*-[(4*S*,6*S*,10*S*)-14-methyl-9,15-dioxo-6-{[4-(3-pyridinyl)benzyl]oxy}-2-oxa-8,14-diazatricyclo[14.3.1.0^{4,8}]icosa-1(20),16,18-trien-10-yl]carbamate |
| **Ex.576** | 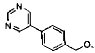 | 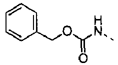 | benzyl *N*-[(4*S*,6*S*,10*S*)-14-methyl-9,15-dioxo-6-{[4-(5-pyrimidinyl)benzyl]oxy}-2-oxa-8,14-diazatricyclo[14.3.1.0^{4,8}]icosa-1(20),16,18-trien-10-yl]carbamate |
| **Ex.577** | 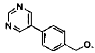 | NH₂ | (4*S*,6*S*,10*S*)-10-amino-14-methyl-6-{[4-(5-pyrimidinyl)benzyl]oxy}-2-oxa-8,14-diazatricyclo[14.3.1.0^{4,8}]icosa-1(20),16,18-triene-9,15-dione |
| **Ex.578** | 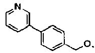 | NH₂ | (4*S*,6*S*,10*S*)-10-amino-14-methyl-6-{[4-(3-pyridinyl)benzyl]oxy}-2-oxa-8,14-diazatricyclo[14.3.1.0^{4,8}]icosa-1(20),16,18-triene-9,15-dione |
| **Ex.579** | 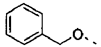 | 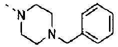 | (4*S*,6*S*,10*S*)-6-(benzyloxy)-10-(4-benzylpiperazino)-14-methyl-2-oxa-8,14-diazatricyclo[14.3.1.0^{4,8}]icosa-1(20),16,18-triene-9,15-dione |
| **Ex.580** | OH | 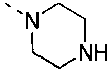 | (4*S*,6*S*,10*S*)-6-hydroxy-14-methyl-10-piperazino-2-oxa-8,14-diazatricyclo[14.3.1.0^{4,8}]icosa-1(20),16,18-triene-9,15-dione |
| **Ex.581** | OH | 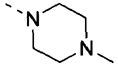 | (4*S*,6*S*,10*S*)-6-hydroxy-14-methyl-10-(4-methylpiperazino)-2-oxa-8,14-diazatricyclo[19.3.1.0^{4,8}]icosa-1(20),16,18-triene-9,15-dione |
| **Ex.582** | OH | 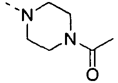 | (4*S*,6*S*,10*S*)-10-(4-acetylpiperazino)-6-hydroxy-14-methyl-2-oxa-8,14-diazatricyclo[14.3.1.0^{4,8}]icosa-1(20),16,18-triene-9,15-dione |
| **Ex.583** | OH | 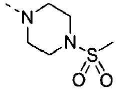 | (4*S*,6*S*,10*S*)-6-hydroxy-14-methyl-10-[4-(methylsulfonyl)piperazino]-2-oxa-8,14-diazatricyclo[14.3.1.0^{4,8}]icosa-1(20),16,18-triene-9,15-dione |
| **Ex.584** | 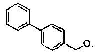 | 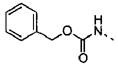 | benzyl *N*-[(4*S*,6*S*,10*S*)-6-([1,1'-biphenyl]-4-ylmethoxy)-14-methyl-9,15-dioxo-2-oxa-8,14-diazatricyclo[14.3.1.0^{4,8}]icosa-1(20),16,18-trien-10-yl]carbamate |
| **Ex.585** | 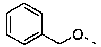 | 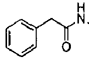 | *N*-[(4*S*,6*S*,10*S*)-6-(benzyloxy)-14-methyl-9,15-dioxo-2-oxa-8,14-diazatricyclo[14.3.1.0^{4,8}]icosa-1(20),16,18-trien-10-yl]-2-phenylacetamide |

**Table 29.4.a: Examples of Core 11d (Ex.586-Ex.589; continued on the following page)**

| **No** | **R²** | **R⁵** | **Starting material** | **General Procedure** | **Reagent** | **Purification Method** | **Yield (isolated salt)** |
|---|---|---|---|---|---|---|---|
| **Ex.586-Ex.587:** *cf. experimental description* | | | | | | | |
| **Ex.588** | CH₂Ph | 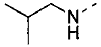 | **Ex.587** | M.2 | isobutyraldehyde | prep.HPLC, method 3 | 61% |
| **Ex.589** | CH₂Ph | 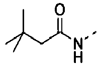 | **Ex.587** | L.1.1 | 3,3-dimethylbutanoyl chloride (1.2 equiv.) pyridine (5 equiv.) | prep.HPLC, method 3 | 70% |

**Table 29.4.b: Examples of Core 11d (Ex.586-Ex.589)**

| **No** | **R²** | **R⁵** | **FORMULA** | **MONOISOTOPIC MASS** | **Rt (purity at 220nm)** | **[M+H]⁺ found** | **LC-MS-Method** |
|---|---|---|---|---|---|---|---|
| **Ex.586-Ex.587:** *cf. experimental description* | | | | | | | |
| **Ex.588** | CH₂Ph | 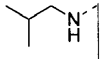 | C29H39N3O3 | 477.3 | 1.67 (98) | 478.3 | Method 10a |
| **Ex.589** | CH₂Ph | 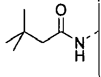 | C31H41N3O4 | 519.3 | 2.13 (98) | 520.3 | Method 10a |

**Table 29.4.c: Examples of Core 11d (Ex.586-Ex.589)**

| **No** | **R²** | **R⁵** | **IUPAC name** |
|---|---|---|---|
| **Ex.586** | CH₂Ph | 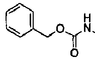 | benzyl *N*-[(4*S*,6*S*,10*S*)-6-benzyl-14-methyl-9,15-dioxo-2-oxa-8,14-diazatricyclo[14.3.1.0^{4,8}]icosa-1(20),16,18-trien-10-yl]carbamate |
| **Ex.587** | CH₂Ph | NH₂ | (4*S*,6*S*,10*S*)-10-amino-6-benzyl-14-methyl-2-oxa-8,14-diazatricyclo[14.3.1.0^{4,8}]icosa-1(20),16,18-triene-9,15-dione |
| **Ex.588** | CH₂Ph | 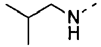 | (4*S*,6*S*,10*S*)-6-benzyl-10-(isobutylamino)-14-methyl-2-oxa-8,14-diazatricyclo[14.3.1.0^{4,8}]icosa-1(20),16,18-triene-9,15-dione |
| **Ex**.**589** | CH₂Ph | 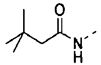 | *N*-[(4*S*,6*S*,10*S*)-6-benzyl-14-methyl-9,15-dioxo-2-oxa-8,14-diazatricyclo[14.3.1.0^{4,8}]icosa-1(20),16,18-trien-10-yl]-3,3-dimethylbutanamide |

**Table 30a: Examples of Core 12 (Ex.196-Ex.214; continued on the following pages)**

| **No** | **R²** | **R⁵** | **Starting material** | **General Procedure** | **Reagent** | **Purification Method** | **Yield (isolated salt)** |
|---|---|---|---|---|---|---|---|
| **Ex.196-Ex.198:** *cf. experimental description* | | | | | | | |
| **Ex.199** | 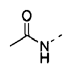 | 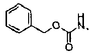 | **Ex.197** ·HCl | L.1.1 | acetic anhydride (10 equiv.) pyridine (2 mL) | FC (CH₂Cl₂/MeOH) then prep HPLC, method 1 | 36% |
| **Ex.200** | 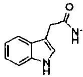 | 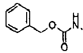 | **Ex.197** ·HCl | L.1.3 | 3-indoleacetic acid (1.5 equiv.), HATU (1.5 equiv.), HOAt (1.5 equiv), i-Pr₂NEt (5 equiv.) | FC (CH₂Cl₂/MeOH) then prep HPLC, method 1 | 22% |
| **Ex.200** | 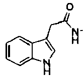 | 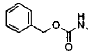 | **Ex.197** ·TFA | L.1.3 | 3-indoleacetic acid (1.5 equiv.), HATU (1.5 equiv.), HOAt (1.5 equiv), i-Pr₂NEt (5 equiv.) | FC (EtOAc, then CH₂Cl₂/MeOH) | 78% |
| **Ex.201** | 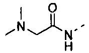 | 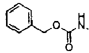 | **Ex.197** ·HCl | L.1.3 | N,N-dimethyl glycine (1.7 equiv.) | FC (CH₂Cl₂/MeOH) and prep HPLC, method 1 | 91%^{*)} (TFA salt) |
| **Ex.202** | 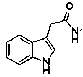 | NH₂ | **Ex.200** | K.1 | H₂, Pd(OH)₂-C | crude product | 97% |
| **Ex.203** | 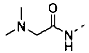 | NH₂ | **Ex.201** | K.1 | H₂, Pd(OH)₂-C | crude product | 99% |
| **Ex.204** | 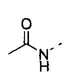 | 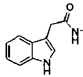 | **Ex.214** | L.1.3 | 3-indoleacetic acid (1.2 equiv.), HATU (1.5 equiv.), HOAt (1.5 equiv), i-Pr₂NEt (3 equiv.) | FC (CH₂Cl₂/MeOH) | 64% |
| **Ex.205** | 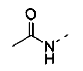 | 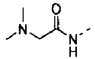 | **Ex.214** | L.1.3 | N,N-dimethyl glycine (1.7 equiv.) | prep HPLC, method 1 | 47% (TFA salt) |
| **Ex.206** | 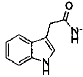 | 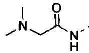 | **Ex.202** | L.1.3 | N,N-dimethyl glycine (1.7 equiv.) | prep HPLC, method 1 | 39% (TFA salt) |
| **Ex.207** | 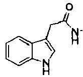 | 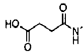 | **Ex.202** | L.1.1 | succinic anhydride (1.05 equiv.) pyridine (49 equiv.) | prep HPLC, method 2 | 48% |
| **Ex.208** | 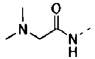 | 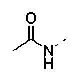 | **Ex.203** | L.1.1 | acetic anhydride (10 equiv.) pyridine/CH₂Cl₂ 1.1 (3 mL) | prep HPLC, method 2 | 59% |
| **Ex.209** | 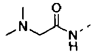 | 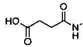 | **Ex.203** | L.1.1 | succinic anhydride (1.05 equiv.) pyridine (49 equiv.) | prep HPLC, method 2 | 35% |
| **Ex.210** | 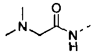 | 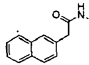 | **Ex.203** | L.1.3 | 2-naphthylacetic acid (1.2 equiv.) | prep HPLC, method 1 | 38% (TFA salt) |
| **Ex.211** | 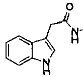 | 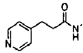 | **Ex.202** | L.1.2 | 3-(pyridine-4-yl)propanoic acid | prep HPLC, method 1 | 44% (TFA salt) |
| **Ex.212** | 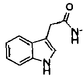 | 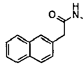 | **Ex.202** | L.1.3 | 2-naphthylacetic acid (1.2 equiv.) | prep HPLC, method 1 then FC (EtOAc/MeOH) | 53% |
| **Ex.213** | 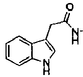 | CH₃(CH₂)₈CONH | **Ex.202** | L.1.1 | decanoyl chloride (2 equiv.) | prep HPLC, method 1 then FC (EtOAc/MeOH) | 35% |
| **Ex.214** | 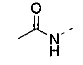 | NH₂ | **Ex.199** | K.1 | H₂, Pd(OH)₂-C | crude product | 98% |

| | | | | | | | |
|---|---|---|---|---|---|---|---|
| *) An analytical sample was further purified by prep. HPLC (method 1), to afford the TFA salt of the corresponding product | | | | | | | |

**Table 30b: Examples of Core 12 (Ex.196-Ex.214; continued on the following page)**

| **No** | **R²** | **R⁵** | **FORMULA** | **MONOISOTOPIC MASS** | **Rt (purity at 220nm)** | **[M+H]⁺ found** | **LC-MS-Method** |
|---|---|---|---|---|---|---|---|
| **Ex.196-Ex.198:** *cf. experimental description* | | | | | | | |
| **Ex.199** | 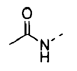 | 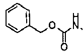 | C29H35N5O7 | 565.3 | 1.92 (100) | 566.4 | Method 1a |
| **Ex.200** | 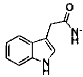 | 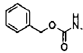 | C37H40N6O7 | 680.3 | 2.23 (98) | 681.4 | Method 1a |
| **Ex.200** | 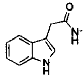 | 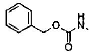 | C37H40N6O7 | 680.3 | 1.68 (93) | 681.5 | Method 2 |
| **Ex.201** | 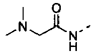 | 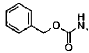 | C31H40N6O7 | 608.3 | 1.28 (99) | 609.4 | Method 2 |
| **Ex.202** | 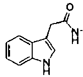 | NH₂ | C29H34N6O5 | 546.3 | 1.32 (76) | 547.4 | Method 2 |
| **Ex.203** | 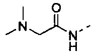 | NH₂ | C23H34N6O5 | 474.3 | 0.77 | 475.5 | Method 9c |
| **Ex.204** | 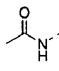 | 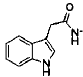 | C31H36N6O6 | 588.3 | 1.49 (87) | 589.2 | Method 4a |
| **Ex.205** | 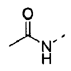 | 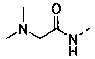 | C25H36N6O6 | 516.3 | 1.42 (100) | 517.3 | Method 1a |
| **Ex.206** | 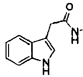 | 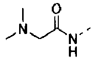 | C33H41N7O6 | 631.3 | 1.84 (97) | 632.4 | Method 1a |
| **Ex.207** | 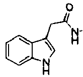 | 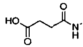 | C33H38N6O8 | 646.3 | 1.42 (100) | 647.4 | Method 2 |
| **Ex.208** | 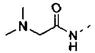 | 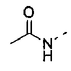 | C25H36N6O6 | 516.3 | 0.95 (100) | 517.4 | Method 2 |
| **Ex.209** | 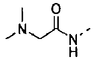 | 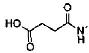 | C27H38N6O8 | 574.3 | 1.02 (92) | 575.4 | Method 3 |
| **Ex.210** | 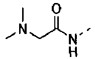 | 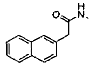 | C35H42N6O6 | 642.3 | 1.37 (100) | 643.4 | Method 2 |
| **Ex.211** | 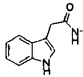 | 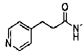 | C37H41N7O6 | 679.3 | 1.34 (92) | 680.5 | Method 2 |
| **Ex.212** | 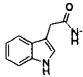 | 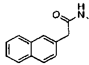 | C41H42N6O6 | 714.3 | 1.75 (86) | 715.5 | Method 2 |
| **Ex.213** | 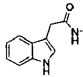 | CH₃(CH₂)₈CONH | C39H52N6O6 | 700.4 | 1.96 (99) | 701.6 | Method 2 |
| **Ex.214** | 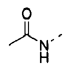 | NH₂ | C21H29N5O5 | 431.2 | 0.81^{*)} | 432.2 | Method 9c |

| | | | | | | | |
|---|---|---|---|---|---|---|---|
| *) Analytical HPLC (5% CH₃CN): 2.52 (93) | | | | | | | |

**Table 30c: Examples of Core 12 (Ex.196-Ex.214; continued on the following pages)**

| **No** | **R²** | **R⁵** | **IUPAC name** |
|---|---|---|---|
| **Ex.196** | 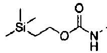 | 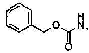 | benzyl *N*-[(4*S*,6*S*,13*S*)-11,15-dimethyl-9,12,16-trioxo-6-({[2-(trimethylsilyl)ethoxy]carbonyl}amino)-2-oxa-8,11,15-triazatricyclo[15.3.1.0^{4,8}]henicosa-1(21),17,19-trien-13-yl]carbamate |
| **Ex.197** | NH₂ | 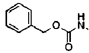 | benzyl *N*-[(4*S*,6*S*,13*S*)-6-amino-11,15-dimethyl-9,12,16-trioxo-2-oxa-8,11,15-triazatricyclo[15.3.1.0^{4,8}]henicosa-1(21),17,19-trien-13-yl]carbamate |
| **Ex.198** | 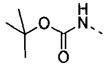 | 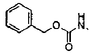 | benzyl *N*-[(4*S*,6*S*,13*S*)-6-[(tert-butoxycarbonyl)amino]-11,15-dimethyl-9,12,16-trioxo-2-oxa-8,11,15-triazatricyclo[15.3.1.0^{4,8}]henicosa-1(21),17,19-trien-13-yl]carbamate |
| **Ex.199** | 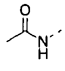 | 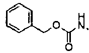 | benzyl *N*-[(4*S*,6*S*,13*S*)-6-(acetylamino)-11,15-dimethyl-9,12,16-trioxo-2-oxa-8,11,15-triazatricyclo[15.3.1.0^{4,8}]henicosa-1(21),17,19-trien-13-yl]carbamate |
| **Ex.200** | 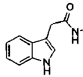 | 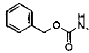 | benzyl *N*-[(4*S*,6*S*,13*S*)-6-{[2-(1H-indol-3-yl)acetyl]amino}-11,15-dimethyl-9,12,16-trioxo-2-oxa-8,11,15-triazatricyclo[15.3.1.0^{4,8}]henicosa-1(21),17,19-trien-13-yl]carbamate |
| **Ex.201** | 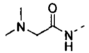 | 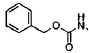 | benzyl *N*-[(4*S*,6*S*,13*S*)-6-{[2-(dimethylamino)acetyl]amino}-11,15-dimethyl-9,12,16-trioxo-2-oxa-8,11,15-triazatricyclo[15.3.1.0^{4,8}]henicosa-1(21),17,19-trien-13-yl]carbamate |
| **Ex.202** | 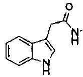 | NH₂ | *N*-[(4*S*,6*S*,13*S*)-13-amino-11,15-dimethyl-9,12,16-trioxo-2-oxa-8,11,15-triazatricyclo[15.3.1.0^{4,8}]henicosa-1(21),17,19-trien-6-yl]-2-(1*H*-indol-3-yl)acetamide |
| **Ex.203** | 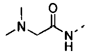 | NH₂ | *N*-[(4*S*,6*S*,13*S*)-13-amino-11,15-dimethyl-9,12,16-trioxo-2-oxa-8,11,15-triazatricyclo[15.3.1.0^{4,8}]henicosa-1(21),17,19-trien-6-yl]-2-(dimethylamino)acetamide |
| **Ex.204** | 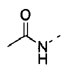 | 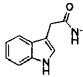 | N-[(4*S*,6*S*,13*S*)-6-(acetylamino)-11,15-dimethyl-9,12,16-trioxo-2-oxa-8,11,15-triazatricyclo[15.3.1.0^{4,8}]henicosa-1(21),17,19-trien-13-yl]-2-(1*H*-indol-3-yl)acetamide |
| **Ex.205** | 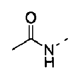 | 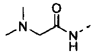 | *N*-[(4*S*,6*S*,13*S*)-6-(acetylamino)-11,15-dimethyl-9,12,16-trioxo-2-oxa-8,11,15-triazatricyclo[15.3.1.0^{4,8}]henicosa-1(21),17,19-trien-13-yl]-2-(dimethylamino)acetamide |
| **Ex.206** | 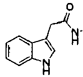 | 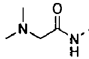 | 2-(dimethylamino)-*N*-[(4*S*,6*S*,13*S*)-6-{[2-(1H-indol-3-yl)acetyl]amino}-11,15-dimethyl-9,12,16-trioxo-2-oxa-8,11,15-triazatricyclo[15.3.1.0^{4,8}]henicosa-1(21),17,19-trien-13-yl]acetamide |
| **Ex.207** | 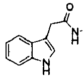 | 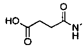 | 4-{[(4*S*,6*S*,13*S*)-6-{[2-(1*H*-indol-3-yl)acetyl]amino}-11,15-dimethyl-9,12,16-trioxo-2-oxa-8,11,15-triazatricyclo[15.3.1.0^{4,8}]henicosa-1(21),17,19-trien-13-yl]amino}-4-oxobutanoic acid |
| **Ex.208** | 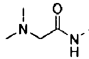 | 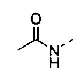 | *N*-[(4*S*,6*S*,13*S*)-13-(acetylamino)-11,15-dimethyl-9,12,16-trioxo-2-oxa-8,11,15-triazatricyclo[15.3.1.0^{4,8}]henicosa-1(21),17,19-trien-6-yl]-2-(dimethylamino)acetamide |
| **Ex.209** | 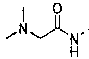 | 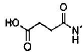 | 4-{[(4*S*,6*S*,13*S*)-6-([2-(dimethylamino)acetyl]amino}-11,15-dimethyl-9,12,16-trioxo-2-oxa-8,11,15-triazatricyclo[15.3.1.0^{4,8}]henicosa-1(21),17,19-trien-13-yl]amino}-4-oxobutanoic acid |
| **Ex.210** | 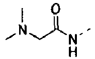 | 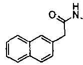 | 2-(dimethylamino)-*N*-[(4*S*,6*S*,13*S*)-11,15-dimethyl-13-{[2-(2-naphthyl)acetyl]amino}-9,12,16-trioxo-2-oxa-8,11,15-triazatricyclo[15.3.1.0^{4,8}]henicosa-1(21),17,19-trien-6-yl]acetamide |
| **Ex.211** | 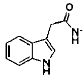 | 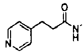 | *N*-[(4*S*,6*S*,13*S*)-6-{[2-(1*H*-indol-3-yl)acetyl]amino}-11,15-dimethyl-9,12,16-trioxo-2-oxa-8,11,15-triazatricyclo[15.3.1.0^{4,8}]henicosa-1(21),17,19-trien-13-yl]-3-(4-pyridinyl)propanamide |
| **Ex.212** | 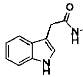 | 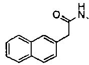 | *N*-[(4*S*,6*S*,13*S*)-11,15-dimethyl-13-{[2-(2-naphthyl)acetyl]amino}-9,12,16-trioxo-2-oxa-8,11,15-triazatricyclo[15.3.1.0^{4,8}]henicosa-1(21),17,19-trien-6-yl]-2-(1*H*-indol-3-yl)acetamide |
| **Ex.213** | 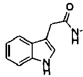 | CH₃(CH₂)₈CONH | *N*-[(4*S*,6*S*,13*S*)-6-{[2-(1*H*-indol-3-yl)acetyl]amino}-11,15-dimethyl-9,12,16-trioxo-2-oxa-8,11,15-triazatricyclo[15.3.1.0^{4,8}]henicosa-1(21),17,19-trien-13-yl]decanamide |
| **Ex.214** | 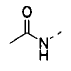 | NH₂ | *N*-[(4*S*,6*S*,13*S*)-13-amino-11,15-dimethyl-9,12,16-trioxo-2-oxa-8,11,15-triazatricyclo[15.3.1.0^{4,8}]henicosa-1(21),17,19-trien-6-yl]acetamide |

**Table 31a: Examples of Core 13 (Ex.215-Ex.230; continued on the following pages)**

| **No** | **R²** | **R⁵** | **Starting material** | **General Procedure** | **Reagent** | **Purification Method** | **Yield (isolated salt)** |
|---|---|---|---|---|---|---|---|
| **Ex.215-Ex.216:** *cf. experimental description* | | | | | | | |
| **Ex.217** | 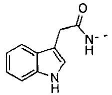 | 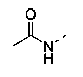 | **Ex.221** | L.1.1 | acetic anhydride (10 equiv.) pyridine/CH₂Cl₂ 1:1 (2 mL) | prep. HPLC, method 1 | 41% |
| **Ex.218** | 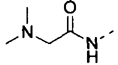 | 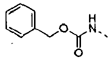 | **Ex.216** ·HCl | L.1.3 | N,N-dimetyl glycine (1.7 equiv.) | FC (CH₂Cl₂/MeOH) | 85%^{*)} |
| **Ex.219** | 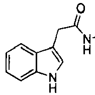 | 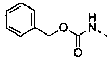 | **Ex.216** ·HCl | L.1.3 | 3-indoleacetic acid (1.1 equiv.) HATU (1.5 equiv.) HOAt (1.5 equiv.) i-Pr₂NEt (5 equiv.) | FC (CH₂Cl₂/MeOH) | 63% |
| **Ex.220** | 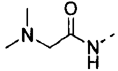 | NH₂ | **Ex.218** | K.1 | H₂, Pd(OH)₂-C | crude product | 93%^{*)} |
| **Ex.221** | 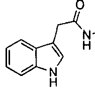 | NH₂ | **Ex.219** | K.1 | H₂, Pd(OH)₂-C | crude product | 94%^{*)} |
| **Ex.222** | 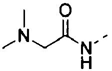 | 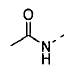 | **Ex.220** | L.1.1 | acetic anhydride (5 equiv.) | prep. HPLC, method 1 | 25% (TFA salt) |
| **Ex.223** | 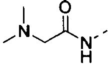 | 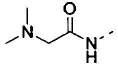 | **Ex.220** | L.1.3 | N,N-dimetyl glycine (1.7 equiv.) | prep. HPLC, method 1 | 24% (TFA salt) |
| **Ex.224** | 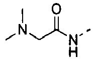 | 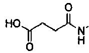 | **Ex.220** | L.1.1 | succinic anhydride (1.05 equiv.) | prep. HPLC, method 1 | 37% (TFA salt) |
| **Ex.225** | 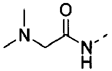 | 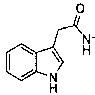 | **Ex.220** | L.1.3 | 3-indoleacetic acid (1.2 equiv.) HATU (1.5 equiv.) HOAt (1.5 equiv.) i-Pr₂NEt (3 equiv.) | prep. HPLC, method 1 | 28% (TFA salt) |
| **Ex.226** | 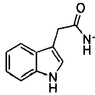 | 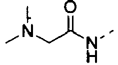 | **Ex.221** | L.1.3 | N,N-dimetyl glycine (1.7 equiv.) | prep. HPLC, method 1 | 45% (TFA salt) |
| **Ex.227** | 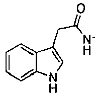 | 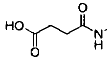 | **Ex.221** | L.1.1 | succinic anhydride (1.05 equiv.) pyridine (49 equiv.) | prep. HPLC, method 1 | 49% |
| **Ex.228** | 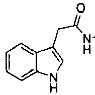 | 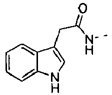 | **Ex.221** | L.1.3 | 3-indoleacetic acid (1.2 equiv.) HATU (1.5 equiv.) HOAt (1.5 equiv.) i-Pr₂NEt (3 equiv.) | prep. HPLC, method 1 | 33% |
| **Ex.229** | 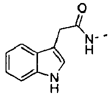 | 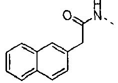 | **Ex.221** | L.1.3 | 2-naphthylacetic acid (1.2 equiv.) | prep. HPLC, method 1 | 56% |
| **Ex.230** | 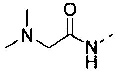 | 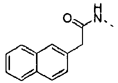 | **Ex.220** | L.1.3 | 2-naphthylacetic acid (1.2 equiv.) | prep. HPLC, method 1 | 52% (TFA salt) |

| | | | | | | | |
|---|---|---|---|---|---|---|---|
| *) Analytical sample further purified by prep. HPLC, method 1 to afford the TFA salt of the product | | | | | | | |

**Table 31b: Examples of Core 13 (Ex.215-Ex.230; continued on the following pages)**

| **No** | **R²** | **R⁵** | **FORMULA** | **MONOISOTOPIC MASS** | **Rt (purity at 220nm)** | **[M+H]⁺ found** | **LC-MS-Method** |
|---|---|---|---|---|---|---|---|
| **Ex.215-Ex.216:** *cf. experimental description* | | | | | | | |
| **Ex.217** | 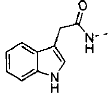 | 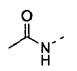 | C31H36N6O6 | 588.3 | 1.82 (94) | 589.2 | Method 1b |
| **Ex.218** | 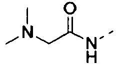 | 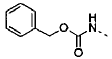 | C31H40N6O7 | 608.3 | 1.36 (100) | 609.2 | Method 4b |
| **Ex.219** | 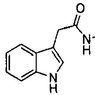 | 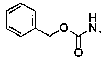 | C37H40N6O7 | 680.3 | 1.64 (96) | 681.3 | Method 2 |
| **Ex.220** | 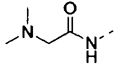 | NH₂ | C23H34N6O5 | 474.3 | 1.19 (92) | 475.2 | Method 5b |
| **Ex.221** | 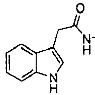 | NH₂ | C29H34N6O5 | 546.3 | 1.38 (96) | 547.2 | Method 4b |
| **Ex.222** | 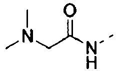 | 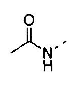 | C25H36N6O6 | 516.3 | 1.23 (97) | 517.2 | Method 5b |
| **Ex.223** | 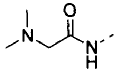 | 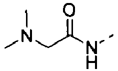 | C27H41N7O6 | 559.3 | 1.33 (94) | 560.3 | Method 5b |
| **Ex.224** | 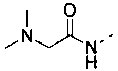 | 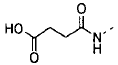 | C27H38N6O8 | 574.3 | 1.04 (100) | 575.2 | Method 5b |
| **Ex.225** | 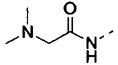 | 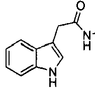 | C33H41N7O6 | 631.3 | 1.30 (97) | 632.3 | Method 4b |
| **Ex.226** | 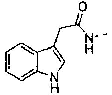 | 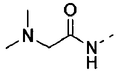 | C33H41N7O6 | 631.3 | 1.39 (92) | 632.2 | Method 4b |
| **Ex.227** | 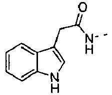 | 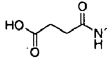 | C33H38N6O8 | 646.3 | 1.49 (94) | 647.2 | Method 4b |
| **Ex.228** | 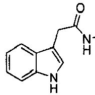 | 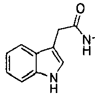 | C39H41N7O6 | 703.3 | 1.75 (91) | 704.3 | Method 4b |
| **Ex.229** | 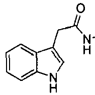 | 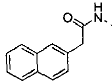 | C41H42N6O6 | 714.3 | 1.91 (92) | 715.3 | Method 4b |
| **Ex.230** | 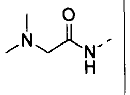 | 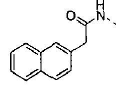 | C35H42N6O6 | 642.3 | 1.50 (100) | 643.2 | Method 4b |

**Table 31c: Examples of Core 13 (Ex.215-Ex.230; continued on the following pages)**

| **No** | **R²** | **R⁵** | **IUPAC name** |
|---|---|---|---|
| **Ex.215** | 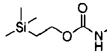 | 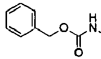 | benzyl *N*-[(4*S*,6*R*,13*S*)-11,15-dimethyl-9,12,16-trioxo-6-({[2-(trimethylsilyl)ethoxy]carbonyl}amino)-2-oxa-8,11,15-triazatricyclo[15.3.1.0^{4,8}]henicosa-1(21),17,19-trien-13-yl]carbamate |
| **Ex.216** | NH₂ | 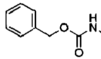 | benzyl *N*-[(4*S*,6*R*,13*S*)-6-amino-11,15-dimethyl-9,12,16-trioxo-2-oxa-8,11,15-triazatricyclo[15.3.1.0^{4,8}]henicosa-1(21),17,19-trien-13-yl]carbamate |
| **Ex.217** | 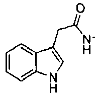 | 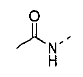 | *N*-[(4*S*,6*R*,13*S*)-13-(acetylamino)-11,15-dimethyl-9,12,16-trioxo-2-oxa-8,11,15-triazatricyclo[15.3.1.0^{4,8}]henicosa-1(21),17,19-trien-6-yl]-2-(1*H*-indol-3-yl)acetamide |
| **Ex.218** | 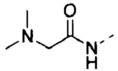 | 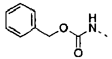 | benzyl *N*-[(4*S*,6*R*,13*S*)-6-{[2-(dimethylamino)acetyl]amino}-11,15-dimethyl-9,12,16-trioxo-2-oxa-8,11,15-triazatricyclo[15.3.1.0^{4,8}]henicosa-1(21),17,19-trien-13-yl]carbamate |
| **Ex.219** | 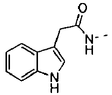 | 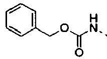 | benzyl *N*-[(4*S*,6*R*,13*S*)-6-{[2-(1-indol-3-yl)acetyl]amino}-11,15-dimethyl-9,12,16-trioxo-2-oxa-8,11,15-triazatricyclo[15.3.1.0^{4,8}]henicosa-1(21),17,19-trien-13-yl]carbamate |
| **Ex.220** | 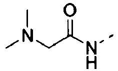 | NH₂ | *N*-[(4*S*,6*R*,13*S*)-13-amino-11,15-dimethyl-9,12,16-trioxo-2-oxa-8,11,15-triazatricyclo[15.3.1.0^{4,8}]henicosa-1(21),17,19-trien-6-yl]-2-(dimethylamino)acetamide |
| **Ex.221** | 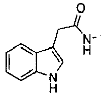 | NH₂ | *N*-[(4*S*,6*R*,13*S*)-13-amino-11,15-dimethyl-9,12,16-trioxo-2-oxa-8,11,15-triazatricyclo[15.3.1.0^{4,8}]henicosa-1(21),17,19-trien-6-yl]-2-(1*H*-indol-3-yl)acetamide |
| **Ex.222** | 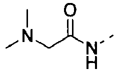 | 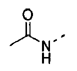 | *N*-[(4*S*,6*R*,13*S*)-13-(acetylamino)-11,15-dimethyl-9,12,16-trioxo-2-oxa-8,11,15-triazatricyclo[15.3.1.0^{4,8}]henicosa-1(21),17,19-trien-6-yl]-2-(dimethylamino)acetamide |
| **Ex.223** | 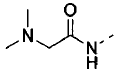 | 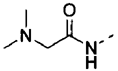 | 2-(dimethylamino)-*N*-[(4*S*,6*R*,13*S*)-13-{[2-(dimethylamino)acetyl]amino}-11,15-dimethyl-9,12,16-trioxo-2-oxa-8,11,15-triazatricyclo[15.3.1.0^{4,8}]henicosa-1(21),17,19-trien-6-yl]acetamide |
| **Ex.224** | 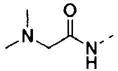 | 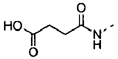 | 4-{[(4*S*,6*R*,13*S*)-6-{[2-(dimethylamino)acetyl]amino}-11,15-dimethyl-9,12,16-trioxo-2-oxa-8,11,15-triazatricyclo[15.3.1.0^{4,8}]henicosa-1(21),17,19-trien-13-yl]amino}-4-oxobutanoic acid |
| **Ex.225** | 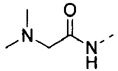 | 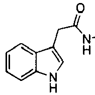 | 2-(dimethylamino)-*N*-[(4*S*,6*R*,13*S*)-13-{[2-(1*H*-indol-3-yl)acetyl]amino}-11,15-dimethyl-9,12,16-trioxo-2-oxa-8,11,15-triazatricyclo[15.3.1.0^{4,8}]henicosa-1(21),17,19-trien-6-yl]acetamide |
| **Ex.226** | 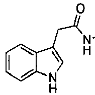 | 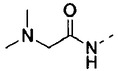 | 2-(dimethylamino)-*N*-[(4*S*,6*R*,13*S*)-6-{[2-(1*H*-indol-3-yl)acetyl]amino}-11,15-dimethyl-9,12,16-trioxo-2-oxa-8,11,15-triazatricyclo[15.3.1.0^{4,8}]henicosa-1(21),17,19-trien-13-yl]acetamide |
| **Ex.227** | 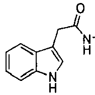 | 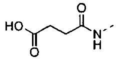 | 4-{[(4*S*,6*R*,13*S*)-6-{[2-(1*H*-indol-3-yl)acetyl]amino}-11,15-dimethyl-9,12,16-trioxo-2-oxa-8,11,15-triazatricyclo[15.3.1.0^{4,8}]henicosa-1(21),17,19-trien-13-yl]amino}-4-oxobutanoic acid |
| **Ex.228** | 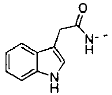 | 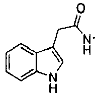 | 2-(1*H*-indol-3-yl)-*N*-[(4*S*,6*R*,13*S*)-13-{[2-(1H-indol-3-yl)acetyl]amino}-11,15-dimethyl-9,12,16-trioxo-2-oxo-8,11,15-triazatricyclo[15.3.1.0^{4,8}]henicosa-1(21),17,19-trien-6-yl]acetamide |
| **Ex.229** | 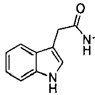 | 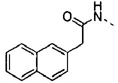 | *N*-[(4*S*,6*R*,13*S*)-11,15-dimethyl-13-{[2-(2-naphthyl)acetyl]amino}-9,12,16-trioxo-2-oxa-8,11,15-triazatricyclo[15.3.1.0^{4,8}]henicosa-1(21),17,19-trien-6-yl]-2-(1*H*-indol-3-yl)acetamide |
| **Ex.230** | 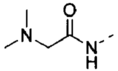 | 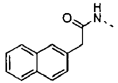 | 2-(dimethylamino)-*N*-[(4*S*,6*R*,13*S*)-11,15-dimethyl-13-{[2-(2-naphthyl)acetyl]amino}-9,12,16-trioxo-2-oxa-8,11,15-triazatricyclo[15.3.1.0^{4,8}]henicosa-1(21),17,19-trien-6-yl]acetamide |

**Table 32a: Examples of Core 14 (Ex.231-Ex.237)**

| **No** | **R²** | **R⁵** | **Starting material** | **General Procedure** | **Reagent** | **Purification Method** | **Yield (isolated salt)** |
|---|---|---|---|---|---|---|---|
| **Ex.231-Ex.232:** *cf. experimental description* | | | | | | | |
| **Ex.233** | 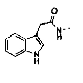 | 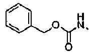 | **Ex.232**·TFA | L.1.3 | 3-indoleacetic acid (1.5 equiv.) HATU (1.5 equiv.) HOAt (1.5 equiv.) i-Pr₂NEt (5 equiv) | FC (EtOAc, then CH₂Cl₂/MeOH) | 68% |
| **Ex.234** | 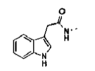 | NH₂ | **Ex.233** | K.1 | H₂, Pd(OH)₂-C | crude product | 99% |
| **Ex.235** | 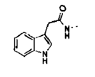 | 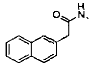 | **Ex.234** | L.1.3 | 2-naphthylacetic acid (1.2 equiv.) | prep. HPLC, method 3 | 68% |
| **Ex.236** | 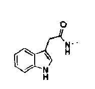 | 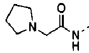 | **Ex.234** | L.1.3 | pyrrolidin-1-acetic acid (1,2 equiv.) | prep. HPLC, method 3 | 28% |
| **Ex.237** | 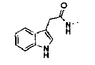 | 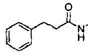 | **Ex.234** | L.1.3 | 3-phenylpropionic acid | prep. HPLC, method 3 | 66% |

**Table 32b: Examples of Core 14 (Ex.231-Ex.237)**

| **No** | **R²** | **R⁵** | **FORMULA** | **MONOISOTOPIC MASS** | **Rt (purity at 220nm)** | **[M+H]⁺ found** | **LC-MS-Method** |
|---|---|---|---|---|---|---|---|
| **Ex.231-Ex.232:** *cf. experimental description* | | | | | | | |
| **Ex.233** | 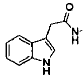 | 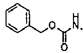 | C37H40N6O7 | 680.3 | 1.80 (96) | 681.4 | Method 2 |
| **Ex.234** | 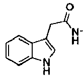 | NH₂ | C29H34N6O5 | 546.3 | 1.47 (93) | 547.3 | Method 2 |
| **Ex.235** | 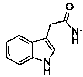 | 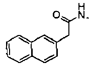 | C41H42N6O6 | 714.3 | 1.80 (90) | 715.4 | Method 2 |
| **Ex.236** | 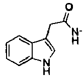 | 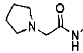 | C35H43N7O6 | 657.3 | 1.45 (94) | 658.4 | Method 2 |
| **Ex.237** | 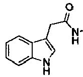 | 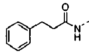 | C38H42N6O6 | 678.3 | 1.81 (96) | 679.4 | Method 2 |

**Table 32c: Examples of Core 14 (Ex.231-Ex.237)**

| **No** | **R²** | **R⁵** | **IUPAC name** |
|---|---|---|---|
| **Ex.231** | 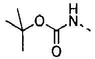 | 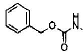 | benzyl N-[(4S,6S,13R)-6-[(tert-butoxycarbonyl)amino]-11,15-dimethyl-9,12,16-trioxo-2-oxa-8,11,15-triazatricyclo[15.3.1.0^{4,8}]henicosa-1(21),17,19-trien-13-yl]carbamate |
| **Ex.232** | NH₂ | 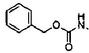 | benzyl *N*-[(4*S*,6*S*,13*R*)-6-amino-11,15-dimethyl-9,12,16-trioxo-2-oxa-8,11,15-triazatricyclo[15.3.1.0^{4,8}]henicosa-1(21),17,19-trien-13-yl]carbamate |
| **Ex.233** | 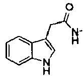 | 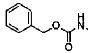 | benzyl *N*-[(4*S*,6*S*,13*R*)-*6*-{[2-(1H-indol-3-yl)acetyl]amino}-11,15-dimethyl-9,12,16-trioxo-2-oxa-8,11,15-triazatricyclo[15.3. 1.0^{4,8}]henicosa-1(21),17,19-trien-13-yl]carbamate |
| **Ex.234** | 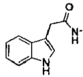 | NH₂ | *N*-[(4*S*,6*S*,13*R*)-13-amino-11,15-dimethyl-9,12,16-trioxo-2-oxa-8,11,15-triazatricyclo[15.3.1.0^{4,8}]henicosa-1(21),17,19-trien-6-yl]-2-(1*H*-indol-3-yl)acetamide |
| **Ex.235** | 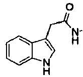 | 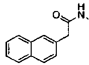 | *N*-[(4*S*,6*S*,13*R*)-11,15-dimethyl-13-{[2-(2-naphthyl)acetyl]amino}-9,12,16-trioxo-2-oxa-8,11,15-triazatricyclo[15.3.1.0^{4,8}]henicosa-1(21),17,19-trien-6-yl]-2-(1*H-*indol-3-yl)acetamide |
| **Ex.236** | 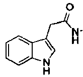 | 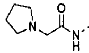 | *N*-[(4*S*,6*S*,13*R*)-11,15-dimethyl-9,12,16-trioxo-13-{[2-(1-pyrrolidinyl)acetyl]amino}-2-oxa-8,11,15-triazatricyclo[15.3.1.0^{4,8}]henicosa-1(21),17,19-trien-6-yl]-2-(1*H*-indol-3-yl)acetamide |
| **Ex.237** | 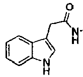 | 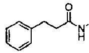 | *N*-[(4*S*,6*S*,13*R*)-6-{[2-(1H-indol-3-yl)acetyl]amino}-11,15-dimethyl-9,12,16-trioxo-2-oxa-8,11,15-triazatricyclo[15.3.1.0^{4,8}]henicosa-1(21),17,19-trien-13-yl]-3-phenylpropanamide |

**Table 33a: Examples of Core 15 and Core 16 (Ex.238-Ex.247; continued on the following page)**

| **No** | **R²** | **R⁵** | **Starting material** | **General Procedure** | **Reagent** | **Purification Method** | **Yield (isolated salt)** |
|---|---|---|---|---|---|---|---|
| **Ex.238-Ex.239:** *cf. experimental description* | | | | | | | |
| **Ex.240** | 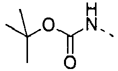 | 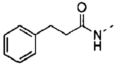 | **Ex.239** | L.1.3 | 3-phenylpoanoic acid | FC (EtOAc/MeOH)*⁾ | 47% |
| **Ex.241** | 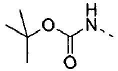 | 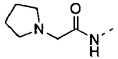 | **Ex.239** | L.1.3 | pyrrolidin-1-acetic acid | FC (CH₂Cl₂/MeOH/aq. NH₃) | 56% |
| **Ex.242** | NH₂ | 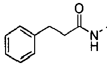 | **Ex.240** | J | HCl-dioxane | crude product | quant. (HCl salt) |
| **Ex.243** | NH₂ | 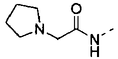 | **Ex.241** | J | HCl-dioxane | crude product | quant. (HCl salt) |
| **Ex.244** | 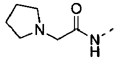 | 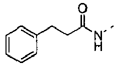 | **Ex.242** | L.1.3 | pyrrolidin-1-acetic acid | FC (CH₂Cl₂/MeOH/aq. NH₃) | 81% |
| **Ex.245** | 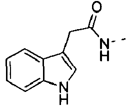 | 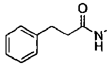 | **Ex.242** | L.1.3 | 3-indoleacetic acid | FC (CH₂Cl₂/MeOH/aq. NH₃) | 59% |
| **Ex.246** | 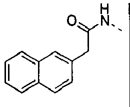 | 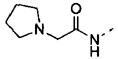 | **Ex.243** | L.1.3 | 2-naphthylacetic acid | FC (CH₂Cl₂/MeOH/aq. NH₃) | 51% |
| **Ex.247** | 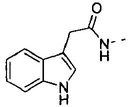 | 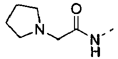 | **Ex.243** | L.1.3 | 3-indoleacetic acid | FC (CH₂Cl₂/MeOH/aq. NH₃) | 58% |

| | | | | | | | |
|---|---|---|---|---|---|---|---|
| *) An analytical sample was further purified by prep. HPLC, method 2 | | | | | | | |

**Table 33b: Examples of Core 15 and Core 16 (Ex.238-Ex.247; continued on the following page)**

| **No** | **R²** | **R⁵** | **FORMULA** | **MONOISOTOPIC MASS** | **Rt (purity at 220nm)** | **[M+H]⁺ found** | **LC-MS-Method** |
|---|---|---|---|---|---|---|---|
| **Ex.238-Ex.239:** *cf. experimental description* | | | | | | | |
| **Ex.240** | 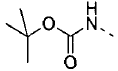 | 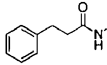 | C33H44N4O6 | 592.3 | 2.12 (90) | 593.3 | Method 4a |
| **Ex.241** | 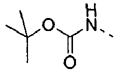 | 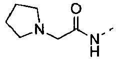 | C30H45N5O6 | 571.3 | 1.58 (91') | 572.3 | Method 4a |
| **Ex.242** | NH₂ | 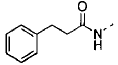 | C28H36N4O4 | 492.3 | 1.53 (88), 1.59 (6) | 493.2 | Method 4a |
| **Ex.243** | NH₂ | 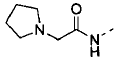 | C25H37N5O4 | 471.3 | 1.08 (91) | 472.4 | Method 4a |
| **Ex.244** | 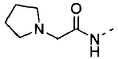 | 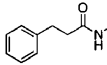 | C34H45N5O5 | 603.3 | 1.63 (93) | 604.4 | Method 4a |
| **Ex.245** | 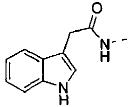 | 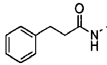 | C38H43N5O5 | 649.3 | 2.01 (91) | 650.3 | Method 4a |
| **Ex.246** | 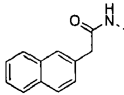 | 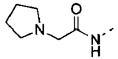 | C37H45N5O5 | 639.3 | 1.69 (90) | 640.3 | Method 4a |
| **Ex.247** | 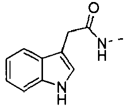 | 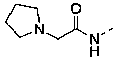 | C35H44N6O5 | 628.3 | 1.52 (94) | 629.3 | Method 4a |

**Table 33c: Examples of Core 15 and Core 16 (Ex.238-Ex.247; continued on the following page)**

| **No** | **R²** | **R⁵** | **IUPAC name** |
|---|---|---|---|
| **Core 15** | | | |
| **Ex.238** | 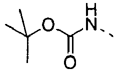 | 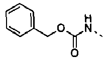 | benzyl *N*-[(4*S*,6*S*,10*S*)-6-[(*tert*-butoxycarbonyl)amino]-15-methyl-9,16-dioxo-2-oxa-8,15-diazatricyclo[15.3.1.0^{4,8}]henicosa-1(21),12,17,19-tetraen-10-yl]carbamate |

| **Core 16** | | | |
|---|---|---|---|
| **Ex.239** | 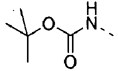 | NH₂ | *tert*-butyl *N*-[(A*S*,6*S*,10*S*)-10-amino-15-methyl-9,16-dioxo-2-oxa-8,15-diazatricyclo[15.3.1.0^{4,8}]henicosa-1(21),17,19-trien-6-yl]carbamate |
| **Ex.240** | 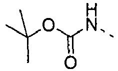 | 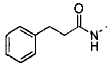 | tert-butyl *N*-[(4*S*,6*S*,10*S*)-15-methyl-9,16-dioxo-10-[(3-phenylpropanoyl)amino]-2-oxa-8,15-diazatricyclo[15.3.1.0^{4,8}]henicosa-1(21),17,19-trien-6-yl]carbamate |
| **Ex.241** | 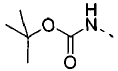 | 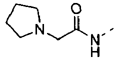 | *tert*-butyl *N*-[(4*S*,6*S*,10*S*)-15-methyl-9,16-dioxo-10-{[2-(1-pyrrolidinyl)acetyl]amino}-2-oxa-8,15-diazatricyclo[15.3.1.0^{4,8}]henicosa-1(21),17,19-trien-6-yl]carbamate |
| **Ex.242** | NH₂ | 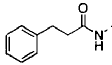 | *N*-[(4*S*,6*S*,10*S*)-6-amino-15-methyl-9,16-dioxo-2-oxa-8,15-diazatricyclo[15.3.1.0^{4,8}]henicosa-1(21),17,19-trien-10-yl]-3-phenylpropanamide |
| **Ex.243** | NH₂ | 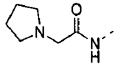 | *N*-[(4*S*,6*S*,10*S*)-6-amino-15-methyl-9,16-dioxo-2-oxa-8,15-diazatricyclo[15.3.1.0^{4,8}]henicosa-1(21),17,19-trien-10-yl]-2-(1-pyrrolidinyl)acetamide |
| **Ex.244** | 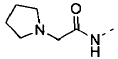 | 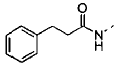 | *N*-[(4*S*,6*S*,10*S*)-15-methyl-9,16-dioxo-6-{[2-(1-pyrrolidinyl)acetyl]amino}-2-oxa-8,15-diazatricyclo[15.3.1.0^{4,8}]henicosa-1(21),17,19-trien-10-yl]-3-phenylpropanamide |
| **Ex.245** | 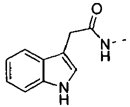 | 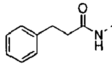 | N-[(4*S*,6*S*,10*S*)-6-{[2-(1H-indol-3-yl)acetyl]amino}-15-methyl-9,16-dioxo-2-oxa-8,15-diazatricyclo[15.3.1.0^{4,8}]henicosa-1(21),17,19-trien-10-yl]-3-phenylpropanamide |
| **Ex.246** | 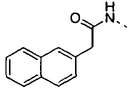 | 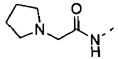 | *N*-[(4*S*,6*S*,10*S*)-15-methyl-9,16-dioxo-10-{[2-(1-pyrrolidinyl)acetyl]amino}-2-oxa-8,15-diazatricyclo[15.3.1.0^{4,8}]henicosa-1(21),17,19-trien-6-yl]-2-(2-naphthyl)acetamide |
| **Ex.247** | 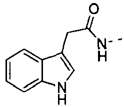 | 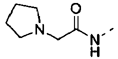 | 2-(1*H*-indol-3-yl)-*N*-[(4*S*,6*S*,10*S*)-15-methyl-9,16-dioxo-10-{[2-(1-pyrrolidinyl)acetyl]amino}-2-oxa-8,15-diazatricyclo[15.3.1.0^{4,8}]henicosa-1(21),17,19-trien-6-yl]acetamide |

**Table 34a: Examples of Core 17 (Ex.248-Ex.271; continued on the following pages)**

| **No** | **R²** | **R⁵⁴** | **Starting material** | **General Procedure** | **Reagent** | **Purification Method** | **Yield (isolated salt)** |
|---|---|---|---|---|---|---|---|
| **Ex.248-Ex.249:** *cf. experimental description* | | | | | | | |
| **Ex.250** | 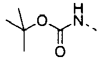 | 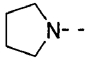 | **Ex.249** | L.2 | pyrrolidine | FC (CH₂Cl₂/MeOH) | 81% |
| **Ex.251** | 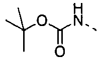 | 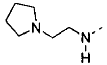 | **Ex.249** | L.2 | N-(2-aminoethyl) pyrrolidine | FC (CH₂Cl₂/MeOH/ aq. NH₃ soln) | 88% |
| **Ex.252** | 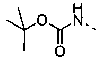 | 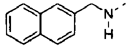 | **Ex.249** | L.2 | 2-naphthylmethylamine (1.5 equiv.) HATU (1.5 equiv.) HOAt (1.5 equiv.) i-Pr₂NEt (3 equiv.) | FC (CH₂Cl₂/MeOH) | 91% |
| **Ex.253** | 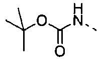 | 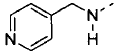 | **Ex.249** | L.2 | 4-(aminomethyl)-pyridine (1.5 equiv.) HATU (1.5 equiv.) HOAt (1.5 equiv.) i-Pr₂NEt (3 equiv.) | FC (CH₂Cl₂/MeOH) *) | 97% |
| **Ex.254** | NH₂ | 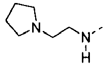 | **Ex.251** | J | HCl-dioxane | crude product | 96% (HCl salt) |
| **Ex.255** | NH₂ | 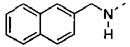 | **Ex.252** | J | HCl-dioxane | crude product | 100% (HCl salt) |
| **Ex.256** | NH₂ | 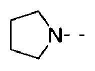 | **Ex.250** | J | HCl-dioxane | crude product | 93% (HCl salt) |
| **Ex.257** | NH₂ | 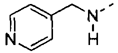 | **Ex.253** | J | HCl-dioxane | crude product | 91% (HCl salt) |
| **Ex.258** | 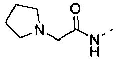 | 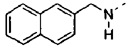 | **Ex.255** | L.1.2 | 1-pyrrolidinacetic acid (2.7 equiv.) i-Pr₂NEt v(4 equiv.) | FC (CH₂Cl₂/MeOH) | 61% |
| **Ex.259** | 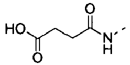 | 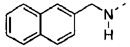 | **Ex.255** | L.1.1 | succininc anhydride (1.05 equiv.) pyridine (49 equiv.) | prep. HPLC, method 1 | 15% |
| **Ex.260** | 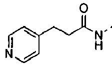 | 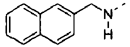 | **Ex.255** | L.1.2 | 3-(pyridine-4-yl)propanoic acid | prep. HPLC, method 1 | 55% (TFA salt) |
| **Ex.261** | 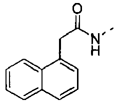 | 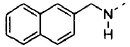 | **Ex.255** | L.1.3 | 1-naphthylacetic acid (1.2 equiv.) | prep. HPLC, method 1 | 69% |
| **Ex.262** | 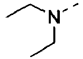 | 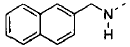 | **Ex.255** | M.1 | acetaldehyde | prep. HPLC, method 1 | 76% (TFA salt) |
| **Ex.263** | 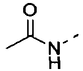 | 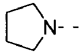 | **Ex.256** | L.1.1 | acetic anhydride (10 equiv.) Pyridine/CH₂Cl₂ 1:1 (3 mL) | prep. HPLC, method 1 | 82% |
| **x.264** | 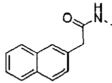 | 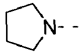 | **Ex.256** | L.1.3 | 2-naphthylacetic acid (1.2 equiv.) | prep. HPLC, method 1 | 61% |
| **Ex.265** | 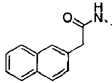 | 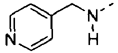 | **Ex.257** | L.1.3 | 2-naphthylacetic acid (1.2 equiv.) | prep. HPLC, method 1 | 59% (TFA salt) |
| **Ex.266** | 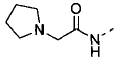 | 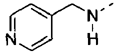 | **Ex.257** | L.1.2 | 1-pyrrolidinacetic acid | prep. HPLC, method 2 | 71% |
| **Ex.267** | 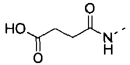 | 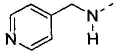 | **Ex.257** | L.1.1 | succininc anhydride (1.5 equiv.) pyridine (49 equiv.) | prep. HPLC, method 1 | 89% (TFA salt) |
| **Ex.268** | 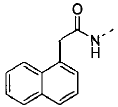 | 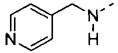 | **Ex.257** | L.1.3 | 1-naphthylacetic acid (1.2 equiv.) | prep. HPLC, method 2 | 45% |
| **Ex.269** | 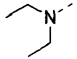 | 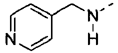 | **Ex.257** | M.1 | acetaldehyde (0.75 mL) | prep. HPLC, method 2 | 70% |
| **Ex.270** | 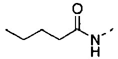 | 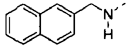 | **Ex.255** | L.1.1 | valeroyl chloride (2 equiv.) | prep. HPLC, method 1 | 77% |
| **Ex.271** | 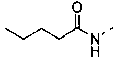 | 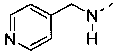 | **Ex.257** | L.1.1 | valeroyl chloride (2 equiv.) | prep. HPLC, method 1 | 58% (TFA salt) |

| | | | | | | | |
|---|---|---|---|---|---|---|---|
| *) An analytical sample was further purified by prep HPLC, method 1 | | | | | | | |

**Table 34b: Examples of Core 17 (Ex.248-Ex.271; continued on the following pages)**

| **No** | **R²** | **R⁵⁴** | **FORMULA** | **MONOISOTOPIC MASS** | **Rt (purity at 220nm)** | **[M+H]⁺ found** | **LC-MS-Method** |
|---|---|---|---|---|---|---|---|
| **Ex.248-Ex.249:** *cf. experimental description* | | | | | | | |
| **Ex.250** | 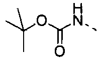 | 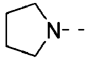 | C30H43N5O7 | 585.4 | 1.58 (96) | 586.4 | Method 2 |
| **Ex.251** | 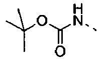 | 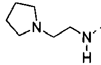 | C32H48N6O7 | 628.4 | 1.35 (98) | 629.5 | Method 2 |
| **Ex.252** | 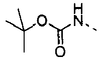 | 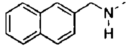 | C37H45N5O7 | 671.3 | 1.86 (88) | 672.4 | Method 2 |
| **Ex.253** | 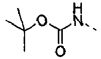 | 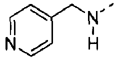 | C32H42N6O7 | 622.3 | 1.29 (92) | 623.4 | Method 2 |
| **Ex.254** | NH₂ | 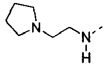 | C27H40N6O5 | 528.3 | 1.31 (97) | 529.5 | Method 3 |
| **Ex.255** | NH₂ | 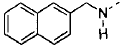 | C32H37N5O5 | 571.3 | 1.45 (94) | 572.4 | Method 2 |
| **Ex.256** | NH₂ | 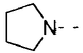 | C25H35N5O5 | 485.3 | 1.06 (96) | 486.4 | Method 2 |
| **Ex.257** | NH₂ | 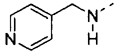 | C27H34N6O5 | 522.3 | 0.85 (96) | 523.3 | Method 2 |
| **Ex.258** | 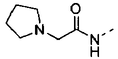 | 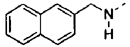 | C38H46N6O6 | 682.4 | 1.47 (95) | 683.5 | Method 2 |
| **Ex.259** | 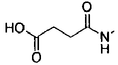 | 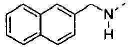 | C36H41N5O8 | 671.3 | 1.59 (87) | 672.4 | Method 2 |
| **Ex.260** | 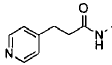 | 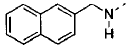 | C40H44N6O6 | 704.3 | 1.47 (100) | 705.5 | Method 2 |
| **Ex.261** | 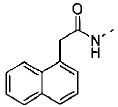 | 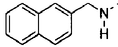 | C44H45N5O6 | 739.3 | 1.93 (99) | 740.5 | Method 2 |
| **Ex.262** | 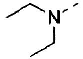 | 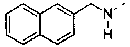 | C36H45N5O5 | 627.3 | 1.50 (100) | 628.5 | Method 2 |
| **Ex.263** | 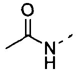 | 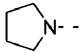 | C27H37N5O6 | 527.3 | 1.23 (99) | 528.3 | Method 2 |
| **Ex.264** | 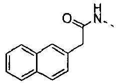 | 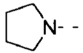 | C37H43N5O6 | 653.3 | 1.68 (100) | 654.4 | Method 2 |
| **Ex.265** | 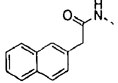 | 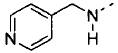 | C39H42N6O6 | 690.3 | 1.44 (99) | 691.5 | Method 2 |
| **Ex.266** | 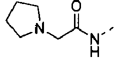 | 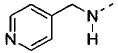 | C33H43N7O6 | 633.3 | 0.99 (94) | 634.5 | Method 2 |
| **Ex.267** | 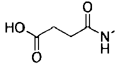 | 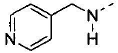 | C31H38N6O8 | 622.3 | 1.05 (94) | 623.3 | Method 2 |
| **Ex.268** | 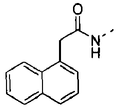 | 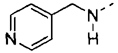 | C39H42N6O6 | 690.3 | 1.68 (93) | 691.5 | Method 3 |
| **Ex.269** | 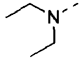 | 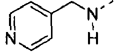 | C31H92N6O5 | 578.3 | 0.96 (96) | 579.5 | Method 2 |
| **Ex.270** | 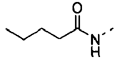 | 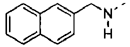 | C37H45N5O6 | 655.3 | 1.81 (100) | 656.4 | Method 2 |
| **Ex.271** | 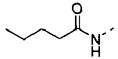 | 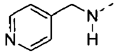 | C32H42N6O6 | 606.3 | 1.25 (100) | 607.4 | Method 2 |

**Table 34c: Examples of Core 17 (Ex.248-Ex.271; continued on the following pages)**

| **No** | **R²** | **R⁵⁴** | **IUPAC name** |
|---|---|---|---|
| **Ex.248** | 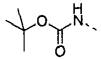 | OCH₂Ph | benzyl (4S,6R,15S)-6-[(tert-butoxycarbonyl)amino]-11,16-dimethyl-9,12,17-trioxo-2-oxa-8,11,16-triazatricyclo[16.2.2.0^{4,8}]docosa-1(20),18,21-triene-15-carboxylate |
| **Ex.249** | 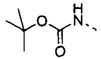 | OH | (4*S*,6*R*,15*S*)-6-[(*tert*-butoxycarbonyl)amino]-11,16-dimethyl-9,12,17-trioxo-2-oxa-8,11,16-triazatricyclo[16.2.2.0^{4,8}]docosa-1(20),18,21-triene-15-carboxylic acid |
| **Ex.250** | 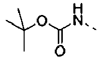 | 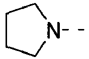 | *tert*-butyl *N*-[(4*S*,6*R*,15*S*)-11,16-dimethyl-9,12,17-trioxo-15-(1-pyrrolidinylcarbonyl)-2-oxa-8,11,16-triazatricyclo[16.2.2.0^{4,8}]docosa-1(20),18,21-trien-6-yl]carbamate |
| **Ex.251** | 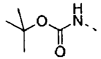 | 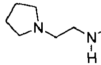 | tert-butyl *N*-[(4*S*,6*R*,15*S*)-11,16-dimethyl-9,12,17-trioxo-15-({[2-(1-pyrrolidinyl)ethyl]amino}carbonyl)-2-oxa-8,11,16-triazatricyclo[16.2.2.0^{4,8}]docosa-1(20),18,21-trien-6-yl]carbamate |
| **Ex.252** | 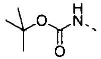 | 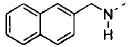 | *tert*-butyl *N*-[(4*S*,6*R*,15*S*)-11,16-dimethyl-15-{[(2-naphthylmethyl)amino]carbonyl}-9,12,17-trioxo-2-oxa-8,11,16-triazatricyclo[16.2.2.0^{4,8}]docosa-1(20),18,21-trien-6-yl]carbamate |
| **Ex.253** | 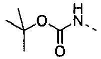 | 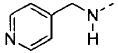 | tert-butyl *N*-[(4*S*,6*R*,15*S*)-11,16-dimethyl-9,12,17-trioxo-15-{[(4-pyridinylmethyl)amino]carbonyl}-2-oxa-8,11,16-triazatricyclo[16.2.2.0^{4,8}]docosa-1(20),18,21-trien-6-yl]carbamate |
| **Ex.254** | NH₂ | 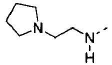 | (4*S*,6*R*,15*S*)-6-amino-11,16-dimethyl-9,12,17-trioxo-*N*-[2-(1-pyrrolidinyl)ethyl]-2-oxa-8,11,16-triazatricyclo[16.2.2.0^{4,8}]docosa-1(20),18,21-triene-15-carboxamide |
| **Ex.255** | NH₂ | 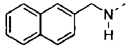 | (4*S*,6*R*,15*S*)-6-amino-11,16-dimethyl-*N*-(2-naphthylmethyl)-9,12,17-trioxo-2-oxa-8,11,16-triazatricyclo[16.2.2.0^{4,8}]docosa-1(20),18,21-triene-15-carboxamide |
| **Ex.256** | NH₂ | 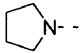 | (4*S*,6*R*,15*S*)-6-amino-11,16-dimethyl-15-(1-pyrrolidinylcarbonyl)-2-oxa-8,11,16-triazatricyclo[16.2.2.0^{4,8}]docosa-1(20),18,21-triene-9,12,17-trione |
| **Ex.257** | NH₂ | 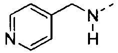 | (4*S*,6*R*,15*S*)-6-amino-11,16-dimethyl-9,12,17-trioxo-*N*-(4-pyridinylmethyl)-2-oxa-8,11,16-triazatricyclo[16.2.2.0^{4,8}]docosa-1(20),18,21-triene-15-carboxamide |
| **Ex.258** | 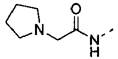 | 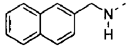 | (4*S*,6*R*,15*S*)-11,16-dimethyl-*N*-(2-naphthylmethyl)-9,12,17-trioxo-6-{[2-(1-pyrrolidinyl)acetyl]amino}-2-oxa-8,11,16-triazatricyclo[16.2.2.0^{4,8}]docosa-1(20),18,21-triene-15-carboxamide |
| **Ex.259** | 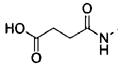 | 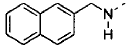 | 4-{[(A*S*,6*R*,15*S*)-11,16-dimethyl-15-{[(2-naphthylmethyl)amino]carbonyl}-9,12,17-trioxo-2-oxa-8,11,16-triazatricyclo[16.2.2.0^{4,8}]docosa-1(20),18,21-trien-6-yl]amino}-4-oxobutanoic acid |
| **Ex.260** | 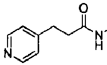 | 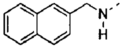 | (4*S*,6*R*,15*S*)-11,16-dimethyl-*N*-(2-naphthylmethyl)-9,12,17-trioxo-6-{[3-(4-pyridinyl)propanoyl]amino}-2-oxa-8,11,16-triazatricyclo[16.2.2.0^{4,8}]docosa-1(20),18,21-triene-15-carboxamide |
| **Ex.261** | 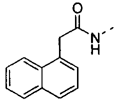 | 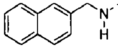 | (4*S*,6*R*,15*S*)-11,16-dimethyl-6-{[2-(1-naphthyl)acetyl]amino}-*N*-(2-naphthylmethyl)-9,12,17-trioxo-2-oxa-8,11,16-triazatricyclo[16.2.2.0^{4,8}]docosa-1(20),18,21-triene-15-carboxamide |
| **Ex.262** | 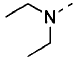 | 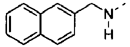 | (4S,6R,15S)-6-(diethylamino)-11,16-dimethyl-N-(2-naphthylmethyl)-9,12,17-trioxo-2-oxa-8,11,16-triazatricyclo[16.2.2.0^{4,8}]docosa-1(20),18,21-triene-15-carboxamide |
| **Ex.263** | 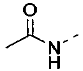 | 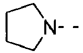 | *N*-[(4*S*,6*R*,15*S*)-11,16-dimethyl-9,12,17-trioxo-15-(1-pyrrolidinylcarbonyl)-2-oxa-8,11,16-triazatricyclo[16.2.2.0^{4,8}]docosa-1(20),18,21-trien-6-yl]acetamide |
| **Ex.264** | 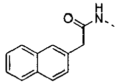 | 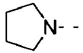 | N-[(4S,6R,15S)-11,16-dimethyl-9,12,17-trioxo-15-(1-pyrrolidinylcarbonyl)-2-oxa-8,11,16-triazatricyclo[16.2.2.0^{4,8}]docosa-1(20),18,21-trien-6-yl]-2-(2-naphthyl)acetamide |
| **Ex.265** | 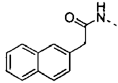 | 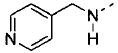 | (4*S*,6*R*,15*S*)-11,16-dimethyl-6-{[2-(2-naphthyl)acetyl]amino}-9,12,17-trioxo-*N-*(4-pyridinylmethyl)-2-oxa-8,11,16-triazatricyclo[16.2.2.0^{4,8}]docosa-1(20),18,21-triene-15-carboxamide |
| **Ex.266** | 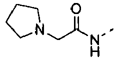 | 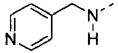 | (4*S*,6*R*,15*S*)-11,16-dimethyl-9,12,17-trioxo-N-(4-pyridinylmethyl)-6-{[2-(1-pyrrolidinyl)acetyl]amino}-2-oxa-8,11,16-triazatricyclo[16.2.2.0^{4,8}]docosa-1(20),18,21-triene-15-carboxamide |
| **Ex.267** | 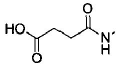 | 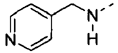 | 4-{[(4*S*,6*R*,15*S*)-11,16-dimethyl-9,12,17-trioxo-15-{[(4-pyridinylmethyl)amino]carbonyl}-2-oxa-8,11,16-triazatricyclo[16.2.2.0^{4,8}]docosa-1(20),18,21-trien-6-yl]amino}-4-oxobutanoic acid |
| **Ex.268** | 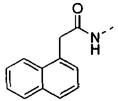 | 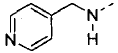 | (4*S*,6*R*,15*S*)-11,16-dimethyl-6-{[2-(1-naphthyl)acetyl]amino}-9,12,17-trioxo-*N-*(4-pyridinylmethyl)-2-oxa-8,11,16-triazatricyclo[16.2.2.0^{4,8}]docosa-1(20),18,21-triene-15-carboxamide |
| **Ex.269** | 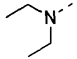 | 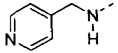 | (4*S*,6*R*,15*S*)-6-(diethylamino)-11,16-dimethyl-9,12,17-trioxo-*N*-(4-pyridinylmethyl)-2-oxa-8,11,16-triazatricyclo[16.2.2.0^{4,8}]docosa-1(20),18,21-triene-15-carboxamide |
| **Ex.270** | 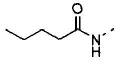 | 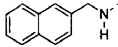 | (4*S*,6*R*,15*S*)-11,16-dimethyl-*N*-(2-naphthylmethyl)-9,12,17-trioxo-6-(pentanoylamino)-2-oxa-8,11,16-triazatricyclo[16.2.2.0^{4,8}]docosa-1(20),18,21-triene-15-carboxamide |
| **Ex.271** | 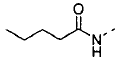 | 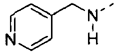 | (4*S*,6*R*,15*S*)-11,16-dimethyl-9,12,17-trioxo-6-(pentanoylamino)-*N*-(4-pyridinylmethyl)-2-oxa-8,11,16-triazatricyclo[16.2.2.0^{4,8}]docosa-1(20),18,21-triene-15-carboxamide |

**Table 35a: Examples of Core 18 (Ex.272-Ex.296; continued on the following pages)**

| **No** | **R²** | **R⁵** | **Starting material** | **General Procedure** | **Reagent** | **Purification Method** | **Yield (isolated salt)** |
|---|---|---|---|---|---|---|---|
| **Ex.272-Ex.274:** *cf. experimental description* | | | | | | | |
| **Ex.275** | 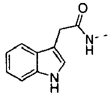 | 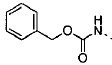 | **Ex.273** (HCl salt) | L.1.3 | 3-indoleacetic acid (1.1 equiv.) HATU (1.5 equiv.) HOAt (1.5 equiv.) i-Pr₂NEt (5 equiv.) | FC (CH₂Cl₂/MeOH) | 53% |
| **Ex.276** | 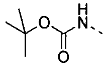 | 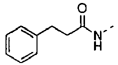 | **Ex.274** | L.1.3 | 3-phenylpropanoic acid(1.2 equiv.) HATU (1.5 equiv.) HOAt (1.5 equiv.) i-Pr₂NEt (3 equiv.) | FC (CH₂Cl₂/MeOH) | 72% |
| **Ex.277** | NH₂ | 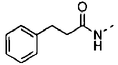 | **Ex.276** | J | HCl-dioxane | crude product | quant. (HCl salt) |
| **Ex.278** | 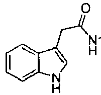 | 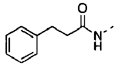 | **Ex.277** | L.1.3 | 3-indoleacetic acid (1.1 equiv.) HATU (1.5 equiv.) HOAt (1.5 equiv.) i-Pr₂NEt (5 equiv.) | FC (CH₂Cl₂/MeOH) | 75% |
| **Ex.279** | 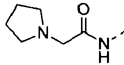 | 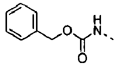 | **Ex.273** (HCl salt) | L.1.3 | pyrrolidine-1-acetic acid (1.2 equiv.) | FC (EtOAc/MeOH) | 67% |
| **Ex.280** | 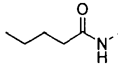 | 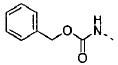 | **Ex.273** (HCl salt) | L.1.1 | valeroyl chloride (2 equiv.) | FC (EtOAc/MeOH) | 71% |
| **Ex.281** | 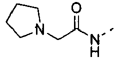 | NH₂ | **Ex.279** | K.1 | H₂, Pd(OH)₂-C | crude product | quant. |
| **Ex.282** | 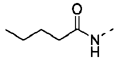 | NH₂ | **Ex.280** | K.1 | H₂, Pd(OH)₂-C | crude product | quant. |
| **Ex.283** | 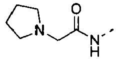 | 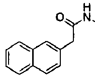 | **Ex.281** | L.1.3 | 2-naphthylacetic acid (1.2 equiv.) | Prep. HPLC, method 3 | 27% |
| **Ex.284** | 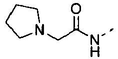 | 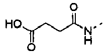 | **Ex.281** | L.1.1 | succinic anhydride (1.5 equiv.) Pyridine (49 equiv.) | Prep. HPLC, method 2 | 51% |
| **Ex.285** | 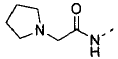 | 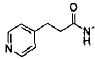 | **Ex.281** | L.1.3 | 3-(pyridine-4-yl)propanoic acid (1.2 equiv.) | Prep. HPLC, method 2 | 12% |
| **Ex.286** | 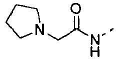 | 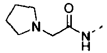 | **Ex.281** | L.1.3 | pyrrolidine-1-acetic acid (1.2 equiv.) | Prep. HPLC, method 2 | 16% |
| **Ex.287** | 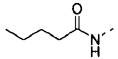 | 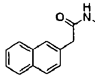 | **Ex.282** | L.1.3 | 2-naphthylacetic acid (1.2 equiv.) | FC (CH₂Cl₂/MeOH/ aq. NH₃) | 79% |
| **Ex.288** | 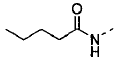 | 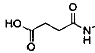 | **Ex.282** | L.1.1 | succinic anhydride (1.5 equiv.) Pyridine (49 equiv.) | Prep. HPLC, method 2 | 57% |
| **Ex.289** | 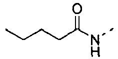 | 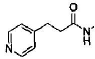 | **Ex.282** | L.1.3 | 3-(pyridine-4-yl)propanoic acid (1.2 equiv.) | Prep. HPLC, method 3 | 17% |
| **Ex.290** | 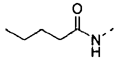 | 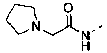 | **Ex.282** | L.1.3 | pyrrolidine-1-acetic acid (1.2 equiv.) | FC (CH₂Cl₂/MeOH/ aq. NH₃) | 65% |
| **Ex.291** | 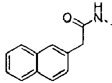 | 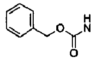 | **Ex.273** (HCl salt) | L.1.3 | 2-naphthylacetic acid (1.2 equiv.) | FC (EtOAc/MeOH)*⁾ | 79% |
| **Ex.292** | 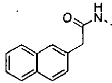 | NH₂ | **Ex.291** | K.1 | H₂, Pd(OH)₂-C | FC (EtOAc/MeOH) | 69% |
| **Ex.293** | 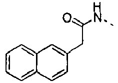 | 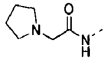 | **Ex.292** | L.1.3 | pyrrolidine-1-acetic acid (1.2 equiv.) | Prep. HPLC, method 3 | 64% |
| **Ex.294** | 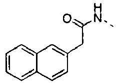 | 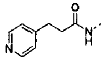 | **Ex.292** | L.1.3 | 3-(pyridine-4-yl)propanoic acid (1.2 equiv.) | Prep. HPLC, method 3 | 70% |
| **Ex.295** | 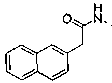 | 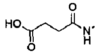 | **Ex.292** | L.1.1 | succinic anhydride (1.5 equiv.) Pyridine (49 equiv.) | Prep. HPLC, method 2 | 73% |
| **Ex.296** | 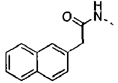 | CH₃(CH₂)₈ CONH | **Ex.292** | L.1.1 | decanoyl chloride (2 equiv.) | Prep. HPLC, method 3 | 40% |

| | | | | | | | |
|---|---|---|---|---|---|---|---|
| *) An analytical sample was further purified by prep. HPLC, method 3 | | | | | | | |

**Table 35b: Examples of Core 18 (Ex.272-Ex.296; continued on the following pages)**

| **No** | **R²** | **R⁵** | **FORMULA** | **MONOISOTOPIC MASS** | **Rt (purity at 220nm)** | **[M+H]⁺ found** | **LC**-**MS**-**Method** |
|---|---|---|---|---|---|---|---|
| **Ex.272-Ex.274:** *cf. experimental description* | | | | | | | |
| **Ex.275** | 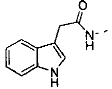 | 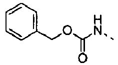 | C36H39N7O7 | 681.3 | 1.53 (97) | 682.5 | Method 2 |
| **Ex.276** | 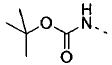 | 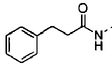 | C32H42N6O7 | 622.3 | 1.57 (95) | 623.4 | Method 2 |
| **Ex.277** | NH₂ | 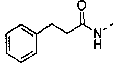 | C27H34N6O5 | 522.3 | 1.10 (98) | 523.4 | Method 2 |
| **Ex**.**278** | 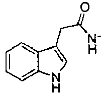 | 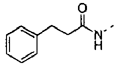 | C37H41N7O6 | 679.3 | 1.50 (98) | 680.5 | Method 2 |
| **Ex.279** | 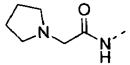 | 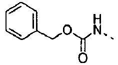 | C32H41N7O7 | 635.3 | 1.32 (98) | 636.3 | Method 2 |
| **Ex.280** | 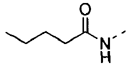 | 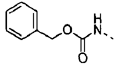 | C31H40N6O7 | 608.3 | 1.54 (98) | 609.3 | Method 2 |
| **Ex.281** | 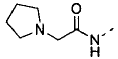 | NH₂ | C24H35N7O5 | 501.3 | 1.32 (98) | 502.3 | Method 5a |
| **Ex.282** | 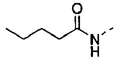 | NH₂ | C23H34N6O5 | 474.3 | 1.24 (94) | 475.1 | Method 4a |
| **Ex.283** | 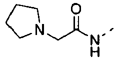 | 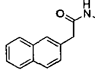 | C36H43N7O6 | 669.3 | 1.42 (95) | 670.3 | Method 4a |
| **Ex.284** | 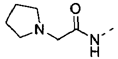 | 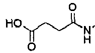 | C28H39N7O8 | 601.3 | 0.99 (100) | 602.2 | Method 5a |
| **Ex.285** | 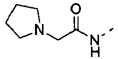 | 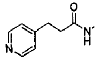 | C32H42N8O6 | 634.3 | 1.47 (98) | 635.2 | Method 5a |
| **Ex.286** | 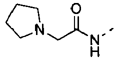 | 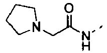 | C30H44N8O6 | 612.3 | 1.55 (99) | 613.3 | Method 5a |
| **Ex.287** | 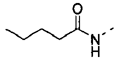 | 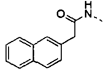 | C35H42N6O6 | 642.3 | 1.77 (98) | 643.3 | Method 4a |
| **Ex.288** | 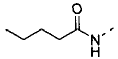 | 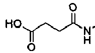 | C27H38N6O8 | 574.3 | 1.32 (100) | 575.2 | Method 4a |
| **Ex.289** | 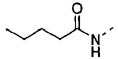 | 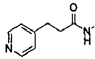 | C31H41N7O6 | 607.3 | 1.25 (95) | 608.3 | Method 4a |
| **Ex.290** | 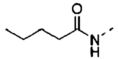 | 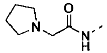 | C29H43N7O6 | 585.3 | 1.30 (95) | 586.4 | Method 4a |
| **Ex.291** | 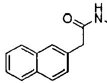 | 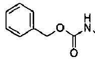 | C38H40N6O7 | 692.3 | 1.91 (97) | 693.3 | Method 4a |
| **Ex.292** | 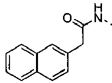 | NH₂ | C30H34N6O5 | 558.3 | 1.55 (99) | 559.3 | Method 4a |
| **Ex.293** | 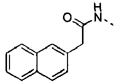 | 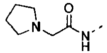 | C36H43N7O6 | 669.3 | 1.52 (96) | 670.3 | Method 4a |
| **Ex.294** | 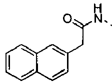 | 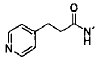 | C38H41N7O6 | 691.3 | 1.46 (97) | 692.3 | Method 4a |
| **Ex.295** | 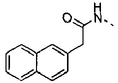 | 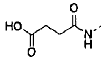 | C34H38N6O8 | 658.3 | 1.58 (99) | 659.2 | Method 4a |
| **Ex.296** | 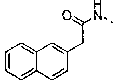 | CH₃(CH₂)₈CONH | C40H52N6O6 | 712.4 | 2.22 (99) | 713.4 | Method 4a |

**Table 35c: Examples of Core 18 (Ex.272-Ex.296; continued on the following pages)**

| **No** | **R²** | **R⁵** | **IUPAC** name |
|---|---|---|---|
| **Ex.272** | 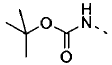 | 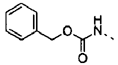 | benzyl *N*-[(4*S*,6*S*,13*S*)-6-[(*tert*-butoxycarbonyl)amino]-11,15-dimethyl-9,12,16-trioxo-2-oxa-8,11,15,19-tetraazatricyclo[15.3.1.0^{4,8}]henicosa-1(21),17,19-trien-13-yl]carbamate |
| **Ex.273** | NH₂ | 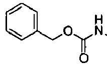 | benzyl *N*-[(4*S*,6*S*,13*S*)-6-amino-11,15-dimethyl-9,12,16-trioxo-2-oxa-8,11,15,19-tetraazatricyclo[15.3.1.0^{4,8}]henicosa-1(21),17,19-trien-13-yl]carbamate |
| **Ex.274** | 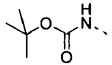 | NH₂ | tert-butyl *N*-[(4*S*,6*S*,13*S*)-13-amino-11,15-dimethyl-9,12,16-trioxo-2-oxa-8,11,15,19-tetraazatricyclo[15.3.1.0^{4,8}]henicosa-1(21),17,19-trien-6-yl]carbamate |
| **Ex.275** | 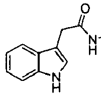 | 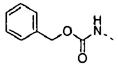 | benzyl *N*-[(4*S*,6*S*,13*S*)-6-{[2-(1*H*-indol-3-yl)acetyl]amino}-11,15-dimethyl-9,12,16-trioxo-2-oxa-8,11,15,19-tetraazatricyclo[15.3.1.0^{4,8}]henicosa-1(21),17,19-trien-13-yl]carbamate |
| **Ex.276** | 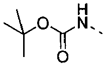 | 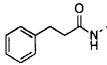 | tert-butyl *N*-[(4*S*,6*S*,13*S*)-11,15-dimethyl-9,12,16-trioxo-13-[(3-phenylpropanoyl)amino]-2-oxa-8,11,15,19-tetraazatricyclo[15.3.1.0^{4,8}]henicosa-1(21),17,19-trien-6-yl]carbamate |
| **Ex.277** | NH₂ | 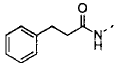 | *N*-[(4*S*,6*S*,13*S*)-6-amino-11,15-dimethyl-9,12,16-trioxo-2-oxa-8,11,15,19-tetraazatricyclo[15.3.1.0^{4,8}]henicosa-1(21),17,19-trien-13-yl]-3-phenylpropanamide |
| **Ex.278** | 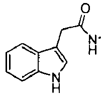 | 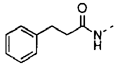 | *N*-[(4*S*,6*S*,13*S*)-6-{[2-(1*H*-indol-3-yl)acetyl]amino}-11,15-dimethyl-9,12,16-trioxo-2-oxa-8,11,15,19-tetraazatricyclo[15.3.1.0^{4,8}]henicosa-1(21),17,19-trien-13-yl]-3-phenylpropanamide |
| **Ex.279** | 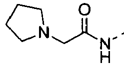 | 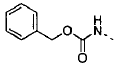 | benzyl *N*-[(4*S*,6*S*,13*S*)-11,15-dimethyl-9,12,16-trioxo-6-{[2-(1-pyrrolidinyl)acetyl]amino}-2-oxa-8,11,15,19-tetraazatricyclo[15.3.1.0^{4,8}]henicosa-1(21),17,19-trien-13-yl]carbamate |
| **Ex.280** | 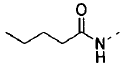 | 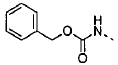 | benzyl *N*-[(4*S*,6*S*,13*S*)-11,15-dimethyl-9,12,16-trioxo-6-(pentanoylamino)-2-oxa-8,11,15,19-tetraazatricyclo[15.3.1.0^{4,8}]henicosa-1(21),17,19-trien-13-yl]carbamate |
| **Ex.281** | 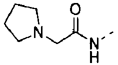 | NH₂ | *N*-[(4*S*,6*S*,13*S*)-13-amino-11,15-dimethyl-9,12,16-trioxo-2-oxa-8,11,15,19-tetraazatricyclo[15.3.1.0^{4,8}]henicosa-1(21),17,19-trien-6-yl]-2-(1-pyrrolidinyl)acetamide |
| **Ex.282** | 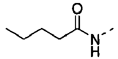 | NH₂ | *N*-[(4*S*,6*S*,13*S*)-13-amino-11,15-dimethyl-9,12,16-trioxo-2-oxa-8,11,15,19-tetraazatricyclo[15.3.1.0^{4,8}]henicosa-1(21),17,19-trien-6-yl]pentanamide |
| **Ex.283** | 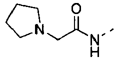 | 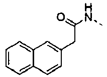 | *N*-[(4*S*,6*S*,13*S*)-11,15-dimethyl-9,12,16-trioxo-6-{[2-(1-pyrrolidinyl)acetyl]amino}-2-oxa-8,11,15,19-tetraazatricyclo[15.3.1.0^{4,8}]henicosa-1(21),17,19-trien-13-yl]-2-(2-naphthyl)acetamide |
| **Ex.284** | 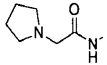 | 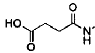 | 4-{[(4*S*,6*S*,13*S*)-11,15-dimethyl-9,12,16-trioxo-6-{[2-(1-pyrrolidinyl)acetyl]amino}-2-oxa-8,11,15,19-tetraazatricyclo[15.3.1.0^{4,8}]henicosa-1(21),17,19-trien-13-yl]amino}-4-oxobutanoic acid |
| **Ex.285** | 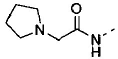 | 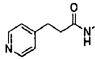 | *N*-[(4*S*,6*S*,13*S*)-11,15-dimethyl-9,12,16-trioxo-6-{[2-(1-pyrrolidinyl)acetyl]amino}-2-oxa-8,11,15,19-tetraazatricyclo[15.3.1.0^{4,8}]henicosa-1(21),17,19-trien-13-yl]-3-(4-pyridinyl)propanamide |
| **Ex.286** | 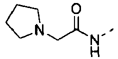 | 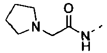 | *N*-[(4*S*,6*S*,13*S*)-11,15-dimethyl-9,12,16-trioxo-13-{[2-(1-pyrrolidinyl)acetyl]amino}-2-oxa-8,11,15,19-tetraazatricyclo[15.3.1.0^{4,8}]henicosa-1(21),17,19-trien-6-yl]-2-(1-pyrrolidinyl)acetamide |
| **Ex.287** | 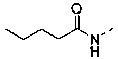 | 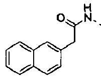 | *N*-[(4*S*,6*S*,13*S*)-11,15-dimethyl-13-{[2-(2-naphthyl)acetyl]amino}-9,12,16-trioxo-2-oxa-8,11,15,19-tetraazatricyclo[15.3.1.0^{4,8}]henicosa-1(21),17,19-trien-6-yl]pentanamide |
| **Ex.288** | 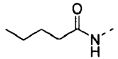 | 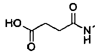 | 4-{[(4*S*,6*S*,13*S*)-11,15-dimethyl-9,12,16-trioxo-6-(pentanoylamino)-2-oxa-8,11,15,19-tetraazatricyclo[15.3.1.0^{4,8}]henicosa-1(21),17,19-trien-13-yl]amino}-4-oxobutanoic acid |
| **Ex.289** | 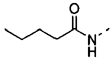 | 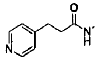 | *N*-[(4*S*,6*S*,13*S*)-11,15-dimethyl-9,12,16-trioxo-13-{[3-(4-pyridinyl)propanoyl]amino}-2-oxa-8,11,15,19-tetraazatricyclo[15.3.1.0^{4,8}]henicosa-1(21),17,19-trien-6-yl]pentanamide |
| **Ex.290** | 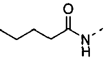 | 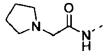 | *N*-[(4*S*,6*S*,13*S*)-11,15-dimethyl-9,12,16-trioxo-13-{[2-(1-pyrrolidinyl)acetyl]amino}-2-oxa-8,11,15,19-tetraazatricyclo[15.3.1.0^{4,8}]henicosa-1(21),17,19-trien-6-yl]pentanamide |
| **Ex.291** | 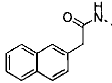 | 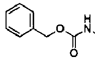 | benzyl *N*-[(4*S*,6*S*,13*S*)-11,15-dimethyl-6-{[2-(2-naphthyl)acetyl]amino}-9,12,16-trioxo-2-oxa-8,11,15,19-tetraazatricyclo[15.3.1.0^{4,8}]henicosa-1(21),17,19-trien-13-yl]carbamate |
| **Ex.292** | 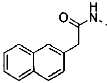 | NH₂ | *N*-[(4*S*,6*S*,13*S*)-13-amino-11,15-dimethyl-9,12,16-trioxo-2-oxa-8,11,15,19-tetraazatricyclo[15.3.1.0^{4,8}]henicosa-1(21),17,19-trien-6-yl]-2-(2-naphthyl)acetamide |
| **Ex.293** | 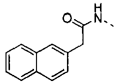 | 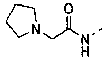 | *N*-[(4*S*,6*S*,13*S*)-11,15-dimethyl-9,12,16-trioxo-13-{[2-(1-pyrrolidinyl)acetyl]amino}-2-oxa-8,11,15,19-tetraazatricyclo[15.3.1.0^{4,8}]henicosa-1(21),17,19-trien-6-yl]-2-(2-naphthyl)acetamide |
| **Ex.294** | 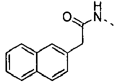 | 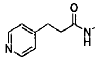 | *N*-[(4*S*,6*S*,13*S*)-11,15-dimethyl-6-{[2-(2-naphthyl)acetyl]amino}-9,12,16-trioxo-2-oxa-8,11,15,19-tetraazatricyclo[15.3.1.0^{4,8}]henicosa-1(21),17,19-trien-13-yl]-3-(4-pyridinyl)propanamide |
| **Ex.295** | 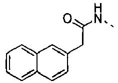 | 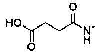 | 4-{[(4*S*,6*S*,13*S*)-11,15-dimethyl-6-{[2-(2-naphthyl)acetyl]amino}-9,12,16-trioxo-2-oxa-8,11,15,19-tetraazatricyclo[15.3.1.0^{4,8}]henicosa-1(21),17,19-trien-13-yl]amino}-4-oxobutanoic acid |
| **Ex.296** | 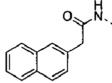 | CH₃(CH₂)₈CONH | *N*-[(4*S*,6*S*,13*S*)-11,15-dimethyl-6-{[2-(2-naphthyl)acetyl]amino}-9,12,16-trioxo-2-oxa-8,11,15,19-tetraazatricyclo[15.3.1.0^{4,8}]henicosa-1(21),17,19-trien-13-yl]decanamide |

**Table 36a: Examples of Core 19 (Ex.297-Ex.310; continued on the following pages)**

| **No** | **R²** | **R⁵⁴** | **Starting material** | **General Procedure** | **Reagent** | **Purification Method** | **Yield (isolated salt)** |
|---|---|---|---|---|---|---|---|
| **Ex.297-Ex.298:** *cf. experimental description* | | | | | | | |
| **Ex.299** | 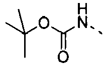 | 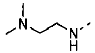 | **Ex.298** | L.2 | N,N-dimethylethylenediamine | Flash Chromatography | 80% |
| **Ex.300** | NH₂ | 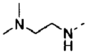 | **Ex.299** | J | HCl-dioxane | No purification | quant. (HCl salt) |
| **Ex.301** | 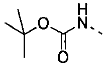 | 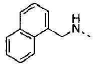 | **Ex.298** | L.2 | 1-naphthylmethylamine | FC (CH₂Cl₂/MeOH) | 75% |
| **Ex.302** | NH₂ | 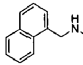 | **Ex.301** | J | HCl-dioxane | Crude product | quant. (HCl salt) |
| **Ex.303** | 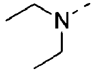 | 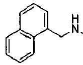 | **Ex.302** | M.1 | acetaldehyde | Prep. HPLC, method 2 | 45% |
| **Ex.304** | 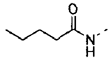 | 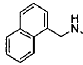 | **Ex.302** | L.1.1 | valeroyl chloride (5 equiv.) | Prep. HPLC, method 2 | 22% |
| **Ex.305** | 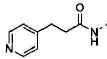 | 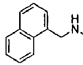 | **Ex.302** | L.1.2 | 3-(pyridine-4-yl)propanoic acid (3.7 equiv.) i-Pr₂NEt (4 equiv.) bicarbonate resin (4equiv.) | FC (CH₂Cl₂/MeOH/ aq. NH₃) then Prep. HPLC, method 3 | 55% |
| **Ex.306** | 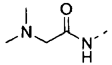 | 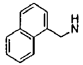 | **Ex.302** | L.1.2 | N,N-dimethyl glycine (6.2 equiv.) carbodiimide resin (2.5 equiv.) i-Pr₂NEt (5 equiv.) bicarbonate resin (5 equiv.) | FC (CH₂Cl₂/MeOH) | 43% |
| **Ex**.**307** | 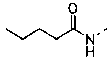 | 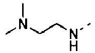 | **Ex.300** | L.1.1 | valeroyl chloride (2 equiv.) | Prep. HPLC, method 2 | 36% |
| **Ex.308** | 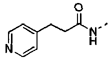 | 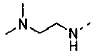 | **Ex.300** | L.1.2 | 3-(pyridine-4-yl)propanoic acid (1.2 equiv.) i-Pr₂NEt (4 equiv.), bicarbonate resin (4equiv.) | FC (CH₂Cl₂/MeOH) | 77% |
| **Ex.309** | 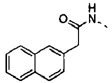 | 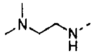 | **Ex.300** | L.1.2^{*)} | 2-naphthylacetic acid i-Pr₂NEt (4 equiv.) | Prep. HPLC, method 2 | 58% |
| **Ex.310** | 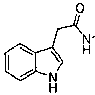 | 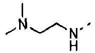 | **Ex.300** | L.1.2 | 3-indoleacetic acid (3.7 equiv.) i-Pr₂NEt (4 equiv.) DMF (0.2 mL) bicarbonate resin (4equiv.) | Prep. HPLC, method 2 | 28% |

| | | | | | | | |
|---|---|---|---|---|---|---|---|
| *) The treatment with (polystyrylmethyl)trimethylammonium bicarbonate was replaced by an aqueous workup (CHCl₃, sat. aq. NaHCO₃ soln) | | | | | | | |

**Table 36b: Examples of Core 19 (Ex.297-Ex.310; continued on the following pages)**

| **No** | **R²** | **R⁵⁴** | **FORMULA** | **MONOISOTOPIC MASS** | **Rt (purity at 220nm)** | **[M+H]⁺ found** | **LC-MS-Method** |
|---|---|---|---|---|---|---|---|
| **Ex.297-Ex.298:** *cf. experimental description* | | | | | | | |
| **Ex.299** | 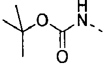 | 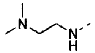 | C33H47N7O7 | 653.4 | 1.62 (97) | 654.4 | Method 4a |
| **Ex.300** | NH₂ | 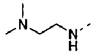 | C28H39N7O5 | 553.3 | 1.11 (64), 1.08 (35) | 554.3 | Method 4a |
| **Ex.301** | 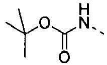 | 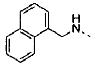 | C40H46N6O7 | 722.3 | 2.19 (89) | 723.4 | Method 4a |
| **Ex.302** | NH₂ | 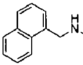 | C35H38N6O5 | 622.3 | 1.70 (79) | 623.3 | Method 4a |
| **Ex.303** | 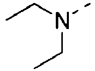 | 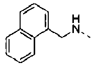 | C39H46N6O5 | 678.4 | 1.75 (100) | 679.4 | Method 4a |
| **Ex.304** | 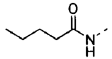 | 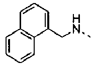 | C40H46N6O6 | 706.4 | 2.09 (100) | 707.4 | Method 4a |
| **Ex.305** | 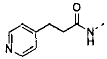 | 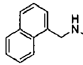 | C43H45N7O6 | 755.3 | 1.67 (57), 1.70 (38) | 756.4 | Method 4a |
| **Ex.306** | 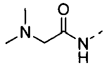 | 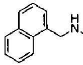 | C39H45N7O6 | 707.3 | 1.71 (86) | 708.3 | Method 4a |
| **Ex.307** | 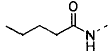 | 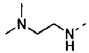 | C33H47N7O6 | 637.4 | 1.50 (638.3) | 638.3 | Method 4a |
| **Ex.308** | 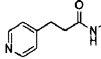 | 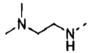 | C36H46N8O6 | 686.4 | 1.57 (92) | 687.4 | Method 5a |
| **Ex.309** | 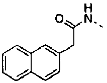 | 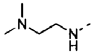 | C40H47N7O6 | 721.4 | 1.68 (98) | 722.4 | Method 4a |
| **Ex.310** | 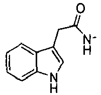 | 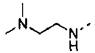 | C38H46N8O6 | 710.4 | 1.52 (97) | 711.4 | Method 4a |

**Table 36c: Examples of Core 19 (Ex.297-Ex.310; continued on the following pages)**

| **No** | **R²** | **R⁵⁴** | **IUPAC name** |
|---|---|---|---|
| **Ex.297** | 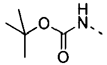 | OCH₂Ph | benzyl (4S,6S,15S)-6-[(tert-butoxycarbonyl)amino]-11,16-dimethyl-9,12,17-trioxo-2-oxa-8,11,16,26-tetraazatetracyclo[16.6.2.0^{4,8}.0^{21,25}]hexacosa-1(24),18,20,22,25-pentaene-15-carboxylate |
| **Ex.298** | 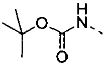 | OH | (4*S*,6*S*,15*S*)-6-[(*tert*-butoxycarbonyl)amino]-11,16-dimethyl-9,12,17-trioxo-2-oxa-8,11,16,26-tetraazatetracyclo[16.6.2.0⁴,⁸.0^{21,25}]hexacosa-1(24),18,20,22,25-pentaene-15-carboxylic acid |
| **Ex.299** | 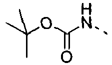 | 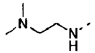 | tert-butyl *N*-[(4*S*,6*S*,15*S*)-15-({[2-(dimethylamino)ethyl]amino}carbonyl)-11,16-dimethyl-9,12,17-trioxo-2-oxa-8,11,16,26-tetraazatetracyclo[16.6.2.0^{4,8}.0^{21,25}]hexacosa-1(24),18,20,22,25-pentaen-6-yl]carbamate |
| **Ex.300** | NH₂ | 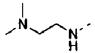 | (4*S*,6*S*,15*S*)-6-amino-*N*-[2-(dimethylamino)ethyl]-11,16-dimethyl-9,12,17-trioxo-2-oxa-8,11,16,26-tetraazatetracyclo[16.6.2.0^{4,8}.0^{21,25}]hexacosa-1(24),18,20,22,25-pentaene-15-carboxamide |
| **Ex.301** | 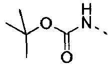 | 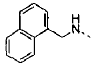 | *tert*-butyl *N*-[(4*S*,6*S*,15*S*)-11,16-dimethyl-15-{[(1-naphthylmethyl)amino]carbonyl}-9,12,17-trioxo-2-oxa-8,11,16,26-tetraazatetracyclo[16.6.2.0^{4,8}.0^{21,25}]hexacosa-1(25),18(26),19,21,23-pentaen-6-yl]carbamate |
| **Ex.302** | NH₂ | 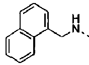 | (4*S*,6*S*,15*S*)-6-amino-11,16-dimethyl-*N*-(1-naphthylmethyl)-9,12,17-trioxo-2-oxa-8,11,16,26-tetraazatetracyclo[16.6.2.0^{4,8}.0^{21,25}]hexacosa-1(25),18(26),19,21,23-pentaene-15-carboxamide |
| **Ex.303** | 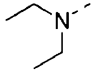 | 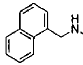 | (4S,6S,15S)-6-(diethylamino)-11,16-dimethyl-N-(1-naphthylmethyl)-9,12,17-trioxo-2-oxa-8,11,16,26-tetraazatetracyclo[16.6.2.0^{4,8}.0^{21,25}]hexacosa-1(25),18(26),19,21,23-pentaene-15-carboxamide |
| **Ex.304** | 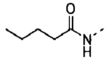 | 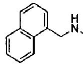 | (4*S*,6*S*,15*S*)-11,16-dimethyl-*N*-(1-naphthylmethyl)-9,12,17-trioxo-6-(pentanoylamino)-2-oxa-8,11,16,26-tetraazatetracyclo[16.6.2.0^{4,8}.0^{21,25}]hexacosa-1(25),18(26),19,21,23-pentaene-15-carboxamide |
| **Ex.305** | 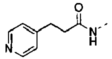 | 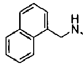 | (4*S*,6*S*,15*S*)-11,16-dimethyl-N-(1-naphthylmethyl)-9,12,17-trioxo-6-{[3-(4-pyridinyl)propanoyl]amino}-2-oxa-8,11,16,26-tetraazatetracyclo[16.6.2.0^{4,8}.0^{21,25}]hexacosa-1(25),18(26),19,21,23-pentaene-15-carboxamide |
| **Ex**.**306** | 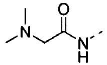 | 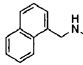 | (4*S*,6*S*,15*S*)-6-{(2-(dimethylamino)acetyl]amino}-11,16-dimethyl-*N*-(1-naphthylmethyl)-9,12,17-trioxo-2-oxa-8,11,16,26-tetraazatetracyclo[16.6.2.0^{4,8}.0^{21,25}]hexacosa-1(25),18(26),19,21,23-pentaene-15-carboxamide |
| **Ex.307** | 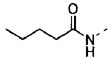 | 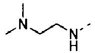 | (4*S*,6*S*,15*S*)-*N*-[2-(dimethylamino)ethyl]-11,16-dimethyl-9,12,17-trioxo-6-(pentanoylamino)-2-oxa-8,11,16,26-tetraazatetracyclo[16.6.2.0^{4,8}.0^{21,25}]hexacosa-1(25),18(26),19,21,23-pentaene-15-carboxamide |
| **Ex.308** | 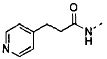 | 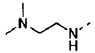 | (4*S*,6*S*,15*S*)-*N*-[2-(dimethylamino)ethyl]-11,16-dimethyl-9,12,17-trioxo-6-{[3-(4-pyridinyl)propanoyl]amino}-2-oxa-8,11,16,26-tetraazatetracyclo[16.6.2.0^{4,8}.0^{21,25}]hexacosa-1(25),18(26),19,21,23-pentaene-15-carboxamide |
| **Ex.309** | 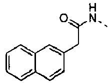 | 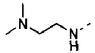 | (4*S*,6*S*,15*S*)-*N*-[2-(dimethylamino)ethyl]-11,16-dimethyl-6-{[2-(2-naphthyl)acetyl]amino}-9,12,17-trioxo-2-oxa-8,11,16,26-tetraazatetracyclo[16.6.2.0^{4,8}.0^{21,25}]hexacosa-1(25),18(26),19,21,23-pentaene-15-carboxamide |
| **Ex.310** | 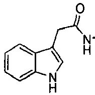 | 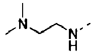 | (4*S*,6*S*,15*S*)-*N*-[2-(dimethylamino)ethyl]-6-{[2-(1*H*-indol-3-yl)acetyl]amino}-11,16-dimethyl-9,12,17-trioxo-2-oxa-8,11,16,26-tetraazatetracyclo[16.6.2.0^{4,8}.0^{21,25}]hexacosa-1(25),18(26),19,21,23-pentaene-15-carboxamide |

**Table 37.1.a: Example of Core 20 (Ex.311)**

| **No** | **R²** | **R⁵** | **R³⁸** | **Starting material** | **General Procedure** | **Reagent** | **Purification Method** | **Yield (isolated salt)** |
|---|---|---|---|---|---|---|---|---|
| **Ex.311:** *cf. experimental description* | | | | | | | | |

**Table 37.1.b: Example of Core 20 (Ex.311)**

| **No** | **R²** | **R⁵** | **R³⁸** | **FORMULA** | **MONOISOTOPIC MASS** | **Rt (purity at 220nm)** | **[M+H]⁺ found** | **LC-MS-Method** |
|---|---|---|---|---|---|---|---|---|
| **Ex.311:** *cf. experimental description* | | | | | | | | |

**Table 37.1.c: Example of Core 20 (Ex.311)**

| **No** | **R²** | **R⁵** | **R³⁸** | **IUPAC name** |
|---|---|---|---|---|
| **Ex.311** | 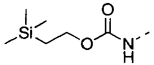 | 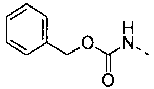 | 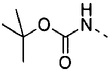 | benzyl *N*-[(4*S*,6*S*,13*S*,17*S*)-17-[(*tert*-butoxycarbonyl)amino]-11,15-dimethyl-9,12,16-trioxo-6-({[2-(trimethylsilyl)ethoxy]carbonyl}amino)-2-oxa-8,11,15-triazatetracyclo[15.6.2.0^{4,8}.0^{20,24}]pentacosa-1(24),20,22-trien-13-yl]carbamate |

**Table 37.2.a: Examples of Core 21 (Ex.312-Ex.341; continued on the following pages)**

| **No** | **X** | **R²** | **R⁵⁴** | **Starting material** | **General Procedure** | **Reagent** | **Purification Method** | **Yield (isolated salt)** |
|---|---|---|---|---|---|---|---|---|
| **Ex.312-Ex.315:** *cf. experimental description* | | | | | | | | |
| **Ex.316** | S | 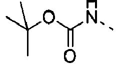 | 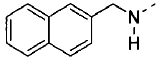 | **Ex.314** | L.2 | 2-naphthylmethyl amine (2 equiv.) | FC (CH₂Cl₂/MeOH) | 86% |
| **Ex.317** | S | 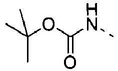 | 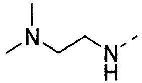 | **Ex.314** | L.2 | N,N-dimethylethyl amine (2 equiv.) | FC (CH₂Cl₂/MeOH) | 72% |
| **Ex.318** | S | NH₂ | 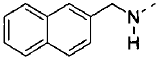 | **Ex.316** | J | HCl (4M dioxane) | crude product | quant. (HCl salt) |
| **Ex.319** | S | NH₂ | 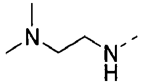 | **Ex.317** | J | HCl (4M dioxane) | crude product | quant. (HCl salt) |
| **Ex.320** | S | 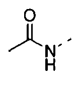 | 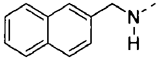 | **Ex.318** | L.1.1 | acetic anhydride (5 equiv.) | prep.,HPLC, method 1 | 43% |
| **Ex.321** | S | 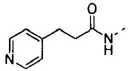 | 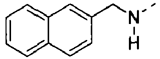 | **Ex.318** | L.1.3 | HATU (1.5 equiv.), HOAt (1.5 equiv.), DIPEA (5 equiv), 3-pyridyl-propanoic acide (1.1 equiv.) | prep. HPLC, method 1 | 39% (TFA salt) |
| **Ex.322** | S | 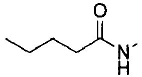 | 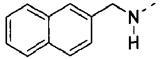 | **Ex.318** | L.1.1 | pentanoic acid chloride (2 equiv.) | prep. HPLC, method 1 | 49% |
| **Ex.323** | S | 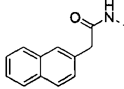 | 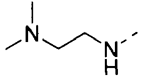 | **Ex.319** | L.1.3 | 2-naphthylacetic acid (2 equiv.) | prep. HPLC, method 2 | 40% |
| **Ex.324** | S | 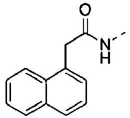 | 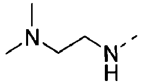 | **Ex.319** | L.1.3 | 1-naphthylacetic acid (2 equiv.) | prep. HPLC, method 2 | 43% |
| **Ex.325** | S | 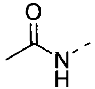 | 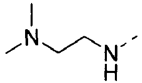 | **Ex.319** | L.1.1 | acetic anhydride (5 equiv.) | prep. HPLC, method 2 | 73% |
| **Ex.326** | S | 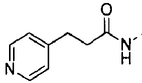 | 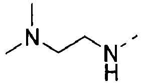 | **Ex.319** | L.1.3 | 3-pyridinyl-propanoic acid (2 equiv.) | prep. HPLC, method 2 | 43% |
| **Ex.327** | S | 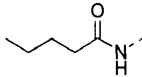 | 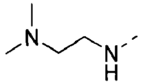 | **Ex.319** | L.1.1 | pentanoyl chloride (4 equiv.) | prep. HPLC, method 2 | 55% |
| **Ex.328** | SO₂ | 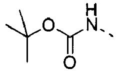 | 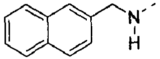 | **Ex.315** | L.2 | 2-naphthylmethylamine (2 equiv.) | prep. HPLC, method 2 | 75% |
| **Ex.329** | SO₂ | 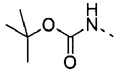 | 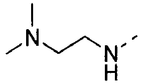 | **Ex.315** | L.2 | N,N-dimethylene-diamine (2 equiv.) | FC (CH2Cl2/MeOH/ aq.NH3) | 97% |
| **Ex.330** | SO₂ | NH₂ | 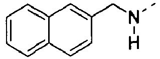 | **Ex.328** | J | HCl(4M dioxane) | crude product | 88% (HCl salt) |
| **Ex.331** | SO₂ | NH₂ | 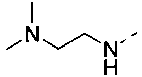 | **Ex.329** | J | HCl(4M dioxane) | crude product | 75% (HCl salt) |
| **Ex.332** | SO₂ | 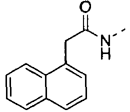 | 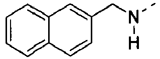 | **Ex.330** | L.1.2 | 1-naphthylacetic acid (1.2 equiv.) | prep. HPLC, method 1 then method 3 | 60% |
| **Ex.333** | SO₂ | 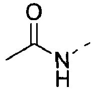 | 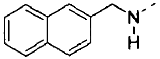 | **Ex.330** | L.1.1 | acetic anhydride (5 equiv). | prep. HPLC, method 2 then method 3 | 65% |
| **Ex.334** | SO₂ | 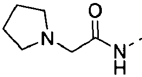 | 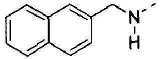 | **Ex.330** | L.1.2 | 1-pyrrolidine-acetic acid (1.8 equiv.) HOBt·H₂O (1.8 equiv.) | prep. HPLC, method 2 | 60% |
| **Ex.335** | SO₂ | 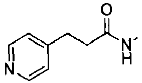 | 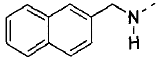 | **Ex.330** | L.1.2 | 4-pyridinyl-propanoic acid (2.5 equiv.) HOBt.H₂O (1.8 equiv.) | prep. HPLC, method 2 | 67% |
| **Ex.336** | SO₂ | 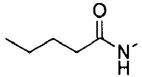 | 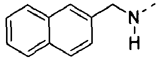 | **Ex.330** | L.1.1 | pentanoyl chloride (6 equiv.) pyridine (10 equiv.), Et₃N (5 equiv.) | prep. HPLC, method 1 then method 3 | 49% |
| **Ex.337** | SO₂ | 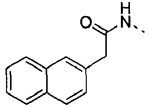 | 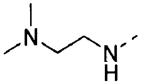 | **Ex.331** | L.1.3 | 2-naphthylacetic acid (2 equiv.) | prep. HPLC, method 2 | 41% |
| **Ex.338** | SO₂ | 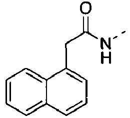 | 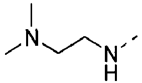 | **Ex.331** | L.1.3 | 1-naphthylacetic acid (2 equiv.) | prep. HPLC, method 2 | 45% |
| **Ex.339** | SO₂ | 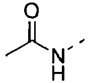 | 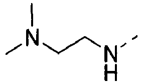 | **Ex.331** | L.1.1 | acetic anhydride (5 equiv.) | prep. HPLC, method 2 | 30% |
| **Ex.340** | SO₂ | 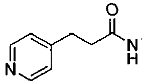 | 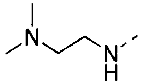 | **Ex.331** | L.1.3 | 3-pyridinyl-propanoic acid (2 equiv.) | prep. HPLC, method 2 | 23% |
| **Ex.341** | SO₂ | 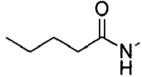 | 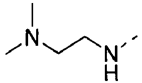 | **Ex.331** | L.1.1 | pentanoyl chloride (4 equiv.) | prep. HPLC, method 2 | 48% |

**Table 37.2.b: Examples of Core 21 (Ex.312-Ex.341; continued on the following pages)**

| **No** | **X** | **R²** | **R⁵⁴** | **FORMULA** | **MONOISOTOPIC MASS** | **Rt (purity at 220nm)** | **[M+H]⁺ found** | **LC-MS-Method** |
|---|---|---|---|---|---|---|---|---|
| **Ex.312-Ex.315:** *cf. experimental description* | | | | | | | | |
| **Ex.316** | S | 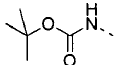 | 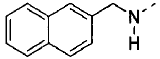 | C37H45N5O6S | 687.3 | 2.20 (95) | 688.2 | Method 4b |
| **Ex.317** | S | 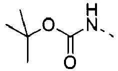 | 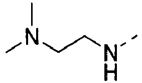 | C30H46N6O6S | 618.3 | 1.57 (95) | 619.2 | Method 4b |
| **Ex.318** | S | NH₂ | 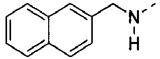 | C32H37N5O4S | 587.3 | 1.66 (91) | 588.0 | Method 4b |
| **Ex.319** | S | NH₂ | 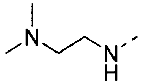 | C25H38N6O4S | 518.3 | 1.44 (87) | 519.2 | Method 5b |
| **Ex.320** | S | 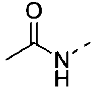 | 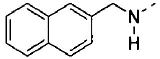 | C34H39N5O5S | 629.3 | 1.88 (92) | 630.2 | Method 4b |
| **Ex.321** | S | 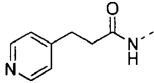 | 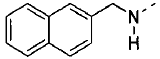 | C40H44N6O5S | 720.3 | 1.56 (93) | 721.3 | Method 2 |
| **Ex.322** | S | 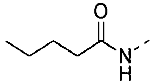 | 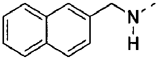 | C37H45N5O5S | 671.3 | 2.08 (91) | 672.3 | Method 4b |
| **Ex.323** | S | 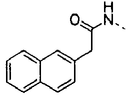 | 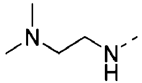 | C37H46N6O5S | 686.3 | 1.67 (96) | 687.2 | Method 4b |
| **Ex.324** | S | 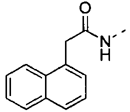 | 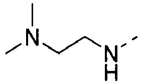 | C37H46N6O5S | 686.3 | 1.66 (96) | 687.2 | Method 4b |
| **Ex.325** | S | 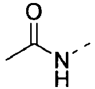 | 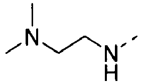 | C27H40N6O5S | 560.3 | 1.19 (98) | 561.2 | Method 4b |
| **Ex.326** | S | 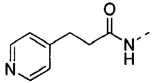 | 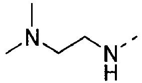 | C33H45N7O5S | 651.3 | 1.12 (89) | 652.2 | Method 4b |
| **Ex.327** | S | 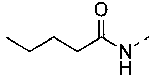 | 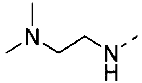 | C30H46N6O5S | 602.3 | 1.46 (97) | 603.0 | Method 4b |
| **Ex**.**328** | SO₂ | 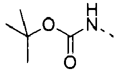 | 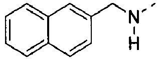 | C37H45N5O8S | 719.3 | 2.08 (98) | 720.2 | Method 4b |
| **Ex.329** | SO₂ | 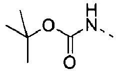 | 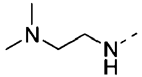 | C30H46N6O8S | 650.3 | 1.45 (94) | 651.2 | Method 4b |
| **Ex.330** | SO₂ | NH₂ | 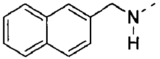 | C32H37N5O6S | 619.3 | 1.58 (94) | 620.0 | Method 4b |
| **Ex.331** | SO₂ | NH₂ | 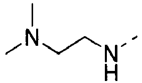 | C25H38N6O6S | 550.3 | 1.28 (80) | 551.2 | Method 5a |
| **Ex.332** | SO₂ | 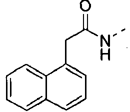 | 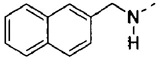 | C44H45N5O7S | 787.3 | 2.14 (96) | 788.2 | Method 4b |
| **Ex.333** | SO₂ | 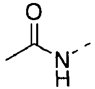 | 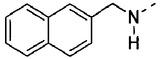 | C34H39N5O7S | 661.3 | 1.78 (95) | 661.8 | Method 4b |
| **Ex.334** | SO₂ | 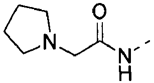 | 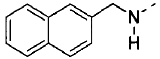 | C38H46N6O7S | 730.3 | 1.64 (95) | 731.2 | Method 4b |
| **Ex.335** | SO₂ | 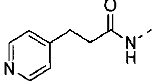 | 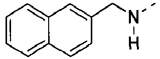 | C40H44N6O7S | 752.3 | 1.63 (98) | 753.2 | Method 4b |
| **Ex.336** | SO₂ | 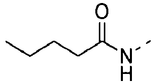 | 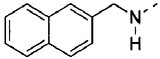 | C37H45N5O7S | 703.3 | 1.98 (96) | 704.2 | Method 4b |
| **Ex.337** | SO₂ | 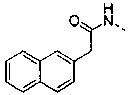 | 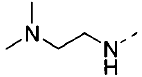 | C37H46N6O7S | 718.3 | 1.61 (96) | 719.3 | Method 4a |
| **Ex.338** | SO₂ | 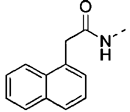 | 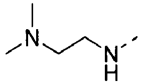 | C37H46N6O7S | 718.3 | 1.59 (96) | 719.3 | Method 4a |
| **Ex.339** | SO₂ | 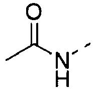 | 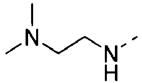 | C27H40N6O7S | 592.3 | 1.33 (95) | 593.3 | Method 5a |
| **Ex.340** | SO₂ | 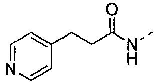 | 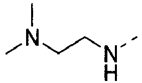 | C33H45N7O7S | 683.3 | 1.49 (100) | 684.1 | Method 5a |
| **Ex.341** | SO₂ | 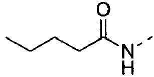 | 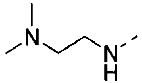 | C30H46N6O7S | 634.3 | 1.61 (91) | 635.3 | Method 5a |

**Table 37.2.c: Examples Core 21 (Ex.312-Ex.341; continued on the following pages)**

| **No** | **R²** | **R⁵⁴** | **X** | **IUPAC name** |
|---|---|---|---|---|
| **Ex.312** | 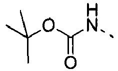 | OCH₂Ph | S | benzyl (4*S*,6*R*,15*S*)-6-[(*tert*-butoxycarbonyl)amino]-11,16-dimethyl-9,12,17-trioxo-2-thia-8,11,16-triazatricyclo[16.3.1.0^{4,8}]docosa-1(22),18,20-triene-15-carboxylate |
| **Ex.313** | 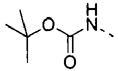 | OCH₂Ph | SO₂ | benzyl (4*S*,6*R*,15*S*)-6-[(tert-butoxycarbonyl)amino]-11,16-dimethyl-2,2,9,12,17-pentaoxo-2λ⁶-thia-8,11,16-triazatricyclo[16.3.1.0^{4,8}]docosa-1(22),18,20-triene-15-carboxylate |
| **Ex.314** | 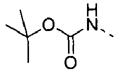 | OH | S | (9*S*,6*R*,15*S*)-6-[(*tert*-butoxycarbonyl)amino]-11,16-dimethyl-9,12,17-trioxo-2-thia-8,11,16-triazatricyclo[16.3.1.0^{4,8}]docosa-1(22),18,20-triene-15-carboxylic acid |
| **Ex.315** | 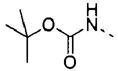 | OH | SO₂ | (4*S*,6*R*,15*S*)-6-[(*tert*-butoxycarbonyl)amino]-11,16-dimethyl-2,2,9,12,17-pentaoxo-2λ⁶-thia-8,11,16-triazatricyclo[16.3.1.0⁴'⁸]docosa-1(22),18,20-triene-15-carboxylic acid |
| **Ex.316** | 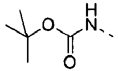 | 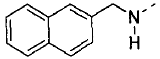 | S | tert-butyl *N*-[(4*S*,6*R*,15*S*)-11,16-dimethyl-15-{[(2-naphthylmethyl)amino]carbonyl}-9,12,17-trioxo-2-thia-8,11,16-triazatricyclo[16.3.1.0^{4,8}]docosa-1(22),18,20-trien-6-yl]carbamate |
| **Ex.317** | 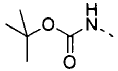 | 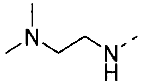 | S | tert-butyl *N*-[(4*S*,6*R*,15*S*)-15-({[2-(dimethylamino)ethyl]amino}carbonyl)-11,16-dimethyl-9,12,17-trioxo-2-thia-8,11,16-triazatricyclo[16.3.1.0^{4,8}]docosa-1(22),18,20-trien-6-yl]carbamate |
| **Ex.318** | NH₂ | 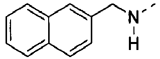 | S | (4*S*,6*R*,15*S*)-6-amino-11,16-dimethyl-*N*-(2-naphthylmethyl)-9,12,17-trioxo-2-thia-8,11,16-triazatric yclo[16.3.1.0^{4,8}]docosa-1(22),18,20-triene-15-carboxamide |
| **Ex.319** | NH₂ | 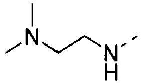 | S | (4*S*,6*R*,15*S*)-6-amino-N-[2-(dimethylamino)ethyl]-11,16-dimethyl-9,12,17-trioxo-2-thia-8,11,16-triazatricyclo[16.3.1.0^{4,8}]docosa-1(22),18,20-triene-15-carboxamide |
| **Ex.320** | 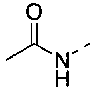 | 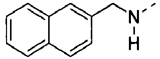 | S | (4*S*,6*R*,15*S*)-6-(acetylamino)-11,16-dimethyl-N-(2-naphthylmethyl)-9,12,17-trioxo-2-thia-8,11,16-triazatricyclo[16.3.1.0^{4,8}]docosa-1(22),18,20-triene-15-carboxamide |
| **Ex.321** | 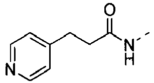 | 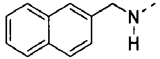 | S | (4*S*,6*R*,15*S*)-11,16-dimethyl-*N*-(2-naphthylmethyl)-9,12,17-trioxo-6-{[3-(4-pyridinyl)propanoyl]amino}-2-thia-8,11,16-triazatricyclo[16.3.1.0^{4,8}]docosa-1(22),18,20-triene-15-carboxamide |
| **Ex.322** | 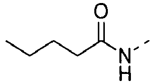 | 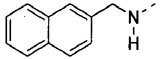 | S | (4*S*,6*R*,15*S*)-11,16-dimethyl-*N*-(2-naphthylmethyl)-9,12,17-trioxo-6-(pentanoylamino)-2-thia-8,11,16-triazatricyclo[16.3.1.0^{4,8}]docosa-1(22),18,20-triene-15-carboxamide |
| **Ex.323** | 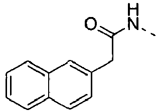 | 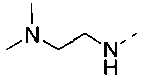 | S | (4*S,*6*R,*15*S*)*-N-*[2-(dimethylamino)ethyl]-11,16-dimethyl-6-{[2-(2-naphthyl)acetyl]amino}-9,12,17-trioxo-2-thia-8,11,16-triazatricyclo[16.3.1.0^{4,8}]docosa-1(22),18,20-triene-15-carboxamide |
| **Ex.324** | 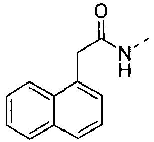 | 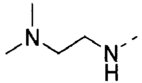 | S | (4*S*,6*R*,15*S*)-*N*-[2-(dimethylamino)ethyl]-11,16-dimethyl-6-{[2-(1-naphthyl)acetyl]amino}-9,12,17-trioxo-2-thia-8,11,16-triazatricyclo[16.3.1.0^{4,8}]docosa-1(22),18,20-triene-15-carboxamide |
| **Ex.325** | 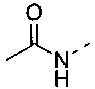 | 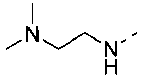 | S | (4*S*,6*R*,15*S*)-6-(acetylamino)-*N*-[2-(dimethylamino)ethyl]-11,16-dimethyl-9,12,17-trioxo-2-thia-8,11,16-triazatricyclo[16.3.1.0^{4,8}]docosa-1(22),18,20-triene-15-carboxamide |
| **Ex.326** | 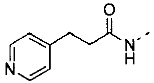 | 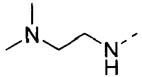 | S | (4*S*,6*R*,15*S*)-*N*-[2-(dimethylamino)ethyl]-11,16-dimethyl-9,12,17-trioxo-6-{[3-(9-pyridinyl)propanoyl]amino}-2-thia-8,11,16-triazatricyclo[16.3.1.0^{4,8}]docosa-1(22),18,20-triene-15-carboxamide |
| **Ex.327** | 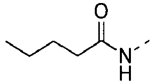 | 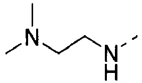 | S | (4*S*,6*R*,15*S*)-*N*-[2-(dimethylamino)ethyl]-11,16-dimethyl-9,12,17-trioxo-6-(pentanoylamino)-2-thia-8,11,16-triazatricyclo[16.3.1.0^{4,8}]docosa-1(22),18,20-triene-15-carboxamide |
| **Ex.328** | 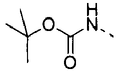 | 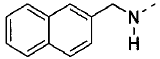 | SO₂ | *tert*-butyl *N*-[(4*S*,6*R*,15*S*)-11,16-dimethyl-15-{[(2-naphthylmethyl)amino]carbonyl}-2,2,9,12,17-pentaoxo-2λ⁶-thia-8,11,16-triazatricyclo[16.3.1.0^{4,8}]docosa-1(22),18,20-trien-6-yl]carbamate |
| **Ex.329** | 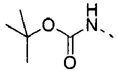 | 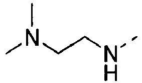 | SO₂ | tert-butyl *N*-[(4*S*,6*R*,15*S*)-15-({[2-(dimethylamino)ethyl]amino}carbonyl)-11,16-dimethyl-2,2,9,12,17-pentaoxo-2λ⁶-thia-8,11,16-triazatricyclo[16.3.1.0^{4,8}]docosa-1(22),18,20-trien-6-yl]carbamate |
| **Ex.330** | NH₂ | 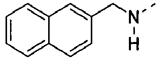 | SO₂ | (4*S*,6*R*,15*S*)-6-amino-11,16-dimethyl-*N*-(2-naphthylmethyl)-2,2,9,12,17-pentaoxo-2λ⁶-thia-8,11,16-triazatricyclo[16.3.1.0^{4,8}]docosa-1(22),18,20-triene-15-carboxamide |
| **Ex.331** | NH₂ | 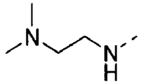 | SO₂ | (4*S*,6*R*,15*S*)-6-amino-*N*-[2-(dimethylamino)ethyl]-11,16-dimethyl-2,2,9,12,17-pentaoxo-2λ⁶-thia-8,11,16-triazatricyclo[16.3.1.0^{4,8}]docosa-1(22),18,20-triene-15-carboxamide |
| **Ex.332** | 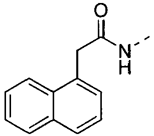 | 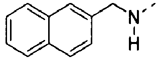 | SO₂ | (4*S*,6*R*,15*S*)-11,16-dimethyl-6-{[2-(1-naphthyl)acetyl]amino}-N-(2-naphthylmethyl)-2,2,9,12,17-pentaoxo-2λ⁶-thia-8,11,16-triazatricyclo[16.3.1.0^{4,8}]docosa-1(22),18,20-triene-15-carboxamide |
| **Ex.333** | 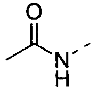 | 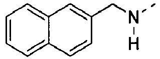 | SO₂ | (4*S*,6*R*,15*S*)-6-(acetylamino)-11,16-dimethyl-*N*-(2-naphthylmethyl)-2,2,9,12,17-pentaoxo-2λ⁶-thia-8,11,16-triazatricyclo[16.3.1.0^{4,8}]docosa-1(22),18,20-triene-15-carboxamide |
| **Ex.334** | 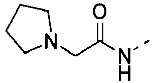 | 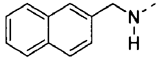 | SO₂ | (4*S*,6*R*,15*S*)-11,16-dimethyl-*N*-(2-naphthylmethyl)-2,2,9,12,17-pentaoxo-6-{[2-(1-pyrrolidinyl)acetyl]amino}-2λ⁶-thia-8,11,16-triazatricyclo[16.3.1.0^{4,8}]docosa-1(22),18,20-triene-15-carboxamide |
| **Ex.335** | 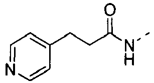 | 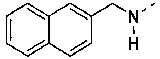 | SO₂ | (4*S*,6*R*,15*S*)-11,16-dimethyl-*N*-(2-naphthylmethyl)-2,2,9,12,17-pentaoxo-6-{[3-(4-pyridinyl)propanoyl]amino}-2λ⁶-thia-8,11,16-triazatricyclo[16.3.1.0^{4,8}]docosa-1(22),18,20-triene-15-carboxamide |
| **Ex.336** | 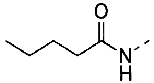 | 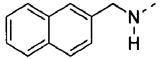 | SO₂ | (4*S*,6*R*,15*S*)-11,16-dimethyl-*N*-(2-naphthylmethyl)-2,2,9,12,17-pentaoxo-6-(pentanoylamino)-2λ⁶-thia-8,11,16-triazatricycio[16.3.1.0^{4,8}]docosa-1(22),18,20-triene-15-carboxamide |
| **Ex.337** | 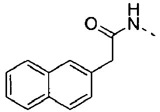 | 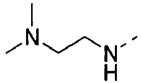 | SO₂ | (4*S*,6*R*,15*S*)-*N*-[2-(dimethylamino)ethyl]-11,16-dimethyl-6-{[2-(2-naphthyl)acetyl]amino}-2,2,9,12,17-pentaoxo-2λ⁶-thia-8,11,16-triazatricyclo[16.3.1.0^{4,8}]docosa-1(22),18,20-triene-15-carboxamide |
| **Ex.338** | 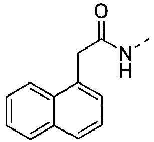 | 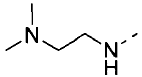 | SO₂ | (4*S*,6*R*,15*S*)-*N*-[2-(dimethylamino)ethyl]-11,16-dimethyl-6-{[2-(1-naphthyl)acetyl]amino}-2,2,9,12,17-pentaoxo-2λ⁶-thia-8,11,16-triazatricyclo[16.3.1.0^{4,8}]docosa-1(22),18,20-triene-15-carboxamide |
| **Ex.339** | 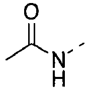 | 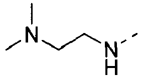 | SO₂ | (4*S*,6*R*,15*S*)-6-(acetylamino)-N-[2-(dimethylamino)ethyl]-11,16-dimethyl-2,2,9,12,17-pentaoxo-2λ⁶-thia-8,11,16-triazatricyclo[16.3.1.0^{4,8}]docosa-1(22),18,20-triene-15-carboxamide |
| **Ex.340** | 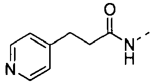 | 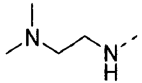 | SO₂ | (4*S*,6*R*,15*S*)-*N*-[2-(dimethylamino)ethyl]-11,16-dimethyl-2,2,9,12,17-pentaoxo-6-{[3-(4-pyridinyl)propanoyl]amino}-2λ⁶-thia-8,11,16-triazatricyclo[16.3.1.0^{4,8}]docosa-1(22),18,20-triene-15-carboxamide |
| **Ex.341** | 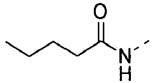 | 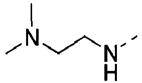 | SO₂ | (4*S*,6*R*,15*S*)-*N*-[2-(dimethylamino)ethyl]-11,16-dimethyl-2,2,9,12,17-pentaoxo-6-(pentanoylamino)-2λ⁶-thia-8,11,16-triazatricyclo[16.3.1.0^{4,8}]docosa-1(22),18,20-triene-15-carboxamide |

**Table 38a: Examples of Core 22 (Ex.342-Ex.362; continued on the following pages)**

| **No** | **R²** | **R⁵** | **Starting material** | **General Procedure** | **Reagent** | **Purification Method** | **Yield (isolated salt)** |
|---|---|---|---|---|---|---|---|
| **Ex.342-Ex.343:** *cf. experimental description* | | | | | | | |
| **Ex.344** | 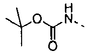 | 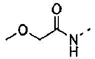 | **Ex.343** | L.1.1 | 2-methoxyacetyl chloride (1.5 equiv.) pyridine (5 equiv.) | FC (hexane/EtOAc/MeOH) | 97% |
| **Ex.345** | NH₂ | 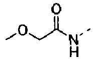 | **Ex.344** | J | HCl-dioxane | crude product | 76% (HCl salt) |
| **Ex.346** | 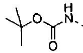 | 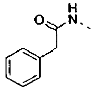 | **Ex.343** | L.1.1 | phenylacetyl chloride (1.5 equiv.) pyridine (5 equiv.) | FC (hexane/EtOAc/MeOH) | 84% |
| **Ex.347** | NH₂ | 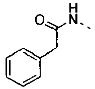 | **Ex.346** | J | HCl-dioxane | crude product | 94% (HCl salt) |
| **Ex.348** | 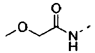 | 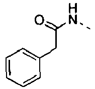 | **Ex.347** | L.1.1 | 2-methoxyacetyl chloride (1.5 equiv.) pyridine (5 equiv.) | FC (hexane/EtOAc/MeOH) | 97% |
| **Ex.349** | 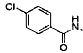 | 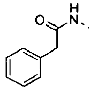 | **Ex.347** | L.1.1 | 4-chlorobenzoyl chloride (1.5 equiv.) pyridine (5 equiv.) | FC (hexane/EtOAc/MeOH) | 77% |
| **Ex.350** | 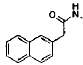 | 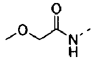 | **Ex.345** | L.1.3 | 2-naphthylacetic acid (1.0 equiv.), HATU (1.0 equiv.) HOAt (1.0 equiv.) pyridine (5 equiv.) | FC (hexane/EtOAc/MeOH) | 77% |
| **Ex.351** | 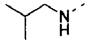 | 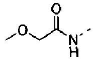 | **Ex.345*⁾** | M.2 | isobutyraldehyde (1.0 equiv.) | FC (hexane/EtOAc/MeOH) | 65% |
| **Ex.352** | 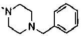 | 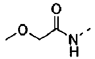 | **Ex.345** | O | N-benzyl-2-chloro-N-(2-chloroethyl)-ethanamine | FC (hexane/EtOAc/MeOH) | 52% |
| **Ex.353** | 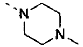 | 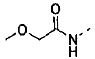 | **Ex.352** | M.4 | H₂, Pd(OH)₂-C aq. formaldehyde soln | FC (CH₂Cl₂/MeOH/aq. NH₃) | 75% |
| **Ex.354** | NH₂ | 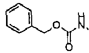 | **Ex.342** | J | HCl-dioxane | crude product | 93% (HCl salt) |
| **Ex.355** | 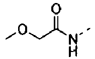 | 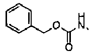 | **Ex.354** | L.1.1 | 2-methoxyacetyl chloride (1.5 equiv.) pyridine (5 equiv.) | FC (hexane/EtOAc/MeOH) | 86% |
| **Ex.356** | 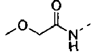 | NH₂ | **Ex.355** | K.1 | H₂, Pd(OH)₂-C | crude product | 94% |
| **Ex.357** | 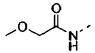 | 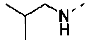 | **Ex.356** | M.2 | isobutyraldehyde (1.0 equiv.) | FC (hexane/EtOAc/MeOH) | 73% |
| **Ex.358** | 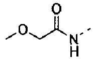 | 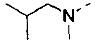 | **Ex.357** | M.3 | aq. formaldehyde soln (2.5 equiv.) NaBH(OAc)₃ (3 equiv.) | FC (hexane/EtOAc/MeOH) | 78% |
| **Ex.359** | 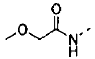 | 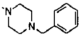 | **Ex.356** | O | N-benzyl-2-chloro-N-(2-chloroethyl)-ethanamine | FC (EtOAc/MeOH) | 52% |
| **Ex.360** | 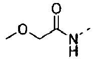 | 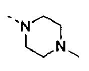 | **Ex.359** | M.4 | H₂, Pd(OH)₂-C aq. formaldehyde soln | FC (CH₂Cl₂/MeOH/aq. NH₃) | 66% |
| **Ex.361** | 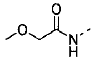 | 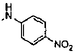 | **Ex.356** | **) | **) | FC (hexane/EtOAc/MeOH) | 59% |
| **Ex.362** | 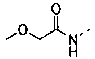 | 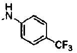 | **Ex.356** | ***) | ***) | prep. HPLC method 1 | 9% |

| | | | | | | | |
|---|---|---|---|---|---|---|---|
| *) Used as free base **) A soln of **Ex.356** (60 mg, 0.15 mmol) in DMSO (0.3 mL) was treated with 1-fluoro-4-nitrobenzene (0.031 mL, 0.3 mmol) and i-Pr₂NEt (0.084 mL, 0.6 mmol) at 50°C for 15 h. Aq. workup (EtOAc, H₂O, 1 M aq. HCl soln; Na₂SO₄) and FC (hexane/EtOAc/MeOH) afforded **Ex.361** (46 mg, 59%). ***) Dioxane (2.0 mL) was added to a mixture of 4-bromobenzotrifluoride (0.05 mL, 0.35 mmol), tris(dibenzylideneacetone)dipalladium(O)-chloroform adduct (30 mg), 9,9-dimethyl-4,5-bis(diphenylphosphino)xanthene (18 mg), **Ex.356** (60 mg, 0.15 mmol) and Cs₂CO₃ (115 mg, 0.35 mmol). The mixture was heated to 90°C for 36 h, followed by aq. workup (CH₂Cl₂, aq. Na₂CO₃ soln; Na₂SO₄) and prep. HPLC method 3 afforded **Ex.362** (7 mg, 9%). | | | | | | | |

**Table 38b: Examples of Core 22 (Ex.342-Ex.362; continued on the following page)**

| **No** | **R²** | **R⁵** | **FORMULA** | **MONOISOTOPIC MASS** | **Rt (purity at 220nm)** | **[M+H]⁺ found** | **LC-MS-Method** |
|---|---|---|---|---|---|---|---|
| **Ex.342-Ex.343:** *cf. experimental description* | | | | | | | |
| **Ex.344** | 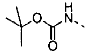 | 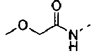 | C24H33FN4O7 | 508.2 | 1.66 (99) | 509.1 | Method 10a |
| **Ex.345** | NH₂ | 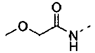 | C19H25FN4O5 | 408.2 | 0.91 (100) | 409.2 | Method 10a |
| **Ex.346** | 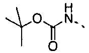 | 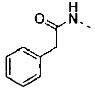 | C29H35FN4O6 | 554.3 | 1.92 (99) | 555.3 | Method 10a |
| **Ex.347** | NH₂ | 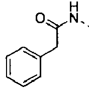 | C24H27FN4O4 | 454.2 | 1.20 (98) | 455.2 | Method 10a |
| **Ex.348** | 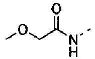 | 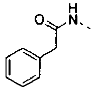 | C27H31FN4O6 | 526.2 | 1.50 (97) | 527.2 | Method 10a |
| **Ex.349** | 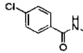 | 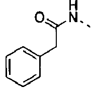 | C31H30ClFN4O5 | 592.2 | 1.93 (97) | 593.2 | Method 10a |
| **Ex.350** | 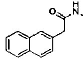 | 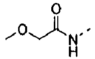 | C31H33FN4O6 | 576.2 | 1.72 (97) | 577.2 | Method 10a |
| **Ex.351** | 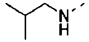 | 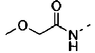 | C23H33FN4O5 | 464.2 | 1.16 (93) | 465.2 | Method 10a |
| **Ex.352** | 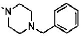 | 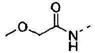 | C30H38FN5O5 | 567.3 | 1.24 (84) | 568.3 | Method 10a |
| **Ex.353** | 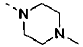 | 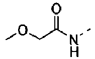 | C24H34FN5O5 | 491.2 | 1.01 (88) | 492.1 | Method 10a |
| **Ex.354** | NH₂ | 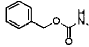 | C24H27FN4O5 | 470.2 | 1.29 (93) | 471.2 | Method 10a |
| **Ex.355** | 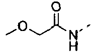 | 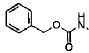 | C27H31FN4O7 | 542.2 | 1.60 (95) | 543.2 | Method 10a |
| **Ex.356** | 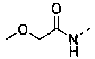 | NH₂ | C19H25FN4O5 | 408.2 | 1.02 (99) | 409.2 | Method 10a |
| **Ex.357** | 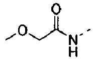 | 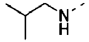 | C23H33FN4O5 | 469.2 | 1.15 (96) | 465.2 | Method 10a |
| **Ex.358** | 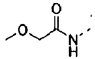 | 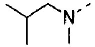 | C24H35FN4O5 | 478.2 | 1.19 (100) | 479.3 | Method 10a |
| **Ex.359** | 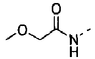 | 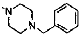 | C30H38FN5O5 | 567.3 | 1.22 (96) | 568.3 | Method 10a |
| **Ex.360** | 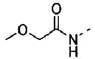 | 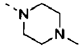 | C24H34FN5O5 | 491.3 | 1.04 (96) | 492.1 | Method 10a |
| **Ex.361** | 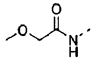 | 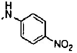 | C25H28FN5O7 | 529.2 | 1.61 (98) | 530.2 | Method 10a |
| **Ex.362** | 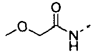 | 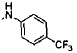 | C26H28F4N4O5 | 552.2 | 1.90 (97) | 553.2 | Method 10a |

**Table 38c: Examples of Core 22 (Ex.342-Ex.362; continued on the following pages)**

| **No** | **R²** | **R⁵** | **IUPAC name** |
|---|---|---|---|
| **Ex.342** | 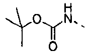 | 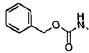 | *tert*-butyl *N-*((2*S,*6*S,*16a*S*)-6-{[(benzyloxy)carbonyl]amino}-12-fluoro-5,10-dioxo-2,3,5,6,7,8,9,10,16,16a-decahydro-1*H*-pyrrolo[2,1-c][1,4,9]benzoxadiazacyclododecin-2-yl)carbamate |
| **Ex.343** | 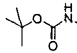 | NH₂ | *tert*-butyl *N*-[(2*S*,6*S*,16a*S*)-6-amino-12-fluoro-5,10-dioxo-2,3,5,6,7,8,9,10,16,16a-decahydro-1*H*-pyrrolo[2,1-*c*][1,4,9]benzoxadiazacyclododecin-2-yl]carbamate |
| **Ex.344** | 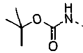 | 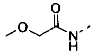 | *tert*-butyl *N-*{(2*S*,6*S*,16a*S*)-12-fluoro-6-[(2-methoxyacetyl)amino]-5,10-dioxo-2,3,5,6,7,8,9,10,16,16a-decahydro-1*H*-pyrrolo[2,1-*c*][1,4,9]benzoxadiazacyclododecin-2-yl}carbamate |
| **Ex.345** | NH₂ | 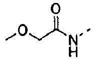 | *N*-[(2*S*,6*S*,16a*S*)-2-amino-12-fluoro-5,10-dioxo-2,3,5,6,7,8,9,10,16,16a-decahydro-1*H*-pyrrolo[2,1-*c*][1,4,9]benzoxadiazacyclododecin-6-yl]-2-methoxyacetamide |
| **Ex.346** | 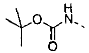 | 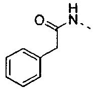 | *tert*-butyl *N*-{(2*S*,6*S*,16a*S*)-12-fluoro-5,10-dioxo-6-[(2-phenylacetyl)amino]-2,3,5,6,7,8,9,10,16,16a-decahydro-1*H*-pyrrolo[2,1-*c*][1,4,9]benzoxadiazacyclododecin-2-yl}carbamate |
| **Ex.347** | NH₂ | 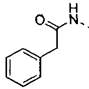 | *N*-[(2*S*,6*S*,16a*S*)-2-amino-12-fluoro-5,10-dioxo-2,3,5,6,7,8,9,10,16,16a-decahydro-1*H-*pyrrolo[2,1-*c*] [1,4,9]benzoxadiazacyclododecin-6-yl]-2-phenylacetamide |
| **Ex.348** | 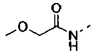 | 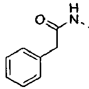 | *N-*{(2*S*,6*S*,16a*S*)-12-fluoro-5,10-dioxo-6-[(2-phenylacetyl)amino]-2,3,5,6,7,8,9,10,16,16a-decahydro-1*H*-pyrrolo[2,1-*c*][1,4,9]benzoxadiazacyclododecin-2-yl}-2-methoxyacetamide |
| **Ex.349** | 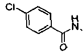 | 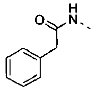 | *N-*{(2*S*,6*S,*16a*S*)-12-fluoro-5,10-dioxo-6-[(2-phenylacetyl)amino]-2,3,5,6,7,8,9,10,16,16a-decahydro-1*H*-pyrrolo[2,1-*c*][1,4,9]benzoxadiazacyclododecin-2-yl}-9-chlorobenzamide |
| **Ex.350** | 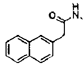 | 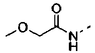 | *N*-((2*S*,6*S*,16a*S*)-12-fluoro-2-{[2-(2-naphthyl)acetyl]amino}-5,10-dioxo-2,3,5,6,7,8,9,10,16,16a-decahydro-1*H*-pyrrolo[2,1-*c*][1,4,9]benzoxadiazacyclododecin-6-yl)-2-methoxyacetamide |
| **Ex.351** | 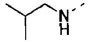 | 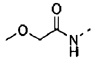 | *N*-[(2*S*,6*S*,16a*S*)-12-fluoro-2-(isobutylamino)-5,10-dioxo-2,3,5,6,7,8,9,10,16,16a-decahydro-1*H*-pyrrolo[2,1-*c*][1,4,9]benzoxadiazacyclododecin-6-yl]-2-methoxyacetamide |
| **Ex.352** | 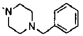 | 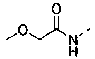 | *N*-[(2*S*,6*S*,16a*S*)-2-(4-benzylpiperazino)-12-fluoro-5,10-dioxo-2,3,5,6,7,8,9,10,16,16a-decahydro-1*H*-pyrrolo[2,1-*c*][1,9,9]benzoxadiazacyclododecin-6-yl]-2-methoxyacetamide |
| **Ex.353** | 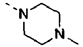 | 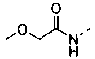 | *N*-[(2*S*,6*S*,16a*S*)-12-fluoro-2-(4-methylpiperazino)-5,10-dioxo-2,3,5,6,7,8,9,10,16,16a-decahydro-1*H*-pyrrolo[2,1-*c*][1,4,9]benzoxadiazacyclododecin-6-yl]-2-methoxyacetamide |
| **Ex.354** | NH₂ | 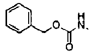 | benzyl *N*-[(2*S*,6*S*,16a*S*)-2-amino-12-fluoro-5,10-dioxo-2,3,5,6,7,8,9,10,16,16a-decahydro-1*H*-pyrrolo[2,1-*c*][1,4,9]benzoxadiazacyclododecin-6-yl]carbamate |
| **Ex.355** | 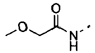 | 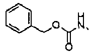 | benzyl *N-*{(2*S*,6*S*,16a*S*)-12-fluoro-2-[(2-methoxyacetyl)amino]-5,10-dioxo-2,3,5,6,7,8,9,10,16,16a-decahydro-1*H*-pyrrolo[2,1-*c*][1,9,9]benzoxadiazacyclododecin-6-yl}carbamate |
| **Ex.356** | 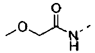 | NH₂ | *N*-[(2*S*,6*S*,16a*S*)-6-amino-12-fluoro-5,10-dioxo-2,3,5,6,7,8,9,10,16,16a-decahydro-1*H*-pyrrolo[2,1-*c*][1,4,9]benzoxadiazacyclododecin-2-yl]-2-methoxyacetamide |
| **Ex.357** | 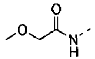 | 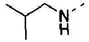 | *N*-[(2*S*,6*S*,16a*S*)-12-fluoro-6-(isobutylamino)-5,10-dioxo-2,3,5,6,7,8,9,10,16,16a-decahydro-1*H*-pyrrolo[2,1-*c*][1,4,9]benzoxadiazacyclododecin-2-yl]-2-methoxyacetamide |
| **Ex.358** | 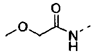 | 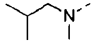 | *N*-{(2*S*,6*S*,16a*S*)-12-fluoro-6-[isobutyl(methyl)amino]-5,10-dioxo-2,3,5,6,7,8,9,10,16,16a-decahydro-1*H*-pyrrolo[2,1-*c*][1,4,9]benzoxadiazacyclododecin-2-yl}-2-methoxyacetamide |
| **Ex.359** | 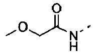 | 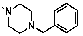 | *N*-[(2*S*,6*S*,16a*S*)-6-(4-benzylpiperazino)-12-fluoro-5,10-dioxo-2,3,5,6,7,8,9,10,16,16a-decahydro-1*H*-pyrrolo[2,1-*c*]̅[1,4,9]benzoxadiazacyclododecin-2-yl]-2-methoxyacetamide |
| **Ex.360** | 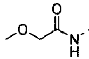 | 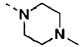 | *N*-[(2*S*,6*S*,16a*S*)-12-fluoro-6-(4-methylpiperazino)-5,10-dioxo-2,3,5,6,7,8,9,10,16,16a-decahydro-1*H*-pyrrolo[2,1-*c*][1,4,9]benzoxadiazacyclododecin-2-yl]-2-methoxyacetamide |
| **Ex.361** | 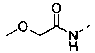 | 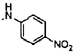 | *N*-[(2*S*,6*S*,16a*S*)-12-fluoro-6-(4-nitroanilino)-5,10-dioxo-2,3,5,6,7,8,9,10,16,16a-decahydro-1*H*-pyrrolo[2,1-*c*][1,4,9]benzoxadiazacyclododecin-2-yl]-2-methoxyacetamide |
| **Ex.362** | 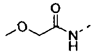 | 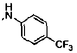 | *N-*{(2*S*,6*S*,16a*S*)-12-fluoro-5,10-dioxo-6-[4-(trifluoromethyl)anilino]-2,3,5,6,7,8,9,10,16,16a-decahydro-1*H*-pyrrolo[2,1-*c*][1,4,9]benzoxadiazacyclododecin-2-yl}-2-methoxyacetamide |

**Table 39a: Examples of Core 23 (Ex.363-Ex.378; continued on the following pages)**

| **No** | **R²** | **R⁵⁴** | **Starting material** | **General Procedure** | **Reagent** | **Purification Method** | **Yield (isolated salt)** |
|---|---|---|---|---|---|---|---|
| **Ex.363-Ex.364:** *cf. experimental description* | | | | | | | |
| **Ex.365** | 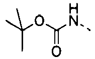 | 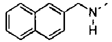 | **Ex.364** | L.2 | 2-naphthylmethylmaine | FC (EtOAc/MeOH) | 97% |
| **Ex.366** | 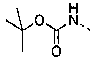 | 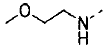 | **Ex.364** | L.2 | 2-methoxyethylamine | FC (EtOAc/MeOH) | 100% |
| **Ex.367** | 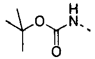 | 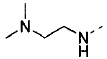 | **Ex.364** | L.2 | N,N-dimethylethylenediamine | FC (CH₂Cl₂/MeOH/ aq. NH₃) | 94% |
| **Ex.368** | NH₂ | 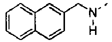 | **Ex.365** | J | HCl-dioxane | FC (CH₂Cl₂/MeOH/ aq. NH₃) | 83% |
| **Ex.369** | NH₂ | 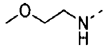 | **Ex.366** | J | HCl-dioxane | FC (CH₂Cl₂/MeOH/ aq. NH₃) | 94% |
| **Ex.370** | NH₂ | 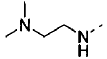 | **Ex.367** | J | HCl-dioxane | FC (CH₂Cl₂/MeOH/ aq. NH₃) | 83% |
| **Ex.371** | 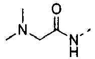 | 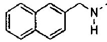 | **Ex.368** | L.1.3 | N,N-dimethyl glycine (1.2 equiv.) HATU (1.5 equiv.) HOAt (1.5 equiv.) i-Pr₂NEt (3 equiv.) | FC (CH₂Cl₂/MeOH) | 57% |
| **Ex.372** | 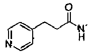 | 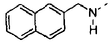 | **Ex. 368** | L.1.3 | 3-(pyridin-4-yl)propanoic acid (1.2 equiv.) HATU (1.5 equiv.) HOAt (1.5 equiv.) *i*-Pr₂NEt (3 equiv.) | FC (CH₂Cl₂/MeOH) | 44% |
| **Ex.373** | 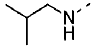 | 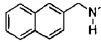 | **Ex.368** | M.2 | isobutyraldehyde | FC (CH₂Cl₂/MeOH) | 70% |
| **Ex.374** | 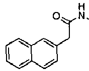 | 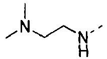 | **Ex.370** | L.1.3 | 2-naphthylacetic acid (1.2 equiv.) HATU (1.5 equiv.) HOAt (1.5 equiv.) *i*-Pr₂NEt (3 equiv.) | FC (CH₂Cl₂/MeOH/ aq. NH₃) | 85% |
| **Ex.375** | 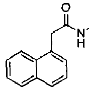 | 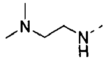 | **Ex.370** | L.1.3 | 1-naphthylacetic acid (1.2 equiv.) HATU (1.5 equiv.) HOAt (1.5 equiv.) *i*-Pr₂NEt (3 equiv.) | FC (CH₂Cl₂/MeOH/ aq. NH₃) | 40% |
| **Ex.376** | 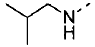 | 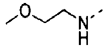 | **Ex.369** | M.2 | isobutyraldehyde | FC (CH₂Cl₂/MeOH) | 48% |
| **Ex.377** | 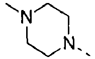 | 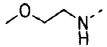 | **Ex.378** | M.4 | H₂, Pd (OH)₂; CH₂O | crude product | 75% |
| **Ex.378** | 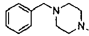 | 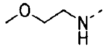 | **Ex.369** | O | N-benzyl-2-chloro-N-(2-chloroethyl)-ethanamine | FC (CH₂Cl₂/MeOH) and prep. HPLC Method 3 | 43% |

**Table 39b: Examples of Core 23 (Ex.363-Ex.378; continued on the following pages)**

| **No** | **R²** | **R⁵⁴** | **FORMULA** | **MONOISOTOPIC MASS** | **Rt (purity at 220nm)** | **[M+H]⁺ found** | **LC-MS-Method** |
|---|---|---|---|---|---|---|---|
| **Ex.363-Ex.364:** *cf. experimental description* | | | | | | | |
| **Ex.365** | 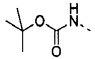 | 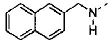 | C34H40N4O7 | 616.3 | 2.04 (91) | 617.2 | Method 10a |
| **Ex.366** | 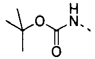 | 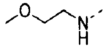 | C26H38N4O8 | 534.3 | 1.53 (100) | 535.2 | Method 10a |
| **Ex.367** | 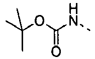 | 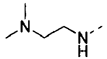 | C27H41N5O7 | 547.3 | 1.25 (98) | 548.3 | Method 10a |
| **Ex.368** | NH₂ | 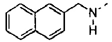 | C29H32N4O5 | 516.2 | 1.38 (96) | 517.2 | Method 10a |
| **Ex.369** | NH₂ | 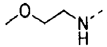 | C21H30N4O6 | 439.2 | 0.71 (99) | 435.2 | Method 10a |
| **Ex.370** | NH₂ | 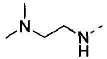 | C22H33N5O5 | 447.3 | 1.02 (99) | 448.2 | Method 11a |
| **Ex.371** | 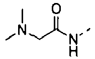 | 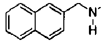 | C33H39N5O6 | 601.2 | 1.39 (95) | 602.3 | Method 10a |
| **Ex.372** | 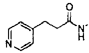 | 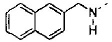 | C37H39N5O6 | 649.3 | 1.41 (96) | 650.3 | Method 10a |
| **Ex.373** | 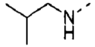 | 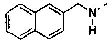 | C33H40N4O5 | 572.3 | 1.54 (94) | 573.2 | Method 10a |
| **Ex.374** | 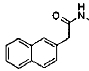 | 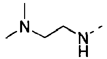 | C34H41N5O6 | 615.3 | 1.38 (98) | 616.3 | Method 10a |
| **Ex.375** | 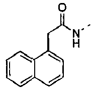 | 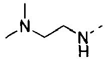 | C34H41N5O6 | 615.3 | 1.36 (90) | 616.3 | Method 10a |
| **Ex.376** | 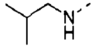 | 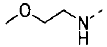 | C25H38N4O6 | 490.3 | 0.99 (97) | 491.3 | Method 10a |
| **Ex.377** | 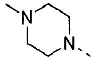 | 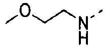 | C26H39N5O6 | 517.3 | 0.83 (97) | 518.3 | Method 10a |
| **Ex.378** | 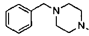 | 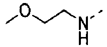 | C32H43N5O6 | 593.3 | 1.14 (97) | 594.3 | Method 10a |

**Table 39c: Examples of Core 23 (Ex.363-Ex.378; continued on the following pages)**

| **No** | **R²** | **R⁵⁴** | **IUPAC name** |
|---|---|---|---|
| **Ex.363** | 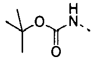 | 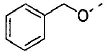 | benzyl (4*S*,6*S*,13*S*)-6-[(*tert*-butoxycarbonyl)amino]-14-methyl-9,15-dioxo-2,11-dioxa-8,14-diazatricyclo[14.3.1.0^{4,8}]icosa-1(20),16,18-triene-13-carboxylate |
| **Ex.364** | 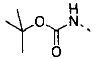 | OH | (4*S*,6*S*,13*S*)-6-[(*tert*-butoxycarbonyl)amino]-14-methyl-9,15-dioxo-2,11-dioxa-8,14-diazatricyclo[14.3.1.0^{4,8}]icosa-1(20),16,18-triene-13-carboxylic acid |
| **Ex.365** | 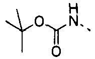 | 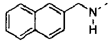 | *tert*-butyl *N*-[(4*S*,6*S*,13*S*)-14-methyl-13-{[(2-naphthylmethyl)amino]carbonyl}-9,15-dioxo-2,11-dioxa-8,14-diazatricyclo[14.3.1.0^{4,8}]icosa-1(20),16,18-trien-6-yl]carbamate |
| **Ex.366** | 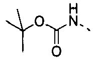 | 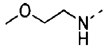 | *tert*-butyl *N*-[(4*S*,6*S*,13*S*)-13-{[(2-methoxyethyl)amino]carbonyl}-14-methyl-9,15-dioxo-2,11-dioxa-8,14-diazatricyclo[14.3.1.0^{4,8}] icosa-1(20),16,18-trien-6-yl]carbamate |
| **Ex.367** | 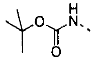 | 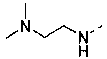 | *tert*-butyl *N*-[(4*S*,6*S*,13*S*)-13-({[2-(dimethylamino)ethyl]amino}carbonyl)-14-methyl-9,15-dioxo-2,11-dioxa-8,14-diazatricyclo[14.3.1.0^{4,8}]icosa-1(20),16,18-trien-6-yl]carbamate |
| **Ex.368** | NH₂ | 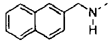 | (4*S*,6*S*,13*S*)-6-amino-14-methyl-N-(2-naphthylmethyl)-9,15-dioxo-2,11-dioxa-8,14-diazatricyclo[14.3.1.0^{4,8}]icosa-1(20),16,18-triene-13-carboxamide |
| **Ex.369** | NH₂ | 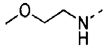 | (4*S*,6*S*,13*S*)-6-amino-*N*-(2-methoxyethyl)-14-methyl-9,15-dioxo-2,11-dioxa-8,14-diazatricyclo[14.3.1.0^{4,8}]icosa-1(20),16,18-triene-13-carboxamide |
| **Ex.370** | NH₂ | 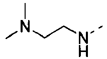 | (4*S*,6*S*,13*S*)-6-amino-N-[2-(dimethylamino)ethyl]-14-methyl-9,15-dioxo-2,11-dioxa-8,14-diazatricyclo[14.3.1.0^{4,8}]icosa-1(20),16,18-triene-13-carboxamide |
| **Ex.371** | 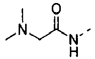 | 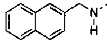 | (4*S*,6*S*,13*S*)-6-{ [2-(dimethylamino)acetyl]amino}-14-methyl-*N*-(2-naphthylmethyl)-9,15-dioxo-2,11-dioxa-8,14-diazatricyclo[14.3.1.0^{4,8}]icosa-1(20),16,18-triene-13-carboxamide |
| **Ex.372** | 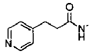 | 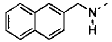 | (4*S*,6*S*,13*S*)-14-methyl-N-(2-naphthylmethyl)-9,15-dioxo-6-{[3-(4-pyridinyl)propanoyl]amino}-2,11-dioxa-8,14-diazatricyclo[14.3.1.0^{4,8}]icosa-1(20),16,18-triene-13-carboxamide |
| **Ex.373** | 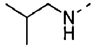 | 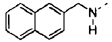 | (4*S*,6*S*,13*S*)-6-(isobutylamino)-14-methyl-*N*-(2-naphthylmethyl)-9,15-dioxo-2,11-dioxa-8,14-diazatricyclo[14.3.1.0^{4,8}]icosa-1(20),16,18-triene-13-carboxamide |
| **Ex.374** | 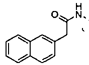 | 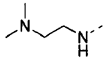 | (4*S*,6*S*,13*S*)-*N*-[2-(dimethylamino)ethyl]-14-methyl-6-{[2-(2-naphthyl)acetyl]amino}-9,15-dioxo-2,11-dioxa-8,14-diazatricyclo[14.3.1.0^{4,8}]icosa-1(20),16,18-triene-13-carboxamide |
| **Ex.375** | 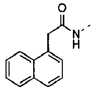 | 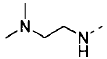 | (4*S*,6*S*,13*S*)-*N*-[2-(dimethylamino)ethyl]-14-methyl-6-{[2-(1-naphthyl)acetyllaminol-9,15-dioxo-2,11-dioxa-8,14-diazatricyclo[14.3.1.0^{4,8}]icosa-1(20),16,18-triene-13-carboxamide |
| **Ex.376** | 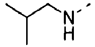 | 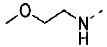 | (4*S*,6*S*,13*S*)-6-(isobutylamino)-*N*-(2-methoxyethyl)-14-methyl-9,15-dioxo-2,11-dioxa-8,14-diazatricyclo[14.3.1.0^{4,8}]icosa-1(20),16,18-triene-13-carboxamide |
| **Ex.377** | 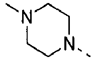 | 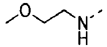 | (4*S*,6*S*,13*S*)-*N*-(2-methoxyethyl)-14-methyl-6-(4-methylpiperazino)-9,15-dioxo-2,11-dioxa-8,14-diazatricyclo[14.3.1.0^{4,8}]icosa-1(20),16,18-triene-13-carboxamide |
| **Ex.378** | 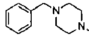 | 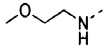 | (4*S*,6*S*,13*S*)-6-(4-benzylpiperazino)-*N*-(2-methoxyethyl)-14-methyl-9,15-dioxo-2,11-dioxa-8,14-diazatricyclo[14.3.1.0^{4,8}]icosa-1(20),16,18-triene-13-carboxamide |

**Table 40.1.a: Examples of Core 24a (Ex.379-Ex.391; continued on the following page)**

| **No** | **R²** | **R⁵** | **Starting material** | **General Procedure** | **Reagent** | **Purification Method** | **Yield (isolated salt)** |
|---|---|---|---|---|---|---|---|
| **Ex.379-Ex.380:** *cf. experimental description* | | | | | | | |
| **Ex.381** | 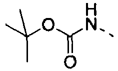 | 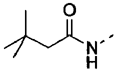 | **Ex.380** | L.1.1 | 3,3-dimethylbutanoyl chloride (1.5 equiv.) Pyridine (5 equiv.) | FC (hexane/EtOAc/ MeOH) | 60% |
| **Ex.382** | 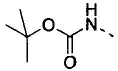 | 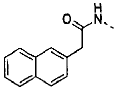 | **Ex.380** | L.1.3 | 2-naphthylacetic acid i-Pr₂NEt (2 equiv.) | FC(hexane/ EtOAc/MeoH),FC (CH2Cl2/MeOH) | 72% |
| **Ex.383** | NH₂ | 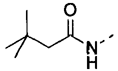 | **Ex.381** | J | HCl-dioxane | crude product | quant. (HCl salt) |
| **Ex.384** | NH₂ | 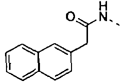 | **Ex.382** | J | HCl-dioxane | crude product | 96% (HCl salt) |
| **Ex.385** | 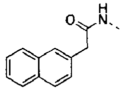 | 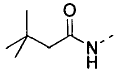 | **Ex.383** | L.1.3 | 2-naphthylacetic acid i-Pr₂NEt (3 equiv.) | FC (hexane/EtOAc/ MeOH) | 79% |
| **Ex.386** | 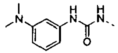 | 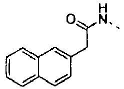 | **Ex.384** | L.4 | **2,5-dioxo-**pyrrolidin-1-yl-3-(dimethylamino) phenylcarbamate | FC (hexane/EtOAc/ MeOH) | 55% |
| **Ex.387** | NH₂ | 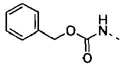 | **Ex.379** | J | HCl-dioxane | crude product | quant. (HCl salt) |
| **Ex.388** | 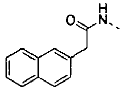 | 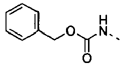 | **Ex.387** | L.1.3 | 2-naphthylacetic acid (1.2 equiv.) HATU (1.5 equiv.) HOAt (1.5 equiv.) i-Pr₂NEt (3 equiv.) | FC (hexane/EtOAc/ MeOH) | 63% |
| **Ex.389** | 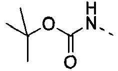 | CH₃(CH₂)₈ CONH | **Ex.380** | L.1.3 | decanoic acid i-Pr₂NEt (2 equiv.) | FC (hexane/EtOAc/ MeOH) | 71% |
| **Ex.390** | NH₂ | CH₃ (CH₂) 8 CONH | **Ex.389** | J | HCl-dioxane | crude product | quant. (HCl salt) |
| **Ex.391** | 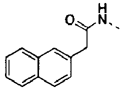 | CH₃(CH₂)₈ CONH | **Ex.390** | L.1.3 | 2-naphthylacetic acid i-Pr₂NEt (3 equiv.) | FC (hexane/EtOAc/ MeOH) | 70% |

**Table 40.1.b: Examples of Core 24a (Ex.379-Ex.391; continued on the following page)**

| **No** | **R²** | **R⁵** | **FORMULA** | **MONOISOTOPIC MASS** | **Rt (purity at 220nm)** | **[M+H]⁺ found** | **LC-MS-Method** |
|---|---|---|---|---|---|---|---|
| **Ex.379-Ex.380:** *cf. experimental description* | | | | | | | |
| **Ex.381** | 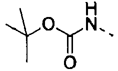 | 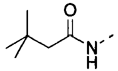 | C28H42N4O6 | 530.3 | 1.84 (99) | 531.3 | Method 10c |
| **Ex.382** | 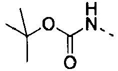 | 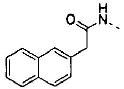 | C34H40N4O6 | 600.3 | 2.01 (99) | 601.3 | Method 10a |
| **Ex.383** | NH₂ | 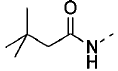 | C23H34N4O4 | 430.3 | 1.15 (98) | 431.3 | Method 10a |
| **Ex.384** | NH₂ | 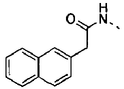 | C29H32N4O4 | 500.2 | 1.39 (93) | 501.3 | Method 10a |
| **Ex.385** | 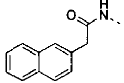 | 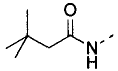 | C35H42N4O5 | 598.3 | 1.91 (90) | 599.2 | Method 10a |
| **Ex.386** | 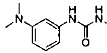 | 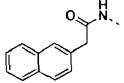 | C38H42N6O5 | 662.3 | 1.52 (98) | 663.3 | Method 10a |
| **Ex.387** | NH₂ | 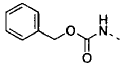 | C25H30N4O5 | 466.2 | 1.22 (56) | 467.3 | Method 10a |
| **Ex.388** | 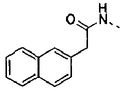 | 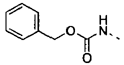 | C37H38N4O6 | 634.3 | 1.99 (97) | 635.3 | Method 10a |
| **Ex.389** | 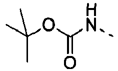 | CH₃(CH₂)₈CONH | C32H50N4O6 | 586.4 | 2.38 (87) | 587.4 | Method 10a |
| **Ex.390** | NH₂ | CH₃(CH₂)₈CONH | C27H42N4O4 | 486.3 | 1.69 (91) | 487.3 | Method 10a |
| **Ex.391** | 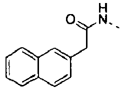 | CH₃(CH₂)₈CONH | C39H50N4O5 | 654.4 | 2.38 (90) | 655.4 | Method 10a |

**Table 40.1.c: Examples of Core 24a (Ex.379-Ex.391; continued on the following pages)**

| **No** | **R²** | **R⁵** | **IUPAC name** |
|---|---|---|---|
| **Ex.379** | 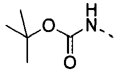 | 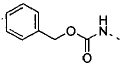 | benzyl *N*-[(4*S*,6*S*,10*S*)-6-[(*tert*-butoxycarbonyl)amino]-9,15-dioxo-2-oxa-8,14-diazatricyclo[14.3.1.0^{4,8}]icosa-1(20),16,18-trien-10-yl]carbamate |
| **Ex.380** | 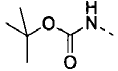 | NH₂ | *tert*-butyl *N*-[(4*S*,6*S*,10*S*)-10-amino-9,15-dioxo-2-oxa-8,14-diazatricyclo[14.3.1.0^{4,8}]icosa-1(20),16,18-trien-6-yl]carbamate |
| **Ex.381** | 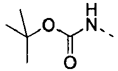 | 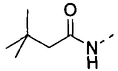 | *tert*-butyl *N*-[(4*S*,6*S*,10*S*)-10-[(3,3-dimethylbutanoyl)amino]-9,15-dioxo-2-oxa-8,14-diazatricyclo[14.3.1.0^{4,8}]icosa-1(20),16,18-trien-6-yl]carbamate |
| **Ex.382** | 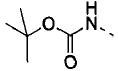 | 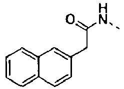 | tert-butyl *N*-[(4*S*,6*S*,10*S*)-10-{[2-(2-naphthyl)acetyl]amino}-9,15-dioxo-2-oxa-8,14-diazatricyclo[14.3. 1.0^{4,8}]icosa-1(20),16,18-trien-6-yl]carbamate |
| **Ex.383** | NH₂ | 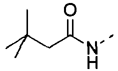 | N-[(4*S*,6*S*,10*S*)-6-amino-9,15-dioxo-2-oxa-8,14-diazatricyclo[14.3.1.0^{4,8}]icosa-1(20),16,18-trien-10-yl]-3,3-dimethylbutanamide |
| **Ex.384** | NH₂ | 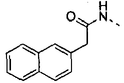 | *N*-[(4*S*,6*S*,10*S*)-6-amino-9,15-dioxo-2-oxa-8,14-diazatricyclo[14.3.1.0^{4,8}]icosa-1(20),16,18-trien-10-yl]-2-(2-naphthyl)acetamide |
| **Ex.385** | 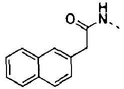 | 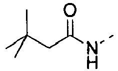 | 3,3-dimethyl-N-[(4*S*,6*S*,10*S*)-6-{[2-(2-naphthyl)acetyl]amino}-9,15-dioxo-2-oxa-8,14-diazatricyclo[14.3.1.0^{4,8}]icosa-1(20),16,18-trien-10-yl]butanamide |
| **Ex.386** | 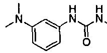 | 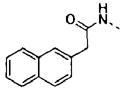 | *N-*[(4*S*,6S,10*S*)-6-({[3-(dimethylamino)anilino]carbonyl}amino)-9,15-dioxo-2-oxa-8,14-diazatricyclo[14.3.1.0^{4,8}]icosa-1(20),16,18-trien-10-yl]-2-(2-naphthyl)acetamide |
| **Ex.387** | NH₂ | 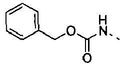 | benzyl *N*-[(4*S*,6*S*,10*S*)-6-amino-9,15-dioxo-2-oxa-8,14-diazatricyclo[14.3.1.0^{4,8}]icosa-1(20),16,18-trien-10-yl]carbamate |
| **Ex.388** | 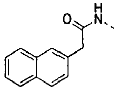 | 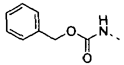 | benzyl *N*-[(4*S*,6*S*,10*S*)-6-{[2-(2-naphthyl)acetyl]amino}-9,15-dioxo-2-oxa-8,14-diazatricyclo[14.3.1.0^{4,8}]icosa-1(20),16,18-trien-10-yl]carbamate |
| **Ex.389** | 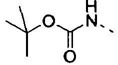 | CH₃(CH₂)₈C ONH | *tert*-butyl *N*-[(4*S*,6*S*,10*S*)-10-(decanoylamino)-9,15-dioxo-2-oxa-8,14-diazatricyclo[14.3.1.0^{4,8}]icosa-1(20),16,18-trien-6-yl]carbamate |
| **Ex.390** | NH₂ | CH₃(CH₂)₈C ONH | *N*- [(4*S*,6*S*,10*S*)-6-amino-9,15-dioxo-2-oxa-8,14-diazatricyclo[14.3.1.0^{4,8}]icosa-1(20),16,18-trien-10-yl]decanamide |
| **Ex.391** | 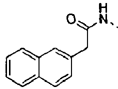 | CH₃(CH₂)₈C ONH | *N*-[(4*S*, 6*S*,10*S*)-6-{[2-(2-naphthyl)acetyl]amino}-9,15-dioxo-2-oxa-8,14-diazatricyclo[14.3.1.0^{4,8}]icosa-1(20),16,18-trien-10-yl]decanamide |

**Table 40.2.a: Examples of Core 24b (Ex.392-Ex.396)**

| **No** | **R²** | **R⁵** | **Starting material** | **General Procedure** | **Reagent** | **Purification Method** | **Yield (isolated salt)** |
|---|---|---|---|---|---|---|---|
| **Ex.392-Ex.393:** *cf. experimental description* | | | | | | | |
| **Ex.394** | 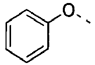 | 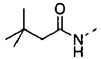 | **Ex.353** | L.1.1 | 3,3-dimezhylbutanoyl chloride (1.5 equiv.) Pyridine (5 equiv.) | FC (hexane/EtOAc/ MeOH) | 68% |
| **Ex.395** | 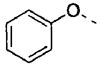 | 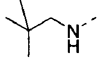 | **Ex.353** | M.2 | pivalaldehyde | FC (hexane/EtOAc/ MeOH) | 40% |
| **Ex.396** | 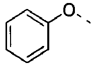 | 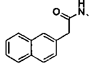 | **Ex.353** | L.1.3 | 2-naphthylacetic acid i-Pr₂NEt (2 equiv.) | FC (hexane/EtOAc/ MeOH) | 37% |

**Table 40.2.b: Examples of Core 24b (Ex.392-Ex.396; continued on the following page)**

| **No** | **R²** | **R⁵** | **FORMULA** | **MONOISOTOPIC MASS** | **Rt (purity at 220nm)** | **[M+H]⁺ found** | **LC-MS-Method** |
|---|---|---|---|---|---|---|---|
| **Ex.392-Ex.393:** *cf. experimental description* | | | | | | | |
| **Ex.394** | 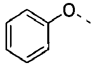 | 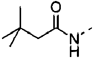 | C29H37N3O5 | 507.3 | 1.97 (95) | 508.2 | Method 10a |
| **Ex.395** | 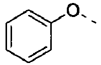 | 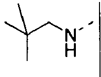 | C28H37N3O4 | 479.3 | 1.62 (99) | 480.2 | Method 10a |
| **Ex.396** | 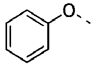 | 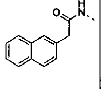 | C35H35N3O5 | 577.3 | 2.12 (97) | 578.0 | Method 10a |

**Table 40.2.c: Examples of Core 24b (Ex.392-Ex.396)**

| **No** | **R²** | **R⁵** | **IUPAC name** |
|---|---|---|---|
| **Ex.392** | 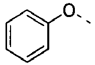 | 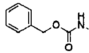 | benzyl *N*-[(4*S*,6*S*,10*S*)-9,15-dioxo-6-phenoxy-2-oxa-8,14-diazatricyclo[14.3.1.0^{4,8}]icosa-l(20),16,18-trien-10-yl]carbamate |
| **Ex.393** | 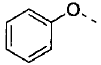 | NH₂ | (4*S*,6*S*,10*S*)-10-amino-6-phenoxy-2-oxa-8,14-diazatricyclo[14.3.1.0^{4,8}]icosa-1(20),16,18-triene-9,15-dione |
| **Ex.394** | 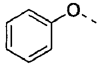 | 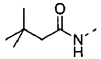 | *N*-[(4*S*,6*S*,10*S*)-9,15-dioxo-6-phenoxy-2-oxa-8,14-diazatricyclo[14.3.1.0^{4,8}]icosa-1(20),16,18-trien-10-yl]-3,3-dimethylbutanamide |
| **Ex.395** | 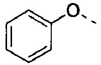 | 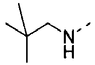 | (4*S*,6*S*,10*S*)-10-(neopentylamino)-6-phenoxy-2-oxa-8,14-diazatricyclo[14.3.1.0^{4,8}]icosa-1(20),16,18-triene-9,15-dione |
| **Ex.396** | 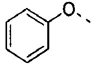 | 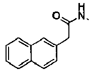 | *N*-[(4*S*,6*S*,10*S*)-9,15-dioxo-6-phenoxy-2-oxa-8,14-diazatricyclo[14.³.1.0^{4,8}]icosa-1(20),16,18-trien-10-yl]-2-(2-naphthyl)acetamide |

**Table 40.3.a: Examples of Core 24c (Ex.398-Ex.402)**

| **No** | **R²** | **R⁵** | **Starting material** | **General Procedure** | **Reagent** | **Purification Method** | **Yield (isolated salt)** |
|---|---|---|---|---|---|---|---|
| **Ex.398 :** *cf. experimental description* | | | | | | | |
| **Ex.399** | OH | OH | **Ex.397** | K.1 | H₂, Pd(OH)₂-C, CH₂Cl₂/MeOH 1:1 | FC (CH₂Cl₂/MeOH) | 97% |
| **Ex.400** | 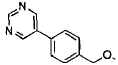 | OH | **Ex.397** | P | 5-pyrimidineboronic acid (1.3 equiv.) | prep. HPLC, method 1 | 61% |
| **Ex.401** | 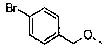 | 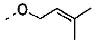 | **Ex.397** | Q | 4-bromo-2-methyl-2-butene (2 equiv.) | FC (CH₂Cl₂/MeOH) | 52% |
| **Ex.402** | OH | 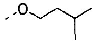 | **Ex.401** | K.1 | H₂, Pd(OH)₂-C, MeOH | FC (CH₂Cl₂/MeOH) | 51% |

**Table 40.3.b: Examples of Core 24c (Ex.398-Ex.402; continued on the following page)**

| **No** | **R²** | **R⁵** | **FORMULA** | **MONOISOTOPIC MASS** | **Rt (purity at 220nm)** | **[M+H]⁺ found** | **LC-MS-Method** |
|---|---|---|---|---|---|---|---|
| **Ex.398:** *cf. experimental description* | | | | | | | |
| **Ex.399** | OH | OH | C17H22N2O5 | 334.2 | 0.79 (98) 335.1 | | Method 10a |
| **Ex.400** | 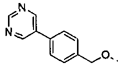 | OH | C28H30N4O5 | 502.2 | 1.40(98) | 503.2 | Method 10a |
| **Ex.401** | 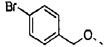 | 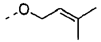 | C29H35BrN2O5 | 570.2 | 2.27 (96) | 573.0/571.0 | Method 10a |
| **Ex.402** | OH | 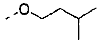 | C22H32N2O5 | 404.2 | 1.49 (93) | 405.2 | Method 10a |

**Table 40.3.c: Examples of Core 24c (Ex.398-Ex.402)**

| **No** | **R2** | **R5** | **IUPAC name** |
|---|---|---|---|
| **Ex.398** | 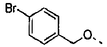 | OH | **(4*S*,6*S*,10*S*)-6-[(4-bromobenzyl)oxy]-10-hydroxy-2-oxa-8,14-**diazatricyclo[14.3.1.0^{4,8}]icosa-1(20),16,18-triene-9,15-dione |
| **Ex.399** | OH | OH | (4*S*,6*S*,10*S*)-6,10-dihydroxy-2-oxa-8,14-diazatricyclo[14.3.1.0^{4,8}]icosa-1(20),16,18-triene-9,15-dione |
| **Ex.400** | 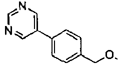 | OH | (4*S*,6*S*,10*S*)-10-hydroxy-6-{[4-(5-pyrimidinyl)benzyl]oxy}-2-oxa-8,19-diazatricyclo[14.3.1.0^{4,8}]icosa-1(20),16,18-triene-9,15-dione |
| **Ex.401** | 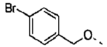 | 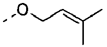 | (4*S*,6*S*,10*S*)-6-[(4-bromobenzyl)oxy]-10-[(3-methyl-2-butenyl)oxy]-2-oxa-8,14-diazatricyclo[14.3.1.0^{4,8}]icosa-1(20),16,18-triene-9,15-dione |
| **Ex.402** | OH | 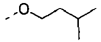 | (4*S*,6*S*,10*S*)-6-hydroxy-10-(isopentyloxy)-2-oxa-8,14-diazatricyclo[14.3.1.0^{4,8}]icosa-1(20),16,18-triene-9,15-dione |

**Table 41a: Examples of Core 25 (Ex.403-Ex.414; continued on the following pages)**

| **No** | **R¹¹** | **R⁵** | **Starting material** | **General Procedure** | **Reagent** | **Purification Method** | **Yield (isolated salt)** |
|---|---|---|---|---|---|---|---|
| **Ex.403-Ex.404:** *cf. experimental description* | | | | | | | |
| **Ex.405** | 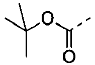 | 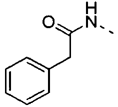 | **Ex.404** | L.1.3 | phenylacetic acid i-Pr₂NEt (2 equiv.) | FC (hexane/EtOAc) | 75% |
| **Ex.406** | H | 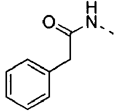 | **Ex.405** | J | HCl-dioxane | crude product | 88%*⁾ |
| **Ex.407** | 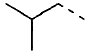 | 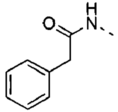 | **Ex.406** | **) | isobutyraldehyde**) | prep. HPLC, method 3 | 72% |
| **Ex.408** | 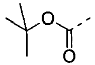 | 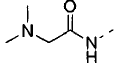 | **Ex.404** | L.1.3 | N,N-dimethylglycine i-Pr₂NEt (3 equiv.) | FC (hexane/EtOAc/MeOH) | 81% |
| **Ex.409** | H | 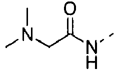 | **Ex**.**408** | J | HCl-dioxane | crude product | 94% (HCl salt) |
| **Ex.410** | 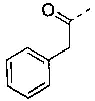 | 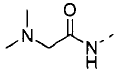 | **Ex.409** | L.1.3 | phenylacetic acid (1.0 equiv) HATU (1.0 equiv.) HOAt (1.0 equiv.) i-Pr₂NEt (2.0 equiv) | FC (EtOAc, then CH₂Cl₂/MeOH) | 46% |
| **Ex.411** | 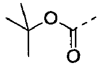 | 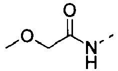 | **Ex.404** | L.1.1 | 2-methoxyacetyl chloride (1.5 equiv.) pyridine (5 equiv.) | FC (hexane/EtOAc /MeOH) | 84% |
| **Ex.412** | H | 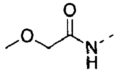 | **Ex.411** | J | HCl-dioxane | crude product | 97% (HCl salt) |
| **Ex.413** | CH₃ | 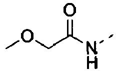 | **Ex.412** | M.5 | aq. formaldehyde soln (2.5 equiv.) | FC (EtOAc/MeOH) | 82% |
| **Ex.414** | 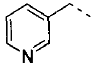 | 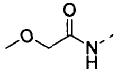 | **Ex.412** | M.5 | nicotin aldehyde | FC (EtOAc/MeOH) | 77% |

| | | | | | | | |
|---|---|---|---|---|---|---|---|
| *) workup (CH₂Cl₂, aq. Na₂CO₃ soln) to yield the free base **) in analogy to procedure M.3; isobutyraldehyde (1.2 equiv.), acetic acid (1.2 equiv.), NaBH(OAc)₃ (2.5 equiv.) in THF | | | | | | | |

**Table 41b: Examples of Core 25 (Ex.403-Ex.414; continued on the following page)**

| **No** | **R¹¹** | **R⁵** | **FORMULA** | **MONOISOTOPIC MASS** | **Rt (purity at 220nm)** | **[M+H]⁺ found** | **LC-MS-Method** |
|---|---|---|---|---|---|---|---|
| **Ex.403-Ex.404:** *cf. experimental description* | | | | | | | |
| **Ex.405** | 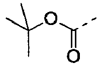 | 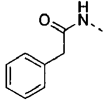 | C31H39FN4O6 | 582.3 | 2.06 (93) | 583.3 | Method 4a |
| **Ex.406** | H | 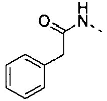 | C26H31FN4O4 | 482.2 | 1.48 (94) | 483.3 | Method 4a |
| **Ex.407** | 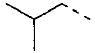 | 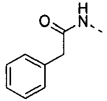 | C30H39FN4O4 | 538.3 | 1.45 (97) | 539.4 | Method 10a |
| **Ex.408** | 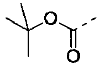 | 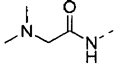 | C27H40FN5O6 | 549.3 | 1.44 (98) | 550.3 | Method 10a |
| **Ex.409** | H | 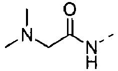 | C22H32FN5O4 | 449.2 | 1.27 (98) | 450.2 | Method 12 |
| **Ex.410** | 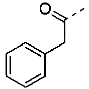 | 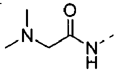 | C30H38FN5O5 | 567.3 | 1.36 (93) | 568.3 | Method 10a |
| **Ex.411** | 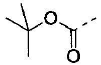 | 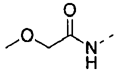 | C26H37FN4O7 | 536.3 | 1.66 (97) | 537.3 | Method 10c |
| **Ex.412** | H | 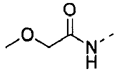 | C21H29FN4O5 | 436.2 | 1.02 (97) | 437.2 | Method 10a |
| **Ex.413** | CH₃ | 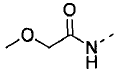 | C22H31FN4O5 | 450.2 | 1.02 (94) | 451.2 | Method 10a |
| **Ex.414** | 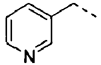 | 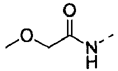 | C27H34FN5O5 | 527.3 | 1.03 (95) | 528.2 | Method 10a |

**Table 41c: Examples of Core 25 (Ex.403-Ex.414, continued on the following page)**

| **No** | **R¹¹** | **R⁵** | **IUPAC name** |
|---|---|---|---|
| **Ex.403** | 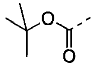 | 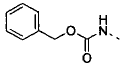 | *tert*-butyl (1*S*,15*S*,18*R*)-15-{[(benzyloxy)carbonyl]amino}-7-fluoro-11-methyl-10,16-dioxo-3-oxa-11,17,20-triazatricyclo[16.2.1.0^{4,9}]henicosa-4,6,8-triene-20-carboxylate |
| **Ex.404** | 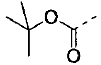 | NH₂ | *tert*-butyl (1*S*,15*S*,18*R*)-15-amino-7-fluoro-11-methyl-10,16-dioxo-3-oxa-11,17,20-triazatricyclo[16.2.1.0^{4,9}]henicosa-4,6,8-triene-20-carboxylate |
| **Ex.405** | 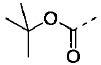 | 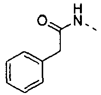 | *tert*-butyl (1*S*,15*S*,18*R*)-7-fluoro-11-methyl-10,16-dioxo-15-[(2-phenylacetyl)amino]-3-oxa-11,17,20-triazatricyclo[16.2.1.0^{4,9}]henicosa-4,6,8-triene-20-carboxylate |
| **Ex.406** | H | 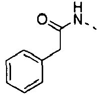 | *N*-[(1*S*,15*S*,18*R*)-7-fluoro-11-methyl-10,16-dioxo-3-oxa-11,17,20-triazatricyclo[16.2.1.0^{4,9}]henicosa-4,6,8-trien-15-yl]-2-phenylacetamide |
| **Ex.407** | 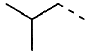 | 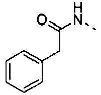 | *N*-[(1*S*,15*S*,18*R*)-7-fluoro-20-isobutyl-11-methyl-10,16-dioxo-3-oxa-11,17,20-triazatricyclo[16.2.1.0^{4,9}]henicosa-4,6,8-trien-15-yl]-2-phenylacetamide |
| **Ex.408** | 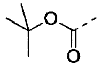 | 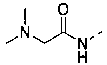 | *tert*-butyl (1*S*,15*S*,18*R*)-15-{[2-(dimethylamino)acetyl]amino}-7-fluoro-11-methyl-10,16-dioxo-3-oxa-11,17,20-triazatricyclo[16.2.1.0^{4,9}]henicosa-4,6,8-triene-20-carboxylate |
| **Ex.409** | H | 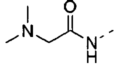 | 2-(dimethylamino)-*N*- [(1*S*,15*S*,18*R*)-7-fluoro-11-methyl-10,16-dioxo-3-oxa-11,17,20-triazatricyclo[16.2. 1.0^{4,9}]henicosa-4,6,8-trien-15-yl]acetamide |
| **Ex.410** | 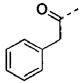 | 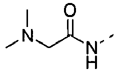 | 2-(dimethylamino)-*N*-[(1*S*,15*S*,18*R*)-7-fluoro-11-methyl-10,16-dioxo-20-(2-phenylacetyl)-3-oxa-11,17,20-triazatricyclo[16.2.1.0^{4,9}]henicosa-4,6,8-trien-15-yl]acetamide |
| **Ex.411** | 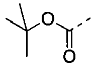 | 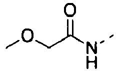 | *tert*-butyl (1*S*,15*S*,18*R*)-7-fluoro-15-[(2-methoxyacetyl)amino]-11-methyl-10,16-dioxo-3-oxa-11,17,20-triazatricyclo[16.2.1.0^{4,9}]henicosa-4,6,8-triene-20-carboxylate |
| **Ex.412** | H | 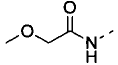 | *N*-[(1*S*,15*S*,18*R*)-7-fluoro-11-methyl-10,16-dioxo-3-oxa-11,17,20-triazatricyclo[16.2.1.0^{4,9}]henicosa-4,6,8-trien-15-yl]-2-methoxyacetamide |
| **Ex.413** | CH₃ | 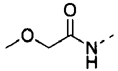 | *N*-[(1*S*,15*S*,18*R*)-7-fluoro-11,20-dimethyl-10,16-dioxo-3-oxa-11,17,20-triazatricyclo[16.2.1.0^{4,9}]henicosa-4,6,8-trien-15-yl]-2-methoxyacetamide |
| **Ex. 414** | 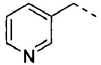 | 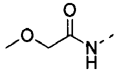 | *N*-[(1*S*,15*S*,18*R*)-7-fluoro-11-methyl-10,16-dioxo-20-(3-pyridinylmethyl)-3-oxa-11,17,20-triazatricyclo[16.2.1.0^{4,9}]henicosa-4,6,8-trien-15-yl]-2-methoxyacetamide |

**Table 42a: Examples of Core 26 (Ex.415-Ex.423; continued on the following page)**

| **No** | **R²** | **R⁵⁴** | **Starting material** | **General Procedure** | **Reagent** | **Purification Method** | **Yield (isolated salt)** |
|---|---|---|---|---|---|---|---|
| **Ex.415-Ex.416:** *cf. experimental description* | | | | | | | |
| **Ex.417** | 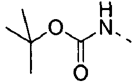 | 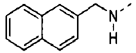 | **Ex.416** | L.2 | 2-naphthyl-methylamine (2 equiv.) | FC (hexane/EtOAc/ MeOH) | 96% |
| **Ex.418** | 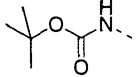 | 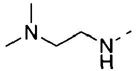 | **Ex.416** | L.2 | 2-dimethyl-aminoetylamine (2 equiv.) | FC (CH₂Cl₂/MeOH/ aq.NH₃) | 81% |
| **Ex.419** | NH₂ | 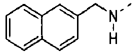 | **Ex.417** | J | HCl (4M dioxane) | crude product | 89% (HCl salt) |
| **Ex.420** | NH₂ | 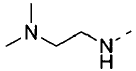 | **Ex.418** | J | HCl (4M dioxane) | crude product | 88% (HCl salt) |
| **Ex.421** | 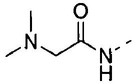 | 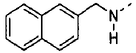 | **Ex.419** | L.1.2 | N,N-dimethylglycine (1.8 equiv.) | prep. HPLC, method 1 | 38% (TFA salt) |
| **Ex.422** | 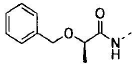 | 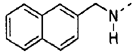 | **Ex.419** | L.1.3 | (R)(+)-2-benzyloxy-propionic acide (2 equiv.) | prep. HPLC, method 1 | 73% |
| **Ex.423** | 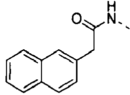 | 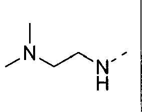 | **Ex.420** | L.1.2 | 2-naphthylacetic acide ((1.2 equiv.) | prep. HPLC, method 1 | 36% (TFA salt) |

**Table 42b: Examples of Core 26 (Ex.415-Ex.423; continued on the following page)**

| **No** | **R²** | **R⁵⁴** | **FORMULA** | **MONOISOTOPIC MASS** | **Rt (purity at 220nm)** | **[M+H]⁺ found** | **LC-MS-Method** |
|---|---|---|---|---|---|---|---|
| **Ex.415-Ex.416:** *cf. experimental description* | | | | | | | |
| **Ex.417** | 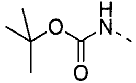 | 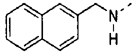 | C35H43N5O7S | 677.3 | 2.08 (89) | 678.2 | Method 4b |
| **Ex.418** | 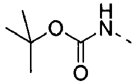 | 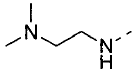 | C28H44N6O7S | 608.3 | 1.42 (96) | 609.2 | Method 4b |
| **Ex.419** | NH₂ | 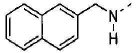 | C30H35N5O5S | 577.2 | 1.61 (84) | 578.0 | Method 4b |
| **Ex.420** | NH₂ | 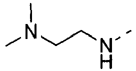 | C23H36N605S | 508.3 | 1.29 (95) | 509.2 | Method 5b |
| **Ex.421** | 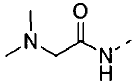 | 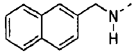 | C34H42N6O6S | 662.3 | 1.86 (91) | 663.2 | Method 5b |
| **x.422** | 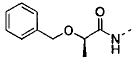 | 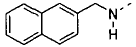 | C40H45N5O7S | 739.3 | 2.10 (97) | 740.2 | Method 5b |
| **Ex.423** | 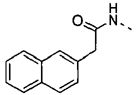 | 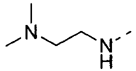 | C35H44N6O6S | 676.3 | 1.85 (97) | 677.2 | Method 5b |

**Table 42c: Examples of Core 26 (15-007-01) (Ex.415-Ex.423, continued on the following pages)**

| **No** | **R²** | **R⁵⁴** | IUPAC name |
|---|---|---|---|
| **Ex.415** | 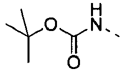 | 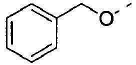 | benzyl (6*S*,15*R*,16a*S*)-15-[(*tert*-butoxycarbonyl)amino]-5,10-dimethyl-4,9,12-trioxo-5,6,7,8,9,10,11,12,15,16,16a,17-dodecahydro-4*H*,14*H*-pyrrolo[2,1-*c*]thieno[2,3-*n*][1,4,7,12]oxatriazacyclopentadecine-6-carboxylate |
| **Ex.416** | 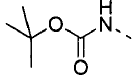 | OH | (6*S*,15*R*,16a*S*)-15-[(*tert*-butoxycarbonyl)amino]-5,10-dimethyl-4,9,12-trioxo-5,6,7,8,9,10,11,12,15,16,16a,17-dodecahydro-4*H*,14*H*-pyrrolo[2,1-*c*]thieno[2,3-*n*][1,4,7,12]oxatriazacyclopentadecine-6-carboxylic acid |
| **Ex.417** | 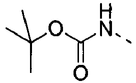 | 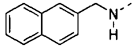 | *tert*-butyl *N*-((6*S*,15*R*,16a*S*)-5,10-dimethyl-6-{[(2-naphthylmethyl)amino]carbonyl}-4,9,12-trioxo-5,6,7,8,9,10,11,12,15,16,16a,17-dodecahydro-4*H*,14*H*-pyrrolo[2,1-*c*]thieno[2,3-*n*][1,4,7,12]oxatriazacyclopentadecin-15-yl)carbamate |
| **Ex.418** | 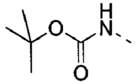 | 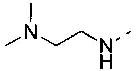 | *tert*-butyl *N*-[(6*S*,15*R*,16a*S*)-6-({[2-(dimethylamino)ethyl]amino}carbonyl)-5,10-dimethyl-4,9,12-trioxo-5,6,7,8,9,10,11,12,15,16,16a,17-dodecahydro-4*H*,14*H*-pyrrolo[2,1-*c*]thieno[2,3-*n*][1,4,7,12]oxatriazacyclopentadecin-15-yl]carbamate |
| **Ex.419** | NH₂ | 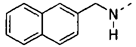 | (6*S*,15*R*,16a*S*)-15-amino-5,10-dimethyl-*N*-(2-naphthylmethyl)-4,9,12-trioxo-5,6,7,8,9,10,11,12,15,16,16a,17-dodecahydro-4*H*,14*H*-pyrrolo[2,1-*c*]thieno[2,3-*n*][1,4,7,12]oxatriazacyclopentadecine-6-carboxamide |
| **Ex.420** | NH₂ | 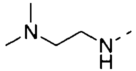 | (6*S*,15*R*,16a*S*)-15-amino-*N*-[2-(dimethylamino)ethyl]-5,10-dimethyl-4,9,12-trioxo-5,6,7,8,9,10,11,12,15,16,16a,17-dodecahydro-4*H*,14*H*-pyrrolo[2,1-*c*]thieno[2,3-*n*][1,4,7,12]oxatriazacyclopentadecine-6-carboxamide |
| **Ex.421** | 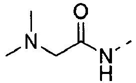 | 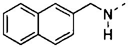 | (6*S*,15*R*,16a*S*)-15-{[2-(dimethylamino)acetyl]amino}-5,10-dimethyl-*N*-(2-naphthylmethyl)-4,9,12-trioxo-5,6,7,8,9,10,11,12,15,16,16a,17-dodecahydro-4*H*,14*H*-pyrrolo[2,1-*c*]thieno[2,3-*n*][1,4,7,12]oxatriazacyclopentadecine-6-carboxamide |
| **Ex.422** | 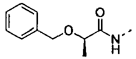 | 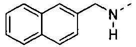 | (6*S*,15*R*,16a*S*)-15-{[(2*R*)-2-(benzyloxy)propanoyl]amino}-5,10-dimethyl-*N-*(2-naphthylmethyl)-4,9,12-trioxo-5,6,7,8,9,10,11,12,15,16,16a,17-dodecahydro-4*H*,14*H*-pyrrolo[2,1-*c*]thieno[2,3-*n*][1,4,7,12]oxatriazacyclopentadecine-6-carboxamide |
| **Ex.423** | 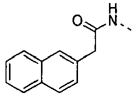 | 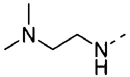 | (6*S*,15*R*,16a*S*)-*N*-[2-(dimethylamino)ethyl]-5,10-dimethyl-15-{[2-(2-naphthyl)acetyl]amino}-4,9,12-trioxo-5,6,7,8,9,10,11,12,15,16,16a,17-dodecahydro-4*H*,14*H*-pyrrolo[2,1-*c*]thieno[2,3-*n*][1,9,7,12]oxatriazacyclopentadecine-6-carboxamide |

**Table 43a: Examples of Core 27 (Ex.424-Ex.434 and Ex.592-Ex.593; continued on the following pages)**

| **No** | **R²** | **R⁵** | **Starting material** | **General Procedure** | **Reagent** | **Purification Method** | **Yield (isolated salt)** |
|---|---|---|---|---|---|---|---|
| **Ex.424-Ex.425:** *cf. experimental description* | | | | | | | |
| **Ex.426** | 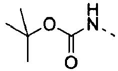 | 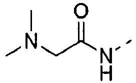 | **Ex.425** | L.1.3 | 2-dimethylamino carboxylic acid (2 equiv.) | prep. HPLC, Methode 3 | 75% |
| | | | | | i-Pr₂EtN (2 equiv.) | | |
| **Ex.427** | NH₂ | 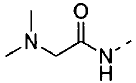 | **Ex.426** | J | HCl (4M dioxane) | crude product | 90% (HCl salt) |
| **Ex.428** | 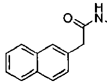 | 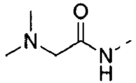 | **Ex.427** | | T3P (50% in EtOAc;1.2 quiv.) 2-naphthylacetic acid (1.2 equiv.) i-Pr₂EtN (4 equiv.), CH₂Cl₂, rt, 15 h | prep. HPLC, methode 1 | 35% (TFA salt) |
| **Ex.429** | 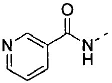 | 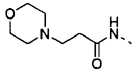 | **268** | S | i) nicotinic acid (2x5 equiv.) | prep. HPLC, methode1 | 25% (TFA salt) |
| | | | | | ii) 3-morpholino-propanoic acid·HCl (1x5 equiv.) | | |
| **Ex.430** | 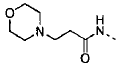 | 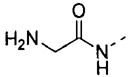 | **268** | S | i) 3-morpholino propanoic acid·HCl (2x5 equiv.) | prep. HPLC, methode 1 | 23% (TFA salt) |
| | | | | | ii) BOC-glycine (1x5 equiv.) | | |
| **Ex.431** | 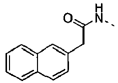 | 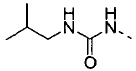 | **268** | S | i) 2-naphthylacetic acid (2x5 equiv.) | prep. HPLC, methode 1 | 37% (TFA salt) |
| | | | | | ii) N-succinimidyl-N-isobutylcarbamate (1 equiv.) | | |
| **Ex.432** | 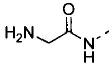 | 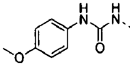 | **268** | S | i) BOC-glycine (2x5 equiv.) | prep. HPLC, methode 1 | 53% (TFA salt) |
| | | | | | ii) 4-methoxy-phenylisocyanate (2x5 equiv.) | | |
| **Ex.433** | 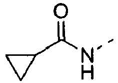 | 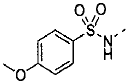 | **268** | S | i) cyclopropane carboxylic acid (2x5 equiv.) | Prep. HPLC, methode 3 | 42% (TFA salt) |
| | | | | | ii) 4-methoxy-benzylsulfonyl chloride (2x5 equiv.) | | |
| **Ex.434** | 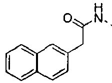 | 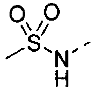 | **268** | S | i) 2-naphthylaceti acid (2x5 equiv.) | Prep. HPLC, Methode 3 | 48% (TFA salt) |
| | | | | | ii) methanesulfonyl chloride (2x5 equiv.) | | |
| **Ex.592** | 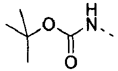 | 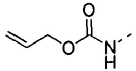 | **Ex.424** | *) | 1.H2, Pd(OH)₂-C | FC (CH₂Cl₂/MeOH 95:5) | 85% |
| | | | | | 2. AllocCl | | |
| **Ex.593** | NH₂ | 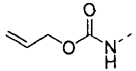 | **Ex.592** | *) | 1. TFA, CH₂Cl₂ | crude product | 87% (HCl salt) |
| | | | | | 2. HCl-dioxane | | |

| | | | | | | | |
|---|---|---|---|---|---|---|---|
| *) cf. detailed description | | | | | | | |

**Table 43b: Examples of Core 27 (Ex.424-Ex.434 and Ex.592-Ex.593; continued on the following page)**

| **No** | **R²** | **R⁵** | **FORMULA** | **MONOISOTOPIC MASS** | **Rt (purity at 220nm)** | **[M+H]⁺ found** | **LC-MS-Method** |
|---|---|---|---|---|---|---|---|
| **Ex.424-Ex.425:** *cf. experimental description* | | | | | | | |
| **Ex.426** | 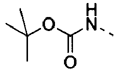 | 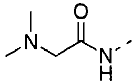 | C27H42N6O6 | 546.3 | 1.35 | 547.4 | Method 4a |
| **Ex.427** | NH₂ | 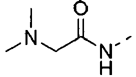 | C22H34N6O4 | 446.3 | 1.23 | 447.3 | Method 5a |
| **Ex.428** | 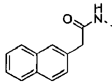 | 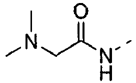 | C34H42N6O5 | 614.3 | 1.32 (95) | 615.3 | Method 10a |
| **Ex.429** | 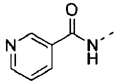 | 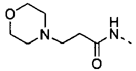 | C31H41N7O6 | 607.3 | 0.78 (96) | 608.3 | Method 10a |
| **Ex.430** | 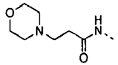 | 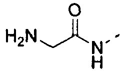 | C27H41N7O6 | 559.3 | 0.61 (91) | 560.2 | Method 10a |
| **Ex.431** | 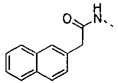 | 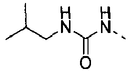 | C35H44N6O5 | 628.3 | 1.63 (95) | 629.3 | Method 10a |
| **Ex.432** | 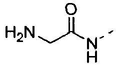 | 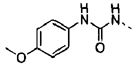 | C28H37N7O6 | 567.3 | 1.09 (98) | 568.2 | Method 10a |
| **Ex.433** | 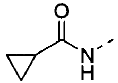 | 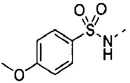 | C29H37N5O7S | 599.2 | 1.38 (96) | 600.2 | Method 10a |
| **Ex.434** | 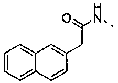 | 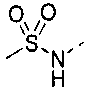 | C31H37N5O6S | 607.3 | 1.49 (90) | 608.2 | Method 10a |
| **Ex.592** | 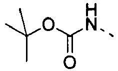 | 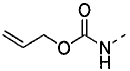 | C27H39N5O7 | 545.2 | 1.56 (96) | 546.2 | Method 10a |
| **Ex.593** | NH₂ | 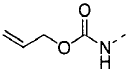 | C22H31N5O5 | 445.2 | 0.98 (96) | 446.1 | Method 10a |

**Table 43c: Examples of Core 27 (Ex.424-Ex.434 and Ex.592-Ex.593; continued on the following page)**

| **No** | **R²** | **R⁵** | **IUPAC name** |
|---|---|---|---|
| **Ex.424** | 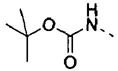 | 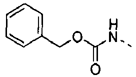 | benzyl *N*-[(A*S*,6*S*,10*S*)-6-[(*tert*-butoxycarbonyl)amino]-15-methyl-9,16-dioxo-2-oxa-8,15,19-triazatricyclo[15.3.1.0^{4,8}]henicosa-1(21),17,19-trien-10-yl]carbamate |
| **Ex.425** | 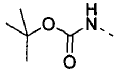 | NH₂ | *tert*-butyl *N*-[(4*S*,6*S*,10*S*)-10-amino-15-methyl-9,16-dioxo-2-oxa-8,15,19-triazatricyclo[15.3.1.0^{4,8}]henicosa-1(21),17,19-trien-6-yl]carbamate |
| **Ex.426** | 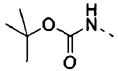 | 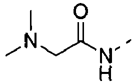 | *tert*-butyl *N*-[(4*S*,6*S*,10*S*)-10-{[2-(dimethylamino)acetyl]amino}-15-methyl-9,16-dioxo-2-oxa-8,15,19-triazatricyclo[15.3.1.0^{4,8}]henicosa-1(21),17,19-trien-6-yl]carbamate |
| **Ex.427** | NH₂ | 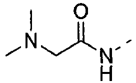 | *N*-[(4*S*,6*S*,10*S*)-6-amino-15-methyl-9,16-dioxo-2-oxa-8,15,19-triazatricyclo[15.3.1.0^{4,8}]henicosa-1(21),17,19-trien-10-yl]-2-(dimethylamino)acetamide |
| **Ex.428** | 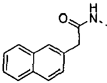 | 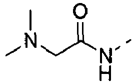 | 2-(dimethylamino)-*N*-[(4*S*,6*S*,10*S*)-15-methyl-6-{[2-(2-naphthyl)acetyl]amino}-9,16-dioxo-2-oxa-8,15,19-triazatricyclo[15.3.1.0^{4,8}]henicosa-1(21),17,19-trien-10-yl]acetamide |
| **Ex.429** | 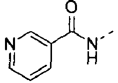 | 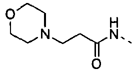 | *N*-[(4*S*,6*S*,10*S*)-15-methyl-10-[(3-morpholinopropanoyl)amino]-9,16-dioxo-2-oxa-8,15,19-triazatricyclo[15.3.1.0^{4,8}]henicosa-1(21),17,19-trien-6-yl]nicotinamide |
| **Ex.430** | 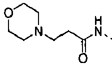 | 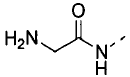 | *N*-[(4*S*,6*S*,10*S*)-10-[(2-aminoacetyl)amino]-15-methyl-9,16-dioxo-2-oxa-8,15,19-triazatricyclo[15.3.1.0^{4,8}]henicosa-1(21),17,19-trien-6-yl]-3-morpholinopropanamide |
| **Ex.431** | 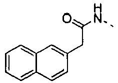 | 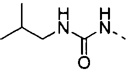 | *N*-[(4*S*,6*S*,10*S*)-10-{[(isobutylamino)carbonyl]amino}-15-methyl-9,16-dioxo-2-oxa-8,15,19-triazatricyclo[15.3.1.0^{4,8}]henicosa-1(21),17,19-trien-6-yl]-2-(2-naphthyl)acetamide |
| **Ex.432** | 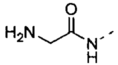 | 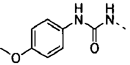 | 2-amino-N-[(4*S*,6*S*,10*S*)-10-{[(4-methoxyanilino)carbonyl]amino}-15-methyl-9,16-dioxo-2-oxa-8,15,19-triazatricyclo[15.3.1.0^{4,8}]henicosa-1(21),17,19-trien-6-yl]acetamide |
| **Ex.433** | 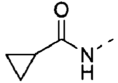 | 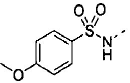 | *N*-[(4*S*,6*S*,10*S*)-10-{[(4-methoxyphenyl)sulfonyl]amino}-15-methyl-9,16-dioxo-2-oxa-8,15,19-triazatricyclo[15.3.1.0^{4,8}]henicosa-1(21),17,19-trien-6-yl]cyclopropanecarboxamide |
| **Ex.434** | 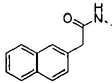 | 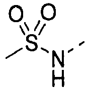 | *N*-[(4*S*,6*S*,10*S*)-15-methyl-10-[(methylsulfonyl)amino]-9,16-dioxo-2-oxa-8,15,19-triazatricyclo[15.3.1.0^{4,8}]henicosa-1(21),17,19-trien-6-yl]-2-(2-naphthyl)acetamide |
| **Ex.592** | 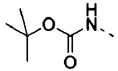 | 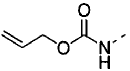 | allyl *N*-[(4*S*,6*S*,10*S*)-6-[(tert-butoxycarbonyl)amino]-15-methyl-9,16-dioxo-2-oxa-8,15,19-triazatricyclo[15.3.1.0^{4,8}]henicosa-1(21),17,19-trien-10-yl]carbamate |
| **Ex.593** | NH₂ | 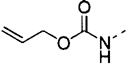 | allyl *N*-[(4*S*,6*S*,10*S*)-6-amino-15-methyl-9,16-dioxo-2-oxa-8,15,19-triazatricyclo[15.3.1.0^{4,8}]henicosa-1(21),17,19-trien-10-yl]carbamate |

**Table 44a: Examples of Core 28 (Ex.435-Ex.446; continued on the following page)**

| **No** | **R²** | **R⁵⁴** | **Starting material** | **General Procedure** | **Reagent** | **Purification Method** | **Yield salt)** |
|---|---|---|---|---|---|---|---|
| **Ex.435-Ex.437:** *cf. experimental description* | | | | | | | |
| **Ex.438** | 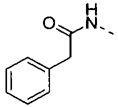 | 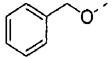 | **Ex.437** | L.1.1 | Phenylacetylchloride (1.1 equiv.), i-Pr₂NEt (3 equiv.) | FC (EtOAc/MeOH) | 75% |
| **Ex.439** | 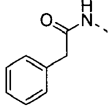 | OH | **Ex.438** | H | H₂, Pd(OH)₂-C | crude product | 99% |
| **Ex.440** | 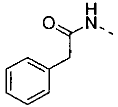 | 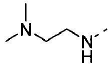 | **Ex.439** | L.2 | 2-dimethylaminoethylamine | FC (CH₂Cl₂/MeOH/N H₄OH)) | 90% |
| **Ex.441** | 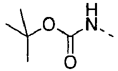 | 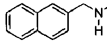 | **Ex.436** | L.2 | HATU (2.6 equiv.) HOAt (2.6 equiv.) i-Pr₂NEt (5 equiv.) 2-naphthyl-methylamine (2.6 equiv.) | Prep. HPLC, method 3 + size exclusion chromatography | 64% |

| **No** | **R²** | **R⁵⁴** | **Starting material** | **General Procedure** | **Reagent** | **Purification Method** | **Yield (isolated salt)** |
|---|---|---|---|---|---|---|---|
| **Ex.442** | NH₂ | 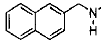 | **Ex.441** | *) | TFA, triisopropylsilane *) | crude product | 72% |
| **Ex.443** | 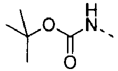 | 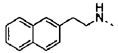 | **Ex.436** | L.2 | HATU (2.6 equiv.) HOAt (2.6 equiv.) i-Pr₂NEt (8 equiv.) 2-Naphthyl-ethylamine hydrochloride | Prep. HPLC, method 3 + size exclusion chromatography | 86% |
| **Ex.444** | NH₂ | 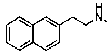 | **Ex.443** | *) | TFA, triisopropylsilane *) | prep. HPLC, method 1 | 43% (TFA salt) |
| **Ex.445** | 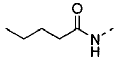 | 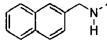 | **Ex.442** | L.1.1 | Valeroylchloride (4 equiv.) | prep. HPLC, method 3 | 72% |
| **Ex.446** | 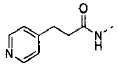 | 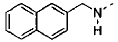 | **Ex.442** | L.1.2 | 3-(pyridin-4-yl)propanoic acid (1.8 equiv.) | prep. HPLC, method 3 | 90% |

| | | | | | | | |
|---|---|---|---|---|---|---|---|
| *) A soln of the Boc amine (0.04 mmol) in CH₂Cl₂ (1 mL) was treated at 0°C with triisopropylsilane (3 equiv.) and TFA (0.2 mL). The mixture was stirred for 2.5 h, treated with sat. aq. Na₂CO₃ soln and extracted with CH₂Cl₂. The organic phase was dried (Na₂SO₄), filtered and concentrated to afford the corresponding amine. | | | | | | | |

**Table 44b: Examples of Core 28 (Ex.435-Ex.446; continued on the following page)**

| **No** | **R²** | **R⁵⁴** | **FORMULA** | **MONOISOTOPIC MASS** | **Rt (purity at 220nm)** | **[M+H]⁺ found** | **LC-MS-Method** |
|---|---|---|---|---|---|---|---|
| **Ex.435-Ex.437:** *cf. experimental description* | | | | | | | |
| **Ex.438** | 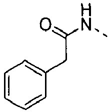 | 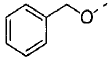 | C40H45N5O7 | 707.3 | 1.93 (97) | 708.4 | Method 10a |
| **Ex.439** | 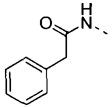 | OH | C33H39N5O7 | 617.3 | 1.50 (97) | 618.3 | Method 10a |
| **Ex.440** | 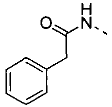 | 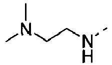 | C37H49N7O6 | 687.4 | 1.38 (98) | 688.4 | Method 10a |
| **Ex.441** | 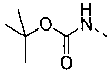 | 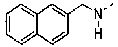 | C41H50N6O7 | 738.4 | 2.08 (98) | 739.1 | Method 10a |
| **Ex.442** | NH₂ | 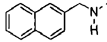 | C36H42N6O5 | 638.3 | 1.50 (94) | 639.2 | Method 10a |
| **Ex.443** | 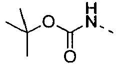 | 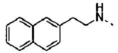 | C42H52N6O7 | 752.4 | 2.12 (96) | 753.1 | Method 10a |
| **Ex.444** | NH₂ | 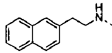 | C37H44N6O5 | 652.3 | 1.57 (86) | 653.3 | Methode 10a |
| **Ex.445** | 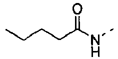 | 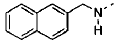 | C41H50N6O6 | 722.4 | 2.11 (99) | 723.3 | Method 4b |
| **Ex.446** | 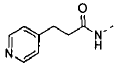 | 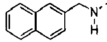 | C44H49N7O6 | 771.4 | 1.73 (99) | 772.3 | Method 4b |

**Table 44c: Examples of Core 28 (Ex.435-Ex.446; continued on the following pages)**

| **No** | **R²** | **R⁵⁴** | **IUPAC name** |
|---|---|---|---|
| **Ex.435** | 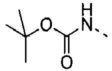 | 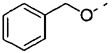 | benzyl (4*S*,6*R*,15*S*)-6-[(*tert*-butoxycarbonyl)amino]-11,16,19,20-tetramethyl-9,12,17-trioxo-2-oxa-8,11,16,20-tetraazatetracyclo[16.5.2.0^{4,8}.0^{21,25}]pentacosa-1(23),18,21,24-tetraene-15-carboxylate |
| **Ex.436** | 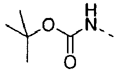 | OH | (4*S*,6*R*,15*S*)-6-[(*tert*-butoxycarbonyl)amino]-11,16,19,20-tetramethyl-9,12,17-trioxo-2-oxa-8,11,16,20-tetraazatetracyclo[16.5.2.0^{4,8}.0^{21,25}]pentacosa-1(23),18,21,24-tetraene-15-carboxylic acid |
| **Ex.437** | NH₂ | 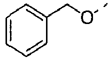 | benzyl (4*S*,6*R*,15*S*)-6-amino-11,16,19,20-tetramethyl-9,12,17-trioxo-2-oxa-8,11,16,20-tetraazatetracyclo[16.5.2.0^{4,8}.0^{21,25}]pentacosa-1(23),18,21,24-tetraene-15-carboxylate |
| **Ex.438** | 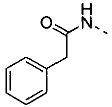 | 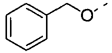 | benzyl (4*S*,6*R*,15*S*)-11,16,19,20-tetramethyl-9,12,17-trioxo-6-[(2-phenylacetyl)amino]-2-oxa-8,11,16,20-tetraazatetracyclo[16.5.2.0^{4,8}.0^{21,25}]pentacosa-1(23),18,21,24-tetraene-15-carboxylate |
| **Ex.439** | 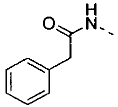 | OH | (4*S*,6*R*,15*S*)-11,16,19,20-tetramethyl-9,12,17-trioxo-6-[(2-phenylacetyl)amino]-2-oxa-8,11,16,20-tetraazatetracyclo[16.5.2.0^{4,8}.0^{21,25}]pentacosa-1(23),18,21,24-tetraene-15-carboxylic acid |
| **Ex.440** | 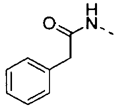 | 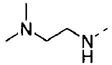 | (4*S*,6*R*,15*S*)-*N*-[2-(dimethylamino)ethyl]-11,16,19,20-tetramethyl-9,12,17-trioxo-6-[(2-phenylacetyl)amino]-2-oxa-8,11,16,20-tetraazatetracyclo[16.5.2.0^{4,8}.0^{21,25}]pentacosa-1(23),18,21,24-tetraene-15-carboxamide |
| **Ex.441** | 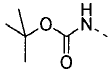 | 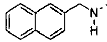 | *tert*-butyl *N*-[(4*S*,6*R*,15*S*)-11,16,19,20-tetramethyl-15-{[(2-naphthylmethyl)amino]carbonyl}-9,12,17-trioxo-2-oxa-8,11,16,20-tetraazatetracyclo[16.5.2.0^{4,8.}0^{21,25}]pentacosa-1(23),18,21,24-tetraen-6-yl]carbamate |
| **Ex.442** | NH₂ | 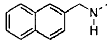 | (4*S*,6*R*,15*S*)-6-amino-11,16,19,20-tetramethyl-N-(2-naphthylmethyl)-9,12,17-trioxo-2-oxa-8,11,16,20-tetraazatetracyclo[16.5.2.0^{4,8}.0^{21,25}]pentacosa-1(23),18,21,24-tetraene-15-carboxamide |
| **Ex.443** | 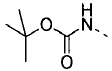 | 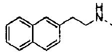 | *tert*-butyl *N*-[(4*S*,6*R*,15*S*)-11,16,19,20-tetramethyl-15-({[2-(2-naphthyl)ethyl]amino}carbonyl)-9,12,17-trioxo-2-oxa-8,11,16,20-tetraazatetracyclo[16.5.2.0^{4,8}.0^{21,25}]pentacosa-1(23),18,21,24-tetraen-6-yl]carbamate |
| **Ex.444** | NH₂ | 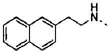 | (4*S*,6*R*,15*S*)-6-amino-11,16,19,20-tetramethyl-N-[2-(2-naphthyl)ethyl]-9,12,17-trioxo-2-oxa-8,11,16,20-tetraazatetracyclo[16.5.2.0^{4,8}.0^{21,25}]pentacosa-1(23),18,21,24-tetraene-15-carboxamide |
| **Ex.445** | 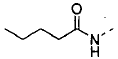 | 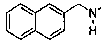 | (4*S*,6*R*,15*S*)-11,16,19,20-tetramethyl-*N*-(2-naphthylmethyl)-9,12,17-trioxo-6-(pentanoylamino)-2-oxa-8,11,16,20-tetraazatetracyclo[16.5.2.0^{4,8}.0^{21,25}]pentacosa-1(23),18,21,24-tetraene-15-carboxamide |
| **Ex.446** | 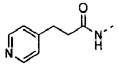 | 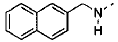 | (4S,6R,15S)-11,16,19,20-tetramethyl-N-(2-naphthylmethyl)-9,12,17-trioxo-6-{[3-(4-pyridinyl)propanoyl]amino}-2-oxa-8,11,16,20-tetraazatetracyclo[16.5.2.0^{4,8}.0^{21,25}]pentacosa-1(23),18,21,24-tetraene-15-carboxamide |

**Table 45a: Examples of Core 29 (Ex.447-Ex.450)**

| **No** | **R²** | **R⁵** | **Starting material** | **General Procedure** | **Reagent** | **Purification Method** | **Yield (isolated salt)** |
|---|---|---|---|---|---|---|---|
| **Ex.447-Ex.448:** *cf. experimental description* | | | | | | | |
| **Ex.449** | OH | 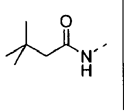 | **Ex.448** | L.1.1 | 3,3-dimethylbutanoyl chloride (4.1 equiv.) pyridine (19 equiv.) | FC (CH₂Cl₂/MeOH) | 72% |
| **Ex.450** | OH | 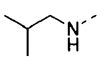 | **Ex.448** | M.2 | isobutyraldehyde (1.1 equiv.) NaBH(OAc)₃ (2.5 equiv.) | prep. HPLC method 1 | 9% (TFA salt) |

**Table 45b: Examples of Core 29 (Ex.447-Ex.450)**

| **No** | **R²** | **R⁵** | **FORMULA** | **MONOISOTOPIC MASS** | **Rt (purity at 220nm)** | **[M+H]⁺ found** | **LC-MS-Method** |
|---|---|---|---|---|---|---|---|
| **Ex.447-Ex.448:** *cf. experimental description* | | | | | | | |
| **Ex.449** | OH | 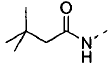 | C24H36N2O4 | 416.3 | 2.01 (91) | 417.3 | Method 10a |
| **Ex.450** | OH | 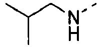 | C22H34N2O3 | 374.3 | 1.41 (98) | 375.2 | Method 10a |

**Table 45c: Examples of Core 29 (Ex.447-Ex.450)**

| **No** | **R²** | **R⁵** | **IUPAC name** |
|---|---|---|---|
| **Ex.447** | 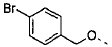 | 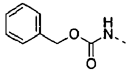 | 1:1 mixture of the 12-(*E*)- and 12-*(Z)*-stereoisomer of benzyl *N*-[(4*S*,6*S*,10*S*)-6-[(4-bromobenzyl)oxy]-9-oxo-2-oxa-8-azatricyclo[14.3.1.0^{4,8}]icosa-1(20),12,16,18-tetraen-10-yl]carbamate |
| **Ex.448** | OH | NH₂ | (4*S*,6*S*,10*S*)-10-amino-6-hydroxy-2-oxa-8-azatricyclo[14.3.1.0^{4,8}]icosa-1(20),16,18-trien-9-one |
| **Ex.449** | OH | 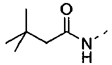 | *N*-[(4*S*,6*S*,10*S*)-6-hydroxy-9-oxo-2-oxa-8-azatricyclo[14.3.1.0^{4,8}]icosa-1(20),16,18-trien-10-yl]-3,3-dimethylbutanamide |
| **Ex.450** | OH | 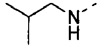 | (4*S*,6*S*,10*S*)-6-hydroxy-10-(isobutylamino)-2-oxa-8-azatricyclo[14.3.1.0^{4,8}]icosa-1(20),16,18-trien-9-one |

**Table 46a: Examples of Core 30 (Ex.451-Ex.459; continued on the following page)**

| **No** | **X** | **R²** | **R⁵** | **Starting material** | **General Procedure** | **Reagent** | **Purification Method** | **Yield (isolated salt)** |
|---|---|---|---|---|---|---|---|---|
| **Ex.451-Ex.452** *cf. experimental description* | | | | | | | | |
| **Ex.453** | SO₂ | 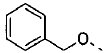 | NH₂ | **Ex.452** | K.2 | H₂, 5%Pd-C NH₃-MeOH cf. detailed description | crude product | 88% |
| **Ex.454** | SO₂ | HO | NH₂ | **Ex.452** | K.1 | H₂, Pd(OH)₂-C MeOH/AcOH 1:1 | FC (CH₂Cl₂/ MeOH/aq.NH₃) | 78% |
| **Ex.455** | SO₂ | 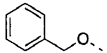 | 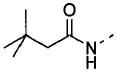 | **Ex.453** | L.1.1 | 3,3-dimethylbutanoyl chloride (1.2 equiv.) pyridine (5 equiv.) | prep. HPLC method 3 | 83% |
| **Ex.456** | SO₂ | 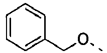 | 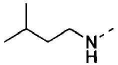 | **Ex.453** | M.2 | isovaleraldehyde | prep. HPLC method 3 | 77% |
| **Ex.457** | SO₂ | HO | 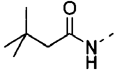 | **Ex.455** | H | H₂, Pd(OH)₂-C MeOH/AcOH 4:1 | crude product | 86% |
| **Ex.458** | SO₂ | HO | 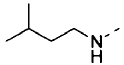 | **Ex.456** | H | H₂, Pd(OH)₂-C MeOH/AcOH 4:1 | prep. HPLC method 3 | 19% |
| **Ex.459*⁾** | SO₂ | HO | 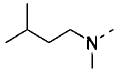 | **Ex.456** | H | H₂, Pd(OH)₂-C MeOH/AcOH 4:1 | prep. HPLC method 3 | 17%*⁾ |

| | | | | | | | | |
|---|---|---|---|---|---|---|---|---|
| *⁾ obtained as a side product | | | | | | | | |

**Table 46b: Examples of Core 30 (Ex.451-Ex.459; continued on the following page)**

| **No** | **X** | **R²** | **R⁵** | **FORMULA** | **MONOISOTOPIC MASS** | **Rt (purity at 220nm)** | **[M+H]⁺ found** | **LC-MS-Method** |
|---|---|---|---|---|---|---|---|---|
| **Ex.451-Ex.452:** *cf. experimental description* | | | | | | | | |
| **Ex.453** | SO₂ | 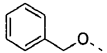 | NH₂ | C25H31N3O5S | 485.2 | 1.29 (98) | 486.2 | Method 10a |
| **Ex.454** | SO₂ | HO | NH₂ | C18H25N3O5S | 395.2 | 0.70 (100) | 396.1 | Method 11a |
| **Ex.455** | SO₂ | 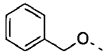 | 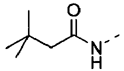 | C31H41N3O6S | 583.3 | 1.90 (95) | 584.3 | Method 10a |
| **Ex.456** | SO₂ | 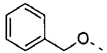 | 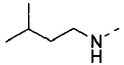 | C3OH41N3O5S | 555.3 | 1.57 (99) | 556.2 | Method 10a |
| **Ex.457** | SO₂ | HO | 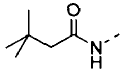 | C24H35N3O6S | 493.2 | 1.27 (95) | 494.2 | Method 10a |
| **Ex.458** | SO₂ | HO | 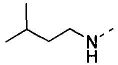 | C23H35N3O5S | 465.2 | 1.03 (95) | 466.2 | Method 10a |
| **Ex.459** | SO₂ | HO | 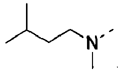 | C24H37N3O5S | 479.2 | 1.03 (94) | 480.2 | Method 10a |

**Table 46c: Examples of Core 30 (Ex.451-Ex.459; continued on the following page)**

| **No** | **X** | **R²** | **R⁵** | **IUPAC name** |
|---|---|---|---|---|
| **Ex.451** | S | 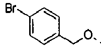 | 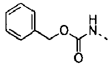 | benzyl *N*-[(4*S*,6*S*,10*S*)-6-[(4-bromobenzyl)oxyl-14-methyl-9,15-dioxo -2-thia-8,14-diazatricyclo[14.3.1.0^{4,8}]icosa-1(20),16,18-trien-10-yl]carbamate |
| **Ex.452** | SO₂ | 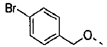 | 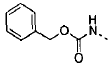 | benzyl *N*-[(4*S*,6*S*,10*S*)-6-[(4-bromobenzyl)oxy]-14-methyl-2,2,9,15-tetraoxo-2λ⁶-thia-8,14-diazatricyclo[14.3.1.0^{4,8}]icosa-1(20),16,18-trien-10-yl]carbamate |
| **Ex.453** | SO₂ | 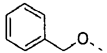 | NH₂ | (4*S*,6*S*,10*S*)-10-amino-6-(benzyloxy)-14-methyl-2λ⁶-thia-8,14-diazatricyclo[14.3.1.0^{4,8}]icosa-1(20),16,18-triene-2,2,9,15-tetrone |
| **Ex.454** | SO₂ | HO | NH₂ | (4*S*,6*S*,10*S*)-10-amino-6-hydroxy-14-methyl-2λ⁶-thia-8,14-diazatricyclo[14.3.1.0^{4,8}]icosa-1(20),16,18-triene-2,2,9,15-tetrone |
| **Ex.455** | SO₂ | 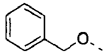 | 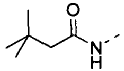 | *N*-[(4*S*,6*S*,10*S*)-6-(benzyloxy)-14-methyl-2,2,9,15-tetraoxo-2λ⁶-thia-8,14-diazatricyclo[14.3.1.0^{4,8}]icosa-1(20),16,18-trien-10-yl]-3,3-dimethylbutanamide |
| **Ex.456** | SO₂ | 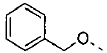 | 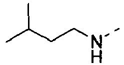 | (4*S*,6*S*,10*S*)-6-(benzyloxy)-10-(isopentylamino)-14-methyl-2λ⁶-thia-8,14-diazatricyclo[14.3.1.0^{4,8}]icosa-1(20),16,18-triene-2,2,9,15-tetrone |
| **Ex.457** | SO₂ | HO | 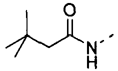 | N-[(4*S*,6*S*,10*S*)-6-hydroxy-14-methyl-2,2,9,15-tetraoxo-2λ⁶-thia-8,14-diazatricyclo[14.3.1.0^{4,8}]icosa-1(20),16,18-trien-10-yl]-3,3-dimethylbutanamide |
| **Ex.458** | SO₂ | HO | 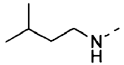 | (4*S*,6*S*,10*S*)-6-hydroxy-10-(isopentylamino)-14-methyl-2λ⁶-thia-8,14-diazatricyclo[14.3.1.0^{4,8}]icosa-1(20),16,18-triene-2,2,9,15-tetrone |
| **Ex.459** | SO₂ | HO | 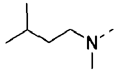 | (4*S*,6*S*,10*S*)-6-hydroxy-10-[isopentyl(methyl)amino]-14-methyl-2λ⁶-thia-8,14-diazatricyclo[14.3.1.0^{4,8}]icosa-1(20),16,18-triene-2,2,9,15-tetrone |

**Table 47a: Examples of Core 31 (Ex.460-Ex.469; continued on the following page)**

| **No** | **L** | **R²** | **R⁵** | **Starting material** | **General Procedure** | **Reagent** | **Purification Method** | **Yield (isolated salt)** |
|---|---|---|---|---|---|---|---|---|
| **Ex.460-Ex.461:** *cf. experimental description* | | | | | | | | |
| **Ex.462** | SO₂ | 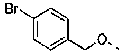 | NH₂ | **Ex.461** | J | HCl-dioxane | crude product | 87% (HCl salt) |
| **Ex.463** | SO₂ | 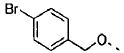 | 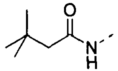 | **Ex.462** | L.1.1 | 3,3-dimethylbutanoyl chloride (1.5 equiv.) pyridine (5 equiv.) | FC (hexane/EtOAc) | 76% |
| **Ex.464** | SO₂ | 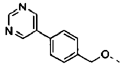 | 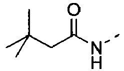 | **Ex.463** | P | pyrimidin-5-yl boronic acid | FC (hexane/EtOAc/ MeOH) and FC (CH₂Cl₂/MeOH) | 67% |
| **Ex.465** | SO₂ | OH | 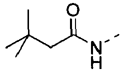 | **Ex.463** | H | H₂, Pd(OH)₂-C | FC (CH₂Cl₂/MeOH/ aq. NH₃) | 98% |
| **Ex.466** | S | 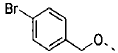 | NH₂ | **Ex.460** | J | HCl-dioxane | crude product | 90% (HCl salt) |
| **Ex.467** | S | 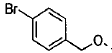 | 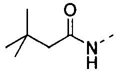 | **Ex.466** | L.1.1 | 3,3-dimethylbutanoyl chloride (1.5 equiv.) pyridine (5 equiv.) | FC (hexane/EtOAc) | 83% |
| **Ex.468** | S | 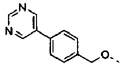 | 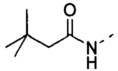 | **Ex.467** | P | pyrimidin-5-yl boronic acid | FC (hexane/EtOAc/ MeOH) | 72% |
| **Ex.469** | S | 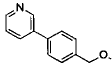 | 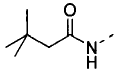 | **Ex.467** | P | pyridin-3-yl boronic acid | FC (hexane/EtOAc/ MeOH) | 66% |

**Table 47b: Examples of Core 31 (Ex.460-Ex.469; continued on the following page)**

| **No** | **L** | **R²** | **R⁵** | **FORMULA** | **MONOISOTOPIC MASS** | **Rt (purity at 220nm)** | **[M+H]⁺ found** | **LC-MS-Method** |
|---|---|---|---|---|---|---|---|---|
| **Ex.460-Ex.461:** *cf. experimental description* | | | | | | | | |
| **Ex.462** | SO₂ | 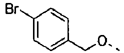 | NH₂ | C24H29BrN2O5S | 536.1 | 1.63 (96) | 539.1/537.1 | Method 10a |
| **Ex.463** | SO₂ | 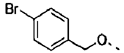 | 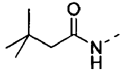 | C30H39BrN2O6S | 634.1 | 2.33 (99) | 635.1/637.1 | Method 10a |
| **Ex.464** | SO₂ | 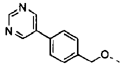 | 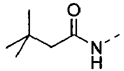 | C34H42N4O6S | 634.3 | 1.89 (93) | 635.2 | Method 10a |
| **Ex.465** | SO₂ | OH | 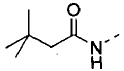 | C23H34N2O6S | 466.2 | 1.47 (97) | 467.2 | Method 10a |
| **Ex.466** | S | 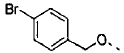 | NH₂ | C24H29BrN2O3S | 504.1 | 1.89 (93) | 507.1/505.1 | Method 10a |
| **Ex.467** | S | 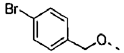 | 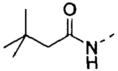 | C30H39BrN2O4S | 602.2 | 2.75 (96) | 605.1/603.1 | Method 10a |
| **Ex.468** | S | 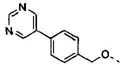 | 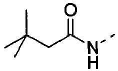 | C34H42N4O4S | 602.3 | 2.33 (90) | 603.3 | Method 10a |
| **Ex.469** | S | 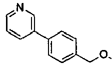 | 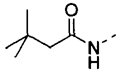 | C35H43N3O4S | 601.3 | 1.92 (98) | 602.3 | Method 10a |

**Table 47c: Examples of Core 31 (Ex.460-Ex.469; continued on the following page)**

| **No** | **L** | **R²** | **R⁵** | **IUPAC name** |
|---|---|---|---|---|
| **Ex.460** | S | 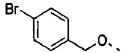 | 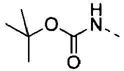 | *tert*-butyl *N*-[(4*S*,6*S*,10*S*)-6-[(4-bromobenzyl)oxy]-9-oxo-2-oxa-15-thia-8-azatricyclo[14.3.1.0^{4,8}]icosa-1(20),16,18-trien-10-yl]carbamate |
| **Ex.461** | SO₂ | 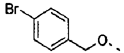 | 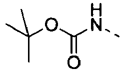 | *tert*-butyl *N*-[(4*S*,6*S*,10*S*)-6-[(4-bromobenzyl)oxy]-9,15,15-trioxo-2-oxa-15λ⁶-thia-8-azatricyclo[14.3.1.0^{4,8}]icosa-1(20),16,18-trien-10-yl]carbamate |
| **Ex.462** | SO₂ | 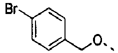 | NH₂ | (4*S*,6*S*,10*S*)-10-amino-6-[(4-bromobenzyl)oxy]-2-oxa-15λ⁶-thia-8-azatricyclo[14.3.1.0^{4,8}]icosa-1(20),16,18-triene-9,15,15-trione |
| **Ex.463** | SO₂ | 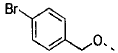 | 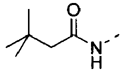 | *N*-[(4*S*,6*S*,10*S*)-6-[(4-bromobenzyl)oxy]-9,15,15-trioxo-2-oxa-15λ⁶-thia-8-azatricyclo[14.3.1.0^{4,8}]icosa-1(20),16,18-trien-10-yl]-3,3-dimethylbutanamide |
| **Ex.464** | SO₂ | 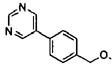 | 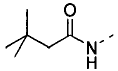 | 3,3-dimethyl-*N*-[(4*S*,6*S*,10*S*)-9,15,15-trioxo-6-{[4-(5-pyrimidinyl)benzyl]oxy}-2-oxa-15λ⁶-thia-8-azatricyclo[14.3.1.0^{4,8}]icosa-1(20),16,18-trien-10-yl]butanamide |
| **Ex.465** | SO₂ | OH | 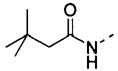 | *N*-[(4*S*,6*S*,10*S*)-6-hydroxy-9,15,15-trioxo-2-oxa-15λ⁶-thia-8-azatricyclo[14.3.1.0^{4,8}]icosa-1(20),16,18-trien-10-yl]-3,3-dimethylbutanamide |
| **Ex.466** | S | 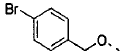 | NH₂ | (4*S*,6*S*,10*S*)-10-amino-6-[(4-bromobenzyl)oxy]-2-oxa-15-thia-8-azatricyclo[14.3.1.0^{4,8}]icosa-1(20),16,18-trien-9-one |
| **Ex.467** | S | 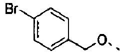 | 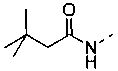 | *N*-[(4*S*,6*S*,10*S*)-6-[(4-bromobenzyl)oxy]-9-oxo-2-oxa-15-thia-8-azatricyclo[14.3.1.0^{4,8}]icosa-1(20),16,18-trien-10-yl]-3,3-dimethylbutanamide |
| **Ex.468** | S | 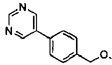 | 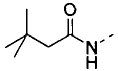 | 3,3-dimethyl-*N*-[(4*S*,6*S*,10*S*)-9-oxo-6-{[4-(5-pyrimidinyl)benzyl]oxy}-2-oxa-15-thia-8-azatricyclo[14.3.1.0^{4,8}]icosa-1(20),16,18-trien-10-yl]butanamide |
| **Ex.469** | S | 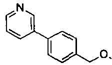 | 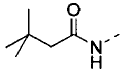 | 3,3-dimethyl-*N*-[(4*S*,6*S*,10*S*)-9-oxo-6-{[4-(3-pyridinyl)benzyl]oxy}-2-oxa-15-thia-8-azatricyclo[14.3.1.0^{4,8}]icosa-1(20),16,18-trien-10-yl]butanamide |

**Table 48a: Examples of Core 32 (Ex.470-Ex.475)**

| **No** | **R²** | **R¹** | **Starting material** | **General Procedure** | **Reagent** | **Purification Method** | **Yield (isolated salt)** |
|---|---|---|---|---|---|---|---|
| **Ex.470:** *cf. experimental description* | | | | | | | |
| **Ex.471** | 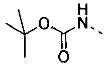 | 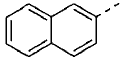 | **Ex.470** | P | cf. experimental description | FC (CH₂Cl₂/MeOH) | 62% |
| **Ex.472** | 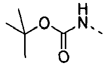 | 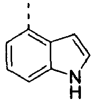 | **Ex.470** | P | cf. experimental description | FC (CH₂Cl₂/MeOH) | 62% |
| **Ex.473** | NH₂ | (Br) | **Ex.470** | J | cf. experimental description | crude product | 92% (HCl salt) |
| **Ex.474** | NH₂ | 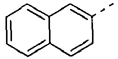 | **Ex.471** | J | cf. experimental description | crude product | 100% (HCl salt) |
| **Ex.475** | NH₂ | 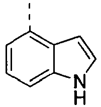 | **Ex.474** | - | cf. experimental description | FC (CH₂Cl₂/MeOH) | 83% |

**Table 48b: Examples of Core 32 (Ex.470-Ex.475)**

| **No** | **R²** | **R¹** | **FORMULA** | **MONOISOTOPIC MASS** | **Rt (purity at 220nm)** | **[M+H]⁺ found** | **LC-MS-Method** |
|---|---|---|---|---|---|---|---|
| **Ex.470:** *cf. experimental description* | | | | | | | |
| **Ex.471** | 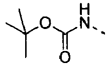 | 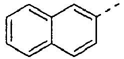 | C47H50N4O6 | 766.9 | 2.68 (95) | 767.4 | Method 10a |
| **Ex.472** | 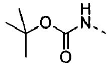 | 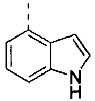 | C45H49N5O6 | 755.4 | 2.37 (92) | 756.4 | Method 10a |
| **Ex.473** | NH₂ | (Br) | C32H35BrN4O4 | 618.2 | 1.75 (98) | 621.1/ 619.1 | Method 10a |
| **Ex.474** | NH₂ | 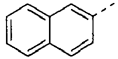 | C42H42N4O4 | 666.3 | 2.02 (96) | 667.3 | Method 10a |
| **Ex.475** | NH₂ | 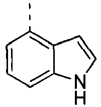 | C40H41N5O4 | 655.3 | 1.76 (90) | 656.3 | Method 10a |

**Table 48c: Examples of Core 32 (Ex.470-Ex.475)**

| **No** | **R²** | **R¹** | **IUPAC name** |
|---|---|---|---|
| **Ex.470** | 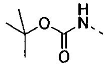 | (Br) | *tert*-butyl (2*R*,7*S*,18a*S*)-14-bromo-7-(naphthalen-2-ylmethyl)-5,9,12-trioxo-1,2,3,5,6,7,8,9,11,12,18,18a-dodecahydrospiro[benzo[*m*]pyrrolo[2,1-*c*][1,4,8,11]oxatriazacyclotetradecine-10,1'-cyclopentane]-2-ylcarbamate |
| **Ex.471** | 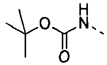 | 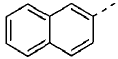 | *tert*-butyl (2*R*,7*S*,18a*S*)-14-(naphthalen-2-yl)-7-(naphthalen-2-ylmethyl)-5,9,12-trioxo-1,2,3,5,6,7,8,9,11,12,18,18a-dodecahydrospiro[benzo[*m*]pyrrolo[2,1-*c*][1,4,8,11]oxatriazacyclotetradecine-10,1'-cyclopentane]-2-ylcarbamate |
| **Ex.472** | 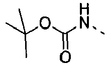 | 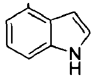 | *tert*-butyl (2*R*,7*S*,18a*S*)-14-(1H-indol-4-yl)-7-(naphthalen-2-ylmethyl)-5,9,12-trioxo-1,2,3,5,6,7,8,9,11,12,18,18a-dodecahydrospiro[benzo[*m*]pyrrolo[2,1-*c*][1,4,8,11]oxatriazacyclotetradecine-10,1'-cyclopentane]-2-ylcarbamate |
| **Ex.473** | NH₂ | (Br) | (2*R*,7*S*,18a*S*)-2-amino-14-bromo-7-(naphthalen-2-ylmethyl)-2,3,7,8,18,18a-hexahydrospiro[benzo[*m*]pyrrolo[2,1-*c*][1,4,8,11]oxatriazacyclotetradecine-10,1'-cyclopentane]-5,9,12(1*H*,6*H*,11*H*)-trione |
| **Ex.474** | NH₂ | 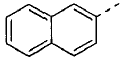 | (2*R*,7*S*,18a*S*)-2-amino-14-(naphthalen-2-yl)-7-(naphthalen-2-ylmethyl)-2,3,7,8,18,18a-hexahydrospiro[benzo[*m*]pyrrolo[2,1-*c*][1,4,8,11]oxatriazacyclotetradecine-10,1'-cyclopentane]-5,9,12(1*H*,6*H*,11*H*)-trione |
| **Ex.475** | NH₂ | 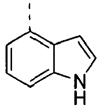 | (2*R*,7*S*,18a*S*)-2-amino-14-(*1*H-indol-4-yl)-7-(naphthalen-2-ylmethyl)-2,3,7,8,18,18a-hexahydrospiro[benzo[*m*]pyrrolo[2,1-*c*][1,4,8,11]oxatriazacyclotetradecine-10,1'-cyclopentane]-5,9,12(1*H*,6*H*,11*H*)-trione |

**Table 49a: Examples of Core 33*) (Ex.476-Ex.481; continued on the following page)**

| **No** | **R⁴** | **R⁵** | **R⁷** | **Starting material** | **General Procedure** | **Reagent** | **Purification Method** | **Yield (isolated salt)** |
|---|---|---|---|---|---|---|---|---|
| **Ex.476** *cf. experimental description* | | | | | | | | |
| **Ex.477** | CH₃ | CH₂Ph (*S*) | H | **259** | **) | 1. Fmoc-Apa-OH | Method 2 | 28% |
| | | | | | | 2. H-MePhe-OAll·pTsOH | | |
| **Ex.478** | H | H | H | 259 | **) | 1. Fmoc-Apa-OH *i*-Pr₂NEt (6 equiv.) | Method 2 | 25% |
| | | | | | | 2. H-Gly-OAll·pTsOH (2 equiv.), *i*-Pr₂NEt (6 equiv.) ***) | | |
| **Ex.479** | H | H | 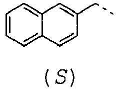 | **259** | **) | 1. Fmoc-β³-H2Nal-OH *i*-Pr₂NEt (6 equiv.) | Method 2 | 8% |
| | | | | | | 2. H-Gly-OAll·pTsOH (2 equiv.), *i*-Pr₂NEt (6 equiv.) ***) | | |
| **Ex.480** | CH₃ | CH₃ (S) | CH₃ | **259** | **) | 1. Fmoc-β³-HAla-OH *i*-Pr₂NEt (6 equiv.) | Method 2 | 40% |
| | | | | | | 2. H-MeAla-OAll·HCl (2 equiv.), *i*-Pr₂NEt (6 equiv.) | | |
| **Ex.481** | CH₃ | CH₃ (*S*) | 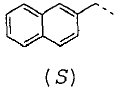 | **259** | **) | 1. Fmoc-β³-H2Nal-OH *i*-Pr₂NEt (6 equiv.) | Method 2 | 16% |
| | | | | | | 2. H-MeAla-OAll·HCl (2 equiv.), *i*-Pr₂NEt (6 equiv.) | | |

| | | | | | | | | |
|---|---|---|---|---|---|---|---|---|
| *) R² = benzamido (Ph-CO-NH-) **) See details in experimental description of **Ex.476** ***) Second coupling was repeated once. | | | | | | | | |

**Table 49b: Examples of Core 33*) (Ex.476-Ex.481)**

| **No** | **R⁴** | **R⁵** | **R⁷** | **FORMULA** | **MONOISOTOPIC MASS** | **Rt (purity at 220nm)** | **[M+H]⁺ found** | **LC-MS-Method** |
|---|---|---|---|---|---|---|---|---|
| **Ex.476:** *cf. experimental description* | | | | | | | | |
| **Ex.477** | CH₃ | CH₂Ph (S) | H | C32H34N4O5 | 554.2 | 1.68 (90) | 555.2 | Method 10b |
| **Ex.478** | H | H | H | C24H26N4O5 | 450.2 | 1.29 (95) | 451.2 | Method 10b |
| **Ex.479** | H | H | 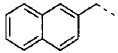 (*S*) | C35H34N4O5 | 590.3 | 1.88 (94) | 591.2 | Method 10b |
| **Ex.480** | CH₃ | CH₃ (*S*) | CH₃ (*S*) | C27H32N4O5 | 492.2 | 1.42 (99) | 493.0 | Method 10b |
| **Ex.481** | CH₃ | CH₃ (*S*) | 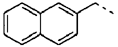 (*S*) | C37H38N4O5 | 618.3 | 1.94 (94) | 619.2 | Method 10b |

| | | | | | | | | |
|---|---|---|---|---|---|---|---|---|
| *)R² = benzamido (Ph-CO-NH-) | | | | | | | | |

**Table 49c: Examples of Core 33*) (Ex.476-Ex.481)**

| **No** | **R⁴** | **R⁵** | **R⁷** | IUPAC name |
|---|---|---|---|---|
| **Ex.476** | CH₃ | CH₃ (*S*) | H | *N*-[(4*S*,6*R*,14*S*)-14,15-dimethyl-9,13,16-trioxo-2-oxa-8,12,15-triazatricyclo[15.3.1.0^{4,8}]henicosa-1(21),17,19-trien-6-yl]benzamide |
| **Ex.477** | CH₃ | CH₂Ph (*S*) | H | *N*-[(4*S*,6*R*,14*S*)-14-benzyl-15-methyl-9,13,16-trioxo-2-oxa-8,12,15-triazatricyclo[15.3.1.0^{4,8}]henicosa-1(21),17,19-trien-6-yl]benzamide |
| **Ex.478** | H | H | H | *N*-[(4*S*,6*R*)-9,13,16-trioxo-2-oxa-8,12,15-triazatricyclo[15.3.1.0^{4,8}]henicosa-1(21),17,19-trien-6-yl]benzamide |
| **Ex.479** | H | H | 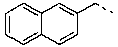 (*S*) | *N*-[(4*S*,6*R*,11*S*)-11-(2-naphthylmethyl)-9,13,16-trioxo-2-oxa-8,12,15-triazatricyclo[15.3.1.0^{4,8}]henicosa-1(21),17,19-trien-6-yl]benzamide |
| **Ex.480** | CH₃ | CH₃ (*S*) | CH₃ (*S*) | *N*-[(4*S*,6*R*,11*S*,14*S*)-11,14,15-trimethyl-9,13,16-trioxo-2-oxa-8,12,15-triazatricyclo[15.3.1.0^{4,8}]henicosa-1(21),17,19-trien-6-yl]benzamide |
| **Ex.481** | CH₃ | CH₃ (*S*) | 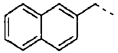 (*S*) | *N*-[(4*S*,6*R*,11*S*,14*S*)-14,15-dimethyl-11-(2-naphthylmethyl)-9,13,16-trioxo-2-oxa-8,12,15-triazatricyclo[15.3.1.0^{4,8}]henicosa-1(21),17,19-trien-6-yl]benzamide |

| | | | | |
|---|---|---|---|---|
| *)R² = benzamido (Ph-CO-NH-) | | | | |

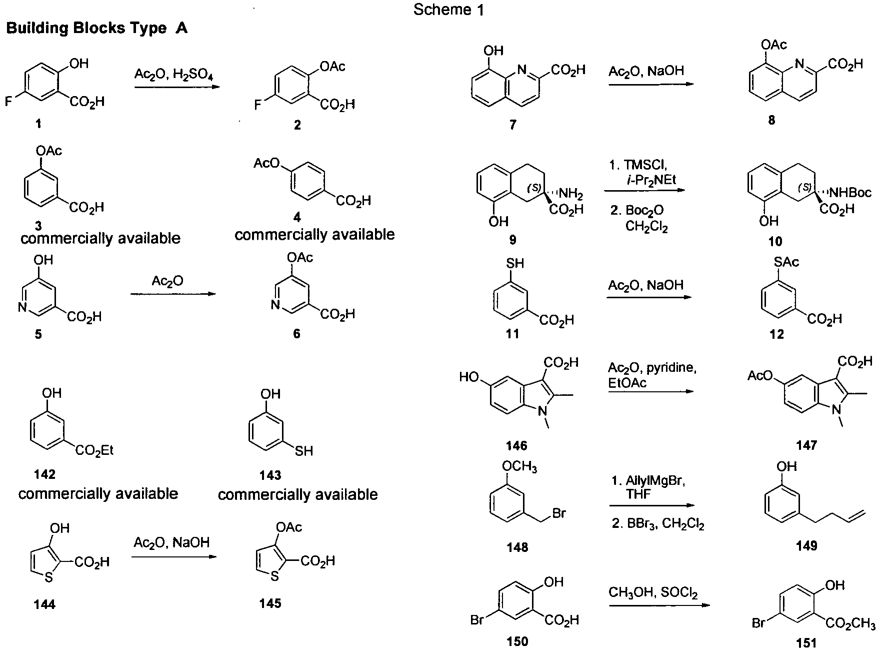

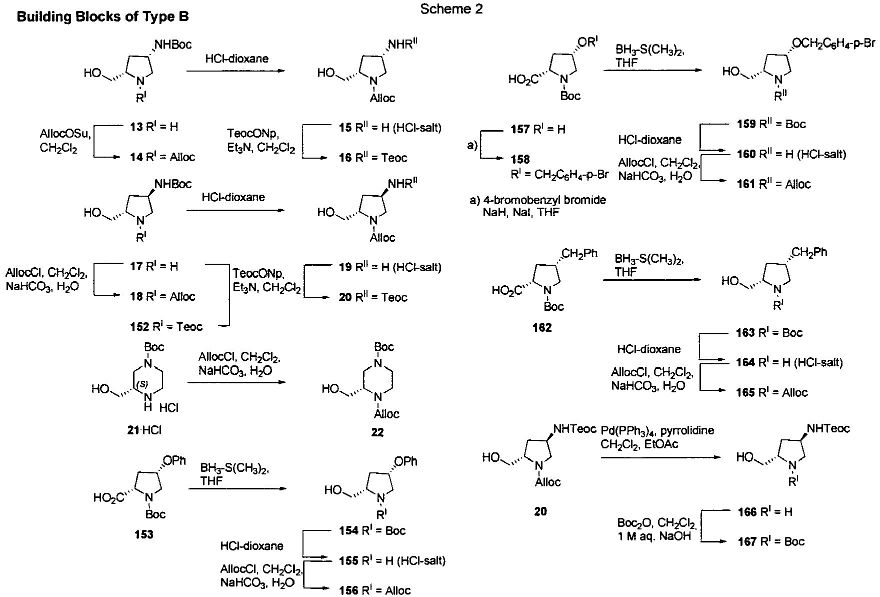

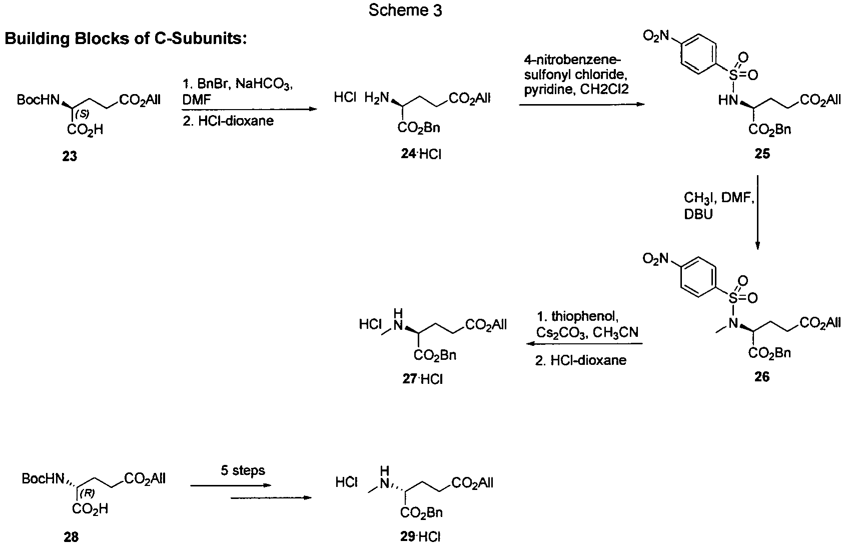

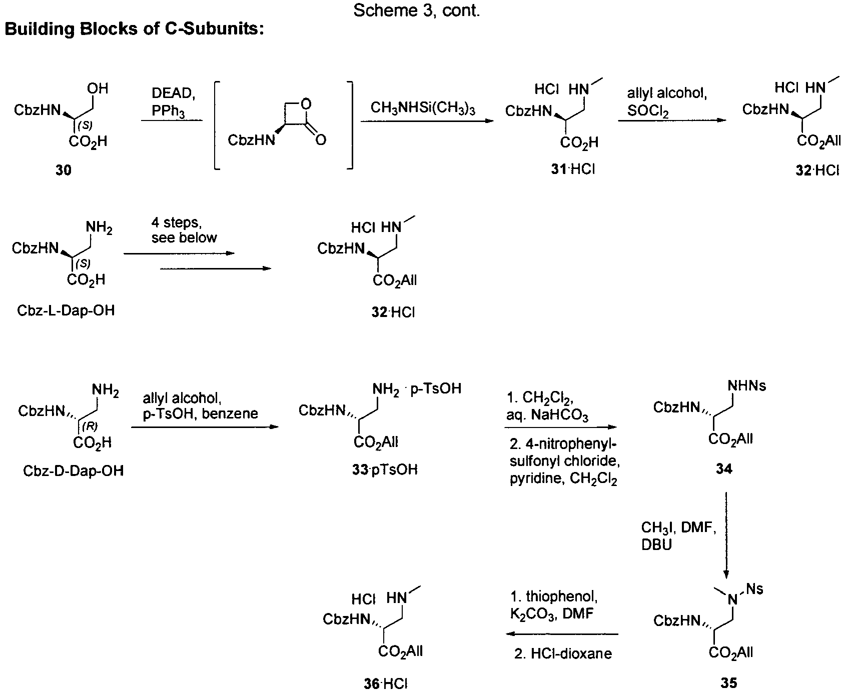

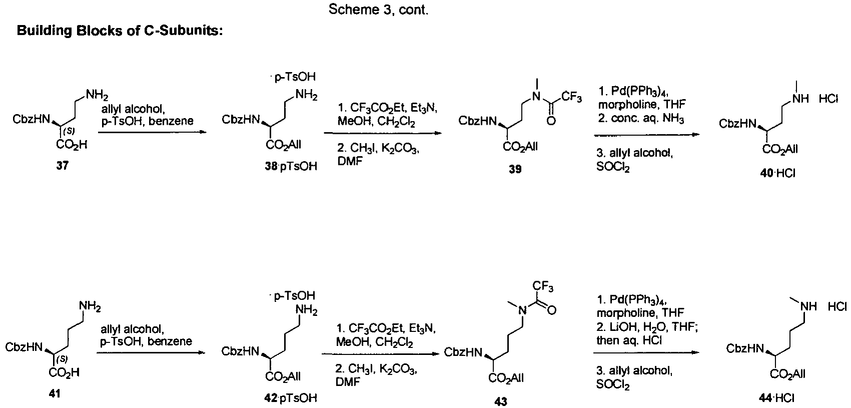

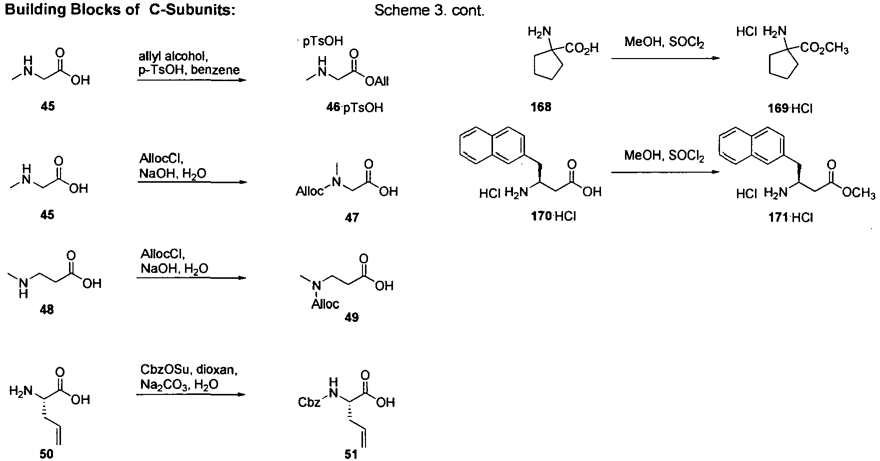

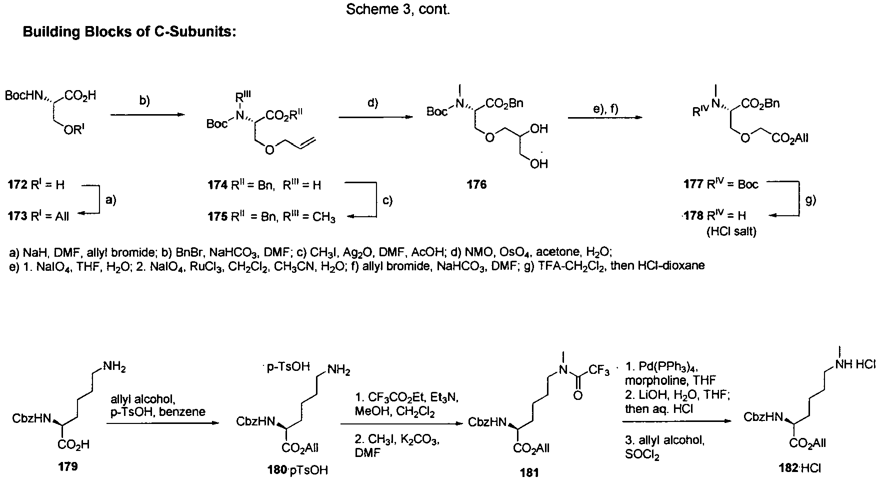

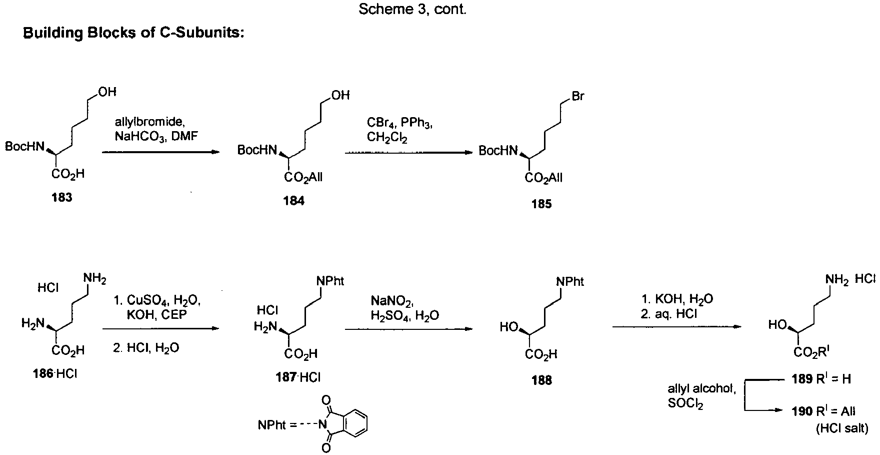

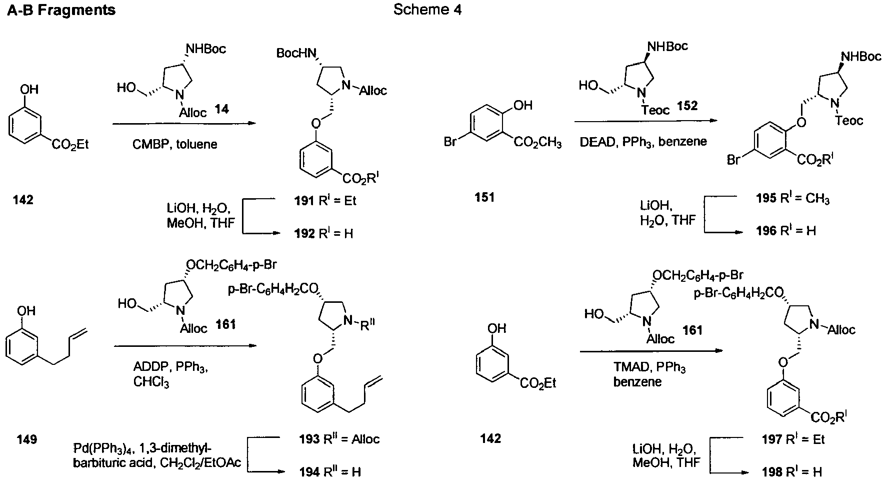

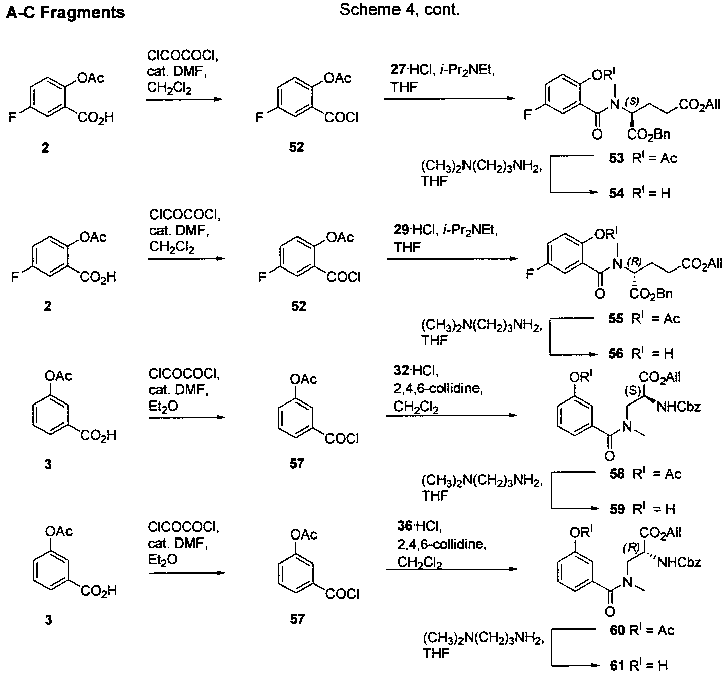

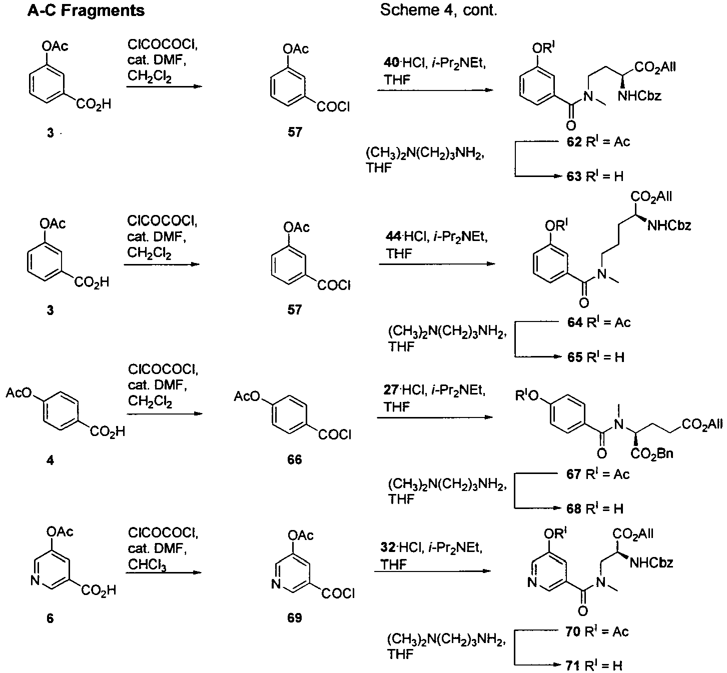

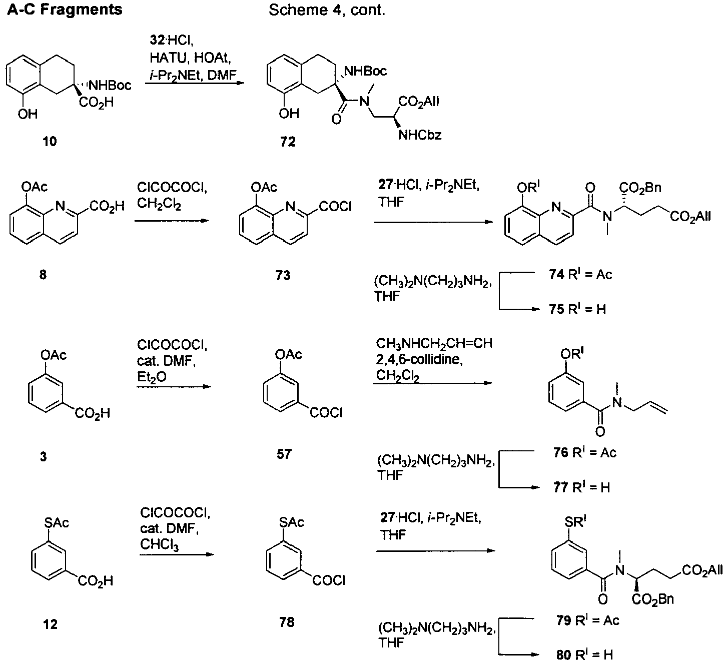

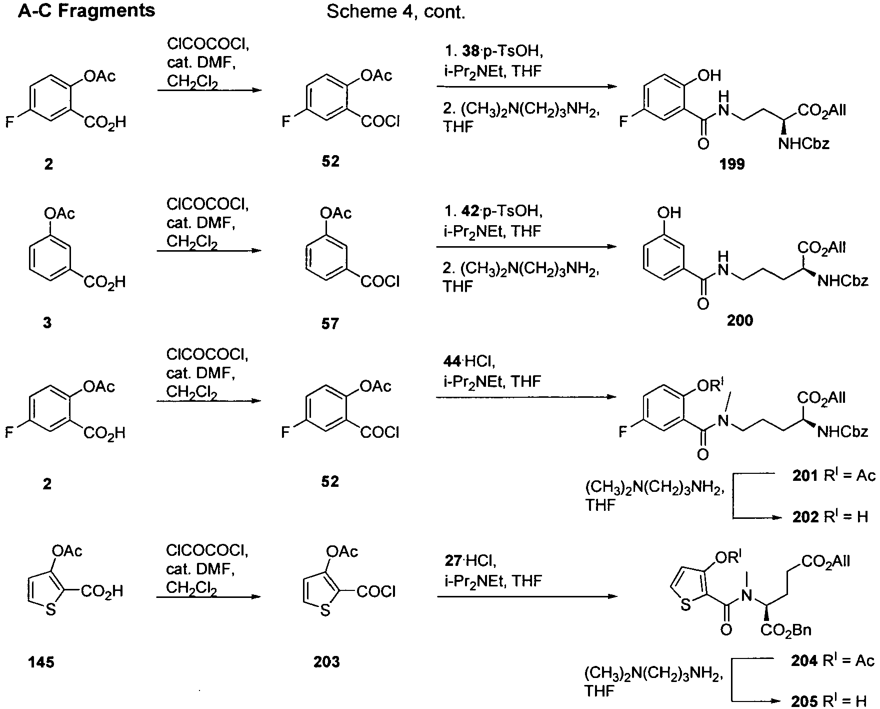

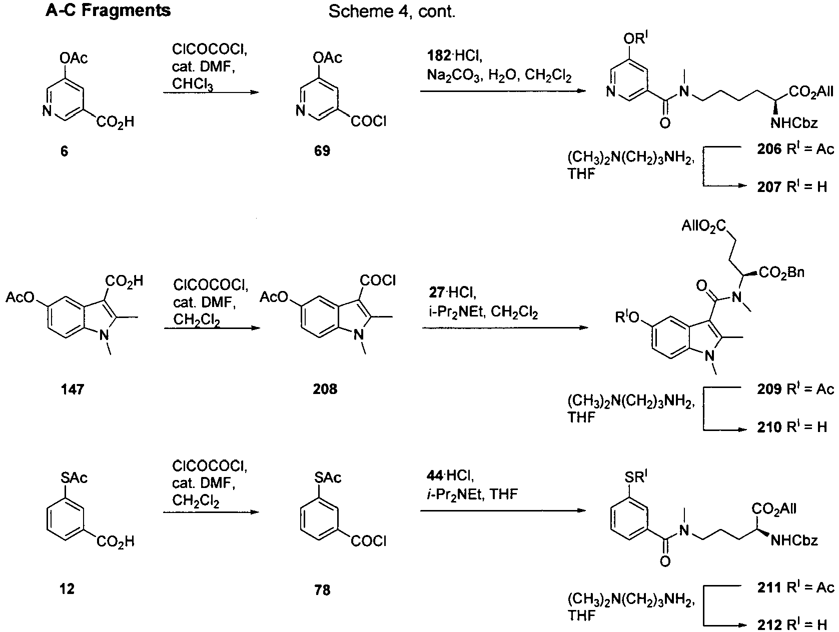

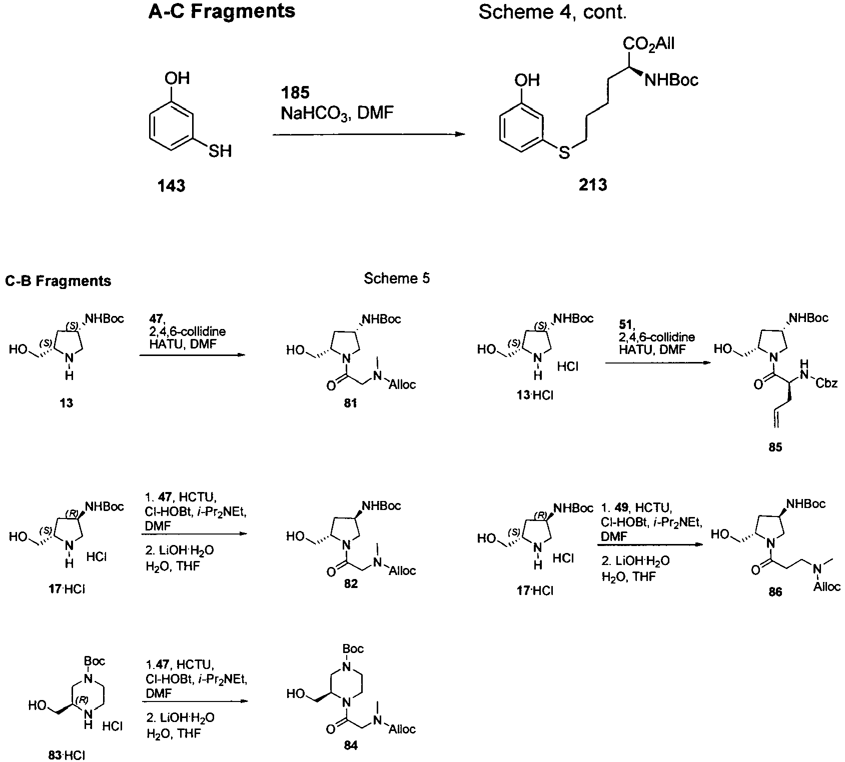

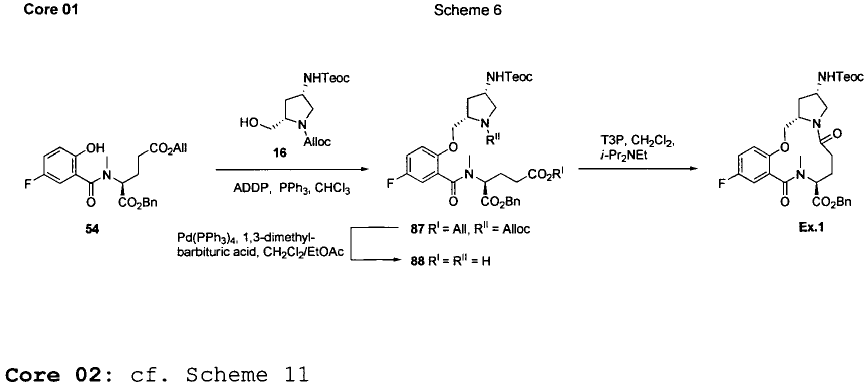

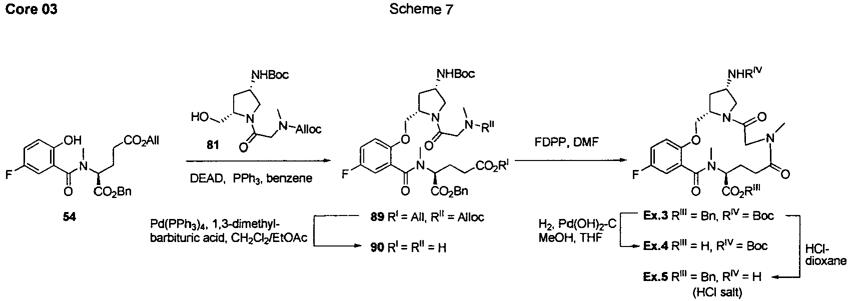

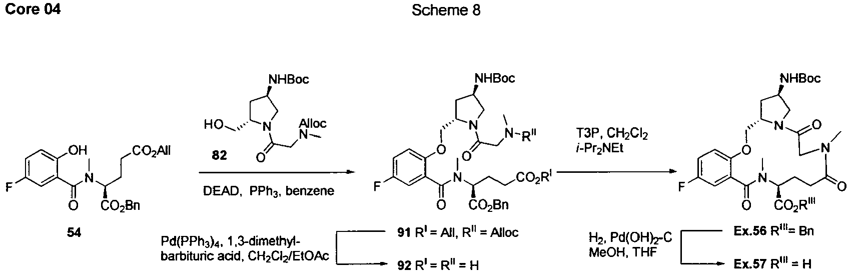

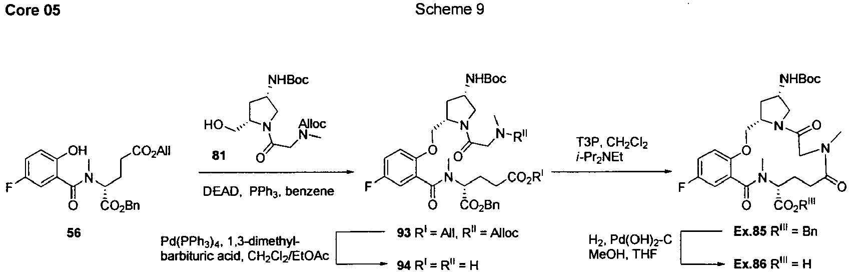

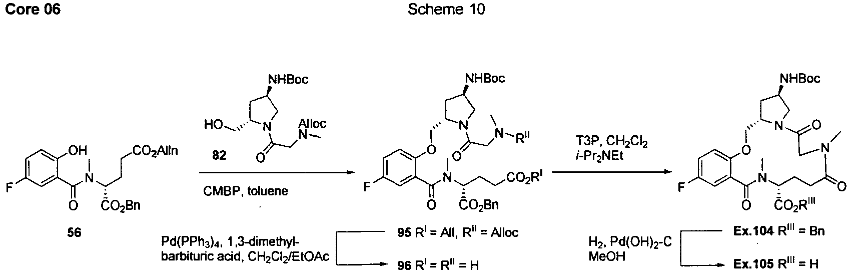

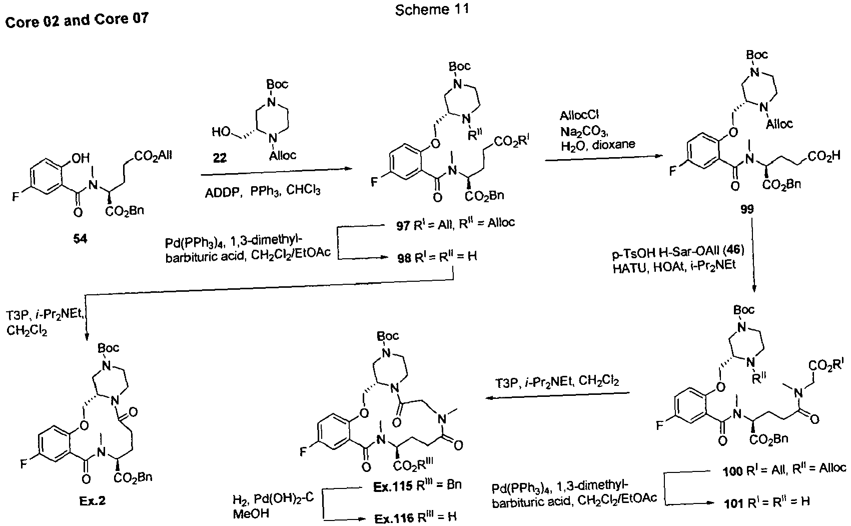

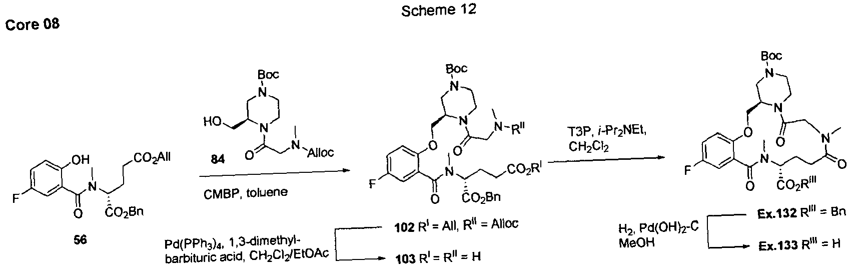

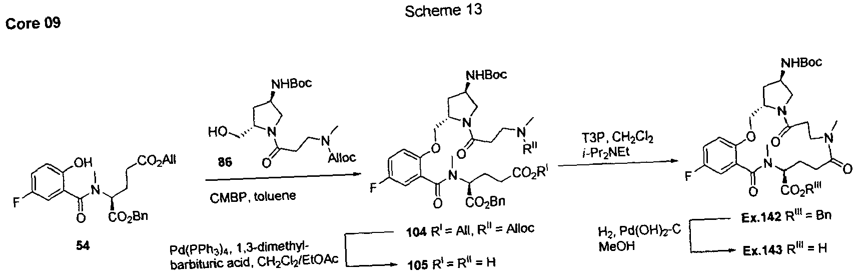

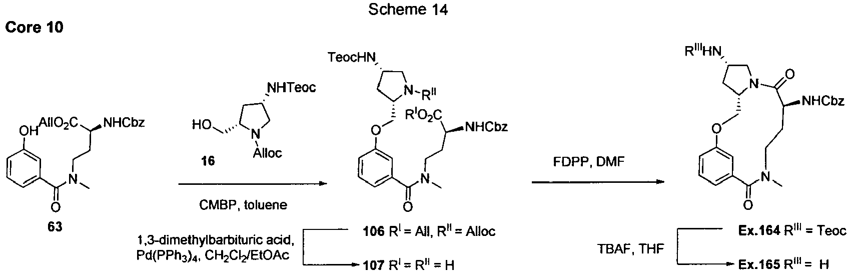

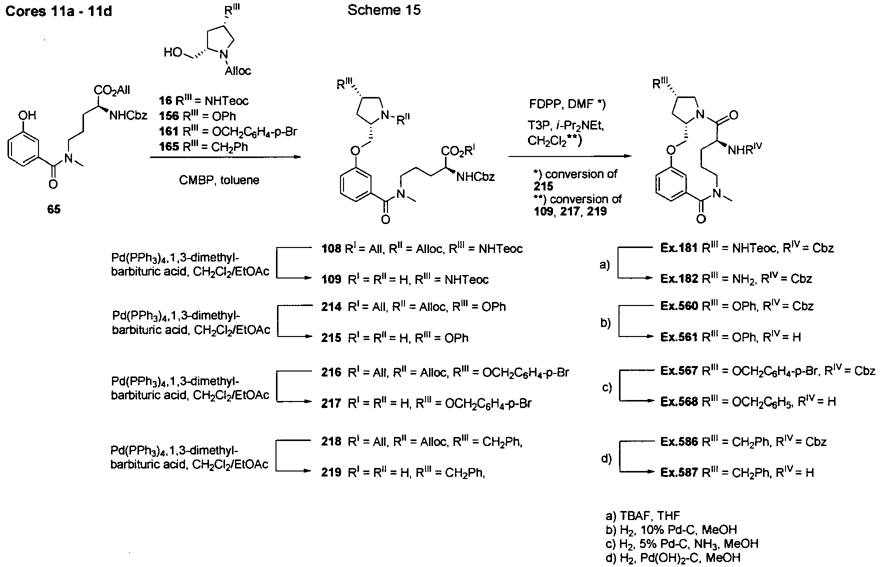

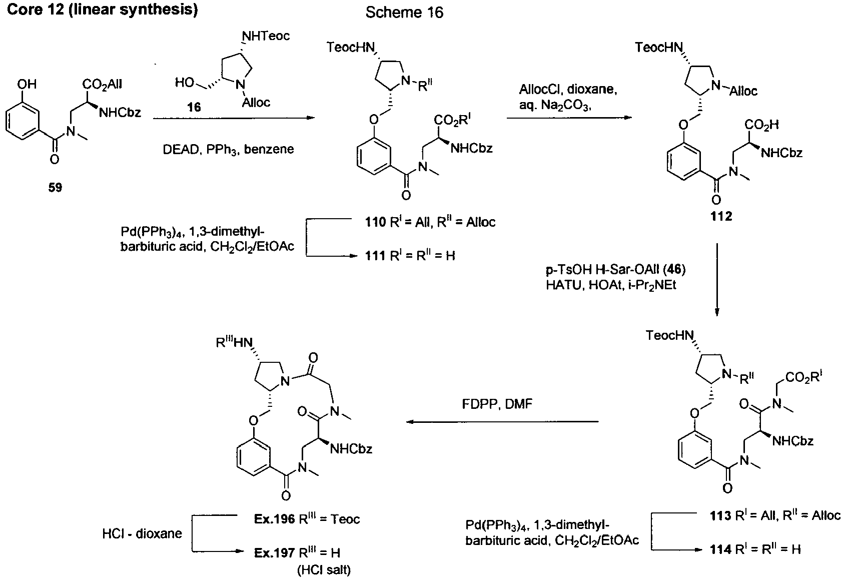

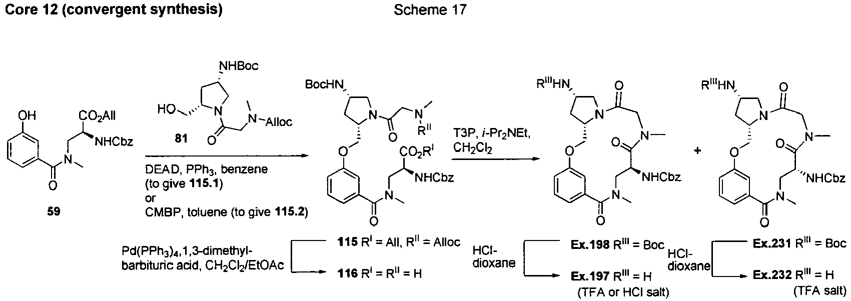

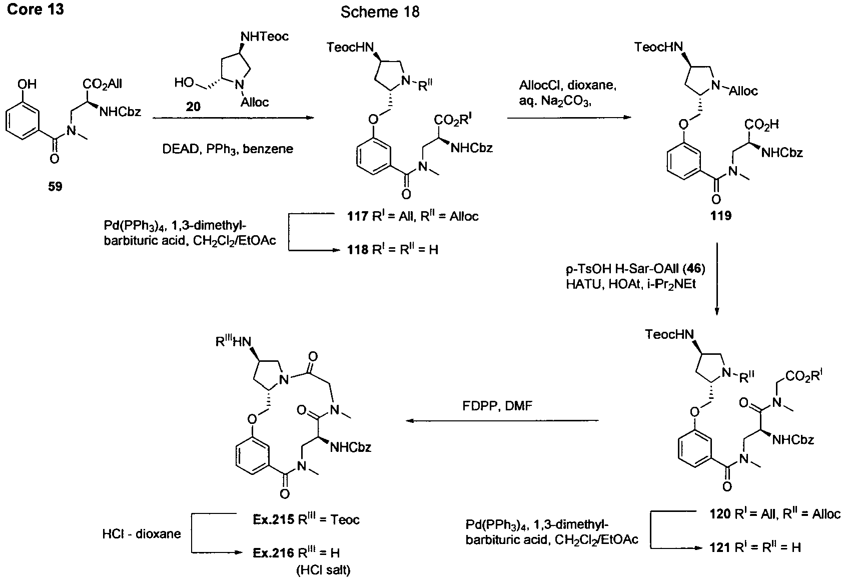

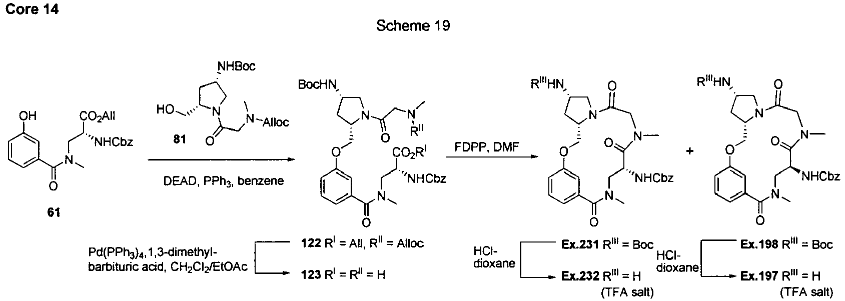

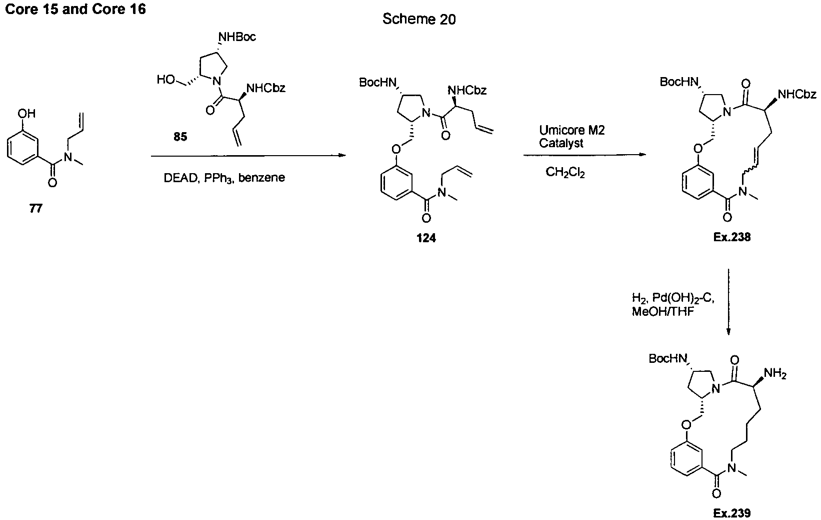

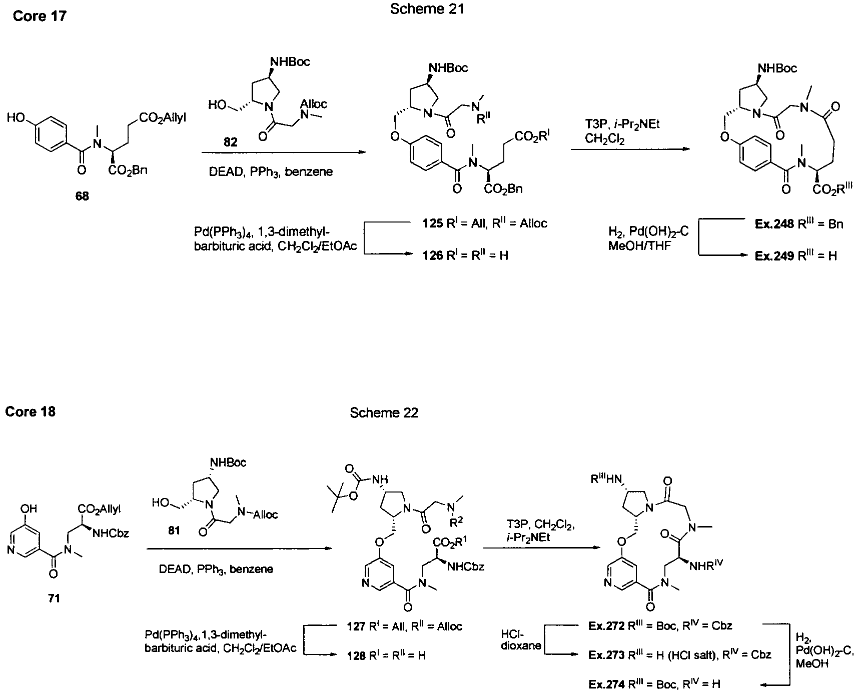

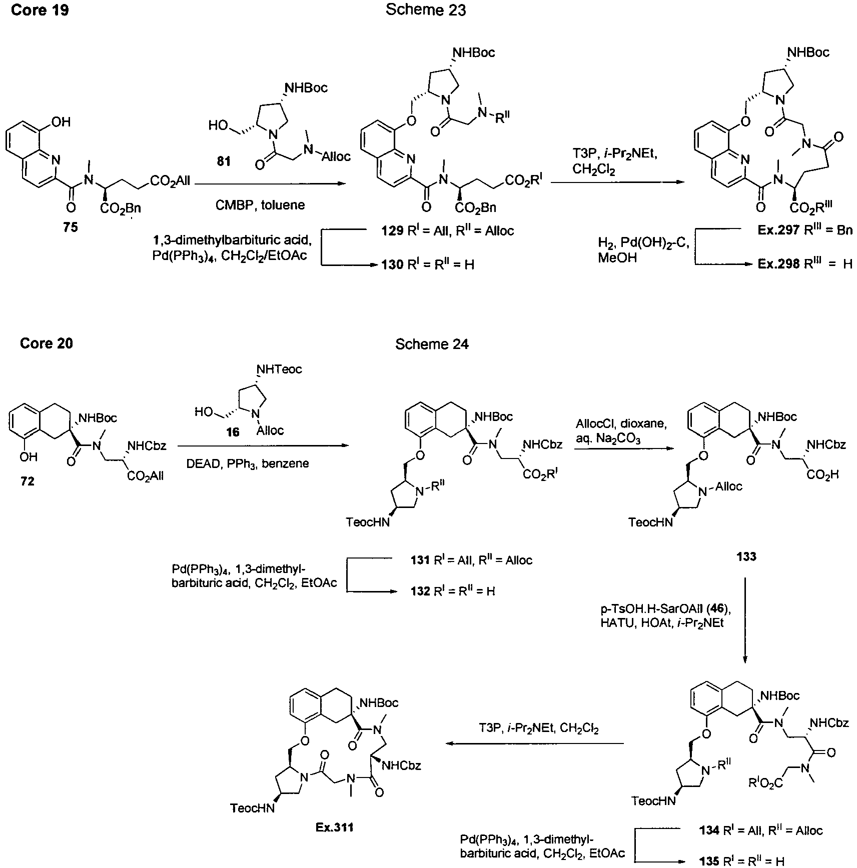

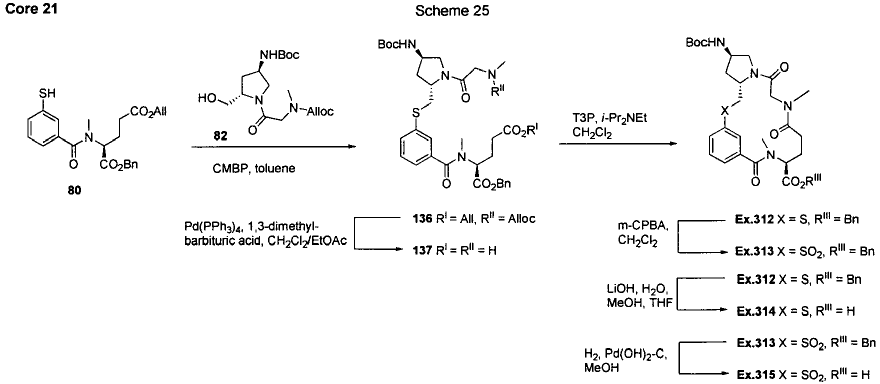

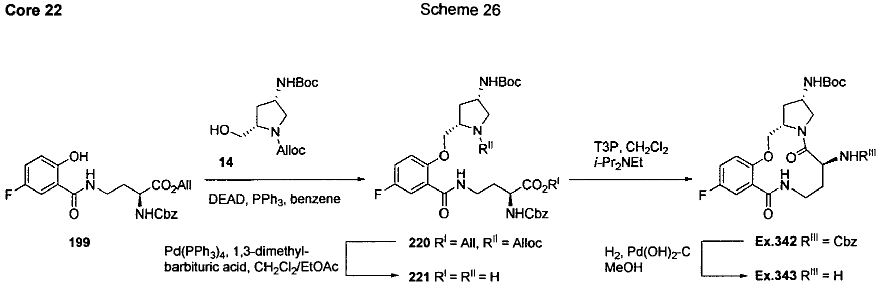

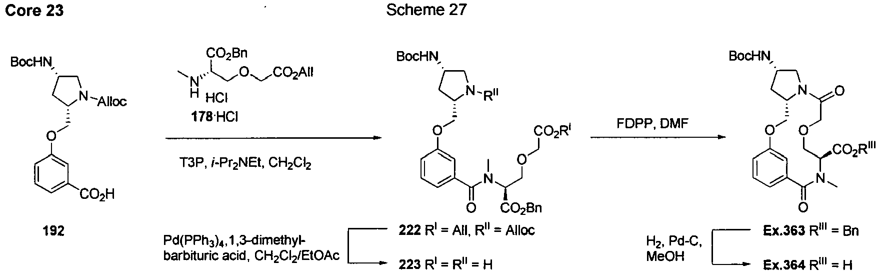

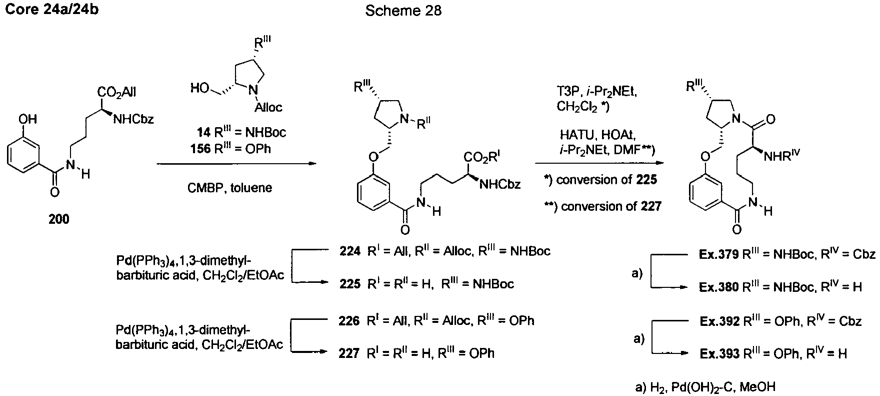

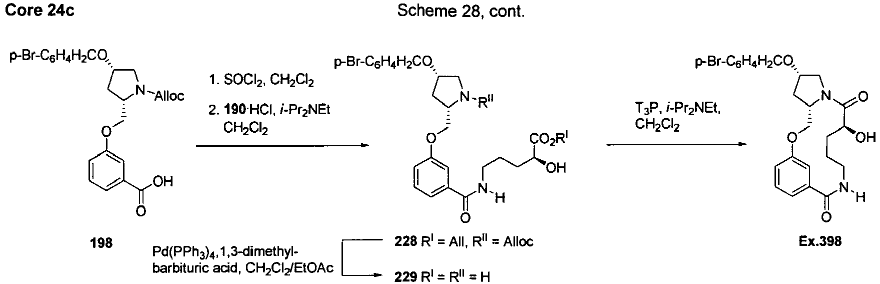

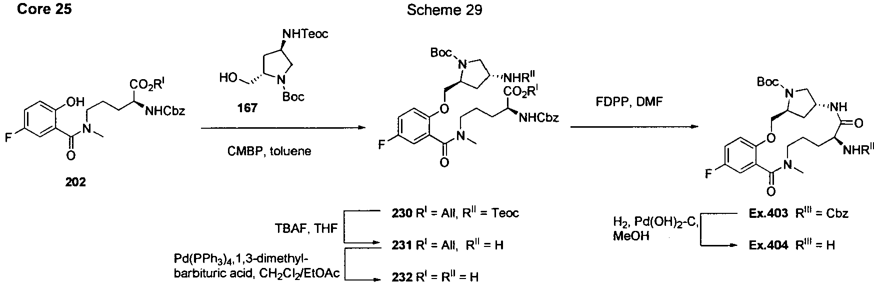

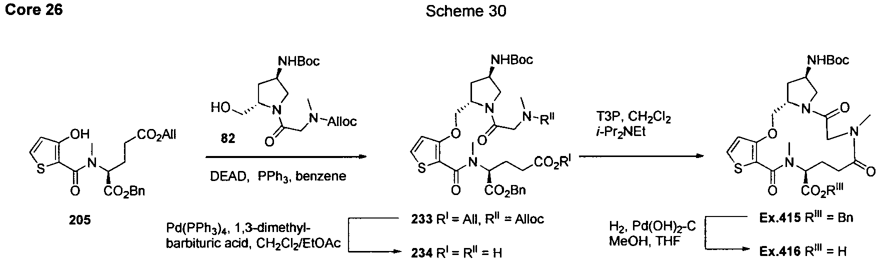

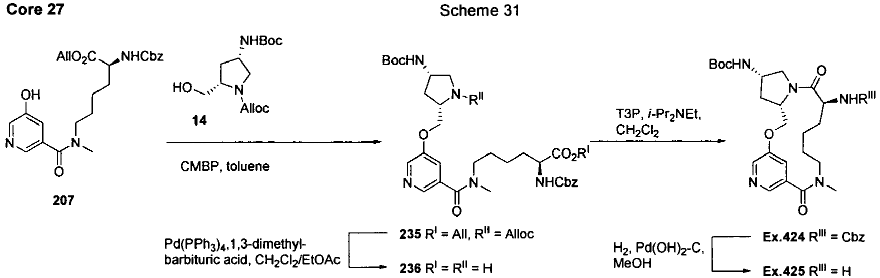

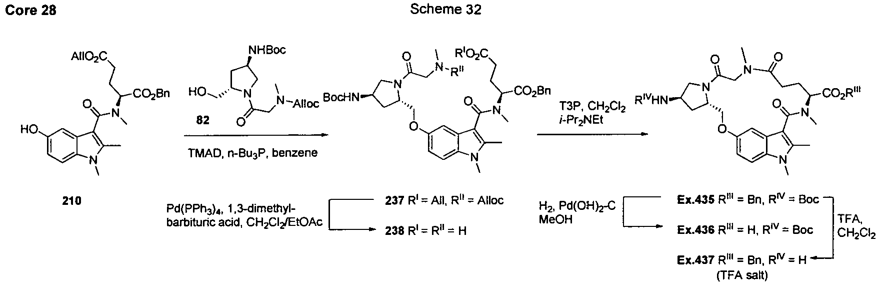

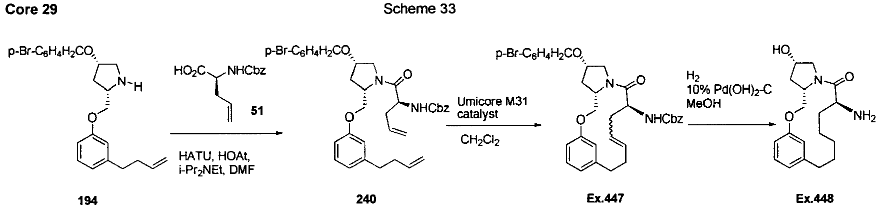

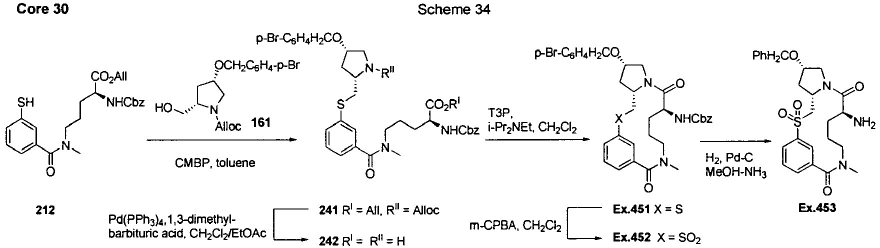

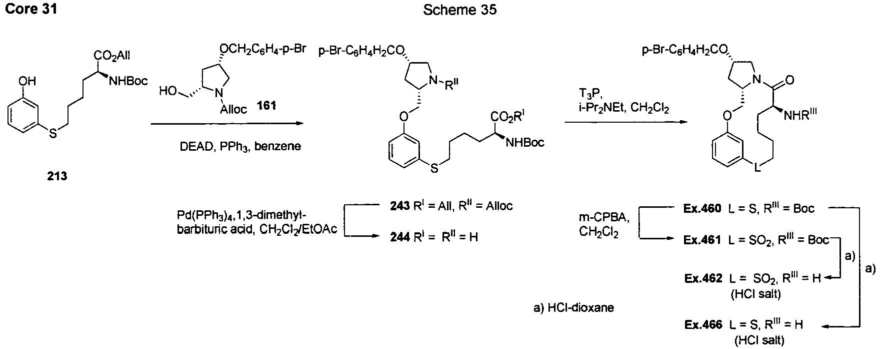

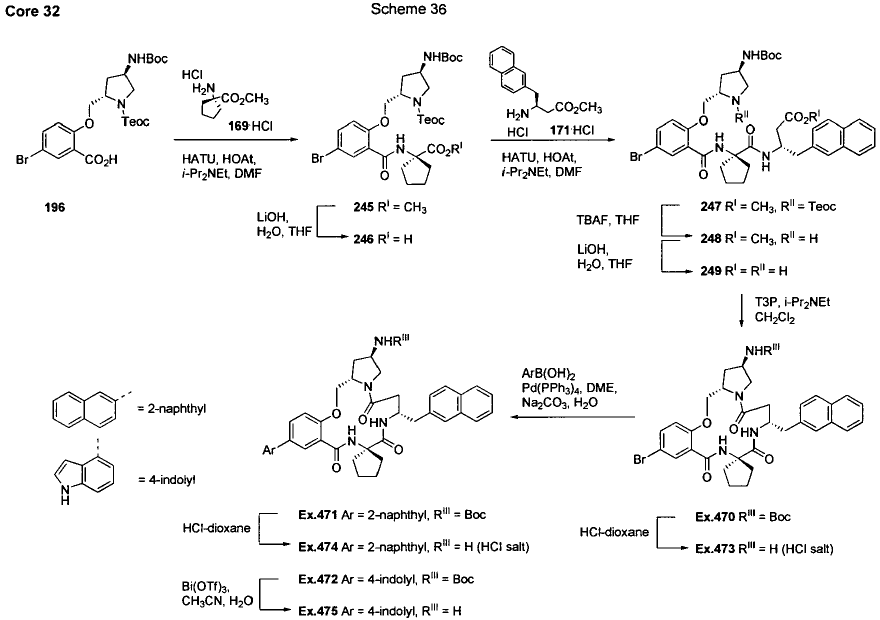

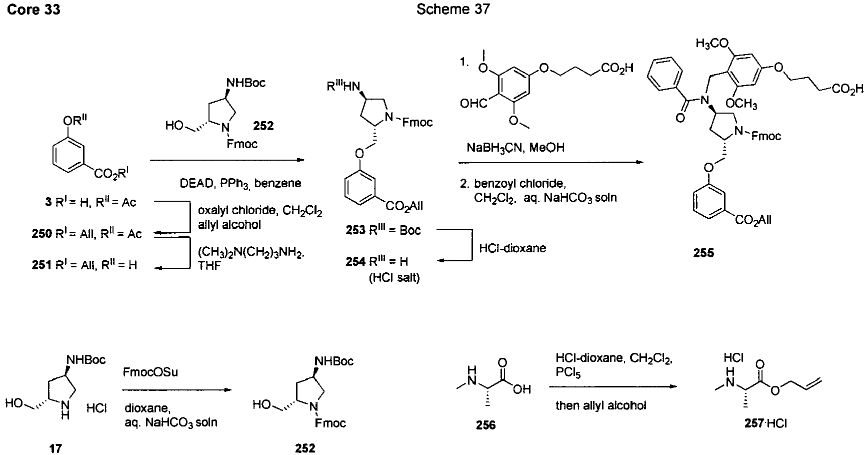

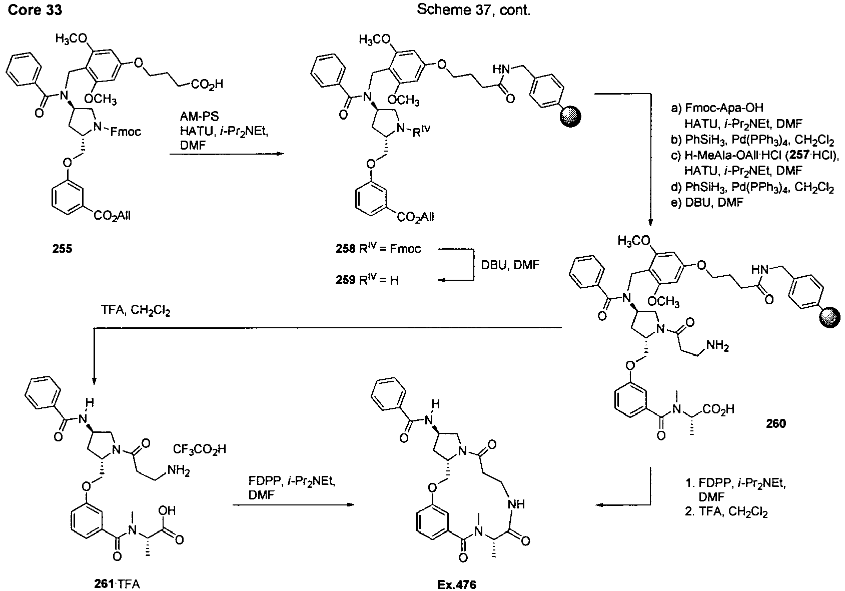

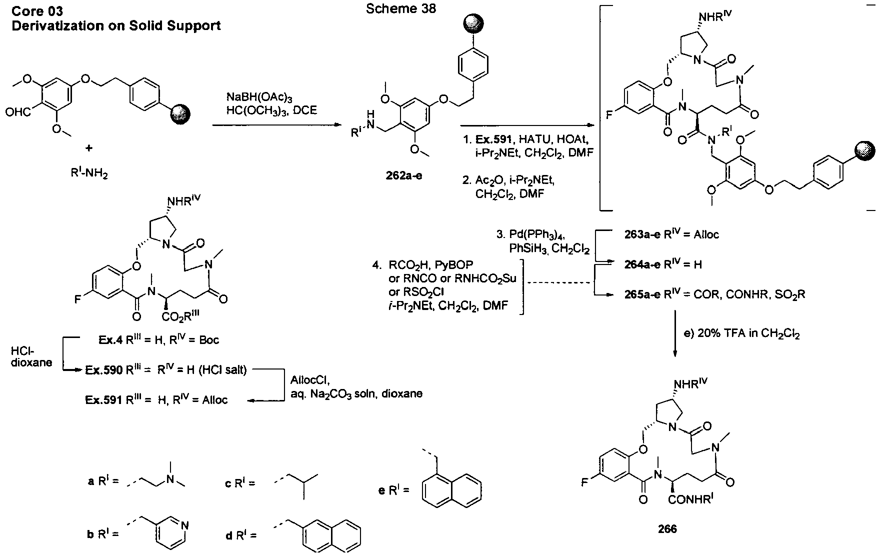

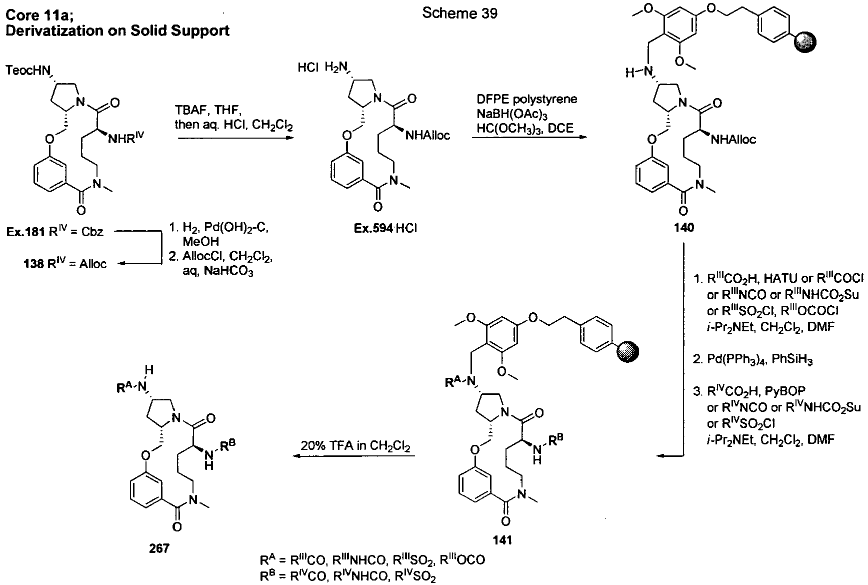

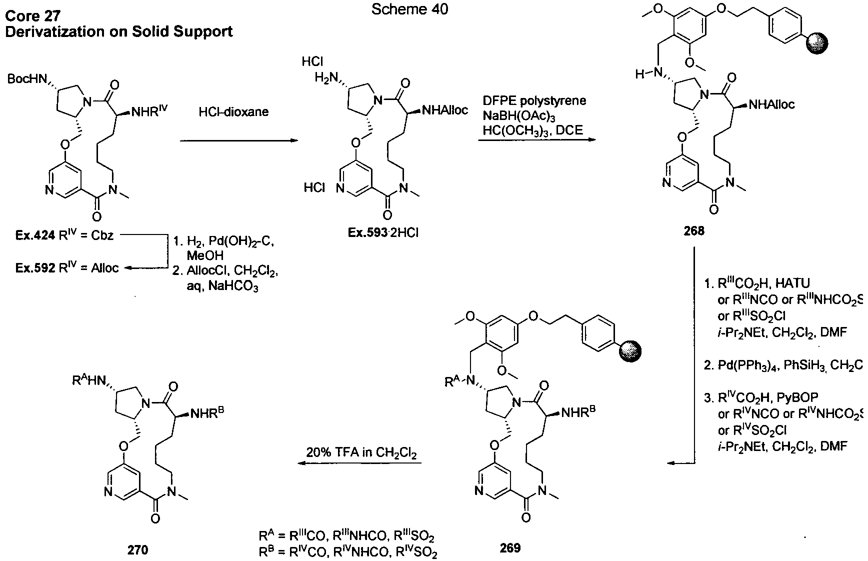

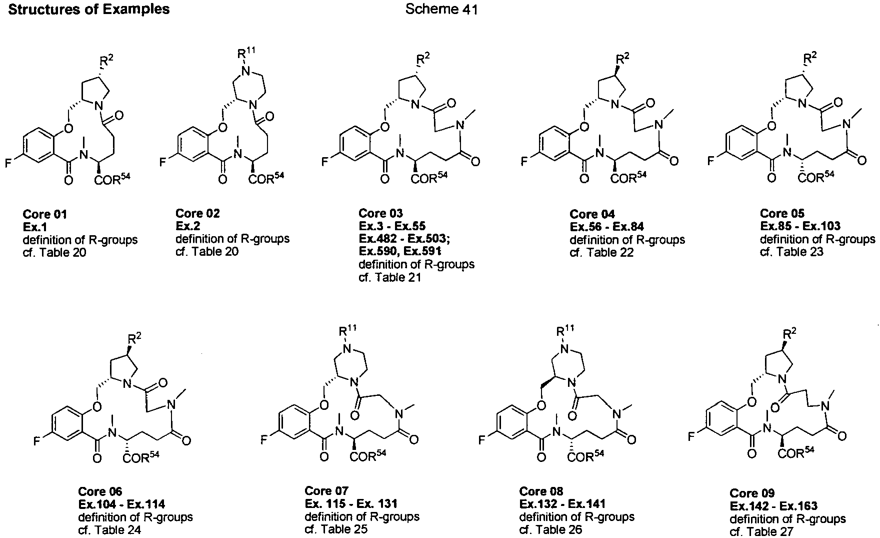

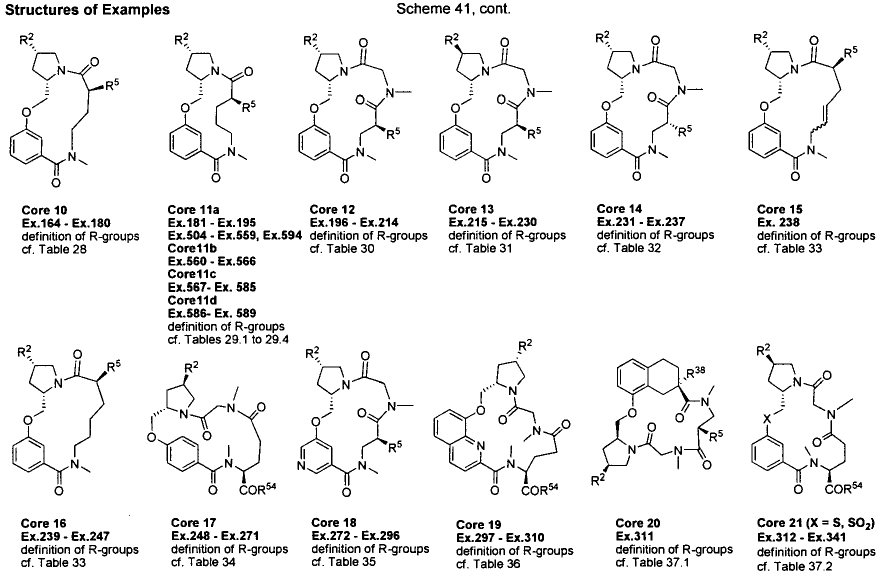

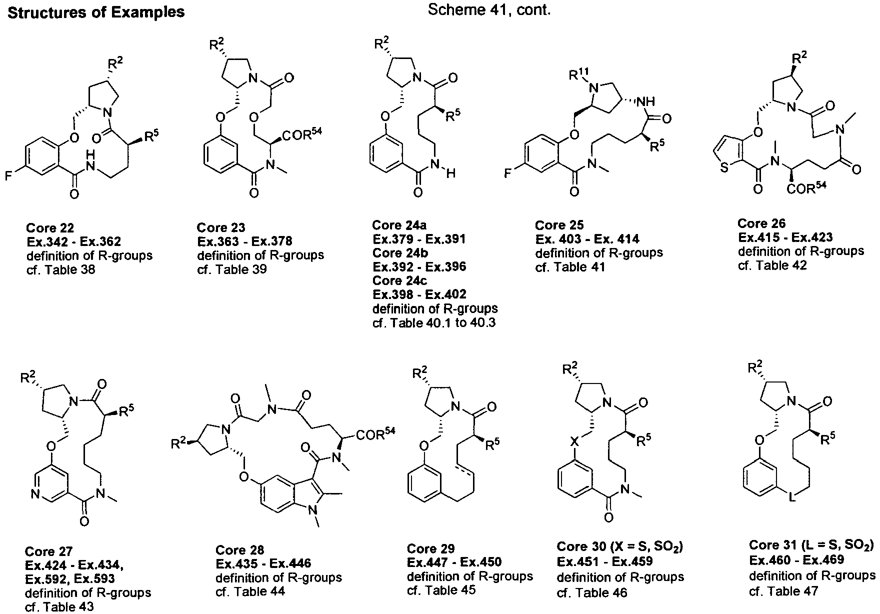

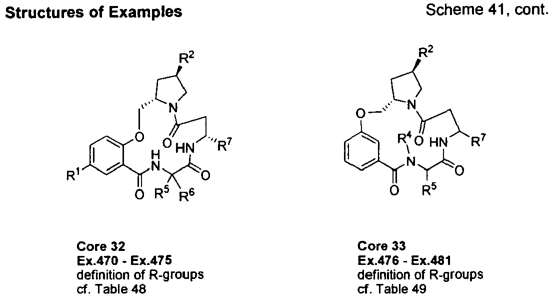

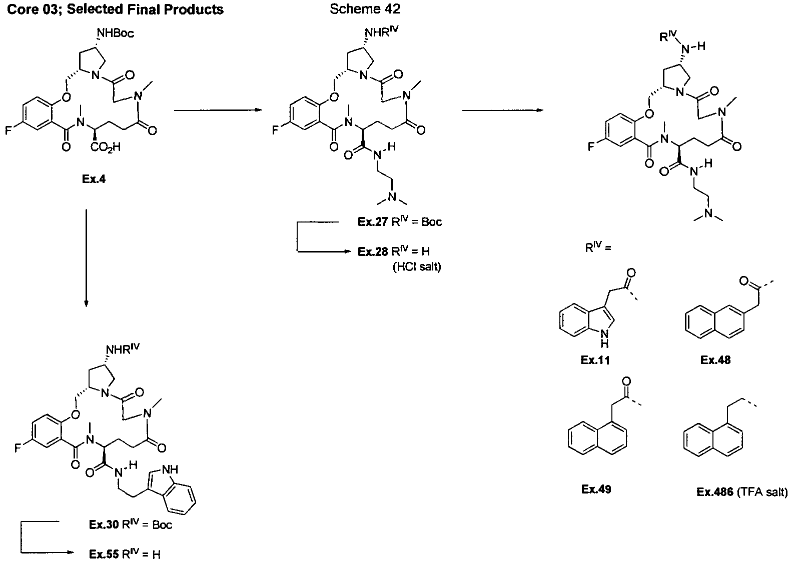

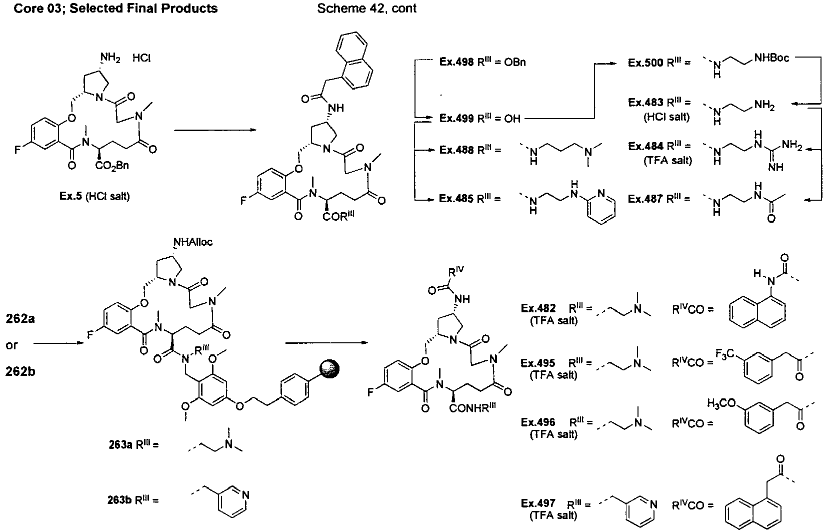

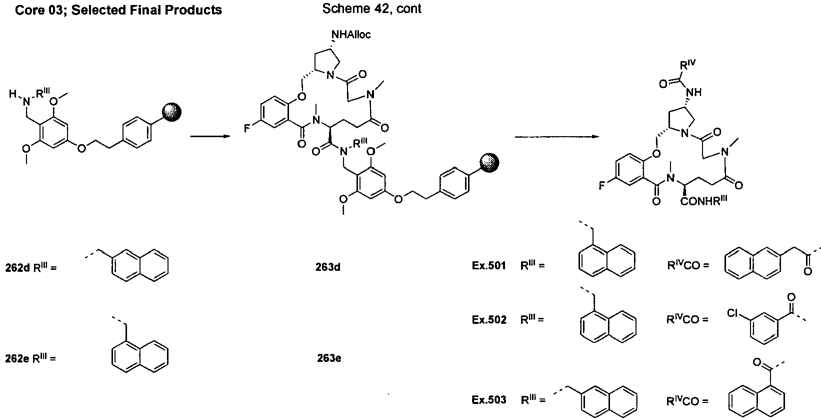

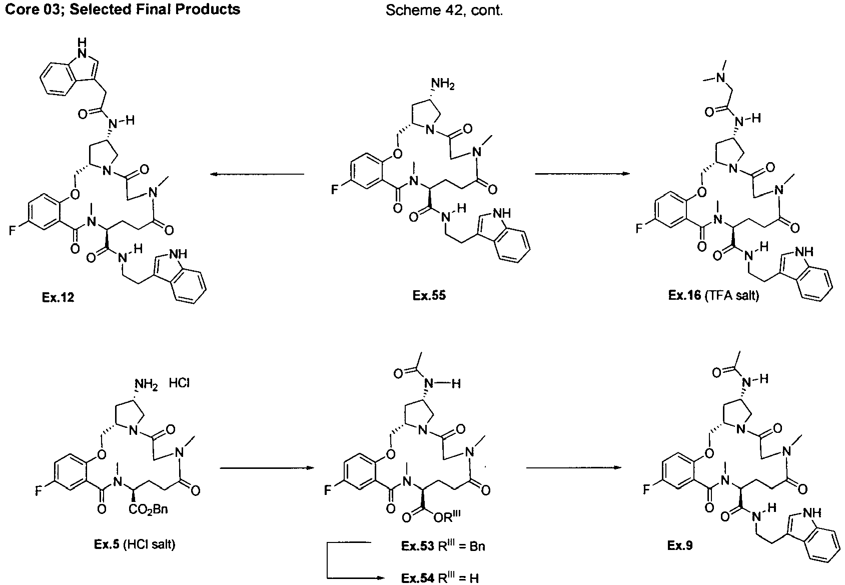

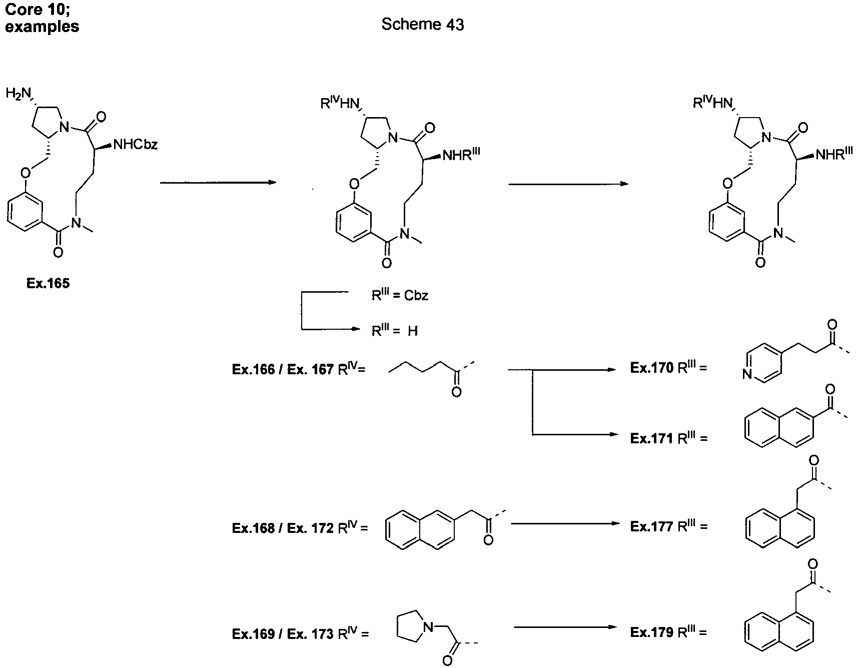

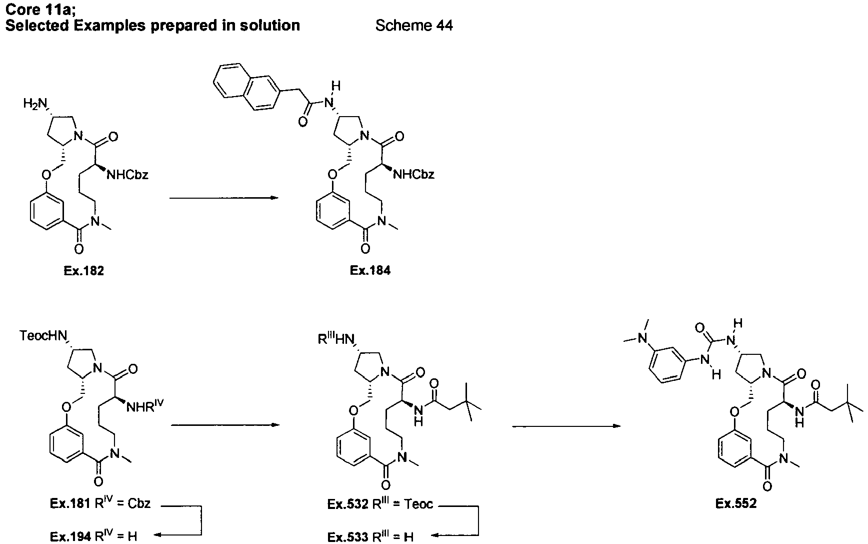

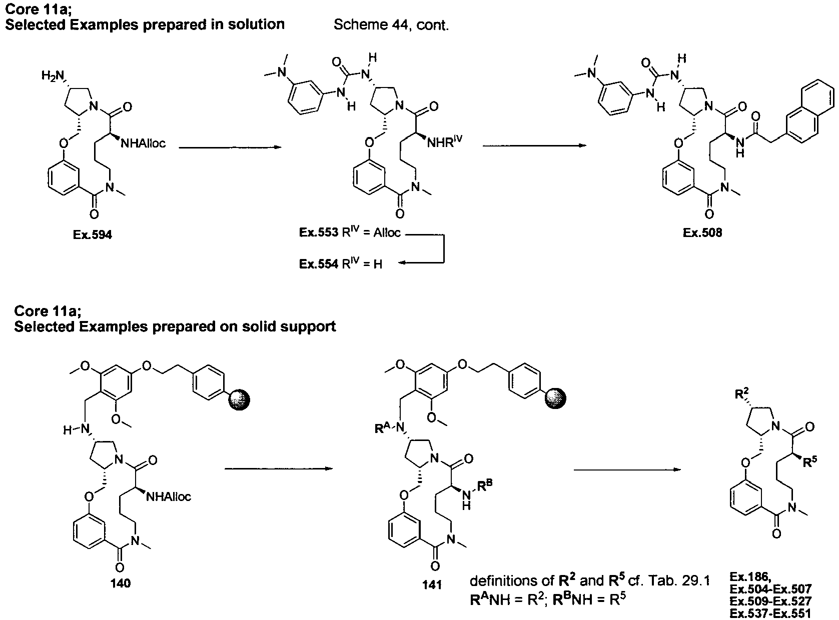

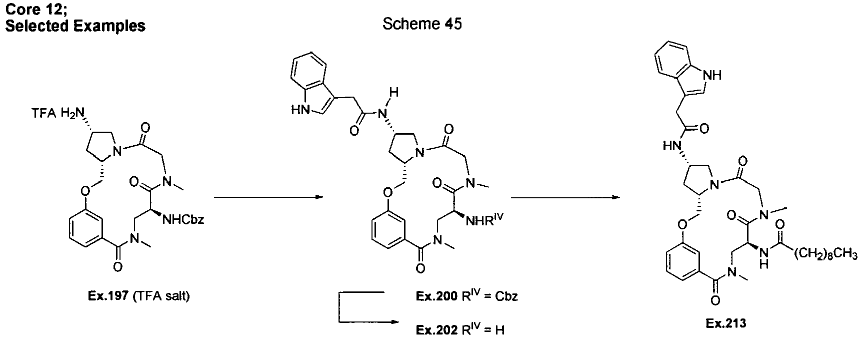

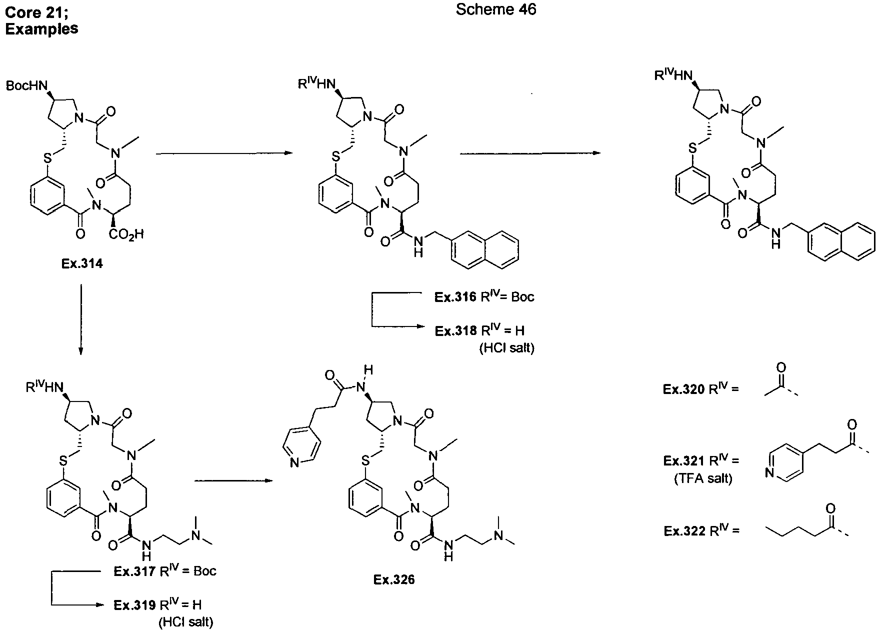

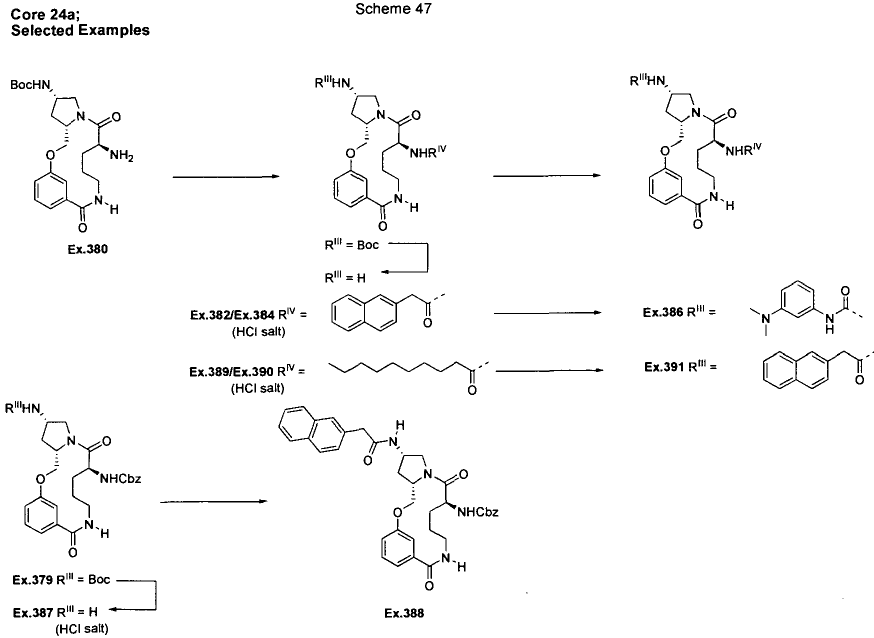

### Biological and pharmacological methods

### 1. Ca²⁺ assays for the GPCRs motilin receptor, prostaglandin F (FP) receptor, 5-hydroxytryptamine 2B (5-HT_{2B}) receptor, and purinoreceptor P2Y₁

Assays were performed using on a FLIPR^{TETRA} fluorometric imaging plate reader (Molecular Devices) with ScreenWorks Version 2 (Molecular Devices) as device operating and data analysis software.

Dose dependent agonist and antagonist activities were determined. Percentage activation and percentage inhibition values were determined.

Percentage activation was determined upon initial addition of the sample compounds followed by 10 minutes incubation at 25°C. Following compound incubation, reference agonists were added at EC₈₀ to determine percentage inhibition.

Reference agonists were purchased from reputable commercial vendors and prepared according to each ligand's specifications. All handling of ligands were done to ensure proper control throughout the experiments.

Example compounds were weighed on a Microbalance (Mettler MX5) and dissolved in 100% DMSO to a final concentration of 2.5 mM and subsequently diluted into the assay buffer.

The Assay buffer was a supplemented HBSS (Hank's Balanced Salt Solution). HBSS was supplemented with 20 mM HEPES (4-(2-hydroxyethyl)-piperazin-1-ethansulfonic acid) and 2.5 mM Probenecid (Sigma P8761).

### Assay plate seeding:

GPCR assays were performed using Ca²⁺ optimized hematopoietic cell lines (rat) with cultures never exceeding 90% confluency. Cells were harvested and seeded (from cultures at less than 90% confluency) at 50000 cells/well for a 96-well plate (12500 cells/well for 384). After seeding, the assay plates were incubated for forty-five (45) minutes at room temperature. After room temperature incubation, the assay plates were incubated at 37°C 5% CO₂ for 24 hours prior to assaying.

### Calcium Dye Loading:

All GPCR assays were performed using Fluo-8 Ca²⁺ dye.

Ca²⁺ dye was prepared at 1 x dye concentration in GPCR assay buffer. After 24 hours of incubation, cells were washed with GPCR assay buffer, and then Ca²⁺-dye (100 µL/well) was added. The plates were incubated for 90 minutes at 30°C 5% CO₂ prior to FLIPR assay.

### Agonist Assay:

Compound plates were prepared to add 50 µL/well during the agonist assay mode. During the FLIPR assay, 50 µL/well from the compound plate was diluted 3-fold into the existing 100 µL/well from the dye loading step. Therefore all compounds were prepared as 3x the final concentration desired in the assay.

### Antagonist Assay:

After completion of the first single addition assay run, assay plate was removed from the FLIPR Tetra and placed at 25°C for seven (7) minutes before antagonist assay.

Using the EC₈₀ values determined during the agonist assay, all pre-incubated sample compound and reference antagonist (if applicable) wells were stimulated at the EC₈₀ of the reference agonist. As reference ligands for the first three assays their obvious natural ligands motilin, prostaglandin F2α, and serotonin (5-HT, 5-Hydroxytryptamine) were used; and for the P2Y₁ assay 2MeSATP (2-methyl-thio-adeno-sine 5'-triphosphate, i.e. (2R,3S,4R)-5-(6-amino-2-methylsulfanyl-purin-9-yl)-3,4-dihydroxy -tetrahydrofuran-2-yl]methyl (hydroxy-phosphonooxy-phosphoryl) hydrogen phosphate) was applied.

After the addition of the reference agonist fluorescence was monitored for 180 sec using FLIPR Tetra.

### Data analysis and results:

From the FLIPR data, with negative control correction enabled, the maximum statistic for each well was exported and percentage activation relative to Eₘₐₓ control was calculated.

The results of the GPCR assay are summarized in Table 50.1 to Table 50.4.

### 2. Assay for the voltage gated ion channel Kᵥ1.3

Assays were performed and analyized with a FLIPR^{TETRA} device as specified above.

Compounds for inhibition of ion channel Kᵥ1.3 were assayed in Jurkat cells recombinantly expressing human Kᵥ1.3. For high throughput screening a thallium-sensitive, fluorescence-based assay was used. Cells were incubated with the Tl⁺ sensitive dye according to the protocol suggested by the manufacturer (FluxOR potassium ion channel assay, Invitrogen AG). Channel activation was carried out as suggested by the supplier by depolarization, accomplished by increasing the extracellular potassium concentration (thallium sulfate containing buffer at concentrations between 10-15 mM Tl⁺ and 30 mM K₂SO₄, FluxOR potassium ion channel assay, Invitrogen AG). To identify blockers of this ion channel the cells were incubated with different concentrations of compounds 5 min before increasing the potassium and thallium concentration. The fluorescence signals by increasing potassium were then compared in the absence and presence of compounds. Dose response curves were established by adding different concentrations of compounds and analyzing the signal. As reference Kᵥ1.3 blockers margatoxin and quinidine in Cl-free buffer were used as described in the literature.

The results of the Kᵥ1.3 assay are depicted in Table 51 below.

### 3. Assays for the Wnt-pathway

For determining the activity of the test compounds on the Wnt-pathway each substances war subjected to two distinct assays: The first (Wnt/Beta-Catenin) assay screened for inhibitors of the Wnt-pathway while the second (MAPK/MEK/B-raf) was conducted in order to confirm that the test compounds modulate the β-catenin-dependent, canonical Wnt pathway and are not acting via a possibly competing β-catenin-independent, noncanonical Wnt messaging system. All examples were inactive in this negative control.

Both assays were performed and analyzed with a Tecan Safire² fluorescence plate reader.

The results are shown in Table 52 below.

### Wnt/Beta-Catenin (APC-/-) - LEF-TCF-bla SW480 - Inhibitor Screen, Constitutively Activated

LEF-TCF-bla SW480 cells are thawed and resuspended in Assay Media (OPTI-MEM, 0.5% dialyzed FBS, 0.1 mM NEAA, 1 mM Sodium Pyruvate, 100 U/mL/100 µg/mL Pen/Strep) to a concentration of 312,500 cells/mL. 4 µL of a 10X serial dilution of ICG-001 (control inhibitor starting concentration, 10,000 nM) or compounds are added to appropriate wells of a TC-Treated assay plate. 32 µL of cell suspension (10,000 cells) is added to the wells. 4 µL of Assay Media is added to all wells to bring the final assay volume to 40 µL. The plate is incubated for 16-24 hours at 37°C/5% CO₂ in a humidified incubator. 8 µL of 1 µM Substrate Loading Solution is added to each well and the plate is incubated for 2 hours at room temperature.

### MAPK/MEK/B-raf - AP1-bla A375 - Inhibitor Screen, Constitutively Activated

AP1-bla A375 cells are thawed and resuspended in Assay Media (OPTI-MEM, 0.5% dialyzed FBS, 0.1 mM NEAA, 1 mM Sodium Pyruvate, 100 U/mL/100 µg/mL Pen/Strep) to a concentration of 312,500 cells/mL. 4 µL of a 10X serial dilution of Raf1 Kinase Inhibitor (control inhibitor starting concentration, 10,000 nM) or compounds are added to appropriate wells of a TC-Treated assay plate. 32 µL of cell suspension (10,000 cells) is added to the wells. 4 µL of Assay Media is added to all wells to bring the final assay volume to 40 µL. The plate is incubated for 16-24 hours at 37°C/5% CO₂ in a humidified incubator. 8 µL of 1 µM Substrate Loading Solution is added to each well and the plate is incubated for 2 hours at room temperature.

### 4. Plasma and metabolic stability assays

Example compounds were dissolved in DMSO/H₂O 90:10 to a final concentration of 10 mM for plasma stability determination and metabolic stability determination.

The assays were conducted according to literature precedents (F.P. Guengerich, Analysis and Characterization of Enzymes; in: Principles and Methods of Toxicology; A.W. Hayes (Ed.) Raven Press: New York, 1989, 777-813; R. Singh et al., In vitro metabolism of a potent HIV-protease inhibitor (141W94) using rat, monkey and human liver S9, Rapid Commun. Mass Spectrom. 1996, 10, 1019-1026).

Results of the stability assays are listed in Table 53 below.

### Plasma stability assay

Human plasma (3-5 donors, Blutspendedienst SRK, Basel) and CD-1 mouse plasma (mixed gender pool >50 animals, Innovative Research, CA, USA) are both sodium citrate stabilized. The assay is performed in triplicates at 10 µM compound concentration and 37°C. Samples are taken at 0, 15, 60, and 240 minutes and stopped by precipitation with 2 volumes of acetonitrile. The supernatant is collected, evaporated and reconstituted in a 5% acetonitrile solution to be analyzed by HPLC/MS/MS. The resulting peak area counts are expressed in percent of the 0 value and used to determine the endpoint stability in % and the half life T½ in minutes. In order to monitor assay integrity the degradation of propantheline is assayed with every experimental set.

### Metabolic stability assay

Microsomes from a human 50 donor mixed gender pool and 1:1 mixtures of microsomes from CD-1 mouse single-gender pools are purchased from Celsis (Belgium). The enzymatic reaction is performed in a buffer containing an NADPH regeneration system and microsomes with the following end concentrations: 100 mM potassium phosphate buffer (all from Sigma), 1 mg/mL glucose-6-phosphate, 1 mg/mL β-nicotinamide adenine dinucleotide phosphate (NADP), 0.65 mg/mL magnesium chloride, 0.8 units/mL of glucose-6-phosphate dehydrogenase (prediluted with 5 mM citrate buffer), 10 µM compound and 1 mg/ml microsomal protein. Compounds are incubated at 37°C in duplicates and samples are taken after 0, 20 and 60 minutes. After acetonitrile precipitation (2 volumes) and HPLC/MS/MS analysis metabolic turnover is expressed in % of the initial 0 minutes value and half life T½ (min) is calculated. Verapamil for human and propranolol for mouse are used as reference and are assayed with every experimental set.

### 5. Pharmacokinetic study (PK) analysis

Pharmacokinetic analyses after single intravenous (i.v.) and two different peroral (p.o.) administrations were performed for the test compounds. 240 grams (± 10 gram) male SD rats were catheterized and used in the study. The vehicle, Solutol HS-15/ 0.9% saline (5:95), was added to give a final concentration of 0.33 mg/ml of the compounds for i.v. application, and 0.5 or 2.0 mg/ml for p.o. applications. The volumes used for applications were 3 ml/kg i.v. and 10 ml/kg p.o. resulting in a final dose of 1 mg/kg for the intravenous dose and 5 or 20 mg/kg for the peroral doses.

Blood samples were withdrawn via the catheter at 1) predose, 5, 15, 30, 60, 120, 240, and 480 minutes for the i.v. application and 2) predose, 15, 30, 60, 120, 240, 480 and 720 minutes for the p.o. applications. Plasma was prepared by centrifugation of heparinized whole blood. Plasma samples (∼120 µl each time point, Li-Heparin, 20 IE/ml) were stored at -80°C until HPLC-MSMS analysis.

### Preparation of the plasma calibration samples:

"Blank" rat plasma from untreated animals was used. Aliquots of plasma of 50 µL each were spiked with Internal Standard (ISTD) and with known amounts test compounds in order to obtain 11 plasma calibration samples in the range 0.25-4000 ng/mL.

### Preparation of plasma samples:

To 50 µL of sample from treated animals 20 µL of internal standard were added. After mixing with 200 µL of acetonitrile, 2% HCOOH were added and each sample was vortexed for several seconds. Precipitated material was removed by centrifugation for 10 min (13200 rpm), the supernatant removed and dried under a constant flow of N2 (Porvair, MiniVap). To reconstitute the dried samples, 200 µL of [DMSO/acetonitrile/H₂O, 4/4/2 (v/v/v) + 2% HCOOH] were added and the sample was shaken for at least 5 minutes.

### LC-MSMS Analysis of samples:

Reconstituted samples were analysed by HPLC/MSMS on a reverse phase analytical column (BEH C18, 100 x 2.1 mm, 1.7 µm, Waters). Mobile phase A: H₂O/acetonitrile (95:5, v:v) 0.1% formic acid, mobile phase B: Acetonitrile/H2O (95:5, v:v) 0.1% formic acid. Compounds were eluted from the column by using a gradient from 1%B to 60%B in 2.5 min.).

### Pharmacokinetic evaluation:

PK analysis was performed on mean values (generally n=3) using WinNonLin software (Version 5.3, Pharsight Corporation). The area under the curve AUC was calculated by the linear trapezoidal rule (linear interpolation, uniform weighting). AUC_{(t-∞)} was estimated as Ct/b (b: elimination rate constant). AUC_{(t-∞)} is the sum of AUC₍₀₋ₜ₎ and AUC_{(t-∞)}. Elimination half-life was calculated by the linear regression on at least three data points during the elimination phase. The time intervals selected for the half-life determinations were evaluated by the correlation coefficient (r²), which should be at least above 0.85 and ideally above 0.96.

In case of i.v. administration the initial concentration at t_{zero} was determined by extrapolation of the curve through the first two time points. Finally bioavailability after p.o. administration was calculated from the dose normalized AUC_{(0-∞)} ration after p.o. *versus* i.v. administration.

The results are shown in Table 54 below.

**Table 50.1: Motilin Receptor Assay**

| **No** | **Antagonist activity [% Inhibition at 10 µM]** | **Antagonist activity IC₅₀ [µM]]** |
|---|---|---|
| **Ex.11** | 79 | 0.78 |
| **Ex.12** | 95 | 2.7 |
| **Ex.48** | 29 | n.d. |
| **Ex.49** | 98 | 0.16 |
| **Ex.482** | 62 | 1.1 |
| **Ex.483** | 100 | 0.023 |
| **Ex.484** | 99 | 0.20 |
| **Ex.485** | 93 | 0.28 |
| **Ex.486** | 93 | 0.18 |
| **Ex.487** | 47 | n.d. |
| **Ex.488** | 23 | n.d. |
| **Ex.495** | 26 | n.d. |
| **Ex.496** | 31 | n.d. |
| **Ex.497** | 24 | n.d. |

**Table 50.2: FP Receptor Assay (continued on the following pages)**

| **No** | **Antagonist activity [% Inhibition at 10 µM]** | **Antagonist activity IC₅₀ [µM]** |
|---|---|---|
| **Ex.12** | 44 | n.d. |
| **Ex.184** | 74 | 0.52 |
| **Ex.186** | 53 | 1.2 |
| **Ex.200** | 38 | 28 |
| **Ex.213** | 78 | 1.7 |
| **Ex.386** | 85 | 2.7 |
| **Ex.388** | 99 | 0.32 |
| **Ex.391** | 93 | 0.25 |
| **Ex.504** | 62 | 0.441 |
| **Ex.505** | 47 | 0.64 |
| **Ex.506** | 55 | 2.2 |
| **Ex.507** | 51 | 1.1 |
| **Ex.508** | 93 | 0.43 |
| **Ex.509** | 54 | 2.1 |
| **Ex.510** | 42 | 1.5 |
| **Ex.511** | 99 | 0.059 |
| **Ex.512** | 97 | 0.120 |
| **Ex.513** | 95 | 0.65 |
| **Ex.515** | 32 | 5.1 |
| **Ex.516** | 97 | 0.59 |
| **Ex.517** | 98 | 0.65 |
| **Ex.518** | 80 | 0.49 |
| **Ex.519** | n.d. | 3.0 |
| **Ex.521** | 81 | 1.0 |
| **Ex.522** | 95 | 0.35 |
| **Ex.523** | 92 | 0.86 |
| **Ex.524** | 85 | 1.7 |
| **Ex.525** | 99 | 0.094 |
| **Ex.526** | 99 | 0.180 |
| **Ex.527** | 73 | 0.204 |
| **Ex.537** | 75 | n.d. |
| **Ex.538** | 80 | n.d. |
| **Ex.539** | 59 | n.d. |
| **Ex.540** | 43 | n.d. |
| **Ex.541** | 65 | n.d. |
| **Ex.542** | 74 | n.d. |
| **Ex.543** | 82 | n.d. |
| **Ex.544** | 81 | n.d. |
| **Ex.545** | 80 | n.d. |
| **Ex.546** | 37 | n.d. |
| **Ex.547** | 47 | n.d. |
| **Ex.548** | 69 | n.d. |
| **Ex.549** | 62 | n.d. |
| **Ex.550** | 73 | n.d. |
| **Ex.551** | 72 | n.d. |
| **Ex.552** | 71 | n.d. |

**Table 50.3: 5-HT_{2B} Receptor Assay**

| **No** | **Agonist activity [% activation at 12.5 µM]** | **Agonist activity EC₅₀[µM]** |
|---|---|---|
| **Ex.9** | 48 | 12 |
| **Ex.12** | 36 | 3.3 |
| **Ex.16** | 45 | 6.6 |
| **Ex.30** | 48 | 3.3 |

**Table 50.4: P2Y₁ Receptor Assay**

| **No** | **Antagonist [% Inhibition at 10 µM]** | **Antagonist activity IC₅₀ [µM]** |
|---|---|---|
| **Ex.316** | 57 | n.d. |
| **Ex.321** | 45 | 7.5 |
| **Ex.320** | 19 | n.d. |
| **Ex.322** | 60 | n.d. |

**Table 51: K_{v1,3} Ionchannel Assay**

| **No** | **K_{v1,3} antagonist activity [% Inhibition at 10 µM]** | **K_{v1,3} antagonist activity IC50 [µM]]** |
|---|---|---|
| **Ex.166** | 20 | n.d. |
| **Ex.168** | 44 | 1.69 |
| **Ex.170** | 10 | n.d. |
| **Ex.171** | 12 | n.d. |
| **Ex.177** | 54 | 3.5 |

**Table 52: Wnt-Pathway Assays**

| **No** | **TCF/LEF receptor gene activity [% inhibition at 10 µM]** | **TCF/LEF receptor gene activity IC₅₀ [µM]** | **AP receptor gene activity IC₅₀ [µM]** |
|---|---|---|---|
| **Ex.501** | 100 | 1.1 | > 50 |
| **Ex.502** | 82 | 5.5 | > 50 |
| **Ex.503** | 68 | 5.8 | > 50 |

**Table 53: Plasma Stability and Metabolic Stability Assays of Selected Examples (continued on the following pages)**

| | **Plasma Stability** | | | | **Metabolic Stability** | | | |
|---|---|---|---|---|---|---|---|---|
| **No** | **T ½ [min] hum** | **240 min hum [% remain.]** | **T ½ [min] mouse/ (rat)** | **240 min mouse/ (rat) [% remain.]** | **T ½ [min] hum** | **60 min hum [% remain.]** | **T ½ [min] mouse/ (rat)** | **60 min mouse/ (rat) [% remain.]** |
| **Ex.9** | 240 | 99 | 240 | 93 | 32 | 20 | 60 | 80 |
| **Ex.11** | 240 | 100 | 240 | 100 | 60 | 74 | 60 | 77 |
| **Ex.12** | 240 | 99 | 240 | 100 | 17 | 7 | 35 | 33 |
| **Ex.16** | 240 | 95 | 240 | 97 | 38 | 31 | 60 | 79 |
| **Ex.30** | 240 | 85 | 240 | 100 | 22 | 2 | 60 | 55 |
| **Ex.37** | 240 | 77 | 240 | 100 | 60 | 100 | 60 | 100 |
| **Ex.43** | 240 | 82 | 240 | 89 | 60 | 100 | 60 | 98 |
| **Ex.49** | 240 | 83 | 240 | 96 | 24 | 10 | 60 | 83 |
| **Ex.78** | 240 | 65 | 240 | 100 | 60 | 78 | 60 | 100 |
| **Ex.91** | 240 | 96 | 240 | 88 | 60 | 91 | 60 | 95 |
| **Ex.93** | 240 | 100 | 240 | 100 | 24 | 1 | 29 | 15 |
| **Ex.95** | 240 | 100 | 240 | 93 | 60 | 76 | 60 | 94 |
| **Ex.98** | 240 | 100 | 240 | 78 | 60 | 97 | 60 | 100 |
| **Ex.102** | 240 | 65 | 240 | 75 | 23 | 4 | 42 | 39 |
| **Ex.103** | 240 | 97 | 240 | 75 | 35 | 22 | 36 | 25 |
| **Ex.138** | 240 | 89 | 240 | 78 | 60 | 62 | 60 | 99 |
| **Ex.184** | 240 | 66 | 240 | 58 | 15 | 0 | 22 | 0 |
| **Ex.200** | 240 | 91 | 240 | 100 | 27 | 12 | 30 | 26 |
| **Ex.208** | 240 | 98 | 240 | 90 | 60 | 84 | 60 | 100 |
| **Ex.213** | n.d. | n.d. | n.d. | n.d. | 16 | 0 | 17 | 4 |
| **Ex.230** | 240 | 100 | 240 | 95 | 60 | 61 | 60 | 90 |
| **Ex.260** | 240 | 100 | 240 | 100 | 37 | 19 | 60 | 69 |
| **Ex.262** | 240 | 100 | 240 | 93 | 21 | 0 | 23 | 4 |
| **Ex.264** | 240 | 94 | 240 | 88 | n.d. | n.d. | 42 | 32 |
| **Ex.266** | 240 | 92 | 240 | 74 | 60 | 100 | 60 | 98 |
| **Ex.267** | 240 | 75 | 240 | 79 | 60 | 99 | 60 | 100 |
| **Ex.272** | 240 | 95 | 240 | 92 | 60 | 65 | 60 | 81 |
| **Ex.482** | 240 | 100 | 240 | 100 | 22 | 11 | 60 | 84 |
| **Ex.483** | 240 | 100 | 240 | 100 | 60 | 55 | 60 | 85 |
| **Ex.484** | 240 | 100 | 240 | 90 | 21 | 11 | 60 | 100 |
| **Ex.485** | 240 | 100 | 240 | 99 | 14 | 8 | 17 | 11 |
| **Ex.486** | 240 | 100 | 240 | 98 | 23 | 13 | 60 | 100 |
| **Ex.168** | 240 | 85 | 240 | 99 | 25 | 17 | 26 | 21 |
| **Ex.170** | 240 | 92 | 240 | 100 | 60 | 60 | 60 | 86 |
| **Ex.167** | n.d | n.d. | 35 | 17 | 60 | 58 | 60 | 65 |
| **Ex.505** | 240 | 100 | 240 | 98 | 21 | 9 | 13 | 3 |
| **Ex.511** | 240 | 100 | n.d. | n.d. | 2 | 1 | 14 | 14 |
| **Ex.512** | 240 | 86 | n.d. | n.d. | 17 | 8 | 12 | 10 |
| **Ex.525** | 240 | 100 | n.d. | n.d. | 16 | 13 | 25 | 29 |
| **Ex.527** | 109 | 26 | n.d. | n.d. | 22 | 23 | 20 | 24 |
| **Ex.533** | 240 | 95 | 240 | 100 | 60 | 59 | 60 | 93 |
| **Ex.561** | 240 | 99 | 240 | 100 | 39 | 42 | 60 (56) | 79 (53) |
| **Ex.568** | 240 | 97 | 240 | 89 | 60 | 53 | 60 (60) | 60 (57) |
| **Ex.563** | 240 | 88 | 240 | 81 | 8 | 1 | 12 (31) | 4 (32) |
| **Ex.570** | 240 | 92 | 240 | 100 | 13 | 20 | 60 (60) | 69 (76) |
| **Ex.571** | 240 | 86 | 240 | 89 | 60 | 90 | 60 (60) | 74 (88) |
| **Ex.572** | 240 | 85 | 240 | 100 | 54 | 49 | 60 (60) | 54 (92) |
| **Ex.574** | 240 | 91 | 240 | 95 | 60 | 94 | 60 (60) | 93 (92) |
| **Ex.577** | 240 | 87 | 240 | 101 | 47 | 41 | 27 | 25 |
| **Ex.578** | 240 | 100 | 240 | 86 | 52 | 46 | 60 | 55 |
| **Ex.580** | 240 | 70 | (240) | (79) | 60 | 100 | n.d. | n.d. |
| **Ex.581** | 240 | 70 | (210) | (77) | 60 | 98 | 60 | 100 |
| **Ex.582** | 240 | 100 | 240 | 96 | 60 | 100 | 60 | 98 |
| **Ex.583** | 240 | 91 | 240 | 83 | 60 | 100 | 60 | 95 |
| **Ex.368** | 240 | 72 | (240) | (100) | 60 | 83 | 60 (60) | 82 (98) |
| **Ex.369** | 240 | 85 | (240) | (79) | 60 | 100 | 60 (60) | 80 (100) |
| **Ex.372** | 240 | 68 | (240) | (100) | 46 | 43 | 60 (60) | 73 (72) |
| **Ex.373** | 240 | 55 | (240) | (100) | 12 | 12 | 36 (7) | 39 (0) |
| **Ex.374** | 240 | 57 | (240) | (82) | 60 | 61 | 60 (60) | 94 (90) |
| **Ex.376** | 240 | 90 | (240) | (89) | 60 | 79 | 60 (60) | 91 (78) |
| **Ex.377** | 240 | 95 | (240) | (100) | 60 | 72 | 60 (60) | 86 (97) |
| **Ex.378** | 240 | 48 | (240) | (100) | 60 | 65 | 60 (60) | 87 (72) |
| **Ex.403** | 240 | 100 | 240 | 65 | 36 | 37 | 57 | 55 |
| **Ex.406** | 240 | 75 | 240 | 89 | 60 | 77 | 60 (60) | 94 (96) |
| **Ex.407** | 240 | 80 | 240 | 98 | 51 | 47 | 60 | 84 |

**Table 54: PK-Data of Selected Examples**

| | | **Ex.561** | | | **Ex.568** | | | **Ex.571** | | |
|---|---|---|---|---|---|---|---|---|---|---|
| | Route | **iv** | **po** | **po** | **iv** | **po** | **po** | **iv** | **po** | **po** |
| Parameter | mg/kg | **1** | **5** | **20** | **1** | **5** | **20** | **1** | **5** | **20** |
| R² | | 0.997 | 1.000 | 0.968 | 0.979 | 0.999 | 0.992 | 1.000 | 0.990 | 0.991 |
| T_{1/2} (terminal) | hr | 2.11 | 3.00 | 2.71 | 1.67 | 1.92 | 2.07 | 1.70 | 2.18 | 2.30 |
| Tmax | hr | 0.08 | 0.50 | 0.25 | 0.08 | 0.25 | 0.25 | 0.08 | 0.25 | 0.25 |
| Cmax | ng/mL | 183.67 | 67.00 | 248.00 | 194.67 | 64.33 | 379.67 | 643.67 | 340.33 | 1516.67 |
| Cmax dose normalized | kg*ng/mL/mg | 183.67 | 13.40 | 12.40 | 194.67 | 12.87 | 18.98 | 643.67 | 68.07 | 75.83 |
| C₀ | ng/mL | 240.24 | | | 284.59 | | | 932.47 | | |
| V (terminal) | mL/kg | 13831.40 | | | 14320.56 | | | 7618.59 | | |
| AUC_{(t-∞)} dose normalized | hr*kg*ng/mL/mg | 219.89 | 24.00 | 36.88 | 167.93 | 29.83 | 36.22 | 321.32 | 93.34 | 87.49 |
| **Bioavailability** | **%** | | **10.9** | **16.8** | | **17.8** | **21.6** | | **29.0** | **27.2** |

## Claims

1. Compounds consisting of a cyclic arrangement of the building blocks **A, B** and **C** and represented by the general formulae **Ia** or **Ib** 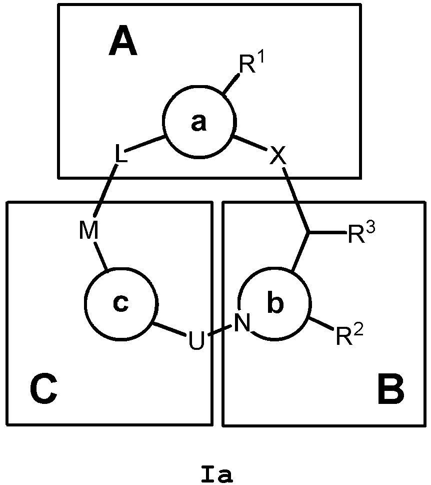 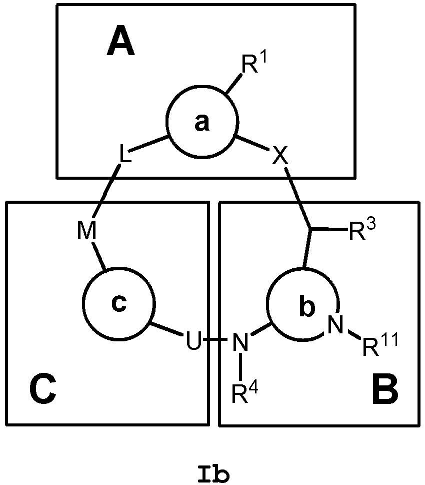 wherein
**building block A** ("Template") is represented by 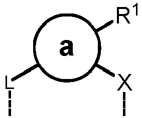
**building block B** ("Modulator") is represented by 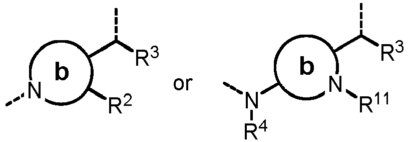
**building block C** ("Bridge") is represented by 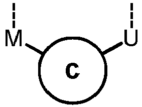 respectively 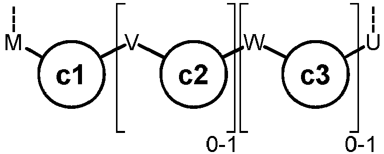 and wherein
M represents a divalent or trivalent radical selected from the group of 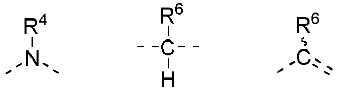
forming an integral part of the
M-L connectivity which in turn represents a divalent radical selected from the group of 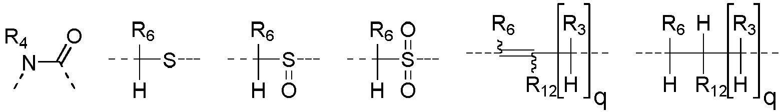
U represents a divalent radical selected from the group of 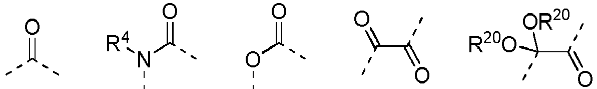
X represents a divalent radical selected from the group of 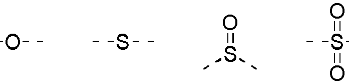
V and W are independently representing a divalent radical selected from the group of 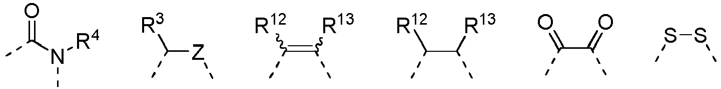
and wherein
said **building block A** is a bivalent radical selected from the group of
| | | | |
|---|---|---|---|
| 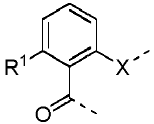 | 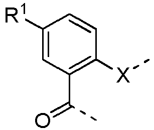 | 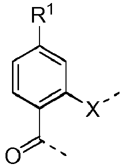 | 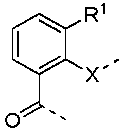 |
| **A1** (a1) | **A2** (a1) | **A3** (a1) | **A4** (a1) |
| 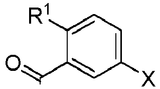 | 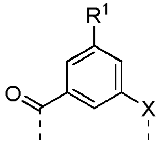 | 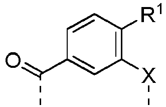 | 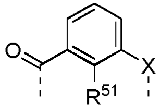 |
| **A5** (a1) | **A6** (a1) | **A7** (a1) | **A8** (a1) |
| 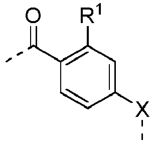 | 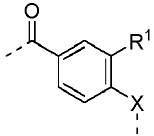 | 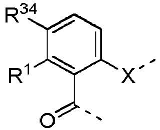 | 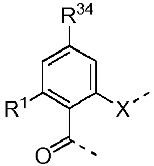 |
| **A9** (a1) | **A10** (a1) | **A11** (a1) | **A12** (a1) |
| 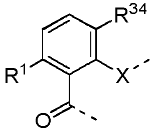 | 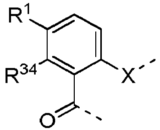 | 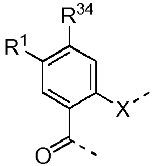 | 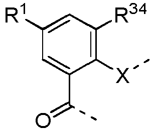 |
| **A13** (a1) | **A14** (a1) | **A15** (a1) | **A16** (a1) |
| 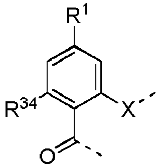 | 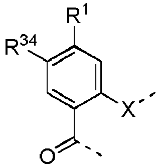 | 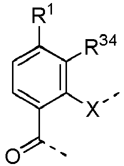 | 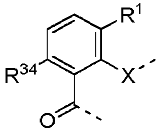 |
| **A17** (a1) | **A18** (a1) | **A19** (a1) | **A20** (a1) |
| 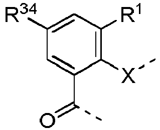 | 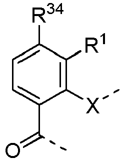 | 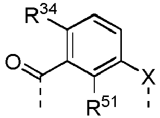 | 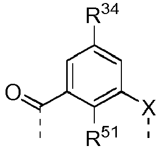 |
| **A21** (a1) | **A22** (a1) | **A23** (a1) | **A29** (a1) |
| 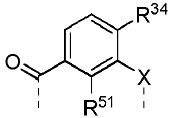 | 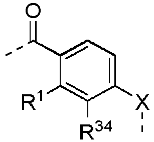 | 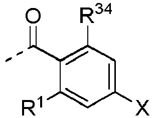 | 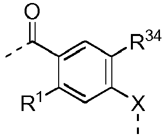 |
| **A25** (a1) | **A26** (a1) | **A27** (a1) | **A28** (a1) |
| 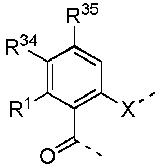 | 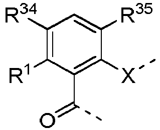 | 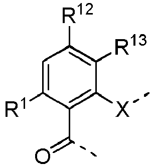 | 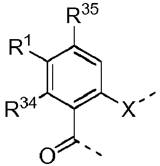 |
| **A29** (a1) | **A30** (a1) | **A31** (a1) | **A32** (a1) |
| 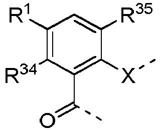 | 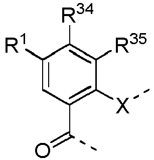 | 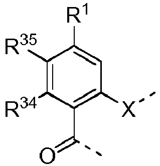 | 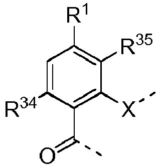 |
| **A33** (a1) | **A34** (a1) | **A35** (a1) | **A36** (a1) |
| 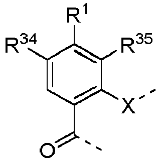 | 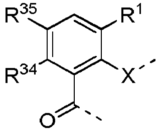 | 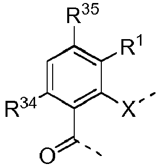 | 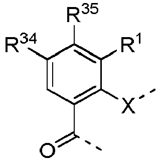 |
| **A37** (a1) | **A38** (a1) | **A39** (a1) | **A40** (a1) |
| 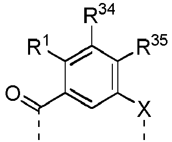 | 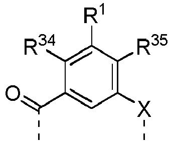 | 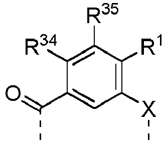 | 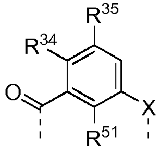 |
| **A41** (a1) | **A42** (a1) | **A43** (a1) | **A44** (a1) |
| 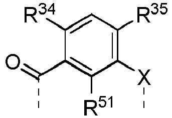 | 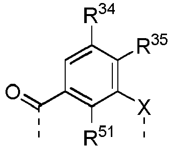 | 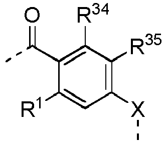 | 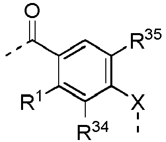 |
| **A45** (a1) | **A46** (a1) | **A47** (a1) | **A48** (a1) |
| 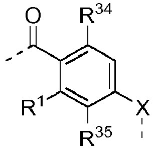 | 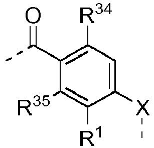 | 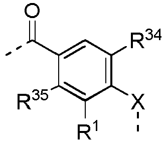 | 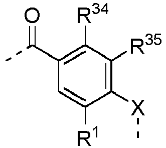 |
| **A49** (a1) | **A50** (a1) | **A51** (a1) | **A52** (a1) |
| 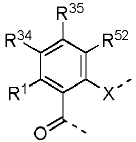 | 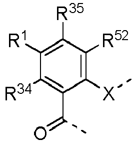 | 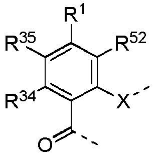 | 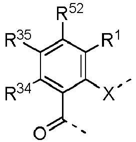 |
| **A53** (a1) | **A59** (a1) | **A55** (a1) | **A56** (a1) |
| 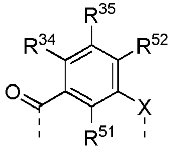 | 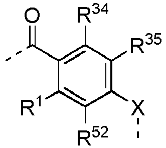 | 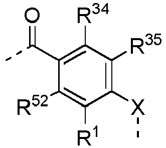 | 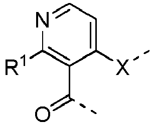 |
| **A57** (a1) | **A58** (a1) | **A59** (a1) | **A60** (a2) |
| 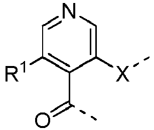 | 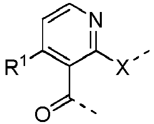 | 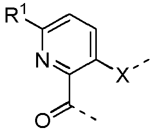 | 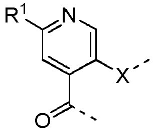 |
| **A61** (a2) | **A62** (a2) | **A63** (a2) | **A64** (a2) |
| 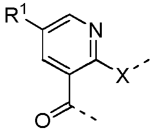 | 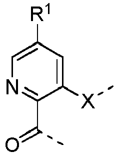 | 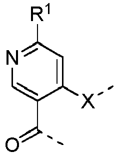 | 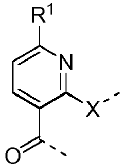 |
| **A65** (a2) | **A66** (a2) | **A67** (a2) | **A68** (a2) |
| 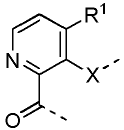 | 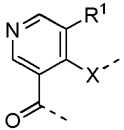 | 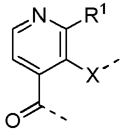 | 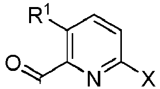 |
| **A69** (a2) | **A70** (a2) | **A71** (a2) | **A72** (a2) |
| 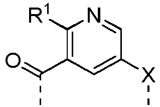 | 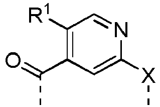 | 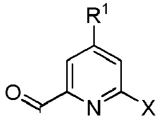 | 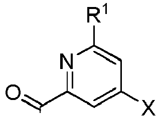 |
| **A73** (a2) | **A74** (a2) | **A75** (a2) | **A76** (a2) |
| 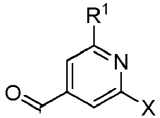 | 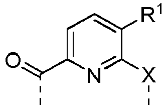 | 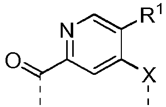 | 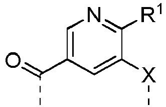 |
| **A77** (a2) | **A78** (a2) | **A79** (a2) | **A80** (a2) |
| 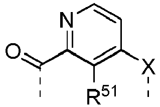 | 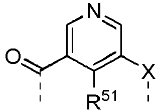 | 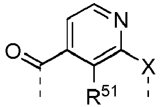 | 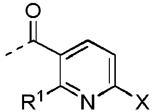 |
| **A81** (a2) | **A82** (a2) | **A83** (a2) | **A84** (a2) |
| 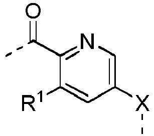 | 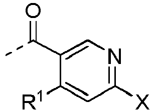 | 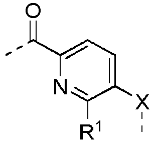 | 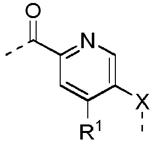 |
| **A85** (a2) | **A86** (a2) | **A87** (a2) | **A88** (a2) |
| 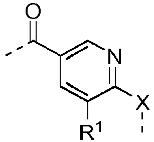 | 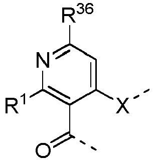 | 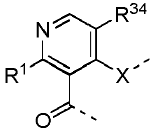 | 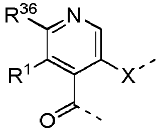 |
| **A89** (a2) | **A90** (a2) | **A91** (a2) | **A92** (a2) |
| 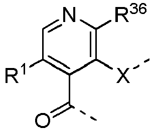 | 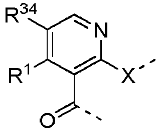 | 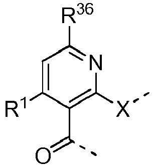 | 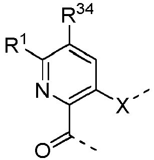 |
| **A93** (a2) | **A94** (a2) | **A95** (a2) | **A96** (a2) |
| 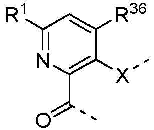 | 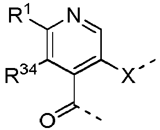 | 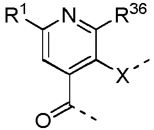 | 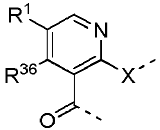 |
| **A97** (a2) | **A98** (a2) | **A99** (a2) | **A100** (a2) |
| 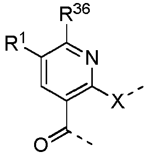 | 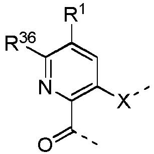 | 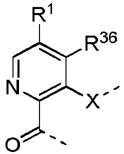 | 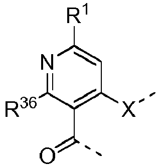 |
| **A101** (a2) | **A102** (a2) | **A103** (a2) | **A104** (a2) |
| 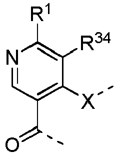 | 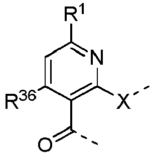 | 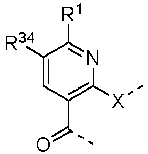 | 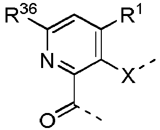 |
| **A105** (a2) | **A106** (a2) | **A107** (a2) | **A108** (a2) |
| 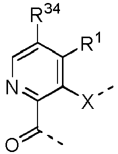 | 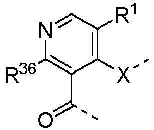 | 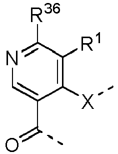 | 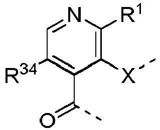 |
| **A109** (a2) | **A110** (a2) | **A111** (a2) | **A112** (a2) |
| 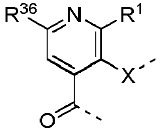 | 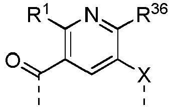 | 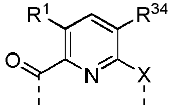 | 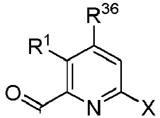 |
| **A113** (a2) | **A114** (a2) | **A115** (a2) | **A116** (a2) |
| 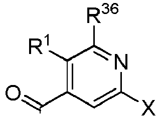 | 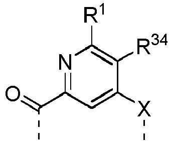 | 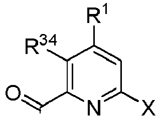 | 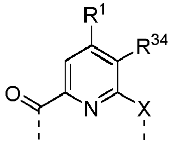 |
| **A117** (a2) | **A118** (a2) | **A119** (a2) | **A120** (a2) |
| 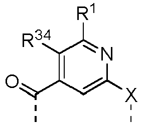 | 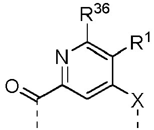 | 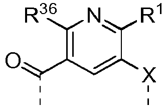 | 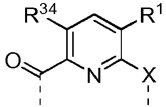 |
| **A121** (a2) | **A122** (a2) | **A123** (a2) | **A124** (a2) |
| 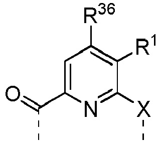 | 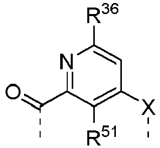 | 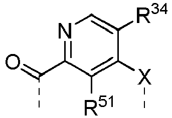 | 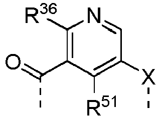 |
| **A125** (a2) | **A126** (a2) | **A127** (a2) | **A128** (a2) |
| 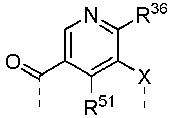 | 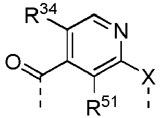 | 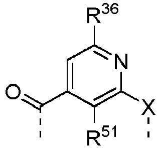 | 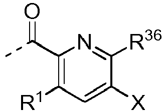 |
| **A129** (a2) | **A130** (a2) | **A131** (a2) | **A132** (a2) |
| 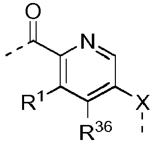 | 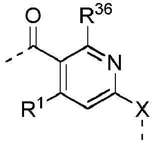 | 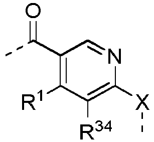 | 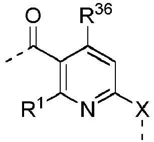 |
| **A133** (a2) | **A134** (a2) | **A135** (a2) | **A136** (a2) |
| 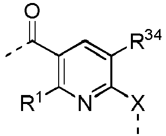 | 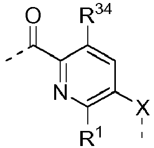 | 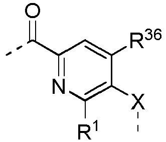 | 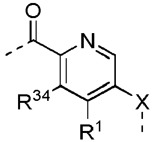 |
| **A137** (a2) | **A138** (a2) | **A139** (a2) | **A140** (a2) |
| 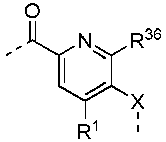 | 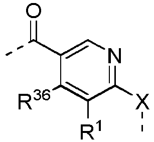 | 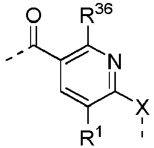 | 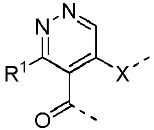 |
| **A141** (a2) | **A142** (a2) | **A143** (a2) | **A144** (a3) |
| 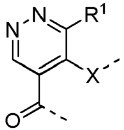 | 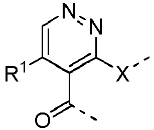 | 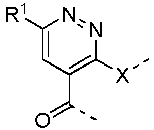 | 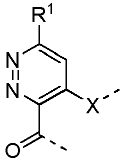 |
| **A145** (a3) | **A146** (a3) | **A147** (a3) | **A148** (a3) |
| 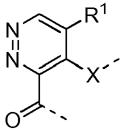 | 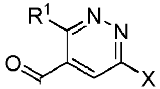 | 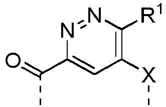 | 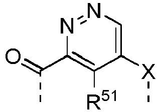 |
| **A149** (a3) | **A150** (a3) | **A151** (a3) | **A152** (a3) |
| 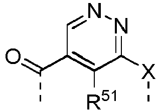 | 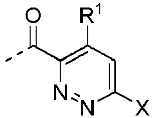 | 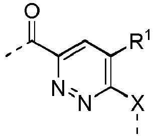 | 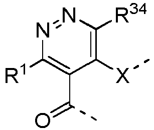 |
| **A153** (a3) | **A154** (a3) | **A155** (a3) | **A156** (a3) |
| 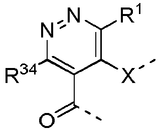 | 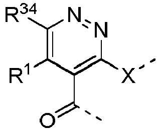 | 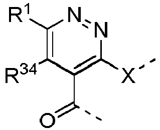 | 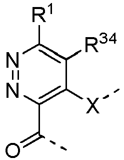 |
| **A157** (a3) | **A158** (a3) | **A159** (a3) | **A160** (a3) |
| 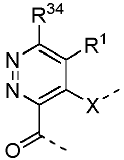 | 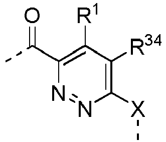 | 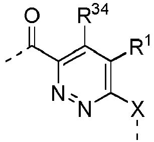 | 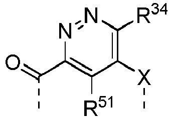 |
| **A161** (a3) | **A162** (a3) | **A163** (a3) | **A169** (a3) |
| 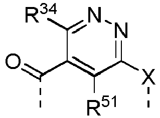 | 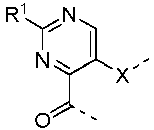 | 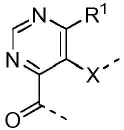 | 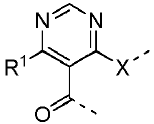 |
| **A165** (a3) | **A166** (a4) | **A167** (a4) | **A168** (a4) |
| 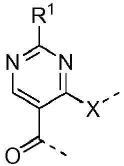 | 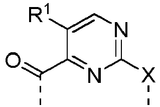 | 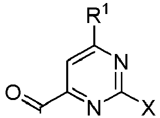 | 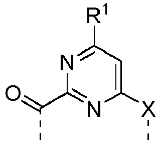 |
| **A169** (a4) | **A170** (a4) | **A171** (a4) | **A172** (a4) |
| 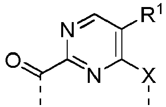 | 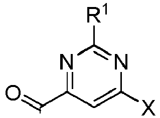 | 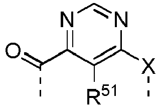 | 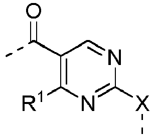 |
| **A173** (a4) | **A174** (a4) | **A175** (a4) | **A176 (a4)** |
| 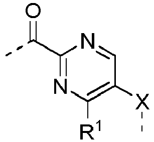 | 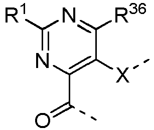 | 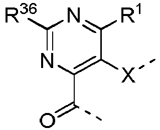 | 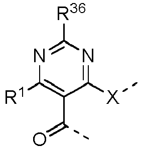 |
| **A177** (a4) | **A178** (a4) | **A179 (a4)** | **A180** (a4) |
| 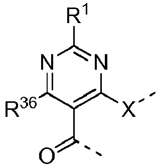 | 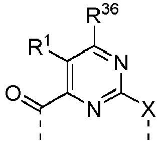 | 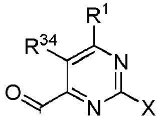 | 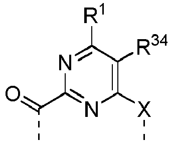 |
| **A181** (a4) | **A182** (a4) | **A183** (a4) | **A184** (a4) |
| 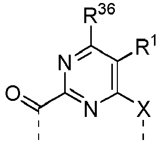 | 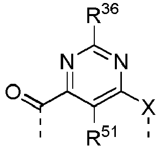 | 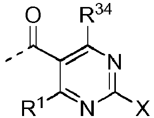 | 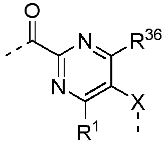 |
| **A185** (a4) | **A186** (a4) | **A187** (a4) | **A188** (a4) |
| 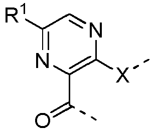 | 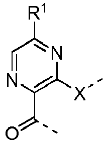 | 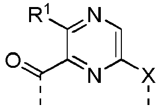 | 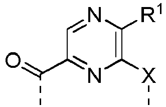 |
| **A189** (a5) | **A190** (a5) | **A191** (a5) | **A192** (a5) |
| 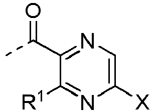 | 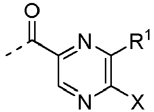 | 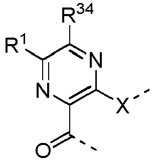 | 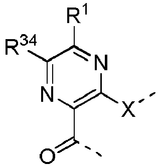 |
| **A193** (a5) | **A194** (a5) | **A195** (a5) | **A196** (a5) |
| 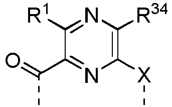 | 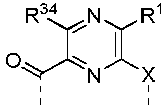 | 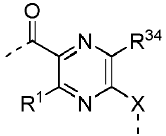 | 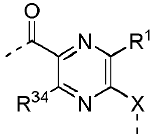 |
| **A197** (a5) | **A198** (a5) | **A199** (a5) | **A200** (a5) |
| 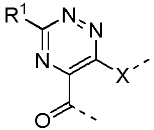 | 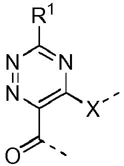 | 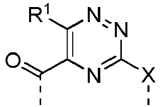 | 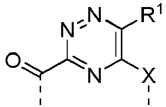 |
| **A201** (a6) | **A202** (a6) | **A203** (a6) | **A204** (a6) |
| 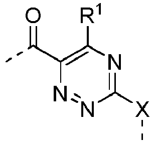 | 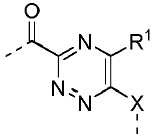 | 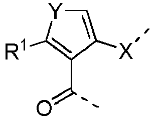 | 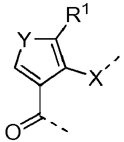 |
| **A205** (a6) | **A206** (a6) | **A207** (a7) | **A208** (a7) |
| 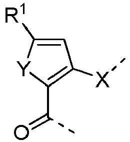 | 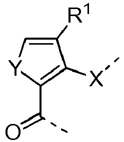 | 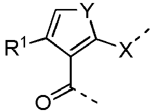 | 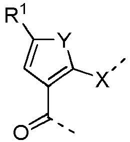 |
| **A209** (a7) | **A210** (a7) | **A211** (a7) | **A212** (a7) |
| 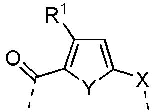 | 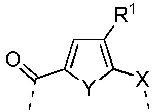 | 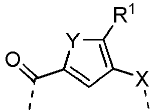 | 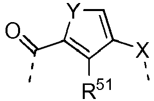 |
| **A213** (a7) | **A214** (a7) | **A215** (a7) | **A216** (a7) |
| 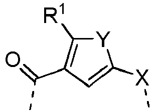 | 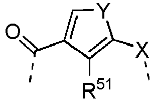 | 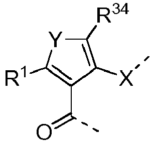 | 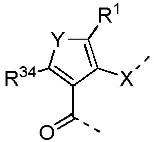 |
| **A217** (a7) | **A218** (a7) | **A219** (a7) | **A220** (a7) |
| 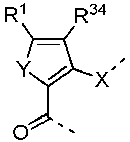 | 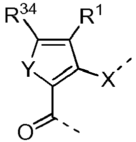 | 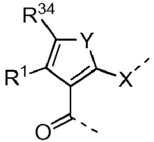 | 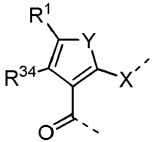 |
| **A221** (a7) | **A222** (a7) | **A223** (a7) | **A224** (a7) |
| 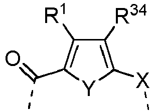 | 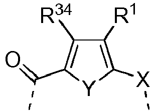 | 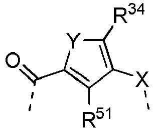 | 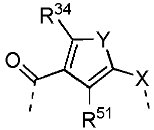 |
| **A225** (a7) | **A226** (a7) | **A227** (a7) | **A228** (a7) |
| 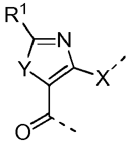 | 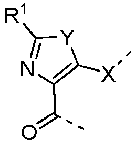 | 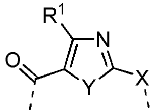 | 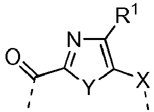 |
| **A229** (a8) | **A230** (a8) | **A231** (a8) | **A232** (a8) |
| 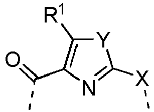 | 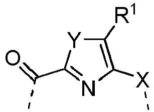 | 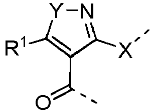 | 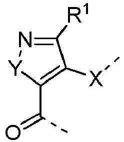 |
| **A233** (a8) | **A234** (a8) | **A235** (a9) | **A236** (a9) |
| 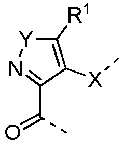 | 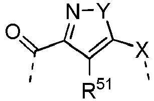 | 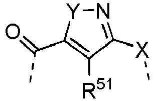 | 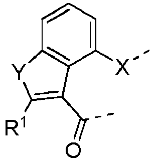 |
| **A237** (a9) | **A238** (a9) | **A239** (a9) | **A240** (a10) |
| 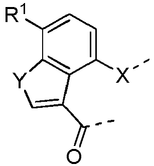 | 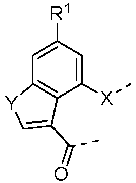 | 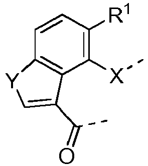 | 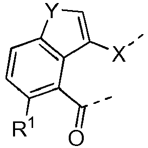 |
| **A241** (a10) | **A242 (a10)** | **A243** (a10) | **A244** (a10) |
| 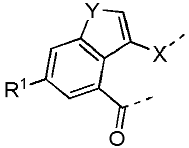 | 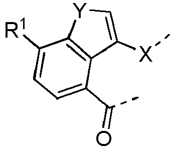 | 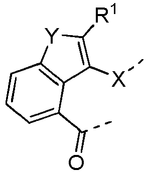 | 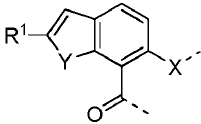 |
| **A245** (a10) | **A246** (a10) | **A247** (a10) | **A248** (a10) |
| 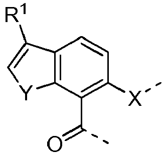 | 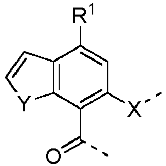 | 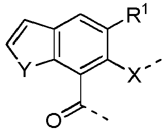 | 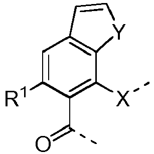 |
| **A249** (a10) | **A250** (a10) | **A251** (a10) | **A252** (a10) |
| 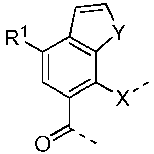 | 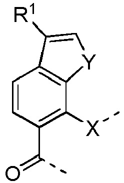 | 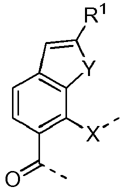 | 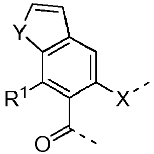 |
| **A253** (a10) | **A254** (a10) | **A255** (a10) | **A256** (a10) |
| 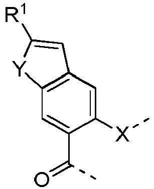 | 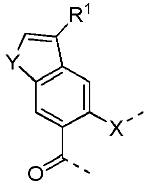 | 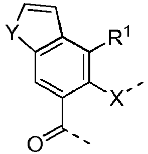 | 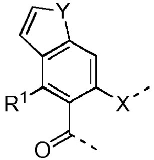 |
| **A257** (a10) | **A258** (a10) | **A259** (a10) | **A260** (a10) |
| 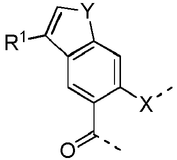 | 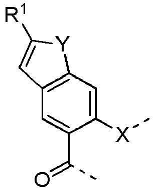 | 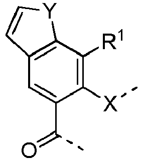 | 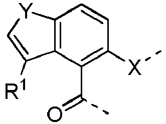 |
| **A261** (a10) | **A262** (a10) | **A263** (a10) | **A264** (a10) |
| 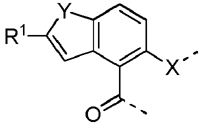 | 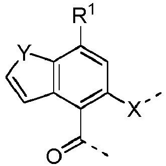 | 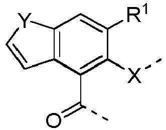 | 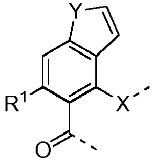 |
| **A265** (a10) | **A266** (a10) | **A267** (a10) | **A268** (a10) |
| 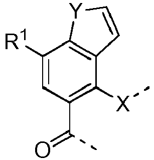 | 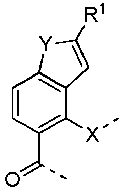 | 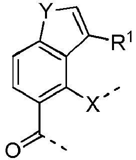 | 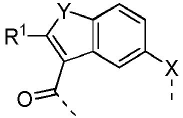 |
| **A269** (a10) | **A270** (a10) | **A271** (a10) | **A272** (a10) |
| 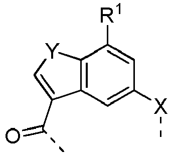 | 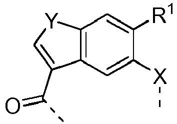 | 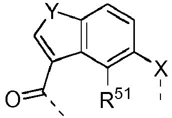 | 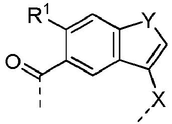 |
| **A273** (a10) | **A274** (a10) | **A275** (a10) | **A276** (a10) |
| 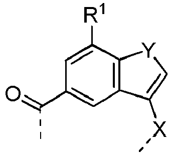 | 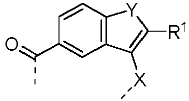 | 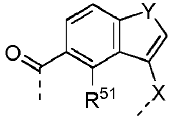 | 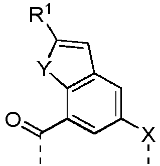 |
| **A277** (a10) | **A278** (a10) | **A279** (a10) | **A280** (a10) |
| 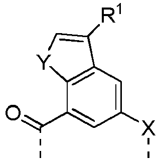 | 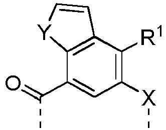 | 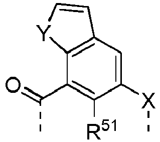 | 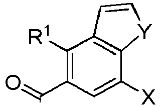 |
| **A281** (a10) | **A282** (a10) | **A283** (a10) | **A284** (a10) |
| 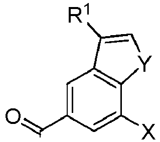 | 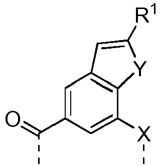 | 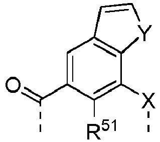 | 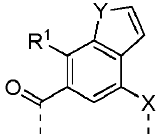 |
| **A285** (a10) | **A286** (a10) | **A287** (a10) | **A288** (a10) |
| 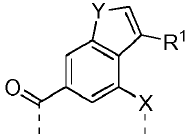 | 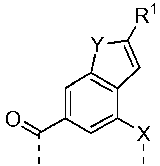 | 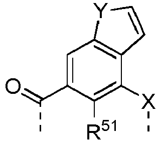 | 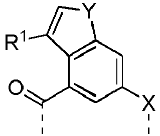 |
| **A289** (a10) | **A290** (a10) | **A291** (a10) | **A292** (a10) |
| 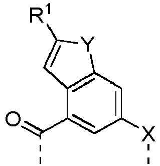 | 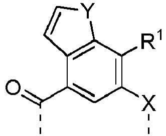 | 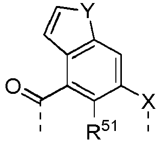 | 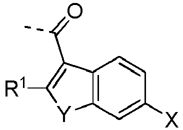 |
| **A293** (a10) | **A294** (a10) | **A295** (a10) | **A296** (a10) |
| 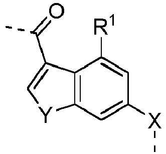 | 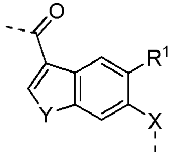 | 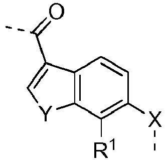 | 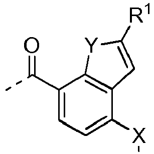 |
| **A297** (a10) | **A298** (a10) | **A299** (a10) | **A300** (a10) |
| 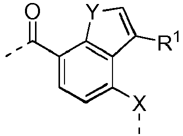 | 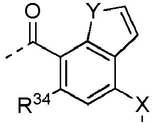 | 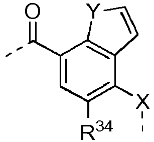 | 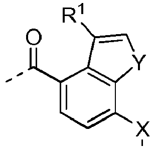 |
| **A301** (a10) | **A302** (a10) | **A303** (a10) | **A304** (a10) |
| 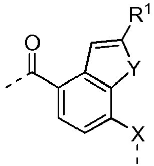 | 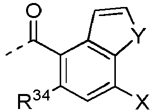 | 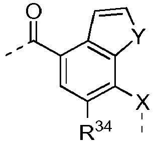 | 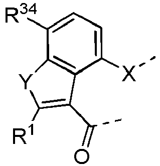 |
| **A305** (a10) | **A306** (a10) | **A307** (a10) | **A308** (a10) |
| 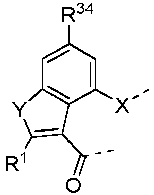 | 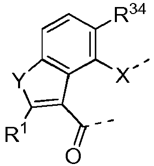 | 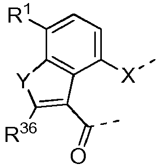 | 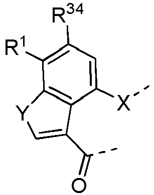 |
| **A309** (a10) | **A310** (a10) | **A311** (a10) | **A312** (a10) |
| 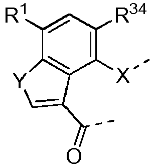 | 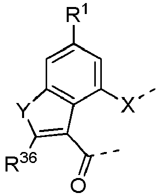 | 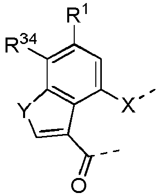 | 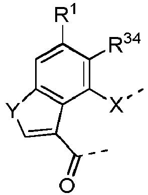 |
| **A313** (a10) | **A314** (a10) | **A315** (a10) | **A316** (a10) |
| 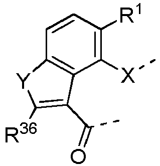 | 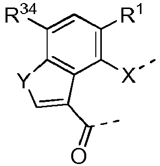 | 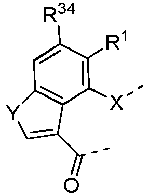 | 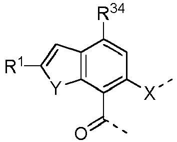 |
| **A317** (a10) | **A318** (a10) | **A319** (a10) | **A320** (a10) |
| 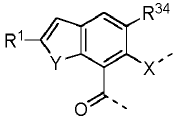 | 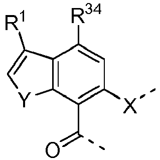 | 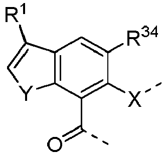 | 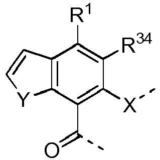 |
| **A321** (a10) | **A322** (a10) | **A323** (a10) | **A324** (a10) |
| 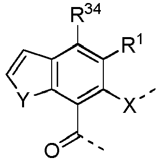 | 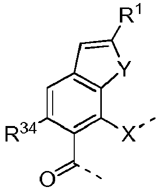 | 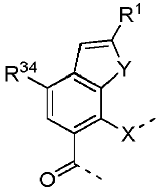 | 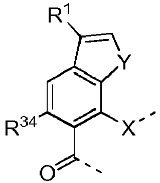 |
| **A325** (a10) | **A326** (a10) | **A327** (a10) | **A328** (a10) |
| 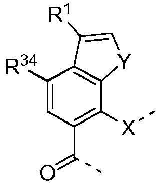 | 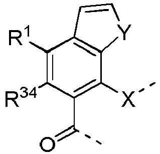 | 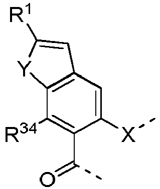 | 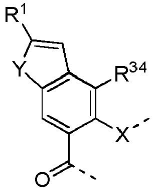 |
| **A329** (a10) | **A330** (a10) | **A331** (a10) | **A332** (a10) |
| 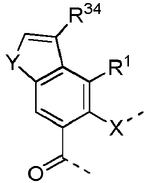 | 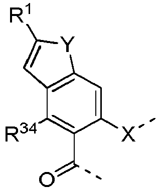 | 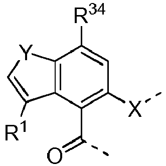 | 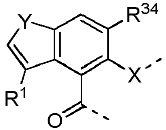 |
| **A333** (a10) | **A334** (a10) | **A335** (a10) | **A336** (a10) |
| 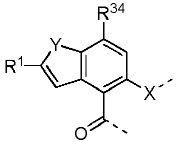 | 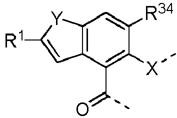 | 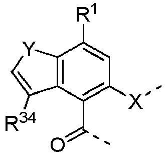 | 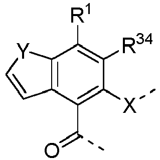 |
| **A337** (a10) | **A338** (a10) | **A339** (a10) | **A340** (a10) |
| 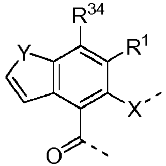 | 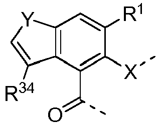 | 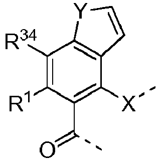 | 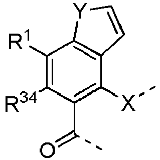 |
| **A341** (a10) | **A342** (a10) | **A343** (a10) | **A344** (a10) |
| 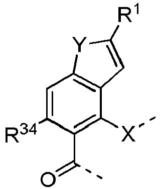 | 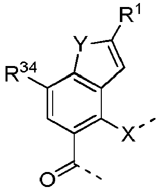 | 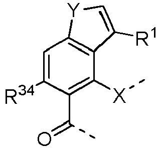 | 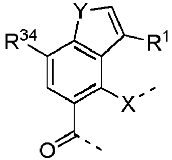 |
| **A345** (a10) | **A346** (a10) | **A347** (a10) | **A348** (a10) |
| 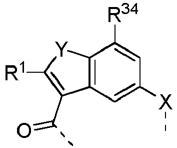 | 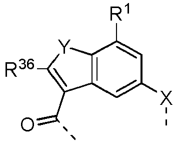 | 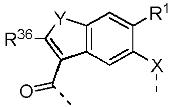 | 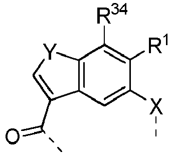 |
| **A349** (a10) | **A350** (a10) | **A351** (a10) | **A352** (a10) |
| 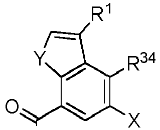 | 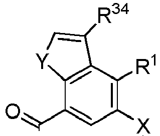 | 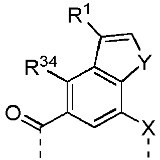 | 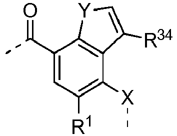 |
| **A353** (a10) | **A354** (a10) | **A355** (a10) | **A356** (a10) |
| 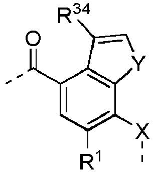 | 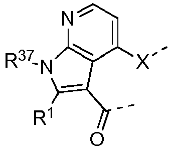 | 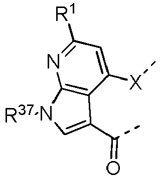 | 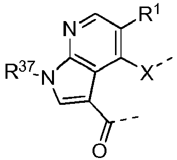 |
| **A357** (a10) | **A358** (a11) | **A359** (a11) | **A360** (a11) |
| 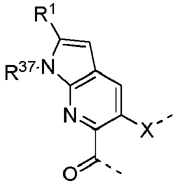 | 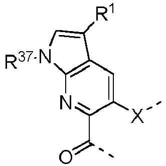 | 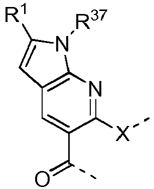 | 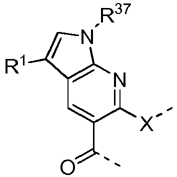 |
| **A361** (a11) | **A362** (a11) | **A363** (a11) | **A364** (a11) |
| 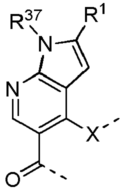 | 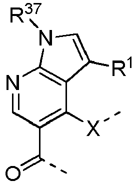 | 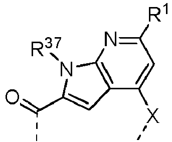 | 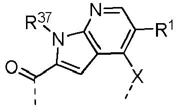 |
| **A365** (a11) | **A366** (a11) | **A367** (a11) | **A368** (a11) |
| 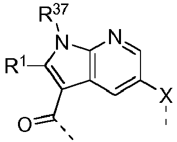 | 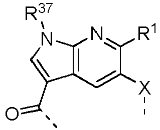 | 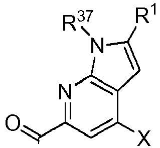 | 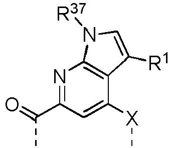 |
| **A369** (a11) | **A370** (a11) | **A371** (a11) | **A372** (a11) |
| 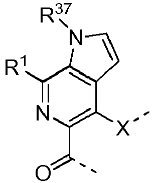 | 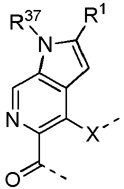 | 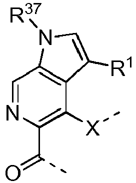 | 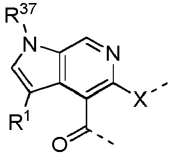 |
| **A373** (a12) | **A374** (a12) | **A375** (a12) | **A376** (a12) |
| 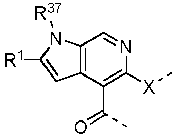 | 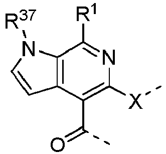 | 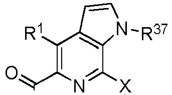 | 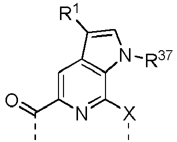 |
| **A377** (a12) | **A378** (a12) | **A379** (a12) | **A380** (a12) |
| 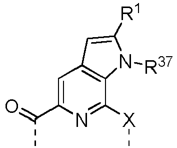 | 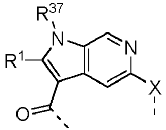 | 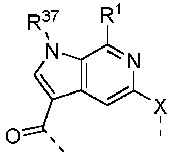 | 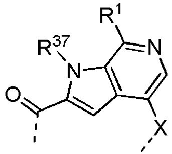 |
| **A381** (a12) | **A382** (a12) | **A383** (a12) | **A384** (a12) |
| 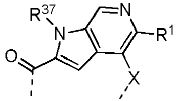 | 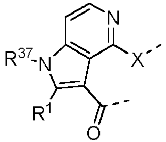 | 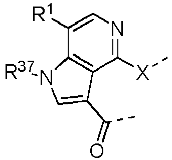 | 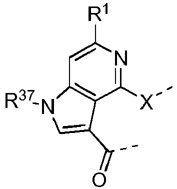 |
| **A385** (a12) | **A386** (a13) | **A387** (a13) | **A388** (a13) |
| 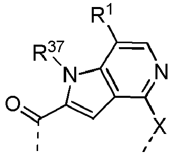 | 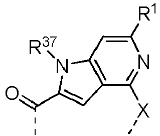 | 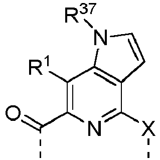 | 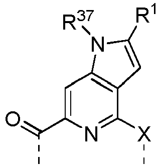 |
| **A389** (a13) | **A390** (a13) | **A391** (a13) | **A392** (a13) |
| 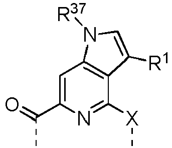 | 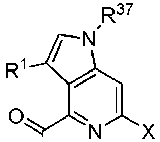 | 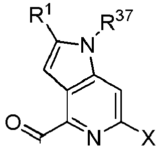 | 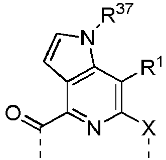 |
| **A393** (a13) | **A394** (a13) | **A395** (a13) | **A396** (a13) |
| 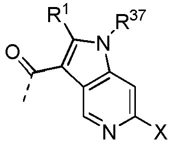 | 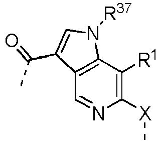 | 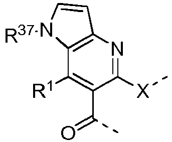 | 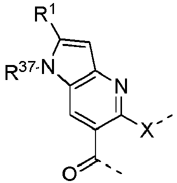 |
| **A397** (a13) | **A398** (a13) | **A399** (a14) | **A400** (a14) |
| 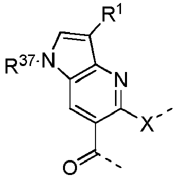 | 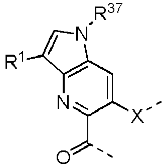 | 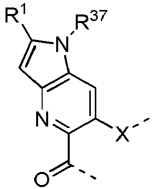 | 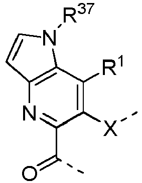 |
| **A401** (a14) | **A402** (a14) | **A403** (a14) | **A404** (a14) |
| 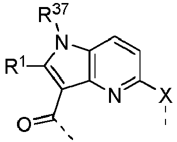 | 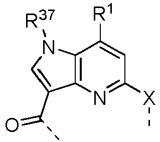 | 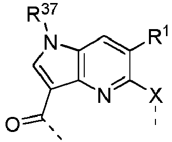 | 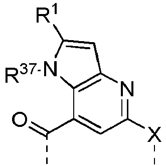 |
| **A405** (a14) | **A406** (a14) | **A407** (a14) | **A408** (a14) |
| 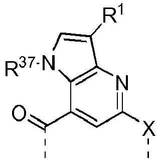 | 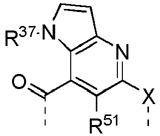 | 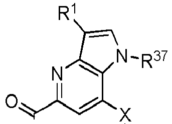 | 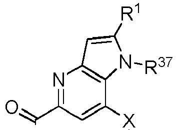 |
| **A409** (a14) | **A410** (a14) | **A411** (a14) | **A412** (a14) |
| 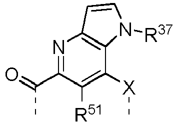 | 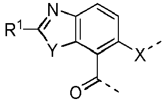 | 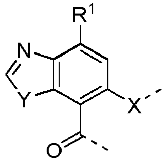 | 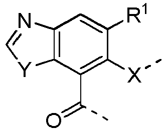 |
| **A413** (a14) | **A414** (a15) | **A415** (**a15**) | **A416** (a15) |
| 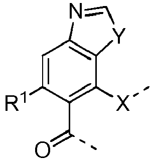 | 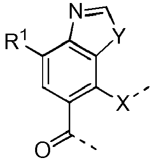 | 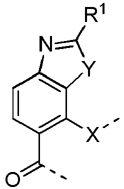 | 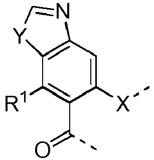 |
| **A417** (a15) | **A418** (a15) | **A419** (a15) | **A420** (a15) |
| 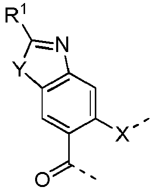 | 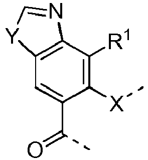 | 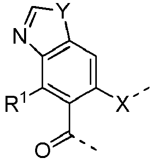 | 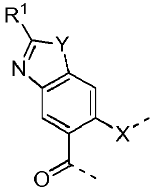 |
| **A421** (a15) | **A422** (a15) | **A423** (a15) | **A424** (a15) |
| 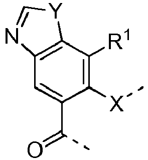 | 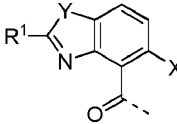 | 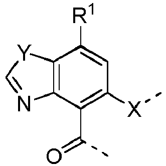 | 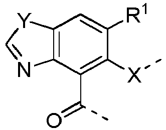 |
| **A425** (a15) | **A426** (a15) | **A427** (a15) | **A428** (a15) |
| 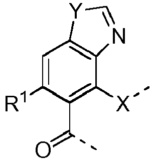 | 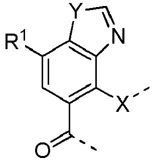 | 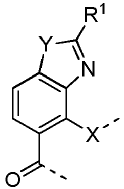 | 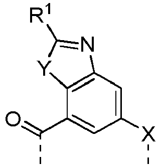 |
| **A429** (a15) | **A430** (a15) | **A431** (a15) | **A432** (a15) |
| 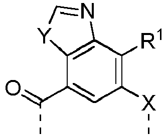 | 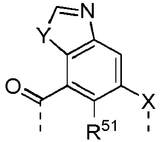 | 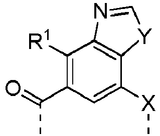 | 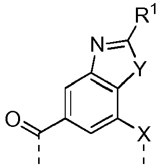 |
| **A433** (a15) | **A434** (a15) | **A435** (a15) | **A436** (a15) |
| 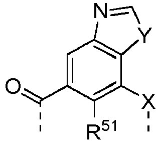 | 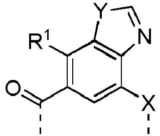 | 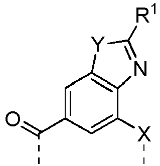 | 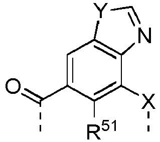 |
| **A437** (a15) | **A438** (a15) | **A439** (a15) | **A440** (a15) |
| 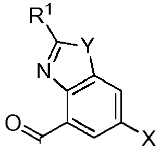 | 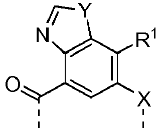 | 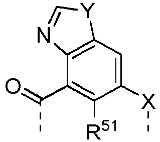 | 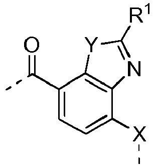 |
| **A441** (a15) | **A442** (a15) | **A443** (a15) | **A444** (a15) |
| 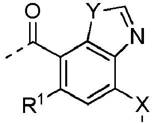 | 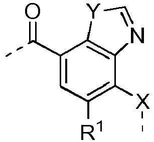 | 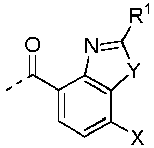 | 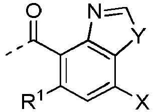 |
| **A445** (a15) | **A446** (a15) | **A447** (a15) | **A448** (a15) |
| 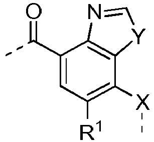 | 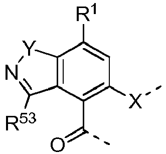 | 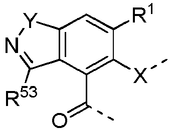 | 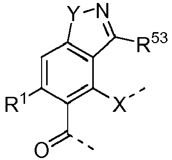 |
| **A449** (a15) | **A450** (a16) | **A451** (a16) | **A452** (a16) |
| 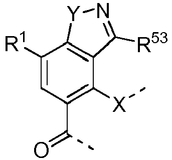 | 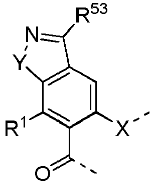 | 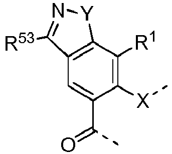 | 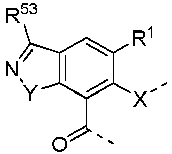 |
| **A453** (a16) | **A454** (a16) | **A455** (a16) | **A456** (a16) |
| 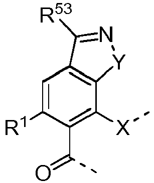 | 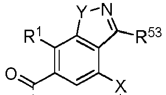 | 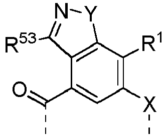 | 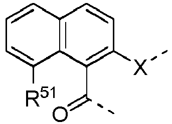 |
| **A457** (a16) | **A458** (a16) | **A459** (a16) | **A460** (a17) |
| 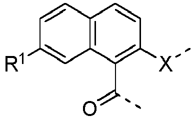 | 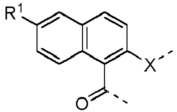 | 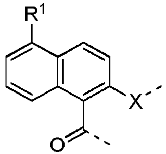 | 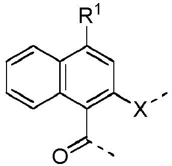 |
| **A461** (a17) | **A462** (a17) | **A463** (a17) | **A464** (a17) |
| 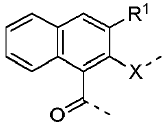 | 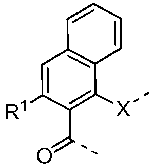 | 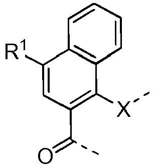 | 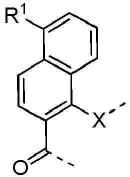 |
| **A465** (a17) | **A466** (a17) | **A467** (a17) | **A468** (a17) |
| 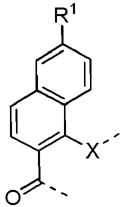 | 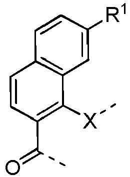 | 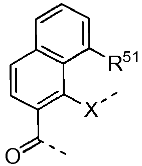 | 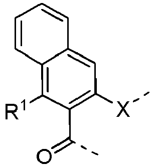 |
| **A469** (a17) | **A470** (a17) | **A471** (a17) | **A472** (a17) |
| 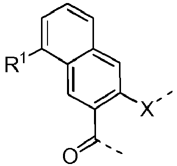 | 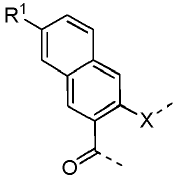 | 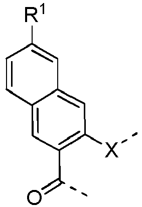 | 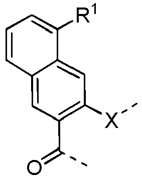 |
| **A473** (a17) | **A474** (a17) | **A475** (a17) | **A476** (a17) |
| 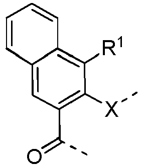 | 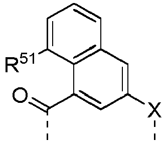 | 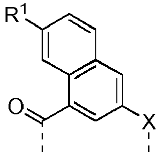 | 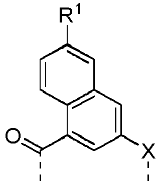 |
| **A477** (a17) | **A478** (a17) | **A479** (a17) | **A480** (a17) |
| 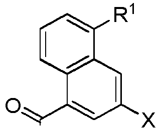 | 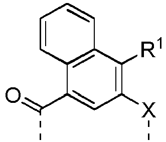 | 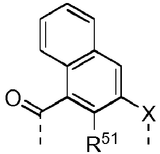 | 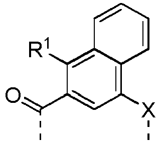 |
| **A481** (a17) | **A482** (a17) | **A483** (a17) | **A484** (a17) |
| 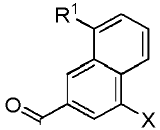 | 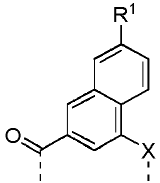 | 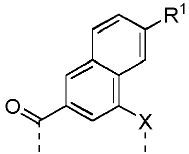 | 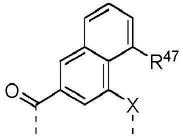 |
| **A485** (a17) | **A486** (a17) | **A487** (a17) | **A488** (a17) |
| 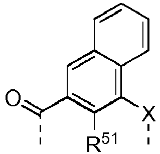 | 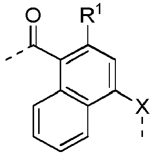 | 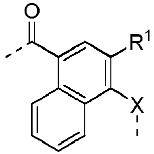 | 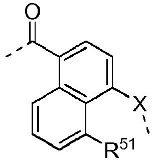 |
| **A489** (a17) | **A490** (a17) | **A491** (a17) | **A492** (a17) |
| 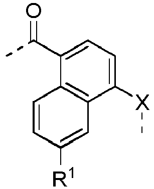 | 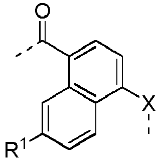 | 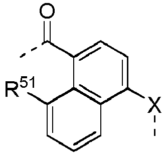 | 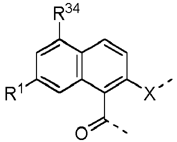 |
| **A493** (a17) | **A494** (a17) | **A495** (a17) | **A496** (a17) |
| 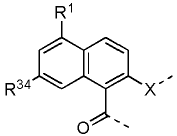 | 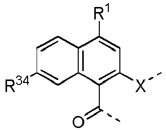 | 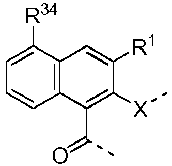 | 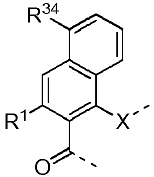 |
| **A497** (a17) | **A498** (a17) | **A499** (a17) | **A500** (a17) |
| 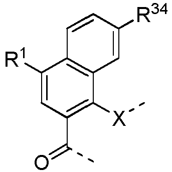 | 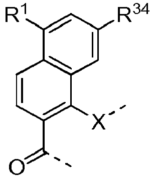 | 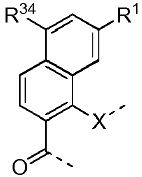 | 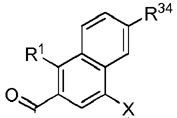 |
| **A501** (a17) | **A502** (a17) | **A503** (a17) | **A504** (a17) |
| 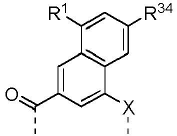 | 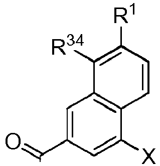 | 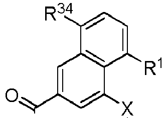 | 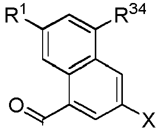 |
| **A505** (a17) | **A506** (a17) | **A507** (a17) | **A508** (a17) |
| 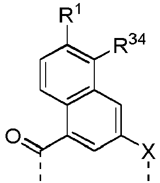 | 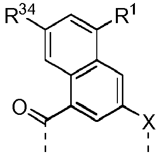 | 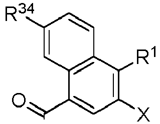 | 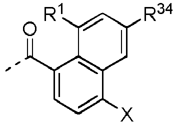 |
| **A509** (a17) | **A510** (a17) | **A511** (a17) | **A512** (a17) |
| 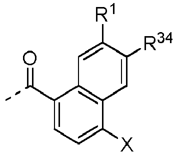 | 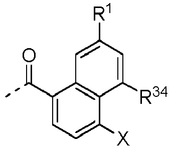 | 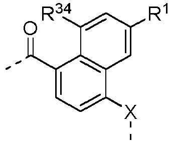 | 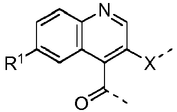 |
| **A513** (a17) | **A514** (a17) | **A515** (a17) | **A516** (a18) |
| 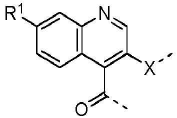 | 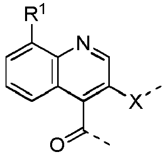 | 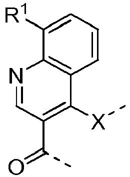 | 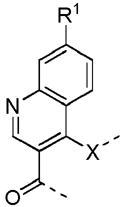 |
| **A517** (a18) | **A518** (a18) | **A519** (a18) | **A520** (a18) |
| 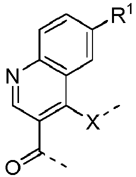 | 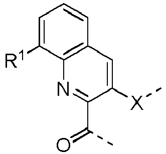 | 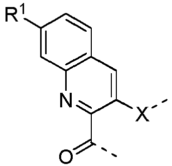 | 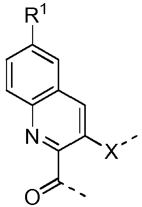 |
| **A521** (a18) | **A522** (a18) | **A523** (a18) | **A524** (a18) |
| 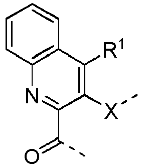 | 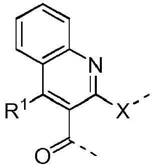 | 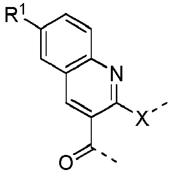 | 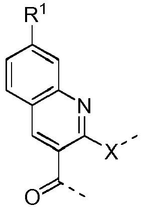 |
| **A525** (a18) | **A526** (a18) | **A527** (a18) | **A528** (a18) |
| 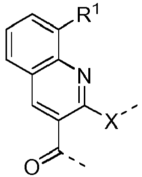 | 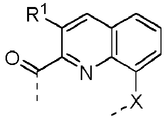 | 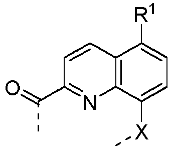 | 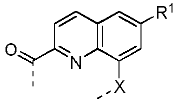 |
| **A529** (a18) | **A530** (a18) | **A531** (a18) | **A532** (a18) |
| 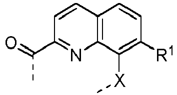 | 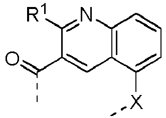 | 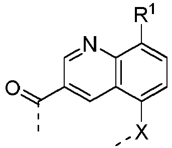 | 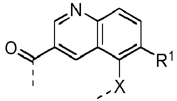 |
| **A533** (a18) | **A534** (a18) | **A535** (a18) | **A536** (a18) |
| 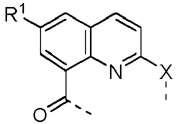 | 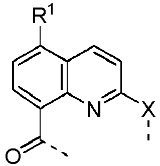 | 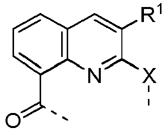 | 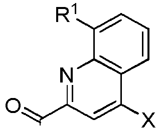 |
| **A537** (a18) | **A538** (a18) | **A539** (a18) | **A540** (a18) |
| 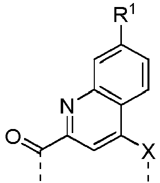 | 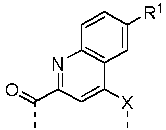 | 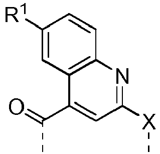 | 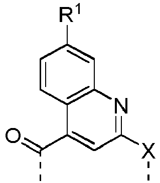 |
| **A541** (a18) | **A542** (a18) | **A543** (a18) | **A544** (a18) |
| 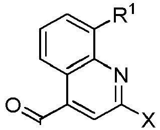 | 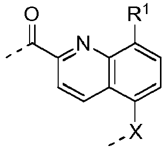 | 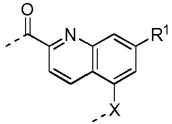 | 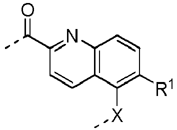 |
| **A545** (a18) | **A546** (a18) | **A547** (a18) | **A548** (a18) |
| 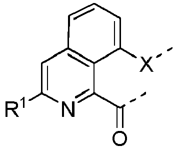 | 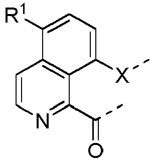 | 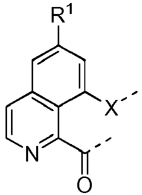 | 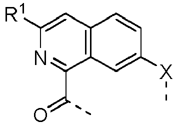 |
| **A549** (a19) | **A550** (a19) | **A551** (a19) | **A552** (a19) |
| 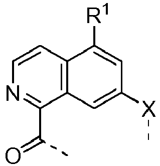 | 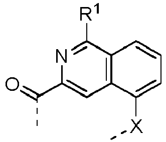 | 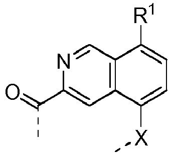 | 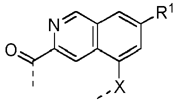 |
| **A553** (a19) | **A554** (a19) | **A555** (a19) | **A556** (a19) |
| 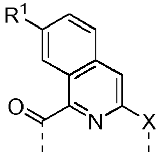 | 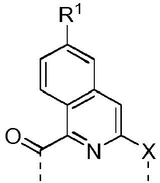 | 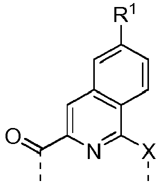 | 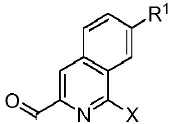 |
| **A557** (a19) | **A558** (a19) | **A559** (a19) | **A560** (a19) |
| 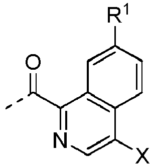 | 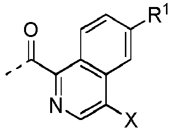 | 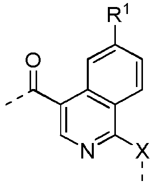 | 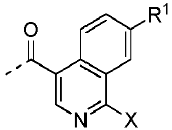 |
| **A561** (a19) | **A562** (a19) | **A563** (a19) | **A564** (a19) |
| 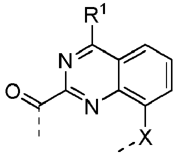 | 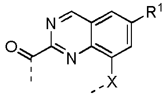 | 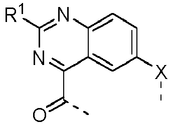 | 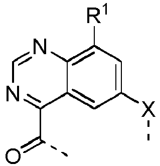 |
| **A565** (a20) | **A566** (a20) | **A567** (a20) | **A568** (a20) |
| 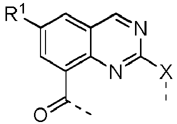 | 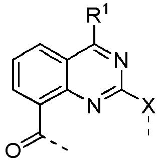 | 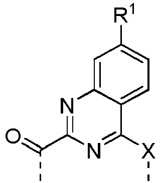 | 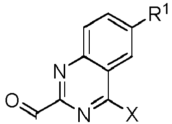 |
| **A569** (a20) | **A570** (a20) | **A571** (a20) | **A572** (a20) |
| 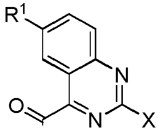 | 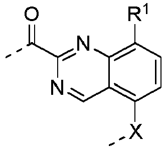 | 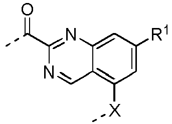 | 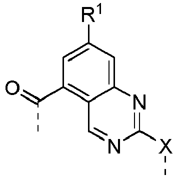 |
| **A573** (a20) | **A574** (a20) | **A575** (a20) | **A576** (a20) |
| 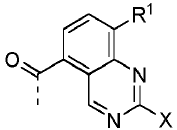 | 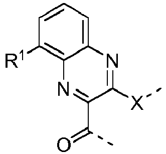 | 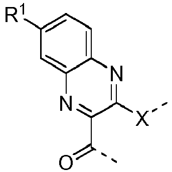 | 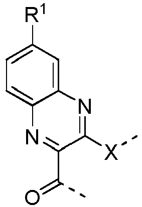 |
| **A577** (a20) | **A578** (a21) | **A579** (a21) | **A580** (a21) |
| 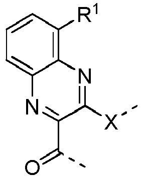 | 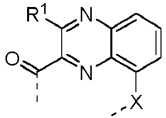 | 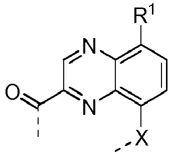 | 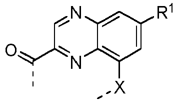 |
| **A581** (a21) | **A582** (a21) | **A583** (a21) | **A584** (a21) |
| 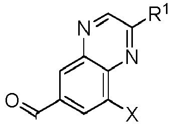 | 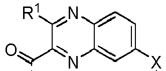 | 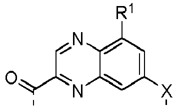 | 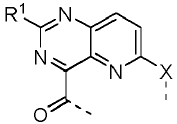 |
| **A585** (a21) | **A586** (a21) | **A587** (a21) | **A588** (a22) |
| 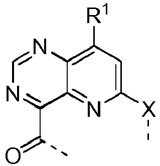 | 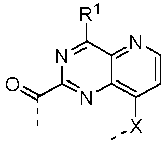 | 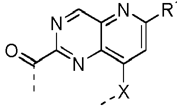 | 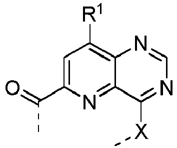 |
| **A589** (a22) | **A590** (a22) | **A591** (a22) | **A592** (a22) |
| 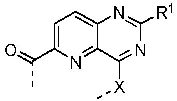 | 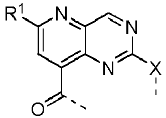 | 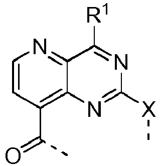 | 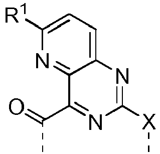 |
| **A593** (a22) | **A594** (a22) | **A595** (a22) | **A596** (a22) |
| 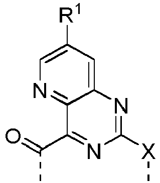 | 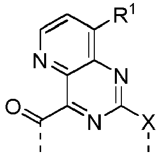 | 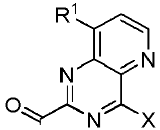 | 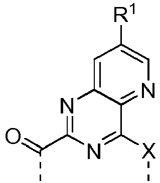 |
| **A597** (a22) | **A598** (a22) | **A599** (a22) | **A600** (a22) |
| 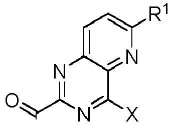 | 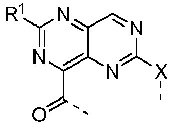 | 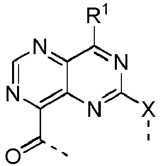 | 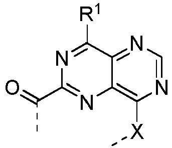 |
| **A601** (a22) | **A602** (a23) | **A603** (a23) | **A604** (a23) |
| 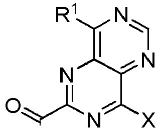 | 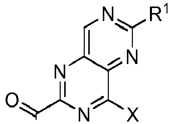 | 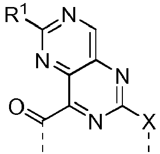 | 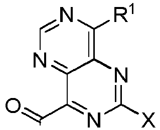 |
| **A605** (a23) | **A606** (a23) | **A607** (a23) | **A608** (a23) |
| 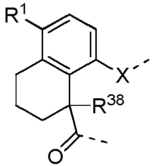 | 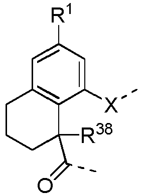 | 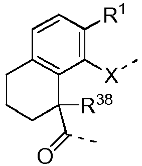 | 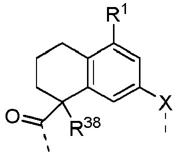 |
| **A609** (a24) | **A610** (a24) | **A611** (a24) | **A612** (a24) |
| 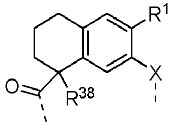 | 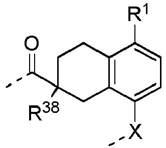 | 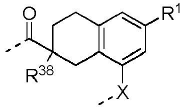 | 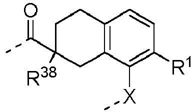 |
| **A613** (a24) | **A614** (a24) | **A615** (a24) | **A616** (a24) |
| 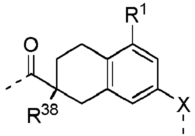 | 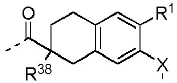 | 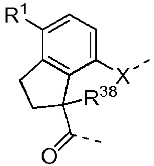 | 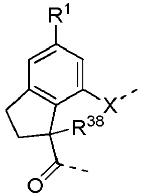 |
| **A617** (a24) | **A618** (a24) | **A619** (a25) | **A620** (a25) |
| 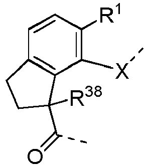 | 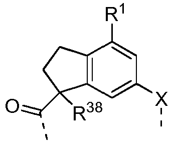 | 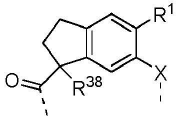 | 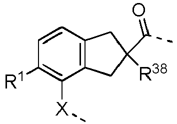 |
| **A621** (a25) | **A622** (a25) | **A623** (a25) | **A624** (a25) |
| 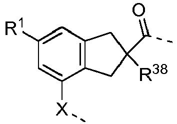 | 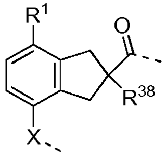 | 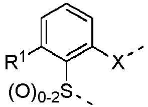 | 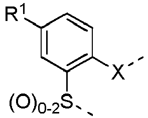 |
| **A625** (a25) | **A626** (a25) | **A627** (a1) | **A628** (a1) |
| 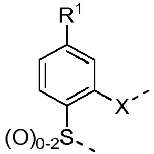 | 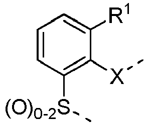 | 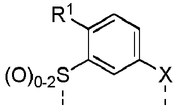 | 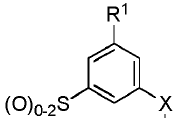 |
| **A629** (a1) | **A630** (a1) | **A631** (a1) | **A632** (a1) |
| 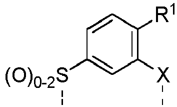 | 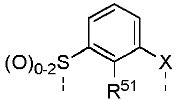 | 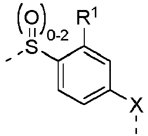 | 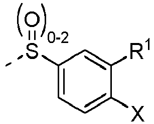 |
| **A633** (a1) | **A634** (a1) | **A635** (a1) | **A636** (a1) |
| 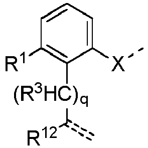 | 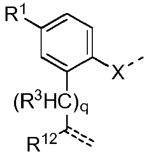 | 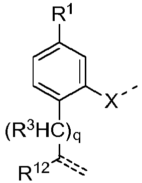 | 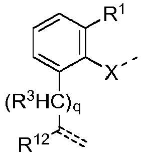 |
| **A637** (a1) | **A638** (a1) | **A639** (a1) | **A640** (a1) |
| 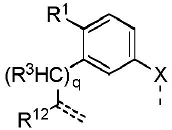 | 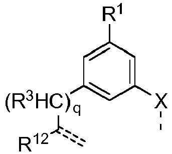 | 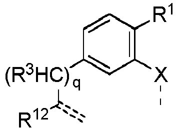 | 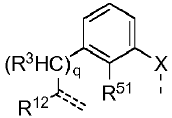 |
| **A641** (a1) | **A642** (a1) | **A643** (a1) | **A644** (a1) |
| 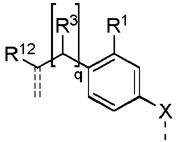 | 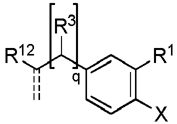 | 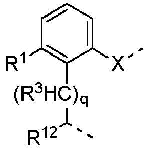 | 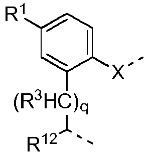 |
| **A645** (a1) | **A646** (a1) | **A647** (a1) | **A648** (a1) |
| 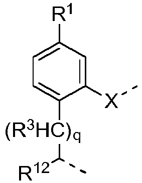 | 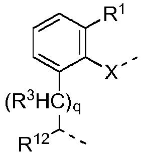 | 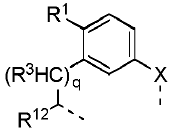 | 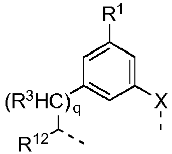 |
| **A649** (a1) | **A650** (a1) | **A651** (a1) | **A652** (a1) |
| 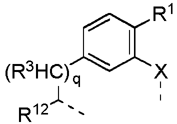 | 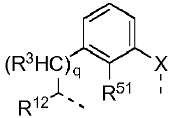 | 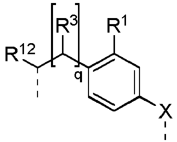 | 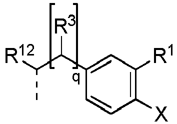 |
| **A653** (a1) | **A654** (a1) | **A655** (a1) | **A656** (a1) |
| 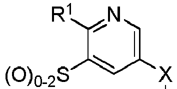 | 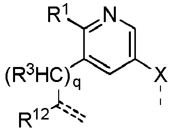 | 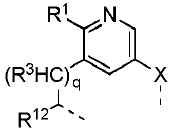 | 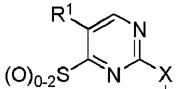 |
| **A657** (a2) | **A658** (a2) | **A659** (a2) | **A660** (a4) |
| 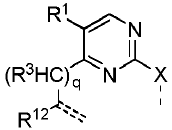 | 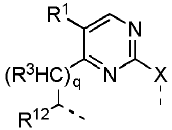 | 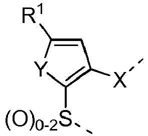 | 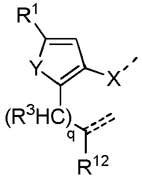 |
| **A661** (a4) | **A662** (a4) | **A663** (a7) | **A664** (a7) |
| 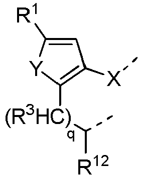 | 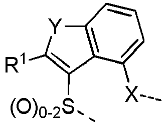 | 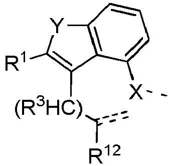 | 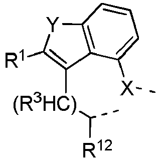 |
| **A665** (a7) | **A666** (a10) | **A667** (a10) | **A668** (a10) |
| 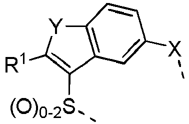 | 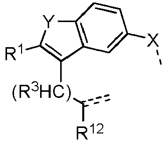 | 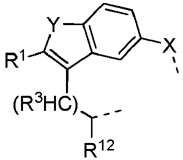 | 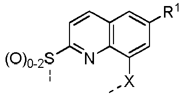 |
| **A669** (a10) | **A670** (a10) | **A671** (a10) | **A672** (a18) |
| 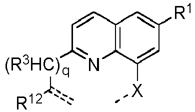 | 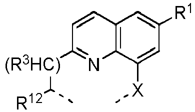 | 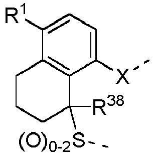 | 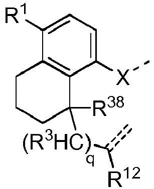 |
| **A673** (a18) | **A674** (a18) | **A675** (a24) | **A676** (a24) |
| 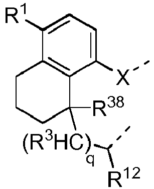 | 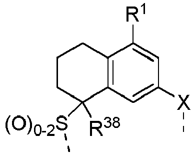 | 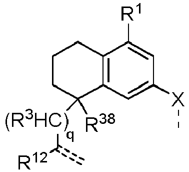 | 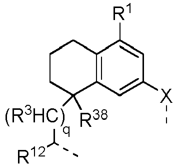 |
| **A677** (a24) | **A678** (a24) | **A679** (a24) | **A680** (a24) |
| 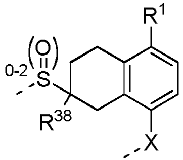 | 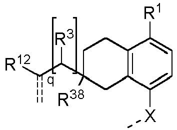 | | 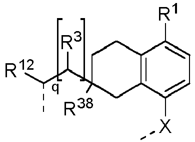 |
| **A681** (a24) | **A682** (a24) | | **A683** (a24) |
**building block B** is a bivalent radical selected from the group of
| | | | |
|---|---|---|---|
| 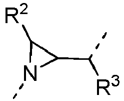 | 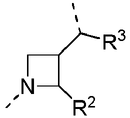 | 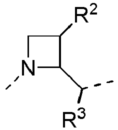 | 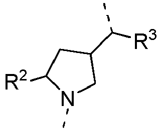 |
| **B1** (b1) | **B2** (b2) | **B3** (b2) | **B4** (b3) |
| 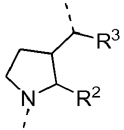 | 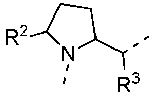 | 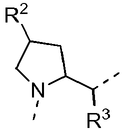 | 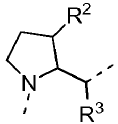 |
| **B5** (b3) | **B6** (b3) | **B7** (b3) | **B8** (b3) |
| 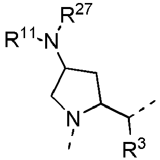 | 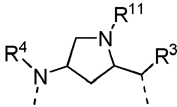 | 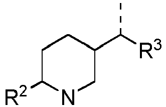 | 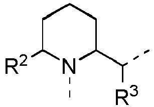 |
| **B9** (b3) | **B10** (b3) | **B11** (b4) | **B12** (b4) |
| 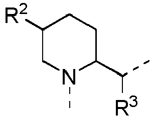 | 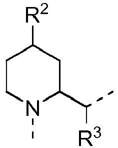 | 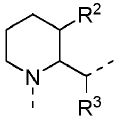 | 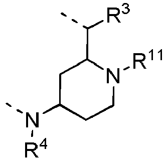 |
| **B13** (b4) | **B14** (b4) | **B15** (b4) | **B16** (b4) |
| 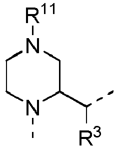 | 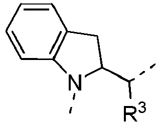 | 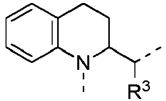 | 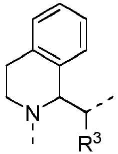 |
| **B17** (b5) | **B18** (b8) | **B19** (b10) | **B20** (b11) |
| 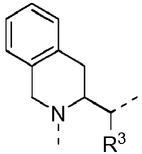 | | 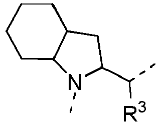 | |
| **B21** (b11) | | **B22** (b12) | |
**building block C** is a bivalent radical selected from the group of
| | |
|---|---|
| 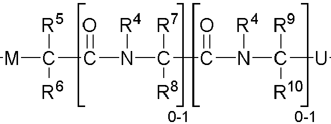 | 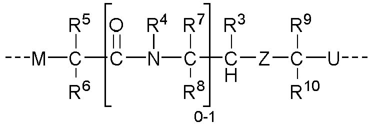 |
| **C1** | **C2** |
| 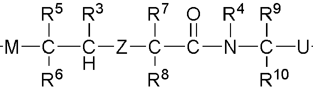 | 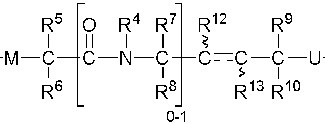 |
| **C3** | **C4** |
| 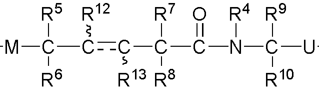 | 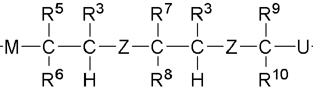 |
| **C5** | **C6** |
| 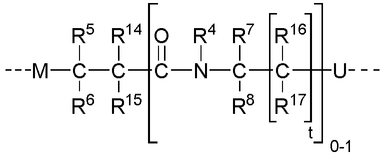 | 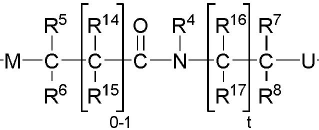 |
| **C7** | **C8** |
| 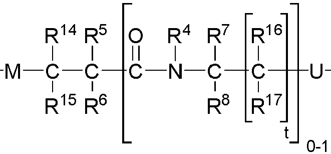 | 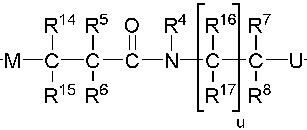 |
| **C9** | **C10** |
| 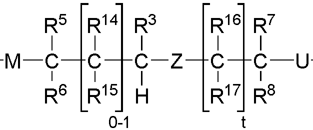 | 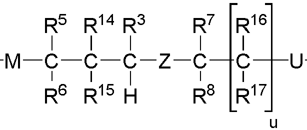 |
| **C11** | **C12** |
| 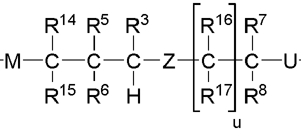 | 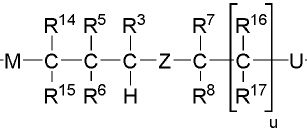 |
| **C13** | **C14** |
| 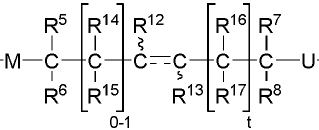 | 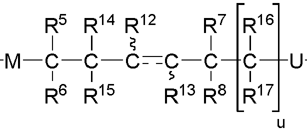 |
| **C15** | **C16** |
| 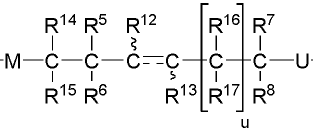 | 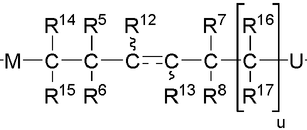 |
| **C17** | **C18** |
| 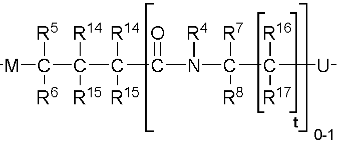 | 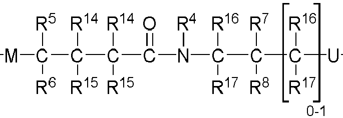 |
| **C19** | **C20** |
| 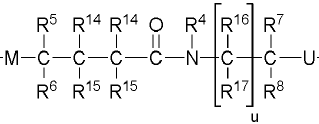 | 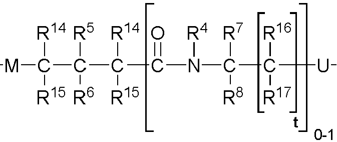 |
| **C21** | **C22** |
| 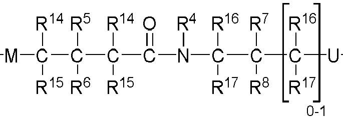 | 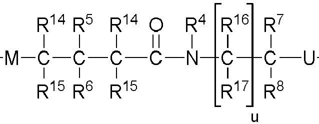 |
| **C23** | **C24** |
| 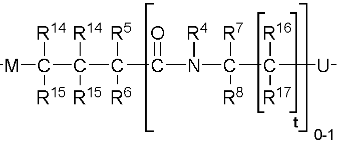 | 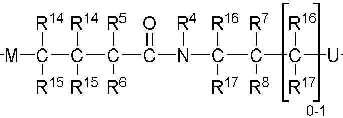 |
| **C25** | **C26** |
| 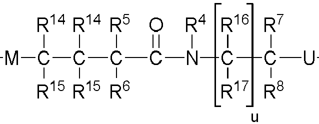 | 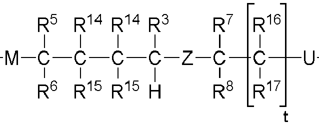 |
| **C27** | **C28** |
| 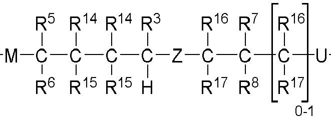 | 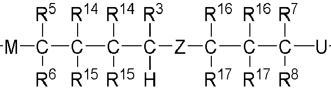 |
| **C29** | **C30** |
| 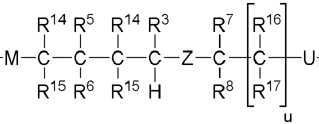 | 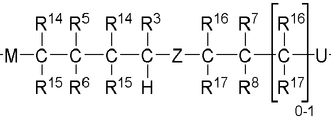 |
| **C31** | **C32** |
| 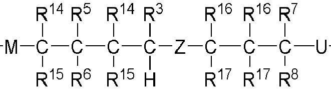 | 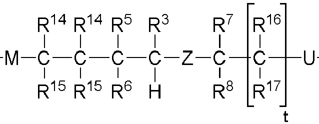 |
| **C33** | **C34** |
| 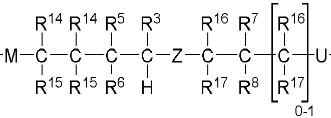 | 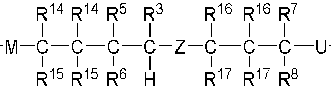 |
| **C35** | **C36** |
| 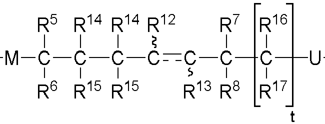 | 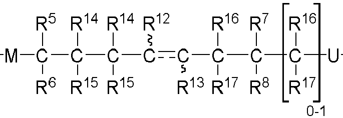 |
| **C37** | **C38** |
| 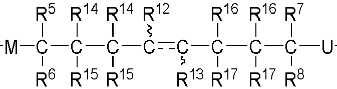 | 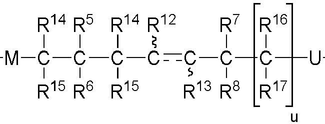 |
| **C39** | **C40** |
| 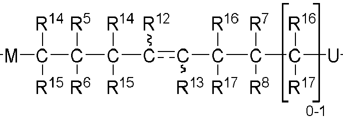 | 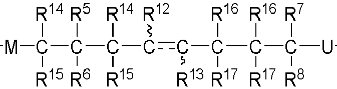 |
| **C41** | **C42** |
| 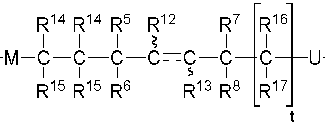 | 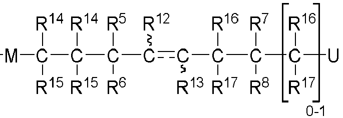 |
| **C43** | **C44** |
| 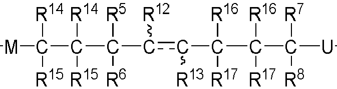 | 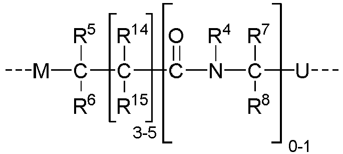 |
| **C45** | **C46** |
| 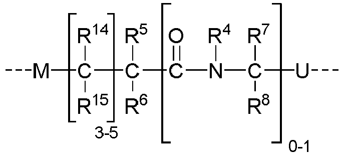 | 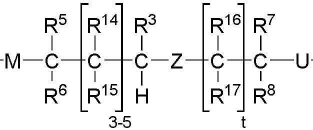 |
| **C47** | **C48** |
| 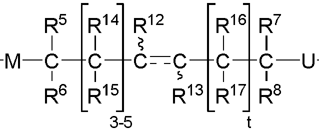 | 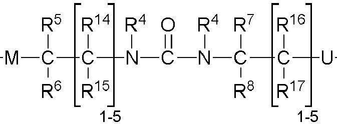 |
| **C49** | **C50** |
| 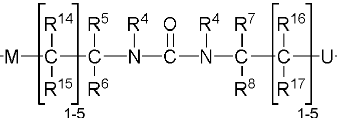 | 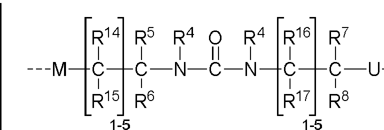 |
| **C51** | **C52** |
| 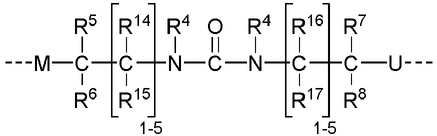 | |
| **C53** | |
| 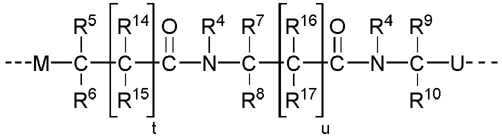 | |
| **C54** | |
| 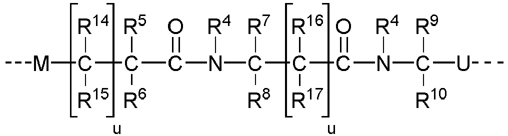 | |
| **C55** | |
| 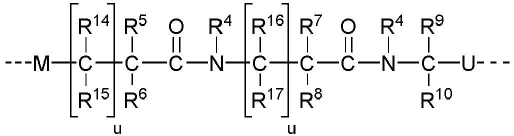 | |
| **C56** | |
| 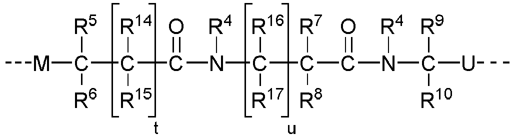 | |
| **C57** | |
| 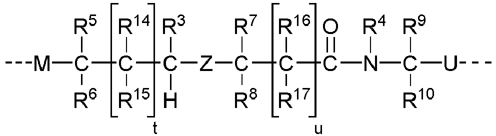 | |
| **C58** | |
| 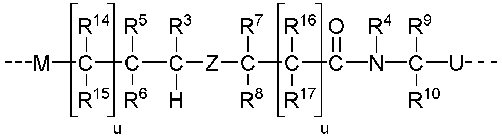 | |
| **C59** | |
| 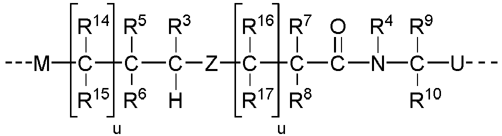 | |
| **C60** | |
| 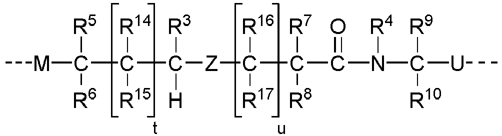 | |
| **C61** | |
| 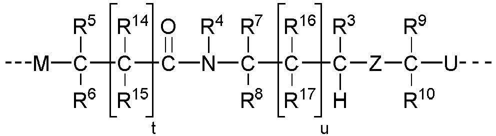 | |
| **C62** | |
| 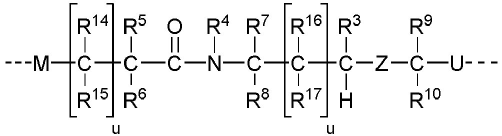 | |
| **C63** | |
| 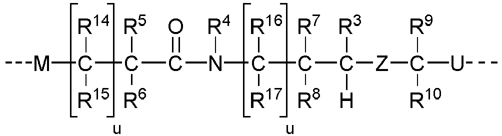 | |
| **C64** | |
| 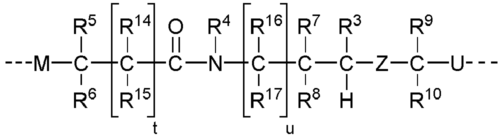 | |
| **C65** | |
| 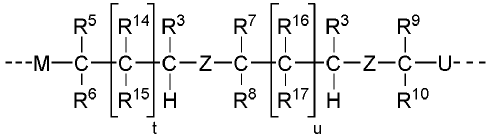 | |
| **C66** | |
| 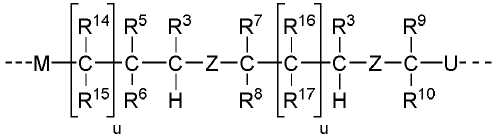 | |
| **C67** | |
| 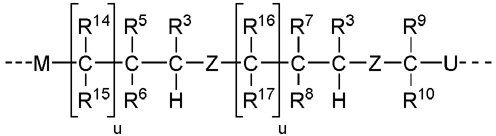 | |
| **C68** | |
| 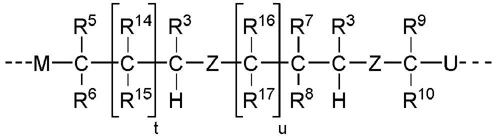 | |
| **C69** | |
| 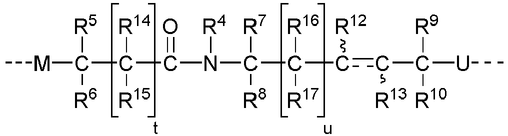 | |
| **C70** | |
| 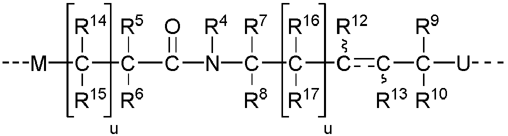 | |
| **C71** | |
| 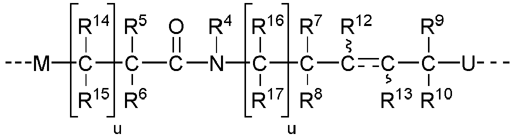 | |
| **C72** | |
| 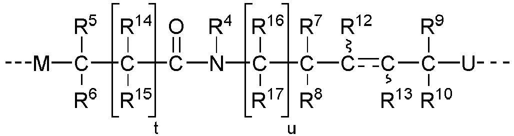 | |
| **C73** | |
| 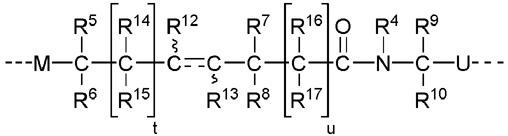 | |
| **C74** | |
| 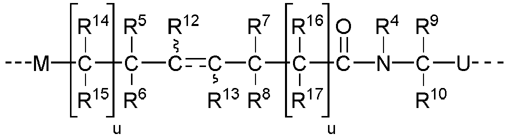 | |
| **C75** | |
| 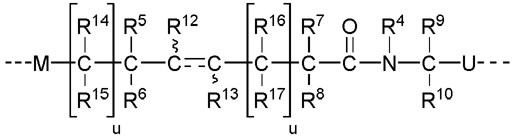 | |
| **C76** | |
| 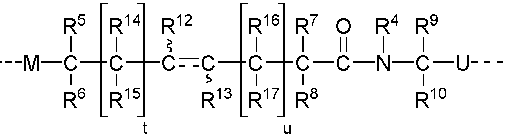 | |
| **C77** | |
| 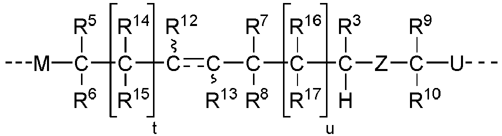 | |
| **C78** | |
| 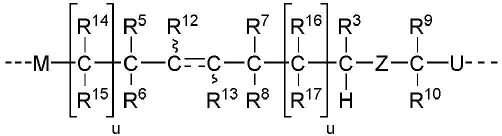 | |
| **C79** | |
| 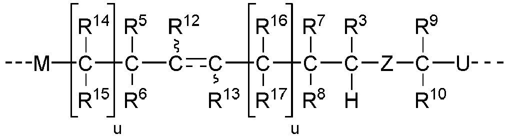 | |
| **C80** | |
| 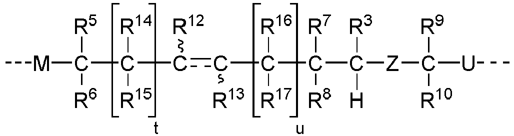 | |
| **C81** | |
| 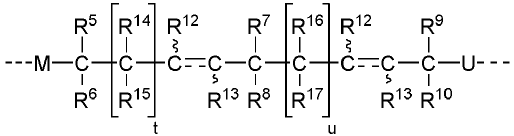 | |
| **C82** | |
| 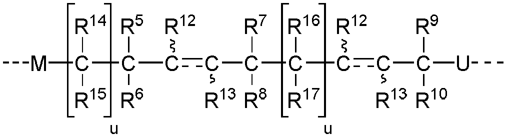 | |
| **C83** | |
| 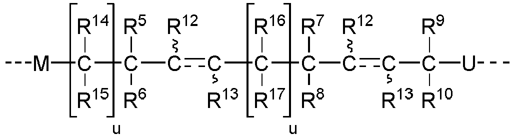 | |
| **C84** | |
| 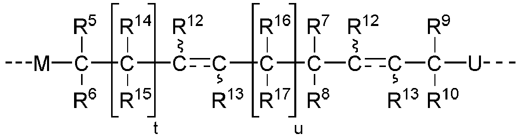 | |
| **C85** | |
| 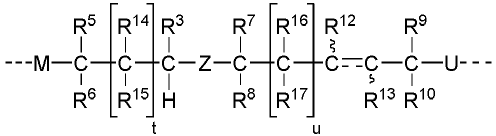 | |
| **C86** | |
| 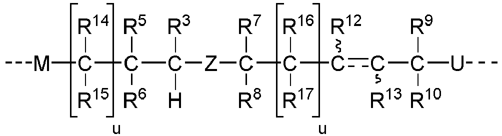 | |
| **C87** | |
| 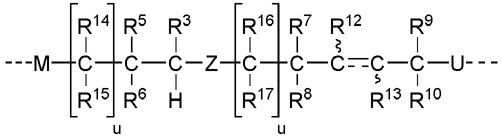 | |
| **C88** | |
| 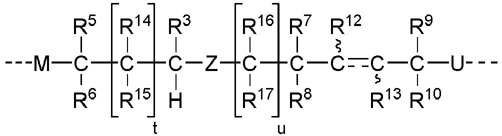 | |
| **C89** | |
| 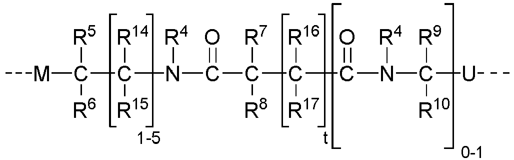 | |
| **C90** | |
| 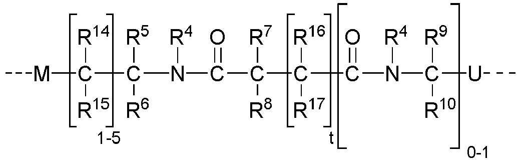 | |
| **C91** | |
| 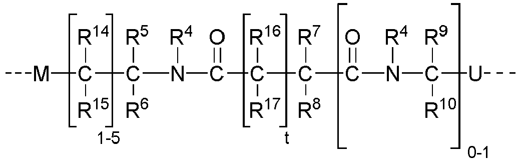 | |
| **C92** | |
| 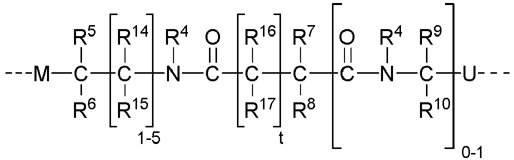 | |
| **C93** | |
| 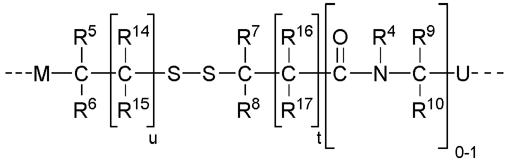 | |
| **C94** | |
| 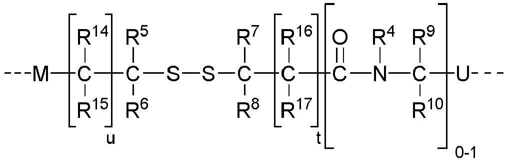 | |
| **C95** | |
| 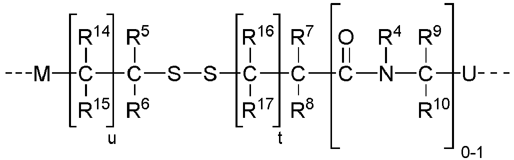 | |
| **C96** | |
| 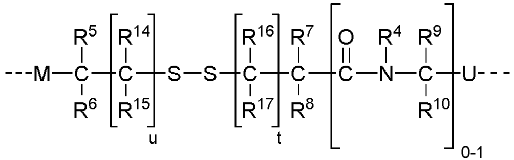 | |
| **C97** | |
| 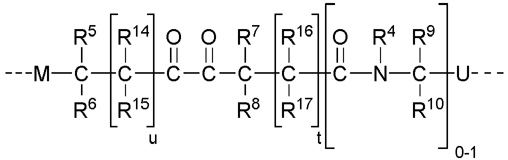 | |
| **C98** | |
| 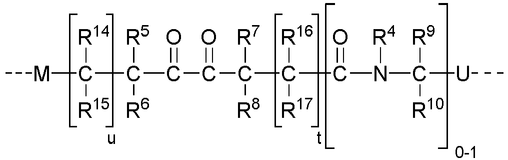 | |
| **C99** | |
| 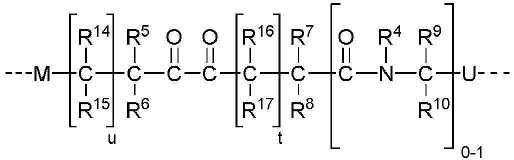 | |
| **C100** | |
| 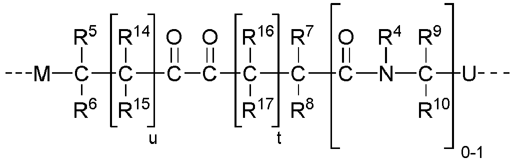 | |
| **C101** | |
and wherein further
R¹ is H; F; Cl; Br; I; CF₃; OCF₃; OCHF₂; NO₂; CN; alkyl; alkenyl; alkynyl; cycloalkyl; heterocycloalkyl; aryl; heteroaryl; arylalkyl; heteroarylalkyl; - (CR¹⁸R¹⁹)_{q}OR²⁰; -(CR¹⁸R¹⁹)_{q}SR²⁰; -(CR¹⁸R¹⁹)_{q}NR⁴R¹¹;-(CR¹⁸R¹⁹)_{q}OCONR⁴R¹¹; -(CR¹⁸R¹⁹)_{q}OCOOR²¹; -(CR¹⁸R¹⁹)_{q}NR⁴COOR²¹; -(CR¹⁸R¹⁹)_{q}NR⁴COR²²; -(CR¹⁸R¹⁹)_{q}NR⁴CONR⁴R¹¹; -(CR¹⁸R¹⁹)_{q}NR⁴SO₂R²³; -(CR¹⁸R¹⁹)_{q}NR⁴SO₂NR⁴R¹¹; -(CR¹⁸R¹⁹)_{q}COOR²¹; -(CR¹⁸R¹⁹)_{q}CONR⁴R¹¹; -(CR¹⁸R¹⁹)_{q}SO₂NR⁴R¹¹; -(CR¹⁸R¹⁹)_{q}PO(OR²¹)₂; -(CR¹⁸R¹⁹)_{q}OPO(OR²¹)₂; -(CR¹⁸R¹⁹)_{q}COR²²; -(CR¹⁸R¹⁹)_{q}SO₂R²³; -(CR¹⁸R¹⁹)_{q}OSO₃R²¹; -(CR¹⁸R¹⁹)_{q}R²⁴; -(CR¹⁸R¹⁹)_{q}R²⁵; or -(CR¹⁸R¹⁹)_{q}R²⁶;
R² is H; CF₃; alkyl; alkenyl; cycloalkyl; heterocycloalkyl; aryl; heteroaryl; arylalkyl; heteroarylalkyl; -(CR¹⁸R¹⁹)_{q}OR²⁰; -(CR¹⁸R¹⁹)_{q}SR²⁰; -(CR¹⁸R¹⁹)_{q}NR⁴R¹¹; -(CR¹⁸R¹⁹)_{q}OCONR⁴R¹¹; -(CR¹⁸R¹⁹)_{q}OCOOR²¹; -(CR¹⁸R¹⁹)_{q}NR⁴COOR²¹; -(CR¹⁸R¹⁹)_{q}NR⁴COR²²; -(CR¹⁸R¹⁹)_{q}NR⁴CONR⁴R¹¹; -(CR¹⁸R¹⁹)_{q}NR⁴SO₂R²³; -(CR¹⁸R¹⁹)_{q}NR⁴SO₂NR⁴R¹¹; -(CR¹⁸R¹⁹)_{q}COOR²¹; -(CR¹⁸R¹⁹)_{q}CONR⁴R¹¹; -(CR¹⁸R¹⁹)_{q}SO₂NR⁴R¹¹; -(CR¹⁸R¹⁹)_{q}PO(OR²¹)₂; -(CR¹⁸R¹⁹)_{q}COR²²; -(CR¹⁸R¹⁹)_{q}SO₂R²³; -(CR¹⁸R¹⁹)_{q}R²⁴; -(CR¹⁸R¹⁹)_{q}R²⁵; or -(CR¹⁸R¹⁹)_{q}R²⁶;
R³ is H; CF₃; alkyl; cycloalkyl; heterocycloalkyl; aryl; heteroaryl; arylalkyl; or heteroarylalkyl;
R⁴ is H; alkyl; alkenyl; cycloalkyl; heterocycloalkyl; aryl; heteroaryl; arylalkyl; heteroarylalkyl; or a suitable N-protecting group;
R⁵, R⁷ and R⁹ are independently defined as is H; F; CF₃; alkyl; alkenyl; cycloalkyl; heterocycloalkyl; aryl; heteroaryl; arylalkyl; heteroarylalkyl; -(CR¹⁸R¹⁹)_{q}OR²⁰; -(CR¹⁸R¹⁹)_{q}SR²⁰; -(CR¹⁸R¹⁹)_{q}NR⁴R¹¹; -(CR¹⁸R¹⁹)_{q}OCONR⁴R¹¹; -(CR¹⁸R¹⁹)_{q}OCOOR²¹; - (CR¹⁸R¹⁹)_{q}NR⁴COOR²¹; -(CR¹⁸R¹⁹)_{q}NR⁴COR²²; -(CR¹⁸R¹⁹)_{q}NR⁴CONR⁴R¹¹; -(CR¹⁸R¹⁹)_{q}NR⁴SO₂R²³; -(CR¹⁸R¹⁹)_{q}NR⁴SO₂NR⁴R¹¹; -(CR¹⁸R¹⁹)_{q}COOR²¹; -(CR¹⁸R¹⁹)_{q}CONR⁴R¹¹; -(CR¹⁸R¹⁹)_{q}SO₂NR⁴R¹¹; -(CR¹⁸R¹⁹)_{q}PO(OR²¹)₂; -(CR¹⁸R¹⁹)_{q}COR²²; -(CR¹⁸R¹⁹)_{q}SO₂R²³; -(CR¹⁸R¹⁹)_{q}R²⁴; -(CR¹⁸R¹⁹)_{q}R²⁵; or -(CR¹⁸R¹9)_{q}R²⁶;
R⁶, R⁸ and R¹⁰ are independently defined as H; F; CF₃; alkyl; alkenyl; cycloalkyl; heterocycloalkyl; aryl; heteroaryl; arylalkyl; or heteroarylalkyl;
R¹¹ is H; alkyl; alkenyl; cycloalkyl; heterocycloalkyl; aryl; heteroaryl; arylalkyl; heteroarylalkyl; a suitable protecting group; -(CR¹⁸R¹⁹)ᵣOR²⁰; -(CR¹⁸R¹⁹)ᵣSR²⁰; -(CR¹⁸R¹⁹)ᵣNR⁴R²⁷; - (CR¹⁸R¹⁹)ᵣOCONR⁴R²⁷; - (CR¹⁸R¹⁹)ᵣOCOOR²¹; - (CR¹⁸R¹⁹)ᵣNR⁴COOR²¹; -(CR¹⁸R¹⁹)ᵣNR⁴CONR⁴R²⁷; -(CR¹⁸R¹⁹)ᵣNR⁴SO₂R²³ ; -(CR¹⁸R¹⁹)ᵣNR⁴SO₂NR⁴R²⁷; -(CR¹⁸R¹⁹)_{q}COOR²¹; -(CR¹⁸R¹⁹)_{q}CONR⁴R²⁷; -(CR¹⁸R¹⁹)_{q}COR²²; -(CR¹⁸R¹⁹)_{q}SO₂R²³; -(CR¹⁸R¹⁹)_{q}SO₂NR⁴R²⁷; -(CR¹⁸R¹⁹)_{q}R²⁴; -(CR¹⁸R¹⁹)ₛR²⁵; -(CR¹⁸R¹⁹)_{q}R²⁶; or -(CR¹⁸R¹⁹)_{q}R³¹;
R¹² and R¹³ are independently defined as H; or alkyl;
R¹⁴ and R¹⁶ are independently defined as H; F; CF₃; alkyl; alkenyl; cycloalkyl; heterocycloalkyl; aryl; heteroaryl; arylalkyl; heteroarylalkyl; -(CR¹⁸R¹⁹)_{q}OR²⁰; -(CR¹⁸R¹⁹)_{q}NR⁴R¹¹; - (CR¹⁸R¹⁹)_{q}NR⁴COOR²¹; -(CR¹⁸R¹⁹)_{q}NR⁴COR²²; -(CR¹⁸R¹⁹)_{q}NR⁴CONR⁴R¹¹; -(CR¹⁸R¹⁹)_{q}NR⁴SO₂R²³; -(CR¹⁸R¹⁹)_{q}NR⁴SO₂NR⁴R¹¹; -(CR¹⁸R¹⁹)_{q}COOR²¹; -(CR¹⁸R¹⁹)_{q}CONR⁴R¹¹; -(CR¹⁸R¹⁹)_{q}SO₂NR⁴R¹¹; or -(CR¹⁸R¹⁹)_{q}COR²²;
R¹⁵ and R¹⁷ are independently defined as H; F; CF₃; alkyl; alkenyl; cycloalkyl; heterocycloalkyl; aryl; heteroaryl; arylalkyl; or heteroarylalkyl;
R¹⁸ is H; F; CF₃; alkyl; alkenyl; cycloalkyl; heterocycloalkyl; aryl; heteroaryl; arylalkyl; heteroarylalkyl; -(CR²⁹R³⁰)ₛOR³¹; -(CR²⁹R³⁰)ₛSR³¹; -(CR²⁹R30)ₛNR²⁸R³¹; -(CR²⁹R³⁰)ₛOCONR²⁸R³¹; -(CR²⁹R³⁰)ₛOCOOR²¹; -(CR²⁹R³⁰)ₛNR²⁸COOR²¹; -(CR²⁹R³⁰)ₛNR²⁸COR³¹; -(CR²⁹R³⁰)ₛNR²⁸CONR²⁸R³¹; -(CR²⁹R³⁰)ₛNR²⁸SO₂R²³; -(CR²⁹R³⁰)ₛNR²⁸SO₂NR²⁸R³¹; -(CR²⁹R³⁰)_{q}COOR²¹; -(CR²⁹R³⁰)_{q}CONR²⁸R³¹; -(CR²⁹R³⁰)_{q}SO₂NR²⁸R³¹; -(CR²⁹R³⁰)_{q}PO(OR²¹)₂; -(CR²⁹R³⁰)_{q}COR³¹; -(CR²⁹R³⁰)_{q}SO²R²³; -(CR²⁹R³⁰)_{q}R²⁴; -(CR²⁹R³⁰)_{q}R²⁵; or -(CR²⁹R³⁰)qR²⁶;
R¹⁹ is H; CF₃; alkyl; alkenyl; cycloalkyl; heterocycloalkyl; aryl; heteroaryl; arylalkyl; or heteroarylalkyl;
R²⁰ is H; alkyl; alkenyl; cycloalkyl; heterocycloalkyl; aryl; heteroaryl; arylalkyl; heteroarylalkyl; -(CR²⁹R³⁰)ᵣOR³¹; -(CR²⁹R³⁰)ᵣSR³¹; -(CR²⁹R³⁰)ᵣNR²⁸R³¹; -(CR²⁹R³⁰)ᵣOCONR²⁸R³¹; -(CR²⁹R³⁰)ᵣNR²⁸COOR²¹; -(CR²⁹R³⁰)ᵣNR²⁸COR³¹; -(CR²⁹R³⁰)ᵣNR²⁸CONR²⁸R³¹; -(CR²⁹R³⁰)ᵣNR²⁸SO₂R²³; -(CR²⁹R³⁰)ᵣNR²⁸SO₂NR²⁸R³¹; -(CR²⁹R³⁰)_{q}COOR²¹; -(CR²⁹R³⁰)_{q}CONR²⁸R³¹; -(CR²⁹R³⁰)_{q}SO₂NR²⁸R³¹; -(CR²⁹R³⁰)_{q}COR³¹; -(CR²⁹R³⁰)_{q}SO₂R²³; -(CR²⁹R³⁰)_{q}R²⁴; -(CR²⁹R³⁰)_{q}R²⁵; -(CR²⁹R³⁰)_{q}R²⁶; or -(CR²⁹R³⁰)_{q}R³¹;
R²¹ is H; alkyl; alkenyl; cycloalkyl; heterocycloalkyl; aryl; heteroaryl; arylalkyl; heteroarylalkyl; or a suitable *O-*protecting group;
R²² is alkyl; alkenyl; cycloalkyl; heterocycloalkyl; aryl; heteroaryl; arylalkyl; heteroarylalkyl; -(CR²⁹R³⁰)ₛOR³¹; -(CR²⁹R³⁰)ₛSR³¹; -(CR²⁹R³⁰)ₛNR²⁸R³¹; -(CR²⁹R³⁰)ₛOCONR²⁸R³¹; -(CR²⁹R³⁰)ₛNR²⁸COOR²¹; -(CR²⁹R³⁰)ₛNR²⁸COR³¹; -(CR²⁹R³⁰)ₛNR²⁸CONR²⁸R³¹; -(CR²⁹R³⁰)ₛNR²⁸SO₂R²³; -(CR²⁹R³⁰)ₛNR²⁸SO₂NR²⁸R³¹; -(CR²⁹R³⁰)ₛCOOR²¹; -(CR²⁹R³⁰)ₛCONR²⁸R³¹ ; -(CR²⁹R³⁰)ₛSO₂NR²⁸R³¹; -(CR²⁹R³⁰)ₜCOR³¹; -(CR²⁹R³⁰)ₛSO₂R²³; -(CR²⁹R³⁰)ₜR²⁴; -(CR²⁹R³⁰)ₜR²⁵; -(CR²⁹R³⁰)ₜR²⁶; or -(CR²⁹R³⁰)ₜR³¹;
R²³ is H; alkyl; alkenyl; cycloalkyl; heterocycloalkyl; aryl; heteroaryl; arylalkyl; heteroarylalkyl; or -(CR³²R³³)ₜR²⁴;
R²⁴ is aryl; heteroaryl; -C₆H₂R³⁴R³⁵R³¹; or a group of one of the formulae
| | | | |
|---|---|---|---|
| 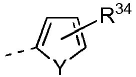 | 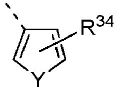 | 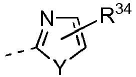 | 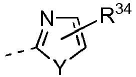 |
| **H1** | **H2** | **H3** | **H4** |
| 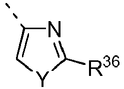 | 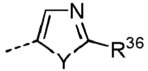 | 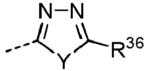 | 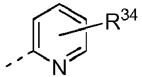 |
| **H5** | **H6** | **H7** | **H8** |
| 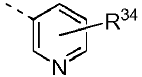 | 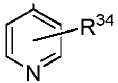 | 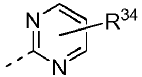 | 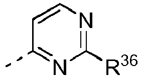 |
| **H9** | **H10** | **H11** | **H12** |
| 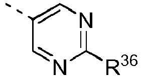 | 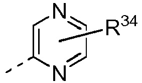 | 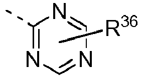 | 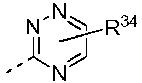 |
| **H13** | **H14** | **H15** | **H16** |
| 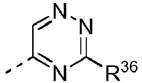 | 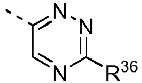 | 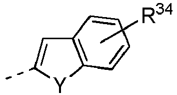 | 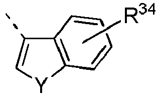 |
| **H17** | **H18** | **H19** | **H20** |
| 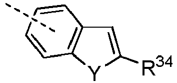 | 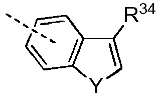 | 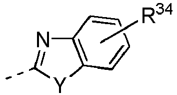 | 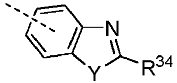 |
| **H21** | **H22** | **H23** | **H24** |
| 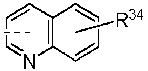 | 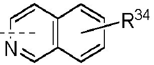 | 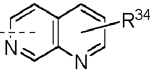 | 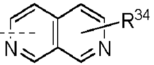 |
| **H25** | **H26** | **H27** | **H28** |
| 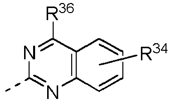 | 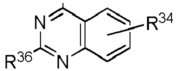 | 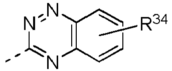 | 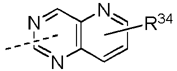 |
| **H29** | **H30** | **H31** | **H32** |
| 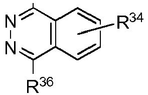 | | 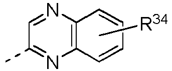 | |
| **H33** | | **H34** | |
R²⁵ is a group of one of the formulae
| | | | |
|---|---|---|---|
| 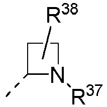 | 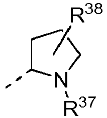 | 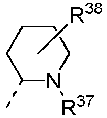 | 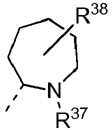 |
| **H35** | **H36** | **H37** | **H38** |
| 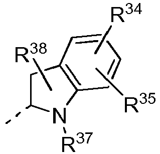 | 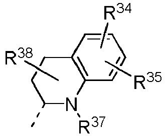 | | 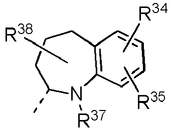 |
| **H39** | **H40** | | **H41** |
R²⁶ is a group of one of the formulae
| | | | |
|---|---|---|---|
| 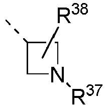 **H42** | 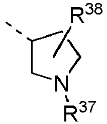 **H43** | 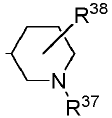 **H44** | 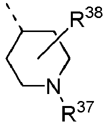 **H45** |
| 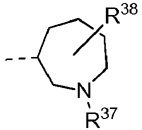 **H46** | 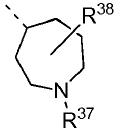 **H47** | 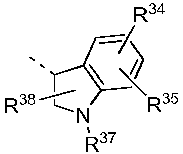 **H48** | 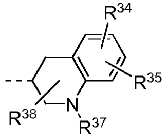 **H49** |
| 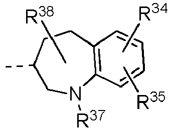 | | | |
| **H50** | | | |
R²⁷ is H; alkyl; lower alkenyl; cycloalkyl; heterocycloalkyl; aryl; heteroaryl; lower arylalkyl; lower heteroarylalkyl; a suitable protecting group; or -(CR²⁹R³⁰)_{q}R²⁴;
R²⁸ is H; alkyl; lower alkenyl; cycloalkyl; heterocycloalkyl; aryl; heteroaryl; lower arylalkyl; lower heteroarylalkyl; a suitable N-protecting group; -(CR³²R³³)ₛOR²¹; -(CR³²R³³)ₛNR⁴³R⁴²; -(CR³²R³³)ₛNR⁴²CONR⁴³R⁴²; -(CR³²R³³)ₛNR⁴²COR²¹; -(CR³²R³³)ₛNR⁴²SO₂NR²¹; -(CR³²R³³)_{q}COOR²¹; -(CR³²R³³)_{q}COR²³; or -(CR³²R³³)_{q}SO₂R²¹;
R²⁹ is H; F; CF₃; alkyl; alkenyl; cycloalkyl; heterocycloalkyl; aryl; heteroaryl; arylalkyl; heteroarylalkyl; -(CR³²R³³)ₛOR³¹; -(CR³²R³³)ₛSR³¹; -(CR³²R³³)ₛNR²⁸R³¹; -(CR³²R³³)ₛOCONR²⁸R³¹; -(CR³²R³³)ₛOCOOR²¹; -(CR³²R³³)ₛNR²⁸COOR²¹; -(CR³²R³³)ₛNR²⁸COR³¹; -(CR³²R³³)ₛNR²⁸CONR²⁸R³¹; -(CR³²R³³)ₛNR²⁸SO₂R²³; -(CR³²R³³)ₛNR²⁸SO₂NR²⁸R³¹; -(CR³²R³³)_{q}COOR²¹; -(CR³²R³³)_{q}CONR²⁸R³¹; -(CR³²R³³)_{q}SO₂NR²⁸R³¹; -(CR³²R³³)_{q}PO(OR²¹)₂; -(CR³²R³³)_{q}COR³¹; -(CR³²R³³)_{q}SO₂R²³; or -(CR³²R³³)_{q}R³¹;
R³⁰ is H; F; CF₃; alkyl; alkenyl; cycloalkyl; heterocycloalkyl; aryl; heteroaryl; arylalkyl; or heteroarylalkyl;
R³¹ is H; alkyl; alkenyl; cycloalkyl; heterocycloalkyl; aryl; heteroaryl; arylalkyl; heteroarylalkyl; or a group of one of the formulae
| | |
|---|---|
| 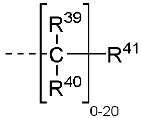 | 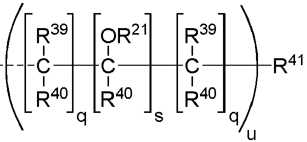 |
| **H51** | **H52** |
| 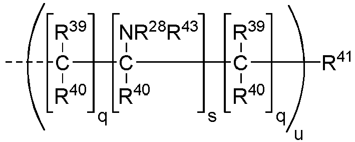 | 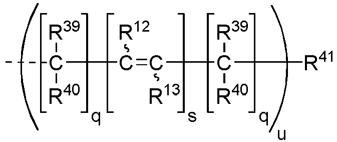 |
| **H53** | **H54** |
| 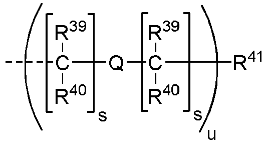 | |
| **H55** | |
R³² and R³³ are independently defined as H; F; CF₃; alkyl; alkenyl; cycloalkyl; heterocycloalkyl; aryl; heteroaryl; arylalkyl; or heteroarylalkyl;
R³⁴ and R³⁵ are independently defined as H; F; Cl; CF₃; OCF₃; OCHF₂; NO₂; CN; alkyl; alkenyl; alkynyl; cycloalkyl; heterocycloalkyl; aryl; heteroaryl; arylalkyl; heteroarylalkyl; -(CR²⁹R³⁰)_{q}OR³¹; -(CR²⁹R³⁰)_{q}SR³¹; -(CR²⁹R³⁰)_{q}NR²⁸R³¹; -(CR²⁹R³⁰)_{q}OCONR²⁸R³¹; -(CR²⁹R³⁰)_{q}NR²⁸COOR²¹; -(CR²⁹R³⁰)_{q}NR²⁸COR³¹; -(CR²⁹R³⁰)_{q}NR²⁸CONR²⁸R³¹; -(CR²⁹R³⁰)_{q}NR²⁸SO₂R²³; -(CR²⁹R³⁰)_{q}NR²⁸SO₂NR²⁸R³¹; -(CR²⁹R³⁰)_{q}COOR²¹; -(CR²⁹R³⁰)_{q}CONR²⁸R³¹_{;} -(CR²⁹R³⁰)_{q}SO₂NR²⁸R³¹; -(CR²⁹R³⁰)_{q}COR³¹; -(CR²⁹R³⁰)_{q}SO₂R²³; or -(CR²⁹R³⁰)_{q}R³¹;
R³⁶ is H; alkyl; alkenyl; alkynyl; cycloalkyl; heterocycloalkyl; aryl; heteroaryl; arylalkyl; heteroarylalkyl; or -NR²⁸R³¹;
R³⁷ is H; alkyl; alkenyl; cycloalkyl; heterocycloalkyl; aryl; heteroaryl; arylalkyl; heteroarylalkyl; a suitable *N*-protecting group; -(CR²⁹R³⁰)ᵣOR³¹; -(CR²⁹R³⁰)ᵣSR³¹; -(CR²⁹R³⁰)ᵣNR²⁸R³¹; -(CR²⁹R³⁰)ᵣOCONR²⁸R³¹; -(CR²⁹R³⁰)ᵣNR²⁸COOR²¹; -(CR²⁹R³⁰)ᵣNR²⁸COR³¹; -(CR²⁹R³⁰)ᵣNR²⁸CONR²⁸R³¹; -(CR²⁹R³⁰)ᵣNR²⁸SO₂R²³; -(CR²⁹R³⁰)ᵣNR²⁸SO₂NR²⁸R³¹; -(CR²⁹R³⁰)_{q}COOR²¹; -(CR²⁹R³⁰)_{q}CONR²⁸R³¹; -(CR²⁹R³⁰)ᵣSO₂NR²⁸R³¹; -(CR²⁹R³⁰)_{q}COR³¹; -(CR²⁹R³⁰)_{q}SO₂R²³; or -(CR²⁹R³⁰)_{q}R³¹;
R³⁸ is H; alkyl; alkenyl; cycloalkyl; heterocycloalkyl; aryl; heteroaryl; arylalkyl; heteroarylalkyl; -(CR²⁹R³⁰)_{q}OR³¹; -(CR²⁹R³⁰)_{q}SR³¹; -(CR²⁹R³⁰)_{q}NR²⁸R³¹; -(CR²⁹R³⁰)_{q}NR²⁸COOR²¹; -(CR²⁹R³⁰)_{q}NR²⁸COR³¹; -(CR²⁹R³⁰)_{q}NR²⁸CONR²⁸R³¹; -(CR²⁹R³⁰)_{q}COOR²¹; -(CR²⁹R³⁰)_{q}CONR²⁸R³¹; - (CR²⁹R³⁰)_{q}COR³¹; or -(CR²⁹R³⁰)_{q}R³¹;
R³⁹ is H; F; CF₃; alkyl; alkenyl; cycloalkyl; heterocycloalkyl; aryl; heteroaryl; arylalkyl; heteroarylalkyl; -(CR³²R³³)ᵤOR²¹; -(CR³²R³³)ᵤNR²⁸R⁴³; -(CR³²R³³)ₜCOOR^{21;} or -(CR³²R³³)ₜCONR²⁸R⁴³;
R⁴⁰ is H; F; CF₃; alkyl; alkenyl; cycloalkyl; heterocycloalkyl; aryl; heteroaryl; arylalkyl; heteroarylalkyl; -(CR³²R³³)ᵤOR²¹; -(CR³²R³³)ᵤNR²⁸R⁴³; -(CR³²R³³)ₜCOOR²¹; or -(CR³²R³³)ₜCONR²⁸R⁴³;
R⁴¹ is H; CF₃; alkyl; alkenyl; cycloalkyl; heterocycloalkyl; aryl; heteroaryl; arylalkyl; heteroarylalkyl; -OR²¹; -NR²⁸R⁴³; -NR²⁸COR²³; -NR²⁸COOR²¹; -NR²⁸SO₂R²³; -NR²⁸CONR²⁸R⁴³; -COOR²¹; -CONR²⁸R⁴³; -C(=NR⁴³) NR28R43; -NR²⁸C(=NR⁴³) NR28R43; or a group of one of the formulae
| | | | |
|---|---|---|---|
| 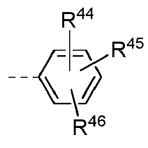 | 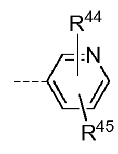 | 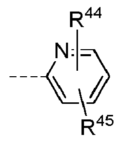 | 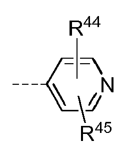 |
| **H56** | **H57** | **H58** | **H59** |
| 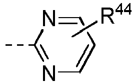 | 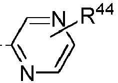 | 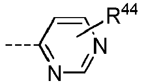 | 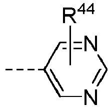 |
| **H60** | **H61** | **H62** | **H63** |
| 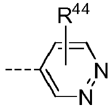 | 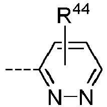 | 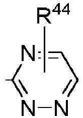 | 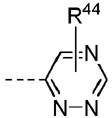 |
| **H64** | **H65** | **H66** | **H67** |
| 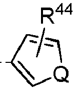 | 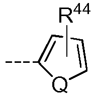 | 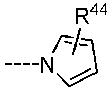 | 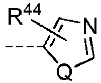 |
| **H68** | **H69** | **H70** | **H71** |
| 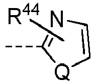 | 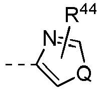 | 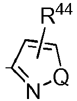 | 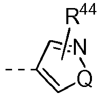 |
| **H72** | **H73** | **H74** | **H75** |
| 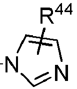 | 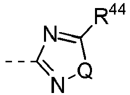 | 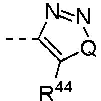 | 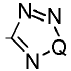 |
| **H76** | **H77** | **H78** | **H79** |
| 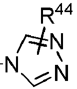 | 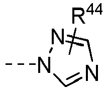 | 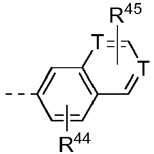 | 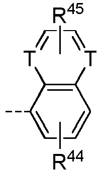 |
| **H80** | **H81** | **H82** | **H83** |
| 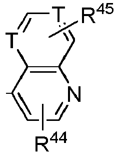 | 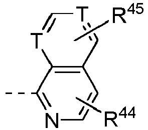 | 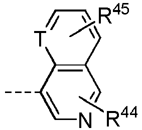 | 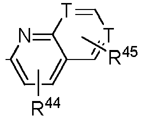 |
| **H84** | **H85** | **H86** | **H87** |
| 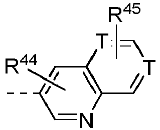 | 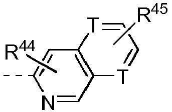 | 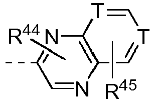 | 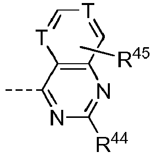 |
| **H88** | **H89** | **H90** | **H91** |
| 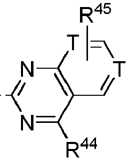 | 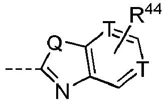 | 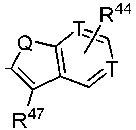 | 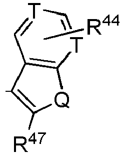 |
| **H92** | **H93** | **H94** | **H95** |
| 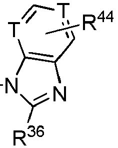 | 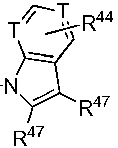 | 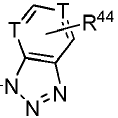 | 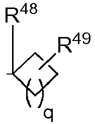 |
| **H96** | **H97** | **H98** | **H99** |
| 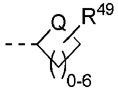 | 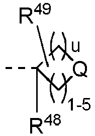 | 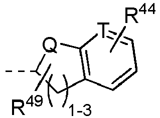 | 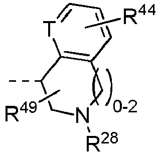 |
| **H100** | **H101** | **H102** | **H103** |
| 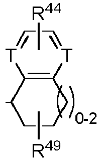 | 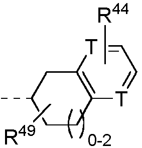 | 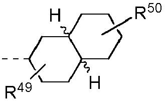 | 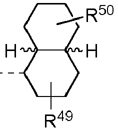 |
| **H104** | **H105** | **H106** | **H107** |
| 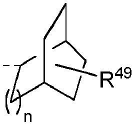 | 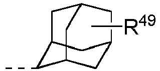 | | 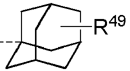 |
| **H108** | **H109** | | **H110** |
R⁴² is H; alkyl; alkenyl; cycloalkyl; cycloheteroalkyl; aryl; heteroaryl; -(CR²³R³³)ₛOR²¹; -(CR²³R³³)ₛNR⁴R⁴³; -(CR²³R³³)_{q}COOR²⁰; or -(CR²³R³³)_{q}CONR⁴R⁴³;
R⁴³ is H; alkyl; alkenyl; cycloalkyl; heterocycloalkyl; aryl; heteroaryl; arylalkyl; heteroarylalkyl; or a suitable N-protecting group;
R⁴⁴, R⁴⁵ and R⁴⁶ are independently defined as H; F; CF₃; OCF₃; OCHF₂; NO₂; CN; alkyl; alkenyl; alkynyl; cycloalkyl; heterocycloalkyl; aryl; heteroaryl; arylalkyl; heteroarylalkyl; -OR²³; -NR²⁸R⁴³; -NR²⁸COR²³; -NR²⁸SO₂R²³; -NR²⁸CONR²⁸R⁴³_{;} -COR²³; or -SO₂R²³;
R⁴⁷ is H; CF₃; alkyl; alkenyl; alkynyl; cycloalkyl; heterocycloalkyl; aryl; heteroaryl; arylalkyl; heteroarylalkyl; -COOR²¹; or -CONR²⁸R⁴³;
R⁴⁸ is H; F; CF₃; alkyl; alkenyl; cycloalkyl; cycloheteroalkyl; aryl; heteroaryl; -(CR²³R³³)ₜOR²⁰; -(CR²³R³³)ₜNR²⁸R⁴³; -(CR²³R³³)ₜCOOR²¹; or -(CR²³R³³)ₜCONR²¹R⁴³;
R⁴⁹ and R⁵⁰ are independently defined as H; F; CF₃; alkyl; alkenyl; cycloalkyl; heterocycloalkyl; aryl; heteroaryl; arylalkyl; heteroarylalkyl; -(CR³²R³³)_{q}OR²¹; -(CR³²R³³)_{q}NR²⁸R⁴³; -(CR³²R³³)_{q}COOR²¹; or -(CR³²R³³)_{q}CONR²⁸R⁴³;
R⁵¹ is H; F; Cl; CF₃; OCF₃; or lower alkyl;
R⁵² is H; F; Cl; CF₃; OCF₃; OCHF₂; NO₂; CN; lower alkyl; lower alkenyl; lower alkynyl; lower cycloalkyl; lower heterocycloalkyl; aryl; heteroaryl; lower arylalkyl; or lower heteroarylalkyl;
R⁵³ is CF₃; OCF₃; CN; lower alkyl; lower cycloalkyl; aryl; lower alkoxy; or aryloxy;
taken together (R⁴ and R¹¹); (R⁴ and R²⁷); (R⁵ and R⁶); (R⁵ and R⁷); (R⁵ and R⁹); (R⁵ and R¹⁴); (R⁵ and R¹⁶); (R⁷ and R⁸); (R⁷ and R⁹); (R⁷ and R¹⁶); (R⁹ and R¹⁰); (R¹⁴ and R¹⁵); (R¹⁶ and R¹⁷); (R¹⁸ and R¹⁹); (R²⁷ and R²⁸); (R²⁸ and R³¹); (R²⁸ and R⁴³); (R²⁹ and R³⁰); (R³² and R³³); (R³⁴ and R³⁵); (R³⁷ and R³⁸); (R³⁹ and R⁴⁰); (R³⁹ and R⁴¹); (R³⁹ and R⁴⁹); (R⁴² and R⁴³); (R⁴⁴ and R⁴⁵); or (R⁴⁴ and R⁴⁶) can form optionally substituted cycloalkyl or heterocycloalkyl moieties;
in addition, the structural elements -NR⁴R¹¹; -NR¹¹R²⁷; -NR²⁷R²⁸; -NR²⁸R³¹ or -NR²⁸R⁴³ can form one of the groups of the formulae
| | | | |
|---|---|---|---|
| 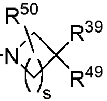 | 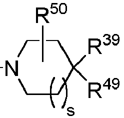 | 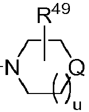 | 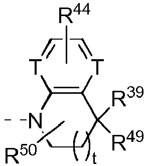 |
| **H111** | **H112** | **H113** | **H114** |
| 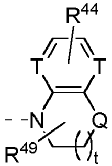 | 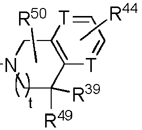 | 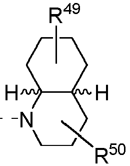 | 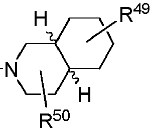 |
| **H115** | **H116** | **H117** | **H118** |
Z is O; S; S(=O); S(=O)₂; or NR²⁸;
Y is O; S; or NR⁴;
Q is O; S; or NR²⁸;
T is CR⁴⁶ or N; in case T occurs several times in the same ring structure each T is defined independently of the other;
q is an integer of 0-4;
r is an integer of 2-4;
s is an integer of 1-4;
t is an integer of 0-2;
u is an integer of 1-2;
and all possible stereoisomers of such compounds;
or salts, solvates, clathrates, N-oxides, isotopically enriched or enantiomerically enriched versions thereof.

2. Compounds according to claim 1 wherein
the building block of type **A** is selected from **A627 to A683;**
the building block of type **B** is selected from **B1** to **B21;**
the building block of type **C** is selected from **C1** to **C101;**
and wherein
M is -N(R⁴)-;
and all possible stereoisomers of such compounds;
or pharmaceutical acceptable salts thereof.

3. Compounds according to claim 1 wherein
the building block of type **A** is selected from
**A1**(a1); **A2**(a1); **A3**(a1); **A4**(a1); **A5**(a1); **A6**(a1); **A7**(a1); **A9**(a1); **A10**(a1); **A73**(a2); **A80**(a2); **A170**(a4); **A171**(a4); **A209**(a7); **A210**(a7); **A240**(a10); **A241**(a10); **A242**(a10); **A293**(a10); **A272**(a10); **A530**(a18); **A531**(a18); **A532**(a18); **A533**(a18); **A609**(a24); **A610**(a24); **A611**(a24); **A612**(a24); **A613**(a24); **A614**(a24); **A615**(a24); **A631**(a1); **A632**(a1); **A633**(a1); **A641**(a1); **A692**(a1); **A643**(a1); **A651**(a1); **A652**(a1); or **A653**(a1);
the building block of type **B** is selected from
**B4**(b3); **B5**(b3); **B6**(b3); **B7**(b3); **B8**(b3); **B9**(b3); **B10**(b3); **B12**(b4); **B13**(b4); **B14**(b4); **B15**(b4); **B16**(b4) or **B17**(b5);
the building block of type **C** is selected from
**C1**; **C2; C3; C4; C5; C6; C7; C8; C9; C10; C11; C12; C13; C14; C15; C16; C17; C18; C19; C20; C21; C25; C26; C27; C28; C29; C30; C34; C35; C36; C37; C38; C39; C43; C44; C45; C46; C47; C48; C49; C50; C51; C52; C53; C54; C55; C56; C57; C58; C59; C60; C61; C62; C63; C64; C65; C66; C67; C68; C69; C70; C71; C72; C73; C74; C75; C76; C77; C78; C79; C80; C81; C86; C87; C88; C89; C90; C91; C92;** or **C93;**
and wherein
R¹ is H; F; Cl; Br; I; CF₃; OCF₃; OCHF₂; NO₂; CN; lower alkyl; lower alkenyl; lower alkynyl; lower cycloalkyl; lower heterocycloalkyl; aryl; heteroaryl; lower arylalkyl; lower heteroarylalkyl; - (CR¹⁸R¹⁹)_{q}OR²⁰; -(CR¹⁸R¹⁹)_{q}NR⁴R¹¹; -(CR¹⁸R¹⁹)_{q}NR⁴COOR²¹; - (CR¹⁸R¹⁹)_{q}NR⁴COR²²; - (CR¹⁸R¹⁹)_{q}NR⁴CONR⁴R¹¹; -(CR¹⁸R¹⁹)_{q}NR⁴SO₂R²³; -(CR¹⁸R¹⁹)_{q}NR⁴SO₂NR⁴R¹¹; - (CR¹⁸R¹⁹)_{q}COOR²¹; -(CR¹⁸R¹⁹)_{q}CONR⁴R¹¹; - (CR¹⁸R¹⁹)_{q}SO₂NR⁴R¹¹; - (CR¹⁸R¹⁹)_{q}PO(OR²¹)₂; -(CR¹⁸R¹⁹)_{q}COR²²; -(CR¹⁸R¹⁹)_{q}SO₂R²³; -(CR¹⁸R¹⁹)_{q}OSO₃R²¹; - (CR¹⁸R¹⁹)_{q}R²⁴; -(CR¹⁸R¹⁹)_{q}R²⁵; or -(CR¹⁸R¹⁹)_{q}R²⁶;
R² is H; CF₃; lower alkyl; lower alkenyl; cycloalkyl; heterocycloalkyl; aryl; heteroaryl; lower arylalkyl; lower heteroarylalkyl; - (CR¹⁸R¹⁹)_{q}OR²⁰; -(CR¹⁸R¹⁹)_{q}NR⁴R¹¹; -(CR¹⁸R¹⁹)_{q}NR⁴COOR²¹; -(CR¹⁸R¹⁹)_{q}NR⁴COR²²; -(CR¹⁸R¹⁹)_{q}NR⁴CONR⁴R¹¹; -(CR¹⁸R¹⁹)_{q}NR⁴SO₂R²³; - (CR¹⁸R¹⁹)_{q}NR⁴SO₂NR⁴R¹¹; -(CR¹⁸R¹⁹)_{q}COOR²¹; -(CR¹⁸R¹⁹)_{q}CONR⁴R¹¹; -(CR¹⁸R¹⁹)_{q}SO₂NR⁴R¹¹; -(CR¹⁸R¹⁹)_{q}PO(OR²¹)₂; -(CR¹⁸R¹⁹)_{q}COR²²; -(CR¹⁸R¹⁹)_{q}SO₂R²³; -(CR¹⁸R¹⁹)_{q}R²⁴; -(CR¹⁸R¹⁹)_{q}R²⁵; or -(CR¹⁸R¹⁹)_{q}R²⁶;
R⁴ is H; lower alkyl; lower alkenyl; lower cycloalkyl; lower heterocycloalkyl; or a suitable N-protecting group;
R⁵, R⁷ and R⁹ are independently defined as H; F; CF₃; lower alkyl; lower alkenyl; lower cycloalkyl; lower heterocycloalkyl; aryl; heteroaryl; lower arylalkyl; lower heteroarylalkyl; -(CR¹⁸R¹⁹)_{q}OR²⁰; - (CR¹⁸R¹⁹)_{q}NR⁴R¹¹; -(CR¹⁸R¹⁹)_{q}NR⁴COOR²¹; -(CR¹⁸R¹⁹)_{q}NR⁴COR²²; -(CR¹⁸R¹⁹)_{q}NR⁴CONR⁴R¹¹; -(CR¹⁸R¹⁹)_{q}NR⁴SO₂R²³; -(CR¹⁸R¹⁹)_{q}NR⁴SO₂NR⁴R¹¹; -(CR¹⁸R¹⁹)_{q}COOR²¹; -(CR¹⁸R¹⁹)_{q}CONR⁴R¹¹; -(CR¹⁸R¹⁹)_{q}SO₂NR⁴R¹¹; - (CR¹⁸R¹⁹)_{q}PO(OR²¹)₂; -(CR¹⁸R¹⁹)_{q}COR²²; - (CR¹⁸R¹⁹)_{q}SO₂R²³; - (CR¹⁸R¹⁹)_{q}R²⁴; -(CR¹⁸R¹⁹)_{q}R²⁵; or -(CR¹⁸R¹⁹)_{q}R²⁶;
R⁶, R⁸ and R¹⁰ are independently defined as H; CF₃; or lower alkyl;
R¹¹ is H; alkyl; alkenyl; cycloalkyl; lower heterocycloalkyl; aryl; heteroaryl; lower arylalkyl; lower heteroarylalkyl; a suitable protecting group; -(CR¹⁸R¹⁹)ᵣOR²⁰; -(CR¹⁸R¹⁹)ᵣNR⁴R²⁷; -(CR¹⁸R¹⁹)ᵣNR⁴COOR²¹; -(CR¹⁸R¹⁹)ᵣNR⁴CONR⁴R²⁷; -(CR¹⁸R¹⁹)ᵣNR⁴SO₂R²³; -(CR¹⁸R¹⁹)ᵣNR⁴SO₂NR⁴R²⁷; -(CR¹⁸R¹⁹)_{q}COOR²¹; -(CR¹⁸R¹⁹)_{q}CONR⁴R²⁷; -(CR¹⁸R¹⁹)_{q}COR²²; -(CR¹⁸R¹⁹)_{q}SO₂R²³; -(CR¹⁸R¹⁹)_{q}SO₂NR⁴R²⁷; -(CR¹⁸R¹⁹)_{q}R²⁴; -(CR¹⁸R¹⁹)ₛR²⁵; -(CR¹⁸R¹⁹)_{q}R²⁶; or -(CR¹⁸R¹⁹)_{q}R³¹;
R¹² and R¹³ are independently defined as H; or lower alkyl;
R¹⁴ and R¹⁶ are independently defined as H; F; CF₃; lower alkyl; lower alkenyl; lower cycloalkyl; lower heterocycloalkyl; aryl; heteroaryl; lower arylalkyl; lower heteroarylalkyl; -(CR¹⁸R¹⁹)_{q}OR²⁰; -(CR¹⁸R¹⁹)_{q}NR⁴R¹¹; -(CR¹⁸R¹⁹)_{q}NR⁴COOR²¹; -(CR¹⁸R¹⁹)_{q}NR⁴COR²²; -(CR¹⁸R¹⁹)_{q}NR⁴CONR⁴R¹¹; -(CR¹⁸R¹⁹)_{q}NR⁴SO₂R²³; -(CR¹⁸R¹⁹)COOR²¹; -(CR¹⁸R¹⁹)_{q}CONR⁴R¹¹; or -(CR¹⁸R¹⁹)_{q}COR²²;
R¹⁵ and R¹⁷ are independently defined as H; CF₃; or lower alkyl;
R¹⁸ is H; F; CF₃; lower alkyl; lower alkenyl; lower cycloalkyl; lower heterocycloalkyl; aryl; heteroaryl; lower arylalkyl; lower heteroarylalkyl; -(CR²⁹R³⁰)ₛOR³¹; -(CR²⁹R³⁰)ₛNR²⁸R³¹; -(CR²⁹R³⁰)ₛNR²⁸COOR²¹; - (CR²⁹R³⁰)ₛNR²⁸COR³¹; -(CR²⁹R³⁰)ₛNR²⁸CONR²⁸R³¹; -(CR²⁹R³⁰)ₛNR²⁸SO₂R²³; -(CR²⁹R³⁰)ₛNR²⁸SO₂NR²⁸R³¹; -(CR²⁹R³⁰)_{q}COOR²¹; -(CR²⁹R³⁰)_{q}CONR²⁸R³¹_{;} -(CR²⁹R³⁰)_{q}SO₂NR²⁸R³¹; -(CR²⁹R³⁰)_{q}PO(OR²¹)₂; -(CR²⁹R³⁰)_{q}COR³¹; -(CR²⁹R³⁰)_{q}SO₂R²³; -(CR²⁹R³⁰)_{q}R²⁴; -(CR²⁹R³⁰)_{q}R²⁵; or -(CR²⁹R³⁰)_{q}R²⁶;
R¹⁹ is H; CF₃; or lower alkyl;
R²⁰ is H; lower alkyl; lower alkenyl; lower cycloalkyl; lower heterocycloalkyl; aryl; heteroaryl; lower arylalkyl; lower heteroarylalkyl; -(CR²⁹R³⁰)ᵣOR³¹; -(CR²⁹R³⁰)ᵣNR²⁸R³¹; -(CR²⁹R³⁰)ᵣNR²⁸COOR²¹; -(CR²⁹R³⁰)ᵣNR²⁸COR³¹; -(CR²⁹R³⁰)ᵣNR²⁸CONR²⁸R³¹; -(CR²⁹R³⁰)ᵣNR²⁸SO₂R²³; -(CR²⁹R³⁰)_{q}COOR²¹; -(CR²⁹R³⁰)_{q}CONR²⁸R³¹; -(CR²⁹R³⁰)_{q}SO₂NR²⁸R³¹; -(CR²⁹R³⁰)_{q}COR³¹; -(CR²⁹R³⁰)_{q}SO₂R²³; -(CR²⁹R³⁰)_{q}R²⁴; -(CR²⁹R³⁰)_{q}R²⁵; -(CR²⁹R³⁰)_{q}R²⁶; or -(CR²⁹R³⁰)_{q}R³¹;
R²² is lower alkyl; lower alkenyl; lower cycloalkyl; lower heterocycloalkyl; aryl; heteroaryl; lower arylalkyl; lower heteroarylalkyl; -(CR²⁹R³⁰)ₛOR³¹; -(CR²⁹R³⁰)ₛNR²⁸R³¹; -(CR²⁹R³⁰)ₛNR²⁸COOR²¹; - (CR²⁹R³⁰)ₛNR²⁸COR³¹; -(CR²⁹R³⁰)ₛNR²⁸CONR²⁸R³¹; -(CR²⁹R³⁰) ₛNR²⁸SO₂R²³; -(CR²⁹R³⁰)ₛCOOR²¹; -(CR²⁹R³⁰)ₛCONR²⁸R³¹; -(CR²⁹R³⁰)ₛSO₂NR²⁸R³¹; -(CR²⁹R³⁰)ₜCOR³¹; -(CR²⁹R³⁰)ₛSO₂R²³; -(CR²⁹R³⁰)ₜR²⁴; -(CR²⁹R³⁰)ₜR²⁵; -(CR²⁹R³⁰)ₜR²⁶; or -(CR²⁹R³⁰)ₜR³¹;
R²⁹ is H; F; CF₃; lower alkyl; lower alkenyl; lower cycloalkyl; lower heterocycloalkyl; aryl; heteroaryl; lower arylalkyl; lower heteroarylalkyl; - (CR³²R³³)ₛOR³¹; -(CR³²R³³)ₛNR²⁸R³¹; -(CR³²R³³)ₛNR²⁸COOR²¹; -(CR³²R³³)ₛNR²⁸COR³¹; -(CR³²R³³)ₛNR²⁸CONR²⁸R³¹; -(CR³²R³³)ₛNR²⁸SO₂R²³; -(CR³²R³³)_{q}COOR²¹; -(CR³²R³³)_{q}CONR²⁸R³¹; -(CR³²R³³)_{q}SO₂NR²⁸R³¹_{;} -(CR³²R³³)_{q}PO(OR²¹)₂; -(CR³²R³³)_{q}COR³¹; -(CR³²R³³)_{q}SO₂R²³; or -(CR³²R³³)_{q}R³¹;
R³⁰ and R³³ are independently defined as H; CF₃; or lower alkyl;
R³⁴ and R³⁵ are independently defined as H; F; Cl; CF₃; OCF₃; OCHF₂; NO₂; CN; lower alkyl; lower alkenyl; lower alkynyl; lower cycloalkyl; lower heterocycloalkyl; aryl; heteroaryl; lower arylalkyl; lower heteroarylalkyl; -(CR²⁹R³⁰)_{q}OR³¹; -(CR²⁹R³⁰)_{q}NR²⁸R³¹; -(CR²⁹R³⁰) _{q}NR²⁸COOR²¹; -(CR²⁹R³⁰)_{q}NR²⁸COR³¹; -(CR²⁹R³⁰)_{q}NR²⁸CONR²⁸R³¹; -(CR²⁹R³⁰)_{q}NR²⁸SO₂R²³; -(CR²⁹R³⁰)_{q}COOR²¹; -(CR²⁹R³⁰)_{q}CONR²⁸R³¹; -(CR²⁹R³⁰)_{q}SO₂NR²⁸R³¹; -(CR²⁹R³⁰)_{q}COR³¹; -(CR²⁹R³⁰)_{q}SO₂R²³; or -(CR²⁹R³⁰)_{q}R³¹;
R³⁷ is H; lower alkyl; lower alkenyl; lower cycloalkyl; lower heterocycloalkyl; aryl; heteroaryl; lower arylalkyl; lower heteroarylalkyl; a suitable N-protecting group; -(CR²⁹R³⁰)ᵣOR³¹; -(CR²⁹R³⁰)ᵣNR²⁸R³¹; -(CR²⁹R³⁰)ᵣNR²⁸COOR²¹; - (CR²⁹R³⁰)ᵣNR²⁸COR³¹; -(CR²⁹R³⁰)ᵣNR²⁸CONR²⁸R³¹; -(CR²⁹R³⁰)ᵣNR²⁸SO₂R²³; -(CR²⁹R³⁰)_{q}COOR²¹; -(CR²⁹R³⁰)_{q}CONR²⁸R³¹; -(CR²⁹R³⁰)ᵣSO₂NR²⁸R³¹; -(CR²⁹R³⁰)_{q}COR³¹; -(CR²⁹R³⁰)_{q}SO₂R²³; or -(CR²⁹R³⁰)_{q}R³¹;
R³⁸ is H; lower alkyl; lower alkenyl; lower cycloalkyl; lower heterocycloalkyl; aryl; heteroaryl; lower arylalkyl; lower heteroarylalkyl; -(CR²⁹R³⁰)_{q}OR³¹; -(CR²⁹R³⁰)_{q}NR²⁸R³¹; -(CR²⁹R³⁰)_{q}NR²⁸COOR²¹; -(CR²⁹R³⁰)_{q}NR²⁸COR³¹; -(CR²⁹R³⁰)_{q}NR²⁸CONR²⁸R³¹; -(CR²⁹R³⁰)_{q}COOR²¹; -(CR²⁹R³⁰)_{q}CONR₂₈R³¹; -(CR²⁹R³⁰)_{q}COR³¹; or -(CR²⁹R³⁰)_{q}R³¹;
Z is O; S; S(=O) ; or S(=O)₂;
U is -C(=O)-; -NR⁴-C(=O)-; -C(=O)-C(=O)-; or -C(-OR²⁰)₂-C(=O)-.

4. Compounds according to claim 1 wherein
the building block of type **A** is selected from
**A1**(a1); **A2**(a1); **A3**(a1); **A4**(a1); **A5**(a1); **A6**(a1); **A7**(a1); **A9**(a1); **A10**(a1); **A73**(a2); **A170**(a4); **A209**(a7); **A240**(a10); **A272**(a10); **A532**(a18); **A614**(a24); **A631**(a1); **A632**(a1); **A633**(a1); **A641**(a1); **A642**(a1); **A643**(a1); **A651**(a1); **A652**(a1); or **A653**(a1);
the building block of type **B** is selected from
**B7**(b3); **B8**(b3); **B9**(b3); **B10**(b3); or **B17**(b5);
the building block of type C is selected from
**C1**; **C2; C4; C7; C8; C9; C10; C11; C12; C13; C14; C15; C16; C17; C18; C19; C20; C21; C25; C26; C27; C28; C29; C30; C34; C35; C36; C37; C38; C39; C43; C44; C45; C46; C47**; **C48; C49; C90; C91; C92; or C93;**
and wherein
R¹ is H; F; Cl; CF₃; OCF₃; OCHF₂; NO₂; CN; lower alkyl; lower alkenyl; lower alkynyl; lower cycloalkyl; lower heterocycloalkyl; aryl; heteroaryl; lower arylalkyl; lower heteroarylalkyl; -(CR¹⁸R¹⁹)_{q}OR²⁰; -(CR¹⁸R¹⁹_{)q}NR⁴R¹¹; -(CR¹⁸R¹⁹)_{q}NR⁴COR²²; -(CR¹⁸R¹⁹)_{q}NR⁴CONR⁴R¹¹; -(CR¹⁸R¹⁹)_{q}NR⁴SO₂R²³; -(CR¹⁸R¹⁹)_{q}NR⁴SO₂NR⁴R¹¹; -(CR¹⁸R¹⁹)_{q}COOR²¹; - (CR¹⁸R¹⁹)_{q}CONR⁴R¹¹; -(CR¹⁸R¹⁹)_{q}SO₂NR⁴R¹¹; -(CR¹⁸R¹⁹)_{q}COR²²; -(CR¹⁸R¹⁹)_{q}SO₂R²³; -(CR¹⁸R¹⁹)_{q}R²⁴; -(CR¹⁸R¹⁹)_{q}R²⁵; or -(CR¹⁸R¹⁹)_{q}R²⁶;
R² is H; CF₃; lower alkyl; lower alkenyl; cycloalkyl; heterocycloalkyl; aryl; heteroaryl; lower arylalkyl; lower heteroarylalkyl; -(CR¹⁸R¹⁹)_{q}OR²⁰; -(CR¹⁸R¹⁹)_{q}NR⁴R¹¹; -(CR¹⁸R¹⁹)_{q}NR⁴COR²²; -(CR¹⁸R¹⁹)_{q}NR⁴CONR⁴R¹¹; -(CR¹⁸R¹⁹)_{q}NR⁴SO₂R²³; -(CR¹⁸R¹⁹)_{q}NR⁴SO₂NR⁴R¹¹; -(CR¹⁸R¹⁹)_{q}COOR²¹; -(CR¹⁸R¹⁹)_{q}CONR⁴R¹¹; -(CR¹⁸R¹⁹)_{q}SO₂NR⁴R¹¹; - (CR¹⁸R¹⁹)_{q}COR²²; -(CR¹⁸R¹⁹)_{q}SO₂R²³; -(CR¹⁸R¹⁹)_{q}R²⁴; -(CR¹⁸R¹⁹)_{q}R²⁵; or -(CR¹⁸R¹⁹)_{q}R²⁶;
R⁴ is H; lower alkyl; lower alkenyl; or a suitable N-protecting group;
R⁵, R⁷ and R⁹ are independently defined as H; CF₃; lower alkyl; lower alkenyl; lower cycloalkyl; lower heterocycloalkyl; aryl; heteroaryl; lower arylalkyl; lower heteroarylalkyl; -(CR¹⁸R¹⁹)_{q}OR²⁰; -(CR¹⁸R¹⁹)_{q}NR⁴R¹¹; -(CR¹⁸R¹⁹)_{q}NR₄COR²²; -(CR¹⁸R¹⁹)_{q}NR⁴CONR⁴R¹¹; -(CR¹⁸R¹⁹)_{q}NR⁴SO₂R²³; -(CR¹⁸R¹⁹)_{q}COOR²¹; -(CR¹⁸R¹⁹)_{q}CONR⁴R¹¹; -(CR¹⁸R¹⁹)_{q}SO₂NR⁴R¹¹; -(CR¹⁸R¹⁹)_{q}COR²²; -(CR¹⁸R¹⁹)_{q}SO₂R²³; -(CR¹⁸R¹⁹)_{q}R²⁴; -(CR¹⁸R¹⁹)_{q}R²⁵; or -(CR¹⁸R¹⁹)_{q}R²⁶;
R⁶, R⁸ and R¹⁰ are independently defined as H; CF₃; or CH₃;
R¹¹ is H; alkyl; alkenyl; cycloalkyl; lower heterocycloalkyl; aryl; heteroaryl; lower arylalkyl; lower heteroarylalkyl; a suitable protecting group; -(CR¹⁸R¹⁹)ᵣOR²⁰; -(CR¹⁸R¹⁹)ᵣNR⁴R²⁷; -(CR¹⁸R¹⁹)ᵣNR⁴CONR⁴R²⁷; -(CR¹⁸R¹⁹)ᵣNR⁴SO₂R²³; -(CR¹⁸R¹⁹)_{q}COOR²¹; - (CR¹⁸R¹⁹)_{q}CONR⁴R²⁷; -(CR¹⁸R¹⁹)_{q}COR²²; - (CR¹⁸R¹⁹)_{q}R²⁴; -(CR¹⁸R¹⁹)ₛR²⁵; -(CR¹⁸R¹⁹)_{q}R²⁶; or -(CR¹⁸R¹⁹)_{q}R³¹;
R¹² and R¹³ are independently defined as H; or lower alkyl;
R¹⁴ and R¹⁶ are independently defined as H; F; CF₃; lower alkyl; lower alkenyl; lower cycloalkyl; lower heterocycloalkyl; aryl; heteroaryl; lower arylalkyl; lower heteroarylalkyl; -(CR¹⁸R¹⁹)_{q}OR²⁰; -(CR¹⁸R¹⁹)_{q}NR⁴R¹¹; -(CR¹⁸R¹⁹)_{q}NR⁴COR²²; -(CR¹⁸R¹⁹)_{q}COOR²¹; or -(CR¹⁸R¹⁹)_{q}CONR⁴R¹¹;
R¹⁵ and R¹⁷ are independently defined as H; CF₃; or CH₃;
R¹⁸ is H; F; CF₃; lower alkyl; lower alkenyl; lower cycloalkyl; lower heterocycloalkyl; aryl; heteroaryl; lower arylalkyl; lower heteroarylalkyl; - (CR²⁹R³⁰)ₛOR³¹; -(CR²⁹R³⁰)ₛNR²⁸R³¹; -(CR²⁹R³⁰)ₛNR²⁸COR³¹; -(CR²⁹R³⁰)ₛNR²⁸CONR²⁸R³¹; -(CR²⁹R³⁰)ₛNR²⁸SO₂R²³; -(CR²⁹R³⁰)_{q}COOR²¹; -(CR²⁹R³⁰)_{q}CONR²⁸R^{31;} -(CR²⁹R³⁰)_{q}SO₂NR²⁸R³¹; -(CR²⁹R³⁰)_{q}COR³¹; -(CR²⁹R³⁰)_{q}SO₂R²³; -(CR²⁹R³⁰)_{q}R²⁴; -(CR²⁹R³⁰)_{q}R²⁵; or -(CR²⁹R³⁰)_{q}R²⁶;
R¹⁹ is H; CF₃; or CH₃;
R²⁰ is H; lower alkyl; lower alkenyl; lower cycloalkyl; lower heterocycloalkyl; aryl; heteroaryl; lower arylalkyl; lower heteroarylalkyl; -(CR²⁹R³⁰)ᵣOR³¹; -(CR²⁹R³⁰)ᵣNR²⁸R³¹; -(CR²⁹R³⁰)ᵣNR²⁸COR³¹; -(CR²⁹R³⁰)ᵣNR²⁸CONR²⁸R³¹; -(CR²⁹R³⁰)ᵣNR²⁸SO₂R²³; -(CR²⁹R³⁰)_{q}COOR²¹; -(CR²⁹R³⁰)_{q}CONR²⁸R³1; -(CR²⁹R³⁰)_{q}SO₂NR²⁸R³¹_{;} -(CR²⁹R³⁰)_{q}COR³¹; -(CR²⁹R³⁰)_{q}SO₂R²³; -(CR²⁹R³⁰)_{q}R²⁴; -(CR²⁹R³⁰)_{q}R²⁵; -(CR²⁹R³⁰)_{q}R²⁶; or -(CR²⁹R³⁰)_{q}R³¹;
R²² is lower alkyl; lower alkenyl; lower cycloalkyl; lower heterocycloalkyl; aryl; heteroaryl; lower arylalkyl; lower heteroarylalkyl; -(CR²⁹R³⁰)ₛOR³¹; -(CR²⁹R³⁰)ₛNR²⁸R³¹; -(CR²⁹R³⁰)ₛNR²⁸COR³¹; -(CR²⁹R³⁰)ₛNR²⁸CONR²⁸R³¹; -(CR²⁹R³⁰)ₛNR²⁸SO₂R²³; -(CR²⁹R³⁰)ₛCOOR²¹; -(CR²⁹R³⁰)ₛCONR²⁸R³¹; -(CR²⁹R³⁰)ₛSO₂NR²⁸R³¹; -(CR²⁹R³⁰)ₜCOR³¹; -(CR²⁹R³⁰)ₛSO₂R²³; -(CR²⁹R³⁰)ₜR²⁴; -(CR²⁹R³⁰)ₜR²⁵; -(CR²⁹R³⁰)ₜR²⁶; or -(CR²⁹R³⁰)ₜR³¹;
R²⁹ is H; F; CF₃; lower alkyl; lower alkenyl; lower cycloalkyl; lower heterocycloalkyl; aryl; heteroaryl; lower arylalkyl; lower heteroarylalkyl; -(CR³²R³³)ₛOR³¹; -(CR³²R³³)ₛNR²⁸R³¹; -(CR³²R³³)ₛNR²⁸COR³¹; -(CR³²R³³)ₛNR²⁸CONR²⁸R³¹; -(CR³²R³³)_{q}COOR²¹; -(CR³²R³³)_{q}CONR²⁸R³¹; -(CR³²R³³)_{q}COR³¹; or -(CR³²R³³)_{q}R³¹;
R³⁰ and R³³ are independently defined as H; CF₃; or CH₃;
R³⁴ and R³⁵ are independently defined as H; F; Cl; CF₃; OCF₃; OCHF₂; lower alkyl; lower alkenyl; lower alkynyl; lower cycloalkyl; lower heterocycloalkyl; aryl; heteroaryl; lower arylalkyl; lower heteroarylalkyl; -(CR²⁹R³⁰)_{q}OR³¹; -(CR²⁹R³⁰)_{q}NR²⁸R³¹; -(CR²⁹R³⁰)_{q}NR²⁸COR³¹; -(CR²⁹R³⁰)_{q}NR²⁸CONR²⁸R³¹; -(CR²⁹R³⁰)_{q}NR²⁸SO₂R²³; -(CR²⁹R³⁰)_{q}COOR²¹; -(CR²⁹R³⁰)_{q}CONR²⁸R³¹; -(CR²⁹R³⁰)_{q}SO₂NR²⁸R³¹; -(CR²⁹R³⁰)_{q}COR³¹; -(CR²⁹R³⁰)_{q}SO₂R²³; or -(CR²⁹R³⁰)_{q}R³¹;
R³⁷ is H; lower alkyl; lower alkenyl; lower cycloalkyl; lower heterocycloalkyl; aryl; heteroaryl; lower arylalkyl; lower heteroarylalkyl; a suitable N-protecting group; -(CR²⁹R³⁰)ᵣOR³¹; -(CR²⁹R³⁰)ᵣNR²⁸R³¹; -(CR²⁹R³⁰)ᵣNR²⁸COOR²¹; -(CR²⁹R³⁰)ᵣNR²⁸COR³¹; -(CR²⁹R³⁰)ᵣNR²⁸CONR²⁸R³¹; -(CR²⁹R³⁰)_{q}COOR²¹; -(CR²⁹R³⁰)_{q}CONR²⁸R³¹; - (CR²⁹R³⁰)_{q}COR³¹; or -(CR²⁹R³⁰)_{q}R³¹;
R³⁸ is H; lower alkyl; lower alkenyl; lower cycloalkyl; lower heterocycloalkyl; aryl; heteroaryl; lower arylalkyl; lower heteroarylalkyl; -(CR²⁹R³⁰)_{q}OR³¹; -(CR²⁹R³⁰)_{q}NR²⁸R³¹; -(CR²⁹R³⁰)_{q}NR²⁸COR³¹; - (CR²⁹R³⁰)_{q}NR²⁸CONR²⁸R³¹; -(CR²⁹R³⁰)_{q}COOR²¹; -(CR²⁹R³⁰)_{q}CONR²⁸R³¹; -(CR²⁹R³⁰)_{q}COR³¹; or -(CR²⁹R³⁰)_{q}R³¹;
Z is O; S or S(=O)₂;
U is -C(=O)-; -NR⁴-C(=O)-; or -C(=O)-C(=O)-.

5. Compounds according to claim 1
wherein
the building block of type **A** is selected from
**A1**(a1); **A2**(a1); **A3**(a1); **A4**(a1); **A5**(a1); **A6**(a1); **A7**(a1); **A9**(a1); **A10**(a1); **A73**(a2); **A170**(a4); **A209**(a7); **A240**(a10); **A272**(a10); **A532**(a18); **A614**(a24); **A631**(a1); **A632**(a1); **A633**(a1); **A641**(a1); **A642**(a1); **A643**(a1); **A651**(a1); **A652**(a1); or **A653**(a1);
the building block of type **B** is selected from
**B7**(b3); **B8**(b3); **B9**(b3); **B10**(b3); or **B17**(b5);
the building block of type **C** is selected from
**C1**; **C8; C9; C11**; **C12; C15; C17; C19; C20; C25; or C47;**
and wherein
R¹ is H; F; Cl; Br; CF₃; OCF₃; OCHF₂; CN; lower alkyl; lower alkenyl; cycloalkyl; heterocycloalkyl; aryl; heteroaryl; lower arylalkyl; lower heteroarylalkyl; -(CR²⁹R³⁰)_{q}OR²⁰_{;} -(CR²⁹R³⁰)_{q}NR⁴R¹¹; -(CR²⁹R³⁰)_{q}NR⁴COR²²; -(CR²⁹R³⁰)_{q}NR⁴CONR⁴R¹¹; -(CR²⁹R³⁰)_{q}NR⁴SO₂R²³; -(CR²⁹R³⁰)_{q}COOR²¹; -(CR²⁹R³⁰)_{q}CONR⁴R¹¹; -(CR²⁹R³⁰)_{q}SO₂NR⁴R¹¹; -(CR²⁹R³⁰)_{q}COR²²; -(CR²⁹R³⁰)_{q}SO₂R²³; -(CR²⁹R³⁰)_{q}R²⁴; -(CR²⁹R³⁰)_{q}R²⁵; or -(CR²⁹R³⁰)_{q}R²⁶;
R² is H; CF₃; lower alkyl; lower alkenyl; lower cycloalkyl; lower heterocycloalkyl; aryl; heteroaryl; lower arylalkyl; lower heteroarylalkyl; -(CR²⁹R³⁰)_{q}OR²⁰; -(CR²⁹R³⁰)_{q}NR⁴R¹¹; -(CR²⁹R³⁰)_{q}NR⁴COR²²; -(CR²⁹R³⁰)_{q}NR⁴CONR⁴R¹¹; -(CR²⁹R³⁰)_{q}NR⁴SO₂R²³; -(CR²⁹R³⁰)_{q}COOR²¹; -(CR²⁹R³⁰)_{q}CONR⁴R¹¹; -(CR²⁹R³⁰)_{q}SO₂NR⁴R¹¹; -(CR²⁹R³⁰)_{q}COR²²; -(CR²⁹R³⁰)_{q}SO₂R²³; -(CR²⁹R³⁰)_{q}R²⁴; -(CR²⁹R³⁰)_{q}R²⁵; or -(CR²⁹R³⁰)_{q}R²⁶;
R⁴ is H; acetyl; lower alkyl; lower alkenyl; or a suitable N-protecting group;
R⁵ R⁷ and R⁹ are independently defined as H; CF₃; lower alkyl; lower alkenyl; lower cycloalkyl; lower heterocycloalkyl; aryl; heteroaryl; lower arylalkyl; lower heteroarylalkyl; -(CR¹⁸R¹⁹)_{q}OR²⁰; -(CR¹⁸R¹⁹)_{q}NR⁴R¹¹; -(CR¹⁸R¹⁹)_{q}NR⁴COR²²; -(CR¹⁸R¹⁹)_{q}NR⁴CONR⁴R¹¹; -(CR¹⁸R¹⁹)_{q}NR⁴SO₂R²³ ; -(CR¹⁸R¹⁹)_{q}COOR²¹; -(CR¹⁸R¹⁹)_{q}CONR⁴R¹¹; -(CR¹⁸R¹⁹)_{q}SO₂NR⁴R¹¹; -(CR¹⁸R¹⁹)_{q}COR²²; -(CR¹⁸R¹⁹)_{q}SO₂R²³; -(CR¹⁸R¹⁹)_{q}R²⁴; -(CR¹⁸R¹⁹)_{q}R²⁵; or -(CR¹⁸R¹⁹)_{q}R²⁶;
R⁶, R⁸ and R¹⁰ are independently defined as H; CF₃; CH₃; or benzyl;
R¹¹ is H; alkyl; alkenyl; cycloalkyl; lower heterocycloalkyl; aryl; heteroaryl; lower arylalkyl; lower heteroarylalkyl; a suitable protecting group; -(CR¹⁸R¹⁹)ᵣOR²⁰; -(CR¹⁸R¹⁹)ᵣNR⁴R²⁷; -(CR¹⁸R¹⁹)ᵣNR⁴CONR⁴R²⁷; -(CR¹⁸R¹⁹)ᵣNR⁴SO₂R²³; -(CR¹⁸R¹⁹)_{q}COOR²¹; -(CR¹⁸R¹⁹)_{q}CONR⁴R²⁷; -(CR¹⁸R¹⁹)_{q}COR²²; -(CR¹⁸R¹⁹_{)q}R²⁴; -(CR¹⁸R¹⁹)ₛR²⁵; -(CR¹⁸R¹⁹)_{q}R²⁶; or -(CR¹⁸R¹⁹)qR³¹;
R¹² and R¹³ are independently defined as H; or lower alkyl;
R¹⁴ and R¹⁶ are independently defined as H; F; CF₃; lower alkyl; lower alkenyl; lower cycloalkyl; lower heterocycloalkyl; aryl; heteroaryl; lower arylalkyl; lower heteroarylalkyl; -(CR²⁹R³⁰)_{q}OR²⁰; -(CR²⁹R³⁰)_{q}NR⁴R¹¹; -(CR²⁹R³⁰)_{q}NR⁴COR²²; -(CR²⁹R³⁰)_{q}COOR²¹; or -(CR²⁹R³⁰)_{q}CONR⁴R¹¹;
R¹⁵ and R¹⁷ are independently defined as H; CF₃; or CH₃;
R¹⁸ is H; F; CF₃; lower alkyl; lower alkenyl; lower cycloalkyl; lower heterocycloalkyl; aryl; heteroaryl; lower arylalkyl; lower heteroarylalkyl; -(CR²⁹R³⁰)ₛOR³¹; -(CR²⁹R³⁰)ₛNR²⁸R³¹; -(CR²⁹R³⁰)ₛNR²⁸COR³¹; -(CR²⁹R³⁰)ₛNR²⁸CONR²⁸R³¹; -(CR²⁹R³⁰)ₛNR²⁸SO₂R²³; -(CR²⁹R³⁰)_{q}COOR²¹; -(CR²⁹R³⁰)_{q}CONR²⁸R³¹; -(CR²⁹R³⁰)_{q}SO₂NR²⁸R³¹; -(CR²⁹R³⁰)_{q}COR³¹; -(CR²⁹R³⁰)_{q}SO₂R²³; -(CR²⁹R³⁰)_{q}R²⁴; -(CR²⁹R³⁰)_{q}R²⁵; or -(CR²⁹R³⁰)_{q}R²⁶;
R¹⁹ is H; CF₃; or CH₃;
R²⁰ is H; lower alkyl; lower alkenyl; lower cycloalkyl; lower heterocycloalkyl; aryl; heteroaryl; lower arylalkyl; lower heteroarylalkyl; -(CR²⁹R³⁰)ᵣOR³¹; -(CR²⁹R³⁰)ᵣNR²⁸R³¹; -(CR²⁹R³⁰)ᵣNR²⁸COR³¹; - (CR²⁹R³⁰)ᵣNR²⁸CONR²⁸R³¹; -(CR²⁹R³⁰)ᵣNR²⁸SO₂R²³; -(CR²⁹R³⁰)_{q}COOR²¹; -(CR²⁹R³⁰)_{q}CONR²⁸R³¹_{;} -(CR²⁹R³⁰)_{q}SO₂NR²⁸R³¹; -(CR²⁹R³⁰)_{q}COR³¹; -(CR²⁹R³⁰)_{q}SO₂R²³; -(CR²⁹R³⁰)_{q}R²⁴; - (CR²⁹R³⁰)_{q}R²⁵; -(CR²⁹R³⁰)_{q}R²⁶; or -(CR²⁹R³⁰)_{q}R³¹;
R²² is lower alkyl; lower alkenyl; lower cycloalkyl; lower heterocycloalkyl; aryl; heteroaryl; lower arylalkyl; lower heteroarylalkyl; -(CR²⁹R³⁰)ₛOR³¹; -(CR²⁹R³⁰)ₛNR²⁸R³¹; -(CR²⁹R³⁰)ₛNR²⁸COR³¹; -(CR²⁹R³⁰)ₛNR²⁸CONR²⁸R³¹; -(CR²⁹R³⁰)ₛNR²⁸SO₂R²³; -(CR²⁹R³⁰)ₛCOOR²¹; -(CR²⁹R³⁰)ₛCONR²⁸R³¹; -(CR²⁹R³⁰)ₛSO₂NR²⁸R³¹; -(CR²⁹R³⁰)ₜCOR³¹; -(CR²⁹R³⁰)ₛSO₂R²³; -(CR²⁹R³⁰)ₜR²⁴; -(CR²⁹R³⁰)ₜR²⁵; -(CR²⁹R³⁰)ₜR²⁶; or -(CR²⁹R³⁰)ₜR³¹;
R²⁴ is aryl; heteroaryl; or -C₆H₂R³⁴R³⁵R³¹;
R²⁵ and R²⁶ are independently defined as heterocycloalkyl;
R²⁷ is H; lower alkyl; lower alkenyl; lower cycloalkyl; lower heterocycloalkyl; aryl; heteroaryl; lower arylalkyl; lower heteroarylalkyl; a suitable protecting group; or -(CR²⁹R³⁰)_{q}R²⁴;
R²⁸ is H; -(CR³²R³³)ₛOR²¹; or -(CR³²R³³)ₛNR⁴³R⁴²;
R²⁹ is H; F; CF₃; lower alkyl; lower alkenyl; lower cycloalkyl; lower heterocycloalkyl; aryl; heteroaryl; lower arylalkyl; lower heteroarylalkyl; -(CR³²R³³)ₛOR³¹; -(CR³²R³³)ₛNR²⁸R³¹; -(CR³²R³³)ₛNR²⁸COR³¹; -(CR³²R³³)ₛNR²⁸CONR²⁸R³¹; -(CR³²R³³)_{q}COOR²¹; -(CR³²R³³)_{q}CONR²⁸R³¹; -(CR³²R³³)_{q}COR³¹; or -(CR³²R³³)_{q}R³¹;
R³⁰ and R³³ are independently defined as H; F; CF₃; or CH₃;
R³¹ is H; alkyl; alkenyl; cycloalkyl; heterocycloalkyl; aryl; heteroaryl; arylalkyl; or heteroarylalkyl;
R³² is H; F; CF₃; lower alkyl; lower alkenyl; lower cycloalkyl; lower heterocycloalkyl; aryl; heteroaryl; lower arylalkyl; or lower heteroarylalkyl;
R³⁴ and R³⁵ are independently defined as H; F; Cl; CF₃; lower alkyl; lower alkenyl; lower cycloalkyl; lower heterocycloalkyl; aryl; heteroaryl; lower arylalkyl; lower heteroarylalkyl; -(CR²⁹R³⁰)_{q}OR³¹; -(CR²⁹R³⁰)_{q}NR²⁸R³¹; -(CR²⁹R³⁰)_{q}NR²⁸COR³¹; -(CR²⁹R³⁰)_{q}NR²⁸CONR²⁸R³¹; -(CR²⁹R³⁰)_{q}NR²⁸SO₂R²³; -(CR²⁹R³⁰)_{q}COOR²¹; -(CR²⁹R³⁰)_{q}CONR²⁸R³¹; -(CR²⁹R³⁰)_{q}SO₂NR²⁸R³¹; -(CR²⁹R³⁰)_{q}COR³¹; -(CR²⁹R³⁰)_{q}SO₂R²³; or -(CR²⁹R³⁰)_{q}R³¹;
R³⁸ is H; lower alkyl; lower alkenyl; lower cycloalkyl; lower heterocycloalkyl; aryl; heteroaryl; lower arylalkyl; lower heteroarylalkyl; -(CR²⁹R³⁰)_{q}OR³¹; -(CR²⁹R³⁰)_{q}NR²⁸R³¹; -(CR²⁹R³⁰)_{q}NR²⁸COR³¹; -(CR²⁹R³⁰)_{q}NR²⁸CONR²⁸R³¹; -(CR²⁹R³⁰)_{q}COOR²¹; -(CR²⁹R³⁰)_{q}CONR²⁸R³¹; -(CR²⁹R³⁰)_{q}COR³¹; or -(CR²⁹R³⁰)_{q}R³¹;
R⁴² and R⁴³ are independently defined as H; lower alkyl; lower alkenyl; lower cycloalkyl; lower heterocycloalkyl; aryl; heteroaryl; lower arylalkyl; lower heteroarylalkyl; or a suitable N-protecting group;
X is O; S; S(=0); or S(=O)₂;
Y is O; S; or NR⁴;
Z is O; S or S(=O)₂;
U is -C(=O)-; -NR⁴-C(=O)-; or -C(=O)-C(=O)-;
or pharmaceutically acceptable salts, solvates, clathrates, isotopically enriched or enantiomerically enriched versions thereof.

6. Compounds according to claim 1 wherein
the building block of type **A** is selected from
**A2**(a1); **A5**(a1); **A9**(a1); **A73**(a2); **A209**(a7); **A272**(a10); **A532**(a18); **A614**(a24); **A691**(a1); or **A651**(a1);
the building block of type **B** is selected from
**B7; B9; B10** or **B17;**
the building block of type **C** is selected from
**C1; C8; C9; C11; C15; C17; C19; C20; C25;** or **C47;**
and wherein
R¹ is H; F; Cl; Br; CH₃; OCH₃; OH; or aryl;
R² is H; CH₃; heterocycloalkyl; aryl; benzyl; heteroaryl; -OR²⁰; -NR⁴R¹¹; -NR⁴COOR²¹; -NR⁴COR²²; -NR⁴CONR⁴R¹¹; or -NR⁴SO₂R²³;
R³ is H;
R⁴ is H; acetyl; lower alkyl; lower alkenyl; or a suitable N-protecting group;
R⁵, R⁷ and R⁹ are independently defined as H; CF₃; lower alkyl; lower alkenyl; lower cycloalkyl; lower heterocycloalkyl; aryl; heteroaryl; lower arylalkyl; lower heteroarylalkyl; -OR²⁰; -NR⁴R¹¹; -NR⁴COR²²; -NR⁴CONR⁴R¹¹; -NR⁴SO₂R²³; -COOR²¹;-CONR⁴R¹¹; or -COR²²;
R⁶, R⁸ and R¹⁰ are defined as H;
R¹¹ is H; alkyl; aryl; heteroaryl; lower arylalkyl; lower heteroarylalkyl; -(CR¹⁸R¹⁹)ᵣOR²⁰; -(CR¹⁸R¹⁹)ᵣNR⁴R²⁷; -(CR¹⁸R¹⁹)_{q}COOR²¹; -COR²²; -(CR¹⁸R¹⁹)_{q}R²⁴; or -(CR¹⁸R¹⁹)_{q}R³¹;
R¹² and R¹³ are defined as H;
R¹⁴ and R¹⁶ are independently defined as H; lower alkyl; or lower arylalkyl;
R¹⁵ and R¹⁷ are defined as H;
R¹⁸ and R¹⁹ are defined as H;
R²⁰ is H; aryl; heteroaryl; lower arylalkyl; lower heteroarylalkyl; or -(CR²⁹R³⁰)_{q}R²⁴;
R²¹ is H; lower alkyl; lower arylalkyl; or a suitable *O-*protecting group;
R²² is lower alkyl; lower alkenyl; aryl; heteroaryl; lower arylalkyl; lower heteroarylalkyl; -(CR²⁹R³⁰)ₛOR³¹; -(CR²⁹R³⁰)ₛNR²⁸R³¹; -(CR²⁹R³⁰) sCOOR²¹; -(CR²⁹R³⁰)ₜR²⁴; or -(CR²⁹R³⁰)ₜR³¹;
R²³ is H; lower alkyl; aryl; heteroaryl; or -R²⁴;
R²⁴ is aryl; heteroaryl; or -C₆H₂R³⁴R³⁵R³¹;
R²⁷ is H; acetyl; CH₃; or -(CH₂)qR²⁴;
R²⁸ is H; -(CR³²R³³)ₛOR²¹; or -(CR³²R³³)ₛNR⁴³R⁴²;
R²⁹ is H; lower alkyl; aryl; or heteroaryl;
R³⁰, R³² and R³³ are independently defined as H; CF₃; or CH₃;
R³¹ is H; alkyl; cycloalkyl; heterocycloalkyl; aryl; heteroaryl; arylalkyl; or heteroarylalkyl;
R³⁴ and R³⁵ are independently defined as H; heterocycloalkyl; -OR³¹; or -NR²⁸R³¹;
R³⁸ is H; -NR²⁸R³¹; -NR²⁸COR³¹; or -NR²⁸COOR³¹;
R⁴² and R⁴³ are independently defined as H; CH₃; or benzyl;
X is O; S; S(=O); or S(=O)₂;
Y is O; S; or NR⁴;
Z is O; S or S(=O)₂;
U is -C(=O)-; -NR⁴-C(=O)-; or -C(=O)-C(=O)-;
or pharmaceutically acceptable salts, solvates, clathrates, isotopically enriched or enantiomerically enriched versions thereof.

7. Compounds according to claim 4 wherein
the building block of type **C** is represented by 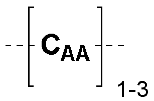 and wherein
**C_{AA}** is an amino acid or its complemetary enatiomer selected from the list of
Ala L-Alanine;
Arg L-Arginine;
Asn L-Asparagine;
Asp L-Aspartic acid;
Cys L-Cysteine;
Glu L-Glutamic acid;
Gln L-Glutamine;
Gly Glycine;
His L-Histidine;
Ile L-Isoleucine;
Leu L-Leucine;
Lys L-Lysine;
Met L-Methionine;
Phe L-Phenylalanine;
Pro L-Proline;
Ser L-Serine;
Thr L-Threonine;
Trp L-Tryptophan;
Tyr L-Tyrosine;
Val L-Valine;
Apa 3-Amino-propanoic acid;
H-β³-HAla-OH (3S)-3-Amino-butyric acid;
H-β³-HVal-OH (3R)-3-Amino-4-methyl-valeric acid;
H-β³-HIle-OH (3R, 4S)-3-Amino-4-methyl-hexanoic acid;
H-β³-HLeu-OH (3S)-3-Amino-5-methyl-hexanoic acid;
H-a³-HMet-OH (3S)-3-Amino-5-methylthio pentanoic acid;
H-β³-HTyr-OH (3S)-3-Amino-4-(4'-hydroxyphenyl)-butyric acid;
H-β³-HHis-OH (3S)-3-Amino-4-(imidazole-4'-yl)-butyric acid;
H-β³-HPhe-OH (3S)-3-Amino-4-phenyl butyric acid;
H-β³-HTrp-OH (3S)-3-Amino-4-(indol-3'-yl)-butyric acid;
H-β³-HSer-OH (3R)-3-Amino-4-hydroxy-butyric acid;
H-β³-HAsp-OH 3-Amino-pentanedioic acid;
H-β³-HGlu-OH (3S)-3-Amino-hexanedioic acid;
H-β³-HLys-OH (3S)-3,7-Diamino-heptanoic acid;
H-β³-HArg-OH (3S)-3-Amino-6-guanidino-hexanoic-acid;
H-β³-HCys-OH (3R)-3-Amino-4-mercapto-butyric acid;
H-β³-HAsn-OH (3S)-3-Amino-4-carbamoyl-butyric acid;
H-β³-HGln-OH (3S)-3-Amino-5-carbamoyl-pentanoic acid;
H-β³-HThr-OH (3R,4R)-3-Amino-4-hydroxy-pentanoic acid;
Gaba 4-Amino-butyric acid;
H-γ⁴-DiHAla-OH (4S)-4-Amino-pentanoic acid;
H-γ⁴-DiHVal-OH (4R)-4-Amino-5-methyl-hexanoic acid;
H-γ⁴-DiHIle-OH (4R, 5S)-4-Amino-5-methyl-heptanoic acid;
H-γ⁴-DiHLeu-OH (4R)-4-Amino-6-methyl-heptanoic acid;
H-γ⁴-DiHMet-OH (4R)-4-Amino-6-methylthio-hexanoic acid;
H-γ⁴-DiHTyr-OH (4R)-4-Amino-5-(4'-hydroxyphenyl)-pentanoic acid;
H-γ⁴-DiHHis-OH (4R)-4-Amino-5-(imidazole-4'-yl)-pentanoic acid;
H-γ⁴-DiHPhe-OH (4R)-4-Amino-5-phenyl-pentanoic acid;
H-γ⁴-DiHTrp-OH (4R)-4-Amino-5-(indol-3'-yl)-pentanoic acid;
H-γ⁴-DiHSer-OH (4R)-4-Amino-5-hydroxy-pentanoic acid;
H-γ⁴-DiHAsp-OH (4R)-4-Amino-hexanedioic acid;
H-γ⁴-DiHGlu-OH 4-Amino-heptanedioic acid;
H-γ⁴-DiHLys-OH (4S)-4,8-Diamino-octanoic acid;
H-γ⁴-DiHArg-OH (4S)-4-Amino-7-guanidino-heptanoic-acid;
H-γ⁴-DiHCys-OH (4R)-4-Amino-5-mercapto-pentanoic acid;
H-γ⁴-DiHAsn-OH (4R)-4-Amino-5-carbamoyl-pentanoic acid;
H-y⁴-DiHGln-OH (3S)-3-Amino-5-carbamoyl-hexanoic acid;
H-y⁴-DiHThr-OH (4R, 5R)-4-Amino-5-hydroxy- hexanoic acid;
Cit L-Citrulline;
Orn L-Ornithine;
tBuA L-t-Butylalanine;
Sar Sarcosine;
Pen L-Penicillamine;
tBuG L-tert.-Butylglycine;
4AmPhe L-para-Aminophenylalanine;
3AmPhe L-meta-Aminophenylalanine;
2AmPhe L-ortho-Aminophenylalanine;
Phe(mC(NH₂)=NH) L-meta-Amidinophenylalanine;
Phe(pC(NH₂)=NH) L-para-Amidinophenylalanine;
Phe(mNHC(NH₂)=NH) L-meta-Guanidinophenylalanine;
Phe(pNHC(NH₂)=NH) L-para-Guanidinophenylalanine;
2Pal (2S)-2-Amino-3-(pyridine-2'-yl)-propionic acid;
4Pal (2S)-2-Amino-3-(pyridine-4'-yl)-propionic acid;
Phg L-Phenylglycine;
Cha L-Cyclohexylalanine;
C₄al L-3-Cyclobutylalanine;
C₅al L-3-Cyclopentylalanine;
Nle L-Norleucine;
2-Nal L-2-Naphthylalanine;
1-Nal L-1-Naphthylalanine;
4ClPhe L-4-Chlorophenylalanine;
3ClPhe L-3-Chlorophenylalanine;
2ClPhe L-2-Chlorophenylalanine;
3,4Cl₂Phe L-3,4-Dichlorophenylalanine;
4FPhe L-4-Fluorophenylalanine;
3FPhe L-3-Fluorophenylalanine;
2FPhe L-2-Fluorophenylalanine;
Thi L-β-2-Thienylalanine;
Tza L-2-Thiazolylalanine;
Mso L-Methionine sulfoxide;
AcLys N-Acetyllysine;
Dap 2,3-Diaminopropionic acid;
Dab 2,4-Diaminobutyric acid;
Dbu (2S)-2,3-Diamino-butyric acid;
Abu γ-Aminobutyric acid (GABA);
Aha ε-Aminohexanoic acid;
Aib α-Aminoisobutyric acid;
ACC 1-Amino cyclopropane carboxylic acid;
ACBC 1-Amino cyclobutane carboxylic acid;
ACPC 1-Amino cyclopentane carboxylic acid;
ACHC 1-Amino cyclohexane carboxylic acid;
Y(Bzl) L-O-Benzyltyrosine;
H(Bzl) (3S)-2-Amino-3-(1'-benzylimidazole-4'-yl)-propionic acid;
Bip L-(4-phenyl)phenylalanine;
S(Bzl) L-O-Benzylserine;
T(Bzl) L-O-Benzylthreonine;
alloT (2S, 3S)-2-Amino-3-hydroxy-butyric acid;
Leu30H (2S, 3R)-2-Amino-3-hydroxy-4-methyl-pentanoic acid;
hAla L-Homo-alanine;
hArg L-Homo-arginine;
hCys L-Homo-cysteine;
hGlu L-Homo-glutamic acid;
hGln L-Homo-glutamine;
hHis L-Homo-histidine;
hIle L-Homo-isoleucine;
hLeu L-Homo-leucine;
hNle L-Homo-norleucine;
hLys L-Homo-lysine;
hMet L-Homo-methionine;
hPhe L-Homo-phenylalanine;
hSer L-Homo-serine;
hThr L-Homo-threonine;
hTrp L-Homo-tryptophan;
hTyr L-Homo-tyrosine;
hVal L-Homo-valine;
hCha L-Homo-cyclohexylalanine;
Bpa L-4-Benzoylphenylalanine;
OctG L-Octylglycine;
Tic (3S)-1,2,3,4-Tetrahydroisoquinoline-3-carboxylic acid;
Tiq (1S)-1,2,3,4-Tetrahydroisoquinoline-1-carboxylic acid;
Oic (2S, 3aS, 7aS)-1-Octahydro-1H-indole-2-carboxylic acid;
4AmPyrr1 (2S, 4S)-4-Amino-pyrrolidine-2-carboxylic acid;
4AmPyrr2 (2S, 4R)-4-Amino-pyrrolidine-2-carboxylic acid;
4PhePyrr1 (2S, 4R)-4-Phenyl-pyrrolidine-2-carboxylic acid;
4PhePyrr2 (2S, 4S)-4-Phenyl-pyrrolidine-2-carboxylic acid;
SPhePyrr1 (2S, 5R)-5-Phenyl-pyrrolidine-2-carboxylic acid;
5PhePyrr2 (2S, 5S)-5-Phenyl-pyrrolidine-2-carboxylic acid;
4Hyp1 (4S)-L-Hydroxyproline;
4Hyp2 (4R)-L-Hydroxyproline;
4Mp1 (4S)-L-Mercaptoproline;
4Mp2 (4R)-L-Mercaptoproline;
Pip L-Pipecolic acid;
H-β³-HCit-OH (3S)-3-Amino-6-carbamidyl-hexanoic acid;
H-β³-HOrn-OH (3S)-3,6-Diamino-hexanoic acid;
H-β³-HtBuA-OH (3S)-3-Amino-5,5-dimethyl-hexanoic acid;
H-β³-HSar-OH N-Methyl-3-amino-propionic acid;
H-β³-HPen-OH (3R)-3-Amino-4-methyl-4-mercapto-pentanoic acid;
H-β³-HtBuG-OH (3R)-3-Amino-4,4-dimethyl-pentanoic acid;
H-β³-H4AmPhe-OH (3S)-3-Amino-4-(4'-aminophenyl)-butyric acid;
H-β³-H3AmPhe-OH (3S)-3-Amino-4-(3'-aminophenyl)-butyric acid;
H-β³-H2AmPhe-OH (3S)-3-Amino-4-(2'-aminophenyl)-butyric acid;
H-β³-HPhe(mC(NH₂)=NH) -OH (3S)-3-Amino-4-(3'-amidinophenyl)-butyric acid;
H-β³-HPhe(pC(NH₂)=NH) -OH (3S)-3-Amino-4-(4'-amidinophenyl)-butyric acid;
H-β³-HPhe(mNHC(NH₂)=NH)-OH (3S) -3-Amino-4-(3'-guanidinophenyl)-butyric acid;
H-β³-HPhe(pNHC(NH₂)=NH)-OH (3S) -3-Amino-4-(4'-guanidino-phenyl)-butyric acid;
H-β³-H2Pal-OH (3S)-3-Amino-4-(pyridine-2'-yl)-butyric acid;
H-β³-H4Pal-OH (3S)-3-Amino-4-(pyridine-4'-yl)-butyric acid;
H-β³-HPhg-OH (3R)-3-Amino-3-phenyl-propionic acid;
H-β³-HCha-OH (3S)-3-Amino-4-cyclohexyl-butyric acid;
H-β³-HC₄al-OH (3S)-3-Amino-4-cyclobutyl-butyric acid;
H-β³-HC₅al-OH (3S)-3-Amino-4-cyclopentyl-butyric acid;
H-β³-HNle-OH (3S)-3-Amino-heptanoic acid;
H-³-H2Nal-OH (3S)-3-Amino-4-(2'-naphthyl)-butyric acid;
H-β³-H1Na1-OH (3S)-3-Amino-4-(1'-naphthyl)-butyric acid;
H-β³-H4ClPhe-OH (3S)-3-Amino-4-(4'-chlorophenyl)-butyric acid;
H-β³-H3ClPhe-OH (3S)-3-Amino-4-(3'-chlorophenyl)-butyric acid;
H-β³-H2ClPhe-OH (3S)-3-Amino-4-(2'-chlorophenyl)-butyric acid;
H-β³-H3,4Cl₂Phe-OH (3S)-3-Amino-4-(3',4'-dichlorophenyl)-butyric acid;
H-β³-H4FPhe-OH (3S)-3-Amino-4-(4'-fluorophenyl)-butyric acid;
H-β³-H3FPhe-OH (3S)-3-Amino-4-(3'-fluorophenyl)-butyric acid;
H-³-H2FPhe-OH (3S)-3-Amino-4-(2'-fluorophenyl)-butyric acid;
H-β³-HThi-OH (3R)-3-Amino-4-(2'-thienyl)-butyric acid;
H-β³-HTza-OH (3R)-3-Amino-4-(2'-thiazolyl)-butyric acid;
H-β³-HMso-OH (3R)-3-Amino-4-methylsulfoxyl-butyric acid;
H-β³-HAcLys-OH (3S)-7-Acetylamino-3-amino-heptanoic acid;
H-β³-HDpr-OH (3R)-3,4-diamino-butyric acid;
H-β³-HA₂Bu-OH (3S)-3,5-Diamino-pentanoic acid;
H-β³-HDbu-OH (3R)-3,4-Diamino-pentanoic acid;
H-β³-HAib-OH Amino-dimethyl acetic acid;
H-β³-HCyp-OH 1-Amino-cyclopentane-1-yl-acetic acid;
H-β³-HY(Bzl)-OH (3S)-3-Amino-4-(4'-benzyloxyphenyl)-butyric acid;
H-β³-HH(Bzl)-OH (3S)-3-Amino-4-(1'-benzylimidazole-4'-yl)-butyric acid;
H-β³-HBip-OH (3S)-3-Amino-4-biphenylyl-butyric acid;
H-β³-HS(Bzl)-OH (3S)-3-Amino-4-(benzyloxy)-butyric acid;
H-β³-HT(Bzl)-OH (3R, 4R)-3-Amino-4-benzyloxy-pentanoic acid;
H-β³-HalloT-OH (3R, 4S)-3-Amino-4-hydroxy-pentanoic acid;
H-β³-HLeu30H-OH (3R, 4R)-3-Amino-4-hydroxy-5-methyl-hexanoic acid;
H-β³-HhAla-OH (3S)-3-Amino-pentanoic acid;
H-β³-HhArg-OH (3S)-3-Amino-7-guanidino-heptanoic acid;
H-β³-HhCys-OH (3R)-Amino-5-mercapto-pentanoic acid;
H-β³-HhGlu-OH (3S)-3-Amino-heptanedioic acid;
H-β³-HhGln-OH (3S)-3-Amino-6-carbamoyl hexanoic acid;
H-β³-HhHis-OH (3S)-3-Amino-5-(imidazole-4'-yl)-pentanoic acid;
H-β³-HhIle-OH (3S, 5S)-3-Amino-5-methyl-heptanoic acid;
H-β³-HhLeu-OH (3S)-3-Amino-6-methyl-heptanoic acid;
H-β³-HhNle-OH (3S)-3-Amino-octanoic acid;
H-β³-DiAoc-OH (3S)-3,8-Diamino-octanoic acid;
H-β³-HhMet-OH (3S)-3-Amino-6-methylthio-hexanoic acid;
H-β³-HhPe-OH (3S)-3-Amino-5-phenyl-pentanoic acid;
H-β³-HhSer-OH (3S)-3-Amino-5-hydroxy-pentanoic acid;
H-β³-HhThr-OH (3S, 5R)-3-Amino-5-hydroxy-hexanoic acid;
H-β³-HhTrp-OH (3S)-3-Amino-5-(indol-3'-yl)-pentanoic acid;
H-β³-HhThr-OH (3S)-3-Amino-5-(4'-hydroxyphenyl)-pentanoic acid;
H-β³-HhCha-OH (3S)-3-Amino-5-cyclohexyl-pentanoic acid;
H-β³-HBpa-OH (3S)-3-Amino-4-(4'-benzoylphenyl)-butyric acid;
H-β³-HOctG-OH (3S)-3-Amino-undecanoic acid;
H-β³-HNle-OH (3S)-3-Amino-heptanoic acid;
H-β³-HTic-OH (3S)-1,2,3,4-Tetrahydroisoquinoline-3-yl-acetic acid;
H-β³-HTiq-OH (1S)-1,2,3,4-Tetrahydroisoquinoline-1-acetic acid;
H-β³-HOic-OH (2S, 3aS, 7aS)-1-Octahydro-1H-indole-2-yl-acetic acid;
H-β³-H4AmPyrr1-OH (2S, 4S)-4-Amino-pyrrolidine-2-acetic acid;
H-β³-H4AmPyrr2-OH (2S, 4R)-4-Amino-pyrrolidine-2-acetic acid;
H-β³-H4PhePyrr1-OH (2S, 4R)-4-Phenyl-pyrrolidine-2-acetic acid;
H-β³-H4PhePyrr2-OH (2S, 4S)-4-Phenyl-pyrrolidine-2-acetic acid;
H-β³-H5PhePyrr1-OH (2S, 5R)-5-Phenyl-pyrrolidine-2-acetic acid;
H-β³-H5PhePyrr2-OH (2S, 5S)-5-Phenyl-pyrrolidine-2-acetic acid;
H-β³-H4Hyp1-OH (2S, 4S)-4-Hydroxy-pyrrolidine-2-acetic acid;
H-β³-H4Hyp2-OH (2S, 4R)-4-Hydroxy-pyrrolidine-2-acetic acid;
H-β³-H4Mp1-OH (2R, 4S)-4-Mercapto-pyrrolidine-2-acetic acid;
H-β³-H4Mp2-OH (2R, 4R)-4-Mercapto-pyrrolidine-2-acetic acid;
H-β³-HPip-OH (2S)-Piperidine-2-acetic acid;
H-β³-HPro-OH (2S)-Pyrrolidine-2-acetic acid;
Ahb 4-Amino-2-hydroxy butyric acid;
H-γ⁴-DiHCit-OH (4S)-4-Amino-7-carbamidyl-heptanoic acid;
H-γ⁴-DiHOrn-OH (4S)-4,7-Diamino-heptanoic acid;
H-γ⁴-DiHtBuA-OH (4R)-4-Amino-6,6-dimethyl-heptanoic acid;
H-γ⁴-DiHSar-OH N-Methyl-4-amino-butyric acid;
H-γ⁴-DiHPen-OH (4R)-4-Amino-5-methyl-5-mercapto-hexanoic acid;
H-γ⁴-DiHtBuG-OH (4R)-4-Amino-5,5-dimethyl-hexanoic acid;
H-γ⁴-DiH4AmPhe-OH (4R)-4-Amino-5-(4'-aminophenyl)-pentanoic acid;
H-γ⁴-DiH3AmPhe-OH (4R)-4-Amino-5-(3'-aminophenyl)-pentanoic acid;
H-γ⁴-DiH2AmPhe-OH (4R)-4-Amino-5-(2'-aminophenyl)-pentanoic acid;
H-γ⁴-DiHPhe(mC(NH₂)=NH)-OH (4R)-4-Amino-5-(3'-amidinophenyl)-pentanoic acid;
H-γ⁴-DiHPhe(pC(NH₂)=NH)-OH (4R)-4-Amino-5-(4'-amidinophenyl)-pentanoic acid;
H-γ⁴-DiHPhe (mNHC (NH₂)=NH)-OH (4R)-4-Amino-5-(3'-guanidino-phenyl)-pentanoic acid;
H-γ⁴-DiHPhe (pNHC (NH₂)=NH)-OH (4R)-4-Amino-5-(4'-guanidino-phenyl)-pentanoic acid;
H-γ⁴-DiH2Pa1-OH (4R)-4-Amino-5-(pyridine-4'-yl)-pentanoic acid;
H-γ⁴-DiH4Pa1-OH (4R)-4-Amino-5-(pyridine-4'-yl)-pentanoic acid;
H-γ⁴-DiHPhg-OH (4R)-4-Amino-4-phenyl-butyric acid;
H-γ⁴-DiHCha-OH (4R)-4-Amino-5-cyclohexyl-pentanoic acid;
H-γ⁴-DiHC₄al-OH (4R)-4-Amino-5-cyclobutyl-pentanoic acid;
H-γ⁴-DiHC₅al-OH (4R)-4-Amino-5-cyclopentyl-pentanoic acid;
H-γ⁴-DiHNle-OH (4S)-4-Amino-octanoic acid;
H-γ⁴-DiH2Nal-OH (4S)-4-Amino-5-(2'-naphthyl)-pentanoic acid;
H-γ⁴-DiH1Nal-OH (4S)-4-Amino-5-(1'-naphthyl)-pentanoic acid;
H-γ⁴-DiH4ClPhe-OH (4R)-4-Amino-5-(4'-chlorophenyl)-pentanoic acid;
H-γ⁴-DiH3ClPhe-OH (4R)-4-Amino-5-(3'-chlorophenyl)-pentanoic acid;
H-γ⁴DiH2ClPhe-OH (4R)-4-Amino-5-(2'-chlorophenyl)-pentanoic acid;
H-γ⁴-DiH3,4Cl₂Phe-OH (4R)-4-Amino-5-(3',4'-dichloro-phenyl)-pentanoic acid
H-γ⁴-DiH4FPhe-OH (4R)-4-Amino-5-(4'-fluorophenyl)-pentanoic acid;
H-γ⁴-DiH3FPhe-OH (4R)-4-Amino-5-(3'-fluorophenyl)-pentanoic acid;
H-γ⁴-DiH2FPhe-OH (4R)-4-Amino-5-(2'-fluorophenyl)-pentanoic acid;
H-γ⁴-DiHThi-OH (4R)-4-Amino-5-(2'-thienyl)-pentanoic acid;
H-γ⁴-DiHTza-OH (4R)-4-Amino-5-(2'- thiazolyl)-pentanoic acid;
H-γ⁴-DiHMso-OH (4R)-4-Amino-5-methylsulfoxyl-pentanoic acid;
H-y⁴-DiHAcLys-OH (4S)-8-Acetylamino-4-amino-ocatanoic acid;
H-y⁴-DiHDpr-OH (4R)-4,5-diamino-pentanoic acid;
H-γ⁴-DiHA₂Bu-OH (4R)-4,5-Diamino-hexanoic acid;
H-γ⁴-DiHDbu-OH (4R)-4,5-Diamion-hexanoic acid;
H-γ⁴-DiHAib-OH 3-Amino-3,3-dimethyl propionic acid;
H-γ⁴-DiHCyp-OH (1'-Amino-cyclopentane-1'-yl)-3-propionic acid;
H-γ⁴-DiHY(Bzl)-OH (4R)-4-Amino-5-(4'-benzyloxyphenyl)-pentanoic acid;
H-γ⁴-DiHH(Bzl)-OH (4R)-4-Amino-5-(1'-benzylimidazole-4'-yl)-pentanoic acid;
H-γ⁴-DiHBip-OH (4R)-4-Amino-5-biphenylyl-pentanoic acid;
H-γ⁴-DiHS(Bzl)-OH (4S)-4-Amino-5-(benzyloxy)-pentanoic acid;
H-γ⁴-DiHT(Bzl)-OH (4R, 5R)-4-Amino-5-benzyloxy-hexanoic acid;
H-γ⁴-DiHalloT-OH (4R, 5S)-4-Amino-5-hydroxy-hexanoic acid;
H-γ⁴-DiHLeu30H-OH (4R, 5R)-4-Amino-5-hydroxy-6-methyl-heptanoic acid;
H-γ⁴-DiHhAla-OH (4S)-4-Amino-hexanoic acid;
H-γ⁴-DiHhArg-OH (4S)-4-Amino-8-guanidino-octanoic acid;
H-γ⁴-DiHhCys-OH (4R)-Amino-6-mercapto-hexanoic acid;
H-γ⁴-DiHhGlu-OH (4S)-4-Amino-ocatanedioic acid;
H-γ⁴-DiHhGln-OH (4S)-4-Amino-7-carbamoyl-heptanoic acid;
H-γ⁴-DiHhHis-OH (4S)-4-Amino-6-(imidazole-4'-yl)-hexanoic acid;
H-γ⁴-DiHhIle-OH (4S, 6S)-4-Amino-6-methyl-octanoic acid;
H-γ⁴-DiHhLeu-OH (4S)-4-Amino-7-methyl-ocatanoic acid;
H-γ⁴-DiHhNle-OH (4S)-4-Amino-nonanoic acid;
H-γ⁴-DiHhLys-OH (4S)-4,9-Diamino-nonanoic acid;
H-γ⁴-DiHhMet-OH (4R)-4-Amino-7-methylthioheptanoic acid;
H-γ⁴-DiHhPhe-OH (4S)-4-Amino-6-phenyl-hexanoic acid;
H-γ⁴-DiHhSer-OH (4R)-4-Amino-6-hydroxy-hexanoic acid;
H-γ⁴-DiHhThr-OH (4R, 6R)-4-Amino-6-hydroxy-heptanoic acid;
H-γ⁴-DiHhTrp-OH (4S)-4-Amino-6-(indol-3'-yl)-hexanoi cacid;
H-y⁴-DiHhTyr-OH (4S)-4-Amino-6-(4'-hydroxyphenyl)-hexanoic acid;
H-γ⁴-DiHhCha-OH (4R)-4-Amino-5-cyclohexyl-pentanoic acid;
H-γ⁴-DihBpa-OH (4R)-4-Amino-5-(4'-benzoylphenyl)-pentanoic acid;
H-γ⁴-DiHOctG-OH (4S)-4-Amino-dodecanoic acid;
H-γ⁴-DiHNle-OH (4S)-4-Amino-octanoic acid;
H-γ⁴-DiHTic-OH (3R)-1',2',3',4'-Tetrahydroisoquinoline-3'-yl-3-propionic acid;
H-γ⁴-DiHTiq-OH (1'R)-1',2',3',4'-Tetrahydroisoquinoline-1'-yl-3-propionic acid;
H-γ⁴-DiHOic-OH (2'S, 3'aS, 7'aS)-1'-Octahydro-1H-indole-2'-yl-3-propionic acid;
H-γ⁴-DiH4AmPyrr1-OH (2'R, 4'S)-4'-Amino-pyrrolidine-2'-yl-3-propionic acid;
H-y⁴-DiH4AmPyrr2-OH (2'R, 4'R)-4'-Amino-pyrrolidine-2'-yl-3-propionic acid;
H-γ⁴-DiH4PhePyrr1-OH (2'R, 4'R)-4'-Phenyl-pyrrolidine-2'-yl-3-propionic acid;
H-γ⁴-DiH4PhePyrr2-OH (2'R, 4'S)-4'-Phenyl-pyrrolidine-2'-yl-3-propionic acid
H-γ⁴-DiH5PhePyrr1-OH (2'S, 5'R)-5'-Phenyl-pyrrolidine-2'-yl-3-propionic acid;
H-γ⁴-DiH5PhePyrr2-OH (2'S, 5'S)-5'-Phenyl-pyrrolidine-2'-yl-3-propionic acid
H-γ⁴-DiH4Hyp1-OH (2'R, 4'S)-4'-Hydroxy-pyrrolidine-2'-yl-2-propionic acid;
H-γ⁴-DiH4Hyp2-OH (2'R, 4'R)-4'-Hydroxy-pyrrolidine-2'-yl-3-propionic acid;
H-γ⁴-DiH4Mp1-OH (2'R, 4'S)-4'-Mercapto-pyrrolidine-2'-yl-3-propionic acid;
H-γ⁴-DiH4Mp2-OH (2'R, 4'R)-4'-Mercapto-pyrrolidine-2'-yl-3-propionic acid;
H-γ⁴-DiHPip-OH (2'S)-Piperidine-2'-yl-3-propionic acid;
H-γ⁴-DiHPro-OH (2'S)-Pyrrolidine-2'-yl-3-propionic acid;
(AEt)G N-(2-Aminoethyl)glycine;
(APr)G N-(3-Amino-n-propyl)glycine;
(ABu)G N-(4-Amino-n-butyl)glycine;
(APe)G N-(5-Amino-n-pentyl)glycine;
(GuEt)G N-(2-Guanidinoethyl)glycine;
(GuPr)G N-(3-Guanidino-n-propyl)glycine;
(GuBu)G N-(4-Guanidino-n-butyl)glycine;
(GuPe)G N-(5-Guanidino-n-pentyl)glycine;
(PEG₃-NH₂)G N-[H₂N-(CH₂)3-(OCH₂-CH₂)₂-O(CH₂)₃]glycine;
(Me)G N-Methylglycine;
(Et)G N-Ethylglycine;
(Bu)G N-Butylglycine;
(Pe)G N-Pentylglycine;
(Ip)G N-Isopropylglycine;
(2MePr)G N-(2-Methylpropyl)glycine;
(3MeBu)G N-(3-Methylbutyl)glycine;
(1MePr)G (1S)-N-(1-Methylpropyl)glycine;
(2MeBu)G (2S)-N-(2-Methylbutyl)glycine;
(MthEt)G N-(Methylthioethyl)glycine;
(MthPr)G N-(Methylthiopropyl)glycine;
(Ben)G N-(Benzyl)glycine;
(PhEt)G N-(2-Phenylethyl)glycine;
(HphMe)G N-([4'-hydroxyphenyl]methyl)glycine;
(HphEt)G N-(2-[4'-hydroxyphenyl]ethyl)glycine
(ImMe)G N-(Imidazol-5-yl-methyl)glycine;
(ImEt)G N-(2-(Imidazol-5'-yl)ethyl)glycine;
(InMe)G N-(Indol-2-yl-methyl)glycine;
(InEt)G N-(2-(Indol-2'-yl)ethyl)glycine;
(CboMe)G N-(Carboxymethyl)glycine;
(CboEt)G N-(2-Carboxyethyl)glycine;
(CboPr)G N-(3-Carboxypropyl)glycine;
(CbaMe)G N-(Carbamoylmethyl)glycine;
(CbaEt)G N-(2-Carbamoylethyl)glycine;
(CbaPr)G N-(3-Carbamoylpropyl)glycine;
(HyEt)G N-(2-Hydroxyethyl)glycine;
(HyPr)G (2R)-N-(2-Hydroxypropyl)glycine; or
(Mcet)G N-(2-Mercaptoethyl)glycine.

8. Compounds according to claim 1, selected from:
(2*S*,11*S*,19a*S*)-*N*-[2-(dimethylamino)ethyl]-15-fluoro-7,12-dimethyl-2-{[(1-naphthylamino)carbonyl]amino}-5,8,13-trioxo-2,3,6,7,8,9,10,11,12,13,19,19a-dodecahydro-1*H*,5*H*-pyrrolo[2,1-c][1,4,7,12]benzoxatriazacyclopentadecine-11-carboxamide;
(2*S*,11*S*,19a*S*)-N-(2-aminoethyl)-15-fluoro-7,12-dimethyl-2-{[2-(1-naphthyl)acetyl]amino}-5,8,13-trioxo-2,3,6,7,8,9,10,11,12,13, 19,19a-dodecahydro-1*H*,5*H*-pyrrolo[2,1-c][1,4,7,12]benzoxatriaza-cyclopentadecine-11-carboxamide;
(2*S*,11*S*,9a*S*)-*N*-(2-{[amino(imino)methyl]amino}ethyl)-15-fluoro-7,12-dimethyl-2-{[2-(1-naphthyl)acetyl]amino}-5,8,13-trioxo-2,3,6,7,8,9,10,11,12,13,19,19a-dodecahydro-1*H*,5*H*-pyrrolo[2,1-c][1,4,7,12]benzoxatriazacyclopentadecine-11-carboxamide;
(2*S*,11*S*,19a*S*)-15-fluoro-7,12-dimethyl-2-{[2-(1-naphthyl)-acetyl]amino}-5,8,13-trioxo-*N*-[2-(2-pyridinylamino)ethyl]-2,3,6,7,8,9,10,11,12,13,19,19a-dodecahydro-1*H*,5*H*-pyrrolo[2,1-c][1,4,7,12]benzoxatriazacyclopentadecine-11-carboxamide;
(2*S*,11*S*,19a*S*)-*N*-[2-(dimethylamino)ethyl]-15-fluoro-7,12-dimethyl-2-{[2-(1-naphthyl)ethyl]amino}-5,8,13-trioxo-2,3,6,7,8,9,10,11,12,13,19,19a-dodecahydro-1*H*,5*H*-pyrrolo[2,1-c][1,4,7,12]benzoxatriazacyclopentadecine-11-carboxamide;
(2*S*,11*S*,19a*S*)-*N*-[2-(acetylamino)ethyl]-15-fluoro-7,12-dimethyl-2-[2-(1-naphthyl)acetyl]amino-5,8,13-trioxo-2,3,6,7,8,9,10,11, 12,13,19,19a-dodecahydro-1*H*,5*H*-pyrrolo[2,1-c][1,4,7,12]benzoxa-triazacyclopentadecine-11-carboxamide;
(2*S*,11*S*,19a*S*)-N-[2-(dimethylamino)ethyl]-15-fluoro-7,12-dimethyl-2-{[2-(2-naphthyl)acetyl]amino}-5,8,13-trioxo-2,3,6,7,8,9,10,11,12,13,19,19a-dodecahydro-1*H*,5*H*-pyrrolo[2,1-c][1,4,7,12]benzoxatriazacyclopentadecine-11-carboxamide;
(2*S*,11*S*,19a*S*)-*N*-[2-(dimethylamino)ethyl]-15-fluoro-7,12-dimethyl-5,8,13-trioxo-2-(2-[3-(trifluoromethyl)phenyl]-acetylamino)-2,3,6,7,8,9,10,11,12,13,19,19a-dodecahydro-1H,5H-pyrrolo[2,1-c][1,4,7,12]benzoxatriazacyclopentadecine-11-carboxamide;
(2*S*,11*S*,19a*S*)-*N*-[2-(dimethylamino)ethyl]-15-fluoro-2-[2-(3-methoxyphenyl)acetyl]amino-7,12-dimethyl-5,8,13-trioxo-2,3,6,7,8,9,10,11,12,13,19,19a-dodecahydro-1*H*,5*H*-pyrrolo[2,1-c][1,4,7,12]benzoxatriazacyclopentadecine-11-carboxamide;
(2*S*,11*S*,19a*S*)-*N*-[3-(dimethylamino)propyl]-15-fluoro-7,12-dimethyl-2-[2-(1-naphthyl)acetyl]amino-5,8,13-trioxo-2,3,6,7,8, 9,10,11,12,13,19,19a-dodecahydro-1*H*,5*H*-pyrrolo[2,1-c]-[1,4,7,12]benzoxatriazacyclopentadecine-11-carboxamide;
(2*S*,11*S*,19a*S*)-15-fluoro-7,12-dimethyl-2-[2-(1-naphthyl)-acetyl]amino-5,8,13-trioxo-N-(3-pyridinylmethyl)-2,3,6,7,8,9,10, 11,12,13,19,19a-dodecahydro-1*H*,5*H*-pyrrolo[2,1-c][1,4,7,12]-benzoxatriazacyclopentadecine-11-carboxamide;
N-[(4*S*,6*S*,10*S*)-14-methyl-6-[2-(2-naphthyl)acetyl]amino-9,15-dioxo-2-oxa-8,14-diazatricyclo[14.3.1.0^{4,8}]icosa-1(20),16,18-trien-10-yl]decanamide;
N- [(4*S*,6*S*,10*S*)-14-methyl-10-{[2-(2-naphthyl)acetyl]amino}-9,15-dioxo-2-oxa-8,14-diazatricyclo[14.3.1.0^{4,8}]icosa-1(20),16,18-trien-6-yl]-2-(2-naphthyl)acetamide;
N-[(4*S*,6*S*,10*S*)-14-methyl-6-[2-(2-naphthyl)acetyl]amino-9,15-dioxo-2-oxa-8,14-diazatricyclo[14.3.1.0^{4,8}]icosa-1(20),16,18-trien-10-yl]-2-(1-naphthyl)acetamide;
*N*-[(4*S*,6*S*,10*S*)-14-methyl-9,15-dioxo-10-[(3-toluidinocarbonyl)-amino]-2-oxa-8,14-diazatricyclo[14.3.1.0^{4,1}]icosa-1(20),16,18-trien-6-yl]-2-(2-naphthyl)acetamide;
*N*-[(4*S*,6*S*,10*S*)-10-([3-(dimethylamino)anilino]carbonylamino)-14-methyl-9,15-dioxo-2-oxa-8,14-diazatricyclo[14.3.1.0^{4,8}]icosa-1(20),16,18-trien-6-yl]-2-(2-naphthyl)acetamide;
*N*-[(4*S*,6*S*,10*S*)-6-({[3-(dimethylamino)anilino]carbonyl}amino)-14-methyl-9,15-dioxo-2-oxa-8,14-diazatricyclo[14.3.1.0^{4,8}]icosa-1(20),16,18-trien-10-yl]-2-(2-naphthyl)acetamide;
*N*-[(4*S*,6*S*,10*S*)-14-methyl-9,15-dioxo-6-[(3-phenylpropanoyl)-amino]-2-oxa-8,14-diazatricyclo[14.3.1.0^{4,8}]icosa-1(20),16,18-trien-10-yl]decanamide;
*N*-[(4*S*,6*S*,10*S*)-14-methyl-6-[(3-morpholinobenzoyl)amino]-9,15-dioxo-2-oxa-8,14-diazatricyclo[14.3.1.0^{4,8}]icosa-1(20),16,18-trien-10-yl]decanamide;
*N*-[(4S,6S,10S)-6-({[3-(dimethylamino)anilino]carbonyl}amino)-14-methyl-9,15-dioxo-2-oxa-8,14-diazatricyclo[14.3.1.0^{4,8}]icosa-1(20),16,18-trien-10-yl]decanamide;
*N*-[(4S,6S,10S)-6-({[3-(dimethylamino)anilino]carbonyl}amino)-14-methyl-9,15-dioxo-2-oxa-8,14-diazatricyclo[14.3.1.0^{4,8}]icosa-1(20),16,18-trien-10-yl]-2-(1-naphthyl)acetamide;
*N*-[(4*S*,6*S*,10*S*)-6-{[(3-methoxyanilino)carbonyl]amino}-14-methyl-9,15-dioxo-2-oxa-8,14-diazatricyclo[14.3.1.0^{4,8}]icosa-1(20),16,18-trien-10-yl]-2-(2-naphthyl)acetamide;
*N*-[(4*S*,6*S*,10*S*)-14-methyl-9,15-dioxo-6-{[2-(3-phenoxyphenyl)-acetyl]amino}-2-oxa-8,14-diazatricyclo[14.3.1.0^{4,8}]icosa-1(20),16,18-trien-10-yl]decanamide;
*N*-[(4*S*,6*S*,10*S*)-14-methyl-6-{[2-(2-naphthyl)acetyl]amino}-9,15-dioxo-2-oxa-8,14-diazatricyclo[14.3.1.0^{4,8}]icosa-1(20),16,18-trien-10-yl]-6-phenylhexanamide;
*N*-[(4*S*,6*S*,10*S*)-14-methyl-6-{[2-(2-naphthyl)acetyl]amino}-9,15-dioxo-2-oxa-8,14-diazatricyclo[14.3.1.0^{4,8}]icosa-1(20),16,18-trien-10-yl]-5-phenoxypentanamide;
(*E*)-*N*-[(4*S*,6*S*,10*S*)-14-methyl-6-[2-(2-naphthyl)acetyl]amino-9,15-dioxo-2-oxa-8,14-diazatricyclo[14.3.1.0^{4,8}]icosa-1(20),16,18-trien-10-yl]-3-decenamide;
*N*-[(4*S*,6*S*,10*S*)-14-methyl-6-{[2-(1-naphthyl)acetyl]amino}-9,15-dioxo-2-oxa-8,14-diazatricyclo[14.3.1.0^{4,8}]icosa-1(20),16,18-trien-10-yl]decanamide;
6-(benzyloxy)-*N*-[(4*S*,6*S*,10*S*)-14-methyl-6-[2-(2-naphthyl)-acetyl]amino-9,15-dioxo-2-oxa-8,14-diazatricyclo[14.3.1.0^{4,8}]-icosa-1(20),16,18-trien-10-yl]hexanamide;
2-[1,1'-biphenyl]-4-yl-N-[(4*S*,6*S*,10*S*)-14-methyl-6-{[2-(2-naphthyl)acetyl]amino}-9,15-dioxo-2-oxa-8,14-diazatricyclo-[14.3.1.0^{4,8}]icosa-1(20),16,18-trien-10-yl]acetamide;
*N*-[(4*S*,6*S*,10*S*)-14-methyl-6-{[2-(2-naphthyl)acetyl]amino}-9,15-dioxo-2-oxa-8,14-diazatricyclo[14.3.1.0^{4,8}]icosa-1(20),16,18-trien-10-yl]-3,3-diphenylpropanamide;
6-(benzyloxy)-*N*-[(4*S*,6*S*,10*S*)-14-methyl-9,15-dioxo-10-[(6-phenylhexanoyl)amino]-2-oxa-8,14-diazatricyclo[14.3.1.0^{4,8}]-icosa-1(20),16,18-trien-6-yl]hexanamide;
2-[1,1'-biphenyl]-3-yl-*N*-[(4*S*,6*S*,10*S*)-6-([3-(dimethylamino)-anilino]carbonylamino)-14-methyl-9,15-dioxo-2-oxa-8,14-diazatricyclo[14.3.1.0^{4,8}]icosa-1(20),16,18-trien-10-yl]-acetamide;
2-[1,1'-biphenyl]-4-yl-*N*-[(4*S*,6*S*,10*S*)-6-([3-(dimethylamino)-anilino]carbonylamino)-14-methyl-9,15-dioxo-2-oxa-8,14-diazatricyclo[14.3.1.0^{4,8}]icosa-1(20),16,18-trien-10-yl]-acetamide;
*N*-[(4*S*,6*S*,10*S*)-10-[([1,1'-biphenyl]-4-ylsulfonyl)amino]-14-methyl-9,15-dioxo-2-oxa-8,14-diazatricyclo[14.3.1.0^{4,8}]icosa-1(20),16,18-trien-6-yl]-2-(2-naphthyl)acetamide;
*N*-[(4*S*,6*S*,10*S*)-6-{[(3-fluoroanilino)carbonyl]amino}-14-methyl-9,15-dioxo-2-oxa-8,14-diazatricyclo[14.3.1.0^{4,8}]icosa-1(20), 16,18-trien-10-yl]-2-(2-naphthyl)acetamide;
*N*-[(4S,6S,10S)-6-({[4-(dimethylamino)anilino]carbonyl}amino)-14-methyl-9,15-dioxo-2-oxa-8,14-diazatricyclo[14.3.1.0^{4,8}]icosa-1(20),16,18-trien-10-yl]-2-(2-naphthyl)acetamide;
phenyl *N*-[(4*S*,6*S*,10*S*)-14-methyl-10-{[2-(2-naphthyl)acetyl]-amino}-9,15-dioxo-2-oxa-8,14-diazatricyclo[14.3.1.0^{4,8}]icosa-1(20),16,18-trien-6-yl]carbamate;
*N*-[(4*S*,6*S*,10*S*)-14-methyl-9,15-dioxo-6-[2-(7-quinolinyl)acetyl]-amino-2-oxa-8,14-diazatricyclo[14.3.1.0^{4,8}]icosa-1(20),16,18-trien-10-yl]-2-(2-naphthyl)acetamide;
*N*-[(4*S*,6*S*,10*S*)-14-methyl-9,15-dioxo-6-{[2-(3-phenoxyphenyl)-acetyl]amino}-2-oxa-8,14-diazatricyclo[14.3.1.0^{4,8}]icosa-1(20),16,18-trien-10-yl]-2-(2-naphthyl)acetamide;
N- [(4*S*,6*S*,10*S*)-14-methyl-10-{[2-(2-naphthyl)acetyl]amino}-9,15-dioxo-2-oxa-8,14-diazatricyclo[14.3.1.0^{4,8}]icosa-1(20),16,18-trien-6-yl]decanamide;
*N*-[(4*S*,6*S*,10*S*)-14-methyl-10-{[2-(1-naphthyl)acetyl]amino}-9,15-dioxo-2-oxa-8,14-diazatricyclo[14.3.1.0^{4,8}]icosa-1(20),16,18-trien-6-yl]decanamide;
*N*-[(4*S*,6*S*,10*S*)-14-methyl-10-{[2-(1-naphthyl)acetyl]amino}-9,15-dioxo-2-oxa-8,14-diazatricyclo[14.3.1.0^{4,8}]icosa-1(20),16,18-trien-6-yl]hexanamide;
2-[1,1'-biphenyl]-4-yl-N-[(4*S*,6*S*,10*S*)-14-methyl-10-{[2-(1-naphthyl)acetyl]amino}-9,15-dioxo-2-oxa-8,14-diazatricyclo-[14.3.1.0^{4,8}]icosa-(20),16,18-trien-6-yl]acetamide;
*N*-[(4*S*,6*S*,10*S*)-10-[(2-[1,1'-biphenyl]-4-ylacetyl)amino]-14-methyl-9,15-dioxo-2-oxa-8,14-diazatricyclo[14.3.1.0^{4,8}]icosa-1(20),16,18-trien-6-yl]-2,2-diphenylacetamide;
*N*-[(4*S*,6*S*,10*S*)-6-[(2-[1,1'-biphenyl]-4-ylacetyl)amino]-14-methyl-9,15-dioxo-2-oxa-8,14-diazatricyclo[14.3.1.0^{4,8}]icosa-1(20),16,18-trien-10-yl]-2,2-diphenylacetamide;
*N*-[(4*S*,6*S*,10*S*)-6-[(2,2-diphenylacetyl)amino]-14-methyl-9,15-dioxo-2-oxa-8,14-diazatricyclo[14.3.1.0^{4,8}]icosa-1(20),16,18-trien-10-yl]decanamide;
*N*-[(4*S*,6*S*,10*S*)-14-methyl-10-{[2-(1-naphthyl)acetyl]amino}-9,15-dioxo-2-oxa-8,14-diazatricyclo[14.3.1.0^{4,8}]icosa-1(20),16,18-trien-6-yl]-2,2-diphenylacetamide;
*N*-[(4*S*,6*S*,10*S*)-6-[(3,3-diphenylpropanoyl)amino]-14-methyl-9,15-dioxo-2-oxa-8,14-diazatricyclo[14.3.1.0^{4,8}]icosa-1(20),16,18-trien-10-yl]decanamide;
2-[1,1'-biphenyl]-4-yl-*N*-[(4*S*,6*S*,10*S*)-10-[(2-[1,1'-biphenyl]-4-ylacetyl)amino]-14-methyl-9,15-dioxo-2-oxa-8,14-diazatricyclo-[14.3.1.0^{4,8}]icosa-1(20),16,18-trien-6-yl]acetamide;
*N*-[(4*S*,6*S*,10*S*)-6-({[3-(dimethylamino)anilino]carbonyl}amino)-14-methyl-9,15-dioxo-2-oxa-8,14-diazatricyclo[14.3.1.0^{4,8}]icosa-1(20),16,18-trien-10-yl]-3,3-dimethylbutanamide;
*N*-[(4*S*,6*S*,10*S*)-6-({[3-(dimethylamino)anilino]carbonyl}amino)-9,15-dioxo-2-oxa-8,14-diazatricyclo[14.3.1.0^{4,8}]icosa-1(20), 16,18-trien-10-yl]-2-(2-naphthyl)acetamide;
benzyl *N*-[(4*S*,6*S*,10*S*)-6-{[2-(2-naphthyl)acetyl]amino}-9,15-dioxo-2-oxa-8,14-diazatricyclo[14.3.1.0^{4,8}]icosa-1(20),16,18-trien-10-yl]carbamate;
*N*-[(4*S*,6*S*,10*S*)-6-{[2-(2-naphthyl)acetyl]amino}-9,15-dioxo-2-oxa-8,14-diazatricyclo[14.3.1.0^{4,8}]icosa-1(20),16,18-trien-10-yl]decanamide;
(4*S*,6*R*,15*S*)-11,16-dimethyl-*N*-(2-naphthylmethyl)-9,12,17-trioxo-6-{[3-(4-pyridinyl)propanoyl]amino}-2-thia-8,11,16-triaza-tricyclo[16.3.1.0^{4,8}]docosa-1(22),18,20-triene-15-carboxamide;
tert-butyl N-[(4*S*,6*R*,15*S*)-11,16-dimethyl-15-{[(2-naphthylmethyl)amino]carbonyl}-9,12,17-trioxo-2-thia-8,11,16-triaza-tricyclo[16.3.1.0^{4,8}]docosa-1(22),18,20-trien-6-yl]carbamate;
(4*S*,6*R*,15*S*)-11,16-dimethyl-*N*-(2-naphthylmethyl)-9,12,17-trioxo-6-(pentanoylamino)-2-thia-8,11,16-triazatricyclo[16.3.1.0^{4,8}]-docosa-1(22),18,20-triene-15-carboxamide;
(4*S*,6*R*,15*S*)-6-(acetylamino)-11,16-dimethyl-*N*-(2-naphthylmethyl)-9,12,17-trioxo-2-thia-8,11,16-triazatricyclo-[16.3.1.0^{4,8}]docosa-1(22),18,20-triene-15-carboxamide;
(2*S*,11*S*,19a*S*)-15-fluoro-7,12-dimethyl-2-(1-naphthoylamino)-*N-*(2-naphthylmethyl)-5,8,13-trioxo-2,3,6,7,8,9,10,11,12,13, 19,19a-dodecahydro-1*H*,5*H*-pyrrolo[2,1-c][1,4,7,12]benzoxatriaza-cyclopentadecine-11-carboxamide;
(2*S*,11*S*,19a*S*)-15-fluoro-7,12-dimethyl-2-[2-(2-naphthyl)acetyl]-amino-*N*-(1-naphthylmethyl)-5,8,13-trioxo-2,3,6,7,8,9,10,11,12, 13,19,19a-dodecahydro-1*H*,5*H*-pyrrolo[2,1-c][1,4,7,12]benzoxa-triazacyclopentadecine-11-carboxamide;
(2*S*,11*S*,19a*S*)-2-[(3-chlorobenzoyl)amino]-15-fluoro-7,12-dimethyl-*N*-(1-naphthylmethyl)-5,8,13-trioxo-2,3,6,7,8,9,10,11, 12,13,19,19a-dodecahydro-1*H*,5*H*-pyrrolo[2,1-*c*][1,4,7,12]benzoxa-triazacyclopentadecine-11-carboxamide.

9. Compounds according to any one of claims 1-8 for use as therapeutically active substances.

10. Compounds according to claim 9 for use as therapeutically active substances having agonistic or antagonistic activity on the motilin receptor (MR receptor), on the serotonin receptor of subtype 5-HT_{2B} (5-HT_{2B} receptor), on the prostaglandin F2α receptor (FP receptor), on the purinoreceptor P2Y₁, on the voltage-gated potassium channel Kᵥ1.3; or on the canonical (β-catenin-dependent) Wnt pathway.

11. A pharmaceutical composition containing a compound or a mixture of compounds according to any one of claims 1-8 and at least one therapeutically inert excipient.

12. A composition according to claim 11 containing a compound or a mixture of compounds according to any one of claims 1-8, having agonistic or antagonistic activity on the motilin receptor (MR receptor), on the serotonin receptor of subtype 5-HT_{2B} (5-HT_{2B} receptor), on the prostaglandin F2α receptor (FP receptor), on the purinoreceptor P2Y₁, on the voltage-gated potassium channel Kᵥ1.3; or on the canonical (β-catenin-dependent) Wnt pathway.

13. A composition according to claim 11 or 12 suitable for oral, topical, transdermal, injection, buccal, transmucosal, pulmonary or inhalation administration, particularly in form of tablets, dragees, capsules, solutions, liquids, gels, plaster, creams, ointments, syrup, slurries, suspensions, spray, nebulizer or suppositories.

14. The use of compounds according to any one of claims 1-8, having agonistic or antagonistic activity on the motilin receptor (MR receptor), on the serotonin receptor of subtype 5-HT_{2B} (5-HT_{2B} receptor), on the prostaglandin F2α receptor (FP receptor), on the purinoreceptor P2Y₁, on the voltage-gated potassium channel Kᵥ1.3; or on the canonical (β-catenin-dependent) Wnt pathway, for the manufacture of a medicament.

15. The use of compounds according to any one of claims 1-8 for the manufacture of a medicament, wherein said medicament is intended for the treatment of **motility disorders of the gastrointestinal tract; CNS** diseases; **ocular hypertension CNS inflammatory disorders; thromboembolic disorders; cell proliferation disorders; or bone and cartilage-related disorders.**

16. The use of compounds according to claims 1-8 for the manufacture of a medicament, wherein said medicament is intended for the treatment of disorders according to claim 15, wherein these disorders are selected from functional dyspepsia, diabetic gastroparesis or constipation type irritable bowl syndrome, migraine, schizophrenia, psychosis, depression or Alzheimer disease, glaucoma, preterm labour or multiple sclerosis.

17. The use of compounds according to anyone of claims 1-9 for pharmaceutical lead finding.

## Patentansprüche

1. Verbindungen, bestehend aus einer cyclischen Anordnung der Basiskomponenten **A, B** und **C** und dargestellt durch die allgemeinen Formeln **Ia** oder **Ib** 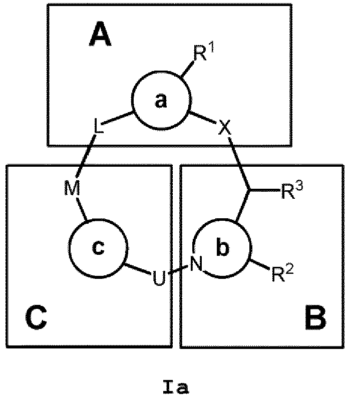 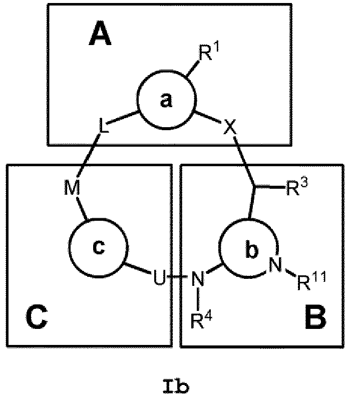 wobei
**Basiskomponente A** ("Templat") dargestellt wird durch 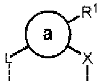
**Basiskomponente B** ("Modulator") dargestellt wird durch 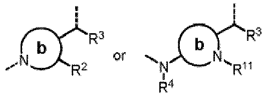
**Basiskomponente C** brücke") dargestellt wird durch 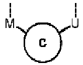 beziehungsweise 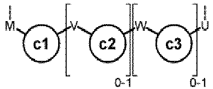 und wobei
M für ein zweiwertiges oder dreiwertiges Radikal steht, ausgewählt aus der Gruppe aus 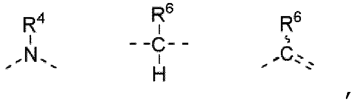 die einen integralen Bestandteil der
M-L-Konnektivität bilden, die wiederum für ein zweiwertiges Radikal steht, ausgewählt aus der Gruppe aus 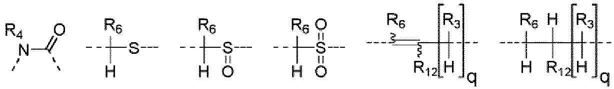
U für ein zweiwertiges Radikal steht, ausgewählt aus der Gruppe aus 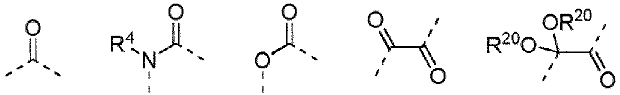
X für ein zweiwertiges Radikal steht, ausgewählt aus der Gruppe aus 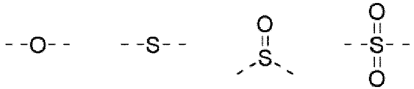
V und W unabhängig voneinander für ein zweiwertiges Radikal stehen, ausgewählt aus der Gruppe aus 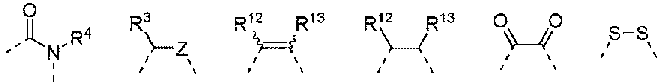
und wobei
die **Basiskomponente A** ein zweiwertiges Radikal ist, ausgewählt aus der Gruppe aus
| | | | |
|---|---|---|---|
| 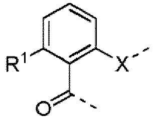 | 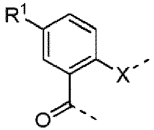 | 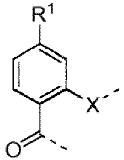 | 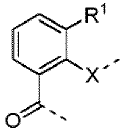 |
| **A1** (a1) | **A2** (a1) | **A3** (a1) | **A4** (a1) |
| 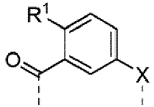 | 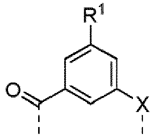 | 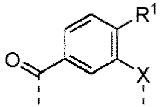 | 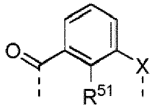 |
| **A5** (a1) | **A6** (a1) | **A7** (a1) | **A8** (a1) |
| 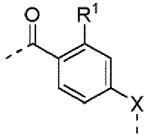 | 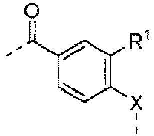 | 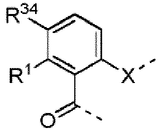 | 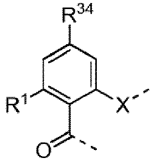 |
| **A9** (a1) | **A10** (a1) | **A11** (a1) | **A12** (a1) |
| 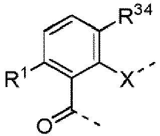 | 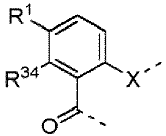 | 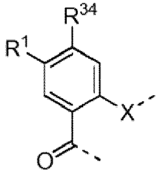 | 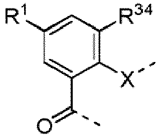 |
| **A13** (a1) | **A14** (a1) | **A15** (a1) | **A16** (a1) |
| 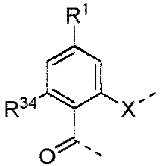 | 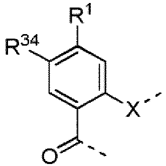 | 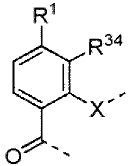 | 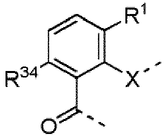 |
| **A17** (a1) | **A18** (a1) | **A19** (a1) | **A20** (a1) |
| 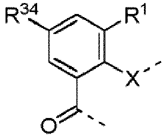 | 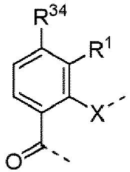 | 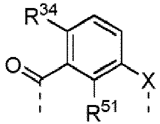 | 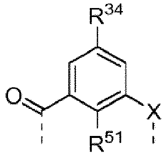 |
| **A21** (a1) | **A22** (a1) | **A23** (a1) | **A24** (a1) |
| 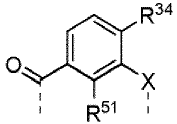 | 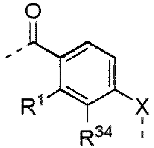 | 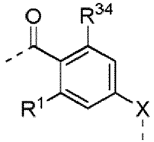 | 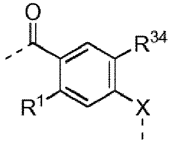 |
| **A25** (a1) | **A26** (a1) | **A27** (a1) | **A28** (a1) |
| 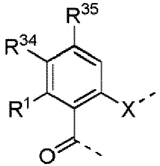 | 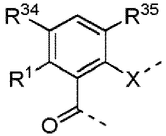 | 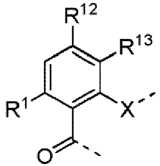 | 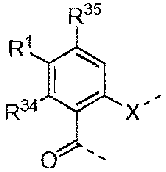 |
| **A29** (a1) | **A30** (a1) | **A31** (a1) | **A32** (a1) |
| 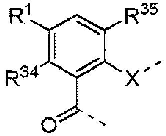 | 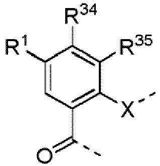 | 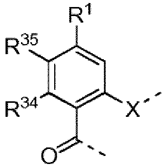 | 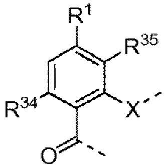 |
| **A33** (a1) | **A34** (a1) | **A35** (a1) | **A36** (a1) |
| 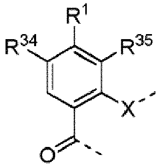 | 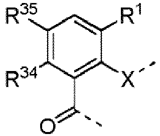 | 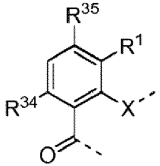 | 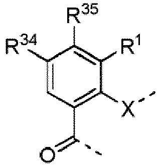 |
| **A37** (a1) | **A38** (a1) | **A39** (a1) | **A40** (a1) |
| 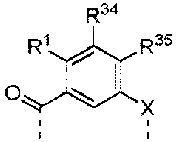 | 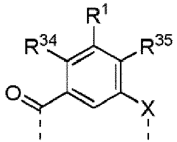 | 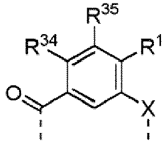 | 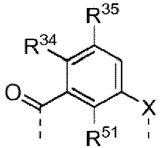 |
| **A41** (a1) | **A42** (a1) | **A43** (a1) | **A44** (a1) |
| 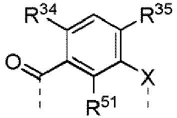 | 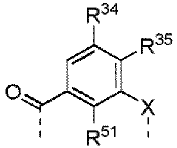 | 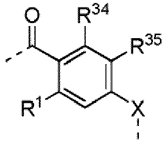 | 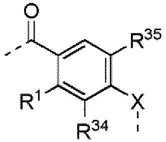 |
| **A45** (a1) | **A46** (a1) | **A47** (a1) | **A48** (a1) |
| 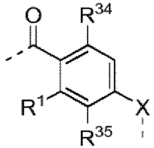 | 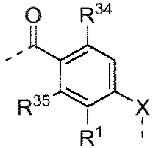 | 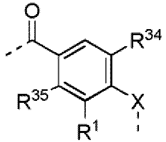 | 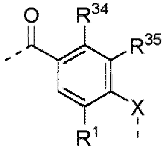 |
| **A49** (a1) | **A50** (a1) | **A51** (a1) | **A52** (a1) |
| 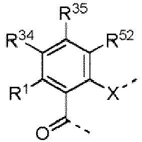 | 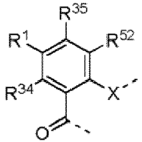 | 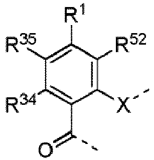 | 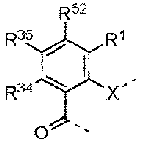 |
| **A53** (a1) | **A54** (a1) | **A55** (a1) | **A56** (a1) |
| 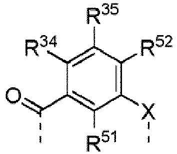 | 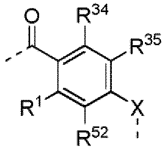 | 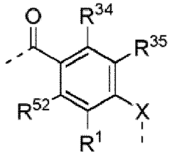 | 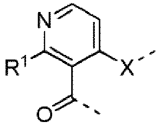 |
| **A57** (a1) | **A58** (a1) | **A59** (a1) | **A60** (a2) |
| 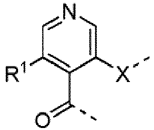 | 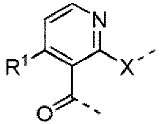 | 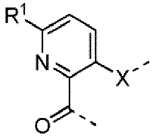 | 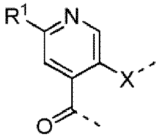 |
| **A61** (a2) | **A62** (a2) | **A63** (a2) | **A64** (a2) |
| 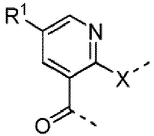 | 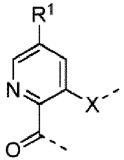 | 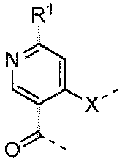 | 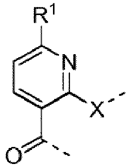 |
| **A65** (a2) | **A66** (a2) | **A67** (a2) | **A68** (a2) |
| 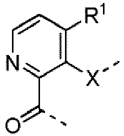 | 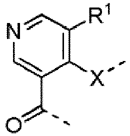 | 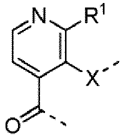 | 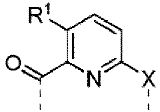 |
| **A69** (a2) | **A70** (a2) | **A71** (a2) | **A72** (a2) |
| 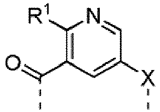 | 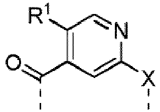 | 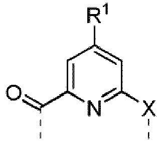 | 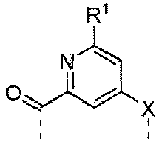 |
| **A73** (a2) | **A74** (a2) | **A75** (a2) | **A76** (a2) |
| 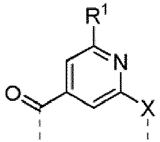 | 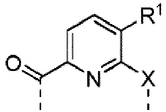 | 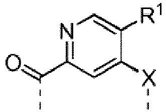 | 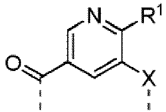 |
| **A77** (a2) | **A78** (a2) | **A79** (a2) | **A80** (a2) |
| 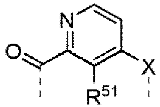 | 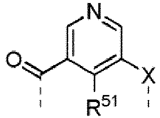 | 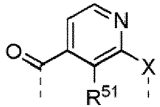 | 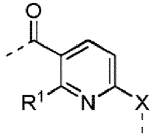 |
| **A81** (a2) | **A82** (a2) | **A83** (a2) | **A84** (a2) |
| 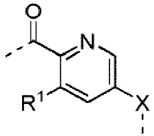 | 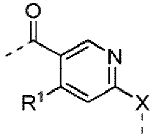 | 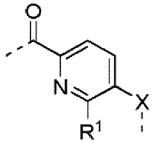 | 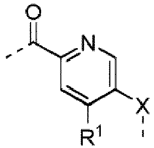 |
| **A85** (a2) | **A86** (a2) | **A87** (a2) | **A88** (a2) |
| 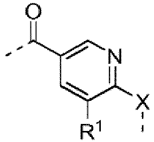 | 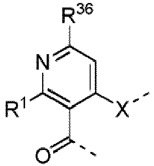 | 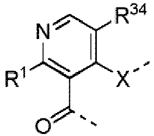 | 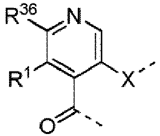 |
| **A89** (a2) | **A90** (a2) | **A91** (a2) | **A92** (a2) |
| 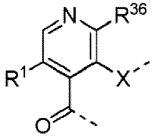 | 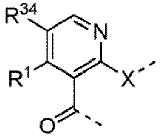 | 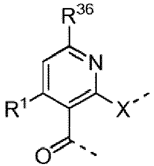 | 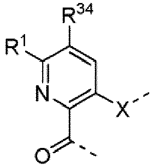 |
| **A93** (a2) | **A94** (a2) | **A95** (a2) | **A96** (a2) |
| 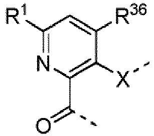 | 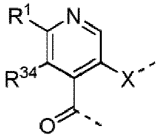 | 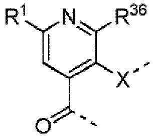 | 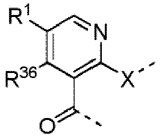 |
| **A97** (a2) | **A98** (a2) | **A99** (a2) | **A100** (a2) |
| 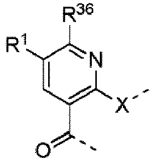 | 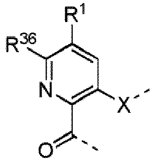 | 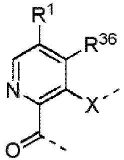 | 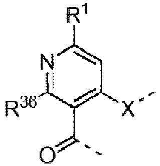 |
| **A101** (a2) | **A102** (a2) | **A103** (a2) | **A104** (a2) |
| 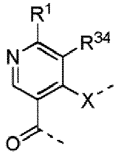 | 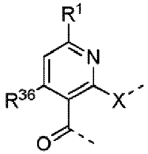 | 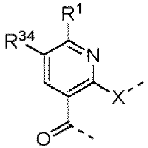 | 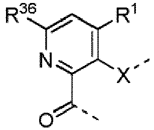 |
| **A105** (a2) | **A106** (a2) | **A107** (a2) | **A108** (a2) |
| 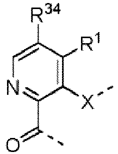 | 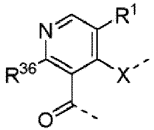 | 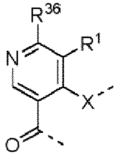 | 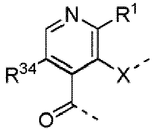 |
| **A109** (a2) | **A110** (a2) | **A111** (a2) | **A112** (a2) |
| 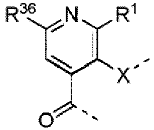 | 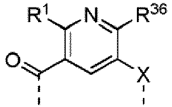 | 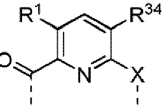 | 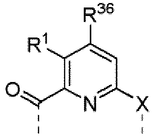 |
| **A113** (a2) | **A114** (a2) | **A115** (a2) | **A116** (a2) |
| 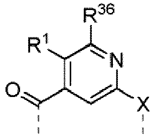 | 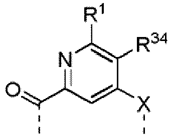 | 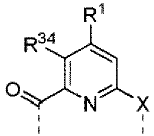 | 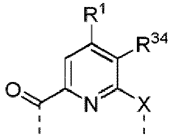 |
| **A117** (a2) | **A118** (a2) | **A119** (a2) | **A120** (a2) |
| 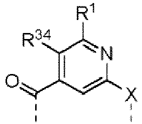 | 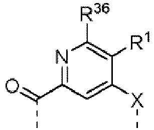 | 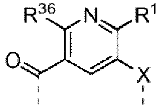 | 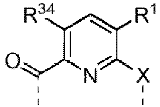 |
| **A121** (a2) | **A122** (a2) | **A123** (a2) | **A124** (a2) |
| 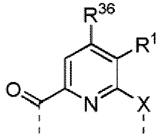 | 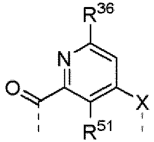 | 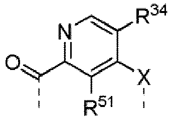 | 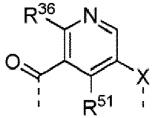 |
| **A125** (a2) | **A126** (a2) | **A127** (a2) | **A128** (a2) |
| 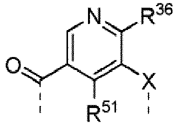 | 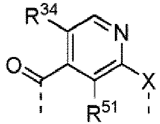 | 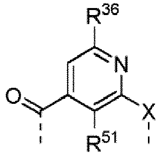 | 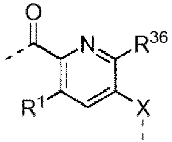 |
| **A129** (a2) | **A130** (a2) | **A131** (a2) | **A132** (a2) |
| 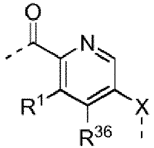 | 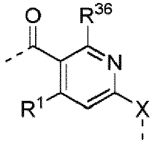 | 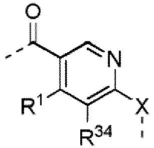 | 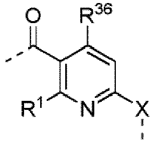 |
| **A133** (a2) | **A134** (a2) | **A135** (a2) | **A136** (a2) |
| 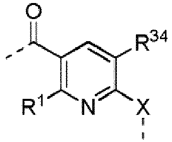 | 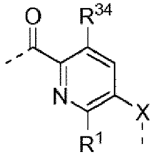 | 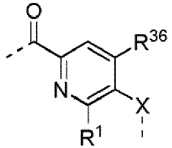 | 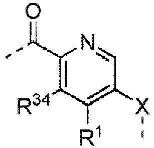 |
| **A137** (a2) | **A138** (a2) | **A139** (a2) | **A140** (a2) |
| 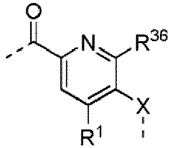 | 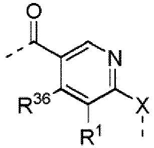 | 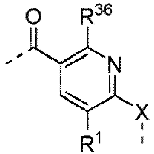 | 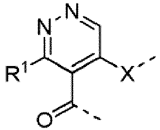 |
| **A141** (a2) | **A142** (a2) | **A143** (a2) | **A144** (a3) |
| 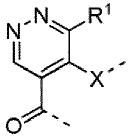 | 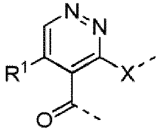 | 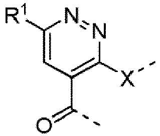 | 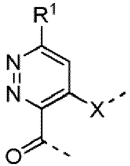 |
| **A145** (a3) | **A146** (a3) | **A147** (a3) | **A148** (a3) |
| 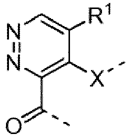 | 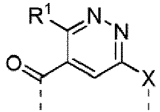 | 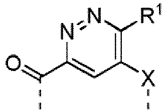 | 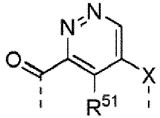 |
| **A149** (a3) | **A150** (a3) | **A151** (a3) | **A152** (a3) |
| 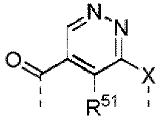 | 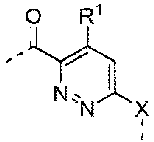 | 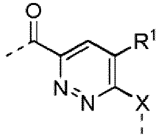 | 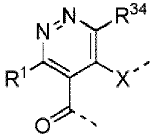 |
| **A153** (a3) | **A154** (a3) | **A155** (a3) | **A156** (a3) |
| 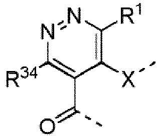 | 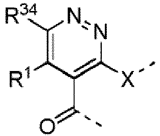 | 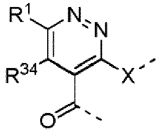 | 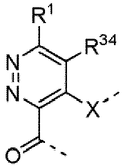 |
| **A157** (a3) | **A158** (a3) | **A159** (a3) | **A160** (a3) |
| 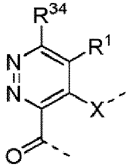 | 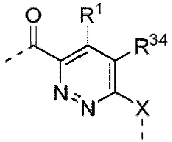 | 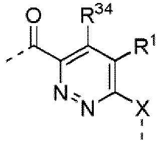 | 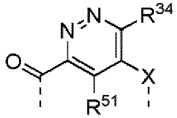 |
| **A161** (a3) | **A162** (a3) | **A163** (a3) | **A164** (a3) |
| 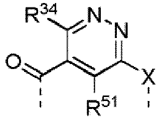 | 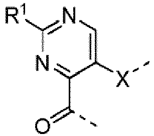 | 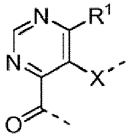 | 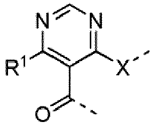 |
| **A165** (a3) | **A166** (a4) | **A167** (a4) | **A168** (a4) |
| 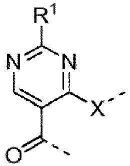 | 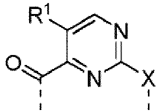 | 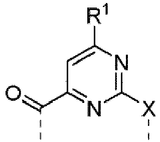 | 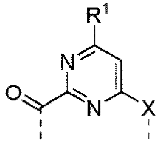 |
| **A169** (a4) | **A170** (a4) | **A171** (a4) | **A172** (a4) |
| 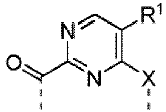 | 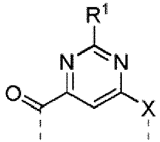 | 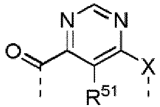 | 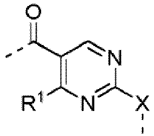 |
| **A173** (a4) | **A174** (a4) | **A175** (a4) | **A176** (a4) |
| 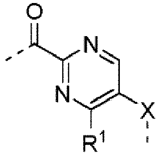 | 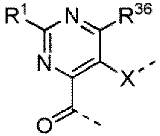 | 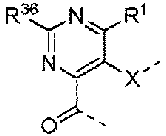 | 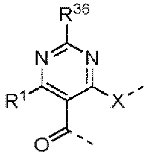 |
| **A177** (a4) | **A178** (a4) | **A179** (a4) | **A180** (a4) |
| 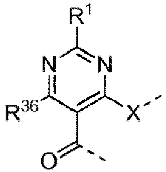 | 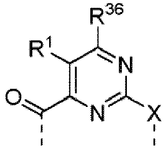 | 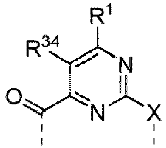 | 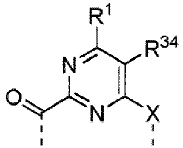 |
| **A181** (a4) | **A182** (a4) | **A183** (a4) | **A184** (a4) |
| 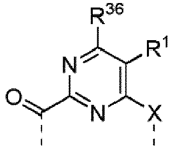 | 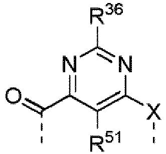 | 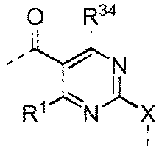 | 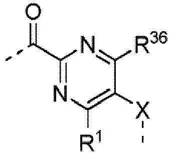 |
| **A185** (a4) | **A186** (a4) | **A187** (a4) | **A188** (a4) |
| 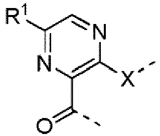 | 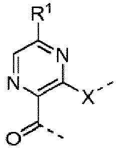 | 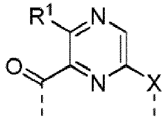 | 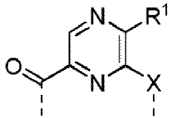 |
| **A189** (a5) | **A190** (a5) | **A191** (a5) | **A192** (a5) |
| 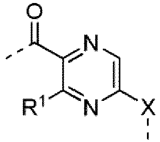 | 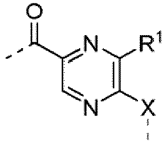 | 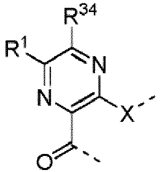 | 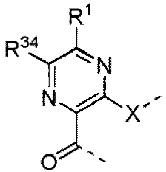 |
| **A193** (a5) | **A194** (a5) | **A195** (a5) | **A196** (a5) |
| 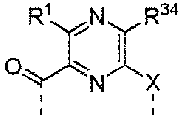 | 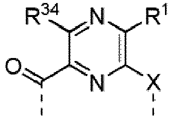 | 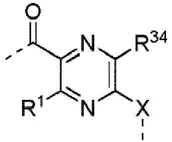 | 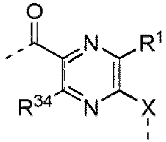 |
| **A197** (a5) | **A198** (a5) | **A199** (a5) | **A200** (a5) |
| 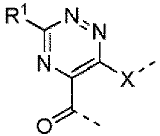 | 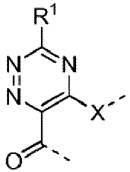 | 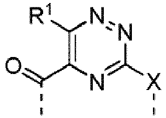 | 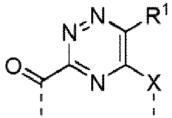 |
| **A201** (a6) | **A202** (a6) | **A203** (a6) | **A204** (a6) |
| 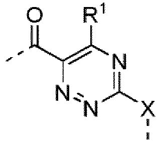 | 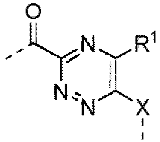 | 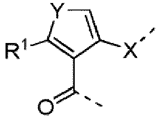 | 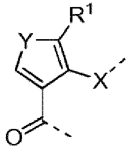 |
| **A205** (a6) | **A206** (a6) | **A207** (a7) | **A208** (a7) |
| 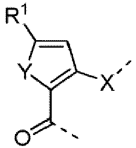 | 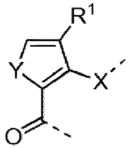 | 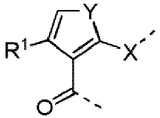 | 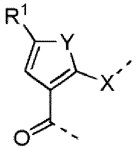 |
| **A209** (a7) | **A210** (a7) | **A211** (a7) | **A212** (a7) |
| 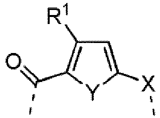 | 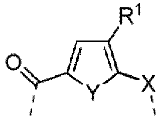 | 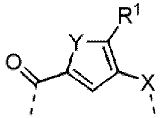 | 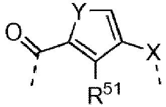 |
| **A213** (a7) | **A214** (a7) | **A215** (a7) | **A216** (a7) |
| 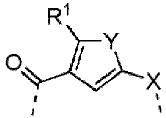 | 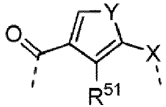 | 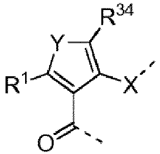 | 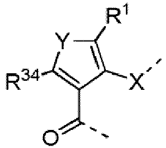 |
| **A217** (a7) | **A218** (a7) | **A219** (a7) | **A220** (a7) |
| 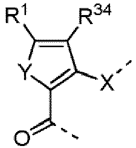 | 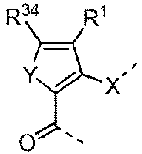 | 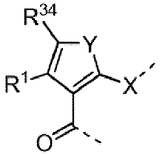 | 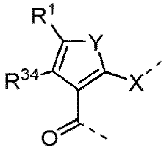 |
| **A221** (a7) | **A222** (a7) | **A223** (a7) | **A224** (a7) |
| 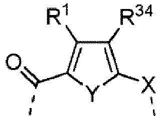 | 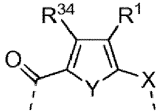 | 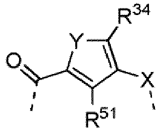 | 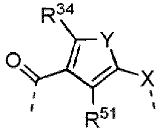 |
| **A225** (a7) | **A226** (a7) | **A227** (a7) | **A228** (a7) |
| 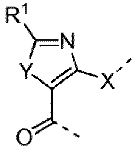 | 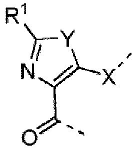 | 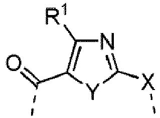 | 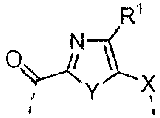 |
| **A229** (a8) | **A230** (a8) | **A231** (a8) | **A232** (a8) |
| 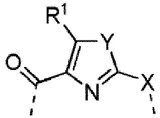 | 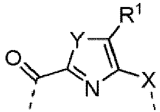 | 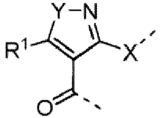 | 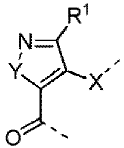 |
| **A233** (a8) | **A234** (a8) | **A235** (a9) | **A236** (a9) |
| 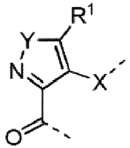 | 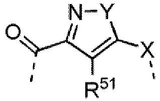 | 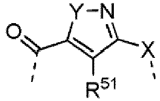 | 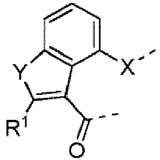 |
| **A237** (a9) | **A238** (a9) | **A239** (a9) | **A240** (a10) |
| 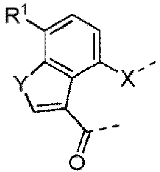 | 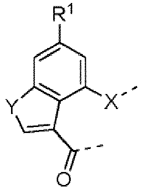 | 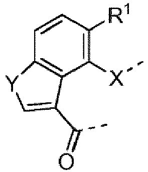 | 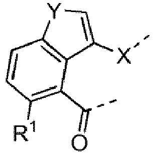 |
| **A241** (a10) | **A242** (a10) | **A243** (a10) | **A244** (a10) |
| 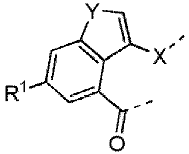 | 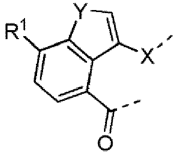 | 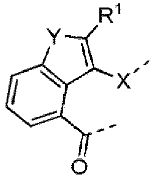 | 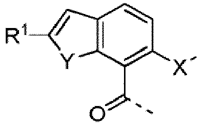 |
| **A245** (a10) | **A246** (a10) | **A247** (a10) | **A248** (a10) |
| 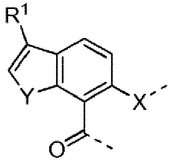 | 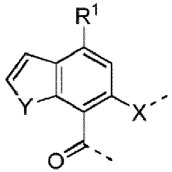 | 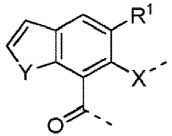 | 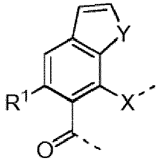 |
| **A249** (a10) | **A250** (a10) | **A251** (a10) | **A252** (a10) |
| 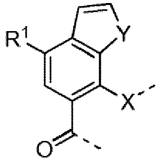 | 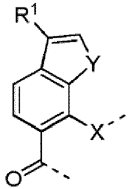 | 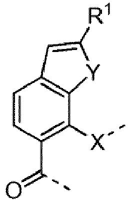 | 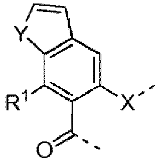 |
| **A253** (a10) | **A254** (a10) | **A255** (a10) | **A256** (a10) |
| 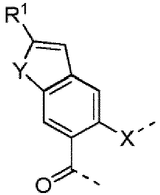 | 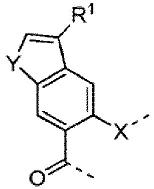 | 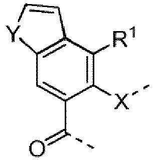 | 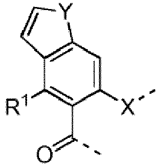 |
| **A257** (a10) | **A258** (a10) | **A259** (a10) | **A260** (a10) |
| 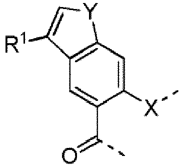 | 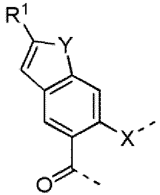 | 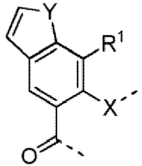 | 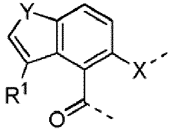 |
| **A261** (a10) | **A262** (a10) | **A263** (a10) | **A264** (a10) |
| 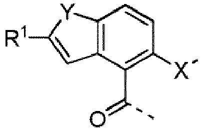 | 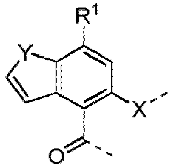 | 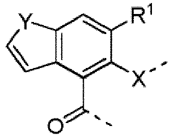 | 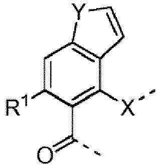 |
| **A265** (a10) | **A266** (a10) | **A267** (a10) | **A268** (a10) |
| 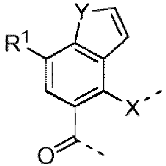 | 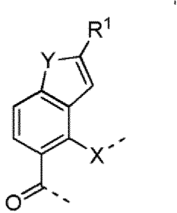 | 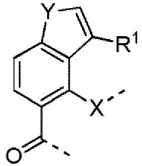 | 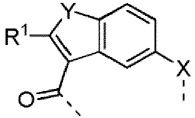 |
| **A269** (a10) | **A270** (a10) | **A271** (a10) | **A272** (a10) |
| 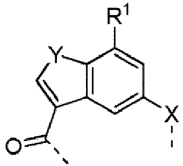 | 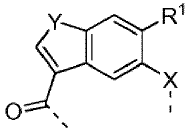 | 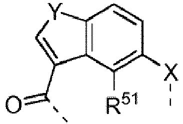 | 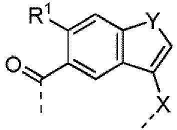 |
| **A273** (a10) | **A274** (a10) | **A275** (a10) | **A276** (a10) |
| 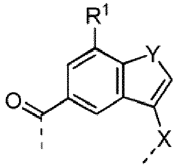 | 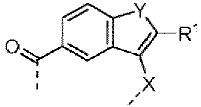 | 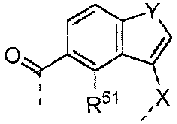 | 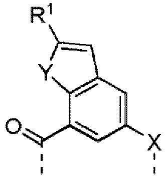 |
| **A277** (a10) | **A278** (a10) | **A279** (a10) | **A280** (a10) |
| 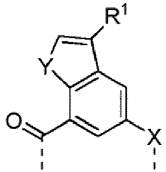 | 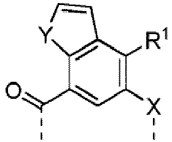 | 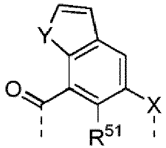 | 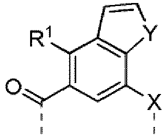 |
| **A281** (a10) | **A282** (a10) | **A283** (a10) | **A284** (a10) |
| 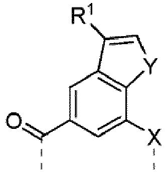 | 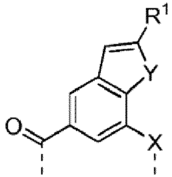 | 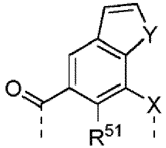 | 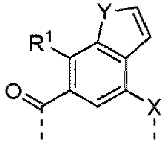 |
| **A285** (a10) | **A286** (a10) | **A287** (a10) | **A288** (a10) |
| 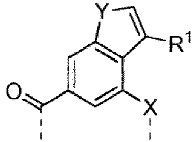 | 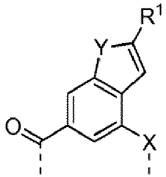 | 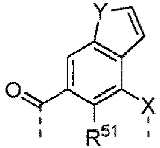 | 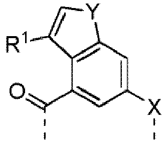 |
| **A289** (a10) | **A290** (a10) | **A291** (a10) | **A292** (a10) |
| 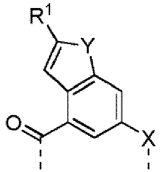 | 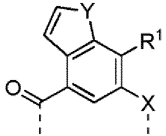 | 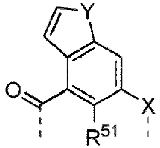 | 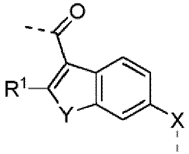 |
| **A293** (a10) | **A294** (a10) | **A295** (a10) | **A296** (a10) |
| 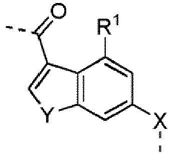 | 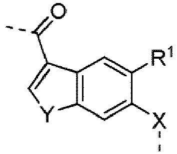 | 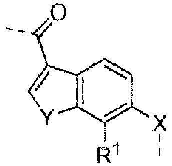 | 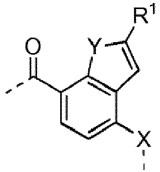 |
| **A297** (a10) | **A298** (a10) | **A299** (a10) | **A300** (a10) |
| 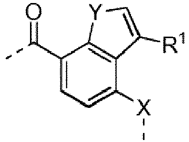 | 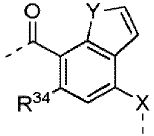 | 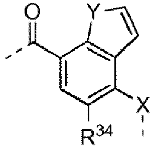 | 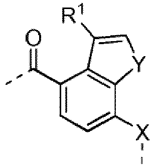 |
| **A301** (a10) | **A302** (a10) | **A303** (a10) | **A304** (a10) |
| 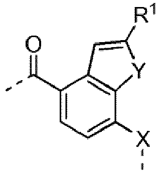 | 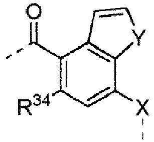 | 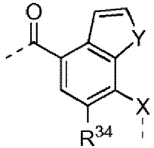 | 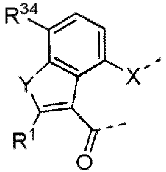 |
| **A305** (a10) | **A306** (a10) | **A307** (a10) | **A308** (a10) |
| 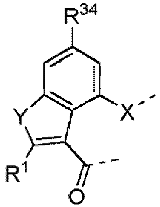 | 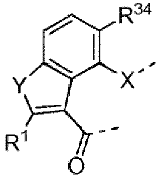 | 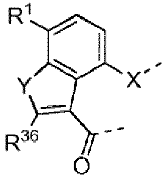 | 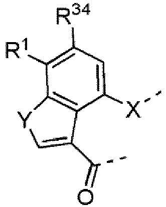 |
| **A309** (a10) | **A310** (a10) | **A311** (a10) | **A312** (a10) |
| 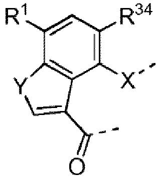 | 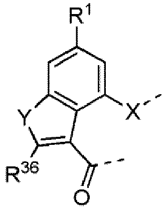 | 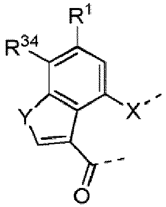 | 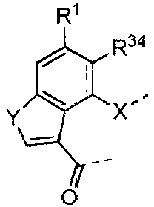 |
| **A313** (a10) | **A314** (a10) | **A315** (a10) | **A316** (a10) |
| 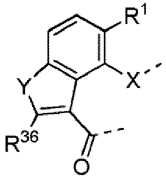 | 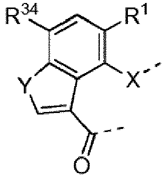 | 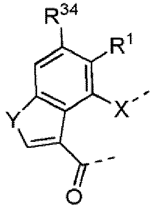 | 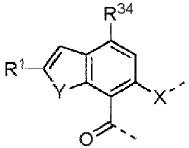 |
| **A317** (a10) | **A318** (a10) | **A319** (a10) | **A320** (a10) |
| 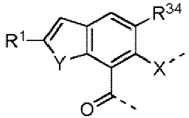 | 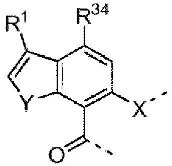 | 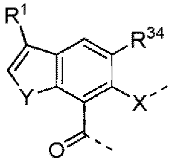 | 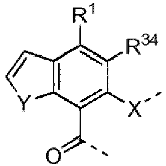 |
| **A321** (a10) | **A322** (a10) | **A323** (a10) | **A324** (a10) |
| 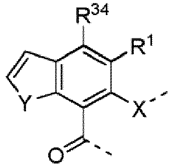 | 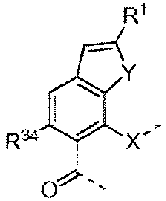 | 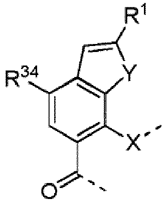 | 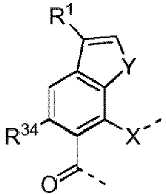 |
| **A325** (a10) | **A326** (a10) | **A327** (a10) | **A328** (a10) |
| 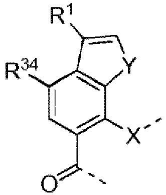 | 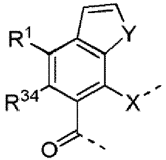 | 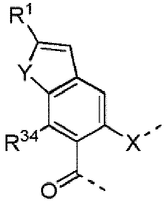 | 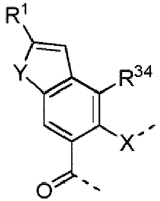 |
| **A329** (a10) | **A330** (a10) | **A331** (a10) | **A332** (a10) |
| 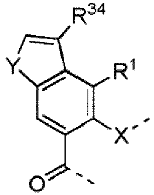 | 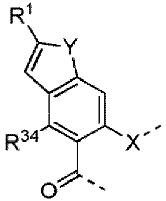 | 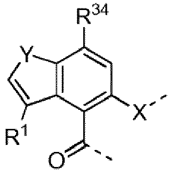 | 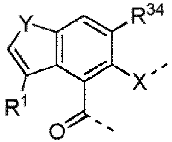 |
| **A333** (a10) | **A334** (a10) | **A335** (a10) | **A336** (a10) |
| 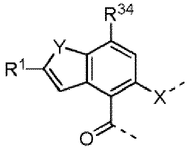 | 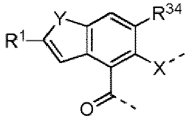 | 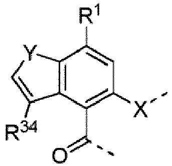 | 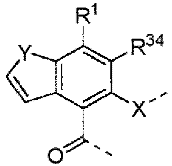 |
| **A337** (a10) | **A338** (a10) | **A339** (a10) | **A340** (a10) |
| 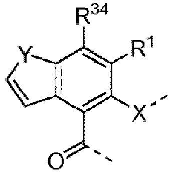 | 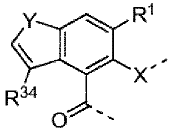 | 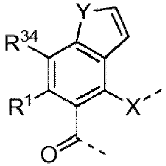 | 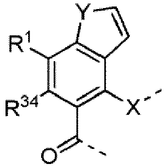 |
| **A341** (a10) | **A342** (a10) | **A343** (a10) | **A344** (a10) |
| 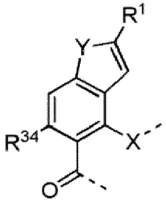 | 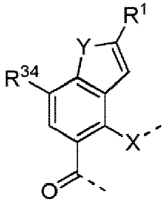 | 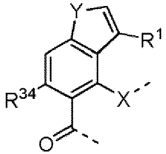 | 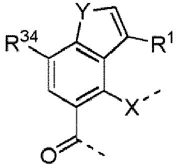 |
| **A345** (a10) | **A346** (a10) | **A347** (a10) | **A348** (a10) |
| 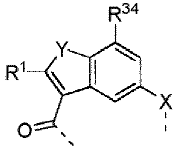 | 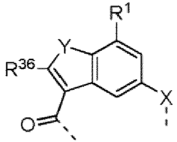 | 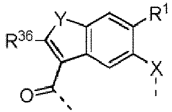 | 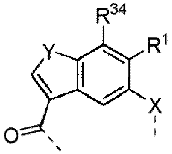 |
| **A349** (a10) | **A350** (a10) | **A351** (a10) | **A352** (a10) |
| 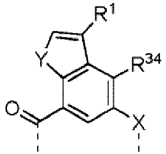 | 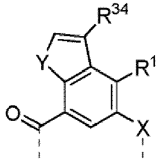 | 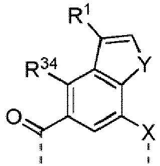 | 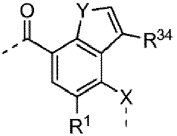 |
| **A353** (a10) | **A354** (a10) | **A355** (a10) | **A356** (a10) |
| 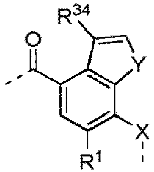 | 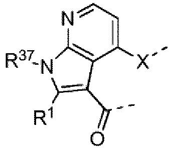 | 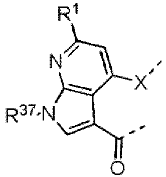 | 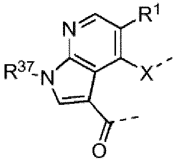 |
| **A357** (a10) | **A358** (a11) | **A359** (a11) | **A360** (a11) |
| 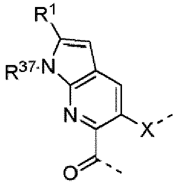 | 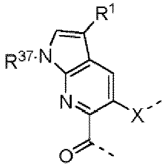 | 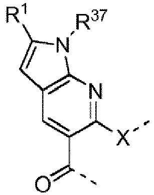 | 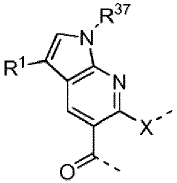 |
| **A361** (a11) | **A362** (a11) | **A363** (a11) | **A364** (a11) |
| 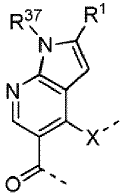 | 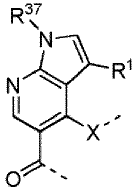 | 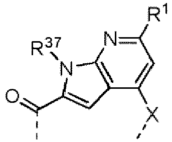 | 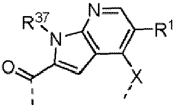 |
| **A365** (a11) | **A366** (a11) | **A367** (a11) | **A368** (a11) |
| 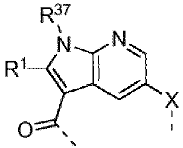 | 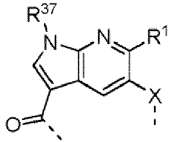 | 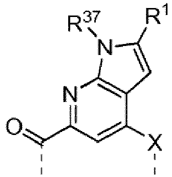 | 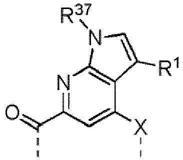 |
| **A369** (a11) | **A370** (a11) | **A371** (a11) | **A372** (a11) |
| 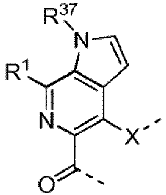 | 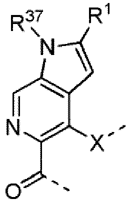 | 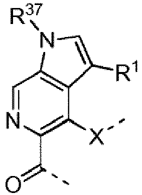 | 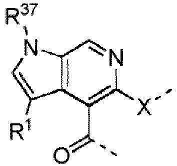 |
| **A373** (a12) | **A374** (a12) | **A375** (a12) | **A376** (a12) |
| 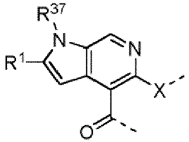 | 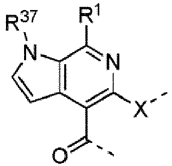 | 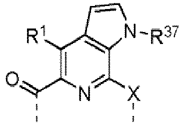 | 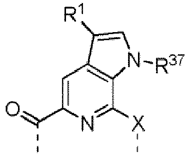 |
| **A377** (a12) | **A378** (a12) | **A379** (a12) | **A380** (a12) |
| 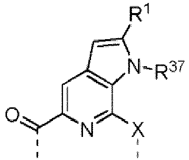 | 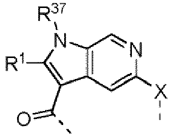 | 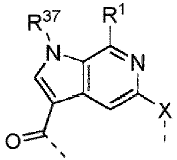 | 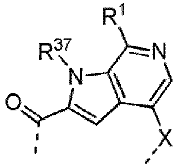 |
| **A381** (a12) | **A382** (a12) | **A383** (a12) | **A384** (a12) |
| 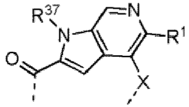 | 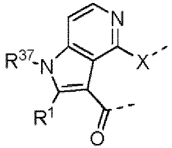 | 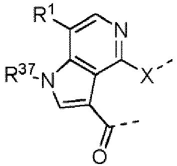 | 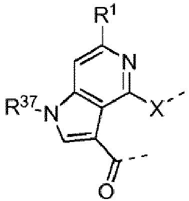 |
| **A385** (a12) | **A386** (a13) | **A387** (a13) | **A388** (a13) |
| 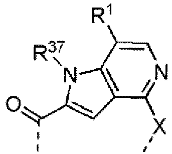 | 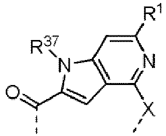 | 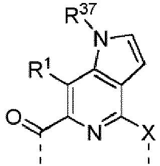 | 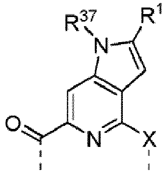 |
| **A389** (a13) | **A390** (a13) | **A391** (a13) | **A392** (a13) |
| 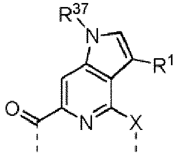 | 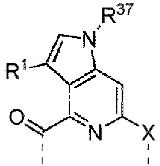 | 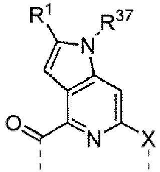 | 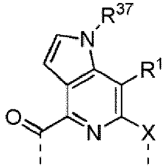 |
| **A393** (a13) | **A394** (a13) | **A395** (a13) | **A396** (a13) |
| 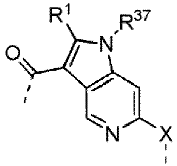 | 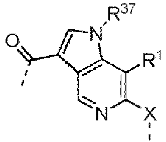 | 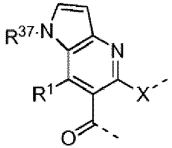 | 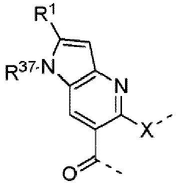 |
| **A397** (a13) | **A398** (a13) | **A399** (a14) | **A400** (a14) |
| 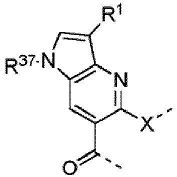 | 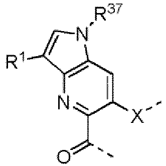 | 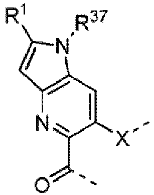 | 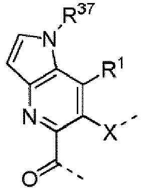 |
| **A401** (a14) | **A402** (a14) | **A403** (a14) | **A404** (a14) |
| 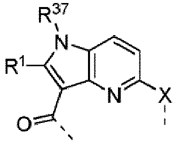 | 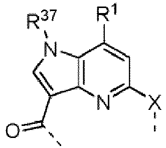 | 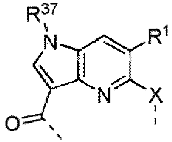 | 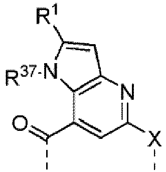 |
| **A405** (a14) | **A406** (a14) | **A407** (a14) | **A408** (a14) |
| 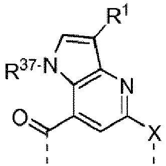 | 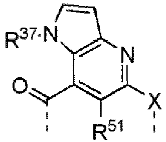 | 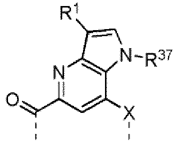 | 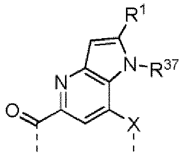 |
| **A409** (a14) | **A410** (a14) | **A411** (a14) | **A412** (a14) |
| 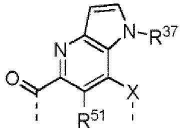 | 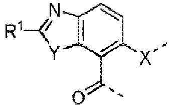 | 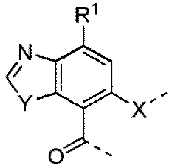 | 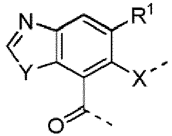 |
| **A413** (a14) | **A414** (a15) | **A415** (a15) | **A416** (a15) |
| 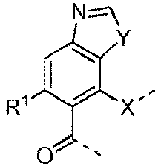 | 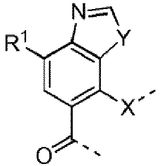 | 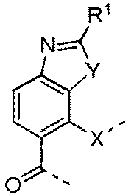 | 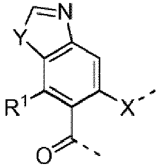 |
| **A417** (a15) | **A418** (a15) | **A419** (a15) | **A420** (a15) |
| 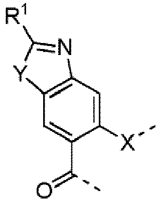 | 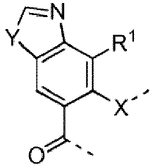 | 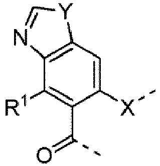 | 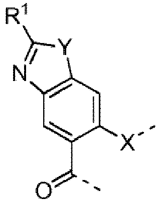 |
| **A421** (a15) | **A422** (a15) | **A423** (a15) | **A424** (a15) |
| 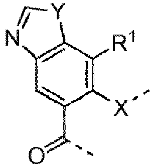 | 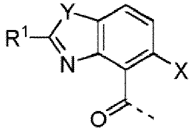 | 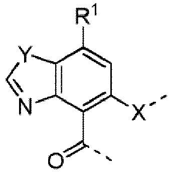 | 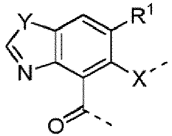 |
| **A425** (a15) | **A426** (a15) | **A427** (a15) | **A428** (a15) |
| 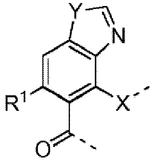 | 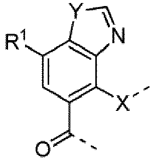 | 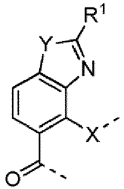 | 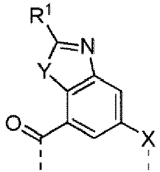 |
| **A429** (a15) | **A430** (a15) | **A431** (a15) | **A432** (a15) |
| 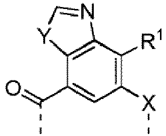 | 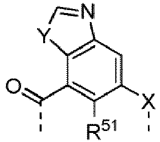 | 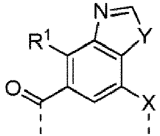 | 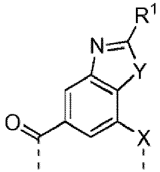 |
| **A433** (a15) | **A434** (a15) | **A435** (a15) | **A436** (a15) |
| 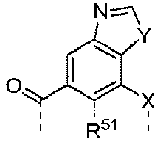 | 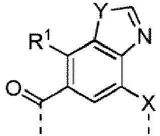 | 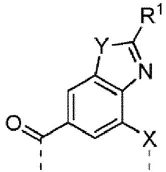 | 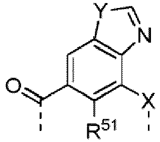 |
| **A437** (a15) | **A438** (a15) | **A439** (a15) | **A440** (a15) |
| 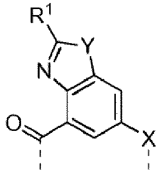 | 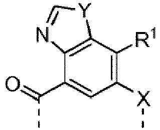 | 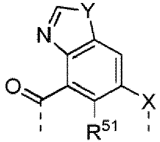 | 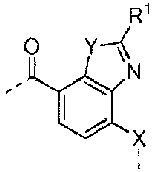 |
| **A441** (a15) | **A442** (a15) | **A443** (a15) | **A444** (a15) |
| 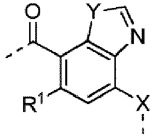 | 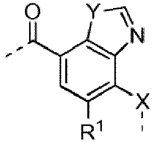 | 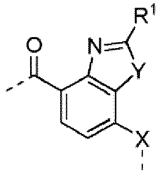 | 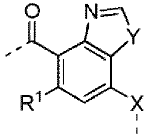 |
| **A445** (a15) | **A446** (a15) | **A447** (a15) | **A448** (a15) |
| 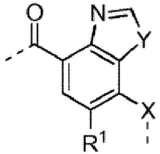 | 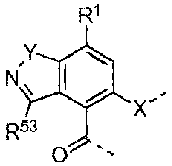 | 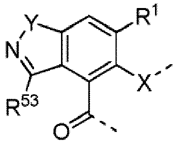 | 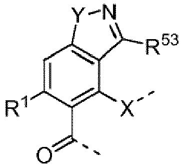 |
| **A449** (a15) | **A450** (a16) | **A451** (a16) | **A452** (a16) |
| 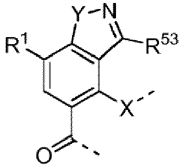 | 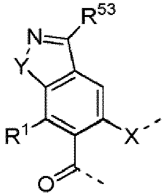 | 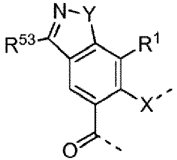 | 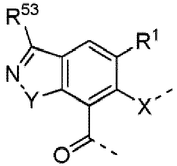 |
| **A453** (a16) | **A454** (a16) | **A455** (a16) | **A456** (a16) |
| 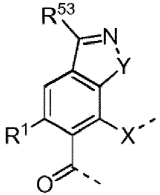 | 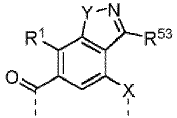 | 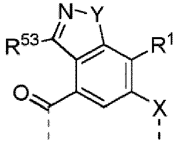 | 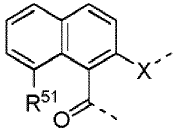 |
| **A457** (a16) | **A458** (a16) | **A459** (a16) | **A460** (a17) |
| 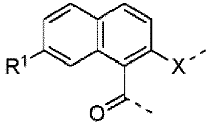 | 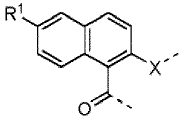 | 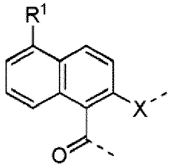 | 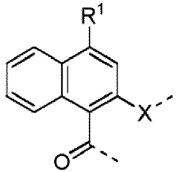 |
| **A461** (a17) | **A462** (a17) | **A463** (a17) | **A464** (a17) |
| 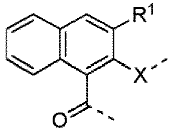 | 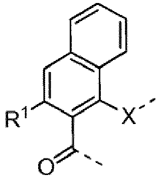 | 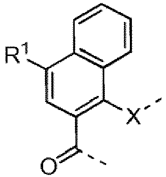 | 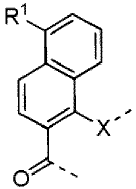 |
| **A465** (a17) | **A466** (a17) | **A467** (a17) | **A468** (a17) |
| 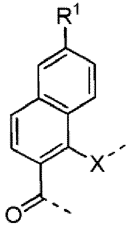 | 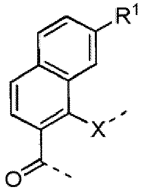 | 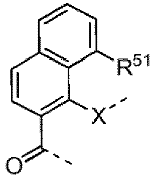 | 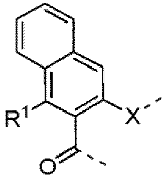 |
| **A469** (a17) | **A470** (a17) | **A471** (a17) | **A472** (a17) |
| 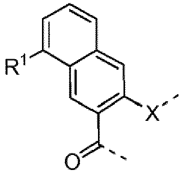 | 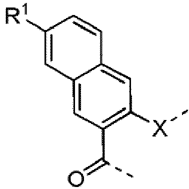 | 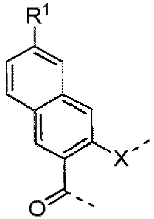 | 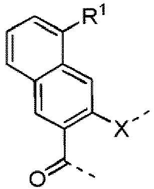 |
| **A473** (a17) | **A474** (a17) | **A475** (a17) | **A476** (a17) |
| 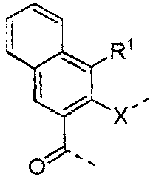 | 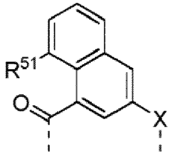 | 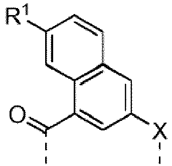 | 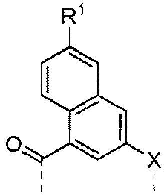 |
| **A477** (a17) | **A478** (a17) | **A479** (a17) | **A480** (a17) |
| 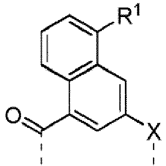 | 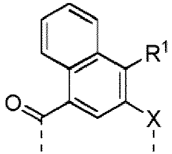 | 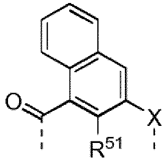 | 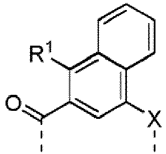 |
| **A481** (a17) | **A482** (a17) | **A483** (a17) | **A484** (a17) |
| 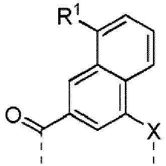 | 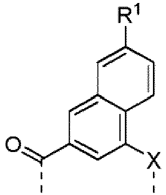 | 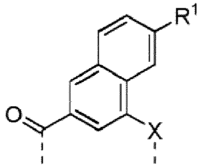 | 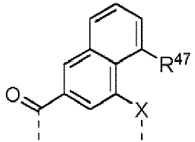 |
| **A485** (a17) | **A486** (a17) | **A487** (a17) | **A488** (a17) |
| 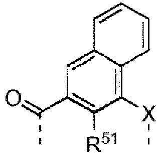 | 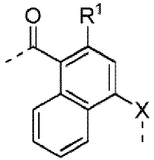 | 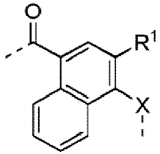 | 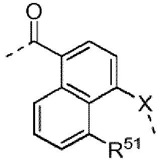 |
| **A489** (a17) | **A490** (a17) | **A491** (a17) | **A492** (a17) |
| 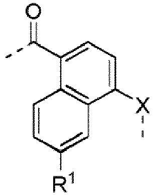 | 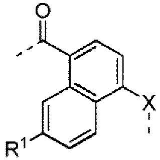 | 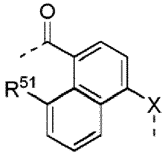 | 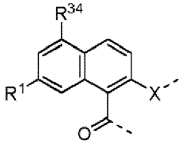 |
| **A493** (a17) | **A494** (a17) | **A495** (a17) | **A496** (a17) |
| 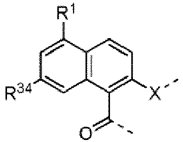 | 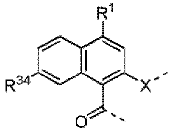 | 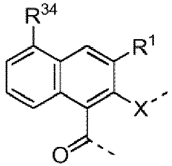 | 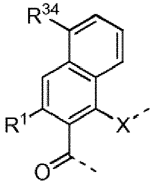 |
| **A497** (a17) | **A498** (a17) | **A499** (a17) | **A500** (a17) |
| 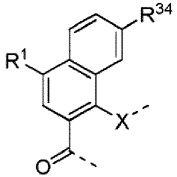 | 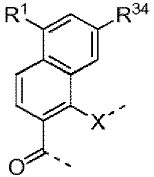 | 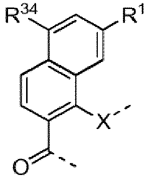 | 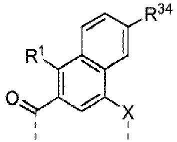 |
| **A501** (a17) | **A502** (a17) | **A503** (a17) | **A504** (a17) |
| 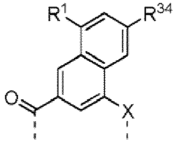 | 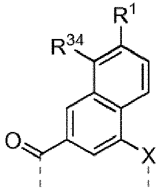 | 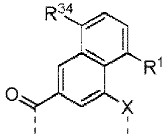 | 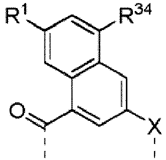 |
| **A505** (a17) | **A506** (a17) | **A507** (a17) | **A508** (a17) |
| 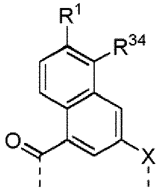 | 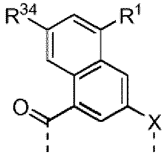 | 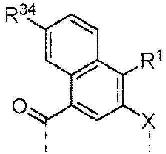 | 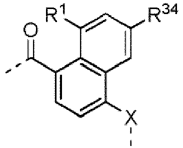 |
| **A509** (a17) | **A510** (a17) | **A511** (a17) | **A512** (a17) |
| 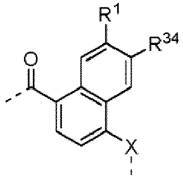 | 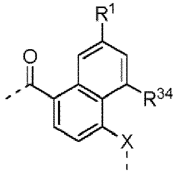 | 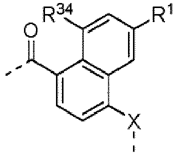 | 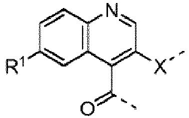 |
| **A513** (a17) | **A514** (a17) | **A515** (a17) | **A516** (a18) |
| 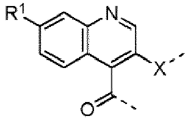 | 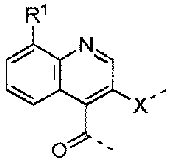 | 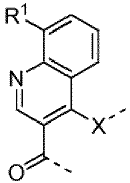 | 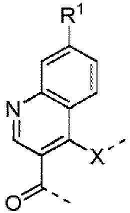 |
| **A517** (a18) | **A518** (a18) | **A519** (a18) | **A520** (a18) |
| 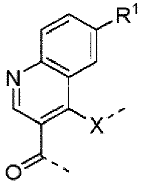 | 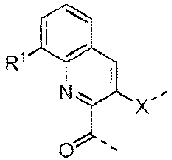 | 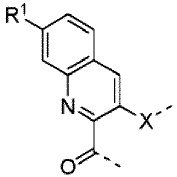 | 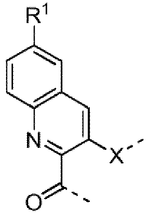 |
| **A521** (a18) | **A522** (a18) | **A523** (a18) | **A524** (a18) |
| 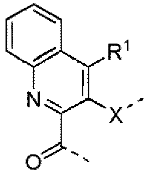 | 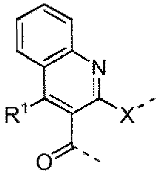 | 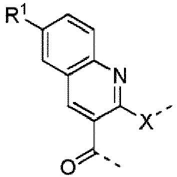 | 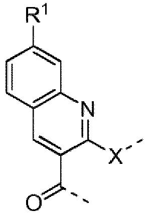 |
| **A525** (a18) | **A526** (a18) | **A527** (a18) | **A528** (a18) |
| 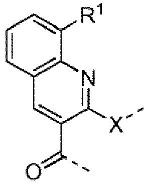 | 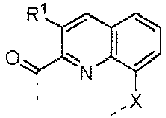 | 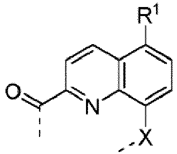 | 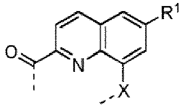 |
| **A529** (a18) | **A530** (a18) | **A531** (a18) | **A532** (a18) |
| 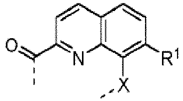 | 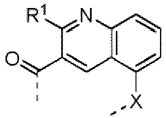 | 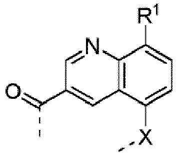 | 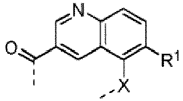 |
| **A533** (a18) | **A534** (a18) | **A535** (a18) | **A536** (a18) |
| 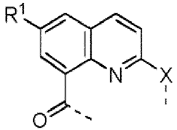 | 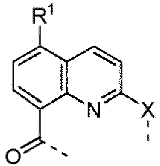 | 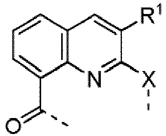 | 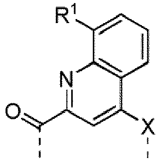 |
| **A537** (a18) | **A538** (a18) | **A539** (a18) | **A540** (a18) |
| 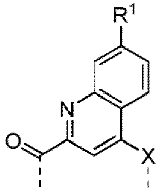 | 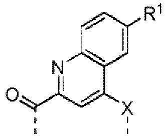 | 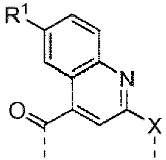 | 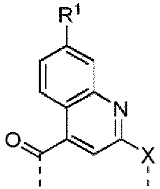 |
| **A541** (a18) | **A542** (a18) | **A543** (a18) | **A544** (a18) |
| 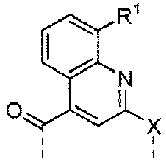 | 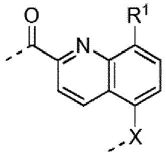 | 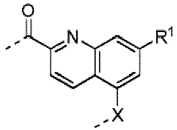 | 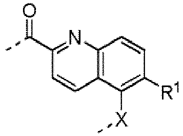 |
| **A545** (a18) | **A546** (a18) | **A547** (a18) | **A548** (a18) |
| 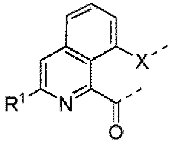 | 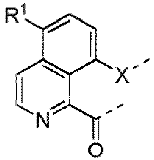 | 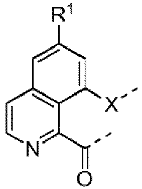 | 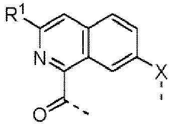 |
| **A549** (a19) | **A550** (a19) | **A551** (a19) | **A552** (a19) |
| 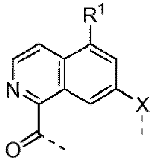 | 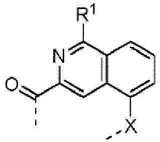 | 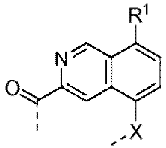 | 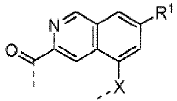 |
| **A553** (a19) | **A554** (a19) | **A555** (a19) | **A556** (a19) |
| 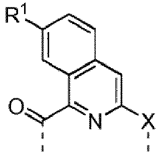 | 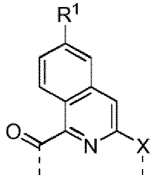 | 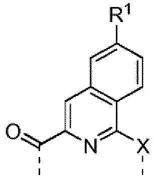 | 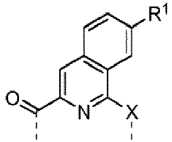 |
| **A557** (a19) | **A558** (a19) | **A559** (a19) | **A560** (a19) |
| 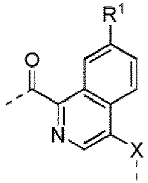 | 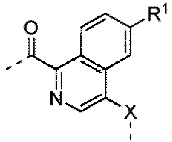 | 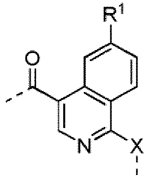 | 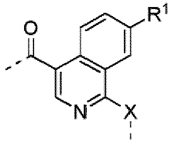 |
| **A561** (a19) | **A562** (a19) | **A563** (a19) | **A564** (a19) |
| 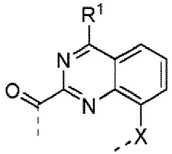 | 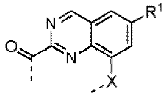 | 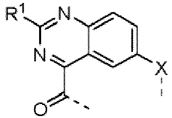 | 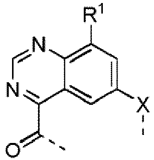 |
| **A565** (a20) | **A566** (a20) | **A567** (a20) | **A568** (a20) |
| 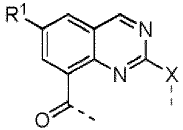 | 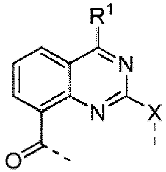 | 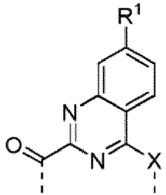 | 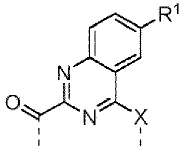 |
| **A569** (a20) | **A570** (a20) | **A571** (a20) | **A572** (a20) |
| 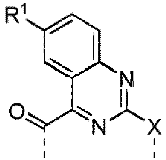 | 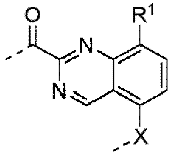 | 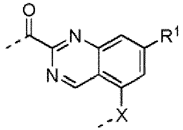 | 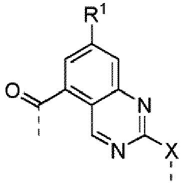 |
| **A573** (a20) | **A574** (a20) | **A575** (a20) | **A576** (a20) |
| 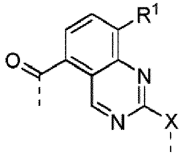 | 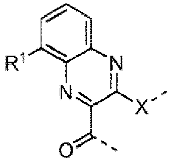 | 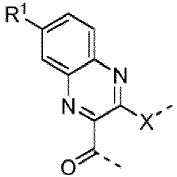 | 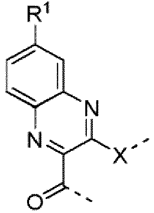 |
| **A577** (a20) | **A578** (a21) | **A579** (a21) | **A580** (a21) |
| 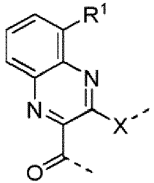 | 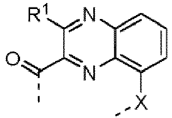 | 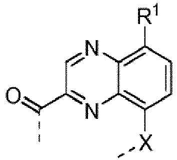 | 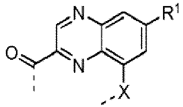 |
| **A581** (a21) | **A582** (a21) | **A583** (a21) | **A584** (a21) |
| 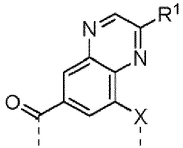 | 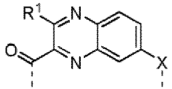 | 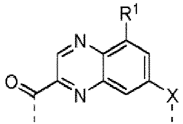 | 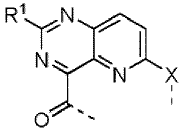 |
| **A585** (a21) | **A586** (a21) | **A587** (a21) | **A588** (a22) |
| 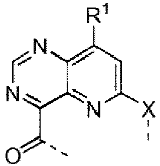 | 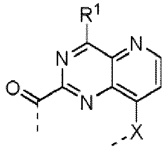 | 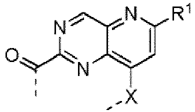 | 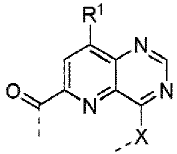 |
| **A589** (a22) | **A590** (a22) | **A591** (a22) | **A592** (a22) |
| 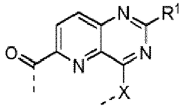 | 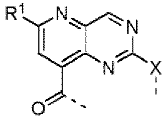 | 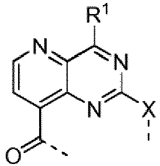 | 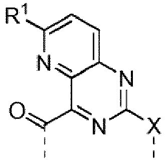 |
| **A593** (a22) | **A594** (a22) | **A595** (a22) | **A596** (a22) |
| 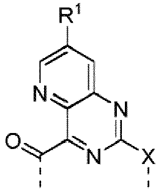 | 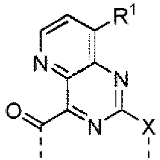 | 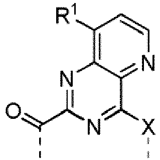 | 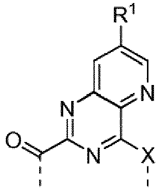 |
| **A597** (a22) | **A598** (a22) | **A599** (a22) | **A600** (a22) |
| 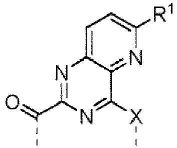 | 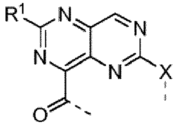 | 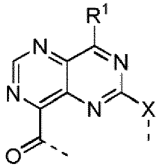 | 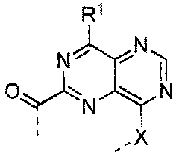 |
| **A601** (a22) | **A602** (a23) | **A603** (a23) | **A604** (a23) |
| 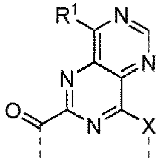 | 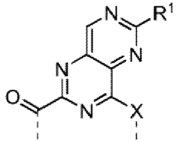 | 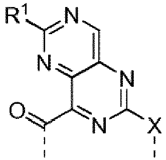 | 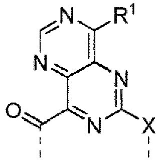 |
| **A605** (a23) | **A606** (a23) | **A607** (a23) | **A608** (a23) |
| 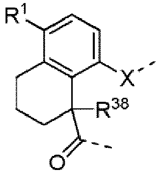 | 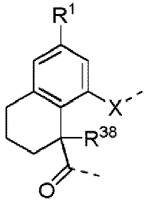 | 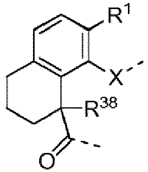 | 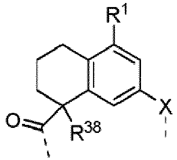 |
| **A609** (a24) | **A610** (a24) | **A611** (a24) | **A612** (a24) |
| 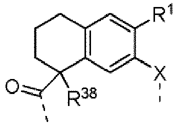 | 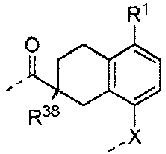 | 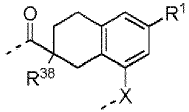 | 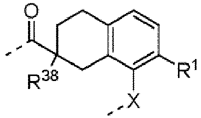 |
| **A613** (a24) | **A614** (a24) | **A615** (a24) | **A616** (a24) |
| 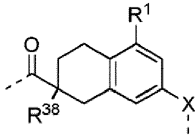 | 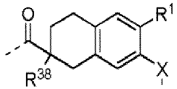 | 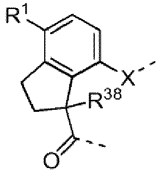 | 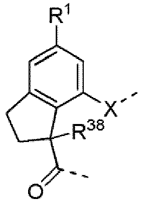 |
| **A617** (a24) | **A618** (a24) | **A619** (a25) | **A620** (a25) |
| 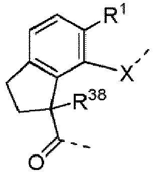 | 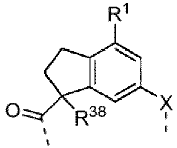 | 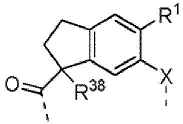 | 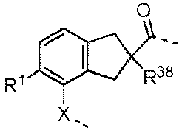 |
| **A621** (a25) | **A622** (a25) | **A623** (a25) | **A624** (a25) |
| 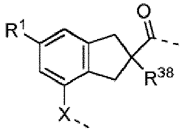 | 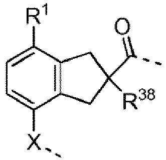 | 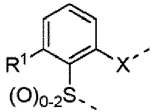 | 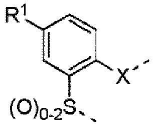 |
| **A625** (a25) | **A626** (a25) | **A627** (a1) | **A628** (a1) |
| 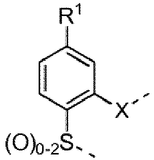 | 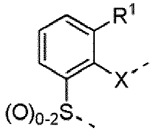 | 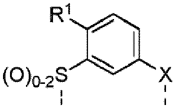 | 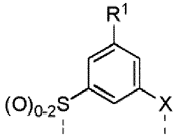 |
| **A629** (a1) | **A630** (a1) | **A631** (a1) | **A632** (a1) |
| 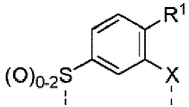 | 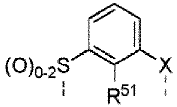 | 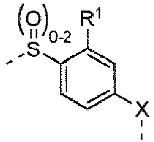 | 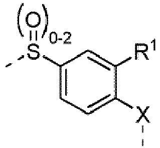 |
| **A633** (a1) | **A634** (a1) | **A635** (a1) | **A636** (a1) |
| 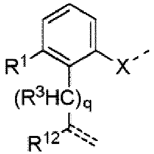 | 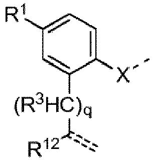 | 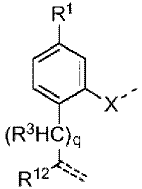 | 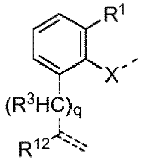 |
| **A637** (a1) | **A638** (a1) | **A639** (a1) | **A640** (a1) |
| 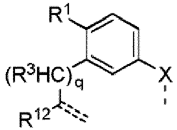 | 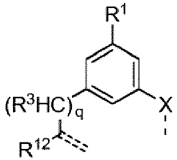 | 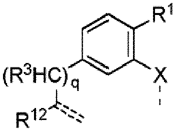 | 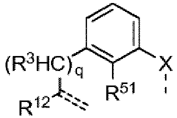 |
| **A641** (a1) | **A642** (a1) | **A643** (a1) | **A644** (a1) |
| 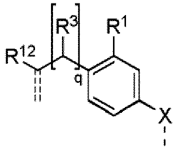 | 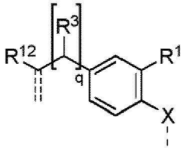 | 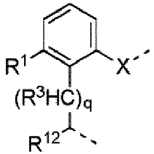 | 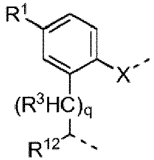 |
| **A645** (a1) | **A646** (a1) | **A647** (a1) | **A648** (a1) |
| 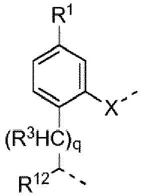 | 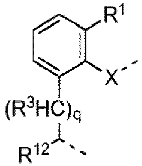 | 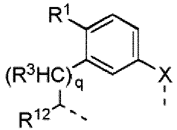 | 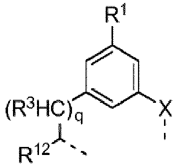 |
| **A649** (a1) | **A650** (a1) | **A651** (a1) | **A652** (a1) |
| 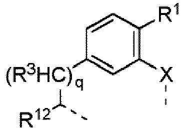 | 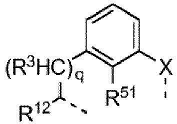 | 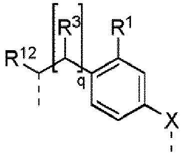 | 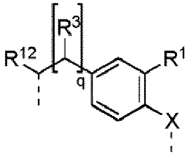 |
| **A653** (a1) | **A654** (a1) | **A655** (a1) | **A656** (a1) |
| 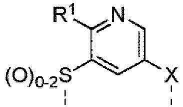 | 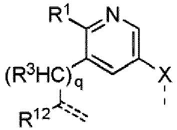 | 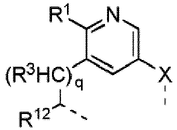 | 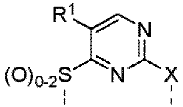 |
| **A657** (a2) | **A658** (a2) | **A659** (a2) | **A660** (a4) |
| 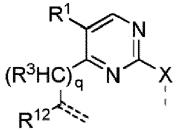 | 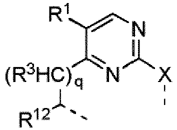 | 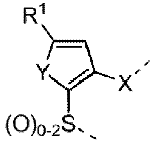 | 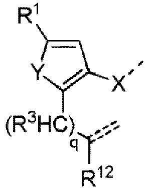 |
| **A661** (a4) | **A662** (a4) | **A663** (a7) | **A664** (a7) |
| 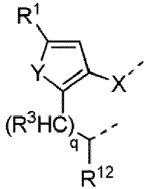 | 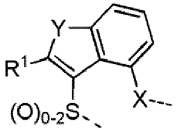 | 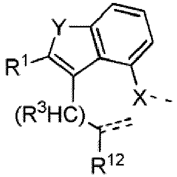 | 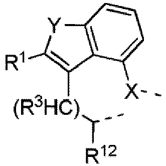 |
| **A665** (a7) | **A666** (a10) | **A667** (a10) | **A668** (a10) |
| 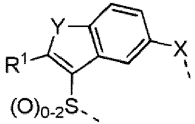 | 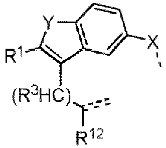 | 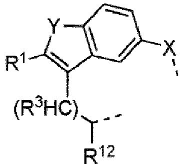 | 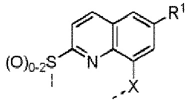 |
| **A669** (a10) | **A670** (a10) | **A671** (a10) | **A672** (a18) |
| 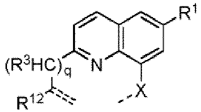 | 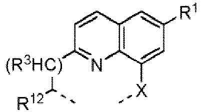 | 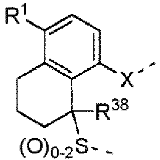 | 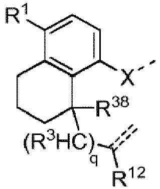 |
| **A673** (a18) | **A674** (a18) | **A675** (a24) | **A676** (a24) |
| 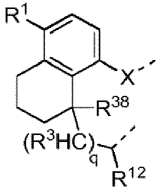 | 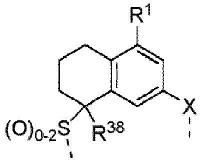 | 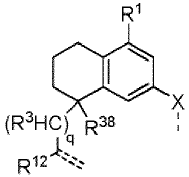 | 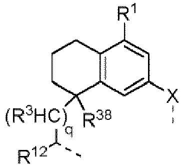 |
| **A677** (a24) | **A678** (a24) | **A679** (a24) | **A680** (a24) |
| 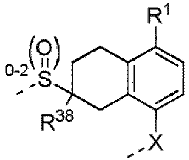 | 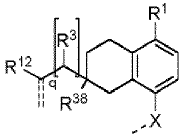 | | 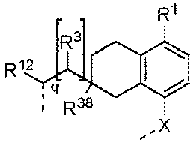 |
| **A681** (a24) | **A682** (a24) | | **A683** (a24) |
die **Basiskomponente B** ein zweiwertiges Radikal ist, ausgewählt aus der Gruppe aus
| | | | |
|---|---|---|---|
| 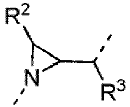 | 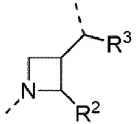 | 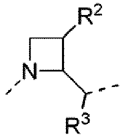 | 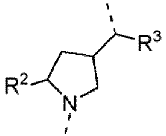 |
| **B1** (b1) | **B2** (b2) | **B3** (b2) | **B4** (b3) |
| | | | |
|---|---|---|---|
| 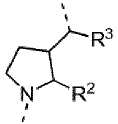 | 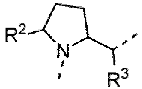 | 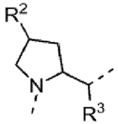 | 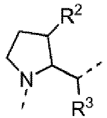 |
| **B5** (b3) | **B6** (b3) | **B7** (b3) | **B8** (b3) |
| 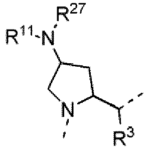 | 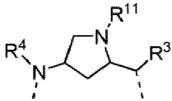 | 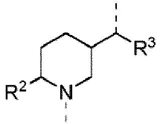 | 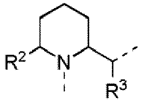 |
| **B9** (b3) | **B10** (b3) | **B11** (b4) | **B12** (b4) |
| 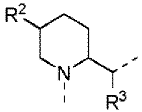 | 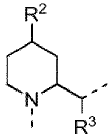 | 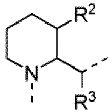 | 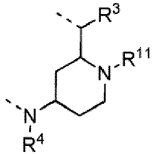 |
| **B13** (b4) | **B14** (b4) | **B15** (b4) | **B16** (b4) |
| 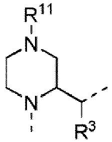 | 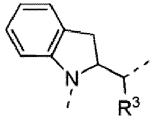 | 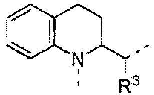 | 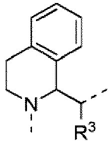 |
| **B17** (b5) | **B18** (b8) | **B19** (b10) | **B20** (b11) |
| 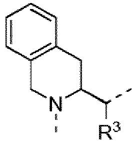 | | 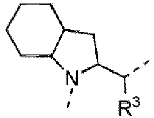 | |
| **B21** (b11) | | **B22** (b12) | |
die **Basiskomponente C** ein zweiwertiges Radikal ist, ausgewählt aus der Gruppe aus
| | |
|---|---|
| 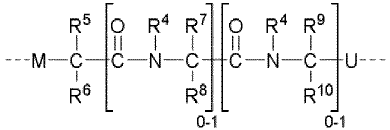 | 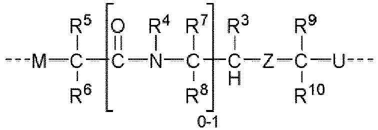 |
| **C1** | **C2** |
| 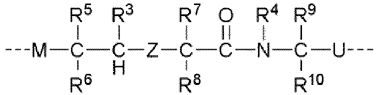 | 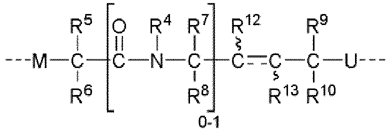 |
| **C3** | **C4** |
| 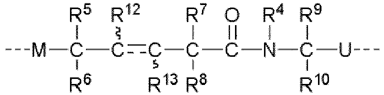 | 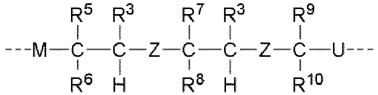 |
| **C5** | **C6** |
| 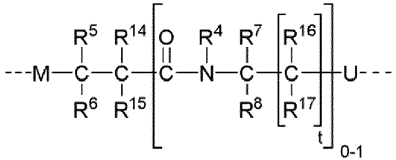 | 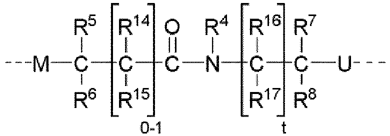 |
| **C7** | **C8** |
| 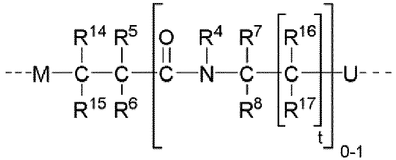 | 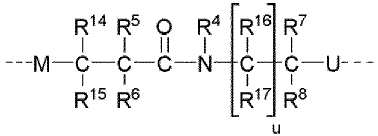 |
| **C9** | **C10** |
| 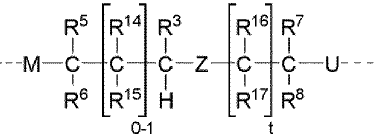 | 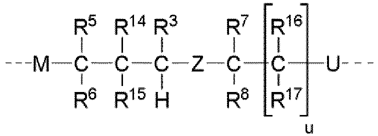 |
| **C11** | **C12** |
| 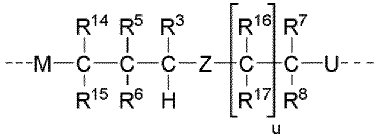 | 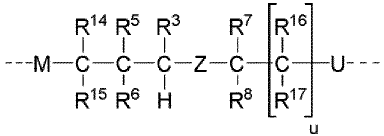 |
| **C13** | **C14** |
| 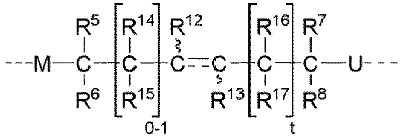 | 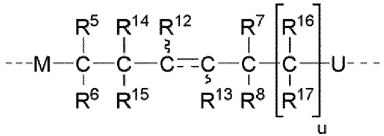 |
| **C15** | **C16** |
| 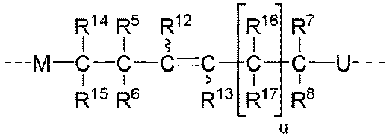 | 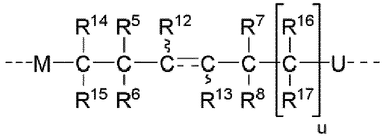 |
| **C17** | **C18** |
| 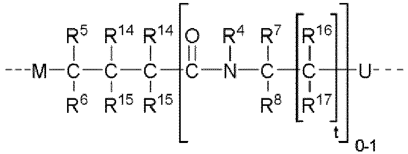 | 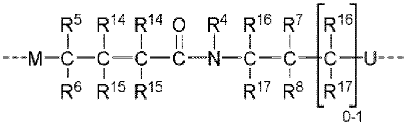 |
| **C19** | **C20** |
| 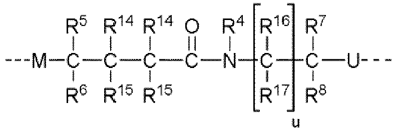 | 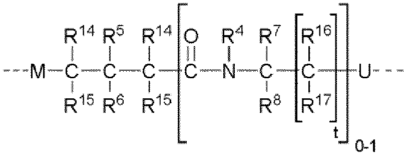 |
| **C21** | **C22** |
| 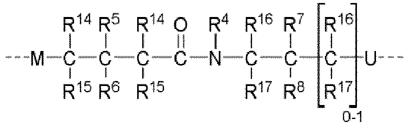 | 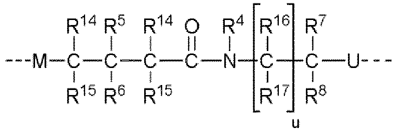 |
| **C23** | **C24** |
| 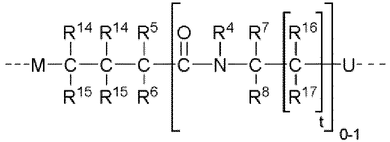 | 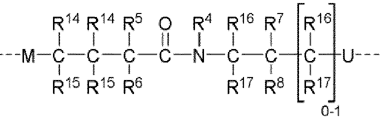 |
| **C25** | **C26** |
| 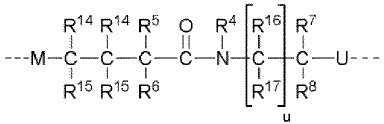 | 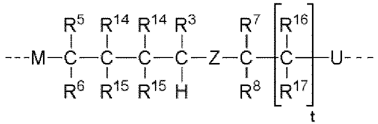 |
| **C27** | **C28** |
| 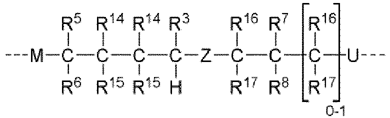 | 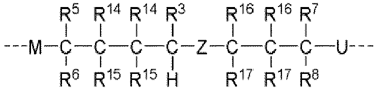 |
| **C29** | **C30** |
| 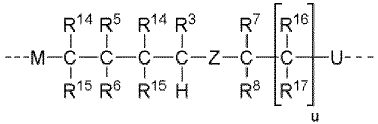 | 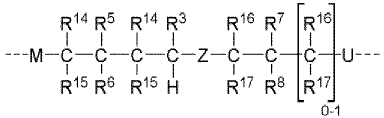 |
| **C31** | **C32** |
| 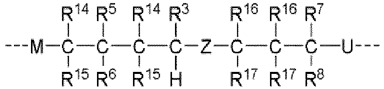 | 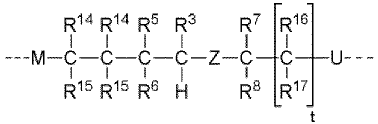 |
| **C33** | **C34** |
| 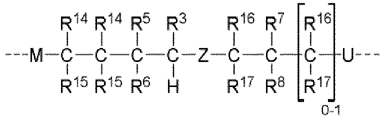 | 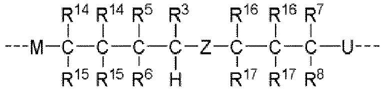 |
| **C35** | **C36** |
| 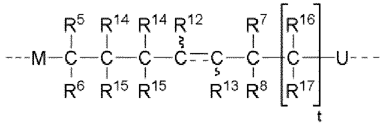 | 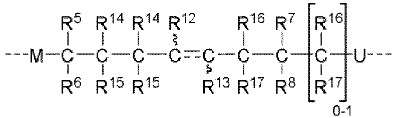 |
| **C37** | **C38** |
| 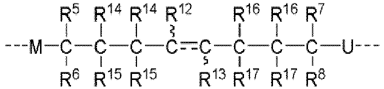 | 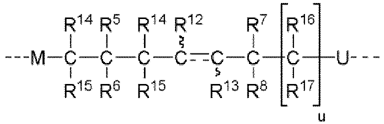 |
| **C39** | **C40** |
| 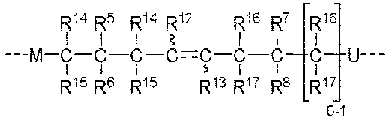 | 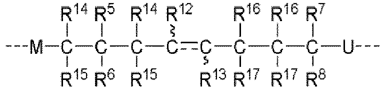 |
| **C41** | **C42** |
| 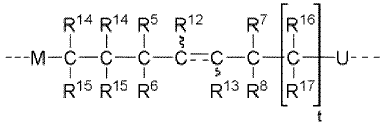 | 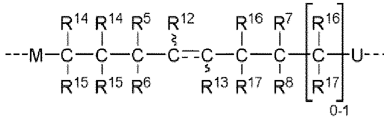 |
| **C43** | **C44** |
| 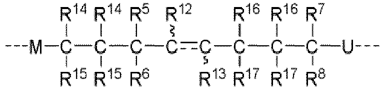 | 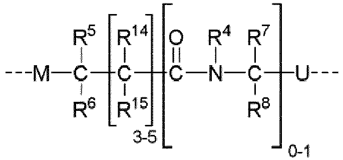 |
| **C45** | **C46** |
| 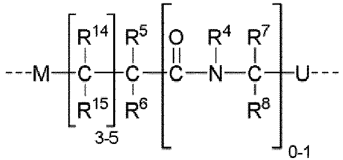 | 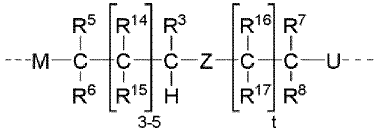 |
| **C47** | **C48** |
| 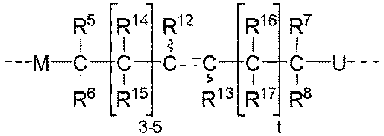 | 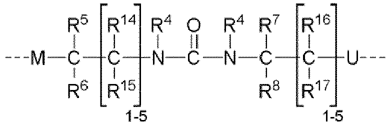 |
| **C49** | **C50** |
| 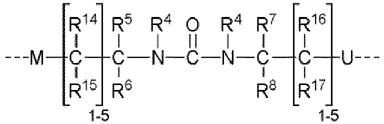 | 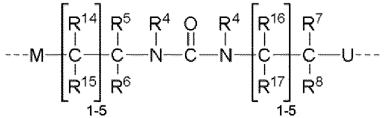 |
| **C51** | **C52** |
| 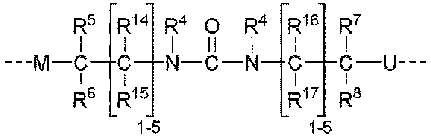 | |
| **C53** | |
| 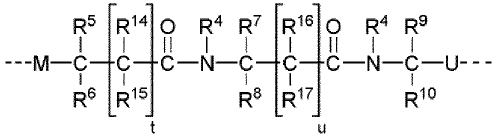 | |
| **C54** | |
| 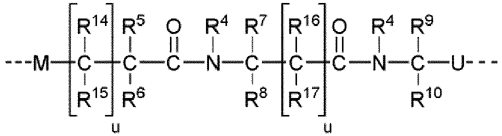 | |
| **C55** | |
| 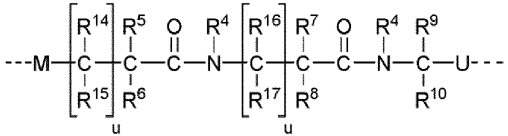 | |
| **C56** | |
| 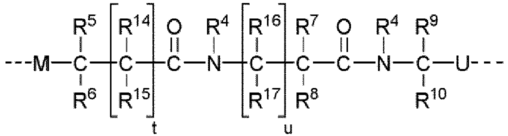 | |
| **C57** | |
| 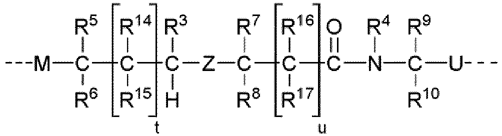 | |
| **C58** | |
| 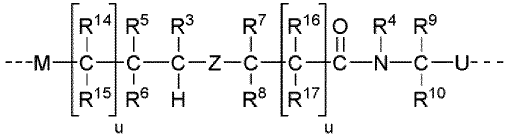 | |
| **C59** | |
| 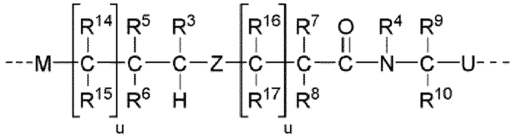 | |
| **C60** | |
| 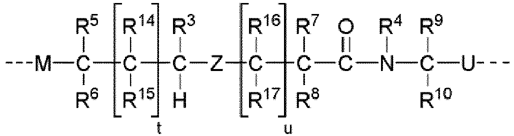 | |
| **C61** | |
| 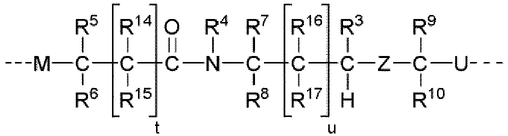 | |
| **C62** | |
| 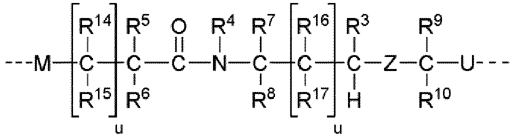 | |
| **C63** | |
| 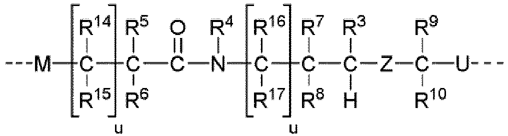 | |
| **C64** | |
| 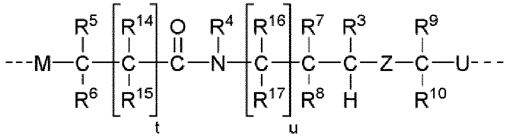 | |
| **C65** | |
| 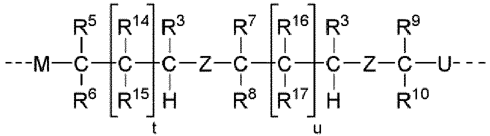 | |
| **C66** | |
| 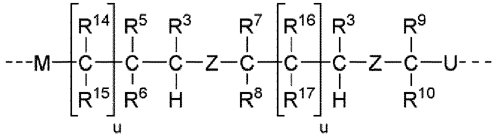 | |
| **C67** | |
| 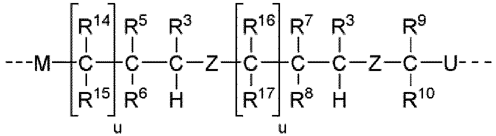 | |
| **C68** | |
| 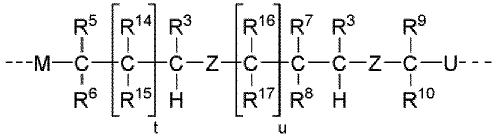 | |
| **C69** | |
| 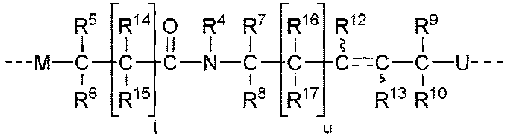 | |
| **C70** | |
| 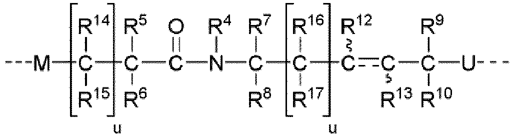 | |
| **C71** | |
| 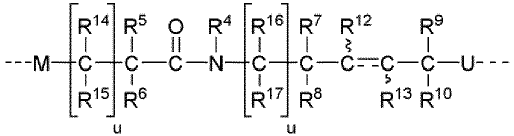 | |
| **C72** | |
| 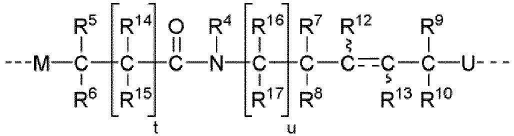 | |
| **C73** | |
| 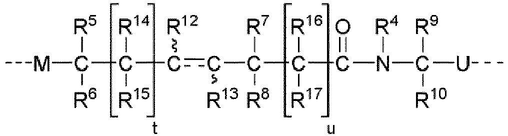 | |
| **C74** | |
| 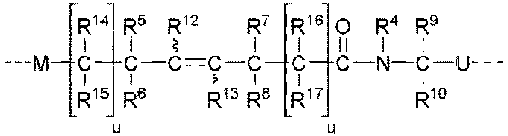 | |
| **C75** | |
| 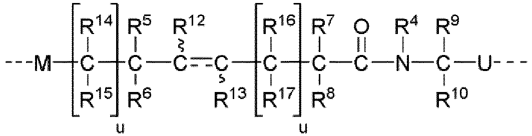 | |
| **C76** | |
| 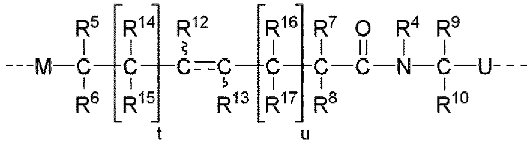 | |
| **C77** | |
| 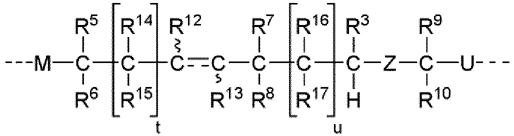 | |
| **C78** | |
| 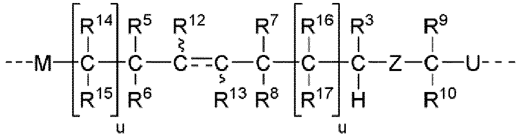 | |
| **C79** | |
| 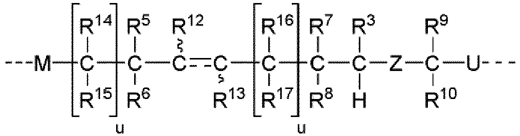 | |
| **C80** | |
| 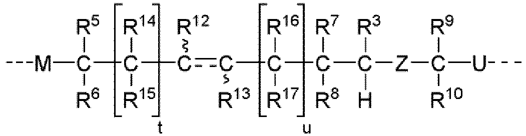 | |
| **C81** | |
| 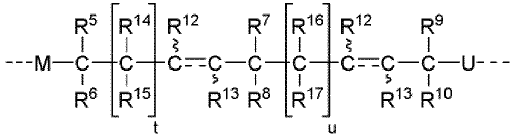 | |
| **C82** | |
| 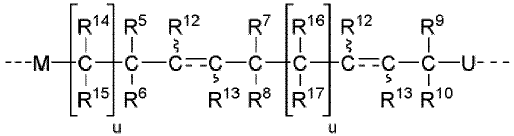 | |
| **C83** | |
| 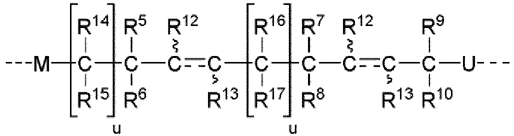 | |
| **C84** | |
| 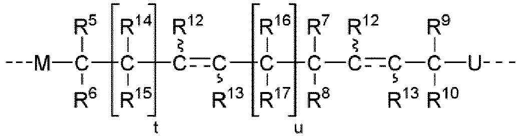 | |
| **C85** | |
| 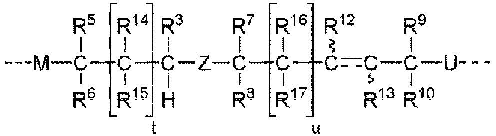 | |
| **C86** | |
| 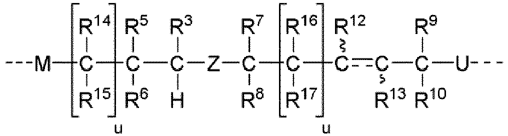 | |
| **C87** | |
| 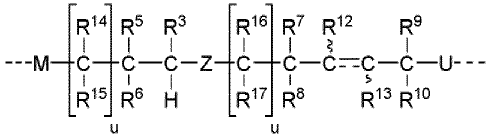 | |
| **C88** | |
| 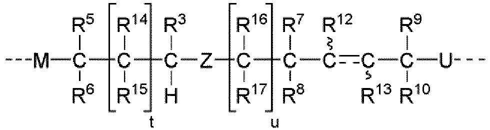 | |
| **C89** | |
| 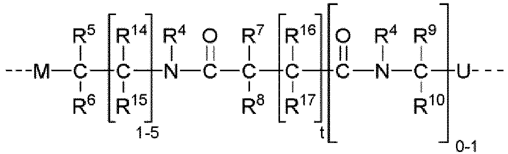 | |
| **C90** | |
| 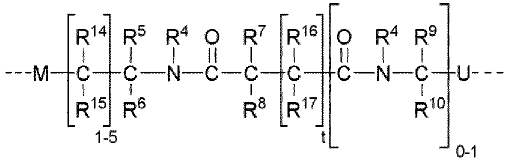 | |
| **C91** | |
| 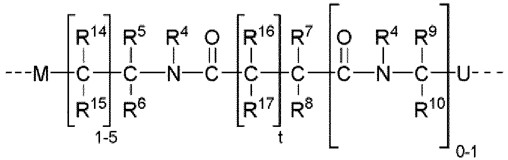 | |
| **C92** | |
| 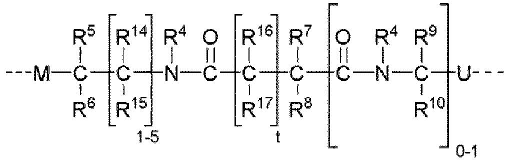 | |
| **C93** | |
| 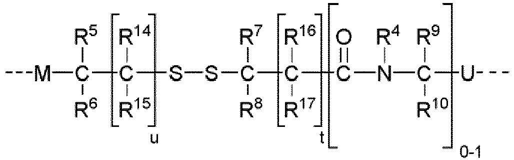 | |
| **C94** | |
| 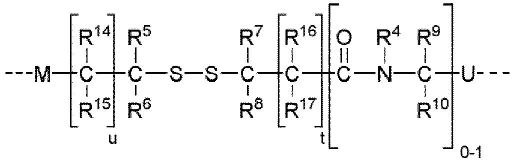 | |
| **C95** | |
| 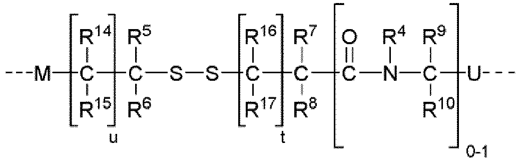 | |
| **C96** | |
| 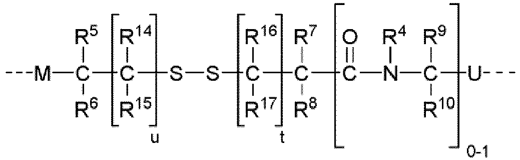 | |
| **C97** | |
| 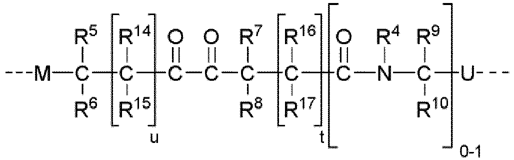 | |
| **C98** | |
| 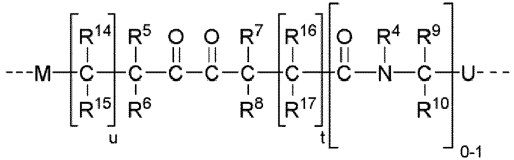 | |
| **C99** | |
| 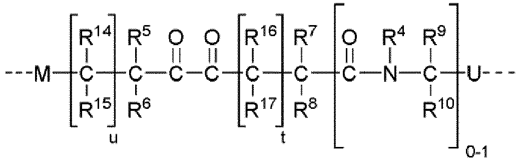 | |
| **C100** | |
| 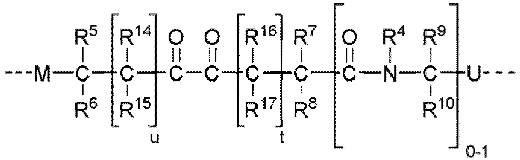 | |
| **C101** | |
und wobei ferner
R¹ für H; F; Cl; Br; I; CF₃; OCF₃; OCHF₂; NO₂; CN; Alkyl; Alkenyl; Alkinyl; Cycloalkyl; Heterocycloalkyl; Aryl; Heteroaryl; Arylalkyl; Heteroarylalkyl; -(CR¹⁸R¹⁹)_{q}OR²⁰; -(CR¹⁸R¹⁹)_{q}SR²⁰; -(CR¹⁸R¹⁹)_{q}NR⁴R¹¹; -(CR¹⁸R¹⁹)_{q}OCONR⁴R¹¹; -(CR¹⁸R¹⁹)_{q}OCOOR²¹; -(CR¹⁸R¹⁹)_{q}NR⁴COOR²¹; -(CR¹⁸R¹⁹)_{q}NR⁴COR²²; -(CR¹⁸R¹⁹)qNR⁴CONR⁴R¹¹; -(CR¹⁸R¹⁹)_{q}NR⁴SO₂R²³; -(CR¹⁸R¹⁹)_{q}NR⁴SO₂NR⁴R¹¹; -(CR¹⁸R¹⁹)_{q}COOR²¹; -(CR¹⁸R¹⁹)_{q}CONR⁴R¹¹; -(CR¹⁸R¹⁹)_{q}SO₂NR⁴R¹¹; -(CR¹⁸R¹⁹)_{q}PO(OR²¹)₂; -(CR¹⁸R¹⁹)_{q}OPO(OR²¹)₂; -(CR¹⁸R¹⁹)_{q}COR²²; -(CR¹⁸R¹⁹)_{q}SO₂R²³; -(CR¹⁸R¹⁹)_{q}OSO_{3R}²¹; -(CR¹⁸R¹⁹)_{q}R²⁴; -(CR¹⁸R¹⁹)_{q}R²⁵; oder -(CR¹⁸R¹⁹)_{q}R²⁶ steht;
R² für H; CF₃; Alkyl; Alkenyl; Cycloalkyl; Heterocycloalkyl; Aryl; Heteroaryl; Arylalkyl; Heteroarylalkyl; -(CR¹⁸R¹⁹)_{q}OR²⁰; -(CR¹⁸R¹⁹)_{q}SR²⁰; -(CR¹⁸R¹⁹)_{q}NR⁴R¹¹; -(CR¹⁸R¹⁹)_{q}OCONR⁴R¹¹; -(CR¹⁸R¹⁹)_{q}OCOOR²¹; -(CR¹⁸R¹⁹)_{q}NR⁴COOR²¹; -(CR¹⁸R¹⁹)_{q}NR⁴COR²²; -(CR¹⁸R¹⁹)_{q}NR⁴CONR⁴R¹¹; -(CR¹⁸R¹⁹)_{q}NR⁴SO₂R²³; -(CR¹⁸R¹⁹)_{q}NR⁴SO₂NR⁴R¹¹; -(CR¹⁸R¹⁹)_{q}COOR²¹; -(CR¹⁸R¹⁹)_{q}CONR⁴R¹¹; -(CR¹⁸R¹⁹)_{q}SO₂NR⁴R¹¹; -(CR¹⁸R¹⁹)_{q}PO(OR²¹)₂; -(CR¹⁸R¹⁹)_{q}COR²²; -(CR¹⁸R¹⁹)_{q}SO₂R²³; -(CR¹⁸R¹⁹)_{q}R²⁴; -(CR¹⁸R¹⁹)_{q}R²⁵; order -(CR¹⁸R¹⁹)_{q}R²⁶ steht;
R³ für H; CF₃; Alkyl; Cycloalkyl; Heterocycloalkyl; Aryl; Heteroaryl; Arylalkyl; oder Heteroarylalkyl steht;
R⁴ für H; Alkyl; Alkenyl; Cycloalkyl; Heterocycloalkyl; Aryl; Heteroaryl; Arylalkyl; Heteroarylalkyl; oder eine geeignete N-Schutzgruppe steht;
R⁵, R⁷ und R⁹ unabhängig definiert sind als H; F; CF₃; Alkyl; Alkenyl; Cycloalkyl; Heterocycloalkyl; Aryl; Heteroaryl; Arylalkyl; Heteroarylalkyl; -(CR¹⁸R¹⁹)_{q}OR²⁰; -(C¹⁸R¹⁹)_{q}SR²⁰; -(CR¹⁸R¹⁹)_{q}NR⁴R¹¹; -(CR¹⁸R¹⁹)_{q}OCONR⁴R¹¹; -(CR¹⁸R¹⁹)_{q}OCOOR²¹; -(CR¹⁸R¹⁹)_{q}NR⁴COOR²¹; -(CR¹⁸R¹⁹)_{q}NR⁴COR²²; -(CR¹⁸R¹⁹)_{q}NR₄CONR¹¹; -(CR¹⁸R¹⁹)_{q}NR⁴SO₂R²³; -(CR¹⁸R¹⁹)_{q}NR⁴SO₂NR⁴R¹¹; -(CR¹⁸R¹⁹)_{q}COOR²¹; -(CR¹⁸R¹⁹)_{q}CONR⁴R¹¹; -(CR¹⁸R¹⁹)_{q}SO₂NR⁴R¹¹; -(CR¹⁸R¹⁹)_{q}PO(OR²¹)₂; -(CR¹⁸R¹⁹)_{q}COR²²; -(CR¹⁸R¹⁹)_{q}SO₂R²³; -(CR¹⁸R¹⁹)_{q}R²⁴; -(CR¹⁸R¹⁹)_{q}R²⁵; order -(CR¹⁸R¹⁹)_{q}R²⁶;
R⁶, R⁸ und R¹⁰ unabhängig definiert sind als H; F; CF₃; Alkyl; Alkenyl; Cycloalkyl; Heterocycloalkyl; Aryl; Heteroaryl; Arylalkyl; oder Heteroarylalkyl;
R¹¹ für H; Alkyl; Alkenyl; Cycloalkyl; Heterocycloalkyl; Aryl; Heteroaryl; Arylalkyl; Heteroarylalkyl; eine geeignete Schutzgruppe; -(CR¹⁸R¹⁹)ᵣOR²⁰; -(CR¹⁸R¹⁹)rSR²⁰; -(CR¹⁸R¹⁹)ᵣNR⁴R²⁷; -(CR¹⁸R¹⁹)ᵣOCONR⁴R²⁷; -(CR¹⁸R"⁹)ᵣOCOOR²¹; -(CR¹⁸R¹⁹)ᵣNR⁴COOR²¹; -(CR¹⁸R¹⁹)ᵣNR⁴CONR⁴R²⁷; -(CR¹⁸R¹⁹)ᵣNR⁴SO₂R²³; -(CR¹⁸R¹⁹)ᵣNR⁴SO₂NR⁴R²⁷; -(CR¹⁸R¹⁹)_{q}COOR²¹; -(CR¹⁸R¹⁹)_{q}CONR⁴R²⁷; -(CR¹⁸R¹⁹)_{q}COR²²; -(CR¹⁸R¹⁹)_{q}SO₂R²³; -(CR¹⁸R¹⁹)_{q}SO₂NR⁴R²⁷; -(CR¹⁸R¹⁹)_{q}R²⁴; -(CR¹⁸R¹⁹)ₛR²⁵; -(CR¹⁸R¹⁹)_{q}R²⁶; order -(CR¹⁸R¹⁹)_{q}R³¹ steht;
R¹² und R¹³ unabhängig definiert sind als H; oder Alkyl;
R¹⁴ und R¹⁶ unabhängig definiert sind als H; F; CF₃; Alkyl; Alkenyl; Cycloalkyl; Heterocycloalkyl; Aryl; Heteroaryl; Arylalkyl; Heteroarylalkyl; -(CR¹⁸R¹⁹)_{q}OR²⁰; -(CR¹⁸R¹⁹)_{q}NR⁴R¹¹; -(CR¹⁸R¹⁹)_{q}NR⁴COOR²¹; -(CR¹⁸R¹⁹)_{q}NR⁴COR²²; -(CR¹⁸R¹⁹)_{q}NR⁴CONR⁴R¹¹; -(CR¹⁸R¹⁹)_{q}NR⁴SO₂R²³; - (CR¹⁸R¹⁹)_{q}NR⁴SO₂NR⁴R¹¹; -(CR¹⁸R¹⁹)_{q}COOR²¹; -(CR¹⁸R¹⁹)_{q}CONR⁴R¹¹; -(CR¹⁸R¹⁹)_{q}SO₂NR⁴R¹¹; order -(CR¹⁸R¹⁹)_{q}COR²²;
R¹⁵ und R¹⁷ unabhängig definiert sind als H; F; CF₃; Alkyl; Alkenyl; Cycloalkyl; Heterocycloalkyl; Aryl; Heteroaryl; Arylalkyl; oder Heteroarylalkyl;
R¹⁸ für H; F; CF₃; Alkyl; Alkenyl; Cycloalkyl; Heterocycloalkyl; Aryl; Heteroaryl; Arylalkyl; Heteroarylalkyl; -(CR²⁹R³⁰)ₛOR³¹; -(CR²⁹R³⁰)ₛSR³¹; -(CR²⁹R³⁰)ₛNR²⁸R³¹; -(CR²⁹R³⁰)ₛOCONR²⁸R³¹; -(CR²⁹R³⁰)ₛOCOOR²¹; -(CR²⁹R³⁰)ₛNR²⁸COOR²¹; -(CR²⁹R³⁰)ₛNR²⁸COR³¹; -(CR²⁹R³⁰)ₛNR²⁸CONR²⁸R³¹; -(CR²⁹R³⁰)ₛNR²⁸SO₂R²³; -(CR²⁹R³⁰)ₛNR²⁸SO₂NR²⁸R³¹; -(CR²⁹R³⁰)_{q}COOR²¹; -(CR²⁹R³⁰)_{q}CONR²⁸R³¹; -(CR²⁹R³⁰)_{q}SO₂NR²⁸R³¹; -(CR²⁹R³⁰)_{q}PO(OR²¹)₂; -(CR²⁹R³⁰)_{q}COR³¹; -(CR²⁹R³⁰)_{q}SO₂R²³; -(CR²⁹R³⁰)_{q}R²⁴; -(CR²⁹R³⁰)_{q}R²⁵; oder -(CR²⁹R³⁰)_{q}R²⁶ steht;
R¹⁹ für H; CF₃; Alkyl; Alkenyl; Cycloalkyl; Heterocycloalkyl; Aryl; Heteroaryl; Arylalkyl; oder Heteroarylalkyl steht;
R²⁰ für H; Alkyl; Alkenyl; Cycloalkyl; Heterocycloalkyl; Aryl; Heteroaryl; Arylalkyl; Heteroarylalkyl; -(CR²⁹R³⁰)ᵣOR³¹; -(CR²⁹R³⁰)ᵣSR³¹; -(CR²⁹R³⁰)ᵣNR²⁸R³¹; -(CR²⁹R³⁰)ᵣOCONR²⁸R³¹; -(CR²⁹R³⁰)ᵣNR²⁸COOR²¹; -(CR²⁹R³⁰)ᵣNR²⁸COR³¹; -(CR²⁹R³⁰)ᵣNR²⁸CONR²⁸R³¹; -(CR²⁹R³⁰)ᵣNR²⁸SO₂R²³; -(CR²⁹R³⁰)ᵣNR²⁸SO₂NR²⁸R³¹; -(CR²⁹R³⁰)_{q}COOR²¹; -(CR²⁹R³⁰)_{q}CONR²⁸R³¹; -(CR²⁹R³⁰)_{q}SO₂NR²⁸R³¹; -(CR²⁹R³⁰)_{q}COR³¹; -(CR²⁹R³⁰)_{q}SO₂R²³; -(CR²⁹R³⁰)_{q}R²⁴; -(CR²⁹R³⁰)_{q}R²⁵; -(CR²⁹R³⁰)_{q}R²⁶; oder -(CR²⁹R³⁰)_{q}R³¹ steht;
R²¹ für H; Alkyl; Alkenyl; Cycloalkyl; Heterocycloalkyl; Aryl; Heteroaryl; Arylalkyl; Heteroarylalkyl; oder eine geeignete *O-*Schutzgruppe steht;
R²² für Alkyl; Alkenyl; Cycloalkyl; Heterocycloalkyl; Aryl; Heteroaryl; Arylalkyl; Heteroarylalkyl; -(CR²⁹R³⁰)ₛOR³¹; -(CR²⁹R³⁰)ₛSR³¹; -(CR²⁹R³⁰)ₛNR²⁸R³¹; -(CR²⁹R³⁰)ₛOCONR²⁸R³¹; -(CR²⁹R³⁰)ₛNR²⁸COOR²¹; -(CR²⁹R³⁰)ₛNR²⁸COR³¹; -(CR²⁹R³⁰)ₛNR²⁸CONR²⁸R³¹; -(CR²⁹R³⁰)ₛNR²⁸SO₂R²³; -(CR²⁹R³⁰)ₛNR²⁸SO₂NR²⁸R³¹; -(CR²⁹R³⁰)ₛCOOR²¹; -(CR²⁹R³⁰)ₛCONR²⁸R³¹; -(CR²⁹R³⁰)ₛSO₂NR²⁸R³¹; -(CR²⁹R³⁰)ₜCOR³¹; -(CR²⁹R³⁰)ₛSO₂R²³; -(CR²⁹R³⁰)ₜR²⁴; -(CR²⁹R³⁰)ₜR²⁵; -(CR²⁹R³⁰)ₜR²⁶; oder -(CR²⁹R³⁰)ₜR³¹ steht;
R²³ für H; Alkyl; Alkenyl; Cycloalkyl; Heterocycloalkyl; Aryl; Heteroaryl; Arylalkyl; Heteroarylalkyl; oder -(CR³²R³³)ₜR²⁴ steht;
R²⁴ für Aryl; Heteroaryl; -C₆H₂R³⁴R³⁵R³¹; oder eine Gruppe aus einer der folgenden Formeln steht
| | | | |
|---|---|---|---|
| 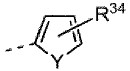 | 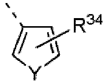 | 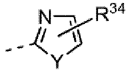 | 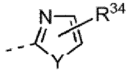 |
| **H1** | **H2** | **H3** | **H4** |
| 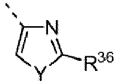 | 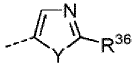 | 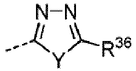 | 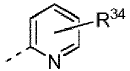 |
| **H5** | **H6** | **H7** | **H8** |
| 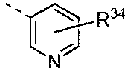 | 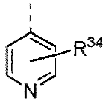 | 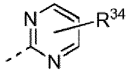 | 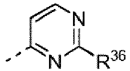 |
| **H9** | **H10** | **H11** | **H12** |
| 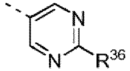 | 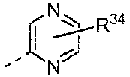 | 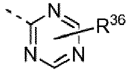 | 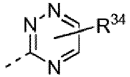 |
| **H13** | **H14** | **H15** | **H16** |
| 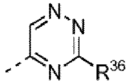 | 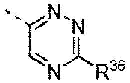 | 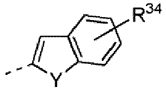 | 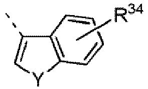 |
| **H17** | **H18** | **H19** | **H20** |
| 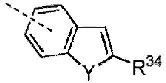 | 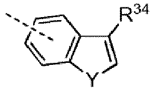 | 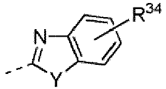 | 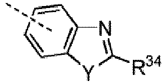 |
| **H21** | **H22** | **H23** | **H24** |
| 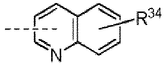 | 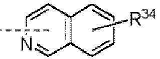 | 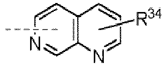 | 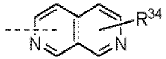 |
| **H25** | **H26** | **H27** | **H28** |
| 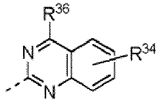 | 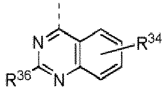 | 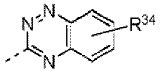 | 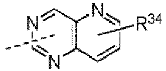 |
| **H29** | **H30** | **H31** | **H32** |
| 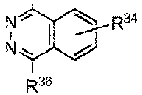 | | 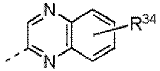 | |
| **H33** | | **H34** | |
R²⁵ für eine Gruppe aus einer der folgenden Formeln steht
| | | | |
|---|---|---|---|
| 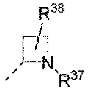 | 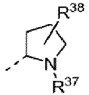 | 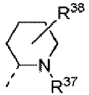 | 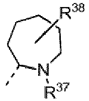 |
| **H35** | **H36** | **H37** | **H38** |
| 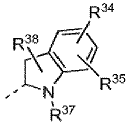 | 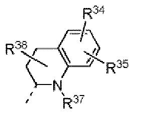 | | 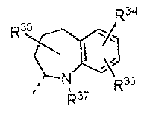 |
| **H39** | **H40** | | **H41** |
R²⁶ für eine Gruppe aus einer der folgenden Formeln steht
| | | | |
|---|---|---|---|
| 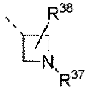 | 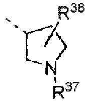 | 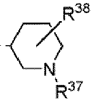 | 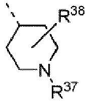 |
| **H42** | **H43** | **H44** | **H45** |
| 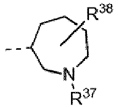 | 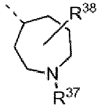 | 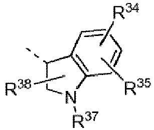 | 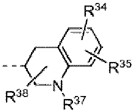 |
| **H46** | **H47** | **H48** | **H49** |
| 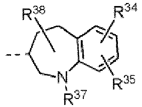 | | | |
| **H50** | | | |
R²⁷ für H; Alkyl; Niederalkenyl; Cycloalkyl; Heterocycloalkyl; Aryl; Heteroaryl; Niederarylalkyl; Niederheteroarylalkyl; eine geeignete Schutzgruppe; oder -(CR²⁹R³⁰)_{q}R²⁴ steht;
R²⁸ für H; Alkyl; Niederalkenyl; Cycloalkyl; Heterocycloalkyl; Aryl; Heteroaryl; Niederarylalkyl; Niederheteroarylalkyl; eine geeignete *N*-Schutzgruppe; -(CR³²R³³)ₛOR²¹; -(CR³²R³³)ₛNR⁴³R⁴²; - (CR³²R³³)ₛNR⁴³CONR⁴³R⁴²; -(CR³²R³³)ₛNR⁴²COR²¹; -(CR³²R³³)ₛNR⁴²SO₂NR²¹; - (CR³²R³³)_{q}COOR²¹; -(CR³²R³³)qCOR23; oder -(CR³²R³³)_{q}SO₂R²¹;
R²⁹ für H; F; CF₃; Alkyl; Alkenyl; Cycloalkyl; Heterocycloalkyl; Aryl; Heteroaryl; Arylalkyl; Heteroarylalkyl; -(CR³²R³³)ₛOR³¹; -(CR³²R³³)ₛSR³¹; -(CR³²R³³)ₛNR²⁸R³¹; -(CR³²R³³)ₛOCONR²⁸R³¹; -(CR³²R³³)ₛOCOOR²¹; -CR³²R³³)ₛNR²⁸COOR²¹; -(CR³²R³³)ₛNR²⁸COR³¹; -(CR³²R³³)ₛNR²⁸CONR²⁸R³¹; -(CR³²R³³)ₛNR²⁸SO₂R²³; -(CR³²R³³)ₛNR²⁸SO₂NR²⁸R³¹; -(CR³²R³³)_{q}COOR²¹; -(CR³²R³³)_{q}CONR²⁸R³¹; -(CR³²R³³)_{q}SO₂NR²⁸R³¹; -(CR³²R³³)_{q}PO(OR²¹)₂; -(CR³²R³³)_{q}COR³¹; -(CR³²R³³)_{q}SO₂R²³; oder -(CR³²R³³)_{q}R³¹ steht;
R³⁰ für H; F; CF₃; Alkyl; Alkenyl; Cycloalkyl; Heterocycloalkyl; Aryl; Heteroaryl; Arylalkyl; oder Heteroarylalkyl steht;
R³¹ für H; Alkyl; Alkenyl; Cycloalkyl; Heterocycloalkyl; Aryl; Heteroaryl; Arylalkyl; oder Heteroarylalkyl; oder eine Gruppe aus einer der folgenden Formeln steht
| | |
|---|---|
| 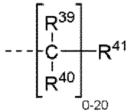 | 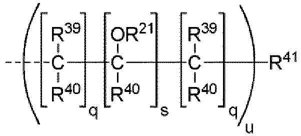 |
| **H51** | **H52** |
| | |
| 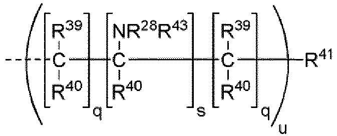 | 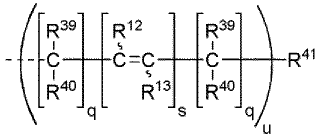 |
| **H53** | **H54** |
| 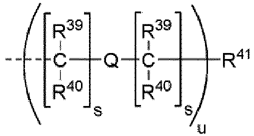 | |
| **H55** | |
R³² und R³³ unabhängig definiert sind als H; F; CF₃; Alkyl; Alkenyl; Cycloalkyl; Heterocycloalkyl; Aryl; Heteroaryl; Arylalkyl; oder Heteroarylalkyl;
R³⁴ und R³⁵ unabhängig definiert sind als H; F; Cl; CF₃; OCF₃; OCHF₂; NO₂; CN; Alkyl; Alkenyl; Alkinyl; Cycloalkyl; Heterocycloalkyl; Aryl; Heteroaryl; Arylalkyl; Heteroarylalkyl; -(CR²⁹R³⁰)_{q}OR³¹; -(CR²⁹R³⁰)_{q}SR³¹; -(CR²⁹R³⁰)_{q}NR²⁸R³¹; -(CR²⁹R³⁰)_{q}OCONR²⁸R³¹; -(CR²⁹R³⁰)_{q}NR²⁸COOR²¹; -(CR²⁹R³⁰)_{q}NR²⁸COR³¹; -(CR²⁹R³⁰)_{q}NR²⁸CONR²⁸R³¹; -(CR²⁹R³⁰)_{q}NR²⁸SO₂R²³; -(CR²⁹R³⁰)_{q}NR²⁸SO₂NR²⁸R³¹; -(CR²⁹R³⁰)_{q}COOR²¹; -(CR²⁹R³⁰)_{q}CONR²⁸R³¹; -(CR²⁹R³⁰)_{q}SO₂NR²⁸R³¹; -(CR²⁹R³⁰)_{q}COR³¹; -(CR²⁹R³⁰)_{q}SO₂R²³; order -(CR²⁹R³⁰)_{q}R³¹;
R³⁶ für H; Alkyl; Alkenyl; Alkinyl; Cycloalkyl; Heterocycloalkyl; Aryl; Heteroaryl; Arylalkyl; Heteroarylalkyl; oder -NR²⁸R³¹ steht;
R³⁷ für H; Alkyl; Alkenyl; Cycloalkyl; Heterocycloalkyl; Aryl; Heteroaryl; Arylalkyl; Heteroarylalkyl; eine geeignete *N*-Schutzgruppe; -(CR²⁹R³⁰)ᵣOR³¹; -(CR²⁹R³⁰)ᵣSR³¹; -(CR²⁹R³⁰)ᵣNR²⁸R³¹; -(CR²⁹R³⁰)ᵣOCONR²⁸R³¹; -(CR²⁹R³⁰)ᵣNR²⁸COOR²¹; -(CR²⁹R³⁰)ᵣNR²⁸COR³¹; -(CR²⁹R³⁰)ᵣNR²⁸CONR²⁸R³¹; -(CR²⁹R³⁰)ᵣNR²⁸SO₂R²³; -(CR²⁹R³⁰)ᵣNR²⁸SO₂NR²⁸R³¹; -(CR²⁹R³⁰)_{q}COOR²¹; -(CR²⁹R³⁰)_{q}CONR²⁸R³¹; -(CR²⁹R³⁰)_{q}SO₂NR²⁸R³¹; -(CR²⁹R³⁰)_{q}COR³¹; -(CR²⁹R³⁰)_{q}SO₂R²³; oder -(CR²⁹R³⁰)_{q}R³¹ steht;
R³⁸ für H; Alkyl; Alkenyl; Cycloalkyl; Heterocycloalkyl; Aryl; Heteroaryl; Arylalkyl; Heteroarylalkyl; -(CR²⁹R³⁰)_{q}OR³¹; -(CR²⁹R³⁰)_{q}SR³¹; -(CR²⁹R³⁰)_{q}NR²⁸R³¹; -(CR²⁹R³⁰)_{q}NR²⁸COOR²¹; -(CR²⁹R³⁰)_{q}NR²⁸COR³¹; -(CR²⁹R³⁰)_{q}NR²⁸CONR²⁸R³¹; -(CR²⁹R³⁰)_{q}COOR²¹; -(CR²⁹R³⁰)_{q}CONR²⁸R³¹; -(CR²⁹R³⁰)_{q}COR³¹; oder -(CR²⁹R³⁰)_{q}R³¹ steht;
R³⁹ für H; F; CF₃; Alkyl; Alkenyl; Cycloalkyl; Heterocycloalkyl; Aryl; Heteroaryl; Arylalkyl; Heteroarylalkyl; -(CR³²R³³)ᵤOR²¹; -(CR³²R³³)ᵤNR²⁸R⁴³; -(CR³²R³³)ₜCOOR²¹; oder -(CR³²R³³)ₜCONR²⁸R⁴³ steht;
R⁴⁰ für H; F; CF₃; Alkyl; Alkenyl; Cycloalkyl; Heterocycloalkyl; Aryl; Heteroaryl; Arylalkyl; Heteroarylalkyl; -(CR³²R³³)ᵤOR²¹; -(CR³²R³³)ᵤNR²⁸R⁴³; -(CR³²R³³)ₜCOOR²¹; oder -(CR³²R³³)ₜCONR²⁸R⁴³ steht;
R⁴¹ für H; CF₃; Alkyl; Alkenyl; Cycloalkyl; Heterocycloalkyl; Aryl; Heteroaryl; Arylalkyl; Heteroarylalkyl; -OR²¹; -NR²⁸R⁴³; -NR²⁸COR²³; -NR²⁸COOR²¹; -NR²⁸SO₂R²³; -NR²⁸CONR²⁸R⁴³; -COOR²¹; -CONR²⁸R⁴³; -C(=NR⁴³)NR²⁸R⁴³; -NR²⁸C(=NR⁴³)NR²⁸R⁴³; oder eine Gruppe aus einer der folgenden Formeln steht
| | | | |
|---|---|---|---|
| 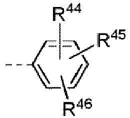 | 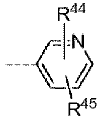 | 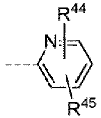 | 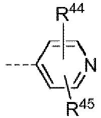 |
| **H56** | **H57** | **H58** | **H59** |
| 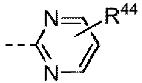 | 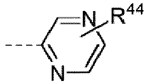 | 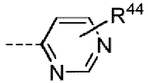 | 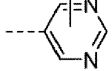 |
| **H60** | **H61** | **H62** | **H63** |
| 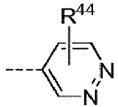 | 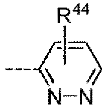 | 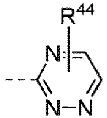 | 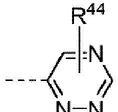 |
| **H64** | **H65** | **H66** | **H67** |
| 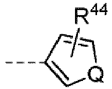 | 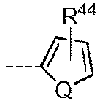 | 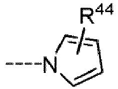 | 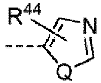 |
| **H68** | **H69** | **H70** | **H71** |
| 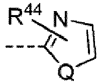 | 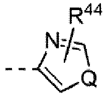 | 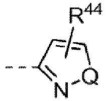 | 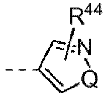 |
| **H72** | **H73** | **H74** | **H75** |
| 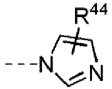 | 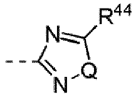 | 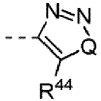 | 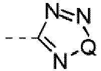 |
| **H76** | **H77** | **H78** | **H79** |
| 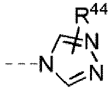 | 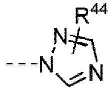 | 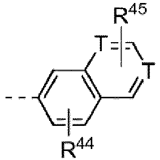 | 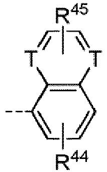 |
| **H80** | **H81** | **H82** | **H83** |
| 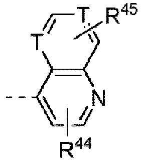 | 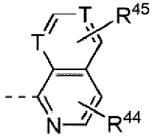 | 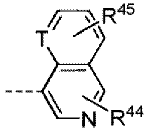 | 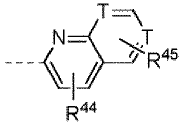 |
| **H84** | **H85** | **H86** | **H87** |
| 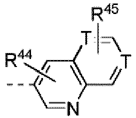 | 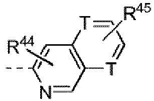 | 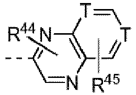 | 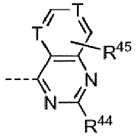 |
| **H88** | **H89** | **H90** | **H91** |
| 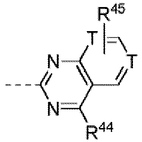 | 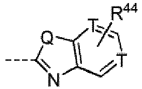 | 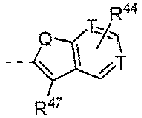 | 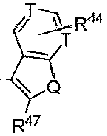 |
| **H92** | **H93** | **H94** | **H95** |
| 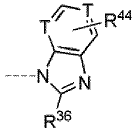 | 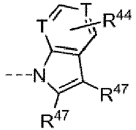 | 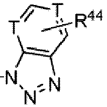 | 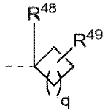 |
| **H96** | **H97** | **H98** | **H99** |
| 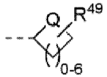 | 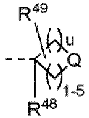 | 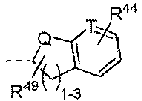 | 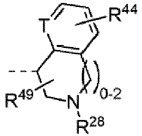 |
| **H100** | **H101** | **H102** | **H103** |
| 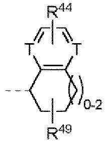 | 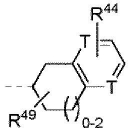 | 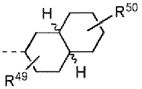 | 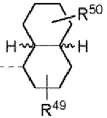 |
| **H104** | **H105** | **H106** | **H107** |
| 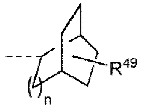 | 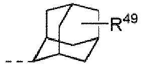 | | 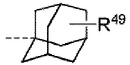 |
| **H148** | **H109** | | **H110** |
R⁴² für H; Alkyl; Alkenyl; Cycloalkyl; Cycloheteroalkyl; Aryl; Heteroaryl; -(CR²³R³³)ₛOR²¹; -(CR²³R³³)ₛNR⁴R⁴³; -(CR²³R³³)_{q}COOR²¹; oder -(CR²³R³³)_{q}CONR⁴R⁴³ steht;
R⁴³ für H; Alkyl; Alkenyl; Cycloalkyl; Heterocycloalkyl; Aryl; Heteroaryl; Arylalkyl; Heteroarylalkyl; oder eine geeignete N-Schutzgruppe steht;
R⁴⁴, R⁴⁵ und R⁴⁶ unabhängig definiert sind als H; F; CF₃; OCF₃; OCHF₂; NO₂; CN; Alkyl; Alkenyl; Alkinyl; Cycloalkyl; Heterocycloalkyl; Aryl; Heteroaryl; Arylalkyl; Heteroarylalkyl; - OR²³; -NR²⁸R⁴³; -NR²⁸COR²³; -NR²⁸SO₂R²³; -NR²⁸CONR²⁸R⁴³; -COR²³; oder -SO₂R²³;
R⁴⁷ für H; CF₃; Alkyl; Alkenyl; Alkinyl; Cycloalkyl; Heterocycloalkyl; Aryl; Heteroaryl; Arylalkyl; Heteroarylalkyl;-COOR²¹; oder -CONR²⁸R⁴³ steht;
R⁴⁸ für H; F; CF₃; Alkyl; Alkenyl; Cycloalkyl; Cycloheteroalkyl; Aryl; Heteroaryl; -(CR²³R³³)ₜOR²¹; -(CR²³R³³)ₜNR²⁸R⁴³; -(CR²³R³³)ₜCOOR²¹; oder -(CR²³R³³)ₜCONR²¹R⁴³ steht;
R⁴⁹ und R⁵⁰ unabhängig definiert sind als H; F; CF₃; Alkyl; Alkenyl; Cycloalkyl; Heterocycloalkyl; Aryl; Heteroaryl; Arylalky; Heteroarylalkyl; ₋(CR³²R³³)_{q}OR²¹; -(CR³²R³³)_{q}NR²⁸R⁴³; -(CR³²R³³)_{q}COOR²¹; oder -(CR³²R³³)_{q}CONR²⁸R⁴³;
R⁵¹ für H; F; Cl; CF₃; OCF₃; oder Niederalkyl steht;
R⁵² für H; F; Cl; CF₃; OCF₃; OCHF₂; NO₂; CN; Niederalkyl; Niederalkenyl; Niederalkinyl; Niedercycloalkyl; Niederheterocycloalkyl; Aryl; Heteroaryl; Niederarylalkyl; oder Niederheteroarylalkyl steht;
R⁵³ für CF₃; OCF₃; CN; Niederalkyl; Niedercycloalkyl; Aryl; Niederalkoxy; oder Aryloxy steht;
(R⁴ und R¹¹); (R⁴ und R²⁷); (R⁵ und R⁶); (R⁵ und R⁷); (R⁵ und R⁹); (R⁵ und R14); (R5 und R16); (R7 und R8); (R7 und R⁹); (R7 und R16); (R⁹ und R¹⁰); (R¹⁴ und R¹⁵); (R¹⁶ und R¹⁷); (R¹⁸ und R¹⁹); (R²⁷ und R²⁸); (R²⁸ und R³¹); (R²⁸ und R⁴³); (R²⁹ und R³⁰); (R³² und R³³); (R³⁴ und R³⁵); (R³⁷ und R³⁸); (R³⁹ und R⁴⁰); (R³⁹ und R⁴¹); (R³⁹ und R⁴⁹); (R⁴² und R⁴³); (R⁴⁴ und R⁴⁵); oder (R⁴⁴ und R⁴⁶) zusammengenommen gegebenenfalls substituierte Cycloalkyl- oder Heterocycloalkylreste bilden können;
darüber hinaus können die strukturellen Elemente -NR⁴R¹¹; -NR¹¹R²⁷; - NR²⁷R²⁸; -NR²⁸R³¹ oder -NR²⁸R⁴³ eine der Gruppen aus den folgenden Formeln bilden
| | | | |
|---|---|---|---|
| 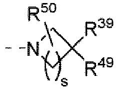 | 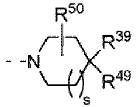 | 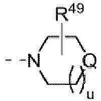 | 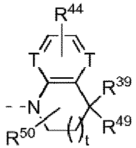 |
| **H111** | **H112** | **H113** | **H114** |
| 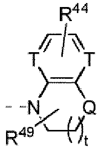 | 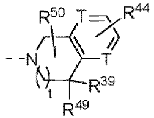 | 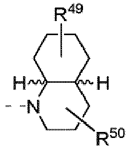 | 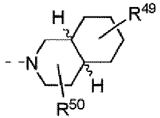 |
| **H115** | **H116** | **H117** | **H118** |
Z für O; S; S(=O); S(=O)₂; oder NR²⁸ steht;
Y für O; S; oder NR⁴ steht;
Q für O; S; oder NR²⁸ steht;
T für CR⁴⁶ oder N steht; falls T mehrmals in derselben Ringstruktur vorkommt, ist jedes T unabhängig voneinander definiert;
q für eine ganze Zahl von 0-4 steht;
r für eine ganze Zahl von 2-4 steht;
s für eine ganze Zahl von 1-4 steht;
t für eine ganze Zahl von 0-2 steht;
u für eine ganze Zahl von 1-2 steht;
und alle möglichen Stereoisomere solcher Verbindungen;
oder Salze, Solvate, Clathrate, N-Oxide, isotopisch angereicherte oder enantiomer angereicherte Versionen davon.

2. Verbindungen nach Anspruch 1, wobei
die Basiskomponente vom Typ **A** ausgewählt ist aus **A627** bis **A683;**
die Basiskomponente vom Typ **B** ausgewählt ist aus **B1** bis **B21;**
die Basiskomponente vom Typ **C** ausgewählt ist aus **C1** bis **C101;** und wobei
M für -N(R⁴)- steht;
und alle möglichen Stereoisomere solcher Verbindungen;
oder pharmazeutisch unbedenkliche Salze davon.

3. Verbindungen nach Anspruch 1, wobei
die Basiskomponente vom Typ **A** ausgewählt ist aus
**A1**(a1); **A2**(a1); **A3**(a1); **A4**(a1); **A5**(a1); **A6**(a1); **A7**(a1); **A9**(a1); **A10**(a1); **A73**(a2); **A80**(a2); **A170**(a4); **A171**(a4); **A209**(a7); **A210**(a7); **A240**(a10); **A241**(a10); **A242**(a10); **A243**(a10); **A272**(a10); **A530**(a18); **A531**(a18); **A532** (a18); **A533**(a18); **A609**(a24); **A610**(a24); **A611**(a24); **A612**(a24); **A613**(a24); **A619**(a24); **A615**(a24); **A631**(a1); **A632** (a1); **A633**(a1); **A641** (a1); **A642** (a1); **A643** (a1); **A651** (a1); **A652**(a1); oder **A653**(a1);
die Basiskomponente vom Typ **B** ausgewählt ist aus
**B4**(b3); **B5**(b3); **B6**(b3); **B7**(b3); **B8**(b3); **B9**(b3); **B10**(b3); **B12**(b4); **B13**(b4); **B14**(b4); **B16**(b4) oder **B17**(b5) ;
die Basiskomponente vom Typ **C** ausgewählt ist aus
**C1**; **C2; C3; C4; C5; C6; C7; C8; C9; C10; C11; C12; C13; C14; C15; C16; C17; C18; C19; C20; C21; C25; C26; C27; C28; C29; C30; C34; C35; C36; C37; C38; C39; C43; C44; C45; C46; C47; C48; C49; C50; C51; C52; C53; C54; C55; C56; C57; C58; C59; C60; C61; C62; C63; C64; C65; C66; C67; C68; C69; C70; C71; C72; C73; C74; C75; C76; C77; C78; C79; C80; C81; C86; C87; C88; C89; C90; C91; C92; oder C93;**
und wobei
R¹ für H; F; Cl; Br; I; CF₃; OCF₃; OCHF₂; NO₂; CN; Niederalkyl; Niederalkenyl; Niederalkinyl; Niedercycloalkyl; Niederheterocycloalkyl; Aryl; Heteroaryl; Niederarylalkyl; Niederheteroarylalkyl; -(CR¹⁸R¹⁹)_{q}OR²⁰; -(CR¹⁸R¹⁹)_{q}NR⁴R¹¹; -(CR¹⁸R¹⁹)_{q}NR⁴COOR²¹; -(CR¹⁸R¹⁹)_{q}NR⁴COR²²; -(CR¹⁸R¹⁹)_{q}NR⁴CONR⁴R¹¹; -(CR¹⁸R¹⁹)_{q}NR⁴SO₂R²³; -(CR¹⁸R¹⁹)_{q}NR⁴SO₂NR⁴R¹¹; -(CR¹⁸R¹⁹)_{q}COOR²¹; -(CR¹⁸R¹⁹)_{q}CONR⁴R¹¹; -(CR¹⁸R¹⁹)_{q}SO₂NR⁴R¹¹; -(CR¹⁸R¹⁹)_{q}PO(OR²¹)₂; -(CR¹⁸R¹⁹)_{q}COR²²; -(CR¹⁸R¹⁹)_{q}SO₂R²³; -(CR¹⁸R¹⁹)_{q}OSO₃R²¹; -(CR¹⁸R¹⁹)_{q}R²⁴; -(CR¹⁸R¹⁹)_{q}R²⁵; order -(CR¹⁸R¹⁹)_{q}R²⁶ steht;
R² für H; CF₃; Niederalkyl; Niederalkenyl; Cycloalkyl; Heterocycloalkyl; Aryl; Heteroaryl; Niederarylalkyl; Niederheteroarylalkyl; -(CR¹⁸R¹⁹)_{q}OR²⁰; -(CR¹⁸R¹⁹)_{q}NR⁴R¹¹; -(CR¹⁸R¹⁹)_{q}NR⁴COOR²¹; -(CR¹⁸R¹⁹)_{q}NR⁴COR²²; -(CR¹⁸R¹⁹)_{q}NR⁴CONR⁴R¹¹; -(CR¹⁸R¹⁹)_{q}NR⁴SO₂R²³; -(CR¹⁸R¹⁹)_{q}NR⁴SO₂NR⁴R¹¹; -(CR¹⁸R¹⁹)_{q}COOR²¹; -(CR¹⁸R¹⁹)_{q}CONR⁴R¹¹; -(CR¹⁸R¹⁹)_{q}SO₂NR⁴R¹¹; -(CR¹⁸R¹⁹)_{q}PO(OR²¹)₂; -(CR¹⁸R¹⁹)_{q}COR²²; -(CR¹⁸R¹⁹)_{q}SO₂R²³; -(CR¹⁸R¹⁹)_{q}R²⁴; -(CR¹⁸R¹⁹)_{q}R²⁵; oder -(CR¹⁸R¹⁹)_{q}R²⁶ steht;
R⁴ für H; Niederalkyl; Niederalkenyl; Niedercycloalkyl; Niederheterocycloalkyl; oder eine geeignete N- Schutzgruppe steht;
R⁵, R⁷ und R⁹ unabhängig definiert sind als H; F; CF₃; Niederalkyl; Niederalkenyl; Niedercycloalkyl; Niederheterocycloalkyl; Aryl; Heteroaryl; Niederarylalkyl; Niederheteroarylalkyl; -(CR¹⁸R¹⁹)_{q}OR²⁰; -(CR¹⁸R¹⁹)_{q}NR⁴R¹¹; -(CR¹⁸R¹⁹)_{q}NR⁴COOR²¹; -(CR¹⁸R¹⁹)_{q}NR⁴COR²²; -(CR¹⁸R¹⁹)_{q}NR₄CONR⁴R¹¹; -(CR¹⁸R¹⁹)_{q}NR⁴SO₂R²³; -(CR¹⁸R¹⁹)_{q}NR⁴SO₂NR⁴R¹¹; -(CR¹⁸R¹⁹)_{q}COOR²¹; -(CR¹⁸R¹⁹)_{q}CONR⁴R¹¹; -(CR¹⁸R¹⁹)_{q}SO₂NR⁴R¹¹; -(CR¹⁸R¹⁹)_{q}PO(OR²¹)₂; -(CR¹⁸R¹⁹)_{q}COR²²; -(CR¹⁸R¹⁹)_{q}SO₂R²³; -(CR¹⁸R¹⁹)_{q}R²⁴; -(CR¹⁸R¹⁹)_{q}R²⁵; oder -(CR¹⁸R¹⁹)_{q}R²⁶;
R⁶, R⁸ und R¹⁰ unabhängig definiert sind als H; CF₃; oder Niederalkyl;
R¹¹ für H; Alkyl; Alkenyl; Cycloalkyl; Niederheterocycloalkyl; Aryl; Heteroaryl; Niederarylalkyl; Niederheteroarylalkyl; eine geeignete Schutzgruppe; -(CR¹⁸R¹⁹)ᵣOR²⁰; -(CR¹⁸R¹⁹)ᵣNR⁴R²⁷; -(CR¹⁸R¹⁹)ᵣNR⁴COOR²¹; -(CR¹⁸R¹⁹)ᵣNR⁴CONR⁴R²⁷; -(CR¹⁸R¹⁹)ᵣNR⁴SO₂R²³; -(CR¹⁸R¹⁹)ᵣNR⁴SO₂NR⁴R²⁷; -(CR¹⁸R¹⁹)_{q}COOR²¹; -(CR¹⁸R¹⁹)_{q}CONR⁴R²⁷; -(CR¹⁸R¹⁹)_{q}COR²²; -(CR¹⁸R¹⁹)_{q}SO₂R²³; -(CR¹⁸R¹⁹)_{q}SO₂NR⁴R²⁷; -(CR¹⁸R¹⁹)_{q}R²⁴; -(CR¹⁸R¹⁹)ₛR²⁵; -(CR¹⁸R¹⁹)_{q}R²⁶; oder -(CR¹⁸R¹⁹)_{q}R³¹ steht;
R¹² und R¹³ unabhängig definiert sind als H; oder Niederalkyl;
R¹⁴ und R¹⁶ unabhängig definiert sind als H; F; CF₃; Niederalkyl; Niederalkenyl; Niedercycloalkyl; Niederheterocycloalkyl; Aryl; Heteroaryl; Niederarylalkyl; Niederheteroarylalkyl; -(CR¹⁸R¹⁹)_{q}OR²⁰; -(CR¹⁸R¹⁹)_{q}NR⁴R¹¹; -(CR¹⁸R¹⁹)_{q}NR⁴COOR²¹; -(CR¹⁸R¹⁹)_{q}NR⁴COR²²; -(CR¹⁸R¹⁹)_{q}NR⁴CONR⁴R¹¹; (CR¹⁸R¹⁹)_{q}NR⁴SO₂R²³; -(CR¹⁸R¹⁹)_{q}COOR²¹; -(CR¹⁸R¹⁹)_{q}CONR⁴R¹¹; order -(CR¹⁸R¹⁹)_{q}COR²²;
R¹⁵ und R¹⁷ unabhängig definiert sind als H; CF₃; oder Niederalkyl;
R¹⁸ für H; F; CF₃; Niederalkyl; Niederalkenyl; Niedercycloalkyl; Niederheterocycloalkyl; Aryl; Heteroaryl; Niederarylalkyl; Niederheteroarylalkyl; -(CR²⁹R³⁰)ₛOR³¹; -(CR²⁹R³⁰)ₛNR²⁸R³¹; -(CR²⁹R³⁰)ₛNR²⁸COOR²¹; -(CR²⁹R³⁰)ₛNR²⁸COR³¹; -(CR²⁹R³⁰)ₛNR²⁸CONR²⁸R³¹; -(CR²⁹R³⁰)ₛNR²⁸SO₂R²³; -(CR²⁹R³⁰)ₛNR²⁸SO₂NR²⁸R³¹; -(CR²⁹R³⁰)_{q}COOR²¹; -(CR²⁹R³⁰)_{q}CONR²⁸R³¹; -(CR²⁹R³⁰)_{q}SO₂NR²⁸R³¹; -(CR²⁹R³⁰)_{q}PO(OR²¹)₂; -(CR²⁹R³⁰)_{q}COR³¹; -(CR²⁹R³⁰)_{q}SO₂R²³; -(CR²⁹R³⁰)_{q}R²⁴; -(CR²⁹R³⁰)_{q}R²⁵; oder -(CR²⁹R³⁰)_{q}R²⁶ steht;
R¹⁹ für H; CF₃; oder Niederalkyl steht;
R²⁰ für H; Niederalkyl; Niederalkenyl; Niedercycloalkyl; Niederheterocycloalkyl; Aryl; Heteroaryl; Niederarylalkyl; Niederheteroarylalkyl; -(CR²⁹R³⁰)ᵣOR³¹; -(CR²⁹R³⁰)ᵣNR²⁸R³¹; -(CR²⁹R³⁰)ᵣNR²⁸COOR²¹; -(CR²⁹R³⁰)ᵣNR²⁸COR³¹; -(CR²⁹R³⁰)ᵣNR²⁸CONR²⁸R³¹; -(CR²⁹R³⁰)ᵣNR²⁸SO₂R²³; -(CR²⁹R³⁰)_{q}COOR²¹; -(CR²⁹R³⁰)_{q}CONR²⁸R³¹; -(CR²⁹R³⁰)_{q}SO₂NR²⁸R³¹; -(CR²⁹R³⁰)_{q}COR³¹; -(CR²⁹R³⁰)_{q}SO₂R²³; -(CR²⁹R³⁰)_{q}R²⁴; -(CR²⁹R³⁰)_{q}R²⁵; -(CR²⁹R³⁰)_{q}R²⁶; oder -(CR²⁹R³⁰)_{q}R³¹ steht;
R²² für Niederalkyl; Niederalkenyl; Niedercycloalkyl; Niederheterocycloalkyl; Aryl; Heteroaryl; Niederarylalkyl; Niederheteroarylalkyl; -(CR²⁹R³⁰)ₛOR³¹; -(CR²⁹R³⁰)ₛNR²⁸R³¹; -(CR²⁹R³⁰)ₛNR²⁸COOR²¹; -(CR²⁹R³⁰)ₛNR²⁸COR³¹; -(CR²⁹R³⁰)ₛNR²⁸CONR²⁸R³¹; -(CR²⁹R³⁰)ₛNR²⁸SO₂R²³; -(CR²⁹R³⁰)ₛCOOR²¹; -(CR²⁹R³⁰)ₛCONR²⁸R³¹; -(CR²⁹R³⁰)ₛSO₂NR²⁸R³¹; -(CR²⁹R³⁰)ₜCOR³¹; -(CR²⁹R³⁰)ₛSO₂R²³; -(CR²⁹R³⁰)ₜR²⁴; -(CR²⁹R³⁰)ₜR²⁵; -(CR²⁹R³⁰)ₜR²⁶; oder -(CR²⁹R³⁰)ₜR³¹ steht;
R²⁹ für H; F; CF₃; Niederalkyl; Niederalkenyl; Niedercycloalkyl; Niederheterocycloalkyl; Aryl; Heteroaryl; Niederarylalkyl; Niederheteroarylalkyl; -(CR³²R³³)OR³¹; -(CR³²R³³)ₛNR²⁸R³¹; -(CR³²R³³)ₛNR²⁸COOR²¹; -(CR³²R³³)ₛNR²⁸COR³¹; -(CR³²R³³)ₛNR²⁸CONR²⁸R³¹; -(CR³²R³³)ₛNR²⁸SO₂R²³; -(CR³²R³³)_{q}COOR²¹; -(CR³²R³³)_{q}CONR²⁸R³¹; -(CR³²R³³)_{q}SO₂NR²⁸R³¹; -(CR³²R³³)_{q}PO(OR²¹)₂; -(CR³²R³³)_{q}COR³¹; -(CR³²R³³)_{q}SO₂R²³; oder -(CR³²R³³)_{q}R³¹ steht;
R³⁰ Und R³³ unabhängig definiert sind als H; CF₃; oder Niederalkyl;
R³⁴ und R³⁵ unabhängig definiert sind als H; F; Cl; CF₃; OCF₃; OCHF₂; NO₂; CN; Niederalkyl; Niederalkenyl; Niederalkinyl; Niedercycloalkyl; Niederheterocycloalkyl; Aryl; Heteroaryl; Niederarylalkyl; Niederheteroarylalkyl; -(CR²⁹R³⁰)_{q}OR³¹; -(CR²⁹R³⁰)_{q}NR²⁸R³¹; -(CR²⁹R³⁰)qNR²⁸COOR²¹; -(CR²⁹R³⁰)_{q}NR²⁸COR³¹; -(CR²⁹R³⁰)_{q}NR²⁸CONR²⁸R³¹; -(CR²⁹R³⁰)_{q}NR²⁸SO₂R²³; -(CR²⁹R³⁰)_{q}COOR²¹; -(CR²⁹R³⁰)_{q}CONR²⁸R³¹; -(CR²⁹R³⁰)_{q}SO₂NR²⁸R³¹; -(CR²⁹R³⁰)_{q}COR³¹; -(CR²⁹R³⁰)_{q}SO₂R²³; oder -(CR²⁹R³⁰)_{q}R³¹;
R³⁷ für H; Niederalkyl; Niederalkenyl; Niedercycloalkyl; Niederheterocycloalkyl; Aryl; Heteroaryl; Niederarylalkyl; Niederheteroarylalkyl; eine geeignete *N*-Schutzgruppe; -(CR²⁹R³⁰)ᵣOR³¹; -(CR²⁹R³⁰)ᵣNR²⁸R³¹; -(CR²⁹R³⁰)ᵣNR²⁸COOR²¹; -(CR²⁹R³⁰)ᵣNR²⁸COR³¹; -(CR²⁹R³⁰)ᵣNR²⁸CONR²⁸R³¹; -(CR²⁹R³⁰)ᵣNR²⁸SO₂R²³; -(CR²⁹R³⁰)_{q}COOR²¹; -(CR²⁹R³⁰)_{q}CONR²⁸R³¹; (CR²⁹R³⁰)_{q}SO₂NR²⁸R³¹; -(CR²⁹R³⁰)_{q}COR³¹; -(CR²⁹R³⁰)_{q}SO₂R²³;
oder -(CR²⁹R³⁰)_{q}R³¹ steht;
R³⁸ für H; Niederalkyl; Niederalkenyl; Niedercycloalkyl; Niederheterocycloalkyl; Aryl; Heteroaryl; Niederarylalkyl; Niederheteroarylalkyl; -(CR²⁹R³⁰)_{q}OR³¹; -(CR²⁹R³⁰)_{q}NR²⁸R³¹; -(CR²⁹R³⁰)_{q}NR²⁸COOR²¹; -(CR²⁹R³⁰)_{q}NR²⁸COR³¹; -(CR²⁹R³⁰)_{q}NR²⁸CONR²⁸R³¹; -(CR²⁹R³⁰)_{q}COOR²¹; -(CR²⁹R³⁰)_{q}CONR²⁸R³¹; -(CR²⁹R³⁰)_{q}COR³¹; oder -(CR²⁹R³⁰)_{q}R³¹ steht;
Z für O; S; S(=O); oder S(=O)₂ steht;
U für -C(=O)-; -NR⁴-C(=O)-; -C(=O)-C(=O)-; oder -C(-OR²⁰)₂-C(=O)- steht.

4. Verbindungen nach Anspruch 1, wobei
die Basiskomponente vom Typ **A** ausgewählt ist aus
**A1**(a1); **A2**(a1); **A3**(a1); **A4**(a1); **A5**(a1); **A6**(a1); **A7**(a1); **A9**(a1); **A10**(a1); **A73**(a2); **A80**(a2); **A170**(a4); **A209**(a7); **A240**(a10); **A272**(a10); **A532** (a18); **A614**(a24); **A631**(a1); **A632** (a1); **A633**(a1); **A691** (a1); **A642** (a1); **A643** (a1); **A651** (a1); **A652**(a1); oder **A653**(a1);
die Basiskomponente vom Typ **B** ausgewählt ist aus
**B7**(b3); **B8**(b3); **B9**(b3**); B10**(b3); oder **B17**(b5);
die Basiskomponente vom Typ **C** ausgewählt ist aus
**C1; C2; C4; C7; C8; C9; C10; C11; C12; C13; C14; C15; C16; C17; C18; C19; C20; C21; C25; C26; C27; C28; C29; C30; C34; C35; C36; C37; C38; C39; C43; C44; C45; C46; C47; C48; C49; C90; C91; C92;** oder **C93;**
und wobei
R¹ für H; F; Cl; CF₃; OCF₃; OCHF₂; NO₂; CN; Niederalkyl; Niederalkenyl; Niederalkinyl; Niedercycloalkyl; Niederheterocycloalkyl; Aryl; Heteroaryl; Niederarylalkyl; Niederheteroarylalkyl; -(CR¹⁸R¹⁹)_{q}OR²⁰; -(CR¹⁸R¹⁹)_{q}NR⁴R¹¹; -(CR¹⁸R¹⁹)_{q}NR⁴COR²²; -(CR¹⁸R¹⁹)_{q}NR⁴CONR⁴R¹¹; -(CR¹⁸R¹⁹)_{q}NR⁴SO₂R²³; -(CR¹⁸R¹⁹)_{q}NR⁴SO₂NR⁴R¹¹; -(CR¹⁸R¹⁹)_{q}COOR²¹; -(CR¹⁸R¹⁹)_{q}CONR⁴R¹¹; -(CR¹⁸R¹⁹)_{q}SO₂NR⁴R¹¹; -(CR¹⁸R¹⁹)_{q}COR²²; -(CR¹⁸R¹⁹)_{q}SO₂R²³; -(CR¹⁸R¹⁹)_{q}R²⁴; -(CR¹⁸R¹⁹)_{q}R²⁵; order -(CR¹⁸R¹⁹)_{q}R²⁶ steht;
R² für H; CF₃; Niederalkyl; Niederalkenyl; Cycloalkyl; Heterocycloalkyl; Aryl; Heteroaryl; Niederarylalkyl; Niederheteroarylalkyl; -(CR¹⁸R¹⁹)_{q}OR²⁰; -(CR¹⁸R¹⁹)_{q}NR⁴R¹¹; -(CR¹⁸R¹⁹)_{q}NR⁴COR²²; -(CR¹⁸R¹⁹)_{q}NR⁴CONR⁴R¹¹; -(CR¹⁸R¹⁹)_{q}NR⁴SO₂R²³; -(CR¹⁸R¹⁹)_{q}NR⁴SO₂NR⁴R¹¹; -(CR¹⁸R¹⁹)_{q}COOR²¹; -(CR¹⁸R¹⁹)_{q}CONR⁴R¹¹; -(CR¹⁸R¹⁹)_{q}SO₂NR⁴R¹¹; -(CR¹⁸R¹⁹)_{q}COR²²; -(CR¹⁸R¹⁹)_{q}SO₂R²³; -(CR¹⁸R¹⁹)_{q}R²⁴; -(CR¹⁸R¹⁹)_{q}R²⁵; order -(CR¹⁸R¹⁹)_{q}R²⁶ steht;
R⁴ für H; Niederalkyl; Niederalkenyl; oder eine geeignete N-Schutzgruppe steht;
R⁵, R⁷ und R⁹ unabhängig definiert sind als H; CF₃; Niederalkyl; Niederalkenyl; Niedercycloalkyl; Niederheterocycloalkyl; Aryl; Heteroaryl; Niederarylalkyl; Niederheteroarylalkyl; -(CR¹⁸R¹⁹)_{q}OR²⁰; -(CR¹⁸R¹⁹)_{q}NR⁴R¹¹; -(CR¹⁸R¹⁹)_{q}NR⁴COR²²; -(CR¹⁸R¹⁹)_{q}NR₄CONR⁴R¹¹; -(CR¹⁸R¹⁹)_{q}NR⁴SO₂R²³; -(CR¹⁸R¹⁹)_{q}COOR²¹; -(CR¹⁸R¹⁹)_{q}CONR⁴R¹¹; -(CR¹⁸R¹⁹)_{q}SO₂NR⁴R¹¹; -(CR¹⁸R¹⁹)_{q}COR²²; -(CR¹⁸R¹⁹)_{q}SO₂R²³; -(CR¹⁸R¹⁹)_{q}R²⁴; -(CR¹⁸R¹⁹)_{q}R²⁵; oder -(CR¹⁸R¹⁹)_{q}R²⁶;
R⁶, R⁸ und R¹⁰ unabhängig definiert sind als H; CF₃; oder CH₃;
R¹¹ für H; Alkyl; Alkenyl; Cycloalkyl; Niederheterocycloalkyl; Aryl; Heteroaryl; Niederarylalkyl; Niederheteroarylalkyl; eine geeignete Schutzgruppe; -(CR¹⁸R¹⁹)ᵣOR²⁰; -(CR¹⁸R¹⁹)ᵣNR⁴R²⁷; - (CR¹⁸R¹⁹)ᵣNR⁴CONR⁴R²⁷; -(CR¹⁸R¹⁹)ᵣNR⁴SO₂R²³; -(CR¹⁸R¹⁹)_{q}COOR²¹; -(CR¹⁸R¹⁹)_{q}CONR⁴R²⁷; -(CR¹⁸R¹⁹)_{q}COR²²; -(CR¹⁸R¹⁹)_{q}R²⁴; -(CR¹⁸R¹⁹)ₛR²⁵; -(CR¹⁸R¹⁹)_{q}R²⁶; oder -(CR¹⁸R¹⁹)_{q}R³¹ steht;
R¹² und R¹³ unabhängig definiert sind als H; oder Niederalkyl;
R¹⁴ und R¹⁶ unabhängig definiert sind als H; F; CF₃; Niederalkyl; Niederalkenyl; Niedercycloalkyl; Niederheterocycloalkyl; Aryl; Heteroaryl; Niederarylalkyl; Niederheteroarylalkyl; -(CR¹⁸R¹⁹)_{q}OR²⁰; -(CR¹⁸R¹⁹)_{q}NR⁴R¹¹; -(CR¹⁸R¹⁹)_{q}NR⁴COR²²; -(CR¹⁸R¹⁹)_{q}COOR²¹; oder - (CR¹⁸R¹⁹)_{q}CONR⁴R¹¹;
R¹⁵ und R¹⁷ unabhängig definiert sind als H; CF₃; oder CH₃;
R¹⁸ für H; F; CF₃; Niederalkyl; Niederalkenyl; Niedercycloalkyl; Niederheterocycloalkyl; Aryl; Heteroaryl; Niederarylalkyl; Niederheteroarylalkyl; -(CR²⁹R³⁰)ₛOR³¹; -(CR²⁹R³⁰)ₛNR²⁸R³¹; -(CR²⁹R³⁰)ₛNR²⁸COR³¹; -(CR²⁹R³⁰)ₛNR²⁸CONR²⁸R³¹; -(CR²⁹R³⁰)ₛNR²⁸SO₂R²³; -(CR²⁹R³⁰)_{q}COOR²¹; -(CR²⁹R³⁰)_{q}CONR²⁸R³¹; -(CR²⁹R³⁰)_{q}SO₂NR²⁸R³¹; -(CR²⁹R³⁰)_{q}COR³¹; -(CR²⁹R³⁰)_{q}SO₂R²³; -(CR²⁹R³⁰)_{q}R²⁴; -(CR²⁹R³⁰)_{q}R²⁵; oder -(CR²⁹R³⁰)_{q}R²⁶ steht;
R¹⁹ für H; CF₃; oder CH₃ steht;
R²⁰ für H; Niederalkyl; Niederalkenyl; Niedercycloalkyl; Niederheterocycloalkyl; Aryl; Heteroaryl; Niederarylalkyl; Niederheteroarylalkyl; -(CR²⁹R³⁰)ᵣOR³¹; -(CR²⁹R³⁰)ᵣNR²⁸R³¹; -(CR²⁹R³⁰)ᵣNR²⁸COR³¹; -(CR²⁹R³⁰)ᵣNR²⁸CONR²⁸R³¹; -(CR²⁹R³⁰)ᵣNR²⁸SO₂R²³; -(CR²⁹R³⁰)_{q}COOR²¹; -(CR²⁹R³⁰)_{q}CONR²⁸R³¹; -(CR²⁹R³⁰)_{q}SO₂NR²⁸R³¹; -(CR²⁹R³⁰)_{q}COR³¹; -(CR²⁹R³⁰)_{q}SO₂R²³; -(CR²⁹R³⁰)_{q}R²⁴; -(CR²⁹R³⁰)_{q}R²⁵; -(CR²⁹R³⁰)_{q}R²⁶; oder (CR²⁹R³⁰)_{q}R³¹ steht;
R²² für Niederalkyl; Niederalkenyl; Niedercycloalkyl; Niederheterocycloalkyl; Aryl; Heteroaryl; Niederarylalkyl; Niederheteroarylalkyl; -(CR²⁹R³⁰)ₛOR³¹; -(CR²⁹R³⁰)ₛNR²⁸R³¹; -(CR²⁹R³⁰)ₛNR²⁸COR³¹; -(CR²⁹R³⁰)ₛNR²⁸CONR²⁸R³¹; -(CR²⁹R³⁰)ₛNR²⁸SO₂R²³; -(CR²⁹R³⁰)ₛCOOR²¹; -(CR²⁹R³⁰)ₛCONR²⁸R³¹; -(CR²⁹R³⁰)ₛSO₂NR²⁸R³¹; -(CR²⁹R³⁰)ₜCOR³¹; -(CR²⁹R³⁰)ₛSO₂R²³; -(CR²⁹R³⁰)ₜR²⁴; -(CR²⁹R³⁰)ₜR²⁵; -(CR²⁹R³⁰)ₜR²⁶; oder -(CR²⁹R³⁰)ₜR³¹ steht;
R²⁹ für H; F; CF₃; Niederalkyl; Niederalkenyl; Niedercycloalkyl; Niederheterocycloalkyl; Aryl; Heteroaryl; Niederarylalkyl; Niederheteroarylalkyl; -(CR³²R³³)ₛOR³¹; -(CR³²R³³)ₛNR²⁸R³¹; -(CR³²R³³)ₛNR²⁸COR³¹; -(CR³²R³³)ₛNR²⁸CONR²⁸R³¹; -(CR³²R³³)_{q}COOR²¹; -(CR³²R³³)_{q}CONR²⁸R³¹; -(CR³²R³³)_{q}COR³¹; oder (CR³²R³³)_{q}R³¹ steht;
R³⁰ Und R³³ unabhängig definiert sind als H; CF₃; oder CH₃;
R³⁴ und R³⁵ unabhängig definiert sind als H; F; Cl; CF₃; OCF₃; OCHF₂; Niederalkyl; Niederalkenyl; Niederalkinyl; Niedercycloalkyl; Niederheterocycloalkyl; Aryl; Heteroaryl; Niederarylalkyl; Niederheteroarylalkyl; -(CR²⁹R³⁰)_{q}OR³¹; -(CR²⁹R³⁰)_{q}NR²⁸R³¹; -(CR²⁹R³⁰)_{q}NR²⁸COR³¹; -(CR²⁹R³⁰)_{q}NR²⁸CONR²⁸R³¹; -(CR²⁹R³⁰)_{q}NR²⁸SO₂R²³; -(CR²⁹R³⁰)_{q}COOR²¹; -(CR²⁹R³⁰)_{q}CONR²⁸R³¹; -(CR²⁹R³⁰)_{q}SO₂NR²⁸R³¹; -(CR²⁹R³⁰)_{q}COR³¹; -(CR²⁹R³⁰)_{q}SO₂R²³; oder (CR²⁹R³⁰)_{q}R³¹;
R³⁷ für H; Niederalkyl; Niederalkenyl; Niedercycloalkyl; Niederheterocycloalkyl; Aryl; Heteroaryl; Niederarylalkyl; Niederheteroarylalkyl; eine geeignete *N*-Schutzgruppe; -(CR²⁹R³⁰)ᵣOR³¹; -(CR²⁹R³⁰)ᵣNR²⁸R³¹; -(CR²⁹R³⁰)ᵣNR²⁸COOR²¹; -(CR²⁹R³⁰)ᵣNR²⁸COR³¹; -(CR²⁹R³⁰)ᵣNR²⁸CONR²⁸R³¹; -(CR²⁹R³⁰)_{q}COOR²¹; -(CR²⁹R³⁰)_{q}CONR²⁸R³¹; -(CR²⁹R³⁰)_{q}COR³¹; oder -(CR²⁹R³⁰)_{q}R³¹ steht;
R³⁸ für H; Niederalkyl; Niederalkenyl; Niedercycloalkyl; Niederheterocycloalkyl; Aryl; Heteroaryl; Niederarylalkyl; Niederheteroarylalkyl; -(CR²⁹R³⁰)_{q}OR³¹; -(CR²⁹R³⁰)_{q}NR²⁸R³¹; -(CR²⁹R³⁰)_{q}NR²⁸COR³¹; -(CR²⁹R³⁰)_{q}NR²⁸CONR²⁸R³¹; -(CR²⁹R³⁰)_{q}COOR²¹; -(CR²⁹R³⁰)_{q}CONR²⁸R³¹; -(CR²⁹R³⁰)_{q}COR³¹; oder (CR²⁹R³⁰)_{q}R³¹ steht;
Z für O; S; oder S(=O)₂ steht;
U für -C(=O)-; -NR⁴-C(=O)-; oder -C(=O)-C(=O)- steht.

5. Verbindungen nach Anspruch 1, wobei
die Basiskomponente vom Typ **A** ausgewählt ist aus
**A1**(a1); **A2**(a1); **A3**(a1); **A4**(a1); **A5**(a1); **A6**(a1); **A7**(a1); **A9**(a1); **A10**(a1); **A73**(a2); **A170**(a4); **A209**(a7); **A240**(a10); **A272**(a10); **A532** (a18); **A614**(a24); **A631**(a1); **A632** (a1); **A633**(a1); **A641** (a1); **A642** (a1); **A643** (a1); **A651** (a1); **A652**(a1); oder **A653**(a1);
die Basiskomponente vom Typ **B** ausgewählt ist aus
**B7**(b3); **B8**(b3); **B9**(b3); **B10**(b3); oder **B17**(b5);
die Basiskomponente vom Typ C ausgewählt ist aus
**C1**; **C8; C9; C11; C12; C15; C17; C19; C20; C25;** oder **C47;**
und wobei
R¹ für H; F; Cl; Br; CF₃; OCF₃; OCHF₂; CN; Niederalkyl; Niederalkenyl; Cycloalkyl; Heterocycloalkyl; Aryl; Heteroaryl; Niederarylalkyl; Niederheteroarylalkyl; -(CR²⁹R³⁰)_{q}OR²⁰; -(CR²⁹R³⁰)_{q}NR⁴R¹¹; -(CR²⁹R³⁰)_{q}NR⁴COR ²²; (CR²⁹R³⁰)_{q}NR⁴CONR⁴R¹¹; -(CR²⁹R³⁰)_{q}NR⁴SO₂R²³; -(CR²⁹R³⁰)_{q}COOR²¹; -(CR²⁹R³⁰)_{q}CONR⁴R¹¹; -(CR²⁹R³⁰)_{q}SO₂NR⁴R¹¹; -(CR²⁹R³⁰)_{q}COR²²; -(CR²⁹R³⁰)_{q}SO₂R²³; -(CR²⁹R³⁰)_{q}R²⁴; -(CR²⁹R³⁰)_{q}R²⁵; oder (CR²⁹R³⁰)_{q}R²⁶ steht;
R² für H; CF₃; Niederalkyl; Niederalkenyl; Niedercycloalkyl; Niederheterocycloalkyl; Aryl; Heteroaryl; Niederarylalkyl; Niederheteroarylalkyl; -(CR²⁹R³⁰)_{q}OR²⁰; -(CR²⁹R³⁰)_{q}NR⁴R¹¹; -(CR²⁹R³⁰)_{q}NR⁴COR²²; -(CR²⁹R³⁰)_{q}NR⁴CONR⁴R¹¹; -(CR²⁹R³⁰)_{q}NR⁴SO₂R²³; -(CR²⁹R³⁰)_{q}COOR²¹; -(CR²⁹R³⁰)_{q}CONR⁴R¹¹; -(CR²⁹R³⁰)_{q}SO₂NR⁴R¹¹; -(CR²⁹R³⁰)_{q}COR²²; -(CR²⁹R³⁰)_{q}SO₂R²³; -(CR²⁹R³⁰)_{q}R²⁴; -(CR²⁹R³⁰)_{q}R²⁵; oder (CR²⁹R³⁰)_{q}R²⁶ steht;
R⁴ für H; Acetyl; Niederalkyl; Niederalkenyl; oder eine geeignete N- Schutzgruppe steht;
R⁵, R⁷ und R⁹ unabhängig definiert sind als H; CF₃; Niederalkyl; Niederalkenyl; Niedercycloalkyl; Niederheterocycloalkyl; Aryl; Heteroaryl; Niederarylalkyl; Niederheteroarylalkyl; -(CR¹⁸R¹⁹)_{q}OR²⁰; -(CR¹⁸R¹⁹)_{q}NR⁴R¹¹; -(CR¹⁸R¹⁹)_{q}NR⁴COR²²; -(CR¹⁸R¹⁹)_{q}NR⁴CONR⁴R¹¹; -(CR¹⁸R¹⁹)_{q}NR⁴SO₂R²³; -(CR¹⁸R¹⁹)_{q}COOR²¹; -(CR¹⁸R¹⁹)_{q}CONR⁴R¹¹; -(CR¹⁸R¹⁹)_{q}SO₂NR⁴R¹¹; -(CR¹⁸R¹⁹)_{q}COR²²; -(CR¹⁸R¹⁹)_{q}SO₂R²³; -(CR¹⁸R¹⁹)_{q}R²⁴; -(CR¹⁸R¹⁹)_{q}R²⁵; oder -(CR¹⁸R¹⁹)_{q}R²⁶;
R⁶, R⁸ und R¹⁰ unabhängig definiert sind als H; CF₃; oder CH₃; oder Benzyl;
R¹¹ für H; Alkyl; Alkenyl; Cycloalkyl; Niederheterocycloalkyl; Aryl; Heteroaryl; Niederarylalkyl; Niederheteroarylalkyl; eine geeignete Schutzgruppe; -(CR¹⁸R¹⁹)ᵣOR²⁰; -(CR¹⁸R¹⁹)ᵣNR⁴R²⁷; -(CR¹⁸R¹⁹)ᵣNR⁴CONR⁴R²⁷; -(CR¹⁸R¹⁹)ᵣNR⁴SO₂R²³; -(CR¹⁸R¹⁹)_{q}COOR²¹; -(CR¹⁸R¹⁹)_{q}CONR⁴R²⁷; -(CR¹⁸R¹⁹)_{q}COR²²; -(CR¹⁸R¹⁹)_{q}R²⁴; -(CR¹⁸R¹⁹)ₛR²⁵; -(CR¹⁸R¹⁹)_{q}R²⁶; oder -(CR¹⁸R¹⁹)_{q}R³¹ steht;
R¹² und R¹³ unabhängig definiert sind als H; oder Niederalkyl;
R¹⁴ und R¹⁶ unabhängig definiert sind als H; F; CF₃; Niederalkyl; Niederalkenyl; Niedercycloalkyl; Niederheterocycloalkyl; Aryl; Heteroaryl; Niederarylalkyl; Niederheteroarylalkyl; -(CR²⁹R³⁰)_{q}OR²⁰; -(CR²⁹R³⁰)_{q}NR⁴R¹¹; -(CR²⁹R³⁰)_{q}NR⁴COR²²; -(CR²⁹R³⁰)_{q}COOR²¹; oder -(CR²⁹R³⁰)_{q}CONR⁴R¹¹;
R¹⁵ und R¹⁷ unabhängig definiert sind als H; CF₃; oder CH₃;
R¹⁸ für H; F; CF₃; Niederalkyl; Niederalkenyl; Niedercycloalkyl; Niederheterocycloalkyl; Aryl; Heteroaryl; Niederarylalkyl; Niederheteroarylalkyl; -(CR²⁹R³⁰)ₛOR³¹; -(CR²⁹R³⁰)ₛNR²⁸R³¹; -(CR²⁹R³⁰)ₛNR²⁸COR³¹; -(CR²⁹R³⁰)ₛNR²⁸CONR²⁸R³¹; -(CR²⁹R³⁰)ₛNR²⁸SO₂R²³; -(CR²⁹R³⁰)_{q}COOR²¹; -(CR²⁹R³⁰)_{q}CONR²⁸R³¹; -(CR²⁹R³⁰)_{q}SO₂NR²⁸R³¹; -(CR²⁹R³⁰)_{q}COR³¹; -(CR²⁹R³⁰)_{q}SO₂R²³; -(CR²⁹R³⁰)_{q}R²⁴; -(CR²⁹R³⁰)_{q}R²⁵; oder -(CR²⁹R³⁰)_{q}R²⁶ steht;
R¹⁹ für H; CF₃; oder CH₃ steht;
R²⁰ für H; Niederalkyl; Niederalkenyl; Niedercycloalkyl; Niederheterocycloalkyl; Aryl; Heteroaryl; Niederarylalkyl; Niederheteroarylalkyl; -(CR²⁹R³⁰)ᵣOR³¹; -(CR²⁹R³⁰)ᵣNR²⁸R³¹; -(CR²⁹R³⁰)ᵣNR²⁸COR³¹; -(CR²⁹R³⁰)ᵣNR²⁸CONR²⁸R³¹; -(CR²⁹R³⁰)ᵣNR²⁸SO₂R²³; -(CR²⁹R³⁰)_{q}COOR²¹; -(CR²⁹R³⁰)_{q}CONR²⁸R³¹; -(CR²⁹R³⁰)_{q}SO₂NR²⁸R³¹; -(CR²⁹R³⁰)_{q}COR³¹; -(CR²⁹R³⁰)_{q}SO₂R²³; -(CR²⁹R³⁰)_{q}R²⁴; -(CR²⁹R³⁰)_{q}R²⁵; -(CR²⁹R³⁰)_{q}R²⁶; oder -(CR²⁹R³⁰)_{q}R³¹ steht;
R²² für Niederalkyl; Niederalkenyl; Niedercycloalkyl; Niederheterocycloalkyl; Aryl; Heteroaryl; Niederarylalkyl; Niederheteroarylalkyl; -(CR²⁹R³⁰)ₛOR³¹; -(CR²⁹R³⁰)ₛNR²⁸R³¹; -(CR²⁹R³⁰)ₛNR²⁸COR³¹; -(CR²⁹R³⁰)ₛNR²⁸CONR²⁸R³¹; -(CR²⁹R³⁰)ₛNR²⁸SO₂R²³; -(CR²⁹R³⁰)ₛCOOR²¹; -(CR²⁹R³⁰)ₛCONR²⁸R³¹; -(CR²⁹R³⁰)ₛSO₂NR²⁸R³¹; -(CR²⁹R³⁰)ₜCOR³¹; -(CR²⁹R³⁰)ₛSO₂R²³; -(CR²⁹R³⁰)ₜR²⁴; -(CR²⁹R³⁰)ₜR²⁵; -(CR²⁹R³⁰)ₜR²⁶; oder (CR²⁹R³⁰)ₜR³¹ steht;
R²⁴ für Aryl; Heteroaryl; oder -C₆H₂R³⁴R³¹ steht;
R²⁵ und R²⁶ unabhängig als Heterocycloalkyl definiert sind;
R²⁷ für H; Niederalkyl; Niederalkenyl; Niedercycloalkyl; Niederheterocycloalkyl; Aryl; Heteroaryl; Niederarylalkyl; Niederheteroarylalkyl; eine geeignete Schutzgruppe; oder -(CR²⁹R³⁰)_{q}R²⁴ steht;
R²⁸ für H; -(CR³²R³³)ₛOR²¹; oder -(CR³²R³³)ₛNR⁴³R⁴² steht;
R²⁹ für H; F; CF₃; Niederalkyl; Niederalkenyl; Niedercycloalkyl; Niederheterocycloalkyl; Aryl; Heteroaryl; Niederarylalkyl; Niederheteroarylalkyl; -(CR³²R³³)ₛOR³¹; -(CR³²R³³)ₛNR²⁸R³¹; -(CR³²R³³)ₛNR²⁸COR³¹; -(CR³²R³³)ₛNR²⁸CONR²⁸R³¹; -(CR³²R³³)_{q}COOR²¹; -(CR³²R³³)_{q}CONR²⁸R³¹; -(CR³²R³³)_{q}COR³¹; oder -(CR³²R³³)_{q}R³¹ steht;
R³⁰ und R³³ unabhängig definiert sind als H; F; CF₃; oder CH₃;
R³¹ für H; Alkyl; Alkenyl; Cycloalkyl; Heterocycloalkyl; Aryl; Heteroaryl; Arylalkyl; oder Heteroarylalkyl steht;
R³² für H; F; CF3; Niederalkyl; Niederalkenyl; Niedercycloalkyl; Niederheterocycloalkyl; Aryl; Heteroaryl; Niederarylalkyl; oder Heteroarylalkyl steht;
R³⁴ und R³⁵ unabhängig definiert sind als H; F; Cl; CF₃; Niederalkyl; Niederalkenyl; Niedercycloalkyl; Niederheterocycloalkyl; Aryl; Heteroaryl; Niederarylalkyl; Niederheteroarylalkyl; -(CR²⁹R³⁰)_{q}OR³¹; -(CR²⁹R³⁰)_{q}NR²⁸R³¹; -(CR²⁹R³⁰)_{q}NR²⁸COR³¹; -(CR²⁹R³⁰)_{q}NR²⁸CONR²⁸R³¹; -(CR²⁹R³⁰)_{q}NR²⁸SO₂R²³; -(CR²⁹R³⁰)_{q}COOR²¹; -(CR²⁹R³⁰)_{q}CONR²⁸R³¹; -(CR²⁹R³⁰)_{q}SO₂NR²⁸R³¹; -(CR²⁹R³⁰)_{q}COR³¹; -(CR²⁹R³⁰)_{q}SO₂R²³; oder -(CR²⁹R³⁰)_{q}R³¹;
R³⁸ für H; Niederalkyl; Niederalkenyl; Niedercycloalkyl; Niederheterocycloalkyl; Aryl; Heteroaryl; Niederarylalkyl; Niederheteroarylalkyl; - (CR²⁹R³⁰)_{q}OR³¹; -(CR²⁹R³⁰)_{q}NR²⁸R³¹; -(CR²⁹R³⁰)_{q}NR²⁸COR³¹; -(CR²⁹R³⁰)_{q}NR²⁸CONR²⁸R³¹; -(CR²⁹R³⁰)_{q}COOR²¹; -(CR²⁹R³⁰)_{q}CONR²⁸R³¹; -(CR²⁹R³⁰)_{q}COR³¹; oder -(CR²⁹R³⁰)_{q}R³¹ steht;
R⁴² und R⁴³ unabhängig definiert sind als H; Niederalkyl; Niederalkenyl; Niedercycloalkyl; Niederheterocycloalkyl; Aryl; Heteroaryl; Niederarylalkyl; Niederheteroarylalkyl; oder eine geeignete *N*-Schutzgruppe;
X für O; S; S(=O); oder S(=O)₂ steht;
Y für O; S; oder NR⁴ steht;
Z für O; S; oder S(=O)₂ steht;
U für -C(=O)-; -NR⁴-C(=O)-; oder -C(=O)-C(=O)- steht;
oder pharmazeutisch unbedenkliche Salze, Solvate, Clathrate, isotopisch angereicherte oder enantiomer angereicherte Versionen davon.

6. Verbindungen nach Anspruch 1, wobei
die Basiskomponente vom Typ **A** ausgewählt ist aus
**A2**(a1); **A5**(a1); **A9**(a1); **A10**(a1); **A73**(a2); **A209**(a7); **A272**(a10); **A532** (a18); **A614**(a24); **A641**(a1); oder **A651**(a1);
die Basiskomponente vom Typ **B** ausgewählt ist aus
**B7; B9; B10** oder **B17;**
die Basiskomponente vom Typ C ausgewählt ist aus
**C1; C8; C9; C11; C15; C17; C19; C20; C25;** oder **C47;**
und wobei
R¹ für H; F; Cl; Br; CH₃; OCH₃; OH; oder Aryl steht;
R² für H; CH₃; Heterocycloalkyl; Aryl; Benzyl; Heteroaryl; -OR²⁰; -NR⁴R¹¹; -NR⁴COOR²¹; -NR⁴COR²²; -NR⁴CONR⁴R¹¹; oder -NR⁴SO₂R²³ steht;
R³ für H steht;
R⁴ für H; Acetyl; Niederalkyl; Niederalkenyl; oder eine geeignete N- Schutzgruppe steht;
R⁵, R⁷ und R⁹ unabhängig definiert sind als H; CF₃; Niederalkyl; Niederalkenyl; Niedercycloalkyl; Niederheterocycloalkyl; Aryl; Heteroaryl; Niederarylalkyl; Niederheteroarylalkyl; -OR²⁰; -NR⁴R¹¹; -NR⁴COR²²; -NR₄CONR⁴R¹¹; -NR⁴SO₂R²³; -COOR²¹; -CONR⁴R¹¹; oder -COR²²;
R⁶, R⁸ und R¹⁰ definiert sind als H;
R¹¹ für H; Alkyl; Aryl; Heteroaryl; Niederarylalkyl; Niederheteroarylalkyl; -(CR¹⁸R¹⁹)ᵣOR²⁰; -(CR¹⁸R¹⁹)ᵣNR⁴R²⁷; -(CR¹⁸R¹⁹)_{q}COOR²¹; -COR²²; -(CR¹⁸R¹⁹)_{q}R²⁴; oder -(CR¹⁸R¹⁹)_{q}R³¹ steht;
R¹² und R¹³ definiert sind als H;
R¹⁴ und R¹⁶ unabhängig definiert sind als H; Niederalkyl; oder Niederarylalkyl;
R¹⁵ und R¹⁷ definiert sind als H;
R¹⁸ und R¹⁹ definiert sind als H;
R²⁰ für H; Aryl; Heteroaryl; Niederarylalkyl; Niederheteroarylalkyl; oder -(CR²⁹R³⁰)_{q}R²⁴ steht;
R²¹ für H; Niederalkyl; Niederarylalkyl; oder eine geeignete *O-*Schutzgruppe steht;
R²² für Niederalkyl; Niederalkenyl; Aryl; Heteroaryl; Niederarylalkyl; Niederheteroarylalkyl; -(CR²⁹R³⁰)ₛOR³¹; -(CR²⁹R³⁰)ₛNR²⁸R³¹; -(CR²⁹R³⁰)ₛCOOR²¹; -(CR²⁹R³⁰)ₜR²⁴; oder -(CR²⁹R³⁰)ₜR³¹ steht;
R²³ für H; Niederalkyl; Aryl; Heteroaryl, oder -R²⁴ steht;
R²⁴ für Aryl; Heteroaryl; oder -C₆H₂R³⁴R³⁵R³¹ steht;
R²⁷ für H; Acetyl; CH₃; oder -(CH₂)_{q}R²⁴ steht;
R²⁸ für H; -(CR³²R³³)ₛOR²¹; oder -(CR³²R³³)ₛNR⁴³R⁴² steht;
R²⁹ für H; Niederalkyl; Aryl; oder Heteroaryl steht;
R³⁰, R³² und R³³ unabhängig definiert sind als H; CF₃; oder CH₃;
R³¹ für H; Alkyl; Cycloalkyl; Heterocycloalkyl; Aryl; Heteroaryl; Arylalkyl; oder Heteroarylalkyl steht;
R³⁴ und R³⁵ unabhängig definiert sind als H; Heterocycloalkyl;-OR³¹; oder -NR²⁸R³¹;
R³⁸ für H_{;} -NR²⁸R³¹;; -NR²⁸COR³¹; oder -NR²⁸COOR³¹ steht;
R⁴² und R⁴³ unabhängig definiert sind als H; CH₃; oder Benzyl;
X für O; S; S(=0); oder S(=O)₂ steht;
Y für O; S; oder NR⁴ steht;
Z für O; S; oder S(=O)₂ steht;
U für -C(=O)-; -NR⁴-C(=O)-; oder -C(=O)-C(=O)- steht;
oder pharmazeutisch unbedenkliche Salze, Solvate, Clathrate, isotopisch angereicherte oder enantiomer angereicherte Versionen davon.

7. Verbindungen nach Anspruch 4, wobei
die Basiskomponente vom Typ C dargestellt wird von 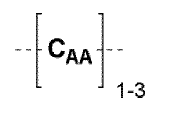 und wobei
**C_{AA}** für eine Aminosäure oder deren komplementäres Enantiomer steht, ausgewählt aus der Liste aus

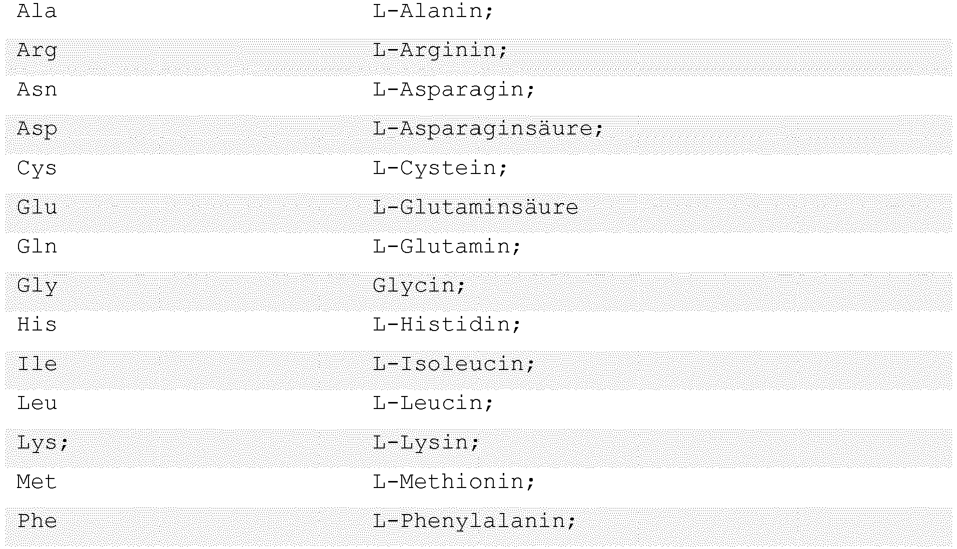

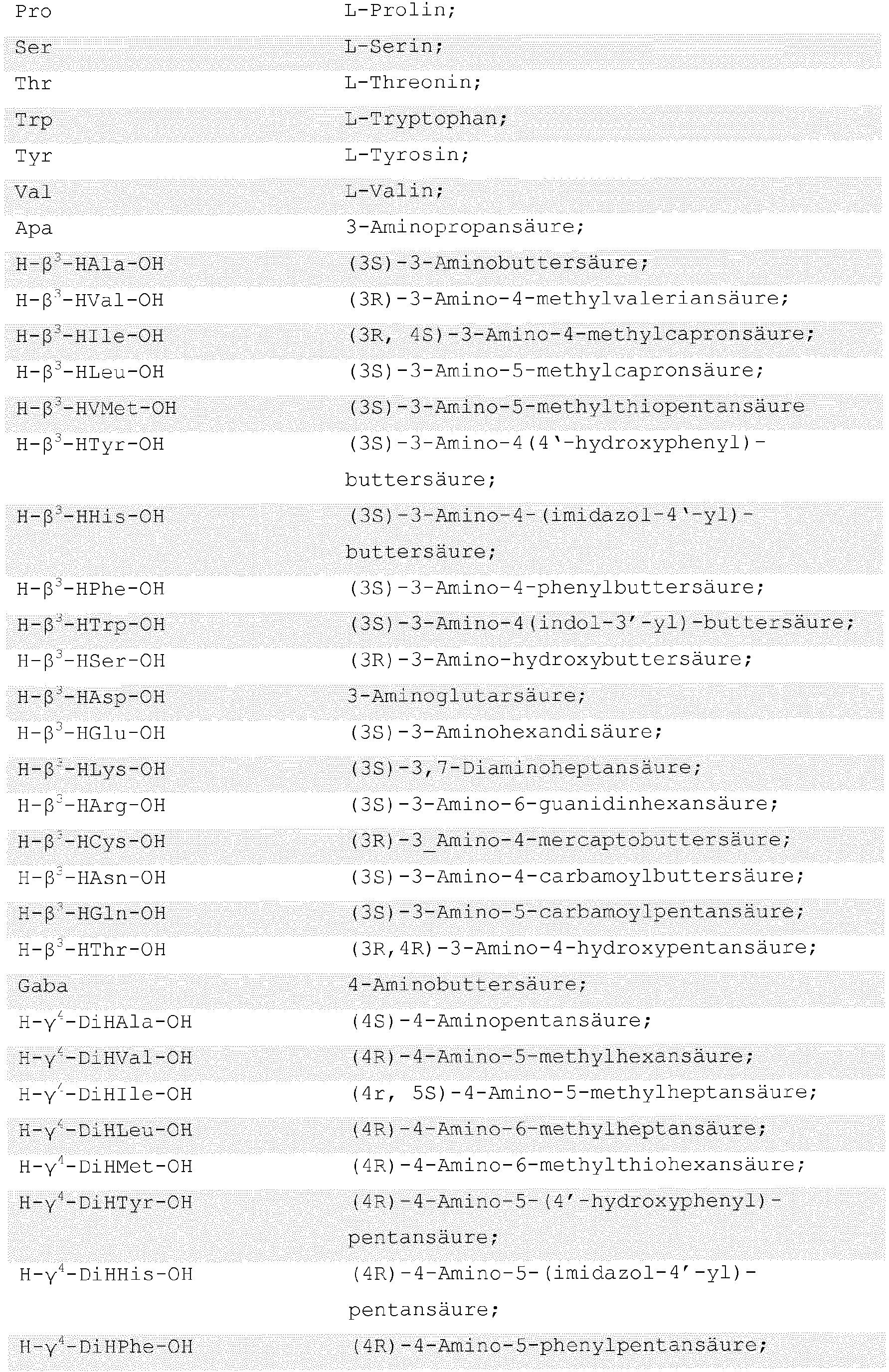

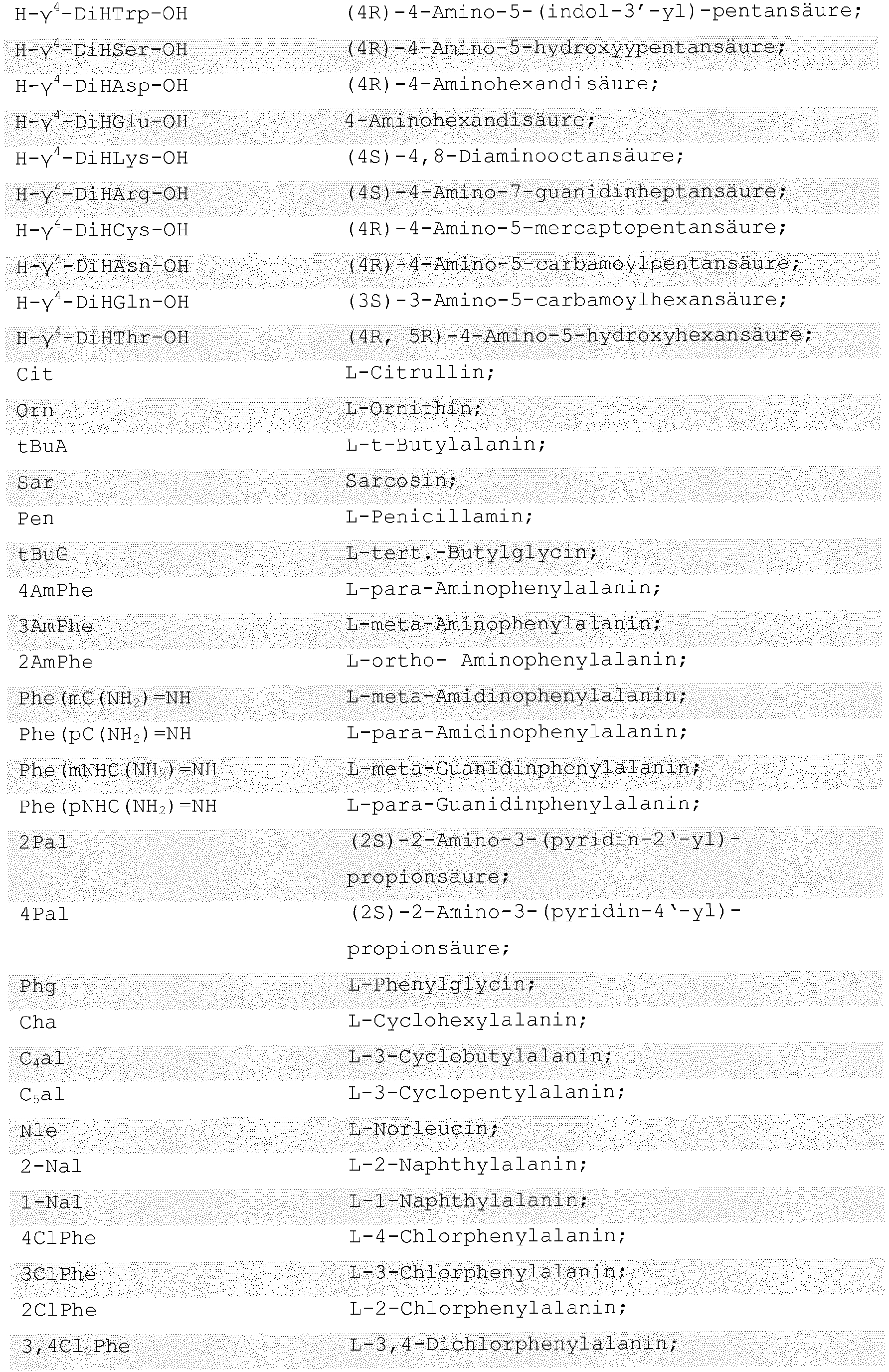

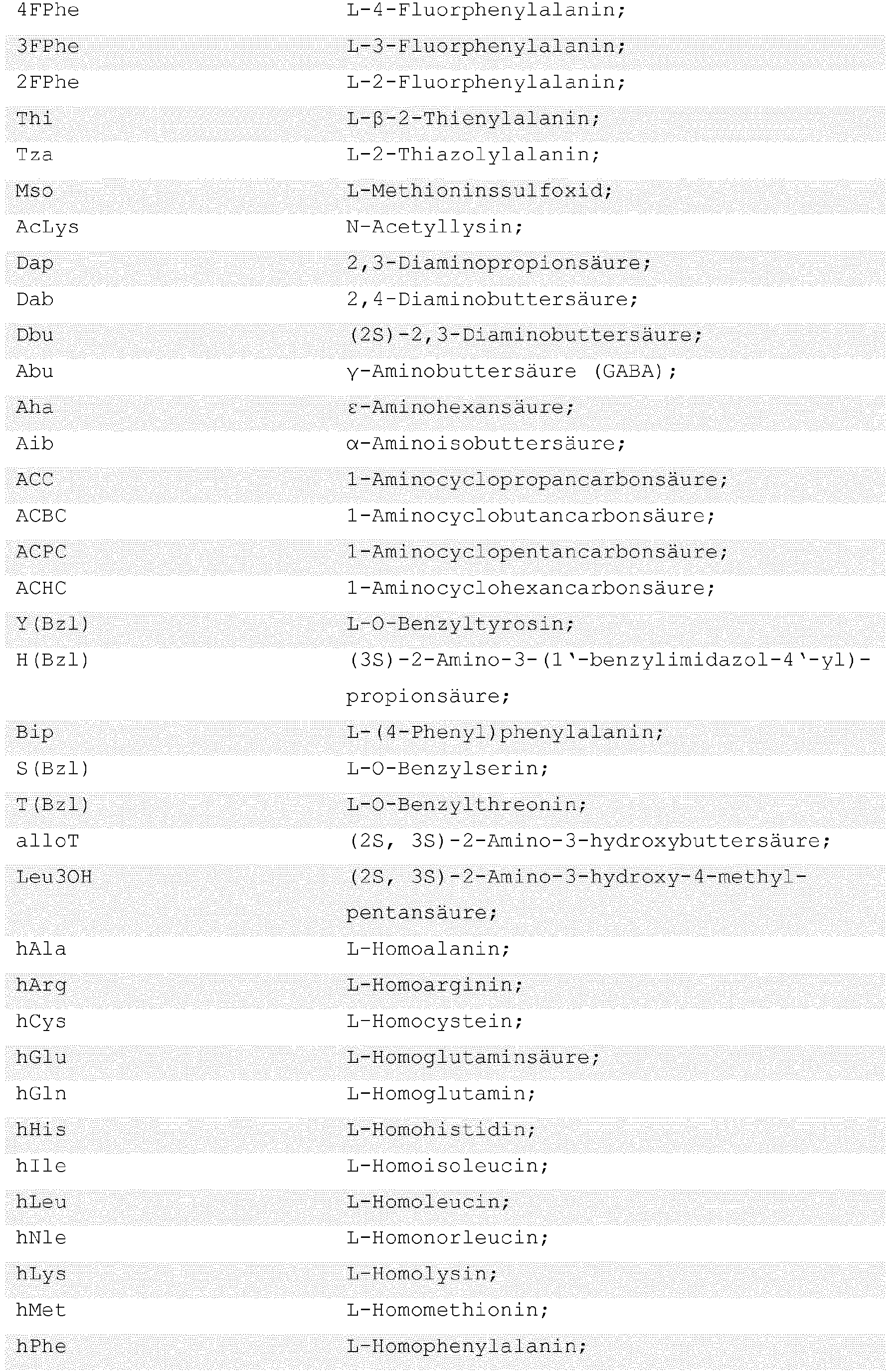

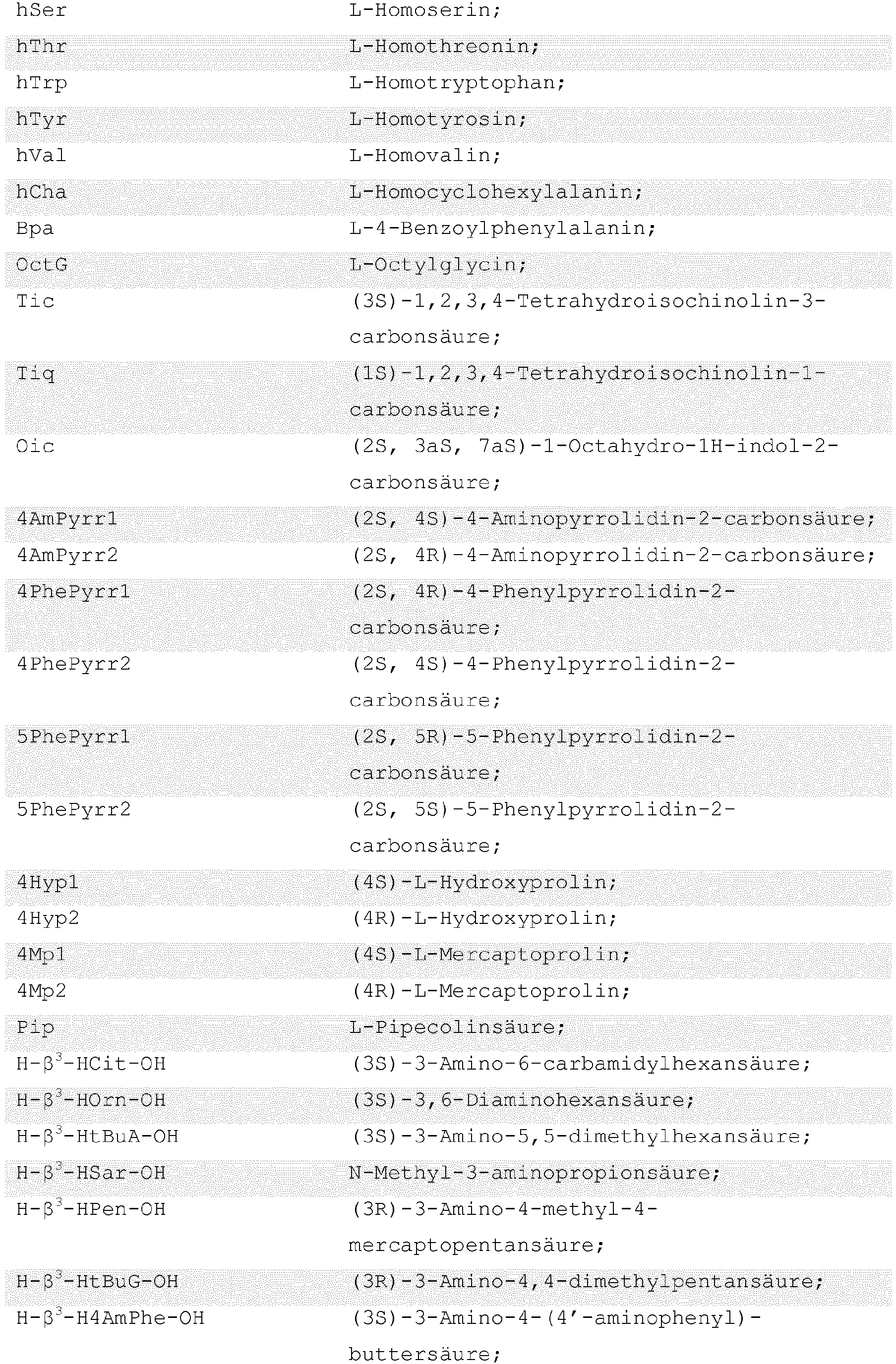

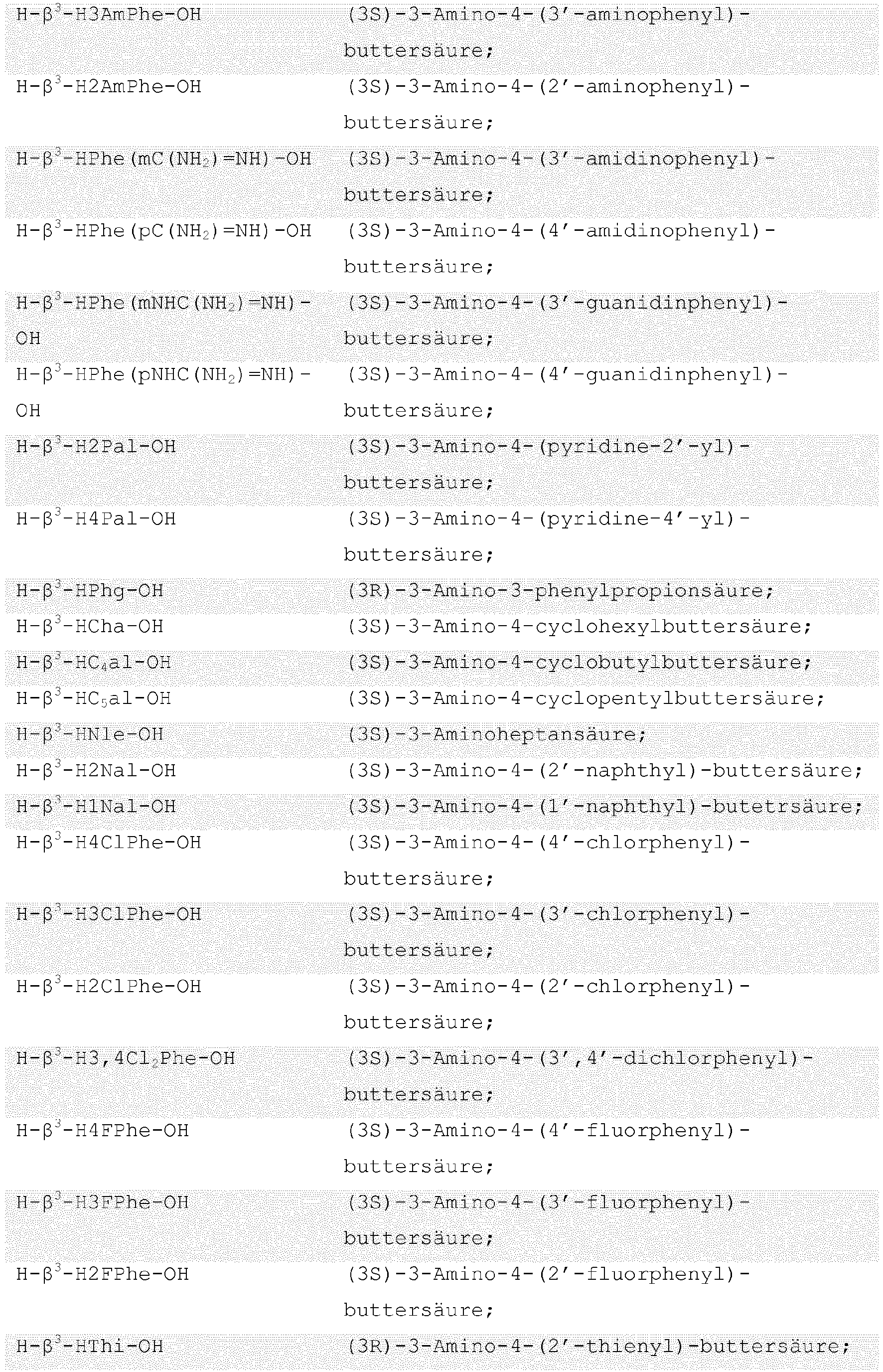

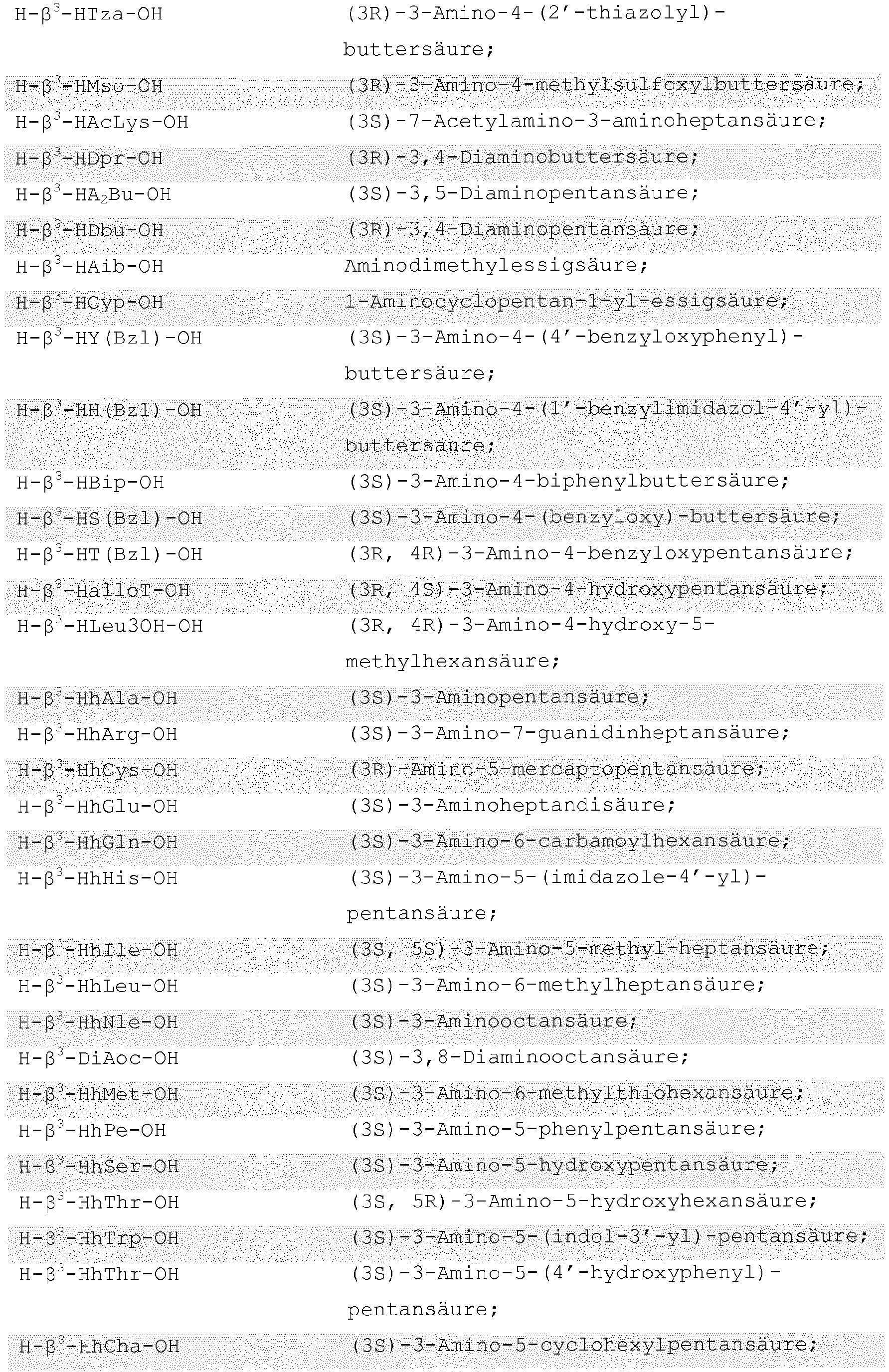

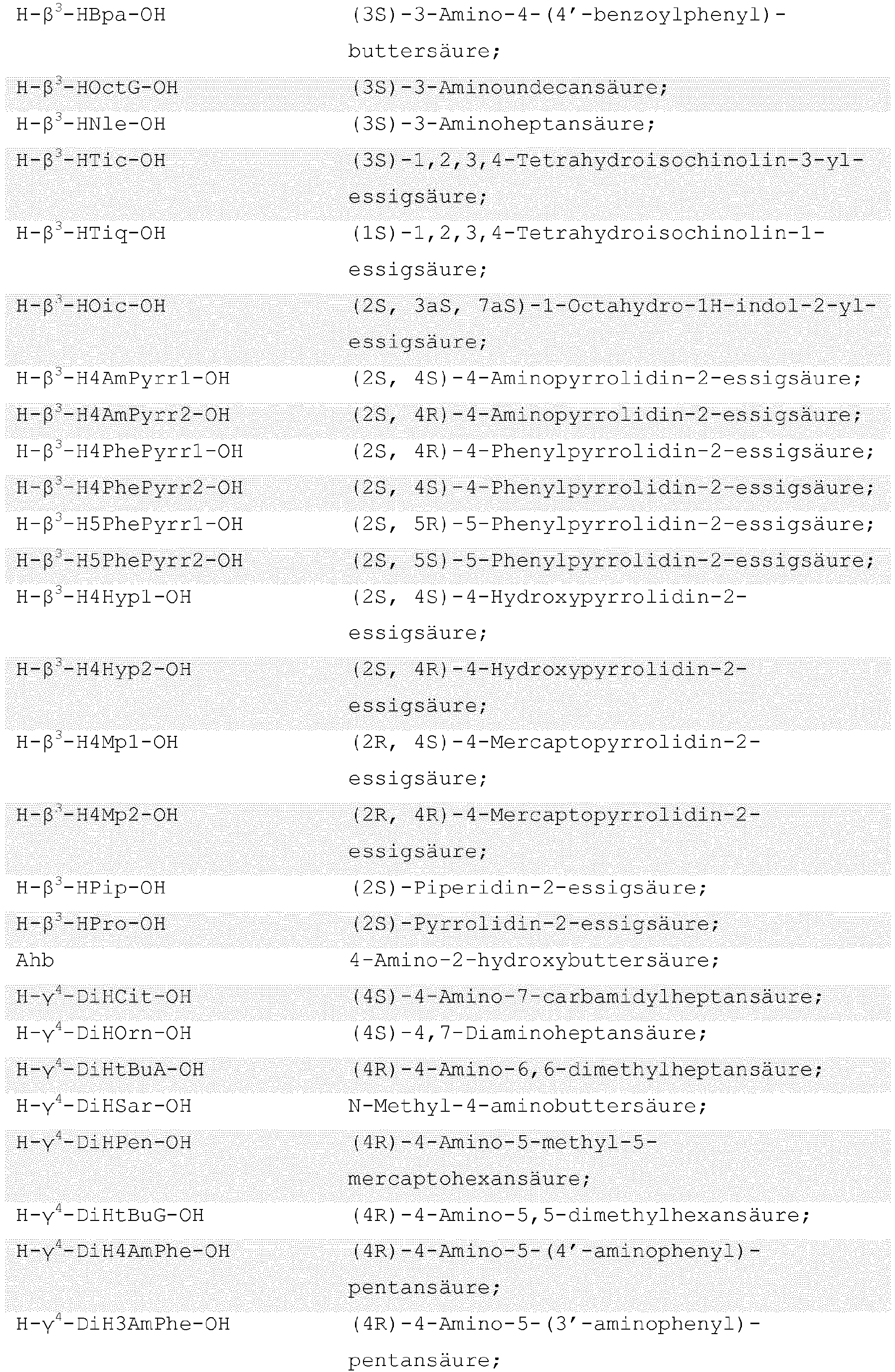

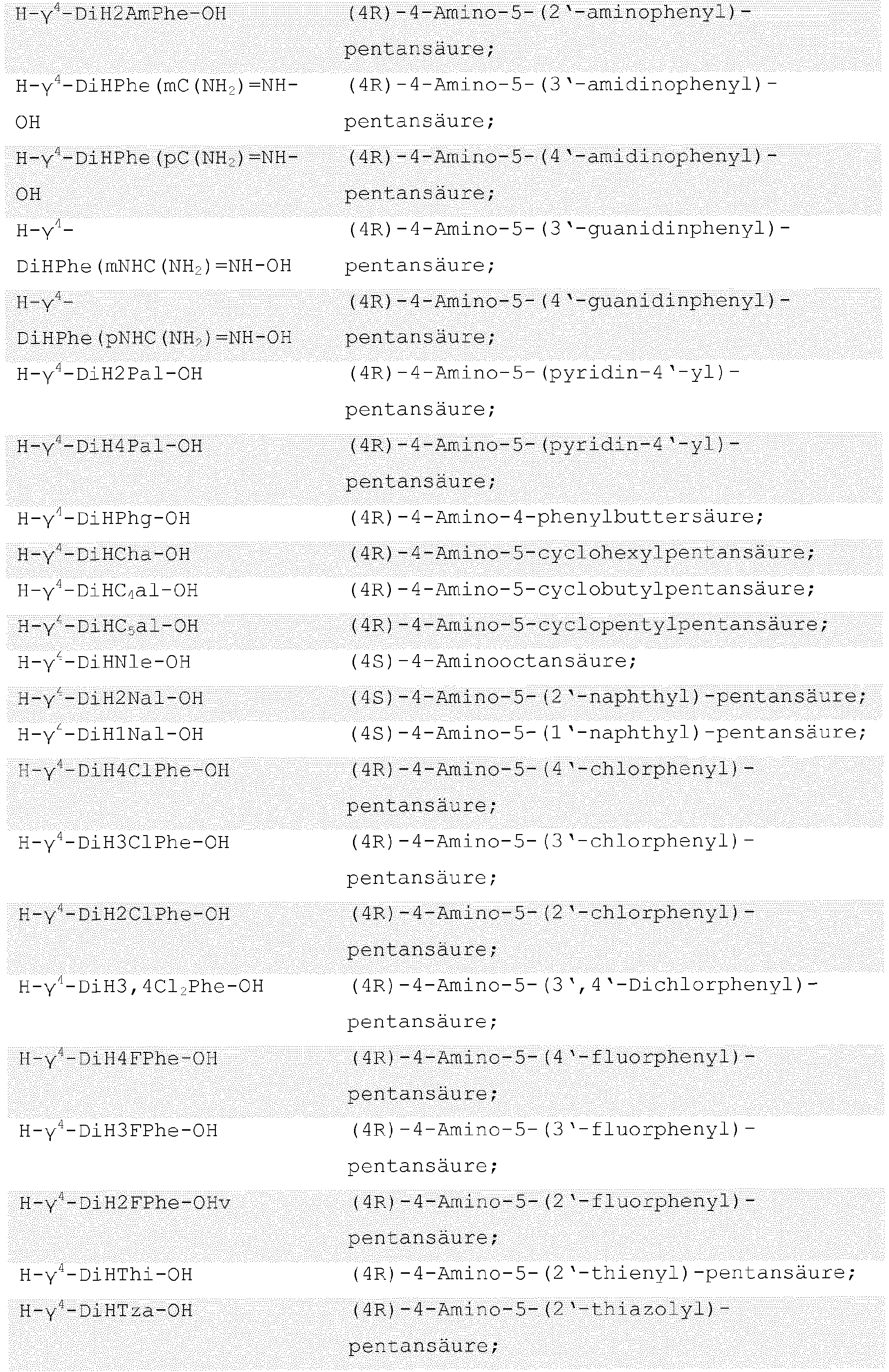

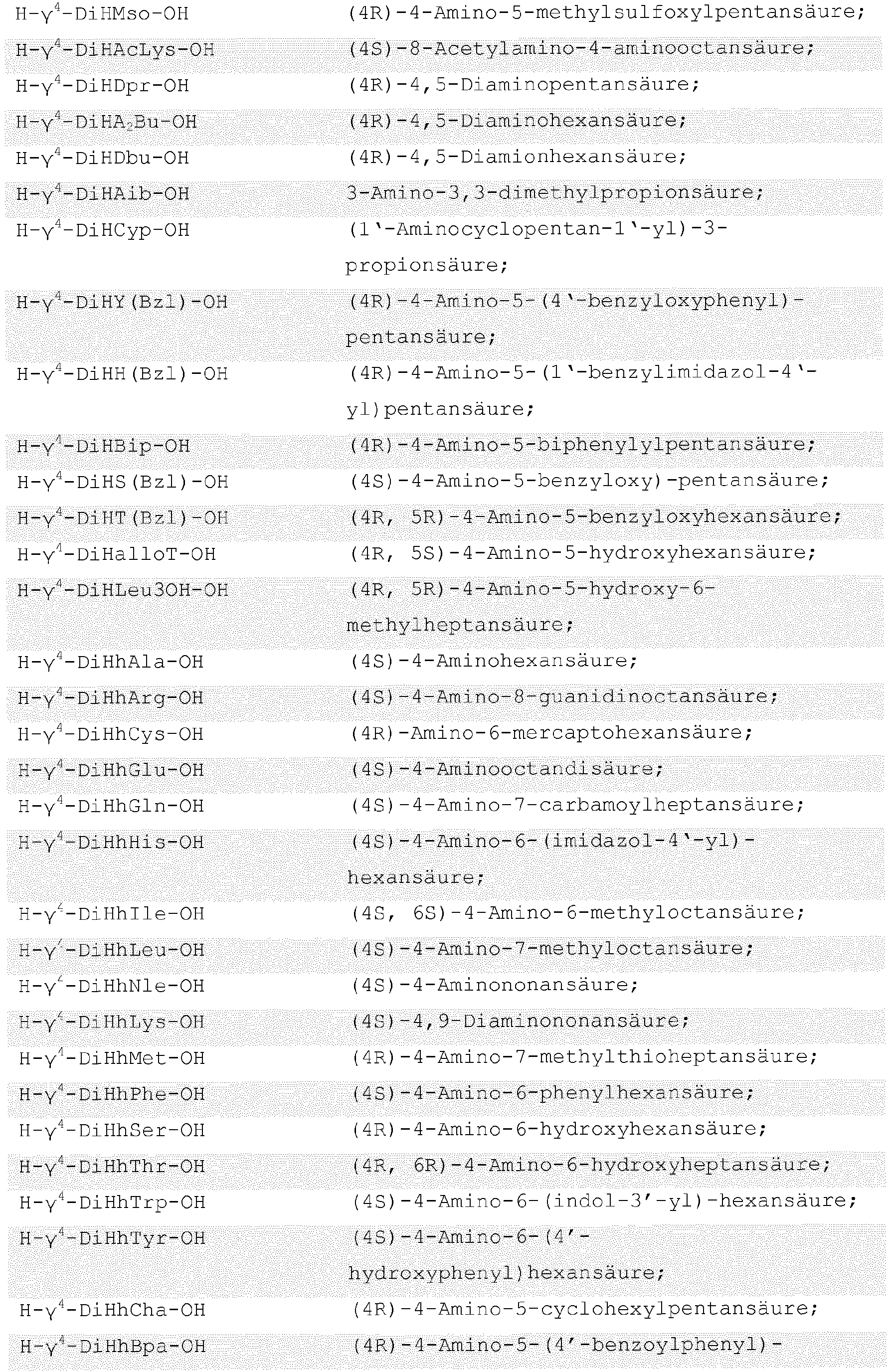

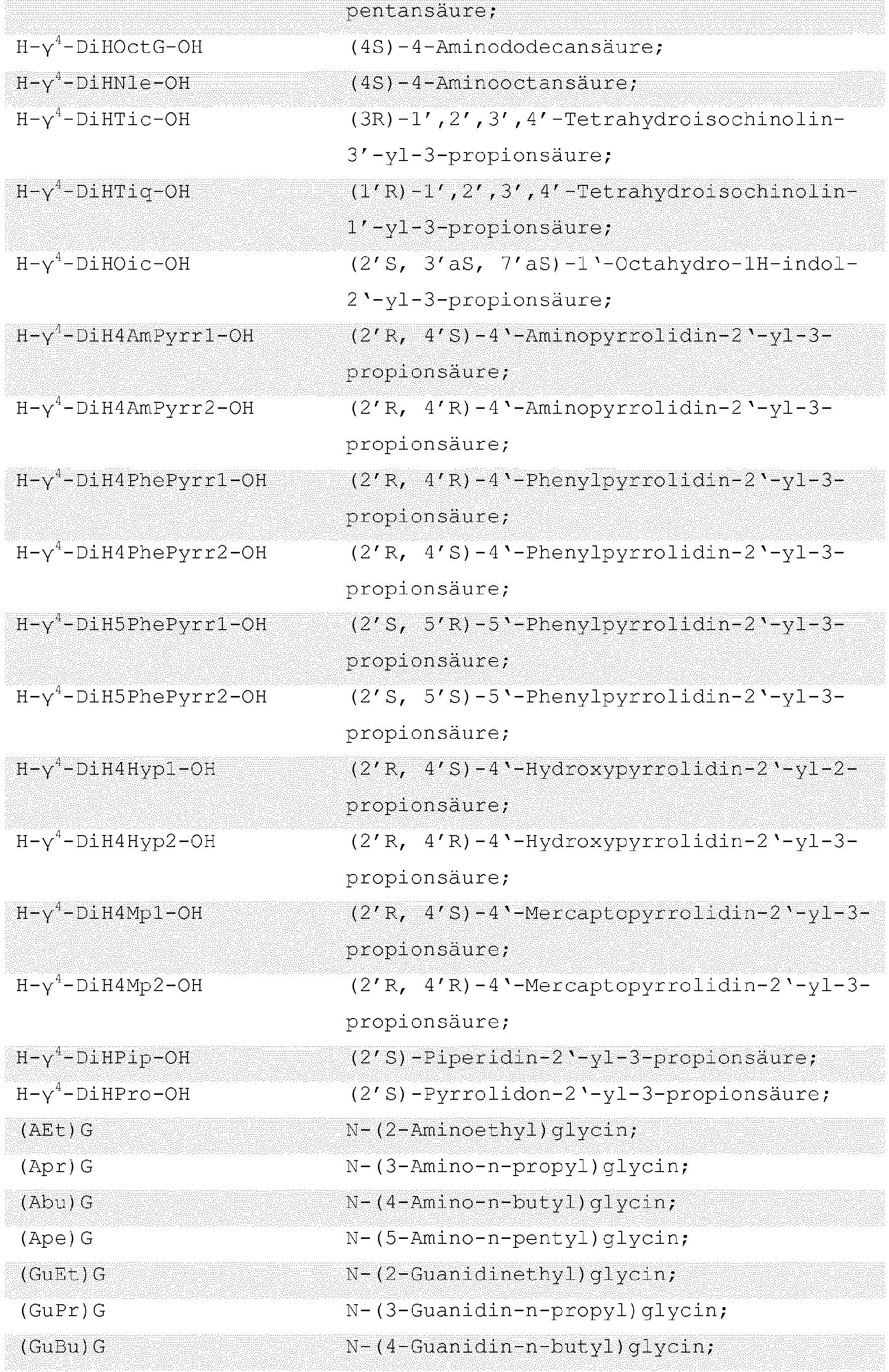

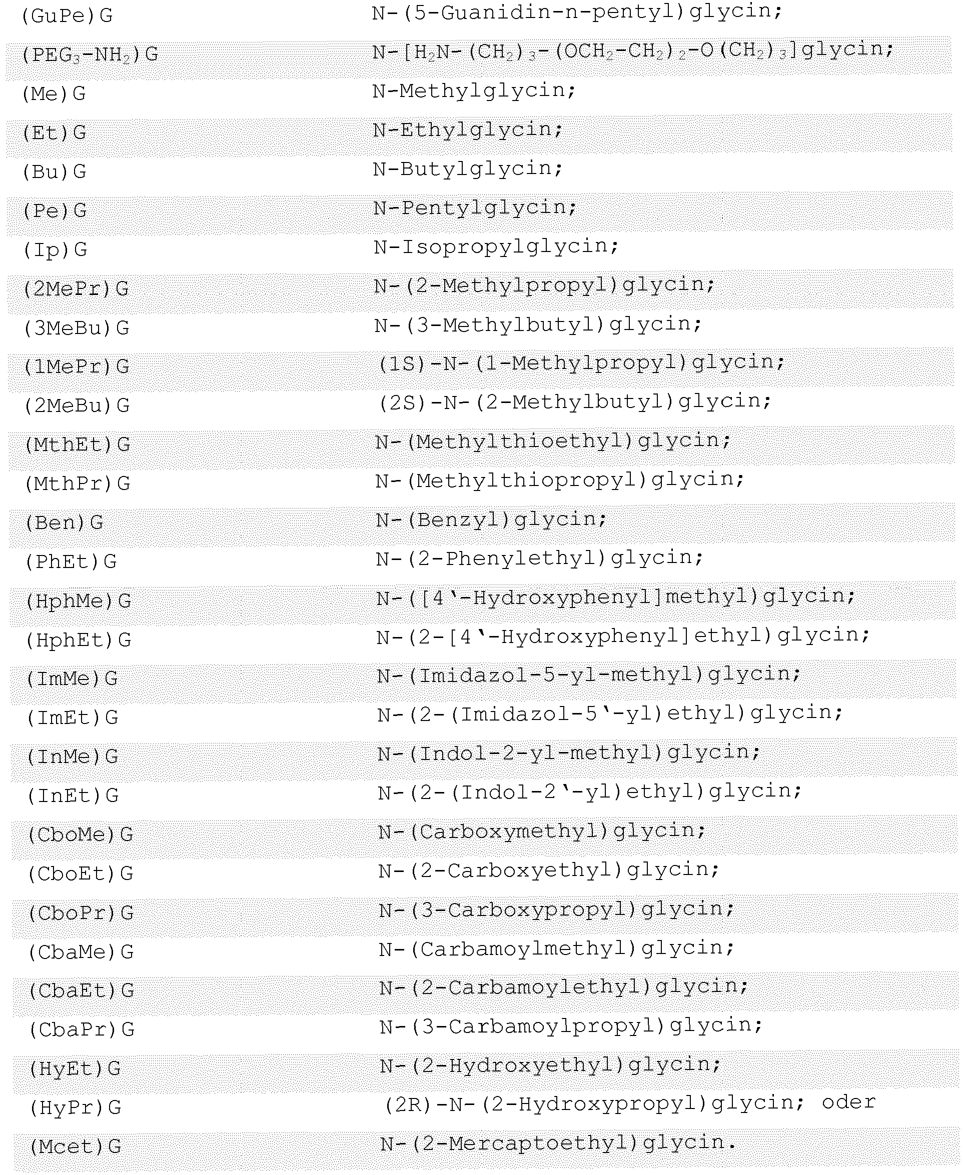

8. Verbindungen nach Anspruch 1, ausgewählt aus:
(2*S*,11*S*,19a*S*)-*N*-[2-(Dimethylamino)ethyl]-15-fluor-7,12-dimethyl-2-{[(1-naphthylamino)carbonyl]amino}-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecin-11-carboxamid;
(2*S*,11*S*,19a*S*)-N-(2-Aminoethyl)-15-fluor-7,12-dimethyl-2-{[2-(1-naphthyl)acetyl]amino}-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecin-11-carboxamid;
(2S,11S,19aS)-*N*-(2-{[Amino(imino)methyl]amino}ethyl)-15-fluor-7,12-dimethyl-2-{[2-(1-naphthyl)acetyl]amino}-5,8,13-trioxo-2,3,6,7,8,9,10,11,12,13,19,19a-dodecahydro-1*H,*5*H*-pyrrolo[2,1-*c*][1,4,7,12]benzoxatriazacyclopentadecin-11-carboxamid;
(2*S*,11*S*,19a*S*)-15-Fluor-7,12-dimethyl-2-{[2-(1-naphthyl)-acetyl]amino}-5,8,13-trioxo-*N*-[2-(2-pyridinylamino)ethyl]-2,3,6,7,5,9,10,11,12,13,19,19a-dodecahydro-1*H*,5*H*-pyrrolo[2,1-*c*][1,4,7,12]benzoxatriazacyclopentadecin-11-carboxamid;
(2*S*,11*S*,19a*S*)-*N*-[2-(Dimethylamino)ethyl]-15-fluor-7,12-dimethyl-2-{[2-(1-naphthyl)ethyl]amino}-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecin-11-carboxamid;
(2*S*,11*S*,19a*S*)-*N*-[2-(Acetylamino)ethyl]-15-fluor-7,12-dimethyl-2-[2-(1-naphthyl)acetyl]amino-5,8,13-trioxo-2,3,6,7,8,9,10,11,12,13,19,19a-dodecahydro-1*H*,5*H*-pyrrolo[2,1-*c*] [1,4,7, 12]benzoxatriazacyclopentadecin-11-carboxamid;
(2*S*,11*S*,19a*S*)-*N*-[2-(Dimethylamino)ethyl]-15-fluor-7,12-dimethyl-2-{[2-(2-naphthyl)acetyl]amino}-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecin-11-carboxamid;
(2*S*,11*S*,19a*S*)-N-[2-(Dimethylamino)ethyl]-15-fluor-7,12-dimethyl-5,8,13-trioxo-2-(2-[3-(trifluormethyl)phenyl]-acetylamino)-2,3,6,7,8,9,10,11,12,13,19,19a-dodecahydro-1*H*,5*H-*pyrrolo[2,1-*c*][1,4,7,12]benzoxatriazacyclopentadecin-11-carboxamid;
(2*S*,11*S*,19a*S*)-*N*-[2-(Dimethylamino)ethyl]-15-fluor-2-[2-(3-methoxyphenyl)acetyl]amino-7,12-dimethyl-5,8,13-trioxo-2,3,6,7,8,9,10,11,12,13,19,19a-dodecahydro-1*H*,5*H*-pyrrolo[2,1-*c*][1,4,7,12]benzoxatriazacyclopentadecin-11-carboxamid;
(2*S*,11*S*,19a*S*)-*N*-[3-(Dimethylamino)propyl]-15-fluor-7,12-dimethyl-2-[2-(1-naphthyl)acetyl]amino-5,8,13-trioxo-2,3,6,7,8, 9,10,11,12,13,19,19a-dodecahydro-1*H*,5*H*-pyrrolo[2,1-*c*][1,4,7,12]benzoxatriazacyclopentadecin-11-carboxamid;
(2*S*,11*S*,19a*S*)-15-Fluor-7,12-dimethyl-2-[2-(1-naphthyl)-acetyl]amino-5,8,13-trioxo-*N*-(3-pyridinylmethyl)-2,3,6,7,8,9,10, 11,12,13,19,19a-dodecahydro-1*H*,5*H*-pyrrolo[2,1-c][1,4,7,12]-benzoxatriazacyclopentadecin-11-carboxamid;
*N*-[(4*S*,6*S*,10*S*)-14-Methyl-6-[2-(2-naphthyl)acetyl]amino-9,15-dioxo-2-oxa-8,14-diazatricyclo[14.3.1.0^{4,8}]icosa-1(20),16,18-trien-10-yl]decanamid;
*N*-[(4*S*,6*S*,10*S*)-14-Methyl-10-{[2-(2-naphthyl)acetyl]amino}-9,15-dioxo-2-oxa-8,14-diazatricyclo[14.3.1.0^{4,8}]icosa-1(20),16,18-trien-6-yl]-2-(2-naphthyl)acetamid;
*N*-[(4*S*,6*S*,10*S*)-14-Methyl-6-[2-(2-naphthyl)acetyl]amino-9,15-dioxo-2-oxa-8,14-diazatricyclo[14.3.1.0^{4,8}]icosa-1(20),16,18-trien-10-yl]-2-(1-naphthyl)acetamid;
*N*-[(4*S*,6*S*,10*S*)-14-Methyl-9,15-dioxo-10-[(3-toluidincarbonyl)-amino]-2-oxa-8,14-diazatricyclo[14.3.1.0^{4,8}]icosa-1(20),16,18-trien-6-yl]-2-(2-naphthyl)acetamid;
*N-*[(4*S*,6*S*,10*S*)-10-([3-(Dimethylamino)aniline]carbonylamino)-14-methyl-9,15-dioxo-2-oxa-8,14-diazatricyclo[14.3.1.0^{4,8}]icosa-1(20),16,18-trien-6-yl]-2-(2-naphthyl)acetamid;
*N*-[(4*S*,6*S*,10*S*)-6-({[3-(Dimethylamino)anilino]carbonyl}amino)-14-methyl-9,15-dioxo-2-oxa-8,14-diazatricyclo[14.3.1.0^{4,8}]icosa-1(20),16,18-trien-10-yl]-2-(2-naphthyl)acetamid;
*N*-[(4*S*,6*S*,10*S*)-14-Methyl-9,15-dioxo-6-[(3-phenylpropanoyl)-amino]-2-oxa-8,14-diazatricyclo[14.3.1.0^{4,8}]icosa-1(20),16,18-trien-10-yl]decanamid;
*N*-[(4*S*,6*S*,10*S*)-14-Methyl-6-[(3-morpholinobenzoyl)amino]-9,15-dioxo-2-oxa-8,14-diazatricyclo[14.3.1.0^{4,8}]icosa-1(20),16,18-15 trien-10-yl]decanamid;
*N*-[(4S,6S,10S)-6-({[3-(Dimethylamino)anilino]carbonyl}amino)-14-methyl-9,15-dioxo-2-oxa-8,14-diazatricyclo[14.3.1.0^{4,8}]icosa-1(20),16,18-trien-10-yl]decanamid;
*N*-[(4S,6S,10S)-6-({[3-(Dimethylamino)anilino]carbonyl}amino)-14-methyl-9,15-dioxo-2-oxa-8,14-diazatricyclo[14.3.1.0^{4,8}]icosa-1(20),16,18-trien-10-yl]-2-(1-naphthyl)acetamid;
*N*-[(4*S*,6*S*,10*S*)-6-{[(3-Methoxyanilino)carbonyl]amino}-14-methyl-9,15-dioxo-2-oxa-8,14-diazatricyclo[14.3.1.0^{4,8}]icosa-1(20),16,18-trien-10-yl]-2-(2-naphthyl)acetamid;
*N*-[(4*S*,6*S*,10*S*)-14-Methyl-9,15-dioxo-6-{[2-(3-phenoxyphenyl)-acetyl]amino}-2-oxa-8,14-diazatricyclo[14.3.1.0^{4,8}]icosa-1(20),16,18-trien-10-yl]decanamid;
*N-*[(4*S*,6*S*,10*S*)-14-Methyl-6-{[2-(2-naphthyl)acetyl]amino}-9,15-dioxo-2-oxa-8,14-diazatricyclo[14.3.1.0^{4,8}]icosa-l20),16,18-trien-10-yl]-6-phenylhexanamid;
*N*-[(4*S*,6*S*,10*S*)-14-Methyl-6-{[2-(2-naphthyl)acetyl]amino}-9,15-dioxo-2-oxa-8,14-diazatricyclo[14.3.1.0^{4,8}]icosa-1(20),16,18-trien-10-yl]-5-phenoxypentanamid;
(*E*)-*N*-[(4*S*,6*S*,10*S*)-14-Methyl-6-[2-(2-naphthyl)acetyl]amino-9,15-dioxo-2-oxa-8,14-diazatricyclo[14.3.1.0^{4,8}]icosa-1(20),16,18-trien-10-yl]-3-decenamid;
*N*-[(4*S*,6*S*,10*S*)-14-Methyl-6-{[2-(1-naphthyl)acetyl]amino}-9,15-dioxo-2-oxa-8,14-diazatricyclo[14.3.1.0^{4,8}]icosa-1(20),16,18-trien-10-yl]decanamid;
6-(Benzyloxy)-*N*-[(4*S*,6*S*,10*S*)-14-methyl-6-[2-(2-naphthyl)-acetyl]amino-9,15-dioxo-2-oxa-8,14-diazatricyclo[14.3.1.0^{4,8}]-icosa-l(20),16,18-trien-10-yl]hexanamid;
2-[1,1'-Biphenyl]-4-yl-*N*-[(4*S*,6*S*,10*S*)-14-methyl-6-{[2-(2-naphthyl)acetyl]amino}-9,15-dioxo-2-oxa-8,14-diazatricyclo-[14.3.1.0⁴,⁸]icosa-1(20),16,18-trien-10-yl]acetamid;
*N*-[(4*S*,6*S*,10*S*)-14-Methyl-6-{[2-(2-naphthyl)acetyl]amino}-9,15-dioxo-2-oxa-8,14-diazatricyclo[14.3.1.0^{4,8}]icosa-l(20),16,18-trien-10-yl]-3,3-diphenylpropanamid;
6-(Benzyloxy)-*N*-[(4*S*,6*S*,10*S*)-14-methyl-9,15-dioxo-10-[(6-phenylhexanoyl)amino]-2-oxa-8,14-diazatricyclo[14.3.1.0^{4,8}]-icosa-1(20),16,18-trien-6-yl]hexanamid;
2-[1,1'-Biphenyl]-3-yl-*N*-[(4*S*,6*S*,10*S*)-6-([3-(dimethylamino)-anilino]carbonylamino)-14-methyl-9,15-dioxo-2-oxa-8,14-diazatricyclo[14.3.1.0^{4,8}]icosa-1(20),16,18-trien-10-yl]-acetamid;
2-[1,1'-Biphenyl]-4-yl-*N*-[(4S,6*S*,10*S*)-6-([3-(dimethylamino)-anilino]carbonylamino)-14-methyl-9,15-dioxo-2-oxa-8,14-diazatricyclo[14.3.1.0^{4,8}]icosa-1(20),16,18-trien-10-yl]-acetamid;
*N-*[(4*S*,6*S*,10*S*)-10-[([1,1'-Biphenyl]-4-ylsulfonyl)amino]-14-methyl-9,15-dioxo-2-oxa-8,14-diazatricyclo[14.3.1.0^{4,8}]icosa-1(20),16,18-trien-6-yl]-2-(2-naphthyl)acetamid;
*N*-[(4*S*,6*S*,10*S*)-6-{[(3-Fluoranilino)carbonyl]amino}-14-methyl-9,15-dioxo-2-oxa-8,14-diazatricyclo[14.3.1.0^{4,8}]icosa-1(20), 16,18-trien-10-yl]-2-(2-naphthyl)acetamid;
*N*-[(4*S*,6*S*,10*S*)-6-({[4-(Dimethylamino)anilino]carbonyl}amino)-14-methyl-9,15-dioxo-2-oxa-8,14-diazatricyclo[14.3.1.0^{4,8}]icosa-1(20),16,18-trien-10-yl]-2-(2-naphthyl)acetamid;
Phenyl-*N*-[(4*S*,6*S*,10*S*)-14-methyl-10-{[2-(2-naphthyl)acetyl]-amino}-9,15-dioxo-2-oxa-8,14-diazatricyclo[14.3.1.0^{4,8}]icosa-1(20),16,18-trien-6-yl]carbamat;
*N*-[(4*S*,6*S*,10*S*)-14-Methyl-9,15-dioxo-6-[2-(7-chinolinyl)acetyl]-amino-2-oxa-8,14-diazatricyclo[14.3.1.0^{4,8}]icosa-1(20),16,18-trien-10-yl]-2-(2-naphthyl)acetamid;
*N*-[(4*S*,6*S*,10*S*)-14-Methyl-9,15-dioxo-6-{[2-(3-phenoxyphenyl)-acetyl]amino}-2-oxa-8,14-diazatricyclo[14.3.1.0^{4,8}]icosa-1(20),16,18-trien-10-yl]-2-(2-naphthyl)acetamid;
*N*-[(4*S*,6*S*,10*S*)-14-Methyl-10-{[2-(2-naphthyl)acetyl]amino}-9,15-dioxo-2-oxa-8,14-diazatricyclo[14.3.1.0^{4,8}]icosa-1(20),16,18-trien-6-yl]decanamid;
*N*-[(4*S*,6*S*,10*S*)-14-Methyl-10-{[2-(1-naphthyl)acetyl]amino}-9,15-dioxo-2-oxa-8,14-diazatricyclo[14.3.1.0^{4,8}]icosa-1(20),16,18-trien-6-yl]decanamid;
*N*-[(4*S*,6*S*,10*S*)-14-Methyl-10-{[2-(1-naphthyl)acetyl]amino}-9,15-dioxo-2-oxa-8,14-diazatricyclo[14.3.1.0^{4,8}]icosa-1(20),16,18-trien-6-yl]hexanamid;
2-[1,1'-Biphenyl]-4-yl-*N*-[(4*S*,6*S*,10*S*)-14-methyl-10-{[2-(1-naphthyl)acetyl]amino}-9,15-dioxo-2-oxa-8,14-diazatricyclo-[14.3.1.0^{4,8}]icosa-1(20),16,18-trien-6-yl]acetamid;
*N*-[(4*S*,6*S*,10*S*)-10-[(2-[1,1'-Biphenyl]-4-ylacetyl)amino]-14-methyl-9,15-dioxo-2-oxa-8,14-diazatricyclo[14.3.1.0^{4,8}]icosa-1(20),16,18-trien-6-yl]-2,2-diphenylacetamid;
*N*-[(4*S*,6*S*,10*S*)-6-[(2-[1,1'-Biphenyl]-4-ylacetyl)amino]-14-methyl-9,15-dioxo-2-oxa-8,14-diazatricyclo[14.3.1.04,8]icosa-1(20),16,18-trien-10-yl]-2,2-diphenylacetamid;
*N-*[(4*S*,6*S*,10*S*)-6-[(2,2-diphenylacetyl)amino]-14-methyl-9,15-dioxo-2-oxa-8,14-diazatricyclo[14.3.1.0^{4,8}]icosa-1(20),16,18-trien-10-yl]decanamid;
*N*-[(4*S*,6*S*,10*S*)-14-Methyl-10-{[2-(1-naphthyl)acetyl]amino}-9,15-dioxo-2-oxa-8,14-diazatricyclo[14.3.1.0^{4,8}]icosa-1(20),16,18-trien-6-yl]-2,2-diphenylacetamid;
*N*-[(4*S*,6*S*,10*S*)-6-[(3,3-Diphenylpropanoyl)amino]-14-methyl-9,15-dioxo-2-oxa-8,14-diazatricyclo[14.3.1.0^{4,8}]icosa-1(20),16,18-trien-10-yl]decanamid;
2-[1,1'-Biphenyl]-4-yl-*N*-[(4*S*,6*S*,10*S*)-10-[(2-[1,1'-biphenyl]-4-ylacetyl)amino]-14-methyl-9,15-dioxo-2-oxa-8,14-diazatricyclo-[14.3.1.0^{4,8}]icosa-1(20),16,18-trien-6-yl]acetamid;
*N*-[(4*S*,6*S*,10*S*)-6-({[3-(Dimethylamino)anilino]carbonyl}amino)-14-methyl-9,15-dioxo-2-oxa-8,14-diazatricyclo[14.3.1.0^{4,8}]icosa-1(20),16,18-trien-10-yl]-3,3-dimethylbutanamid;
*N*-[(4*S*,6*S*,10*S*)-6-({[3-(Dimethylamino)anilino]carbonyl}amino)-9,15-dioxo-2-oxa-8,14-diazatricyclo[14.3.1.0^{4,8}]icosa-1(20), 16,18-trien-10-yl]-2-(2-naphthyl)acetamid;
Benzyl-*N*-[(4*S*,6*S*,10*S*)-6-{[2-(2-naphthyl)acetyl]amino}-9,15-dioxo-2-oxa-8,14-diazatricyclo[14.3.1.0^{4,8}]icosa-1(20),16,18-trien-10-yl] carbamat;
*N*-[(4*S*,6*S*,10*S*)-6-{[2-(2-Naphthyl)acetyl]amino}-9,15-dioxo-2-oxa-8,14-diazatricyclo[14.3.1.0^{4,8}]icosa-1(20),16,18-trien-10-yl] decanamid;
(4*S*,6*R*,15*S*)-11,16-Dimethyl-*N*-(2-naphthylmethyl)-9,12,17-trioxo-6-{[3-(4-pyridinyl)propanoyl]amino}-2-thia-8,11,16-triaza-tricyclo[16.3.1.0^{4,8}]docosa-l(22),18,20-trien-15-carboxamid; tert-Butyl-*N*-[(4*S*,6*R*,15*S*)-11,16-dimethyl-15-{[(2-naphthylmethyl)amino]carbonyl}-9,12,17-trioxo-2-thia-8,11,16-triaza-tricyclo[16.3.1.0^{4,8}]docosa-1(22),18,20-trien-6-yl]carbamat;
(4*S*,6*R*,15*S*)-11,16-Dimethyl-*N*-(2-naphthylmethyl)-9,12,17-trioxo-6-(pentanoylamino)-2-thia-8,11,16-triazatricyclo[16.3.1.0^{4,8}]-docosa-1(22),18,20-trien-15-carboxamid;
(4*S*,6*R*,15*S*)-6-(Acetylamino)-11,16-dimethyl-*N*-(2-naphthylmethyl)-9,12,17-trioxo-2-thia-8,11,16-triazatricyclo-[16.3.1.0^{4,8}]docosa-l(22),18,20-trien-15-carboxamid;
(2S,11S,19aS)-15-Fluor-7,12-dimethyl-2-(1-naphthoylamino)-N-(2-naphthylmethyl)-5,8,13-trioxo-2,3,6,7,8,9,10,11,12,13, 19,19a-dodecahydro-1H,5H-pyrrolo[2,1-*c*][1,4,7,12]benzoxatriaza-cyclopentadecin-11-carboxamid;
(2*S*,11*S*,19a*S*)-15-Fluor-7,12-dimethyl-2-[2-(2-naphthyl)acetyl]-amino-*N*-(1-naphthylmethyl)-5,8,13-trioxo-2,3,6,7,8,9,10,11,12, 13,19,19a-dodecahydro-1H,5H-pyrrolo[2,1-*c*][1,4,7,12]benzoxa-triazacyclopentadecin-11-carboxamid;
(2*S*,11*S*,19a*S*)-2-[(3-Chlorobenzoyl)amino]-15-fluor-7,12-dimethyl-N-(1-naphthylmethyl)-5,8,13-trioxo-2,3,6,7,8,9,10,11, 12,13,19,19a-dodecahydro-1H,5H-pyrrolo[2,1-*c*][1,4,7,12]benzoxa-triazacyclopentadecin-11-carboxamid.

9. Verbindungen nach einem der Ansprüche 1-8 zur Verwendung als therapeutisch wirksame Substanzen.

10. Verbindungen nach Anspruch 9 zur Verwendung als therapeutisch wirksame Substanzen mit agonistischer oder antagonistischer Aktivität am Motilinrezeptor (MR-Rezeptor), am Serotoninrezeptor des Untertyps 5-HT₂₈ (5-HT₂₈-Rezeptor), am Prostaglandin-F2α-Rezeptor (FP-Rezeptor), am Purinorezeptor P2Y₁, am spannungsabhängigen Kaliumkanal K_{V}1.3; oder am kanonischen (β-kateninabhängigen) Wnt-Signalweg.

11. Pharmazeutische Zusammensetzung, die eine Verbindung oder ein Verbindungsgemisch nach einem der Ansprüche 1-8 und mindestens einen therapeutisch inerten Hilfsstoff enthält.

12. Zusammensetzung nach Anspruch 11, die eine Verbindung oder ein Verbindungsgemisch nach einem der Ansprüche 1-8 enthält, die bzw. das agonistische oder antagonistische Aktivität am Motilinrezeptor (MR-Rezeptor), am Serotoninrezeptor des Untertyps 5-HT₂₈ (5-HT₂₈-Rezeptor), am Prostaglandin-F2α-Rezeptor (FP-Rezeptor), am Purinorezeptor P2Y₁, am spannungsabhängigen Kaliumkanal K_{V}1.3; oder am kanonischen (β-kateninabhängigen) Wnt-Signalweg aufweist.

13. Zusammensetzung nach Anspruch 11 oder 12, die zur oralen, topischen, transdermalen Verabreichung, zur Verabreichung mittels Injektion, zur bukkalen, transmucosalen, pulmonalen Verabreichung oder zur Verabreichung mittels Inhalation, insbesondere in Form von Tabletten, Dragees, Kapseln, Lösungen, Flüssigkeiten, Gelen, Pflastern, Cremes, Salben, Sirupen, Breien, Suspensionen, Sprays, Zerstäubern oder Zäpfchen, geeignet sind.

14. Verwendung von Verbindungen nach einem der Ansprüche 1-8 mit agonistischer oder antagonistischer Aktivität Aktivität am Motilinrezeptor (MR-Rezeptor), am Serotoninrezeptor des Untertyps 5-HT₂₈ (5-HT₂₈-Rezeptor), am Prostaglandin-F2α-Rezeptor (FP-Rezeptor), am Purinorezeptor P2Y₁, am spannungsabhängigen Kaliumkanal Kᵥ1.3; oder am kanonischen (β-kateninabhängigen) Wnt-Signalweg zur Herstellung eines Medikaments.

15. Verwendung von Verbindungen nach einem der Ansprüche 1-8 zur Herstellung eines Medikaments, wobei das Medikament für die Behandlung von **Motilitätsstörungen des Magen-Darm-Trakts;** Erkrankungen des **zentralen Nervensystems; okuläre Hypertension; entzündliche Störungen des zentralen Nervensystems; thromboembolische Störungen; Zellproliferationsstörungen; oder mit Knochen und Knorpeln zusammenhängende Störungen** gedacht ist.

16. Verwendung von Verbindungen nach Ansprüchen 1-8 zur Herstellung eines Medikaments, wobei das Medikament zur Behandlung von Störungen nach Anspruch 15 gedacht ist, wobei diese Störungen ausgewählt sind aus funktioneller Dyspepsie, diabetischer Gastroparese oder Verstopfung vom Typ Reizdarmsyndrom, Migräne, Schizophrenie, Psychose, Depression oder Alzheimer-Krankheit, Glaukom, vorzeitigen Wehen oder Multipler Sklerose.

17. Verwendung von Verbindungen nach einem der Ansprüche 1-9 zur pharmazeutischen Leitstrukturentwicklung.

## Revendications

1. Composés constitués d'un arrangement cyclique des blocs constitutifs A, B et C et représentés par les formules générales la ou Ib : 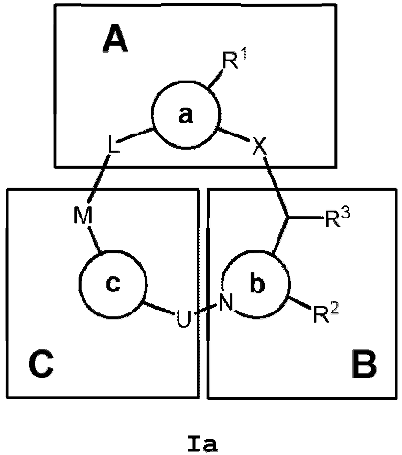 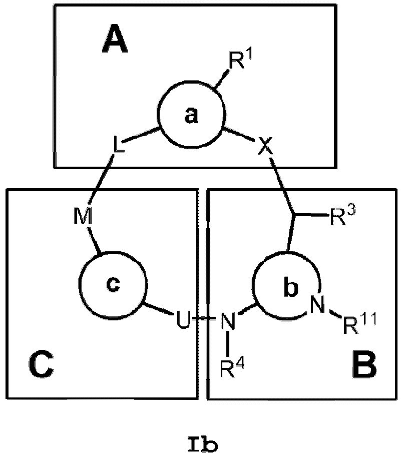 où
le bloc constitutif A ("gabarit") est représenté par : 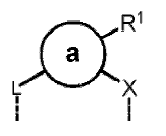
le bloc constitutif B ("modulateur") est représenté par : 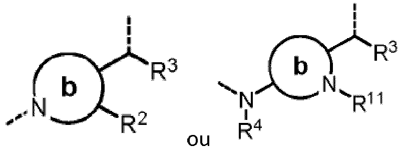
le bloc constitutif C ("pont") est représenté par : 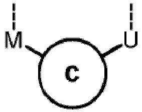 ou respectivement 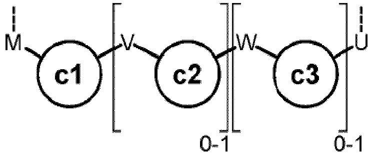
et où
M représente un radical bivalent ou trivalent choisi dans le groupe constitué de : 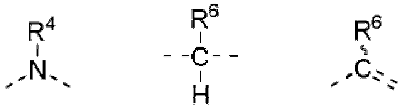
formant une partie intégrante de la connectivité M-L qui, en retour, représente un radical bivalent choisi dans le groupe constitué de : 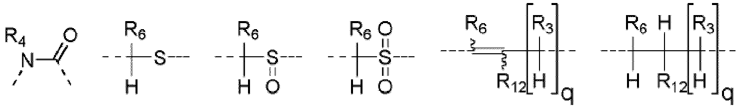
U représente un radical bivalent choisi dans le groupe constitué de : 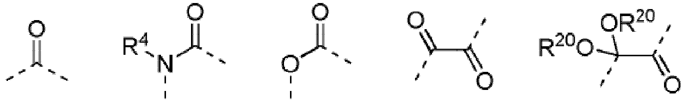
X représente un radical bivalent choisi dans le groupe constitué de : 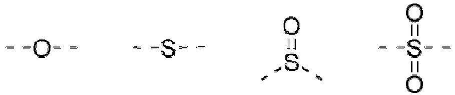
V et W représentent indépendamment un radical bivalent choisi dans le groupe constitué de : 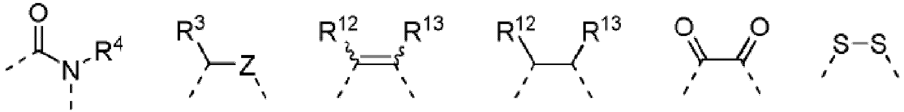
et où
ledit groupe constitutif A est un radical bivalent choisi dans le groupe constitué de :
| | | | |
|---|---|---|---|
| 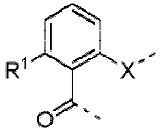 | 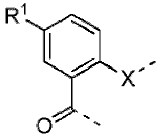 | 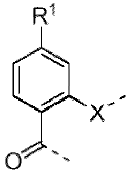 | 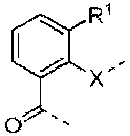 |
| **A1** (a1) | **A2** (a1) | **A3** (a1) | **A4** (a1) |
| 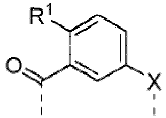 | 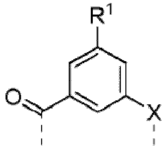 | 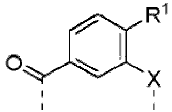 | 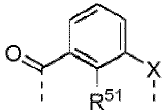 |
| **A5** (a1) | **A6** (a1) | **A7** (a1) | **A8** (a1) |
| 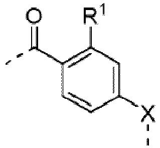 | 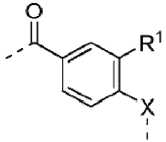 | 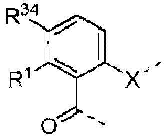 | 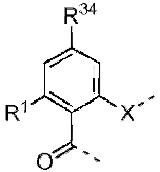 |
| **A9** (a1) | **A10** (a1) | **A11** (a1) | **A12** (a1) |
| 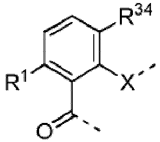 | 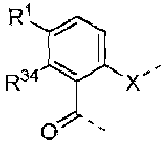 | 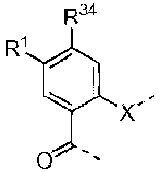 | 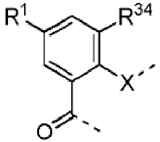 |
| **A13** (a1) | **A14** (a1) | **A15** (a1) | **A16** (a1) |
| 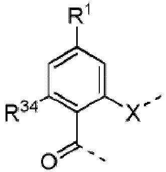 | 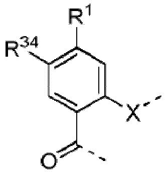 | 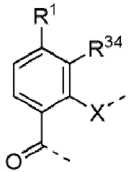 | 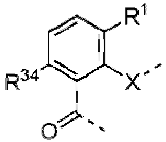 |
| **A17** (a1) | **A18** (a1) | **A19** (a1) | **A20** (a1) |
| 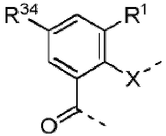 | 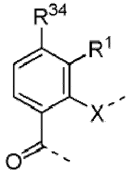 | 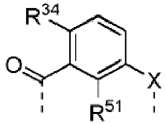 | 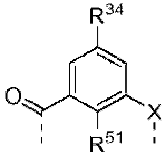 |
| **A21** (a1) | **A22** (a1) | **A23** (a1) | **A24** (a1) |
| 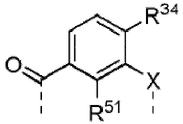 | 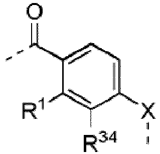 | 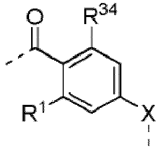 | 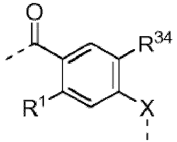 |
| **A25** (a1) | **A26** (a1) | **A27** (a1) | **A28** (a1) |
| 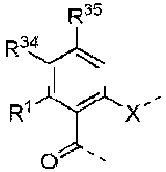 | 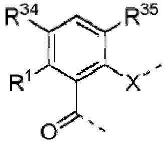 | 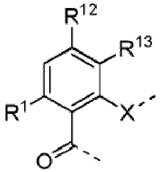 | 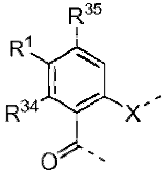 |
| **A29** (a1) | **A30** (a1) | **A31** (a1) | **A32** (a1) |
| 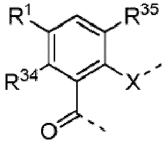 | 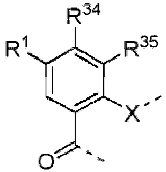 | 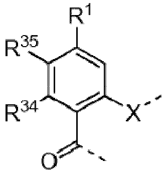 | 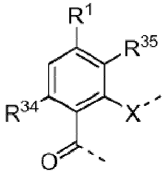 |
| **A33** (a1) | **A34** (a1) | **A35** (a1) | **A36** (a1) |
| 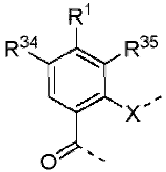 | 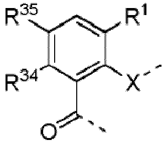 | 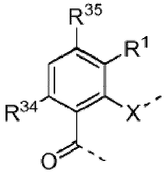 | 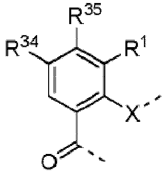 |
| **A37** (a1) | **A38** (a1) | **A39** (a1) | **A40** (a1) |
| 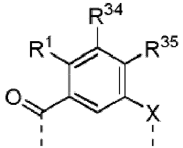 | 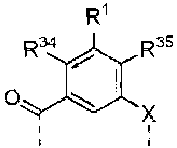 | 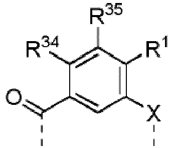 | 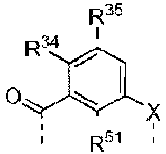 |
| **A41** (a1) | **A42** (a1) | **A43** (a1) | **A44** (a1) |
| 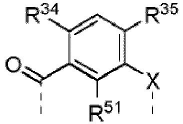 | 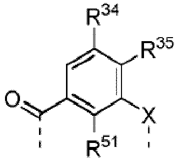 | 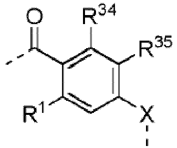 | 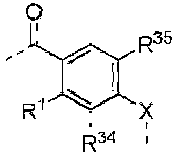 |
| **A45** (a1) | **A46** (a1) | **A47** (a1) | **A8** (a1) |
| 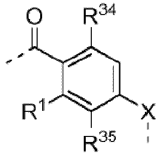 | 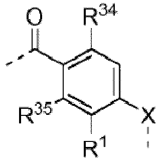 | 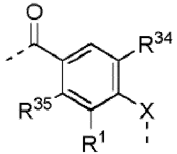 | 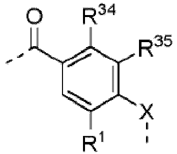 |
| **A49** (a1) | **A50** (a1) | **A51** (a1) | **A52** (a1) |
| 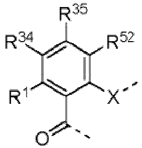 | 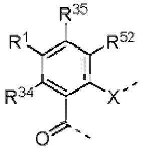 | 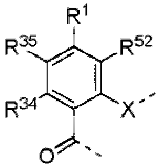 | 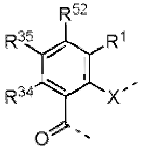 |
| **A53** (a1) | **A54** (a1) | **A55** (a1) | **A56** (a1) |
| 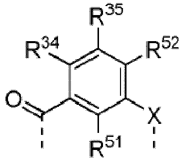 | 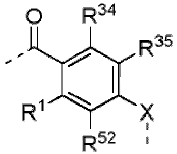 | 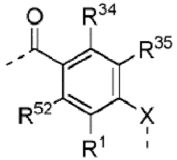 | 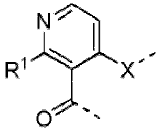 |
| **A57** (a1) | **A58** (a1) | **A59** (a1) | **A60** (a2) |
| 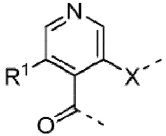 | 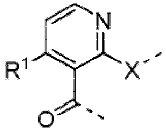 | 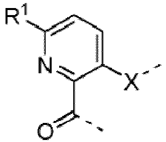 | 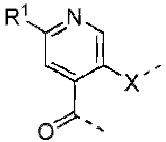 |
| **A61** (a2) | **A62** (a2) | **A63** (a2) | **A64** (a2) |
| 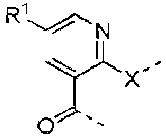 | 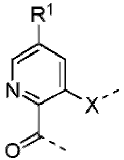 | 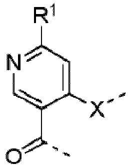 | 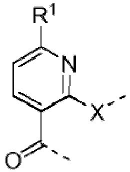 |
| **A65** (a2) | **A66** (a2) | **A67** (a2) | **A68** (a2) |
| 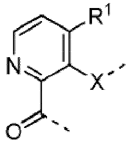 | 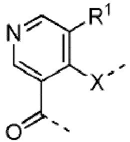 | 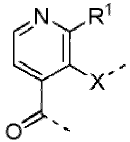 | 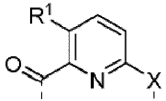 |
| **A69** (a2) | **A70** (a2) | **A71** (a2) | **A72** (a2) |
| 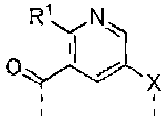 | 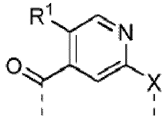 | 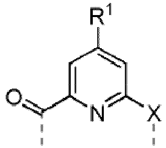 | 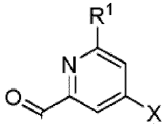 |
| **A73** (a2) | **A74** (a2) | **A75** (a2) | **A76** (a2) |
| 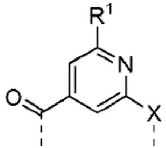 | 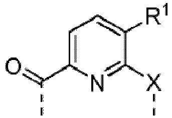 | 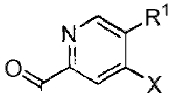 | 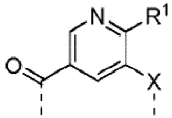 |
| **A77** (a2) | **A78** (a2) | **A79** (a2) | **A80** (a2) |
| 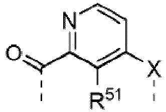 | 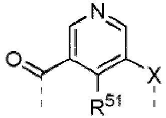 | 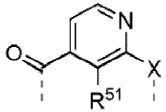 | 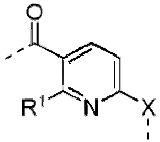 |
| **A81** (a2) | **A82** (a2) | **A83** (a2) | **A84** (a2) |
| 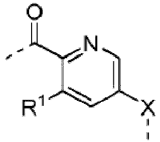 | 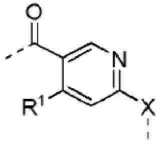 | 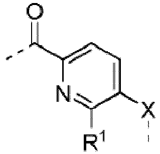 | 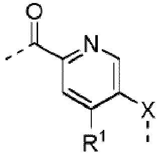 |
| **A85** (a2) | **A86** (a2) | **A87** (a2) | **A88** (a2) |
| 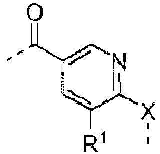 | 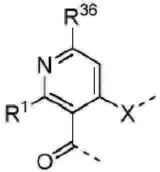 | 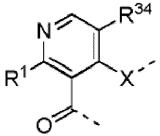 | 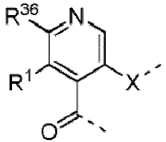 |
| **A89** (a2) | **A90** (a2) | **A91** (a2) | **A92** (a2) |
| 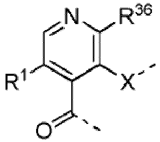 | 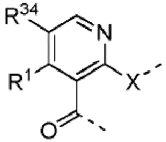 | 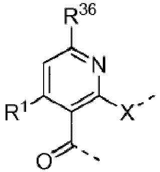 | 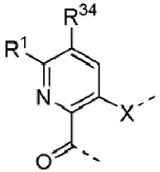 |
| **A93** (a2) | **A94** (a2) | **A95** (a2) | **A95** (a2) |
| 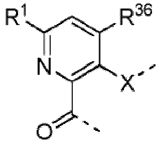 | 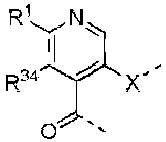 | 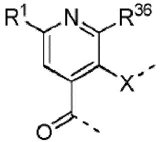 | 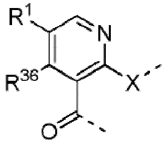 |
| **A97** (a2) | **A98** (a2) | **A99** (a2) | **A100** (a2) |
| 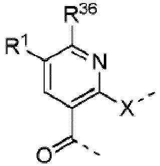 | 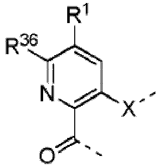 | 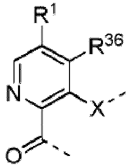 | 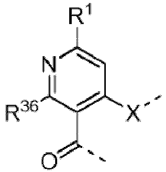 |
| **A101** (a2) | **A102** (a2) | **A103** (a2) | **A104** (a2) |
| 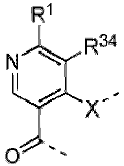 | 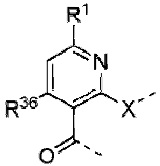 | 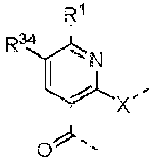 | 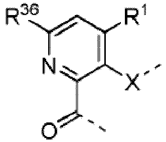 |
| **A105** (a2) | **A106** (a2) | **A107** (a2) | **A108** (a2) |
| 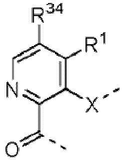 | 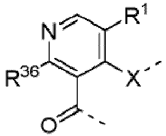 | 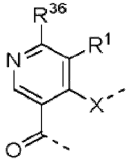 | 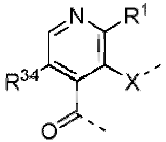 |
| **A109** (a2) | **A110** (a2) | **A111** (a2) | **A112** (a2) |
| 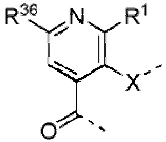 | 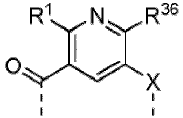 | 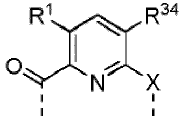 | 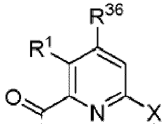 |
| **A113** (a2) | **A114** (a2) | **A115** (a2) | **A116** (a2) |
| 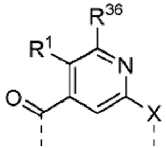 | 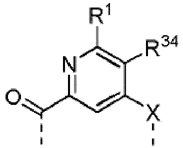 | 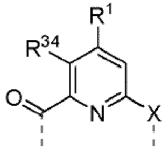 | 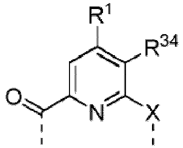 |
| **A117** (a2) | **A118** (a2) | **A119** (a2) | **A120** (a2) |
| 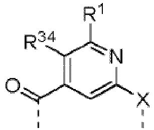 | 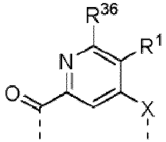 | 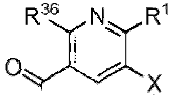 | 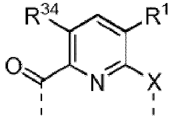 |
| **A121** (a2) | **A122** (a2) | **A123** (a2) | **A124** (a2) |
| 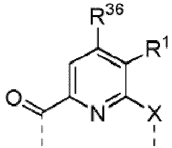 | 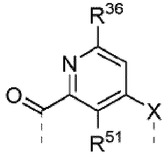 | 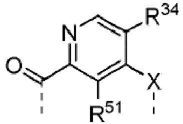 | 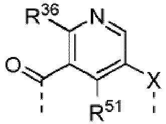 |
| **A125** (a2) | **A126** (a2) | **A127** (a2) | **A128** (a2) |
| 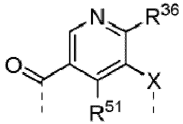 | 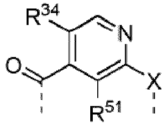 | 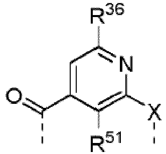 | 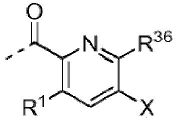 |
| **A129** (a2) | **A130** (a2) | **A131** (a2) | **A132** (a2) |
| 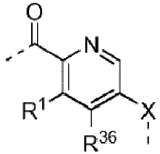 | 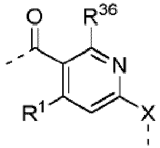 | 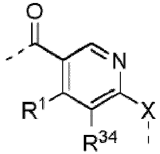 | 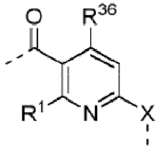 |
| **A133** (a2) | **A134** (a2) | **A135** (a2) | **A136** (a2) |
| 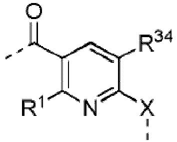 | 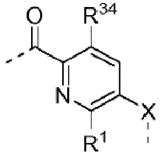 | 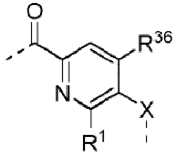 | 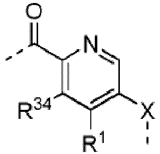 |
| **A137** (a2) | **A138** (a2) | **A139** (a2) | **A140** (a2) |
| 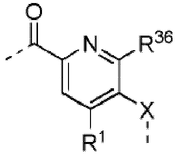 | 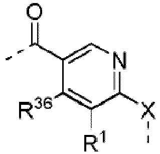 | 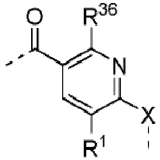 | 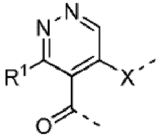 |
| **A141** (a2) | **A142** (a2) | **A143** (a2) | **A144** (a3) |
| 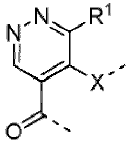 | 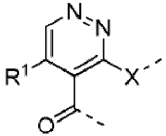 | 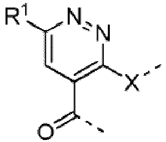 | 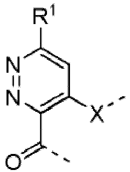 |
| **A145** (a3) | **A146** (a3) | **A147** (a3) | **A148** (a3) |
| 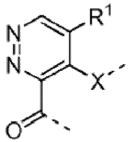 | 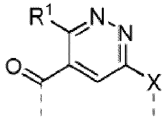 | 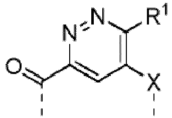 | 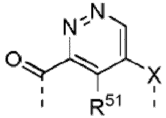 |
| **A149** (a3) | **A150** (a3) | **A151** (a3) | **A152** (a3) |
| 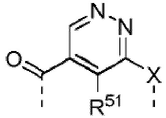 | 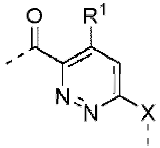 | 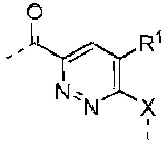 | 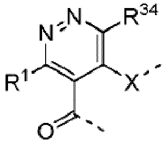 |
| **A153** (a3) | **A154** (a3) | **A155** (a3) | **A156** (a3) |
| 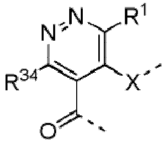 | 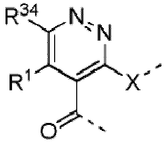 | 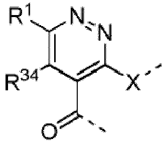 | 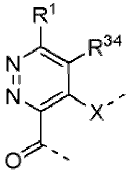 |
| **A157** (a3) | **A158** (a3) | **A159** (a3) | **A160** (a3) |
| 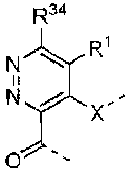 | 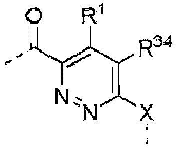 | 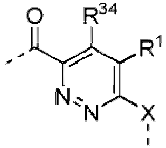 | 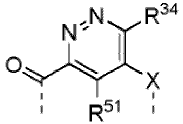 |
| **A161** (a3) | **A162** (a3) | **A163** (a3) | **A164** (a3) |
| 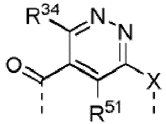 | 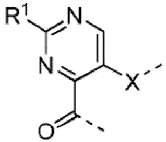 | 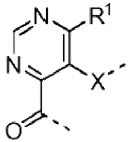 | 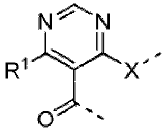 |
| **A165** (a3) | **A166** (a4) | **A167** (a4) | **A168** (a4) |
| 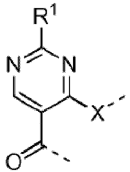 | 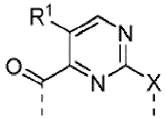 | 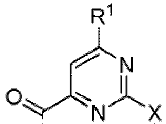 | 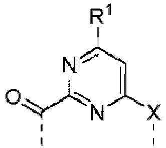 |
| **A169** (a4) | **A170** (a4) | **A171** (a4) | **A172** (a4) |
| 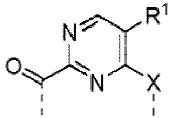 | 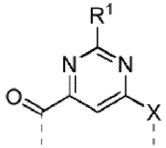 | 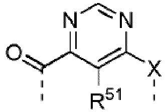 | 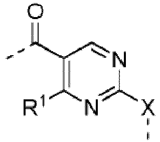 |
| **A173** (a4) | **A174** (a4) | **A175** (a4) | **A176** (a4) |
| 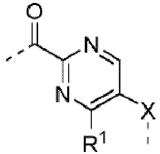 | 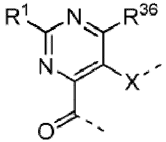 | 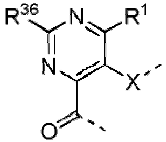 | 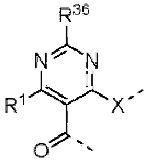 |
| **A177** (a4) | **A178** (a4) | **A179** (a4) | **A180** (a4) |
| 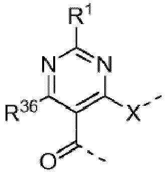 | 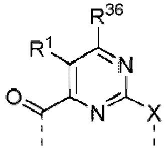 | 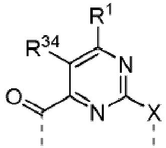 | 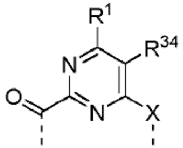 |
| **A181** (a4) | **A182** (a4) | **A183** (a4) | **A184** (a4) |
| 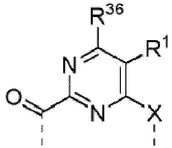 | 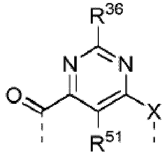 | 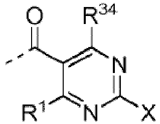 | 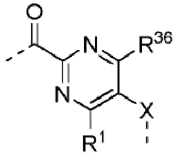 |
| **A185** (a4) | **A186** (a4) | **A187** (a4) | **A188** (a4) |
| 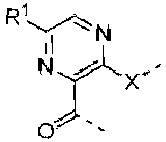 | 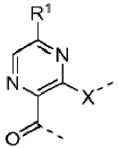 | 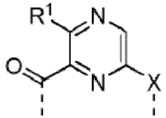 | 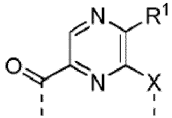 |
| **A189** (a5) | **A190** (a5) | **A191** (a5) | **A192** (a5) |
| 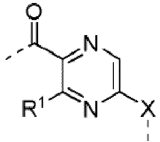 | 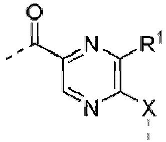 | 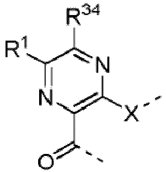 | 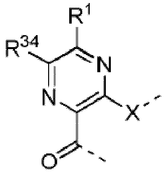 |
| **A193** (a5) | **A194** (a5) | **A195** (a5) | **A196** (a5) |
| 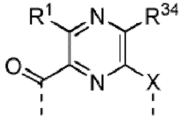 | 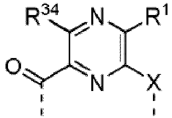 | 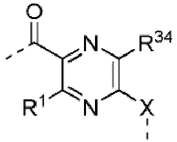 | 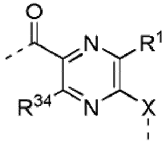 |
| **A197** (a5) | **A198** (a5) | **A199** (a5) | **A200** (a5) |
| 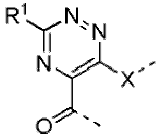 | 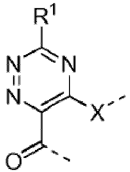 | 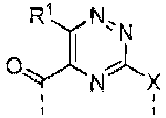 | 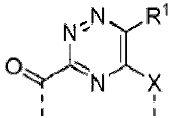 |
| **A201** (a6) | **A202** (a6) | **A203** (a6) | **A204** (a6) |
| 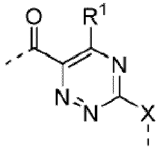 | 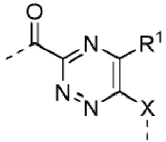 | 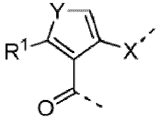 | 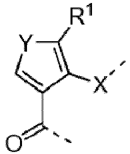 |
| **A205** (a6) | **A206** (a6) | **A207** (a7) | **A208** (a7) |
| 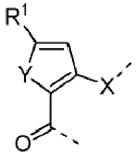 | 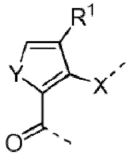 | 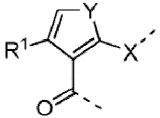 | 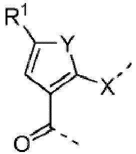 |
| **A209** (a7) | **A210** (a7) | **A211** (a7) | **A212** (a7) |
| 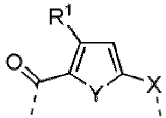 | 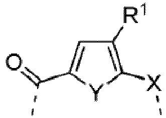 | 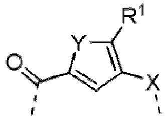 | 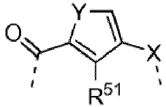 |
| **A213** (a7) | **A214** (a7) | **A215** (a7) | **A216** (a7) |
| 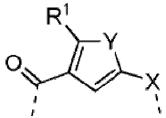 | 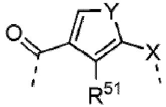 | 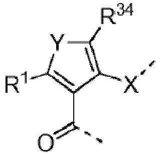 | 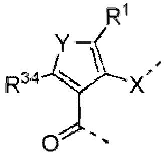 |
| **A217** (a7) | **A218** (a7) | **A219** (a7) | **A220** (a7) |
| 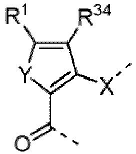 | 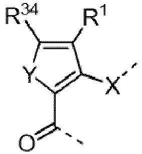 | 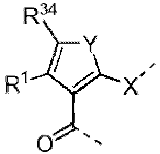 | 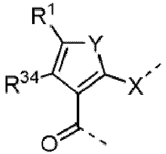 |
| **A221** (a7) | **A222** (a7) | **A223** (a7) | **A224** (a7) |
| 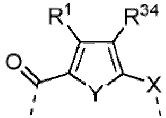 | 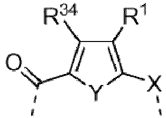 | 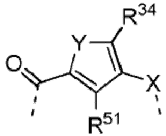 | 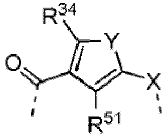 |
| **A225** (a7) | **A226** (a7) | **A227** (a7) | **A228** (a7) |
| 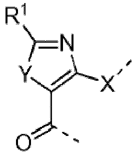 | 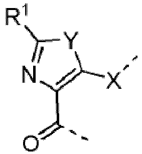 | 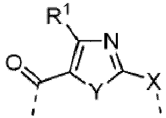 | 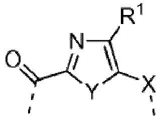 |
| **A229** (a8) | **A230** (a8) | **A231** (a8) | **A232** (a8) |
| 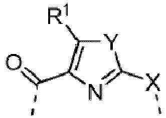 | 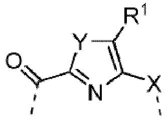 | 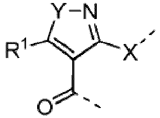 | 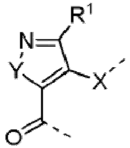 |
| **A233** (a8) | **A234** (a8) | **A235** (a9) | **A236** (a9) |
| 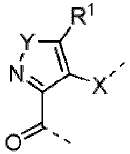 | 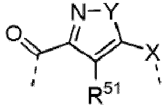 | 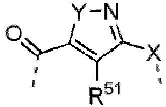 | 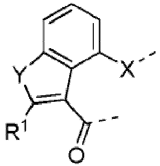 |
| **A237** (a9) | **A238** (a9) | **A239** (a9) | **A240** (a10) |
| 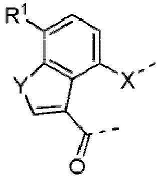 | 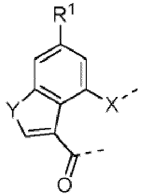 | 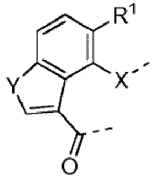 | 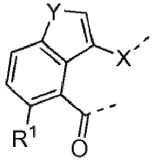 |
| **A241** (a10) | **A242 (**a10) | **A243** (a10) | **A244** (a10) |
| 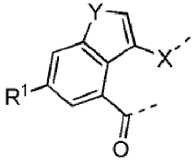 | 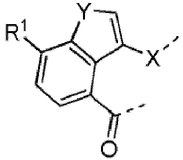 | 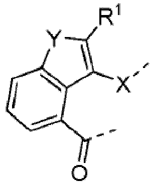 | 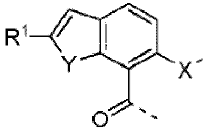 |
| **A245** (a10) | **A246** (a10) | **A247** (a10) | **A248** (a10) |
| 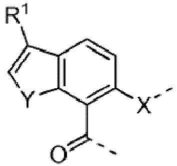 | 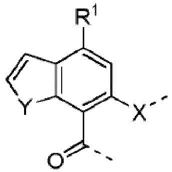 | 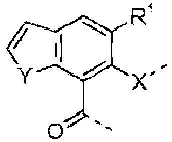 | 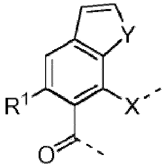 |
| **A249** (a10) | **A250** (a10) | **A251** (a10) | **A252** (a10) |
| 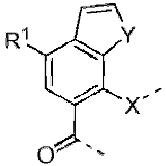 | 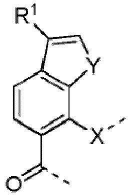 | 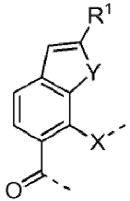 | 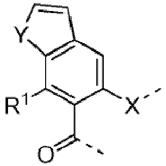 |
| **A253** (a10) | **A254** (a10) | **A255** (a10) | **A256** (a10) |
| 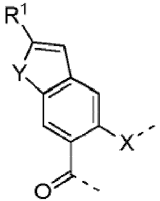 | 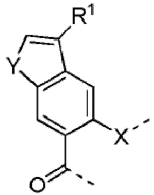 | 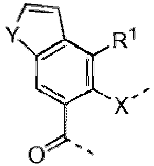 | 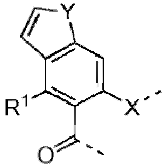 |
| **A257** (a10) | **A258** (a10) | **A259** (a10) | **A260** (a10) |
| 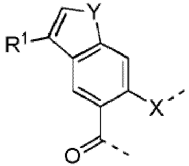 | 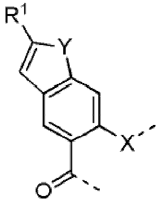 | 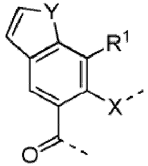 | 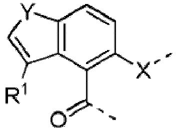 |
| **A261** (a10) | **A262** (a10) | **A263** (a10) | **A264** (a10) |
| 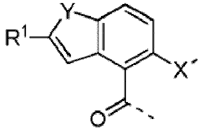 | 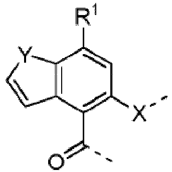 | 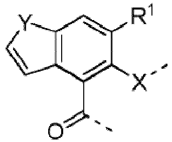 | 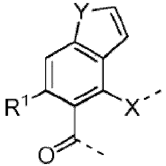 |
| **A265** (a10) | **A266** (a10) | **A267** (a10) | **A268** (a10) |
| 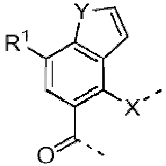 | 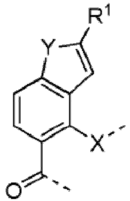 | 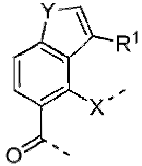 | 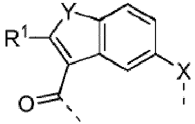 |
| **A269** (a10) | **A270** (a10) | **A271** (a10) | **A272** (a10) |
| 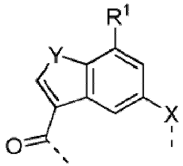 | 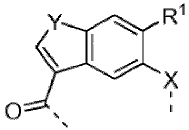 | 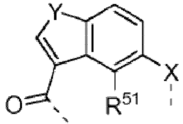 | 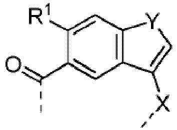 |
| **A273** (a10) | **A274** (a10) | **A275** (a10) | **A276** (a10) |
| 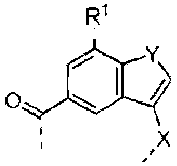 | 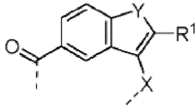 | 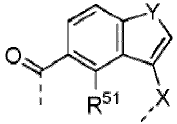 | 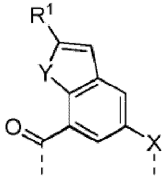 |
| **A277** (a10) | **A278** (a10) | **A279** (a10) | **A280** (a10) |
| 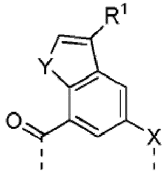 | 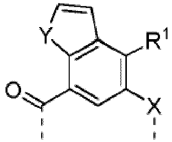 | 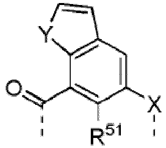 | 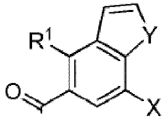 |
| **A281** (a10) | **A282** (a10) | **A283** (a10) | **A284** (a10) |
| 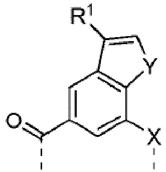 | 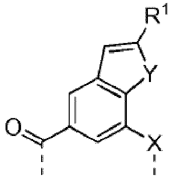 | 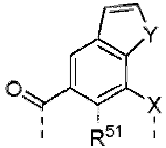 | 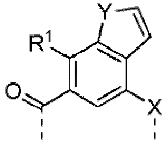 |
| **A285** (a10) | **A286** (a10) | **A287** (a10) | **A288** (a10) |
| 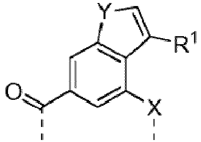 | 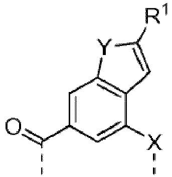 | 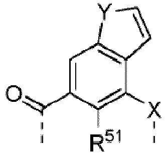 | 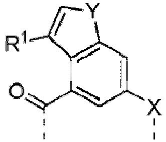 |
| **A289** (a10) | **A290** (a10) | **A291** (a10) | **A292** (a10) |
| 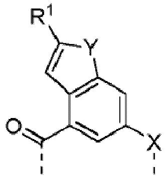 | 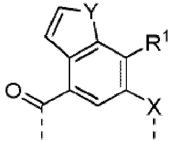 | 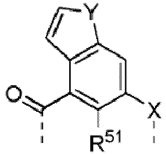 | 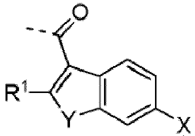 |
| **A293** (a10) | **A294** (a10) | **A295** (a10) | **A290** (a10) |
| 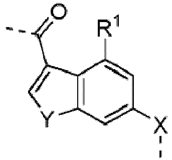 | 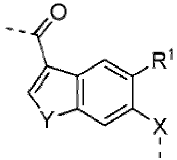 | 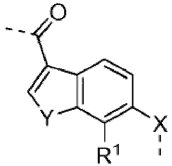 | 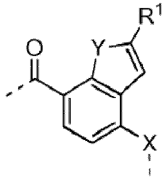 |
| **A297** (a10) | **A298** (a10) | **A299** (a10) | **A300** (a10) |
| 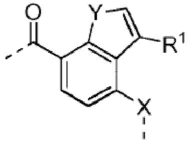 | 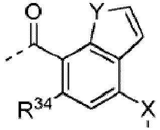 | 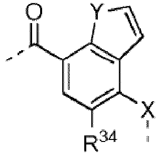 | 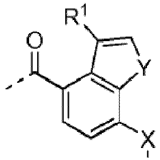 |
| **A301** (a10) | **A302** (a10) | **A303** (a10) | **A304** (a10) |
| 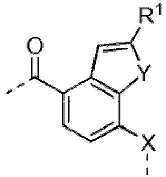 | 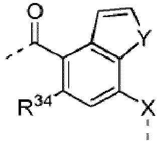 | 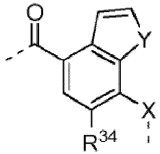 | 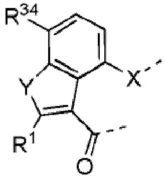 |
| **A305** (a10) | **A306** (a10) | **A307** (a10) | **A308** (a10) |
| 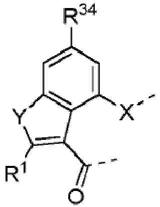 | 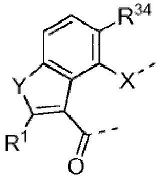 | 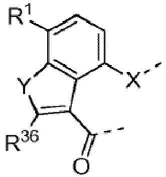 | 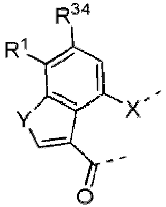 |
| **A309** (a10) | **A310** (a10) | **A311** (a10) | **A312** (a10) |
| 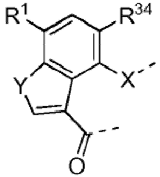 | 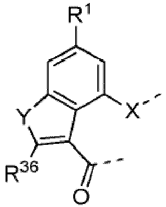 | 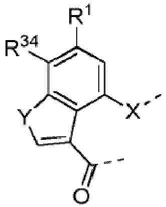 | 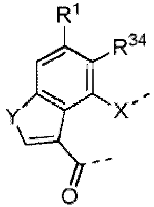 |
| **A313** (a10) | **A314** (a10) | **A315** (a10) | **A316** (a10) |
| 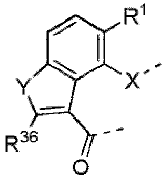 | 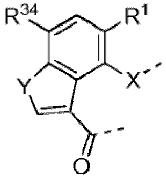 | 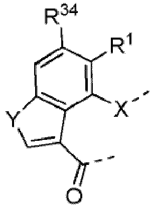 | 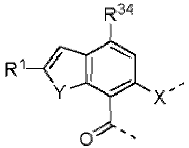 |
| **A317** (a10) | **A318** (a10) | **A319** (a10) | **A320** (a10) |
| 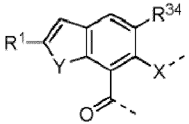 | 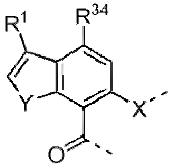 | 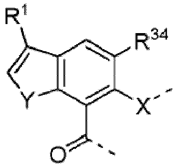 | 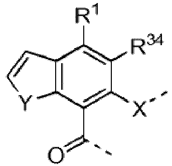 |
| **A321** (a10) | **A322** (a10) | **A323** (a10) | **A324** (a10) |
| 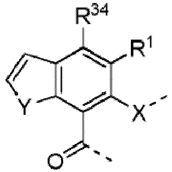 | 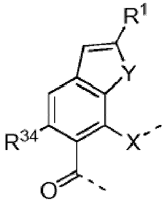 | 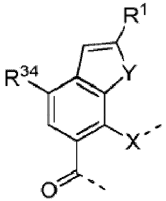 | 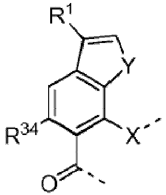 |
| **A325** (a10) | **A326** (a10) | **A327** (a10) | **A328** (a10) |
| 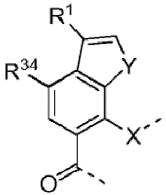 | 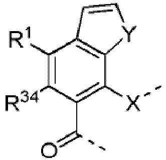 | 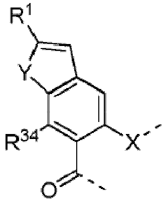 | 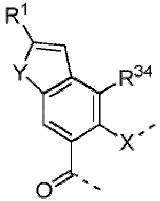 |
| **A329** (a10) | **A330** (a10) | **A331** (a10) | **A332** (a10) |
| 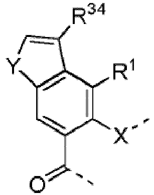 | 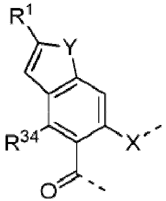 | 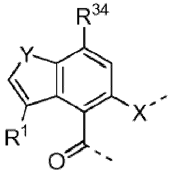 | 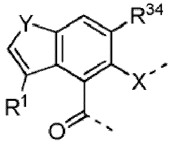 |
| **A333** (a10) | **A334** (a10) | **A335** (a10) | **A336** (a10) |
| 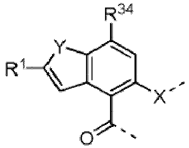 | 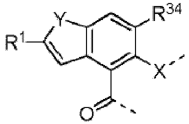 | 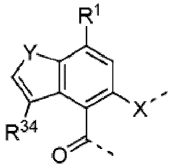 | 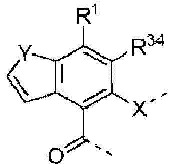 |
| **A337** (a10) | **A338** (a10) | **A339** (a10) | **A340** (a10) |
| 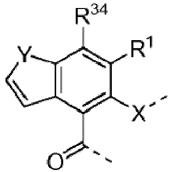 | 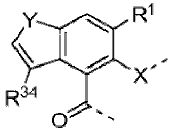 | 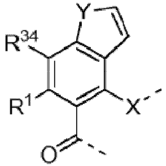 | 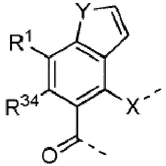 |
| **A341** (a10) | **A342** (a10) | **A343** (a10) | **A344** (a10) |
| 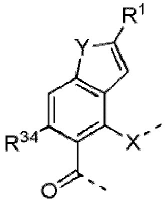 | 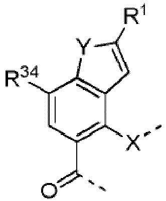 | 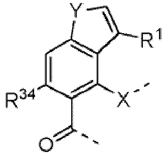 | 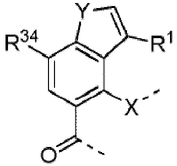 |
| **A345** (a10) | **A346** (a10) | **A347** (a10) | **A348** (a10) |
| 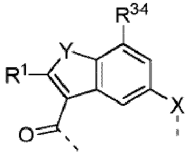 | 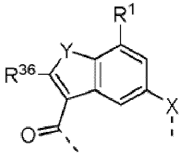 | 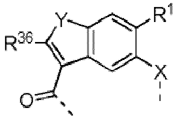 | 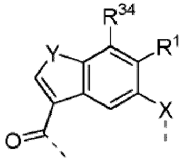 |
| **A349** (a10) | **A350** (a10) | **A351** (a10) | **A352** (a10) |
| 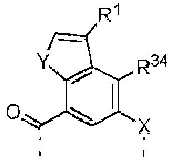 | 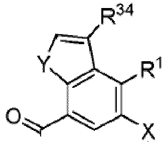 | 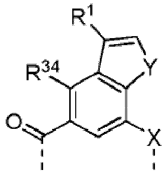 | 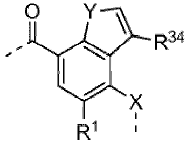 |
| **A353** (a10) | **A354** (a10) | **A355** (a10) | **A356** (a10) |
| 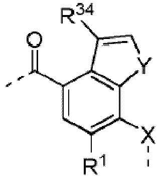 | 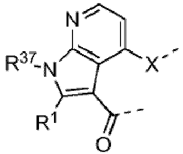 | 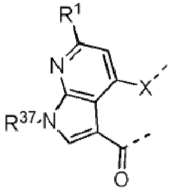 | 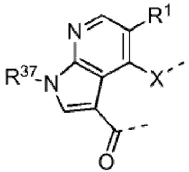 |
| **A357** (a10) | **A358** (a11) | **A359** (a11) | **A360** (a11) |
| 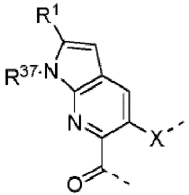 | 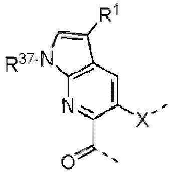 | 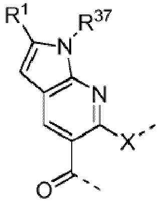 | 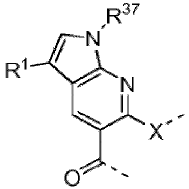 |
| **A361** (a11) | **A362** (a11) | **A363** (a11) | **A364** (a11) |
| 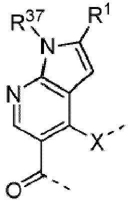 | 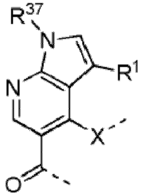 | 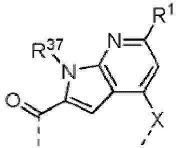 | 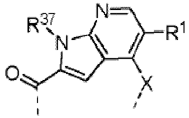 |
| **A365** (a11) | **A366** (a11) | **A367** (a11) | **A368** (a11) |
| 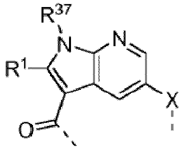 | 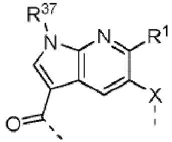 | 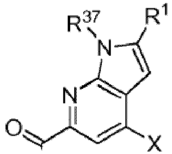 | 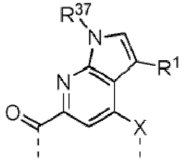 |
| **A369** (a11) | **A370** (a11) | **A371** (a11) | **A372** (a11) |
| 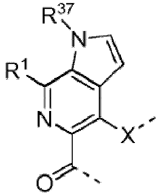 | 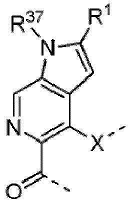 | 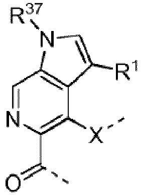 | 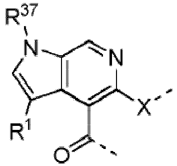 |
| **A373** (a12) | **A374** (a12) | **A375** (a12) | **A376** (a12) |
| 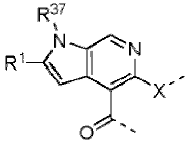 | 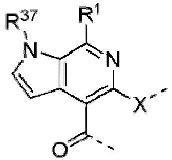 | 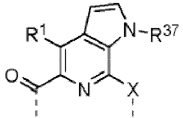 | 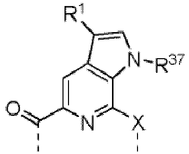 |
| **A377** (a12) | **A378** (a12) | **A379** (a12) | **A380** (a12) |
| 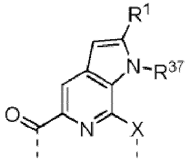 | 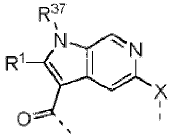 | 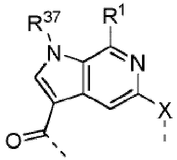 | 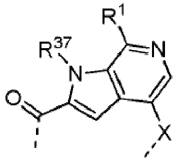 |
| **A381** (a12) | **A382** (a12) | **A383** (a12) | **A384** (a12) |
| 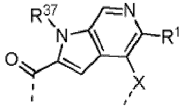 | 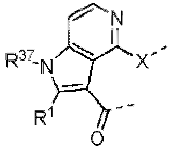 | 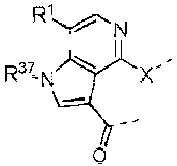 | 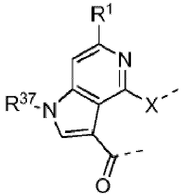 |
| **A385** (a12) | **A386** (a13) | **A387** (a13) | **A388** (a13) |
| 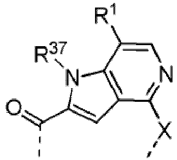 | 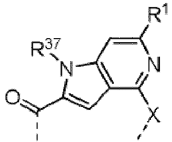 | 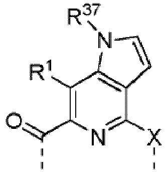 | 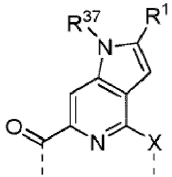 |
| **A389** (a13) | **A390** (a13) | **A391** (a13) | **A392** (a13) |
| 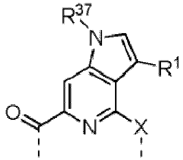 | 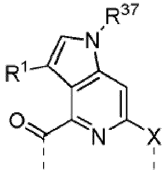 | 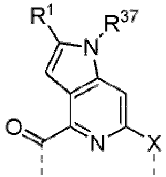 | 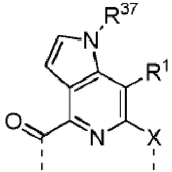 |
| **A393** (a13) | **A394** (a13) | **A395** (a13) | **A396** (a13) |
| 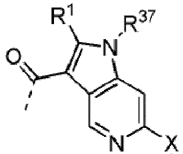 | 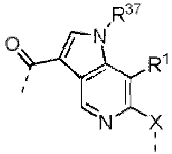 | 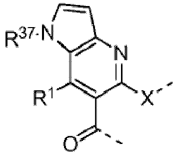 | 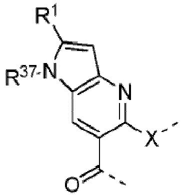 |
| **A397** (a13) | **A398** (a13) | **A399** (a14) | **A400** (a14) |
| 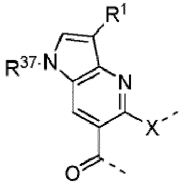 | 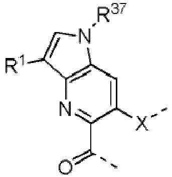 | 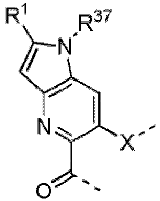 | 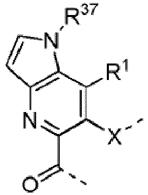 |
| **A401** (a14) | **A402** (a14) | **A403** (a14) | **A404** (a14) |
| 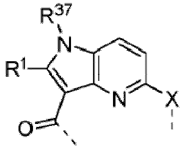 | 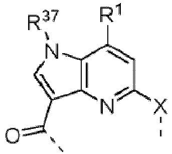 | 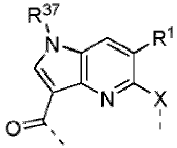 | 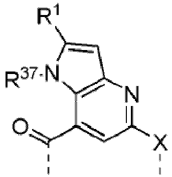 |
| **A405** (a14) | **A406** (a14) | **A407** (a14) | **A408** (a14) |
| 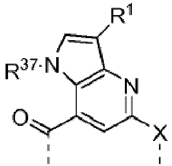 | 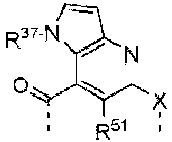 | 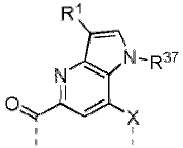 | 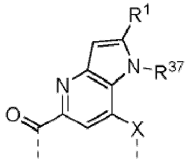 |
| **A409** (a14) | **A410** (a14) | **A411** (a14) | **A412** (a14) |
| 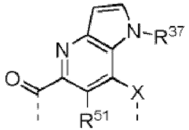 | 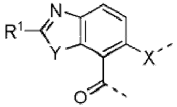 | 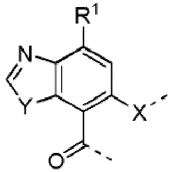 | 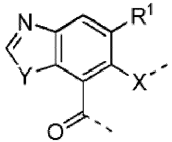 |
| **A413** (a14) | **A414** (a15) | **A415** (a15) | **A416** (a15) |
| 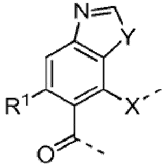 | 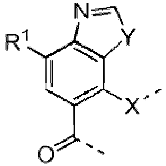 | 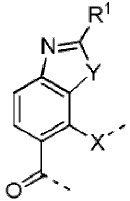 | 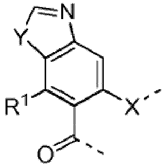 |
| **A417** (a15) | **A418** (a15) | **A419** (a15) | **A420** (a15) |
| 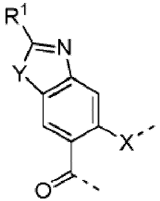 | 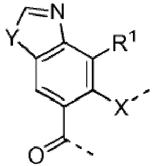 | 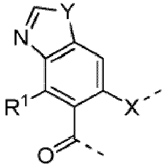 | 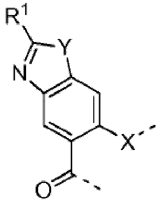 |
| **A421** (a15) | **A422** (a15) | **A423** (a15) | **A424** (a15) |
| 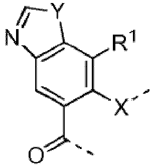 | 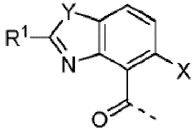 | 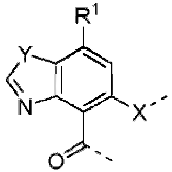 | 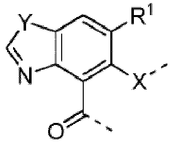 |
| **A425** (a15) | **A426** (a15) | **A427** (a15) | **A428** (a15) |
| 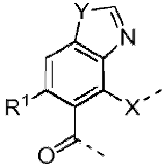 | 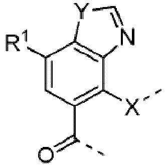 | 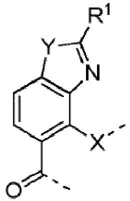 | 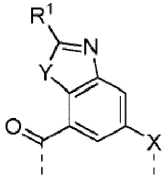 |
| **A429** (a15) | **A430** (a15) | **A431** (a15) | **A432** (a15) |
| 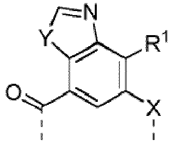 | 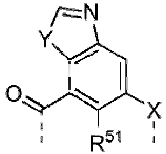 | 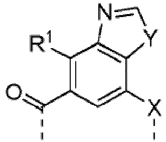 | 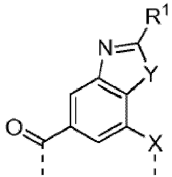 |
| **A433** (a15) | **A434** (a15) | **A435** (a15) | **A436** (a15) |
| 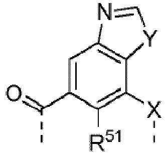 | 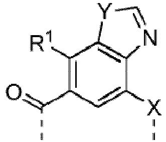 | 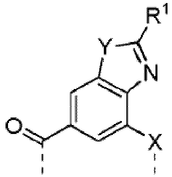 | 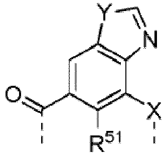 |
| **A437** (a15) | **A438** (a15) | **A439** (a15) | **A440** (a15) |
| 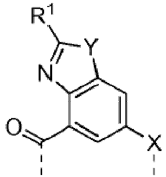 | 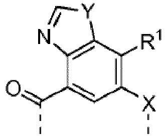 | 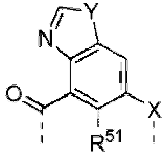 | 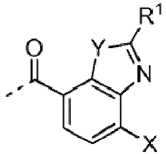 |
| **A441** (a15) | **A442** (a15) | **A443** (a15) | **A444** (a15) |
| 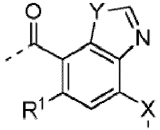 | 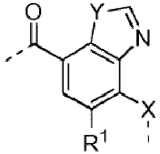 | 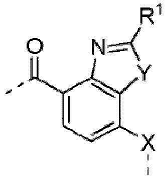 | 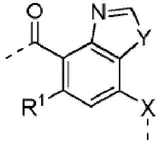 |
| **A445** (a15) | **A446** (a15) | **A447** (a15) | **A448** (a15) |
| 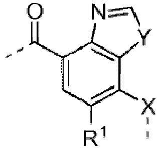 | 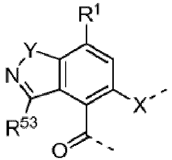 | 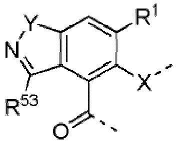 | 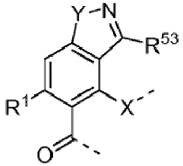 |
| **A449** (a15) | **A450** (a16) | **A451** (a16) | **A452** (a16) |
| 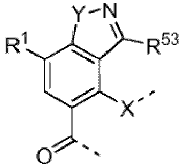 | 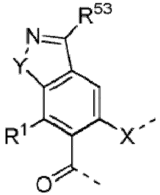 | 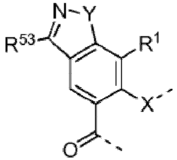 | 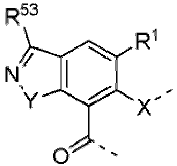 |
| **A453** (a16) | **A454** (a16) | **A455** (a16) | **A456** (a16) |
| 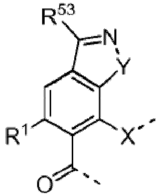 | 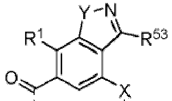 | 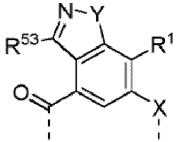 | 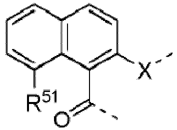 |
| **A457** (a16) | **A458** (a16) | **A459** (a16) | **A460** (a17) |
| 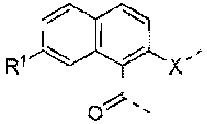 | 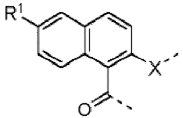 | 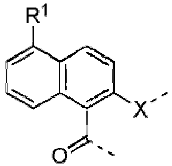 | 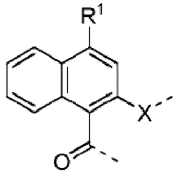 |
| **A461** (a17) | **A462** (a17) | **A463** (a17) | **A464** (a17) |
| 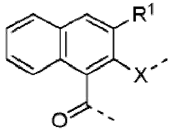 | 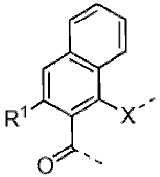 | 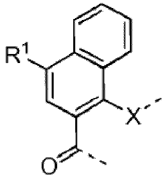 | 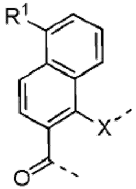 |
| **A465** (a17) | **A466** (a17) | **A467** (a17) | **A468** (a17) |
| 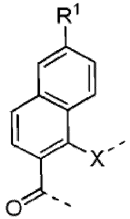 | 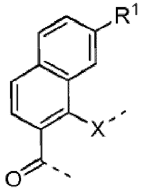 | 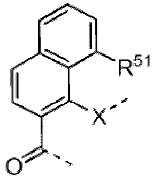 | 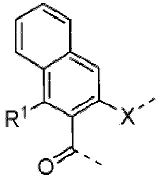 |
| **A469** (a17) | **A470** (a17) | **A471** (a17) | **A472** (a17) |
| 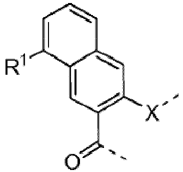 | 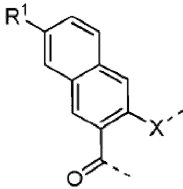 | 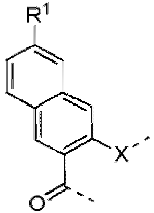 | 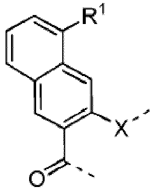 |
| **A473** (a17) | **A474** (a17) | **A475** (a17) | **A476** (a17) |
| 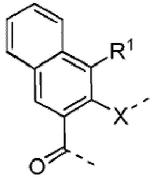 | 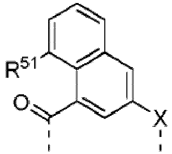 | 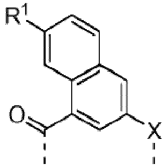 | 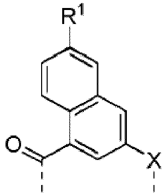 |
| **A477** (a17) | **A478** (a17) | **A479** (a17) | **A480** (a17) |
| 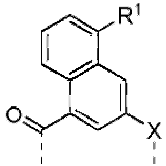 | 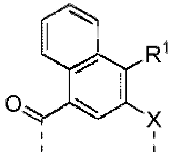 | 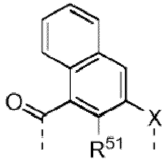 | 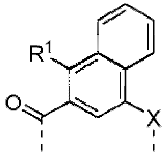 |
| **A481** (a17) | **A482** (a17) | **A483** (a17) | **A484** (a17) |
| 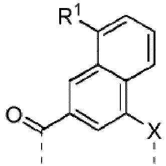 | 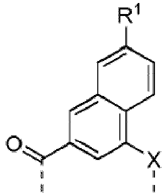 | 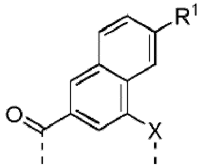 | 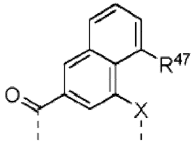 |
| **A485** (a17) | **A486** (a17) | **A487** (a17) | **A488** (a17) |
| 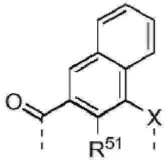 | 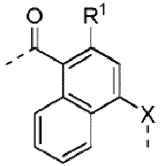 | 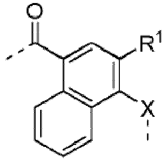 | 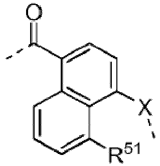 |
| **A489** (a17) | **A490** (a17) | **A491** (a17) | **A492** (a17) |
| 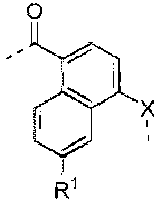 | 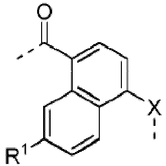 | 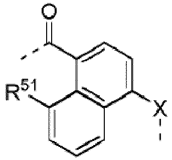 | 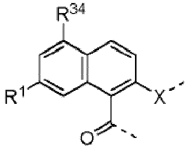 |
| **A493** (a17) | **A494** (a17) | **A495** (a17) | **A496** (a17) |
| 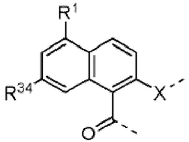 | 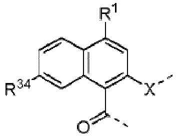 | 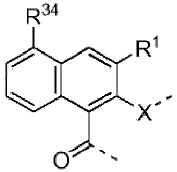 | 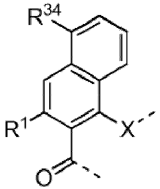 |
| **A497** (a17) | **A498** (a17) | **A499** (a17) | **A500** (a17) |
| 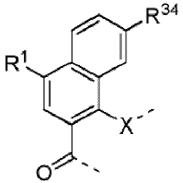 | 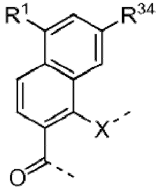 | 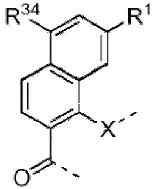 | 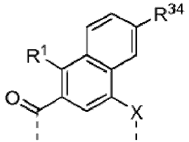 |
| **A501** (a17) | **A502** (a17) | **A503** (a17) | **A504** (a17) |
| 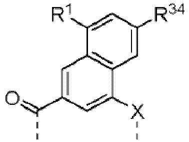 | 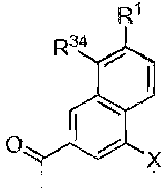 | 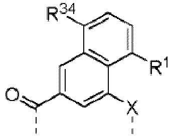 | 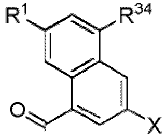 |
| **A505** (a17) | **A506** (a17) | **A507** (a17) | **A508** (a17) |
| 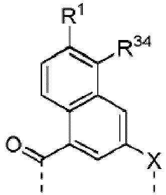 | 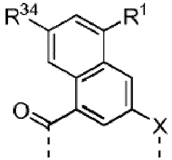 | 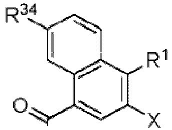 | 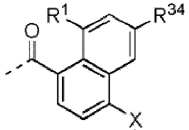 |
| **A509** (a17) | **A510** (a17) | **A511** (a17) | **A512** (a17) |
| 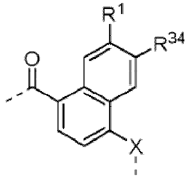 | 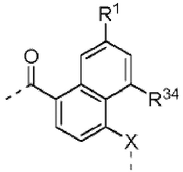 | 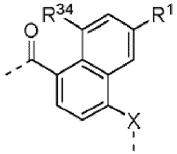 | 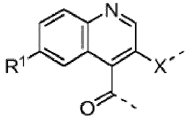 |
| **A513** (a17) | **A514** (a17) | **A515** (a17) | **A516** (a18) |
| 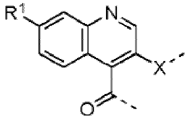 | 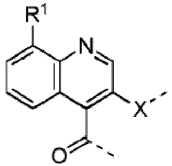 | 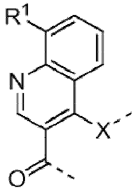 | 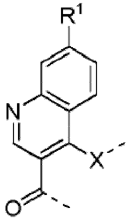 |
| **A517** (a18) | **A518** (a18) | **A519** (a18) | **A520** (a18) |
| 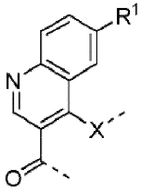 | 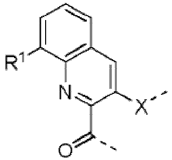 | 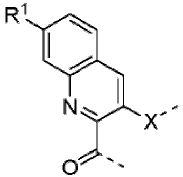 | 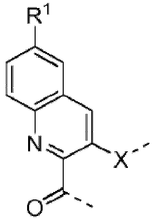 |
| **A521** (a18) | **A522** (a18) | **A523** (a18) | **A524** (a18) |
| 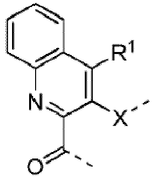 | 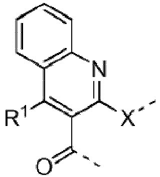 | 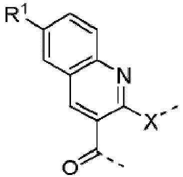 | 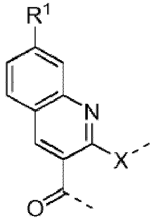 |
| **A525** (a18) | **A526** (a18) | **A527** (a18) | **A528** (a18) |
| 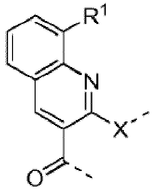 | 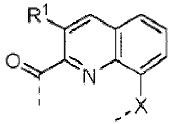 | 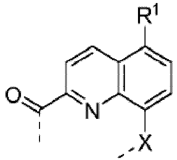 | 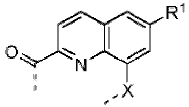 |
| **A529** (a18) | **A530** (a18) | **A531** (a18) | **A532** (a18) |
| 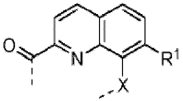 | 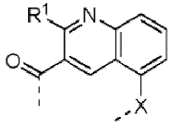 | 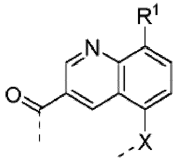 | 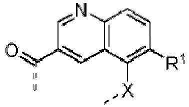 |
| **A533** (a18) | **A534** (a18) | **A535** (a18) | **A536** (a18) |
| 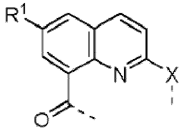 | 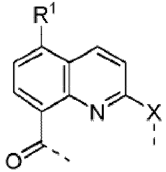 | 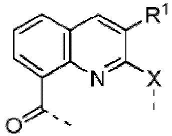 | 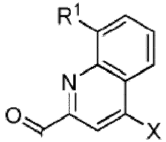 |
| **A537** (a18) | **A538** (a18) | **A539** (a18) | **A540** (a18) |
| 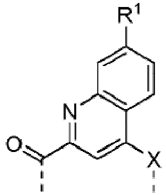 | 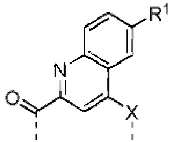 | 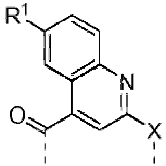 | 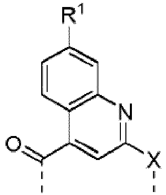 |
| **A541** (a18) | **A542** (a18) | **A543** (a18) | **A544** (a18) |
| 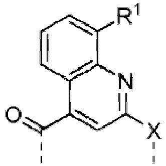 | 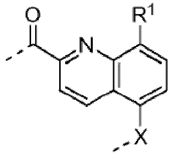 | 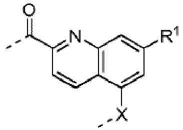 | 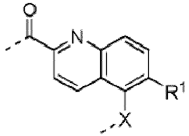 |
| **A545** (a18) | **A546** (a18) | **A547** (a18) | **A548** (a18) |
| 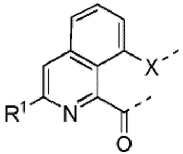 | 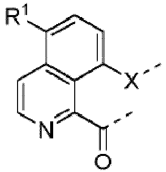 | 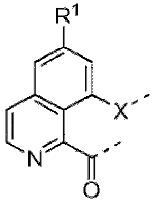 | 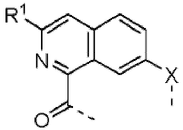 |
| **A549** (a19) | **A550** (a19) | **A551** (a19) | **A552** (a19) |
| 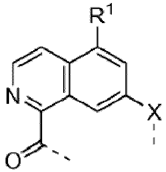 | 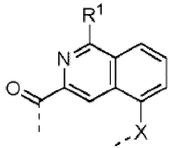 | 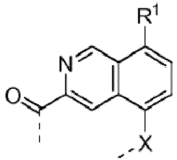 | 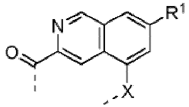 |
| **A553** (a19) | **A554** (a19) | **A555** (a19) | **A556** (a19) |
| 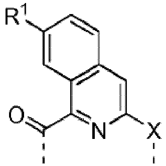 | 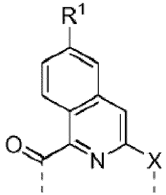 | 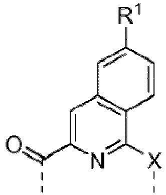 | 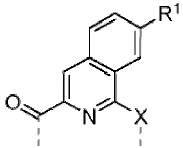 |
| **A557** (a19) | **A558** (a19) | **A559** (a19) | **A560** (a19) |
| 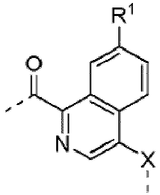 | 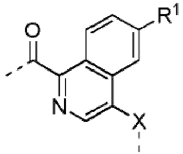 | 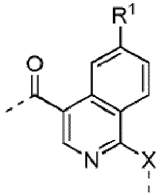 | 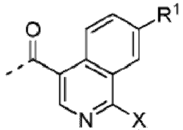 |
| **A561** (a19) | **A562** (a19) | **A563** (a19) | **A564** (a19) |
| 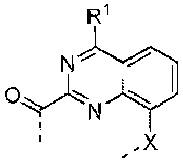 | 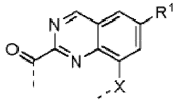 | 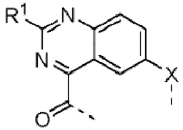 | 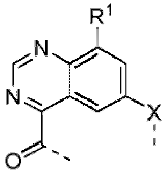 |
| **A565** (a20) | **A566** (a20) | **A567** (a20) | **A568** (a20) |
| 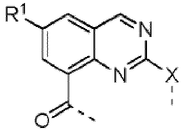 | 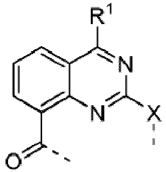 | 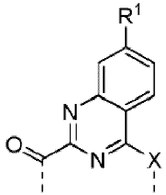 | 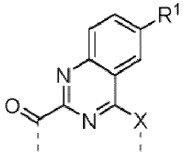 |
| **A569** (a20) | **A570** (a20) | **A571** (a20) | **A572** (a20) |
| 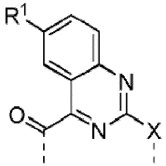 | 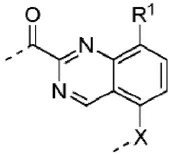 | 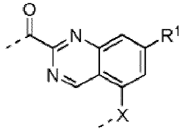 | 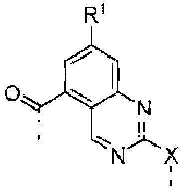 |
| **A573** (a20) | **A574** (a20) | **A575** (a20) | **A576** (a20) |
| 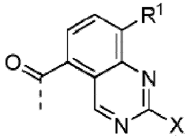 | 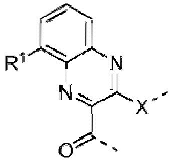 | 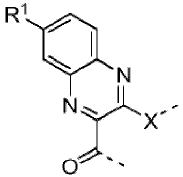 | 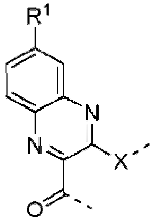 |
| **A577** (a20) | **A578** (a21) | **A579** (a21) | **A580** (a21) |
| 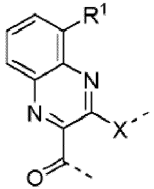 | 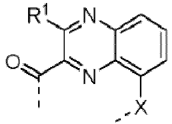 | 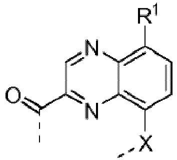 | 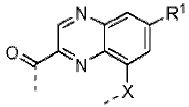 |
| **A581** (a21) | **A582** (a21) | **A583** (a21) | **A584** (a21) |
| 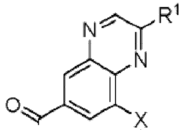 | 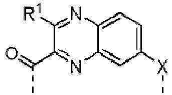 | 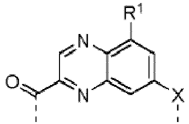 | 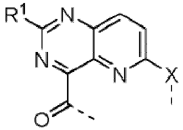 |
| **A585** (a21) | **A586** (a21) | **A587** (a21) | **A588** (a22) |
| 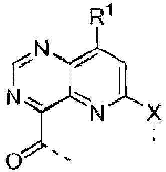 | 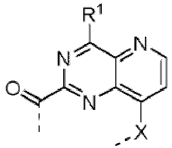 | 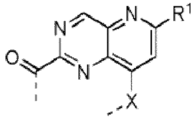 | 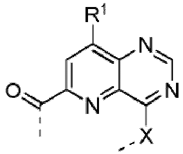 |
| **A589** (a22) | **A590** (a22) | **A591** (a22) | **A592** (a22) |
| 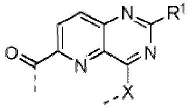 | 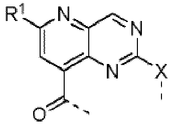 | 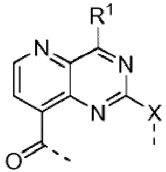 | 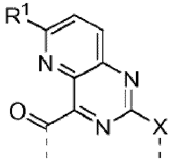 |
| **A593** (a22) | **A594** (a22) | **A595** (a22) | **A596** (a22) |
| 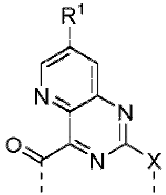 | 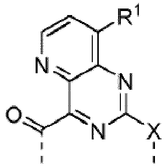 | 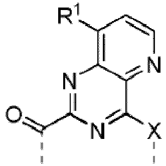 | 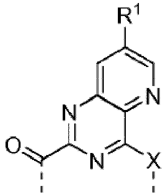 |
| **A597** (a22) | **A598** (a22) | **A599** (a22) | **A600** (a22) |
| 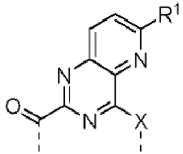 | 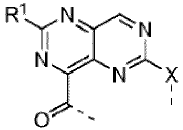 | 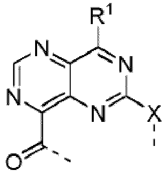 | 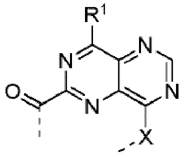 |
| **A601** (a22) | **A602** (a23) | **A603** (a23) | **A604** (a23) |
| 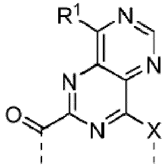 | 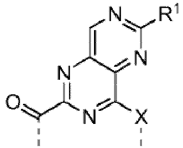 | 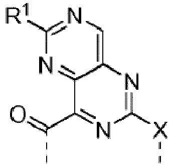 | 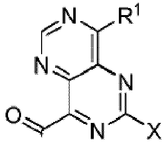 |
| **A605** (a23) | **A606** (a23) | **A607** (a23) | **A608** (a23) |
| 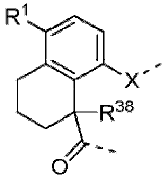 | 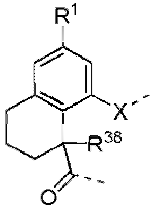 | 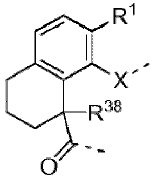 | 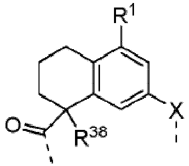 |
| **A609** (a24) | **A610** (a24) | **A611** (a24) | **A612** (a24) |
| 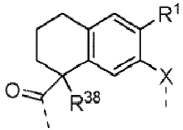 | 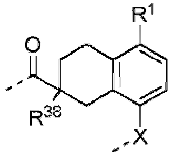 | 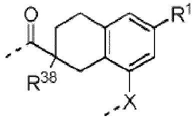 | 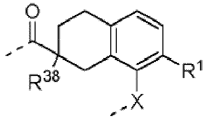 |
| **A613** (a24) | **A614** (a24) | **A615** (a24) | **A616** (a24) |
| 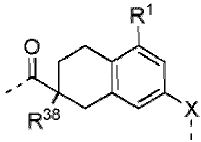 | 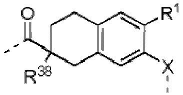 | 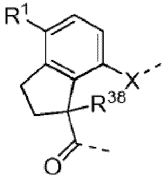 | 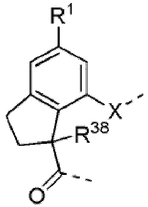 |
| **A617** (a24) | **A618** (a24) | **A619** (a25) | **A620** (a25) |
| 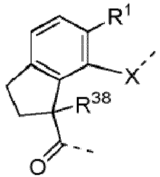 | 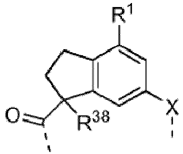 | 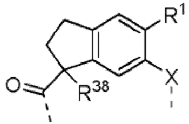 | 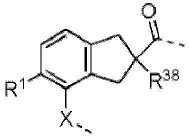 |
| **A621** (a25) | **A622** (a25) | **A623** (a25) | **A624** (a25) |
| 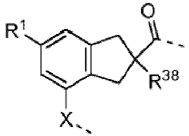 | 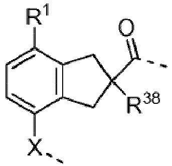 | 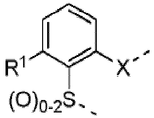 | 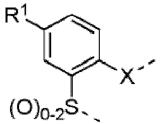 |
| **A625** (a25) | **A626** (a25) | **A627** (a1) | **A628** (a1) |
| 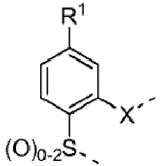 | 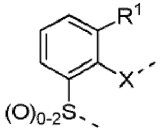 | 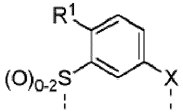 | 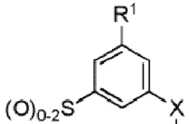 |
| **A629** (a1) | **A630** (a1) | **A631** (a1) | **A632** (a1) |
| 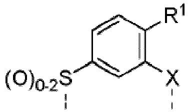 | 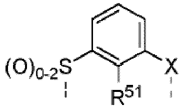 | 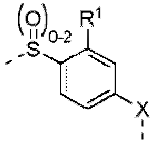 | 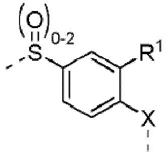 |
| **A633** (a1) | **A634** (a1) | **A635** (a1) | **A636** (a1) |
| 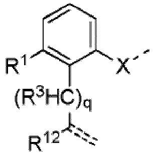 | 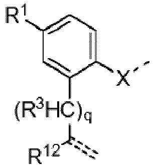 | 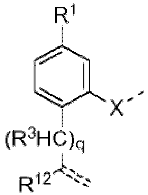 | 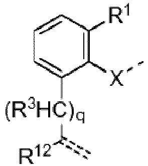 |
| **A637** (a1) | **A638** (a1) | **A639** (a1) | **A640** (a1) |
| 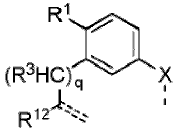 | 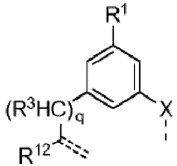 | 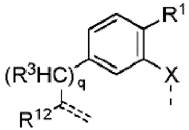 | 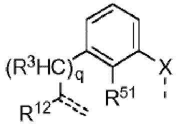 |
| **A641** (a1) | **A642** (a1) | **A643** (a1) | **A644** (a1) |
| 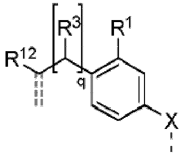 | 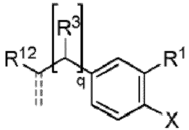 | 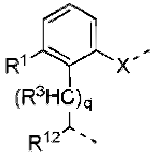 | 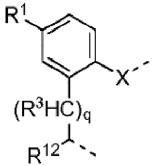 |
| **A645** (a1) | **A646** (a1) | **A647** (a1) | **A648** (a1) |
| 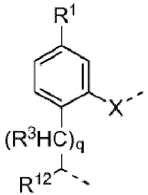 | 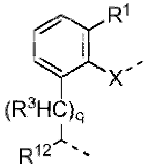 | 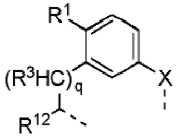 | 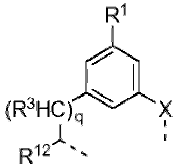 |
| **A649** (a1) | **A650** (a1) | **A651** (a1) | **A652** (a1) |
| 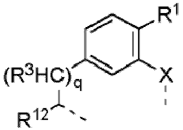 | 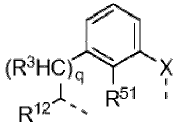 | 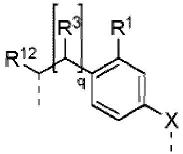 | 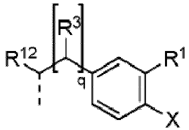 |
| **A653** (a1) | **A654** (a1) | **A655** (a1) | **A656** (a1) |
| 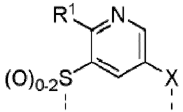 | 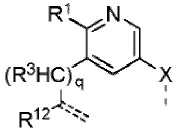 | 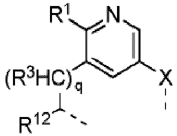 | 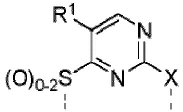 |
| **A657** (a2) | **A658** (a2) | **A659** (a2) | **A660** (a4) |
| 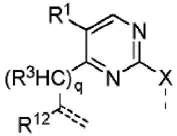 | 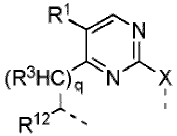 | 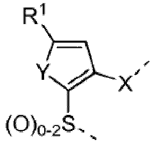 | 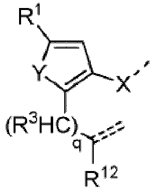 |
| **A661** (a4) | **A662** (a4) | **A663** (a7) | **A664** (a7) |
| 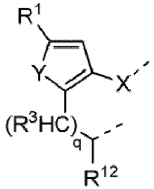 | 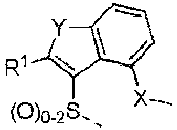 | 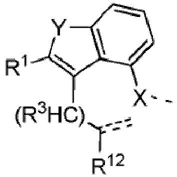 | 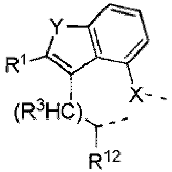 |
| **A665** (a7) | **A666** (a10) | **A667** (a10) | **A668** (a10) |
| 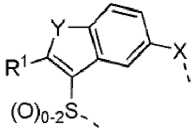 | 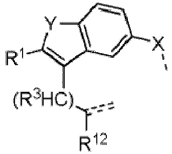 | 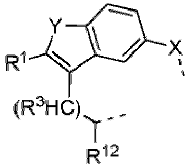 | 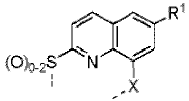 |
| **A669** (a10) | **A670** (a10) | **A671** (a10) | **A672** (a18) |
| 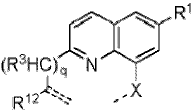 | 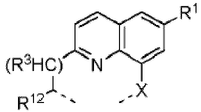 | 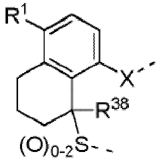 | 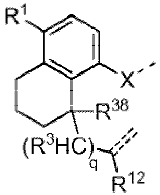 |
| **A673** (a18) | **A674** (a18) | **A675** (a24) | **A676** (a24) |
| 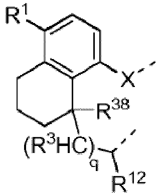 | 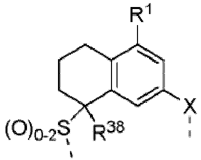 | 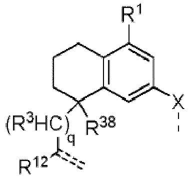 | 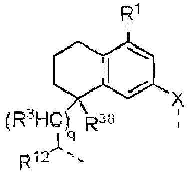 |
| **A677** (a24) | **A679** (a24) | **A679** (a24) | **A680** (a24) |
| 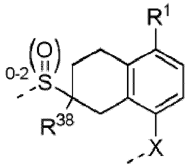 | 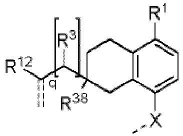 | | 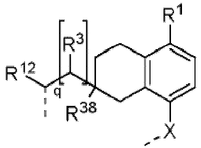 |
| **A681** (a24) | **A682** (a24) | | **A683** (a24) |
le groupe constitutif B est un radical bivalent choisi dans le groupe constitué de :
| | | | |
|---|---|---|---|
| 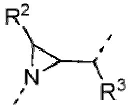 | 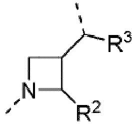 | 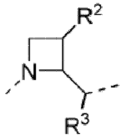 | 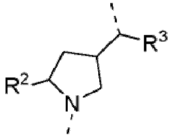 |
| **B1** (b1) | **B2** (b2) | **B3** (b2) | **B4** (b3) |
| 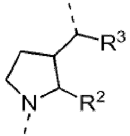 | 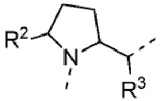 | 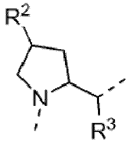 | 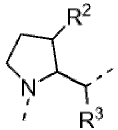 |
| **B5** (b3) | **B6** (b3) | **B7** (b3) | **B8** (b3) |
| 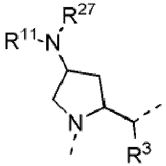 | 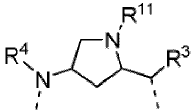 | 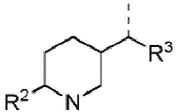 | 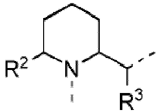 |
| **B9** (b3) | **B10** (b3) | **B11** (b4) | **B12** (b4) |
| 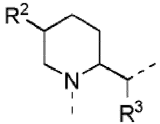 | 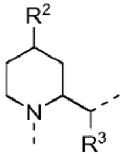 | 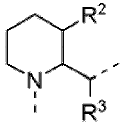 | 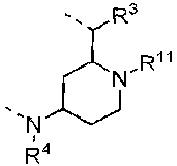 |
| **B13** (b4) | **B14** (b4) | **B15** (b4) | **B16** (b4) |
| 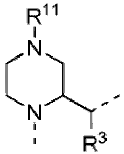 | 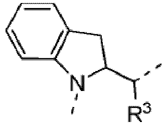 | 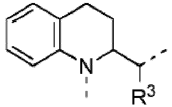 | 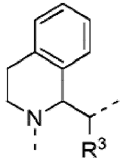 |
| **B17** (b5) | **B18** (b8) | **B19** (b10) | **B20** (b11) |
| 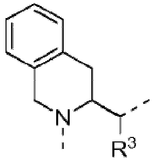 | | 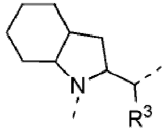 | |
| **B21** (b11) | | **B22** (b12) | |
le groupe constitutif C est un radical bivalent choisi dans le groupe constitué de :
| | |
|---|---|
| 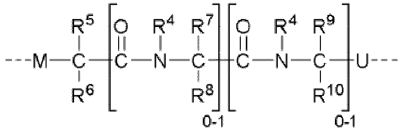 | 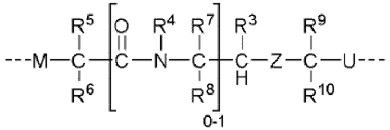 |
| **C1** | **C2** |
| 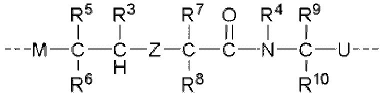 | 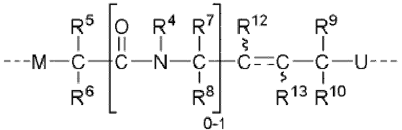 |
| **C3** | **C4** |
| 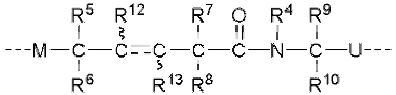 | 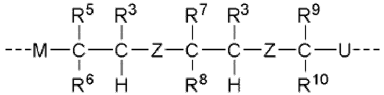 |
| **C5** | **C6** |
| 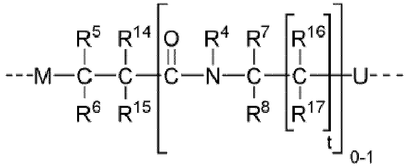 | 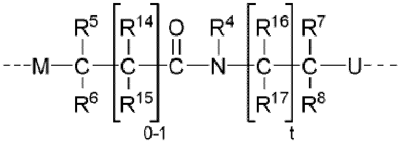 |
| **C7** | **C8** |
| 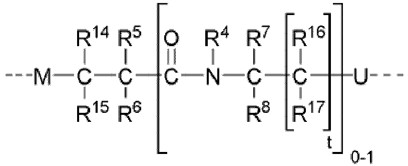 | 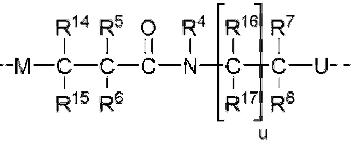 |
| **C9** | **C10** |
| 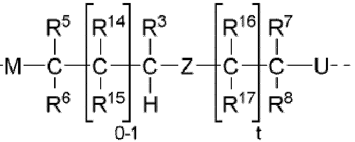 | 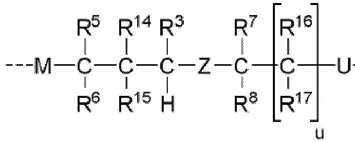 |
| **C11** | **C12** |
| 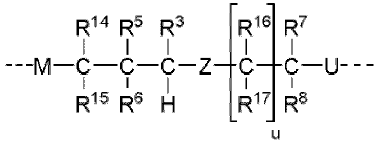 | 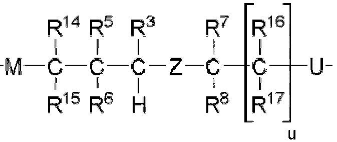 |
| **C13** | **C14** |
| 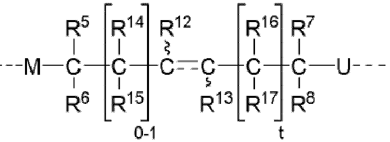 | 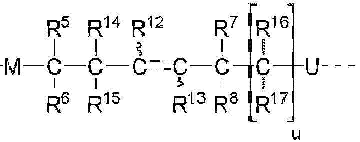 |
| **C15** | **C16** |
| 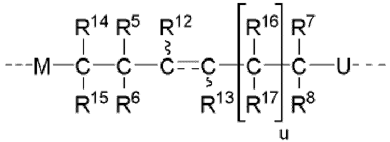 | 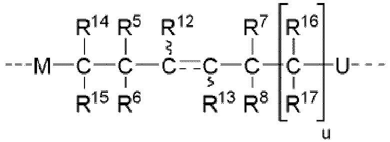 |
| **C17** | **C18** |
| 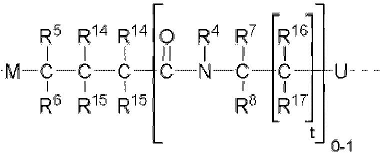 | 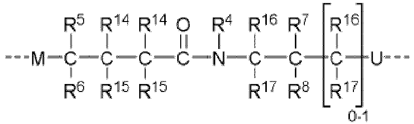 |
| **C19** | **C20** |
| 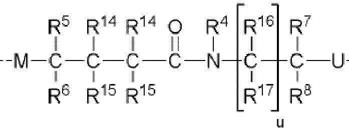 | 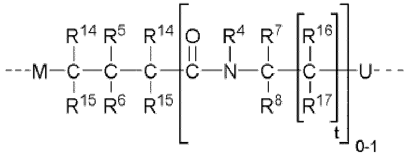 |
| **C21** | **C22** |
| 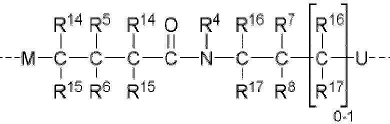 | 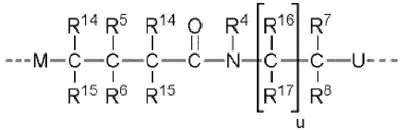 |
| **C23** | **C24** |
| 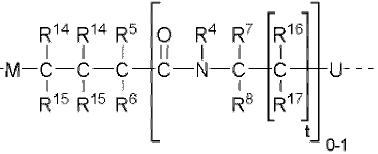 | 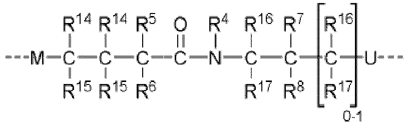 |
| **C25** | **C26** |
| 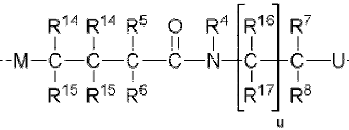 | 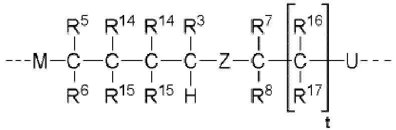 |
| **C27** | **C28** |
| 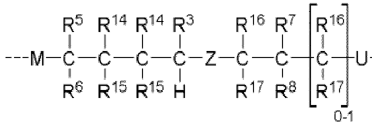 | 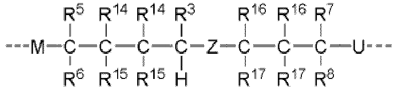 |
| **C29** | **C30** |
| 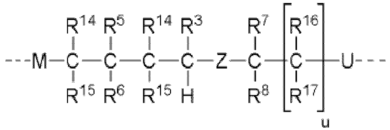 | 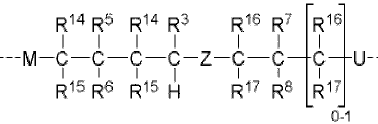 |
| **C31** | **C32** |
| 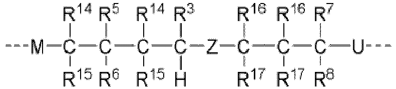 | 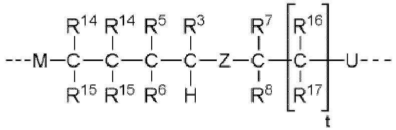 |
| **C33** | **C34** |
| 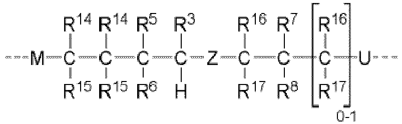 | 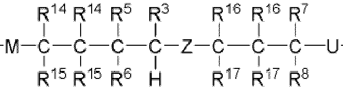 |
| **C35** | **C36** |
| 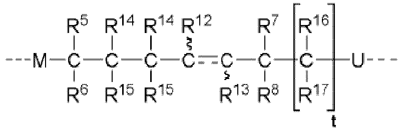 | 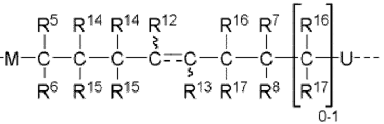 |
| **C37** | **C38** |
| 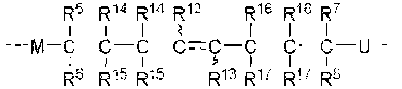 | 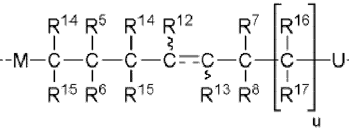 |
| **C39** | **C40** |
| 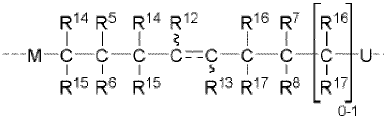 | 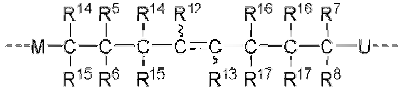 |
| **C41** | **C42** |
| 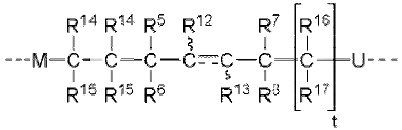 | 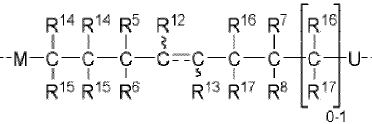 |
| **C43** | **C44** |
| 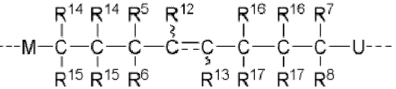 | 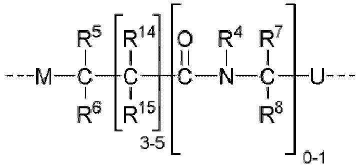 |
| **C45** | **C46** |
| 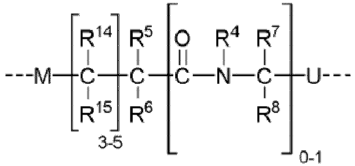 | 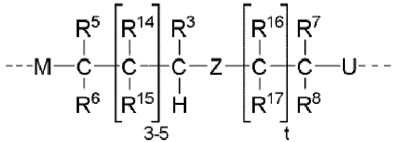 |
| **C47** | **C48** |
| 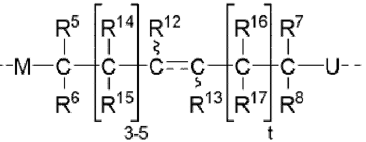 | 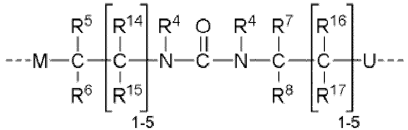 |
| **C49** | **C50** |
| 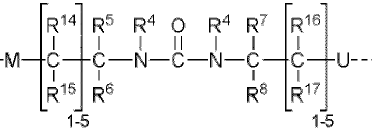 | 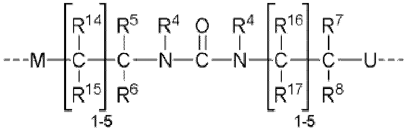 |
| **C51** | **C52** |
| 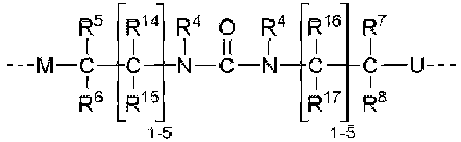 | |
| **C53** | |
| 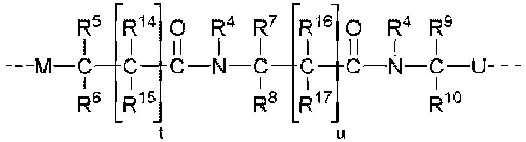 | |
| **C54** | |
| 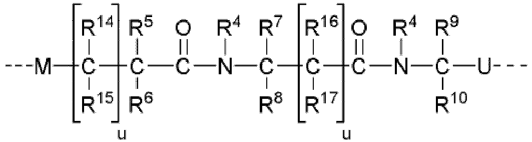 | |
| **C55** | |
| 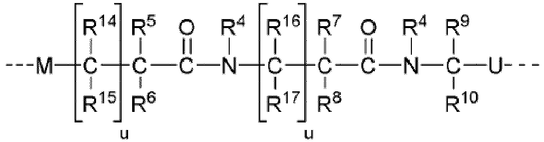 | |
| **C56** | |
| 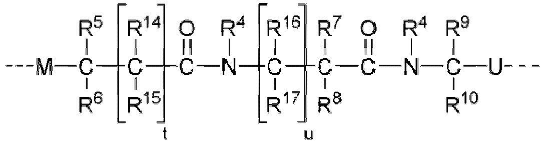 | |
| **C57** | |
| 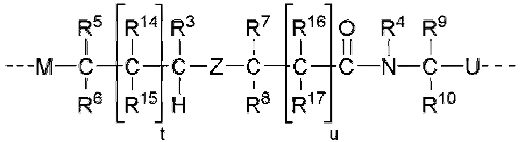 | |
| **C58** | |
| 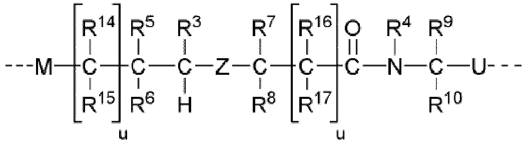 | |
| **C59** | |
| 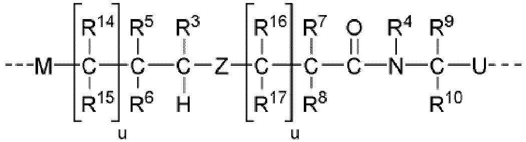 | |
| **C60** | |
| 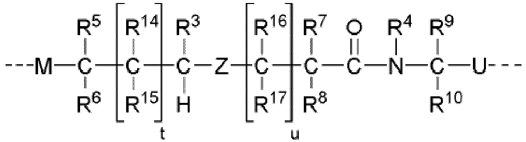 | |
| **C61** | |
| 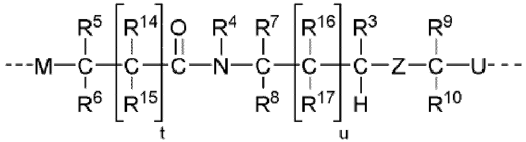 | |
| **C62** | |
| 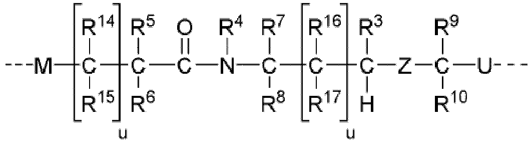 | |
| **C63** | |
| 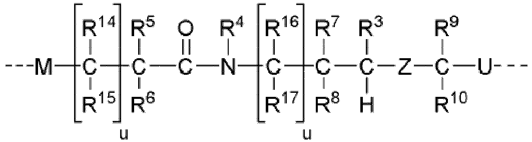 | |
| **C64** | |
| 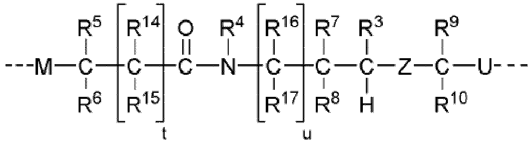 | |
| **C65** | |
| 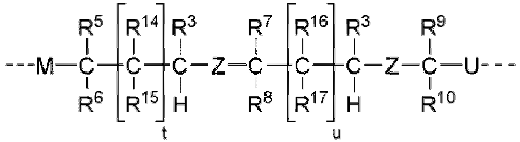 | |
| **C66** | |
| 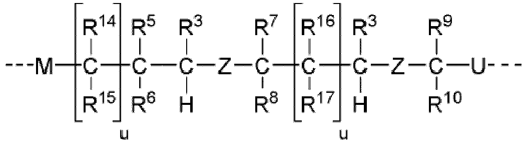 | |
| **C67** | |
| 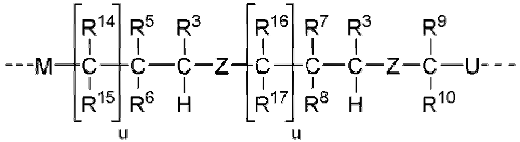 | |
| **C68** | |
| 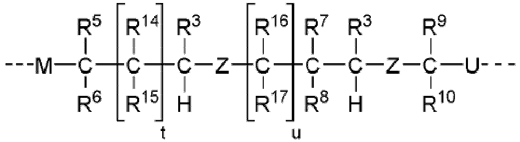 | |
| **C69** | |
| 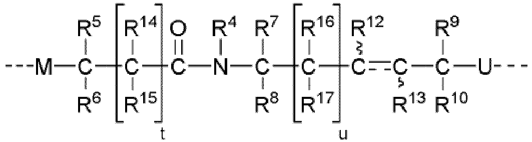 | |
| **C70** | |
| 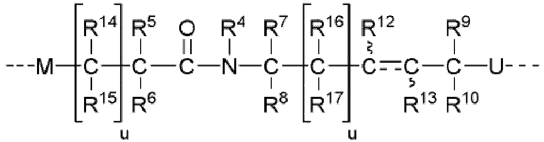 | |
| **C71** | |
| 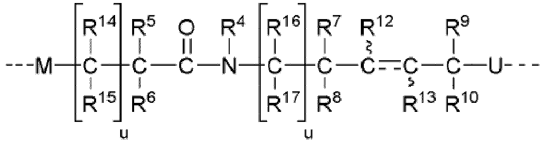 | |
| **C72** | |
| 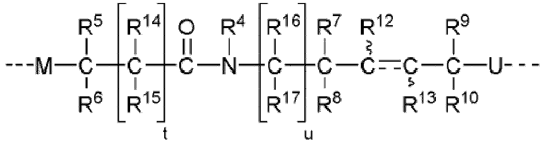 | |
| **C73** | |
| 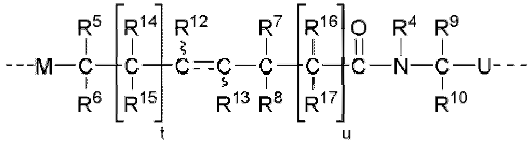 | |
| **C74** | |
| 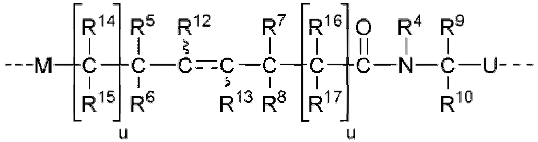 | |
| **C75** | |
| 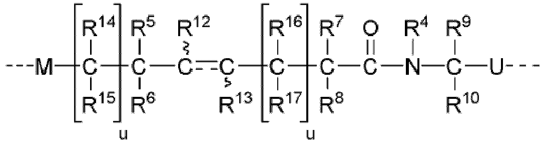 | |
| **C76** | |
| 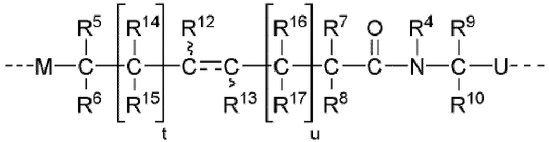 | |
| **C77** | |
| 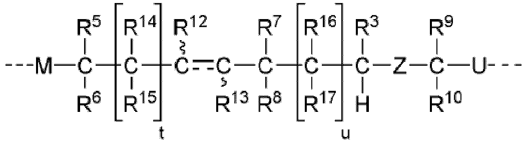 | |
| **C78** | |
| 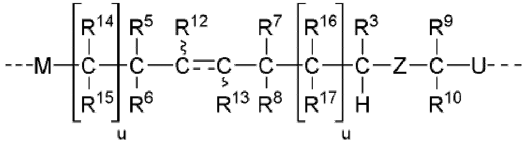 | |
| **C79** | |
| 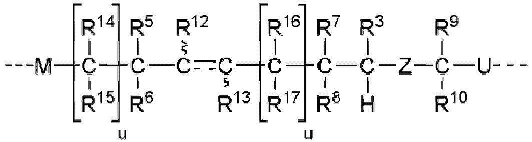 | |
| **C80** | |
| 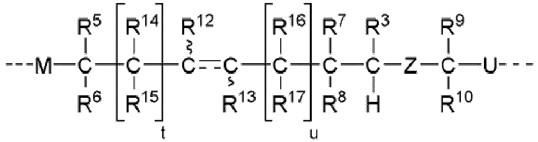 | |
| **C81** | |
| 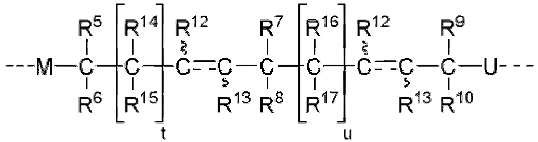 | |
| **C82** | |
| 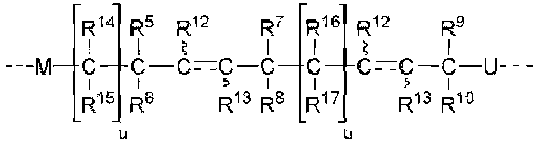 | |
| **C83** | |
| 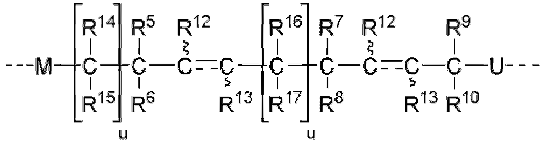 | |
| **C84** | |
| 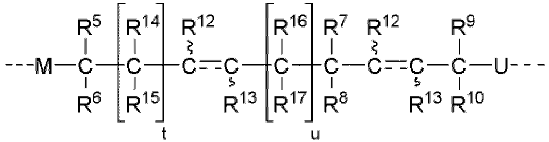 | |
| **C85** | |
| 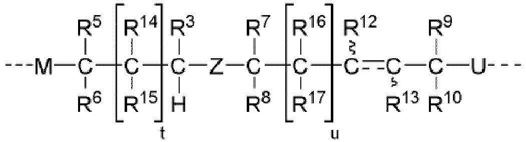 | |
| **C86** | |
| 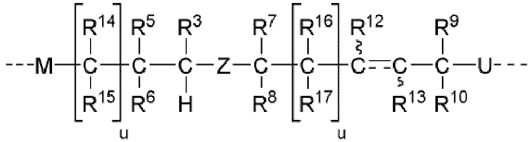 | |
| **C87** | |
| 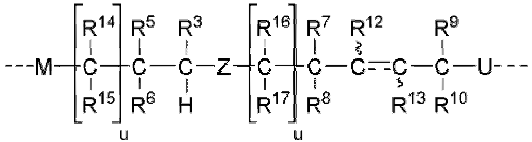 | |
| **C88** | |
| 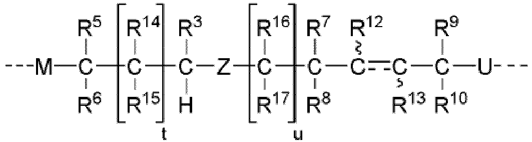 | |
| **C89** | |
| 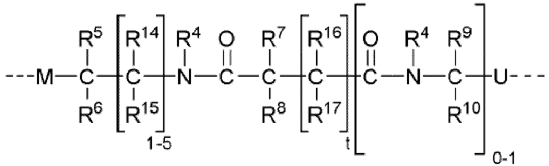 | |
| **C90** | |
| 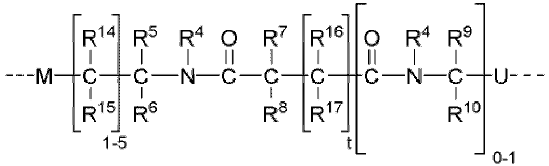 | |
| **C91** | |
| 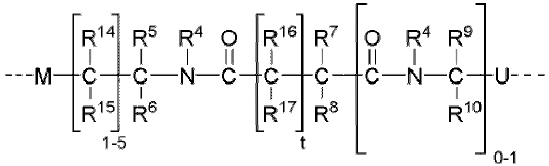 | |
| **C92** | |
| 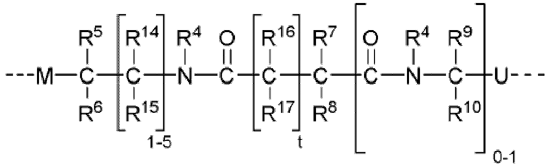 | |
| **C93** | |
| 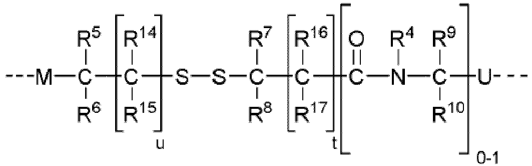 | |
| **C94** | |
| 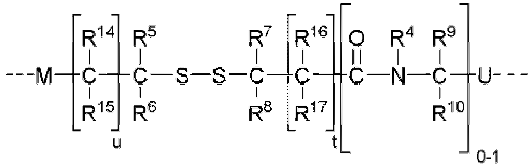 | |
| **C95** | |
| 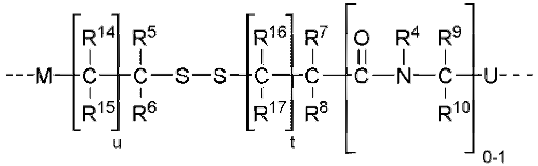 | |
| **C96** | |
| 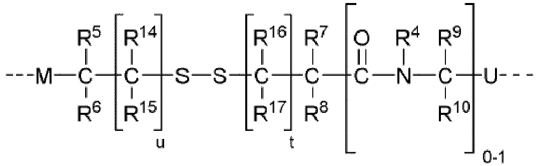 | |
| **C97** | |
| 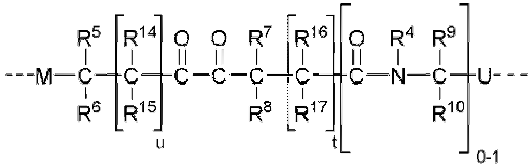 | |
| **C98** | |
| 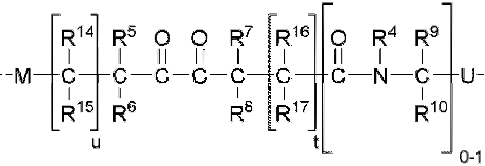 | |
| **C99** | |
| 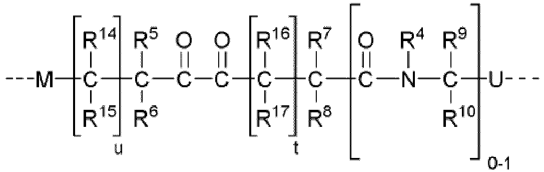 | |
| **C100** | |
| 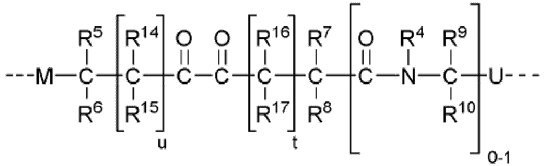 | |
| **C101** | |
et où, en outre,
R¹ représente H, F, Cl, Br, I, CF₃, OCF₃, OCHF₂, NO₂, CN, un alkyle, un alcényle, un alcynyle, un cycloalkyle, un hétérocycloalkyle, un aryle, un hétéroaryle, un arylalkyle, un hétéroarylalkyle, - (CR¹⁸R¹⁹)_{q}OR²⁰, -(CR¹⁸R¹⁹)_{q}SR²⁰, -(CR¹⁸R¹⁹)_{q}NR⁴R¹¹, -(CR¹⁸R¹⁹)_{q}OCONR⁴R¹¹, -(CR¹⁸R¹⁹)_{q}OCOOR²¹,-(CR¹⁸R¹⁹)_{q}NR⁴COOR²¹, -(CR¹⁸R¹⁹)_{q}NR⁴COR²², -(CR¹⁸R¹⁹)_{q}NR⁴CONR⁴R¹¹, -(CR¹⁸R¹⁹)_{q}NR⁴SO₂R²³,-(CR¹⁸R¹⁹)_{q}NR⁴SO₂NR⁴R¹¹ -(CR¹⁸R¹⁹)_{q}COOR²¹, -(CR¹⁸R¹⁹)_{q}CONR⁴R¹¹, -(CR¹⁸R¹⁹)_{q}SO₂NR⁴R¹¹,-(CR¹⁸R¹⁹)_{q}PO(OR²¹)₂, -(CR¹⁸R¹⁹)_{q}OPO(OR²¹)₂, -(CR¹⁸R¹⁹)_{q}COR²², -(CR¹⁸R¹⁹)_{q}SO₂R²³, -(CR¹⁸R¹⁹)_{q}OSO₃R²¹, -(CR¹⁸R¹⁹)_{q}R²⁴, -(CR¹⁸R¹⁹)_{q}R²⁵ ou -(CR¹⁸R¹⁹)_{q}R²⁶ ;
R² représente H, CF₃, un alkyle, un alcényle, un cycloalkyle, un hétérocycloalkyle, un aryle, un hétéroaryle, un arylalkyle, un hétéroarylalkyle, -(CR¹⁸R¹⁹)_{q}OR²⁰, -(CR¹⁸R¹⁹)_{q}SR²⁰, -(CR¹⁸R¹⁹)_{q}NR⁴R¹¹ (CR¹⁸R¹⁹)_{q}OCONR⁴R¹¹, -(CR¹⁸R¹⁹)_{q}OCOOR²¹, -(CR¹⁸R¹⁹)_{q}NR⁴COOR²¹, -(CR¹⁸R¹⁹)_{q}NR⁴COR²²,-(CR¹⁸R¹⁹)qNR⁴CONR⁴R¹¹ -(CR¹⁸R¹⁹)_{q}NR⁴SO₂R²³, -(CR¹⁸R¹⁹)_{q}NR⁴SO₂NR⁴R¹¹, -(CR¹⁸R¹⁹)_{q}COOR²¹,-(CR¹⁸R¹⁹)_{q}CONR⁴R¹¹, -(CR¹⁸R¹⁹)_{q}SO₂R⁴R¹¹, -(CR¹⁸R¹⁹)_{q}PO(OR²¹)₂, -(CR¹⁸R¹⁹)qCOR²² -(CR¹⁸R¹⁹)_{q}SO₂R²³,-(CR¹⁸R¹⁹)_{q}R²⁴, -(CR¹⁸R¹⁹)_{q}R²⁵ ou -(CR¹⁸R¹⁹)_{q}R²⁶;
R³ représente H, CF₃, un alkyle, un cycloalkyle, un hétérocycloalkyle, un aryle, un hétéroaryle, un arylalkyle ou un hétéroarylalkyle ;
R⁴ représente H, un alkyle, un alcényle, un cycloalkyle, un hétérocycloalkyle, un aryle, un hétéroaryle, un arylalkyle, un hétéroarylalkyle ou un groupe N-protecteur adéquat ;
R⁵, R⁷ et R⁹ sont indépendamment définis comme H, F, CF₃, un alkyle, un alcényle, un cycloalkyle, un hétérocycloalkyle, un aryle, un hétéroaryle, un arylalkyle, un hétéroarylalkyle, -(CR¹⁸R¹⁹)_{q}OR²⁰,-(CR¹⁸R¹⁹)_{q}SR²⁰, -(CR¹⁸R¹⁹)_{q}NR⁴R¹¹, -(CR¹⁸R¹⁹)_{q}OCONR⁴R¹¹, -(CR¹⁸R¹⁹)_{q}OCOOR²¹, -(CR¹⁸R¹⁹)_{q}NR⁴COOR²¹, -(CR¹⁸R¹⁹)_{q}NR⁴COR²², -(CR¹⁸R¹⁹)_{q}NR⁴CONR⁴R¹¹, -(CR¹⁸R¹⁹)_{q}NR⁴SO₂R²³, -(CR¹⁸R¹⁹)_{q}NR⁴SO₂NR⁴R¹¹,-(CR¹⁸R¹⁹)_{q}COOR²¹, -(CR¹⁸R¹⁹)_{q}CONR⁴R¹¹, -(CR¹⁸R¹⁹)_{q}SO₂R⁴R¹¹, -(CR¹⁸R¹⁹)_{q}PO(OR²¹)₂, (CR¹⁸R¹⁹)_{q}COR²²,-(CR¹⁸R¹⁹)_{q}SO₂R²³, -(CR¹⁸R¹⁹)_{q}R²⁴, -(CR¹⁸R¹⁹)_{q}R²⁵ ou -(CR¹⁸R¹⁹)_{q}R²⁶;
R⁶, R⁸ et R¹⁰ sont indépendamment définis comme H, F, CF₃, un alkyle, un alcényle, un cycloalkyle, un hétérocycloalkyle, un aryle, un hétéroaryle, un arylalkyle ou un hétéroarylalkyle ;
R¹¹ représente H, un alkyle, un alcényle, un cycloalkyle, un hétérocycloalkyle, un aryle, un hétéroaryle, un arylalkyle, un hétéroarylalkyle, un groupe protecteur adéquat, -(CR¹⁸R¹⁹)ᵣOR²⁰,-(CR¹⁸R¹⁹)ᵣSR²⁰, -(CR¹⁸R¹⁹)ᵣNR⁴R²⁷, -(CR¹⁸R¹)ᵣOCONR⁴R²⁷, -(CR¹⁸R¹⁹)ᵣOCOOR²¹, -(CR¹⁸R¹⁹)ᵣNR⁴COOR²¹,-(CR¹⁸R¹⁹)ᵣNR⁴CONR⁴R²⁷, (CR¹⁸R¹⁹)ᵣNR⁴SO₂R²³, -(CR¹⁸R¹⁹)ᵣNR⁴SO₂NR⁴R²⁷, -(CR¹⁸R¹⁹)qCOOR²¹,-(CR¹⁸R¹⁹)_{q}CONR⁴R²⁷ -(CR¹⁸R¹⁹)_{q}COR²², -(CR¹⁸R¹⁹)_{q}SO₂R²³, -(CR¹⁸R¹⁹)_{q}SO₂NR⁴R²⁷, -(CR¹⁸R¹⁹)_{q}R²⁴,-(CR¹⁸R¹⁹)ₛR²⁵, -(CR¹⁸R¹⁹)_{q}R²⁶ ou -(CR¹⁸R¹⁹)_{q}R³¹;
R¹² et R¹³ sont indépendamment définis comme H ou un alkyle ;
R¹⁴ et R¹⁶ sont indépendamment définis comme H, F, CF₃, un alkyle, un alcényle, un cycloalkyle, un hétérocycloalkyle, un aryle, un hétéroaryle, un arylalkyle, un hétéroarylalkyle, -(CR¹⁸R¹⁹)_{q}OR²⁰, (CR¹⁸R¹⁹)_{q}NR⁴R¹¹, -(CR¹⁸R¹⁹)_{q}NR⁴COOR²¹, -(CR¹⁸R¹⁹)_{q}NR⁴COR²², -(CR¹⁸R¹⁹)_{q}NR⁴CONR⁴R¹¹,-(CR¹⁸R¹⁹)_{q}NR⁴SO₂R²³, -(CR¹⁸R¹⁹)_{q}NR⁴SO₂NR⁴R¹¹, -(CR¹⁸R¹⁹)_{q}COOR²¹, -(CR¹⁸R¹⁹)_{q}CONR⁴R¹¹,-(CR¹⁸R¹⁹)_{q}SO₂R⁴R¹¹ ou (CR¹⁸R¹⁹)_{q}COR²² ;
R¹⁵ et R¹⁷ sont indépendamment définis comme H, F, CF₃, un alkyle, un alcényle, un cycloalkyle, un hétérocycloalkyle, un aryle, un hétéroaryle, un arylalkyle ou un hétéroarylalkyle ;
R¹⁸ représente H, F, CF₃, un alkyle, un alcényle, un cycloalkyle, un hétérocycloalkyle, un aryle, un hétéroaryle, un arylalkyle, un hétéroarylalkyle, -(CR²⁹R³⁰)ₛOR³¹, -(CR²⁹R³⁰)ₛSR³¹, -(CR²⁹R³⁰)ₛNR²⁸R³¹,-(CR²⁹R³⁰)ₛOCONR²⁸R³¹, -(CR²⁹R³⁰)ₛOCOOR²¹, -(CR²⁹R³⁰)ₛNR²⁸COOR²¹, -(CR²⁹R³⁰)ₛNR²⁸COR³¹,-(CR²⁹R³⁰)ₛNR²⁸CONR²⁸R³¹, -(CR²⁹R³⁰)ₛNR²⁸SO₂R²³, -(CR²⁹R³⁰)ₛNR²⁸SO₂NR²⁸R³¹, -(CR²⁹R³⁰)_{q}COOR²¹,-(CR²⁹R³⁰)_{q}CONR²⁸R³¹, -(CR²⁹R³⁰)_{q}SO₂NR²⁸R³¹, -(CR²⁹R³⁰)_{q}PO(OR²¹)₂, -(CR²⁹R³⁰)_{q}COR³¹,-(CR²⁹R³⁰)_{q}SO₂R²³, -(CR²⁹R³⁰)_{q}R²⁴, -(CR²⁹R³⁰)_{q}R²⁵ OU -(CR²⁹R³⁰)_{q}R²⁶ ;
R¹⁹ représente H, CF₃, un alkyle, un alcényle, un cycloalkyle, un hétérocycloalkyle, un aryle, un hétéroaryle, un arylalkyle ou un hétéroarylalkyle ;
R²⁰ représente H, un alkyle, un alcényle, un cycloalkyle, un hétérocycloalkyle, un aryle, un hétéroaryle, un arylalkyle, un hétéroarylalkyle, -(CR²⁹R³⁰)ᵣOR³¹, -(CR²⁹R³⁰)ᵣSR³¹, -(CR²⁹R³⁰)ᵣNR²⁸R³¹,-(CR²⁹R³⁰)ᵣOCONR²⁸R³¹, -(CR²⁹R³⁰)ᵣNR²⁸COOR²¹, -(CR²⁹R³⁰)ᵣNR²⁸COR³¹, -(CR²⁹R³⁰)ᵣNR²⁸CONR²⁸R³¹,-(CR²⁹R³⁰)ᵣNR²⁸SO₂R²³, -(CR²⁹R³⁰)ᵣNR²⁸SO₂NR²⁸R³¹, (CR²⁹R³⁰)_{q}COR³¹, -(CR²⁹R³⁰)_{q}SO₂R²³, -(CR²⁹R³⁰)_{q}R²⁴,-(CR²⁹R³⁰)_{q}R²⁵, -(CR²⁹R³⁰)_{q}R²⁶ ou (CR²⁹R³⁰)_{q}R³¹ ;
R²¹ représente H, un alkyle, un alcényle, un cycloalkyle, un hétérocycloalkyle, un aryle, un hétéroaryle, un arylalkyle, un hétéroarylalkyle ou un groupe O-protecteur adéquat ;
R²² représente un alkyle, un alcényle, un cycloalkyle, un hétérocycloalkyle, un aryle, hétéroaryle,
un arylalkyle, un hétéroarylalkyle, -(CR²⁹R³⁰)ₛOR³¹, -(CR²⁹R³⁰)ₛSR³¹, -(CR²⁹R³⁰)ₛNR²⁸R³¹,-(CR²⁹R³⁰)ₛOCONR²⁸R³¹, -(CR²⁹R³⁰)ₛNR²⁸COOR²¹, -(CR²⁹R³⁰)ₛNR²⁸COR³¹, -(CR²⁹R³⁰)ₛNR²⁸CONR²⁸R³¹,-(CR²⁹R³⁰)ₛNR²⁸SO₂R²³, -(CR²⁹R³⁰)ₛNR²⁸SO₂NR²⁸R³¹, -(CR²⁹R³⁰)ₛCOOR²¹, -(CR²⁹R³⁰)ₛCONR²⁸R³¹-(CR²⁹R³⁰)ₛSO₂NR²⁸R³¹, -(CR²⁹R³⁰)ₜCOR³¹, -(CR²⁹R³⁰)ₛSO₂R²³, -(CR²⁹R³⁰)ₜR²⁴, -(CR²⁹R³⁰)ₜR²⁵, -(CR²⁹R³⁰)ₜR²⁶ ou - -(CR²⁹R³⁰)ₜR³¹ ;
R²³ représente H, un alkyle, un alcényle, un cycloalkyle, un hétérocycloalkyle, un aryle, un hétéroaryle, un arylalkyle, un hétéroarylalkyle ou -(CR³²R³³)ₜR²⁴ ;
R²⁴ représente un aryle, un hétéroaryle, -C₆H₂R³⁴R³⁵R³¹ ou un groupe d'une des formules suivantes :
| | | | |
|---|---|---|---|
| 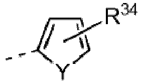 | 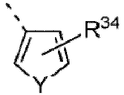 | 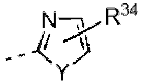 | 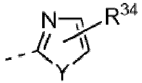 |
| **H1** | **H2** | **H3** | **H4** |
| 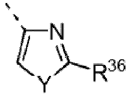 | 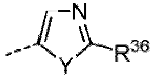 | 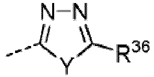 | 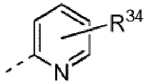 |
| **H5** | **H6** | **H7** | **H8** |
| 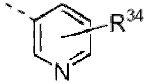 | 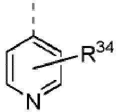 | 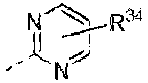 | 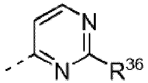 |
| **H9** | **H10** | **H11** | **H12** |
| 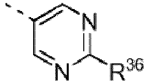 | 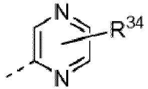 | 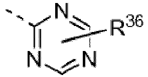 | 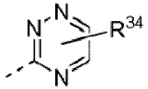 |
| **H13** | **H14** | **H15** | **H16** |
| 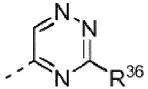 | 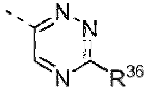 | 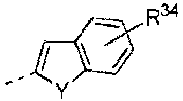 | 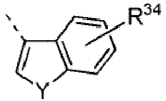 |
| **H17** | **H18** | **H19** | **H20** |
| 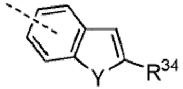 | 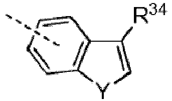 | 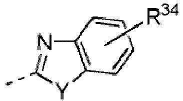 | 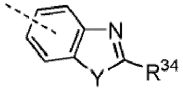 |
| **H21** | **H22** | **H23** | **H24** |
| 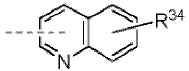 | 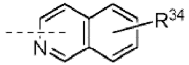 | 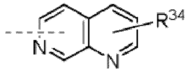 | 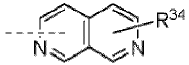 |
| **H25** | **H26** | **H27** | **H28** |
| 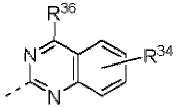 | 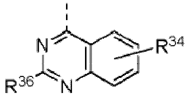 | 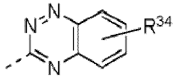 | 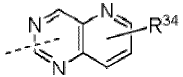 |
| **H29** | **H30** | **H31** | **H32** |
| 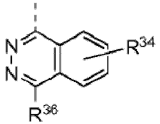 | | 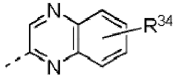 | |
| **H33** | | **H34** | |
R²⁵ représente un groupe d'une des formules suivantes :
| | | | |
|---|---|---|---|
| 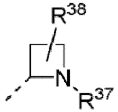 | 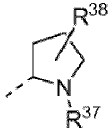 | 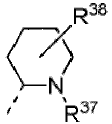 | 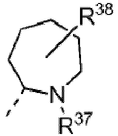 |
| **H35** | **H36** | **H37** | **H38** |
| 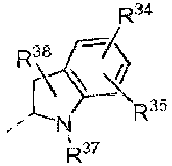 | 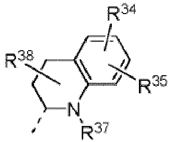 | | 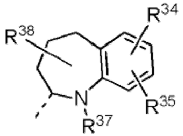 |
| **H39** | **H40** | | **H41** |
R²⁶ représente un groupe d'une des formules suivantes :
| | | | |
|---|---|---|---|
| 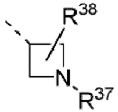 | 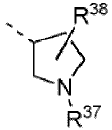 | 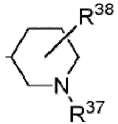 | 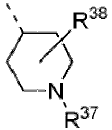 |
| **H42** | **H43** | **H44** | **H45** |
| 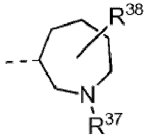 | 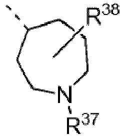 | 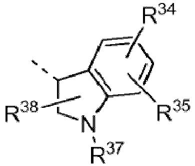 | 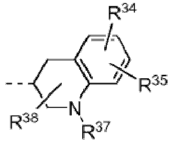 |
| **H46** | **H47** | **H48** | **H49** |
| 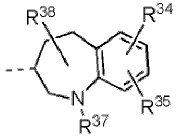 | | | |
| **H50** | | | |
R²⁷ représente H, un alkyle, un alcényle inférieur, un cycloalkyle, un hétérocycloalkyle, un aryle, un hétéroaryle, un arylalkyle inférieur, un hétéroarylalkyle inférieur, un groupe protecteur adéquat ou -(CR²⁹R³⁰)_{q}R²⁴ ;
R²⁸ représente H, un alkyle, un alcényle inférieur, un cycloalkyle, un hétérocycloalkyle, un aryle, un hétéroaryle, un arylalkyle inférieur, un hétéroarylalkyle inférieur, un groupe N-protecteur adéquat, -(CR³²R³³)ₛOR²¹, -(CR³²R³³)ₛNR⁴²R⁴³, -(CR³²R³³)ₛNR⁴²CONR⁴²R⁴³, -(CR³²R³³)ₛNR⁴²COR²¹,-(CR³²R³³)ₛNR⁴²SO₂R²¹, -(CR³²R³³)_{q}COOR²¹, -(CR³²R³³)_{q}COR²³ ou -(CR³²R³³)_{q}SO₂R²¹ ;
R²⁹ représente H, F, CF₃, un alkyle, un alcényle, un cycloalkyle, un hétérocycloalkyle, un aryle, un hétéroaryle, un arylalkyle, un hétéroarylalkyle, -(CR³²R³³)ₛOR³¹, -(CR³²R³³)ₛSR³¹ -(CR³²R³³)ₛNR²⁸R³¹,-(CR³²R³³)ₛOCONR²⁸R³¹, -(CR³²R³³)ₛOCOOR²¹, -(CR³²R³³)ₛNR²⁸COOR²¹, -(CR³²R³³)ₛNR²⁸COR³¹,-(CR³²R³³)ₛNR²⁸CONR²⁸R³¹, -(CR³²R³³)ₛNR²⁸SO₂R²³, -(CR³²R³³)ₛNR²⁸SO₂NR²⁸R³¹, -(CR³²R³³)_{q}COOR²¹,-(CR³²R³³)_{q}CONR²⁸R³¹, -(CR³²R³³)_{q}SO₂NR²⁸R³¹, -(CR³²R³³)_{q}PO(OR²¹)₂, (CR³²R³³)_{q}COR³¹, -(CR³²R³³)_{q}SO₂R²³ ou -(CR³²R³³)_{q}R³¹ ;
R³⁰ représente H, F, CF₃, un alkyle, un alcényle, un cycloalkyle, un hétérocycloalkyle, un aryle, un hétéroaryle, un arylalkyle ou un hétéroarylalkyle ;
R³¹ représente H, un alkyle, un alcényle, un cycloalkyle, un hétérocycloalkyle, un aryle, un hétéroaryle, un arylalkyle, un hétéroarylalkyle ou un groupe d'une des formules suivantes :
| | |
|---|---|
| 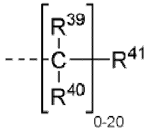 | 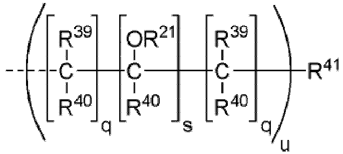 |
| **H51** | **H52** |
| 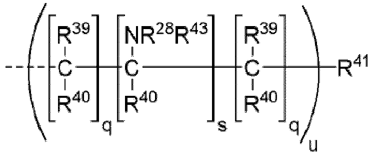 | 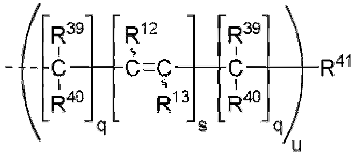 |
| **H53** | **H54** |
| 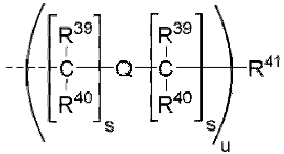 | |
| **H55** | |
R³² et R³³ sont indépendamment définis comme H, F, CF₃, un alkyle, un alcényle, un cycloalkyle, un hétérocycloalkyle, un aryle, un hétéroaryle, un arylalkyle ou un hétéroarylalkyle ;
R³⁴ et R³⁵ sont indépendamment définis comme H, F, Cl, CF₃, OCF₃, OCHF₂, NO₂, CN, un alkyle, un alcényle, un alcynyle, un cycloalkyle, un hétérocycloalkyle, un aryle, un hétéroaryle, un arylalkyle, un hétéroarylalkyle -(CR²⁹R³⁰)_{q}OR³¹, -(CR²⁹R³⁰)_{q}SR³¹, -(CR²⁹R³⁰)_{q}NR²⁸R³¹, -(CR²⁹R³⁰)_{q}OCONR²⁸R³¹,-(CR²⁹R³⁰)_{q}NR²⁸COOR²¹, -(CR²⁹R³⁰)_{q}NR²⁸COR³¹, -(CR²⁹R³⁰)_{q}NR²⁸CONR²⁸R³¹, -(CR²⁹R³⁰)_{q}NR²⁸SO₂R²³,-(CR²⁹R³⁰)_{q}NR²⁸SO₂NR²⁸R³¹, -(CR²⁹R³⁰)_{q}COOR³¹, -(CR²⁹R³⁰)_{q}CONR²⁸R³¹, -(CR²⁹R³⁰)_{q}SO₂NR²⁸R³¹,-(CR²⁹R³⁰)_{q}COR³¹, -(CR²⁹R³⁰)_{q}SO₂R²³ ou -(CR²⁹R³⁰)_{q}R³¹ ;
R³⁶ représente H, un alkyle, un alcényle, un alcynyle, un cycloalkyle, un hétérocycloalkyle, un aryle, un hétéroaryle, un arylalkyle, un hétéroarylalkyle ou -NR²⁸R³¹ ;
R³⁷ représente H, un alkyle, un alcényle, un cycloalkyle, un hétérocycloalkyle, un aryle, un hétéroaryle, un arylalkyle, un hétéroarylalkyle, un groupe N-protecteur adéquat, -(CR²⁹R³⁰)ᵣOR³¹,-(CR²⁹R³⁰)ᵣSR³¹, -(CR²⁹R³⁰)ᵣNR²⁸R³¹, -(CR²⁹R³⁰)ᵣOCONR²⁸R³¹, -(CR²⁹R³⁰)ᵣNR²⁸COOR²¹,-(CR²⁹R³⁰)ᵣNR²⁸COR³¹, -(CR²⁹R³⁰)rNR²⁸CONR²⁸R³¹, -(CR²⁹R³⁰)_{q}COOR²¹, -(CR²⁹R³⁰)_{q}CONR²⁸R³¹,-(CR²⁹R³⁰)_{q}COR²¹ ou -(CR²⁹R³⁰)_{q}R²¹ ;
R³⁸ représente H, un alkyle, un alcényle, un cycloalkyle, un hétérocycloalkyle, un aryle, un hétéroaryle, un arylalkyle, un hétéroarylalkyle, -(CR²⁹R³⁰)_{q}OR³¹, -(CR²⁹R³⁰)_{q}SR³¹, -(CR²⁹R³⁰)_{q}NR²⁸R³¹,-(CR²⁹R³⁰)_{q}NR²⁸COOR²¹, -(CR²⁹R³⁰)_{q}NR²⁸COR³¹, -(CR²⁹R³⁰)_{q}NR²⁸CONR²⁸R³¹, -(CR²⁹R³⁰)_{q}COOR²¹,-(CR²⁹R³⁰)_{q}CONR²⁸R³¹, -(CR²⁹R³⁰)_{q}COR²¹ ou -(CR²⁹R³⁰)_{q}R³¹;
R³⁹ représente H, F, CF₃, un alkyle, un alcényle, un cycloalkyle, un hétérocycloalkyle, un aryle, un hétéroaryle, un arylalkyle, un hétéroarylalkyle, -(CR²⁹R³⁰)ᵤOR³¹, -(CR²⁹R³⁰)ᵤNR²⁸R³¹,-(CR²⁹R³⁰)ₜCOOR²¹ ou -(CR²⁹R³⁰)ₜCONR²⁸R⁴³ ;
R⁴⁰ représente H, F, CF₃, un alkyle, un alcényle, un cycloalkyle, un hétérocycloalkyle, un aryle, un hétéroaryle, un arylalkyle, un hétéroarylalkyle, -(CR³²R³³)ᵤOR²¹, -(CR³²R³³)ᵤNR²⁸R⁴³,-(CR³²R³³)ₜCOOR²¹ ou -(CR³²R³³)ₜCONR²⁸R⁴³;
R⁴¹ représente H, CF₃, un alkyle, un alcényle, un cycloalkyle, un hétérocycloalkyle, un aryle, un hétéroaryle, un arylalkyle, un hétéroarylalkyle, -OR²¹, -NR²⁸R⁴³, -NR²⁸COR²³, -NR²⁸COOR²¹,-NR²⁸SO₂R²³, -NR²⁸CONR²⁸R⁴³, -COOR²¹, -CONR²⁸R⁴³, -C(=NR⁴³)NR²⁸R⁴³, -NR²⁸C(=NR⁴³)NR²⁸R⁴³ ou un groupe d'une des formules suivantes :
| | | | |
|---|---|---|---|
| 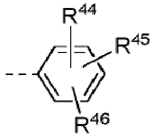 | 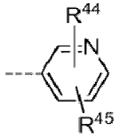 | 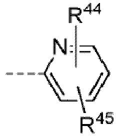 | 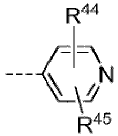 |
| **H56** | **H57** | **H58** | **H59** |
| 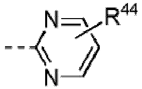 | 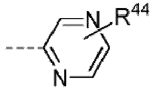 | 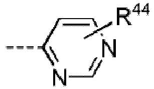 | 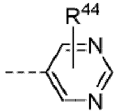 |
| **H60** | **H61** | **H62** | **H63** |
| 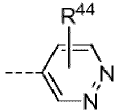 | 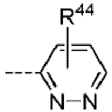 | 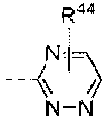 | 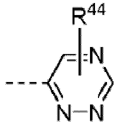 |
| **H64** | **H65** | **H66** | **H67** |
| 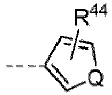 | 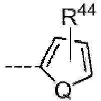 | 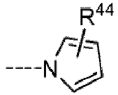 | 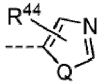 |
| **H68** | **H69** | **H70** | **H71** |
| 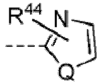 | 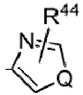 | 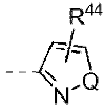 | 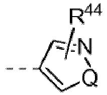 |
| **H72** | **H73** | **H74** | **H75** |
| 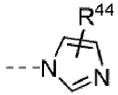 | 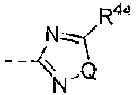 | 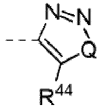 | 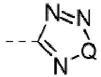 |
| **H76** | **H77** | **H78** | **H79** |
| 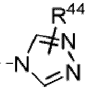 | 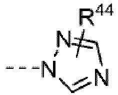 | 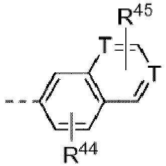 | 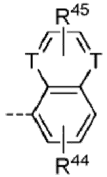 |
| **H80** | **H81** | **H82** | **H83** |
| 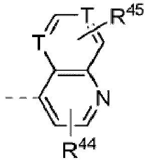 | 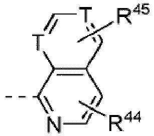 | 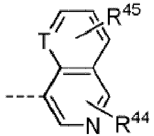 | 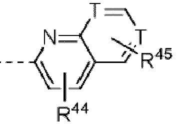 |
| **H84** | **H85** | **H86** | **H87** |
| 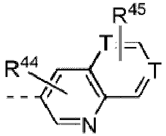 | 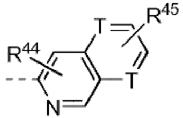 | 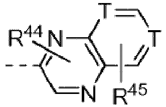 | 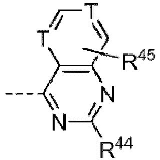 |
| **H88** | **H89** | **H90** | **H91** |
| 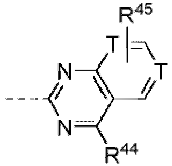 | 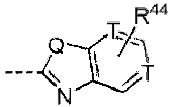 | 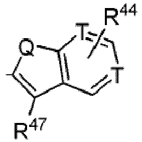 | 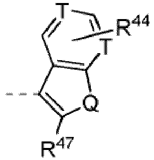 |
| **H92** | **H93** | **H94** | **H95** |
| 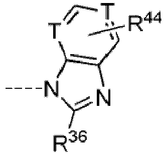 | 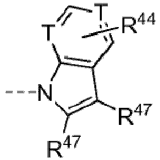 | 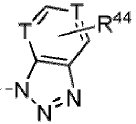 | 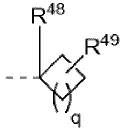 |
| **H96** | **H97** | **H98** | **H99** |
| 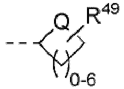 | 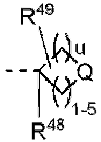 | 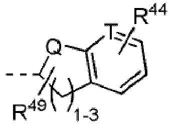 | 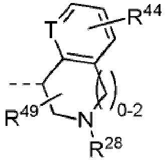 |
| **H100** | **H101** | **H102** | **H103** |
| 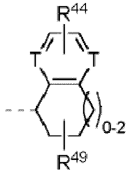 | 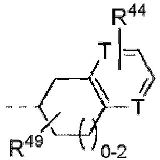 | 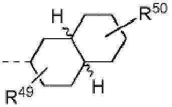 | 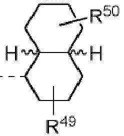 |
| **H104** | **H105** | **H106** | **H107** |
| 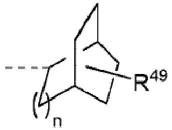 | 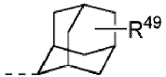 | | 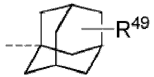 |
| **H108** | **H109** | | **H110** |
R⁴² représente H, un alkyle, un alcényle, un cycloalkyle, un hétérocycloalkyle, un aryle, un hétéroaryle, -(CR²³R³³)ₛOR²¹, -(CR²³R³³)ₛNR⁴R⁴³ -(CR²³R³³)_{q}COOR²¹ ou -(CR²³R³³)_{q}CONR⁴R⁴³ ;
R⁴³ représente H, un alkyle, un alcényle, un cycloalkyle, un hétérocycloalkyle, un aryle, un hétéroaryle, un arylalkyle, un hétéroarylalkyle ou un groupe N-protecteur adéquat ;
R⁴⁴, R⁴⁵ et R⁴⁶ sont indépendamment définis comme H, F, CF₃, OCF₃, OCHF₂, NO₂, CN, un alkyle, un alcényle, un alcynyle, un cycloalkyle, un hétérocycloalkyle, un aryle, un hétéroaryle, un arylalkyle, un hétéroarylalkyle, -OR²³, -NR²⁸R⁴³, -NR²⁸COR²³, -NR²⁸SO₂R²³, -NR²⁸CONR²⁸R⁴³, -COR²³ ou -SO₂R²³ ;
R⁴⁷ représente H, CF₃, un alkyle, un alcényle, un alcynyle, un cycloalkyle, un hétérocycloalkyle, un aryle, un hétéroaryle, un arylalkyle, un hétéroarylalkyle, -COOR²¹ ou -CONR²⁸R⁴³ ;
R⁴⁸ représente H, F, CF₃, un alkyle, un alcényle, un cycloalkyle, un hétérocycloalkyle, un aryle, un hétéroaryle, -(CR²³R³³)ₜOR²¹, -(CR²³R³³)ₜNR⁴R⁴³, -(CR²³R³³)ₜCOOR²¹ ou -(CR²³R³³)ₜCONR⁴R⁴³ ;
R⁴⁹ et R⁵⁰ sont indépendamment définis comme H, F, CF₃, un alkyle, un alcényle, un cycloalkyle, un hétérocycloalkyle, un aryle, un hétéroaryle, un arylalkyle, un hétéroarylalkyle, -(CR³²R³³)_{q}OR²¹,-(CR³²R³³)_{q}NR²⁸R⁴³, -(CR³²R³³)_{q}COOR²¹ ou -(CR³²R³³)_{q}CONR²⁸R⁴³ ;
R⁵¹ représente H, F, Cl, CF₃, OCF₃ ou un alkyle inférieur ;
R⁵² représente H, F, Cl, CF₃, OCF₃, NO₂, CN, un alkyle inférieur, un alcényle inférieur, un alcynyle inférieur, un cycloalkyle inférieur, un hétérocycloalkyle inférieur, un aryle, un hétéroaryle, un arylalkyle inférieur ou un hétéroarylalkyle inférieur ;
R⁵³ représente CF₃, OCF₃, CN, un alkyle inférieur, un cycloalkyle inférieur, un aryle alcoxy inférieur ou un aryloxy ;
pris ensemble R⁴ et R¹¹, R⁴ et R²⁷, R⁵ et R⁶ R⁵ et R⁷, R⁵ et R⁹, R⁵ et R¹⁴, R⁵ et R¹⁶ R⁷ et R⁸, R⁷ et R⁹, R⁷ et R¹⁶, R⁹ et R¹⁰, R¹⁴ et R¹⁵, R¹⁶ et R¹⁷, R¹⁸ et R¹⁹, R²⁷ et R²⁸, R²⁸ et R³¹, R²⁸ et R⁴³, R²⁹ et R³⁰, R³² et R³³, R³⁴ et R³⁵, R³⁷ et R³⁸, R³⁹ et R⁴⁰, R³⁹ et R⁴¹, R³⁹ et R⁴⁹, R⁴² et R⁴³, R⁴⁴ et R⁴⁵, ou R⁴⁴ et R⁴⁶, peuvent former facultativement des entités cycloalkyles substituées ou hétérocycloalkyles substituées ;
de plus, les éléments structurels -NR⁴R¹¹, -NR¹¹R²⁷, -NR²⁷R²⁸, -NR²⁸R³¹ ou -NR²⁸R⁴³ peuvent former un des groupes de formule :
| | | | |
|---|---|---|---|
| 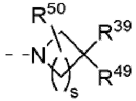 | 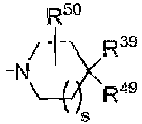 | 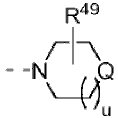 | 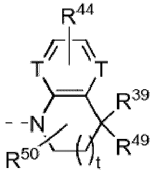 |
| **H111** | **H112** | **H113** | **H114** |
| 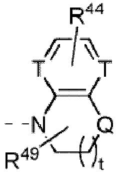 | 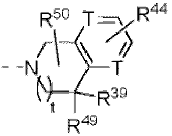 | 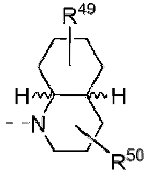 | 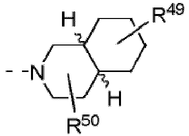 |
| **H115** | **H116** | **H117** | **H118** |
Z représente O, S, S(=O), S(=O)₂ ou NR²⁸ ;
Y représente O, S ou NR⁴ ;
Q représente O, S ou NR²⁸ ;
T représente CR⁴⁶ ou N ; au cas où T apparaît plusieurs fois dans la même structure cyclique, chaque T est défini indépendamment de l'autre ;
q représente un entier entre 0 et 4 ;
r représente un entier entre 2 et 4 ;
s représente un entier entre 1 et 4 ;
t représente un entier entre 0 et 2 ;
u représente un entier entre 1 et 2 ;
et tous les stéréoisomères possibles de tels composés ; ou sels, solvates, clathrates, N-oxydes, versions isotopiquement ou énantiomériquement enrichies de ceux-ci.

2. Composés selon la revendication 1, dans lesquels :
le bloc constitutif de type A est choisi de A627 à A683
le bloc constitutif de type B est choisi de B1 à B21
le bloc constitutif de type C est choisi de C1 à C101
et où M représente -N(R⁴)-,
et tous les stéréoisomères possibles de tels composés, ou leurs sels pharmaceutiquement acceptables.

3. Composés selon la revendication 1, dans lesquels :
le bloc constitutif de type A est choisi parmi :
A1(a1), A2(a1), A3(a1), A4(a1), A5(a1), A6(a1), A7(a1), A9(a1), A10(a1), A73(a2), A80(a2), A170(a4), A171(a4), A209(a7), A210(a7), A240(a10), A241(a10), A242(a10), A243(10), A272(a10), A530(a18), A531(a18), A532(a18), A533(a18), A609(a24), A610(a24), A611(a24), A612(a24), A613(a24), A614(a24), A615(a24), A631(a1), A632(a1), A633(a1), A641(a1), A642(a1), A643(a1), A651(a1), A652(a1) ou A653(a1) ;
le bloc constitutif de type B est choisi parmi :
B4(b3), B5(b3), B6(b3), B7(b3), B8(b3), B9(b3), B10(b3), B12(b4), B13(b4), B14(b4), B15(b4), B16(b4) ou B17(b5) ;
le bloc constitutif de type C est choisi parmi :
C1, C2, C3, C4, C5, C6, C7, C8, C9, C10, C11, C12, C13, C14, C15, C16, C17, C18, C19, C20, C21, C25, C26, C27, C28, C29, C30, C34, C35, C36, C37, C38, C39, C43, C44, C45, C46, C47, C48, C49, C50, C51, C52, C53, C54, C55, C56, C57, C58, C59, C60, C61, C62, C63, C64, C65, C66, C67, C68, C69, C70, C71, C72, C73, C74, C75, C76, C77, C78, C79, C80, C81, C86, C87, C88, C89, C90, C91, C92 ou C93 ;
et dans lesquels
R¹ représente H, F, Cl, Br, I, CF₃, OCF₃, OCHF₂, NO₂, CN, un alkyle inférieur, un alcényle inférieur, un alcynyle inférieur, un cycloalkyle inférieur, un hétérocycloalkyle inférieur, un aryle, un hétéroaryle, un arylalkyle inférieur, un hétéroarylalkyle inférieur, -(CR¹⁸R¹⁹)_{q}OR²⁰,-(CR¹⁸R¹⁹)_{q}NR⁴R¹¹, -(CR¹⁸R¹⁹)_{q}NR⁴COOR²¹, -(CR¹⁸R¹⁹)_{q}NR⁴COR²², -(CR¹⁸R¹⁹)_{q}NR⁴CONR⁴R¹¹,-(CR¹⁸R¹⁹)_{q}NR⁴SO₂R²³, -(CR¹⁸R¹⁹)_{q}NR⁴SO₂NR⁴R¹¹, -(CR¹⁸R¹⁹)_{q}COOR²¹, -(CR¹⁸R¹⁹)_{q}CONR⁴R¹¹,-(_{CR}¹⁸_{R}¹⁹)_{q}SO₂NR⁴_{R}¹¹, -(CR¹⁸R¹⁹)_{q}PO(OR²¹)₂, -(CR¹⁸R¹⁹)_{q}COR²², -(CR¹⁸R¹⁹)_{q}SO₂R²³, -(_{CR}¹⁸_{R}¹⁹)_{q}OSO₃R²¹,-(CR¹⁸R¹⁹)_{q}R²⁴, -(CR¹⁸R¹⁹)_{q}R²⁵ ou ₋(CR¹⁸R¹⁹)_{q}R²⁶ ;
R² représente H, CF₃, un alkyle inférieur, un alcényle inférieur, un cycloalkyle inférieur, un hétérocycloalkyle inférieur, un aryle, un hétéroaryle, un arylalkyle inférieur, un hétéroarylalkyle inférieur, -(CR¹⁸R¹⁹)_{q}OR²⁰, -(CR¹⁸R¹⁹)_{q}NR⁴R¹¹, -(CR¹⁸R¹⁹)_{q}NR⁴COOR²¹, -(CR¹⁸R¹⁹)_{q}NR⁴COR²², - (CR¹⁸R¹⁹)_{q}NR⁴CONR⁴R¹¹, -(CR¹⁸R¹⁹)_{q}NR⁴SO₂R²³, -(CR¹⁸R¹⁹)_{q}NR⁴SO₂NR⁴R¹¹, -(CR¹⁸R¹⁹)_{q}COOR²¹, - (CR¹⁸R¹⁹)_{q}CONR⁴R¹¹, -(CR¹⁸R¹⁹)_{q}SO₂R⁴R¹¹, -(CR¹⁸R¹⁹)_{q}PO(OR²¹)₂, -(CR¹⁸R¹⁹)_{q}COR²², -(CR¹⁸R¹⁹)_{q}SO₂R²³, - (CR¹⁸R¹⁹)_{q}R²⁴, -(CR¹⁸R¹⁹)_{q}R²⁵ ou -(CR¹⁸R¹⁹)qR²⁶ ;
R⁴ représente H, un alkyle inférieur, un alcényle inférieur, un cycloalkyle inférieur, un hétérocycloalkyle inférieur ou un groupe N-protecteur adéquat ;
R⁵, R⁷ et R⁹ sont indépendamment définis comme H, F, CF₃, un alkyle inférieur, un alcényle inférieur, un cycloalkyle inférieur, un hétérocycloalkyle inférieur, un aryle, un hétéroaryle, un arylalkyle inférieur, un hétéroarylalkyle inférieur, -(CR¹⁸R¹⁹)_{q}OR²⁰, -(CR¹⁸R¹⁹)_{q}NR⁴R¹¹, - (CR¹⁸R¹⁹)_{q}NR⁴COR²², -(CR¹⁸R¹⁹)_{q}NR⁴CONR⁴R¹¹, -(CR¹⁸R¹⁹)_{q}NR⁴SO₂R²³, -(CR¹⁸R¹⁹)qNR⁴CONR⁴R¹¹, - (CR¹⁸R¹⁹)_{q}COOR²¹, -(CR¹⁸R¹⁹)_{q}CONR⁴_{R}¹¹, -(CR¹⁸R¹⁹)_{q}SO₂R⁴R¹¹, -(CR¹⁸R¹⁹)_{q}PO(OR²¹)₂, -(CR¹⁸R¹⁹)_{q}COR²², - (CR¹⁸R¹⁹)_{q}SO₂R²³, -(CR¹⁸R¹⁹)_{q}R²⁴, -(CR¹⁸R¹⁹)_{q}R²⁵ ou -(CR¹⁸R¹⁹)_{q}R²⁶ ;
R⁶, R⁸ et R¹⁰ sont indépendamment définis comme H, CF₃ ou un alkyle inférieur ;
R¹¹ représente H, un alkyle, un alcényle, un cycloalkyle, un hétérocycloalkyle inférieur, un aryle, un hétéroaryle, un arylalkyle inférieur, un hétéroarylalkyle inférieur, un groupe protecteur adéquat, -(CR¹⁸R¹⁹)rOR²⁰, -(CR¹⁸R¹⁹)ᵣNR⁴R²⁷, -(CR¹⁸R¹⁹)ᵣNR⁴COOR²¹, -(CR¹⁸R¹⁹)ᵣNR⁴CONR⁴R²⁷, (CR¹⁸R¹⁹)ᵣNR⁴SO₂R²³, -(CR¹⁸R¹⁹)ᵣNR⁴SO₂NR⁴R²⁷, -(CR¹⁸R¹⁹)_{q}COOR²¹, -(CR¹⁸R¹⁹)_{q}CONR⁴R²⁷, - (CR¹⁸R¹⁹)_{q}COR²², -(CR¹⁸R¹⁹)_{q}SO₂R²³, -(CR¹⁸R¹⁹)_{q}SO₂NR⁴_{R}²⁷, -(CR¹⁸R¹⁹)_{q}R²⁴, -(CR¹⁸R¹⁹)ₛR²⁵, - (CR¹⁸R¹⁹)_{q}R²⁶ ou -(CR¹⁸R¹⁹)_{q}R³¹ ;
R¹² et R¹³ sont indépendamment définis comme H ou comme un alkyle inférieur ;
R¹⁴ et R¹⁶ sont indépendamment définis comme H, F, CF₃, un alkyle inférieur, un alcényle inférieur, un cycloalkyle inférieur, un hétérocycloalkyle inférieur, un aryle, un hétéroaryle, un arylalkyle inférieur, un hétéroarylalkyle inférieur, -(CR¹⁸R¹⁹)_{q}OR²⁰, (CR¹⁸R¹⁹)_{q}NR⁴R¹¹, -(CR¹⁸R¹⁹)_{q}NR⁴COOR²¹, - (CR¹⁸R¹⁹)_{q}NR⁴COR²², -(CR¹⁸R¹⁹)_{q}NR⁴CONR⁴R¹¹, -(CR¹⁸R¹⁹)_{q}NR⁴SO₂R²³, -(CR¹⁸R¹⁹)qCOOR²¹, - (CR¹⁸R¹⁹)_{q}CONR⁴R¹¹ ou (CR¹⁸R¹⁹)_{q}COR²² ;
R¹⁵ et R¹⁷ sont indépendamment définis comme H, CF₃ ou comme un alkyle inférieur ;
R¹⁸ représente H, F, CF₃, un alkyle inférieur, un alcényle inférieur, un cycloalkyle inférieur, un hétérocycloalkyle inférieur, un aryle, un hétéroaryle, un arylalkyle inférieur, un hétéroarylalkyle inférieur, -(CR²⁹R³⁰)ₛOR³¹, -(CR²⁹R³⁰)ₛNR²⁸R³¹, -(CR²⁹R³⁰)ₛNR²⁸COOR²¹, -(CR²⁹R³⁰)ₛNR²⁸COR³¹, - (CR²⁹R³⁰)ₛNR²⁸CONR²⁸R³¹, -(CR²⁹R³⁰)ₛNR²⁸SO₂R²³, -(CR²⁹R³⁰)ₛNR²⁸SO₂NR²⁸R³¹, -(CR²⁹R³⁰)_{q}COOR²¹, - (CR²⁹R³⁰)_{q}CONR²⁸R³¹, -(CR²⁹_{R}³⁰)_{q}SO₂NR²⁸R³¹, -(CR²⁹R³⁰)_{q}PO(OR²¹)₂, -(CR²⁹R³⁰)_{q}COR³¹, - (CR²⁹R³⁰)qSO₂R²³, -(CR²⁹R³⁰)_{q}R²⁴, -(CR²⁹R³⁰)_{q}R²⁵ ou -(CR²⁹R³⁰)_{q}R²⁶ ;
R¹⁹ représente H, CF₃ ou un alkyle inférieur ;
R²⁰ représente H, un alkyle inférieur, un alcényle inférieur, un cycloalkyle inférieur, un hétérocycloalkyle inférieur, un aryle, un hétéroaryle, un arylalkyle inférieur, un hétéroarylalkyle inférieur, -(CR²⁹R³⁰)ᵣOR³¹, -(CR²⁹R³⁰)ᵣNR²⁸R³¹, -(CR²⁹R³⁰)ᵣNR²⁸COOR²¹, -(CR²⁹R³⁰)ᵣNR²⁸COR³¹, - (CR²⁹R³⁰)ᵣNR²⁸CONR²⁸R³¹, -(CR²⁹R³⁰)ᵣNR²⁸SO₂R²³, -(CR²⁹R³⁰)_{q}COOR²¹, -(CR²⁹R³⁰)_{q}CONR²⁸R³¹, (CR²⁹R³⁰)_{q}SO₂ NR²⁸R³¹, (CR²⁹R³⁰)_{q}COR²³, -(CR²⁹R³⁰)_{q}SO₂R²³, -(CR²⁹R³⁰)_{q}R²⁴, -(CR²⁹R³⁰)_{q}R²⁵, - (CR²⁹R³⁰)_{q}R²⁶ ou (CR²⁹R³⁰)_{q}R³¹ ;
R²² représente un alkyle inférieur, un alcényle inférieur, un cycloalkyle inférieur, un hétérocycloalkyle inférieur, un aryle, un hétéroaryle, un arylalkyle inférieur, un hétéroarylalkyle inférieur, -(CR²⁹R³⁰)ₛOR³¹, -(CR²⁹R³⁰)ₛNR²⁸R³¹, -(CR²⁹R³⁰)ₛNR²⁸COOR²¹, -(CR²⁹R³⁰)ₛNR²⁸COR³¹, - (CR²⁹R³⁰)ₛNR²⁸CONR²⁸R³¹, -(CR²⁹R³⁰)ₛNR²⁸SO₂R²³, -(CR²⁹R³⁰)ₛCOOR²¹, -(CR²⁹R³⁰)ₛCONR²⁸R³¹, - (CR²⁹R³⁰)ₛSO₂NR²⁸_{R}³¹, -(CR²⁹R³⁰)ₜCOR³¹, -(_{CR}²⁹_{R}³⁰)ₛSO₂R²³, -(CR²⁹R³⁰)ₜR²⁴, -(CR²⁹R³⁰)ₜR²⁵, -(CR²⁹R³⁰)ₜR²⁶ ou -(CR²⁹R³⁰)ₜR³¹ ;
R²⁹ représente H, F, CF₃, un alkyle inférieur, un alcényle inférieur, un cycloalkyle inférieur, un hétérocycloalkyle inférieur, un aryle, un hétéroaryle, un arylalkyle inférieur, un hétéroarylalkyle inférieur, -(CR³²R³³)ₛOR³¹, -(CR³²R³³)ₛNR²⁸R³¹, -(CR³²R³³)ₛNR²⁸COOR²¹, -(_{CR}³²_{R}³³)ₛNR²⁸COR³¹, - (CR³²R³³)ₛNR²⁸CONR²⁸R³¹, -(CR³²R³³)ₛNR²⁸SO₂R²³, -(CR³²R³³)_{q}COOR²¹, -(CR³²R³³)_{q}CONR²⁸R³¹,-(CR³²R³³)_{q}SO₂NR²⁸R³¹, -(CR³²R³³)_{q}PO(OR²¹)2, -(CR³²R³³)_{q}COR³¹, -(CR³²R³³)_{q}SO₂R²³ ou -(CR³²R³³)_{q}R³¹ ;
R³⁰ et R³³ sont indépendamment définis comme H, CF₃ ou comme un alkyle inférieur ;
R³⁴ et R³⁵ sont indépendamment définis comme H, F, Cl, CF₃, OCF₃, OCHF₂, NO₂, CN, un alkyle inférieur, un alcényle inférieur, un alcynyle inférieur, un cycloalkyle inférieur, un hétérocycloalkyle inférieur, un aryle, un hétéroaryle, un arylalkyle inférieur ou un hétéroarylalkyle inférieur,-(CR²⁹R³⁰)_{q}OR³¹, -(CR²⁹R³⁰)_{q}NR²⁸R³¹, -(CR²⁹R³⁰)_{q}NR²⁸COOR²¹, -(CR²⁹R³⁰)_{q}NR²⁸COR³¹, - (CR²⁹R³⁰)_{q}NR²⁸CONR²⁸R³¹, -(CR²⁹R³⁰)_{q}NR²⁸SO₂R²³, -(CR²⁹R³⁰)_{q}COOR³¹, -(CR²⁹R³⁰)_{q}CONR²⁸R³¹,-(CR²⁹R³⁰)_{q}SO₂NR²⁸R³¹, -(CR²⁹R³⁰)_{q}COR³¹, -(CR²⁹R³⁰)_{q}SO₂R²³ ou -(CR²⁹R³⁰)_{q}R³¹ ;
R³⁷ représente H, un alkyle inférieur, un alcényle inférieur, un cycloalkyle inférieur, un hétérocycloalkyle inférieur, un aryle, un hétéroaryle, un arylalkyle inférieur, un hétéroarylalkyle inférieur, un groupe N-protecteur adéquat, -(CR²⁹R³⁰)ᵣOR³¹, -(CR²⁹R³⁰)ᵣNR²⁸R³¹, - (CR²⁹R³⁰)ᵣNR²⁸COOR²¹, -(CR²⁹R³⁰)ᵣNR²⁸COR³¹, -(CR²⁹R³⁰)ᵣNR²⁸CONR²⁸R³¹, -(CR²⁹R³⁰)_{q}COOR²¹,-(CR²⁹R³⁰)_{q}CONR²⁸R³¹, -(CR²⁹R³⁰)_{q}COR²¹ ou -(CR²⁹R³⁰)_{q}R²¹ ;
R³⁸ représente H, un alkyle inférieur, un alcényle inférieur, un cycloalkyle inférieur, un hétérocycloalkyle inférieur, un aryle, un hétéroaryle, un arylalkyle inférieur, un hétéroarylalkyle inférieur, -(CR²⁹R³⁰)_{q}OR³¹, -(CR²⁹R³⁰)_{q}NR²⁸R³¹, -(CR²⁹R³⁰)_{q}NR²⁸COR³¹, -(CR²⁹R³⁰)_{q}NR²⁸CONR²⁸R³¹, - (CR²⁹R³⁰)_{q}COOR²¹, -(CR²⁹R³⁰)_{q}CONR²⁸R³¹, -(CR²⁹R³⁰)_{q}COR²¹ ou -(CR²⁹R³⁰)_{q}R³¹ ;
Z représente O, S ou S(=O)₂ ;
U représente -C(=O)-, -NR⁴-C(=O)-, -C(=O) -C(=O)- ou -C(-OR²⁰)₂-C(=O)-.

4. Composés selon la revendication 1, dans lesquels :
le bloc constitutif de type A est choisi parmi :
A1(a1), A2(a1), A3(a1), A4(a1), A5(a1), A6(a1), A7(a1), A9(a1), A10(a1), A73(a2), A170(a4), A209(a7), A240(a10), A272(a10), A532(a18), A614(a24), A631(a1), A632(a1), A633(a1), A641(a1), A642(a1), A643(a1), A651(a1), A652(a1) ou A653(a1) ;
le bloc constitutif de type B est choisi parmi :
B7(b3), B8(b3), B9(b3), B10(b3) ou B17(b5) ;
le bloc constitutif de type C est choisi parmi :
C1, C2, C4, C7, C8, C9, C10, C11, C12, C13, C14, C15, C16, C17, C18, C19, C20, C21, C25, C26, C27, C28, C29, C30, C34, C35, C36, C37, C38, C39, C43, C44, C45, C46, C47, C48, C49, C90, C91, C92 ou C93 ;
et dans lesquels
R¹H, F, Cl, CF₃, OCF₃, OCHF₂, NO_{2,} CN, un alkyle inférieur, un alcényle inférieur, un alcynyle inférieur, un cycloalkyle inférieur, un hétérocycloalkyle inférieur, un aryle, un hétéroaryle, u arylalkyle inférieur, un hétéroarylalkyle inférieur, -(CR¹⁸R¹⁹)_{q}OR²⁰, -(CR¹⁸R¹⁹)_{q}NR⁴R¹¹, - (CR¹⁸R¹⁹)qNR⁴COR²², -(CR¹⁸R¹⁹)_{q}NR⁴CONR⁴R¹¹, -(CR¹⁸R¹⁹)_{q}NR⁴SO₂R²³, -(CR¹⁸R¹⁹)qNR⁴SO₂NR⁴_{R}¹¹, - (CR¹⁸R¹⁹)_{q}COOR²¹, -(CR¹⁸R¹⁹)_{q}CONR⁴R¹¹, -(CR¹⁸R¹⁹)_{q}SO₂NR⁴_{R}11_{,} -(CR¹⁸R¹⁹)_{q}COR²², -(CR¹⁸R¹⁹)_{q}SO₂R²³, - (CR¹⁸R¹⁹)_{q}R²⁴, -(CR¹⁸R¹⁹)_{q}R²⁵ ou -(CR¹⁸R¹⁹)_{q}R²⁶ ;
R² représente H, CF₃, un alkyle inférieur, un alcényle inférieur, un cycloalkyle inférieur, un hétérocycloalkyle inférieur, un aryle, un hétéroaryle, un arylalkyle inférieur, un hétéroarylalkyle inférieur, -(CR¹⁸R¹⁹)_{q}OR²⁰, -(CR¹⁸R¹⁹)_{q}NR⁴R¹¹, -(CR¹⁸R¹⁹)_{q}NR⁴COR²², -(CR¹⁸R¹⁹)_{q}NR⁴CONR⁴R¹¹,-(CR¹⁸R¹⁹)_{q}NR⁴SO₂R²³, -(CR¹⁸R¹⁹)_{q}NR⁴SO₂NR⁴R¹¹, -(CR¹⁸R¹⁹)_{q}COOR²¹, -(CR¹⁸R¹⁹)_{q}CONR⁴R¹¹,-(CR¹⁸R¹⁹)_{q}SO₂R⁴R¹¹, -(CR¹⁸R¹⁹)_{q}COR²², -(CR¹⁸R¹⁹)_{q}SO₂R²³, -(CR¹⁸R¹⁹)_{q}R²⁴, -(CR¹⁸R¹⁹_{)q}R²⁵ ou-(CR¹⁸R¹⁹)_{q}R²⁶ ;
R⁴ représente H, un alkyle inférieur, un alcényle inférieur ou un groupe N-protecteur adéquat ;
R⁵, R⁷ et R⁹ sont indépendamment définis comme H, CF₃, un alkyle inférieur, un alcényle inférieur, un cycloalkyle inférieur, un hétérocycloalkyle inférieur, un aryle, un hétéroaryle, un arylalkyle inférieur, un hétéroarylalkyle inférieur, -(CR¹⁸R¹⁹)_{q}OR²⁰, -(CR¹⁸R¹⁹)_{q}NR⁴R¹¹, -(CR¹⁸R¹⁹)_{q}NR⁴COR²², - (CR¹⁸R¹⁹)_{q}NR⁴CONR⁴R¹¹, -(CR¹⁸R¹⁹)_{q}NR⁴SO₂R²³, -(CR¹⁸R¹⁹)_{q}COOR²¹, -(CR¹⁸R¹⁹)_{q}CONR⁴R¹¹,-(CR¹⁸R¹⁹)_{q}SO₂R⁴R¹¹, -(CR¹⁸R¹⁹)_{q}COR²², -(CR¹⁸R¹⁹)_{q}SO₂R²³, -(CR¹⁸R¹⁹)_{q}R²⁴, -(CR¹⁸R¹⁹)_{q}R²⁵ ou-(CR¹⁸R¹⁹)_{q}R²⁶ ;
R⁶, R⁸ et R¹⁰ sont indépendamment définis comme H, CF₃ ou CH₃ ;
R¹¹ représente H, un alkyle, un alcényle, un cycloalkyle, un hétérocycloalkyle inférieur, un aryle, un hétéroaryle, un arylalkyle inférieur, un hétéroarylalkyle inférieur, un groupe protecteur adéquat, -(CR¹⁸R¹⁹)ᵣOR²⁰, -(CR¹⁸R¹⁹)ᵣNR⁴R²⁷, -(CR¹⁸R¹⁹)ᵣNR⁴CONR⁴R²⁷, -(CR¹⁸R¹⁹),NR⁴SO₂R²³,-(CR¹⁸R¹⁹)_{q}COOR²¹, -(CR¹⁸R¹⁹)_{q}CONR⁴R²⁷, -(CR¹⁸R¹⁹)_{q}COR²², -(CR¹⁸R¹⁹)_{q}R²⁴, -(CR¹⁸R¹⁹)ₛR²⁵,-(CR¹⁸R¹⁹)_{q}R²⁶ ou -(CR¹⁸R¹⁹)_{q}R³¹ ;
R¹² et R¹³ sont indépendamment définis comme H ou comme un alkyle inférieur ;
R¹⁴ et R¹⁶ sont indépendamment définis comme H, F, CF₃, un alkyle inférieur, un alcényle inférieur, un cycloalkyle inférieur, un hétérocycloalkyle inférieur, un aryle, un hétéroaryle, un arylalkyle inférieur, un hétéroarylalkyle inférieur, -(CR¹⁸R¹⁹)qOR²⁰, (CR¹⁸R¹⁹)_{q}NR⁴R¹¹, -(CR¹⁸R¹⁹)_{q}NR⁴COR²², - (CR¹⁸R¹⁹)_{q}COOR²¹ ou -(CR¹⁸R¹⁹)_{q}CONR⁴R¹¹ ;
R¹⁵ et R¹⁷ sont indépendamment définis comme H, CF₃ ou CH₃ ;
R¹⁸ représente H, F, CF₃, un alkyle inférieur, un alcényle inférieur, un cycloalkyle inférieur, un hétérocycloalkyle inférieur, un aryle, un hétéroaryle, un arylalkyle inférieur, un hétéroarylalkyle inférieur, -(CR²⁹R³⁰)ₛOR³¹ -(CR²⁹R³⁰)ₛNR²⁸R³¹, -(CR²⁹_{R}³⁰)ₛNR²⁸COR³¹, -(CR²⁹R³⁰)ₛNR²⁸CONR²⁸R³¹, - (CR²⁹R³⁰)ₛNR²⁸SO₂R²³, -(CR²⁹R³⁰)_{q}COOR²¹, -(CR²⁹R³⁰)_{q}CONR²⁸R³¹, -(CR²⁹R³⁰)_{q}SO₂NR²⁸R³¹, - (CR²⁹R³⁰)_{q}COR³¹, -(CR²⁹R³⁰)_{q}SO₂R²³, -(CR²⁹R³⁰)_{q}R²⁴, -(CR²⁹R³⁰)_{q}R²⁵ ou -CR²⁹R³⁰)_{q}R²⁶ ;
R¹⁹ représente H, CF₃ ou CH₃ ;
R²⁰ représente H, un alkyle inférieur, un alcényle inférieur, un cycloalkyle inférieur, un hétérocycloalkyle inférieur, un aryle, un hétéroaryle, un arylalkyle inférieur, un hétéroarylalkyle inférieur, -(CR²⁹R³⁰)ᵣOR³¹, -(CR²⁹R³⁰)ᵣNR²⁸R³¹, -(CR²⁹R³⁰)ᵣNR²⁸COR³¹, -(CR²⁹R³⁰)ᵣNR²⁸CONR²⁸R³¹, - (CR²⁹R³⁰)ᵣNR²⁸SO₂R²³, -(CR²⁹R³⁰)_{q}COOR²¹, -(CR²⁹R³⁰)_{q}CONR²⁸R³¹, -(CR²⁹R³⁰)_{q}NR²⁸SO₂NR²⁸R³¹,-(CR²⁹R³⁰)_{q}COR³¹, -(CR²⁹R³⁰)_{q}SO₂R²³, -(CR²⁹R³⁰)_{q}R²⁴, -(CR²⁹R³⁰)_{q}R²⁵, -(CR²⁹R³⁰)qR²⁶ ou (CR²⁹R³⁰)_{q}R³¹ ;
R²² représente un alkyle inférieur, un alcényle inférieur, un cycloalkyle inférieur, un hétérocycloalkyle inférieur, un aryle, hétéroaryle, un arylalkyle inférieur, un hétéroarylalkyle inférieur, -(CR²⁹R³⁰)ₛOR³¹, -(CR²⁹R³⁰)ₛNR²⁸R³¹, -(CR²⁹R³⁰)ₛNR²⁸COR³¹, -(CR²⁹R³⁰)ₛNR²⁸CONR²⁸R³¹, - (CR²⁹R³⁰)ₛNR²⁸SO₂R²³, -(CR²⁹R³⁰)ₛCOOR²¹, -(CR²⁹R³⁰)ₛCONR²⁸R³¹, -(CR²⁹R³⁰)ₛSO₂NR²⁸R³¹, - (CR²⁹R³⁰)ₜCOR³¹, -(CR²⁹R³⁰)ₛSO₂R²³, -(CR²⁹R³⁰)ₜR²⁴, -(CR²⁹R³⁰)ₜR²⁵, -(CR²⁹R³⁰)ₜR²⁶ ou -(CR²⁹R³⁰)ₜR³¹ ;
R²⁹ représente H, F, CF₃, un alkyle inférieur, un alcényle inférieur, un cycloalkyle inférieur, un hétérocycloalkyle inférieur, un aryle, un hétéroaryle, un arylalkyle inférieur, un hétéroarylalkyle inférieur, -(CR³²R³³)ₛOR³¹, -(CR³²R³³)ₛNR²⁸R³¹, -(CR³²R³³)ₛNR²⁸COR³¹, -(CR³²R³³)ₛNR²⁸CONR²⁸R³¹, - (CR³²R³³)_{q}COOR²¹, -(CR³²R³³)_{q}CONR²⁸R³¹, -(_{CR}³²_{R}³³)_{q}COR³¹ ou -(CR³²R³³)_{q}R³¹ ;
R³⁰ et R³³ sont indépendamment définis comme H, CF₃ ou CH₃ ;
R³⁴ et R³⁵ sont indépendamment définis comme H, F, Cl, CF₃, OCF₃, OCHF₂, un alkyle inférieur, un alcényle inférieur, un alcynyle inférieur, un cycloalkyle inférieur, un hétérocycloalkyle inférieur, un aryle, un hétéroaryle, un arylalkyle inférieur ou un hétéroarylalkyle inférieur, -(CR²⁹R³⁰)_{q}OR³¹, - (CR²⁹R³⁰)_{q}NR²⁸R³¹, -(CR²⁹R³⁰)_{q}NR²⁸COR³¹, -(CR²⁹R³⁰)_{q}NR²⁸CONR²⁸R³¹, -(CR²⁹R³⁰)_{q}NR²⁸SO₂R²³, - (CR²⁹R³⁰)_{q}COOR³¹, -(CR²⁹R³⁰)_{q}CONR²⁸R³¹, -(CR²⁹R³⁰)_{q}SO₂NR²⁸R³¹, -(CR²⁹R³⁰)_{q}COR³¹, -(CR²⁹R³⁰)_{q}SO₂R²³ ou -(CR²⁹R³⁰)_{q}R³¹ ;
R³⁷ représente H, un alkyle inférieur, un alcényle inférieur, un cycloalkyle inférieur, un hétérocycloalkyle inférieur, un aryle, un hétéroaryle, un arylalkyle inférieur, un hétéroarylalkyle inférieur, un groupe N-protecteur adéquat, -(CR²⁹R³⁰)ᵣOR³¹, -(CR²⁹R³⁰)ᵣNR²⁸R³¹, - (CR²⁹R³⁰)ᵣNR²⁸COOR²¹, -(CR²⁹R³⁰)ᵣNR²⁸COR³¹, -(CR²⁹R³⁰)ᵣNR²⁸CONR²⁸R³¹, -(CR²⁹R³⁰⁾_{q}COOR²¹, - (CR²⁹R³⁰)_{q}CONR²⁸R³¹, -(CR²⁹R³⁰)_{q}COR²¹ ou -(CR²⁹R³⁰)_{q}R²¹ ;
R³⁸ représente H, un alkyle inférieur, un alcényle inférieur, un cycloalkyle inférieur, un hétérocycloalkyle inférieur, un aryle, un hétéroaryle, un arylalkyle inférieur, un hétéroarylalkyle inférieur, -(CR²⁹R³⁰)_{q}OR³¹, -(CR²⁹R³⁰)_{q}NR²⁸R³¹, -(CR²⁹R³⁰)_{q}NR²⁸COR³¹, -(CR²⁹R³⁰)_{q}NR²⁸CONR²⁸_{R}³¹, - (CR²⁹R³⁰)_{q}COOR²¹, -(CR²⁹R³⁰)_{q}CONR²⁸R³¹, -(CR²⁹R³⁰)_{q}COR²¹ ou -(CR²⁹R³⁰)_{q}R³¹ ;
Z représente O, S ou S(=O)₂ ;
U représente -C(=O)-, -NR⁴-C(=O)- ou -C(=O)-C(=O)-.

5. Composés selon la revendication 1, dans lesquels :
le bloc constitutif de type A est choisi parmi :
A1(a1), A2(a1), A3(a1), A4(a1), A5(a1), A6(a1), A7(a1), A9(a1), A10(a1), A73(a2), A170(a4), A209(a7), A240(a10), A272(a10), A532(a18), A614(a24), A631(a1), A632(a1), A633(a1), A641(a1), A642(a1), A643(a1), A651(a1), A652(a1) ou A653(a1) ;
le bloc constitutif de type B est choisi parmi :
B7(b3), B8(b3), B9(b3), B10(b3) ou B17(b5) ;
le bloc constitutif de type C est choisi parmi :
C1, C8, C9, C11, C12, C15, C17, C19, C20, C25 ou C47 ;
et dans lesquels
R¹ représente H, F, Cl, Br, CF₃, OCF₃, OCHF₂, CN, un alkyle inférieur, un alcényle inférieur, un alcynyle inférieur, un cycloalkyle inférieur, un hétérocycloalkyle inférieur, un aryle, un hétéroaryle, un arylalkyle inférieur, un hétéroarylalkyle inférieur, -(CR¹⁸R¹⁹)_{q}OR²⁰, - (CR¹⁸R¹⁹)_{q}NR⁴R¹¹, -(CR¹⁸R¹⁹)_{q}NR⁴COR²², -(CR¹⁸R¹⁹)_{q}NR⁴CONR⁴R¹¹, -(CR¹⁸R¹⁹)_{q}NR⁴SO₂R²³, - (CR¹⁸R¹⁹)_{q}COOR²¹, -(CR¹⁸R¹⁹)_{q}CONR⁴R¹¹, -(CR¹⁸R¹⁹)_{q}SO₂NR⁴R¹¹, -(CR¹⁸R¹⁹)_{q}COR²², -(CR¹⁸R¹⁹)_{q}SO₂R²³, - (CR¹⁸R¹⁹)_{q}R²⁴, -(CR¹⁸R¹⁹)_{q}R²⁵ ou -(CR¹⁸R¹⁹)_{q}R²⁶ ;
R² représente H, CF₃, un alkyle inférieur, un alcényle inférieur, un cycloalkyle inférieur, un hétérocycloalkyle inférieur, un aryle, un hétéroaryle, un arylalkyle inférieur, un hétéroarylalkyle inférieur, -(CR¹⁸R¹⁹)_{q}OR²⁰, -(CR¹⁸R¹⁹)_{q}NR⁴R¹¹, -(CR¹⁸R¹⁹)_{q}NR⁴COR²², -(CR¹⁸R¹⁹)_{q}NR⁴CONR⁴R¹¹, - (CR¹⁸R¹⁹)_{q}NR⁴SO₂R²³, -(CR¹⁸R¹⁹)_{q}COOR²¹, -(CR¹⁸R¹⁹)_{q}CONR⁴R¹¹, -(CR¹⁸R¹⁹)_{q}SO₂R⁴_{R}¹¹_{,} -(CR¹⁸R¹⁹)_{q}COR²², -(CR¹⁸R¹⁹)_{q}SO₂R²³, -(CR¹⁸R¹⁹)_{q}R²⁴, -(CR¹⁸R¹⁹)_{q}R²⁵ ou -(CR¹⁸R¹⁹)_{q}R²⁶ ;
R⁴ représente H, l'acétyle, un alkyle inférieur, un alcényle inférieur ou un groupe N-protecteur adéquat ;
R⁵, R⁷ et R⁹ sont indépendamment définis comme H, CF₃, un alkyle inférieur, un alcényle inférieur, un cycloalkyle inférieur, un hétérocycloalkyle inférieur, un aryle, un hétéroaryle, un arylalkyle inférieur, un hétéroarylalkyle inférieur, -(CR¹⁸R¹⁹)_{q}OR²⁰, -(CR¹⁸R¹⁹)_{q}NR⁴R¹¹, -(CR¹⁸R¹⁹)_{q}NR⁴COR²², - (CR¹⁸R¹⁹)_{q}NR⁴CONR⁴R¹¹, -(CR¹⁸R¹⁹)_{q}NR⁴SO₂R²³, -(CR¹⁸R¹⁹)_{q}COOR²¹, -(CR¹⁸R¹⁹)qCONR⁴R¹¹, - (CR¹⁸R¹⁹)_{q}NR⁴SO₂R²³, -(CR¹⁸R¹⁹)_{q}COOR²¹, -(CR¹⁸R¹⁹)_{q}CONR⁴_{R}¹¹, -(CR¹⁸R¹⁹)_{q}SO_{2R}⁴_{R}¹¹, -(CR¹⁸R¹⁹)_{q}COR²², -(CR¹⁸R¹⁹)_{q}SO₂R²³, -(CR¹⁸R¹⁹)_{q}R²⁴, -(CR¹⁸R¹⁹)_{q}R²⁵ ou -(CR¹⁸R¹⁹)_{q}R²⁶ ;
R⁶, R⁸ et R¹⁰ sont indépendamment définis comme H, CF₃ ou CH₃, ou benzyle ;
R¹¹ représente H, un alkyle, un alcényle, un cycloalkyle, un hétérocycloalkyle inférieur, un aryle, un hétéroaryle, un arylalkyle inférieur, un hétéroarylalkyle inférieur, un groupe protecteur adéquat, -(CR¹⁸R¹⁹)ᵣOR²⁰, -(CR¹⁸R¹⁹)ᵣNR⁴R²⁷, -(CR¹⁸R¹⁹)ᵣNR⁴CONR⁴R²⁷, -(CR¹⁸R¹⁹)_{q}COR²²,-(CR¹⁸R¹⁹)_{q}R²⁴, -(CR¹⁸R¹⁹)ₛR²⁵, -(CR¹⁸R¹⁹)_{q}R²⁶ ou -(CR¹⁸R¹⁹)_{q}R³¹ ;
R¹² et R¹³ sont indépendamment définis comme H ou comme un alkyle inférieur ;
R¹⁴ et R¹⁶ sont indépendamment définis comme H, F, CF₃, un alkyle inférieur, un alcényle inférieur, un cycloalkyle inférieur, un hétérocycloalkyle inférieur, un aryle, un hétéroaryle, un arylalkyle inférieur, un hétéroarylalkyle inférieur, -(CR¹⁸R¹⁹)_{q}OR²⁰, (CR¹⁸R¹⁹)_{q}NR⁴R¹¹, -(CR¹⁸R¹⁹)_{q}NR⁴COR²², - (CR¹⁸R¹⁹)_{q}COOR²¹ ou -(CR¹⁸R¹⁹)_{q}CONR⁴R¹¹ ;
R¹⁵ et R¹⁷ sont indépendamment définis comme H, CF₃ ou CH₃ ;
R¹⁸ représente H, F, CF₃, un alkyle inférieur, un alcényle inférieur, un cycloalkyle inférieur, un hétérocycloalkyle inférieur, un aryle, un hétéroaryle, un arylalkyle inférieur, un hétéroarylalkyle inférieur, -(CR²⁹R³⁰)ₛOR³¹, -(CR²⁹R³⁰)ₛNR²⁸R³¹, -(CR²⁹R³⁰)ₛNR²⁸COR³¹, -(CR²⁹R³⁰)ₛNR²⁸CONR²⁸R³¹, - (CR²⁹R³⁰)ₛNR²⁸SO₂R²³, -(CR²⁹R³⁰)_{q}COOR²¹, -(_{CR}²⁹R³⁰)_{q}CONR²⁸R³¹, -(CR²⁹R³⁰)_{q}SO₂NR²⁸R³¹, - (CR²⁹R³⁰)_{q}COR³¹, -(CR²⁹R³⁰)_{q}SO₂R²³, -(CR²⁹R³⁰)_{q}R²⁴, -(CR²⁹R³⁰)qR²⁵ ou -(CR²⁹R³⁰)_{q}R²⁶ ;
R¹⁹ représente H, CF₃ ou CH₃ ;
R²⁰ représente H, un alkyle inférieur, un alcényle inférieur, un cycloalkyle inférieur, un hétérocycloalkyle inférieur, un aryle, un hétéroaryle, un arylalkyle inférieur, un hétéroarylalkyle inférieur, -(CR²⁹R³⁰)ᵣOR³¹, -(CR²⁹R³⁰)ᵣNR²⁸R³¹, -(CR²⁹R³⁰)ᵣNR²⁸COR³¹, -(CR²⁹R³⁰)ᵣNR²⁸CONR²⁸R³¹, - (CR²⁹R³⁰)ᵣNR²⁸SO₂R²³, -(CR²⁹R³⁰)_{q}COOR²¹, -(CR²⁹R³⁰)_{q}CONR²⁸R³¹, -(CR²⁹R³⁰)_{q}NR²⁸SO₂NR²⁸R³¹, - (CR²⁹R³⁰)_{q}COR³¹, -(CR²⁹R³⁰)_{q}SO₂R²³, -(CR²⁹R³⁰)_{q}R²⁴, -(CR²⁹R³⁰)_{q}R²⁵, -(CR²⁹R³⁰)_{q}R²⁶ ou (_{CR}²⁹_{R}³⁰)_{q}R³¹ ;
R²² représente un alkyle inférieur, un alcényle inférieur, un cycloalkyle inférieur, un hétérocycloalkyle inférieur, un aryle, un hétéroaryle, un arylalkyle inférieur, un hétéroarylalkyle inférieur, -(CR²⁹R³⁰)ₛOR³¹, -(CR²⁹R³⁰)ₛNR²⁸R³¹, -(CR²⁹R³⁰)ₛNR²⁸COR³¹, -(CR²⁹R³⁰)ₛNR²⁸CONR²⁸R³¹, - (CR²⁹R³⁰)ₛNR²⁸SO₂R²³, -(CR²⁹R³⁰)ₛCOOR²¹, -(CR²⁹R³⁰)ₛCONR²⁸R³¹, -(CR²⁹R³⁰)ₛSO₂NR²⁸R³¹, - (CR²⁹R³⁰)ₜCOR³¹, -(CR²⁹R³⁰)ₛSO₂R²³, -(CR²⁹R³⁰)ₜR²⁴, -(CR²⁹R³⁰)ₜR²⁵, -(CR²⁹R³⁰)ₜR²⁶ ou -(CR²⁹R³⁰)ₜR³¹ ;
R²⁴ représente un aryle, un hétéroaryle ou -C₆H₂R³⁴R³⁵R³¹ ;
R²⁵ et R²⁶ sont indépendamment définis comme des hétérocycloalkyles ;
R²⁷ représente H, un alkyle inférieur, un alcényle inférieur, un cycloalkyle inférieur, un hétérocycloalkyle inférieur, un aryle, un hétéroaryle, un arylalkyle inférieur, un hétéroarylalkyle inférieur, un groupe protecteur adéquat ou -(CR²⁹R³⁰)_{q}R²⁴ ;
R²⁸ représente H, -(CR³²R³³)ₛOR²¹ ou -(CR³²R³³)ₛNR⁴²R⁴³ ;
R²⁹ représente H, F, CF₃, un alkyle inférieur, un alcényle inférieur, un cycloalkyle inférieur, un hétérocycloalkyle inférieur, un aryle, un hétéroaryle, un arylalkyle inférieur, un hétéroarylalkyle inférieur, -(CR³²R³³)ₛOR³¹, -(CR³²R³³)ₛNR²⁸R³¹, -(CR³²R³³)ₛNR²⁸COR³¹, -(CR³²R³³)ₛNR²⁸CONR²⁸R³¹, - (CR³²R³³)_{q}COOR²¹, -(CR³²R³³)_{q}CONR²⁸R³¹, -(CR³²R³³)_{q}COR³¹ ou -(CR³²R³³)_{q}R³¹ ;
R³⁰ et R³³ sont indépendamment définis comme H, F, CF₃ ou CH₃ ;
R³¹ représente H, un alkyle, un alcényle, un cycloalkyle, un hétérocycloalkyle, un aryle, un hétéroaryle, un arylalkyle, un hétéroarylalkyle ;
R³² représente H, F, CF₃, un alkyle inférieur, un alcényle inférieur, un cycloalkyle inférieur, un hétérocycloalkyle inférieur, un aryle, un hétéroaryle, un arylalkyle inférieur ou un hétéroarylalkyle inférieur ;
R³⁴ et R³⁵ sont indépendamment définis comme H, F, Cl, CF₃, un alkyle inférieur, un alcényle inférieur, un cycloalkyle inférieur, un hétérocycloalkyle inférieur, un aryle, un hétéroaryle, un arylalkyle inférieur ou un hétéroarylalkyle inférieur, -(CR²⁹R³⁰)_{q}OR³¹, -(CR²⁹R³⁰)_{q}NR²⁸R³¹, - (CR²⁹R³⁰)_{q}NR²⁸COR³¹, -(CR²⁹R³⁰)_{q}NR²⁸CONR²⁸R³¹, -(CR²⁹R^{3o})_{q}NR²⁸SO₂R²³, -(CR²⁹R³⁰)_{q}COOR³¹, - (CR²⁹R³⁰)_{q}CONR²⁸R³¹, -(CR²⁹R³⁰)_{q}SO₂NR²⁸R³¹, -(CR²⁹R³⁰)_{q}COR³¹, -(CR²⁹R³⁰)_{q}SO₂R²³ ou -(CR²⁹R³⁰)_{q}R³¹ ;
R³⁸ représente H, un alkyle inférieur, un alcényle inférieur, un cycloalkyle inférieur, un hétérocycloalkyle inférieur, un aryle, un hétéroaryle, un arylalkyle inférieur, un hétéroarylalkyle inférieur, -(CR²⁹R³⁰)_{q}OR³¹, -(CR²⁹R³⁰)_{q}NR²⁸R³¹, -(CR²⁹R³⁰)_{q}NR²⁸COR³¹, -(CR²⁹R³⁰)_{q}NR²⁸CONR²⁸R³¹,-(CR²⁹R³⁰)_{q}COOR²¹, -(CR²⁹R³⁰)_{q}CONR²⁸R³¹, -(CR²⁹R³⁰_{)q}COR²¹ ou -(CR²⁹R³⁰)_{q}R³¹ ;
R⁴² et R⁴³ sont indépendamment définis comme H, un alkyle inférieur, un alcényle inférieur, un cycloalkyle inférieur, un hétérocycloalkyle inférieur, un aryle, un hétéroaryle, un arylalkyle inférieur, un hétéroarylalkyle inférieur ou un groupe N-protecteur adéquat ;
X représente O, S, S(=O) ou S(=O)₂ ;
Y représente O, S ou NR⁴ ;
Z représente O, S ou S(=O)₂ ;
U représente -C(=O)-, -NR⁴-C(=O)- ou -C(=O)-C(=O)- ;
ou leurs sels pharmaceutiquement acceptables, solvates, clathrates, versions isotopiquement ou énantiomériquement enrichies.

6. Composés selon la revendication 1, dans lesquels :
le bloc constitutif de type A est choisi parmi :
A2(a1), A5(a1), A9(a1), A73(a2), A209(a7), A272(a10), A532(a18), A614(a24), A641(a1) ou A651(a1) ;
le bloc constitutif de type B est choisi parmi :
B7, B9, B10 ou B17 ;
le bloc constitutif de type C est choisi parmi :
C1, C8, C9, C11, C12, C15, C17, C19, C20, C25 ou C47 ;
et dans lesquels
R¹ représente H, F, Cl, Br, CH₃, OH ou un aryle ;
R² représente H, CH₃, un hétérocycloalkyle, un aryle, un benzyle, un hétéroaryle, -OR²⁰, -NR⁴R¹¹,-NR⁴COR²², -NR⁴CONR⁴R¹¹ ou -NR⁴SO²R²³ ;
R³ représente H ;
R⁴ représente H, l'acétyle, un alkyle inférieur, un alcényle inférieur ou un groupe N-protecteur adéquat ;
R⁵, R⁷ et R⁹ sont indépendamment définis comme H, CF₃, un alkyle inférieur, un alcényle inférieur, un cycloalkyle inférieur, un hétérocycloalkyle inférieur, un aryle, un hétéroaryle, un arylalkyle inférieur, un hétéroarylalkyle inférieur, -OR²⁰, -NR⁴R¹¹, -NR⁴COR²², -NR⁴CONR⁴R¹¹, -NR⁴SO₂R²³,-COOR²¹, -CONR⁴R¹¹, -COR²² ;
R⁶, R⁸ et R¹⁰ sont indépendamment définis comme H ;
R¹¹ représente H, un alkyle, un aryle, un hétéroaryle, un arylalkyle inférieur, un hétéroarylalkyle inférieur, -(CR¹⁸R¹⁹)ᵣOR²⁰, -(CR¹⁸R¹⁹)ᵣNR⁴R²⁷, -(CR¹⁸R¹⁹)_{q}COOR²¹, -COR²², -(CR¹⁸R¹⁹)qR²⁴ ou-(CR¹⁸R¹⁹)_{q}R³¹ ;
R¹² et R¹³ sont indépendamment définis comme H ;
R¹⁴ et R¹⁶ sont indépendamment définis comme H, un alkyle inférieur ou un arylalkyle inférieur ;
R¹⁵ et R¹⁷ sont indépendamment définis comme H ;
R¹⁸ et R¹⁹ sont indépendamment définis comme H ;
R²⁰ représente H, un aryle, un hétéroaryle, un arylalkyle inférieur, un hétéroarylalkyle inférieur ou -(CR²⁹R³⁰)_{q}R²⁴ ;
R²¹ représente H, un alkyle inférieur, un arylalkyle inférieur ou un groupe O-protecteur adéquat ;
R²² représente un alkyle inférieur, un alcényle inférieur, un aryle, un hétéroaryle, un arylalkyle inférieur, un hétéroarylalkyle inférieur, -(CR²⁹R³⁰)ₛOR³¹, -(CR²⁹R³⁰)ₛNR²⁸R³¹, -(CR²⁹R³⁰)ₛCOOR²¹,-(CR²⁹R³⁰)ₜR²⁴ ou -(CR²⁹R³⁰)ₜR³¹;
R²³ représente H, un alkyle inférieur, un aryle, un hétéroaryle ou -R²⁴ ;
R²⁴ représente un aryle, un hétéroaryle ou -C₆H₂R³⁴R³⁵R³¹ ;
R²⁷ représente H, l'acétyle, CH₃ ou -(CH₂)_{q}R²⁴ ;
R²⁸ représente H, -(CR³²R³³)ₛOR²¹ ou -(CR³²R³³)ₛNR⁴²R⁴³ ;
R²⁹ représente H, un alkyle inférieur, un aryle ou un hétéroaryle ;
R³⁰, R³² et R³³ sont indépendamment définis comme H, CF₃ ou CH₃ ;
R³¹ représente H, un alkyle, un cycloalkyle, un hétérocycloalkyle, un aryle, un hétéroaryle, un arylalkyle, un hétéroarylalkyle ;
R³⁴ et R³⁵ sont indépendamment définis comme H, un hétérocycloalkyle, -OR³¹ ou -NR²⁸R³¹ ;
R³⁸ représente H, -NR²⁸R³¹, -NR²⁸COR³¹ ou -NR²⁸COOR³¹ ;
R⁴² et R⁴³ sont indépendamment définis comme H, CH₃ ou un benzyle ;
X représente O, S, S(=O) ou S(=O)₂ ;
Y représente O, S ou NR⁴ ;
Z représente O, S ou S(=O)₂ ;
U représente -C(=O)-, -NR⁴-C(=O)- ou -C(=O)-C(=O)- ;
ou leurs sels pharmaceutiquement acceptables, solvates, clathrates, versions isotopiquement ou énantiomériquement enrichies.

7. Composés selon la revendication 4, dans lesquels :
le bloc constitutif de type C est représenté par : 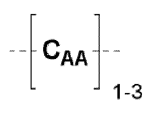
où C_{AA} est un acide aminé ou son énantiomère complémentaire choisi dans la liste de :
Ala L-alanine
Arg L-arginine
Asn L-asparagine
Asp acide L-aspartique
Cys L-cystéine
Glu acide L-glutamique
Gln L-glutamine
Gly glycine
His L-histidine
Ile L-isoleucine
Leu L-leucine
Lys L-lysine
Met L-méthionine
Phe L-phénylalanine
Pro L-proline
Ser L-sérine
Thr L-thréonine
Trp L-tryptophane
Tyr L-tyrosine
Val L-valine
Apa acide 3-aminopropanoïque
H-β³-HAla-OH acide (3S)-3-aminobutyrique
H-β³-HVal-OH acide (3R)-3-amino-4-méthylvalérique
H-β³-HIle-OH acide (3R,4S)-3-amino-4-méthylhexanoïque
H-β³-HLeu-OH acide (3S)-3-amino-5-méthylhexanoïque
H-β³-HMet-OH acide (3S)-3-amino-5-méthylthiopentanoïque
H-β³-HTyr-OH acide (3S)-3-amino-4-(4'-hydroxyphényl)butyrique
H-β³-HHis-OH acide (3S)-3-amino-4-(imidazol-4'-yl)butyrique
H-β³-HPhe-OH acide (3S)-3-amino-4-phénylbutyrique
H-β³-HTrp-OH acide (3S)-3-amino-4-(indol-3'-yl)butyrique
H-β³-HSer-OH acide (3S)-3-amino-4-hydroxybutyrique
H-β³-HAsp-OH acide 3-amino-4-pentanedioïque
H-β³-HGlu-OH acide (3S)-3-aminohexanedioïque
H-β³-HLys-OH acide (3S)-3,7-diaminoheptanoïque
H-β³-HArg-OH acide (3S)-3-amino-6-guanidinohexanoïque
H-β³-HCys-OH acide (3R)-3-amino-4-mercaptobutyrique
H-β³-HAsn-OH acide (3S)-3-amino-4-carbamoylbutyrique
H-β³-HGln-OH acide (3S)-3-amino-4-carbamoylpentanoïque
H-β³-HThr-OH acide (3R,4S)-3-amino-4-hydroxypentanoïque
Gaba acide 4-aminobutyrique
H-γ⁴-DiHAla-OH acide (4S)-4-aminopentanoïque
H-γ⁴-DiHVal-OH acide (4R)-4-amino-5-méthylhexanoïque
H-γ⁴-DiHIle-OH acide (4R,5S)-4-amino-5-méthylheptanoïque
H-γ⁴-DiHLeu-OH acide (4R)-4-amino-6-méthylheptanoïque
H-γ⁴-DiHMet-OH acide (4R)-4-amino-6-méthylthiohexanoïque
H-γ⁴-DiHTyr-OH acide (4R)-4-amino-5-(4'-hydroxypényl)pentanoïque
H-γ⁴-DiHHis-OH acide (4R)-4-amino-5-(imidazol-4'-yl)pentanoïque
H-γ⁴-DiHPhe-OH acide (4R)-4-amino-4-phénylpentanoïque
H-γ⁴-DiHTrp-OH acide (4R)-4-amino-5-(indol-3'-yl)pentanoïque
H-γ⁴-DiHSer-OH acide (4R)-4-amino-5-hydroxypentanoïque
H-γ⁴-DiHAsp-OH acide (4R)-4-aminohexanedioïque
H-γ⁴-DiHGlu-OH acide 4-aminoheptanedioïque
H-γ⁴-DiHLys-OH acide (4S)-4,8-diaminooctanoïque
H-γ⁴-DiHArg-OH acide (4S)-4-amino-7-guanidinoheptanoïque
H-γ⁴-DiHCys-OH acide (4R)-4-amino-5-mercaptopentanoïque
H-γ⁴-DiHAsn-OH acide (4S)-4-amino-5-carbamoylpentanoïque
H-γ⁴-DiHGln-OH acide (3S)-3-amino-5-carbamoylhexanoïque
H-γ⁴-DiHThr-OH acide (4R,5R)-4-amino-5-hydroxyhexanoïque
Cit L-citrulline
Orn L-ornithine
tBuA L-t-butylalanine
Sar sarcosine
Pen L-pénicillamine
tBuG L-tertiobutylglycine
4AmPhe L-para-aminophénylalanine
3AmPhe L-méta-aminophénylalanine
2AmPhe L-ortho-aminophénylalanine
Phe(mC(NH₂)=NH) L-méta-amidinophénylalanine
Phe(pC(NH₂)=NH) L-para-amidinophénylalanine
Phe(mNHC(NH₂)=NH) L-méta-guanidinophénylalanine
Phe(mNHC(NH₂)=NH) L-para-guanidinophénylalanine
2Pal acide (2S)-2-amino-3-(pyridin-2'-yl)propionique
4Pal acide (2S)-2-amino-3-(pyridin-4'-yl)propionique
Phg L-phénylglycine
Cha L-cyclohexylalanine
C₄al L-3-cyclobutylalanine
C₅al L-3-cyclopentylalanine
Nle L-norleucine
2-Nal L-2-naphtylalanine
1-Nal L-1-naphtylalanine
4ClPhe L-4-chlorophénylalanine
3ClPhe L-3-chlorophénylalanine
2ClPhe L-2-chlorophénylalanine
3,4Cl₂Phe L-3,4-dichlorophénylalanine
4FPhe L-4-fluorophénylalanine
3FPhe L-3-fluorophénylalanine
2FPhe L-2-fluorophénylalanine
Thi L-β-2-thiénylalanine
Tza L-2-thiazolylalanine
Mso L-méthionine sulfoxyde
AcLys N-acétyllysine
Dap acide 2,3-diaminopropionique
Dab acide 2,3-diaminobutyrique
Dbu acide (2S)-2,3-diaminobutyrique
Abu acide γ-aminobutyrique (GABA)
Aha acide ε-aminohexanoïque
Aib acide α-aminoisobutyrique
ACC acide 1-aminocyclopropane carboxylique
ACBC acide 1-aminocyclobutane carboxylique
ACPC acide 1-aminocyclopentane carboxylique
ACHC acide 1-aminocyclohexane carboxylique
Y(Bzl) L-O-benzyltyrosine
H(Bzl) acide (3S)-2-amino-3-(1'-benzylimidazol-4'-yl)propionique
Bip L-(4-phényl)phénylalanine
S(Bzl) L-O-benzylsérine
T(Bzl) L-O-benzylthréonine
alloT acide (2S,3S)-2-amino-3-hydroxybutyrique
Leu3OH acide (2S,3R)-2-amino-3-hydroxy-4-méthylpentanoïque
hAla L-homoalanine
hArg L-homoarginine
hCys L-homocystéine
hGlu acide L-homoglutamique
hGln L-homoglutamine
hHis L-homohistidine
hIle L-homoisoleucine
hLeu L-homoleucine
hNle L-homonorleucine
hLys L-homolysine
hMet L-homométhionine
hPhe L-homophénylalanine
hSer L-homosérine
hThr L-homothréonine
hTrp L-homotryptophane
hTyr L-homotyrosine
hVal L-homovaline
hCha L-homocyclohexylalanine
Bpa L-4-benzoylphénylalanine
OctG L-octylglycine
Tic acide (3S)-1,2,3,4-tétrahydroisoquinoline-3-carboxylique
Tiq acide (1S)-1,2,3,4-tétrahydroisoquinoline-1-carboxylique
Oic acide (2S,3aS,7aS)-1-octahydro-1H-indole-2-carboxylique
4AmPyrr1 acide (2S,4S)-4-aminopyrrolidine-2-carboxylique
4AmPyrr2 acide (2S,4R)-4-aminopyrrolidine-2-carboxylique
4PhePyrr1 acide (2S,4R)-4-phénylpyrrolidine-2-carboxylique
4PhePyrr2 acide (2S,4S)-4-phénylpyrrolidine-2-carboxylique
5PhePyrr1 acide (2S,5R)-5-phénylpyrrolidine-2-carboxylique
5PhePyrr2 acide (2S,5S)-5-phénylpyrrolidine-2-carboxylique
4Hyp1 (4S)-L-hydroxyproline
4Hyp2 (4R)-L-hydroxyproline
4Mp1 (4S)-L-mercaptoproline
4Mp2 (4R)-L-mercaptoproline
Pip acide L-pipécolique
H-β³-HCit-OH acide (3S)-3-amino-6-carbamidylhexanoïque
H-β³-HOrn-OH acide (3S)-3,6-diaminohexanoïque
H-β³-HtBuA-OH acide (3S)-3-amino-5,5-diméthylhexanoïque
H-β³-HSar-OH acide N-méthyl-3-aminopropionique
H-β³-HPen-OH acide (3R)-3-amino-4-méthyl-4-mercaptopentanoïque
H-β³-HtBuG-OH acide (3R)-3-amino-4,4-diméthylpentanoïque
H-β³-H4AmPhe-OH acide (3S)-3-amino-4-(4'-aminophényl)butyrique
H-β³-H3AmPhe-OH acide (3S)-3-amino-4-(3'-aminophényl)butyrique
H-β³-H2AmPhe-OH acide (3S)-3-amino-4-(2'-aminophényl)butyrique
H-β³-HPhe(mC(NH₂)=NH)-OH acide (3S)-3-amino-4-(3'-amidinophényl)butyrique
H-β³-HPhe(pC(NH₂)=NH)-OH acide (3S)-3-amino-4-(4'-amidinophényl)butyrique
H-β³-HPhe(mNHC(NH₂)=NH)-OH acide (3S)-3-amino-4-(3'-guanidinophényl)butyrique
H-β³-HPhe(pNHC(NH₂)=NH)-OH acide (3S)-3-amino-4-(4'-guanidinophényl)butyrique
H-β³-H2Pal-OH acide (3S)-3-amino-4-(pyridin-2'-yl)butyrique
H-β³-H4Pal-OH acide (3S)-3-amino-4-(pyridin-4'-yl)butyrique
H-β³-HPhg-OH acide (3R)-3-amino-3-phénylpropionique
H-β³-HCha-OH acide (3S)-3-amino-4-cyclohexylbutyrique
H-β³-HC₄al-OH acide (3S)-3-amino-4-cyclobutylbutyrique
H-β³-HC₅al-OH acide (3S)-3-amino-4-cyclopentylbutyrique
H-β³-HNle-OH acide (3S)-3-aminoheptanoïque
H-β³-H2Nal-OH acide (3S)-3-amino-4-(2'-naphtyl)butyrique
H-β³-H1Nal-OH acide (3S)-3-amino-4-(1'-naphtyl)butyrique
H-β³-H4ClPhe-OH acide (3S)-3-amino-4-(4'-chlorophényl)butyrique
H-β³-H3ClPhe-OH acide (3S)-3-amino-4-(3'-chlorophényl)butyrique
H-β³-H2ClPhe-OH acide (3S)-3-amino-4-(2'-chlorophényl)butyrique
H-β³-H3,4Cl₂Phe-OH acide (3S)-3-amino-4-(3',4'-dichlorophényl)butyrique
H-β³-H4FPhe-OH acide (3S)-3-amino-4-(4'-fluorophényl)butyrique
H-β³-H3FPhe-OH acide (3S)-3-amino-4-(3'-fluorophényl)butyrique
H-β³-H2FPhe-OH acide (3S)-3-amino-4-(2'-fluorophényl)butyrique
H-β³-HThi-OH acide (3R)-3-amino-4-(2'-thiényl)butyrique
H-β³-HTza-OH acide (3R)-3-amino-4-(2'-thiazolyl)butyrique
H-β³-HMso-OH acide (3R)-3-amino-4-méthylsulfoxylbutyrique
H-β³-HAcLys-OH acide (3S)-7-acétylamino-3-aminoheptanoïque
H-β³-HDpr-OH acide (3R)-3,4-diaminobutyrique
H-β³-HA₂Bu-OH acide (3S)-3,5-diaminopentanoïque
H-β³-HDbu-OH acide (3R)-3,4-diaminopentanoïque
H-β³-HAib-OH acide aminodiméthylacétique
H-β³-HCyp-OH acide 1-aminocyclopentan-1-ylacétique
H-β³-HY(Bzl)-OH acide (3S)-3-amino-4-(4'-benzyloxyphényl)butyrique
H-β³-HH(Bzl)-OH acide (3S)-3-amino-4-(1'-benzylimidazol-4'-yl)butyrique
H-β³-HBip-OH acide (3S)-3-amino-4-diphénylylbutyrique
H-β³-HS(Bzl)-OH acide (3S)-3-amino-4-benzyloxybutyrique
H-β³-HT(Bzl)-OH acide (3R,4S)-3-amino-4-benzyloxypentanoïque
H-β³-HalloT-OH acide (3R,4S)-3-amino-4-hydroxypentanoïque
H-β³-HLeu3OH-OH acide (3R,4S)-3-amino-4-hydroxy-5-méthylhexanoïque
H-β³-HhAla-OH acide (3S)-3-aminopentanoïque
H-β³-HhArg-OH acide (3S)-3-amino-7-guanidinoheptanoïque
H-β³-HhCys-OH acide (3R)-amino-5-mercaptopentanoïque
H-β³-HhGlu-OH acide (3S)-3-aminoheptanedioïque
H-β³-HhGln-OH acide (3S)-3-amino-6-carbamoylhexanoïque
H-β³-HhHis-OH acide (3S)-3-amino-5-(imidazole-4'-yl)pentanoïque
H-β³-HhIle-OH acide (3S,5S)-3-amino-5-méthylheptanoïque
H-β³-HhLeu-OH acide (3S)-3-amino-6-méthylheptanoïque
H-β³-HhNle-OH acide (3S)-3-aminooctanoïque
H-β³-DiAoc-OH acide (3S)-3,8-diaminooctanoïque
H-β³-HhMet-OH acide (3S)-3-amino-6-méthylthiohexanoïque
H-β³-HhPe-OH acide (3S)-3-amino-5-phénylpentanoïque
H-β³-HhSer-OH acide (3S)-3-amino-5-hydroxypentanoïque
H-β³-HhThr-OH acide (3S,5R)-3-amino-5-hydroxyhexanoïque
H-β³-HhTrp-OH acide (3S)-3-amino-5-(indol-3'-yl)pentanoïque
H-β³-HhTyr-OH acide (3S)-3-amino-5-(4'-hydroxyphényl)pentanoïque
H-β³-HhCha-OH acide (3S)-3-amino-5-cyclohexylpentanoïque
H-β³-HhBpa-OH acide (3S)-3-amino-4-(4'-benzoylphényl)butyrique
H-β³-HOctG-OH acide (3S)-3-amino-undécanoïque
H-β³-HNle-OH acide (3S)-3-aminoheptanoïque
H-β³-HTic-OH acide (3S)-1,2,3,4-tétrahydroisoquinolin-3-ylacétique
H-β³-HTiq-OH acide (1S)-1,2,3,4-tétrahydroisoquinolin-3-ylacétique
H-β³-HOic-OH acide (2S,3aS,7aS)-1-octahydro-1H-indol-2-ylacétique
H-β³-H4AmPyrr1-OH acide (2S,4S)-4-aminopyrrolidin-2-acétique
H-β³-H4AmPyrr2-OH acide (2S,4R)-4-aminopyrrolidin-2-acétique
H-β³-H4PhePyrr1-OH acide (2S,4R)-4-phénylpyrrolidin-2-acétique
H-β³-H4PhePyrr2-OH acide (2S,4S)-4-phénylpyrrolidin-2-acétique
H-β³-H4Hyp1-OH acide (2S,4S)-4-hydroxypyrrolidin-2-acétique
H-β³-H4Hyp2-OH acide (2S,4R)-4-hydroxypyrrolidin-2-acétique
H-β³-H4Mp1-OH acide (2R,4S)-4-mercaptopyrrolidin-2-acétique
H-β³-H4Mp2-OH acide (2R,4R)-4-mercaptopyrrolidin-2-acétique
H-β³-HPip-OH acide (2S)-pipéridin-2-acétique
H-β³-HPro-OH acide (2S)-pyrrolidin-2-acétique
Ahb acide 4-amino-2-hydroxybutyrique
H-γ⁴-DiHCit-OH acide (4S)-4-amino-7-carbamidylhexanoïque
H-γ⁴-DiHOrn-OH acide (4S)-4,7-diaminoheptanoïque
H-γ⁴-DiHtBuA-OH acide (4R)-4-amino-6,6-diméthylheptanoïque
H-γ⁴-DiHSar-OH acide N-méthyl-4-aminobutyrique
H-γ⁴-DiHPen-OH acide (4R)-4-amino-5-méthyl-5-mercaptohexanoïque
H-γ⁴-DiHtBuG-OH acide (4R)-4-amino-5,5-diméthylhexanoïque
H-γ⁴-DiH4AmPhe-OH acide (4R)-4-amino-5-(4'-aminophényl)pentanoïque
H-γ⁴-DiH3AmPhe-OH acide (4R)-4-amino-5-(3'-aminophényl)pentanoïque
H-γ⁴-DiH2AmPhe-OH acide (4R)-4-amino-5-(2'-aminophényl)pentanoïque
H-γ⁴-DiHPhe(mC(NH₂)=NH)-OH acide (4R)-4-amino-5-(3'-amidinophényl)pentanoïque
H-γ⁴-DiHPhe(pC(NH₂)=NH)-OH acide (4R)-4-amino-5-(4'-amidinophényl)pentanoïque
H-γ⁴-DiHPhe(mNHC(NH₂)=NH)-OH acide (4S)-4-amino-5-(3'-guanidinophényl)pentanoïque
H-γ⁴-DiHPhe(pNHC(NH₂)=NH)-OH acide (4S)-4-amino-5-(4'-guanidinophényl)pentanoïque
H-γ⁴-DiH2Pal-OH acide (4R)-4-amino-5-(pyridin-2'-yl)pentanoïque
H-γ⁴-DiH4Pal-OH acide (4R)-4-amino-5-(pyridin-4'-yl)pentanoïque
H-γ⁴-DiHPhg-OH acide (4R)-4-amino-4-phénylbutyrique
H-γ⁴-DiHCha-OH acide (4R)-4-amino-5-cyclohexylpentanoïque
H-γ⁴-DiHC₄al-OH acide (4R)-4-amino-5-cyclobutylpentanoïque
H-γ⁴-DiHC₅al-OH acide (4R)-4-amino-5-cyclopentylpentanoïque
H-γ⁴-DiHNle-OH acide (4S)-4-aminooctanoïque
H-γ⁴-DiH2Nal-OH acide (4S)-4-amino-5-(2'-naphtyl)pentanoïque
H-γ⁴-DiH1Nal-OH acide (4S)-4-amino-5-(1'-naphtyl)pentanoïque
H-γ⁴-DiH4ClPhe-OH acide (4R)-4-amino-5-(4'-chlorophényl)pentanoïque
H-γ⁴-DiH3ClPhe-OH acide (4R)-4-amino-5-(3'-chlorophényl)pentanoïque
H-γ⁴-DiH2ClPhe-OH acide (4R)-4-amino-5-(2'-chlorophényl)pentanoïque
H-γ⁴-DiH3,4Cl₂Phe-OH acide (4R)-4-amino-5-(3',4'-dichlorophényl)pentanoïque
H-γ⁴-DiH4FPhe-OH acide (4R)-4-amino-5-(4'-fluorophényl)pentanoïque
H-γ⁴-DiH3FPhe-OH acide (4R)-4-amino-4-(4'-fluorophényl)pentanoïque
H-γ⁴-DiH2FPhe-OH acide (4R)-4-amino-5-(2'-fluorophényl)pentanoïque
H-γ⁴-DiHThi-OH acide (4R)-4-amino-5-(2'-thiényl)pentanoïque
H-γ⁴-DiHTza-OH acide (4R)-4-amino-5-(2'-thiazolyl)pentanoïque
H-γ⁴-DiHMso-OH acide (4R)-4-amino-5-méthylsulfoxylpentanoïque
H-γ⁴-DiHAcLys-OH acide (4S)-8-acétylamino-4-aminooctanoïque
H-γ⁴-DiHDpr-OH acide (4R)-4,5-diaminopentanoïque
H-γ⁴-DiHA₂Bu-OH acide (4S)-4,5-diaminohexanoïque
H-γ⁴-DiHDbu-OH acide (4R)-4,5-diaminohexanoïque
H-γ⁴-DiHAib-OH acide 3-amino-3,3-diméthylpropionique
H-γ⁴-DiHCyp-OH acide (1'-aminocyclopentan-1'-yl)-3-propionique
H-γ⁴-DiHY(Bzl)-OH acide (4R)-4-amino-5-(4'-benzyloxyphényl)pentanoïque
H-γ⁴-DiHH(Bzl)-OH acide (4R)-4-amino-5-(1'-benzylimidazol-4'-yl)pentanoïque
H-γ⁴-DiHBip-OH acide (4R)-4-amino-5-diphénylylpentanoïque
H-γ⁴-DiHS(Bzl)-OH acide (4S)-4-amino-5-benzyloxypentanoïque
H-γ⁴-DiHT(Bzl)-OH acide (4R,5R)-4-amino-5-benzyloxyhexanoïque
H-γ⁴-DiHalloT-OH acide (4R,4S)-4-amino-5-hydroxyhexanoïque
H-γ⁴-DiHLeu3OH-OH acide (4R,5R)-4-amino-5-hydroxy-6-méthylheptanoïque
H-γ⁴-DiHhAla-OH acide (4S)-4-aminohexanoïque
H-γ⁴-DiHhArg-OH acide (4S)-4-amino-8-guanidinooctanoïque
H-γ⁴-DiHhCys-OH acide (4R)-amino-6-mercaptohexanoïque
H-γ⁴-DiHhGlu-OH acide (4S)-4-aminooctanedioïque
H-γ⁴-DiHhGln-OH acide (4S)-4-amino-7-carbamoylheptanoïque
H-γ⁴-DiHhHis-OH acide (4S)-4-amino-6-(imidazole-4'-yl)hexanoïque
H-γ⁴-DiHhIle-OH acide (4S,6S)-4-amino-6-méthyloctanoïque
H-γ⁴-DiHhLeu-OH acide (4S)-4-amino-7-méthyloctanoïque
H-γ⁴-DiHhNle-OH acide (4S)-4-aminononanoïque
H-γ⁴-DiAoc-OH acide (4S)-4,9-diaminononanoïque
H-γ⁴-DiHhMet-OH acide (4S)-4-amino-7-méthylthioheptanoïque
H-γ⁴-DiHhPe-OH acide (4R)-4-amino-6-phénylhexanoïque
H-γ⁴-DiHhSer-OH acide (4S)-4-amino-6-hydroxyhexanoïque
H-γ⁴-DiHhThr-OH acide (4R,6R)-4-amino-6-hydroxyheptanoïque
H-γ⁴-DiHhTrp-OH acide (4S)-4-amino-6-(indol-3'-yl)hexanoïque
H-γ⁴-DiHhTyr-OH acide (4S)-4-amino-6-(4'-hydroxyphényl)hexanoïque
H-γ⁴-DiHhCha-OH acide (4R)-4-amino-5-cyclohexylhexanoïque
H-γ⁴-DiHhBpa-OH acide (4R)-4-amino-5-(4'-benzoylphényl)pentanoïque
H-γ⁴-DiHOctG-OH acide (4S)-4-amino-dodécanoïque
H-γ⁴-DiHNle-OH acide (4S)-4-aminooctanoïque
H-γ⁴-DiHTic-OH acide (3R)-1',2',3',4'-tétrahydroisoquinolin-3'-yl-3-propionique
H-γ⁴-DiHTiq-OH acide (1'R)-1',2',3',4'-tétrahydroisoquinolin-1'-yl-3-propionique
H-γ⁴-DiHOic-OH acide (2'S,3'aS,7'aS)-1'-octahydro-1H-indol-2'-yl-3-propionique
H-γ⁴-DiH4AmPyrr1-OH acide (2'R,4'S)-4'-aminopyrrolidin-2'-yl-3-propionique
H-γ⁴-DiH4AmPyrr2-OH acide (2'R,4'R)-4'-aminopyrrolidin-2'-yl-3-propionique
H-γ⁴-DiH4PhePyrr1-OH acide (2'R,4'R)-4'-phénylpyrrolidin-2'-yl-3-propionique
H-γ⁴-DiH4PhePyrr2-OH acide (2'R,4'S)-4'-phénylpyrrolidin-2'-yl-3-propionique
H-γ⁴-DiH5PhePyrr1-OH acide (2'S,5'R)-5'-phénylpyrrolidin-2'-yl-3-propionique
H-γ⁴-DiH5PhePyrr2-OH acide (2'S,5'S)-5'-phénylpyrrolidin-2'-yl-3-propionique
H-γ⁴-DiH4Hyp1-OH acide (2'R,4'S)-4'-hydroxypyrrolidin-2'-yl-2-propionique
H-γ⁴-DiH4Hyp2-OH acide (2'R,4'R)-4'-hydroxypyrrolidin-2'-yl-3-propionique
H-γ⁴-DiH4Mp1-OH acide (2'R,4'S)-4'-mercaptopyrrolidin-2'-yl-3-propioniquen-prop
H-γ⁴-DiH4Mp2-OH acide (2'R,4'R)-4'-mercaptopyrrolidin-2'-yl-3-propionique
H-γ⁴-DiHPip-OH acide (2'S)-pipéridin-2'-yl-3-propionique
H-γ⁴-DiHPro-OH acide (2'S)-pyrrolidin-2'-yl-3-propionique
(AEt)G N-(2-aminoéthyl)glycine
(APr)G N-(3-amino-n-propyl)glycine
(ABu)G N-(4-amino-n-butyl)glycine
(APe)G N-(5-amino-n-pentyl)glycine
(GuEt)G N-(2-guanidinoéthyl)glycine
(GuPr)G N-(3-guanidino-n-propyl)glycine
(GuBu)G N-(4-guanidino-n-butyl)glycine
(GuPe)G N-(5-guanidino-n-pentyl)glycine
(Me)G N-méthylglycine
(Et)G N-éthylglycine
(Bu)G N-butylglycine
(Pe)G N-pentylglycine
(Ip)G N-isopropylglycine
(2MePr)G N-(2-méthylpropyl)glycine
(3MeBu)G N-(3-méthylbutyl)glycine
(1MePr)G (1S)-N-(1-méthylpropyl)glycine
(2MeBu)G (2S)-N-(2-méthylbutyl)glycine
(MthEt)G N-(méthylthioéthyl)glycine
(MthPr)G N-(méthylthiopropyl)glycine
(Ben)G N-benzylglycine
(PhEt)G N-(2-phényléthyl)glycine
(HphMe)G N-([4'-hydroxyphényl]méthyl)glycine
(HphEt)G N-(2-[4'-hydroxyphényl]éthyl)glycine
(ImMe)G N-(imidazol-5-yl-méthyl)glycine
(ImEt)G N-(2-(imidazol-5'-yl)-éthyl)glycine
(InMe)G N-(indol-2-yl-méthyl)glycine
(InEt)G N-(2-(indol-2'-yl)éthyl)glycine
(CboMe)G N-(carboxyméthyl)glycine
(CboEt)G N-(2-carboxyéthyl)glycine
(CboPr)G N-(3-carboxypropyl)glycine
(CbaMe)G N-(carbamoylméthyl)glycine
(CbaEt)G N-(2-carbamoyléthyl)glycine
(CbaPr)G N-(3-carbamoylpropyl)glycine
(HyEt)G N-(2-hydroxyéthyl)glycine
(HyPr)G (2R)-N-(2-hydroxypropyl)glycine, ou
(Mct)G N-(2-mercaptoéthyl)glycine.

8. Composés selon la revendication 1, choisis parmi :
(2S,11S,19aS)-N-[2-diméthylamino)éthyl]-15-fluoro-7,12-diméthyl-2-{[(1-napthylamino)carbonyl]amino}-5,8,13-trioxo-2,3,6,7,8,9,10,11,12,13,19,19a-dodécahydro-1H,5H-pyrrolo[2,1-c][1,4,7,12]benzoxatriazacyclopentadécine-11-carboxamide ;
(2S,11S,19aS)-N-(2-aminoéthyl)-15-fluoro-7,12-diméthyl-2-{[2-(1-napthyl)acétyl]amino}-5,8,13-trioxo-2,3,6,7,8,9,10,11,12,13,19,19a-dodécahydro-1H,5H-pyrrolo[2,1-c][1,4,7,12]benzoxatriazacyclopentadécine-11-carboxamide ;
(2S,11S,19aS)-N-(2-{[amino(imino)méthyl]éthyl}-15-fluoro-7,12-diméthyl-2-{[2-(1-napthyl)acétyl]amino}-5,8,13-trioxo-2,3,6,7,8,9,10,11,12,13,19,19a-dodécahydro-1H,5H-pyrrolo[2,1-c][1,4,7,12]benzoxatriazacyclopentadécine-11-carboxamide ;
(2S,11S,19aS)-15-fluoro-7,12-diméthyl-2-{[2-(1-napthyl)acétyl]amino}-5,8,13-trioxo-2,3,6,7,8,9,10,11,12,13,19,19a-dodécahydro-1H,5H-pyrrolo[2,1-c][1,4,7,12]benzoxatriazacyclopentadécine-11-carboxamide ;
(2S,11S,19aS)-N-[2-(diméthylamino)éthyl]-15-fluoro-7,12-diméthyl-2-{[2-(1-napthyl)éthyl]amino}-5,8,13-trioxo-2,3,6,7,8,9,10,11,12,13,19,19a-dodécahydro-1H,5H-pyrrolo[2,1-c][1,4,7,12]benzoxatriazacyclopentadécine-11-carboxamide ;
(2S,11S,19aS)-N-[2-(acétylamino)éthyl]-15-fluoro-7,12-diméthyl-2-[2-(1-naphtyl)éthyl]amino-5,8,13-trioxo-2,3,6,7,8,9,10,11,12,13,19,19a-dodécahydro-1H,5H-pyrrolo[2,1-c][1,4,7,12]benzoxatriazacyclopentadécine-11-carboxamide ;
(2S,11S,19aS)-N-[2-(diméthylamino)éthyl]-15-fluoro-7,12-diméthyl-2-[2-(1-naphtyl)acétyl]amino-5,8,13-trioxo-2,3,6,7,8,9,10,11,12,13,19,19a-dodécahydro-1H,5H-pyrrolo[2,1-c][1,4,7,12]benzoxatriazacyclopentadécine-11-carboxamide ;
(2S,11S,19aS)-N-[2-(diméthylamino)éthyl]-15-fluoro-7,12-diméthyl-5,8,13-trioxo-2-(2-[3-(trifluorométhyl)phényl]acétylamino)-2,3,6,7,8,9,10,11,12,13,19,19a-dodécahydro-1H,5H-pyrrolo[2,1-c][1,4,7,12]benzoxatriazacyclopentadécine-11-carboxamide ;
(2S,11S,19aS)-N-[2-(diméthylamino)éthyl]-15-fluoro-2-[2-(3-méthoxyphényl)acétyl]amino-7,12-diméthyl-5,8,13-trioxo-2,3,6,7,8,9,10,11,12,13,19,19a-dodécahydro-1H,5H-pyrrolo[2,1-c][1,4,7,12]benzoxatriazacyclopentadécine-11-carboxamide ;
(2S,11S,19aS)-N-[3-(diméthylamino)propyl]-15-fluoro-7,12-diméthyl-2-[2-(1-naphtyl)acétyl]amino-5,8,13-trioxo-2,3,6,7,8,9,10,11,12,13,19,19a-dodécahydro-1H,5H-pyrrolo[2,1-c][1,4,7,12]benzoxatriazacyclopentadécine-11-carboxamide ;
(2S,11S,19aS)-15-fluoro-7,12-diméthyl-2-[2-(1-naphtyl)acétyl]amino-5,8,13-trioxo-N-(3-pyridinylméthyl)-2,3,6,7,8,9,10,11,12,13,19,19a-dodécahydro-1H,5H-pyrrolo[2,1-c][1,4,7,12]benzoxatriazacyclopentadécine-11-carboxamide ;
N-[(4S,6S,10S)-14-méthyl-6-[2-(2-naphtyl)acétyl]amino-9,15-dioxo-2-oxa-8,14-diazatricyclo[14.3.1.0^{4,8}]icosa-1(20),16,18-trién-10-yl]décanamide ;
N-[(4S,6S,10S)-14-méthyl-10-{[2-(2-naphtyl)acétyl]amino}-9,15-dioxo-2-oxa-8,14-diazatricyclo[14.3.1.0^{4,8}]icosa-1(20),16,18-trién-6-yl]-2-(2-naphtyl)acétamide ;
N-[(4S,6S,10S)-14-méthyl-6-[2-(2-naphtyl)acétyl]amino-9,15-dioxo-2-oxa-8,14-diazatricyclo[14.3.1.0^{4,8}]icosa-1(20),16,18-trién-10-yl]-2-(1-naphtyl)acétamide ;
N-[(4S,6S,10S)-14-méthyl-9,15-dioxo-10-[(3-toluidinocarbonyl)amino]-2-oxa-8,14-diazatricyclo[14.3.1.0^{4,8}]icosa-1(20),16,18-trién-6-yl]-2-(2-naphtyl)acétamide ;
N-[(4S,6S,10S)-10-([3-(diméthylamino)anilino]carbonylamino)-14-méthyl-9,15-dioxo-2-oxa-8,14-diazatricyclo[14.3.1.0^{4,8}]icosa-1(20),16,18-trién-6-yl]-2-(2-naphtyl)acétamide ;
N-[(4S,6S,10S)-6-({[3-(diméthylamino)aniline]carbonyl}amino)-14-méthyl-9,15-dioxo-2-oxa-8,14-diazatricyclo[14.3.1.0^{4,8}]icosa-1(20),16,18-trién-10-yl]-2-(2-naphtyl)acétamide ;
N-[(4S,6S,10S)-14-méthyl-9,15-dioxo-6-[(3-phénylpropanoyl)amino]-2-oxa-8,14-diazatricyclo[14.3.1.0^{4,8}]icosa-1(20),16,18-trién-10-yl]décanamide ;
N-[(4S,6S,10S)-14-méthyl-6-[(3-morpholinobenzoyl)amino]-9,15-dioxo-2-oxa-8,14-diazatricyclo[14.3.1.0^{4,8}]icosa-1(20),16,18-trién-10-yl]décanamide ;
N-[(4S,6S,10S)-6-({[3-(diméthylamino)anilino]carbonyl}amino)-14-méthyl-9,15-dioxo-2-oxa-8,14-diazatricyclo[14.3.1.0^{4,8}]icosa-1(20),16,18-trién-10-yl]décanamide ;
N-[(4S,6S,10S)-6-({[3-(diméthylamino)anilino]carbonyl}amino)-14-méthyl-9,15-dioxo-2-oxa-8,14-diazatricyclo[14.3.1.0^{4,8}]icosa-1(20),16,18-trién-10-yl]-2-(1-naphtyl)acétamide ;
N-[(4S,6S,10S)-6-{[(3-méthoxyanilino)carbonyl]amino}-14-méthyl-9,15-dioxo-2-oxa-8,14-diazatricyclo[14.3.1.0^{4,8}]icosa-1(20),16,18-trién-10-yl]-2-(2-naphtyl)acétamide ;
N-[(4S,6S,10S)-14-méthyl-9,15-dioxo-6-{[2-(3-phénoxyphényl)acétyl]amino}-2-oxa-8,14-diazatricyclo[14.3.1.0^{4,8}]icosa-1(20),16,18-trién-10-yl]décanamide ;
N-[(4S,6S,10S)-14-méthyl-6-{[2-(2-naphtyl)acétyl]amino]-9,15-dioxo-2-oxa-8,14-diazatricyclo[14.3.1.0^{4,8}]icosa-1(20),16,18-trién-10-yl]-6-phénylhexamide ;
N-[(4S,6S,10S)-14-méthyl-6-{[2-(2-naphtyl)acétyl]amino]-9,15-dioxo-2-oxa-8,14-diazatricyclo[14.3.1.0^{4,8}]icosa-1(20),16,18-trién-10-yl]-5-phénoxypentanamide ;
(E)-N-[(4S,6S,10S)-14-méthyl-6-[2-(2-naphtyl)acétyl]amino-9,15-dioxo-2-oxa-8,14-diazatricyclo[14.3.1.0^{4,8}]icosa-1(20),16,18-trién-10-yl]-3-décénamide ;
N-[(4S,6S,10S)-14-méthyl-6-{[2-(1-naphtyl)acétyl]amino]-9,15-dioxo-2-oxa-8,14-diazatricyclo[14.3.1.0^{4,8}]icosa-1(20),16,18-trién-10-yl]décanamide ;
6-benzyloxy-N-[(4S,6S,10S)-14-méthyl-6-[2-(2-naphtyl)acétyl]amino-9,15-dioxo-2-oxa-8,14-diazatricyclo[14.3.1.0^{4,8}]icosa-1(20),16,18-trién-10-yl]hexanamide ;
2-(1,1'-diphényl]-4-yl-N-[(4S,6S,10S)-14-méthyl-6-{[2-(2-naphtyl)acétyl]amino]-9,15-dioxo-2-oxa-8,14-diazatricyclo[14.3.1.0^{4,8}]icosa-1(20),16,18-trién-10-yl]acétamide ;
N-[(4S,6S,10S)-14-méthyl-6-{[2-(2-naphtyl)acétyl]amino]-9,15-dioxo-2-oxa-8,14-diazatricyclo[14.3.1.0^{4,8}]icosa-1(20),16,18-trién-10-yl]-3,3-diphénylpropanamide;
6-benzyloxy-N-[(4S,6S,10S)-14-méthyl-9,15-dioxo-10-[(6-phénylhexanoyl)amino]-2-oxa-8,14-diazatricyclo[14.3.1.0^{4,8}]icosa-1(20),16,18-trién-6-yl]hexanamide ;
2-(1,1'-diphényl)-3-yl-N-[(4S,6S,10S)-6-([3-diméthylamino)aniline]carbonylamino)-14-méthyl-9,15-dioxo-2-oxa-8,14-diazatricyclo[14.3.1.0^{4,8}]icosa-1(20),16,18-trién-10-yl]acétamide ;
2-(1,1'-diphényl)-4-yl-N-[(4S,6S,10S)-6-([3-diméthylamino)aniline]carbonylamino)-14-méthyl-9,15-dioxo-2-oxa-8,14-diazatricyclo[14.3.1.0^{4,8}]icosa-1(20),16,18-trién-10-yl]acétamide ;
N-[(4S,6S,10S)-10-[([1,1'-diphényl]-4-ylsulfonyl)amino]-14-méthyl-2-oxa-8,14-diazatricyclo[14.3.1.0^{4,8}]icosa-1(20),16,18-trién-6-yl]-2-(naphtyl)acétamide ;
N-[(4S,6S,10S)-6-{[(3-fluoroanilino)carbonyl]amino}-14-méthyl-9,15-dioxo-2-oxa-8,14-diazatricyclo[14.3.1.0^{4,8}]icosa-1(20),16,18-trién-10-yl]-2-(2-naphtyl)acétamide ;
N-[(4S,6S,10S)-6-({[4-(diméthylamino)anilino]carbonyl}amino)-14-méthyl-9,15-dioxo-2-oxa-8,14-diazatricyclo[14.3.1.0^{4,8}]icosa-1(20),16,18-trién-10-yl]-2-(2-naphtyl)acétamide ;
phényl-N-[(4S,6S,10S)-14-méthyl-10-{[2-(2-naphtyl)acétyl]amino}-9,15-dioxo-2-oxa-8,14-diazatricyclo[14.3.1.0^{4,8}]icosa-1(20),16,18-trién-6-yl]carbamate ;
N-[(4S,6S,10S)-14-méthyl-9,15-dioxo-6-[2(7-quinolinyl)acétyl]amino-2-oxa-8,14-diazatricyclo[14.3.1.0^{4,8}]icosa-1(20),16,18-trién-10-yl]-2-(2-naphtyl)acétamide ;
N-[(4S,6S,10S)-14-méthyl-9,15-dioxo-6-{[2-(3-phénoxyphényl)acétyl]amino}-2-oxa-8,14-diazatricyclo[14.3.1.0^{4,8}]icosa-1(20),16,18-trién-10-yl]-2-(2-naphtyl)acétamide ;
N-[(4S,6S,10S)-14-méthyl-10-{[2-(2-naphtyl)acétyl]amino}-9,15-dioxo-2-oxa-8,14-diazatricyclo[14.3.1.0^{4,8}]icosa-1(20),16,18-trién-6-yl]décanamide ;
N-[(4S,6S,10S)-14-méthyl-10-{[2-(1-naphtyl)acétyl]amino}-9,15-dioxo-2-oxa-8,14-diazatricyclo[14.3.1.0^{4,8}]icosa-1(20),16,18-trién-6-yl]décanamide ;
N-[(4S,6S,10S)-14-méthyl-10-{[2-(1-naphtyl)acétyl]amino}-9,15-dioxo-2-oxa-8,14-diazatricyclo[14.3.1.0^{4,8}]icosa-1(20),16,18-trién-6-yl]hexanamide ;
2-(1,1'-diphényl)-4-yl-N-[(4S,6S,10S)-14-méthyl-10-{[2-(1-naphtyl)acétyl]amino}-9,15-dioxo-2-oxa-8,14-diazatricyclo[14.3.1.0^{4,8}]icosa-1(20),16,18-trién-6-yl]acétamide ;
N-[(4S,6S,10S)-10-[(2-[1,1'-diphényl]-4-ylacétyl)amino]-14-méthyl-9,15-dioxo-2-oxa-8,14-diazatricyclo[14.3.1.0^{4,8}]icosa-1(20),16,18-trién-6-yl]-2,2-diphénylacétamide ;
N-[(4S,6S,10S)-6-[(2-[1,1'-diphényl]-4-ylacétyl)amino]-14-méthyl-9,15-dioxo-2-oxa-8,14-diazatricyclo[14.3.1.0^{4,8}]icosa-1(20),16,18-trién-6-yl]-2,2-diphénylacétamide ;
N-[(4S,6S,10S)-6-[(2,2-diphénylacétyl)amino]-14-méthyl-9,15-dioxo-2-oxa-8,14-diazatricyclo[14.3.1.0^{4,8}]icosa-1(20),16,18-trién-6-yl]décanamide ;
N-[(4S,6S,10S)-14-méthyl-10-{[2-(1-naphtyl)acétyl]amino}-9,15-dioxo-2-oxa-8,14-diazatricyclo[14.3.1.0^{4,8}]icosa-1(20),16,18-trién-6-yl]-2,2-diphénylacétamide ;
N-[(4S,6S,10S)-6-[(3,3-diphénylpropanoyl)amino]-14-méthyl-9,15-dioxo-2-oxa-8,14-diazatricyclo[14.3.1.0^{4,8}]icosa-1(20),16,18-trién-6-yl]décanamide ;
2-(1,1'-diphényl)-4-yl-N-[(4S,6S,10S)-14-méthyl-10-[(2-[1,1'-diphényl]-4-ylacétyl)amino]-14-méthyl-9,15-dioxo-2-oxa-8,14-diazatricyclo[14.3.1.0^{4,8}]icosa-1(20),16,18-trién-6-yl]acétamide ;
N-[(4S,6S,10S)-6-({[3-(diméthylamino)anilino]carbonyl}amino)-14-méthyl-9,15-dioxo-2-oxa-8,14-diazatricyclo[14.3.1.0^{4,8}]icosa-1(20),16,18-trién-10-yl]-3,3-diméthylbutanamide ;
N-[(4S,6S,10S)-6-({[3-(diméthylamino)anilino]carbonyl}amino)-9,15-dioxo-2-oxa-8,14-diazatricyclo[14.3.1.0^{4,8}]icosa-1(20),16,18-trién-10-yl]-2-(2-naphtyl)acétamide ;
benzyl-N-[(4S,6S,10S)-6-{[2-(2-naphtyl)acétyl]amino}-9,15-dioxo-2-oxa-8,14-diazatricyclo[14.3.1.0^{4,8}]icosa-1(20),16,18-trién-10-yl]carbamate ;
N-[(4S,6S,10S)-6-{[2-(2-naphtyl)acétyl]amino}-9,15-dioxo-2-oxa-8,14-diazatricyclo[14.3.1.0^{4,8}]icosa-1(20),16,18-trién-10-yl]décanamide ;
(4S,6R,15S)-11,16-diméthyl-N-(2-naphtylméthyl)-9,12-17-trioxo-6-{[3-(4-pyridinyl)propanoyl]amino}-2-thia-8,11,16-triazatricyclo[16.3.1.0^{4,8}]docosa-1(22),18,20-trién-15-carboxamide ;
tert-butyl-N-[(4S,6R,15S)-11,16-diméthyl-15-{[2-naphtylméthyl)amino]carbonyl}-9,12,17-trioxo-2-thia-8,11,16-triazatricyclo[16.3.1.0^{4,8}]docosa-1(22),18,20-trién-6-yl]carbamate ;
(4S,6R,15S)-11,16-diméthyl-N-(2-naphtylméthyl)-9,12-17-trioxo-6-(pentanoylamino)-2-thia-8,11,16-triazatricyclo[16.3.1.0^{4,8}]docosa-1(22),18,20-trién-15-carboxamide ;
(4S,6R,15S)-6-acétylamino-11,16-diméthyl-N-(2-naphtylméthyl)-9,12-17-trioxo-2-thia-8,11,16-triazatricyclo[16.3.1.0^{4,8}]docosa-1(22),18,20-trién-15-carboxamide ;
(2S-11S-19aS)-15-fluoro-7,12-diméthyl-2-(1-naphtoylamino)-N-(2-naphtylméthyl)-5,8,13-trioxo-2,3,6,7,8,9,10,11,12,13,19,19a-dodécahydro-1H,5H-pyrrolo[2,1-c][1,4,7,12]benzoxatriazacyclopentadécine-11-carboxamide ;
(2S-11S-19aS)-15-fluoro-7,12-diméthyl-2-[2-(2-naphtyl)acétyl]amino-N-(1-naphtylméthyl)-5,8,13-trioxo-2,3,6,7,8,9,10,11,12,13,19,19a-dodécahydro-1H,5H-pyrrolo[2,1-c][1,4,7,12]benzoxatriazacyclopentadécine-11-carboxamide ;
(2S-11S-19aS)-2-[(3-chlorobenzoyl)amino]-15-fluoro-7,12-diméthyl-N-(1-naphtylméthyl)-5,8,13-trioxo-2,3,6,7,8,9,10,11,12,13,19,19a-dodécahydro-1H,5H-pyrrolo[2,1-c] [1,4,7,12] benzoxatriazacyclopentadécine-11-carboxamide.

9. Composés selon l'une quelconque des revendications 1 à 8 destiné à être utilisé comme substances thérapeutiquement actives.

10. Composés selon la revendication 9 destinés à être utilisés comme substances thérapeutiquement actives ayant une activité agoniste ou antagoniste de l'activité du récepteur de la motiline (récepteur MR), du récepteur de la sérotonine du sous-type 5-HT_{2B} (récepteur 5-HT_{2B}), du récepteur de la prostaglandine F2α (récepteur FP), du purinorécepteur P2Y₁, du canal à potassium tensiodépendant Kᵥ1.3 ou de la voie canonique Wnt (dépendante de la caténine β).

11. Composition pharmaceutique contenant un composé ou un mélange de composés selon l'une quelconque des revendications 1 à 8 et au moins un excipient thérapeutiquement inerte.

12. Composition selon la revendication 11 contenant un composé ou un mélange de composés selon l'une quelconque des revendications 1 à 8 ayant une activité agoniste ou antagoniste de l'activité du récepteur de la motiline (récepteur MR), du récepteur de la sérotonine du sous-type 5-HT_{2B} (récepteur 5-HT_{2B}), du récepteur de la prostaglandine F2α (récepteur FP), du purinorécepteur P2Y₁, du canal à potassium tensiodépendant Kᵥ1.3 ou de la voie canonique Wnt (dépendante de la caténine β).

13. Composition selon la revendication 11 ou 12 convenant à une administration orale, topique, transdermique, par injection, buccale, transmucosale, pulmonaire ou par inhalation, en particulier sous forme de comprimés, dragées, capsules, solutions, liquides, gels, emplâtres, crèmes, onguents, sirops, suspensions, sprays, vaporisateurs ou suppositoires.

14. Utilisation de composés selon l'une quelconque des revendications 1 à 8 ayant une activité agoniste ou antagoniste de l'activité du récepteur de la motiline (récepteur MR), du récepteur de la sérotonine du sous-type 5-HT_{2B} (récepteur 5-HT_{2B}), du récepteur de la prostaglandine F2α (récepteur FP), du purinorécepteur P2Y₁, du canal à potassium tensiodépendant Kᵥ1.3 ou de la voie canonique Wnt (dépendante de la caténine β) dans la fabrication d'un médicament.

15. Utilisation de composés selon l'une quelconque des revendications 1 à 8 pour la fabrication d'un médicament, ledit médicament étant destiné au traitement de troubles de la motilité du conduit gastro-intestinal, de maladies du système nerveux central, de l'hypertension oculaire, de troubles inflammatoires du système nerveux central, de troubles thromboemboliques, de troubles de la prolifération cellulaire ou de troubles en lien avec les os et les cartilages.

16. Utilisation de composés selon l'une quelconque des revendications 1 à 8 pour la fabrication d'un médicament, ledit médicament étant destiné au traitement de troubles selon la revendication 15, ces troubles étant choisis parmi la dyspepsie fonctionnelle, la gastroparésie diabétique ou une constipation du type de la maladie du côlon irritable, la migraine, la schizophrénie, la psychose, la dépression ou la maladie d'Alzheimer, le glaucome, le travail prématuré ou la sclérose en plaques.

17. Utilisation de composés selon l'une quelconque des revendications 1 à 9 pour la découverte de pistes pharmaceutiques.
